# EUROPEAN PATENT SPECIFICATION

(11) **EP 4 041 714 B1**
(45) Date of publication and mention of the grant of the patent: **13.12.2023**
(21) Application number: 20793814.3
(22) Date of filing: 09.10.2020
(51) Int. Cl.: C07D 231/12, C07D 233/64, C07D 213/56, C07D 471/04, C07D 241/12, C07D 401/12, C07D 403/12, C07D 417/12, C07D 277/30, A61K 31/4164, A61K 31/426, A61K 31/4418, A61K 31/4439, A61K 31/4965, A61K 31/497, A61P 35/00, A61K 31/4196

(54) **BI-ARYL DIHYDROOROTATE DEHYDROGENASE INHIBITORS**
BI-ARYL-DIHYDROOROTAT-DEHYDROGENASE-INHIBITOREN
INHIBITEURS DE LA DIHYDROOROTATE DÉSHYDROGÉNASE BI-ARYLE

(30) Priority: 10.10.2019 US 201962913281 P
(43) Date of publication of application: 17.08.2022
(73) Proprietor: Janssen Biotech, Inc., Horsham, PA 19044 (US)
(72) Inventor: CISAR, Justin, Spring House, Pennsylvania 19477 (US); KUDUK, Scott, Spring House, Pennsylvania 19477 (US); DERATT, Lindsey, Spring House, Pennsylvania 19477 (US); SIMONNET, Yvan Rene Ferdinand, Spring House, Pennsylvania 19477 (US)
(74) Representative: Carpmaels & Ransford LLP
(86) International application number: PCT/IB2020/059503
(87) International publication number: WO 2021/070132

(56) References cited:
- WO-A1-2018/077923
- CHRISTIAN SVEN ET AL: "The novel dihydroorotate dehydrogenase (DHODH) inhibitor BAY 2402234 triggers differentiation and is effective in the treatment of myeloid malignancies", BLOOD CANCER JOURNAL, NATURE PUBLISHING GROUP UK, LONDON, vol. 33, no. 10, 2 April 2019 (2019-04-02) , pages 2403-2415, XP036897088, ISSN: 0887-6924, DOI: 10.1038/S41375-019-0461-5 [retrieved on 2019-04-02]

## Description

### FIELD OF THE INVENTION

The present invention relates to novel compounds that are dihydroorotate dehydrogenase (DHODH) inhibitors. These compounds may be useful for the treatment of a disease, disorder, or medical condition where there is an advantage in inhibiting DHODH. The invention also relates to pharmaceutical compositions comprising one or more of such compounds, to processes to prepare such compounds and compositions, and to such compounds or pharmaceutical compositions for use in the treatment of cancer, and autoimmune and inflammatory diseases, syndromes, and disorders.

### BACKGROUND OF THE INVENTION

Acute myelogenous leukemia (AML) is a clonal disease of the blood and bone marrow resulting from mutations that occur in normal hematopoietic stem cells. AML is a heterogenous disease in that it presents with a range of cytogenetic, morphological and immunophenotypic features, and is characterized by an accumulation of clonal, abnormal myeloid progenitor cells, known as myeloblasts. These cells demonstrate disruption of normal myeloid differentiation and excessive proliferation, resulting in the decreased formation of hematopoietic cells. Disease remission can be achieved with standard induction chemotherapy, but refractory and relapsed disease remains a challenge due to persistence of leukemic stem cells. Therefore, AML represents an unmet medical need with >20,000 new cases per year in the US with 5-year overall survival below 30% (Stein ET et al., Health Qual Life Outcomes 16: 193, 2018).

Differentiation therapy is considered an attractive approach to AML treatment based on the knowledge that differentiation and loss of stem cell self-renewal are coupled in normal cells. Treatment of acute promyelocytic leukemia, which represents 10-15% of all AML, with all-trans retinoic acid is the paradigm for differentiation therapy. Retinoic acid targets the promyelocytic leukemia protein (PML)-retinoic acid receptor-α (RAR-α) fusion protein encoded by a t(15,17) chromosomal translocation. Targeting PML-RAR specifically lifts the transcriptionally mediated differentiation block induced by the fusion protein and early clinical trials with single agentATRA demonstrated complete hematologic remission in all treated patients (McCulloch D et al. Onco Targets Ther 2017; 10: 1585-1601; Nowak D et al. Blood 113: 3655, 2009).

Although differentiation therapy is successful, it is only applicable to a small population of AML patients. Research efforts have aimed at identifying additional differentiation inducing agents, but with limited success. Recently dihydroorotate dehydrogenase (DHODH) emerged as a potentially more broadly applicable differentiation target in a phenotypic screen aimed at identifying small molecules that overcome blockade of the maturation of primary murine bone marrow cells expressing the homeobox protein HoxA9. This protein is a key transcription factor involved in balancing stem cell maintenance/differentiation and is normally expressed in hematopoietic progenitor cells and downregulated upon induction of differentiation and has been found to be widely overexpressed in AML (Sykes et al., Cell 167: 171, 2016).

DHODH is a flavin mononucleotide (FMN) flavoprotein located in the inner mitochondrial membrane that catalyzes the oxidation of dihydroorotate to orotate, the fourth step in the de novo pyrimidine biosynthesis pathway. Inhibition of DHODH leads to decreased pyrimidine synthesis important precursors for nucleotide synthesis, but also glycoprotein and phospholipid biosynthesis (Reis RAG et al., Archives Biochem Biophysics 632: 175, 2017; Vyas VK et al., Mini Rev Med Chem 11: 1039, 2011). DHODH is a validated target for the treatment of autoimmune diseases with the FDA approved small molecule DHODH inhibitors leflunomide and teriflunomide for rheumatoid arthritis and multiple sclerosis, respectively (Lolli ML et al., Recent patents on AntiCancer Drug Discovery 13: 86, 2018).

Since the first observation by Sykes et al. demonstrating that DHODH inhibition drives AML differentiation in vitro, as evidenced by upregulation of the differentiation markers CD11b and CD14, and results in dose dependent anti-leukemic effects, decreased leukemic stem cells and prolonged survival in vivo, additional evidence emerged demonstrating that small molecule DHODH inhibitors mediate antiproliferative activity against AML cells with concomitant cell cycle arrest, upregulation of CD11b and CD14, and induction of apoptosis (Wu D et al.. Haematologica 103: 1472, 2018; Sainas S et al., J Med Chem 61: 6034, 2018; Cao L et al., Mol Cancer Ther, October 23rd Epub ahead of print). Moreover, preclinical solid tumor in vitro and in vivo models demonstrated effectiveness of DHODH inhibition and DHODH was identified as a synthetic lethality in PTEN and KRAS mutant solid tumors (Pharmacology and Therapeutics, Epub October 19th, 2018; Mathur D et al., Cancer Discovery 7: 1, 2017; Cell Chemical Biology 25: 1, 2018).

Thus, there remains a need for DHODH inhibitors that provide a therapeutic benefit to patients suffering from cancer and/or inflammatory and immunological diseases.

### SUMMARY OF THE INVENTION

Embodiments of the present invention relate to compounds, pharmaceutical compositions containing them, methods of making and purifying them, and compounds and pharmaceutical compositions comprising one or more of such compounds for use in treating a subject suffering from or diagnosed with a disease, disorder, or medical condition comprising inhibiting or altering DHODH enzymatic activity, wherein the disease, disorder, or medical condition is an autoimmune or inflammatory disorder, or cancer.

Embodiments of this invention include a compound of Formula I 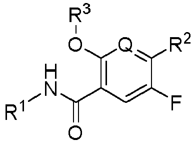 wherein
Q is CH or N;
R¹ is 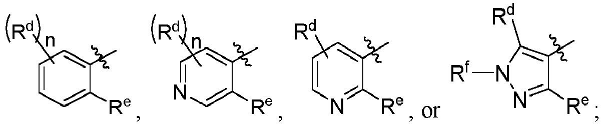 wherein
   R^{d} is selected from the group consisting of: -H, -Cl, -F, and -C(₁₋₃)alkyl; wherein said -C₍₁₋₃)alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, and -C(₁₋₃)alkyl; wherein said -C₍₁₋₃₎alkyl is optionally substituted with: -OH, -OCH₃, -SCH₃, or -OCF₃; and wherein said -C₍₁₋₃)alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is
   imidazolyl; wherein said imidazolyl is optionally substituted with up to three substituents independently selected from the group comprising -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -NH₂, -NHSO₂CH₃, -NHC(O)CH₃, - OH, -CO₂H, -Cl, or R^{a};
   pyrazinonyl; wherein said pyrazinonyl is optionally substituted with one or two groups independently selected from the group comprising R^{a};
   thiazolyl; wherein said thiazolyl is optionally substituted with one or two substituents independently selected from the group comprising R^{a};
   5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
   pyridinyl; wherein said pyridinyl is optionally substituted with one or two substituents independently selected from the group comprising R^{a}, -Cl, or -CO₂H;
   pyridazinyl; wherein said pyridazinyl is optionally substituted with one or two groups independently selected from the group comprising R^{a}, -Cl, or -CO₂H;
   4,5,6,7-tetrahydrothiazolo[5,4-*c*]pyridinyl; wherein said 4,5,6,7-tetrahydrothiazolo[5,4-c]pyridinyl is optionally substituted with R^{a};
   pyrazolyl; wherein said pyrazolyl is optionally substituted with one or two substituents independently selected from the group comprising R^{a};
   oxazolyl; wherein said oxazolyl is optionally substituted with one or two substituents independently selected from the group comprising R^{a};
   1,3,4-oxadiazolyl; wherein said 1,3,4-oxadiazolyl is optionally substituted with R^{a};
   5,6,7,8-tetrahydroimidazo[1,5-*a*]pyridinyl; wherein said 5,6,7,8-tetrahydroimidazo[1,5-*a*]pyridinyl is optionally substituted with R^{a};
   1,2,4-thiadiazolyl; wherein said 1,2,4-thiadiazolyl is optionally substituted with R^{a};
   5,6,7,8-tetrahydro-1,7-naphthyridinyl; wherein said 5,6,7,8-tetrahydro-1,7-naphthyridinyl is optionally substituted with R^{a}; or
   5,6,7,8-tetrahydro-1,6-naphthyridinyl; wherein said 5,6,7,8-tetrahydro-1,6-naphthyridinyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -Cl, -OH or up to three fluorine atoms; and wherein said cyclopropyl is optionally substituted with -OH, -CH₂OH, or up to two fluorine atoms;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said -C₍₁₋₆)alkyl is optionally substituted with up to six fluorine atoms;
or a pharmaceutically acceptable salt, solvate, stereoisomer, isotopic variant, or N-oxide thereof.

The present invention further provides a compound of Formula (I) or a pharmaceutically acceptable salt, isotope, N-oxide, solvate, or stereoisomer thereof, for use in treating or ameliorating a disease, syndrome, condition, or disorder in a subject, including a mammal and/or human in which the disease, syndrome, condition, or disorder is affected by the inhibition of DHODH enzymatic activity, including but not limited to, cancer and/or inflammatory or immunological diseases.

Additional embodiments, features, and advantages of the invention will be apparent from the following detailed description and through practice of the invention.

### DETAILED DESCRIPTION OF THE INVENTION

Unless defined otherwise, all technical and scientific terms used herein have the same meaning as is commonly understood by one of ordinary skill in art. As used in the specification and the appended claims, unless specified to the contrary, the following terms have the meaning indicated in order to facilitate the understanding of the present invention.

The singular forms "a", "an" and "the" encompass plural references unless the context clearly indicates otherwise.

Unless qualified specifically in particular instances of use, the term "alkyl" refers to a straight- or branched-chain alkyl group having from 1 to 8 carbon atoms in the chain. Examples of alkyl groups include methyl (Me), ethyl (Et), n-propyl, isopropyl, butyl, isobutyl, sec-butyl, tert-butyl (tBu), pentyl, isopentyl, tert-pentyl, hexyl, isohexyl, and groups that in light of the ordinary skill in the art and the teachings provided herein would be considered equivalent to any one of the foregoing examples.

The term Cₙ₋ₘ alkyl refers to an aliphatic chain, whether straight or branched, with a total number N of carbon members in the chain that satisfies n ≤ N ≤ m, with m > n. For example and without limitation, the term "C₁₋₆alkyl" refers to straight- or branched-chain alkyl group having from 1 to 6 carbon atoms in the chain. "C₁₋₄alkyl" refers to straight- or branched-chain alkyl group having from 1 to 4 carbon atoms in the chain.

The term "cycloalkyl" refers to a saturated or partially saturated, monocyclic, fused polycyclic, or spiro polycyclic carbocycle having from 3 to 12 ring atoms per carbocycle. "C₃-₆cycloalkyl" refers to a carbocycle having from 3 to 6 ring atoms per carbocycle. Illustrative examples of cycloalkyl groups include the following entities, in the form of properly bonded moieties:

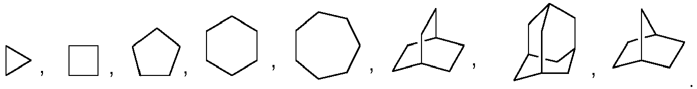

The term "imidazolyl" refers to the radical formed by removing a hydrogen atom from imidazole; for greater clarity, the term "imidazole" refers to

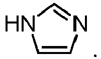

The term "pyrazinonyl" refers to the radical formed by removing a hydrogen atom from pyrazinone; for greater clarity, the term "pyrazinone" refers to

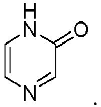

The term "triazolyl" refers to the radical formed by removing a hydrogen atom from triazole; for greater clarity, the term "triazole" refers to

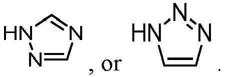

The term "pyrazinyl" refers to the radical formed by removing a hydrogen atom from pyrazine; for greater clarity, the term "pyrazine" refers to

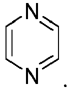

The term "thiazolyl" refers to the radical formed by removing a hydrogen atom from thiazole; for greater clarity, the term "thiazole" refers to

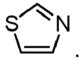

The term "5,6,7,8-tetrahydro-[1,2,4]triazolo[4,3-*a*]pyrazinyl" refers to the radical formed by removing a hydrogen atom from 5,6,7,8-tetrahydro-[1,2,4]triazolo[4,3-*a*]pyrazine; for greater clarity, the term "5,6,7,8-tetrahydro-[1,2,4]triazolo[4,3-*a*]pyrazine" refers to

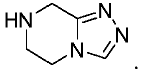

The term "pyridinyl" refers to the radical formed by removing a hydrogen atom from pyridine; for greater clarity, the term "pyridine" refers to

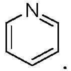

The term "pyridazinyl" refers to the radical formed by removing a hydrogen atom from pyridazine; for greater clarity, the term "pyridazine" refers to

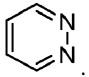

The term "4,5,6,7-tetrahydrothiazolo[5,4-c]pyridinyl" refers to the radical formed by removing a hydrogen atom from 4,5,6,7-tetrahydrothiazolo[5,4-*c*]pyridine; for greater clarity, the term "4,5,6,7-tetrahydrothiazolo[5,4-*c*]pyridine" refers to

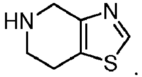

The term "pyrazolyl" refers to the radical formed by removing a hydrogen atom from pyrazole; for greater clarity, the term "pyrazole" refers to

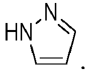

The term "oxazolyl" refers to the radical formed by removing a hydrogen atom from oxazole; for greater clarity, the term "oxazole" refers to

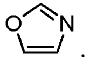

The term "1,3,4-oxadiazolyl" refers to the radical formed by removing a hydrogen atom from 1,3,4-oxadiazole; for greater clarity, the term "1,3,4-oxadiazole" refers to

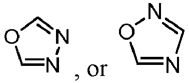

The term "5,6,7,8-tetrahydroimidazo[1,5-*a*]pyridinyl" refers to the radical formed by removing a hydrogen atom from 5,6,7,8-tetrahydroimidazo[1,5-*a*]pyridine; for greater clarity, the term "5,6,7,8-tetrahydroimidazo[1,5-*a*]pyridine" refers to

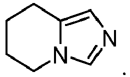

The term "1,2,4-thiadiazolyl" refers to the radical formed by removing a hydrogen atom from 1,2,4-thiadiazole; for greater clarity, the term "1,2,4-thiadiazole" refers to

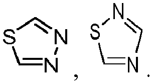

The term "5,6,7,8-tetrahydro-1,7-naphthyridinyl" refers to the radical formed by removing a hydrogen atom from 5,6,7,8-tetrahydro-1,7-naphthyridine; for greater clarity, the term "5,6,7,8-tetrahydro-1,7-naphthyridine" refers to

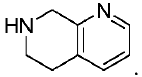

The term "5,6,7,8-tetrahydro-1,6-naphthyridinyl" refers to the radical formed by removing a hydrogen atom from 5,6,7,8-tetrahydro-1,6-naphthyridine; for greater clarity, the term "5,6,7,8-tetrahydro-1,6-naphthyridine" refers to

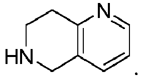

With reference to substituents, the term "independently" refers to the situation where when more than one substituent is possible, the substituents may be the same or different from each other.

The term "substituted" means that the specified group or moiety bears one or more substituents. The term "unsubstituted" means that the specified group bears no substituents. The term "optionally substituted" means that the specified group is unsubstituted or substituted by one or more substituents. Where the term "substituted" is used to describe a structural system, the substitution is meant to occur at any valency-allowed position on the system.

The term "variable point of attachment" means that a group is allowed to be attached at more than one alternative position in a structure. The attachment will always replace a hydrogen atom on one of the ring atoms. In other words, all permutations of bonding are represented by the single diagram, as shown in the illustrations below.

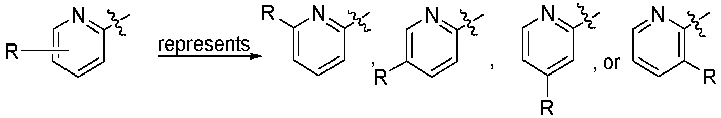

Those skilled in the art will recognize that that if more than one such substituent is present for a given ring, the bonding of each substituent is independent of all of the others. The groups listed or illustrated above are not exhaustive.

As used herein, the term "or" means "and/or" unless stated otherwise.

As used herein, the terms "including", "containing" and "comprising" are used in their open, non-limiting sense.

As used herein, the term "composition" is intended to encompass a product comprising the specified ingredients in the specified amounts, as well as any product which results, directly or indirectly, from combination of the specified ingredients in the specified amounts.

As used herein, the term "treat", "treating", or "treatment" of any disease, condition, syndrome or disorder refers, in one embodiment, to ameliorating the disease, condition, syndrome or disorder (i.e. slowing or arresting or reducing the development of the disease or at least one of the clinical symptoms thereof). In another embodiment, "treat", "treating", or "treatment" refers to alleviating or ameliorating at least one physiological or biochemical parameter associated with or causative of the disease, condition, syndrome or disorder, including those which may not be discernible by the patient. In a further embodiment, "treat", "treating", or "treatment" refers to modulating the disease, condition, syndrome or disorder either physically (*e*.*g*. stabilization of a discernible symptom), physiologically, (*e*.*g*. stabilization of a physical parameter), or both. In yet another embodiment, "treat", "treating", or "treatment" refers to preventing or delaying the onset or development or progression of the disease, condition, syndrome or disorder.

The terms "subject" and "patient" are used interchangeably herein and may refer to an animal, preferably a mammal, most preferably a human.

As used herein, the terms active compound, pharmaceutical agent and active ingredient are used interchangeably to refer to a pharmaceutically active compound. Other ingredients in a drug composition, such as carriers, diluents or excipients, may be substantially or completely pharmaceutically inert. A pharmaceutical composition (also referred to herein as a composition or formulation) may comprise the active ingredient in combination with one or more carriers and/or one or more excipients and/or one or more diluents.

The term "therapeutically effective amount" (used interchangeably herein with "effective amount") refers to an amount (e.g., of an active compound or pharmaceutical agent, such as a compound of the present invention), which elicits the biological or medicinal response in a tissue system, animal or human that is being sought by a researcher, veterinarian, medical doctor or other clinician, including reduction or inhibition of an enzyme or a protein activity, or ameliorating symptoms, alleviating conditions, slowing or delaying disease progression, or preventing a disease. Stated another way, the term therapeutically effective amount may refer to an amount that, when administered to a particular subject, achieves a therapeutic effect by inhibiting, alleviating or curing a disease, condition, syndrome or disorder in the subject or by prophylactically inhibiting, preventing or delaying the onset of a disease, condition, syndrome or disorder, or symptom(s) thereof. A therapeutically effective amount may be an amount which relieves to some extent one or more symptoms of a disease, condition, syndrome or disorder in a subject; and/or returns to normal either partially or completely one or more physiological or biochemical parameters associated with or causative of the disease, condition, syndrome or disorder; and/or reduces the likelihood of the onset of the disease, condition, syndrome or disorder, or symptom(s) thereof.

"Pharmaceutically acceptable" means that, which is useful in preparing a pharmaceutical composition that is generally safe, non-toxic and neither biologically nor otherwise undesirable and includes that which is acceptable for veterinary as well as human pharmaceutical use.

A "pharmaceutically acceptable salt" is intended to mean a salt of an acid or base of a compound represented by Formula (I) (as well as compounds of Formula (II) and others described herein) that is non-toxic, biologically tolerable, or otherwise biologically suitable for administration to the subject. See, generally, S.M. Berge, et al., "Pharmaceutical Salts", J. Pharm. Sci., 1977, 66:1-19, and Handbook of Pharmaceutical Salts, Properties, Selection, and Use, Stahl and Wermuth, Eds., Wiley-VCH and VHCA, Zurich, 2002. Preferred pharmaceutically acceptable salts are those that are pharmacologically effective and suitable for contact with the tissues of patients without undue toxicity, irritation, or allergic response.

Non-limiting examples of pharmaceutically acceptable salts include sulfates, pyrosulfates, bisulfates, sulfites, bisulfites, phosphates, monohydrogen-phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1-sulfonates, naphthalene-2-sulfonates, and mandelates.

A compound of Formula (I) may possess a sufficiently acidic group, a sufficiently basic group, or both types of functional groups, and accordingly react with a number of inorganic or organic bases, and inorganic and organic acids, to form a pharmaceutically acceptable salt.

Compounds of Formula (I) may contain at least one nitrogen of basic character, so desired pharmaceutically acceptable salts may be prepared by any suitable method available in the art, for example, treatment of the free base with an inorganic acid, such as hydrochloric acid, hydrobromic acid, sulfuric acid, sulfamic acid, nitric acid, boric acid, phosphoric acid, and the like, or with an organic acid, such as acetic acid, phenylacetic acid, propionic acid, stearic acid, lactic acid, ascorbic acid, maleic acid, hydroxymaleic acid, isethionic acid, succinic acid, valeric acid, fumaric acid, malonic acid, pyruvic acid, oxalic acid, glycolic acid, salicylic acid, oleic acid, palmitic acid, lauric acid, a pyranosidyl acid, such as glucuronic acid or galacturonic acid, an alpha-hydroxy acid, such as mandelic acid, citric acid, or tartaric acid, an amino acid, such as aspartic acid or glutamic acid, an aromatic acid, such as benzoic acid, 2-acetoxybenzoic acid, naphthoic acid, or cinnamic acid, a sulfonic acid, such as laurylsulfonic acid, p-toluenesulfonic acid, methanesulfonic acid, ethanesulfonic acid, any compatible mixture of acids such as those given as examples herein, and any other acid and mixture thereof that are regarded as equivalents.

Compounds of Formula (I) may contain a carboxylic acid moiety, a desired pharmaceutically acceptable salt may be prepared by any suitable method, for example, treatment of the free acid with an inorganic or organic base, such as an amine (primary, secondary or tertiary), an alkali metal hydroxide, alkaline earth metal hydroxide, any compatible mixture of bases such as those given as examples herein, and any other base and mixture thereof that are regarded as equivalents or acceptable substitutes in light of the ordinary level of skill in this technology. Illustrative examples of suitable salts include organic salts derived from amino acids, such as glycine and arginine, ammonia, carbonates, bicarbonates, primary, secondary, and tertiary amines, and cyclic amines, such as benzylamines, pyrrolidines, piperidine, morpholine, piperazine, N-methyl-glucamine and tromethamine and inorganic salts derived from sodium, calcium, potassium, magnesium, manganese, iron, copper, zinc, aluminum, and lithium.

Each compound used herein may be discussed interchangeably with respect to its chemical formula, chemical name, abbreviation, etc.

Any formula given herein is intended to represent compounds having structures depicted by the structural formula as well as certain variations or forms. In particular, compounds of any formula given herein may have asymmetric centers and therefore exist in different enantiomeric forms. All optical isomers and stereoisomers of the compounds of the general formula, and mixtures thereof, are considered within the scope of such formula. The compounds of this invention may possess one or more asymmetric centers; such compounds can therefore be produced as individual (*R*)- or (*S*)-stereoisomers or as mixtures thereof. Thus, any formula given herein is intended to represent a racemate, one or more of its enantiomeric forms, one or more of its diastereomeric forms, and mixtures thereof. Additionally, any formula given herein is intended to refer also to any one of hydrates, solvates, polymorphs and of such compounds, and mixtures thereof, even if such forms are not listed explicitly.

The term "*R*" at a stereocenter designates that the stereocenter is purely of the *R-*configuration as defined in the art; likewise, the term "*S*" means that the stereocenter is purely of the *S*-configuration. As used herein, the term "*RS*" refers to a stereocenter that exists as a mixture of the *R*- and *S*-configurations.

Compounds containing one stereocenter drawn without a stereo bond designation are a mixture of 2 enantiomers. Compounds containing 2 stereocenters both drawn without stereo bond designations are a mixture of 4 diastereomers. Compounds with 2 stereocenters both labeled "RS" and drawn with stereo bond designations are a 2-component mixture with relative stereochemistry as drawn. Unlabeled stereocenters drawn without stereo bond designations are a mixture of the *R*and *S*-configurations. For unlabeled stereocenters drawn with stereo bond designations, the absolute stereochemistry is as depicted.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

Reference to a compound herein stands for a reference to any one of: (a) the recited form of such compound, and (b) any of the forms of such compound in the medium in which the compound is being considered when named. For example, reference herein to a compound such as R-COOH, encompasses reference to any one of, for example, R-COOH(s), R-COOH(sol), and R-COO-(sol). In this example, R-COOH(s) refers to the solid compound, as it could be for example in a tablet or some other solid pharmaceutical composition or preparation; R-COOH(sol) refers to the undissociated form of the compound in a solvent; and R-COO-(sol) refers to the dissociated form of the compound in a solvent, such as the dissociated form of the compound in an aqueous environment, whether such dissociated form derives from R-COOH, from a salt thereof, or from any other entity that yields R-COO- upon dissociation in the medium being considered. In another example, an expression such as "exposing an entity to compound of formula R-COOH" refers to the exposure of such entity to the form, or forms, of the compound R-COOH that exists, or exist, in the medium in which such exposure takes place. In still another example, an expression such as "reacting an entity with a compound of formula R-COOH" refers to the reacting of (a) such entity in the chemically relevant form, or forms, of such entity that exists, or exist, in the medium in which such reacting takes place, with (b) the chemically relevant form, or forms, of the compound R-COOH that exists, or exist, in the medium in which such reacting takes place. In this regard, if such entity is for example in an aqueous environment, it is understood that the compound R-COOH is in such same medium, and therefore the entity is being exposed to species such as R-COOH(aq) and/or R-COO-(aq), where the subscript "(aq)" stands for "aqueous" according to its conventional meaning in chemistry and biochemistry. A carboxylic acid functional group has been chosen in these nomenclature examples; this choice is not intended, however, as a limitation but it is merely an illustration. It is understood that analogous examples can be provided in terms of other functional groups, including but not limited to hydroxyl, basic nitrogen members, such as those in amines, and any other group that interacts or transforms according to known manners in the medium that contains the compound. Such interactions and transformations include, but are not limited to, dissociation, association, tautomerism, solvolysis, including hydrolysis, solvation, including hydration, protonation, and deprotonation. No further examples in this regard are provided herein because these interactions and transformations in a given medium are known by any one of ordinary skill in the art.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number in an enriched form. Examples of isotopes that can be incorporated into compounds of the invention in a form that exceeds natural abundances include isotopes of hydrogen, carbon, nitrogen, oxygen, phosphorous, fluorine, chlorine, and iodine, such as ²H (or chemical symbol D), ³H (or chemical symbol T), ¹¹C, ¹³C, ¹⁴C, ¹⁵N, ¹⁸O, ¹⁷O, ³¹P, ³²P, ³⁵S, ¹⁸F, ³⁶Cl, and ¹²⁵I, respectively. Such isotopically labelled compounds are useful in metabolic studies (preferably with ¹⁴C), reaction kinetic studies (with, for example ²H or ³H), detection or imaging techniques [such as positron emission tomography (PET) or single-photon emission computed tomography (SPECT)] including drug or substrate tissue distribution assays, or in radioactive treatment of patients. In particular, an ¹⁸F or ¹¹C labeled compound may be particularly preferred for PET or SPECT studies. Further, substitution with heavier isotopes such as deuterium (i.e., ²H, or D) may afford certain therapeutic advantages resulting from greater metabolic stability, for example increased *in vivo* half-life or reduced dosage requirements. Isotopically labeled compounds of this invention can generally be prepared by carrying out the procedures disclosed in the schemes or in the examples and preparations described below by substituting a readily available isotopically labeled reagent for a non-isotopically labeled reagent.

When the same plurality of substituents is assigned to various groups, the specific individual substituent assignment to each of such groups is meant to be independently made with respect to the specific individual substituent assignments to the remaining groups. By way of illustration, but not as a limitation, if each of groups Q and R can be H or F, the choice of H or F for Q is made independently of the choice of H or F for R, so the choice of assignment for Q does not determine or condition the choice of assignment for R, or vice-versa, unless it is expressly indicated otherwise. Illustrative claim recitation in this regard would read as "each of Q and R is independently H or F", or "each of Q and R is independently selected from the group consisting of H and F".

In another example, a zwitterionic compound would be encompassed herein by referring to a compound that is known to form a zwitterion, even if it is not explicitly named in its zwitterionic form. Terms such as zwitterion, zwitterions, and their synonyms zwitterionic compound(s) are standard IUPAC-endorsed names that are well known and part of standard sets of defined scientific names. In this regard, the name zwitterion is assigned the name identification CHEBI:27369 by the Chemical Entities of Biological Interest (ChEBI) dictionary of molecular entities. As generally well known, a zwitterion or zwitterionic compound is a neutral compound that has formal unit charges of opposite sign. Sometimes these compounds are referred to by the term "inner salts". Other sources refer to these compounds as "dipolar ions", although the latter term is regarded by still other sources as a misnomer. As a specific example, aminoethanoic acid (the amino acid glycine) has the formula H₂NCH₂COOH, and it exists in some media (in this case in neutral media) in the form of the zwitterion ⁺H₃NCH₂COO⁻. Zwitterions, zwitterionic compounds, inner salts and dipolar ions in the known and well-established meanings of these terms are within the scope of this invention, as would in any case be so appreciated by those of ordinary skill in the art. Because there is no need to name each and every embodiment that would be recognized by those of ordinary skill in the art, no structures of the zwitterionic compounds that are associated with the compounds of this invention are given explicitly herein. They are, however, part of the embodiments of this invention. No further examples in this regard are provided herein because the interactions and transformations in a given medium that lead to the various forms of a given compound are known by any one of ordinary skill in the art.

When referring to any formula given herein, the selection of a particular moiety from a list of possible species for a specified variable is not intended to define the same choice of the species for the variable appearing elsewhere. In other words, where a variable appears more than once, the choice of the species from a specified list is independent of the choice of the species for the same variable elsewhere in the formula, unless stated otherwise.

By way of a first example on substituent terminology, if substituent S¹ₑₓₐₘₚₗₑ is one of S₁ and S₂, and substituent S²ₑₓₐₘₚₗₑ is one of S₃ and S₄, then these assignments refer to embodiments of this invention given according to the choices S¹ₑₓₐₘₚₗₑ is S₁ and S²ₑₓₐₘₚₗₑ is S₃; S¹ₑₓₐₘₚₗₑ is S₁ and S²ₑₓₐₘₚₗₑ is S₄; S¹ₑₓₐₘₚₗₑ is S₂ and S²ₑₓₐₘₚₗₑ is S₃; S¹ₑₓₐₘₚₗₑ is S₂ and S²ₑₓₐₘₚₗₑ is S₄, and equivalents of each one of such choices. The shorter terminology "S¹ₑₓₐₘₚₗₑ is one of S₁ and S₂, and S²ₑₓₐₘₚₗₑ is one of S₃ and S₄" is accordingly used herein for the sake of brevity, but not by way of limitation. The foregoing first example on substituent terminology, which is stated in generic terms, is meant to illustrate the various substituent assignments described herein.

Furthermore, when more than one assignment is given for any member or substituent, embodiments of this invention comprise the various groupings that can be made from the listed assignments, taken independently, and equivalents thereof. By way of a second example on substituent terminology, if it is herein described that substituent Sₑₓₐₘₚₗₑ is one of S₁, S₂, and S₃, this listing refers to embodiments of this invention for which Sₑₓₐₘₚₗₑ is S₁; Sₑₓₐₘₚₗₑ is S₂; Sₑₓₐₘₚₗₑ is S₃; Sₑₓₐₘₚₗₑ is one of S₁ and S₂, Sₑₓₐₘₚₗₑ is one of S₁ and S₃; Sₑₓₐₘₚₗₑ is one of S₂ and S₃; Sₑₓₐₘₚₗₑ is one of S₁, S₂ and S₃; and Sₑₓₐₘₚₗₑ is any equivalent of each one of these choices. The shorter terminology "Sₑₓₐₘₚₗₑ is one of S₁, S₂, and S₃" is accordingly used herein for the sake of brevity, but not by way of limitation. The foregoing second example on substituent terminology, which is stated in generic terms, is meant to illustrate the various substituent assignments described herein.

Embodiments of this invention include compounds of Formula I 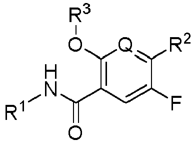 wherein
Q is CH or N;
R¹ is 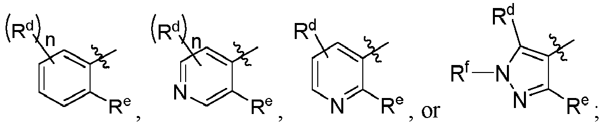 wherein
   R^{d} is selected from the group consisting of: -H, -Cl, -F, and -C₍₁₋₃₎alkyl; wherein said -C₍₁₋₃)alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, and -C₍₁₋₃₎alkyl; wherein said -C₍₁₋₃₎alkyl is optionally substituted with: -OH, -OCH₃, -SCH₃, or -OCF₃; and wherein said -C₍₁₋₃)alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is
   imidazolyl; wherein said imidazolyl is optionally substituted with up to three substituents independently selected from the group comprising -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -NH₂, -NHSO₂CH₃, -NHC(O)CH₃, - OH, -CO₂H, -Cl, or R^{a};
   pyrazinonyl; wherein said pyrazinonyl is optionally substituted with one or two groups independently selected from the group comprising R^{a};
   thiazolyl; wherein said thiazolyl is optionally substituted with one or two substituents independently selected from the group comprising R^{a};
   5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
   pyridinyl; wherein said pyridinyl is optionally substituted with one or two substituents independently selected from the group comprising R^{a}, -Cl, or -CO₂H;
   pyridazinyl; wherein said pyridazinyl is optionally substituted with one or two groups independently selected from the group comprising R^{a}, -Cl, or -CO₂H;
   4,5,6,7-tetrahydrothiazolo[5,4-*c*]pyridinyl; wherein said 4,5,6,7-tetrahydrothiazolo[5,4-c]pyridinyl is optionally substituted with R^{a};
   pyrazolyl; wherein said pyrazolyl is optionally substituted with one or two substituents independently selected from the group comprising R^{a};
   oxazolyl; wherein said oxazolyl is optionally substituted with one or two substituents independently selected from the group comprising R^{a};
   1,3,4-oxadiazolyl; wherein said 1,3,4-oxadiazolyl is optionally substituted with R^{a};
   5,6,7,8-tetrahydroimidazo[1,5-*a*]pyridinyl; wherein said 5,6,7,8-tetrahydroimidazo[1,5-*a*]pyridinyl is optionally substituted with R^{a};
   1,2,4-thiadiazolyl; wherein said 1,2,4-thiadiazolyl is optionally substituted with R^{a};
   5,6,7,8-tetrahydro-1,7-naphthyridinyl; wherein said 5,6,7,8-tetrahydro-1,7-naphthyridinyl is optionally substituted with R^{a}; or
   5,6,7,8-tetrahydro-1,6-naphthyridinyl; wherein said 5,6,7,8-tetrahydro-1,6-naphthyridinyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -Cl, -OH or up to three fluorine atoms; and wherein said cyclopropyl is optionally substituted with -OH, -CH₂OH, or up to two fluorine atoms;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said -C₍₁₋₆)alkyl is optionally substituted with up to six fluorine atoms;
or a pharmaceutically acceptable salt, solvate, stereoisomer, isotopic variant, or N-oxide thereof.

Embodiments of this invention include compounds of Formula II 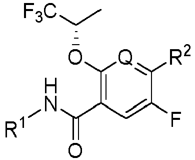 wherein
Q is CH or N;
R¹ is 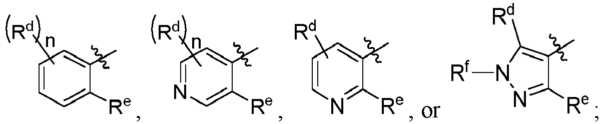 wherein
   R^{d} is selected from the group consisting of: -Cl, -F, and -C₍₁₋₃₎alkyl; wherein said -C₍₁₋₃₎alkyl is optionally substituted with -OH, -OCH₃, -SCH₃, or -OCF₃; or said -C₍₁₋₃₎alkyl is optionally substituted with up to three fluorine atoms;
   R^{e} is selected from the group consisting of: -Cl, -F, and -C₍₁₋₃₎alkyl; wherein said -C₍₁₋₃₎alkyl is optionally substituted with -OH, -OCH₃, -SCH₃, or -OCF₃; or said -C₍₁₋₃₎alkyl is optionally substituted with up to three fluorine atoms;
   R^{f} is -H; and
   n is 1;
R² is
   imidazolyl; wherein said imidazolyl is optionally substituted with up to three substituents independently selected from the group comprising -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -NH₂, -NHSO₂CH₃, -NHC(O)CH₃, - OH, -CO₂H, -Cl, or R^{a};
   pyrazinonyl; wherein said pyrazinonyl is optionally substituted with one or two groups independently selected from the group comprising R^{a};
   thiazolyl; wherein said thiazolyl is optionally substituted with one or two substituents independently selected from the group comprising R^{a};
   5,6,7,8-tetrahydro-[1,2,4]triazolo[4,3-*a*]pyrazinyl;
   pyridinyl; wherein said pyridinyl is optionally substituted with one or two substituents independently selected from the group comprising R^{a}, -Cl, or -CO₂H;
   pyridazinyl; wherein said pyridazinyl is optionally substituted with one or two groups independently selected from the group comprising R^{a};
   4,5,6,7-tetrahydrothiazolo[5,4-*c*]pyridinyl;
   pyrazolyl; wherein said pyrazolyl is optionally substituted with one or two substituents independently selected from the group comprising R^{a};
   oxazolyl; wherein said oxazolyl is optionally substituted with one or two substituents independently selected from the group comprising R^{a};
   1,3,4-oxadiazolyl; wherein said 1,3,4-oxadiazolyl is substituted with cyclopropyl;
   5,6,7,8-tetrahydroimidazo[1,5-*a*]pyridinyl; wherein said 5,6,7,8-tetrahydroimidazo[1,5-*a*]pyridinyl is optionally substituted with R^{a};
   1,2,4-thiadiazolyl; wherein said 1,2,4-thiadiazolyl is substituted with cyclopropyl;
   5,6,7,8-tetrahydro-1,7-naphthyridinyl; or
   5,6,7,8-tetrahydro-1,6-naphthyridinyl;
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
or a pharmaceutically acceptable salt, solvate, stereoisomer, isotopic variant, or N-oxide thereof.

Embodiments of this invention include compounds of Formula I or II, wherein
Q is CH or N;
R¹ is 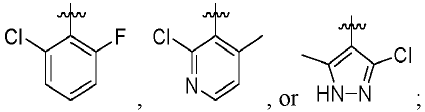
R² is
   imidazolyl; wherein said imidazolyl is optionally substituted with up to three substituents independently selected from the group comprising -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -OH, -CO₂H, -Cl, or R^{a};
   pyrazinonyl; wherein said pyrazinonyl is optionally substituted with one or two groups independently selected from the group comprising R^{a};
   thiazolyl; wherein said thiazolyl is optionally substituted with two substituents independently selected from the group comprising R^{a};
   5,6,7,8-tetrahydro-[1,2,4]triazolo[4,3-*a*]pyrazinyl;
   pyridinyl; wherein said pyridinyl is optionally substituted with one or two substituents independently selected from the group comprising R^{a}, -Cl, or -CO₂H;
   pyridazinyl; wherein said pyridazinyl is optionally substituted with R^{a};
   4,5,6,7-tetrahydrothiazolo[5,4-*c*]pyridinyl;
   pyrazolyl; wherein said pyrazolyl is optionally substituted with one or two substituents independently selected from the group comprising R^{a};
   oxazolyl; wherein said oxazolyl is optionally substituted with two substituents independently selected from R^{a};
   1,3,4-oxadiazolyl, wherein said 1,3,4-oxadiazolyl is substituted with cyclopropyl;
   5,6,7,8-tetrahydroimidazo[1,5-*a*]pyridinyl wherein said 5,6,7,8-tetrahydroimidazo[1,5-*a*]pyridinyl is optionally substituted with R^{a};
   1,2,4-thiadiazolyl; wherein said 1,2,4-thiadiazolyl is substituted with cyclopropyl;
   5,6,7,8-tetrahydro-1,7-naphthyridinyl; or
   5,6,7,8-tetrahydro-1,6-naphthyridinyl;
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
or a pharmaceutically acceptable salt, solvate, stereoisomer, isotopic variant, or N-oxide thereof.

Embodiments of this invention include compounds of Formula I or II, wherein
Q is CH or N;
R¹ is 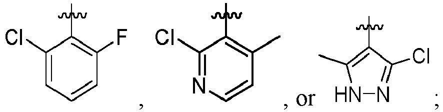
R² is
   imidazolyl; wherein said imidazolyl is substituted with two or three substituents independently selected from the group comprising -C₍₁₋₃₎alkyl, or -CN; wherein said -C(₁₋₃)alkyl is optionally substituted with -OH;
   triazolyl; wherein said triazolyl is substituted with two groups independently selected from the group comprising -C(O)NH₂, -CF₃, or -C₍₁₋₃₎alkyl; wherein said -C₍₁₋₃₎alkyl is optionally substituted with -OH;
   pyrazinyl; wherein said pyrazinyl is optionally substituted with up to three substituents independently selected from the group comprising -C₍₁₋₃₎alkyl, -OH, cyclopropyl. - C(O)NH₂, -CO₂H, -Cl, -CF₃; wherein said cyclopropyl is optionally substituted with - CH₂OH; and wherein said -C₍₁₋₃₎alkyl is optionally substituted with -OH;
   pyrazinonyl; wherein said pyrazinonyl is optionally substituted with one or two -C₍₁₋₃)alkyl groups;
   thiazolyl; wherein said thiazolyl is substituted with two substituents independently selected from the group comprising -C₍₁₋₃₎alkyl, cyclopropyl, or -C(O)NH₂; wherein said -C₍₁₋₃₎alkyl, is optionally substituted with -OH, or up to two fluorine atoms;
   5,6,7,8-tetrahydro-[1,2,4]triazolo[4,3-*a*]pyrazinyl;
   pyridinyl; wherein said pyridinyl is substituted with one or two substituents independently selected from the group comprising -CONH₂, -C₍₁₋₃₎alkyl, cyclopropyl, - Cl, or -CO₂H; wherein said -C₍₁₋₃₎alkyl is optionally substituted with -OH;
   pyridazinyl; wherein said pyridazinyl is substituted with -C₍₁₋₃₎alkyl; wherein said -C₍₁₋₃)alkyl is optionally substituted with -OH;
   4,5,6,7-tetrahydrothiazolo[5,4-*c*]pyridinyl;
   pyrazolyl; wherein said pyrazolyl is substituted with two substituents independently selected from the group comprising -C₍₁₋₃₎alkyl; wherein said -C₍₁₋₃₎alkyl is optionally substituted with -OH;
   oxazolyl; wherein said oxazolyl is substituted with two substituents independently selected from -C₍₁₋₃₎alkyl; wherein said -C₍₁₋₃₎alkyl is optionally substituted with -OH;
   1,3,4-oxadiazolyl; wherein said 1,3,4-oxadiazolyl is substituted with cyclopropyl;
   5,6,7,8-tetrahydroimidazo[1,5-*a*]pyridinyl wherein said 5,6,7,8-tetrahydroimidazo[1,5-*a*]pyridinyl is substituted with -C₍₁₋₃₎alkyl; wherein said -C₍₁₋₃₎alkyl is optionally substituted with -OH;
   1,2,4-thiadiazolyl; wherein said 1,2,4-thiadiazolyl is substituted with cyclopropyl;
   5,6,7,8-tetrahydro-1,7-naphthyridinyl; or
   5,6,7,8-tetrahydro-1,6-naphthyridinyl;
or a pharmaceutically acceptable salt, solvate, stereoisomer, isotopic variant, or N-oxide thereof.

Embodiments of this invention include compounds of Formula I or II, wherein
Q is CH or N;
R¹ is 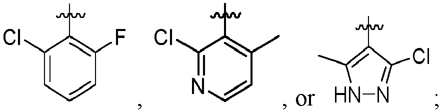
R² is
   imidazolyl; wherein said imidazolyl is substituted with two or three substituents independently selected from the group comprising -C₍₁₋₂₎alkyl, or -CN; wherein said -C₍₁₋₂₎alkyl is optionally substituted with -OH;
   triazolyl; wherein said triazolyl is substituted with two groups independently selected from the group comprising -C(O)NH₂, -CF₃, -CH₃, -CH₂CH₃, or -CH₂OH;
   pyrazinyl; wherein said pyrazinyl is optionally substituted with up to three substituents independently selected from the group comprising -CH₃, -CH₂CH₂CH₃, -OH, -CH₂OH, cyclopropyl. -C(O)NH₂, -CO₂H, -Cl, -CF₃, wherein said cyclopropyl is optionally substituted with -CH₂OH;
   pyrazinonyl; wherein said pyrazinonyl is substituted with -CH₃ and -CH₂CH₂CH₃;
   thiazolyl; wherein said thiazolyl is substituted with two substituents independently selected from the group comprising -C₍₁₋₂₎alkyl, cyclopropyl, or -C(O)NH₂; wherein said -C₍₁₋₂₎alkyl is optionally substituted with -OH, or up to two fluorine atoms;
   5,6,7,8-tetrahydro-[1,2,4]triazolo[4,3-*a*]pyrazinyl;
   pyridinyl; wherein said pyridinyl is substituted with one or two substituents independently selected from the group comprising -CONH₂, -CH₃, cyclopropyl, -Cl, - CH₂OH, or -CO₂H;
   pyridazinyl; wherein said pyridazinyl is substituted with -CH₃;
   4,5,6,7-tetrahydrothiazolo[5,4-*c*]pyridinyl;
   pyrazolyl; wherein said pyrazolyl is substituted with two substituents independently selected from the group comprising -C₍₁₋₂₎alkyl, wherein said -C₍₁₋₂₎alkyl is optionally substituted with -OH;
   oxazolyl; wherein said oxazolyl is substituted with -CH₂OH, and -CH₂CH₃;
   1,3,4-oxadiazolyl; wherein said 1,3,4-oxadiazolyl is substituted with cyclopropyl;
   5,6,7,8-tetrahydroimidazo[1,5-*a*]pyridinyl; wherein said 5,6,7,8-tetrahydroimidazo[1,5-*a*]pyridinyl is substituted with -CH₂OH;
   1,2,4-thiadiazolyl; wherein said 1,2,4-thiadiazolyl is substituted with cyclopropyl;
   5,6,7,8-tetrahydro-1,7-naphthyridinyl; or
   5,6,7,8-tetrahydro-1,6-naphthyridinyl;
or a pharmaceutically acceptable salt, solvate, stereoisomer, isotopic variant, or N-oxide thereof.

Embodiments of this invention include compounds of Formula I or II, wherein
Q is CH or N;
R¹ is 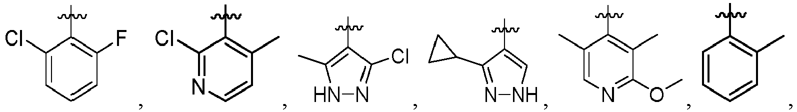 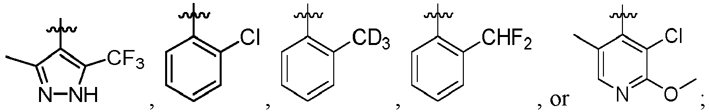
R² is 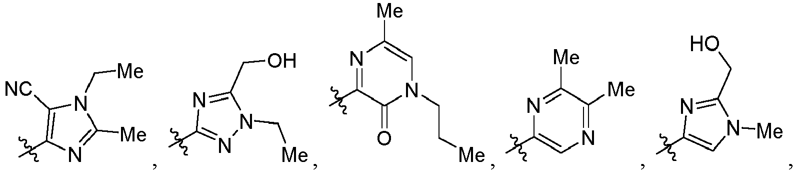 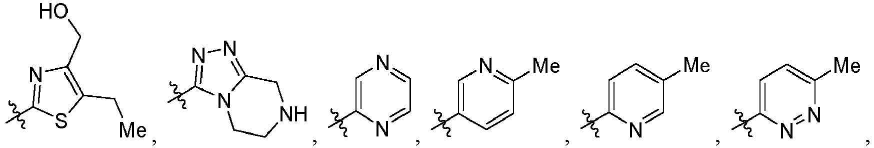 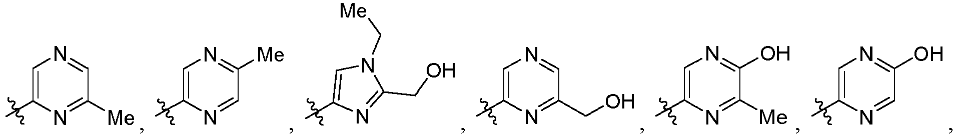 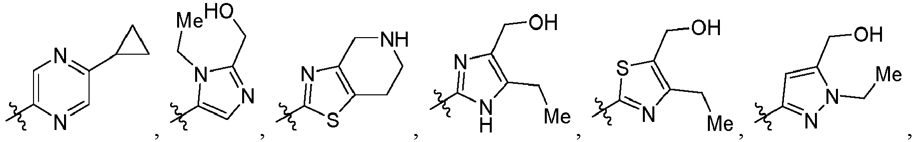 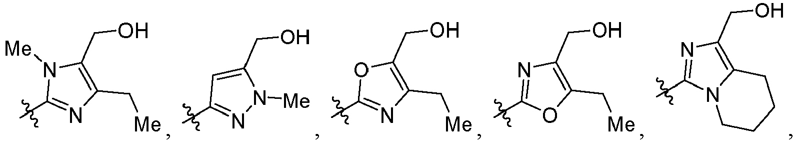 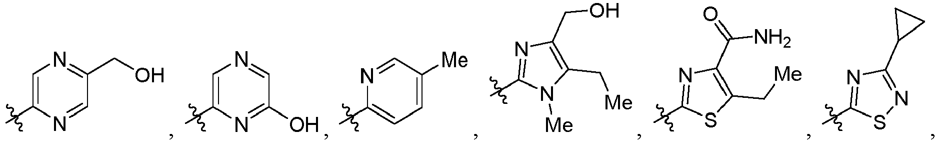 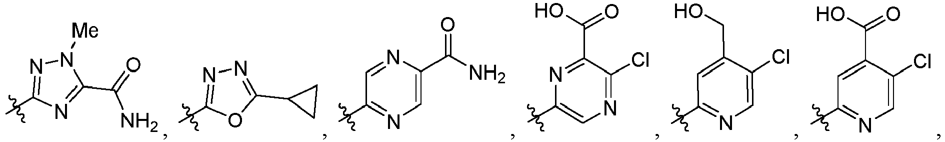 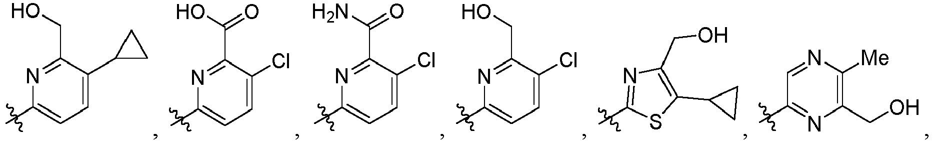 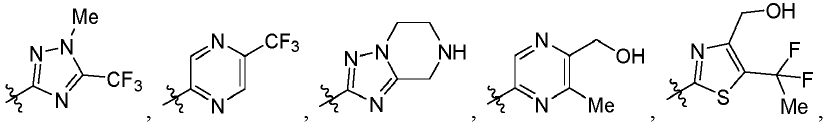 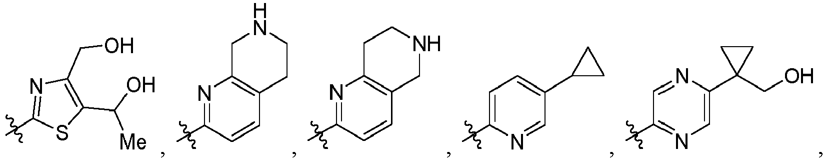 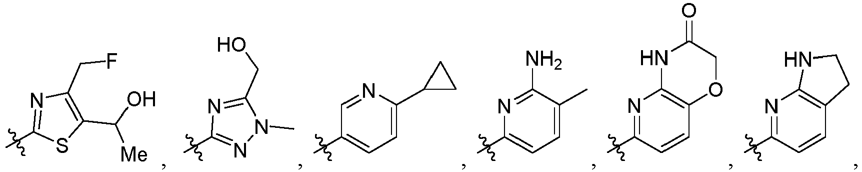 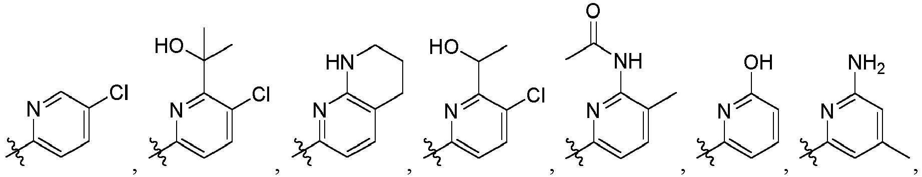 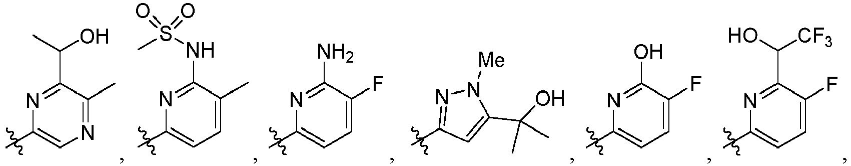 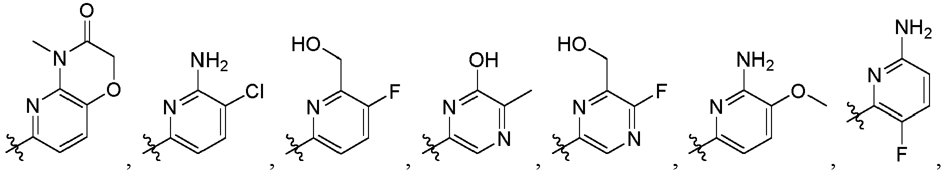 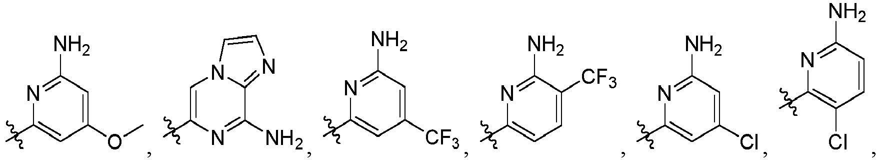 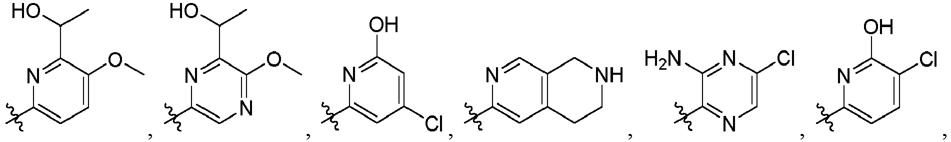 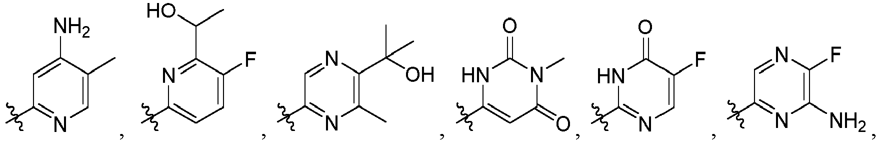 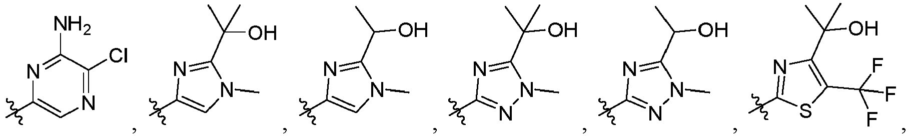 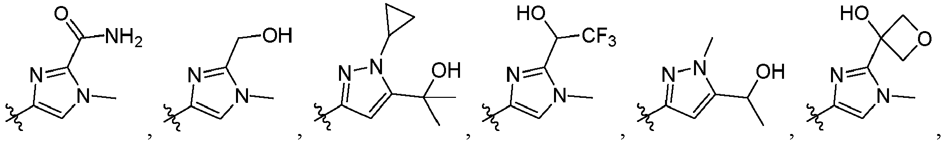 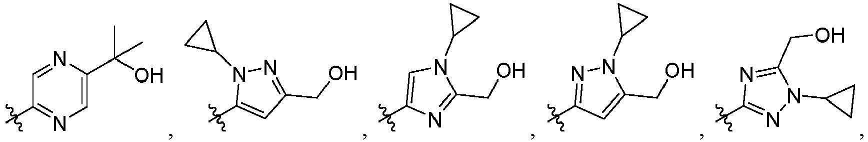 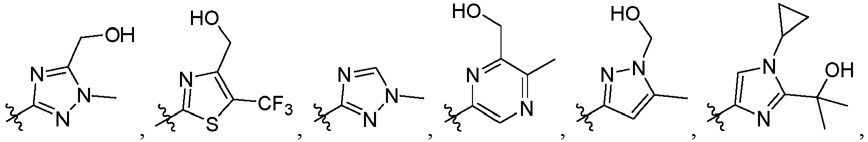 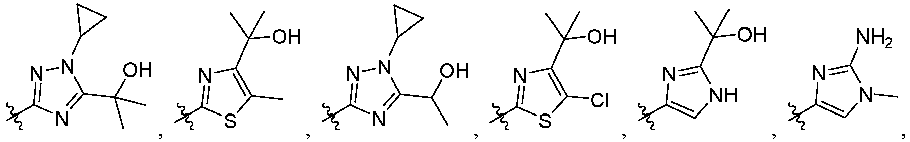 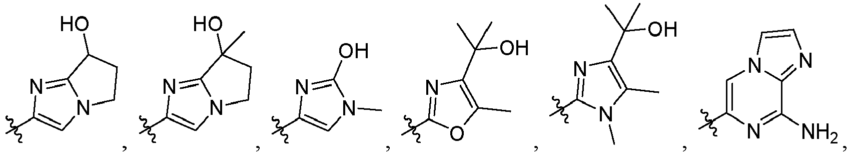 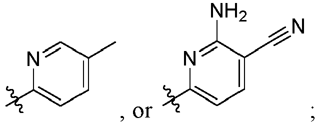
or a pharmaceutically acceptable salt, solvate, stereoisomer, isotopic variant, or N-oxide thereof.

Embodiments of this invention include a compound of Formula I or II, selected from the group consisting of 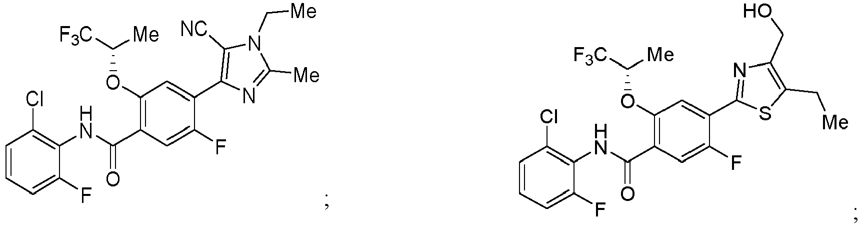 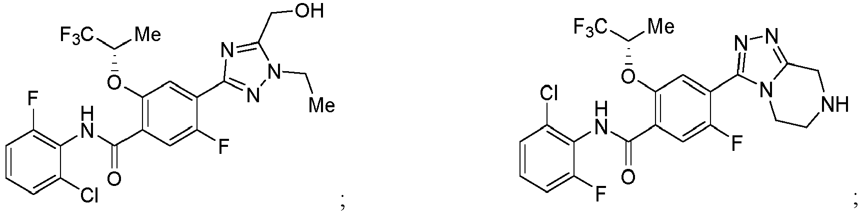 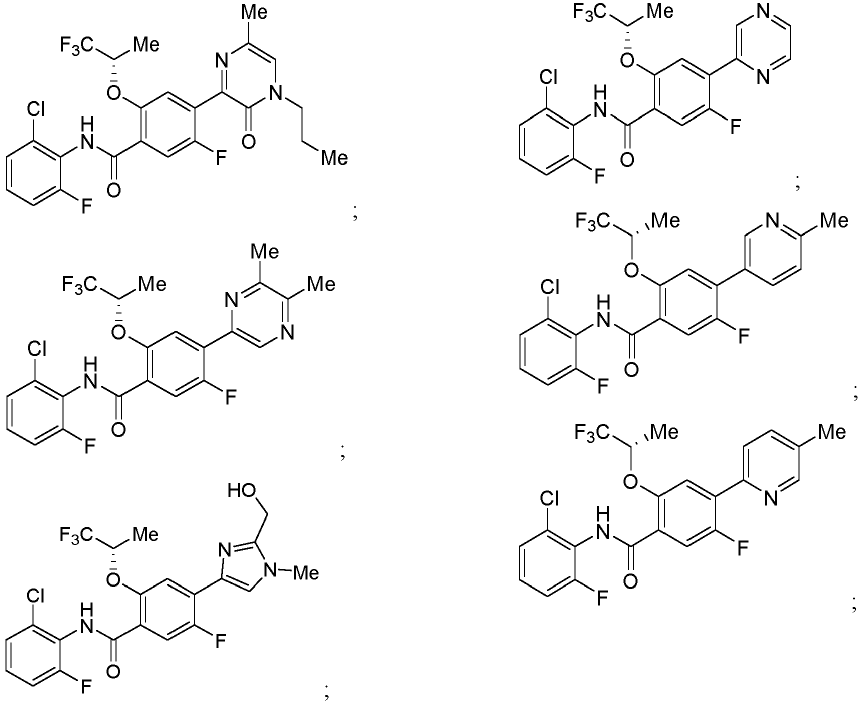 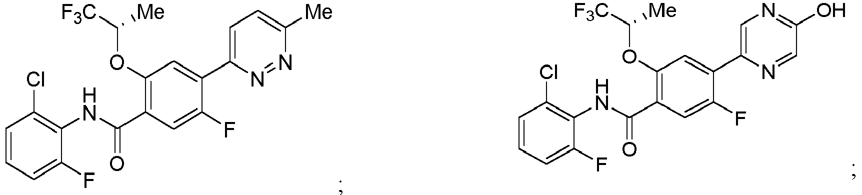 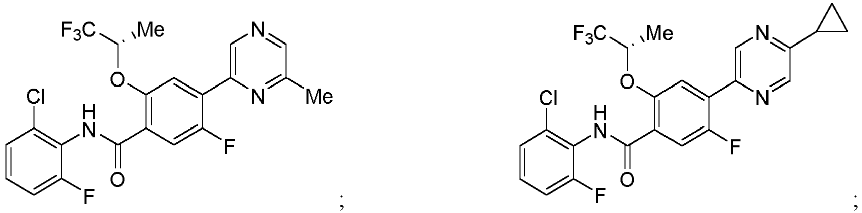 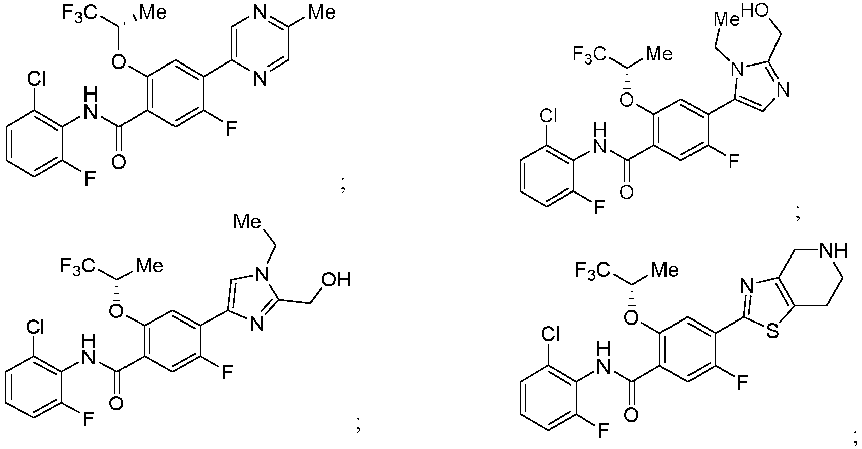 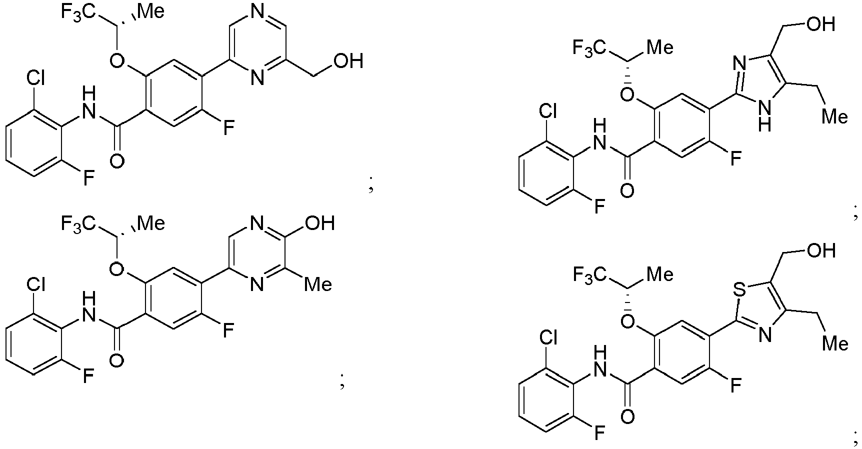 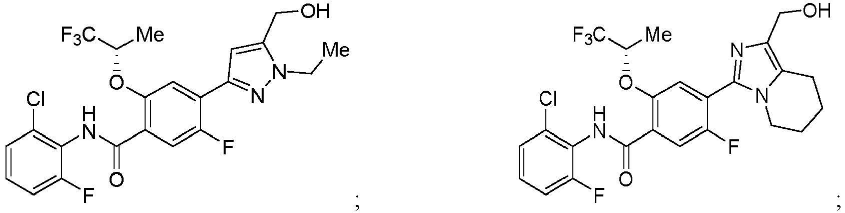 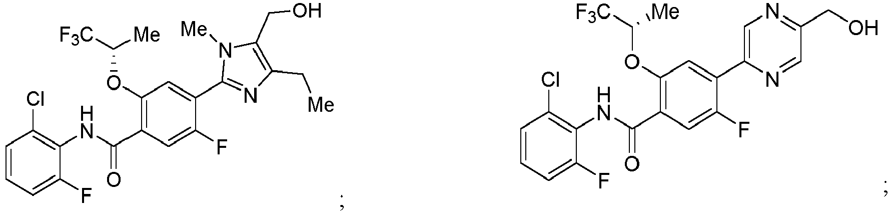 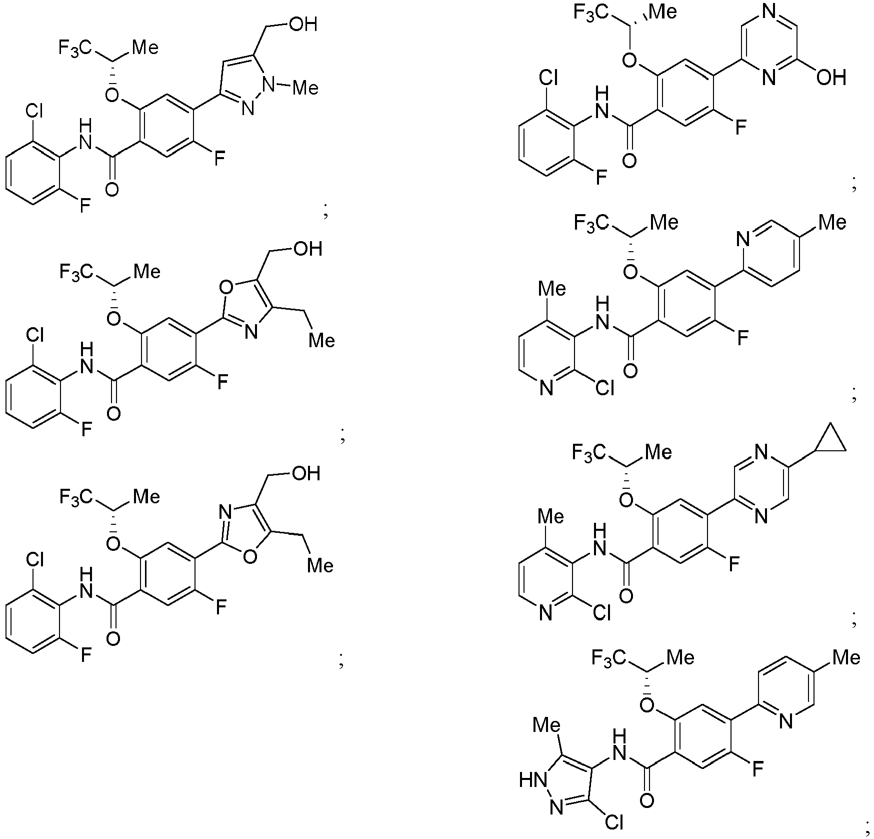 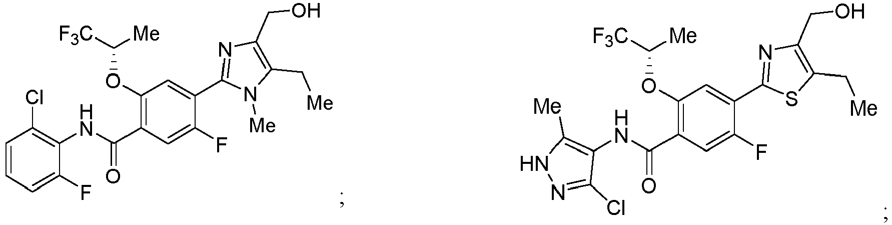 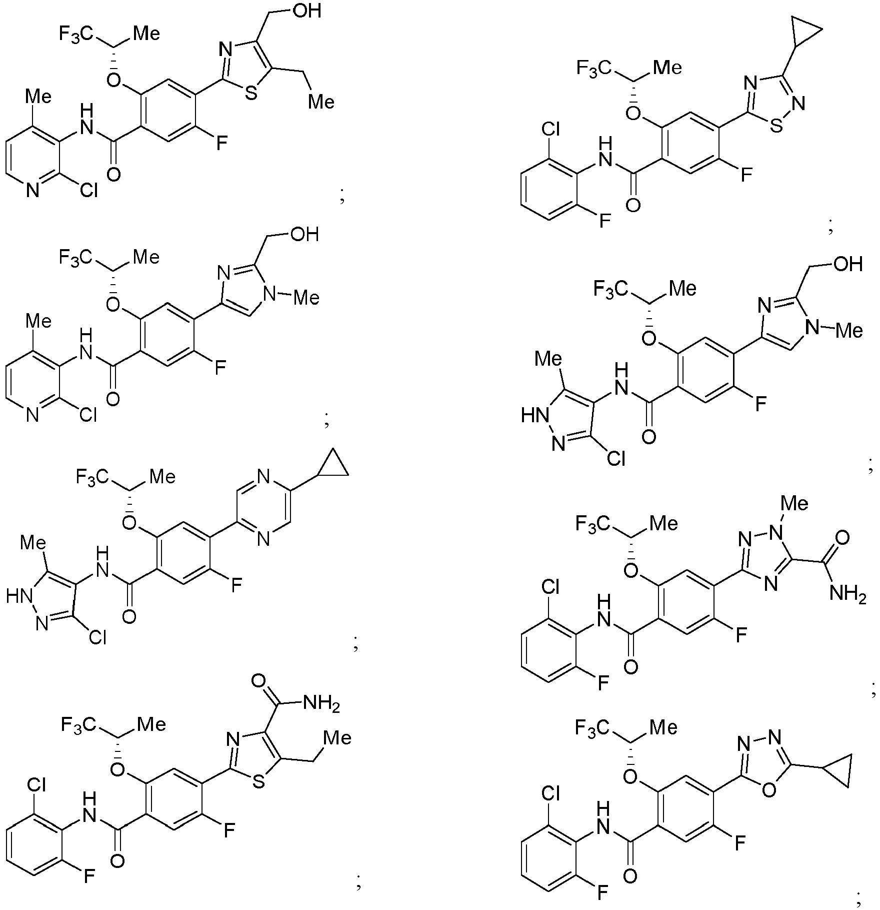 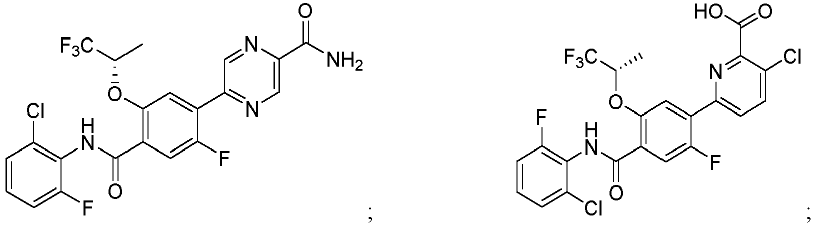 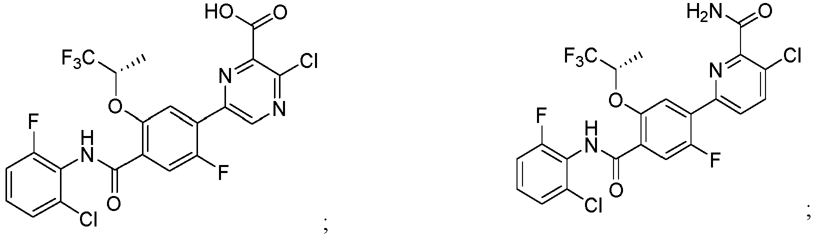 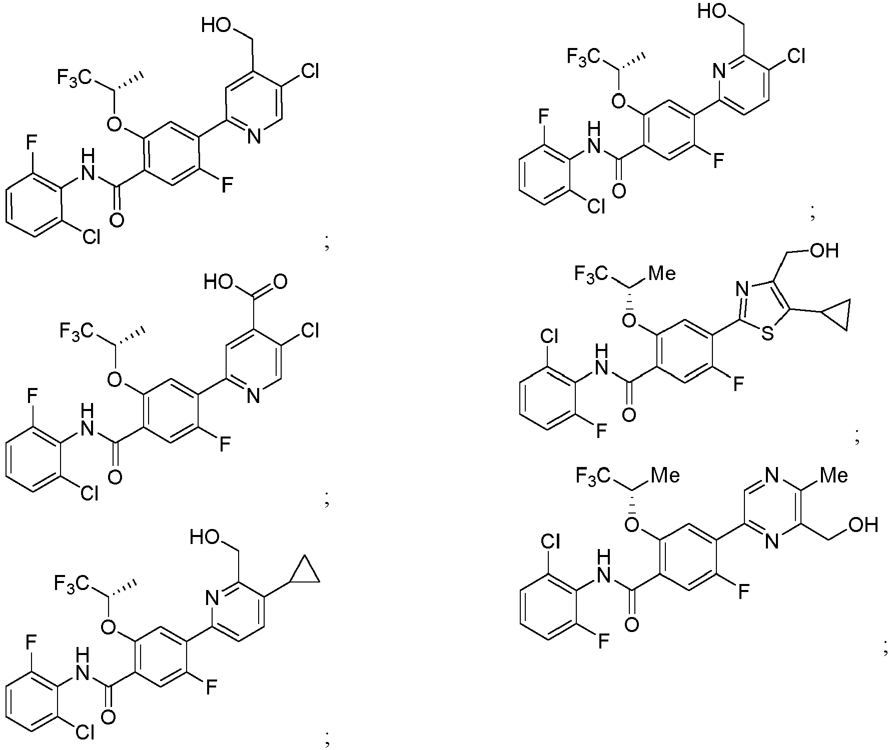 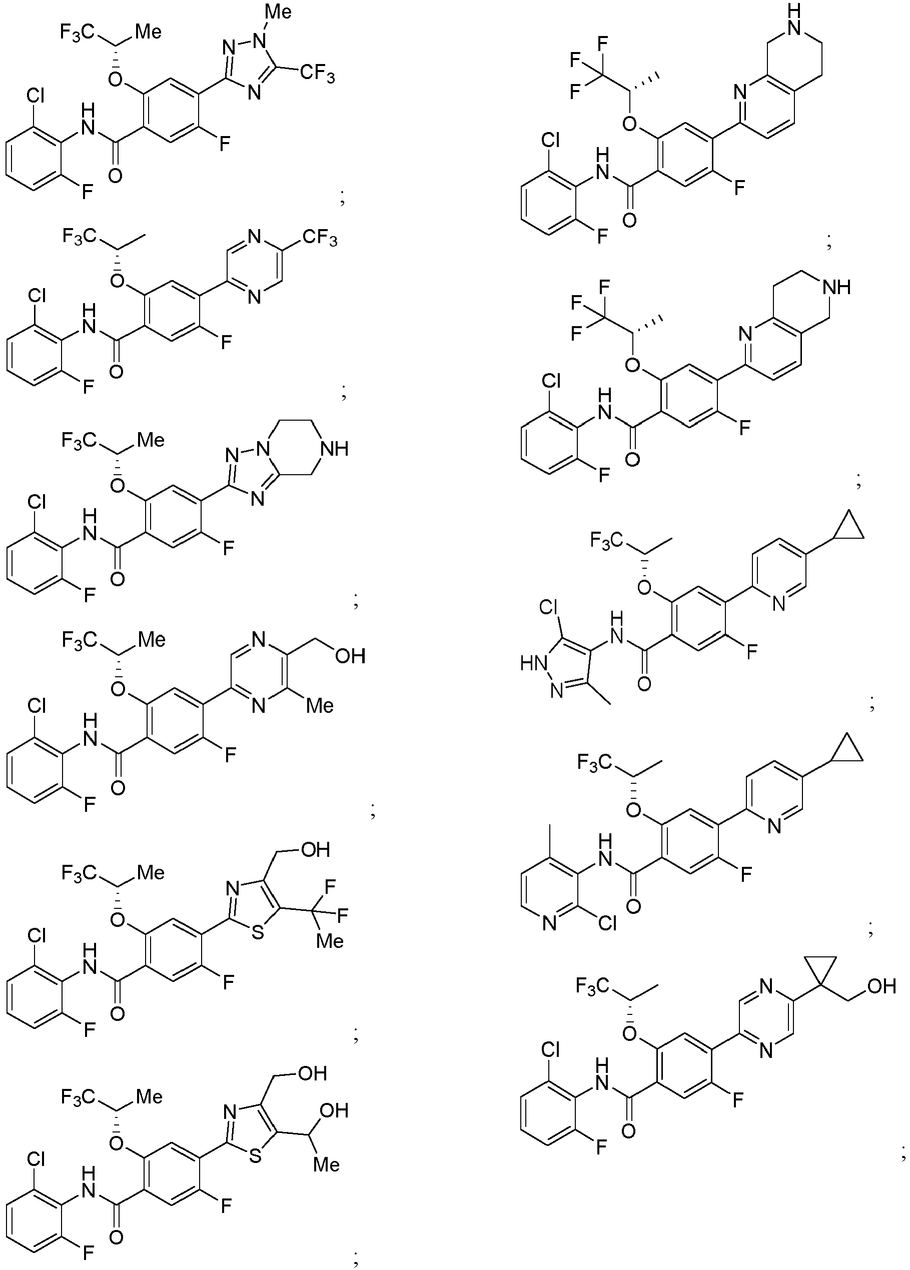 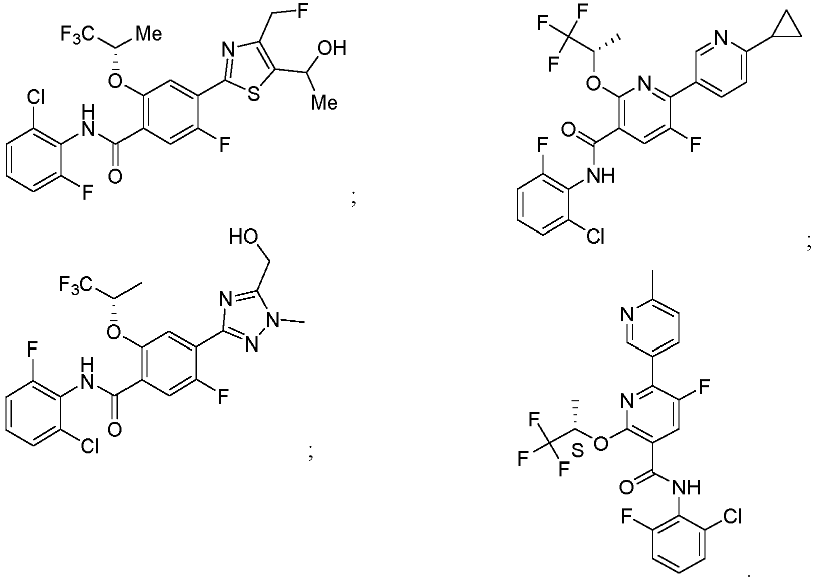 and or a pharmaceutically acceptable salt, solvate, stereoisomer, isotopic variant, or N-oxide thereof.

Embodiments of this invention include compounds of Formula I wherein:
Q is CH or N;
R¹ is 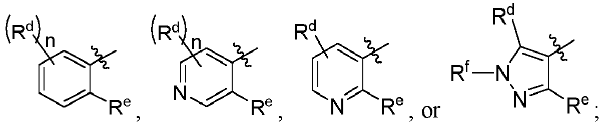 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is imidazolyl; wherein said imidazolyl is optionally substituted with up to three substituents independently selected from the group comprising -CN, or R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 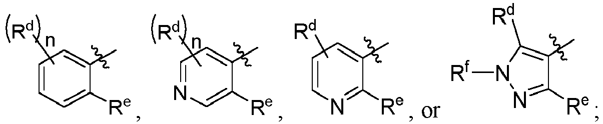 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is triazolyl; wherein said triazolyl is optionally substituted with one or two groups independently selected from the group comprising R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 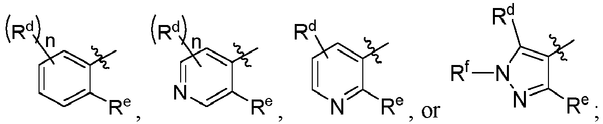 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is pyrazinyl; wherein said pyrazinyl is optionally substituted with up to three substituents independently selected from the group comprising -NH₂, -NHSO₂CH₃, -NHC(O)CH₃, - OH, -CO₂H, -Cl, or R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 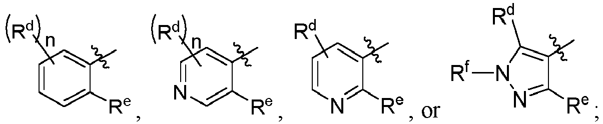 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is pyrazinonyl; wherein said pyrazinonyl is optionally substituted with one or two groups independently selected from the group comprising R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 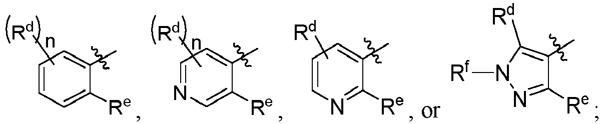 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is thiazolyl; wherein said thiazolyl is optionally substituted with one or two substituents independently selected from the group comprising R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 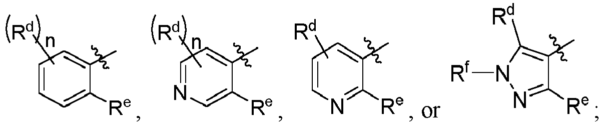 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 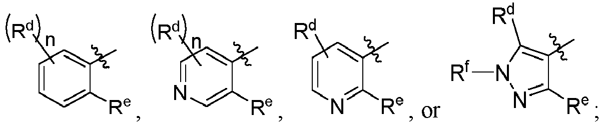 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is pyridinyl; wherein said pyridinyl is optionally substituted with one or two substituents independently selected from the group comprising R^{a}, -Cl, or -CO₂H;
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 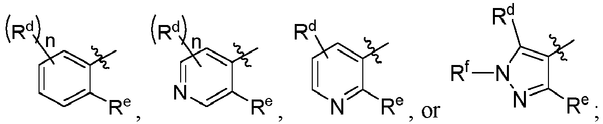 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is pyridazinyl; wherein said pyridazinyl is optionally substituted with one or two groups independently selected from the group comprising R^{a}, -Cl, or -CO₂H;
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 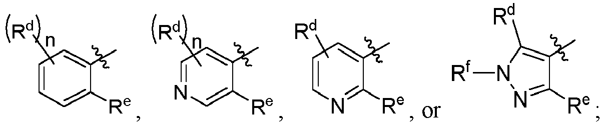 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 4,5,6,7-tetrahydrothiazolo[5,4-c]pyridinyl; wherein said 4,5,6,7-tetrahydrothiazolo[5,4-c]pyridinyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include ccompounds of Formula I wherein:
Q is CH or N;
R¹ is 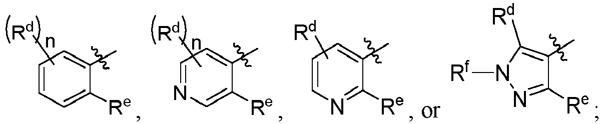 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is pyrazolyl; wherein said pyrazolyl is optionally substituted with one or two substituents independently selected from the group comprising R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 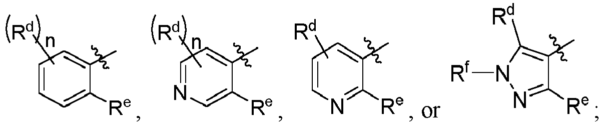 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is oxazolyl; wherein said oxazolyl is optionally substituted with one or two substituents independently selected from the group comprising R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R° is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 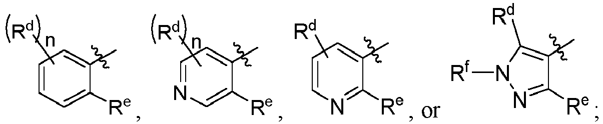 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 1,3,4-oxadiazolyl; wherein said 1,3,4-oxadiazolyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 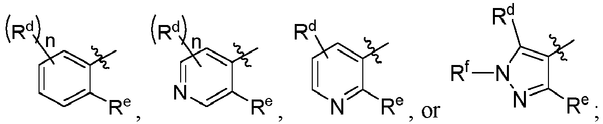 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 5,6,7,8-tetrahydroimidazo[1,5-*a*]pyridinyl; wherein said 5,6,7,8-tetrahydroimidazo[1,5-*a*]pyridinyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆)alkyl is optionally substituted with -Cl, -OH, or -OCH₃; and wherein said -C₍₁₋₆)alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 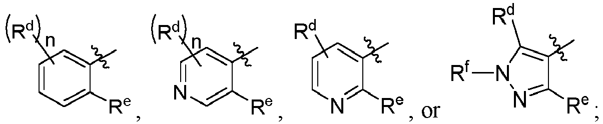 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 1,2,4-thiadiazolyl; wherein said 1,2,4-thiadiazolyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 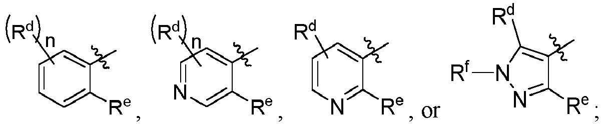 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 5,6,7,8-tetrahydro-1,7-naphthyridinyl; wherein said 5,6,7,8-tetrahydro-1,7-naphthyridinyl is optionally substituted with R^{a}; or
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 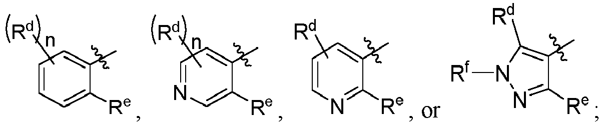 wherein
   R^{d} is selected from the group consisting of: H, Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{e} is selected from the group consisting of: Cl, F, and C₍₁₋₃₎alkyl; wherein said C₍₁₋₃₎alkyl is optionally substituted with: OH, OCH₃, SCH₃, or OCF₃; and wherein said C₍₁₋₃₎alkyl is optionally substituted with up to six fluorine atoms;
   R^{f} is selected from the group consisting of: H, and C₍₁₋₃₎alkyl; wherein said C₍₁₋₃₎alkyl is optionally substituted with a member selected from the group consisting of: OH, OCH₃, SCH₃, and OCF₃; and wherein said C₍₁₋₃₎alkyl is optionally substituted with up to six fluorine atoms; and
   n is 1, or 2;
R² is 5,6,7,8-tetrahydro-1,6-naphthyridinyl; wherein said 5,6,7,8-tetrahydro-1,6-naphthyridinyl is optionally substituted with R^{a};
R^{a} is -C(O)NH₂, -CF₃, cyclopropyl, or -C₍₁₋₃₎alkyl; wherein said -C₍₁₋₃₎alkyl is optionally substituted with -OH or up to two fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include a compound of Formula II, selected from the group consisting of: 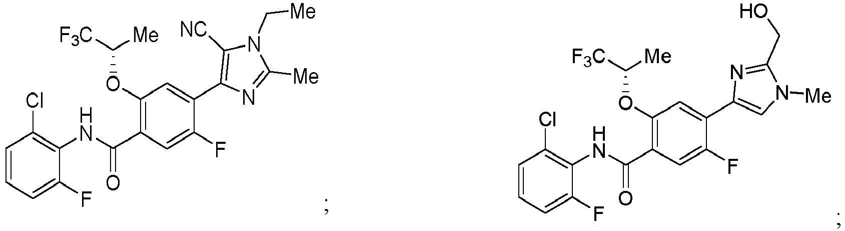 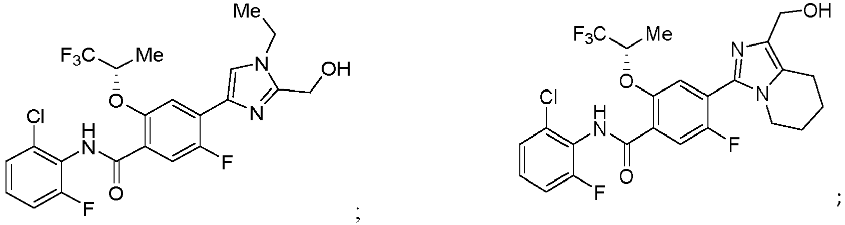 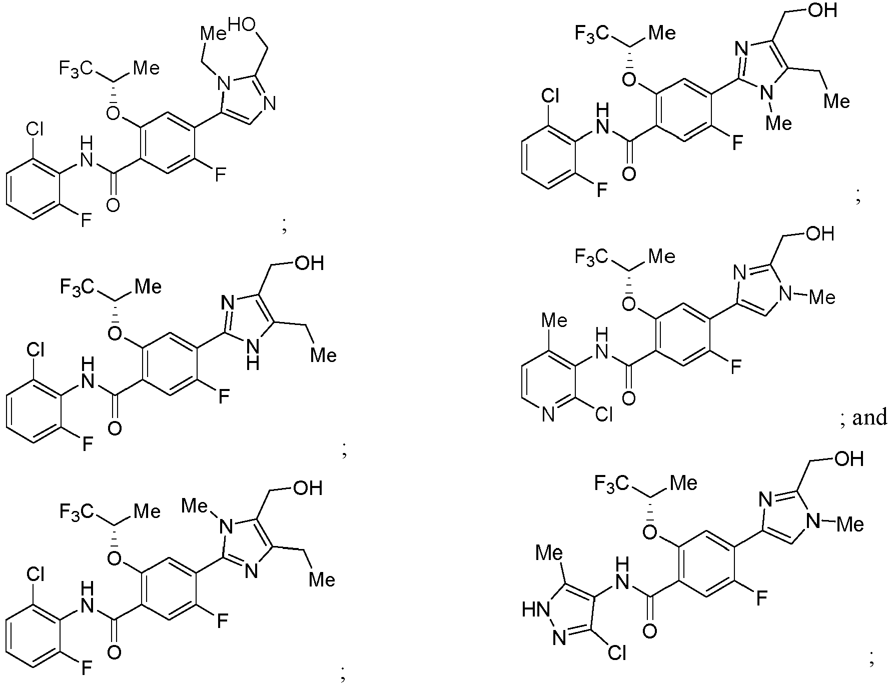 or a pharmaceutically acceptable salt, solvate, stereoisomer, isotopic variant, or N-oxide thereof.

Embodiments of this invention include a compound of Formula II, selected from the group consisting of: 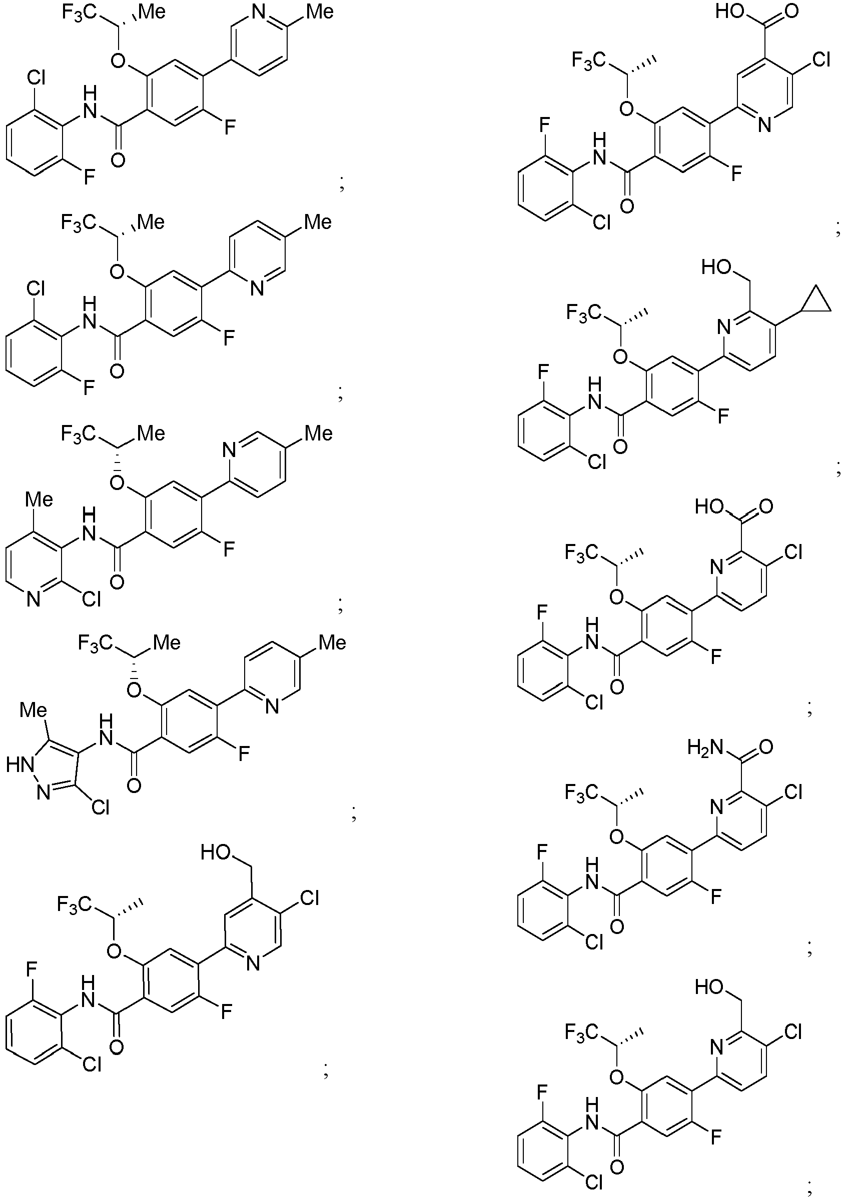 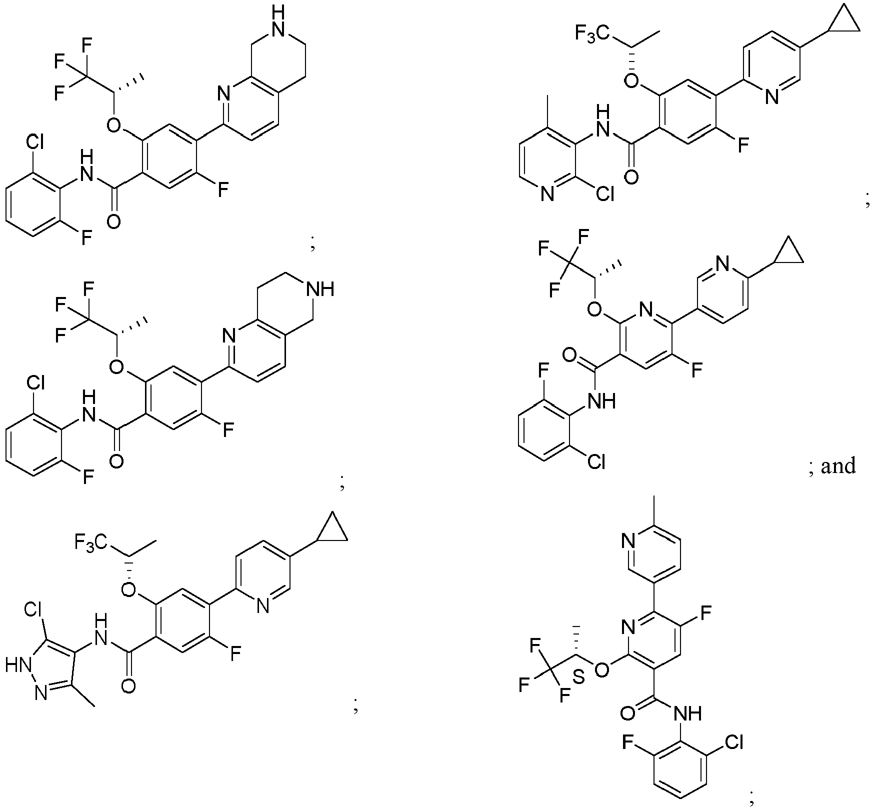 or a pharmaceutically acceptable salt, solvate, stereoisomer, isotopic variant, or N-oxide thereof.

Embodiments of this invention include a compound of Formula II, selected from the group consisting of: 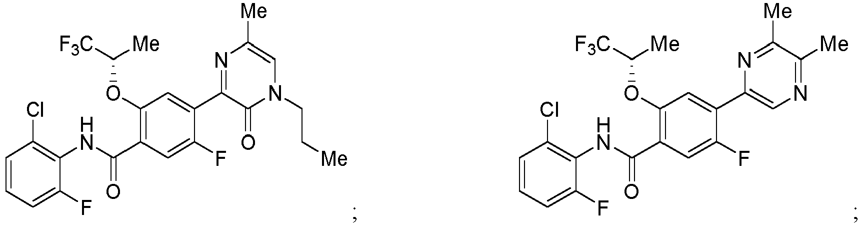 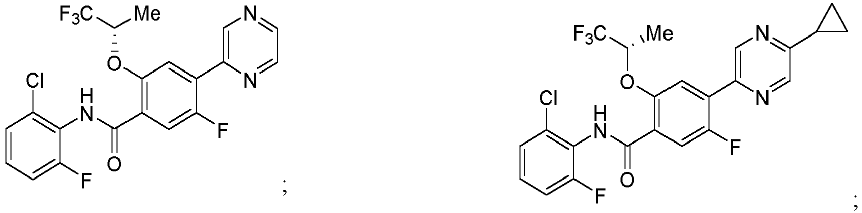 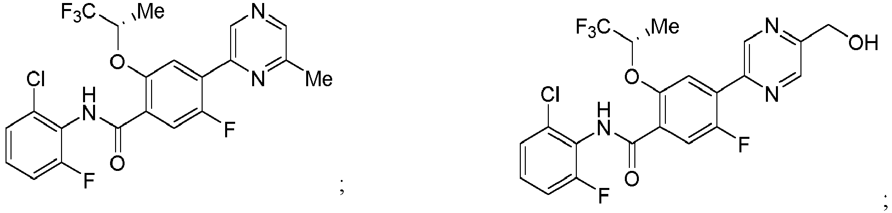 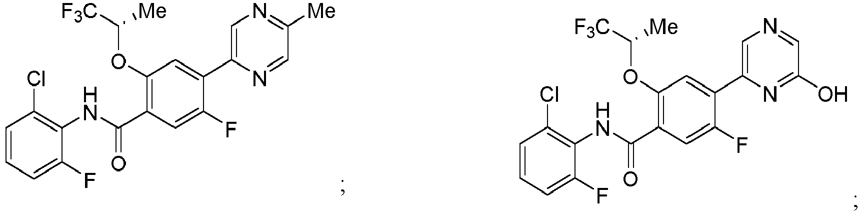 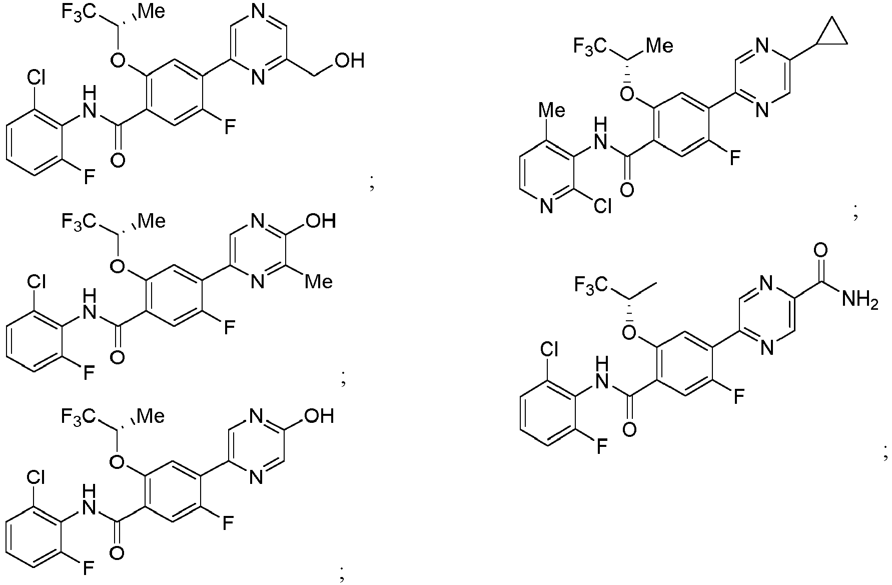 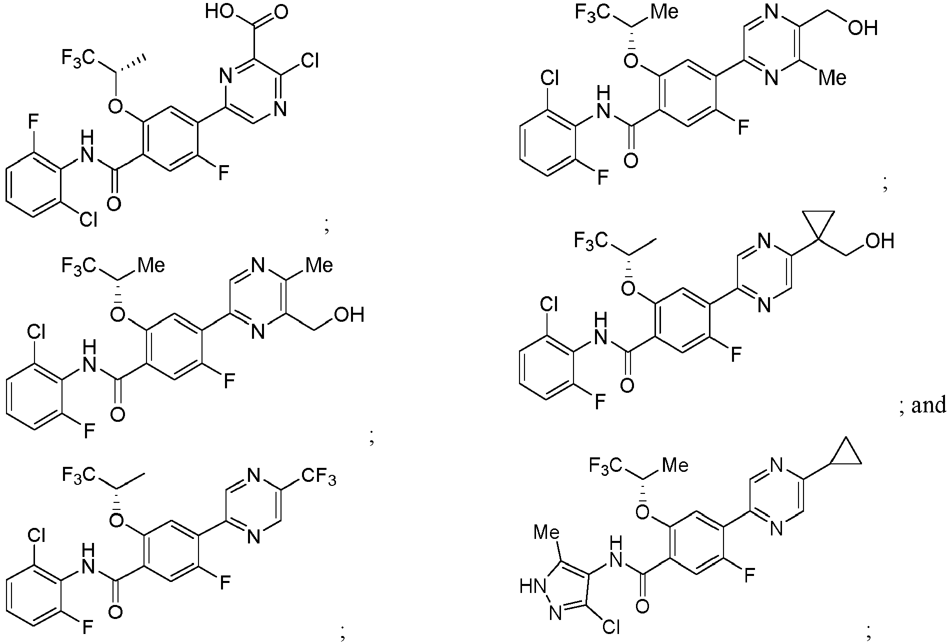 or a pharmaceutically acceptable salt, solvate, stereoisomer, isotopic variant, or N-oxide thereof.

Embodiments of this invention include a compound of Formula II, selected from the group consisting of: 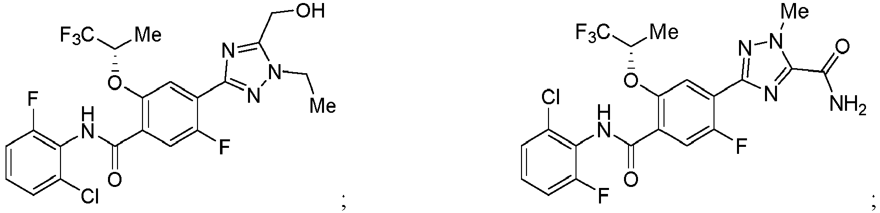 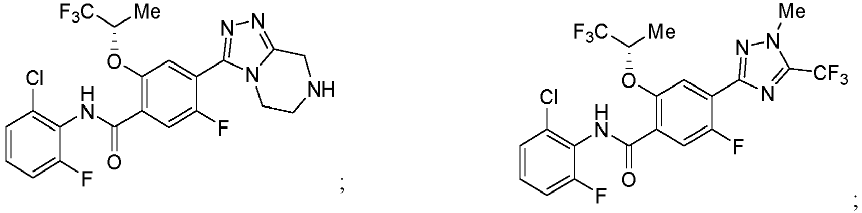 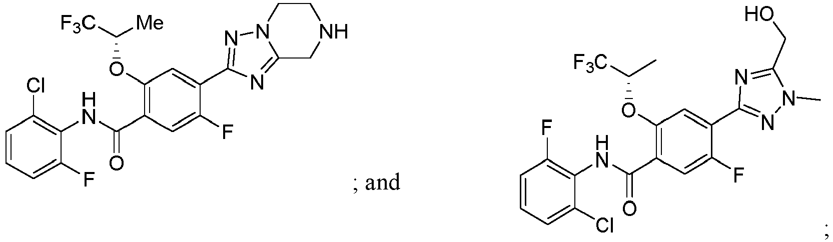 or a pharmaceutically acceptable salt, solvate, stereoisomer, isotopic variant, or N-oxide thereof.

Embodiments of this invention include a compound of Formula II, selected from the group consisting of: 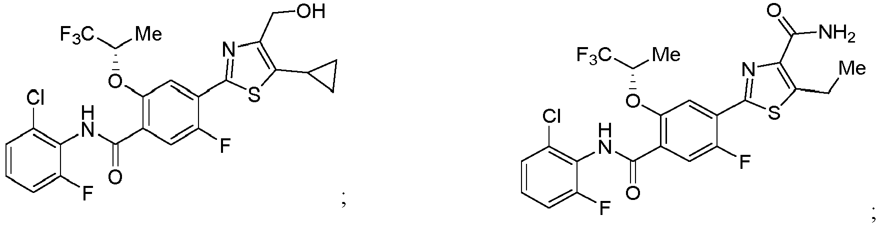 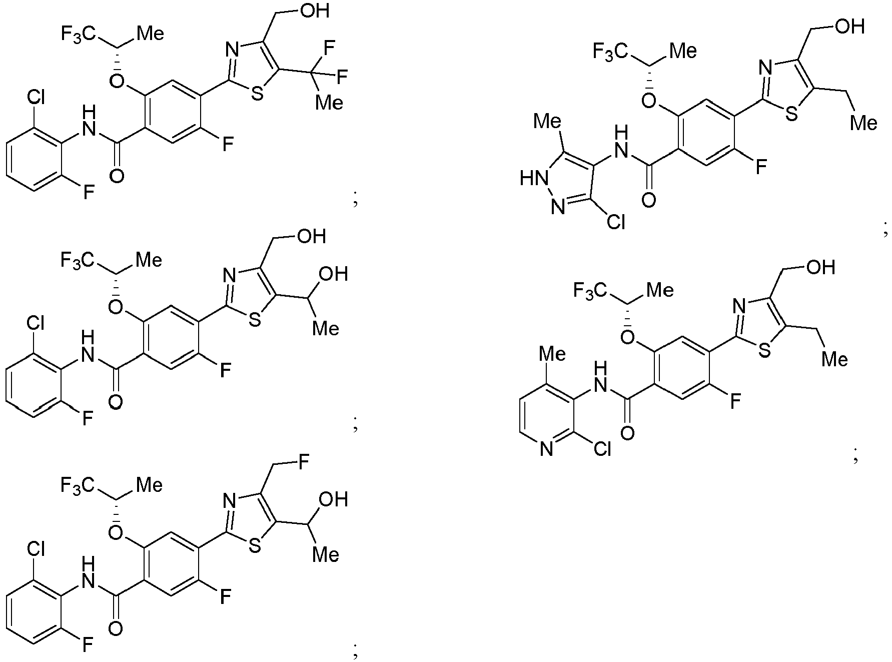 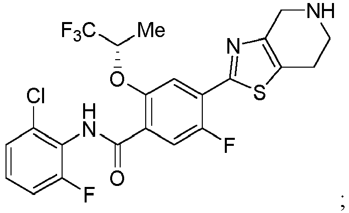 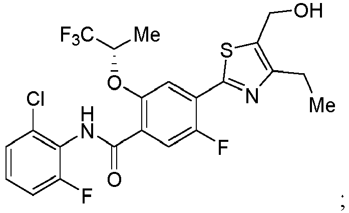 and 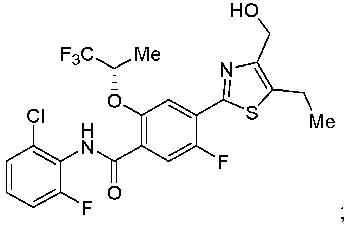 or a pharmaceutically acceptable salt, solvate, stereoisomer, isotopic variant, or N-oxide thereof.

Embodiments of this invention include a compound of Formula II, selected from the group consisting of: 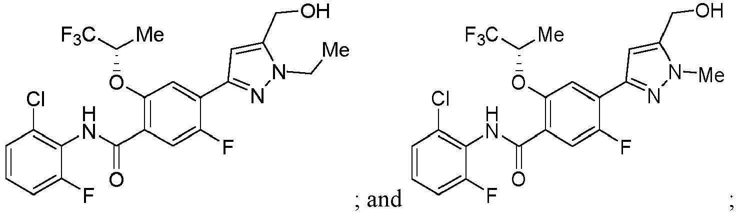 or a pharmaceutically acceptable salt, solvate, stereoisomer, isotopic variant, or N-oxide thereof.

Embodiments of this invention include a compound of Formula II, selected from the group consisting of: 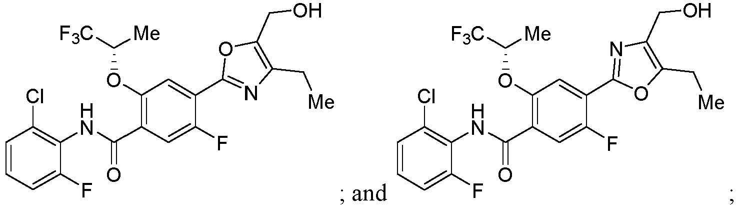 or a pharmaceutically acceptable salt, solvate, stereoisomer, isotopic variant, or N-oxide thereof.

Embodiments of this invention include a compound of Formula II, which is: 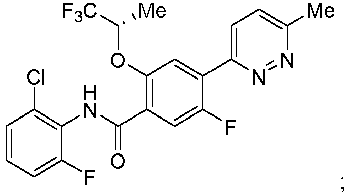 or a pharmaceutically acceptable salt, solvate, stereoisomer, isotopic variant, or N-oxide thereof.

Embodiments of this invention include a compound of Formula II, selected from the group consisting of: 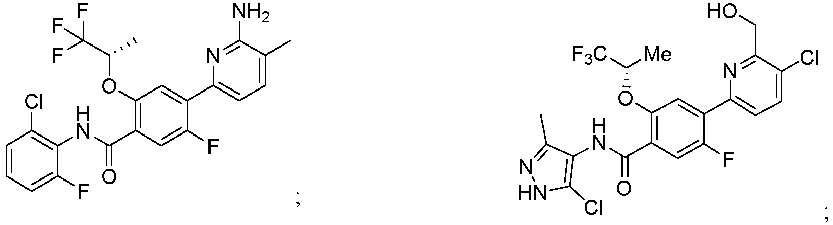 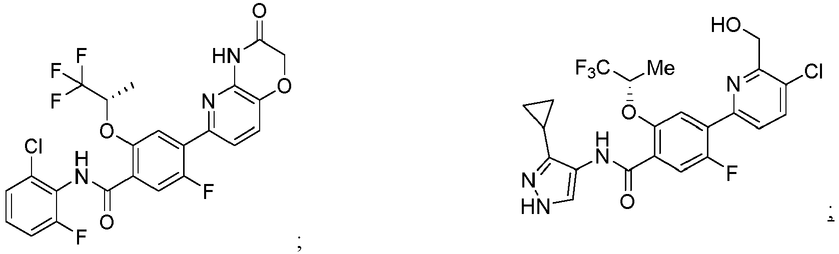 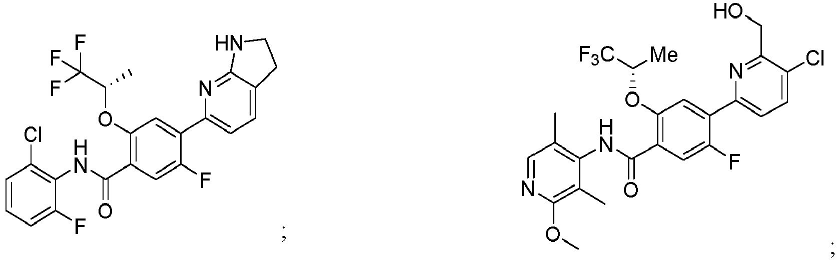 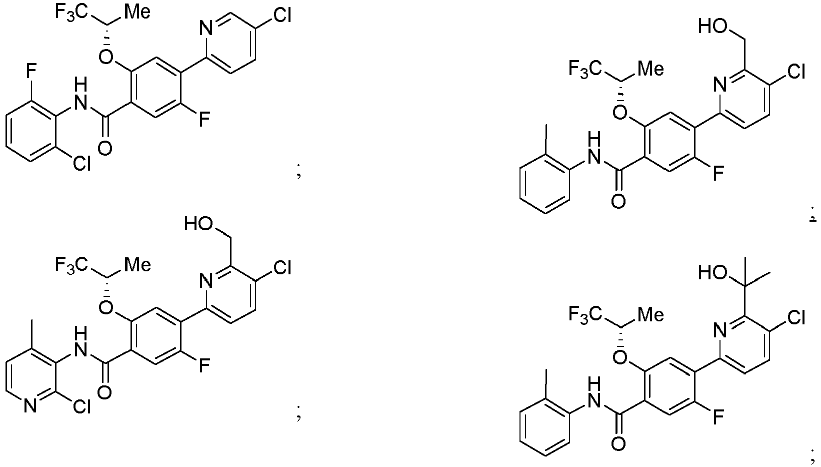 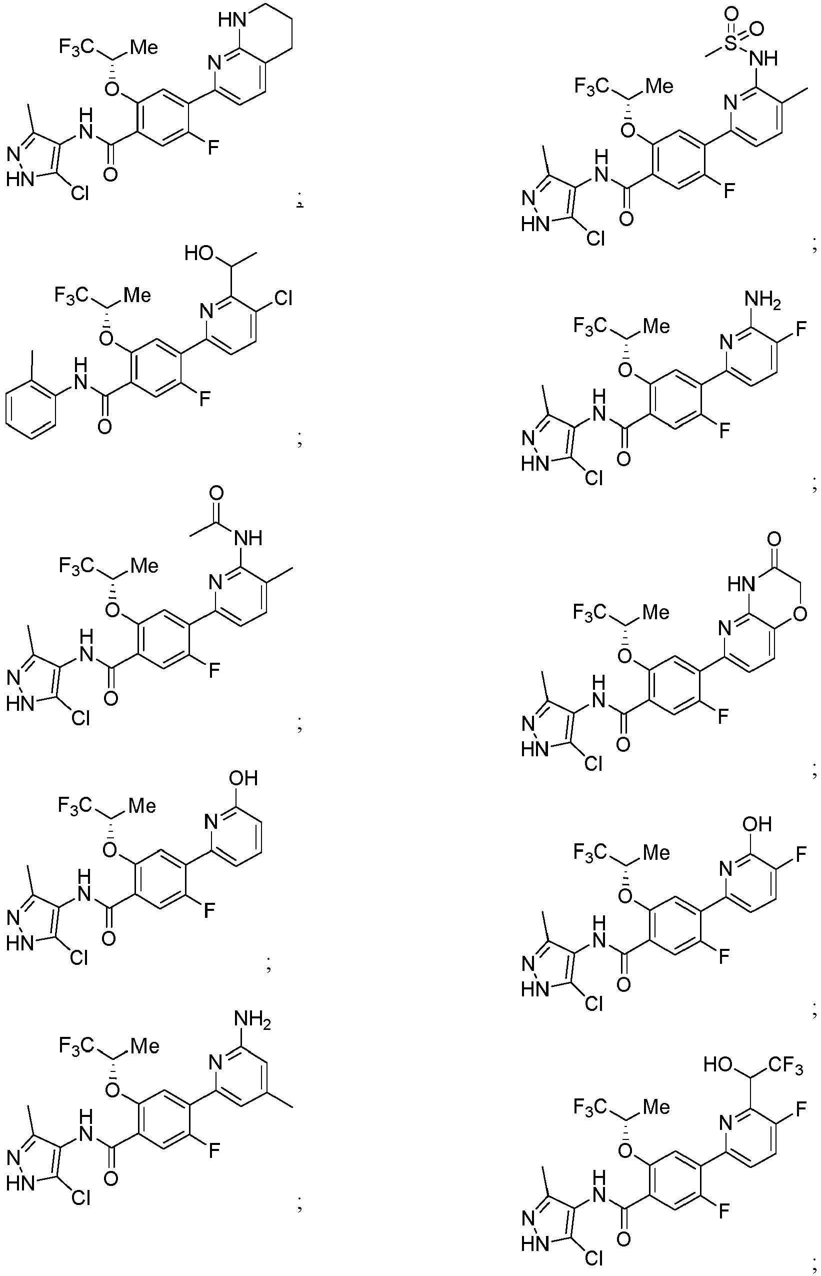 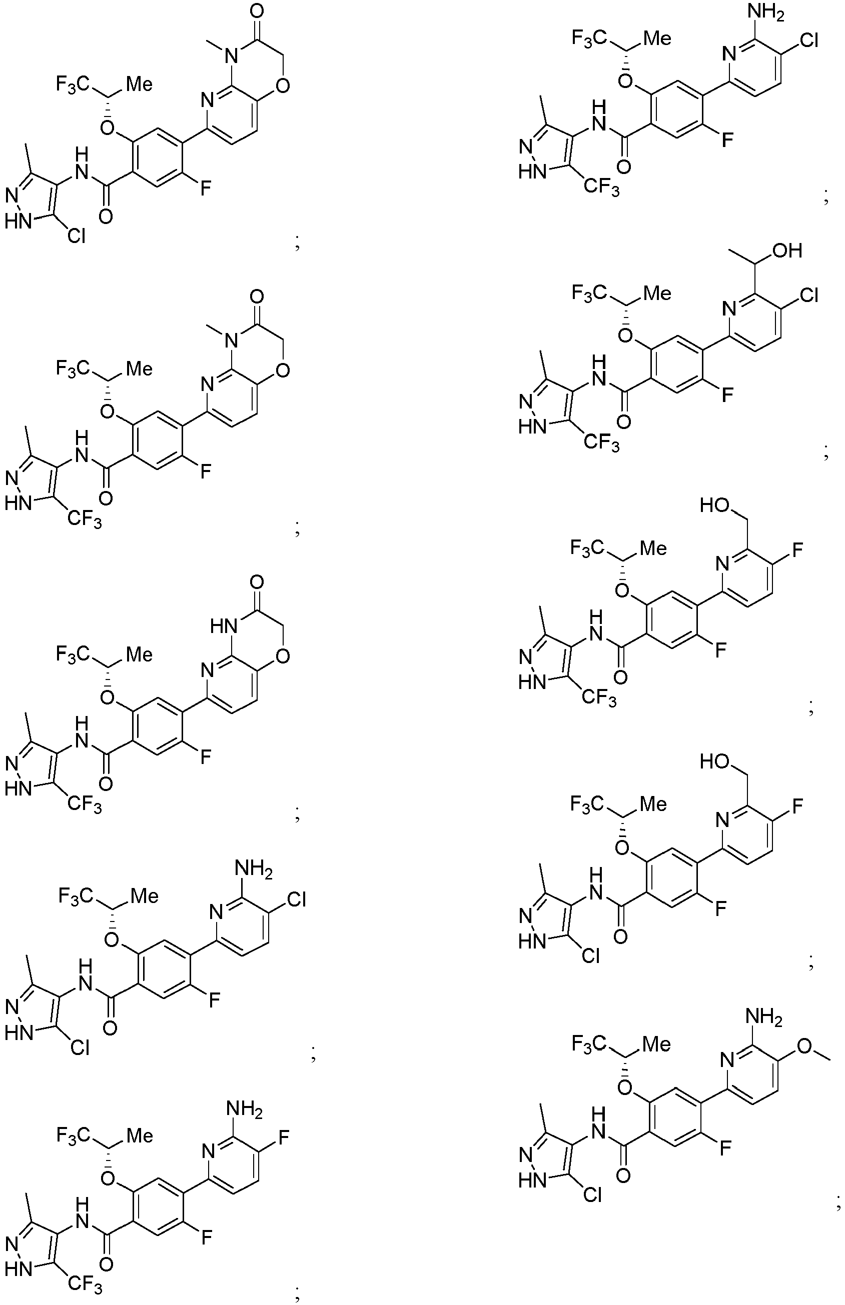 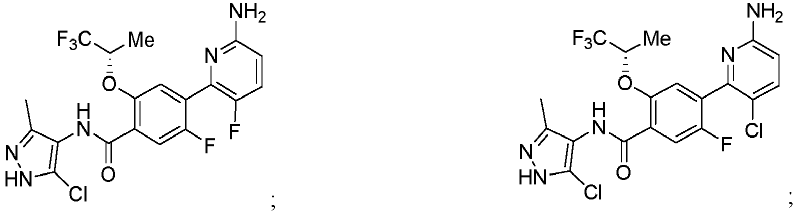 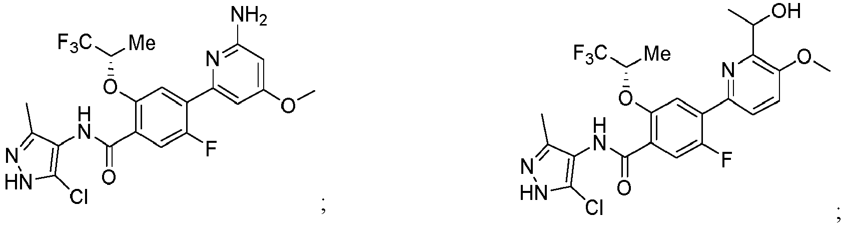 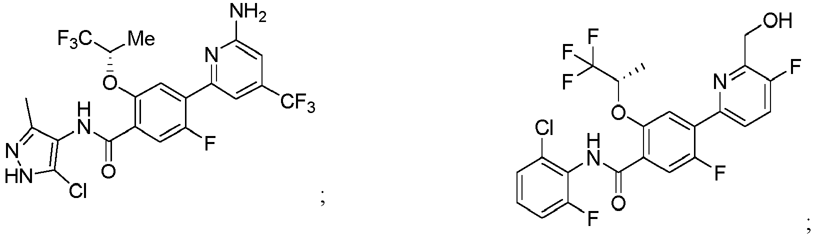 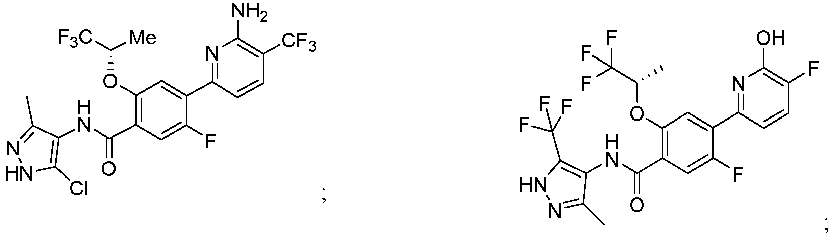 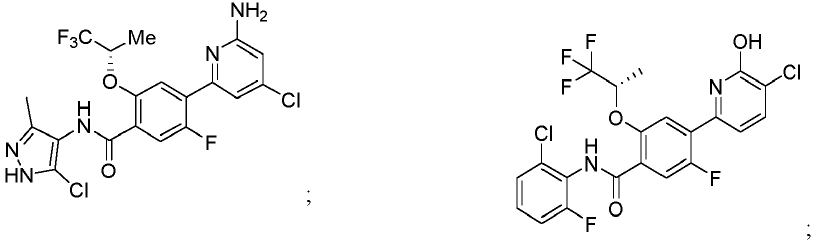 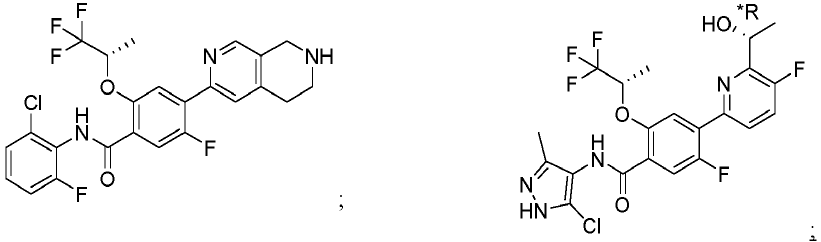 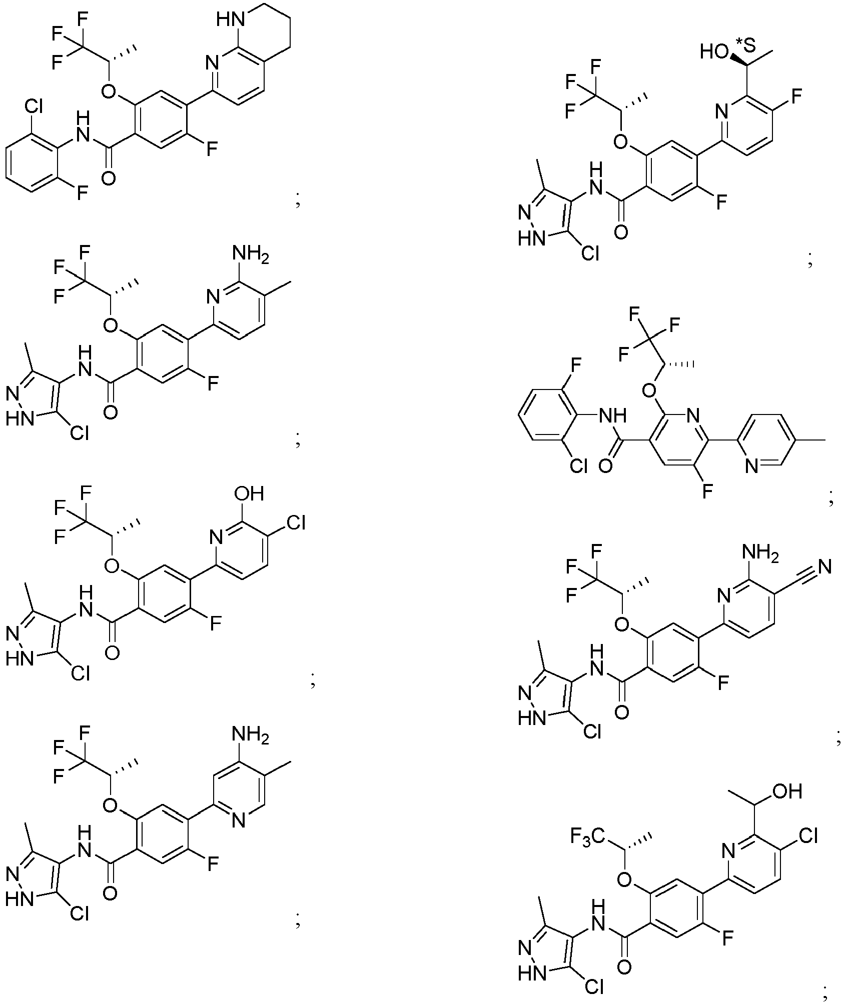 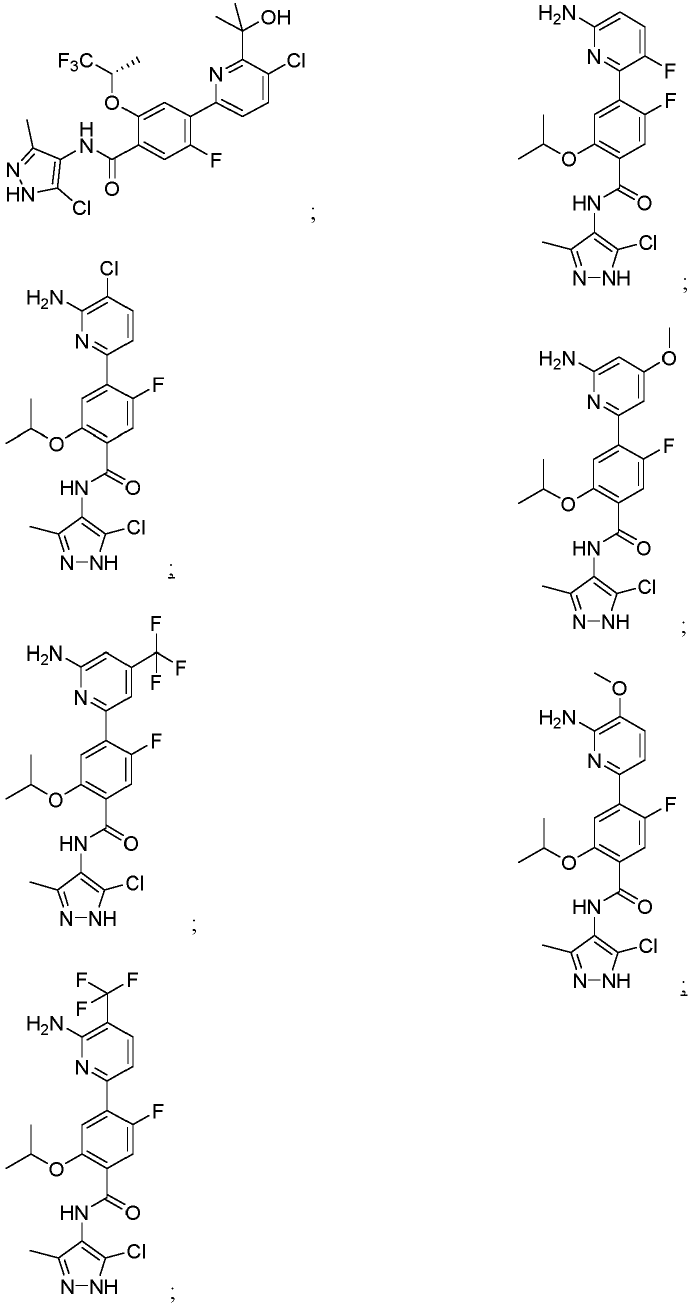 and or a pharmaceutically acceptable salt, solvate, stereoisomer, isotopic variant, or N-oxide thereof.

Embodiments of this invention include a compound of Formula II, selected from the group consisting of: 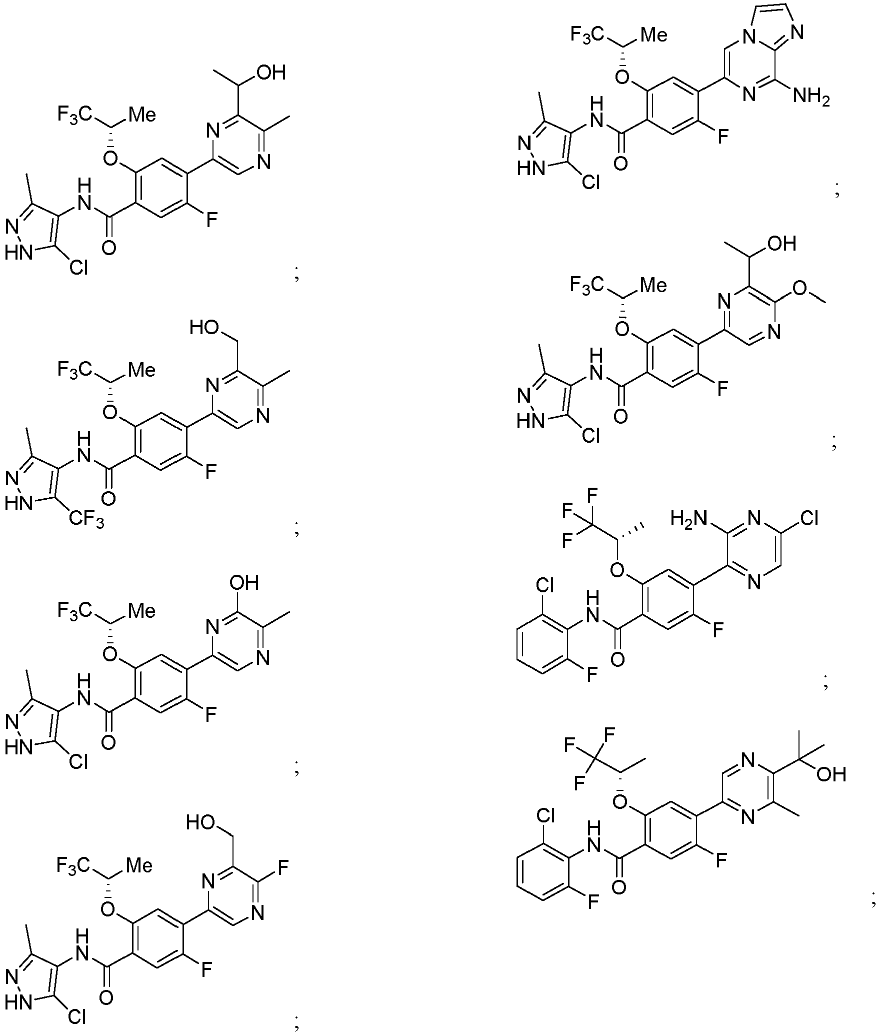 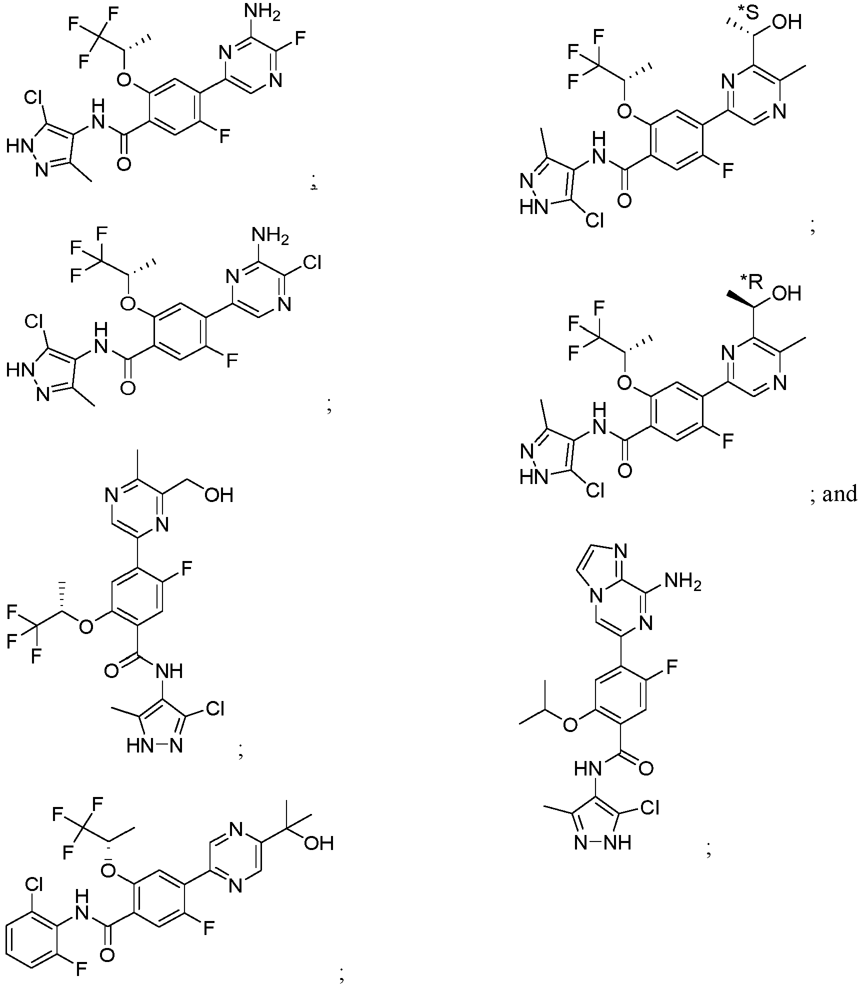 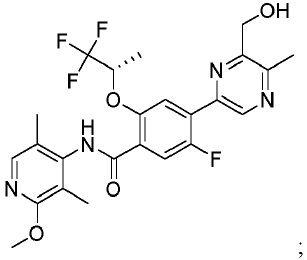 or a pharmaceutically acceptable salt, solvate, stereoisomer, isotopic variant, or N-oxide thereof.

Embodiments of this invention include a compound of Formula II, selected from the group consisting of: 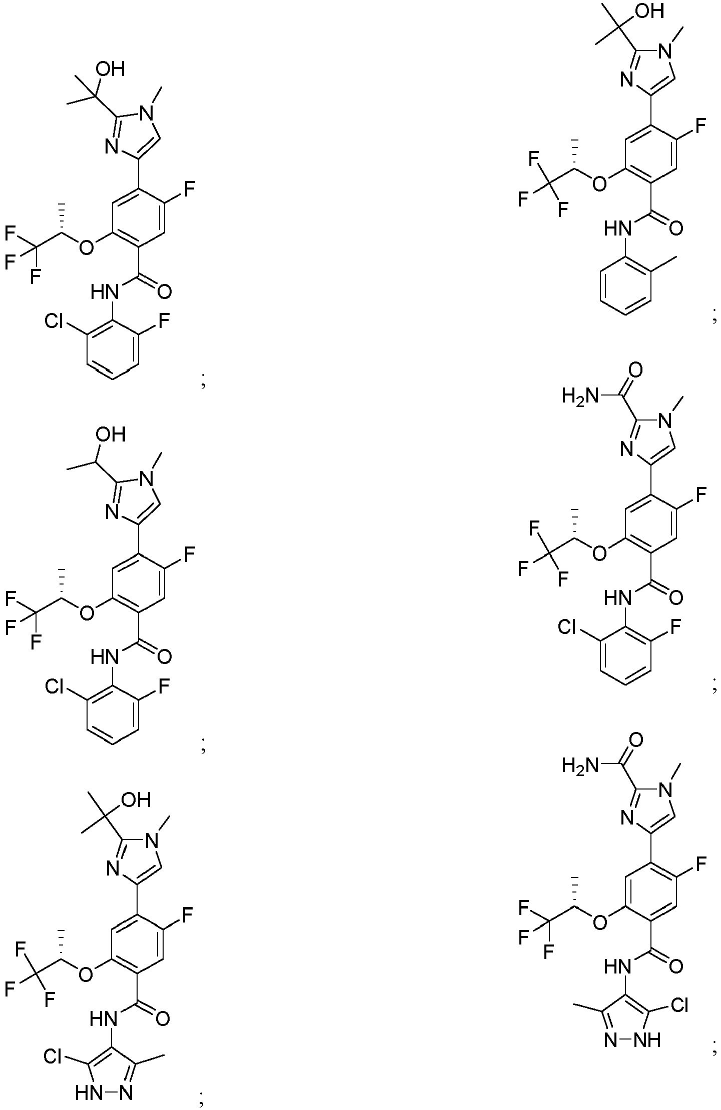 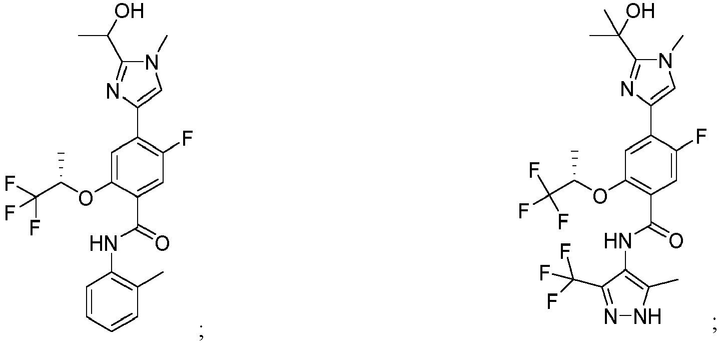 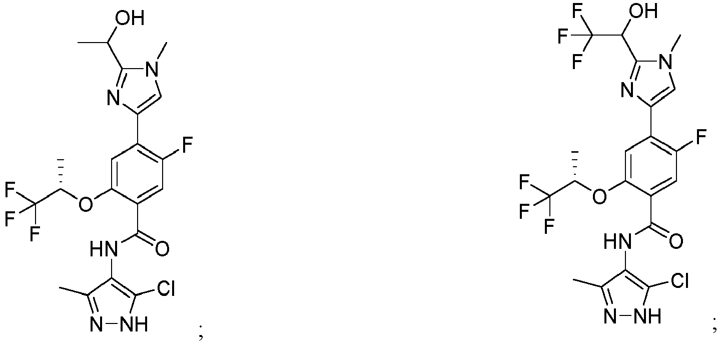 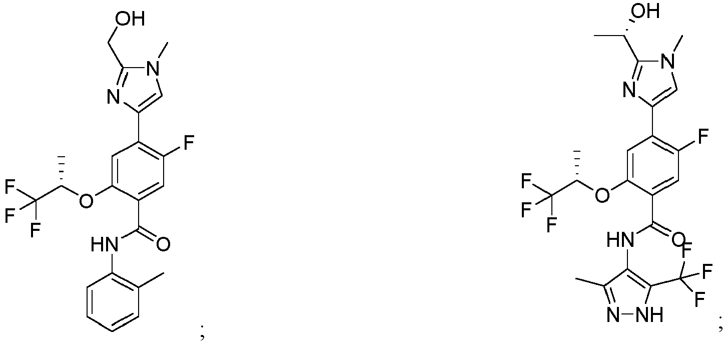 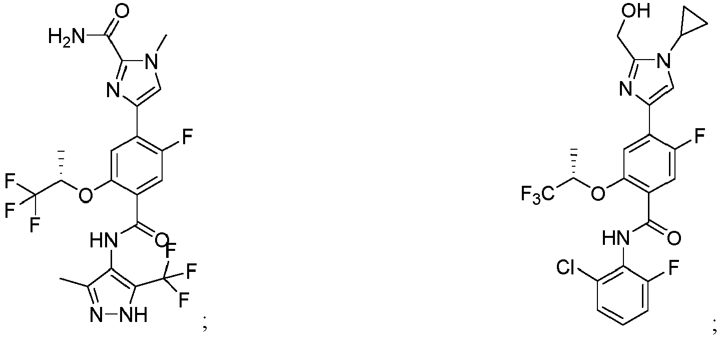 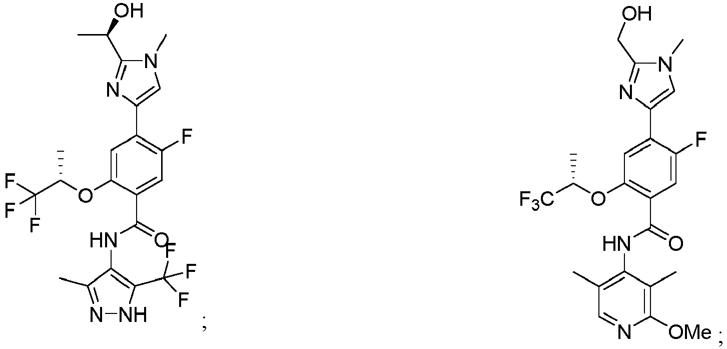 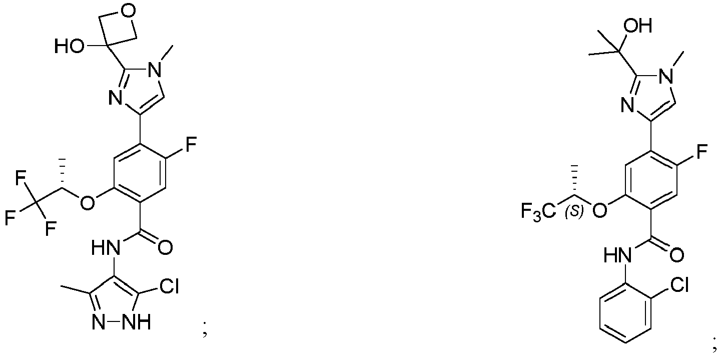 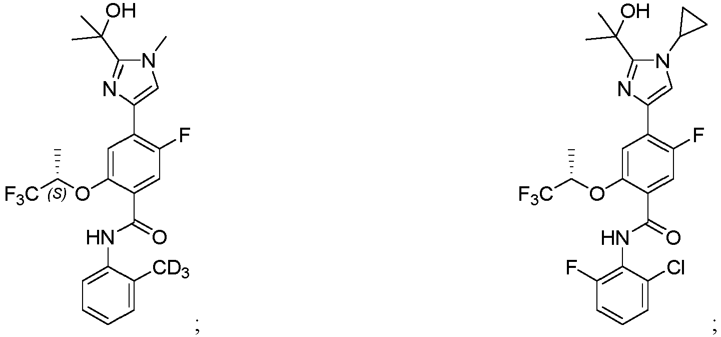 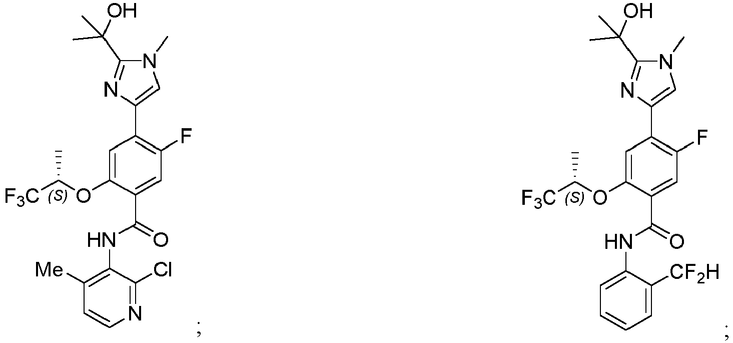 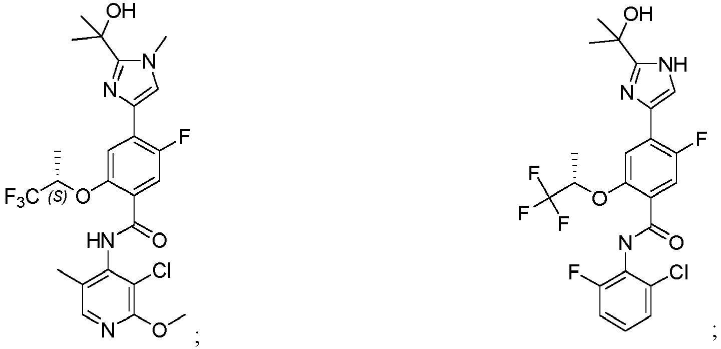 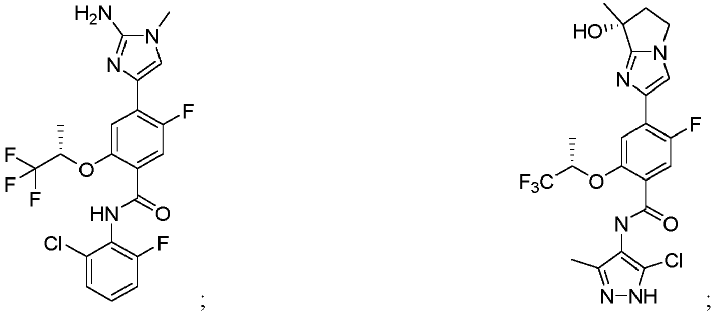 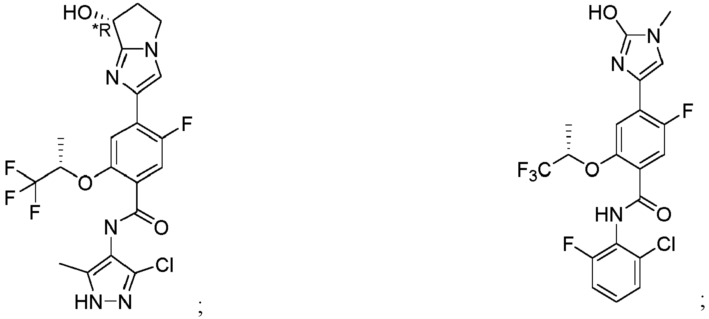 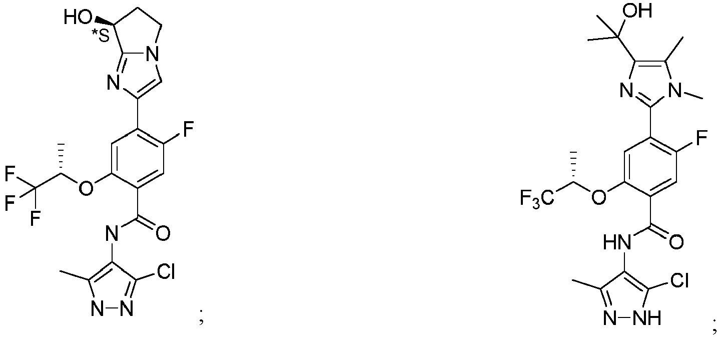 and 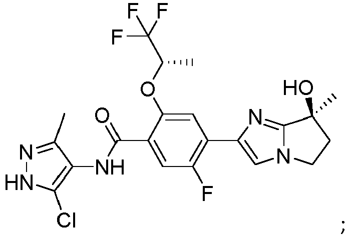 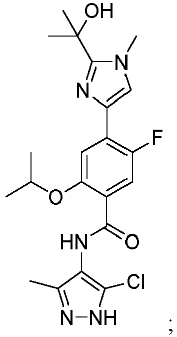 or a pharmaceutically acceptable salt, solvate, stereoisomer, isotopic variant, or N-oxide thereof.

Embodiments of this invention include a compound of Formula II, selected from the group consisting of: 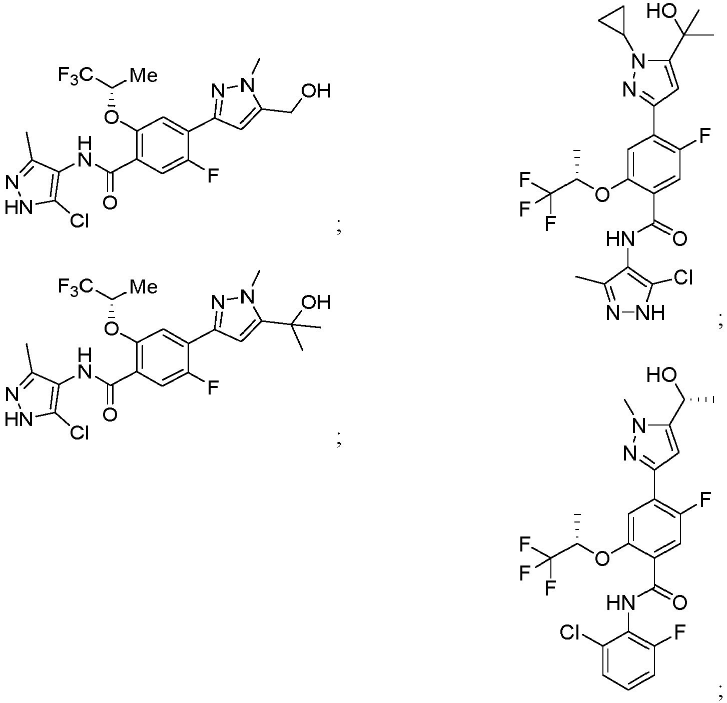 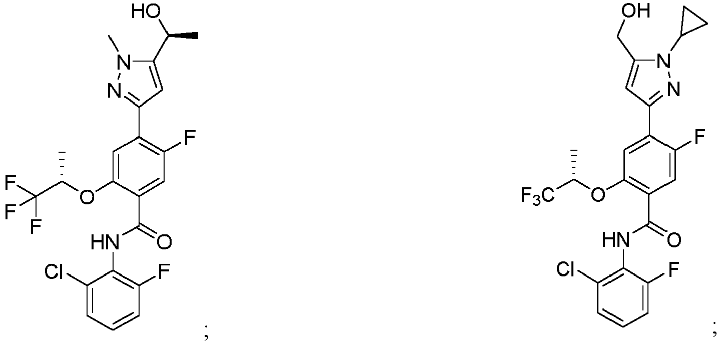 and 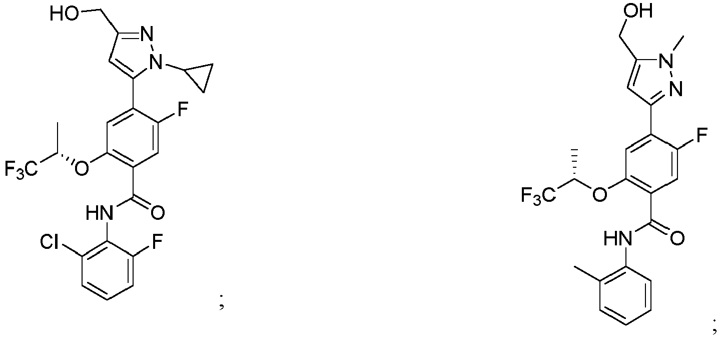 or a pharmaceutically acceptable salt, solvate, stereoisomer, isotopic variant, or N-oxide thereof.

Embodiments of this invention include the compound of Formula II, which is: 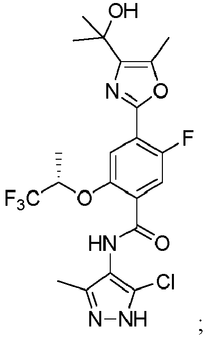 or a pharmaceutically acceptable salt, solvate, stereoisomer, isotopic variant, or N-oxide thereof.

Embodiments of this invention include a compound of Formula II, selected from the group consisting of: 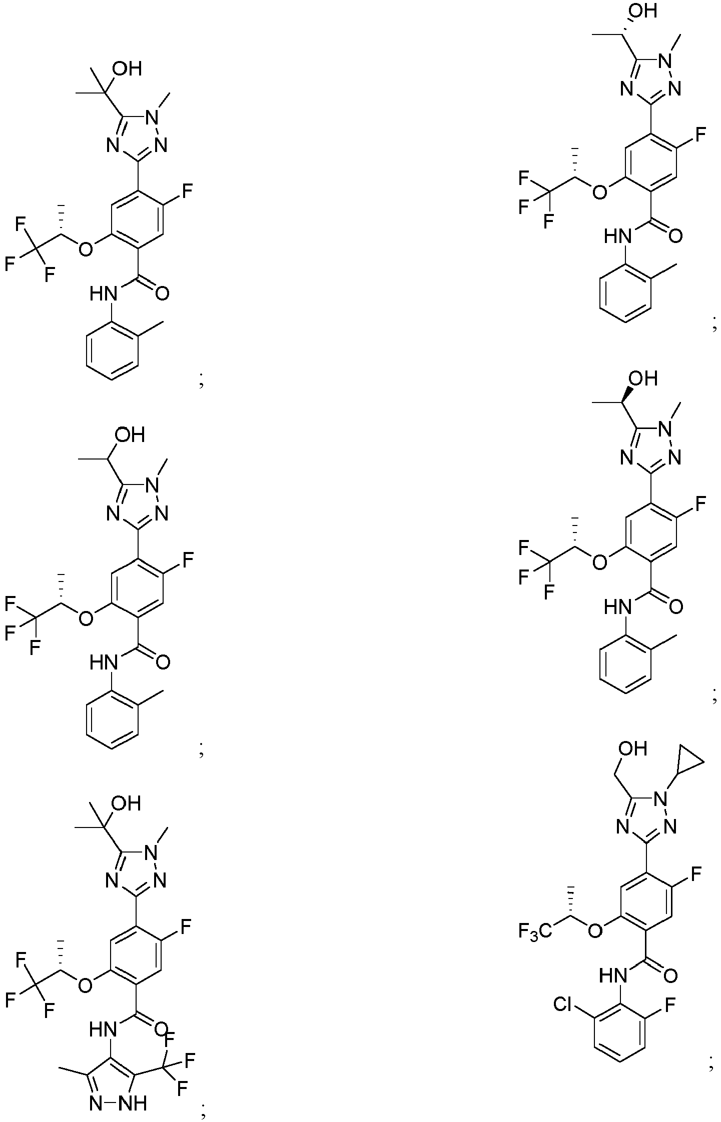 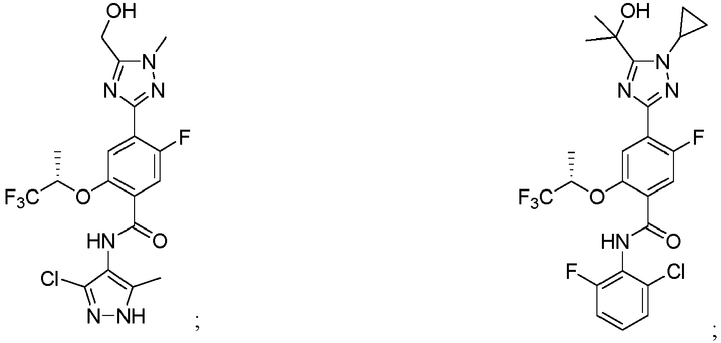 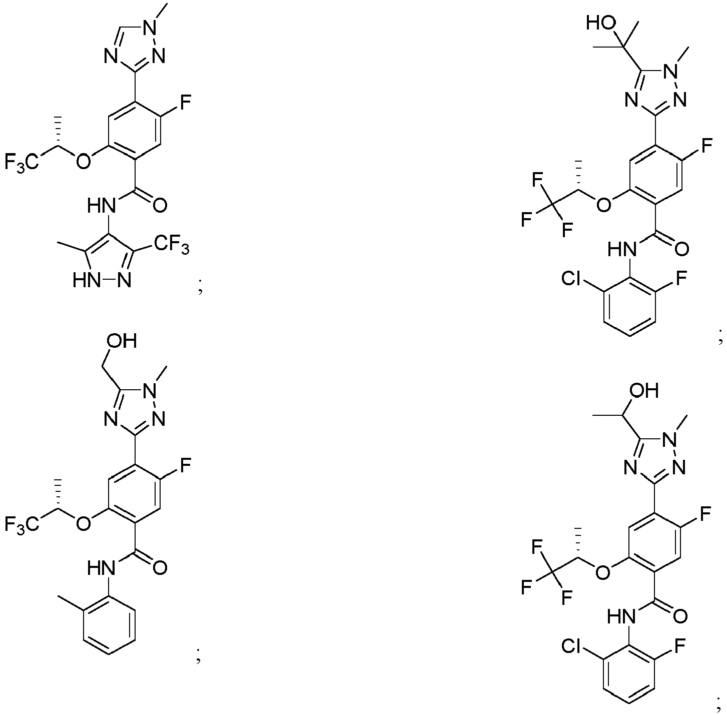 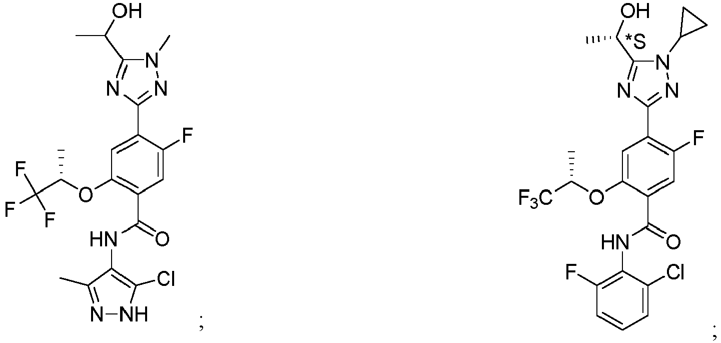 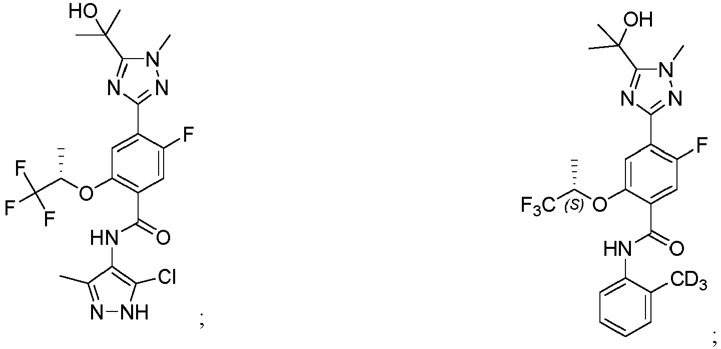 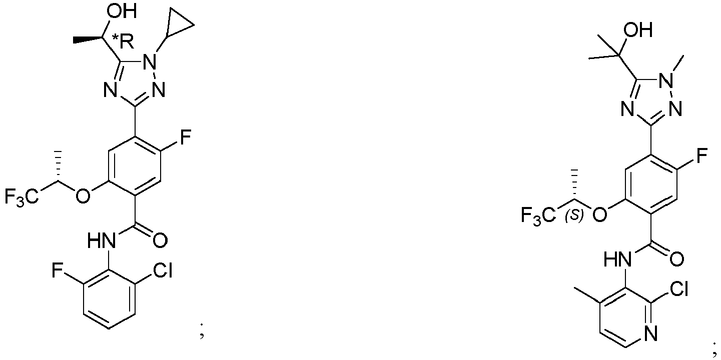 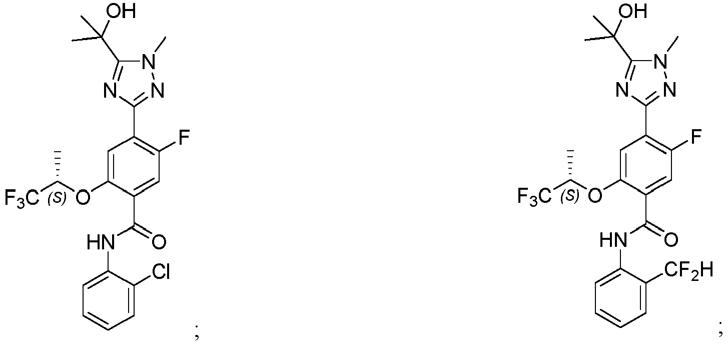 and 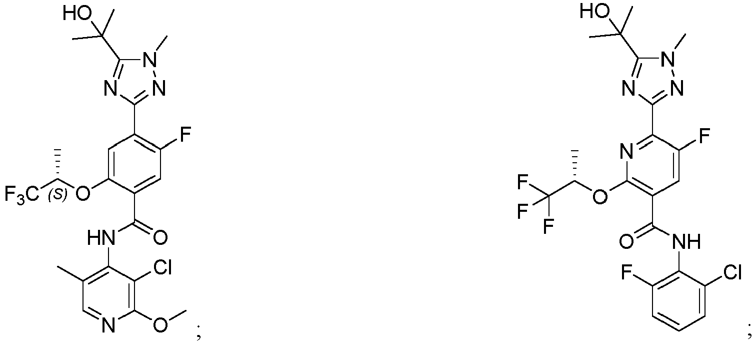 or a pharmaceutically acceptable salt, solvate, stereoisomer, isotopic variant, or N-oxide thereof.

Embodiments of this invention include a compound of Formula II, selected from the group consisting of: 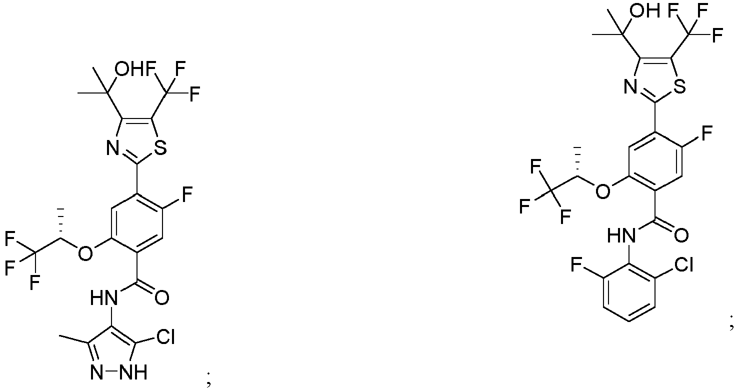 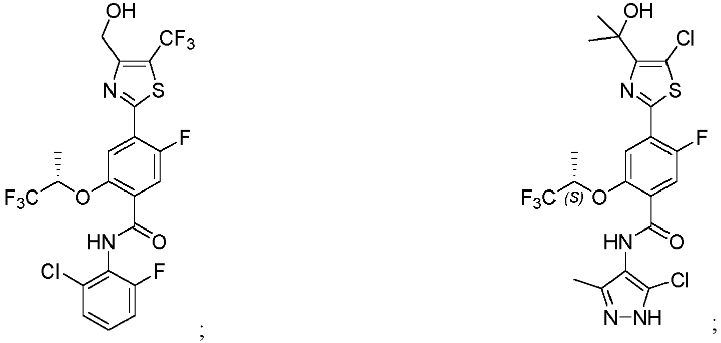 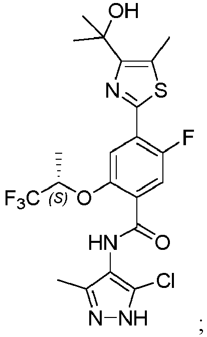 and or a pharmaceutically acceptable salt, solvate, stereoisomer, isotopic variant, or N-oxide thereof.

Embodiments of this invention include a compound of Formula II, selected from the group consisting of: 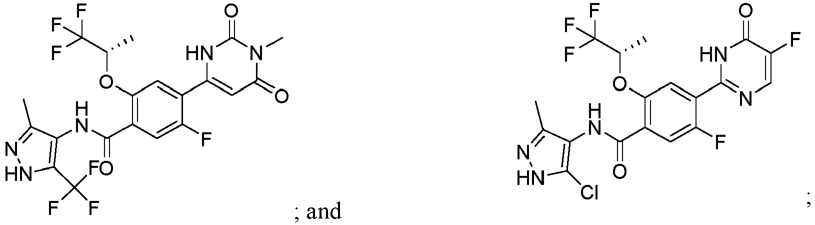 or a pharmaceutically acceptable salt, solvate, stereoisomer, isotopic variant, or N-oxide thereof.

Embodiments of this invention include a compound of Formula II, which is: 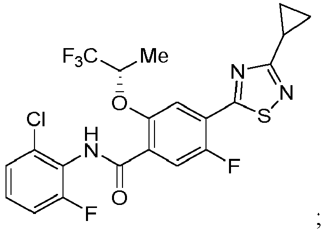 or a pharmaceutically acceptable salt, solvate, stereoisomer, isotopic variant, or N-oxide thereof.

Embodiments of this invention include a compound of Formula II, which is: 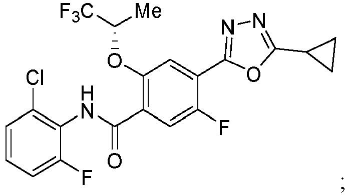 or a pharmaceutically acceptable salt, solvate, stereoisomer, isotopic variant, or N-oxide thereof.

Disclosed herein are embodiments including a pharmaceutical composition comprising: (A) a compound according to any of the embodiments described herein, such as compounds of Formula I or II, or a pharmaceutically acceptable salt, solvate, stereoisomer, isotopic variant, or N-oxide thereof; and (B) at least one pharmaceutically acceptable excipient.

Disclosed herein are embodiments including a pharmaceutical composition comprising an effective amount of a compound selected from the group consisting of: 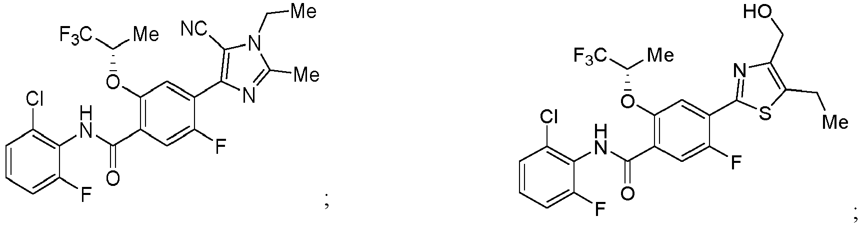 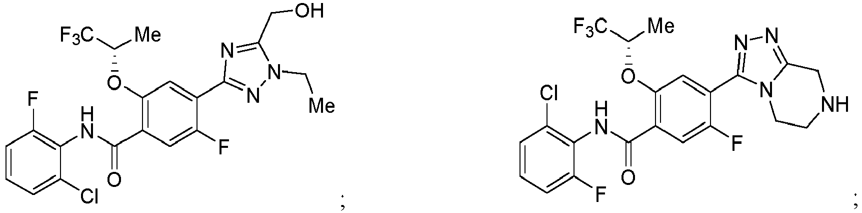 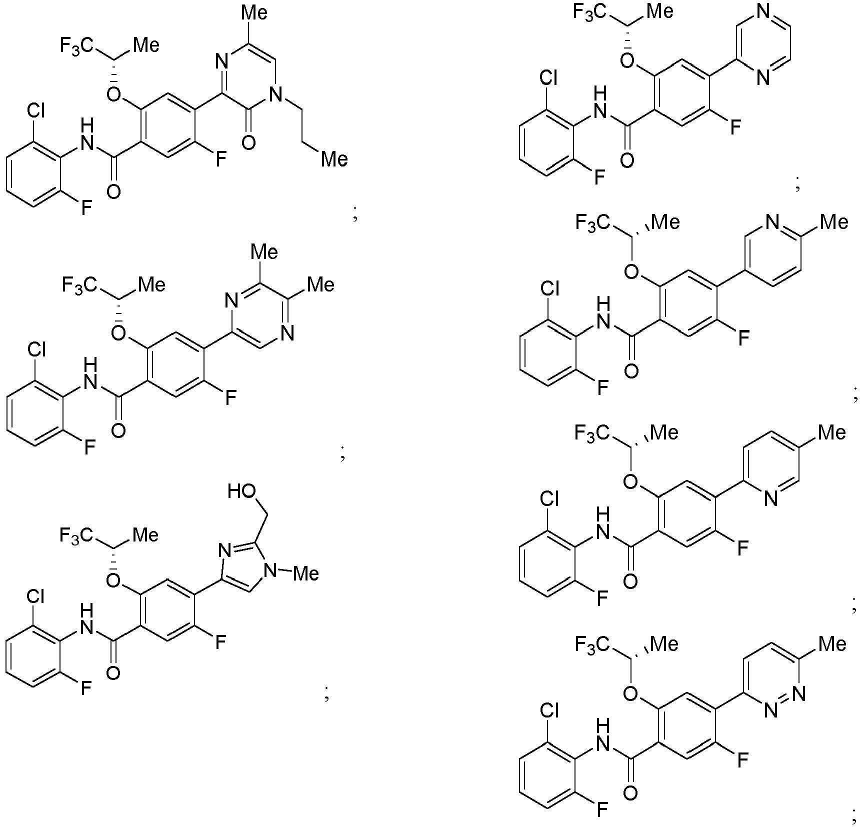 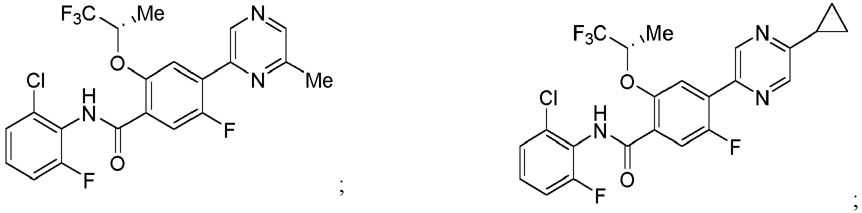 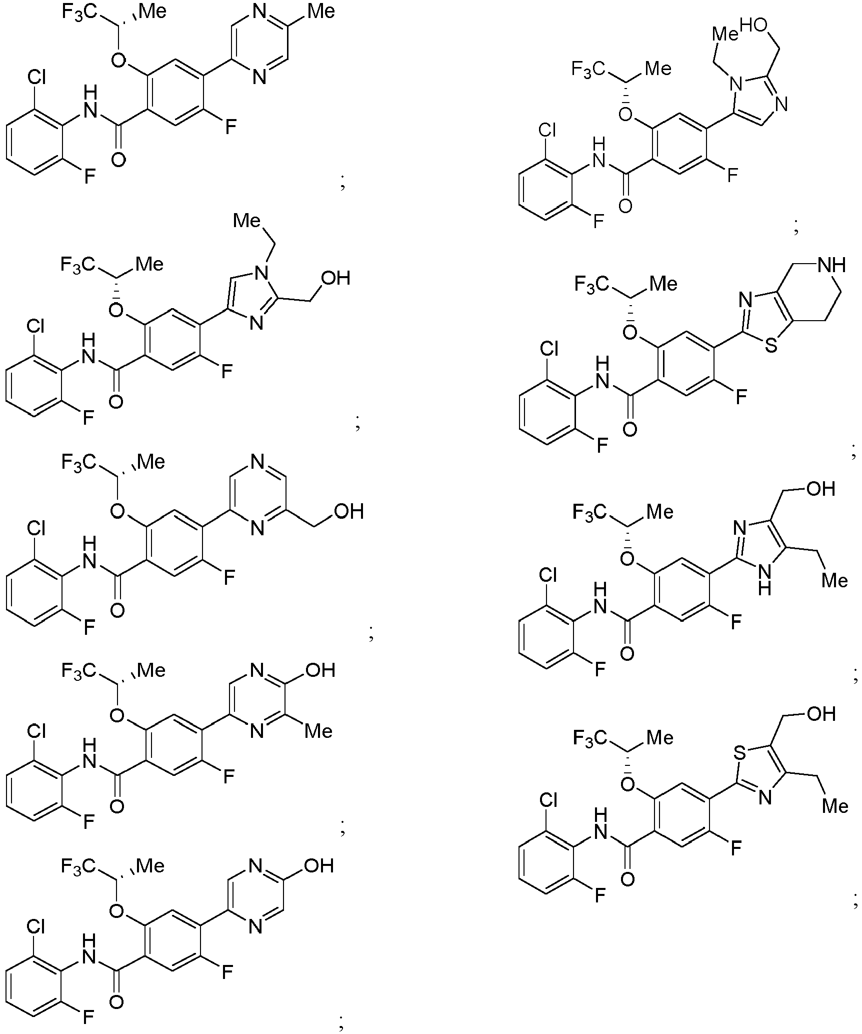 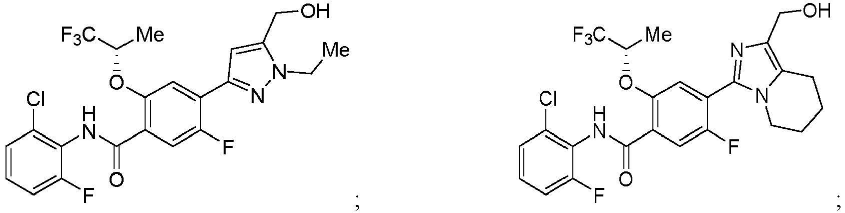 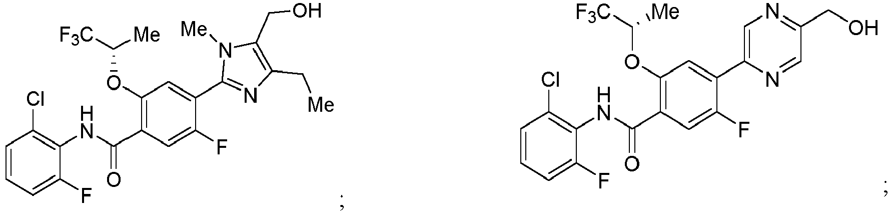 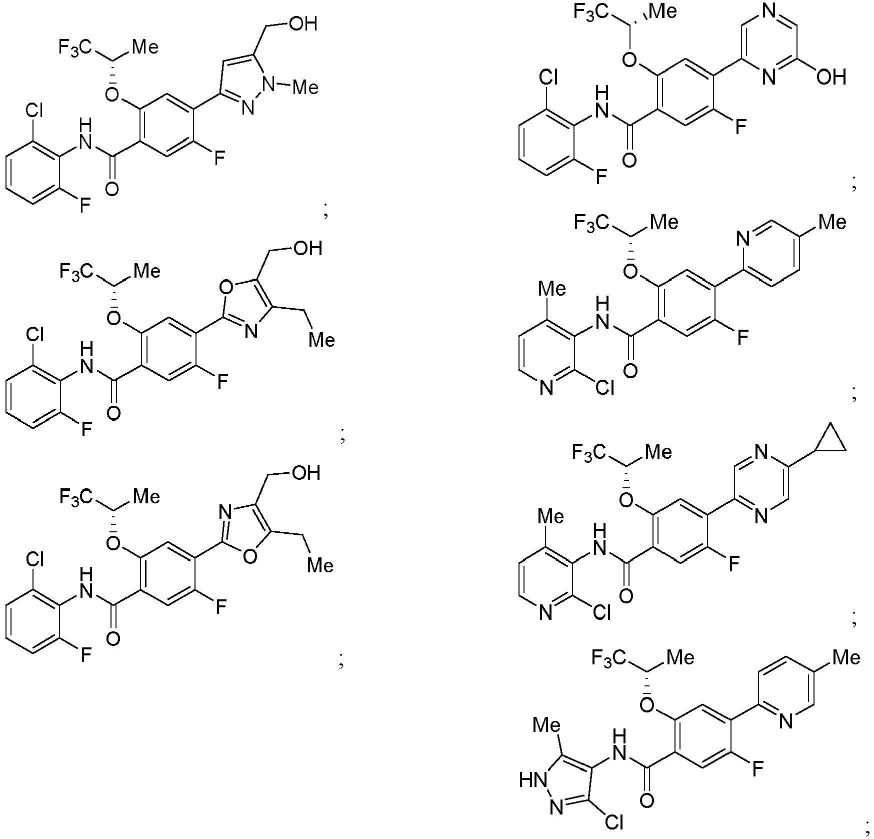 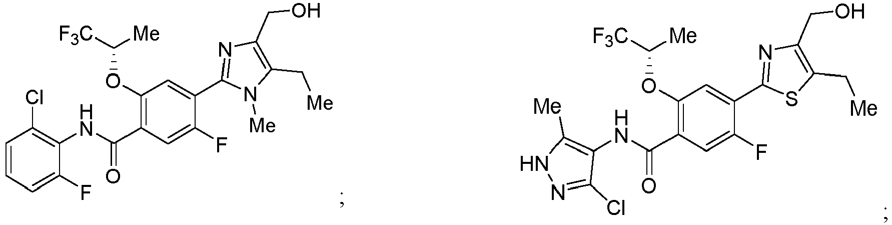 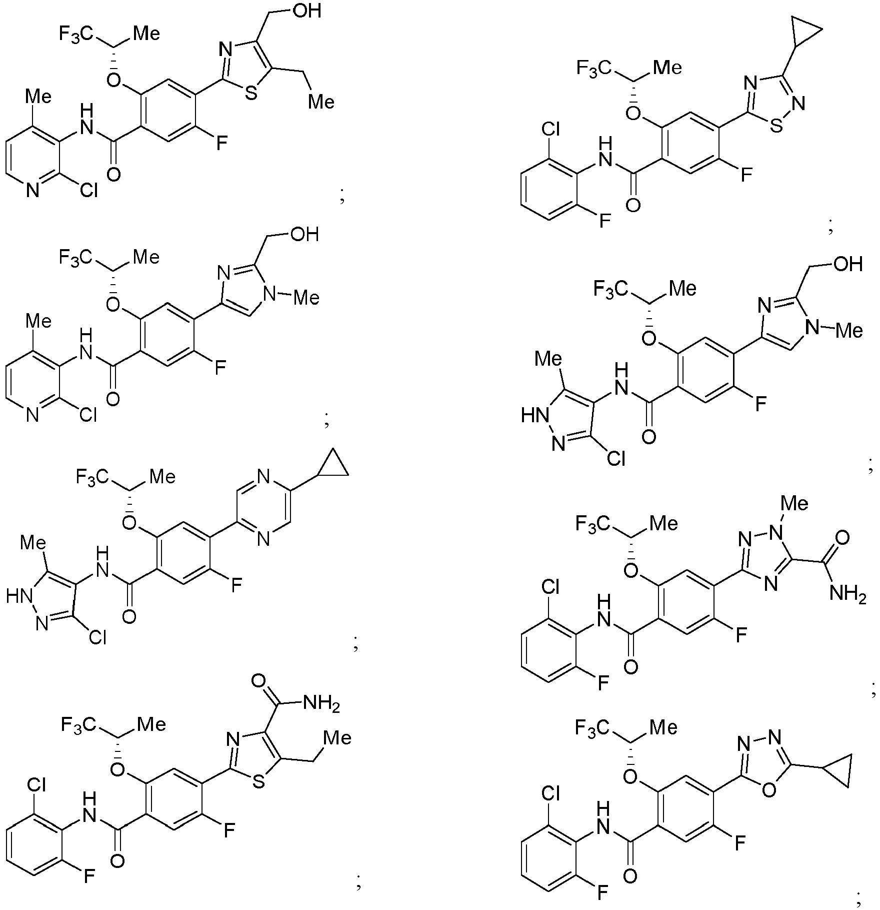 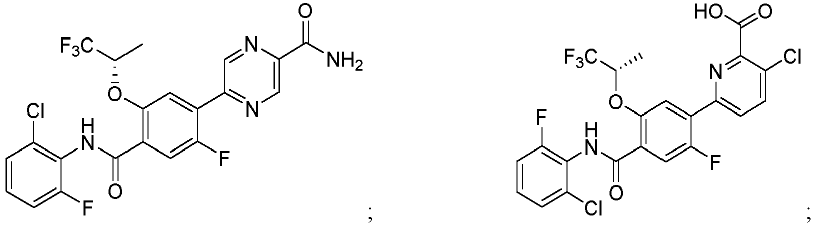 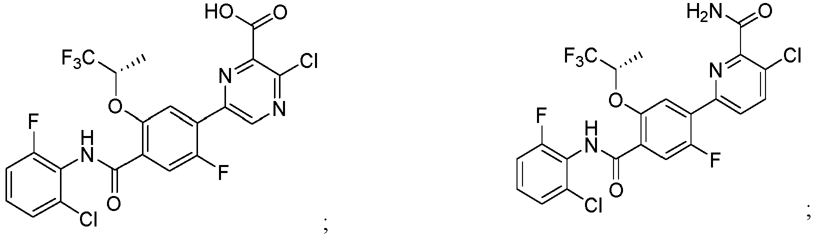 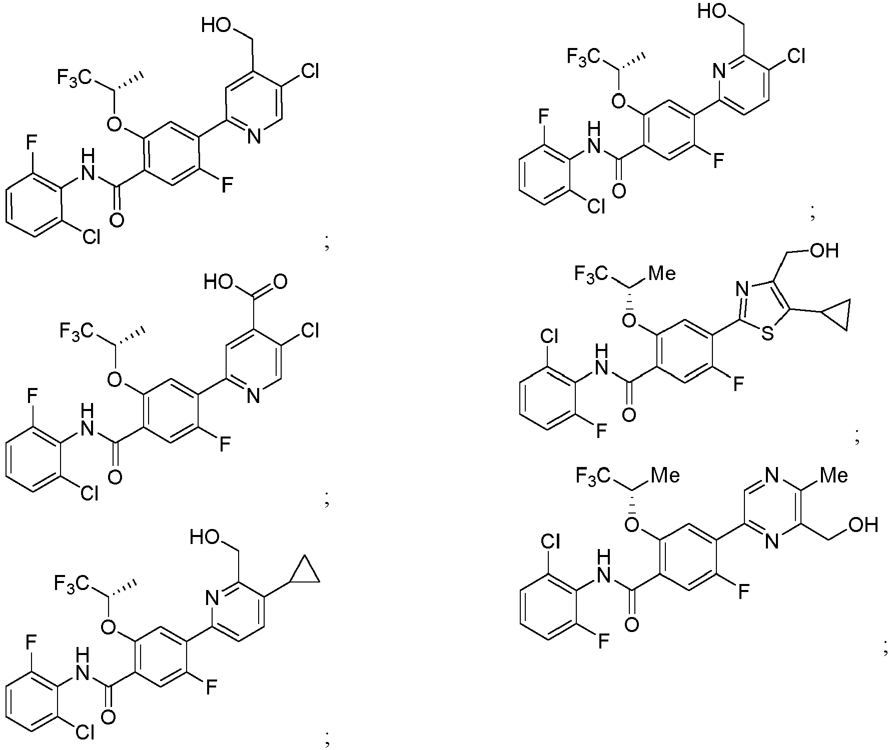 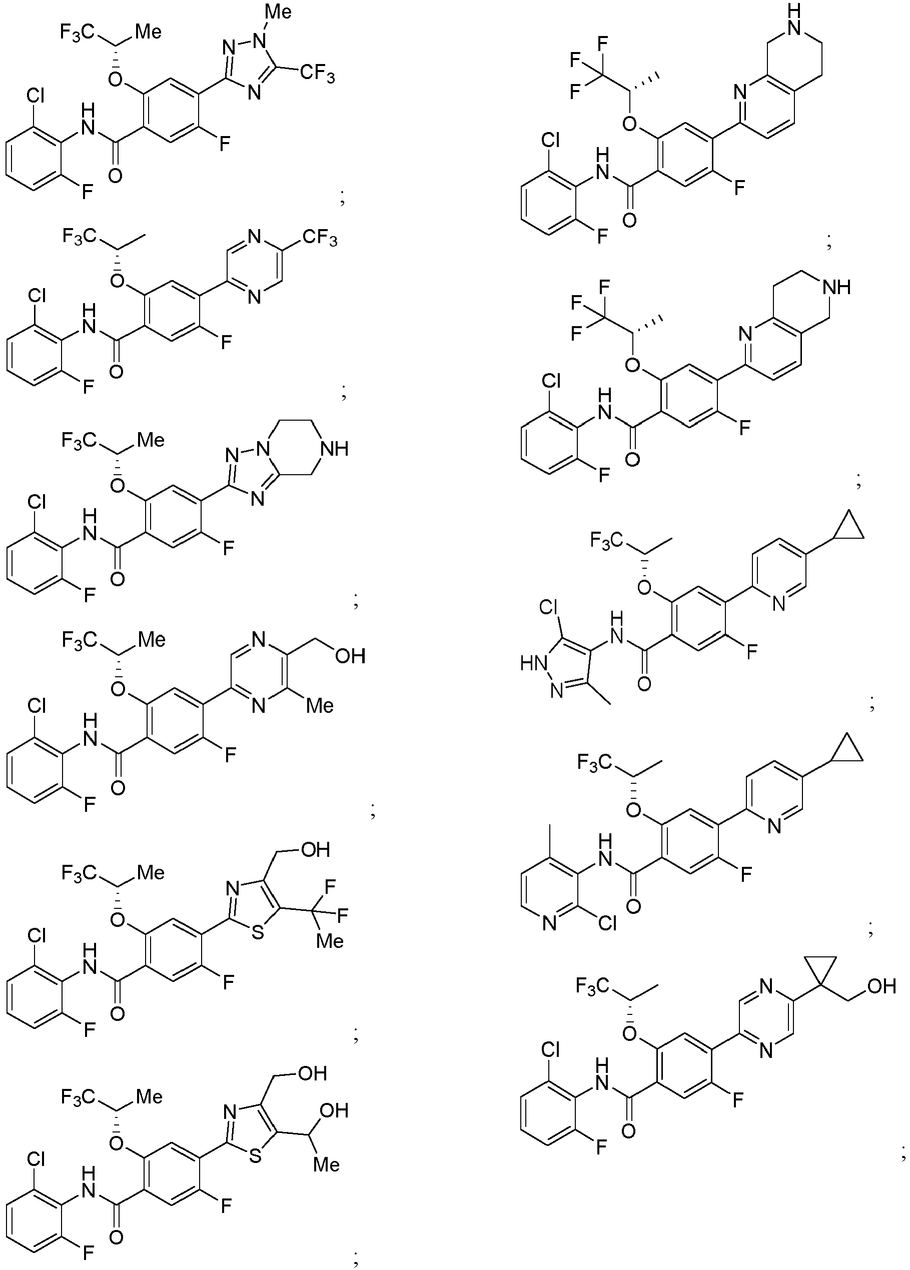 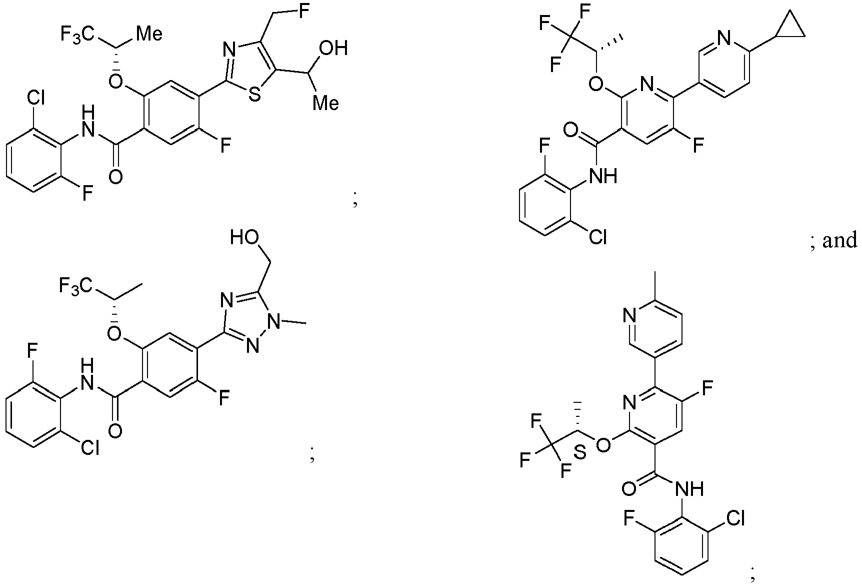 or a pharmaceutically acceptable salt, solvate, stereoisomer, isotopic variant, or N-oxide thereof; and at least one pharmaceutically acceptable excipient.

Another embodiment of the invention is a pharmaceutical composition comprising an effective amount of a compound selected from the group consisting of: 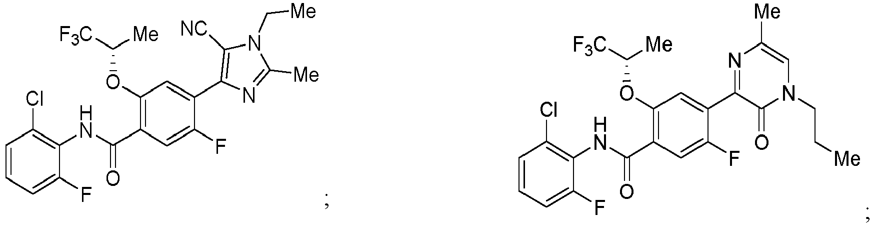 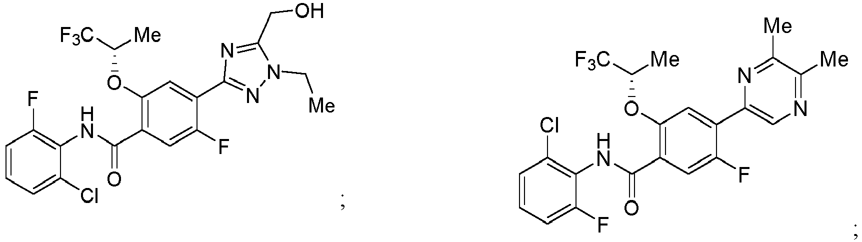 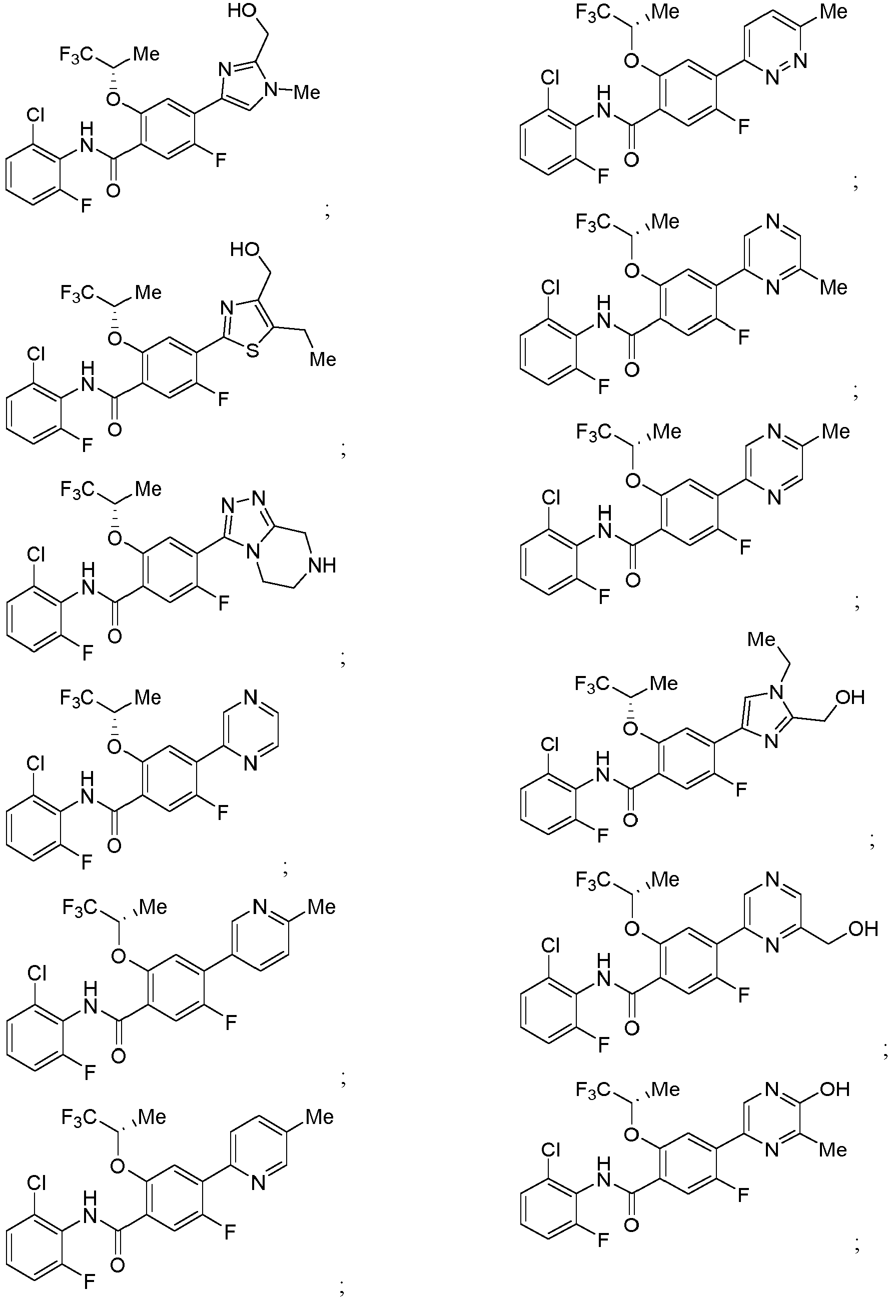 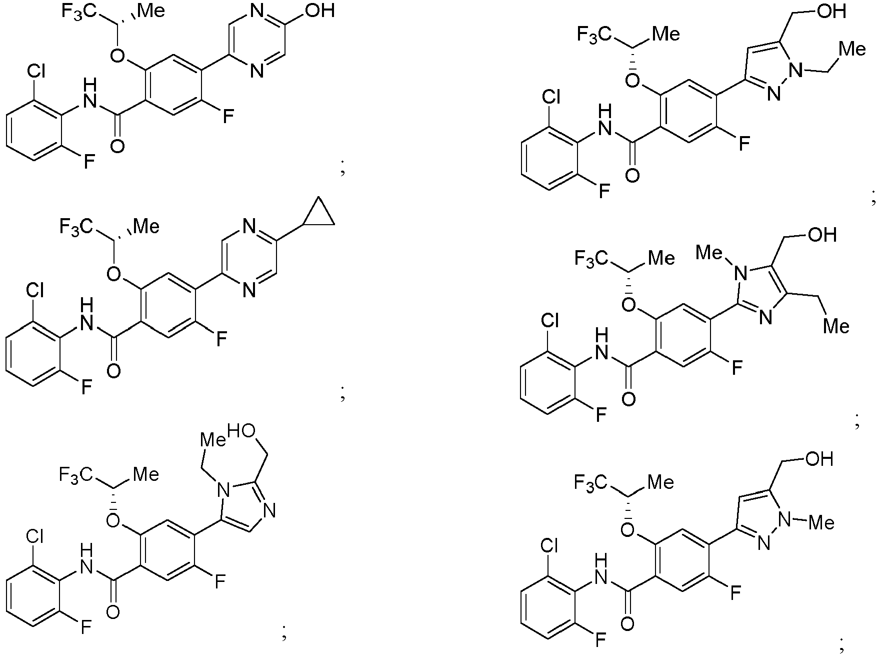 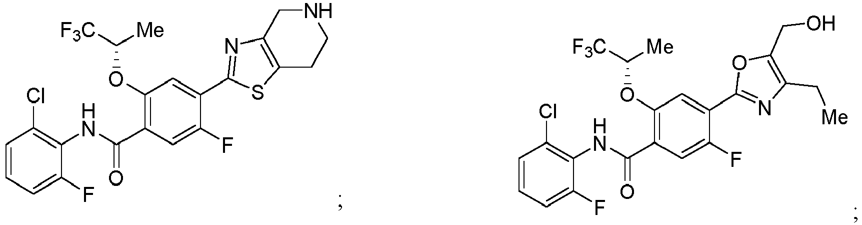 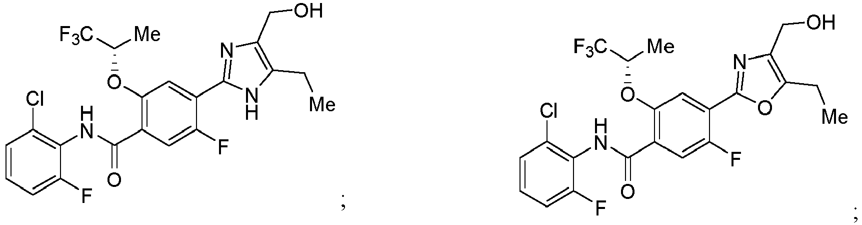 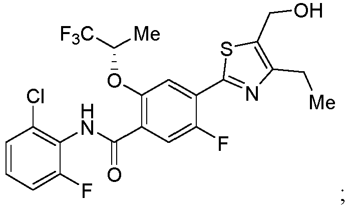 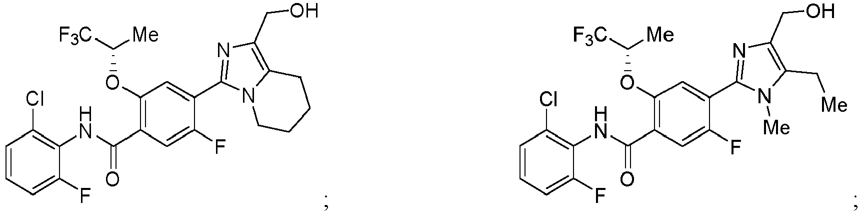 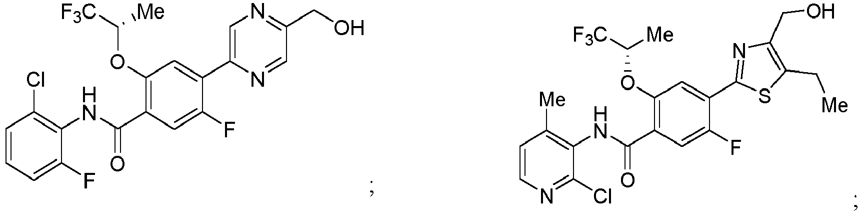 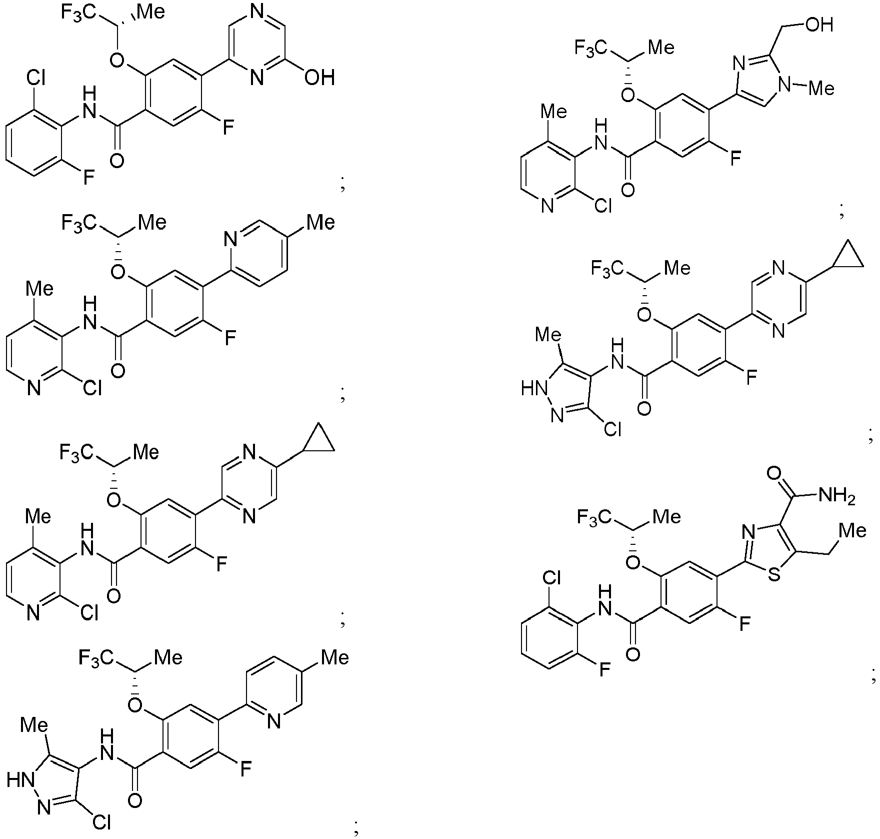 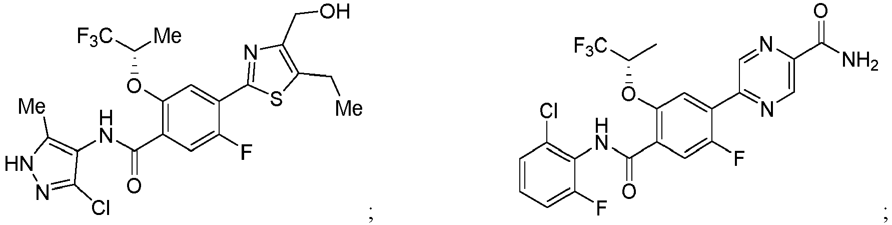 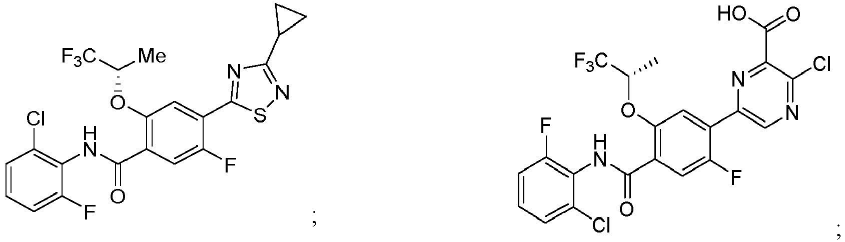 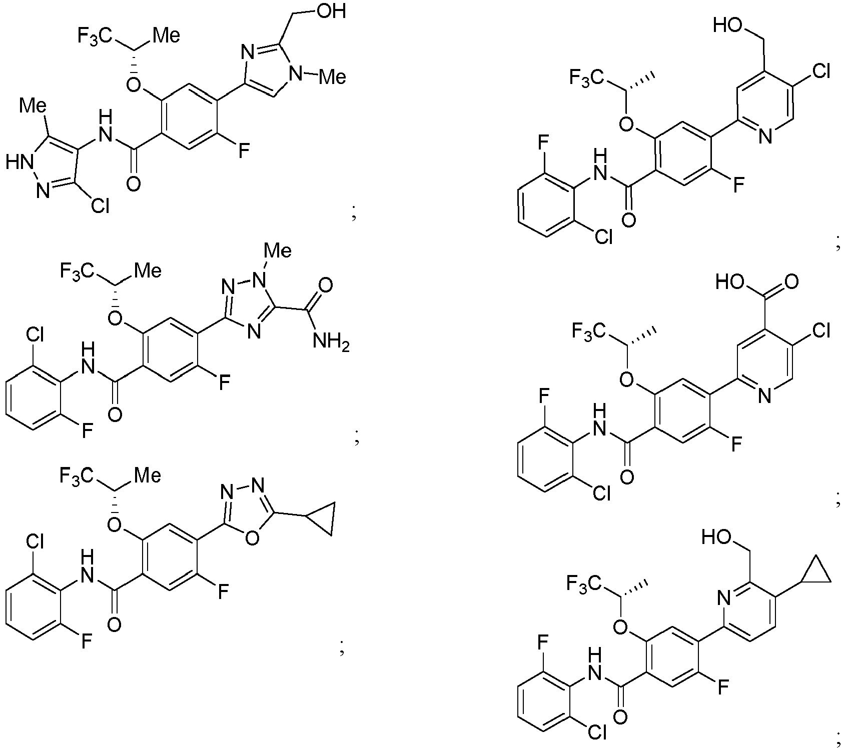 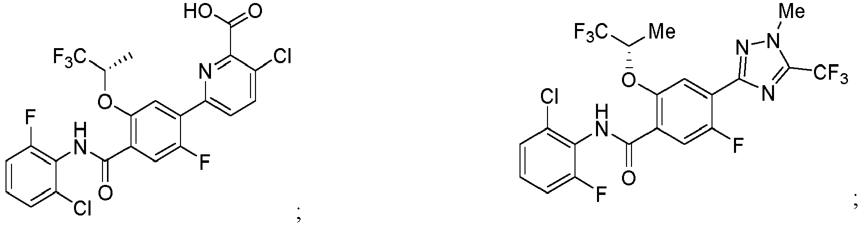 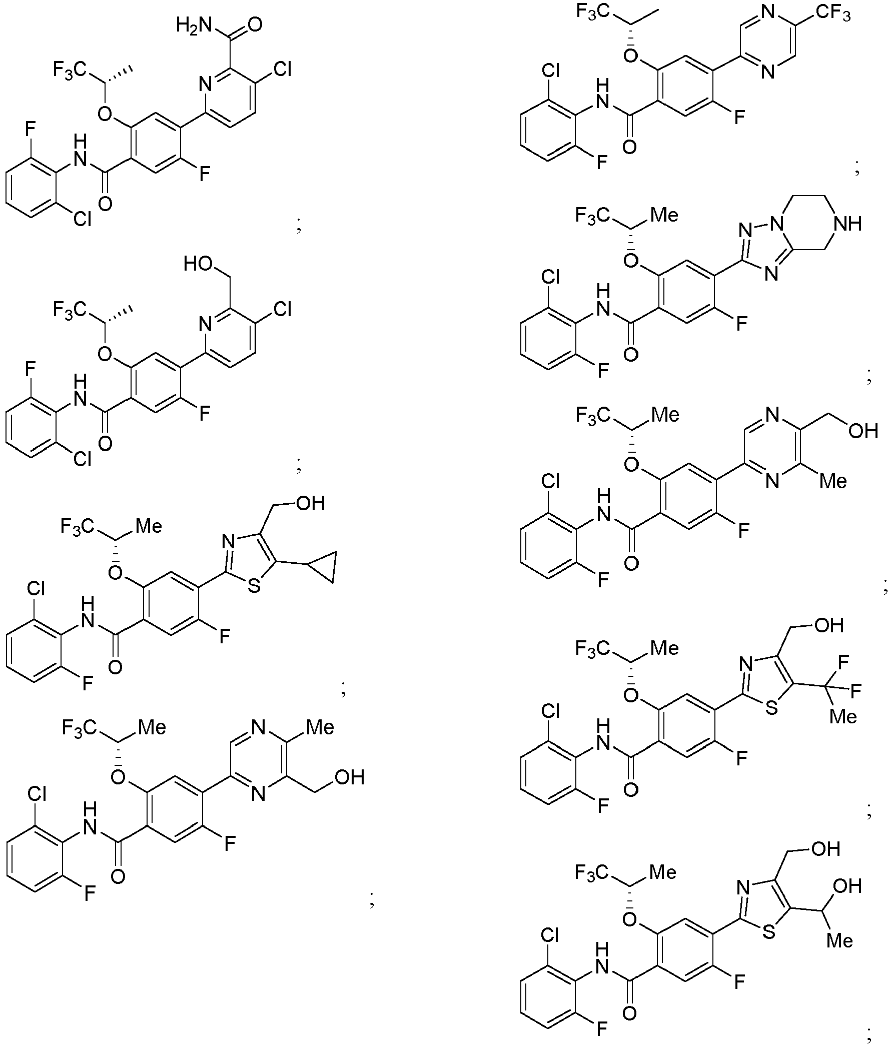 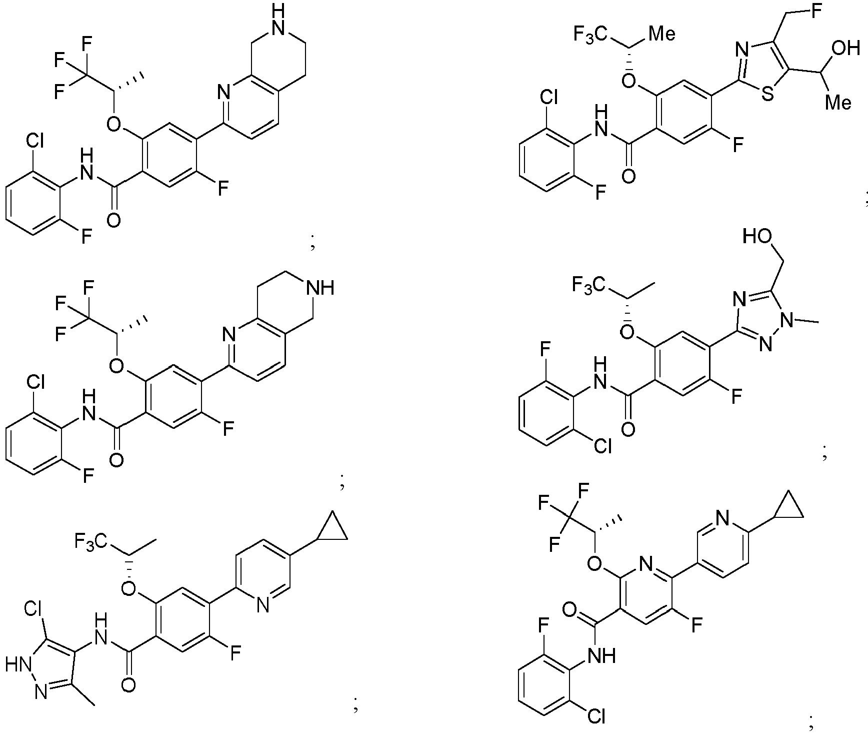 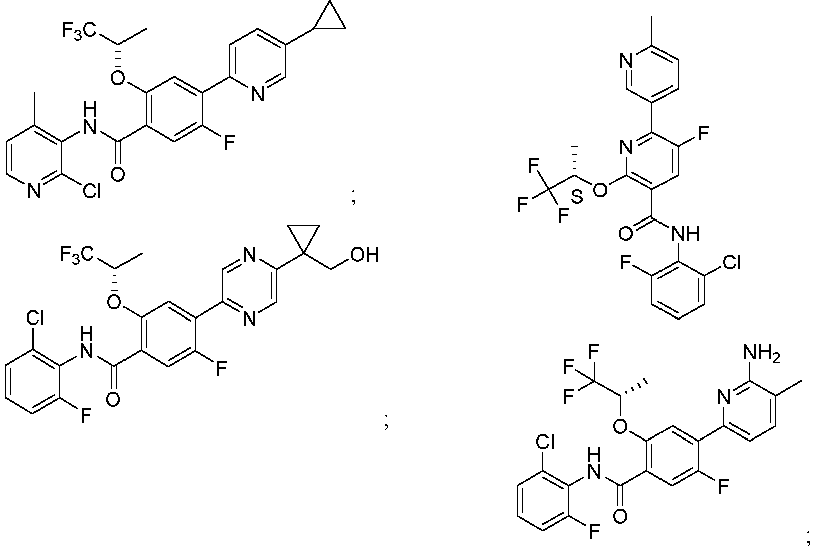 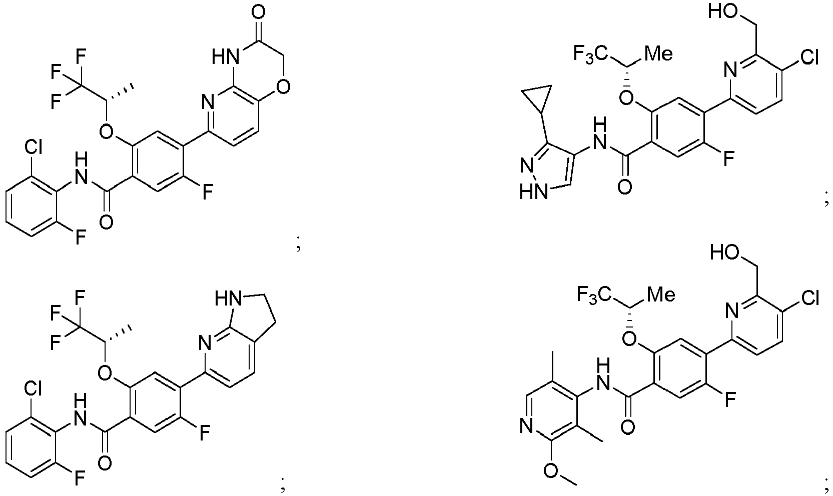 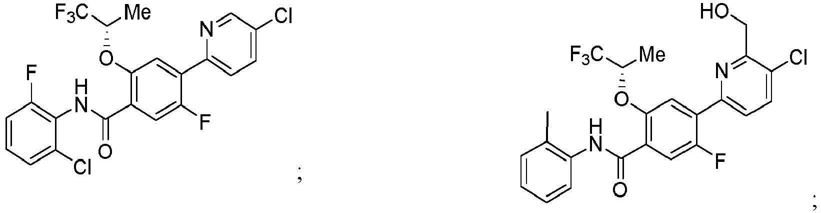 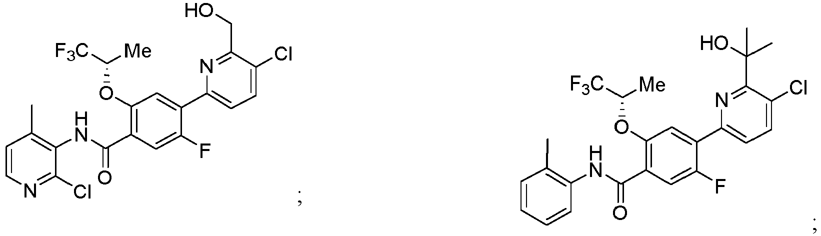 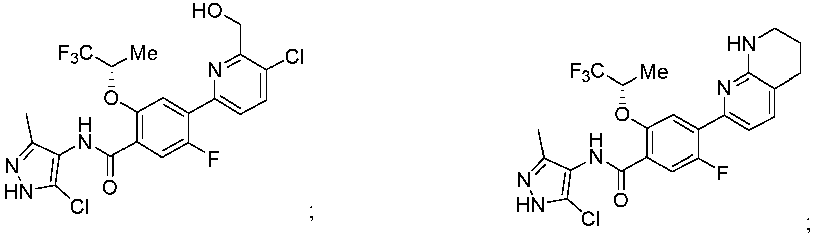 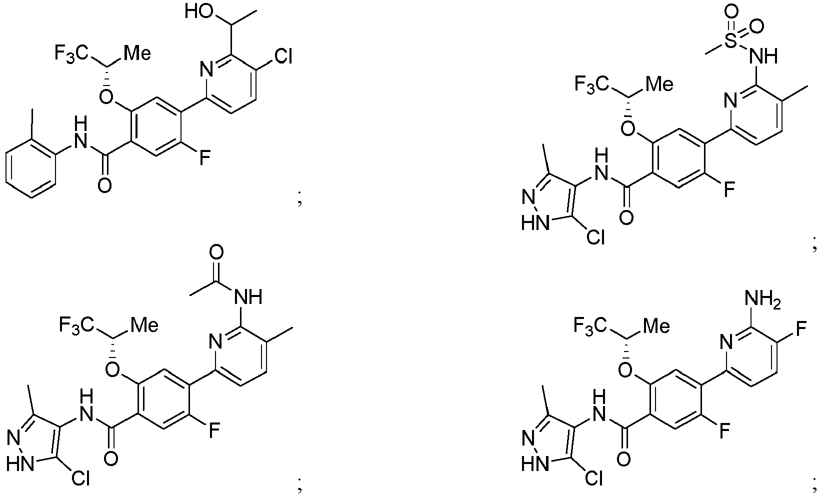 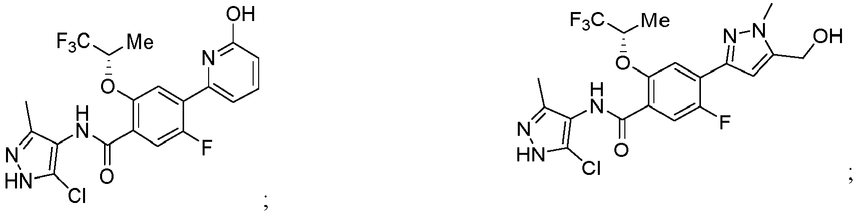 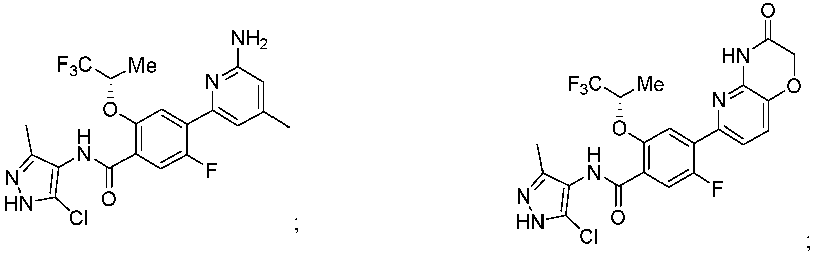 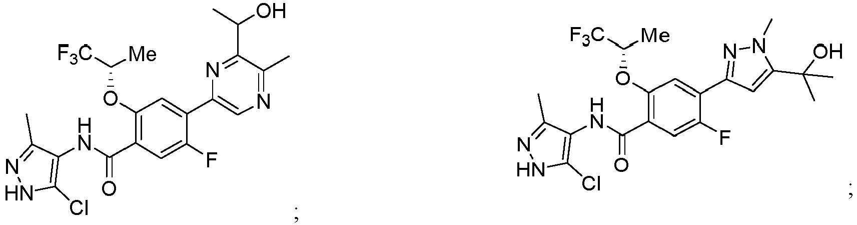 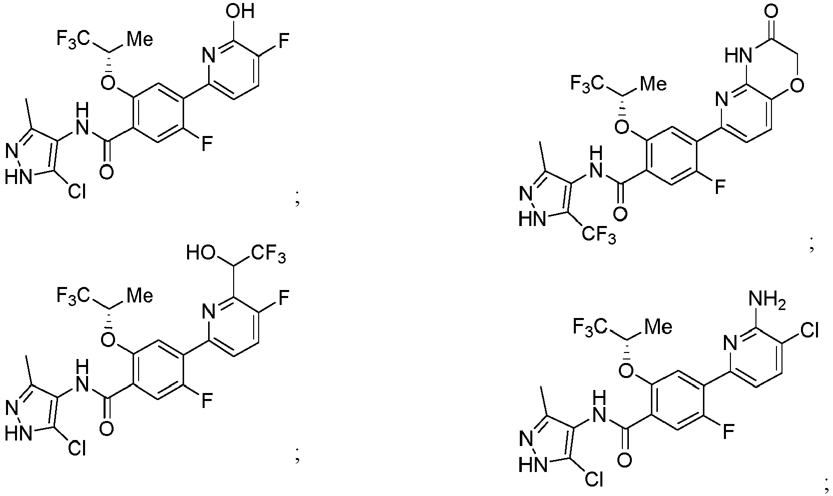 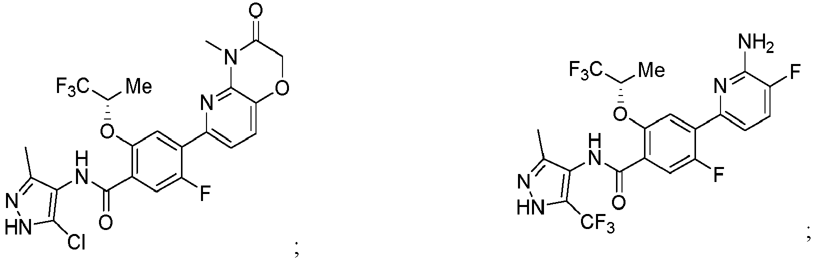 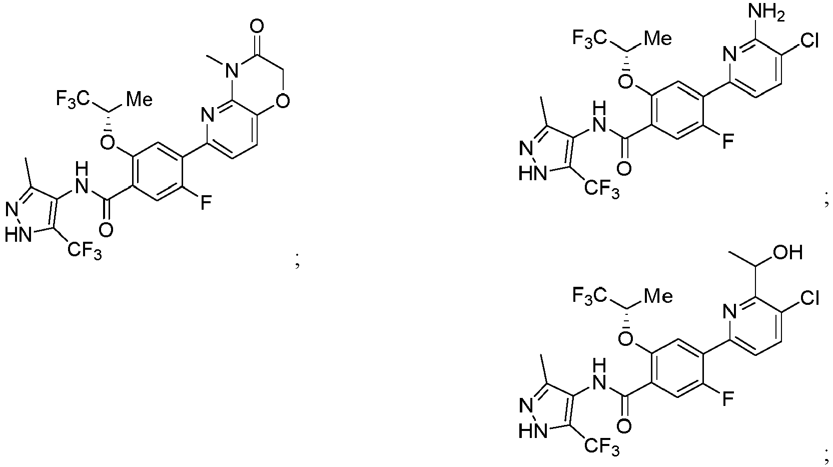 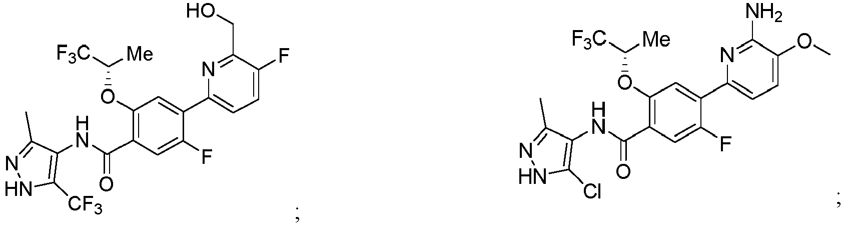 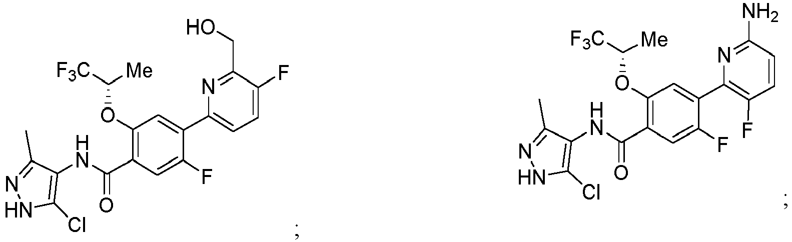 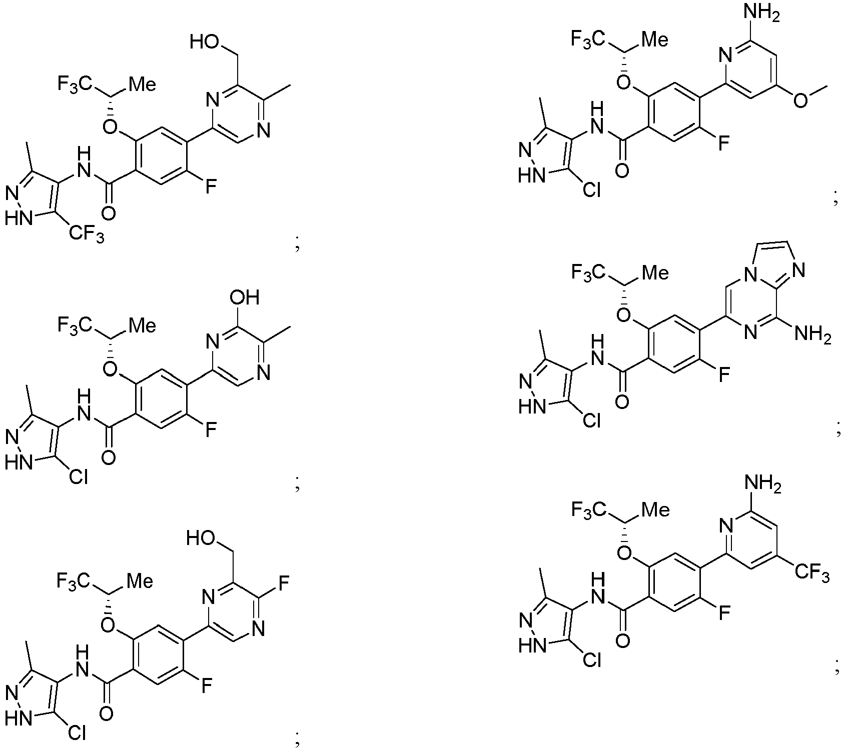 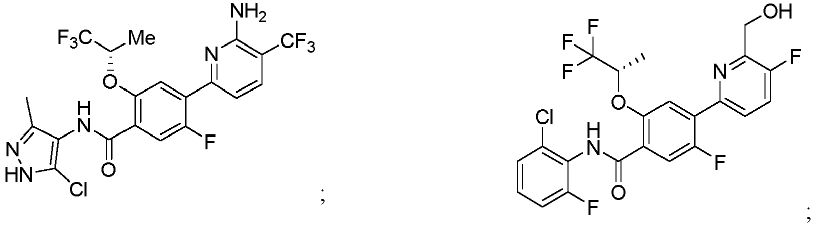 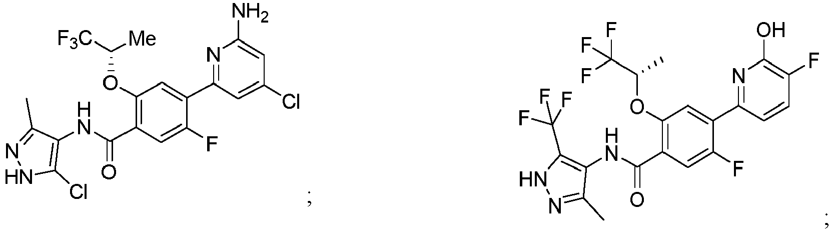 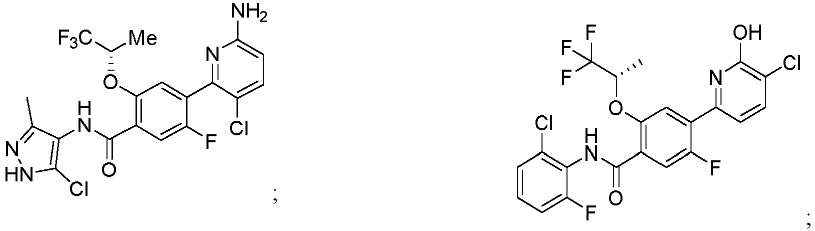 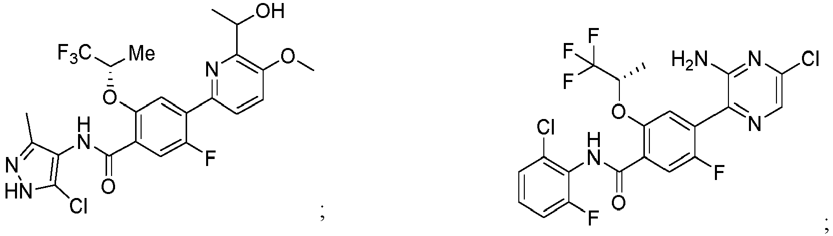 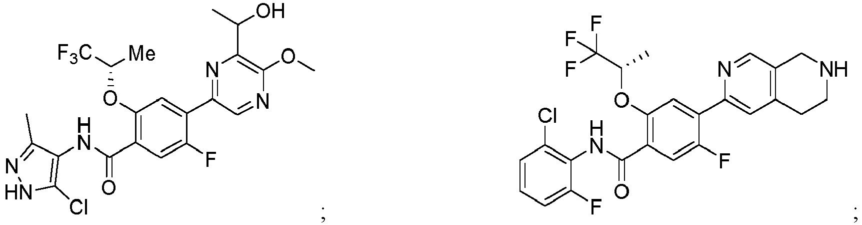 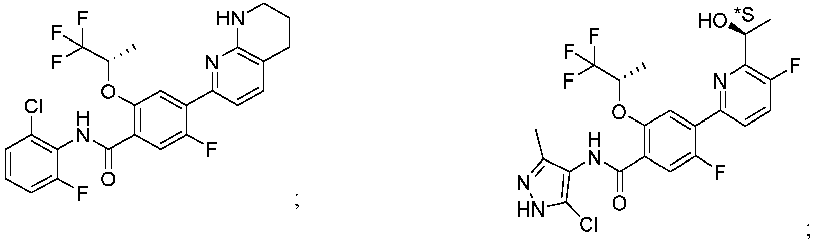 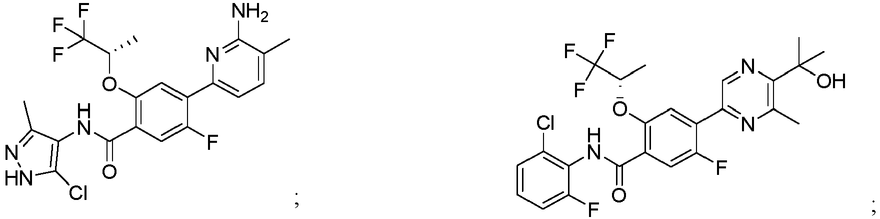 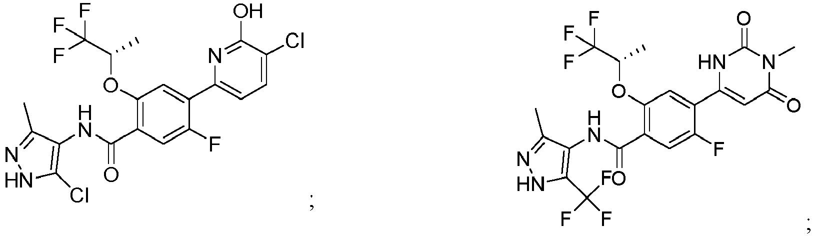 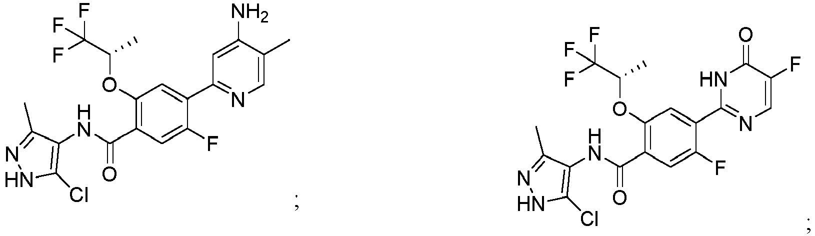 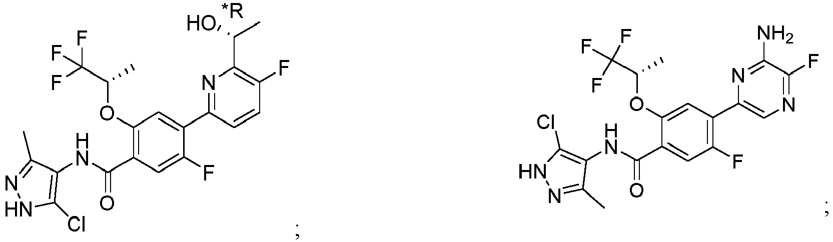 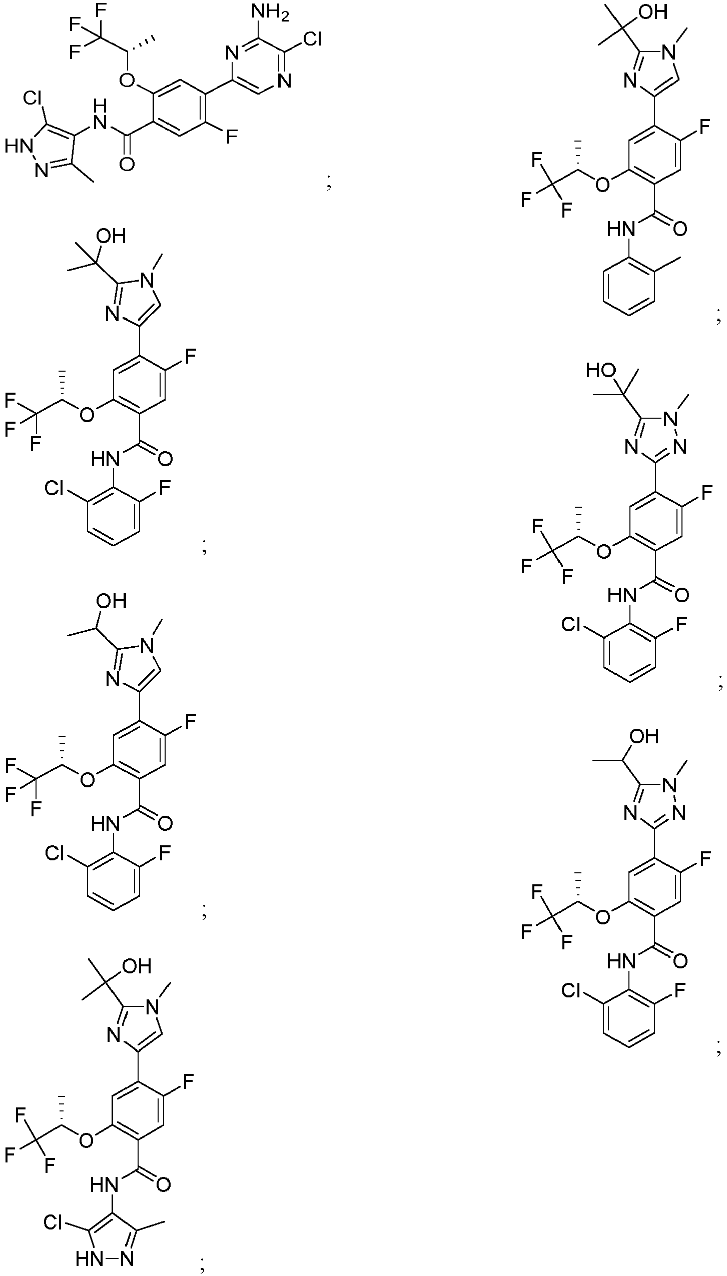 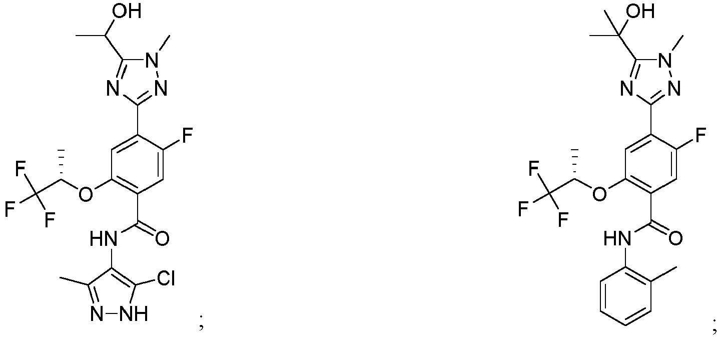 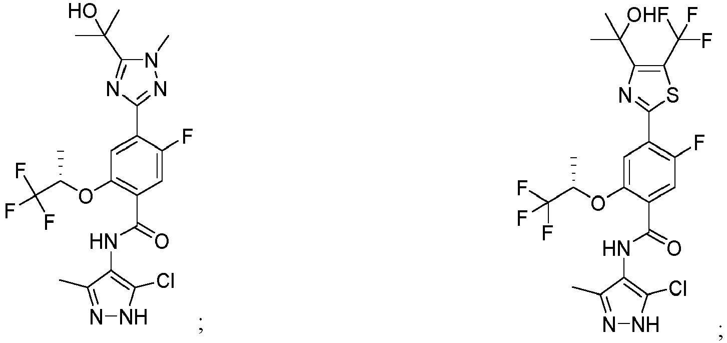 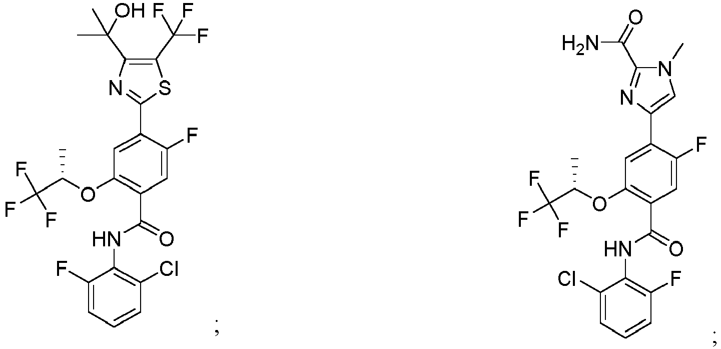 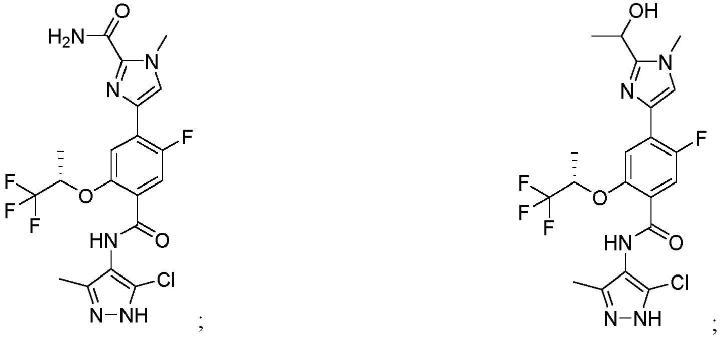 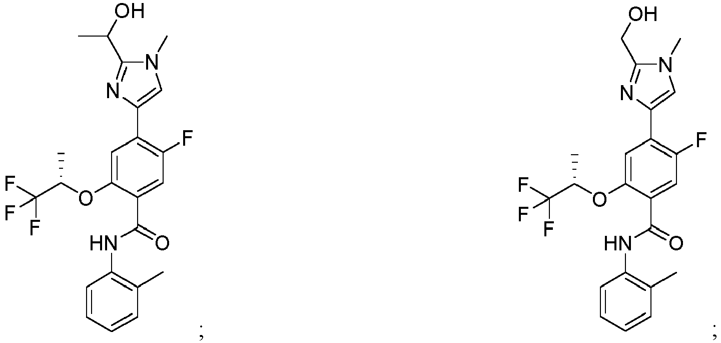 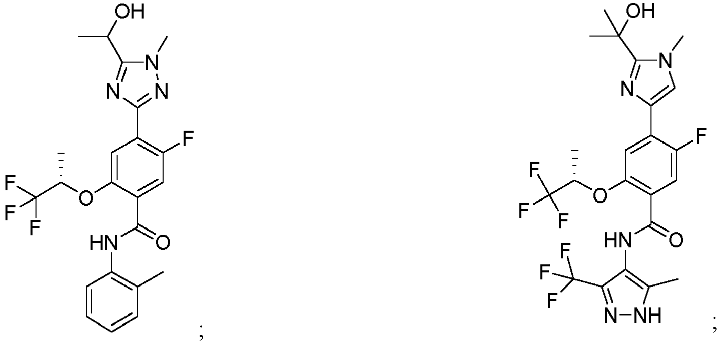 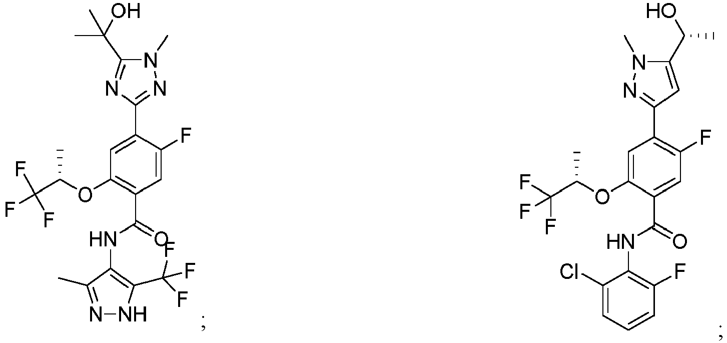 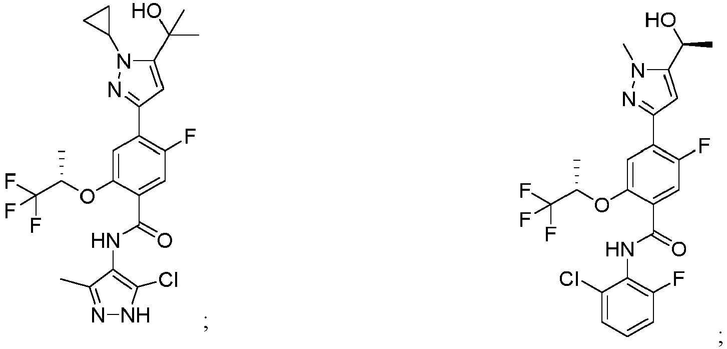 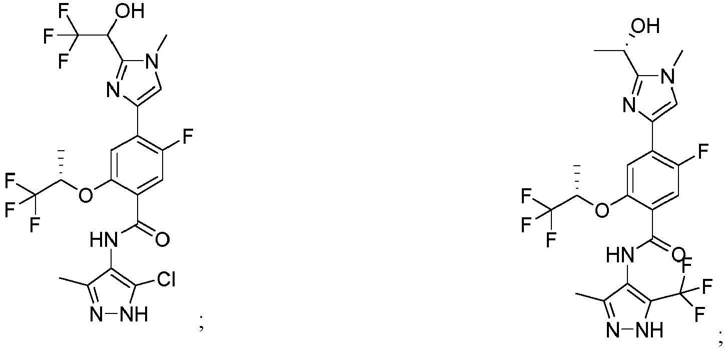 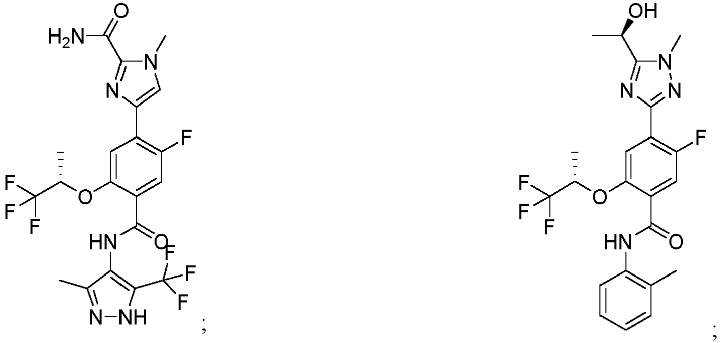 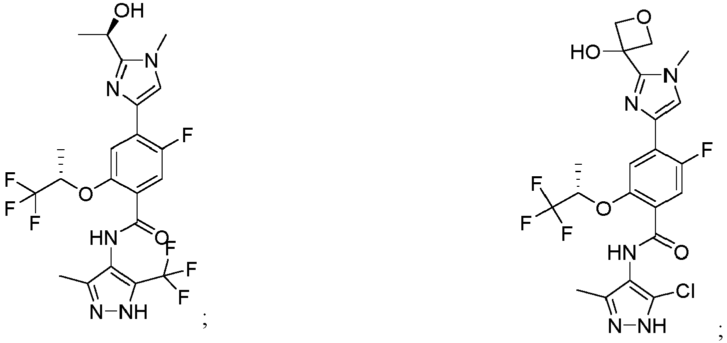 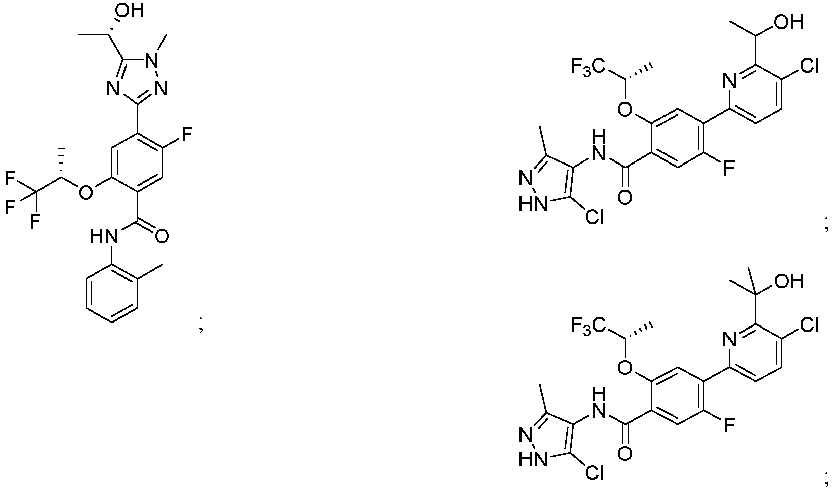 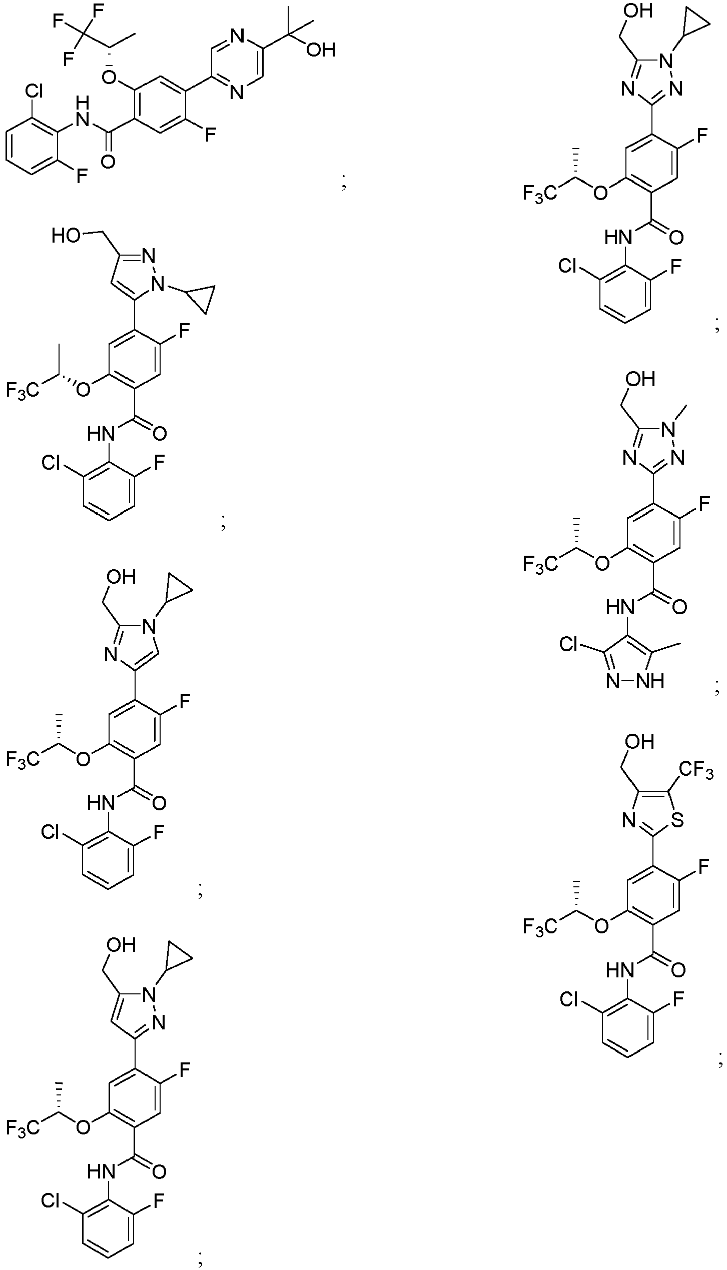 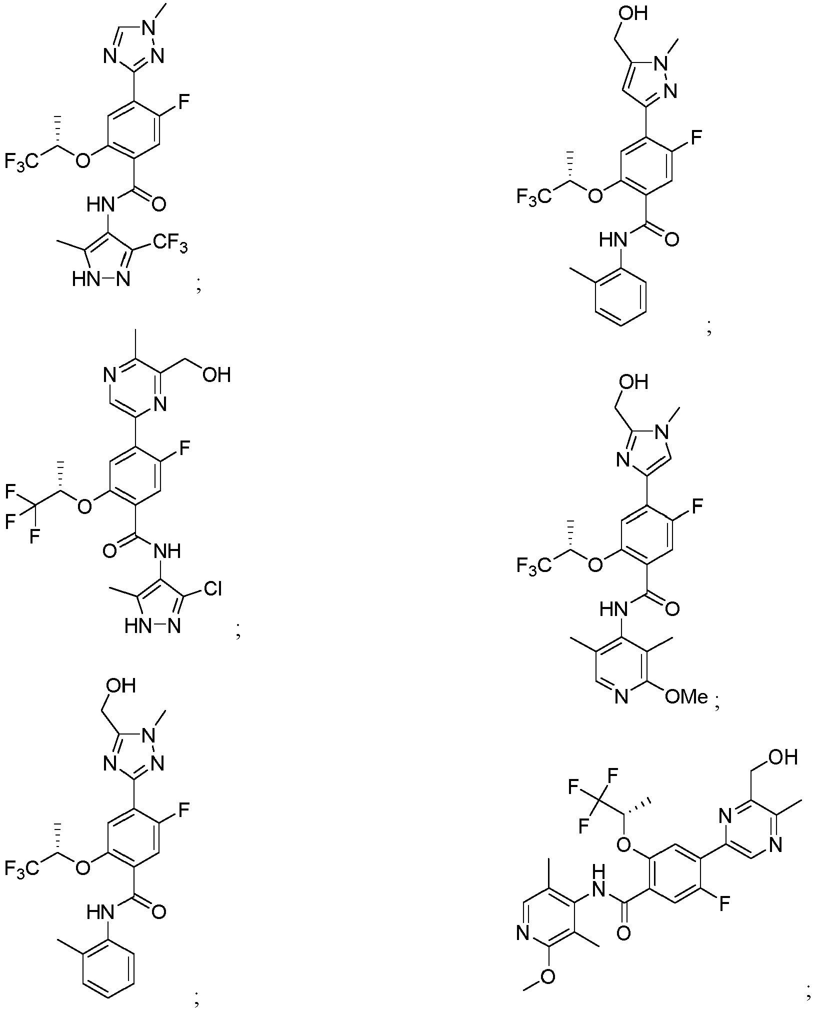 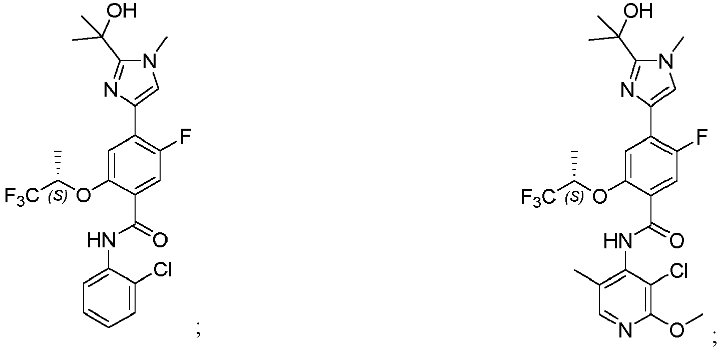 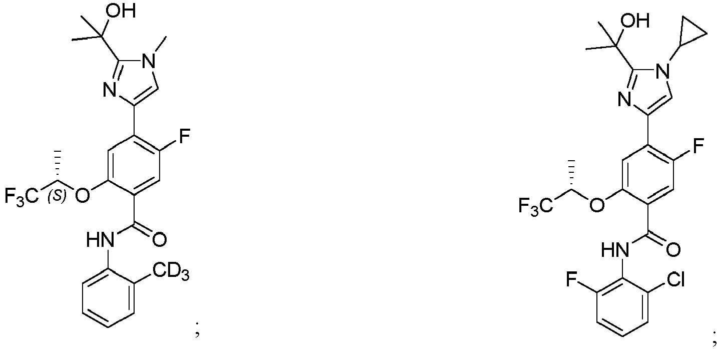 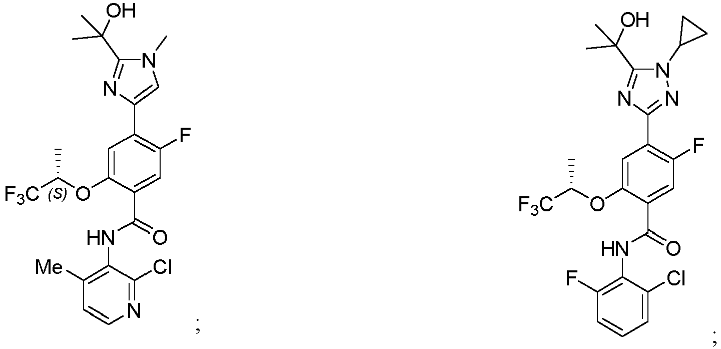 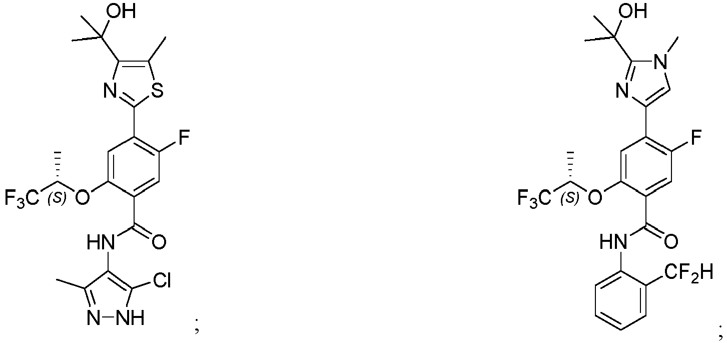 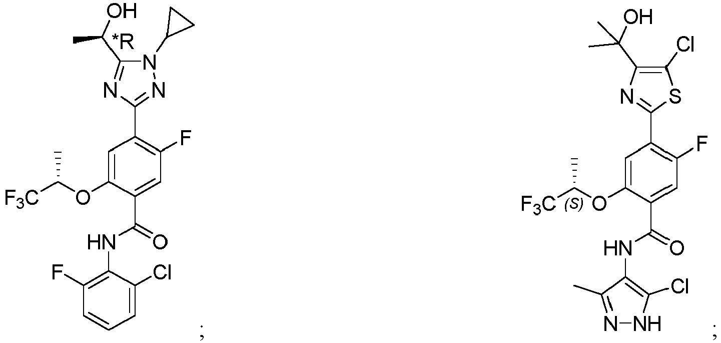 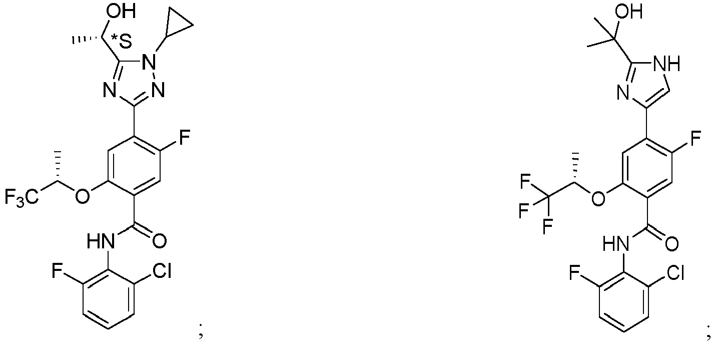 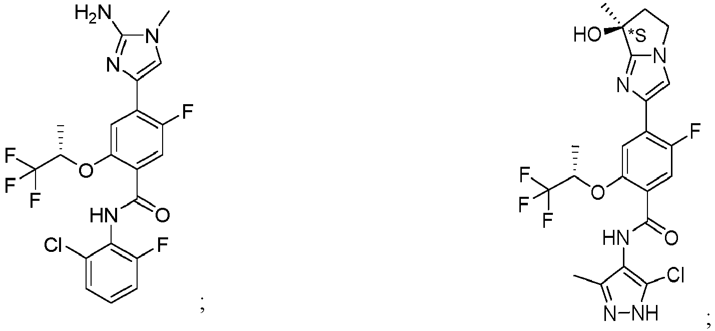 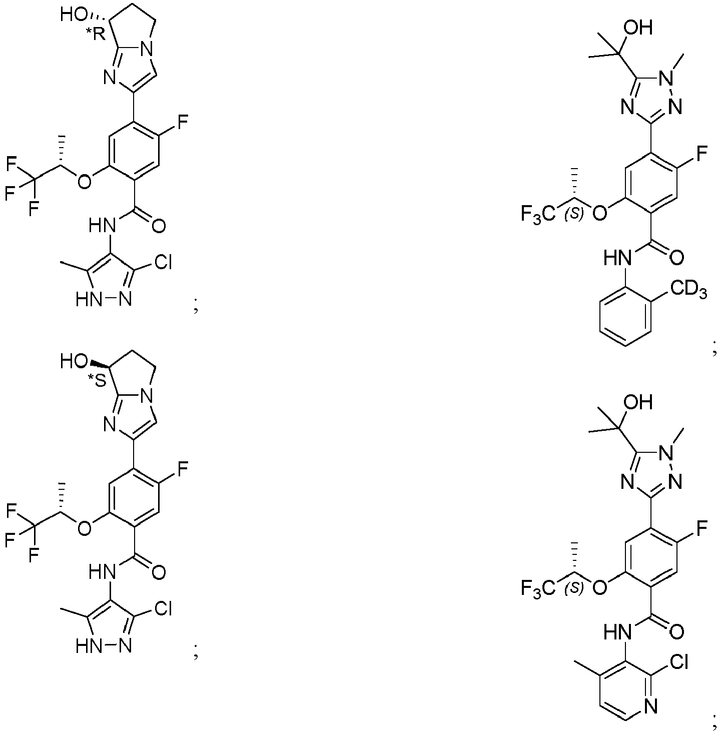 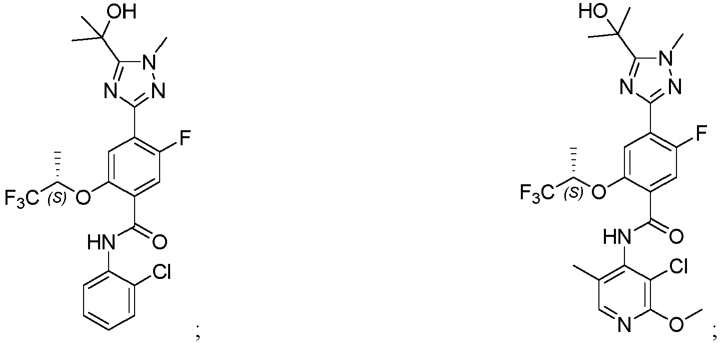 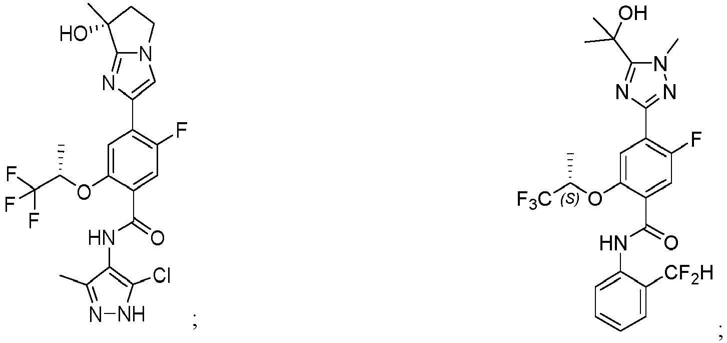 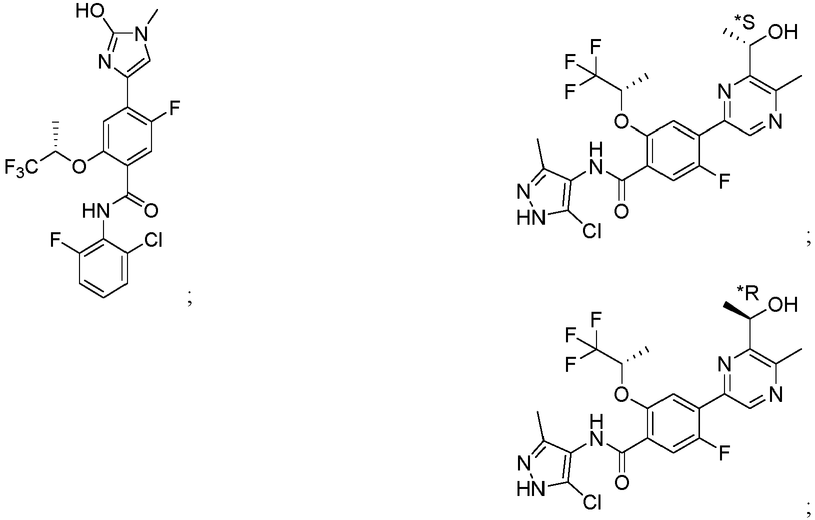 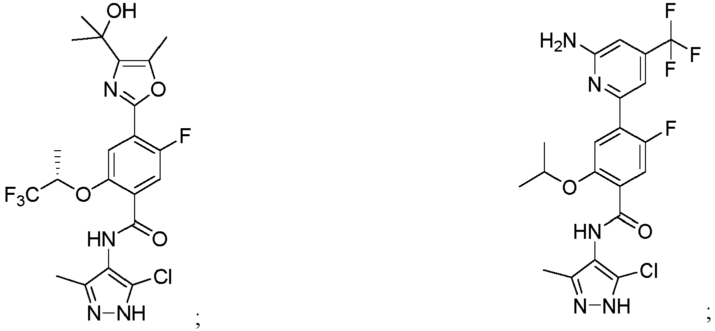 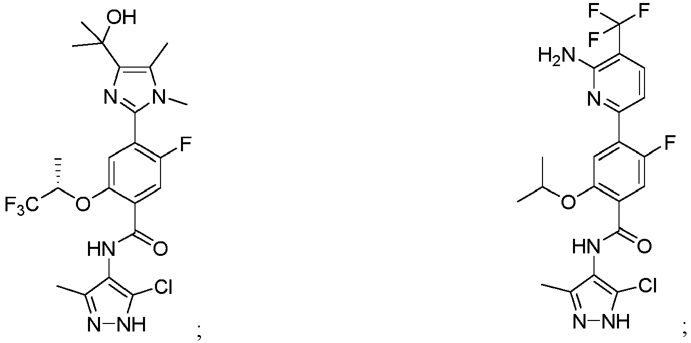 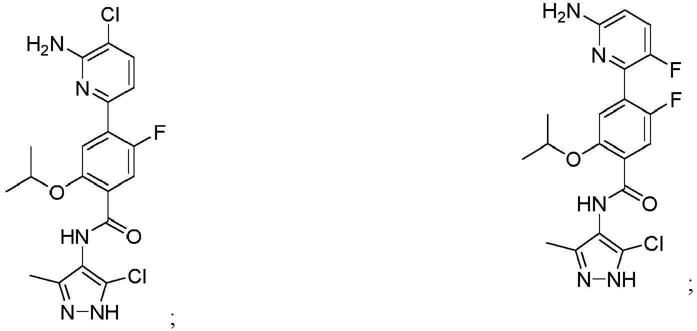 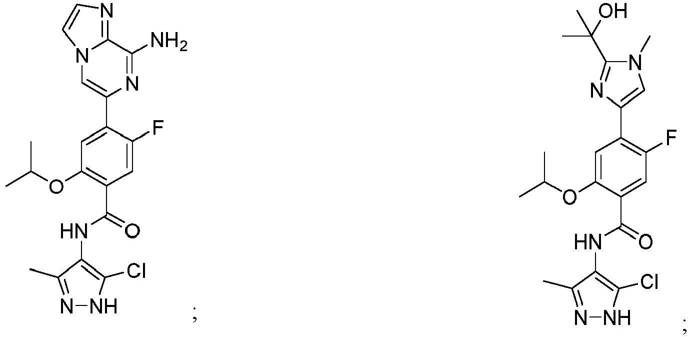 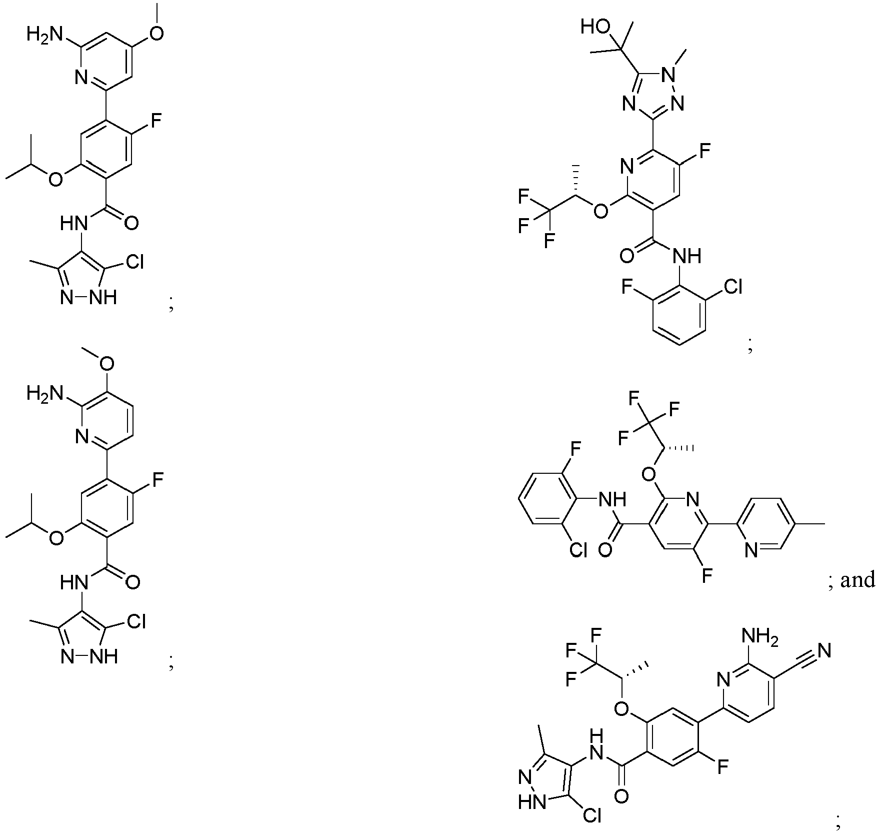 or a pharmaceutically acceptable salt, solvate, stereoisomer, isotopic variant, or N-oxide thereof; and at least one pharmaceutically acceptable excipient.

Embodiments of this invention include compounds of Formula I wherein:
Q is CH or N;
R¹ is 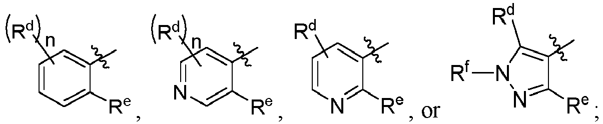 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is
   imidazolyl; wherein said imidazolyl is optionally substituted with up to three substituents independently selected from the group comprising 3-hydroxyoxetanyl, -NH₂, -OH, -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -NHSO₂CH₃, -NHC(O)CH₃, -OCH₃, - OH, -NH₂, -CO₂H, -Cl, -F, or R^{a};
   pyrazinonyl; wherein said pyrazinonyl is optionally substituted with one or two groups independently selected from the group comprising R^{a};
   thiazolyl; wherein said thiazolyl is optionally substituted with one or two substituents independently selected from the group comprising R^{a};
   5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
   5,6,7,8-tetrahydro-[1,2,4]triazolo[1,5-*a*]pyrazinyl; wherein said 5,6,7,8-tetrahydro-[1,2,4]triazolo[4,3-*a*]pyrazinyl is optionally substituted with R^{a};
   pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
   pyridazinyl; wherein said pyridazinyl is optionally substituted with one or two groups independently selected from the group comprising R^{a}, -Cl, or -CO₂H;
   4,5,6,7-tetrahydrothiazolo[5,4-*c*]pyridinyl; wherein said 4,5,6,7-tetrahydrothiazolo[5,4-*c*]pyridinyl is optionally substituted with R^{a};
   pyrazolyl; wherein said pyrazolyl is optionally substituted with one or two substituents independently selected from the group comprising R^{a};
   oxazolyl; wherein said oxazolyl is optionally substituted with one or two substituents independently selected from the group comprising R^{a};
   1,3,4-oxadiazolyl; wherein said 1,3,4-oxadiazolyl is optionally substituted with R^{a};
   5,6,7,8-tetrahydroimidazo[1,5-*a*]pyridinyl; wherein said 5,6,7,8-tetrahydroimidazo[1,5-*a*]pyridinyl is optionally substituted with R^{a};
   1,2,4-thiadiazolyl; wherein said 1,2,4-thiadiazolyl is optionally substituted with R^{a};
   5,6,7,8-tetrahydro-1,7-naphthyridinyl; wherein said 5,6,7,8-tetrahydro-1,7-naphthyridinyl is optionally substituted with R^{a};
   5,6,7,8-tetrahydro-1,6-naphthyridinyl; wherein said 5,6,7,8-tetrahydro-1,6-naphthyridinyl is optionally substituted with R^{a};
   5,6,7,8-tetrahydro-1,8-naphthyridinyl; wherein said 5,6,7,8-tetrahydro-1,8-naphthyridinyl is optionally substituted with R^{a};
   1,2,3,4-tetrahydro-2,7-naphthyridinyl; wherein said 1,2,3,4-tetrahydro-2,7-naphthyridinyl is optionally substituted with R^{a};
   2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with R^{a};
   2,3-dihydro-1*H*-pyrrolo[2,3*-b*]pyridinyl; wherein said 2,3-dihydro-1*H*-pyrrolo[2,3-*b*]pyridinyl is optionally substituted with R^{a};
   imidazo[1,2-*a*]pyrazinyl, wherein said imidazo[1,2-*a*]pyrazinyl is optionally substituted with -NH2 or R^{a};
   2,6-dioxo-1,2,3,6-tetrahydropyrimidinyl, wherein said 2,6-dioxo-1,2,3,6-tetrahydropyrimidinyl is optionally substituted with R^{a};
   6-oxo-1,6-dihydropyrimidinyl, wherein said 6-oxo-1,6-dihydropyrimidinyl is optionally substituted with fluorine or R^{a};
   6,7-dihydro-5*H*-pyrrolo[1,2-*a*]imidazolyl, wherein said 6,7-dihydro-5iH-pyrrolo[1,2-a]imidazolyl is optionally substituted with one or two substituents selected from the group consisting of R^{a} and -OH;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -OH, -CH₂OH, or up to two fluorine atoms;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said -C₍₁₋₆₎alkyl is optionally substituted with up to six fluorine atoms;;
or a pharmaceutically acceptable salt, solvate, stereoisomer, isotopic variant, or N-oxide thereof.

Embodiments of this invention include compounds of Formula II wherein:
Q is CH or N;
R¹ is 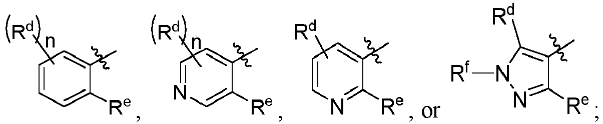 wherein
   R^{d} is selected from the group consisting of: H, Cl, F, and C₍₁₋₃₎alkyl; wherein said C₍₁₋₃₎alkyl is optionally substituted with OH, OCH₃, SCH₃, or OCF₃; or said C₍₁₋₃₎alkyl is optionally substituted with up to three fluorine atoms;
   R^{e} is selected from the group consisting of: Cl, F, -CD₃, C₍₁₋₃₎alkyl, and cyclopropyl; wherein said C₍₁₋₃₎alkyl is optionally substituted with OH, OCH₃, SCH₃, or OCF₃; or said C₍₁₋₃₎alkyl is optionally substituted with up to three fluorine atoms;
   R^{f} is H; and
   n is 1, or 2;
R² is
   imidazolyl; wherein said imidazolyl is optionally substituted with up to three substituents independently selected from the group comprising 3-hydroxyoxetanyl, -NH₂, -OH, -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -NHSO₂CH₃, -NHC(O)CH₃, -OCH₃, - OH, -NH₂, -CO₂H, -Cl, -F, or R^{a};
   pyrazinonyl; wherein said pyrazinonyl is optionally substituted with one or two groups independently selected from the group comprising R^{a};
   thiazolyl; wherein said thiazolyl is optionally substituted with one or two substituents independently selected from the group comprising R^{a};
   5,6,7,8-tetrahydro-[1,2,4]triazolo[4,3-*a*]pyrazinyl;
   5,6,7,8-tetrahydro-[1,2,4]triazolo[1,5-*a*]pyrazinyl;
   pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
   pyridazinyl; wherein said pyridazinyl is optionally substituted with one or two groups independently selected from the group comprising R^{a};
   4,5,6,7-tetrahydrothiazolo[5,4-ic]pyridinyl;
   pyrazolyl; wherein said pyrazolyl is optionally substituted with one or two substituents independently selected from the group comprising R^{a};
   oxazolyl; wherein said oxazolyl is optionally substituted with one or two substituents independently selected from the group comprising R^{a};
   1,3,4-oxadiazolyl; wherein said 1,3,4-oxadiazolyl is substituted with cyclopropyl;
   5,6,7,8-tetrahydroimidazo[1,5-*a*]pyridinyl; wherein said 5,6,7,8-tetrahydroimidazo[1,5-*a*]pyridinyl is optionally substituted with R^{a};
   1,2,4-thiadiazolyl; wherein said 1,2,4-thiadiazolyl is substituted with cyclopropyl;
   5,6,7,8-tetrahydro-1,7-naphthyridinyl;
   5,6,7,8-tetrahydro-1,6-naphthyridinyl;
   5, 6, 7, 8-tetrahydro-1, 8-naphthyridinyl;
   1,2,3,4-tetrahydro-2,7-naphthyridinyl;
   2*H*-pyrido[3,2-*b*][1,4]oxazin-3(4*H*)-onyl, wherein said 2*H*-pyrido[3,2-ib][1,4]oxazin-3(4*H*)-onyl is optionally substituted with R^{a};
   2,3-dihydro-1*H*-pyrrolo[2,3-*b*]pyridinyl;
   imidazo[1,2-ia]pyrazinyl, wherein said imidazo[1,2-ia]pyrazinyl is substituted with -NH₂;
   2,6-dioxo-1,2,3,6-tetrahydropyrimidinyl, wherein said 2,6-dioxo-1,2,3,6-tetrahydropyrimidinyl is optionally substituted with R^{a};
   6-oxo-1,6-dihydropyrimidinyl, wherein said 6-oxo-1,6-dihydropyrimidinyl is substituted with fluorine;
   6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of R^{a} and -OH;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms;; and wherein said cyclopropyl is optionally substituted with -CH₂OH;
or a pharmaceutically acceptable salt, solvate, stereoisomer, isotopic variant, or N-oxide thereof.

Embodiments of this invention include compounds of Formula I or Formula II wherein:
Q is CH or N;
R¹ is 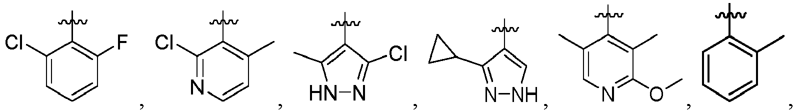 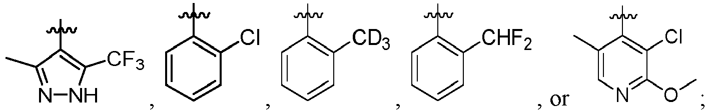
R² is
   imidazolyl; wherein said imidazolyl is optionally substituted with up to three substituents independently selected from the group comprising 3-hydroxyoxetanyl, -NH₂, -OH, -CN, or R^{a};
   triazolyl; wherein said triazolyl is optionally substituted with one or two groups independently selected from the group comprising R^{a};
   pyrazinyl; wherein said pyrazinyl is optionally substituted with up to three substituents independently selected from the group comprising -OCH₃, -OH, -NH₂, -CO₂H, -Cl, -F, or R^{a};
   pyrazinonyl; wherein said pyrazinonyl is optionally substituted with one or two groups independently selected from the group comprising R^{a};
   thiazolyl; wherein said thiazolyl is optionally substituted with two substituents independently selected from the group comprising R^{a};
   5,6,7,8-tetrahydro-[1,2,4]triazolo[4,3-*a*]pyrazinyl;
   5,6,7,8-tetrahydro-[1,2,4]triazolo[1,5-*a*]pyrazinyl;
   pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
   pyridazinyl; wherein said pyridazinyl is optionally substituted with R^{a};
   4,5,6,7-tetrahydrothiazolo[5,4-*c*]pyridinyl;
   pyrazolyl; wherein said pyrazolyl is optionally substituted with one or two substituents independently selected from the group comprising R^{a};
   oxazolyl; wherein said oxazolyl is optionally substituted with two substituents independently selected from R^{a};
   1,3,4-oxadiazolyl; wherein said 1,3,4-oxadiazolyl is substituted with cyclopropyl;
   5,6,7,8-tetrahydroimidazo[1,5-*a*]pyridinyl wherein said 5,6,7,8-tetrahydroimidazo[1,5-*a*]pyridinyl is optionally substituted with R^{a};
   1,2,4-thiadiazolyl; wherein said 1,2,4-thiadiazolyl is substituted with cyclopropyl;
   5,6,7,8-tetrahydro-1,7-naphthyridinyl;
   5,6,7,8-tetrahydro-1,6-naphthyridinyl;
   5, 6, 7, 8-tetrahydro-1, 8-naphthyridinyl;
   1,2,3,4-tetrahydro-2,7-naphthyridinyl;
   2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with -CH₃;
   2,3-dihydro-1*H*-pyrrolo[2,3-*b*]pyridinyl;
   imidazo[1,2-*a*]pyrazinyl, wherein said imidazo[1,2-*a*]pyrazinyl is substituted with -NH₂;
   2,6-dioxo-1,2,3,6-tetrahydropyrimidinyl, wherein said 2,6-dioxo-1,2,3,6-tetrahydropyrimidinyl is substituted with -CH₃;
   6-oxo-1,6-dihydropyrimidinyl, wherein said 6-oxo-1,6-dihydropyrimidinyl is substituted with fluorine;
   6,7-dihydro-5iH-pyrrolo[1,2-*a*]imidazolyl, wherein said 6,7-dihydro-5*H*-pyrrolo[1,2-*a*]imidazolyl is optionally substituted with one or two substituents selected from the group consisting of -CH₃ and -OH;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
or a pharmaceutically acceptable salt, solvate, stereoisomer, isotopic variant, or N-oxide thereof.

Embodiments of this invention include compounds of Formula I or Formula II wherein:
Q is CH or N;
R¹ is 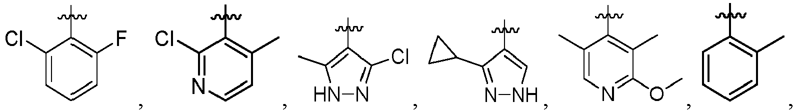 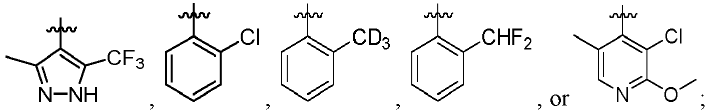
R² is
   imidazolyl; wherein said imidazolyl is substituted with one, two, or three substituents independently selected from the group comprising -C₍₁₋₃₎alkyl, 3-hydroxyoxetanyl, cyclopropyl, -C(O)NH₂, -NH₂, -OH, or -CN; wherein said -C₍₁₋₃₎alkyl is optionally substituted with -OH and additionally optionally substituted with up to three fluorine atoms;
   triazolyl; wherein said triazolyl is substituted with up to two groups independently selected from the group comprising -C(O)NH₂, -CF₃, cyclopropyl, or -C₍₁₋₃₎alkyl, wherein said -C₍₁₋₃₎alkyl is optionally substituted with -OH;
   pyrazinyl; wherein said pyrazinyl is optionally substituted with up to three substituents independently selected from the group comprising -C₍₁₋₃₎alkyl, -OCH₃, -OH, -NH₂,
   cyclopropyl. -C(O)NH₂, -CO₂H, -Cl, -F, or -CF₃; wherein said cyclopropyl is optionally substituted with -CH₂OH; and wherein said -C₍₁₋₃₎alkyl is optionally substituted with - OH;
   pyrazinonyl; wherein said pyrazinonyl is optionally substituted with one or two -C₍₁₋₃₎alkyl groups;
   thiazolyl; wherein said thiazolyl is substituted with two substituents independently selected from the group comprising -C₍₁₋₃₎alkyl, cyclopropyl, -Cl, or -C(O)NH₂; wherein said -C₍₁₋₃₎alkyl, is optionally substituted with -OH, or up to three fluorine atoms;
   5,6,7,8-tetrahydro-[1,2,4]triazolo[4,3-*a*]pyrazinyl;
   5,6,7,8-tetrahydro-[1,2,4]triazolo[1,5-*a*]pyrazinyl;
   pyridinyl; wherein said pyridinyl is substituted with one or two substituents independently selected from the group comprising -CONH₂, -C₍₁₋₃₎alkyl, cyclopropyl, - Cl, -F, -CN, -NHC(O)CH₃, -NHSO₂CH₃, -CO₂H, -OCH₃, -OH, or -NH₂; wherein said - C₍₁₋₃₎alkyl is optionally substituted with up to three fluorine atoms, or the -C₍₁₋₃₎alkyl is optionally substituted with -OH and additionally optionally substituted with up to three fluorine atoms;
   pyridazinyl; wherein said pyridazinyl is substituted with -C₍₁₋₃₎alkyl; wherein said -C₍₁₋₃₎alkyl is optionally substituted with -OH;
   4,5,6,7-tetrahydrothiazolo[5,4-*c*]pyridinyl;
   pyrazolyl; wherein said pyrazolyl is substituted with two substituents independently selected from the group comprising -C₍₁₋₃₎alkyl, and cyclopropyl; wherein said -C₍₁₋₃₎alkyl is optionally substituted with -OH;
   oxazolyl; wherein said oxazolyl is optionally substituted with two substituents independently selected from -C₍₁₋₃₎alkyl; wherein said -C₍₁₋₃₎alkyl is optionally substituted with -OH;
   1,3,4-oxadiazolyl; wherein said 1,3,4-oxadiazolyl is substituted with cyclopropyl; 5,6,7,8-tetrahydroimidazo[1,5-*a*]pyridinyl wherein said 5,6,7,8-tetrahydroimidazo[1,5-*a*]pyridinyl is substituted with -C₍₁₋₃₎alkyl; wherein said -C₍₁₋₃₎alkyl is optionally substituted with -OH;
   1,2,4-thiadiazolyl; wherein said 1,2,4-thiadiazolyl is substituted with cyclopropyl;
   5,6,7,8-tetrahydro-1,7-naphthyridinyl;
   5,6,7,8-tetrahydro-1,6-naphthyridinyl;
   5, 6, 7, 8-tetrahydro-1, 8-naphthyridinyl;
   1,2,3,4-tetrahydro-2,7-naphthyridinyl;
   2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with -CH₃;
   2,3-dihydro-1*H*-pyrrolo[2,3-*b*]pyridinyl;
   imidazo[1,2-*a*]pyrazinyl, wherein said imidazo[1,2-*a*]pyrazinyl is substituted with -NH₂;
   2,6-dioxo-1,2,3,6-tetrahydropyrimidinyl, wherein said 2,6-dioxo-1,2,3,6-tetrahydropyrimidinyl is substituted with -CH₃;
   6-oxo-1,6-dihydropyrimidinyl, wherein said 6-oxo-1,6-dihydropyrimidinyl is substituted with fluorine;
   6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of -CH₃ and -OH;
or a pharmaceutically acceptable salt, solvate, stereoisomer, isotopic variant, or N-oxide thereof.

Embodiments of this invention include compounds of Formula I or Formula II wherein:
Q is CH or N;
R¹ is 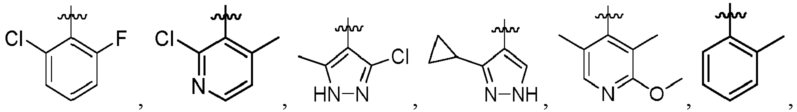 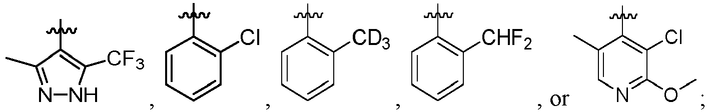
R² is
   imidazolyl; wherein said imidazolyl is substituted with one, two, or three substituents independently selected from the group comprising -C₍₁₋₃₎alkyl, 3-hydoxyoxetanyl, cyclopropyl, -C(O)NH₂, -NH₂, -OH, or -CN; wherein said -C₍₁₋₃₎alkyl is optionally substituted with -OH and additionally optionally substituted with up to three fluorine atoms;
   triazolyl; wherein said triazolyl is substituted with up to two groups independently selected from the group comprising -C(O)NH₂, -CF₃, cyclopropyl, or -C₍₁₋₃₎alkyl, wherein said -C₍₁₋₃₎alkyl is optionally substituted with -OH;
   pyrazinyl; wherein said pyrazinyl is optionally substituted with up to three substituents independently selected from the group comprising -C₍₁₋₃₎alkyl, -OCH₃, -OH, -NH₂, cyclopropyl. -C(O)NH₂, -CO₂H, -Cl, -F, or -CF₃, wherein said cyclopropyl is optionally substituted with -CH₂OH, and wherein said -C₍₁₋₃₎alkyl is optionally substituted with - OH;
   pyrazinonyl; wherein said pyrazinonyl is substituted with -CH₃ and -CH₂CH₂CH₃;
   thiazolyl; wherein said thiazolyl is substituted with two substituents independently selected from the group comprising -C₍₁₋₃₎alkyl, cyclopropyl, -Cl, or -C(O)NH₂; wherein said -C₍₁₋₃₎alkyl is optionally substituted with -OH, or up to three fluorine atoms;
   5,6,7,8-tetrahydro-[1,2,4]triazolo[4,3-*a*]pyrazinyl;
   5,6,7,8-tetrahydro-[1,2,4]triazolo[1,5-*a*]pyrazinyl;
   pyridinyl; wherein said pyridinyl is substituted with one or two substituents independently selected from the group comprising -CONH₂, -CF₃, -C₍₁₋₃₎alkyl, cyclopropyl, -Cl, -F, -CN, -NHC(O)CH₃, -NHSO₂CH₃, -CO₂H, -OCH₃, -OH, or -NH₂, wherein said -C₍₁₋₃₎alkyl is optionally substituted with -OH and additionally optionally substituted with up to three fluorine atoms;
   pyridazinyl; wherein said pyridazinyl is substituted with -CH₃;
   4,5,6,7-tetrahydrothiazolo[5,4-*c*]pyridinyl;
   pyrazolyl; wherein said pyrazolyl is substituted with two substituents independently selected from the group comprising -C₍₁₋₃₎alkyl, and cyclopropyl, wherein said -C₍₁₋₃₎alkyl is optionally substituted with -OH;
   oxazolyl; wherein said oxazolyl is substituted with with two substituents independently selected from -C₍₁₋₃₎alkyl; wherein said -C₍₁₋₃₎alkyl is optionally substituted with -OH;
   1,3,4-oxadiazolyl; wherein said 1,3,4-oxadiazolyl is substituted with cyclopropyl;
   5,6,7,8-tetrahydroimidazo[1,5-*a*]pyridinyl; wherein said 5,6,7,8-tetrahydroimidazo[1,5-*a*]pyridinyl is substituted with -CH₂OH;
   1,2,4-thiadiazolyl; wherein said 1,2,4-thiadiazolyl is substituted with cyclopropyl;
   5,6,7,8-tetrahydro-1,7-naphthyridinyl;
   5,6,7,8-tetrahydro-1,6-naphthyridinyl;
   5, 6, 7, 8-tetrahydro-1, 8-naphthyridinyl;
   1,2,3,4-tetrahydro-2,7-naphthyridinyl;
   2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with -CH₃;
   2,3-dihydro-1*H*-pyrrolo[2,3-*b*]pyridinyl;
   imidazo[1,2-*a*]pyrazinyl, wherein said imidazo[1,2-*a*]pyrazinyl is substituted with -NH₂;
   2,6-dioxo-1,2,3,6-tetrahydropyrimidinyl, wherein said 2,6-dioxo-1,2,3,6-tetrahydropyrimidinyl is substituted with -CH₃;
   6-oxo-1,6-dihydropyrimidinyl, wherein said 6-oxo-1,6-dihydropyrimidinyl is substituted with fluorine;
   6,7-dihydro-5*H*-pyrrolo[1,2-*a*]imidazolyl, wherein said 6,7-dihydro-5H-pyrrolo[1,2-*a*]imidazolyl is optionally substituted with one or two substituents selected from the group consisting of -CH₃ and -OH;
or a pharmaceutically acceptable salt, solvate, stereoisomer, isotopic variant, or N-oxide thereof.

Embodiments of this invention include compounds of Formula I or Formula II selected from the group consisting of: 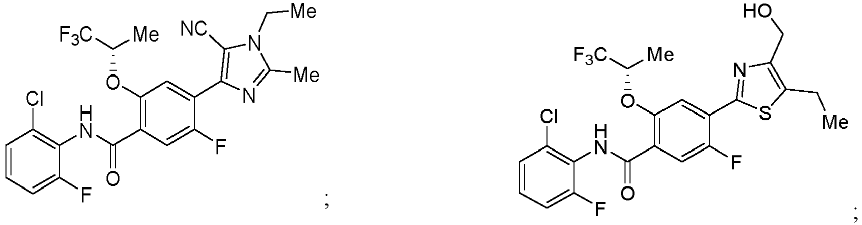 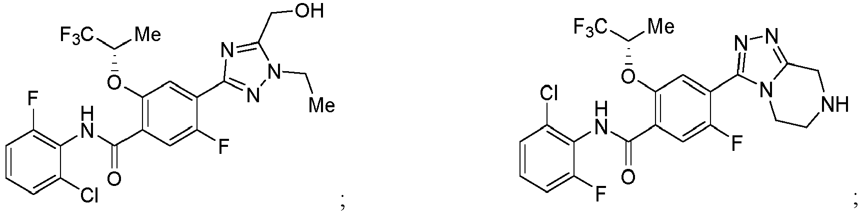 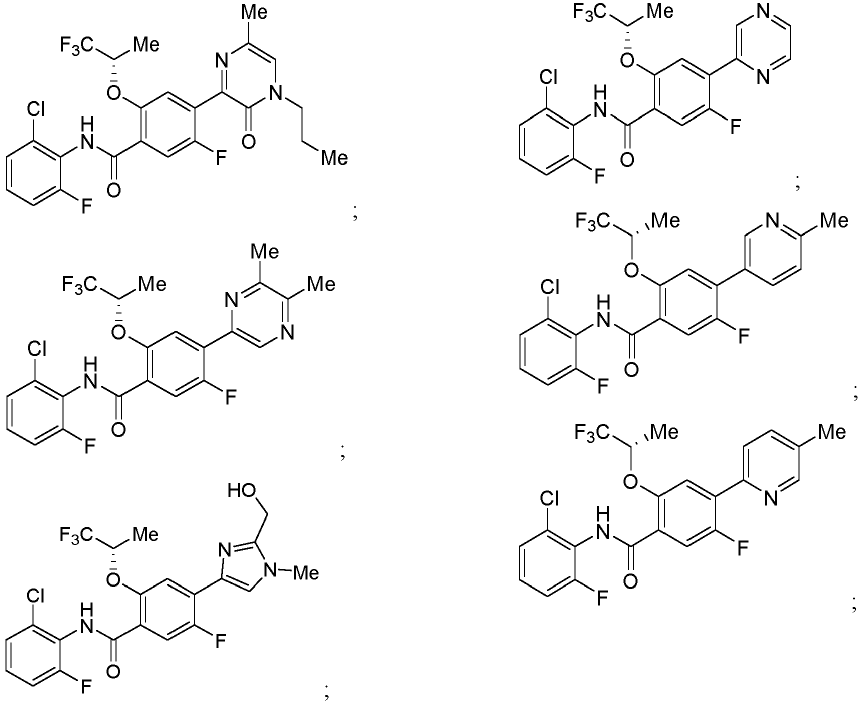 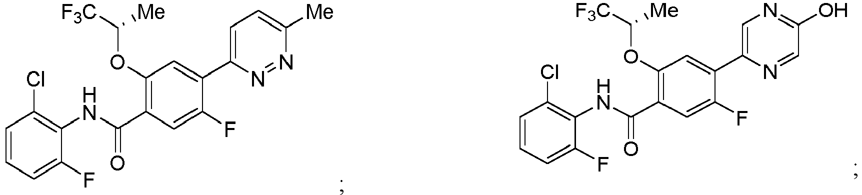 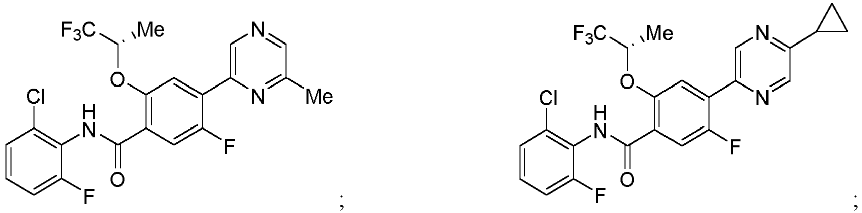 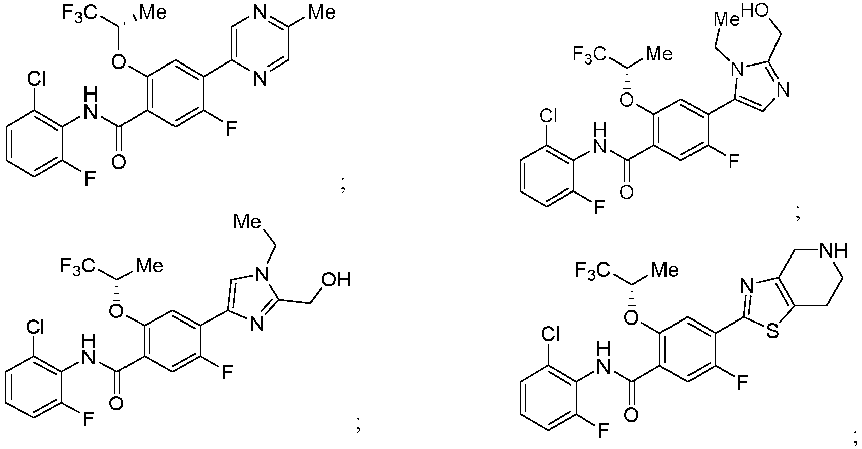 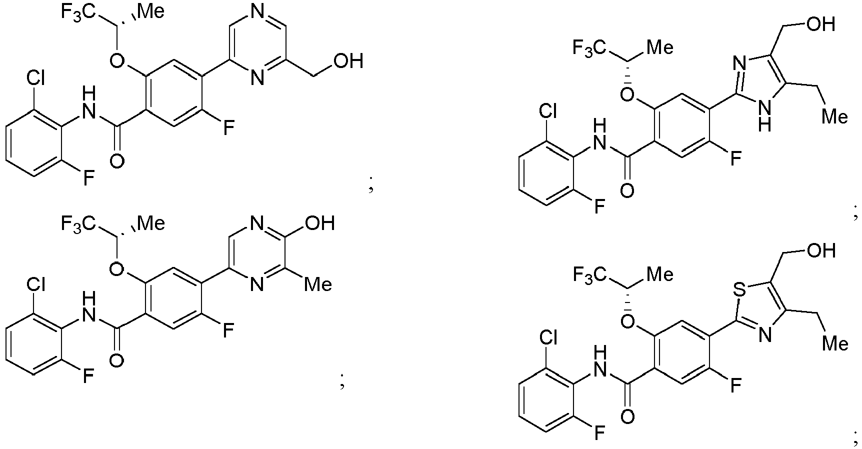 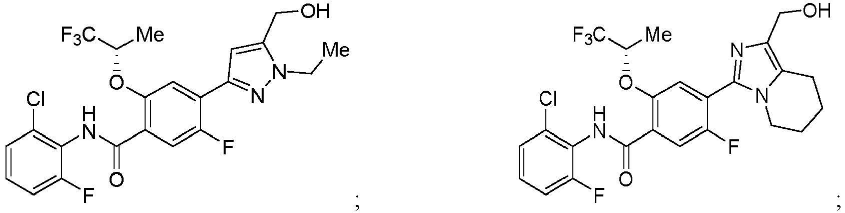 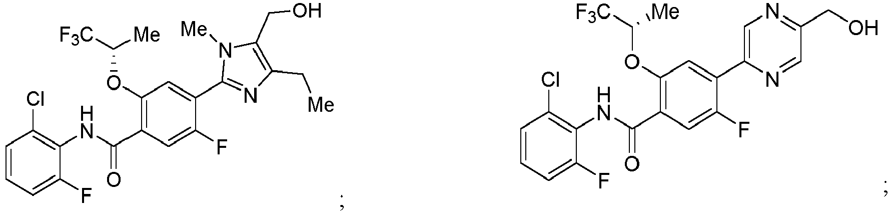 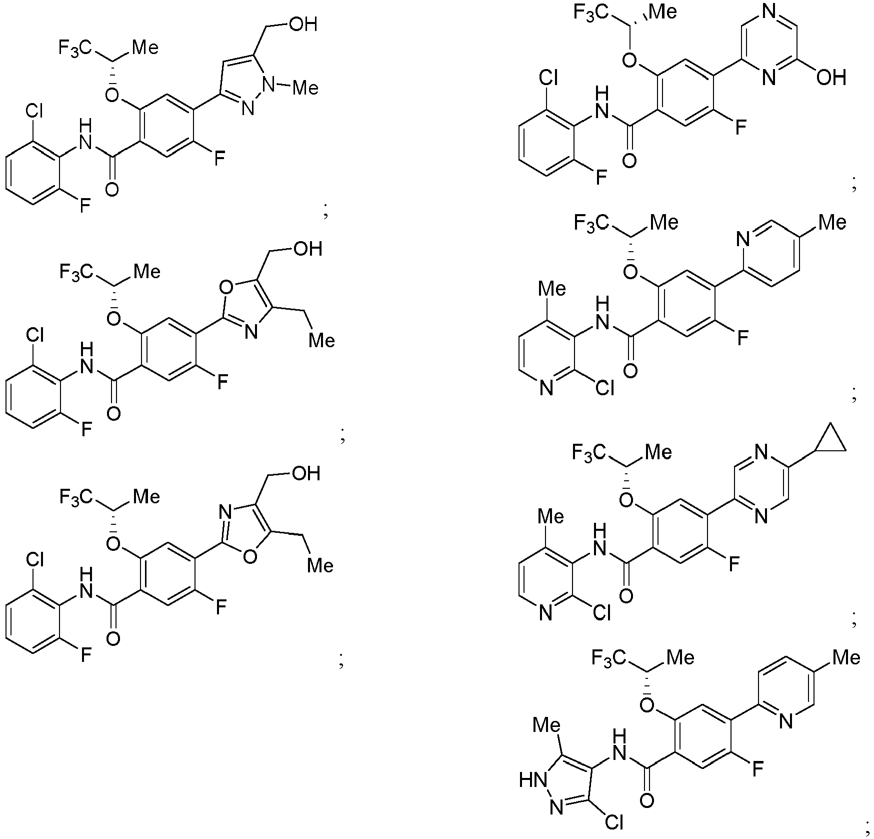 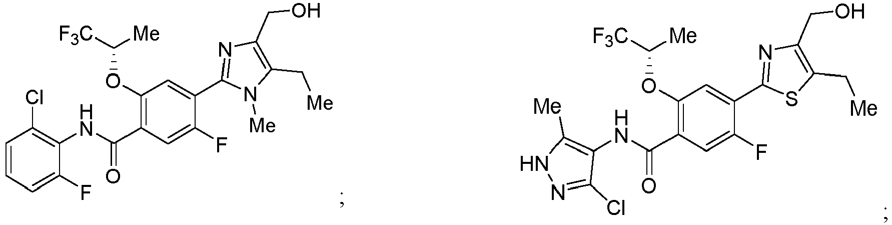 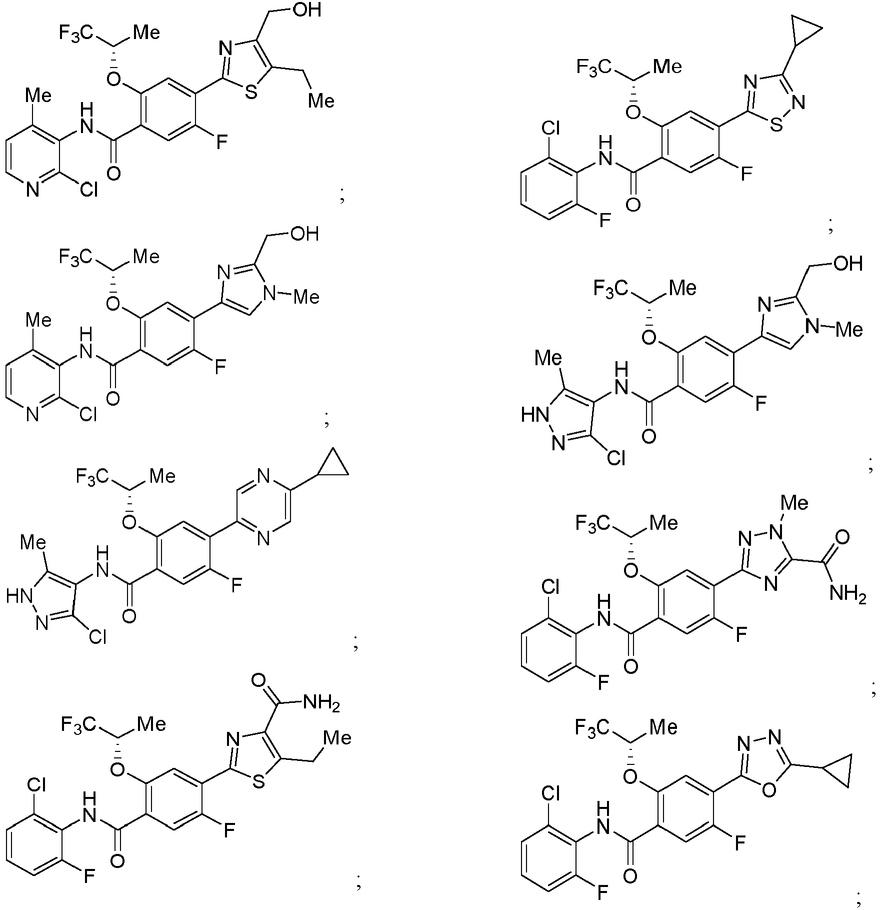 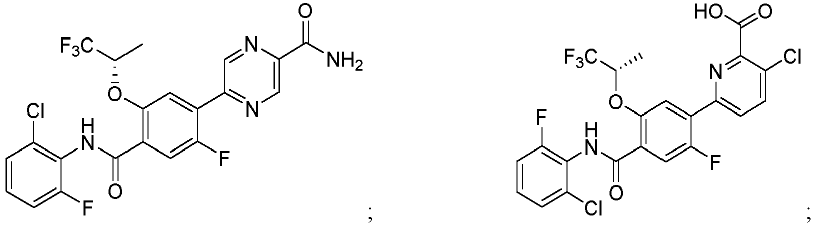 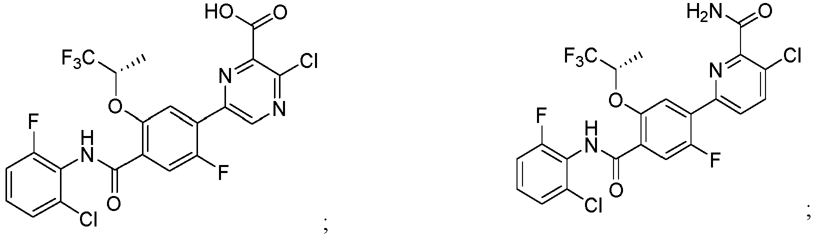 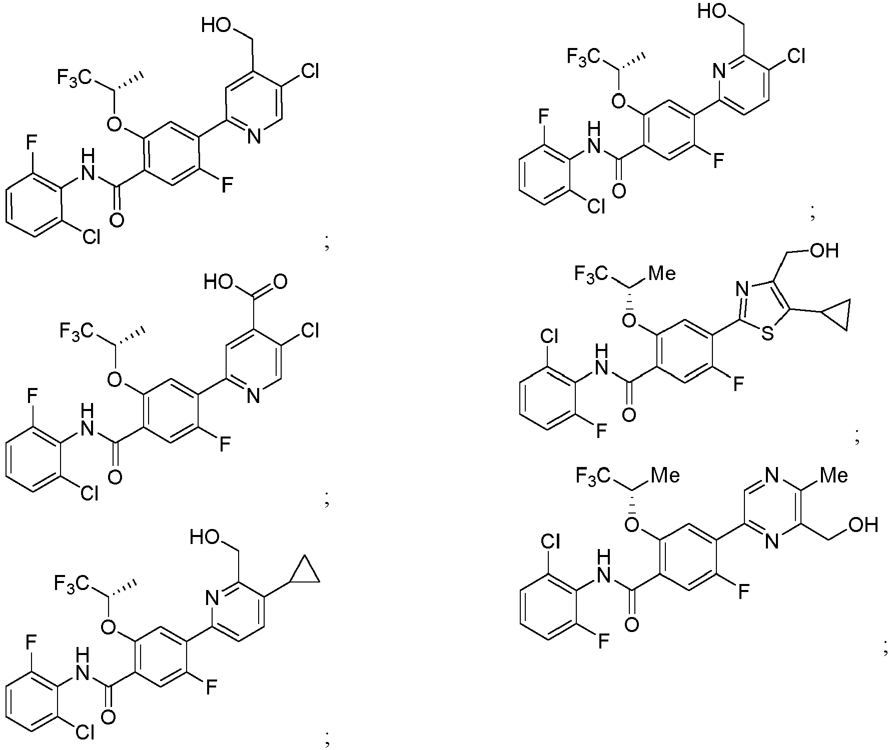 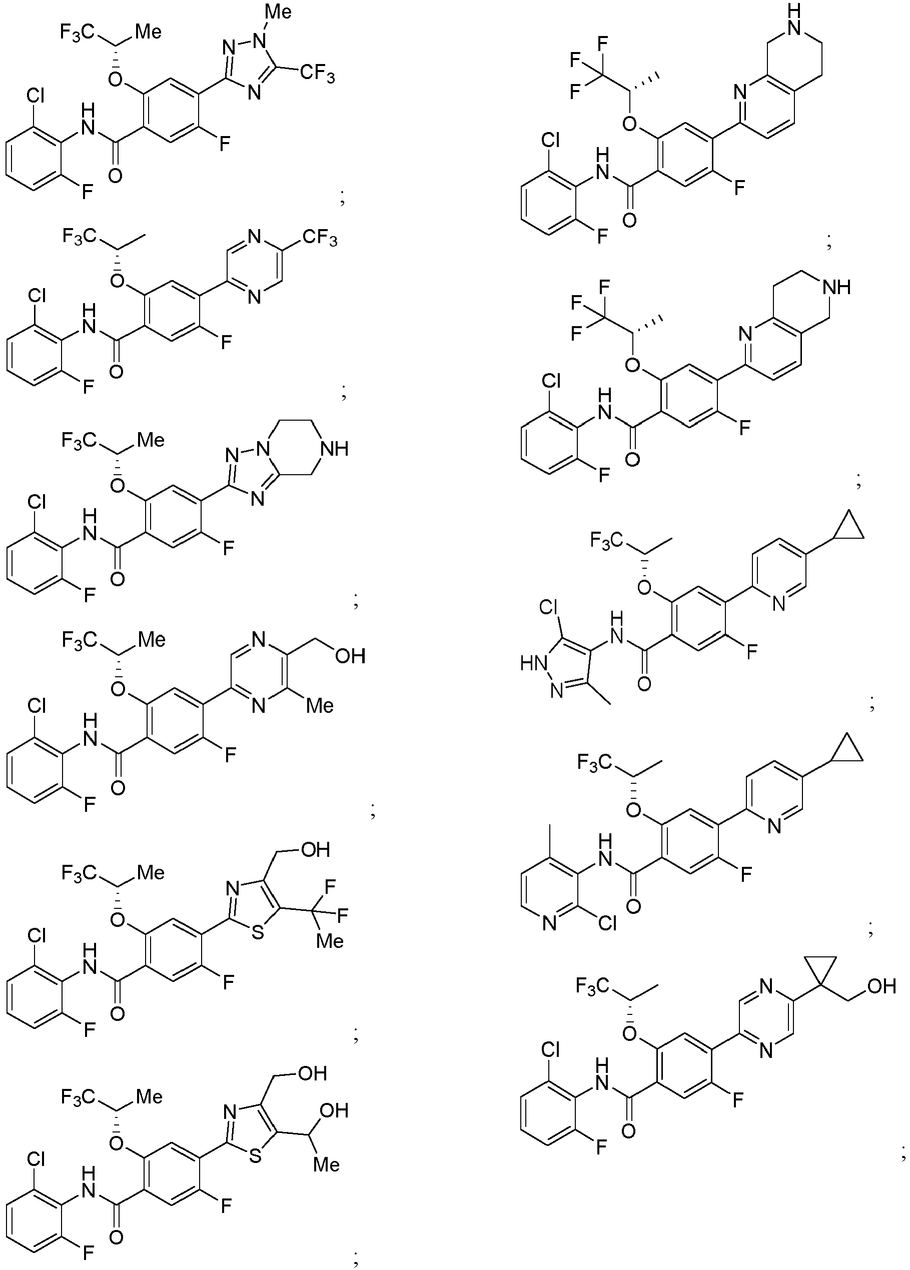 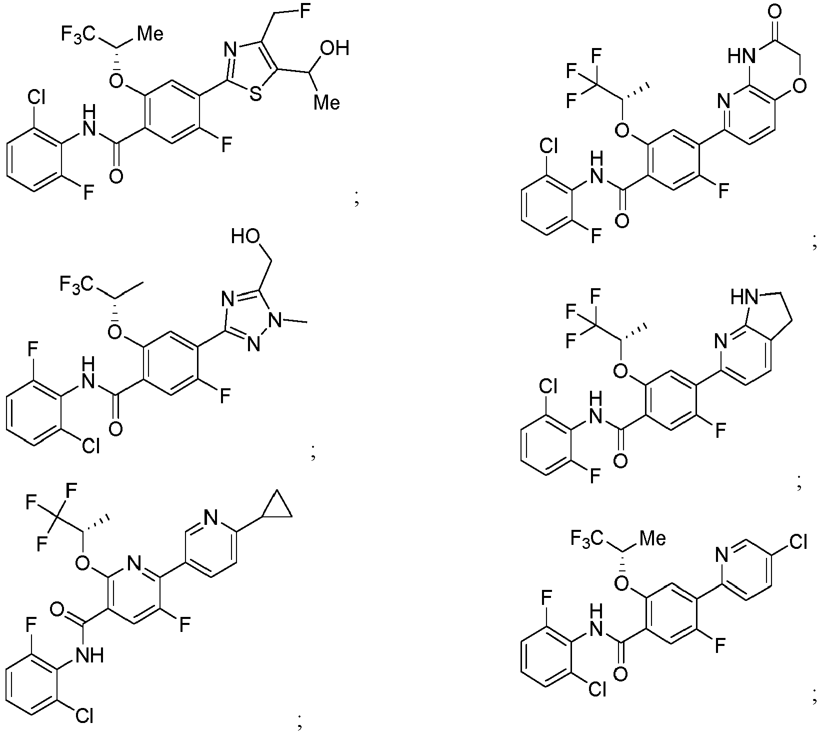 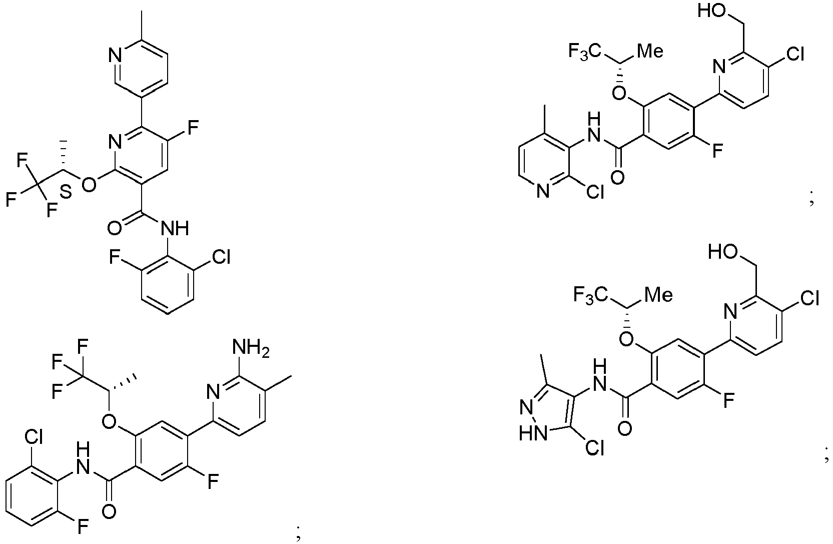 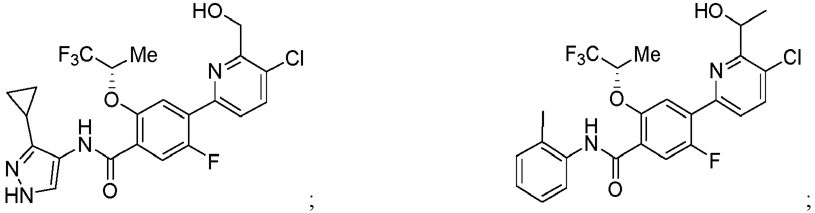 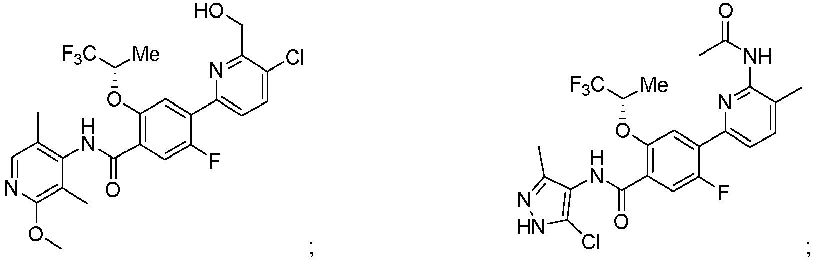 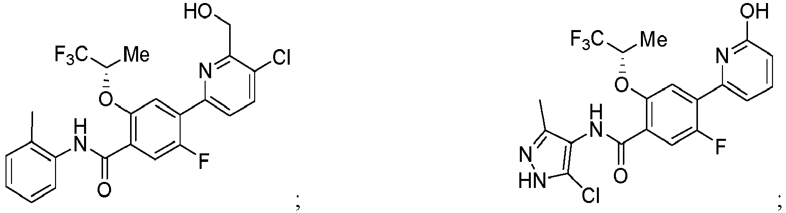 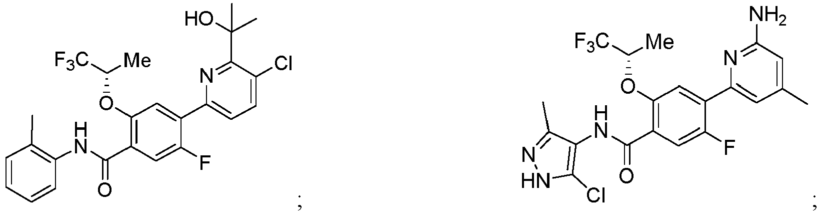 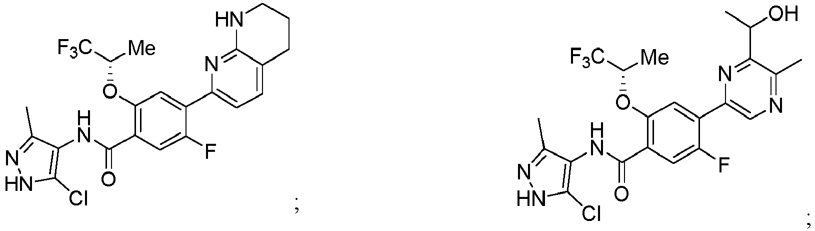 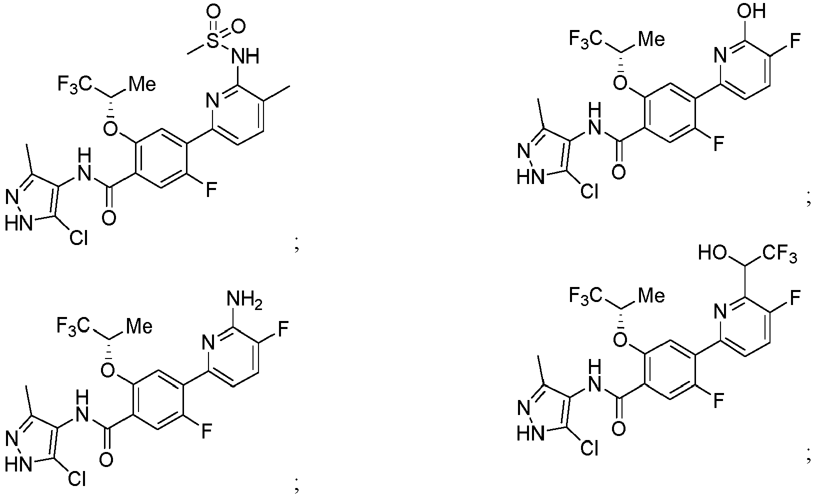 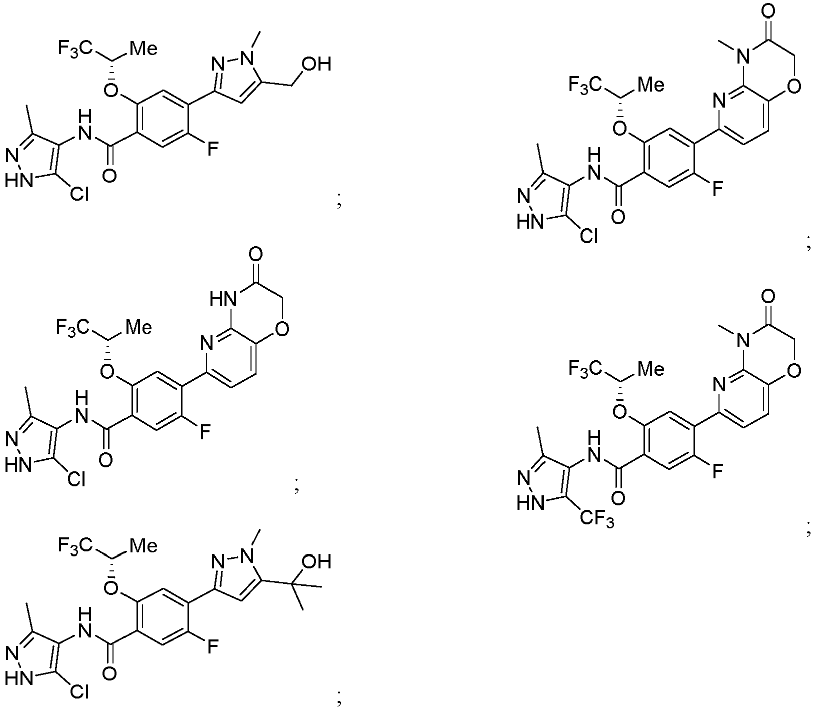 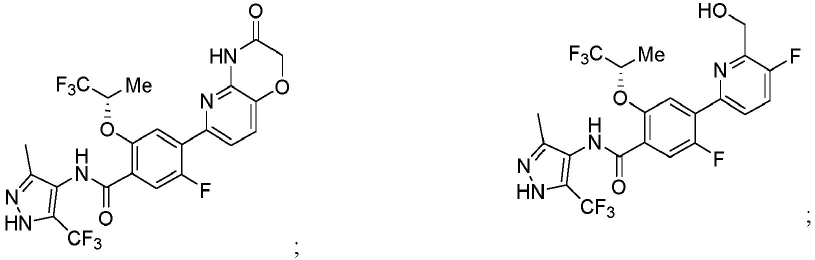 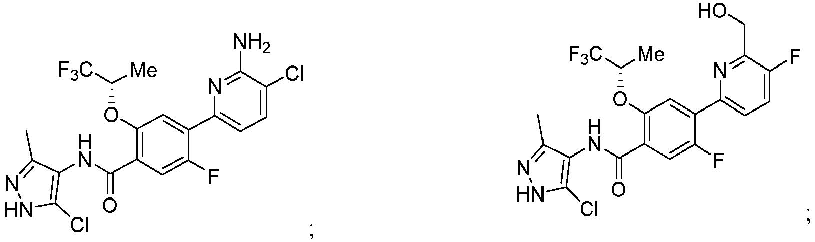 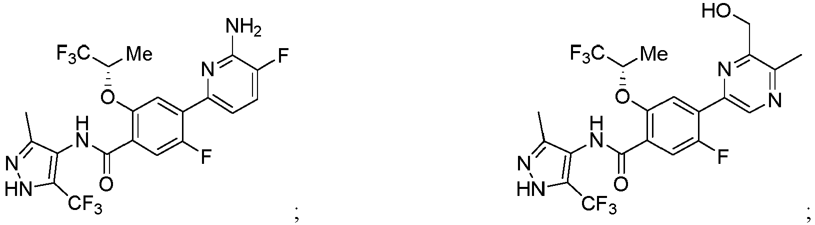 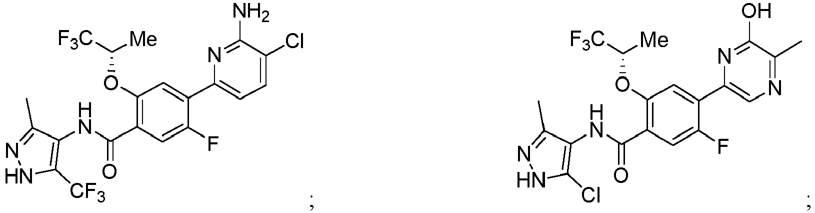 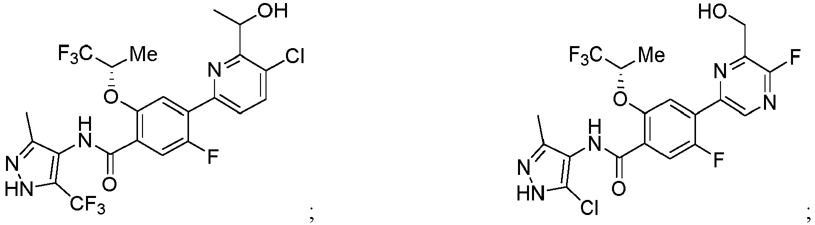 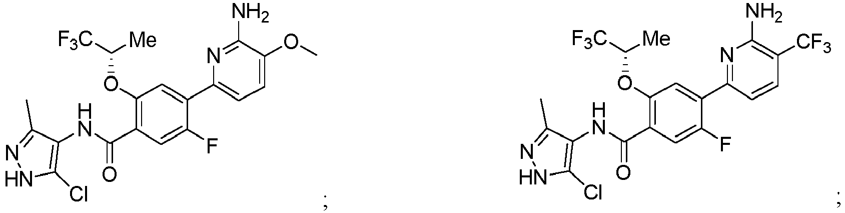 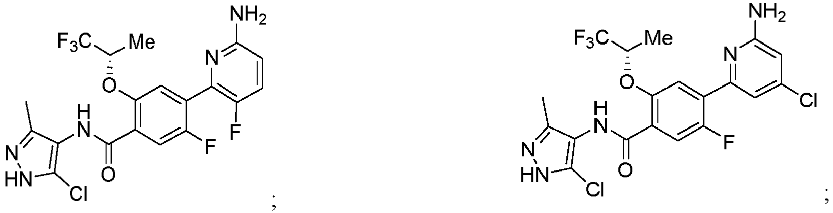 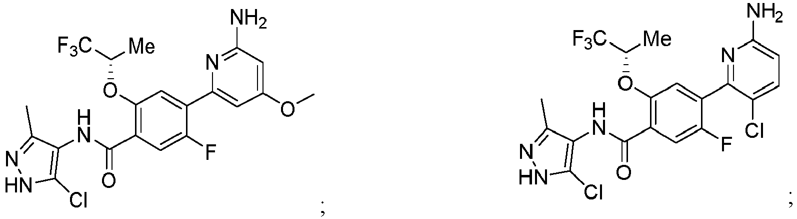 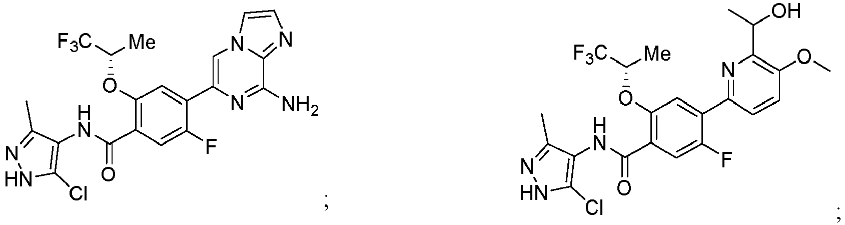 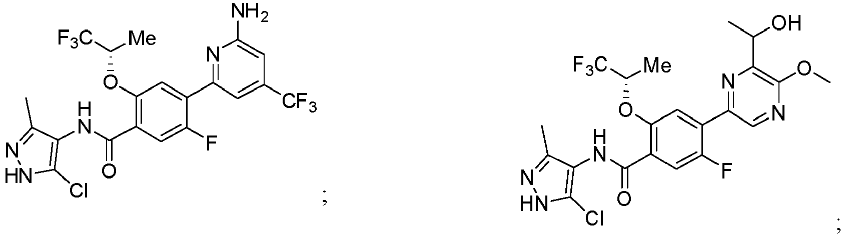 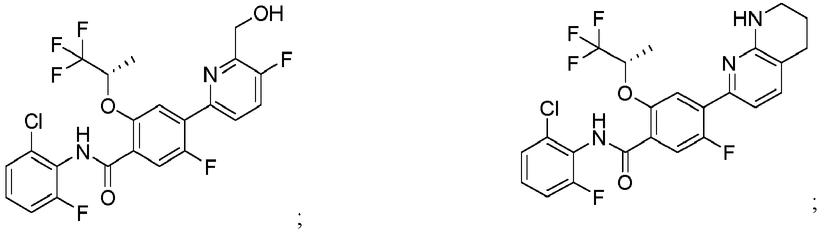 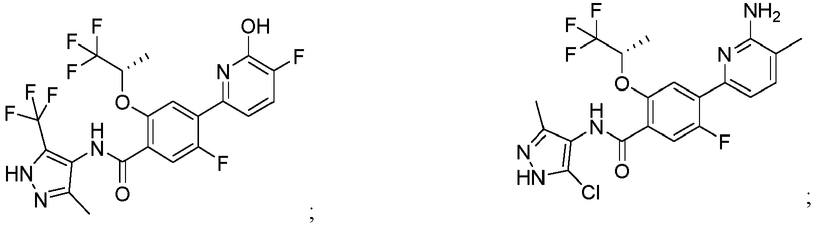 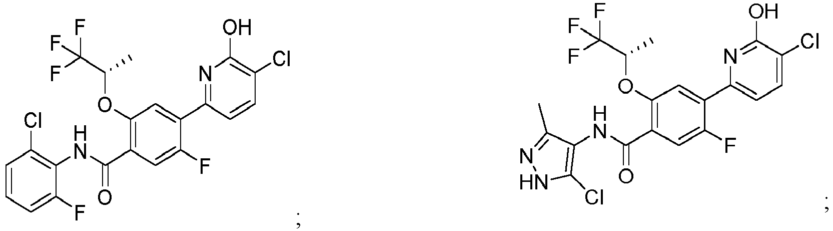 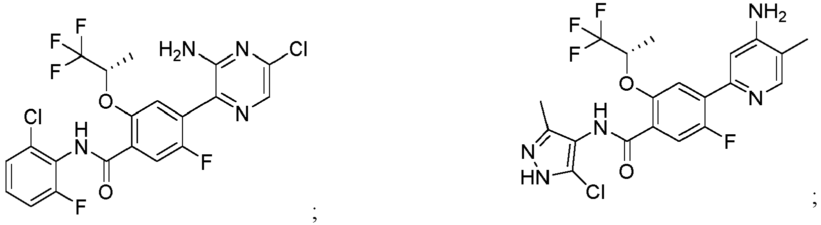 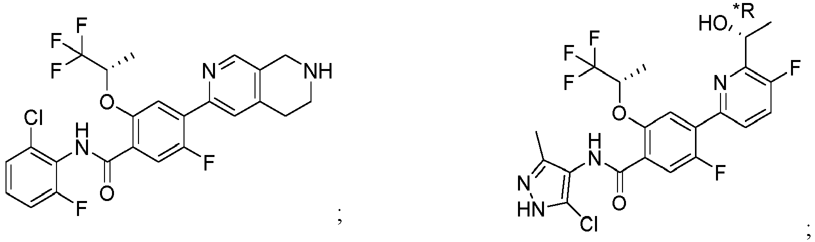 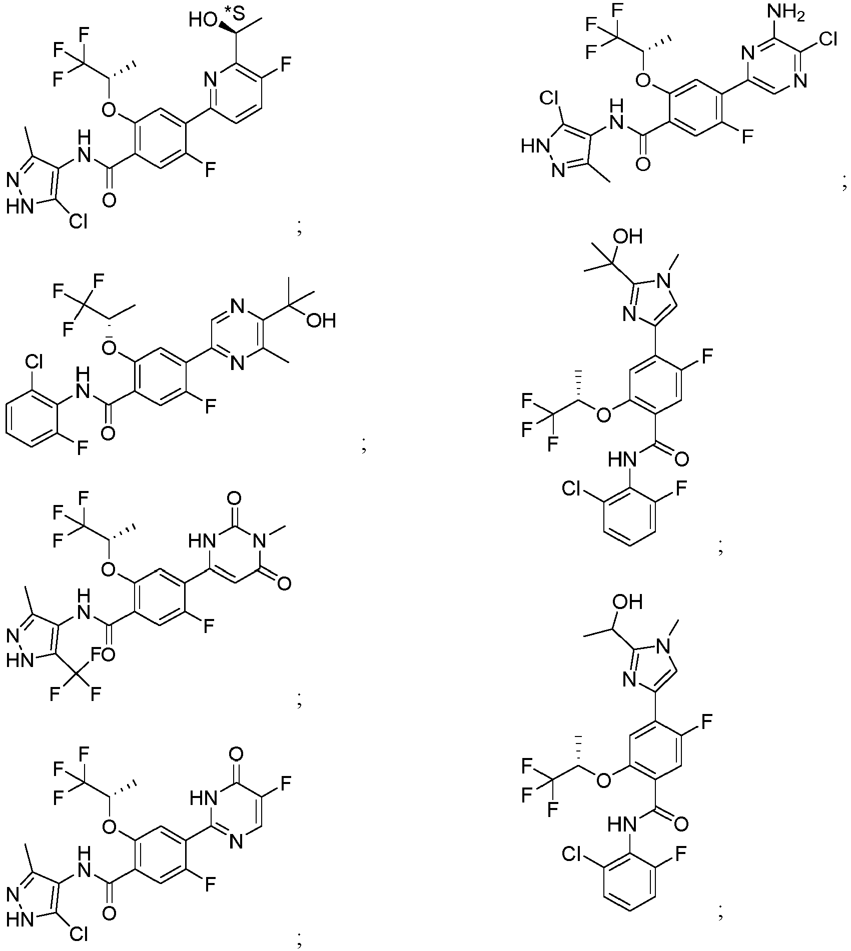 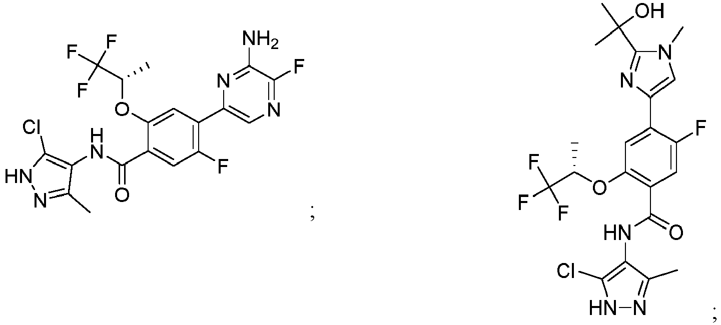 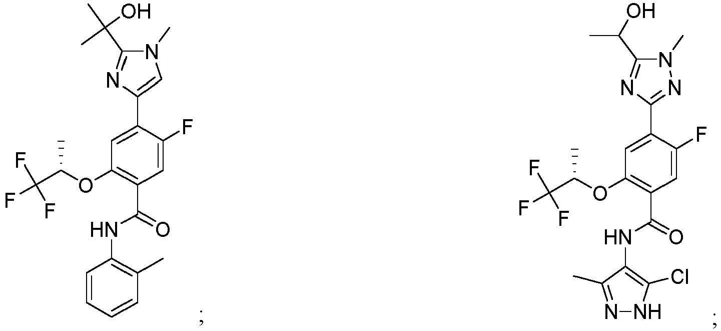 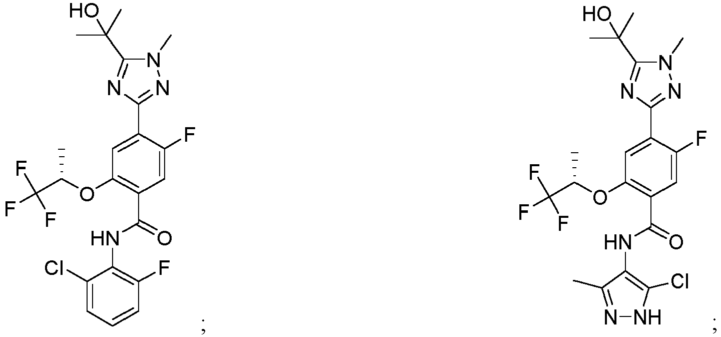 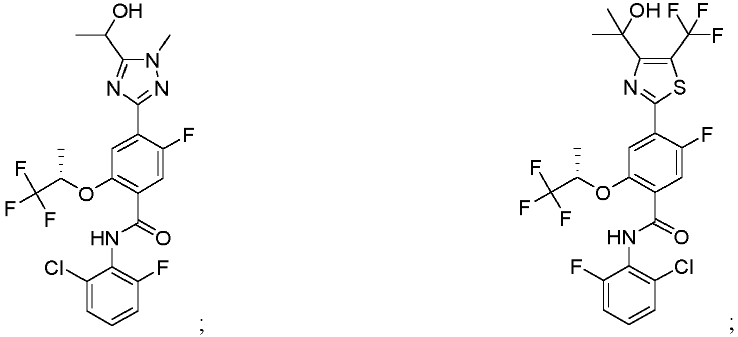 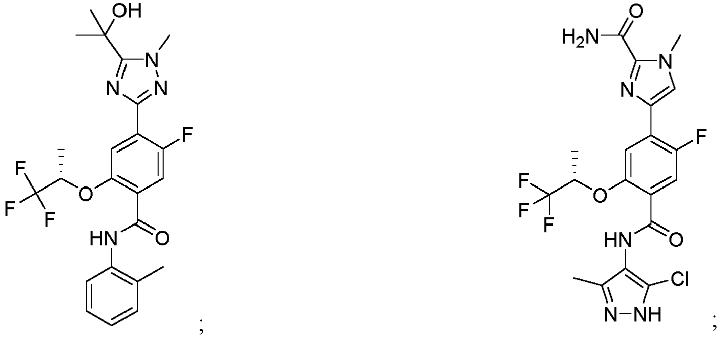 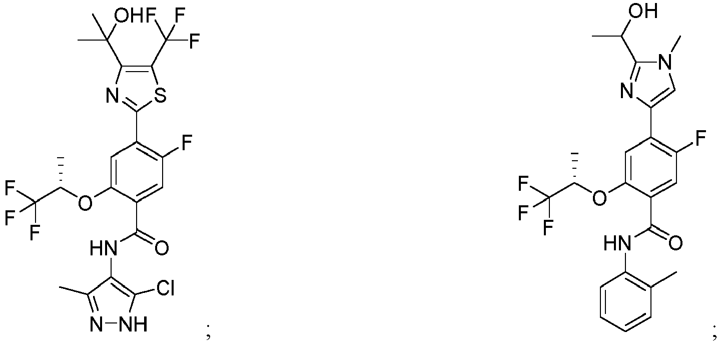 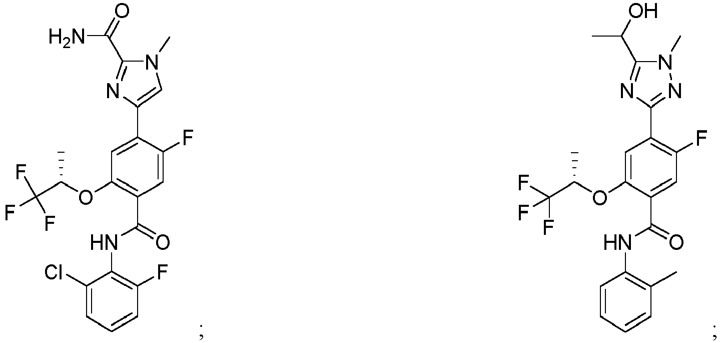 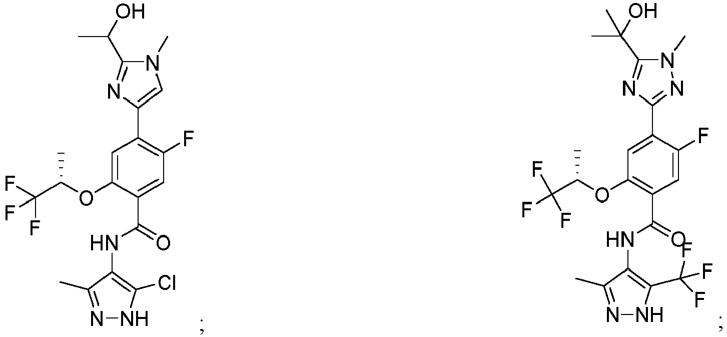 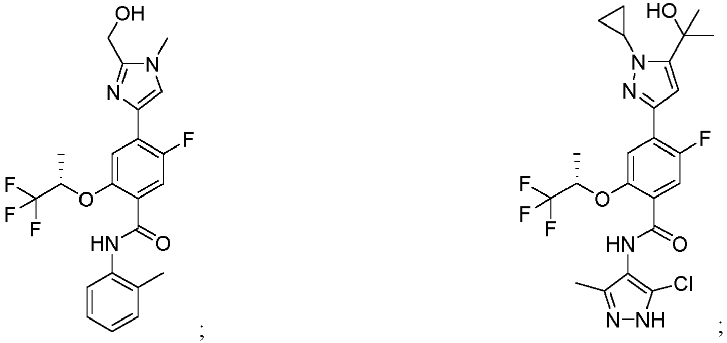 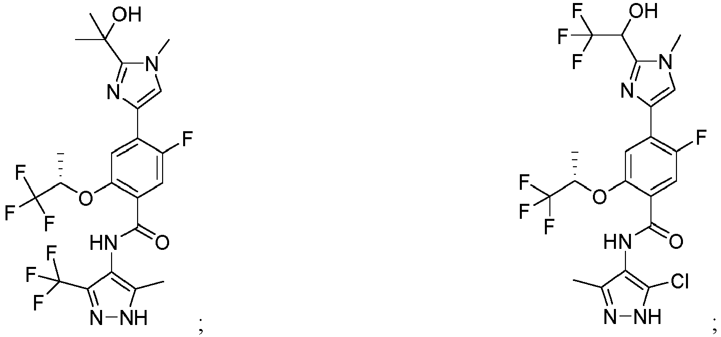 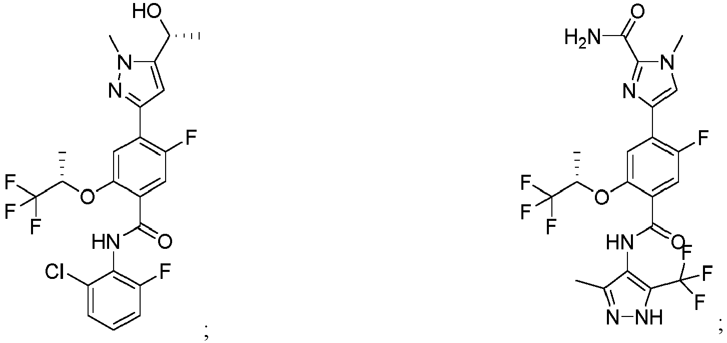 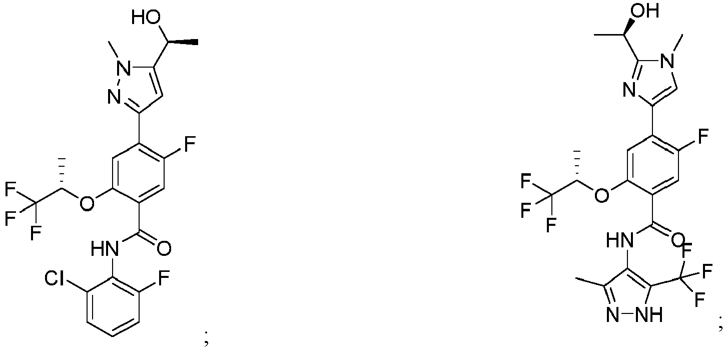 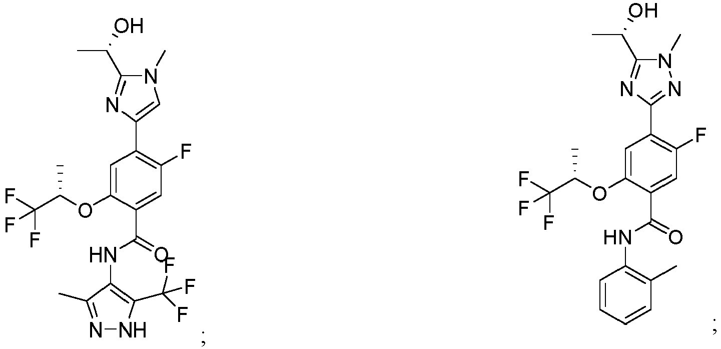 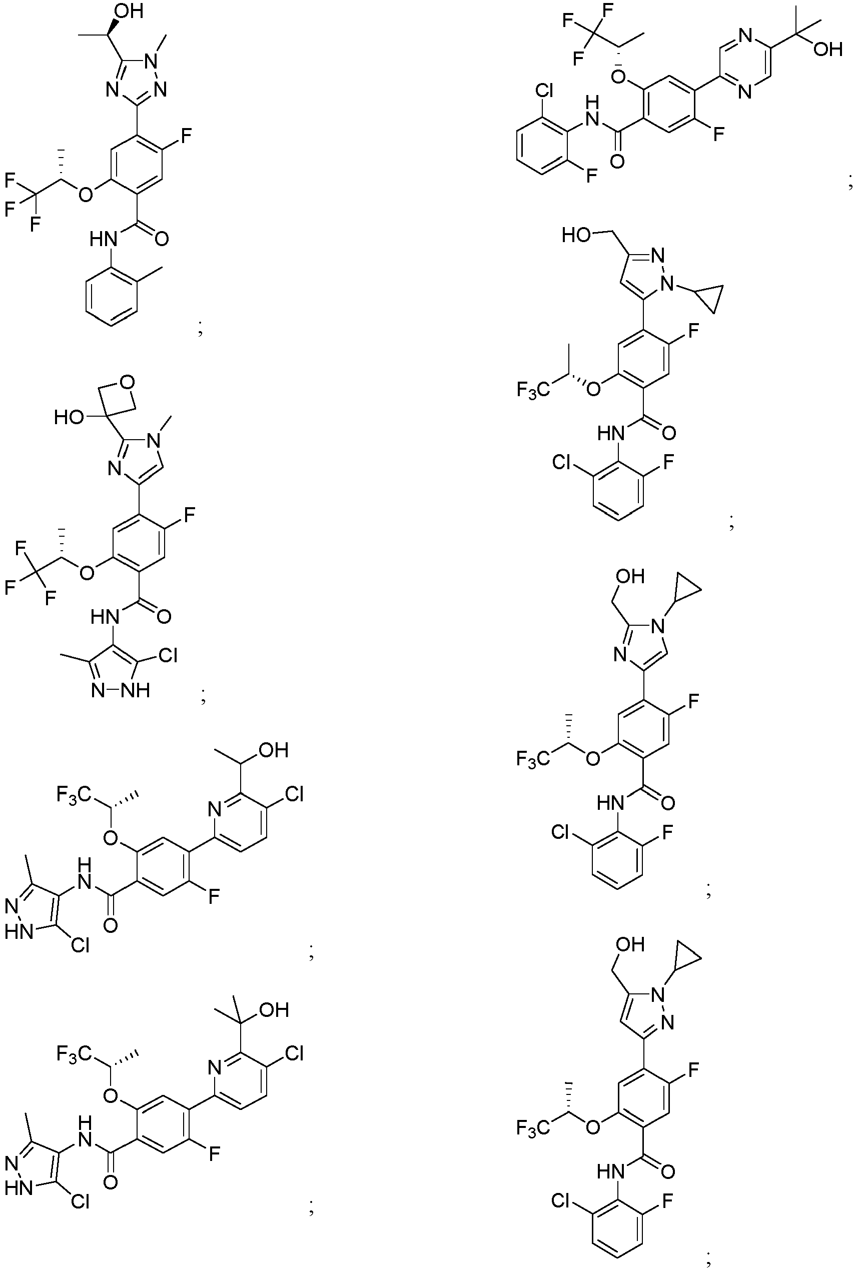 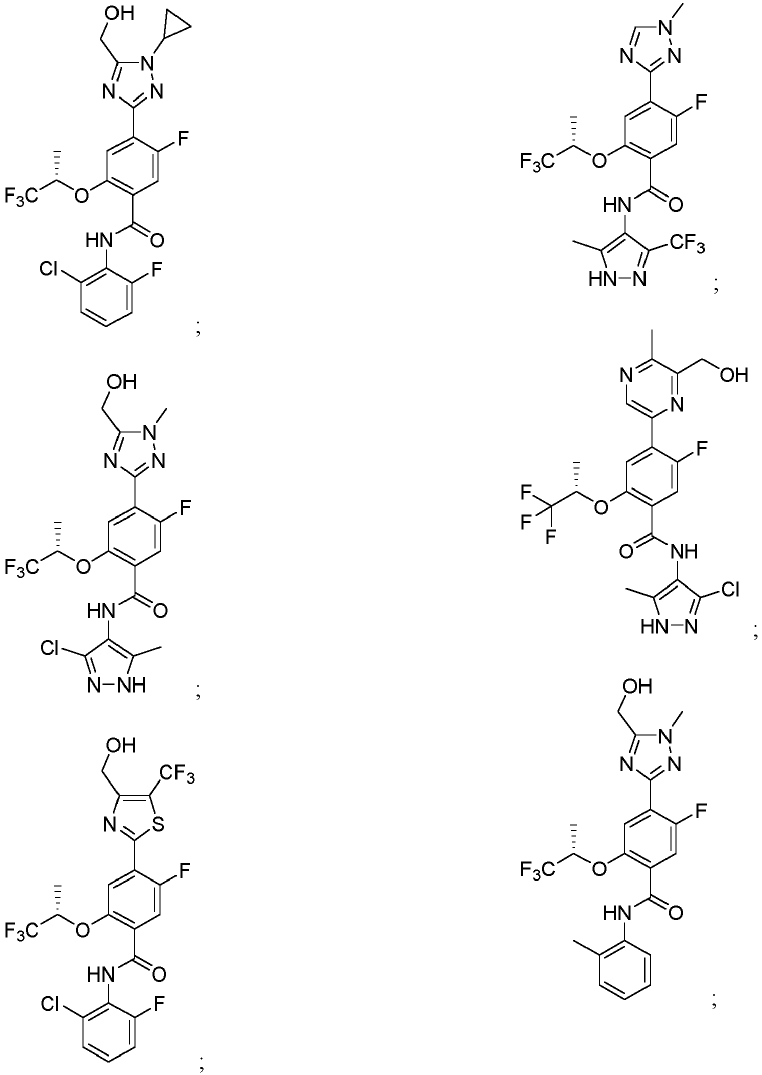 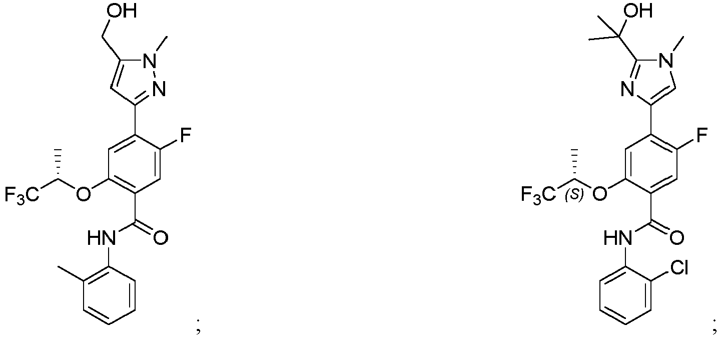 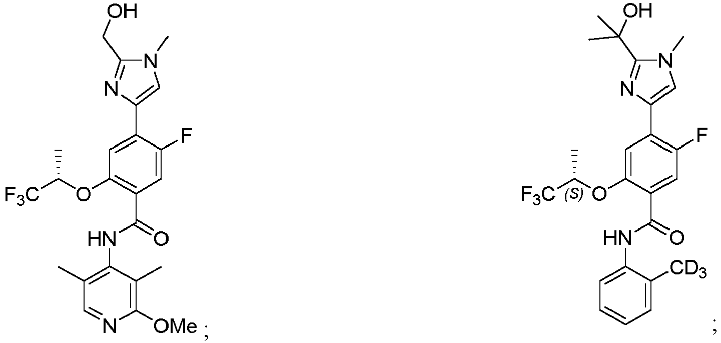 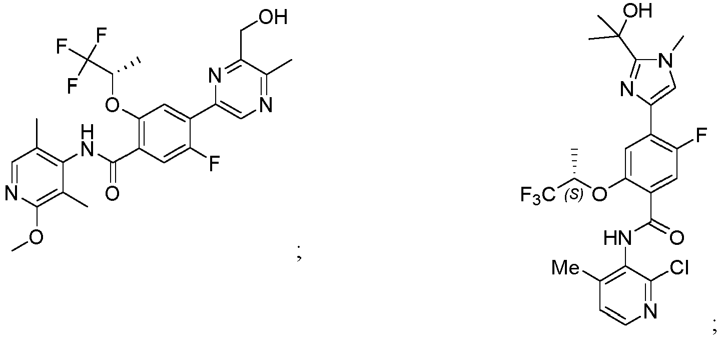 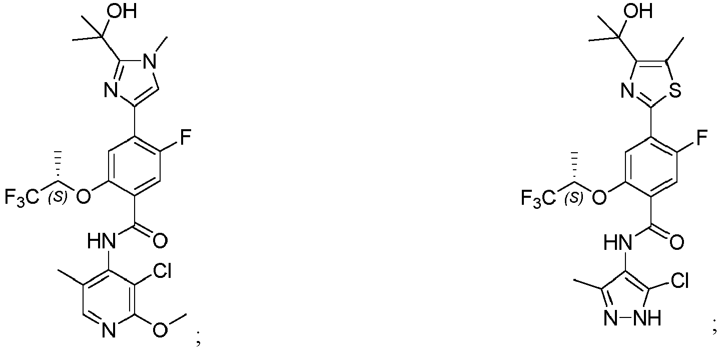 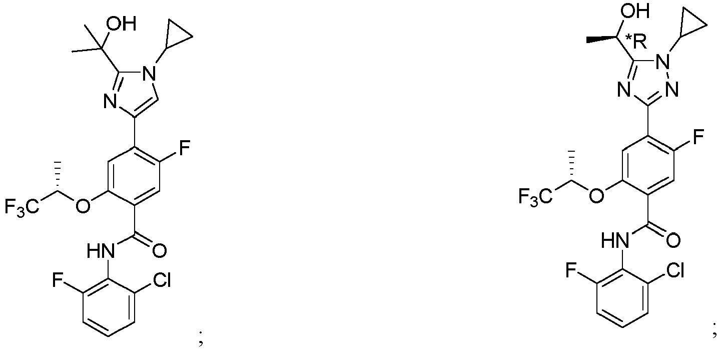 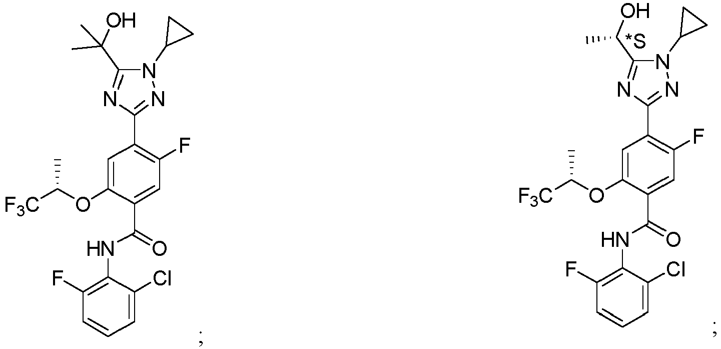 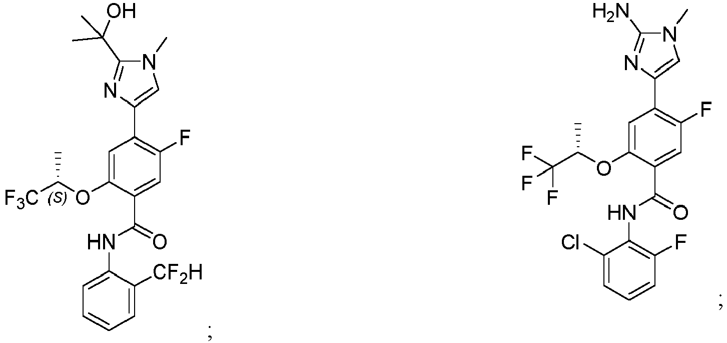 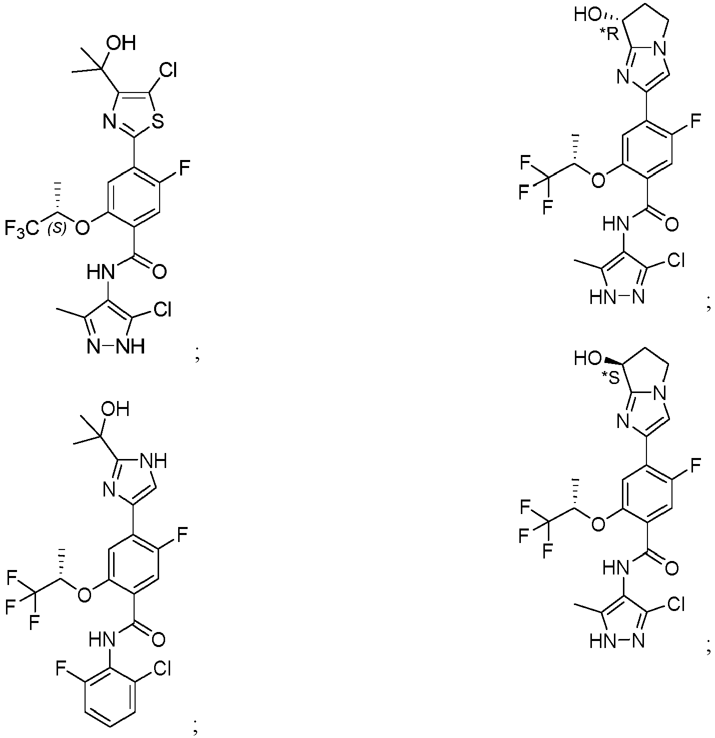 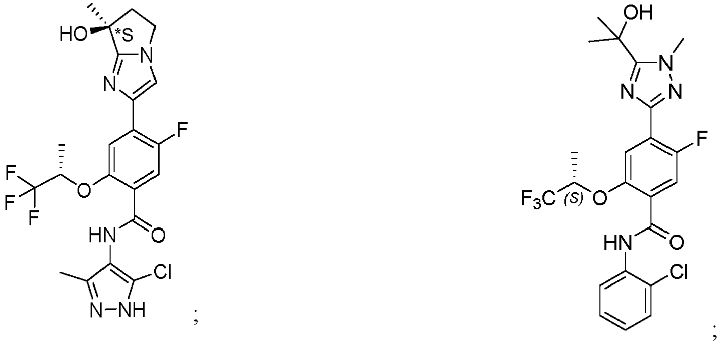 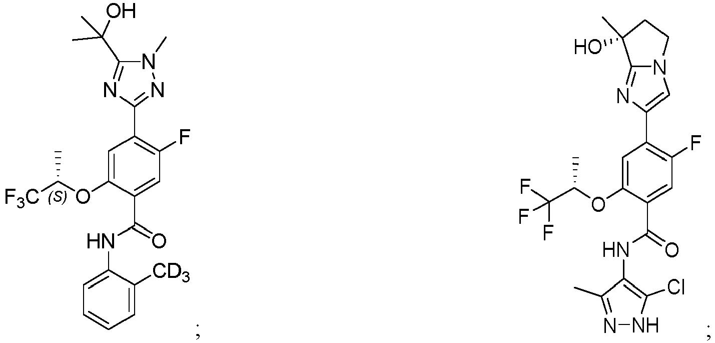 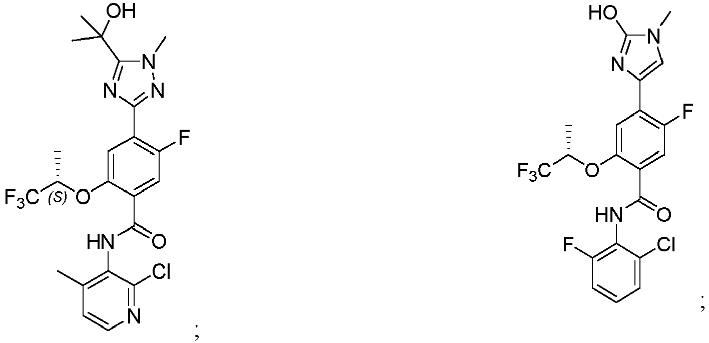 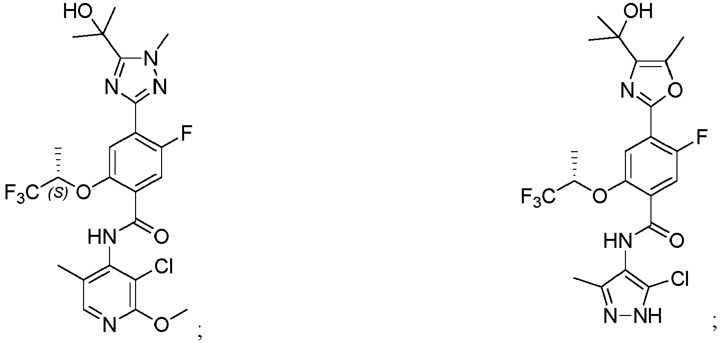 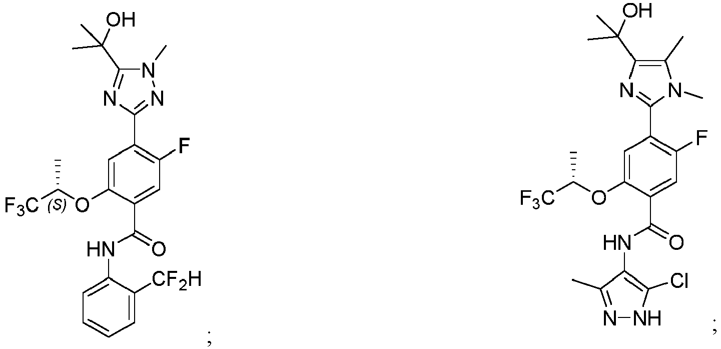 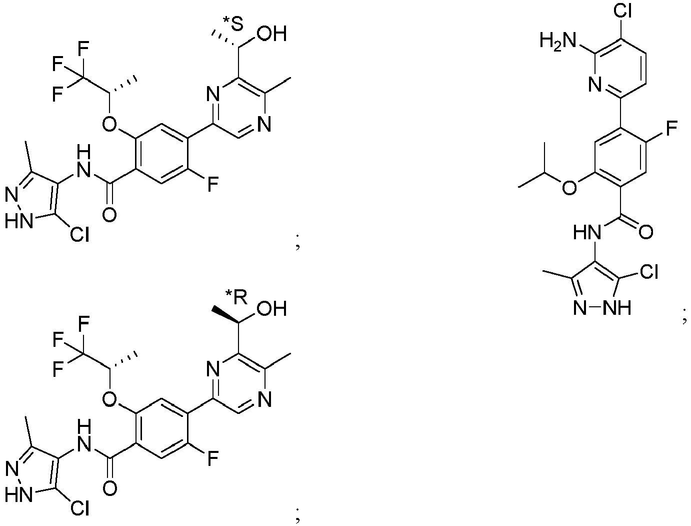 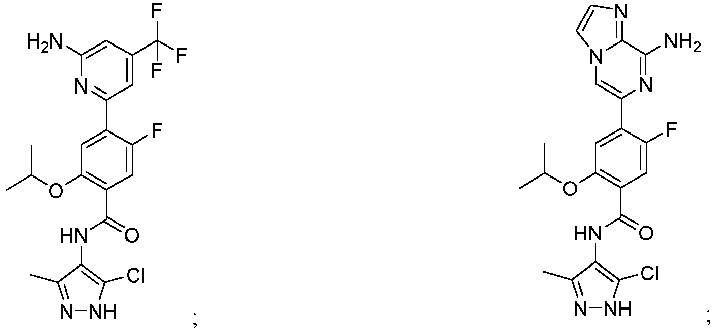 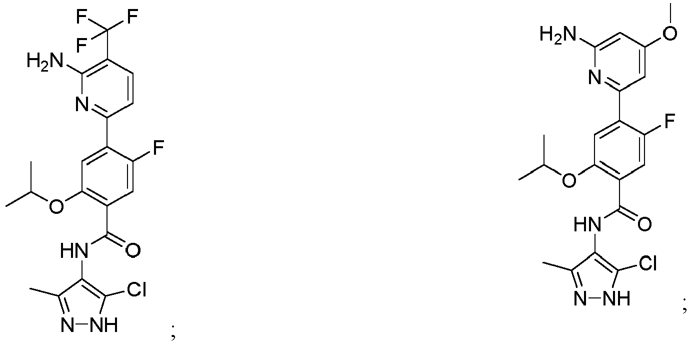 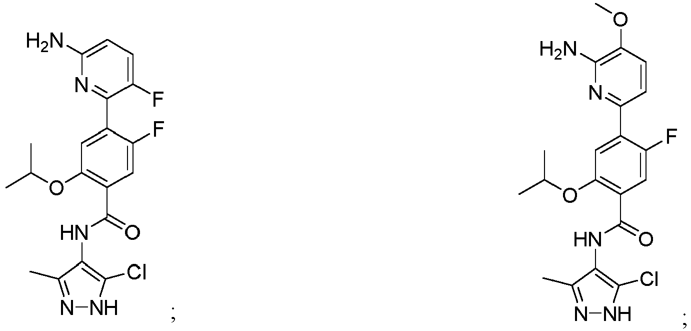 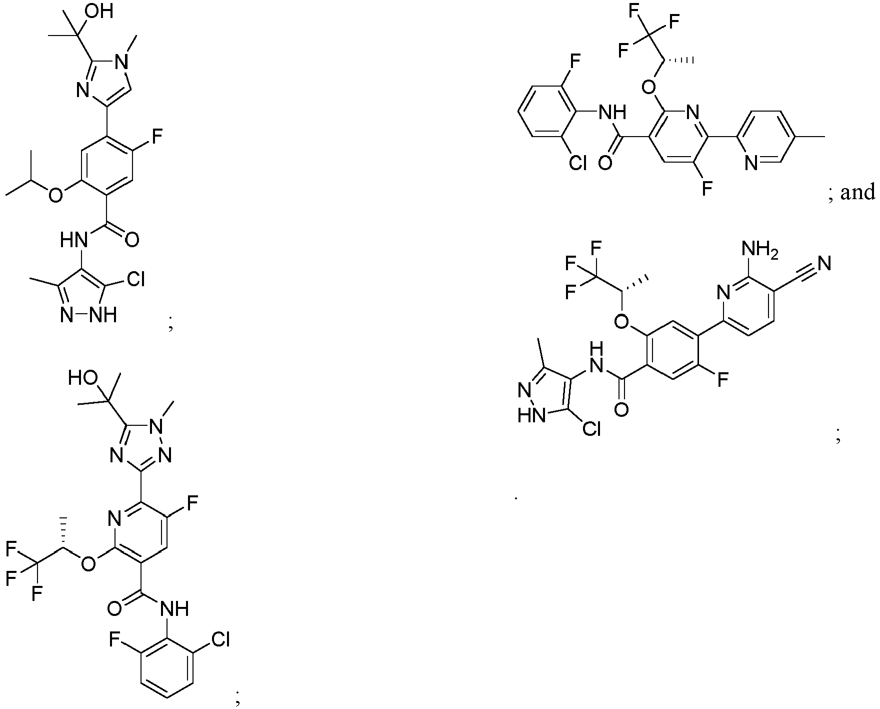 or a pharmaceutically acceptable salt, solvate, stereoisomer, isotopic variant, or N-oxide thereof.

Embodiments of this invention include compounds of Formula I wherein:
Q is CH or N;
R¹ is 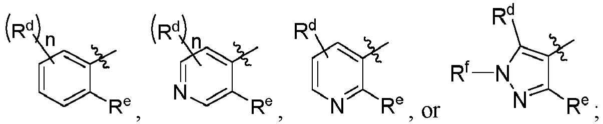 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is imidazolyl; wherein said imidazolyl is optionally substituted with up to three substituents independently selected from the group comprising 3-hydroxyoxetanyl, -NH₂, -OH, -CN, or R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 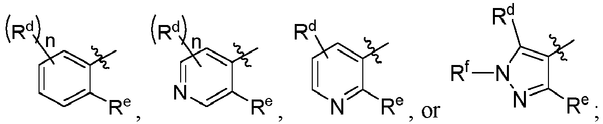 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is triazolyl; wherein said triazolyl is optionally substituted with one or two groups independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 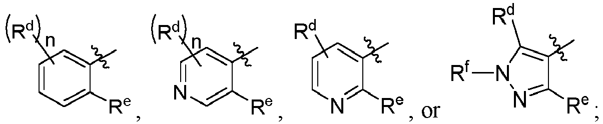 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is pyrazinyl; wherein said pyrazinyl is optionally substituted with up to three substituents independently selected from the group comprising -NHSO₂CH₃, -NHC(O)CH₃, -OCH₃, - OH, -NH₂, -CO₂H, -Cl, -F, or R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 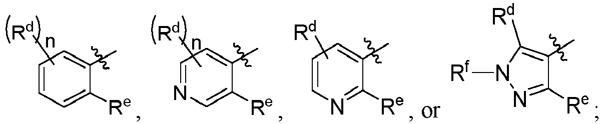 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is pyrazinonyl; wherein said pyrazinonyl is optionally substituted with one or two groups independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 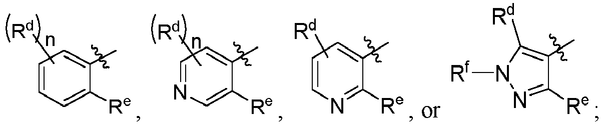 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is thiazolyl; wherein said thiazolyl is optionally substituted with one or two substituents independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 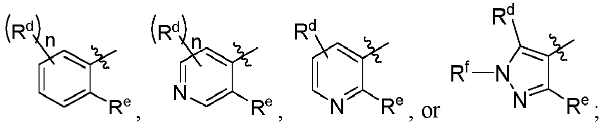 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 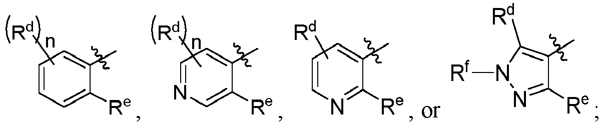 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 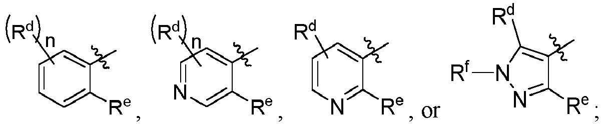 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is pyridazinyl; wherein said pyridazinyl is optionally substituted with one or two groups independently selected from the group comprising R^{a}, -Cl, or -CO₂H;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 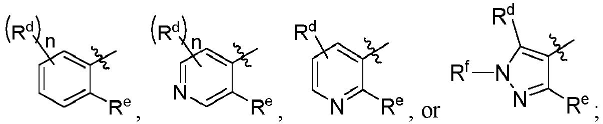 wherein
   R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{e} is selected from the group consisting of: -Cl, -F, and -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 4,5,6,7-tetrahydrothiazolo[5,4-*c*]pyridinyl; wherein said 4,5,6,7-tetrahydrothiazolo[5,4-*c*]pyridinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 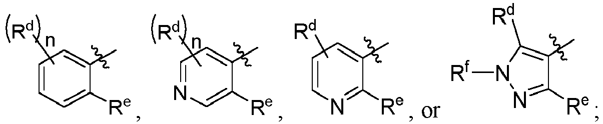 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is pyrazolyl; wherein said pyrazolyl is optionally substituted with one or two substituents independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 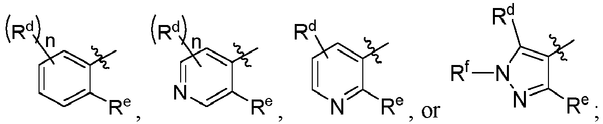 wherein
   R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{e} is selected from the group consisting of: -Cl, -F, and -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is oxazolyl; wherein said oxazolyl is optionally substituted with one or two substituents independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 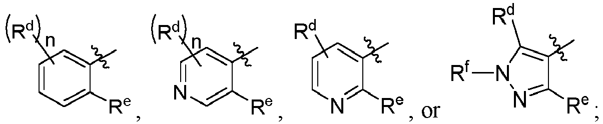 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 1,3,4-oxadiazolyl; wherein said 1,3,4-oxadiazolyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 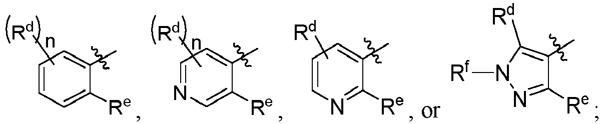 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 5,6,7,8-tetrahydroimidazo[1,5-a]pyridinyl; wherein said 5,6,7,8-tetrahydroimidazo[1,5-a]pyridinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 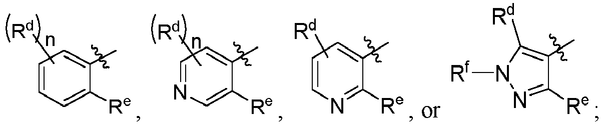 wherein
   R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{e} is selected from the group consisting of: -Cl, -F, and -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 1,2,4-thiadiazolyl; wherein said 1,2,4-thiadiazolyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 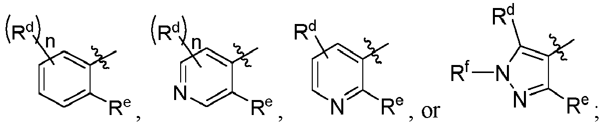 wherein
   R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{e} is selected from the group consisting of: -Cl, -F, and -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 5,6,7,8-tetrahydro-1,7-naphthyridinyl; wherein said 5,6,7,8-tetrahydro-1,7-naphthyridinyl is optionally substituted with R^{a}; or
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Embodiments of this invention include compounds of Formula I wherein:
Q is CH or N;
R¹ is 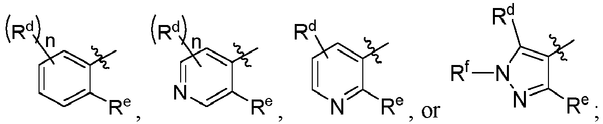 wherein
   R^{d} is selected from the group consisting of: -H, -Cl, -F, and -C₍₁₋₃₎alkyl; wherein said -C₍₁₋₃₎alkyl is optionally substituted with: -OH, -OCH₃, -SCH₃, or -OCF₃; and wherein said - C₍₁₋₃₎alkyl is optionally substituted with up to six fluorine atoms;
   R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
   R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
   n is 1, or 2;
R² is 5,6,7,8-tetrahydro-1,6-naphthyridinyl; wherein said 5,6,7,8-tetrahydro-1,6-naphthyridinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
   R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
   R^{c} is -CH₃ or -CHF₂; or R^{b} and R^{c} may be taken together with the carbon to which they are bound to form -C₍₃₋₆₎cycloalkyl; wherein said -C₍₃₋₆₎cycloalkyl is optionally substituted with up to four substituents independently selected from the group consisting of: -Cl, -F, - OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

Disclosed herein are embodiments including at least one compound according to any of the embodiments described herein, such as compounds of Formula I or II, or a pharmaceutically acceptable salt, solvate, stereoisomer, isotopic variant, or N-oxide thereof, for use in treating a subject suffering from or diagnosed with a disease, disorder, or medical condition comprising inhibiting or altering dihydroorotate oxygenase enzyme activity in the subject, wherein the disorder, disease or medical condition is selected from the group consisting of: inflammatory disorders, autoimmune disorders, and cancer.

Disclosed herein are embodiments including at least one compound of Formula I or II, or a pharmaceutically acceptable salt, solvate, stereoisomer, isotopic variant, or N-oxide thereof, for use in treating a subject suffering from or diagnosed with a disease, disorder, or medical condition comprising inhibiting or altering dihydroorotate oxygenase enzyme activity in the subject, wherein the disorder, disease or medical condition is selected from the group consisting of: lymphomas, leukemias, carcinomas, and sarcomas.

Disclosed herein are embodiments including at least one compound of Formula I or II, or a pharmaceutically acceptable salt, solvate, stereoisomer, isotopic variant, or N-oxide thereof, for use in treating a subject suffering from or diagnosed with a disease, disorder, or medical condition comprising inhibiting or altering dihydroorotate oxygenase enzyme activity in the subject, wherein the disorder, disease or medical condition is selected from the group consisting of: acute lymphoblastic leukemia, acute myeloid leukemia, (acute) T-cell leukemia, acute lymphoblastic leukemia, acute lymphocytic leukemia, acute monocytic leukemia, acute promyelocytic leukemia, bisphenotypic B myelomonocytic leukemia, chronic lymphocytic leukemia, chronic myelogenous leukemia, chronic myeloid leukemia, chronic myelomonocytic leukemia, large granular lymphocytic leukemia, plasma cell leukemia, and also myelodysplastic syndrome, which can develop into an acute myeloid leukemia.
Disclosed herein are embodiments including at least one compound of Formula I or II, or a pharmaceutically acceptable salt, solvate, stereoisomer, isotopic variant, or N-oxide thereof, for use in treating a subject suffering from or diagnosed with a disease, disorder, or medical condition comprising inhibiting or altering dihydroorotate oxygenase enzyme activity in the subject, wherein the disorder, disease or medical condition is acute myeloid leukemia.

Also within the scope of the invention are enantiomers and diastereomers of the compounds of Formula (I). Also within the scope of the invention are the pharmaceutically acceptable salts, N-oxides or solvates of the compounds of Formula (I). Pharmaceutically acceptable prodrugs of compounds of Formula (I) may be prepared, and pharmaceutically active metabolites of the compounds of Formula (I) may also be prepared.

Also within the scope of the invention are isotopic variations of compounds of Formula (I), such as, e.g., deuterated compounds of Formula (I). Also within the scope of the invention are the pharmaceutically acceptable salts, N-oxides or solvates of the isotopic variations of the compounds of Formula (I). Pharmaceutically acceptable prodrugs of the isotopic variations of the compounds of Formula (I) may be prepared, and pharmaceutically active metabolites of the isotopic variations of the compounds of Formula (I) may also be prepared.

Even though the compounds of embodiments of the present invention (including their pharmaceutically acceptable salts and pharmaceutically acceptable solvates) can be administered alone, they will generally be administered in admixture with a pharmaceutically acceptable carrier, a pharmaceutically acceptable excipient and/or a pharmaceutically acceptable diluent selected with regard to the intended route of administration and standard pharmaceutical or veterinary practice.

Thus, particular embodiments of the present invention are directed to pharmaceutical and veterinary compositions comprising compounds of Formula (I) and at least one pharmaceutically acceptable carrier, pharmaceutically acceptable excipient, and/or pharmaceutically acceptable diluent. By way of example, in the pharmaceutical compositions of embodiments of the present invention, the compounds of Formula (I) may be admixed with any suitable binder(s), lubricant(s), suspending agent(s), coating agent(s), solubilizing agent(s), and combinations thereof.

An embodiment of the invention relates to a pharmaceutical composition comprising an effective amount of at least one compound selected from compounds of Formula (I), and pharmaceutically acceptable salts, isotopes, N-oxides, solvates, and stereoisomers thereof, in accordance with any embodiment described herein; and at least one pharmaceutically acceptable excipient.

Another embodiment of the invention is a pharmaceutical composition comprising an effective amount of at least one compound selected from compounds of Formula (I).

An additional embodiment of the invention is a pharmaceutical composition comprising an effective amount of a compound of Formula I or II (*e.g*., a compound selected from Examples 1-211), or a pharmaceutically acceptable salt, isotope, N-oxide, solvate, or stereoisomer of the compound of Formula I or II; and at least one pharmaceutically acceptable excipient.

Solid oral dosage forms such as, tablets or capsules, containing one or more compounds of the present invention may be administered in at least one dosage form at a time, as appropriate. It is also possible to administer the compounds in sustained release formulations.

Additional oral forms in which the present inventive compounds may be administered include elixirs, solutions, syrups, and suspensions; each optionally containing flavoring agents and coloring agents.

Alternatively, one or more compounds of Formula I or II can be administered by inhalation (intratracheal or intranasal) or in the form of a suppository or pessary, or they may be applied topically in the form of a lotion, solution, cream, ointment or dusting powder. For example, they can be incorporated into a cream comprising, consisting of, and/or consisting essentially of an aqueous emulsion of polyethylene glycols or liquid paraffin. They can also be incorporated, at a concentration of between about 1 % and about 10 % by weight of the cream, into an ointment comprising, consisting of, and/or consisting essentially of a wax or soft paraffin base together with any stabilizers and preservatives as may be required. An alternative means of administration includes transdermal administration by using a skin or transdermal patch.

The pharmaceutical compositions of the present invention (as well as the compounds of the present invention alone) can also be injected parenterally, for example, intracavernosally, intravenously, intramuscularly, subcutaneously, intradermally, or intrathecally. In this case, the compositions will also include at least one of a suitable carrier, a suitable excipient, and a suitable diluent.

For parenteral administration, the pharmaceutical compositions of the present invention are best used in the form of a sterile aqueous solution that may contain other substances, for example, enough salts and monosaccharides to make the solution isotonic with blood.

For buccal or sublingual administration, the pharmaceutical compositions of the present invention may be administered in the form of tablets or lozenges, which can be formulated in a conventional manner.

By way of further example, pharmaceutical compositions containing at least one of the compounds of Formula I or II as the active ingredient can be prepared by mixing the compound(s) with a pharmaceutically acceptable carrier, a pharmaceutically acceptable diluent, and/or a pharmaceutically acceptable excipient according to conventional pharmaceutical compounding techniques. The carrier, excipient, and diluent may take a wide variety of forms depending upon the desired route of administration (e.g., oral, parenteral, etc.). Thus, for liquid oral preparations such as, suspensions, syrups, elixirs and solutions, suitable carriers, excipients and diluents include water, glycols, oils, alcohols, flavoring agents, preservatives, stabilizers, coloring agents and the like; for solid oral preparations such as, powders, capsules, and tablets, suitable carriers, excipients and diluents include starches, sugars, diluents, granulating agents, lubricants, binders, disintegrating agents and the like. Solid oral preparations also may be optionally coated with substances such as, sugars, or be enterically coated so as to modulate the major site of absorption and disintegration. For parenteral administration, the carrier, excipient and diluent will usually include sterile water, and other ingredients may be added to increase solubility and preservation of the composition. Injectable suspensions or solutions may also be prepared utilizing aqueous carriers along with appropriate additives such as, solubilizers and preservatives.

According to particular embodiments, a therapeutically effective amount of a compound of Formula I or II or a pharmaceutical composition thereof may comprise a dose range from about 0.1 mg to about 3000 mg, or any particular amount or range therein, in particular from about 1 mg to about 1000 mg, or any particular amount or range therein, of active ingredient in a regimen of about 1 to about (4x) per day for an average (70 kg) human; although, it is apparent to one skilled in the art that the therapeutically effective amount for a compound of Formula I or II will vary as will the diseases, syndromes, conditions, and disorders being treated.

An embodiment of the present invention is directed to a pharmaceutical composition for oral administration, comprising a compound of Formula I or II in an amount of from about 1 mg to about 500 mg.

Advantageously, a compound of Formula I or II may be administered in a single daily dose, or the total daily dosage may be administered in divided doses of two, three and (4x) daily.

Optimal dosages of a compound of Formula I or II to be administered may be readily determined and will vary with the particular compound used, the mode of administration, the strength of the preparation, and the advancement of the disease, syndrome, condition or disorder. In addition, factors associated with the particular subject being treated, including subject gender, age, weight, diet and time of administration, will result in the need to adjust the dose to achieve an appropriate therapeutic level and desired therapeutic effect. The above dosages are thus exemplary of the average case. There can be, of course, individual instances wherein higher or lower dosage ranges are merited, and such are within the scope of this invention.

Compounds of Formula I or II may be administered in any of the foregoing compositions and dosage regimens or by means of those compositions and dosage regimens established in the art whenever use of a compound of Formula I or II is administered to a subject in need thereof.

According to particular embodiments, the present invention provides one or more compounds of Formula I or II, for use in treating, ameliorating and / or preventing a disease, a syndrome, a condition or a disorder that is affected by the inhibition of DHODH enzymatic activity, wherein the disease, syndrome, condition, or disorder is an inflammatory disorder, an autoimmune disorder, or cancer.

In a further aspect the present invention provides a compound of Formula I or II, or a pharmaceutically acceptable salt, solvate, stereoisomer, isotopic variant, or N-oxide thereof, or a sub-embodiment thereof, for use in treating a subject suffering from or diagnosed with a disease, disorder, or medical condition comprising inhibiting or altering Dihydroorotate Dehydrogenase (DHODH) enzymatic activity, and contacting DHODH with said compound of Formula I or II, or a pharmaceutically acceptable salt, solvate, stereoisomer, isotopic variant, or N-oxide thereof, or a sub-embodiment thereof, thereby inhibiting or otherwise altering DHODH enzymatic activity, wherein the disease, disorder, or medical condition is selected from the group consisting of: inflammatory disorders, autoimmune disorders, and cancer.

An additional embodiment of the present invention provides a compound of Formula I or II, for use in treating diseases, disorders, or medical conditions mediated or otherwise affected by dihydroorotate dehydrogenase (DHODH) enzyme activity, comprising administering said compound of Formula I or II to a subject in need thereof, wherein the disease, disorder, or medical condition is selected from the group consisting of: inflammatory disorders, autoimmune disorders, and cancer.

As used herein, the term "DHODH inhibitor" may refer to an agent that inhibits or reduces DHODH activity.

In one embodiment, the term "therapeutically effective amount" (or "effective amount") refers to the amount of a compound of the present invention that, when administered to a subject, is effective to (1) at least partially alleviate, inhibit, prevent, and/ or ameliorate a condition, or a disorder or a disease (i) mediated by DHODH enzymatic activity; or (ii) associated with DHODH enzymatic activity; or (iii) characterized by activity (normal or abnormal) of DHODH enzyme; or (2) reduce or inhibit the activity of DHODH enzyme; or (3) reduce or inhibit the expression of DHODH; or (4) modify the protein levels of DHODH. Without being bound by a particular theory, DHODH inhibitors are believed to act by inhibiting nucleic acid synthesis, cell cycle arrest or altering post-translational glycosylation of proteins involved in regulating myeloid differentiation within progenitor tumor cells.

An additional embodiment of the invention is at least one compound selected from: compounds of Formula I or II, enantiomers and diastereomers of the compounds of Formula I or II, isotopic variations of the compounds of Formula I or II, and pharmaceutically acceptable salts of all of the foregoing, for use in treating a subject suffering from or diagnosed with a disease, disorder, or medical condition mediated or otherwise affected by DHODH enzymatic activity, comprising administering to a subject in need of such treatment an effective amount of at least one compound selected from: said compounds of Formula I or II, enantiomers and diastereomers of the compounds of Formula I or II, isotopic variations of the compounds of Formula I or II, and pharmaceutically acceptable salts of all of the foregoing, wherein the disease, disorder, or medical condition is selected from the group consisting of: inflammatory disorders, autoimmune disorders, and cancer. Stated another way, according to an embodiment, there is provided at least one compound selected from: compounds of Formula I or II, and pharmaceutically acceptable salts of all the foregoing, for use in treating a subject suffering from or diagnosed with a disease, disorder, or medical condition, such as cancer, comprising administering to the subject an effective amount of at least one compound selected from: said compounds of Formula I or II, and pharmaceutically acceptable salts of all the foregoing (e.g., by inhibiting or otherwise altering dihydroorotate oxygenase enzyme activity in the subject), wherein the disease, disorder, or medical condition is selected from the group consisting of: inflammatory disorders, autoimmune disorders, and cancer.

In another embodiment, inhibitors of DHODH of the present invention may be used for the treatment of immunological diseases including, but not limited to, autoimmune and inflammatory disorders, e.g. arthritis, inflammatory bowel disease, gastritis, ankylosing spondylitis, ulcerative colitis, pancreatitis, Crohn's disease, celiac disease, multiple sclerosis, systemic lupus erythematosus, lupus nephritis, rheumatic fever, gout, organ or transplant rejection, chronic allograft rejection, acute or chronic graft-versus-host disease, dermatitis including atopic, dermatomyositis, psoriasis, Behcet's diseases, uveitis, myasthenia gravis, Grave's disease, Hashimoto thyroiditis, Sjogren's syndrome, blistering disorders, antibody-mediated vasculitis syndromes, immune-complex vasculitides, allergic disorders, asthma, bronchitis, chronic obstructive pulmonary disease (COPD), cystic fibrosis, pneumonia, pulmonary diseases including edema, embolism, fibrosis, sarcoidosis, hypertension and emphysema, silicosis, respiratory failure, acute respiratory distress syndrome, BENTA disease, berylliosis, and polymyositis.

As used herein, unless otherwise noted, the term "affect" or "affected" (when referring to a disease, disorder, or medical condition that is affected by the inhibition or alteration of DHODH enzymatic activity) includes a reduction in the frequency and / or severity of one or more symptoms or manifestations of said disease, syndrome, condition or disorder; and / or includes the prevention of the development of one or more symptoms or manifestations of said disease, syndrome, condition or disorder or the development of the disease, condition, syndrome or disorder.

An additional embodiment of the invention provides a compound of Formula I or II, or a pharmaceutically acceptable salt, isotope, N-oxide, solvate, or stereoisomer thereof, for use in treating cancer comprising administering to a subject in need thereof, a therapeutically effective amount of said compound of Formula I or II, or a pharmaceutically acceptable salt, isotope, N-oxide, solvate, or stereoisomer thereof.

According to an embodiment, the cancer is selected from but not limited to, lymphomas, leukemias, carcinomas, and sarcomas.

An additional embodiment of the invention provides a compound of Formula I or II, or a pharmaceutically acceptable salt, isotope, N-oxide, solvate, or stereoisomer thereof, for use in the treatment of one or more cancer types.

According to particular embodiments, the compound of Formula I or II, or a pharmaceutically acceptable salt, isotope, N-oxide, solvate, or stereoisomer thereof, for use in treatment described herein is directed to the treatment of cancer, wherein the cancer is selected from but not limited to:
leukemias including but not limited to acute lymphoblastic leukemia (ALL), acute myeloid leukemia (AML), (acute) T-cell leukemia, acute monocytic leukemia, acute promyelocytic leukemia (APL), bisphenotypic B myelomonocytic leukemia, chronic myeloid leukemia (CML), chronic myelomonocytic leukemia (CMML), large granular lymphocytic leukemia, plasma cell leukemia, and also myelodysplastic syndrome (MDS), which can develop into an acute myeloid leukemia,
lymphomas including but not limited to AIDS-related lymphoma, Hodgkin lymphoma, non-Hodgkin's lymphoma (NHL), T-non-Hodgkin lymphoma (T-NHL), subtypes of NHL such as Diffuse Large Cell Lymphoma (DLBCL), activated B-cell DLBCL, germinal center B-cell DLBCL, double-hit lymphoma and double-expressor lymphoma; anaplastic large cell lymphoma, marginal B cell lymphoma and primary mediastinal B-cell lymphoma, immunoblastic large cell lymphoma, Burkitt lymphoma, follicular lymphoma, hairy cell leukemia, Hodgkin's disease, mantle cell lymphoma (MCL), lymphoplasmatic lymphoma, precursor B -lymphoblastic lymphoma, lymphoma of the central nervous system, small lymphocytic lymphoma (SLL) and chronic lymphocytic leukemia (CLL); T-cell NHL such as precursor T-lymphoblastic lymphoma/leukemia, peripheral T-cell lymphoma (PTCL), cutaneous T-cell lymphoma (CTCL), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma
sarcomas including but not limited to sarcoma of the soft tissue, gliosarcoma, osteosarcoma, malignant fibrous histiocytoma, lymphosarcoma, and rhabdomyosarcoma;
   and
other cancers, such as solid tumors, including but not limited to breast cancer, colorectal carcinoma, gastric cancer, gliosarcoma, head & neck cancer, hepatocellular carcinoma, lung cancer, multiple myeloma, neuroblastoma, ovarian cancer, pancreatic cancer, prostate cancer, renal cell carcinoma and sarcoma.

In an embodiment, cancers that may benefit from a treatment with inhibitors of DHODH of the present invention include, but are not limited to, lymphomas, leukemias, carcinomas, and sarcomas, e.g. non-Hodgkin's lymphoma, diffuse large B-cell lymphoma (DLBCL), mantle cell lymphoma (MCL), follicular lymphoma (FL), marginal zone lymphoma, T-cell lymphoma, Hodgkin's lymphoma, Burkitt's lymphoma, multiple myeloma, brain (gliomas), glioblastomas, breast cancer, colorectal/colon cancer, prostate cancer, lung cancer including non-small-cell, gastric cancer, endometrial cancer, melanoma, pancreatic cancer, liver cancer, kidney cancer, squamous cell carcinoma, ovarian cancer, sarcoma, osteosarcoma, thyroid cancer, bladder cancer, head & neck cancer, testicular cancer, Ewing's sarcoma, rhabdomyosarcoma, medulloblastoma, neuroblastoma, cervical cancer, renal cancer, urothelial cancer, vulval cancer, esophageal cancer, salivary gland cancer, nasopharangeal cancer, buccal cancer, cancer of the mouth, and GIST (gastrointestinal stromal tumor).

In another embodiment of the present invention, the compounds of the present invention may be employed in combination with one or more other medicinal agents, more particularly with one or more anti-cancer agents, e.g. chemotherapeutic, anti-proliferative or immunomodulating agents, or with adjuvants in cancer therapy, e.g. immunosuppressive or anti-inflammatory agents. Additional non-limiting examples of anti-cancer agents that may be administered in combination with a compound of the present invention include biologic compounds, such as monoclonal antibodies (e.g., that mediate effector function upon binding to cancer cell-associated antigens, or block interaction of a receptor expressed on cancer cells with a soluble or cell bound ligand), bispecific antibodies that mediate immune cell redirection, etc. According to an embodiment, there is provided a compound of Formula I or II, or a pharmaceutically acceptable salt, isotope, N-oxide, solvate, or stereoisomer thereof, and an effective amount of one or more additional anti-cancer agents, for use in treating cancer wherein the compound of the present invention and the additional anti-cancer agent(s) are administered either simultaneously (e.g., as part of the same pharmaceutical composition) or sequentially. According to an embodiment, a pharmaceutical composition comprises an effective amount of a compound of the present invention (e.g., selected from the compounds described in Examples 1-211, pharmaceutically acceptable salts, isotopes, N-oxides, solvates, and stereoisomers thereof), an effective amount of one or more additional anti-cancer agents, and optionally one or more excipients.

An additional embodiment of the invention provides a compound of Formula I or II, or pharmaceutically acceptable salts, isotopes, N-oxides, solvates, or stereoisomers thereof, for use in the treatment of cancers, lymphomas and leukemias alone or in combination with classic antitumoral compounds, as part of chemotherapeutic regimens, well known by the one skilled in the art.

### DEFINITIONS

As used herein, the following terms are intended to have the following meanings (additional definitions are provided where needed throughout the Specification):
The term **"C*_{a-b}*"** (where *a* and *b* are integers referring to a designated number of carbon atoms) refers to an alkyl, alkenyl, alkynyl, alkoxy or cycloalkyl radical or to the alkyl portion of a radical in which alkyl appears as the prefix root containing from *a* to *b* carbon atoms inclusive. For example, C₁₋₄ denotes a radical containing 1, 2, 3 or 4 carbon atoms.

The term **"alkyl,"** whether used alone or as part of a substituent group, refers to a saturated branched or straight chain monovalent hydrocarbon radical, wherein the radical is derived by the removal of one hydrogen atom from a single carbon atom. Unless specifically indicated (e.g. by the use of a limiting term such as "terminal carbon atom"), substituent variables may be placed on any carbon chain atom. Typical alkyl radicals include, but are not limited to, methyl, ethyl, propyl, isopropyl and the like. Examples include C₁₋₈alkyl, C₁₋₆alkyl and C₁₋₄alkyl groups.

The term **"substituted,"** refers to a core molecule on which one or more hydrogen atoms have been replaced with one or more functional radical moieties. Substitution is not limited to a core molecule, but may also occur on a substituent radical, whereby the substituent radical becomes a linking group.

The term **"independently selected"** refers to one or more substituents selected from a group of substituents, wherein the substituents may be the same or different.

The atom label **"D"** refers to deuterium. Deuterium is an isotope of hydrogen, which has one proton and one neutron.

The substituent nomenclature used in the disclosure of the present invention was derived by first indicating the atom having the point of attachment, followed by the linking group atoms toward the terminal chain atom from left to right, substantially as in:

(C₁₋₆)alkylC(O)NH(C₁₋₆)alkyl(Ph)

or by first indicating the terminal chain atom, followed by the linking group atoms toward the atom having the point of attachment, substantially as in:

Ph(C₁₋₆)alkylamido(C₁₋₆)alkyl

either of which refers to a radical of the formula:

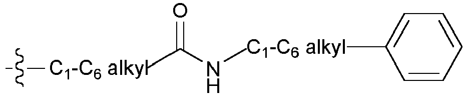

Lines drawn into ring systems from substituents indicate that the bond may be attached to any of the suitable ring atoms.

When any variable (e.g. R_{d}) occurs more than one time in any embodiment of Formula I or Formula II, each definition is intended to be independent.

The terms "comprising", "including", and "containing" are used herein in their open, non-limited sense.

### GENERAL SYNTHETIC METHODS

Exemplary compounds useful in methods of the invention will now be described by reference to the illustrative synthetic schemes for their general preparation below and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Unless otherwise specified, the variables are as defined above in reference to Formula (I). Reactions may be performed between the melting point and the reflux temperature of the solvent, and preferably between 0 °C and the reflux temperature of the solvent. Reactions may be heated employing conventional heating or microwave heating. Reactions may also be conducted in sealed pressure vessels above the normal reflux temperature of the solvent.

Abbreviations used in the instant specification, particularly the schemes and examples, are as follows:
- ACN or MeCN: acetonitrile
- Ad₂nBuP-Pd-G₃: cataCXium-A-Pd-G3
- aq.: aqueous
- BINAP: (2,2'-bis(diphenylphosphino)-1,1'-binaphthyl)
- *n*-BuLi: n-butyllithium
- calcd.: calculated
- DAST: diethylaminosulfur trifluoride
- DIEA, DIPEA: *N*,*N*-diisopropylethylamine
- DCE: 1,2-dichloroethane
- DCM: dichloromethane
- DME: dimethoxyethane
- DMF: *N*,*N*-dimethylformamide
- DMP: Dess-Martin periodinane; 3-oxo-1,3-dihydro-1λ⁵,2-benziodoxole-1,1,1 -triyl triacetate
- DMSO: dimethylsulfoxide
- dppf: 1,1'-bis(diphenylphosphino)ferrocene
- EtOAc: ethyl acetate
- ESI: electrospray ionization
- Et: ethyl
- h or hr(s): hour or hours
- HPLC: high performance liquid chromatography
- LiHMDS: lithium bis(trimethylsilyl)amide
- Me: methyl
- MeOH: methanol
- MHz: megahertz
- min: minute or minutes
- MS: mass spectrometry
- NBS: *N*-bromosuccinimide
- NMR: nuclear magnetic resonance
- NMO: *N*-methylmorpholine-*N*-oxide
- OAc: acetate
- Py: pyridine
- RP: reverse-phase
- rt or RT: room temperature
- Rₜ: retention time
- Sec: second or seconds
- S-Phos Pd G3: (2-dicyclohexylphosphino-2',6'-dimethoxybiphenyl) [2-(2'-amino-1,1'-biphenyl)]palladium(II) methanesulfonate
- TBAF: tetrabutylammonium fluoride
- TBS: tert-Butyldimethylsilyl
- TEA: triethylamine
- TFA: trifluoroacetic acid
- Tf₂O: triflic anhydride
- THF: tetrahydrofuran
- TLC: thin layer chromatography
- T₃P: propylphosphonic anhydride
- XPhos Pd G3: (2-Dicyclohexylphosphino-2',4',6'-triisopropyl-1,1'-biphenyl)[2-(2'-amino-1,1'-biphenyl)]palladium(II) methanesulfonate

### PREPARATIVE EXAMPLES

Exemplary compounds useful in methods of the invention will now be described by reference to the illustrative synthetic schemes for their general preparation below and the specific examples to follow. 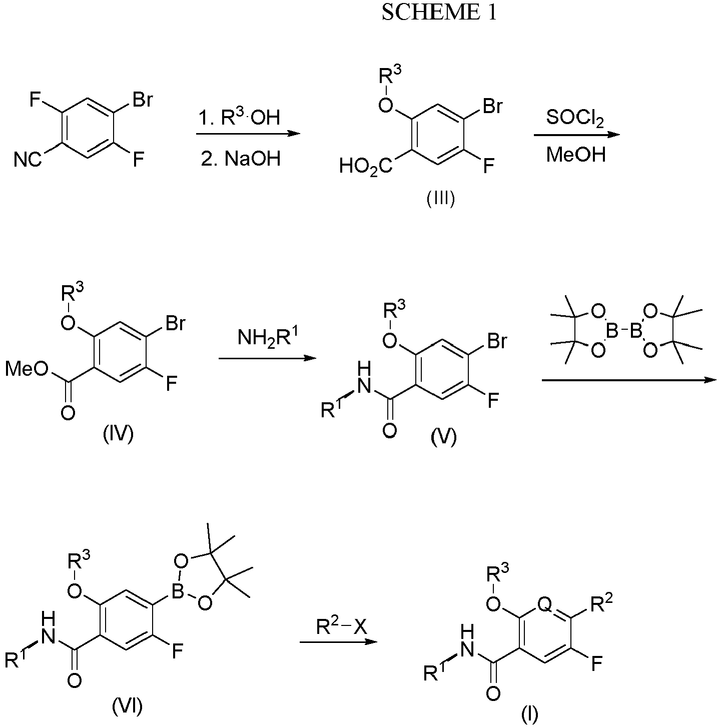 where
R¹, R², and R³ are as defined in Formula I
Q is CH
and X is a halogen or triflate

Compounds of Formula I where Q is CH may be made according to the procedure outlined in Scheme 1. Starting material 4-bromo-2,5-difluorobenzonitrile is treated with an alcohol of formula R³CH(OH)CH₃, where R³ is as defined in Formula I, and a suitable base such as K₂CO₃, Na₂CO₃, Cs₂CO₃, NaH, KHMDS, LiHMDS, or NaHMDS, preferably K₂CO₃, in a suitable solvent such as THF, DMF, or ACN, preferably DMF to give an aryl ether. Subsequent hydrolysis of the cyano group in the presence of a base such as NaOH, LiOH, NaOH, preferably NaOH in a solvent such as EtOH, THF, DMF, water, or ACN, or a mixture thereof, preferably in EtOH affords an aryl acid of Formula (III). Further reaction with thionyl chloride, oxalyl chloride, or the like, in a solvent such as MeOH provides a methyl ester of the Formula (IV).

Amide (V) is prepared by reaction of amine NH₂R¹, where R¹ is as defined in Formula I, with a suitable base, such as LiHMDS, at 0 °C for 30 min followed by treatment with 4-bromo-5-fluoro-*N*-methyl-2-((1,1,1-trifluoropropan-2-yl)oxy)benzamide at rt for 5 to 24 hrs. The amide of Formula (V) is reacted with a boron donor, such as 4,4,4',4',5,5,5',5'-octamethyl-2,2'-bi(1,3,2-dioxaborolane) and a palladium catalyst such as Pd(dppf)Cl₂ or the like, in the presence of a base such as KOAc or the like, in a solvent such as dioxane at 80 °C for 3 to 24 hrs to provide a compound of Formula (VI). Further reaction with R²-X, where R² is as defined in Formula I, and X is a leaving group, such as Cl, Br, I, or OTf, in the presence of a catalyst such as Pd(dppf)Cl₂, Ad₂nBuP-Pd-G3, Xphos-Pd-G3, or the like, in the presence of a base such as Na₂CO₃, K₂CO₃, K₃PO₄, NaOH or the like, in a solvent such as THF, dioxane, water or mixtures thereof at 50-90 °C for 2hr to 24 hrs provides a compound of Formula I, where Q is CH. 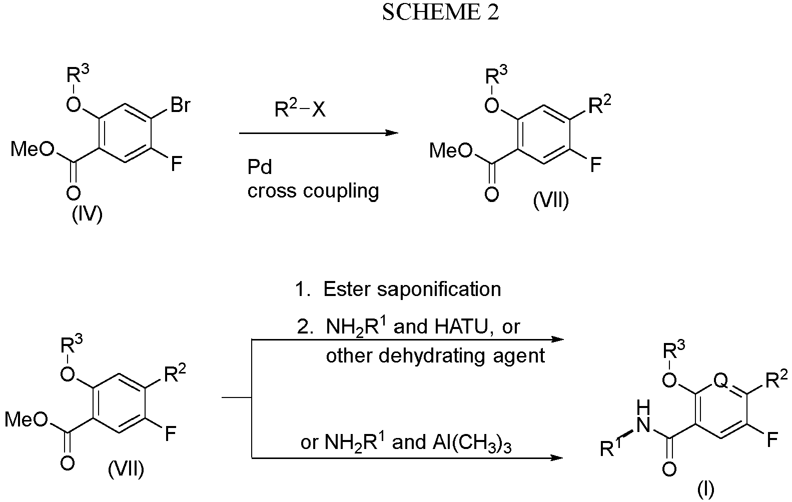 where
R¹, R², and R³ are as defined in Formula I
Q is CH
and X is a halogen or triflate

Compounds of Formula (I), where Q is CH, may alternatively be prepared according to Scheme 2. Compounds of Formula (IV) can be reacted with R²-X, where R² is as defined in Formula I, and X is a leaving group, such as Cl, Br, I, or OTf, in the presence of a catalyst such as Pd(dppf)Cl₂, Ad₂nBuP-Pd-G3, Xphos-Pd-G3, or the like, in the presence of a base such as Na₂CO₃, K₂CO₃, K₃PO₄, NaOH or the like, in a solvent such as THF, dioxane, water or mixtures thereof at 50-90 °C for 2hr to 24 hrs to provide a compound of Formula (VII). A compound of Formula (VII) may be converted to a compound of Formula (I) where Q is CH. One approach involves ester saponification, followed by activation with T3P, HOBt, TCFH, HATU, POCl₃ or the like followed by reaction with NH₂-R¹ in the presence of a base such as TEA, DIPEA, pyridine, N-methylimidazole, in a solvent such as THF, DCM, DMF, ACN or the like. Alternatively, an amine compound, NH₂-R¹, is combined with trimethylaluminum; in a solvent such as toluene, dichloromethane, and the like; for a period of 15 minutes to 4 hours, preferably 30 min; and then subsequently treated with a compound of (VII) to obtain a compound of Formula (I) where Q is CH. 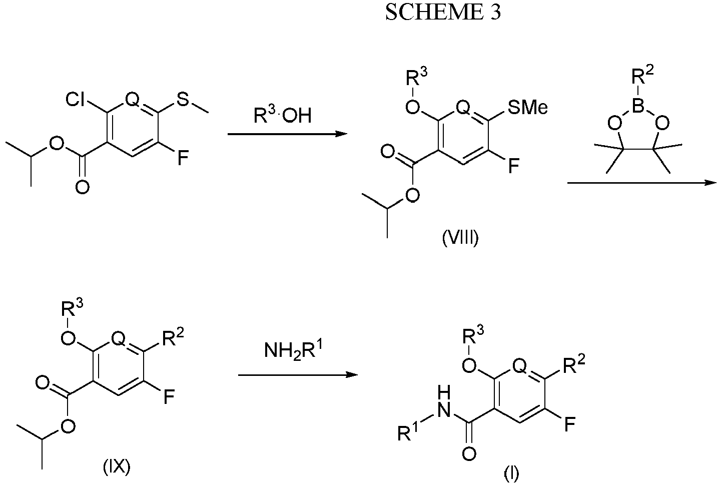 where
R¹, R², and R³ are as defined in Formula I
Q is CH or N

Compounds of Formula (I), where Q is N or CH, may alternatively be prepared according to Scheme 3. Isopropyl 2-chloro-5-fluoro-6-(methylthio)nicotinate can be reacted with R³CH(OH)CH₃ in the presence of a base such as Cs₂CO₃, K₂CO₃, Na₂CO₃, and a catalyst, such as methanesulfonato(2-dicyclohexylphosphino-2',4',6'-tri-i-propyl-1, 1'-biphenyl)(2'-amino-1, 1'-biphenyl-2-yl)palladium(II), XPhos-Pd, or the like, in a solvent such as toluene, THF, DMF, or the like, at 90 °C for 18 to 24 hrs to yield a compound of the general formula (VIII). A compound of the general formula (VIII) can be reacted with a heteroaryl boronic ester or boronic acid, for example a compound of the formula 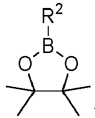 where R² is as defined in Formula (I), in the presence of a copper source such as copper (I) 3-methylsalicylate and catalytic palladium catalyst such as tetrakis(triphenylphosphine)palladium in a solvent such as THF, DMF, dioxane, ACN or the like at 100 °C for 1 hr to yield a compound of Formula (IX). An ester of Formula (IX) can be reacted with an amine, NH-R¹ directly, as described in SCHEME 2 or converted to the carboxylic acid and then reacted with the amine, NH-R¹, as described in SCHEME 2, to provide a compound of Formula (I).

Custom R^{a} substituted R² building blocks, used in Schemes 1-3, may be purchased or prepared as shown in Schemes 4-9.

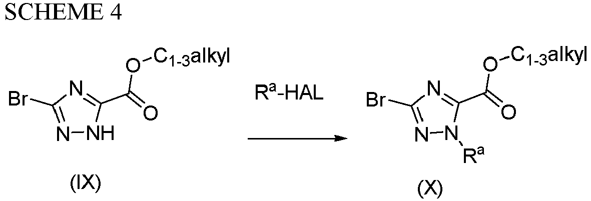

According to SCHEME 4, a compound of formula (IX), R^{a} is C₁₋₆alkyl or CD₃, is prepared from a commercially available or synthetically accessible compound of formula (X), by employing an alkyl halide of formula R^{a}-HAL, where HAL is I or Br, and R^{a} is defined in the claims including C₁₋₆alkyl or CD₃, and the like; a base, such as Cs₂CO₃, K₂CO₃, and the like; in a suitable solvent such as DMF, acetone, DMSO, and the like; at ambient temperature.

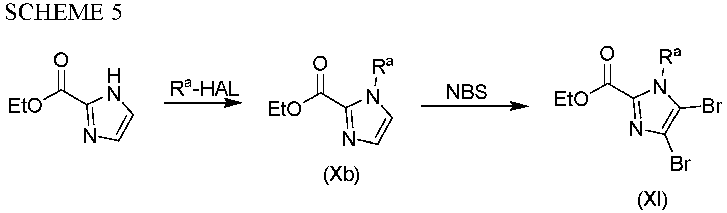

According to Scheme 5, commercially available or synthetically accessible ethyl 1H-imidazole-2-carboxylate is alkylated employing a base such as Cs₂CO₃, K₂CO₃, and the like; an alkylating agent such as formula R^{a}-HAL, where HAL is I or Br, and R^{a} is defined in the claims including C₁₋₆alkyl or CD₃; in a suitable solvent such as DMF, acetone, DMSO and the like; to provide a compound of the formula Xb. A compound of the formula Xb is brominated employing bromination conditions known to one skilled in the art, for example, using a suitable brominating agent such as NBS, and the like; in a suitable solvent such as DMF, to afford a compound of formula (XI), where R^{a} is defined in the claims including C₁₋₆alkyl or CD₃.

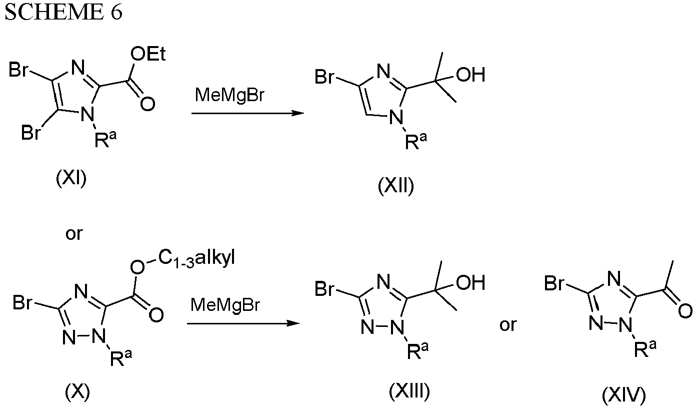

According to SCHEME 6, a commercially available or synthetically accessible ester of formula (XI), where R^{a} is C₁₋₆alkyl or CD₃ or defined in the claims, is reacted with a Grignard reagent, such as MeMgBr; in a suitable solvent such as THF, ether, 1,4-dioxane and the like; at low temperatures, preferably 0 °C; to provide a compound of formula (XII), where R^{a} is C₁₋₆alkyl or CD₃. In a similar fashion, a compound of formula (X), where R^{a} is C₁₋₆alkyl or CD₃; is reacted with a Grignard reagent such as MeMgBr, to provide a compound of formula (XIII). It is noted that under certain controlled conditions, only a single addition occurs to provide a compound of formula (XIV), where R^{a} is C₁₋₆alkyl.

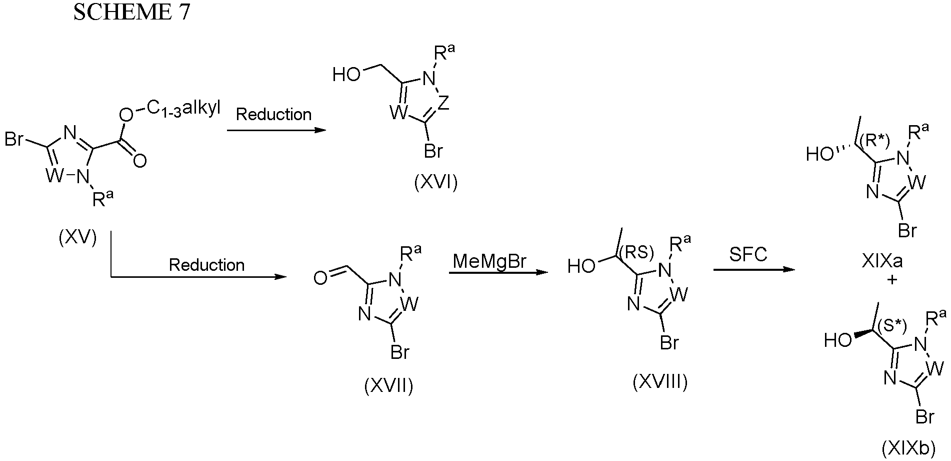

According to SCHEME 7, a commercially available or synthetically accessible ester compound of formula (XV) where R^{a} is C₁₋₆alkyl, and W is CH or N, is reduced employing conditions known to one skilled in the art. For example, treatment of a compound of formula (XV) where R^{a} is C₁₋₆alkyl, and W is CH or N; with a suitable reducing agent such as NaBH₄, and the like; an additive such as CaCl₂, and the like; in a suitable solvent such as EtOH, MeOH, and the like; at ambient temperatures provides a compound of formula (XVI). A commercially available or synthetically accessible ester compound of formula (XV) where R^{a} is C₁₋₆alkyl or CD₃, and W is CH or N, is reduced to the aldehyde compound of formula (XVII) employing conditions known to one skilled in the art. For example, a compound of formula (XV) where R^{a} is C₁₋₆alkyl or CD₃, and W is CH or N is reacted with a reducing agent such as DIBALH; in a suitable solvent, such as DCM, DCE, and the like; at low temperatures such as -78 °C to rt; to provide a compound of formula (XVII). A compound of formula (XVII) is reacted with Grignard reagent, such as MeMgBr, employing conditions previously described to provide a compound of formula (XVIII) as a racemic mixture of compounds. The racemic mixture of compounds is separated by SFC to provide the secondary alcohols of formulas (XIXa) and (XIXb) in enantiopure form.

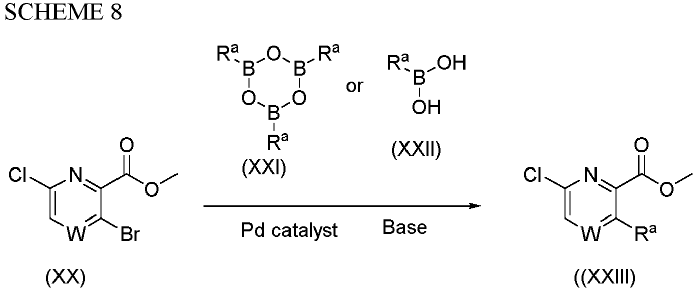

Compounds of Formula (XXIII), where W is CH or N, may be prepared according to Scheme 8. Compounds of Formula (XX) can be reacted with Compounds of Formula XXI or XXII, where R^{a} is as defined in the claims, in the presence of a catalyst such as Pd(dppf)Cl₂, Ad₂nBuP-Pd-G3, Xphos-Pd-G3, or the like, in the presence of a base such as Na₂CO₃, K₂CO₃, K₃PO₄, NaOH or the like, in a solvent such as DME, THF, dioxane, water or mixtures thereof to provide a compound of Formula (XXIII).

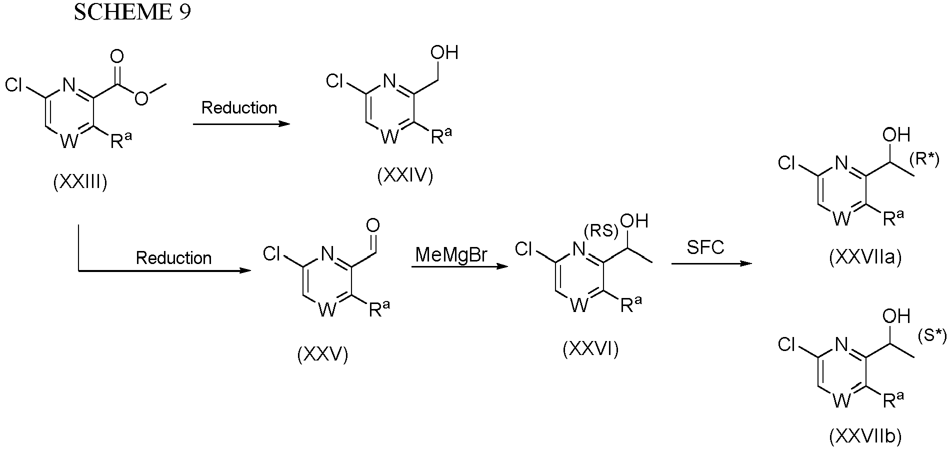

According to SCHEME 9, a commercially available or synthetically accessible ester compound of formula (XXIII) where R^{a} is C₁₋₆alkyl, and W is CH or N, is reduced employing conditions known to one skilled in the art. For example, treatment of a compound of formula (XXIII) where R^{a} is C₁₋₆alkyl, and W is CH or N; with a suitable reducing agent such as NaBH₄, LAH, and the like; an additive such as CaCl₂, and the like; in a suitable solvent such as THF, EtOH, MeOH, and the like; at ambient temperatures provides a compound of formula (XXIV). A commercially available or synthetically accessible ester compound of formula (XXIII) where R^{a} is C₁₋₆alkyl or CD₃, and W is CH or N, is reduced to the aldehyde compound of formula (XXV) employing conditions known to one skilled in the art. For example, a compound of formula (XXIII) where R^{a} is C₁₋₆alkyl or CD₃, and W is CH or N is reacted with a reducing agent such as DIBALH; in a suitable solvent, such as DCM, DCE, and the like; at low temperatures such as -78 °C to rt; to provide a compound of formula (XXV). A compound of formula (XXV) is reacted with Grignard reagent, such as MeMgBr, employing conditions previously described to provide a compound of formula (XXVI) as a racemic mixture of compounds. The racemic mixture of compounds is separated by SFC to provide the secondary alcohols of formulas (XXVIIa) and (XXVIIb) in enantiopure form.

Compounds of Formula (I) may be converted to their corresponding salts using methods known to one of ordinary skill in the art. For example, an amine of Formula (I) is treated with trifluoroacetic acid, HCl, or citric acid in a solvent such as Et₂O, CH₂Cl₂, THF, MeOH, chloroform, or isopropanol to provide the corresponding salt form. Alternately, trifluoroacetic acid or formic acid salts are obtained as a result of reverse phase HPLC purification conditions. Cyrstalline forms of pharmaceutically acceptable salts of compounds of Formula (I) may be obtained in crystalline form by recrystallization from polar solvents (including mixtures of polar solvents and aqueous mixtures of polar solvents) or from non-polar solvents (including mixtures of non-polar solvents).

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention.

Compounds prepared according to the schemes described above may be obtained as single forms, such as single enantiomers, by form-specific synthesis, or by resolution. Compounds prepared according to the schemes above may alternately be obtained as mixtures of various forms, such as racemic (1:1) or non-racemic (not 1:1) mixtures. Where racemic and non-racemic mixtures of enantiomers are obtained, single enantiomers may be isolated using conventional separation methods known to one of ordinary skill in the art, such as chiral chromatography, recrystallization, diastereomeric salt formation, derivatization into diastereomeric adducts, biotransformation, or enzymatic transformation. Where regioisomeric or diastereomeric mixtures are obtained, as applicable, single isomers may be separated using conventional methods such as chromatography or crystallization.

The following specific examples are provided to further illustrate the invention and various preferred embodiments.

### EXAMPLES

In obtaining the compounds described in the examples below and the corresponding analytical data, the following experimental and analytical protocols were followed unless otherwise indicated.

Unless otherwise stated, reaction mixtures were magnetically stirred at room temperature (rt) under a nitrogen atmosphere. Where solutions were "dried," they were generally dried over a drying agent such as Na₂SO₄ or MgSO₄. Where mixtures, solutions, and extracts were "concentrated", they were typically concentrated on a rotary evaporator under reduced pressure.

Normal-phase silica gel chromatography (FCC) was performed on silica gel (SiO₂) using prepacked cartridges.

Preparative reverse-phase high performance liquid chromatography (RP HPLC) was performed on either:
METHOD A. A Gilson GX-281 semi-prep-HPLC with Phenomenex Synergi C18(10µm, 150 × 25mm), or Boston Green ODS C18(5µm, 150 × 30mm), and mobile phase of 5-99% ACN in water (with 0.225%FA) over 10 min and then hold at 100% ACN for 2 min, at a flow rate of 25 mL/min.
   or
METHOD B. A Gilson GX-281 semi-prep-HPLC with Phenomenex Synergi C18(10µm, 150 × 25mm), or Boston Green ODS C18(5µm, 150 × 30mm), and mobile phase of 5-99% ACN in water (0.1%TFA) over 10 min and then hold at 100% ACN for 2 min, at a flow rate of 25 mL/min.
   or
METHOD C. A Gilson GX-281 semi-prep-HPLC with Phenomenex Synergi C18(10µm, 150 × 25mm), or Boston Green ODS C18(5µm, 150 × 30mm), and mobile phase of 5-99% ACN in water (0.05%HCl) over 10 min and then hold at 100% ACN for 2 min, at a flow rate of 25 mL/min.
   or
METHOD D. a Gilson GX-281 semi-prep-HPLC with Phenomenex Gemini C18 (10µm, 150 × 25mm), AD(10µm, 250mm × 30mm), or Waters XBridge C18 column (5µm, 150 × 30mm), mobile phase of 0-99% ACN in water (with 0.05% ammonia hydroxide v/v) over 10 min and then hold at 100% ACN for 2 min, at a flow rate of 25 mL/min.
   or
METHOD E. a Gilson GX-281 semi-prep-HPLC with Phenomenex Gemini C18 (10µm, 150 × 25mm), or Waters XBridge C18 column (5µm, 150 × 30mm), mobile phase of 5-99% ACN in water(10mM NH₄HCO₃) over 10 min and then hold at 100% ACN for 2 min, at a flow rate of 25 mL/min.
   or
METHOD F. Teledyne ISCO ACCQPrep HP150 semi-prep-HPLC with Phenomenex Gemini-NX C18 (5 µm, 150 × 30 mm), mobile phase of 10-100 % ACN in water(10mM NH₄OH) over 10 min and then hold at 100% ACN for 2 min, at a flow rate of 30 mL/min.

Preparative supercritical fluid high performance liquid chromatography (SFC) was performed either on a Thar 80 Prep-SFC system, or Waters 80Q Prep-SFC system from Waters. The ABPR was set to 100bar to keep the CO₂ in SF conditions, and the flow rate may verify according to the compound characteristics, with a flow rate ranging from 50g/min to 70g/min. The column temperature was ambient temperature

Mass spectra (MS) were obtained on a SHIMADZU LCMS-2020 MSD or Agilent 1200\G6110A MSD using electrospray ionization (ESI) in positive mode unless otherwise indicated. Calculated (calcd.) mass corresponds to the exact mass.

Nuclear magnetic resonance (NMR) spectra were obtained on Bruker model AVIII 400 spectrometers. Definitions for multiplicity are as follows: s = singlet, d = doublet, t= triplet, q = quartet, m = multiplet, br = broad. It will be understood that for compounds comprising an exchangeable proton, said proton may or may not be visible on an NMR spectrum depending on the choice of solvent used for running the NMR spectrum and the concentration of the compound in the solution.

Chemical names were generated using ChemDraw Ultra 12.0, ChemDraw Ultra 14.0 (CambridgeSoft Corp., Cambridge, MA) or ACD/Name Version 10.01 (Advanced Chemistry).

Compounds designated as *R** or *S** are enantiopure compounds where the absolute configuration was not determined.

### Intermediate 1: (S)-N-(2-Chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide.

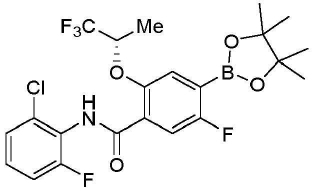

### Step A. (S)-4-bromo-5-fluoro-2-((1,1,1-trifluoropropan-2-yl)oxy)benzonitrile

To a solution of 4-bromo-2,5-difluorobenzonitrile (11 g, 50.5 mmol) in DMF (150 mL) was added K₂CO₃ (20.2 g, 146 mmol), and then added (S)-1,1,1-trifluoropropan-2-ol (6.33 g, 55.5 mmol) dropwise. The reaction mixture was diluted with water (1 L) and extracted with ethyl acetate (500 mL ×3). The combined organic layers were washed with brine (400 mL × 2), dried over Na₂SO₄, filtered and concentrated under reduced pressure to give the residue. The residue was purified by column chromatography (SiO₂, petroleum ether/ethyl acetate =1/0 to 20/1) to give title compound as white solid. ¹H NMR (400 MHz, CDCl₃) δ 7.36 (d, *J* = 7.3 Hz, 1H), 7.29 (d, *J* = 5.4 Hz, 1H), 4.67 (m, 1H), 1.62 (d, *J* = 6.5 Hz, 3H).

### Step B. (S)-4-bromo-5-fluoro-2-((1,1,1-trifluoropropan-2-yl)oxy)benzoic acid

To a solution of (S)-4-bromo-5-fluoro-2-((1,1,1-trifluoropropan-2-yl)oxy)benzonitrile (13 g, 41.7 mmol) in EtOH (52 mL) was added a solution of NaOH (6.23 g, 156 mmol) in H₂O (78 mL) under N₂ atmosphere. The mixture was stirred at 90 °C for 16 hr under N₂ atmosphere. The reaction mixture was quenched by addition ice HCl (1N, 200 mL) at 0 °C, and then extracted with ethyl acetate (250 mL × 2). The combined organic layers were washed with brine, dried over Na₂SO₄, filtered and concentrated in vacuo to give the title compound as a brown solid. MS (ESI): mass calcd. For C₁₀H₇BrF₄O₃, 330.0/332.0; m/z found, 331.0/333.0 [M+H]⁺. ¹H NMR (400 MHz, CDCl₃) δ 7.88 (d, *J* = 8.4 Hz, 1H), 7.30 (d, *J* = 5.2 Hz, 1H), 4.82 - 4.74 (m, 1H), 1.60 (d, *J* = 6.4 Hz, 3H)

### Step C. (S)-methyl 4-bromo-5-fluoro-2-((1,1,1-trifluoropropan-2-yl)oxy)benzoate

To a solution of (S)-4-bromo-5-fluoro-2-((1,1,1-trifluoropropan-2-yl)oxy)benzoic acid (11.5 g, 34.7 mmol) in MeOH (150 mL) was dropwise added SOCl₂ (8.27g, 69.5 mmol, 5.0 mL) at -10 °C for 15 min. The mixture was stirred at 70 °C for 5 h. The reaction mixture was concentrated in vacuo. The residue was diluted with H₂O (100 mL) and extracted with DCM (100 mL ×2). The combined organic layers were washed with brine (50 mL), dried over anhydrous Na₂SO₄, filtered and concentrated in vacuo. The residue was purified by column chromatography (SiO₂, petroleum ether/ethyl acetate=1/0 to 50/1) to give the title compound as white oil. MS (ESI): mass calcd. For C₁₁H₉BrF₄O₃, 344.0/346.0; m/z found, 345.4/347.4 [M+H]⁺. ¹H NMR (400 MHz, CDCl₃) δ 7.63 (d, *J* = 8.6 Hz, 1H), 7.29 (d, *J* = 1.5 Hz, 1H), 4.64 - 4.61 (m, 1H), 3.92 (s, 3H), 1.59-1.55 (m, 3H)

### Step D. (S)-4-bromo-N-(2-chloro-6-fluorophenyl)-5-fluoro-2-((1,1,1-trifluoropropan-2-yl)oxy)benzamide

To a mixture of 2-chloro-6-fluoroaniline (8.44 g, 57.96 mmol) in THF (60 mL) was added LiHMDS (1 M in THF, 43.47 mL) at 0 °C , then the mixture was stirred at 0 °C for 0.5 hr. (S)-methyl 4-bromo-5-fluoro-2-((1,1,1-trifluoropropan-2-yl)oxy)benzoate (5 g, 14.49 mmol) in THF(60 mL) was added to the mixture and warmed to 25 °C and stirred for 5 hr. The reaction mixture was poured into HCl (1M, 250 mL) and extracted with ethyl acetate (300 mL×2). The combined organic phase was dried with anhydrous Na₂SO₄, concentrated in vacuo to give the crude product. The residue was purified by column chromatography (SiO₂, petroleum ether/ethyl acetate=100/1 to 30/1) to give the title compound as a white solid. MS (ESI): mass calcd For C₁₆H₁₀BrClF₅NO₂, 457.0/459.0; m/z found, 458.0/460.0 [M+H]⁺.

### Step E. (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide

To a mixture of (S)-4-bromo-N-(2-chloro-6-fluorophenyl)-5-fluoro-2-((1,1,1-trifluoropropan-2-yl)oxy)benzamide (3.3 g, 7.20 mmol), 4,4,4',4',5,5,5',5'-octamethyl-2,2'-bi(1,3,2-dioxaborolane) (2.74 g, 10.8 mmol), KOAc (1.41 g, 14.4 mmol) in dioxane (60 mL) was added Pd(dppf)Cl₂ (526 mg, 719 µmol) under N₂. The mixture was stirred at 80 °C for 16 hr under N₂ atmosphere. The mixture was poured into water (200 mL) and extracted with ethyl acetate (100 mL×2). The combined organic phase was washed with brine (150 mL×2), dried with anhydrous Na₂SO₄, filtered and concentrated in vacuo. The residue was purified by column chromatography (SiO₂, petroleum ether/ethyl acetate=50/1 to 5/1) to give the title compound as a brown oil. MS (ESI): mass calcd for C₂₂H₂₂BClF₅NO₄, 505.1; m/z found, 506.1/424.3 [M+H]⁺.

### Intermediate 2: (S)-methyl 5-fluoro-4-(4,4,5,5-tetramethyl-1,3,2-dioxaborolan-2-yl)-2-((1,1,1-trifluoropropan-2-yl)oxy)benzoate.

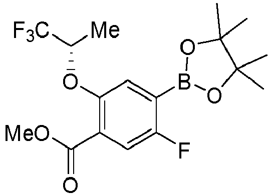

To a mixture of (S)-methyl 4-bromo-5-fluoro-2-((1,1,1-trifluoropropan-2-yl)oxy)benzoate (2 g, 5.80 mmol) , 4,4,5,5-tetramethyl-2-(4,4,5,5-tetramethyl-1,3,2-dioxaborolan-2-yl)-1,3,2-dioxaborolane (2.21 g, 8.69 mmol) and KOAc (1.14 g, 11.59 mmol) in dioxane (40 mL) was added Pd(dppf)Cl₂ (424.07 mg, 579.57 µmol) under N₂. The mixture was stirred at 80 °C for 16 h. The reaction mixture was quenched by addition 1 N HCl (60 mL) at 0 °C, and extracted with ethyl acetate (80 mL). The organic layer was washed with brine (60 mL), dried over Na₂SO₄, filtered and concentrated in vacuo. The residue was purified by column chromatography (SiO₂, petroleum ether/ethyl acetate=1/0 to 30/1) to give the title compound as colorless oil. ¹H NMR (400MHz, CDCl₃) δ = 7.47 (d, *J* = 8.8 Hz, 1H), 7.34 (d, *J* = 4.4 Hz, 1H), 4.76 - 4.62 (m, 1H), 3.90 (s, 3H), 1.54 (d, *J* = 6.5 Hz, 3H), 1.38 (s, 12H).

### Example 1: (S)-N-(2-chloro-6-fluorophenyl)-4-(5-cyano-1-ethyl-2-methyl-1H-imidazol-4-yl)-5-fluoro-2-(1,1,1-trifluoropropan-2-yl)oxy)benzamide

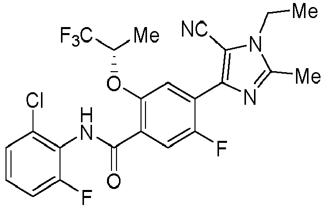

### Step A. 4,5-dibromo-2-methyl-1H-imidazole

A solution of Br₂ (4.87 g, 30.4 mmol, 1.57 mL) in DMF (2 ml) was added to a stirred mixture of 2-methyl-1H-imidazole (1.00 g, 12.2 mmol) and KHCO₃ (3.05 g, 30.5 mmol) in DMF (10 ml) at an ambient temperature. The mixture was stirred at 70 °C for 3 h. The mixture was poured into NH₃•H₂O (100 mL). The aqueous phase was extracted with ethyl acetate (150 mL×5). The combined organic phase was dried with anhydrous Na₂SO₄, concentrated in vacuum. The residue was purified by column chromatography (SiO₂, petroleum ether/ethyl acetate=1/0 to 1/1) to give the title compound as a white solid. MS (ESI): mass calcd for C₄H₄Br₂N₂, 237.9/239.9; m/z found, 238.9/240.9 [M+H]⁺. ¹H NMR (400 MHz, DMSO-*d*₆) δ = 13.85 (s, 1H), 2.24 (s, 3H).

### Step B. 4,5-dibromo-1-ethyl-2-methyl-1H-imidazole

A solution of EtI (1.02 g, 6.57 mmol, 525.11 µL) in DMF (5 ml) was added to a stirred mixture of the 4,5-dibromo-2-methyl-1H-imidazole (1.5 g, 6.25 mmol) and K₂CO₃ (1.21 g, 8.75 mmol) in DMF (20 ml). The stirred mixture was heated at 70 °C for 2 h. The reaction mixture was quenched by addition water (100 mL×2) at 0 °C, and then extracted with ethyl acetate (150 mL). The combined organic layers were washed with brine (300 mL), dried over Na₂SO₄, filtered and concentrated in vacuo. The residue was purified by column chromatography (SiO₂, petroleum ether/ethyl acetate=1/0 to 2/1) to give the title compound as a yellow oil. MS (ESI): mass calcd for C₆H₈Br₂N₂, 265.9/267.9; m/z found, 267.0/269.0 [M+H]⁺. ¹H NMR (400 MHz, CDCl₃) δ = 3.98 - 3.92 (m, 2H), 2.42 (s, 3H), 1.33 - 1.29 (m, 3H).

### Step C. 4-bromo-1-ethyl-2-methyl-1H-imidazole-5-carbaldehyde

A solution of 4,5-dibromo-1-ethyl-2-methyl-1H-imidazole (1.6 g, 5.97 mmol) in THF (20 mL) was cooled to -78 °C under nitrogen atmosphere. A solution of n-BuLi (2.5 M in hexane, 2.39 mL) added dropwise over 30 min. The mixture was then stirred at -78 °C for 1 h. DMF (1.31 g, 17.91 mmol, 1.38 mL) was added dropwise over 30 min. The reaction mixture was stirred at - 78 °C for 60 min. The mixture was poured into 1 N HCl (100 mL). The aqueous phase was extracted with ethyl acetate (150 mL×5). The combined organic phase was dried with anhydrous Na₂SO₄, concentrated in vacuo to give the title compound as yellow oil. ¹H NMR (400MHz, CDCl₃) δ = 9.68 (s, 1 H), 4.33 - 4.28 (m, 2 H), 2.45 (s, 3 H), 1.36 - 1.32 (m, 3 H).

### Step D. 4-bromo-1-ethyl-2-methyl-1H-imidazole-5-carbonitrile

A mixture of 4-bromo-1-ethyl-2-methyl-1H-imidazole-5-carbaldehyde (500 mg, 2.30 mmol), T₃P (1.61 g, 2.53 mmol, 1.51 mL, 50% purity), NH₂OH•HCl (176.08 mg, 2.53 mmol) and TEA(699.27 mg, 6.91 mmol, 961.85 µL) in DMF (5 mL) was degassed and purged with N₂ for 3 times. The mixture was stirred at 100 °C for 5 hr under N₂ atmosphere. T₃P (732.92 mg, 2.30 mmol, 684.97 µL), NH₂OH•HCl (160.07 mg, 2.30 mmol) was added to the mixture and stirred at 100 °C for 16 hr under N₂ atmosphere. The reaction mixture was quenched by addition water (100 mL×2) at 0 °C, and then extracted with ethyl acetate (150 mL). The combined organic layers were washed with brine (300 mL), dried over Na₂SO₄, filtered and concentrated in vacuo. The residue was purified by column chromatography (SiO₂, petroleum ether/ethyl acetate=1/0 to 8/1) to give the title compound as a yellow oil. MS (ESI): mass calcd for C₇H₈BrN₃, 213.0/215.0; m/z found, 213.9/215.9 [M+H]⁺. ¹H NMR (400MHz, CDCl₃) δ = 4.08 - 4.02 (m, 2 H), 2.45 (s, 3 H), 1.46 - 1.43 (m, 3 H).

### Step E. (S)-N-(2-chloro-6-fluorophenyl)-4-(5-cyano-1-ethyl-2-methyl-1H-imidazol-4-yl)-5-fluoro-2-(1,1,1-trifluoropropan-2-yl)oxy)benzamide.

To a mixture of 4-bromo-1-ethyl-2-methyl-1H-imidazole-5-carbonitrile (25 mg, 116.79 µmol), (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 115 mg), Na₂CO₃ (27 mg, 257 µmol) in dioxane (1 mL) and H₂O (0.2 mL) was added Pd(dppf)Cl₂ (9 mg, 12 µmol) under N₂. The mixture was stirred at 70 °C for 16 hrs under N₂ atmosphere. The mixture was poured into water (20 mL) and extracted with ethyl acetate (15 mL×2). The combined organic phase was washed with brine (20 mL×2), dried with anhydrous Na₂SO₄, filtered and concentrated in vacuo. The residue was purified by prep-HPLC (METHOD A) to give the title compound as a white solid. MS (ESI): mass calcd for C₂₃H₁₈ClF₅N₄O₂, 512.1; m/z found, 513.1 [M+H]⁺. ¹H NMR (400MHz, CDCl₃) δ = 9.09 (s, 1H), 8.15 (d, *J* = 11.0 Hz, 1H), 7.58 (d, *J* = 5.4 Hz, 1H), 7.32 - 7.28 (m, 1H), 7.26 - 7.21 (m, 1H), 7.17 -7.10 (m, 1H), 5.09-5.03 (m, 1H), 4.16 (q, *J* = 7.3 Hz, 2H), 2.55 (s, 3H), 1.67 (d, *J* = 6.5 Hz, 3H), 1.51 (t, *J* = 7.3 Hz, 3H).

### Example 2: (S)-N-(2-chloro-6-fluorophenyl)-4-(1-ethyl-5-(hydroxymethyl)-1H-1,2,4-triazol-3-yl)-5-fluoro-2-((1,1,1-trifluoropropan-2-yl)oxy)benzamide.

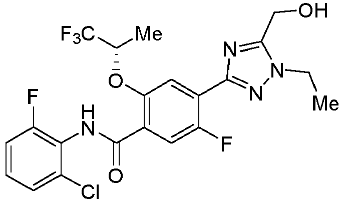

Step A. 3,5-dibromo-1-ethyl-1H-1,2,4-triazole. To a solution of 3,5-dibromo-1H-1,2,4-triazole (6 g, 26.4 mmol), DIEA (6.84 g, 52.9 mmol, 9.2 mL) in MeCN (60 mL) was added EtI (6.19 g, 39.7 mmol, 3.17 mL). The mixture was stirred at 60 °C for 1.5 hrs. TLC (petroleum ether/ ethyl acetate=1/1) showed starting material was consumed completely and major one new spot was formed. The mixture was concentrated in vacuo. The residue was poured into water (60 mL) and extracted with ethyl acetate (60 mL×2). The combined organic phase was washed with brine (100 mL×2), dried with anhydrous Na₂SO₄, filtered and concentrated in vacuo. The residue was purified by column chromatography (SiO₂, petroleum ether/ethyl acetate=20/1) to give the title compound as a light yellow solid. MS (ESI): mass calcd for C₄H₅Br₂N₃, 252.9; m/z found, 253.7 [M+H]⁺. ¹H NMR (400 MHz, CDCl₃) δ = 4.19 (q, *J* = 7.3 Hz, 2H), 1.47 (t, *J* = 7.3 Hz, 3H).

Step B. 3-bromo-1-ethyl-1H-1,2,4-triazole-5-carbaldehyde. To a solution of 3,5-dibromo-1-ethyl-1H-1,2,4-triazole (0.5 g, 1.96 mmol) in THF (20 mL) was added n-BuLi (2.5 M in hexane, 784 µL) drop-wise at -78 °C under N₂. The mixture reaction was stirred at -78 °C for 1 hr. The reaction was added DMF (430 mg, 5.88 mmol, 453 µL) dropwise at -78 °C. The mixture was stirred at - 78 °C for 1 hr. The mixture was poured into 0.5 N HCl (50 mL) and extracted with DCM (50 mL×2). The combined organic phase was washed with brine (70 mL×2), dried with anhydrous Na₂SO₄, filtered and concentrated in vacuo to give the title compound as a light yellow oil. ¹H NMR (400 MHz, CDCl₃) δ = 9.91 (s, 1H), 4.60 (q, *J* = 7.2 Hz, 2H), 1.49 (t, *J* = 7.2 Hz, 3H).

Step C. (3-bromo-1-ethyl-1H-1,2,4-triazol-5-yl)methanol. To a solution of 3-bromo-1-ethyl-1H-1,2,4-triazole-5-carbaldehyde (0.200 g, 541 µmol) in THF (5 mL) was added NaBH₄ (30.7 mg, 812 µmol) dropwise at 0 °C. The mixture was stirred at 16 °C for 0.25 hr. The mixture was poured into 1N HCl (10 mL) and extracted with DCM (10 mL×2). The combined organic phase was washed with brine (15 mL×2), dried with anhydrous Na₂SO₄, filtered and concentrated in vacuo to give the title compound as a light yellow oil. MS (ESI): mass calcd for C₅H₈BrN₃O, 205.0/207.0; m/z found, /206.5/208.5 [M+H]⁺. ¹H NMR (400 MHz, CDCl₃) δ = 4.77 (s, 2H), 4.26 - 4.22 (m, 2H), 3.48 - 3.03 (m, 1H), 1.52 - 1.48 (m, 3H).

Step D. (S)-N-(2-chloro-6-fluorophenyl)-4-(1-ethyl-5-(hydroxymethyl)-1H-1,2,4-triazol-3-yl)-5-fluoro-2-((1,1,1-trifluoropropan-2-yl)oxy)benzamide. The title compound was prepared in a manner analogous to Example 1, Step E however using (3-bromo-1-ethyl-1H-1,2,4-triazol-5-yl)methanol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd for C₂₁H₁₈ClF₅N₄O₃, 504.1; m/z found, 505.1 [M+H]⁺. ¹H NMR (400 MHz, CDCl₃) δ = 9.11 (s, 1H), 8.15 (d, *J* = 11.2 Hz, 1H), 7.78 (d, *J* = 5.3 Hz, 1H), 7.33 -7.28 (m, 1H), 7.26 - 7.21 (m, 1H), 7.17 - 7.11 (m, 1H), 5.11 (m, 1H), 4.89 (d, *J* = 5.7 Hz, 2H), 4.35 (q, *J* = 7.2 Hz, 2H), 2.94 (t, *J* = 6.1 Hz, 1H), 1.68 (d, *J* = 6.4 Hz, 3H), 1.57 (t, *J* = 7.3 Hz, 3H).

### Example 3: (S)-N-(2-chloro-6-fluorophenyl)-5-fluoro-4-(6-methyl-3-oxo-4-propyl-3,4-dihydropyrazin-2-yl)-2-((1,1,1 -trifluoropropan-2-yl)oxy)benzamide.

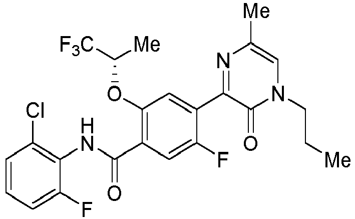

Step A. 2-(allylamino)acetonitrile. To a mixture of prop-2-en-1-amine (50 g, 876 mmol), NaI (13.1 g, 87.6 mmol), K₂CO₃ (242 g, 1.75 mol) and TEA (354 g, 3.50 mol) in DMF (800 mL) was added 2-bromoacetonitrile (84.04 g, 700.6 mmol), and then the mixture was stirred at 25 °C for 16 hr under N₂ atmosphere. The insoluble solids were filtered out and the filtrate was quenched with water (1 L). The aqueous phase was extracted with ethyl acetate (500 mL × 2). The combined organic phase was washed with brine (1 L), dried with anhydrous Na₂SO₄, filtered and concentrated in vacuo to give the title compound as brown oil.

Step B. 1-allyl-3,5-dibromopyrazin-2(1H)-one. To a solution of 2-(allylamino)acetonitrile (21 g, crude) in DCM (200 mL) was added oxalyl dibromide (42.43 g, 196.61 mmol) at 0 °C. The mixture was stirred at 40 °C for 16 hr. The mixture was concentrated in vacuo. The residue was purified by column chromatography (SiO₂, petroleum ether/ethyl acetate=1/1 to 5/1) to give the title compound as a yellow oil. MS (ESI): mass calcd. for C₇H₆Br₂N₂O, 291.8/293.8; m/z found, 292.8 / 294.8 [M+H]⁺.

Step C. 1-allyl-5-bromo-3-methoxypyrazin-2(1H)-one. A mixture of 1-allyl-3,5-dibromopyrazin-2(1H)-one (6.8 g, 23.1 mmol), CH₃ONa (3.75 g, 69.4 mmol) in MeOH (70 mL) was degassed and purged with N₂ for 3 times. The mixture was stirred at 15 °C for 3 hr. The mixture was poured into HCl (1M, 150 mL). The aqueous phase was extracted with ethyl acetate (200 mL). The combined organic phase was dried with anhydrous Na₂SO₄, filtered and concentrated in vacuo. The residue was purified by column chromatography (SiO₂, petroleum ether/ethyl acetate=1/1 to 3/1) to give the title compound as a yellow solid.

Step D. 5-bromo-3-methoxy-1-propylpyrazin-2(1H)-one. A mixture of 1-allyl-5-bromo-3-methoxypyrazin-2(1H)-one (2 g, 8.16 mmol), Pd/C (200 mg) in EtOAc (50 mL) was degassed and purged with H₂ for 3 times. The mixture was stirred at 15 °C for 1 hr under H₂(15 psi) atmosphere. The mixture was filtered and concentrated in vacuo. The residue was purified by column chromatography (SiO₂, petroleum ether/ethyl acetate=100/1 to 1/1) to give the title compound as a white solid.

Step E, 3-methoxy-5-methyl-1-propylpyrazin-2(1H)-one. To a solution of 5-bromo-3-methoxy-1-propylpyrazin-2(1H)-one (400 mg, 1.62 mmol) and 2,4,6-trimethyl-1,3,5,2,4,6-trioxatriborinane (3 M in EtOAc, 809.43 µL) in DME (5 mL) were added K₂CO₃ (447.47 mg, 3.24 mmol) and Pd(dppf)Cl₂ (118.45 mg, 161.89 µmol). The mixture was stirred at 80 °C for 16 hr. The reaction mixture was concentrated in vacuo. The residue was purified by column chromatography (SiO₂, petroleum ether/ethyl acetate=10/1 to 1/1) to give the title compound as a yellow oil. MS (ESI): mass calcd. for C₉H₁₄N₂O₂, 182.1; m/z found, 183.2 [M+H]⁺. ¹H NMR (400 MHz, CDCl₃) δ = 6.52 (s, 1H), 3.96 (d, *J* = 7.2 Hz, 3H), 3.85-3.76 (m, 2H), 2.14 (s, 3H), 1.81-1.72 (m, 2H), 0.98-0.91 (m, 3H).

Step F. 3-bromo-5-methyl-1-propylpyrazin-2(1H)-one. A mixture of 3-methoxy-5-methyl-1-propylpyrazin-2(1H)-one (0.1 g, 400.62 µmol) and POBr₃ (172.28 mg, 600.93 µmol) in DMF (1 mL) was stirred at 80 °C for 5 hr. The reaction mixture was diluted with water (20 mL) and adjusted pH to 7 with Na₂CO₃ (sat. aqueous). The mixture was extracted with ethyl acetate (10 mL × 2). The combined organic layers were washed with brine (10 mL × 2), dried over Na₂SO₄, filtered and concentrated in vacuo to give the title compound as a yellow oil. MS (ESI): mass calcd. for C₈H₁₁BrN₂O, 230.0/232.0; m/z found, 231.0, 232.8 [M+H]⁺. ¹H NMR (400 MHz, CDCl₃) δ = 6.90 (s, 1H), 3.86 (d, *J* = 7.2 Hz, 2H), 2.27 (s, 3H), 1.84-1.58 (m, 2H), 0.97 (t, *J* = 7.6 Hz, 3H).

Step G. (S)-N-(2-chloro-6-fluorophenyl)-5-fluoro-4-(6-methyl-3-oxo-4-propyl-3,4-dihydropyrazin-2-yl)-2-((1,1,1-trifluoropropan-2-yl)oxy)benzamide. To a solution of 3-bromo-5-methyl-1-propylpyrazin-2(1H)-one(60 mg, 223.29 µmol) and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 180 mg) in THF (3 mL) and H₂O (0.3 mL) were added Ad₂nBuP-Pd-G₃ (16.26 mg, 22.33 µmol) and K₃PO₄ (142.19 mg, 669.87 µmol). The mixture was stirred at 80 °C for 16 hr. The mixture was combined with another batch (20 mg, scale) and then concentrated in vacuo. The residue was purified by column chromatography (SiO₂, petroleum ether/ethyl acetate=10/1 to 1/1) to give a yellow oil. The yellow oil was purified by prep-HPLC (method A) to give the title compound as a yellow solid. MS (ESI): mass calcd. for C₂₄H₂₁ClF₅N₃O₃, 529.1; m/z found, 530.2 [M+H]⁺. ¹H NMR (400 MHz, CDCl₃) δ = 9.08 (s, 1H), 8.09 (d, *J* = 10.4 Hz, 1H), 7.40 (d, *J* = 5.2 Hz, 1H), 7.28-7.27 (m, 1H), 7.25-7.24 (m, 1H), 7.23-7.22 (m, 1H), 7.06 (s, 1H), 4.99-4.92 (m, 1H), 3.95-3.88 (m, 2H), 2.38 (s, 3H), 1.89-1.80 (s, 2H), 1.64 (d, *J* = 6.4 Hz, 3H), 1.01 (t, J = 7.2 Hz, 3H).

### Example 4: (S)-N-(2-chloro-6-fluorophenyl)-4-(5,6-dimethylpyrazin-2-yl)-5-fluoro-2-((1,1,1-trifluoropropan-2-yl)oxy)benzamide

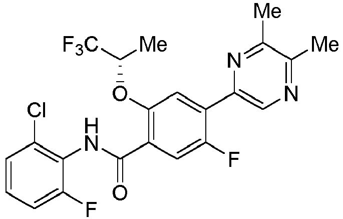

The title compound was prepared in a manner analogous to Example 1, Step E however using 5-chloro-2,3-dimethylpyrazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd for C₂₂H₁₇ClF₅N₃O₂, 485.1; m/z found, 486.1 [M+H]⁺. ¹H NMR (400MHz, CDCl₃) δ = 9.11 (s, 1H), 8.94 (d, *J* = 2.1 Hz, 1H), 8.15 (d, *J* = 11.8 Hz, 1H), 7.87 (d, *J* = 5.8 Hz, 1H), 7.32 - 7.28 (m, 1H), 7.27 - 7.22 (m, 1H), 7.17 - 7.11 (m, 1H), 5.12 - 5.06 (m, 1H), 2.65 (d, *J* = 11.3 Hz, 6H), 1.69 (d, *J* = 6.5 Hz, 3H), 1.30 - 1.20 (m, 1H), 0.95 - 0.74 (m, 1H).

### Example 5: (S)-N-(2-chloro-6-fluorophenyl)-5-fluoro-4-(2-(hydroxymethyl)-1-methyl-1H-imidazol-4-yl)-2-((1,1,1-trifluoropropan-2-yl)oxy)benzamide

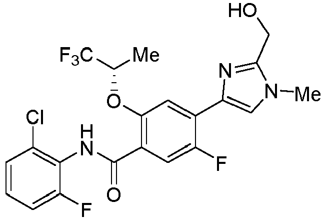

Step A. (4-bromo-1-methyl-1H-imidazol-2-yl)methanol. To a stirred solution of methyl 4-bromo-1-methyl-1H-imidazole-2-carboxylate (0.30 g, 1.4 mmol) in THF (5 mL) was added LiBH₄ (36 mg, 1.6 mmol) at 0 °C. The mixture was stirred at 16 °C for 1 hr. The mixture was added 1N HCl (0.5 mL) and diluted with ethyl acetate (20 mL). The mixture was dried with anhydrous Na₂SO₄, filtered and concentrated in vacuo. The residue was purified by prep-TLC (SiO₂, dichloromethane / methanol=10/1) to give the title compound as a white solid. MS (ESI): mass calcd for C₅H₇BrN₂O, 190.0; m/z found, 191.6 [M+H]⁺. ¹H NMR (400 MHz, DMSO-*d*₆) δ = 7.28 - 7.17 (m, 1H), 5.34 (t, *J* = 5.7 Hz, 1H), 4.41 (d, *J* = 5.7 Hz, 2H), 3.62 (s, 3H)

Step B. (S)-N-(2-chloro-6-fluorophenyl)-5-fluoro-4-(2-(hydroxymethyl)-1-methyl-1H-imidazol-4 -yl)-2-((1,1,1-trifluoropropan-2-yl)oxy)benzamide. The title compound was prepared in a manner analogous to Example 1, Step E however using (4-bromo-1-methyl-1H-imidazol-2-yl)methanol a s the aryl halide and (S)-N-(2-chloro-6-fluorophenyl)-5-fluoro-4-(4,4,5,5-tetramethyl-1,3,2-dioxa borolan-2-yl)-2-((1,1,1-trifluoropropan-2-yl)oxy)benzamide (Intermediate 1) as the boronate. MS (ESI): mass calcd for C₂₁H₁₇ClF₅N₃O₃, 489.1; m/z found, 490.1 [M+H]⁺. ¹H NMR (400 MHz, C DCl₃) δ = 9.17 (s, 1H), 8.06 (d, *J* = 11.9 Hz, 1H), 7.84 (d, *J* = 5.7 Hz, 1H), 7.49 (d, *J* = 3.8 Hz, 1 H), 7.31 - 7.28 (m, 1H), 7.26 - 7.20 (m, 1H), 7.16 - 7.09 (m, 1H), 5.28 - 5.21 (m, 1H), 4.78 (d, *J=* 1.7 Hz, 2H), 3.77 (s, 3H), 1.68 (d, *J* = 6.4 Hz, 3H).

### Example 6: (S)-N-(2-chloro-6-fluorophenyl)-4-(5-ethyl-4-(hydroxymethyl)thiazol-2-yl)-5-fluoro-2-((1,1,1-trifluoropropan-2-yl)oxy)benzamide

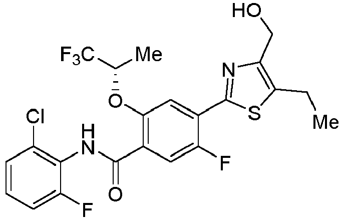

Step A. (2-bromo-5-ethylthiazol-4-vl)methanol. To a stirred solution of methyl 2-bromo-5-ethylthiazole-4-carboxylate (0.2 g, 799.64 µmol) in THF (2 mL) was added LiBH₄ (21 mg, 960 µmol) at 0 °C. The mixture was stirred at 16 °C for 40 hrs. The mixture was added 1N HCl (0.5 mL) and diluted with ethyl acetate (20 mL). The mixture was dried with anhydrous Na₂SO₄, filtered and concentrated in vacuo to give the title compound as a colorless oil. MS (ESI): mass calcd for C₆H₈BrNOS 221.0; m/z found, 244.8 [M+Na]⁺. ¹H NMR (400 MHz, CDCl₃) δ = 4.63 (s, 2H), 2.82 (q, *J* = 7.5 Hz, 2H), 2.42 - 2.25 (m, 1H), 1.28 (t, *J* = 7.5 Hz, 3H).

Step B. (S)-N-(2-chloro-6-fluorophenyl)-4-(5-ethyl-4-(hydroxymethyl)thiazol-2-yl)-5-fluoro-2-((1,1,1-trifluoropropan-2-yl)oxy)benzamide. The title compound was prepared in a manner analo gous to Example 1, Step E however using (2-bromo-5-ethylthiazol-4-yl)methanol as the aryl hali de and (S)-N-(2-chloro-6-fluorophenyl)-5-fluoro-4-(4,4,5,5-tetramethyl-1,3,2-dioxaborolan-2-yl) -2-((1,1,1-trifluoropropan-2-yl)oxy)benzamide (Intermediate 1) as the boronate. MS (ESI): mass calcd for C₂₂H₁₈ClF₅N₂O₃S, 520.1; m/z found, 521.0 [M+H]⁺. ¹H NMR (400 MHz, CDCl₃) δ = 9. 13 (s, 1H), 8.16 (d, *J* = 11.6 Hz, 1H), 8.00 (d, *J* = 5.5 Hz, 1H), 7.32 - 7.28 (m, 1H), 7.26 - 7.21 (m, 1H), 7.18 - 7.11 (m, 1H), 5.19 - 5.13 (m, 1H), 4.80 (d, *J* = 5.1 Hz, 2H), 2.95 (q, *J* = 7.6 Hz, 2H), 2.57 (t, *J* = 5.5 Hz, 1H), 1.69 (d, *J* = 6.5 Hz, 3H), 1.38 (t, *J* = 7.6 Hz, 3H).

### Example 7: (S)-N-(2-chloro-6-fluorophenyl)-5-fluoro-4-(5,6,7,8-tetrahydro-[1,2,4]triazolo[4,3-alpyrazin-3-yl)-2-((1,1,1-trifluoropropan-2-yl)oxy)benzamide

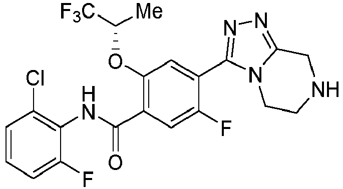

### Step A, (S)-tert-butyl 3-(4-((2-chloro-6-fluorophenyl)carbamoyl)-2-fluoro-5-((1,1,1-trifluoropropan-2-yl)oxy)phenyl)-5,6-dihydro-[1,2,4]triazolo[4,3-a]pyrazine-7(8H)-carboxylate.

To a mixture of (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 200 mg), tert-butyl 3-bromo-5,6-dihydro-[1,2,4]triazolo[4,3-a]pyrazine-7(8H)-carboxylate (109 mg, 359 µmol), K₃PO₄ (1.5 M, 718 µL) in toluene (3.6 mL) was added SPhos Pd G3 (28 mg, 36 µmol) under N₂. The mixture was stirred at 60 °C for 16 hrs under N₂ atmosphere. The reaction mixture with another batch (200 mg, scale) was poured into water (40 mL) and extracted with ethyl acetate (40 mL×2). The combined organic phase was concentrated in vacuo to give crude product. The residue was purified by prep-HPLC (METHOD A) to give the title compound as a white solid. MS (ESI): mass calcd for C₂₆H₂₅ClF₅N₅O₄, 601.2; m/z found, 602.3 [M+H]⁺. ¹H NMR (400 MHz, CDCl₃) δ = 9.08 (s, 1H), 8.21 (d, *J* = 10.8 Hz, 1H), 7.54 (d, *J* = 5.0 Hz, 1H), 7.34 - 7.27 (m, 2H), 7.19 - 7.11 (m, 1H), 5.13 - 5.07 (m, 1H), 5.03 - 4.89 (m, 2H), 4.11 - 4.00 (m, 2H), 3.95 - 3.78 (m, 2H), 1.68 (d, *J* = 6.5 Hz, 3H), 1.53 (s, 9H).

Step B. (S)-N-(2-chloro-6-fluorophenyl)-5-fluoro-4-(5,6,7,8-tetrahydro-[1,2,4]triazolo[4,3-a]pyrazin-3-yl)-2-((1,1,1-trifluoropropan-2-yl)oxy)benzamide. A mixture of (S)-tert-butyl 3-(4-((2-chloro-6-fluorophenyl)carbamoyl)-2-fluoro-5-((1,1,1-trifluoropropan-2-yl)oxy)phenyl)-5,6-dihydro-[1,2,4]triazolo[4,3-a]pyrazine-7(8H)-carboxylate (90 mg, 149 µmol) in HCl/dioxane (1.5 mL) was stirred at 20 °C for 1 hr. The mixture was concentrated in vacuo. The residue was purified by prep-HPLC (METHOD D) to give the title compound as a white solid. MS (ESI): mass calcd for C₂₁H₁₇ClF₅N₅O₂, 501.1; m/z found, 502.1 [M+H]⁺. ¹H NMR (400MHz, CDCl₃) δ = 9.07 (s, 1H), 8.19 (d, *J* = 10.6 Hz, 1H), 7.47 (d, *J* = 5.3 Hz, 1H), 7.33 - 7.27 (m, 2H), 7.27 - 7.23 (m, 1H), 7.19 - 7.12 (m, 1H), 5.11 - 4.99 (m, 1H), 4.36 (s, 2H), 4.09 - 3.94 (m, 2H), 3.33 - 3.19 (m, 2H), 1.68 (d, *J* = 6.5 Hz, 3H).

### Example 8: (S)-N-(2-chloro-6-fluorophenyl)-5-fluoro-4-(pyrazin-2-yl)-2-((1,1,1-trifluoropropan-2-yl)oxy)benzamide

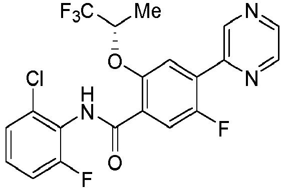

The title compound was prepared in a manner analogous to Example 1, Step E however using 2-bromopyrazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd for C₂₀H₁₃ClF₅N₃O₂, 457.1; m/z found, 458.0 [M+H]⁺. ¹H NMR (400 MHz, CDCl₃) δ = 9.27 (s, 1H), 9.12 (s, 1H), 8.75 - 8.70 (m, 1H), 8.64 (d, *J* = 2.5 Hz, 1H), 8.19 (d, *J* = 11.8 Hz, 1H), 7.89 (d, *J* = 5.6 Hz, 1H), 7.33 - 7.28 (m, 1H), 7.27 - 7.22 (m, 1H), 7.18 - 7.12 (m, 1H), 5.16 - 5.06 (m, 1H), 1.70 (d, *J* = 6.5 Hz, 3H).

### Example 9: (S)-N-(2-chloro-6-fluorophenyl)-5-fluoro-4-(6-methylpyridin-3-yl)-2-((1,1,1-trifluoropropan-2-yl)oxy)benzamide

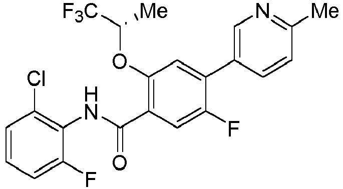

The title compound was prepared in a manner analogous to Example 1, Step E however using 5-bromo-2-methylpyridine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate MS (ESI): mass calcd for C₂₂H₁₆ClF₅N₂O₂, 470.1; m/z found, 471.1 [M+H]⁺. ¹H NMR (400 MHz, CDCl₃) δ = 9.05 (s, 1H), 8.69 (s, 1H), 8.14 (d, *J* = 10.8 Hz, 1H), 7.82 (br d, *J* = 8.1 Hz, 1H), 7.33 - 7.28 (m, 2H), 7.27 - 7.22 (m, 1H), 7.18 - 7.12 (m, 1H), 7.09 (d, *J* = 5.8 Hz, 1H), 5.01 - 4.92 (spt, *J* = 6.1 Hz, 1H), 2.65 (s, 3H), 1.68 (d, *J* = 6.5 Hz, 3H)

### Example 10: (S)-N-(2-chloro-6-fluorophenyl)-5-fluoro-4-(5-methylpyridin-2-yl)-2-((1,1,1-trifluoropropan-2-yl)oxy)benzamide

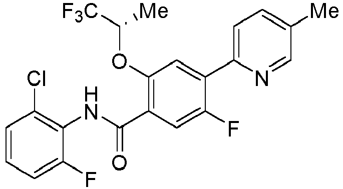

The title compound was prepared in a manner analogous to Example 1, Step E however using 2-bromo-5-methylpyridine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd for C₂₂H₁₆ClF₅N₂O₂, 470.1; m/z found, 471.1 [M+H]⁺. ¹H NMR (400 MHz, CDCl₃) δ = 9.16 (s, 1H), 8.58 (d, *J* = 2.1 Hz, 1H), 8.12 (d, *J* = 11.9 Hz, 1H), 7.90 - 7.85 (m, 2H), 7.64 - 7.62 (m, 1H), 7.32 - 7.28 (m, 1H), 7.26 - 7.21 (m, 1H), 7.16 - 7.11 (m, 1H), 5.22 - 5.03 (m, 1H), 2.43 (s, 3H), 1.68 (d, *J* = 6.4 Hz, 3H).

### Example 11: (S)-N-(2-chloro-6-fluorophenyl)-5-fluoro-4-(6-methylpyridazin-3-yl)-2-((1,1,1-trifluoropropan-2-vl)oxy)benzamide.

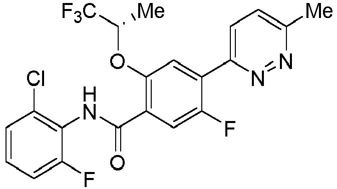

The title compound was prepared in a manner analogous to Example 1, Step E however using 3-bromo-6-methylpyridazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₅ClF₅N₃O₂, 471.1; m/z found, 472.0 [M+H]⁺.¹H NMR (400MHz, CDCl₃) δ = 9.15 (s, 1H), 8.18 (d, *J*=11.9 Hz, 1H), 8.12 (d, *J*=5.8 Hz, 1H), 8.04 (dd, *J*=1.5, 8.8 Hz, 1H), 7.47 (d, *J*=8.8 Hz, 1H), 7.33 - 7.29 (m, 1H), 7.27 - 7.23 (m, 1H), 7.17-7.12 (m, 1H), 5.20 - 5.10 (m, 1H), 2.82 (s, 3H), 1.69 (d, *J*=6.4 Hz, 3H)

### Example 12: (S)-N-(2-chloro-6-fluorophenyl)-5-fluoro-4-(6-methylpyrazin-2-yl)-2-((1,1,1-trifluoropropan-2-yl)oxy)benzamide.

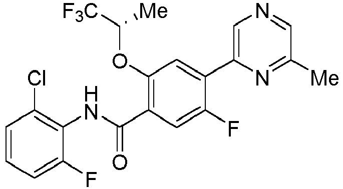

The title compound was prepared in a manner analogous to Example 1, Step E however using 2-bromo-6-methylpyrazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate MS (ESI): mass calcd. For C₂₁H₁₅ClF₅N₃O₂, 471.1; m/z found, 472.0 [M+H]⁺. ¹H NMR (400MHz, CDCl₃) δ = 9.11 (s, 1H), 9.04 (d, *J*=2.3 Hz, 1H), 8.51 (s, 1H), 8.17 (d, *J*=11.5 Hz, 1H), 7.87 (d, *J*=5.5 Hz, 1H), 7.33 - 7.29 (m, 1H), 7.27 - 7.22 (m, 1H), 7.18 - 7.11 (m, 1H), 5.13 - 5.06 (m, 1H), 2.69 (s, 3H), 1.70 (d, *J*=6.4 Hz, 3H)

### Example 13: (S)-N-(2-chloro-6-fluorophenyl)-5-fluoro-4-(5-methylpyrazin-2-yl)-2-((1,1,1-trifluoropropan-2-vl)oxy)benzamide.

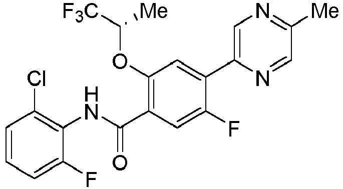

The title compound was prepared in a manner analogous to Example 1, Step E however using 2-bromo-5-methylpyrazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₅ClF₅N₃O₂, 471.1; m/z found, 472.1 [M+H]⁺. ¹H NMR (400MHz, CDCl₃) δ = 9.14 (t, *J*=1.8 Hz, 1H), 9.11 (s, 1H), 8.59 (d, *J*=1.1 Hz, 1H), 8.17 (d, *J*=11.7 Hz, 1H), 7.87 (d, *J*=5.6 Hz, 1H), 7.33 - 7.29 (m, 1H), 7.27 - 7.22 (m, 1H), 7.18 - 7.11 (m, 1H), 5.15 - 5.05 (m, 1H), 2.67 (s, 3H), 1.69 (d, *J*=6.4 Hz, 3H)

### Example 14: (S)-N-(2-chloro-6-fluorophenyl)-4-(1-ethyl-2-(hydromethyl)-1H-imidazol-4-yl)-5-fluoro-2-(1,1,1-trifluoropropan-2-yl)oxy)benzamide.

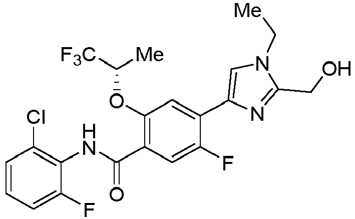

Step A. ethyl 1-ethyl-1H-imidazole-2-carboxylate. To a solution of ethyl 1H-imidazole-2-carboxylate (5.0 g, 36 mmol) and NaH (2.14 g, 53.5 mmol, 60% purity) in DMF (50 mL) was added EtI (8.35 g, 53.5 mmol, 4.28 mL). The mixture was stirred at 20 °C for 12 hr. The reaction mixture was quenched by addition brine (10 mL) at 25 °C, and then diluted with ethyl acetate (30 mL) and extracted with ethyl acetate (50 mL × 3). The combined organic layers were washed with brine (50 mL × 3), dried over Na₂SO₄, filtered and concentrated in vacuo. The residue was purified by column chromatography (SiO₂, petroleum ether/ethyl acetate=0/1) to give the title compound was obtained as a yellow solid.

Step B. Mixture of ethyl 4-bromo-1-ethyl-1H-imidazole-2-carboxylate and ethyl 5-bromo-1-ethyl-1H-imidazole-2-carboxylate. A mixture of ethyl 1-ethyl-1H-imidazole-2-carboxylate (2.6 g, 15.5 mmol), NBS (4.40 g, 24.73 mmol) in DMF (10 mL) and DCM (10 mL) was degassed and purged with N₂ for 3 times. The mixture was stirred at 15 °C for 36 hr under N₂ atmosphere. The reaction mixture was quenched by addition water (10 mL) at 20 °C, and then diluted with DCM (10 mL) and extracted with DCM (10 mL × 3). The combined organic layers were washed with brine (10 mL × 3), dried over Na₂SO₄, filtered and concentrated in vacuo. The residue was purified by column chromatography (SiO₂, petroleum ether/ethyl acetate=30/1 to 1/1) to give the title compound was obtained as a white solid.

Step C. (4-bromo-1-ethyl-1H-imidazol-2-yl)methanol and (5-bromo-1-ethyl-1H-imidazol-2-yl)methanol. A mixture of ethyl 4-bromo-1-ethyl-1H-imidazole-2-carboxylate and ethyl 5-bromo-1-ethyl-1H-imidazole-2-carboxylate (1.68 g, 3.40 mmol) in THF (10 mL) was degassed and purged with N₂ for 3 times. The mixture was added LiBH₄ (222 mg, 10.2 mmol) and stirred at 20 °C for 3 hr under N₂ atmosphere. The solvent was removed with reduced pressure to give crude product. The residue was purified by prep-HPLC (method E) to give (4-bromo-1-ethyl-1H-imidazol-2-yl)methanol as a white solid and as a white solid.

Step D. (S)-N-(2-chloro-6-fluorophenyl)-4-(1-ethyl-2-(hydroxymethyl)-1H-imidazol-4-yl)-5-fluoro-2-((1,1,1-trifluoropropan-2-yl)oxy)benzamide. A mixture of (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 0.2 g), (4-bromo-1-ethyl-1H-imidazol-2-yl)methanol (116 mg, 567 µmol), XPhos Pd G3 (32 mg, 38 µmol), Na₂CO₃ (110 mg, 1.04 mmol) in dioxane (10 mL) and H₂O (2 mL) was degassed and purged with N₂ for 3 times. The mixture was stirred at 70 °C for 12 hr under N₂ atmosphere. The solvent was removed with reduced pressure. The residue was purified by prep-HPLC (method A) to give the title compound was obtained as a white solid. MS (ESI): mass calcd. For C₂₂H₁₉ClF₅N₃O₃, 503.1; m/z found, 504.1 [M+H]⁺. ¹H NMR (400MHz, CDCl₃) δ = 9.09 (s, 1H), 7.97 (d, *J*=12.0 Hz, 1H), 7.78 (d, *J*=5.6 Hz, 1H), 7.46 (d, *J*=3.6 Hz, 1H), 7.21-7.18 (m, 1H), 7.16-7.05 (m, 1H), 5.18-5.14 (m, 1H), 4.69 (d, *J*=2.4 Hz, 2H), 4.05-3.99 (m, 2H), 1.60 (d, *J*=2.4 Hz, 3H), 1.43 (t, *J*=6.5 Hz, 3H).

### Example 15: (S)-N-(2-chloro-6-fluorophenyl)-5-fluoro-4-(6-(hydroxymethyl)pyrazin-2-yl)-2-((1,1,1-trifluoropropan-2-yl)oxy)benzamide.

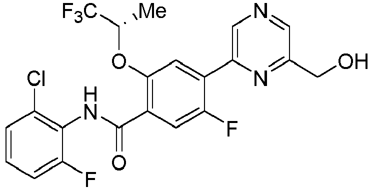

The title compound was prepared in a manner analogous to Example 1, Step E however using (6-bromopyrazin-2-yl)methanol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₅ClF₅N₃O₃, 487.1; m/z found, 488.1 [M+H]⁺. ¹H NMR (400MHz, CDCl₃) δ = 9.15 (d, *J*=2.1 Hz, 1H), 9.10 (s, 1H), 8.72 (s, 1H), 8.20 (d, *J*=11.6 Hz, 1H), 7.80 (d, *J*=5.6 Hz, 1H), 7.33 - 7.30 (m, 1H), 7.27 - 7.23 (m, 1H), 7.19 - 7.11 (m, 1H), 5.10 - 5.03 (m, 1H), 4.98 (d, *J*=5.3 Hz, 2H), 2.91 (t, *J*=5.4 Hz, 1H), 1.70 (d, *J*=6.5 Hz, 3H)

### Example 16: (S)-N-(2-chloro-6-fluorophenyl)-5-fluoro-4-(5-hydroxy-6-methylpyrazin-2-yl)-2-((1,1,1-trifluoropropan-2-yl)oxy)benzamide.

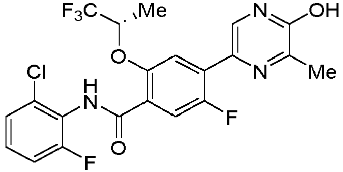

The title compound was prepared in a manner analogous to Example 1, Step E however using - bromo-3-methylpyrazin-2-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₅ClF₅N₃O₃, 487.1; m/z found, 488.1 [M+H]⁺. ¹H NMR (400MHz, CDCl₃) δ = 9.12 (s, 1H), 8.10 (d, *J*=12.7 Hz, 1H), 8.01 - 7.86 (m, 2H), 7.33 - 7.29 (m, 1H), 7.26 - 7.22 (m, 1H), 7.14 (br t, *J*=8.2 Hz, 1H), 5.12 - 5.05 (m, 1H), 2.62 (s, 3H), 1.70 (d, *J*=6.5 Hz, 3H)

### Example 17: (S)-N-(2-chloro-6-fluorophenyl)-5-fluoro-4-(5-hydroxypyrazin-2-yl)-2-((1,1,1-trifluoropropan-2-yl)oxy)benzamide.

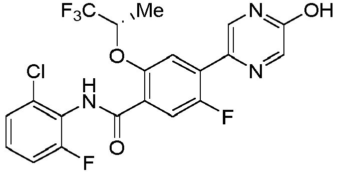

The title compound was prepared in a manner analogous to Example 1, Step E however using 5-bromopyrazin-2-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₀H₁₃ClF₅N₃O₃, 473.1; m/z found, 474.0 [M+H]⁺. ¹H NMR (400MHz, CDCl₃) δ = 9.13 (s, 1H), 8.38 (s, 1H), 8.13 (d, *J*=12.6 Hz, 1H), 8.09 (s, 1H), 7.90 (d, *J*=6.0 Hz, 1H), 7.33 - 7.29 (m, 1H), 7.26 - 7.22 (m, 1H), 7.18 - 7.10 (m, 1H), 5.19 - 4.97 (m, 1H), 1.70 (d, *J*=6.5 Hz, 3H).

### Example 18: (S)-N-(2-chloro-6-fluorophenyl)-4-(5-cyclopropylpyrazin-2-yl)-5-fluoro-2-((1,1,1-trifluoropropan-2-yl)oxy)benzamide.

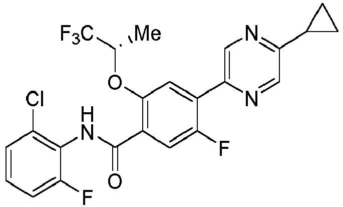

The title compound was prepared in a manner analogous to Example 1, Step E however using 2-bromo-5-cyclopropylpyrazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₃H₁₇ClF₅N₃O₂, 497.1; m/z found, 498.1 [M+H]⁺. ¹H NMR (400MHz, CDCl₃) δ = 9.11 (s, 1H), 9.05 (t, *J*=1.8 Hz, 1H), 8.60 (d, *J*=1.5 Hz, 1H), 8.15 (d, *J*=11.8 Hz, 1H), 7.86 (d, *J*=5.6 Hz, 1H), 7.33 - 7.29 (m, 1H), 7.27 - 7.22 (m, 1H), 7.17 - 7.10 (m, 1H), 5.17 - 5.03 (m, 1H), 2.22 - 2.09 (m, 1H), 1.69 (d, *J*=6.4 Hz, 3H), 1.23 - 1.09 (m, 4H).

### Example 19: (S)-N-(2-chloro-6-fluorophenyl)-4-(1-ethyl-2-(hydroxymethyl)-1H-imidazol-5-yl)-5-fluoro-2-(1,1,1-trifluoropropan-2-yl)oxy)benzamide.

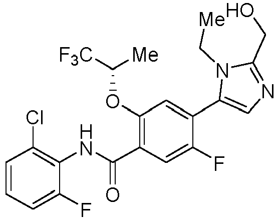

The title compound was prepared in a manner analogous to Example 14, Step D, however the title compound was isolated. MS (ESI): mass calcd. For C₂₂H₁₉ClF₅N₃O₃, 503.1; m/z found, 504.1 [M+H]⁺. ¹H NMR (400MHz, CDCl₃) δ = 8.94 (s, 1H), 8.06 (d, *J*=6.0 Hz, 1H), 7.23-7.20(m, 1H), 7.19-7.18(m, 1H),7.07-7.03(m, 1H), 7.02(s,1H), 6.95(d, *J*=5.2 Hz, 1H), 4.93-4.82(m, 1H), 4.73(s, 2H), 3.98-3.93(m, 2H), 1.60(d, *J*=6.4 Hz, 3H), 1.18 (t, *J*=7.2 Hz, 3H).

### Example 20: (S)-N-(2-chloro-6-fluorophenyl)-5-fluoro-4-(4,5,6,7-tetrahydrothiazolo[4,5-c]pyridin-2-yl)-2-((1,1,1-trifluoropropan-2-yl)oxy)benzamide.

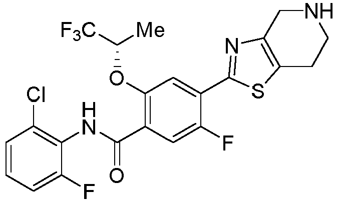

### Step A. (S)-tert-butyl 2-(4-((2-chloro-6-fluorophenyl)carbamoyl)-2-fluoro-5-((1,1,1-trifluoropropan-2-yl)oxy)phenyl)-6,7-dihydrothiazolo[4,5-c]pyridine-5(4H)-carboxylate

The title compound was prepared in a manner analogous to Example 1, Step E however using tert-butyl 2-bromo-6,7-dihydrothiazolo[4,5-c]pyridine-5(4H)-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd for C₂₇H₂₅ClF₅N₃O₄S, 617.1; m/z found, 618.3 [M+H]⁺.

Step B. (S)-N-(2-chloro-6-fluorophenyl)-5-fluoro-4-(4,5,6,7-tetrahydrothiazolo[4,5-c]pyridin-2-yl)-2-((1,1,1-trifluoropropan-2-yl)oxy)benzamide. A mixture of (S)-tert-butyl 2-(4-((2-chloro-6-fluorophenyl)carbamoyl)-2-fluoro-5-((1,1,1-trifluoropropan-2-yl)oxy)phenyl)-6,7-dihydrothiazolo[4,5-c]pyridine-5(4H)-carboxylate (70 mg, 113 µmol) in HCl/dioxane (2 mL) was stirred at 20 °C for 1 hr. The mixture was concentrated in vacuo. The residue was purified by prep-HPLC (METHOD D) to give the title compound as a white solid. MS (ESI): mass calcd for C₂₂H₁₇ClF₅N₃O₂S, 517.1; m/z found, 518.1 [M+H]⁺. ¹H NMR (400 MHz, CDCl₃) δ = 9.13 (s, 1H), 8.15 (d, *J* = 11.5 Hz, 1H), 7.99 (d, *J* = 5.5 Hz, 1H), 7.32 - 7.27 (m, 1H), 7.27 - 7.22 (m, 1H), 7.17 - 7.11 (m, 1H), 5.18 - 5.12 (m, 1H), 4.16 (s, 2H), 3.23 (t, *J* = 5.7 Hz, 2H), 2.95 (t, *J* = 5.5 Hz, 2H), 1.69 (d, *J* = 6.4 Hz, 3H)

### Example 21: (S)-N-(2-chloro-6-fluorophenyl)-4-(5-ethyl-4-(hydroxymethyl)-1H-imidazol-2-yl)-5-fluoro-2-((1,1,1-trifluoropropan-2-yl)oxy)benzamide.

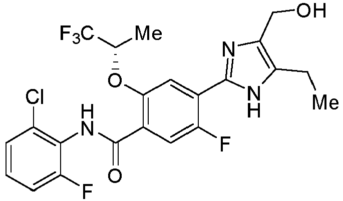

Step A. Methyl 5-ethyl-1H-imidazole-4-carboxylate. To a solution of methyl 3-oxopentanoate (25 g, 192 mmol, 23.8 mL) in AcOH (25 mL) was added NaNO₂ (19.88 g, 288.2 mmol) in H₂O (25 mL) dropwise at 0~15 °C. The reaction mixture was stirred at 0~15 °C for 4 hrs. The mixture was added HCHO (20.19 g, 672.3 mmol, 18.52 mL) at 0 °C. Then HCl (111 g, 1.15 mol, 108 mL, 38% purity) was added slowly maintaining the exothermic reaction temperature at 0~15 °C. After addition, the reaction mixture was stirred at 0~15 °C for 12 hrs. Then NH₃•H₂O (113.5 g, 907.2 mmol, 124.8 mL, 28% purity) was added to the reaction mixture and the reaction mixture was allowed to rise to 70 °C with pH at 5. The reaction mixture was stirred at 70 °C for 1 hr and the then NH₃•H₂O was added until the mixture was neutral. The reaction mixture was cooled to 0~10 °C and filtered. The filter cake was wash with DCM (60 ml×3). Then the aqueous layer was extracted with ethyl acetate (100 ml×8). The combined organic phases were dried over anhydrous Na₂SO₄, filtered and concentrated in vacuo to give the title compound as a yellow solid. MS (ESI): mass calcd. for C₇H₁₀N₂O₂, 154.1; m/z found, 155.1 [M+H]+.

Step B. methyl 2-bromo-5-ethyl-1H-imidazole-4-carboxylate. To a solution of methyl 5-ethyl-1H-imidazole-4-carboxylate (3.5 g, 23 mmol) in DCM (35 mL) was added NBS (4.04 g, 22.7 mmol). Then the reaction mixture was stirred at 25 °C for 2 hrs. The solvent was removed under reduced pressure. The residue was purified by column chromatography (SiO₂, petroleum ether/ethyl acetate =2/1) to give the title compound as a yellow solid. MS (ESI): mass calcd. for C₇H₉BrN₂O₂, 231.9; m/z found, 233.0, 235.0 [M+H]+.

Step C. (2-bromo-5-ethyl-1H-imidazol-4-yl)methanol. To a mixture of methyl 2-bromo-5-ethyl-1H-imidazole-4-carboxylate (1.0 g, 4.3 mmol) in THF (10 mL) was added LiAlH₄ (171 mg, 4.51 mmol) under N₂, and then the mixture was stirred at 0 °C for 3 hr under N₂ atmosphere. Water (2 mL) was added to the mixture. The residue was purified by prep-HPLC (method D) to give the title compound as a yellow oil. MS (ESI): mass calcd. for C₆H₉BrN₂O, 203.9; m/z found, 205.0, 207.0 [M+H]+.

Step D. (S)-N-(2-chloro-4-methylpyridin-3-yl)-5-fluoro-4-(2-(hydroxymethyl)-1-methyl-1H-imid azol-4-yl)-2-((1,1,1-trifluoropropan-2-yl)oxy)benzamide. The title compound was prepared in a m anner analogous to Example 14, Step D however using (2-bromo-5-ethyl-1H-imidazol-4-yl)meth anol (58 mg, 280 µmol) as the aryl halide and boronic acid piece name (S)-N-(2-chloro-6-fluorop henyl)-5-fluoro-4-(4,4,5,5-tetramethyl-1,3,2-dioxaborolan-2-yl)-2-((1,1,1-trifluoropropan-2-yl)o xy)benzamide (Intermediate 1) as the boronate. MS (ESI): mass calcd. for C₂₂H₁₉ClF₅N₃O₃, 503. 1; m/z found, 504.1 [M+H]+. ¹H NMR (400MHz, CDCl₃) δ = 9.07 (s, 1H), 8.05 (d, *J* = 13.6 Hz, 1H), 7.92 (d, *J* = 6 Hz, 1H), 7.21-7.19 (m, 1H), 7.18-7.17 (m, 1H), 7.16-7.05 (m, 1H), 5.45-5.25 (m, 1H), 4.62(s, 2H), 2.67 (q, *J* = 7.6 Hz 2H), 1.59(d, *J* = 6.4 Hz 3H), 1.23 (t, *J* = 7.2 Hz 3H).

### Example 22: (S)-N-(2-chloro-6-fluorophenyl)-4-(4-ethyl-5-(hydroxymethyl)thiazol-2-yl)-5-fluoro-2-((1,1,1-trifluoropropan-2-yl)oxy)benzamide.

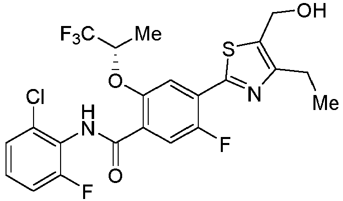

Step A. ethyl 2-amino-4-ethylthiazole-5-carboxylate. A mixture of thiourea (6.39 g, 84.0 mmol) and ethyl 2-chloro-3-oxopentanoate (10 g, 56 mmol) in EtOH (100 mL) was stirred at 80 °C for 6 hrs. The mixture was concentrated in vacuo. The residue was poured into NaHCO₃ (sat. 800 mL) and extracted with ethyl acetate (200 mL×2). The combined organic phase was washed with brine (300 mL×2), dried with anhydrous Na₂SO₄, filtered and concentrated in vacuo. The residue was washed with petroleum ether (200 mL) and concentrated in vacuo to give the title compound as a brown solid. MS (ESI): mass calcd for C₈H₁₂N₂O₂S, 200.1; m/z found, 201.6 [M+H]⁺. ¹H NMR (400 MHz, CDCl₃) δ = 5.51 (s, 2H), 4.27 (q, *J* = 7.1 Hz, 2H), 2.96 (q, *J* = 7.5 Hz, 2H), 1.33 (t, *J* = 7.1 Hz, 3H), 1.23 (t, *J* = 7.6 Hz, 3H).

Step B. ethyl 2-bromo-4-ethylthiazole-5-carboxylate. A mixture of ethyl 2-amino-4-ethylthiazole-5-carboxylate (2.5 g, 12 mmol) and CuBr₂ (3.35 g, 15.0 mmol) in CH₃CN (50 mL) was added tert-butyl nitrite (1.93 g, 18.7 mmol, 2.23 mL) dropwise at 0 °C under N₂. Then the mixture was stirred at 20°C for 15 hrs. The mixture was poured into water (300 mL) and extracted with ethyl acetate (100 mL×2). The combined organic phase was washed with brine (150 mL×2), dried with anhydrous Na₂SO₄, filtered and concentrated in vacuo. The residue was purified by column chromatography (SiO₂, petroleum ether/ethyl acetate=1/0) to give the title compound as a colourless oil. MS (ESI): mass calcd for C₈H₁₀BrNO₂S, 263.0; m/z found, 266.4 [M+H]⁺. ¹H NMR (400 MHz, CDCl₃) δ = 4.33 (q, *J* = 7.2 Hz, 2H), 3.13 (q, *J* = 7.5 Hz, 2H), 1.36 (t, *J* = 7.2 Hz, 3H), 1.28 (t, *J* = 7.5 Hz, 3H).

Step C. (2-bromo-4-ethylthiazol-5-yl)methanol. To a stirred solution of ethyl 2-bromo-4-ethylthiazole-5-carboxylate (0.3 g, 1.1 mmol) in THF (3 mL) was added LiBH₄ (29.7 mg, 1.36 mmol) at 0 °C. And the mixture was stirred at 20 °C for 15 hrs. The mixture was added 1N HCl (0.5 mL) and diluted with ethyl acetate (20 mL). The mixture was dried with anhydrous Na₂SO₄, filtered and concentrated in vacuo. The residue was purified by prep-HPLC (METHOD A) to give the title compound as a white solid. MS (ESI): mass calcd for C₆H₈BrNOS, 221.0/221.0; m/z found, 222.5/224.5 [M+H]⁺.

Step D. (S)-N-(2-chloro-6-fluorophenyl)-4-(4-ethyl-5-(hydroxymethyl)thiazol-2-yl)-5-fluoro-2-((1.1.1-trifluoropropan-2-yl)oxy)benzamide. The title compound was prepared in a manner analogous to Example 1, Step E however using (2-bromo-4-ethylthiazol-5-yl)methanol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd for C₂₂H₁₈ClF₅N₂O₃S, 520.1; m/z found, 521.2 [M+H]⁺. ¹H NMR (400 MHz, CDCl₃) δ = 9.12 (s, 1H), 8.15 (d, *J* = 11.5 Hz, 1H), 8.03 (d, *J* = 5.5 Hz, 1H), 7.33 - 7.28 (m, 1H), 7.27 - 7.22 (m, 1H), 7.16 - 7.12 (m, 1H), 5.19 - 5.10 (m, 1H), 4.91 (d, *J* = 5.4 Hz, 2H), 2.89 - 2.83 (m, 2H), 1.91 (t, *J* = 5.6 Hz, 1H), 1.70 (d, *J* = 6.5 Hz, 3H), 1.36 (t, *J* = 7.6 Hz, 3H).

### Example 23: (S)-N-(2-chloro-6-fluorophenyl)-4-(1-ethyl-5-(hydroxymethyl)-1H-pyrazol-3-yl)-5-fluoro-2-((1,1,1-trifluoropropan-2-yl)oxy)benzamide.

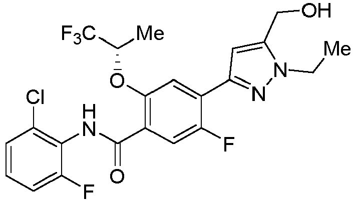

Step A. methyl 2-ethyl-5-oxo-2,5-dihydro-1H-pyrazole-3-carboxylate. To a solution of ethylhydrazine (3.38 g, 35.0 mmol, HCl) and TEA (11.39 g, 112.6 mmol, 15.67 mL) in H₂O (40 mL) was stirred at 15 °C for 0.5 h. Then dimethyl but-2-ynedioate (8.0 g, 56 mmol) in MeOH (80 mL) was added to the mixture. The mixture was stirred at 70 °C for 12 h. The reaction mixture was quenched by addition 1 N HCl (40 mL), and then extracted with ethyl acetate (40 mL×2). The combined organic layers were dried over Na₂SO₄, filtered and concentrated in vacuo. The residue was purified by column chromatography (SiO₂, petroleum ether/ethyl acetate=30/1 to 20/1) (method A) to give the title compound was obtained as yellow solid. ¹H NMR (400MHz, CDCl₃) δ = 6.16 (s, 1H), 4.45 (q, *J*=7.2 Hz, 2H), 3.94 - 3.85 (m, 3H), 1.42 (t, *J*=7.2 Hz, 3H).

Step B. methyl 1-ethyl-3-(((trifluoromethyl)sulfonyl)oxy)-1H-pyrazole-5-carboxylate. To a solution of methyl 2-ethyl-5-oxo-1H-pyrazole-3-carboxylate (340 mg, 2.00 mmol) and Py (1.58 g, 20.0 mmol, 1.61 mL) in DCM (9 mL) was added Tf₂O (676 mg, 2.40 mmol, 396 µL) dropwise at 0 °C under N₂. The mixture was stirred at 20 °C for 2 h. The reaction mixture was quenched by addition 1N HCl (20 mL), and then extracted with DCM (20 mL×2). The combined organic layers were washed with brine (30 mL), dried over Na₂SO₄, filtered and concentrated in vacuo. The residue was purified by column chromatography (SiO₂, petroleum ether/ethyl acetate = 3/1) to give the title compound as colorless oil. MS (ESI): mass calcd. For C₈H₉F₅N₂O₅S, 302.0; m/z found, 303.0 [M+H]⁺. ¹H NMR (400MHz, CDCl₃) δ = 6.70 - 6.64 (m, 1H), 4.58 (q, *J*=7.2 Hz, 2H), 3.96 - 3.88 (m, 3H), 1.45 (t, *J*=7.2 Hz, 3H).

Step C. (S)-N-(2-chloro-6-fluorophenyl)-4-(1-ethyl-5-(hydroxymethyl)-1H-pyrazol-3-yl)-5-fluoro-2-((1,1,1-trifluoropropan-2-yl)oxy)benzamide. To a mixture of (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 320 mg), methyl 1-ethyl-3-(((trifluoromethyl)sulfonyl)oxy)-1H-pyrazole-5-carboxylate (296.9 mg, 982.2 µmol) and CsF (230 mg, 1.51 mmol) in dioxane (15 mL) and H₂O (3 mL) was added Pd(dppf)Cl₂ (55.3 mg, 75.6 µmol) at 15 °C under N₂. The mixture was stirred at 80 °C for12 h. The reaction mixture was quenched by addition 1 N HCl (20mL), and then extracted with ethyl acetate (20 mL×2). The combined organic layers were washed with brine (20 mL), dried over Na₂SO₄, filtered and concentrated in vacuo. The residue was purified by column chromatography (SiO₂, petroleum ether/ethyl acetate=50/1 to 15/1) to give the title compound was obtained as a white solid. MS (ESI): mass calcd. For C₂₃H₁₉ClF₅N₃O₄, 531.1; m/z found, 532.2 [M+H]⁺. ¹H NMR (400MHz, CDCl₃) δ = 9.12 (s, 1H), 8.10 (d, *J*=11.6 Hz, 1H), 7.78 (d, *J*=5.6 Hz, 1H), 7.38 (d, *J*=4.0 Hz, 1H), 7.32 - 7.28 (m, 1H), 7.26 - 7.21 (m, 1H), 7.18 - 7.09 (m, 1H), 5.14 - 5.05 (m, 1H), 4.71 (q, *J*=7.1 Hz, 2H), 3.94 (s, 3H), 1.68 (d, *J*=6.4 Hz, 3H), 1.53 (t, *J*=7.2 Hz, 3H).

Step D. (S)-N-(2-chloro-6-fluorophenyl)-4-(1-ethyl-5-(hydroxymethyl)-1H-pyrazol-3-yl)-5-fluoro-2-((1,1,1-trifluoropropan-2-yl)oxy)benzamide. To a solution of (S)-N-(2-chloro-6-fluorophenyl)-4-(1-ethyl-5-(hydroxymethyl)-1H-pyrazol-3-yl)-5-fluoro-2-((1,1,1-trifluoropropan-2-yl)oxy)benzamide (80 mg, 150 µmol) in THF (1 mL) as added LiBH₄ (2 M in THF, 602 µL) at 0 °C under N₂. The mixture was stirred at 0 °C for 2 hr. The reaction mixture was quenched by addition 1 N HCl (10 mL) at 0 °C, and then extracted with ethyl acetate (10 mL×2). The combined organic layers were washed with brine (15 mL), dried over Na₂SO₄, filtered and concentrated in vacuo. The residue was purified by prep-HPLC (method A) to give the title compound as white solid. MS (ESI): mass calcd. For C₂₂H₁₉ClF₅N₃O₃, 503.1; m/z found, 504.1 [M+H]⁺. ¹H NMR (400MHz, CDCl₃) δ = 9.13 (s, 1H), 8.07 (d, *J*=11.7 Hz, 1H), 7.76 (d, *J*=5.6 Hz, 1H), 7.31 - 7.28 (m, 1H), 7.27 - 7.21 (m, 1H), 7.16 - 7.10 (m, 1H), 6.77 (d, *J*=4.3 Hz, 1H), 5.12-5.09 (m, 1H), 4.75 (d, *J*=5.7 Hz, 2H), 4.32 (q, *J*=7.3 Hz, 2H), 1.73 (br t, *J*=5.7 Hz, 1H), 1.67 (d, *J*=6.5 Hz, 3H), 1.56 - 1.53 (m, 3H).

### Example 24: (S)-N-(2-chloro-6-fluorophenyl)-4-(4-ethyl-5-(hydroxymethyl)-1-methyl-1H-imidazol-2-yl)-5-fluoro-2-(1,1,1-trifluoropropan-2-yl)oxy)benzamide.

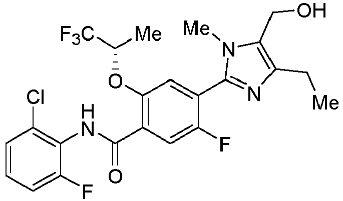

Step A. methyl 2-bromo-4-ethyl-1-methyl-1H-imidazole-5-carboxylate and methyl 2-bromo-5-ethyl-1-methyl-1H-imidazole-4-carboxylate. To a mixture of methyl 2-bromo-5-ethyl-1H-imidazole-4-carboxylate (300 mg, 1.29 mmol) in DMF (5 mL) was added NaH (103 mg, 2.57 mmol, 60% purity) in one portion at 0 °C under N₂. The mixture was stirred at 0 °C for 30 min, then added MeI (274 mg, 1.93 mmol, 120 µL) at 0 °C and stirred at 20 °C for 4 hours. The mixture was poured into 1N HCl (15 mL). The aqueous phase was extracted with ethyl acetate (25 mL×2). The combined organic phase was washed with brine (15 mL×2), dried with anhydrous Na₂SO₄, filtered and concentrated in vacuo. The residue was purified by silica gel chromatography (1000 mesh silica gel, petroleum ether/ethyl acetate=15/1 to 2/1) to give two regioisomers: methyl 2-bromo-4-ethyl-1-methyl-1H-imidazole-5-carboxylate as yellow solid. MS (ESI): mass calcd. for C₈H₁₁BrN₂O₂, 246.0; m/z found, 247.1, 249.1 [M+H]⁺. ¹H NMR (400 MHz, CDCl₃) δ = 3.87 (d, *J* = 6.7 Hz, 6H), 2.93 - 2.79 (m, 2H), 1.29 - 1.16 (m, 3H). Methyl 2-bromo-5-ethyl-1-methyl-1H-imidazole-4-carboxylate as yellow solid. MS (ESI): mass calcd. for C₈H₁₁BrN₂O₂, 246.0; m/z found, 247.1, 249.1 [M+H]⁺. ¹H NMR (400 MHz, CDCl₃) δ = 3.89 (s, 3H), 3.63 - 3.54 (m, 3H), 3.12 - 2.97 (m, 2H), 1.25 - 1.15 (m, 3H).

Step B. (S)-methyl 2-(4-((2-chloro-6-fluorophenyl)carbamoyl)-2-fluoro-5-((1,1,1 - trifluoropropan-2-yl)oxy)phenyl)-4-ethyl-1-methyl-1H-imidazole-5-carboxylate. The title compound was prepared in a manner analogous to Example 1, Step E however using methyl 2-bromo-4-ethyl-1-methyl-1H-imidazole-5-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₄H₂₁ClF₅N₃O₄, 545.1, m/z found, 546.2 [M+H]⁺.

Step C. (S)-N-(2-chloro-6-fluorophenyl)-4-(4-ethyl-5-(hydroxymethyl)-1-methyl-1H-imidazol-2-yl)-5-fluoro-2-((1,1,1-trifluoropropan-2-yl)oxy)benzamide. A mixture of (S)-methyl 2-(4-((2-chloro-6-fluorophenyl)carbamoyl)-2-fluoro-5-((1,1,1-trifluoropropan-2-yl)oxy)phenyl)-4-ethyl-1-methyl-1H-imidazole-5-carboxylate (82.1 mg, 150 µmol), LAH (8.6 mg, 225 µmol) in THF (1 mL) was degassed and purged with N₂ 3 times. The mixture was stirred at 25 °C for 2 hr under N₂ atmosphere. The mixture was quenched with water (3 mL). The solvent was removed in vacuo and redisolved in MeOH (2 mL). The residue was purified by prep-HPLC (method A) to give the title compound as a white solid. MS (ESI): mass calcd. for C₂₃H₂₁ClF₅N₃O₃, 517.12, m/z found, 518.1 [M+H]⁺. ¹H NMR (400MHz, CDCl₃) δ = 9.12 (s, 1H), 8.14 (d, *J* = 10.8 Hz, 1H), 7.28-7.37 (m, 3H), 7.15-7.17 (m, 1H), 5.10-5.13 (m, 1H), 4.72(s, 2H), 3.66 (s, 3H), 2.69 (q, *J* = 7.6 Hz 2H), 1.63(d, *J* = 8.0 Hz 3H), 1.30 (t, *J* = 8.0 Hz 3H).

### Example 25: (S)-N-(2-chloro-6-fluorophenyl)-5-fluoro-4-(5-(hydroxymethyl)-1-methyl-1H-pyrazol-3-yl)-2-((1,1,1-trifluoropropan-2-yl)oxy)benzamide.

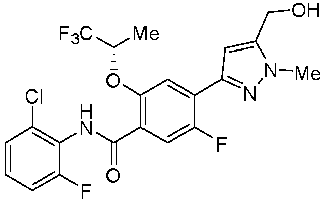

Step A. methyl 2-methyl-5-oxo-2,5-dihydro-1H-pyrazole-3-carboxylate. To a solution of methylhydrazine (810 mg, 7.04 mmol, 926 µL, 40% in water) and TEA (712 mg, 7.04 mmol, 979 µL) in MeOH (10 mL) and H₂O (4 mL) was added dimethyl but-2-ynedioate (1.00 g, 7.04 mmol). The mixture was stirred at 70 °C for 12 hrs. The reaction mixture was diluted with H₂O (20 mL) and then adjusted the pH to 7 with HCl (0.5 N). The mixture was extracted with ethyl acetate (10 mL×5). The combined organic layers were washed with brine (30 mL), dried over anhydrous Na₂SO₄, filtered and concentrated in vacuo. The residue was purified by column chromatography (SiO₂, petroleum ether/ethyl acetate=5/1 to 2/1) to give title compound as a yellow solid. MS (ESI): mass calcd. For C₈H₈N₂O₃, 156.1; m/z found, 157.2 [M+H]⁺. ¹H NMR (400MHz, CDCl₃) δ = 6.16 (s, 1 H), 4.02 (s, 3H), 3.89 (s, 3H).

Step B. methyl 1-methyl-3-(((trifluoromethyl)sulfonyl)oxy)-1H-pyrazole-5-carboxylate. To a solution of methyl 2-methyl-5-oxo-1H-pyrazole-3-carboxylate (580 mg, 3.71 mmol) and Py (2.94 g, 37.15 mmol, 3.00 mL) in DCM (15 mL) was added Tf₂O (1.26 g, 4.46 mmol, 735 µL) at 0 °C under N₂. The mixture was stirred at 20 °C for 2 hrs. Additional Tf₂O (1.05 g, 3.71 mmol, 612.89 µL) was added to the mixture at 0 °C. The mixture was stirred at 20 °C for 16 hrs. The reaction mixture was adjusted pH to 3 with HCl (1 N), and then extracted with ethyl acetate (40 mL×3). The combined organic layers were washed with brine (40 mL), dried over Na₂SO₄, filtered and concentrated in vacuo. The residue was purified by column chromatography (SiO₂, petroleum ether/ethyl acetate=200/1 to 100/1) to give the title compound as yellow oil. MS (ESI): mass calcd. For C₇H₇F₃N₂O₅S, 288.0; m/z found, 289.4[M+H]⁺. ¹H NMR (400MHz, CDCl₃) δ = 6.67 (s, 1 H), 4.16 (s, 3H), 3.92 (s, 3H).

Step C. (S)-methyl 3-(4-((2-chloro-6-fluorophenyl)carbamoyl)-2-fluoro-5-((1,1,1-trifluoropropan-2-yl)oxy)phenyl)-1-methyl-1H-pyrazole-5-carboxylate. A mixture of (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide(Intermediate 1, 150 mg), methyl 1-methyl-3-(((trifluoromethyl)sulfonyl)oxy)-1H-pyrazole-5-carboxylate (132.69 mg, 460.42 µmol), CsF (107.60 mg, 708.34 µmol, 26.12 µL) and Pd(dppf)Cl₂ (25.91 mg, 35.42 µmol) in dioxane (2 mL) and H₂O (0.2 mL) was degassed and purged with N₂ for 3 times. The mixture was stirred at 80 °C for 12 hr under N₂ atmosphere. The reaction mixture was diluted with H₂O (10 mL) and extracted with ethyl acetate (10 mL×3). The combined organic layers were washed with brine (10 mL×2), dried over anhydrous Na₂SO₄, filtered and concentrated in vacuo. The residue was purified by column chromatography (SiO₂, petroleum ether/ethyl acetate=15/1 to 10/1) to give the title compound as white solid. MS (ESI): mass calcd. For C₂₂H₁₇ClF₅O₃O₄, 517.1; m/z found, 518.2 [M+H]⁺. ¹H NMR (400MHz, CDCl₃) δ = 9.11 (s, 1H), 8.11 (d, *J* = 11.6 Hz, 1H), 7.77 (d, *J* = 5.6 Hz, 1H), 7.39 (d, *J* = 4.0 Hz, 1H), 7.31 - 7.29 (m, 1H), 7.25 - 7.24 (m, 1H), 7.16 - 7.13 (m, 1H), 5.12 - 5.06 (m, 1H), 4.29 (s, 3H), 3.94 (s, 3H), 1.69 (s, 3H)

Step D. (S)-N-(2-chloro-6-fluorophenyl)-5-fluoro-4-(5-(hydroxymethyl)-1-methyl-1H-pyrazol-3-yl)-2-((1,1,1-trifluoropropan-2-yl)oxy)benzamide. To a solution of (S)-N-(2-chloro-6-fluorophenyl)-5-fluoro-4-(5-(hydroxymethyl)-1-methyl-1H-pyrazol-3-yl)-2-((1,1,1-trifluoropropan-2-yl)oxy)benzamide (120 mg, 205 µmol) in THF (2 mL) was added LiBH₄ (2 M in THF, 411 µL) at 0 °C. The mixture was stirred at 20 °C for 3 hrs. Additional LiBH₄ (2 M in THF, 411 µL) was added to the mixture at 0 °C. The mixture was stirred at 20 °C for 1 hrs. The reaction mixture was poured into iced aqueous HCl (0.5 N, 10 mL) and extracted with ethyl acetate (10 mL×3). The combined organic layers were washed with brine (20 mL×2), dried over anhydrous Na₂SO₄, filtered and concentrated in vacuo. The residue was purified by prep-HPLC (method A) to give the title compound as white solid. MS (ESI): mass calcd. For C₂₁H₁₇ClF₅N₃O₃, 489.1; m/z found, 490.1 [M+H]⁺. ¹H NMR (400MHz, CDCl₃) δ = 9.13(s, 1H), 8.07(d, *J* = 11.6 Hz, 1H), 7.77 (d, *J* = 5.6 Hz, 1H), 7.31 - 7.28(m, 1H), 7.25 - 7.24 (m, 1H), 7.15 - 11(m, 1H), 6.78(d, *J* = 4.0 Hz, 1H), 5.14- 5.10 (m, 1H), 5.08(d, *J* = 6.4 Hz, 2H), 4.02 (s, 3H), 1.77 (t, *J* = 6.0 Hz,1H), 1.67 (d, *J* = 6.4 Hz, 3H).

### Example 26: (S)-N-(2-chloro-6-fluorophenyl)-4-(4-ethyl-5-(hydroxymethyl)oxazol-2-yl)-5-fluoro-2-((1,1,1-trifluoropropan-2-yl)oxy)benzamide.

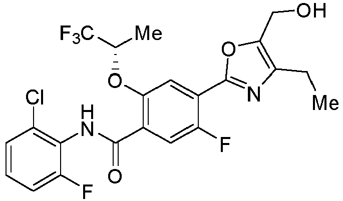

Step A. ethyl 2-chloro-3-oxopentanoate. To a stirred of ethyl 3-oxopentanoate (30 g, 208 mmol) was added sulfuryl chloride (28.0 g, 208 mmol, 20.8 mL) dropwise at 0 °C under N₂. The mixture was stirred at 20°C for 12 hr. The combined organic phase was washed with brine (50 mL×2), dried with anhydrous Na₂SO₄, filtered and concentrated in vacuo. The residue was purified by column chromatography (SiO₂, petroleum ether/ethyl acetate=1/0) to give the title compound as colourless oil.

Step B. ethyl 2-amino-4-ethyloxazole-5-carboxylate. A mixture of urea (5.04 g, 84.0 mmol, 4.50 mL) and ethyl 2-chloro-3-oxopentanoate (10.0 g, 56.0 mmol) in EtOH (100 mL) was stirred at 80°C for 40 hrs. TLC (petroleum ether/ethyl acetate=10/1) indicated 70% of starting material remained and one new spot with larger polarity was detected. The mixture was concentrated in vacuo. The residue was poured into saturated NaHCO₃ (500 mL) and extracted with ethyl acetate (100 mL×2). The combined organic phase was washed with brine (200 mL×2), dried with anhydrous Na₂SO₄, filtered and concentrated in vacuo. The residue was purified by column chromatography (SiO₂, petroleum ether/ethyl acetate=10/1 to 1/1) to give the title compound as light yellow solid. ¹H NMR (400MHz, CDCl₃) δ = 5.68 (s, 2H), 4.35-4.29 (m, 2H), 2.80-2.73 (m, 2H), 1.36 (t, *J*=5.6 Hz, 3H), 1.20 (t, *J*=5.6 Hz, 3H).

Step C. ethyl 2-bromo-4-ethyloxazole-5-carboxylate. To a solution of ethyl 2-amino-4-ethyloxazole-5-carboxylate (1.5 g, 8.1 mmol) and CuBr₂ (2.18 g, 9.77 mmol, 458 µL) in MeCN (20mL) was added tert-butyl nitrite (1.26 g, 12.2 mmol, 1.45 mL) at 0 °C. The reaction mixture was stirred at 25 °C for 12 hr. The reaction mixture was concentrated under reduced pressure to remove solvent. The residue was diluted with water (60 mL) and extracted with ethyl acetate (40 mL × 3). The combined organic layers were washed with brine, dried over anhydrous Na₂SO₄, filtered and concentrated in vacuo. The residue was purified by column chromatography (SiO₂, petroleum ether/ethyl acetate=200/1 to 50/1) . Additional purification by prep-HPLC(Method E) gave the title compound. MS (ESI): mass calcd. for C₈H₁₀NO₃Br, 247.0/249.0; m/z found, 247.9/249.9 [M+H]⁺.

Step D. (S)-ethyl 2-(4-((2-chloro-6-fluorophenyl)carbamoyl)-2-fluoro-5-((1,1,1-trifluoropropan-2-yl)oxy)phenyl)-4-ethyloxazole-5-carboxylate. The title compound was prepared in a manner analogous to Example 1, Step E however using ethyl 2-bromo-4-ethyloxazole-5-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₄H₂₀Cl F₅N₂O₅, 546.1; m/z found, 547.0 [M+H]⁺.

Step E. (S)-N-(2-chloro-6-fluorophenyl)-4-(4-ethyl-5-(hydroxymethyl)oxazol-2-yl)-5-fluoro-2-((1,1,1-trifluoropropan-2-yl)oxy)benzamide. To a solution of (S)-ethyl 2-(4-((2-chloro-6-fluorophenyl)carbamoyl)-2-fluoro-5-((1,1,1-trifluoropropan-2-yl)oxy)phenyl)-4-ethyloxazole-5-carboxylate (80 mg, 146 µmol) in THF (2 mL) was added LiBH₄ (2 M in THF, 439 µL) at 0 °C. The mixture was stirred at 25 °C for 8 h. The reaction mixture was stirred for another 6 h. The reaction mixture combined with another batch (30 mg) was diluted with water (10 mL) and extracted with ethyl acetate (10 mL×3). The combined organic layers were washed with brine (10 mL×1), dried over anhydrous Na₂SO₄, filtered and concentrated in vacuo. The residue was purified by prep-HPLC (method A) to give the title compound as a white solid. MS (ESI): mass calcd. for C₂₂H₁₈Cl F₅N₂O₄, 504.1; m/z found, 505.1 [M+H]⁺. ¹H NMR (400 MHz, CDCl₃) δ 9.08 (s, 1H), 8.15 (d, *J* = 11.1 Hz,1H), 7.74 (d, *J* = 5.4 Hz, 1H), 7.32 - 7.28 (m, 1H), 7.26 - 7.23 (m, 1H), 7.17 - 7.11 (m, 1H), 5.12-5.08 (m 1H), 4.76 (br s, 2H), 2.67 (q, *J* = 7.5 Hz, 2H), 1.89 (br s, 1H), 1.68 (d, *J* = 6.5 Hz, 3H), 1.32 (t, *J* = 7.6 Hz, 3H).

### Example 27: (S)-N-(2-chloro-6-fluorophenyl)-4-(5-ethyl-4-(hydroxymethyl)oxazol-2-yl)-5-fluoro-2-((1,1,1-trifluoropropan-2-yl)oxy)benzamide.

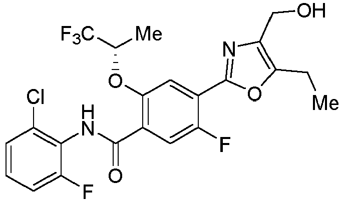

Step A. ethyl 3-bromo-2-oxopentanoate. To a solution of ethyl 2-oxopentanoate (10 g, 69.4 mmol) in CHCl₃ (100 mL) was added Br₂ (11.1 g, 69.4 mmol, 3.58 mL) at 0 °C. The mixture was stirred at 15 °C for 18 hrs. TLC (petroleum ether/ethyl acetate=10/1) indicated starting material was consumed completely and one main new spot formed. The reaction mixture was diluted with DCM (200 mL) and then washed with NaHCO₃ (sat. aq. 200 mL×2), H₂O (200 mL) and brine (200 mL×2). The combined organic layers were washed dried over anhydrous Na₂SO₄, filtered and concentrated in vacuo to give the title compound as yellow oil. ¹H NMR (400MHz, CDCl₃) δ = 5.00 - 4.96 (m, 1H), 4.41 - 4.36 (m, 2H), 2.16 - 2.11 (m, 1H), 2.06 - 2.02 (m, 1H), 1.40 (t, *J* = 7.2 Hz, 3H), 1.08 (t, *J* = 7.2 Hz, 3H)

Step B. (S)-ethyl 2-(4-((2-chloro-6-fluorophenyl)carbamoyl)-2-fluoro-5-((1,1,1-trifluoropropan-2-yl)oxy)phenyl)-5-ethyloxazole-4-carboxylate. The title compound was prepared according to the representative procedures of Example 26, Steps A-E, except substituting ethyl 3-bromo-2-oxopentanoate for ethyl 2-chloro-3-oxopentanoate. MS (ESI): mass calcd. for C₂₄H₂₀ClF₅N₂O₅, 546.1; m/z found, 547.2 [M+ H]⁺. ¹H NMR (400MHz, CDCl₃) δ = 9.08 (s, 1H), 8.16 (d, *J* = 7.2 Hz, 1H), 7.83 (d, *J* = 5.6 Hz, 1H), 7.31 - 7.28 (m, 1H), 7.26 - 7.23 (m, 1H), 7.17 - 7.12 (m, 1H), 5.20 - 6.13 (m, 1H), 4.49 - 4.43 (m, 2H), 3.21 - 3.15 (s, 2H), 1.68 - 1.66 (m, 3H), 1.47 - 1.43 (m, 3H), 1.40 - 1.3 (m, 3H).

### Example 28: (S)-N-(2-chloro-6-fluorophenyl)-5-fluoro-4-(1-(hydroxymethyl)-5,6,7,8-tetrahydroimidazo[1,5-a]pyridin-3-yl)-2-((1,1,1-trifluoropropan-2-yl)oxy)benzamide.

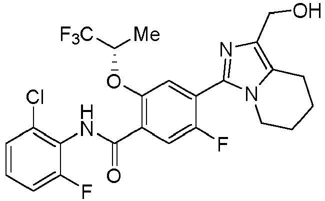

Step A. ethyl 5,6,7,8-tetrahydroimidazo[1,5-a]pyridine-1-carboxylate. A solution of ethyl imidazo[1,5-a]pyridine-1-carboxylate (2.0 g, 10.5 mmol) in EtOH (120 mL) was degassed by N₂ bubbling for 10 min before the addition of HCl (30.6 g, 318.92 mmol, 30.0 mL, 38% purity) and Pd/C (200 mg, 10% purity). The resulting mixture was stirred at 60 °C under H₂ (40 psi) for 4 h. The mixture was filtered with diatomite and concentered in vacuo. The residue was purified by column chromatography (SiO₂, petroleum ether/ethyl acetate=10/1 to 0/1) to give the title compound as a yellow solid. MS (ESI): mass calcd. For C₁₀H₁₄N₂O₂, 194.1; m/z found, 195.1 [M+H]⁺. ¹H NMR (400MHz, DMSO-*d₆*) δ = 7.59 (s, 1H), 4.19 (q, *J* = 7.2 Hz, 2H), 4.02 - 3.97 (m, 2H), 2.94 (t, *J* = 6.4 Hz, 2H), 1.90 - 1.74 (m, 4H), 1.26 (t, *J* = 7.2 Hz, 3H).

Step B. ethyl 3-bromo-5.6.7.8-tetrahydroimidazo[1,5-a]pyridine-1-carboxylate. To a solution of ethyl 5,6,7,8-tetrahydroimidazo[1,5-a]pyridine-1-carboxylate (500 mg, 2.57 mmol) in MeCN (10 mL) was added NBS (504 mg, 2.83 mmol). The mixture was stirred at 80 °C for 16 h. The mixture was poured into water (60 mL). The aqueous phase was extracted with ethyl acetate (50 mL×2). The combined organic layers were washed with brine (10 mL×2), dried over Na₂SO₄, filtered and concentrated in vacuo. The residue was purified by column chromatography (SiO₂, petroleum ether/ethyl acetate=10/1 to 3/1) to give the title compound as a white solid. MS (ESI): mass calcd. For C₁₀H₁₃BrN₂O₂, 272.0; m/z found, 273.0 [M+H]⁺.

Step C. (S)-ethyl 3-(4-((2-chloro-6-fluoropheyl)carbamoyl)-2-fluoro-5-((1,1,1-trifluoropropan-2-yl)oxy)phenyl)-5,6,7,8-tetrahydroimidazo[1.5-a]pyridine-1-carboxylate. To a solution of (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 150 mg), ethyl 3-bromo-5,6,7,8-tetrahydroimidazo[1,5-a]pyridine-1-carboxylate (136.3 mg, 499.1 µmol), XPhos Pd G₃ (32.5 mg, 38.4 µmol), Na₂CO₃ (89.5 mg, 844 µmol) and H₂O (100 mg, 5.55 mmol, 100 µL) in dioxane (1 mL) were degassed with N₂ and heated to 60 °C for 16 h. The mixture was poured into water (30 mL). The aqueous phase was extracted with ethyl acetate (30 mL×2). The combined organic layers were washed with brine (10 mL×2), dried over Na₂SO₄, filtered and concentrated in vacuo. The residue was purified by column chromatography (SiO₂, petroleum ether/ethyl acetate=10/1 to 3/1) to give the title compound as a white solid. MS (ESI): mass calcd. For C₂₆H₂₃ClF₅N₃O₄, 571.1; m/z found, 572.1 [M+H]⁺.

Step D. (S)-N-(2-chloro-6-fluorophenyl)-5-fluoro-4-(1-(hydroxymethyl)-5,6,7,8-tetrahydroimidazo[1,5-a]pyridin-3-yl)-2-((1,1,1-trifluoropropan-2-yl)oxy)benzamide. To a solution of (S)-ethyl 3-(4-((2-chloro-6-fluorophenyl)carbamoyl)-2-fluoro-5-((1,1,1-trifluoropropan-2-yl)oxy)phenyl)-5,6,7,8-tetrahydroimidazo[1,5-a]pyridine-1-carboxylate (44 mg, 77 µmol) in THF (1 mL) was added LiBH₄ (2 M in THF, 154 µL) at 0 °C. The mixture was stirred at 0 °C for 2 h. The mixture was poured into HCl (1 N, 20 mL). The mixture was filtered and extracted with ethyl acetate (20 mL×2). The combined organic phase was washed with NaHCO₃ (10 mL×2) and brine (10 mL×2), dried with anhydrous Na₂SO₄, filtered and concentrated in vacuo. The residue was purified by prep-HPLC (method A) to give the title compound as an off-white solid. MS (ESI): mass calcd. For C₂₄H₂₁ClF₅N₃O₃, 529.1; m/z found, 530.1 [M+H]⁺. ¹H NMR (400MHz, CDCl₃) δ = 9.09 (s, 1H), 8.11 (d, *J* = 10.6 Hz, 1H), 7.33 - 7.28 (m, 2H), 7.26 - 7.21 (m, 1H), 7.16 - 7.11 (m, 1H), 5.08 (td, *J* = 6.4, 12.4 Hz, 1H), 4.62 (s, 2H), 3.96 - 3.87 (m, 2H), 2.90 (t, *J* = 6.0 Hz, 2H), 1.96 - 1.89 (m, 4H), 1.66 - 1.64 (m, 3H).

### Example 29: (S)-N-(2-chloro-6-fluoropheyl)-5-fluoro-4-(5-(hydroxymethyl)pyrazin-2-yl)-2-((1,1,1-trifluoropropan-2-yl)oxy)benzamide.

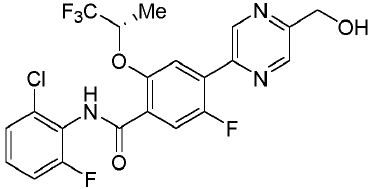

The title compound was prepared in a manner analogous to Example 1, Step E however using (5-bromopyrazin-2-yl)methanol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₅ClF₅N₃O₃, 487.1; m/z found, 488.1 [M+H]⁺. ¹H NMR (400MHz, CDCl₃) δ = 9.20 (t, *J* = 1.8 Hz, 1H), 9.10 (s, 1H), 8.76 (d, *J* = 1.6 Hz, 1H), 8.18 (d, *J* = 11.6 Hz, 1H), 7.90 (d, *J* = 5.8 Hz, 1H), 7.32 - 7.29 (m, 1H), 7.26 - 7.22 (m, 1H), 7.16 - 7.14 (m, 1H), 5.10 (td, *J* = 6.2, 12.2 Hz, 1H), 4.94 (d, *J* = 4.8 Hz, 2H), 3.02 (t, *J* = 5.6 Hz, 1H), 1.69 (d, *J* = 6.4 Hz, 3H).

### Example 30: (S)-N-(2-chloro-6-fluorophenyl)-5-fluoro-4-(6-hydroxypyrazin-2-yl)-2-((1,1,1-trifluoropropan-2-yl)oxy)benzamide.

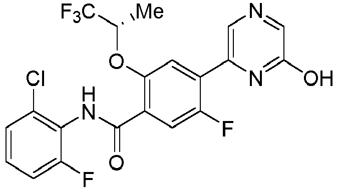

The title compound was prepared in a manner analogous to Example 1, Step E however using 6-bromopyrazin-2-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₀H₁₃ClF₅N₃O₃, 473.1; m/z found, 474.1 [M+H]⁺. ¹H NMR (400MHz, CDCl₃) δ = 9.03 (s, 1H), 8.16 - 8.08 (m, 3H), 7.56 (d, *J* = 5.6 Hz, 1H), 7.26 - 7.19 (m, 2H), 7.11 - 7.06 (m, 1H), 5.50 (td, *J* = 6.0, 12.2 Hz, 1H), 1.67 (d, *J* = 6.4 Hz, 3H).

### Example 31: (S)-N-(2-chloro-4-methylpyridin-3-yl)-5-fluoro-4-(5-methylpyridin-2-yl)-2-((1,1,1-trifluoropropan-2-yl)oxy)benzamide.

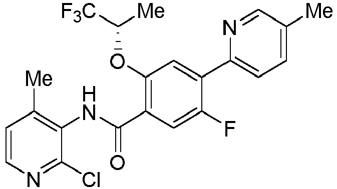

Step A. (S)-methyl 5-fluoro-4-(5-methylpyridin-2-yl)-2-((1,1,1-trifluorporopan-2-yl)oxy (benzoate. To a mixture of (S)-methyl 5-fluoro-4-(4,4,5,5-tetramethyl-1,3,2-dioxaborolan-2-yl)-2-((1,1,1-trifluoropropan-2-yl)oxy)benzoate (Intermediate 2, 0.40 g, 857 µmol), 2-bromo-5-methylpyridine (221 mg, 1.29 mmol), Na₂CO₃ (200 mg, 1.88 mmol) in dioxane (5 mL) and H₂O (0.5 mL) was added Pd(dppf)Cl₂ (62.7 mg, 85.7 µmol) under N₂. The mixture was stirred at 70 °C for 2 hrs under N₂ atmosphere. The mixture was poured into water (10 mL) and extracted with ethyl acetate (10 mL×2). The combined organic phase was washed with brine (15 mL×2), dried with anhydrous Na₂SO₄, filtered and concentrated in vacuo. The residue was purified by prep-TLC (SiO₂, petroleum ether / ethyl acetate = 3/1) to give the title compound as a white solid. MS (ESI): mass calcd for C₁₇H₁₅P₄NO₃, 357.1; m/z found, 358.5 [M+H]⁺.

Step B. (S)-N-(2-chloro-4-methylpyridin-3-yl)-5-fluoro-4-(5-methylpyridin-2-yl)-2-((1,1,1-trifluoropropan-2-yl)oxy)benzamide. To a stirred solution of 2-chloro-4-methylpyridin-3-amine (160 mg, 1.12 mmol) in DCM (1.5 mL) was added AlMe₃ (2 M in toluene, 420 µL) under N₂. The mixture was stirred at 16 °C for 0.5 hrs, followed by treatment with (S)-methyl 5-fluoro-4-(5-methylpyridin-2-yl)-2-((1,1,1-trifluoropropan-2-yl)oxy)benzoate (100 mg, 280 µmol) and stirred at 60 °C for 12 hours. The stirred reaction was added 0.5 mL 1N HCl and stirred for 2 min. The mixture was poured into water (15 mL) and extracted with DCM (15 mL×2). The combined organic phase was washed with brine (20 mL×2), dried with anhydrous Na₂SO₄, filtered and concentrated in vacuo. The residue was purified by prep-HPLC (METHOD A) to give the title compound as a white solid. MS (ESI): mass calcd for C₂₂H₁₈ClF₄N₃O₂, 467.1; m/z found, 468.1 [M+H]⁺. ¹H NMR (400 MHz, CDCl₃) δ = 9.25 (s, 1H), 8.61 - 8.56 (m, 1H), 8.22 (d, *J* = 5.0 Hz, 1H), 8.10 (d, *J* = 11.9 Hz, 1H), 7.92 - 7.86 (m, 2H), 7.65 - 7.62 (m, 1H), 7.20 (d, *J* = 5.0 Hz, 1H), 5.20 - 5.14 (m, 1H), 2.43 (s, 3H), 2.37 (s, 3H), 1.71 (d, *J* = 6.4 Hz, 3H).

### Example 32: (S)-N-(2-chloro-4-methylpyridin-3-yl)-4-(5-cyclopropylpyrazin-2-yl)-5-fluoro-2-((1,1,1 -trifluoropropan-2-yl)oxy)benzamide.

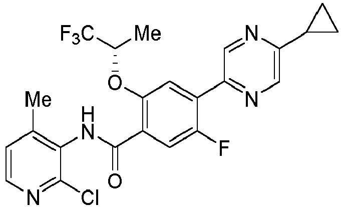

The title compound was prepared in a manner analogous to Example 31, Steps A-B, however substituting 2-bromo-5-cyclopropylpyrazine for 2-bromo-5-methylpyridine in Step A. MS (ESI): mass calcd. For C₂₃H₁₉ClF₄N₄O₂, 494.1; m/z found, 495.1 [M+H]⁺.¹H NMR (400MHz, CDCl₃) δ = 9.22 (s, 1H), 9.06 (t, *J*=1.8 Hz, 1H), 8.61 (d, *J*=1.5 Hz, 1H), 8.23 (d, *J*=4.9 Hz, 1H), 8.13 (d, *J*=11.6 Hz, 1H), 7.87 (d, *J*=5.6 Hz, 1H), 7.21 (d, *J*=5.0 Hz, 1H), 5.15 - 5.12 (m, 1H), 2.37 (s, 3H), 2.22 - 2.11 (m, 1H), 1.72 (d, *J*=6.5 Hz, 3H), 1.23 - 1.12 (m, 4H).

### Example 33: (S)-N-(3-chloro-5-methyl-1H-pyrazol-4-yl)-5-fluoro-4-(5-methylpyridin-2-yl)-2-((1,1,1-trifluoropropan-2-yl)oxy)benzamide.

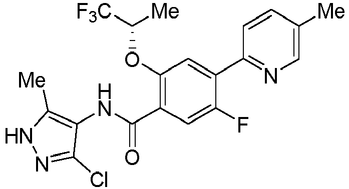

Step A. 3-chloro-5-methyl-1H-pyrazol-4-amine. To a solution of 3-chloro-5-methyl-4-nitro-1H-pyrazole (2.00 g, 12.4 mmol) in EtOH (40 mL) and H₂O (20 mL) were added NH₄Cl (3.97 g, 74.3 mmol) and then Fe (3.46 g, 61.9 mmol). The mixture was stirred at 20 °C for 16 hr. The mixture was filtered and washed with ethyl acetate (20 mL×2). The mixture was diluted with water (50 mL) and extracted with ethyl acetate (30 mL × 2). The combined organic layers were washed with brine (30 mL × 2), dried over Na₂SO₄, filtered and concentrated in vacuo to give the title compound as a brown solid. ¹H NMR (400MHz, CDCl₃) δ = 12.06 (s, 2H), 3.55 (s, 1H), 2.07 (s, 3H).

Step B. (S)-N-(3-chloro-5-methyl-1H-pyrazol-4-yl)-5-fluoro-4-(5-methylpyridin-2-yl)-2-((1,1,1-trifluoropropan-2-yl)oxy)benzamide. To a stirred solution of 3-chloro-5-methyl-1H-pyrazol-4-amine (49.71 mg, 377.83 µmol) in DCM (1 mL) was added AlMe₃ (2 M in toluene, 146.94 µL) under N₂ and the mixture was stirred at 20 °C for 0.5 hrs. The reaction was added (S)-methyl 5-fluoro-4-(5-methylpyridin-2-yl)-2-((1,1,1-trifluoropropan-2-yl)oxy)benzoate (Example 31, Step A, 30 mg, 84 µmol) and stirred at 60 °C for 19.5 hours. The mixture was added AlMe₃ (2 M, 105 µL) and THF (1 mL) at 20 °C. The mixture was stirred at 60 °C for 5 hrs. To the stirred reaction was added 0.5 mL 1N HCl and stirred for 2 min. The mixture was poured into ethyl acetate (40 mL) and dried with anhydrous Na₂SO₄, filtered and concentrated in vacuo. The residue was purified by prep-HPLC (METHOD A) to give the title compound as a white solid. MS (ESI): mass calcd for C₂₀H₁₇ClF₄N₄O₂, 456.1; m/z found, 457.1 [M+H]⁺. ¹H NMR (400 MHz, DMSO-*d₆*) δ = 12.84 (s, 1H), 9.44 (s, 1H), 8.61 (s, 1H), 7.82 - 7.74 (m, 3H), 7.50 (d, *J* = 10.8 Hz, 1H), 5.43 - 5.37 (m, 1H), 2.38 (s, 3H), 2.16 (s, 3H), 1.47 (d, *J* = 6.4 Hz, 3H).

### Example 34: (S)-N-(2-chloro-6-fluorophenyl)-4-(5-ethyl-4-(hydroxymethyl)-1-methyl-1H-imidazol-2-yl)-5-fluoro-2-((1,1,1-trifluoropropan-2-yl)oxy)benzamide.

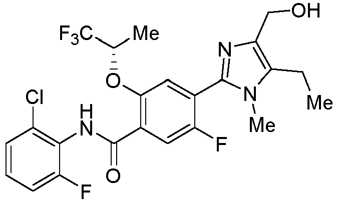

Step A. (S)-methyl 2-(4-((2-chloro-6-fluorophenyl)carbamoyl)-2-fluoro-5-((1,1,1-trifluoropropan-2-yl)oxy)phenyl)-5-ethyl-1-methyl-1H-imidazole-4-carboxylate. To a mixture of (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 50 mg) and methyl 2-bromo-5-ethyl-1-methyl-1H-imidazole-4-carboxylate (Example 24, Step A, 29.17 mg, 118.06 µmol) in dioxane (2 mL) and H₂O (0.2 mL) was added Na₂CO₃ (25.0 mg, 236 µmol) followed by Pd(dppf)Cl₂ (8.6 mg, 11.8 µmol) in one portion under N₂. The mixture was stirred at 80 °C for 12 hours. The mixture was poured into water (30 mL). The aqueous phase was extracted with ethyl acetate (20 mL×3). The combined organic phase was washed with brine (15 mL×2), dried with anhydrous Na₂SO₄, filtered and concentrated in vacuo. The residue was purified by prep-TLC (petroleum ether/ethyl acetate=1/1) to give the title compound as yellow solid. MS (ESI): mass calcd. for C₂₄H₂₁ClF₅N₃O₄, 545.1, m/z found, 546.2 [M+H]⁺.

Step B. (S)-N-(2-chloro-6-fluorophenyl)-4-(5-ethyl-4-(hydroxymethyl)-1-methyl-1H-imidazol-2-yl)-5-fluoro-2-((1,1,1-trifluoropropan-2-yl)oxy)benzamide. To a mixture of (S)-methyl 2-(4-((2-chloro-6-fluorophenyl)carbamoyl)-2-fluoro-5-((1,1,1-trifluoropropan-2-yl)oxy)phenyl)-5-ethyl-1-methyl-1H-imidazole-4-carboxylate (35 mg, 50 µmol) in THF (1 mL) was added LiAlH₄ (3.6 mg, 96 µmol) in one portion at 0 °C under N₂. The mixture was stirred at 25 °C for 2 hours. The mixture was poured into 1N HCl (5 mL). The aqueous phase was extracted with ethyl acetate (8mL×4). The combined organic phase was washed with brine (3 mL×2), dried with anhydrous Na₂SO₄, filtered and concentrated in vacuo. The residue was purified by prep-HPLC (method A) to give the title compound as white solid. MS (ESI): mass calcd. for C₂₃H₂₁ClF₅N₃O₃, 517.12, m/z found, 518.1 [M+H]⁺. ¹H NMR (400 MHz, CDCl₃) δ = 9.10 (s, 1H), 8.14 (d, *J* = 10.5 Hz, 1H), 7.37 - 7.33 (m, 1H), 7.33 - 7.28 (m, 2H), 7.18 - 7.11 (m, 1H), 5.08 (s, 1H), 4.66 (s, 1H), 3.61 - 3.47 (m, 3H), 2.83 - 2.64 (m, 2H), 1.67 - 1.65 (m, 3H) 1.30 - 1.24 (m, 3H).

### Example 35: (S)-N-(2-chloro-4-methylpyridin-3-yl)-4-(5-ethyl-4-(hydroxymethyl)thiazol-2-yl)-5-fluoro-2-((1,1,1-trifluoropropan-2-yl)oxy)benzamide.

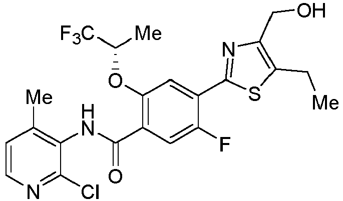

Step A. (2-bromo-5-ethylthiazol-4-yl)methanol. To a solution of methyl 2-bromo-5-ethylthiazole-4-carboxylate (400 mg, 1.60 mmol) in THF (5 mL) was added LiBH₄ (2 M in THF, 1.60 mL) at 0°C. The mixture was stirred at 25 °C for 5 h. The reaction mixture was quenched by addition HCl (1 M, 2 mL) at 0 °C, and then addition ethyl acetate (30 mL). The combined organic layers were dried over Na₂SO₄, filtered and concentrated in vacuo. The residue was purified by prep-TLC (SiO₂, petroleum ether/ethyl acetate = 3/2) to give the title compound as a yellow oil. ¹H NMR (400MHz, CDCl₃) δ = 4.63 (s, 2H), 2.95-2.75 (m, 2H), 1.28 (t, *J*=8.0 Hz, 3H).

Step B. (S)-methyl 4-(5-ethyl-4-(hydroxymethyl)thiazol-2-yl)-5-fluoro-2-((1,1,1-trifluoropropan-2-yl)oxy)benzoate. To a mixture of (S)-methyl 5-fluoro-4-(4,4,5,5-tetramethyl-1,3,2-dioxaborolan-2-yl)-2-((1,1,1-trifluoropropan-2-yl)oxy)benzoate (Intermediate 2, 300 mg, 642 µmol), (2-bromo-5-ethyl-thiazol-4-yl)methanol (171 mg, 771 µmol) and CsF (195 mg, 1.29 mmol, 47 µL) in dioxane (5 mL) and H₂O (1 mL) was added Pd(dppf)Cl₂ (47.0 mg, 64.3 µmol) at 15 °C under N₂. The mixture was stirred at 60 °C for 12 h. The reaction mixture with another two batches (50 mg, 50 mg scale) was diluted with ethyl acetate (30 mL), then dried over Na₂SO₄, filtered and concentrated in vacuo. The residue was purified by column chromatography (SiO₂, petroleum ether/ethyl acetate=100/1 to 30/1) to give the title compound (240 mg, 471.31 µmol, 80% purity) as yellow solid. ¹H NMR (400MHz, CDCl₃) δ = 7.94 (d, *J*=5.9 Hz, 1H), 7.68 (d, *J*=11.0 Hz, 1H), 4.87 - 4.74 (m, 3H), 3.93 (s, 3H), 2.93 (q, *J*=7.5 Hz, 2H), 1.59 (d, *J*=6.6 Hz, 3H), 1.37 (d, *J*=6.0 Hz, 3H).

Step C. (S)-N-(2-chloro-4-methylpyridin-3-yl)-4-(5-ethyl-4-(hydroxymethyl)thiazol-2-yl)-5-fluoro-2-((1,1.1-trifluoropropan-2-yl)oxy)benzamide. To a solution of 2-chloro-4-methyl-pyridin-3-amine (89.6 mg, 628 µmol) in DCM (1.5 mL) was added AlMe₃ (2 M in toluene, 236 µL) at 15 °C under N₂. The mixture was stirred at 15 °C for 0.5 h. (S)-methyl 4-(5-ethyl-4-(hydroxymethyl)thiazol-2-yl)-5-fluoro-2-((1,1,1-trifluoropropan-2-yl)oxy)benzoate (80 mg, 157 µmol) was added. The mixture was stirred at 60 °C for 12 h. The reaction mixture was quenched by addition 1 N HCl (20 mL), and then extracted with DCM (15 mL × 2). The combined organic layers were dried over Na₂SO₄, filtered and concentrated in vacuo. The residue was purified by prep-HPLC (method A) to give the title compound as white solid. MS (ESI): mass calcd. For C₂₂H₂₀ClF₄N₃O₃S, 517.1; m/z found, 518.1 [M+H]⁺. ¹H NMR (400MHz, CDCl₃) δ = 9.22 (s, 1H), 8.23 (d, *J*=4.9 Hz, 1H), 8.14 (d, *J*=11.5 Hz, 1H), 8.01 (d, *J*=5.4 Hz, 1H), 7.21 (d, *J*=5.0 Hz, 1H), 5.23 - 5.12 (m, 1H), 4.80 (d, *J*=5.6 Hz, 2H), 2.95 (q, *J*=7.6 Hz, 2H), 2.41 (t, *J*=5.7 Hz, 1H), 2.36 (s, 3H), 1.73 (s, 1H), 1.76 - 1.69 (m, 1H), 1.38 (t, *J*=7.6 Hz, 3H).

### Example 36: (S)-N-(2-chloro-4-methylpyridin-3-yl)-5-fluoro-4-(2-(hydroxymethyl)-1-methyl-1H-imidazol-4-yl)-2-((1,1,1-trifluoropropan-2-yl)oxy)benzamide.

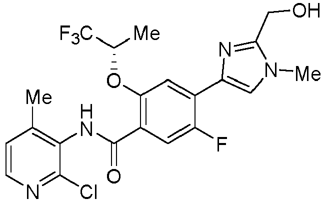

Step A. (4-bromo-1-methyl-1H-imidazol-2-yl)methanol. To a solution of methyl methyl 4-bromo-1-methyl-1H-imidazole-2-carboxylate (360 mg, 1.64 mmol) in THF (3 mL) was added LiBH₄ (2 M in THF, 986 µL) at 0 °C. The mixture was stirred at 25 °C for 2h. The reaction mixture was quenched by addition HCl (1 M, 2mL) at 0 °C, and then addition ethyl acetate (30 mL). The combined organic layers were dried over Na₂SO₄, filtered and concentrated in vacuo. The residue was purified by prep-TLC (SiO₂, petroleum ether/ ethyl acetate = 1/1) to give the title compound was obtained as a white solid.

Step B. (S)-methyl 5-fluoro-4-(2-(hydroxymethyl)-1-methyl-1H-imidazol-4-yl)-2-((1,1,1-trifluoropropan-2-yl)oxy)benzoate. To a mixture of (S)-methyl 5-fluoro-4-(4,4,5,5-tetramethyl-1,3,2-dioxaborolan-2-yl)-2-((1,1,1-trifluoropropan-2-yl)oxy)benzoate (Intermediate 2, 50 mg, 107.10 µmol), 4-bromo-1-methyl-imidazol-2-yl)methanol (28.6 mg, 150 µmol) and XPhos Pd Gs (9.07 mg, 10.71 µmol) in dioxane (1 mL) and H₂O (0.1 mL) was added Na₂CO₃ (22.70 mg, 214.20 µmol) at 15 °C under N₂. The mixture was stirred at 70 °C for 12 h. The reaction mixture another batch (50 mg, scale) was diluted with ethyl acetate (20 mL) and dried over Na₂SO₄, filtered and concentrated under reduced pressure. The residue was purified by column chromatography (SiO₂, petroleum ether/ethyl acetate=1/1) to give the title compound as yellow solid. MS (ESI): mass calcd. For C₁₆H₁₆F₄N₂O₄, 376.1; m/z found, 377.1 [M+H]⁺.

Step C. (S)-N-(2-chloro-4-methylpyridin-3-yl)-5-fluoro-4-(2-(hydroxymethyl)-1-methyl-1H-imidazol-4-yl)-2-((1,1.1-trifluoropropan-2-yl)oxy)benzamide. To a solution of 2-chloro-4-methylpyridin-3-amine (75.8 mg, 531 µmol) in DCM (1.5 mL) was added AlMe₃ (2 M in toluene, 199 µL) at 15 °C under N₂. The mixture was stirred at 15 °C for 0.5 h. (S)-methyl 5-fluoro-4-(2-(hydroxymethyl)-1-methyl-1H-imidazol-4-yl)-2-((1,1,1-trifluoropropan-2-yl)oxy)benzoate (50 mg, 133 µmol) was added. The mixture was stirred at 60 °C for 12 h. The reaction mixture was quenched by addition 1 N HCl (20 mL), and then extracted with DCM (15 mL × 2). The combined organic layers were dried over Na₂SO₄, filtered and concentrated in vacuo. The residue was purified by prep-HPLC (method A) to give the title compound as white solid. MS (ESI): mass calcd. For C₂₁H₁₉ClF₄N₄O₃, 486.1; m/z found, 487.1 [M+H]⁺. ¹H NMR (400MHz, CDCl₃) δ = 9.26 (s, 1H), 8.21 (d, *J*=4.9 Hz, 1H), 8.03 (d, *J*=11.9 Hz, 1H), 7.85 (d, *J*=5.6 Hz, 1H), 7.51 (d, *J*=3.9 Hz, 1H), 7.19 (d, *J*=5.0 Hz, 1H), 5.25 (td, *J*=6.3, 12.5 Hz, 1H), 4.79 (d, *J*=1.1 Hz, 2H), 3.78 (s, 3H), 2.36 (s, 3H), 1.71 (d, *J*=6.5 Hz, 3H).

### Example 37: (S)-N-(3-chloro-5-methyl-1H-pyrazol-4-yl)-4-(5-cyclopropylpyrazin-2-yl)-5-fluoro-2-(1,1,1-trifluoropropan-2-yl)oxy)benzamide.

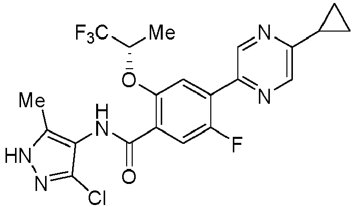

The title compound was prepared according to the representative procedures of Example 31, Step A-B, except substituting 2-bromo-5-cyclopropylpyrazine for 2-bromo-5-methylpyridine and 3-chloro-5-methyl-1H-pyrazol-4-amine for 2-chloro-4-methylpyridin-3-amine . MS (ESI): mass calcd. For C₂₁H₁₈ClF₄N₅O₂, 483.1; m/z found, 484.1 [M+H]⁺. ¹H NMR (400MHz, DMSO-*d₆*) δ = 12.85 (br s, 1H), 9.49 (s, 1H), 8.89 (d, *J*=1.6 Hz, 1H), 8.82 (d, *J*=1.5 Hz, 1H), 7.80 (d, *J*=6.0 Hz, 1H), 7.54 (d, *J*=10.5 Hz, 1H), 5.45-5.39 (m, 1H), 2.36 - 2.25 (m, 1H), 2.16 (s, 3H), 1.47 (d, *J*=6.4 Hz, 3H), 1.16 - 1.09 (m, 2H), 1.06 - 1.01 (m, 2H).

### Example 38: (S)-2-(4-((2-chloro-6-fluorophenyl)carbamoyl)-2-fluoro-5-((1,1,1-trifluoropropan-2-yl)oxy)phenyl)-5-ethylthiazole-4-carboxamide.

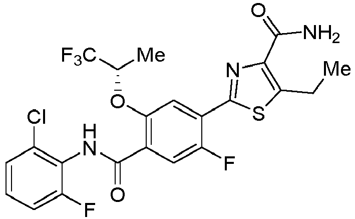

Step A. (S)-methyl 2-(4-((2-chloro-6-fluorophenyl)carbamoyl)-2-fluoro-5-((1,1,1-trifluoropropan-2-yl)oxy)phenyl)-5-ethylthiazole-4-carboxylate. The title compound was prepared in a manner analogous to Example 1, Step E however using methyl 2-bromo-5-ethylthiazole-4-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd For C₂₃H₁₈ClF₅N₂O₄S, 548.1; m/z found, 549.2 [M+H]⁺.

Step B. (S)-2-(4-((2-chloro-6-fluorophenyl)carbamoyl)-2-fluoro-5-((1,1,1 -trifluoropropan-2-yl)oxy)phepyl)-5-ethylthiazole-4-carboxylic acid. To a solution of (S)-methyl 2-(4-((2-chloro-6-fluorophenyl)carbamoyl)-2-fluoro-5-((1,1,1-trifluoropropan-2-yl)oxy)phenyl)-5-ethylthiazole-4-carboxylate (0.1 g, 160 µmol) in THF (1 mL) and H₂O (1 mL) was added NaOH (19.2 mg, 481 µmol) at 20 °C, and the mixture was stirred at 20 °C for 16 hrs. The mixture was poured into water (5 mL) and extracted with ethyl acetate (5 mL×2). The combined organic phase was washed with brine (10 mL×2), dried with anhydrous Na₂SO₄, filtered and concentrated in vacuo to give the title compound as a white solid. MS (ESI): mass calcd. for C₂₂H₁₆ClF₅N₂O₄S, 534.0; m/z found, 535.1 [M+H]⁺.

Step C. (S)-2-(4-((2-chloro-6-fluorophenyl)carbamoyl)-2-fluoro-5-((1,1,1-trifluoropropan-2-yl)oxy)phepyl)-5-ethylthiazole-4-carboxamide. To a mixture of (S)-2-(4-((2-chloro-6-fluorophenyl)carbamoyl)-2-fluoro-5-((1,1,1-trifluoropropan-2-yl)oxy)phenyl)-5-ethylthiazole-4-carboxylic acid (65 mg, 100 µmol) and NH₄Cl (53.8 mg, 1.00 mmol) in DMF (2 mL) was added HOBt (20.4 mg, 15' µmol), PYBOP (78.4 mg, 150.8 µmol) and DIPEA (195 mg, 1.51 mmol, 263 µL) in one portion under N₂. The mixture was stirred at 20 °C for 12 hours. The mixture was purified by prep-HPLC (METHOD A) to give the title compound as a white solid. MS (ESI): mass calcd for C₂₂H₁₇ClF₅N₃O₃S, 533.1; m/z found, 534.0 [M+H]⁺. ¹H NMR (400 MHz, DMSO-*d₆)* δ = 10.05 (s, 1H), 8.30 (d, *J* = 5.8 Hz, 1H), 8.17 (s, 1H), 7.74 (s, 1H), 7.62 (d, *J* = 10.5 Hz, 1H), 7.48 - 7.33 (m, 3H), 5.58 - 5.52 (M, 1H), 3.38 - 3.34 (m, 2H), 1.51 (d, *J* = 6.4 Hz, 3H), 1.30 (t, *J* = 7.4 Hz, 3H).

### Example 39: (S)-N-(3-chloro-5-methyl-1H-pyrazol-4-yl)-4-(5-ethyl-4-(hydroxymethyl)thiazol-2-yl)-5-fluoro-2-((1,1,1-trifluoropropan-2-yl)oxy)benzamide.

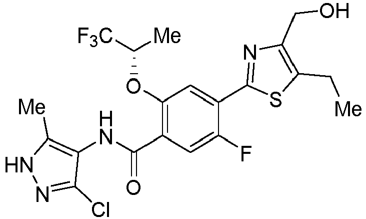

The title compound was prepared according to the representative procedures of Example 31, Step A-B, except substituting (2-bromo-5-ethylthiazol-4-yl)methanol (Example 35, Step A) for 2-bromo-5-methylpyridine and 3-chloro-5-methyl-1H-pyrazol-4-amine for 2-chloro-4-methylpyridin-3-amine . MS (ESI): mass calcd. For C₂₀H₁₉ClF₄N₄O₃S, 506.1; m/z found, 507.1 [M+H]⁺. ¹H NMR (400MHz, DMSO-*d₆*) δ = 12.85 (br s, 1H), 9.51 (s, 1H), 7.97 (d, *J*=5.9 Hz, 1H), 7.59 (d, *J*=10.8 Hz, 1H), 5.48 - 5.35 (m, 1H), 5.17 (t, *J*=*5.5* Hz, 1H), 4.62 (d, *J*=5.4 Hz, 2H), 2.97 (q, *J*=7.5 Hz, 2H), 2.15 (s, 3H), 1.48 (d, *J*=6.4 Hz, 3H), 1.28 (t, *J*=7.5 Hz, 3H).

### Example 40: (S)-N-(2-chloro-6-fluorophenyl)-4-(3-cyclopropyl-1.2,4-thiadiazol-5-yl)-5-fluoro-2-((1,1,1-trifluoropropan-2-yl)oxy)benzamide.

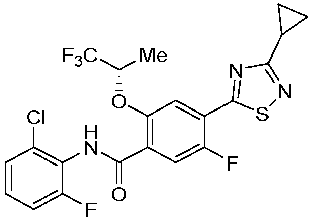

The title compound was prepared in a manner analogous to Example 1, Step E however using 5-bromo-3-cyclopropyl-1,2,4-thiadiazole as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₁H₁₅ClF₅N₃O₂S, 503.0; m/z found, 504.0 [M+H]⁺. ¹H NMR (CDCl₃) δ = 9.09 (s, 1H), 8.22 (d, J=11.2 Hz, 1H), 7.99 (d, J=4.9 Hz, 1H), 7.22-7.34 (m, 1H), 7.10-7.18 (m, 1H), 5.12 (dt, J=12.2, 6.1 Hz, 1H), 2.40-2.54 (m, 1H), 1.71 (d, J=6.4 Hz, 3H), 1.12-1.30 (m, 4H).

### Example 41: (S)-N-(3-chloro-5-methyl-1H-pyrazol-4-yl)-5-fluoro-4-(2-(hydroxymethyl)-1-methyl-1H-imidazol-4-yl)-2-((1,1,1-trifluoropropan-2-yl)oxy)benzamide.

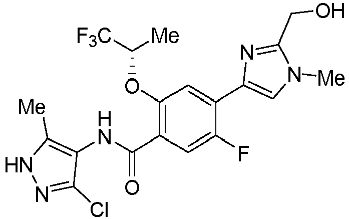

### Step A. (S)-5-fluoro-4-(2-(hydroxymethyl)-1-methyl-1H-imidazol-4-yl)-2-((1,1,1-trifluoropropan-2-yl)oxy)benzoate.

The title compound was prepared in a manner analogous to Example 14, Step D however using (4-bromo-1-methyl-1H-imidazol-2-yl)methanol (Example 36, Step A) as the aryl halide and (S)-methyl 5-fluoro-4-(4,4,5,5-tetramethyl-1,3,2-dioxaborolan-2-yl)-2-((1,1,1-trifluoropropan-2-yl)oxy)benzoate (Intermediate 2) as the boronate. MS (ESI): mass calcd. For C₁₆H₁₆ClF₄N₂O₄, 376.1; m/z found, 377[M+H]⁺.

Step B. (S)-5-fluoro-4-(2-(hydroxymethyl)-1-methyl-1H-imidazol-4-yl)-2-((1,1,1-trifluoropropan-2-yl)oxy)benzoic acid. To a mixture of (S)-5-fluoro-4-(2-(hydroxymethyl)-1-methyl-1H-imidazol-4-yl)-2-((1,1,1-trifluoropropan-2-yl)oxy)benzoate (300 mg, 0.797 mmol) was added THF (2 mL), MeOH (2 mL), water (2 mL), and NaOH (64 mg, 1.6 mmol). The reaction was stirred at rt for 16 hrs. The mixture was concentrated under vacuum to give the residue. The residue was diluted with water (10 mL), adjusted pH 5~6 with 2M HCl solution (0.5 mL). The precipitate was filtered off and dried. The filtrate was extracted with EtOAc (30 mL*3). The combined organic layers were dried over Na2SO4, filtered, concentrated to dryness to give the title compound as a white solid. MS (ESI): mass calcd. For C₁₅H₁₄ClF₄N₂O₄, 362.1; m/z found, 363 [M+H]⁺.

Step C. (S)-N-(3-chloro-5-methyl-1H-pyrazol-4-yl)-5-fluoro-4-(2-(hydroxymethyl)-1-methyl-1H-imidazol-4-yl)-2-((1,1,1-trifluoropropan-2-yl)oxy)benzamide. To a mixture of (S)-5-fluoro-4-(2-(hydroxymethyl)-1-methyl-1H-imidazol-4-yl)-2-((1,1,1-trifluoropropan-2-yl)oxy)benzoic acid (75 mg, 0.207 mmol) and DCM (1.5 mL), was added 3-chloro-5-methyl-1H-pyrazol-4-amine (95 mg, 0.73 mmol) and TEA (395 mg, 0.62 mmol). After stirring for 10 min, T3P (395 mg, 0.62 mmol) was added and the reaction was heated to 50 C for 16 hr. The mixutre was diluted with DCM (3 mL), washed with sat. aq. NaHCO3 (5 mL), and extracted with DCM (10 mL*3), dried over Na2SO4, filtered, concentrated under vacuum to give the crude product. The crude product was purified by preparative reversed phase HPLC (Stationary phase: Boston Prime C18, 5 µm, 150 × 30 mm; Mobile phase: water (0.04% NH₃H₂O + 10 mM NH₄HCO₃) (A) - MeCN (B), gradient elution: 50 - 80% B in A over 7 min, flow rate: 25 mL/min) to give the title compound as a-white powder. MS (ESI): mass calcd. For C₁₉H₁₃ClF₄N₅O₃, 475.1; m/z found, 476.1[M+H]⁺. ¹H NMR (400MHz, DMSO-*d₆*) δ = 12.94 - 12.72 (m, 1H), 9.30 (s, 1H), 8.41 (s, 1H), 7.82 (d, *J*=6.1 Hz, 1H), 7.64 (d, *J*=4.3 Hz, 1H), 7.46 (d, *J*=11.1 Hz, 1H), 5.36-5.30 (m, 2H), 4.57 (s, 2H), 3.75 (s, 4H), 2.15 (s, 3H), 1.49 (d, *J*=6.4 Hz, 3H).

### Example 42: (S)-3-(4-((2-chloro-6-fluorophenyl)carbamoyl)-2-fluoro-5-((1.1.1-trifluoropropan-2-yl)oxy)phenyl)-1-methyl-1H-1,2,4-triazole-5-carboxamide.

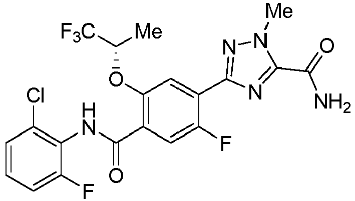

Intermediate 1 (82 mg, 0.16 mmol), 3-bromo-1-methyl-1H-1,2,4-triazole-5-carbonitrile (45.5 mg, 0.24 mmol), Pd(dppf)Cl₂·CH₂Cl₂ (26.5 mg, 0.032 mmol), Na₂CO₃ (52.0 mg, 0.49 mmol) in 1,4-dioxane (2.0 mL) and water (1.0 mL) were added to a microwave vial. The mixture was sparged with Argon then heated to 80 °C for 16 h. The reaction mixture was cooled to room temperature then diluted with EtOAc and water. The organics were extracted with EtOAc (3x). The combined organic layers were washed with brine, dried over sodium sulfate, filtered and concentrated. Purification by silica gel column chromatography using a 10 to 50% EtOAc/heptane gradient afforded the title compound as a white solid. MS (ESI): mass calcd. for C₂₀H₁₅ClF₅N₅O₃, 503.1; m/z found, 504.0 [M+H]⁺. ¹H NMR (400 MHz, CDCl₃) δ = 9.09 (s, 1H), 8.16 (d, J=11.2 Hz, 1H), 7.75 (d, J=5.4 Hz, 1H), 7.22-7.35 (m, 2H), 7.14 (t, J=8.6 Hz, 1H), 5.71 (br s, 1H), 4.98-5.14 (m, 1H), 4.38 (s, 3H), 1.69 (d, J=6.4 Hz, 3H).

### Example 43: (S)-N-(2-chloro-6-fluoropheyl)-4-(5-cyclopropyl-1,3,4-oxadiazol-2-yl)-5-fluoro-2-((1,1,1-trifluoropropan-2-yl)oxy)benzamide.

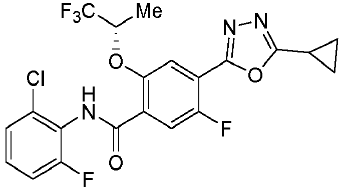

Intermediate 1 (95 mg, 0.188 mmol), 2-bromo-5-cyclopropyl-1,3,4-oxadiazole (53.3 mg, 0.28 mmol), Pd(dppf)Cl₂·CH₂Cl₂ (18.6 mg, 0.023 mmol), Na₂CO₃ (60.2 mg, 0.57 mmol) in 1,4-dioxane (2.3 mL) and water (1.1 mL) were added to a microwave vial. The mixture was sparged with Argon then heated to 80 °C for 16 h. The reaction mixture was cooled to room temperature then diluted with EtOAc and water. The organics were extracted with EtOAc (3x). The combined organic layers were washed with brine, dried over sodium sulfate, filtered and concentrated. Purification by silica gel column chromatography using a 10 to 50% EtOAc/heptane gradient afforded the title compound. The compound was repurified by reverse phase HPLC (30 × 100 mm Gemini C18 column, 10-100% MeCN/10 mM NH₄OH in water) to provide the title compound as a white solid. MS (ESI): mass calcd. for C₂₁H₁₅ClF₅N₃O₃, 487.1; m/z found, 488.0 [M+H]⁺. ¹H NMR (400 MHz, CDCl₃) δ ppm 9.06 (s, 1H), 8.19 (d, J=10.8 Hz, 1H), 7.79 (d, J=4.9 Hz, 1H), 7.21-7.33 (m, 1H), 7.14 (t, J=8.6 Hz, 1H), 4.99-5.14 (m, 1H), 2.24-2.34 (m, 1H), 1.69 (d, J=6.8 Hz, 3H), 1.20-1.34 (m, 4H).

### Example 44. (S)-5-(4-((2-chloro-6-fluorophenyl)carbamoyl)-2-fluoro-5-((1,1,1-trifluoropropan-2-yl)oxy)phenyl)pyrazine-2-carboxamide

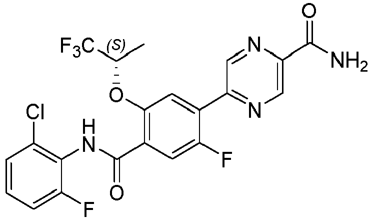

The title compound was prepared in a manner analogous to Example 1, Step E however using 5-bromopyrazine-2-carboxamide as the aryl halide and (S)-N-(2-chloro-6-fluorophenyl)-5-fluoro-4-(4,4,5,5-tetramethyl-1 ,3,2-dioxaborolan-2-yl)-2-((1,1,1-trifluoropropan-2-yl)oxy)benzamide (Intermediate 1) as the boronate. ¹H NMR (400 MHz, DMSO-*d₆*) δ ppm 10.11 (s, 1 H), 9.35 (d, *J*=1.3 Hz, 1H), 9.15 (s, 1H), 8.37 (s, 1 H), 7.87 - 8.02 (m, 2 H), 7.60 (d, *J*=10.0 Hz, 1 H), 7.34 - 7.49 (m, 3 H), 5.47 (dt, *J*=12.7, 6.3 Hz, 1 H), 1.48 (d, *J*=6.3 Hz, 3 H); ¹⁹F NMR (376 MHz, DMSO-*d₆)* δ ppm -77.13 (br s, 3 F), -115.33 (br s, 1 F), -123.04 - -122.98 (m, 1 F); ESI-MS: *m*/*z* 501.0 [M+H]⁺;

### Example 45: (S)-3-chloro-6-(4-((2-chloro-6-fluorophenyl)carbamoyl)-2-fluoro-5-((1,1,1-trifluoropropan-2-yl)oxy)phenyl)pyrazine-2-carboxylic acid.

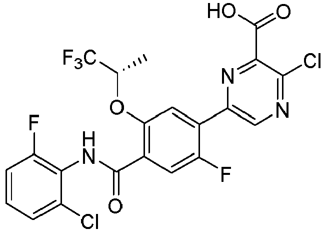

The title compound was prepared in a manner analogous to Example 1, Step E however using 6-bromo-3-chloro-pyrazine-2-carboxylic acid as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate MS (ESI): mass calcd. for C₂₁H₁₂C₁₂F₅N₃O₄ , 535.0; m/z found, 536.1 [M+H]⁺. ¹H NMR (400 MHz, DMSO-d6) δ ppm 10.13 (s, 1 H) 8.66 (s, 1 H) 7.76 (d, J=5.87 Hz, 1 H) 7.52 - 7.58 (m, 1 H) 7.45 (s, 2 H) 7.34 - 7.40 (m, 1 H) 6.62 (s, 1 H) 5.33 - 5.46 (m, 1 H) 1.47 (d, J=6.36 Hz, 3 H).

### Example 46: (S)-4-(5-chloro-4-(hydroxymethyl)pyridin-2-yl)-N-(2-chloro-6-fluorophenyl)-5-fluoro-2-(1,1,1-trifluoropropan-2-yl)oxy)benzamide.

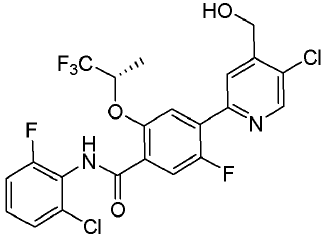

Step A methyl (S)-5-chloro-2-(4-((2-chloro-6-fluorophenyl)carbamoyl)-2-fluoro-5-((1,1,1-trifluoropropan-2-yl)oxy)phenyl)isonicotinate. The title compound was prepared in a manner analogous to Example 1, Step E however using methyl 2-bromo-5-chloroisonicotinate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₃H₁₅Cl₂F₅N₂O₄, 548.0; m/z found, 549.0 [M+H]⁺.

Step B. (S)-4-(5-chloro-4-(hydroxymethyl)pyridin-2-yl)-N-(2-chloro-6-fluorophenyl)-5-fluoro-2-((1,1,1-trifluoropropan-2-yl)oxy)benzamide. Lithium borohydride (21 mg, 0.95 mmol) was added to a solution of methyl (S)-5-chloro-2-(4-((2-chloro-6-fluorophenyl)carbamoyl)-2-fluoro-5-((1,1,1-trifluoropropan-2-yl)oxy)phenyl)isonicotinate (130 mg, 0.237 mmol) in THF (1.5 mL) at 0 °C. The mixture was stirred at 0 °C for 45 min, was quenched by addition of water, then DCM was added. The combined organic phase was dried over MgSCO₄, filtered and evaporated. The crude product was purified by reverse phase chromatography (Phenomenex Gemini-NX, C18, 150x30mm, 5 µm; 30 mL/min; Buffer A: 20 mM NH4OH/water Buffer B: MeCN; gradient: 10% B for 2 min then linear gradient to 100 % B over 11 min and hold at 100% B for 2 min). and afforded the title compound. MS (ESI): mass calcd. for C₂₂H₁₅Cl₂F₅N₂O₃, 520.0; m/z found, 521.1 [M+H]⁺. ¹H NMR (400 MHz, CHLOROFORM-d) δ ppm 9.15 (s, 1 H) 8.62 (s, 1 H) 8.18 (s, 1 H) 8.13 (d, J=11.74 Hz, 1 H) 7.85 (d, J=5.87 Hz, 1 H) 7.28 - 7.33 (m, 1 H) 7.20 - 7.26 (m, 1 H) 7.04 - 7.18 (m, 1 H) 5.11 (dt, J=12.23, 6.11 Hz, 1 H) 4.87 (d, J=5.38 Hz, 2 H) 1.68 (d, J=6.36 Hz, 3 H).

### Example 47: (S)-5-chloro-2-(4-((2-chloro-6-fluorophenyl)carbamoyl)-2-fluoro-5-((1,1,1-trifluoropropan-2-yl)oxy)phenyl)isonicotinic acid.

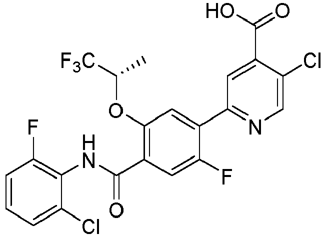

The title compound was prepared in a manner analogous to Example 1, Step E however using 2-bromo-5-chloroisonicotinic acid as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₂H₁₃Cl₂F₅N₂O₄, 534.0; m/z found, 535.0 [M+H]⁺. ¹H NMR (400 MHz, DMSO-d6) δ ppm 10.08 (s, 1 H) 8.72 (br s, 1 H) 7.82 (br d, J=5.38 Hz, 2 H) 7.52 (br d, J=10.76 Hz, 1 H) 7.41 - 7.47 (m, 2 H) 7.38 (br d, J=9.78 Hz, 1 H) 5.37 - 5.52 (m, 1 H) 1.46 (s, 2 H).

### Example 48, (S)-N-(2-chloro-6-fluorophenyl)-4-(5-cyclopropyl-6-(hydroxymethyl)pyridin-2-yl)-5-fluoro-2-((1,1,1-trifluoropropan-2-yl)oxy)benzamide

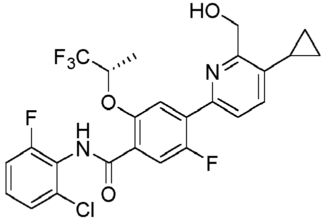

Step A. Methyl 6-chloro-3-cyclopropylpicolinate. A stirred solution of cyclopropylboronic acid (171 mg, 1.20 mmol), methyl 3-bromo-6-chloropicolinate (250 mg, 0.998 mmol), tripotassium phosphate, anhydrous (423 mg, 1.20 mmol) in toluene (4 mL) was purged with N₂, then 1,1'-bis(diphenylphosphino)ferrocene-palladium(II)dichloride dichloromethane complex (82 mg, 0.10 mmol) was added at rt. The reaction mixture was purged again with N₂, and stirred at 90 °C for 4h, then was diluted with ethyl acetate and water. The combined organic phase was washed with brine, dried over MgSO₄, filtered and evaporated in vacuo to give the crude product as a brown oil. DCM was added to this crude product, an insoluble was filtered and the filtrate was evaporated.

The filtrate was purified by chromatography (SiO₂, Heptane/Ethyl acetate=100/0 to Heptane/Ethyl acetate= 40/60) and yielded the title compound as colorless oil. MS (ESI): mass calcd. for C₁₀H₁₀ClNO₂, 211.0; m/z found, 212.0 [M+H]⁺.

Step B. methyl (S)-6-(4-((2-chloro-6-fluorophenyl)carbamoyl)-2-fluoro-5-((1,1,1-trifluoropropan-2-yl)oxy)phenyl)-3-cyclopropylpicolinate. To a mixture of Methyl 6-chloro-3-cyclopropylpicolinate (135 mg, 0.344 mmol) and Intermediate 1 (116 mg, 0.23 mmol) was added K₂CO₃ (79 mg, 0.574 mmol), 1,4-dioxane (2.9 ml) and water (1.4 mL). Pd(dppf)Cl₂ (9 mg, 0.012 mmol) was added and the reaction was heated to 80 C for 30 min. The reaction was diluted with EtOAc and washed with water. Organics were dried filtered and concentrated. Purification by SiO₂ chromatography (heptane/EtOAc) yielded the title compound as a brown oil. MS (ESI): mass calcd. for C₂₆H₂₀ClF₅N₂O₄, 554.1; m/z found, 555.2 [M+H]⁺.

Step C. (S)-N-(2-chloro-6-fluorophenyl)-4-(5-cyclopropyl-6-(hydroxymethyl)pyridin-2-yl)-5-fluoro-2-((1,1,1-trifluoropropan-2-yl)oxy)benzamide. The title compound was prepared according to the representative procedures of Example 46, Step B, except substituting methyl (S)-6-(4-((2-chloro-6-fluorophenyl)carbamoyl)-2-fluoro-5-((1,1,1-trifluoropropan-2-yl)oxy)phenyl)-3-cyclopropylpicolinate for (S)-4-(5-chloro-4-(hydroxymethyl)pyridin-2-yl)-N-(2-chloro-6-fluorophenyl)-5-fluoro-2-((1,1,1-trifluoropropan-2-yl)oxy)benzamide. MS (ESI): mass calcd. for C₂₅H₂₀ClF₅N₂O₃, 526.1; m/z found, 527.1 [M+H]⁺. ¹H NMR (400 MHz, CHLOROFORM-d) δ ppm 9.13 (s, 1 H) 8.14 (d, J=11.74 Hz, 1 H) 7.60 - 7.91 (m, 2 H) 7.43 (d, J=8.31 Hz, 1 H) 7.29 (br d, J=0.98 Hz, 1 H) 7.24 (s, 1 H) 7.14 (t, J=8.80 Hz, 1 H) 5.03 (br d, J=6.36 Hz, 1 H) 4.99 (d, J=3.91 Hz, 2 H) 4.73 - 4.77 (m, 1 H) 1.75 - 1.86 (m, 1 H) 1.68 (d, J=6.85 Hz, 3 H).

### Example 49: (S)-3-chloro-6-(4-((2-chloro-6-fluorophenyl)carbamoyl)-2-fluoro-5-((1,1,1-trifluoropropan-2-yl)oxy)phenyl)picolinic acid

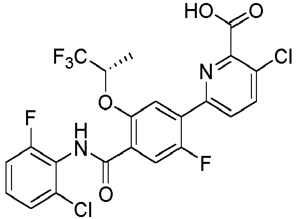

Step A. 6-bromo-3-chloropicolinic acid. To a solution of methyl 6-bromo-3-chloropicolinate (300 mg, 1.20 mmol) in MeOH (3.5 mL) was added a solution of NaOH (3M in H₂O) (1.30 mL, 3 M, 3.83 mmol) at rt. The reaction mixture was heated at 70 °C for 3 hours, cooled down at rt and stirred at rt for 12 h. A solution of HCl 1N was added until pH= 2. The aqueous phase was extracted with EtOAc. The combined organic phase was washed with brine, dried with anhydrous MgSO₄, filtered and concentrated in vacuum to afford the desired product. MS (ESI): mass calcd. for C₆H₃BrClNO₂, 234.9; m/z found, 235.9 [M+H]⁺.

Step B. (S)-3-chloro-6-(4-((2-chloro-6-fluorophenyl)carbamoyl)-2-fluoro-5-((1,1,1-trifluoropropan-2-yl)oxy)phenyl)picolinic acid. The title compound was prepared in a manner analogous to Example 48, Step B however using 6-bromo-3-chloropicolinic acid as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₂H₁₃C₁₂F₅N₂O₄, 534.0; m/z found, 535.0 [M+H]⁺. ¹H NMR (400 MHz, DMSO-d6) δ ppm 10.09 (s, 1 H) 8.06 - 8.26 (m, 1 H) 7.81 - 7.95 (m, 1 H) 7.77 (br d, J=4.89 Hz, 1 H) 7.33 - 7.59 (m, 6 H) 5.22 - 5.57 (m, 1 H) 1.35 - 1.59 (m, 3 H).

### Example 50: (S)-3-Chloro-6-(4-((2-chloro-6-fluorophenyl)carbamoyl)-2-fluoro-5-((1,1,1-trifluoropropan-2-yl)oxy)phenyl)picolinamide.

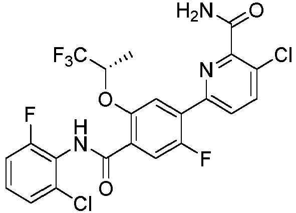

The title compound was prepared in a manner analogous to Example 48, Step B however using 6-bromo-3-chloropicolinamide as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₂H₁₄Cl₂F₅N₃O₃, 533.0; m/z found, 534.2 [M+H]⁺. ¹H NMR (400 MHz, CHLOROFORM-d) δ ppm 9.09 (s, 1 H) 8.16 (d, J=11.25 Hz, 1 H) 7.98 (s, 2 H) 7.69 (d, J=5.87 Hz, 1 H) 7.40 (br s, 1 H) 7.30 (s, 2 H) 7.15 (s, 1 H) 5.65 - 5.76 (m, 1 H) 5.02 (s, 1 H) 1.69 (d, J=6.85 Hz, 3 H).

### Example 51: (S)-4-(5-Chloro-6-(hydroxymethyl)pyridin-2-yl)-N-(2-chloro-6-fluorophenyl)-5-fluoro-2-((1,1,1-trifluoropropan-2-yl)oxy)benzamide.

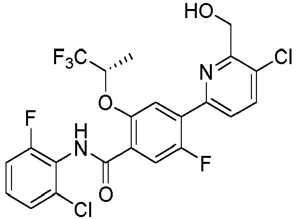

Step A. Methyl (S)-3-chloro-6-(4-((2-chloro-6-fluorophenyl)carbamoyl)-2-fluoro-5-((1,1,1-trifluoropropan-2-yl)oxy)phenyl)picolinate. The title compound was prepared in a manner analogous to Example 48, Step B however using methyl 6-bromo-3-chloropicolinate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₃H₁₅Cl₂F₅N₂O₄, 548.0; m/z found, 549.0 [M+H]⁺. ¹H NMR (400 MHz, CHLOROFORM-d) δ ppm 9.12 (s, 1 H) 8.14 (d, J=11.74 Hz, 1 H) 8.02 (dd, J=8.56, 1.22 Hz, 1 H) 7.93 (d, J=8.80 Hz, 1 H) 7.85 (d, J=5.87 Hz, 1 H) 7.29 (s, 2 H) 7.14 (s, 1 H) 5.09 (dt, J=12.35, 6.30 Hz, 1 H) 4.07 (s, 3 H) 1.68 (d, J=6.36 Hz, 3 H).

Step B. (S)-4-(5-Chloro-6-(hvdroxymethyl)pyridin-2-yl)-N-(2-chloro-6-fluorophenvl)-5-fluoro-2-((1,1,1-trifluoropropan-2-yl)oxy)benzamide. The title compound was prepared according to the representative procedures of Example 46, Step B, except substituting methyl (S)-3-chloro-6-(4-((2-chloro-6-fluorophenyl)carbamoyl)-2-fluoro-5-((1,1,1-trifluoropropan-2-yl)oxy)phenyl)picolinate. MS (ESI): mass calcd. for C₂₂H₁₅Cl₂F₅N₂O₃, 520.0; m/z found, 521.1 [M+H]⁺. ¹H NMR (400 MHz, CHLOROFORM-d) δ ppm 9.11 (s, 1 H) 8.16 (d, J=11.74 Hz, 1 H) 7.81 - 7.91 (m, 2 H) 7.76 (d, J=5.87 Hz, 1 H) 7.30 (br t, J=6.60 Hz, 2 H) 7.22 - 7.25 (m, 1 H) 7.11 - 7.19 (m, 1 H) 5.00 - 5.11 (m, 1 H) 4.91 (d, J=4.89 Hz, 2 H) 4.18 (t, J=4.65 Hz, 1 H) 1.69 (d, J=6.36 Hz, 3 H).

### Example 52: (S)-N-(2-Chloro-6-fluorophenyl)-4-(5-cyclopropyl-4-(hydroxymethyl)thiazol-2-yl)-5-fluoro-2-(1,1,1-trifluoropropan-2-yl)oxy)benzamide.

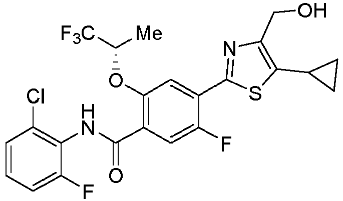

Step A. Ethyl 5-cyclopropylthiazole-4-carboxylate. To a mixture of ethyl 5-bromothiazole-4-carboxylate (2 g, 8.47 mmol) and cyclopropylboronic acid (1.46 g, 16.9 mmol) in toluene (20 mL) was added K₃PO₄ (3.60 g, 16.9 mmol) followed by Pd(dppf)Cl₂ (620 mg, 847 µmol) under N₂. The mixture was stirred at 90 °C for 12 hours. The mixture was concentrated in vacuo. The residue was purified by silica gel chromatography (1000 mesh silica gel, petroleum ether/ethyl acetate=15/1, 5/1) to give the title compound as a yellow oil. MS (ESI): mass calcd. for C₉H₁₁NO₂S, 197.0; m/z found, 198.2 [M+H]⁺.

Step B, Ethyl 2-bromo-5-cyclopropylthiazole-4-carboxylate. To a mixture of ethyl 5-cyclopropylthiazole-4-carboxylate (50 mg, 253 µmol) in DMF (2 mL) was added CBr₄ (92.5 mg, 279 µmol) followed by t-BuONa (97.4 mg, 1.0 mmol) under N₂. The mixture was stirred at 20 °C for 1 hours. The mixture was poured into 1N HCl (10 mL). The aqueous phase was extracted with ethyl acetate (15 mL × 2). The combined organic phase was washed with brine (10 mL × 2), dried with anhydrous Na₂SO₄, filtered and concentrated in vacuum. The residue was purified by prep-TLC (petroleum ether/ethyl acetate=10/1) to give the title compound as a yellow oil. MS (ESI): mass calcd. for C₉H₁₀BrNO₂S, 274.9; m/z found, 275.9 [M+H]⁺.

Step C. (S)-Ethyl 2-(4-((2-chloro-6-fluorophenyl)carbamoyl)-2-fluoro-5-((1,1,1-trifluoropropan-2-yl)oxy)phenyl)-5-cyclopropylthiazole-4-carboxylate. The title compound was prepared in a manner analogous to Example 1, Step E however using ethyl 2-bromo-5-cyclopropylthiazole-4-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₅H₂₀ClF₅N₂O₄S, 574.0; m/z found, 575.2 [M+H]⁺.

Step D. (S)-N-(2-Chloro-6-fluorophenyl)-4-(5-cyclopropyl-4-(hydroxymethyl)thiazol-2-yl)-5-fluoro-2-((1,1,1-trifluoropropan-2-yl)oxy)benzamide. To a mixture of (S)-ethyl 2-(4-((2-chloro-6-fluorophenyl)carbamoyl)-2-fluoro-5-((1,1,1-trifluoropropan-2-yl)oxy)phenyl)-5-cyclopropylthiazole-4-carboxylate (80 mg, 139 µmol) in THF (4 mL) was added LiBH₄ (2 M in THF, 417 µL) at 0 °C under N₂. The mixture was stirred at 20 °C for 2 hours. The mixture was poured into water (5 mL). The aqueous phase was extracted with ethyl acetate (15 mL × 2). The combined organic phase was washed with brine (10 mL × 2), dried with anhydrous Na₂SO₄, filtered and concentrated in vacuo. The residue was purified by prep-HPLC (METHOD A) to give the title compound as a white solid. MS (ESI): mass calcd. for C₂₃H₁₈ClF₅N₂O₃S, 532.0; m/z found, 533.0 [M+H]⁺. ¹H NMR (400 MHz, CDCl₃) δ = 9.11 (s, 1H), 8.15 (d, *J* = 11.6 Hz, 1H), 7.98 (d, *J* = 5.2 Hz, 1H), 7.33 - 7.28 (m, 1H), 7.26 - 7.22 (m, 1H), 7.17 - 7.11 (m, 1H), 5.18 - 5.11 (m, 1H), 4.90 (d, *J* = 6 Hz, 2H), 2.44 - 2.39 (m, 1H), 2.16 - 2.08 (m, 1H), 1.69 (d, *J* = 6.8 Hz, 3H), 1.22 - 1.16 (m, 2H), 0.86 - 0.80 (m, 2H).

### Example 53: (S)-N-(2-Chloro-6-fluorophenyl)-5-fluoro-4-(6-(hydroxymethyl)-5-methylpyrazin-2-yl)-2-((1,1,1-trifluoropropan-2-yl)oxy)benzamide.

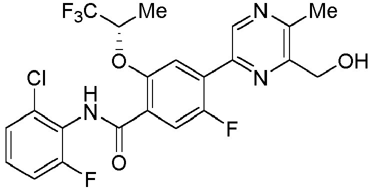

Step A, Methyl 6-chloro-3-methylpyrazine-2-carboxylate. To a solution of (0.50 g, 2.0 mmol) and 2,4,6-trimethyl-1,3,5,2,4,6-trioxatriborinane (599 mg, 2.39 mmol, 667 µL) in DME (10 mL) was added Pd(dppf)Cl₂ (73 mg, 99 µmol) and K₂CO₃ (550 mg, 3.98 mmol). The mixture was stirred at 100 °C for 16 hr. The residue was purified by column chromatography (SiO₂, petroleum ether/ethyl acetate=50/1 to 5/1) and then prep-TLC (SiO₂, petroleum ether/ethyl acetate=5/1, Rf=0.6) to give the title compound as a colorless oil. MS (ESI): mass calcd. for C₇H₇ClN₂O₂, 186.0; m/z found, 187.1 [M+H]⁺.

Step B. (S)-Methyl 6-(4-((2-chloro-6-fluorophenyl)carbamoyl)-2-fluoro-5-((1,1,1-trifluoropropan-2-yl)oxy)phenyl)-3-methylpyrazine-2-carboxylate. The title compound was prepared in a manner analogous to Example 1, Step E however using methyl 6-chloro-3-methylpyrazine-2-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1 -trifluoropropan-2-yl)oxy)benzamide (Intermediate 1) as the boronate. MS (ESI): mass calcd. for C₂₃H₁₇ClF₅N₃O₄, 529.0; m/z found, 530.1 [M+H]⁺.

Step C. (S)-N-(2-Chloro-6-fluorophenyl)-5-fluoro-4-(6-(hydroxymethyl)-5-methylpyrazin-2-yl)-2-((1,1,1-trifluoropropan-2-yl)oxy)benzamide. To a solution of (S)-methyl 6-(4-((2-chloro-6-fluorophenyl)carbamoyl)-2-fluoro-5-((1,1,1-trifluoropropan-2-yl)oxy)phenyl)-3-methylpyrazine-2-carboxylate (100 mg, 110 µmol) in THF (10 mL) was added LiAlH₄ (20 mg, 527 µmol) at - 60 °C. The mixture was stirred at -60 °C for 1 hr. Additional LiAlH₄ (20 mg, 527 µmol) was added to the mixture and the mixture was stirred at -60 °C for 1 hr. The mixture was poured into NH₄Cl (20 mL, sat.) and extracted with ethyl acetate (20 mL × 2). The combined organic layers were washed with brine (20 mL × 2), dried over Na₂SO₄, filtered and concentrated in vacuo. The residue was purified by prep-HPLC(METHOD A) to give the title compound as a yellow solid. MS (ESI): mass calcd. for C₂₂H₁₇ClF₅N₃O₃, 501.1; m/z found, 502.1 [M+H]⁺. ¹H NMR (400MHz, CDCl₃) δ = 9.09 (s, 1H), 9.04 (d, *J*=2.1 Hz, 1H), 8.19 (d, *J*=11.6 Hz, 1H), 7.76 (d, *J*=5.6 Hz, 1H), 7.33 - 7.28 (m, 1H), 7.27 - 7.21 (m, 1H), 7.19 - 7.10 (m, 1H), 5.12 - 4.95 (m, 1H), 4.89 (d, *J*=3.5 Hz, 2H), 4.08 - 3.84 (m, 1H), 2.60 (s, 3H), 1.69 (d, *J*=6.5 Hz, 3H).

### Example 54: (S)-N-(2-Chloro-6-fluorophenyl)-5-fluoro-4-(1-methyl-5-(trifluoromethyl)-1H-1,2,4-triazol-3-yl)-2-((1,1,1-trifluoropropan-2-yl)oxy)benzamide.

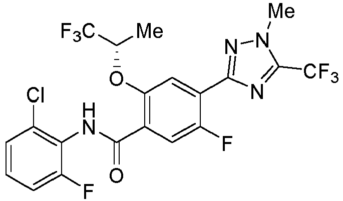

The title compound was prepared in a manner analogous to Example 1, Step E however using 3-bromo-1-methyl-5-(trifluoromethyl)-1H-1,2,4-triazole as the aryl halide and (S)-N-(2-Chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₀H₁₃ClF₈N₄O₂, 528.1; m/z found, 529.0 [M+H]⁺. ¹H NMR (400 MHz, CDCl₃) δ = 9.10 (s, 1H), 8.17 (d, J=10.8 Hz, 1H), 7.79 (d, J=4.9 Hz, 1H), 7.21-7.34 (m, 1H), 7.14 (t, J=8.8 Hz, 1H), 5.03-5.17 (m, 1H), 4.17 (s, 3H), 1.68 (d, J=6.4 Hz, 3H).

### Example 55, (S)-N-(2-Chloro-6-fluorophenyl)-5-fluoro-4-(5-(trifluoromethyl)pyrazin-2-yl)-2-((1,1,1-trifluoropropan-2-yl)oxy)benzamide.

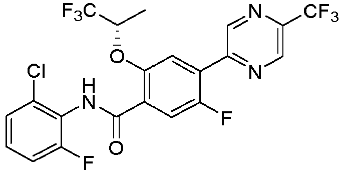

The title compound was prepared in a manner analogous to Example 1, Step E however using 2-bromo-5-(trifluoromethyl)pyrazine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. ¹H NMR (400 MHz, DMSO-*d₆*) δ ppm 10.15 (s, 1 H), 9.41 (s, 1 H), 9.33 (s, 1 H), 7.92 (d, *J*=5.8 Hz, 1 H), 7.62 (d, *J*=10.0 Hz, 1 H), 7.33 - 7.50 (m, 3 H), 5.45 (dt, *J*=12.7, 6.3 Hz, 1 H), 1.48 (d, *J*=6.3 Hz, 3 H); ¹⁹F NMR (376 MHz, DMSO-*d₆*) δ ppm -66.18 (s, 1 F), -77.14 (br s, 1 F), -115.35 (br s, 1 F), -123.07 (s, 1 F); ESI-MS: *m*/*z* 526.0 [M+H]⁺.

### Example 56: (S)-N-(2-Chloro-6-fluorophenyl)-5-fluoro-4-(5,6,7,8-tetrahydro-[1,2,4]triazolo[1,5-a]pyrazin-2-yl)-2-((1,1,1-trifluoropropan-2-yl)oxy(benzamide.

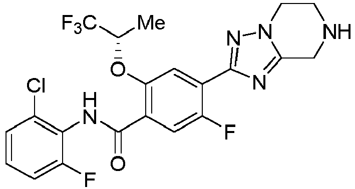

Step A. *tert*-Butyl (S)-2-(4-((2-hloro-6-fluorophenyl)carbamoyl)-2-fluoro-5-((1,1,1-trifluoropropan-2-yl)oxy)phenyl)-5,6-dihydro-[1,2,4]triazolo[1,5-a]pyrazine-7(8H)-carboxylate. (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1, 93 mg, 0.18 mmol), *tert*-butyl 2-bromo-5,6-dihydro[1,2,4]triazolo[1,5-A]pyrazine-7(8H)-carboxylate (111.5 mg, 0.37 mmol), Pd(dppf)Cl₂·CH₂Cl₂ (15.0 mg, 0.018 mmol), Na₂CO₃ (39.0 mg, 0.37 mmol) in 1,4-dioxane (1.8 mL) and water (1.0 mL) were added to a microwave vial. The mixture was sparged with Argon then heated to 80 °C for 16 h. The reaction mixture was cooled to room temperature then diluted with EtOAc and water. The organics were extracted with EtOAc (3x). The combined organic layers were washed with brine, dried over sodium sulfate, filtered and concentrated. Purification by silica gel column chromatography using 20 to 40% EtOAc/heptane gradient afforded the title compound as a white solid. MS (ESI): mass calcd. for C₂₆H₂₅ClF₅N₅O₄, 601.2; m/z found, 602.1 [M+H]⁺. ¹H NMR (400 MHz, CDCl₃) δ = 9.11 (s, 1H), 8.16 (d, J = 11.2 Hz, 1H), 7.79 (d, J = 5.4 Hz, 1H), 7.35 - 7.19 (m, 1H), 7.13 (t, J = 8.6 Hz, 1H), 5.22 - 4.96 (m, 1H), 4.85 (s, 2H), 4.33 (br t, J = 4.9 Hz, 2H), 4.08 - 3.92 (m, 2H), 1.67 (d, J = 6.4 Hz, 3H), 1.56 (s, 9H).

Step B. (S)-N-(2-Chloro-6-fluorophenyl)-5-fluoro-4-(5,6,7,8-tetrahydro-[1,2,4]triazolo[1,5-a]pyrazin-2-yl)-2-((1,1,1-trifluoropropan-2-yl)oxy)benzamide. To a solution of *tert*-butyl (S)-2-(4-((2-chloro-6-fluorophenyl)carbamoyl)-2-fluoro-5-((1,1,1-trifluoropropan-2-yl)oxy)phenyl)-5,6-dihydro-[1,2,4]triazolo[1,5-a]pyrazine-7(8H)-carboxylate (90 mg, 0.15 mmol) in 1,4-dioxane (1.0 mL) was added 4N HCl in dioxane (112 µL, 0.45 mmol). The mixture was stirred for 16 h at room temperature then purified by reverse phase HPLC using a 30 × 100 mm Gemini C18 column and a gradient of 10 to 100% MeCN/10 mM NH₄OH in water to provide the title compound as a white solid. MS (ESI): mass calcd. for C₂₁H₁₇ClF₅N₅O₂, 501.1; m/z found, 502.0 [M+H]⁺. ¹H NMR (400 MHz, CDCl₃) δ = 9.11 (s, 1H), 8.15 (d, J=11.2 Hz, 1H), 7.80 (d, J=4.9 Hz, 1H), 7.20-7.35 (m, 1H), 7.13 (t, J=8.8 Hz, 1H), 5.03-5.20 (m, 1H), 4.19-4.35 (m, 4H), 3.40 (t, J=5.4 Hz, 2H), 1.66 (d, J=6.4 Hz, 3H).

### Example 57: (S)-N-(2-Chloro-6-fluorophenyl)-5-fluoro-4-(5-(hydroxymethyl)-6-methylpyrazin-2-yl)-2-((1,1,1-trifluoropropan-2-yl)oxy)benzamide.

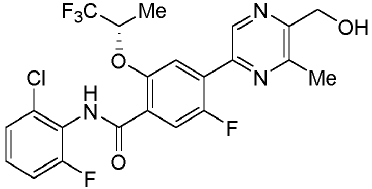

Step A. (S)-Methyl 3-chloro-5-(4-((2-chloro-6-fluorophenyl)carbamoyl)-2-fluoro-5-((1,1,1-trifluoropropan-2-yl)oxy)phenyl)pyrazine-2-carboxylate. The title compound was prepared in a manner analogous to Example 1, Step E however using methyl 3,5-dichloropyrazine-2-carboxylate as the aryl halide and (S)-N-(2-chloro-6-fluorophenyl)-5-fluoro-4-(4,4,5,5-tetramethyl-1 ,3 ,2-dioxaborolan-2-yl)-2-(( 1,1,1-trifluoropropan-2-yl)oxy)benzamide (Intermediate 1) as the boronate. MS (ESI): mass calcd. for C₂₂H₁₄Cl₂F₅N₃O₄, 549.0; m/z found, 550.1 [M+H]⁺.

Step B. (S)-Methyl 5-(4-((2-chloro-6-fluorophenyl)carbamoyl)-2-fluoro-5-((1,1,1-trifluoropropan-2-yl)oxy)phenyl)-3-methylpyrazine-2-carboxylate. To a solution of (S)-methyl 3-chloro-5-(4-((2-chloro-6-fluorophenyl)carbamoyl)-2-fluoro-5-((1,1,1-trifluoropropan-2-yl)oxy)phenyl)pyrazine-2-carboxylate (580 mg, 601 µmol) and 2,4,6-trimethyl-1,3,5,2,4,6-trioxatriborinane (394 mg, 1.57 mmol) in DME (10 mL) were added Pd(dppf)Cl₂ (38.6 mg, 52.7 µmol) and K₂CO₃ (291 mg, 2.11 mmol). The mixture was stirred at 100 °C for 16 hr. The mixture was concentrated in vacuo. The residue was purified by column chromatography (SiO₂, petroleum ether/ethyl acetate=50/1 to 5/1) to give the title compound as a brown solid. MS (ESI): mass calcd. for C₂₃H₁₇ClF₅N₃O₄, 529.0; m/z found, 530.1 [M+H]⁺.

Step C. (S)-N-(2-Chloro-6-fluorophenyl)-5-fluoro-4-(5-(hydroxymethyl)-6-methylpyrazin-2-yl)-2-((1,1,1-trifluoropropan-2-yl)oxy)benzamide. To a solution of (S)-methyl 5-(4-((2-chloro-6-fluorophenyl)carbamoyl)-2-fluoro-5-((1,1,1-trifluoropropan-2-yl)oxy)phenyl)-3-methylpyrazine-2-carboxylate (60 mg, 113 µmol) in THF (5 mL) was added LiBH₄ (2 M in THF, 227 µL) at 0°C, the mixture was stirred at 0 °C for 1 h. The reaction mixture combined with other batches was quenched by addition of 1N HCl (30 mL) at 0 °C, and then extracted with EtOAc (20 mL × 2). The combined organic layers were washed with brine (40 mL), dried over Na₂SO₄, filtered and concentrated in vacuo. The residue was purified by prep-HPLC(METHOD A) to give the title compound as a white solid. MS (ESI): mass calcd. for C₂₂H₁₇ClF₅N₃O₃, 501.0; m/z found, 502.1 [M+H]⁺. ¹H NMR (400MHz, CDCl₃) δ = 9.11 (s, 1H), 9.04 (s, 1H), 8.18 (d, *J* = 11.7 Hz, 1H), 7.90 (d, *J* = 5.7 Hz, 1H), 7.33 - 7.29 (m, 1H), 7.27 - 7.23 (m, 1H), 7.18 - 7.12 (m, 1H), 5.09 (td, *J* = 6.0, 12.3 Hz, 1H), 4.86 (d, *J* = 3.5 Hz, 2H), 4.10 (br s, 1H), 2.62 (s, 3H), 1.70 (d, *J* = 6.5 Hz, 3H).

### Example 58: (S)-N-(2-Chloro-6-fluorophenyl)-4-(5-(1,1-difluoroethyl)-4-(hydroxymethyl)thiazol-2-yl)-5-fluoro-2-(1,1,1-trifluoropropan-2-yl)oxy)benzamide.

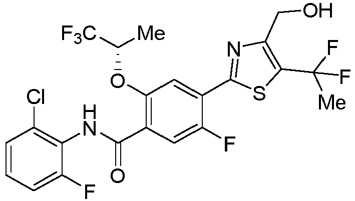

Step A. Ethyl 3-chloro-2,4-dioxopentanoate. A solution of sulfuryl chloride (5.55 g, 41.1 mmol, 4.1 mL) in DCM (10 mL) was added dropwise to a solution of ethyl 2,4-dioxopentanoate (5.0 g, 32 mmol, 4.5 mL) in DCM (80 mL). The mixture was stirred at 20 °C for 8 hrs. The reaction mixture was washed with H₂O (150 mL × 6), dried over anhydrous Na₂SO₄, filtered and concentrated under reduced pressure to give the title compound as a yellow oil. MS (ESI): mass calcd. for C₇H₉ClO₄, 192.0; m/z found, 193.1 [M+H]⁺.

Step B. Ethyl 5-acetyl-2-aminothiazole-4-carboxylate. To a solution of thiourea (1.72 g, 22.6 mmol) in EtOH (29 mL) was added ethyl 3-chloro-2,4-dioxopentanoate (2.9 g, 15 mmol) under N₂. The mixture was stirred at 80 °C for 12 hr. The reaction mixture was concentrated under reduced pressure to remove EtOH. The residue was diluted with EtOAc(30 mL) and H₂O(30 mL), and adjusted the pH to 7 with sat. NaHCO₃. The mixture was extracted with EtOAc(50 mL × 3 ). The combined organic layers were washed with brine(50 mL × 2), dried over anhydrous Na₂SO₄, filtered and concentrated under reduced pressure. The crude product was purified by reverse-phase chromatography (0.1% FA condition) to give the title compound as a yellow solid. MS (ESI): mass calcd. for C₈H₁₀N₂O₃S, 214.0; m/z found, 215.1 [M+H]⁺.

Step C. Ethyl 5-acetyl-2-bromothiazole-4-carboxylate. To a mixture of ethyl 5-acetyl-2-amino-thiazole-4-carboxylate (300 mg, 1.40 mmol) and CuBr₂ (375 mg, 1.68 mmol) in MeCN (5 mL) was added *tert*-butyl nitrite (217 mg, 2.10 mmol, 250 µL) dropwise at 0°C under N₂. The mixture was stirred at 20 °C for 12 hours. The mixture was poured into water (30 mL). The aqueous phase was extracted with ethyl acetate (30 mL × 2). The combined organic phase was washed with brine (20 mL × 2), dried with anhydrous Na₂SO₄, filtered and concentrated in vacuum. The residue was purified by silica gel chromatography (1000 mesh silica gel, petroleum ether/ethyl acetate=100/1 to 10/1) to give the title compound as yellow oil. MS (ESI): mass calcd. for C₈H₈BrNO₃S, 276.9; m/z found, 278.1 [M+H]⁺.

Step D. Ethyl 2-bromo-5-(1,1-difluoroethyl)thiazole-4-carboxylate. To a mixture of ethyl 5-acetyl-2-bromothiazole-4-carboxylate (150 mg, 539 µmol) in DCM (4 mL) was added EtOH (2.48 mg, 53.9 µmol) and DAST (869 mg, 5.39 mmol, 713 µL) dropwise at 0 °C under N₂. The mixture was stirred at 20 °C for 12 hours. The mixture was poured into water (20 mL). The aqueous phase was extracted with ethyl acetate (20 mL × 2). The combined organic phase was washed with brine (15 mL × 2), dried with anhydrous Na₂SO₄, filtered and concentrated in vacuum. The residue was purified by prep-TLC (petroleum ether/ethyl acetate=10/1) to give the title compound as yellow oil. MS (ESI): mass calcd. for C₈H₈BrF₂NO₂S, 298.9; m/z found, 300.0 [M+H]⁺.

Step E. (S)-Ethyl 2-(4-((2-chloro-6-fluorophenyl)carbamoyl)-2-fluoro-5-((1,1,1-trifluoropropan-2-yl)oxy)phenyl)-5-(1,1-difluoroethyl)thiazole-4-carboxylate. The title compound was prepared in a manner analogous to Example 1, Step E however using ethyl 2-bromo-5-(1,1-difluoroethyl)thiazole-4-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₄H₁₈ClF₇N₂O₄S, 598.0; m/z found, 599.1 [M+H]⁺.

Step F. (S)-N-(2-Chloro-6-fluorophenyl)-4-(5-(1.1-difluoroethyl)-4(hydroxymethyl)thiazol-2-yl)5-fluoro-2-((1,1,1-trifluoropropan-2-yl)oxy)benzamide. To a mixture of (S)-ethyl 2-(4-((2-chloro-6-fluorophenyl)carbamoyl)-2-fluoro-5-((1,1,1-trifluoropropan-2-yl)oxy)phenyl)-5-(1,1-difluoroethyl)thiazole-4-carboxylate (70 mg, 117 µmol) in THF (2 mL) was added LiBH₄ (2 M in THF, 351 µL) drop-wise at 0 °C under N₂. The mixture was stirred at 0 °C for 1 hour. The mixture was poured into 0.5N HCl (3 mL). The aqueous phase was extracted with ethyl acetate (15 mL × 3). The combined organic phase was washed with brine (10 mL × 2), dried with anhydrous Na₂SO₄, filtered and concentrated in vacuo. The residue was purified by prep-HPLC (METHOD A) to give the title compound as white solid. MS (ESI): mass calcd. for C₂₂H₁₆CIF₇N₂O₃S, 556.0; m/z found, 557.0 [M+H]⁺. ¹H NMR (400 MHz, CDCl₃) δ = 9.10 (s, 1H), 8.20 (d, *J* = 11.4 Hz, 1H), 8.03 (d, *J* = 5.4 Hz, 1H), 7.33 - 7.28 (m, 1H), 7.26 - 7.22 (m, 1H), 7.20 - 7.10 (m, 1H), 5.14 (td, *J* = 6.1, 12.3 Hz, 1H), 4.94 (br d, *J* = 5.6 Hz, 2H), 2.54 (br t, *J* = 6.0 Hz, 1H), 2.13 (t, *J* = 18.1 Hz, 3H), 1.74 - 1.60 (m, 3H).

### Example 59: N-(2-Chloro-6-fluorophenyl)-5-fluoro-4-(5-(1-hydroxyethyl)-4-(hydroxymethylthiazol-2-yl)-2-(((S)-1,1,1-trifluoropropan-2-yl)oxy)benzamide.

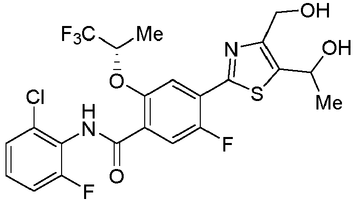

Step A. (S)-Ethyl 5-acetyl-2-(4-((2-chloro-6-fluorophenyl)carbamoyl)-2-fluoro-5-((1.1,1 trifluoropropan-2-yl)oxy)phenyl)thiazole-4-carboxylate. The title compound was prepared in a manner analogous to Example 1, Step E however using ethyl 5-acetyl-2-bromothiazole-4-carboxylate (Example 58, Step C) as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₄H₁₈ClF₅N₂O₅S, 576.0; m/z found, 576.9 [M+H]⁺.

Step B. N-(2-Chloro-6-fluorophenyl)-5-fluoro-4-(5-(1-hydroxyethyl)-4-(hydroxymethyl)thiazol-2-yl)-2-(((S)-1,1,1-trifluoropropan-2-yl)oxy)benzamide. To a mixture of (S)-ethyl 5-acetyl-2-(4-((2-chloro-6-fluorophenyl)carbamoyl)-2-fluoro-5-((1,1,1-trifluoropropan-2-yl)oxy)phenyl)thiazole-4-carboxylate (180 mg, 312 µmol) in THF (4 mL) was added LiBH₄ (2 M in THF, 936 µL) at 0 °C under N₂. The mixture was stirred at 25 °C for 2 hours. The residue was poured into 0.5N HCl (10 mL). The aqueous phase was extracted with ethyl acetate (15 mL × 2). The combined organic phase was washed with brine (10 mL × 2), dried with anhydrous Na₂SO₄, filtered and concentrated in vacuum. The residue was purified by prep-HPLC (METHOD A) to give the title compound as white solid. MS (ESI): mass calcd. for C₂₂H₁₈ClF₅N₂O₄S, 536.0; m/z found, 537.1 [M+H]⁺. ¹H NMR (400 MHz, CDCl₃) δ = 9.11 (s, 1H), 8.17 (d, *J* = 11.5 Hz, 1H), 8.00 (d, *J* = 5.5 Hz, 1H), 7.32 - 7.28 (m, 1H), 7.26 - 7.23 (m, 1H), 7.14 (t, *J* = 8.5 Hz, 1H), 5.38 (br d, *J* = 4.5 Hz, 1H), 5.14 (td, *J* = 6.2, 12.3 Hz, 1H), 4.93 (d, *J* = 4.4 Hz, 2H), 2.82 (br s, 1H), 2.61 (br s, 1H), 1.72 - 1.62 (m, 6H).

### Example 60. (S)-N-(2-Chloro-6-fluorophenyl)-5-fluoro-4-(5,6,7,8-tetrahydro-1,7-naphthyridin-2-yl)-2-(1,1,1-trifluoropropan-2-yl)oxy)benzamide.

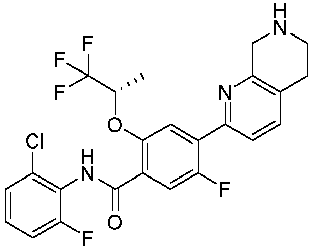

The title compound was prepared according to the representative procedures of Example 56, Steps A-B, except substituting tert-butyl 2-chloro-5,8-dihydro-1,7-naphthyridine-7(6H)-carboxylate for tert-butyl 2-bromo-5,6-dihydro[1,2,4]triazolo[1,5-A]pyrazine-7(8H)-carboxylate. MS (ESI): mass calcd. for C₂₄H₁₉ClF₅N₃O₂, 511.1; m/z found, 512.2 [M+ H]⁺. ¹H NMR (400 MHz, CHLOROFORM-d) δ = 9.15 (s, 1H), 8.11 (d, *J*=11.74 Hz, 1H), 7.80 (d, *J*=5.87 Hz, 1H), 7.72 (d, *J*=7.83 Hz, 1H), 7.52 (d, *J*=7.83 Hz, 1H), 7.28-7.32 (m, 1H), 7.19-7.25 (m, 1H), 7.08-7.18 (m, 1H), 5.09 (td, *J*=6.11, 12.23 Hz, 1H), 4.20 (s, 2H), 3.21 (t, *J*=5.87 Hz, 2H), 2.90 (t, *J*=5.87 Hz, 2H), 1.67 (d, *J*=6.36 Hz, 3H).

### Example 61. (S)-N-(2-Chloro-6-fluorophenyl)-5-fluoro-4-(5,6,7,8-tetrahydro-1,6-naphthyridin-2-yl)-2-((1,1,1-trifluoropropan-2-yl)oxy)benzamide.

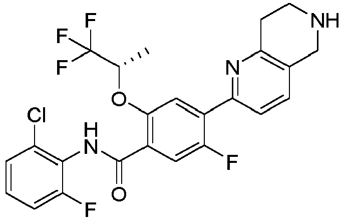

The title compound was prepared according to the representative procedures of Example 56, Steps A-B, except substituting tert-butyl 2-chloro-7,8-dihydro-1,6-naphthyridine-6(5H)-carboxylate for tert-butyl 2-bromo-5,6-dihydro[1,2,4]triazolo[1,5-A]pyrazine-7(8H)-carboxylate. MS (ESI): mass calcd. for C₂₄H₁₉ClF₅N₃O₂, 511.1; m/z found, 512.1 [M+ H]⁺. ¹H NMR (400 MHz, CHLOROFORM-d) δ 9.15 (s, 1H), 8.11 (d, *J*=11.74 Hz, 1H), 7.81 (d, *J*=5.87 Hz, 1H), 7.71 (d, *J*=7.83 Hz, 1H), 7.45 (d, *J*=7.83 Hz, 1H), 7.28-7.35 (m, 1H), 7.19-7.25 (m, 1H), 7.14 (t, *J*=8.85 Hz, 1H), 5.11 (td, *J*=6.11, 12.23 Hz, 1H), 4.10 (s, 2H), 3.30 (t, *J*=5.87 Hz, 2H), 3.06 (t, *J*=5.87 Hz, 2H), 1.64-1.71 (m, 4H), 0.03-0.19 (m, 1H).

### Example 62, (S)-N-(5-Chloro-3-methyl-1H-pyrazol-4-yl)-4-(5-cyclopropylpyridin-2-yl)-5-fluoro-2-((1,1,1-trifluoropropan-2-yl)oxy)benzamide.

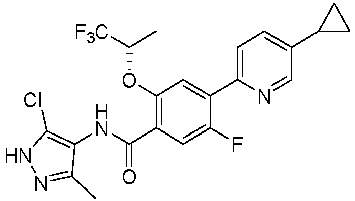

Step A. 5-Chloro-3-methyl-1H-pyrazol-4-amine. Zinc powder (972 mg, 14.8 mmol) and ammonium chloride (1.6 g, 30 mmol) were added to a stirred solution of 5-chloro-3-methyl-4-nitro-1*H-*pyrazole ( 300 mg, 1.9 mmol) in THF (15 mL) and H₂O (3 mL) at 0 °C. The resulting mixture was warmed to room temperature and stirred for 1 hour. The mixture was filtered, the filtrate was washed with water and extracted with EtOAc. The organic layers were concentrated under vacuum to the title compound as red solid (140 mg crude). ¹H NMR (400 MHz, DMSO-*d₆*) δ ppm 12.07 (br s, 1 H), 3.56 (br s, 2 H), 2.08 (s, 3 H).

Step B. Methyl (S)-4-(5-cyc1opropylpyridin-2-yl)-5-fluoro-2-((1,1,1-trifluoropropan-2-yl)oxy)benzoate. The title compound was prepared in a manner analogous to Example 14, Step D however using 2-chloro-5-cyclopropylpyridine as the aryl halide and (S)-methyl 5-fluoro-4-(4,4,5,5-tetramethyl-1,3,2-dioxaborolan-2-yl)-2-((1,1,1-trifluoropropan-2-yl)oxy)benzoate (Intermediate 2) as the boronate. MS (ESI): mass calcd. for C₁₉H₁₇F₄NO₃, 383.1; m/z found, 384.1 [M+ H]⁺.¹H NMR (400 MHz, CHLOROFORM-*d*) δ ppm 8.53 (d, *J*=2.3 Hz, 1 H), 7.74 - 7.81 (m, 2 H), 7.64 (d, *J*=11.3 Hz, 1 H), 7.38 (dd, *J*=8.3, 2.3 Hz, 1 H), 4.79 (spt, *J*=6.2 Hz, 1 H), 3.92 (s, 3 H), 1.90 - 2.02 (m, 1 H), 1.57 (d, *J*=6.5 Hz, 3 H), 1.06 - 1.14 (m, 2 H), 0.76 - 0.84 (m, 2 H); ¹⁹F NMR (376 MHz, CHLOROFORM-*d*) δ ppm -78.43 (s, 1 F), -123.29 (s, 1 F).

Step C. (S)-4-(5-Cyclopropylpyridin-2-yl)-5-fluoro-2-((1,1,1-trifluoropropan-2-yl)oxy)benzoic acid. To methyl (S)-4-(5-cyclopropylpyridin-2-yl)-5-fluoro-2-((1,1,1-trifluoropropan-2-yl)oxy)benzoate (590 mg, 1.4 mmol) in THF (5 mL), MeOH (5 mL) and H₂O (5 mL) was added NaOH (112 mg, 2.8 mmol). The mixture was stirred at room temperature for 16 hours. The mixture was concentrated under vacuum, then 2M aqueous HCl solution was added to adjust the pH to 2~3. The solid was filtered and dried to give the title compound as a white powder. MS (ESI): mass calcd. for C₁₈H₁₅F₄NO₃, 369.1; m/z found, 370.0 [M+ H]⁺. ¹H NMR (400 MHz, CHLOROFORM-*d*) δ ppm 8.56 (d, *J*=2.0 Hz, 1 H), 7.93 (d, *J*=11.3 Hz, 1 H), 7.80 - 7.89 (m, 2 H), 7.42 (dd, *J*=8.3, 2.3 Hz, 1 H), 4.99 - 5.11 (m, 1 H), 1.93 - 2.05 (m, 1 H), 1.63 (d, *J*=6.5 Hz, 3 H), 1.07 - 1.17 (m, 2 H), 0.75 - 0.86 (m, 2 H); ¹⁹F NMR (376 MHz, CHLOROFORM-*d*) δ ppm -78.45 (s, 1 F), -121.57 (s, 1 F).

Step D. (S)-N-(5-Chloro-3-methyl-1H-pyrazol-4-yl)-4-(5-cyclopropylpyridin-2-yl)-5-fluoro-2-((1,1,1-trifluoropropan-2-yl)oxy)benzamide. To a mixture of (S)-4-(5-cyclopropylpyridin-2-yl)-5-fluoro-2-((1,1,1-trifluoropropan-2-yl)oxy)benzoic acid (100 mg, 0.26 mmol) and 5-chloro-3-methyl-1H-pyrazol-4-amine (51 mg, 0.39 mmol) in DCM (3 mL) was added Et₃N (65 mg, 0.64 mmol). The mixture was stirred at room temperature for 10 min, then T₃P (50% solution in EtOAc, 177 mg, 0.39 mmol) was added. The resulting reaction mixture was heated at 50 °C and stirred for 16 hours. The mixture was washed with saturated aqueous NaHCO₃ solution and extracted with EtOAc. The combined organic layers were concentrated under vacuum. The residue was purified by preparative high-performance liquid chromatography (Stationary phase: Boston Prime C18, 5 µm, 150 × 30 mm; Mobile phase: water (0.04% NH₃H₂O + 10 mM NH₄HCO₃) (A) - MeCN (B), gradient elution: 55 - 85% B in A over 7 min, flow rate: 25 mL/min). The pure fraction was lyophilized to give the title compound as off-white powder. MS (ESI): mass calcd. for C₂₂H₁₉ClF₄N₄O₂, 482.1; m/z found, 483.1 [M+H]⁺. ¹H NMR (400 MHz, CHLOROFORM-*d*) δ ppm 10.99 (br s, 1 H), 8.84 (s, 1 H), 8.54 (d, J=2.3 Hz, 1 H), 8.07 (d, *J*=11.8 Hz, 1 H), 7.85 (d, *J*=6.0 Hz, 2 H), 7.41 (dd, J=8.3, 2.3 Hz, 1 H), 5.11 (spt, J=6.1 Hz, 1 H), 2.30 (s, 3 H), 1.94 - 2.01 (m, 1 H), 1.68 (d, J=6.5 Hz, 3 H), 1.05 - 1.18 (m, 2 H), 0.78 - 0.84 (m, 2 H); ¹⁹F NMR (376 MHz, CHLOROFORM-*d*) δ ppm -78.31 (s, 1 F), -122.47 (s, 1 F).

### Example 63. (S)-N-(2-Chloro-4-methylpvridin-3-vl)-4-(5-cyclopropylpyridin-2-yl)-5-fluoro-2-((1,1,1-trifluoropropan-2-yl)oxy)benzamide.

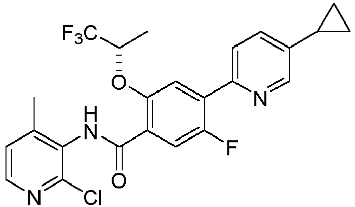

To the mixture of (S)-4-(5-cyclopropylpyridin-2-yl)-5-fluoro-2-((1,1,1-trifluoropropan-2-yl)oxy)benzoic acid (Example 62, Step C, 180 mg, 0.46 mmol), 3-amino-2-chloro-4-methylpyridine (66 mg, 0.46 mmol) and pyridine (183 mg, 2.3 mmol) in DCM (3 mL) was added POCl₃ (142 mg, 0.93 mmol). The resulting reaction mixture was stirred at room temperature for 16 hours. The mixture was quenched with saturated aqueous NaHCO₃ solution and extracted with EtOAc. The combined organic layers were concentrated under vacuum. The residue was purified by preparative high-performance liquid chromatography (Stationary phase: Boston Prime C18, 5 µm, 150 × 30 mm; Mobile phase: water (0.04% NH₃H₂O + 10 mM NH₄HCO₃) (A) - MeCN (B), gradient elution: 65 - 95% B in A over 7 min, flow rate: 25 mL/min). The pure fraction was lyophilized to give the title compound as a pale yellow powder. MS (ESI): mass calcd. for C₂₄H₂₀ClF₄N₃O₂, 493.1; m/z found, 494.0 [M+ H]⁺.¹H NMR (400 MHz, CHLOROFORM-*d*) δ ppm 9.25 (s, 1 H), 8.55 (d, J=2.0 Hz, 1 H), 8.22 (d, J=5.0 Hz, 1 H), 8.09 (d, J=12.0 Hz, 1 H), 7.89 (d, J=6.0 Hz, 1 H), 7.86 (d, J=7.3 Hz, 1 H), 7.41 (dd, J=8.3, 2.5 Hz, 1 H), 7.20 (d, J=5.0 Hz, 1 H), 5.10 - 5.22 (m, 1 H), 2.36 (s, 3 H), 1.94 - 2.02 (m, 1 H), 1.70 (d, J=6.3 Hz, 3 H), 1.09 - 1.15 (m, 2 H), 0.79 - 0.84 (m, 2 H); ¹⁹F NMR (376 MHz, CHLOROFORM-*d*) δ ppm -78.17 (s, 1 F), -122.44 (s, 1 F).

### Example 64. (S)-N-(2-Chloro-6-fluorophenyl)-5-fluoro-4-(5-(1-(hydroxymethyl)cyclopropyl)pyrazin-2-yl)-2-((1,1,1-trifluoropropan-2-yl)oxy)benzamide.

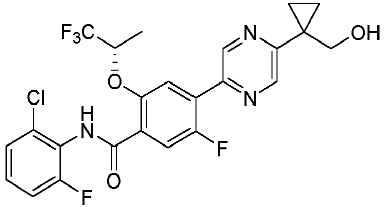

Step A. Ethyl 2-(5-chloropyrazin-2-yl)acetate. Diethyl malonate (7.45 g, 46.5 mmol), picolinic acid (382 mg, 3.1 mmol) and Cs₂CO₃ (15.2 g, 46.5 mmol) was added to a solution of 2-bromo-5-chloropyrazine (3 g, 15.5 mmol) in dioxane (30 mL) under N₂. Then CuI (1.2 g, 6.2 mmol) was added to the mixture and stirred at 105 °C overnight then cooled down to room temperature. The reaction was concentrated, the residue was diluted with EtOAc and washed with water. The organic layer was washed with brine and dried over Na₂SO₄, filtered and concentrated. The residue was purified by flash column chromatography over silica gel (gradient elution: 0 - 30% EtOAc in petroleum ether) to give the title compound as a light yellow oil. MS (ESI): mass calcd. for C₈H₉ClN₂O₂, 200.0; m/z found, 201.2 [M+ H]⁺.¹H NMR (400 MHz, CHLOROFORM-*d*) δ ppm 8.53 (s, 1 H), 8.36 (s, 1 H), 4.15 - 4.20 (m, 2 H), 3.84 (s, 2 H), 1.24 - 1.28 (m, 3 H).

Step B. Ethyl 1-(5-chloropyrazin-2-yl)cyclopropane-1-carboxylate. NaH (60% purity, 550 mg, 13.8 mmol) was slowly added into the solution of ethyl 2-(5-chloropyrazin-2-yl)acetate (1.2 g, 6.0 mmol) in DMF (15 mL) at 0 °C. The mixture reaction was stirred at room temperature for half an hour. Then 1,2-dibromoethane (1.2 g, 6.6 mmol) was added and the mixture was stirred at room temperature for 2 hours. The reaction was quenched with water and extracted with EtOAc. The organic layer was washed with brine, dried over Na₂SO₄, filtered and concentrated. The residue was purified by flash column chromatography over silica gel (gradient elution: 0 - 30% EtOAc in petroleum ether) to give the title compound as white solid. MS (ESI): mass calcd. for C₁₀H₁₁ClN₂O₂, 226.1; m/z found, 227.4 [M+ H]⁺. ¹H NMR (400 MHz, CHLOROFORM-*d*) δ ppm 8.66 (d, *J*=1.2 Hz, 1 H), 8.43 (d, J=1.2 Hz, 1 H), 4.13 - 4.19 (m, 2 H), 1.68 - 1.76 (m, 2H), 1.45 - 1.50 (m, 2 H), 1.21 (t, J=7.2 Hz, 3 H).

Step C. Ethyl (S)-1-(5-(4-((2-chloro-6-fluorophenyl)carbamoyl)-2-fluoro-5-((1,1,1-trifluoropropan-2-yl)oxy)phenyl)pyrazin-2-yl)cyclopropane-1-carboxylate. The title compound was prepared in a manner analogous to Example 48, Step B however using ethyl 1-(5-chloropyrazin-2-yl)cyclopropane-1-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₆H₂₁ClF₅N₃O₄, 559.1; m/z found, 560.1 [M+ H]⁺.

Step D (S)-N-(2-Chloro-6-fluorophenyl)-5-fluoro-4-(5-(1-(hydroxymethyl)cyclopropyl)pyrazin-2-yl)-2-((1,1.1-trifluoropropan-2-yl)oxy)benzamide. To a stirred mixture of LiAlH₄ (10 mg, 0.26 mmol) in 3 mL of THF was added a solution of ethyl (S)-1-(5-(4-((2-chloro-6-fluorophenyl)carbamoyl)-2-fluoro-5-((1,1,1 -trifluoropropan-2-yl)oxy)phenyl)pyrazin-2-yl)cyclopropane-1-carboxylate (90 mg, 0.13 mmol) in 2 mL of THF slowly at 0 °C. The mixture was stirred at room temperature for 0.5 hour. Water (0.012 mL), 15% aqueous NaOH solution (0.008 mL) and water (0.036 mL) were added to the mixture in order slowly at 0 °C. Then Na₂SO₄ was added to the mixture and stirred at room temperature for 0.5 hour. The mixture was filtered and filtrate was concentrated. The residue was purified by preparative reversed phase HPLC (Stationary phase: Boston Prime C18, 5 µm, 150 x 30 mm; Mobile phase: water (0.04% NH₃H₂O + 10 mM NH₄HCO₃) (A) - MeCN (B), gradient elution: 50 - 80% B in A over 8 min, flow rate: 25 mL/min). The pure fraction was lyophilized to give the title compound as a light yellow powder. MS (ESI): mass calcd. for C₂₄H₁₉ClF₅N₃O₃, 527.0; m/z found, 528.1 [M+ H]⁺.¹H NMR (400 MHz, METHANOL-*d*₄) δ ppm 8.99 (s, 1 H), 8.92 (d, J=1.5 Hz, 1 H), 7.93 (d, J=5.7 Hz, 1 H), 7.71 (d, *J*=11.0 Hz, 1 H), 7.32 - 7.41 (m, 2 H), 7.18 - 7.27 (m, 1 H), 5.29 (dt, J=12.5, 6.3 Hz, 1 H), 3.95 (s, 2 H), 1.59 (d, J=6.4 Hz, 3 H), 1.32 - 1.39 (m, 2 H), 1.05 - 1.12 (m, 2 H); ¹⁹F NMR (376 MHz, METHANOL-*d*₄) δ ppm -79.76 (br d, *J*=8.1 Hz, 1 F), -120.22 - -113.00 (m, 1 F), -127.45 - -121.67 (m, 1 F).

### Example 65: N-(2-Chloro-6-fluorophenyl)-5-fluoro-4-(4-(fluoromethyl)-5-(1-hydroxyethyl)thiazol-2-yl)-2-(((S)-1,1,1-trifluoropropan-2-yl)oxy(benzamide.

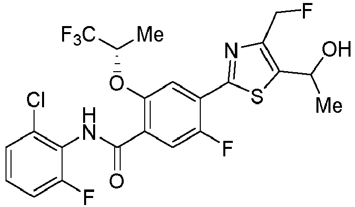

To a solution of N-(2-chloro-6-fluorophenyl)-5-fluoro-4-(5-(1-hydroxyethyl)-4-(hydroxymethyl)thiazol-2-yl)-2-(((S)-1,1,1-trifluoropropan-2-yl)oxy)benzamide (Example 59, Step B, 50 mg, 93.13 µmol) and 4A MS (100 mg) in THF (8 mL) was added TosF (13.05 mg, 186.25 µmol) followed by TBAF (1 M in THF, 279.38 µL) at 0 °C under N₂. The mixture was stirred at 20 °C for 5 hcours. The mixture was poured into water (30 mL). The aqueous phase was extracted with ethyl acetate (20 mL × 2). The combined organic phase was washed with brine (15 mL × 2), dried with anhydrous Na₂SO₄, filtered and concentrated in vacuum. The residue was purified by prep-HPLC (METHOD A) to give the title compound as a white solid. MS (ESI): mass calcd. for C₂₂H₁₈ClF₅N₂O₄S, 538.1; m/z found, 539 [M+H]⁺. ¹H NMR (400 MHz, CDCl₃) δ = 9.12 (s, 1H), 8.18 (d, *J =* 11.5 Hz, 1H), 8.02 (d, *J =* 5.4 Hz, 1H), 7.36 - 7.28 (m, 1H), 7.26 - 7.23 (m, 1H), 7.17 - 7.12 (m, 1H), 5.72 - 5.67 (m, 1H), 5.61 - 5.53 (m, 1H), 5.45 (br s, 1H), 5.19 - 5.10 (m, 1H), 2.27 (br s, 1H), 1.70 (d, J = 6.4 Hz, 6H).

### Example 66. (S)-N-(2-Chloro-6-fluorophenyl)-5-fluoro-4-(5-(hydroxymethyl)-1-methyl-1H-1,2,4-triazol-3-yl)-2-((1,1,1-trifluoropropan-2-yl)oxy)benzamide.

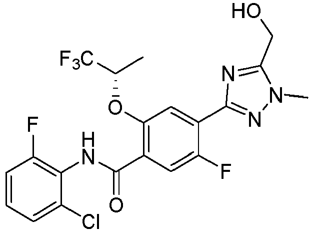

Step A. Methyl 3-bromo-1-methyl-1H-1,2,4-triazole-5-carboxylate. A mixture of methyl 3-bromo-1*H*-1,2,4-triazole-5-carboxylate (500 mg, 2.4 mmol) and K₂CO₃ (671 mg, 4.9 mmol) in acetone (2 mL) was stirred at room temperature for 15 minutes under N₂. MeI (516 mg, 3.6 mmol) in acetone (1 mL) was added to the mixture. The mixture was stirred at room temperature for 4 hours. The reaction was quenched by the addition of aqueous saturated NH₄Cl solution. The organic layer was separated, and the aqueous layer was extracted with EtOAc. The combined organic extract was washed with brine, dried over Na₂SO₄, filtered and concentrated under vacuum. The crude product was purified by chromatography over silica gel (eluent: 0 - 10% EtOAc in petroleum ether) to give the title compound as a yellow powder. MS (ESI): mass calcd. for C₅H₆BrN₃O₂, 219.0; m/z found, 222.0 [M+ H]⁺. ¹H NMR (400 MHz, CHLOROFORM-*d*) δ ppm 4.24 (s, 3 H), 4.02 (s, 3 H).

Step B. (3-Bromo-1-methyl-1H-1,2,4-triazol-5-yl)methanol. To a mixture of methyl 3-bromo-1-methyl-1H-1,2,4-triazole-5-carboxylate (250 mg, 1.1 mmol) in EtOH (15 mL) at 0 °C was added NaBH₄ (52 mg, 1.3 mmol) and CaCl₂ (76 mg, 0.68 mmol) under N₂. The reaction mixture was warmed to room temperature and stirred at room temperature for 12 hours. The mixture was concentrated to dryness. The mixture was diluted with water and extracted with EtOAc. The organic layer was washed with brine, dried with Na₂SO₄, filtered and concentrated to give the title compound as colorless oil. MS (ESI): mass calcd. for C₄H₆BrN₃O, 191.0; m/z found, 192.0 [M+ H]⁺. ¹H NMR (400 MHz, CHLOROFORM-*d*) δ ppm 4.76 (d, J=6.6 Hz, 2 H), 3.93 (s, 3 H), 3.56 (t, J=6.0 Hz, 1 H).

Step C. (S)-N-(2-Chloro-6-fluorophenyl)-5-fluoro-4-(5-(hydroxymethyl)-1-methyl-1H-1,2,4-triazol-3-yl)-2-((1,1,1-trifluoropropan-2-yl)oxy)benzamide. The title compound was prepared in a manner analogous to Example 1, Step E however using (3-bromo-1-methyl-1H-1,2,4-triazol-5-yl)methanol (50 mg, 0.25 mmol) as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₀H₁₆ClF₅N₄O₃, 490.1; m/z found, 491.0 [M+ H]⁺. ¹H NMR (400 MHz, DMSO-*d₆*) δ ppm 10.05 (s, 1 H), 7.83 (d, *J*=5.8 Hz, 1 H), 7.31 - 7.53 (m, 4 H), 5.71 (t, *J*=5.8 Hz, 1 H), 5.31 - 5.45 (m, 1 H), 4.69 (d, J=5.8 Hz, 2 H), 3.97 (s, 3 H), 1.46 (d, *J*=6.3 Hz, 3 H); ¹⁹F NMR (376 MHz, DMSO-*d₆*) δ ppm -77.01 (s, 3 F), -115.41 (br s, 1 F), -119.45 (s, 1 F).

### Example 67: (S)-N-(2-Chloro-6-fluorophenyl)-6'-cyclopropyl-3-fluoro-6-((1,1,1-trifluoropropan-2-yl)oxy)- [2,3'-bipyridine]-5 -carboxamide.

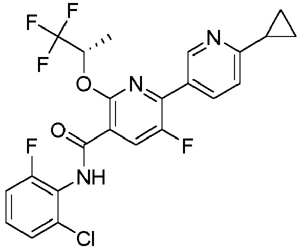

Step A. Isopropyl 2-chloro-5-fluoro-6-(methylthio)nicotinate. To a solution of isopropyl 2,6-dichloro-5-fluoronicotinate (prepared as described in WO2016/97862, 2016, A2; 5 g, 19.8 mmol) in 40 mL of THF was added a solution of sodium thiomethoxide (1.39 g, 19.8 mmol) in 10 mL of THF at 0 °C. The resultant mixture was stirred at rt for 15 hours. The reaction mixture was poured into water (10 mL) and extracted with ethyl acetate (20 mL × 2). The combined organic extracts were washed with brine, dried over MgSO₄, filtered, and concentrated to dryness under reduced pressure. The crude product was purified by chromatography (SiO₂, Heptane/Ethyl acetate=100/0 to Heptane/Ethyl acetate= 40/60). The pure fractions were collected, and the solvent was evaporated until dryness to afford the title compound (5.56 g, quantitative). MS (ESI): mass calcd. for C₁₀H₁₁ClFNO₂S , 263.0; m/z found, 264.0 [M+H]+.

Step B. Isopropyl (S)-5-fluoro-6-(methylthio)-2-((1,1,1-trifluoropropan-2-yl)oxy)nicotinate. Into a sealed tube, purged and maintained with an inert atmosphere of nitrogen, was placed isopropyl 2-chloro-5-fluoro-6-(methylthio)nicotinate (1.28 g, 4.85 mmol), (S)-1,1,1-trifluoro-2-propanol (1.00 g, 8.74 mmol) and Cs₂CO₃ (4.74 g, 14.6 mmol) in toluene (25.6 mL, 240.9 mmol) and then methanesulfonato(2-dicyclohexylphosphino-2',4',6'-tri-i-propyl-1,1'-biphenyl)(2'-amino-1,1'-biphenyl-2-yl)palladium(II) (82.2 mg, 0.097 mmol) was added. The reaction mixture was stirred at 90°C overnight, was hydrolyzed with H₂O, extracted with DCM. The organic layer was washed with H₂O, dried over MgSO₄, filtered and concentrated in vacuum. The residue was purified by chromatography (SiO₂, Heptane/Ethyl acetate= 100 to Heptane/Ethyl acetate= 60-40). The fractions were collected and the solvent was evaporated until dryness to give the title compound. MS (ESI): mass calcd. for C₁₃H₁₅F₄NO₃S , 341.1; m/z found, 342.0 [M+H]+.

Step C. Isopropyl (S)-6'-cyclopropyl-3-fluoro-6-((1,1,1-trifluoropropan-2-yl)oxy-[2,3'-bipyridine]-5-carboxylate. In a microwave vial with a stir bar was added tetrakis(triphenylphosphine)palladium(0) (72.3 mg, 0.063 mmol) to a solution of isopropyl (S)-5-fluoro-6-(methylthio)-2-((1,1,1-trifluoropropan-2-yl)oxy)nicotinate (213.5 mg, 0.63 mmol), 6-cyclopropyl-3-pyridinyl boronic acid pinacol ester (230 mg, 0.94 mmol) and copper(I) 3-methylsalicylate (402.9 mg, 1.88 mmol) in THF (5 mL) which was flushed with argon for 10 min. Then the reaction mixture was degassed and bubbled with argon again for an additional 5 min. The mixure was then heated in a microwave at 100 °C for 1hr. The reaction mixture was hydrolyzed with H₂O, extracted with DCM. The organic layer was washed with H₂O, dried over MgSO4, filtered and concentrated in vacuum. The residue was purified by silica gel chromatography (SiO₂, Heptane/Ethyl acetate= 100 to Heptane/Ethyl acetate= 60-40). The fractions were collected and the solvent was evaporated until dryness to afford the title compound. MS (ESI): mass calcd. for C₂₀H₂₀F₄N₂O₃ , 412.1; m/z found, 413.1 [M+H]+

Step D. (S)-N-(2-Chloro-6-fluorophenyl)-6'-cyclopropyl-3-fluoro-6-(1,1,1-trifluoropropan-2-yl)oxy)-[2,3'-bipyridine]-5-carboxamide. To a solution of 2-chloro-6-fluoroaniline (46.6 mg, 0.32 mmol) in 0.2 mL of (DCM) was added trimethylaluminium 2N in toluene (160 µl, 0.32 mmol) at 0 °C. The reaction was stirred at 0 °C for 20 min. A solution of isopropyl (S)-6'-cyclopropyl-3-fluoro-6-((1,1,1-trifluoropropan-2-yl)oxy)-[2,3'-bipyridine]-5-carboxylate (44 mg, 0.107 mmol) in 0.4 mL of (DCM) was added and this reaction mixture was stirred at 60 °C for 2.5h. The reaction was cooled to 0 °C and few drops of MeOH was slowly added (emulsion forms) and 1N HCl was slowly added. The mixture was diluted in EtOAc and washed with brine. The organic layers were dried over MgSO₄, filtered and concentrated in vacuum. The residue was purified by reverse phase chromatography (Phenomenex Gemini-NX, C18, 150x30mm, 5 um; 30 mL/min; Buffer A: 20 mM NH4OH/water Buffer B: MeCN; gradient: 20% B for 2 min then linear gradient to 80 % B over 10 min and hold at 80% B). The pure fractions were collected, and the solvent was evaporated until dryness to afford the title compound. MS (ESI): mass calcd. for C₂₃H₁₇ClF₅N₃O₂, 497.1; m/z found, 498.1 [M+H]+. 1H NMR (400 MHz, CHLOROFORM-d) δ ppm 9.18 (br d, J=10.76 Hz, 2 H) 8.48 (d, J=10.76 Hz, 1 H) 8.21 (dd, J=8.31, 0.98 Hz, 1 H) 7.30 (s, 2 H) 7.10 - 7.21 (m, 1 H) 5.97 - 6.21 (m, 1 H) 1.69 (d, J=6.85 Hz, 4 H) 1.02 - 1.16 (m, 4 H).

### Example 68. (S)-N-(2-chloro-6-fluorophenyl)-3-fluoro-6'-methyl-6-(1,1,1-trifluoropropan-2-yl)oxy)-[2,3'-bipyridine]-5-carboxamide.

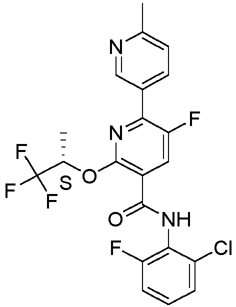

The title compound was prepared according to the representative procedures of Example 67, Step C-D, except substituting 6-methylpyridine-3-boronic acid for 6-cyclopropyl-3-pyridinyl boronic acid pinacol ester. MS (ESI): mass calcd. for C₂₁H₁₅ClF₅N₃O₂ , 471.1; m/z found, 472.0 [M+H]+. 1H NMR (400 MHz, CHLOROFORM-d) δ ppm 9.19 (s, 2 H) 8.50 (d, J=10.76 Hz, 1 H) 8.15 - 8.30 (m, 1 H) 7.28 - 7.35 (m, 2 H) 7.15 (s, 1 H) 6.09 (s, 1 H) 2.66 (s, 3 H) 1.70 (d, J=6.85 Hz, 3 H).

### Example 69. (S)-4-(6-Amino-5-methylpyridin-2-yl)-N-(2-chloro-6-fluorophenyl)-5-fluoro-2-(1,1,1-trifluoropropan-2-yl)oxy)benzamide.

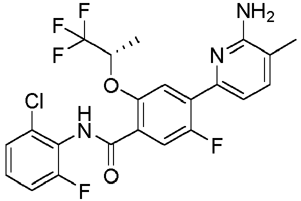

The title compound was prepared in a manner analogous to Example 1, Step E however using 6-bromo-3-methylpyridin-2-amine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₂H₁₇ClF₅N₃O₂, 485.1; m/z found, 486.1 [M+H]⁺. ¹H NMR (CHLOROFORM-d, 400 MHz) δ 9.15 (s, 1H), 8.08 (d, 1H, J=12.2 Hz), 7.82 (d, 1H, J=5.9 Hz), 7.40 (d, 1H, J=7.3 Hz), 7.3-7.3 (m, 2H), 7.2-7.3 (m, 1H), 7.13 (t, 1H, *J*=8.7 Hz), 5.09 (spt, 1H, J=6.2 Hz), 4.55 (br s, 2H), 2.2-2.2 (m, 3H), 1.6-1.7 (m, 3H).

### Example 70. (S)-N-(2-chloro-6-fluorophenyl)-5-fluoro-4-(3-oxo-3,4-dihydro-2H-pyrido[3,2-b][1,4]oxazin-6-yl)-2-(1,1,1-trifluoropropan-2-yl)oxy)benzamide.

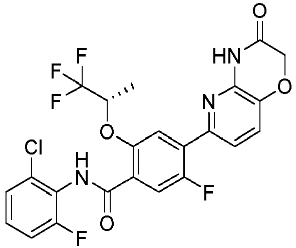

The title compound was prepared in a manner analogous to Example 1, Step E however using 6-bromo-2H-pyrido[3,2-b][1,4]oxazin-3(4H)-one as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₃H₁₅ClF₅N₃O₄, 527.1; m/z found, 528 [M+H]⁺. ¹H NMR (CHLOROFORM-d, 400 MHz) δ 8.05 (br d, 1H, *J*=11.7 Hz), 7.81 (br d, 1H, *J*=5.9 Hz), 7.65 (br d, 1H, *J*=8.3 Hz), 7.2-7.4 (m, 4H), 7.1-7.2 (m, 1H), 5.1-5.2 (m, 1H), 4.73 (s, 2H), 1.68 (br d, 3H, *J=*6.4 Hz).

### Example 71. (S)-N-(2-Chloro-6-fluorophenyl)-4-(2,3-dihydro-1H-pyrrolo[2,3-b]pyridin-6-yl)-5-fluoro-2-(1,1,1-trifluoropropan-2-yl)oxy)benzamide.

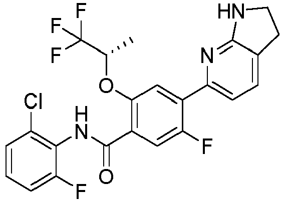

The title compound was prepared in a manner analogous to Example 1, Step E however using 6-chloro-2,3-dihydro-1H-pyrrolo[2,3-b]pyridine for 2-bromo-pyrazine as the aryl halide and and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₃H₁₇ClF₅N₃O₂, 497.1; m/z found, 498.1 [M+H]⁺. ¹H NMR (CHLOROFORM-d, 400 MHz) δ 9.15 (s, 1H), 8.07 (d, 1H, *J=*11.7 Hz), 7.78 (d, 1H, J=5.4 Hz), 7.36 (d, 1H, J=7.3 Hz), 7.3-7.3 (m, 1H), 7.2-7.3 (m, 2H), 7.13 (s, 1H), 5.10 (td, 1H, J=6.3, 12.3 Hz), 4.64 (br s, 1H), 3.69 (t, 2H, J=8.3 Hz), 3.14 (t, 2H, J=8.3 Hz), 1.65 (br d, 3H, J=6.4 Hz).

### Example 72: (S)-N-(2-Chloro-6-fluorophenyl)-4-(5-chloropyridin-2-yl)-5-fluoro-2-((1,1,1-trifluoropropan-2-yl)oxy)benzamide.

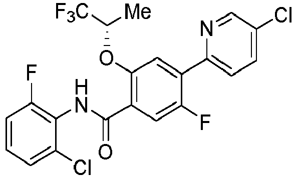

The title compound was prepared in a manner analogous to Example 1, Step E however using 2-bromo-5-chloropyridine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₁H₁₃Cl₂F₅N₂O₂, 490.3; m/z found, 491.1 [M+H]⁺. ¹H NMR (400 MHz, CDCl₃) δ = 9.14 (s, 1 H) 8.69 (d, J=1.96 Hz, 1 H) 8.14 (d, J=12.23 Hz, 1 H) 7.95 (d, J=8.31 Hz, 1 H) 7.88 (d, J=5.87 Hz, 1 H) 7.80 (dd, J=8.31, 2.45 Hz, 1 H) 7.28 - 7.32 (m, 1 H) 7.24 (br s, 1 H) 7.14 (br t, J=8.80 Hz, 2 H) 5.07 - 5.18 (m, 1 H) 1.69 (d, J=6.36 Hz, 3 H).

### Example 73: (S)-N-(2-Chloro-4-methylpyridin-3-yl)-4-(5-chloro-6-(hydroxymethyl)pyridin-2-yl)-5-fluoro-2-((1,1,1-trifluoropropan-2-yl)oxy)benzamide.

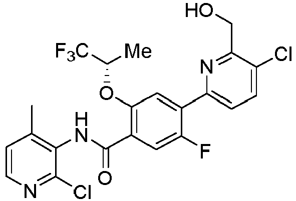

Step A. (6-Bromo-3-chloropvridin-2-vl)methanol. Lithium borohydride (35 mg, 1.6 mmol) was added to a solution of methyl 6-bromo-3-chloropicolinate (100 mg, 0.4 mmol) in THF (2.5 mL) at 0 °C and the reaction mixture was stirred at 0 °C for 45 min. The reaction mixture was quenched by addition of water, then 3M aqueous NaOH. DCM was added and the suspension was filtered on Celite^{®}. The organic layer was dried over MgSO₄, filtered and concentrated under reduced pressure to give the title compound. MS (ESI): mass calcd. for C₆H₅BrClNO, 220.9; m/z found, 222.0 [M+H]⁺. ¹H NMR (400 MHz, CDCl₃) δ = 7.54 (d, J=7.83 Hz, 1 H) 7.40 (d, J=8.31 Hz, 1 H) 4.79 (d, J=4.89 Hz, 2 H) 3.72 (t, J=5.14 Hz, 1 H).

Step B. (S)-N-(2-Chloro-4-methylpyridin-3-yl)-4-(5-chloro-6-(hydroxymethyl)pyridin-2-yl)-5-fluoro-2-((1,1,1-trifluoropropan-2-yl)oxy)benzamide. The title compound was prepared according to the representative procedure of Example 31, Steps A-B, except substituting (6-bromo-3-chloropyridin-2-yl)methanol for 2-bromo-5-methylpyridine. MS (ESI): mass calcd. for C₂₂H₁₇Cl₂F₄N₃O₃, 517.1; m/z found, 518.1 [M+H]⁺. ¹H NMR (400 MHz, CDCl₃) δ = 9.21 (s, 1 H) 8.22 - 8.24 (m, 1 H) 8.11 - 8.17 (m, 1 H) 7.86 (d, J=4.89 Hz, 2 H) 7.73 - 7.78 (m, 1 H) 7.20 (s, 1 H) 5.07 (t, J=6.11 Hz, 1 H) 4.91 (d, J=4.40 Hz, 2 H) 4.14 - 4.19 (m, 1 H) 2.36 (s, 3 H) 1.72 (d, J=6.36 Hz, 4 H).

### Example 74: (S)-N-(5-Chloro-3-methyl-1H-pyrazol-4-yl)-4-(5-chloro-6-(hydroxymethyl)pyridin-2-yl)-5-fluoro-2-((1,1,1-trifluoropropan-2-yl)oxy)benzamide.

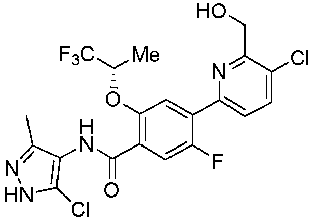

The title compound was prepared according to the representative procedure of Example 31, Step A-B, except substituting (6-bromo-3-chloropyridin-2-yl)methanol (Example 73, Step A) for 2-bromo-5-methylpyridine and 3-chloro-5-methyl-1H-pyrazol-4-amine for 2-chloro-4-methylpyridin-3-amine. MS (ESI): mass calcd. for C₂₀H₁₆Cl₂F₄N₄O₃, 506.1; m/z found, 507.0 [M+H]⁺. ¹H NMR (400 MHz, CDCl₃) δ = 9.72 (br d, J=3.42 Hz, 1 H) 8.80 (s, 1 H) 8.12 (d, J=11.74 Hz, 1 H) 7.78 - 7.92 (m, 2 H) 7.74 (d, J=5.87 Hz, 1 H) 4.96 - 5.14 (m, 1 H) 4.91 (d, J=4.89 Hz, 2 H) 4.18 (t, J=4.65 Hz, 1 H) 2.33 (s, 3 H) 1.70 (d, J=6.36 Hz, 4 H).

### Example 75: (S)-4-(5-Chloro-6-(hydroxymethyl)pyridin-2-yl)-N-(3-cyclopropyl-1H-pyrazol-4-yl)-5-fluoro-2-((1,1,1 -trifluoropropan-2-yl)oxy)benzamide.

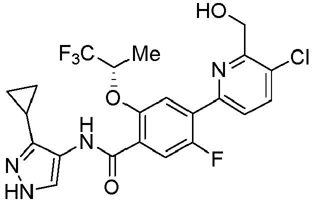

The title compound was prepared according to the representative procedure of Example 31, Step A-B, except substituting (6-bromo-3-chloropyridin-2-yl)methanol (Example 73, Step A) for 2-bromo-5-methylpyridine and 3-cyclopropyl-1H-pyrazol-4-amine for 2-chloro-4-methylpyridin-3-amine. MS (ESI): mass calcd. for C₂₂H₁₉ClF₄N₄O₃, 498.1; m/z found, 499.0 [M+H]⁺. ¹H NMR (400 MHz, CDCl₃) δ = 9.31 (s, 1 H) 8.26 (s, 1 H) 8.12 (d, J=11.74 Hz, 1 H) 7.85 (d, J=6.36 Hz, 2 H) 7.76 (d, J=5.87 Hz, 1 H) 4.94 - 5.04 (m, 1 H) 4.91 (s, 2 H) 4.10 - 4.28 (m, 1 H) 1.67 (d, J=6.36 Hz, 5 H) 0.98 (br d, J=7.83 Hz, 2 H) 0.89 (br d, J=4.89 Hz, 2 H).

### Example 76: (S)-4-(5-Chloro-6-(hydroxymethyl)pyridin-2-yl)-5-fluoro-N-(2-methoxy-3,5-dimethylpyridin-4-yl)-2-((1,1,1-trifluoropropan-2-yl)oxy)benzamide.

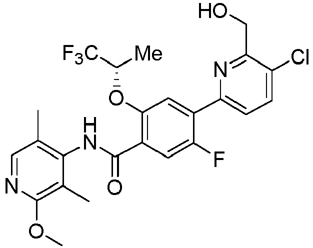

Step A. (S)-4-Bromo-5-fluoro-2-((1,1,1-trifluoropropan-2-yl)oxy)benzonitrile. To a solution of 4-bromo-2,5-difluorobenzonitrile (20 g, 91.7 mmol) and K₂CO₃ (36.8 g, 266.1 mmol) in DMF (240 ml) was added dropwise (S)-1,1,1-trifluoro-2-propanol (9.1 ml, 100.9 mmol) in DMF (10 ml) at 0 °C. The mixture was stirred at rt for 15 hours. The reaction mixture was poured into H₂O (300 ml). The aqueous phase was extracted with ethyl acetate (2x 100 ml). The combined organic phase was washed with brine, dried over MgSO₄, filtered and concentrated under reduced pressure. The residue was purified by flash column chromatography (SiO₂, heptane/ethyl acetate=100/0 to 70/30) to give the title compound as a white solid. MS (ESI): mass calcd for C₁₀H₆BrF₄NO, 216.9; m/z found. ¹H NMR (400 MHz, DMSO-*d₆*) δ = 7.34 - 7.41 (m, 1 H) 7.24 - 7.30 (m, 1 H) 4.60 - 4.72 (m, 1 H) 1.61 (d, J=6.36 Hz, 3 H.

Step B. (S)-4-Bromo-5-fluoro-2-((1,1,1-trifluoropropan-2-yl)oxy)benzoic acid. To a solution of (S)-4-bromo-5-fluoro-2-((1,1,1-trifluoropropan-2-yl)oxy)benzonitrile (20.1 g, 63.8 mmol) in EtOH (100 ml) was added dropwise a solution of NaOH (9.45g, 236.2 mmol) in water (150 ml) at 0 °C. The mixture was heated at 90 °C for 16 h. The reaction mixture was quenched by addition a solution of HCl 2N (100 mL×2) at 0 °C, and then extracted with DCM. The combined organic layers were washed with brine, dried over MgSO₄, filtered and concentrated under reduced pressure. The residue was purified by flash column chromatography (SiO₂, heptane/ethyl acetate=100/0 to 40/60) to give the title compound as a white solid. MS (ESI): mass calcd for C₁₀H₇BrF₄NO₃, 230.0; m/z found, 230.9 [M+H]⁺. ¹H NMR (400 MHz, DMSO-*d₆)* δ = 7.81 - 7.88 (m, 1 H) 7.29 (d, J=5.38 Hz, 1 H) 4.69 - 4.81 (m, 1 H) 1.61 (d, J=6.36 Hz, 3 H).

Step C. (S)-5-Fluoro-4-(4,4,5,5-tetramethyl-1,3,2-dioxaborolan-2-yl)-2-((1,1,1-trifluoropropan-2-yl)oxy)benzoic acid. Solid Pd(dppf)Cl₂ (299.3 mg, 0.362 mmol) was added to a solution of (S)-4-bromo-5-fluoro-2-((1,1,1-trifluoropropan-2-yl)oxy)benzoic acid (2.4 g, 7.25 mmol), bis(pinacolato)diboron (2.76 g, 10.87 mmol), potassium acetate (2.13 g, 21.75 mmol) in 1,4-dioxane (50 mL). The mixture was degassed by bubbling nitrogen for 15 minutes, heated at 80 °C for 24 hours. The reaction mixture was allowed to cool to room temperature. Water and AcOEt were added. The organic layer was separated, was washed with saturated NaHCO₃, dried over MgSO₄, filtered and concentrated under reduced presssure. The crude product was purified by column chromatography (heptane/ ethyl acetate=100/0 to heptane/ ethyl acetate= 40/60) to give the title compound. MS (ESI): mass calcd for C₁₆H₁₉BF₄O₅, 378.13; m/z found, 297.1 [M+H₃O⁺ - pinacol]⁺. ¹H NMR (400 MHz, DMSO-*d₆*) δ = 7.75 (br d, J=8.80 Hz, 1 H) 7.37 (br d, J=3.42 Hz, 1 H) 4.74 - 5.01 (m, 1 H) 1.60 (br d, J=6.36 Hz, 3 H) 1.38 (s, 13 H).

Step D. (S)-5-Fluoro-4-(4,4,5,5-tetramethyl-1,3,2-dioxaborolan-2-yl)-2-((1,1,1-trifluoropropan-2-yl)oxv)benzoyl chloride. A solution of (S)-5-fluoro-4-(4,4,5,5-tetramethyl-1,3,2-dioxaborolan-2-yl)-2-((1,1,1-trifluoropropan-2-yl)oxy)benzoic acid (610 mg, 1.613 mmol) in SOCl₂ (1.5 mL) was stirred and refluxed at 80 °C for 6 hours. The mixture was cooled down to room temperature and concentrated under vacuum. The residue as colorless oil, was used as such for next step without purification.

Step E. (S)-(2-Fluoro-4-((2-methoxy-3,5-dimethylpyridin-4-yl)carbamoyl)-5-((1,1,1-trifluoropropan-2-yl)oxy)phenyl)boronic acid. A solution of (S)-5-fluoro-4-(4,4,5,5-tetramethyl-1,3,2-dioxaborolan-2-yl)-2-((1,1,1-trifluoropropan-2-yl)oxy)benzoyl chloride (639 mg, 1.611 mmol), 2-methoxy-3,5-dimethyl-pyridin-4-amine (294 mg, 1.934 mmol), Et₃N (336 µL, 2.417 mmol) in DCM (2 mL) was heated at 60 °C for 1 h 30 min. The reaction mixture was allowed to cool to room temperature. Water and DCM were added. The organic layer was separated, washed with saturated NaHCO₃, dried over MgSO₄, filtered and concentrated under reduced pressure. The crude product was purified by column chromatographye (Heptane/Ethyl acetate=100/0 to Heptane/Ethyl acetate= 0/100). to give the title compound. MS (ESI): mass calcd for C₁₈H₁₉BF₄N₂O₅, 430.1; m/z found, 431.1 [M+H]⁺. ¹H NMR (400 MHz, DMSO-*d₆*) δ = 7.84 - 7.94 (m, 1 H) 7.57 (s, 1 H) 4.86 - 5.11 (m, 1 H) 4.11 (br s, 2 H) 3.96 (s, 2 H) 3.91 (s, 3 H) 2.15 (s, 3 H) 2.11 (s, 2 H) 2.05 (s, 3 H) 2.01 (s, 3 H) 1.81 (br dd, J=15.90, 12.47 Hz, 3 H) 1.64 (br d, J=6.36 Hz, 2 H).

Step F. (S)-4-(5-Chloro-6-(hydroxymethyl)pyridin-2-yl)-5-fluoro-N-(2-methoxy-3,5-dimethylpyridin-4-yl)-2-((1,1,1-trifluoropropan-2-yl)oxy)benzamide. The title compound was prepared in a manner analogous to Example 1, Step E however using (6-bromo-3-chloropyridin-2-yl)methanol (Example 73, Step A) as the aryl halide and (S)-(2-fluoro-4-((2-methoxy-3,5-dimethylpyridin-4-yl)carbamoyl)-5-((1,1,1-trifluoropropan-2-yl)oxy)phenyl)boronic acid as the boronate. MS (ESI): mass calcd for C₂₄H₂₂ClF₄N₃O₄, 527.1; m/z found, 528.2 [M+H]⁺. ¹H NMR (400MHz, CDCl₃) δ = 8.95 (s, 1 H) 8.16 (d, J=11.74 Hz, 1 H) 7.92 (s, 1 H) 7.86 (d, J=5.87 Hz, 2 H) 7.74 (d, J=5.38 Hz, 1 H) 4.98 - 5.13 (m, 1 H) 4.91 (d, J=4.40 Hz, 2 H) 4.18 (t, J=4.65 Hz, 1 H) 3.97 (s, 3 H) 2.17 (s, 3 H) 2.10 - 2.15 (m, 3 H) 1.68 (d, J=6.36 Hz, 3 H).

### Example 77: (S)-4-(5-Chloro-6-(hydroxymethyl)pyridin-2-yl)-5-fluoro-N-(o-tolyl)-2-((1,1,1-trifluoropropan-2-vl)oxy)benzamide.

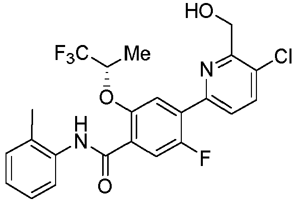

Step A. (S)-5-Fluoro-4-(4,4,5,5-tetramethyl-1,3,2-dioxaborolan-2-yl)-N-(o-tolyl)-2-((1,1,1-trifluoropropan-2-vl)oxv)benzamide. A mixture of (S)-5-fluoro-4-(4,4,5,5-tetramethyl-1,3,2-dioxaborolan-2-yl)-2-((1,1,1-trifluoropropan-2-yl)oxy)benzoic acid (Example 76, Step C, 1.06 g, 2.803 mmol), o-toluidine (358 µL, 3.364 mmol), HATU (1.12 g, 3.084 mmol), Et₃N (1.17 mL, 8.41 mmol) in DCM (6 mL) was stirred at room temperature for 4 hours. Water was added and the reaction mixture was extracted with DCM. The organic layer was decanted, washed with water, dried over MgSO₄, filtered and concentrated under reduced pressure. The crude product was purified by preparative (Heptane/Ethyl acetate=80/20 to Heptane/Ethyl acetate= 50/50) to give the title compound. MS (ESI): mass calcd. for C₂₃H₂₆BF₄NO₄, 467.2; m/z found, 386.0 [M+H₃0⁺ - pinacol]⁺. ¹H NMR (400 MHz, CDCl₃) δ = 8.92 - 9.20 (m, 1 H) 7.92 (s, 2 H) 7.36 (d, J=3.91 Hz, 1 H) 7.22 - 7.26 (m, 2 H) 7.13 (s, 1 H) 4.81 - 5.06 (m, 1 H) 2.31 (s, 3 H) 1.60 (d, J=6.36 Hz, 3 H) 1.39 (s, 12 H).

Step B. (S)-4-(5-Chloro-6-(hydroxymethyl)pyridin-2-yl)-5-fluoro-N-(o-tolyl)-2-((1,1,1-trifluoropropan-2-vl)oxy)benzamide. The title compound was prepared in a manner analogous to Example 1, Step E however using (6-bromo-3-chloropyridin-2-yl)methanol (Example 73, Step A) as the aryl halide and (S)-5-fluoro-4-(4,4,5,5-tetramethyl-1,3,2-dioxaborolan-2-yl)-N-(o-tolyl)-2-((1,1,1-trifluoropropan-2-yl)oxy)benzamide as the boronate. MS (ESI): mass calcd. for C₂₃H₁₉ClF₄N₂O₃, 482.1; m/z found, 483.1 [M+H]⁺. ¹H NMR (400 MHz, CDCl₃) δ = 9.11 (s, 1 H) 8.13 (d, J=11.74 Hz, 1 H) 7.92 (d, J=7.83 Hz, 1 H) 7.85 (d, J=6.85 Hz, 2 H) 7.76 (d, J=5.87 Hz, 1 H) 7.27 - 7.31 (m, 1 H) 7.25 (br s, 1 H) 7.13 - 7.19 (m, 1 H) 4.98 (dt, J=12.47, 5.99 Hz, 1 H) 4.91 (d, J=4.40 Hz, 2 H) 4.19 (t, J=4.65 Hz, 1 H) 2.33 (s, 3 H) 1.66 (d, J=6.36 Hz, 3 H).

### Example 78: (S)-4-(5-Chloro-6-(2-hydroxypropan-2-yl)pyridin-2-yl)-5-fluoro-N-(o-tolyl)-2-((1,1,1 -trifluoropropan-2-vl)oxy)benzamide.

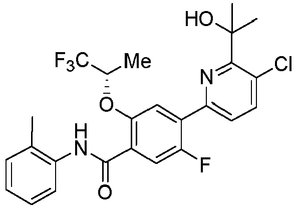

Step A: 2-(6-Bromo-3-chloropyridin-2-yl)propan-2-ol. Methylmagnesium bromide 3M in E₂tO (665 µL, 2 mmol) was added dropwise to a solution of methyl 6-bromo-3-chloropicolinate (200 mg, 0.8 mmol) in THF (6 mL) under nitrogen at -78°C. The reaction mixture was stirred for 1 hour at -78 °C then stirred at room temperature over the weekend. The reaction mixture was carefully diluted with EtOAc and NH₄Cl solution in water and ice. The layers were separated, and the water layer was extracted one more time with EtOAc. The combined organic layers were once washed with brine, dried over MgSO₄, filtered and concentrated under reduced pressure. The crude product was purified by column chromatography (Heptane/Ethyl acetate=80/20 to Heptane/Ethyl acetate= 50/50) to give the title compound. MS (ESI): mass calcd. for C₈H₉BrClNO, 248.9; m/z found, 249.9 [M+H]⁺. ¹H NMR (400 MHz, CDCl₃) δ = 7.57 (d, J=8.31 Hz, 1 H) 7.38 (d, J=8.31 Hz, 1 H) 1.68 (s, 5 H) 1.60 (s, 2 H).

Step B: (S)-4-(5-Chloro-6-(2-hydroxypropan-2-yl)pyridin-2-yl)-5-fluoro-N-(o-tolyl)-2-((1,1,1-trifluoropropan-2-yl)oxy)benzamide. The title compound was prepared in a manner analogous to Example 1, Step E however using 2-(6-bromo-3-chloropyridin-2-yl)propan-2-ol as the aryl halide and (S)-5-fluoro-4-(4,4,5,5-tetramethyl-1,3,2-dioxaborolan-2-yl)-N-(o-tolyl)-2-((1,1,1-trifluoropropan-2-yl)oxy)benzamide (Example 77, Step A) as the boronate. MS (ESI): mass calcd. for C₂₅H₂₃ClF₄N₂O₃, 510.1; m/z found, 511.1 [M+H]⁺. ¹H NMR (400 MHz, CDCl₃) δ = 9.11 (s, 1 H) 8.13 (d, J=11.74 Hz, 1 H) 7.91 (d, J=7.83 Hz, 1 H) 7.87 (s, 2 H) 7.72 (d, J=5.87 Hz, 1 H) 7.28 (br s, 1 H) 7.25 (br s, 1 H) 7.16 (d, J=7.34 Hz, 1 H) 4.91 - 5.06 (m, 1 H) 2.33 (s, 3 H) 1.77 (s, 6 H) 1.66 (d, J=6.36 Hz, 3 H).

### Example 79: (S)-N-(5-Chloro-3-methyl-1H-pyrazol-4-yl)-5-fluoro-4-(5,6,7,8-tetrahydro-1,8-naphthyridin-2-yl)-2-((1,1,1-trifluoropropan-2-yl)oxy)benzamide.

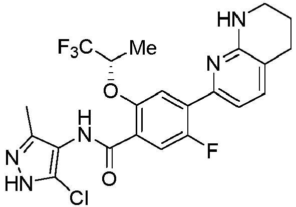

Step A. (S)-(4-((5-Chloro-3 -methyl-1 H-pyrazol-4-yl)carbamoyl)-2-fluoro-5 -((1,1,1-trifluoropropan-2-yl)oxy)phenyl)boronic acid. To a solution of (S)-5-fluoro-4-(4,4,5,5-tetramethyl-1,3,2-dioxaborolan-2-yl)-2-((1,1,1-trifluoropropan-2-yl)oxy)benzoic acid (Example 76, Step C, 1.46 g, 3.86 mmol) and TEA (805 µL, 5.8 mmol) in DMF (3.65 mL) cooled to 0 °C was added HATU (1.76 g, 4.63 mmol) and the reaction was stirred for 5 min at 0 °C. A solution of 5-chloro-3-methyl-4-amino-1H-pyrazole (Example 62, Step A, 559 mg, 4.25 mmol) in DMF (2 mL) was added and the reaction mixture was stirred at 0 °C for 1.5 hr. The reaction was concentrated and the residue was purified by prep-HPLC (METHOD D) to give the title compound. MS (ESI): mass calcd. for C₁₄H₁₃BClF₄N₃O₄, 409.0; m/z found, 410.0 [M+H]⁺. ¹H NMR (400MHz, CDCl₃) δ = 9.36 (s, 1 H) 7.41 (d, J=4.40 Hz, 1 H) 7.24 (d, J=8.80 Hz, 1 H) 5.10 - 5.34 (m, 1 H) 2.14 (s, 4 H) 1.45 (d, J=6.36 Hz, 4 H) 1.33 (s, 2 H).

Step B. tert-Butyl (S)-7-(4-((5-chloro-3-methyl-1H-pyrazol-4-yl)carbamoyl)-2-fluoro-5-((1,1,1-trifluoropropan-2-yl)oxy)phenyl)-3,4-dihydro-1,8-naphthyridine-1(2H)-carboxylate. The title compound was prepared in a manner analogous to Example 1, Step E however using tert-butyl 7-bromo-1,2,3,4-tetrahydro-1,8-naphthyridrine-1-carboxylate as the aryl halide and (S)-(4-((5-chloro-3-methyl-1H-pyrazol-4-yl)carbamoyl)-2-fluoro-5-((1,1,1 -trifluoropropan-2-yl)oxy)phenyl)boronic acid as the boronate. MS (ESI): mass calcd. for C₂₇H₂₈ClF₄N₅O₄, 597.1; m/z found, 598.2 [M+H]⁺.

Step C. (S)-N-(5-Chloro-3-methyl-1H-pyrazol-4-yl)-5-fluoro-4-(5,6,7,8-tetrahydro-1,8-naphthyridin-2-yl)-2-((1,1,1-trifluoropropan-2-yl)oxy)benzamide. TFA (800 µL) was added to a solution of tert-butyl (S)-7-(4-((5-chloro-3-methyl-1H-pyrazol-4-yl)carbamoyl)-2-fluoro-5-((1,1,1-trifluoropropan-2-yl)oxy)phenyl)-3,4-dihydro-1,8-naphthyridine-1(2H)-carboxylate (60 mg, 0.1 mmol) in DCM (800 µL). The reaction was stirred at RT for 4 hrs. The reaction was concentrated, dissolved in DCM (4 mL) and washed with 1N NaOH (4mL). The organic layers were dried over MgSO₄, filtered and concentrated under reduced pressire. The residue was purified by prep-HPLC (METHOD D) to give the title compound. MS (ESI): mass calcd. for C₂₂H₂₀ClF₄N₅O₂, 497.1; m/z found, 498.1 [M+H]⁺. ¹H NMR (400MHz, DMSO) δ = 12.80 - 13.14 (m, 1 H) 9.48 (s, 1 H) 7.68 (d, J=5.87 Hz, 1 H) 7.53 (br d, J=10.27 Hz, 1 H) 6.98 (br d, J=6.36 Hz, 1 H) 5.27 - 5.37 (m, 1 H) 2.78 (br d, J=5.38 Hz, 2 H) 2.15 (s, 3 H) 1.81 - 1.88 (m, 2 H) 1.48 (d, J=6.36 Hz, 3 H).

### Example 80: 4-(5-Chloro-6-(1-hydroxyethyl)pyridin-2-yl)-5-fluoro-N-(o-tolyl)-2-(((S)-1,1,1-trifluoropropan-2-yl)oxy)benzamide.

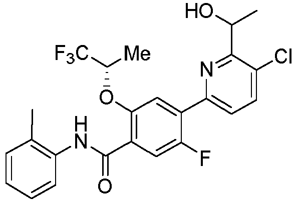

Step A: 1-(6-Bromo-3-chloropyridin-2-yl)ethan-1-ol. NaBH₄ (77.4 mg, 2.047 mmol) was added to a solution of 1-(6-bromo-3-chloropyridin-2-yl)ethan-1-one (240 mg, 1.024 mmol) in MeOH (5 mL) at 0 °C. The reaction mixture was stirred for 30 min at RT and was concentrated and the residue was taken up in DCM and washed with H₂O and brine, dried over MgSO₄, filtered and concentrated under reduced pressure to give the title compound. MS (ESI): mass calcd. for C₇H₇BrClNO, 234.9; m/z found, 235.9 [M+H]⁺. ¹H NMR (400 MHz, CDCl₃) δ = 7.53 (d, J=8.31 Hz, 1 H) 7.37 (d, J=8.31 Hz, 1 H) 5.05 - 5.19 (m, 1 H) 3.80 (d, J=8.80 Hz, 1 H) 1.47 (d, J=6.85 Hz, 4 H).

Step B: 4-(5-Chloro-6-(1-hydroxyethyl)pyridin-2-yl)-5-fluoro-N-(o-tolyl)-2-(((S)-1,1,1-trifluoropropan-2-vl)oxy)benzamide. The title compound was prepared in a manner analogous to Example 1, Step E however using 1-(6-bromo-3-chloropyridin-2-yl)ethan-1-ol as the aryl halide and (S)-5-fluoro-4-(4,4,5,5-tetramethyl-1,3,2-dioxaborolan-2-yl)-N-(o-tolyl)-2-((1,1,1-trifluoropropan-2-yl)oxy)benzamide (Example 77, Step A)as the boronate. MS (ESI): mass calcd. for C₂₄H₂₁ClF₄N₂O₃, 496.1; m/z found, 497.1 [M+H]⁺. ¹H NMR (400 MHz, CDCl₃) δ = 9.11 (br s, 1 H) 8.12 (d, J=11.74 Hz, 1 H) 7.92 (d, J=7.83 Hz, 1 H) 7.83 (s, 2 H) 7.75 (t, J=5.14 Hz, 1 H) 7.27 - 7.31 (m, 1 H) 7.12 - 7.20 (m, 1 H) 5.15 - 5.32 (m, 1 H) 4.97 (td, J=6.11, 2.93 Hz, 1 H) 4.32 (dd, J=7.83, 3.91 Hz, 1 H) 2.33 (s, 3 H) 1.66 (dd, J=6.36, 3.91 Hz, 3 H) 1.56 (d, J=6.36 Hz, 3 H).

### Example 81: (S)-4-(6-Acetamido-5-methylpyridin-2-yl)-N-(5-chloro-3-methyl-1H-pyrazol-4-yl)-5-fluoro-2-((1,1,1-trifluoropropan-2-vl)oxy)benzamide.

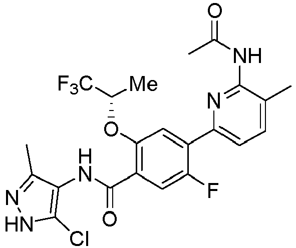

Step A: N-(6-Bromo-3-methylpyridin-2-yl)acetamide. To a solution of 6-bromo-3-methylpyridin-2-amine (120 mg, 0.642 mmol) in HOAc (900 µL) at 0° C was added Ac2O (64 µL). The solution was allowed to warm to rt and stirred at 45°C for 7h. The crude mixture was diluted with EtOAc and water, and the organic layer was washed with brine, dried over MgSO4, filtered and concentrated under reduced pressure in vacuo. The crude product was purified by column chromatography (Heptane/Ethyl acetate=100/0 to Heptane/Ethyl acetate= 40/60) to give the title compound. MS (ESI): mass calcd. for C₈H₉BrN₂O, 228.0; m/z found, 229.1 [M+H]⁺.

Step B: (S)-4-(6-Acetamido-5-methylpyridin-2-yl)-N-(5-chloro-3-methyl-1H-pyrazol-4-yl)-5-fluoro-2-(11,1,1-trifluoropropan-2-yl)oxy)benzamide. The title compound was prepared in a manner analogous to Example 1, Step E however using N-(6-bromo-3-methylpyridin-2-yl)acetamide as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate.. MS (ESI): mass calcd. for C₂₂H₂₀ClF₄N₅O₃, 513.1; m/z found, 514.1 [M+H]⁺. ¹H NMR (400 MHz, CDCl₃) δ = 8.82 (s, 1 H) 8.08 (d, J=11.74 Hz, 1 H) 7.77 (d, J=5.87 Hz, 1 H) 7.73 (d, J=1.47 Hz, 1 H) 7.65 - 7.69 (m, 1 H) 7.51 (d, J=5.87 Hz, 1 H) 4.98 - 5.08 (m, 1 H) 2.40 (s, 3 H) 2.35 (s, 3 H). 2.33 (s, 3 H) 1.69 (d, J=6.36 Hz, 4 H).

### Example 82: (S)-N-(5-Chloro-3-methyl-1H-pyrazol-4-yl)-5-fluoro-4-(6-hydroxypyridin-2-yl)-2-((1,1,1-trifluoropropan-2-yl)oxy)benzamide.

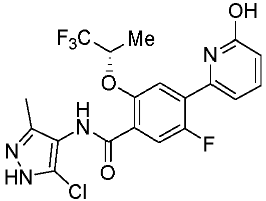

The title compound was prepared in a manner analogous to Example 1, Step E however using 2-bromo-6-hydroxypyridine as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅ClF₄N₄O₃, 458.1; m/z found, 459.0 [M+H]⁺. ¹H NMR (400 MHz, CDCl₃) δ = 8.86 (s, 1 H) 8.12 (d, J=11.25 Hz, 1 H) 7.64 (d, J=5.38 Hz, 1 H) 7.59 (dd, J=9.05, 7.09 Hz, 1 H) 6.84 (dd, J=7.34, 1.47 Hz, 1 H) 6.51 (d, J=9.29 Hz, 1 H) 5.75 (br d, J=5.87 Hz, 1 H) 2.34 (s, 3 H) 1.73 (d, J=6.36 Hz, 3 H).

### Example 83: (S)-4-(6-Amino-4-methylpyridin-2-yl)-N-(5-chloro-3-methyl-1H-pyrazol-4-yl)-5-fluoro-2-(1,1,1-trifluoropropan-2-vl)oxy)benzamide.

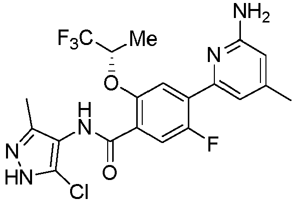

The title compound was prepared in a manner analogous to Example 1, Step E however using 6-chloro-4-methylpyridin-2-amine as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₈ClF₄N₅O₂, 471.1; m/z found, 472.0 [M+H]⁺. ¹H NMR (400 MHz, MeOH) δ = 7.69 (d, J=5.87 Hz, 1 H) 7.62 (d, J=10.76 Hz, 1 H) 6.95 (s, 1 H) 6.45 - 6.49 (m, 1 H) 5.26 (quin, J=6.36 Hz, 1 H) 2.29 (s, 3 H) 2.25 (s, 3 H) 1.60 (d, J=6.36 Hz, 3 H).

### Example 84: N-(5-Chloro-3-methyl-1H-pyrazol-4-yl)-5-fluoro-4-(6-(1-hydroxyethyl)-5-methylpyrazin-2-yl)-2-(((S)-1,1,1-trifluoropropan-2-vl)oxy)benzamide.

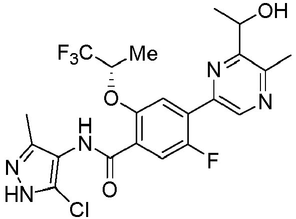

Step A. 6-Chloro-3-methylpyrazine-2-carbaldehyde. MnO₂ (2.3 g, 26.48 mmol) was added to a solution of (6-chloro-3-methylpyrazin-2-yl)methanol (Example 101, Step B, 420 mg, 2.65 mmol) in THF (15 mL). The reaction mixture was stirred at 50 °C overnight. The mixture was filtered over a pad of Celite^{®}. The solid was rinsed with DCM (10 mL), the filtrate was concentrated under reduced pressure to give the title compound (430 mg crude, 2.75 mmol) as pale yellow oil. ¹H NMR (400MHz, CDCl₃) δ = 10.15 (s, 1H), 8.68 (s, 1H), 2.90 (s, 3H).

Step B. 1-(6-Chloro-3-methylpyrazin-2-yl)ethanol. To a solution of 6-chloro-3-methylpyrazine-2-carbaldehyde (430 mg crude, 2.75 mmol) in THF (21.5 mL) was added MeMgBr (3 M solution in ether, 0.92 mL, 2.76 mmol) dropwise at 0 °C under N₂. The resulting mixture was stirred at 0 °C for 1 hour. The mixture was quenched with sat. aq. NH₄Cl (3 mL). THF was evaporated under vacuum. The aqueous layer was extracted with DCM (5 mL × 3). The combined organic layers were dried over Na₂SO₄, filtered and concentrated under reduced pressure. The crude product was purified by flash column chromatography (SiO₂, gradient elution: 0 - 50% petroleum ether in ethyl acetate) to give the title compound as a pale yellow oil. MS (ESI): mass calcd. for C₇H₉ClN₂O, 172.0; m/z found, 173.0 [M+H]⁺. ¹H NMR (400MHz, CDCl₃) δ = 8.44 (s, 1H), 5.07 - 4.98 (m, 1H), 3.56 (d, J=8.5 Hz, 1H), 2.58 (s, 3H), 1.49 (d, J=6.5 Hz, 3H).

Step C. N-(5-Chloro-3-methyl-1H-pyrazol-4-yl)-5-fluoro-4-(6-(1-hydroxyethyl)-5-methylpyrazin-2-yl)-2-(((S)-1,1,1-trifluoropropan-2-yl)oxy)benzamide. Thetitlecompoundwas prepared in a manner analogous to Example 1, Step E however using 1-(6-chloro-3-methylpyrazin-2-yl)ethan-1-ol as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₁H₂₀ClF₄N₅O₃, 501.1; m/z found, 502.2 [M+H]⁺. ¹H NMR (400 MHz, CDCl₃) δ = 9.63 - 9.96 (m, 1 H) 9.03 (d, J=2.45 Hz, 1 H) 8.79 (d, J=1.96 Hz, 1 H) 8.14 (d, J=11.25 Hz, 1 H) 7.72 (dd, J=5.62, 2.20 Hz, 1 H) 5.10 - 5.28 (m, 1 H) 4.82 - 5.03 (m, 1 H) 2.67 (s, 3 H) 2.34 (s, 3 H) 1.71 (dd, J=6.36, 2.93 Hz, 3 H) 1.55 (s, 2 H).

### Example 85, (S)-N-(5-Chloro-3-methyl-1H-pyrazol-4-yl)-5-fluoro-4-(5-methyl-6-(methylsulfonamido)pyridin-2-yl)-2-((1,1,1-trifluoropropan-2-yl)oxy)benzamide.

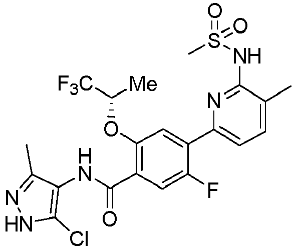

Step A. N-(6-Bromo-3-methylpyridin-2-yl)methanesulfonamide. A solution of mesyl chloride (25 µL, 0.321 mmol) in DCM (1.2 mL) was added to a solution of 6-bromo-3-methylpyridin-2-amine (120 mg, 0.642 mmol) in DCM (2.4 mL) and pyridine (2.4 mL) at 0 °C. The areaction mixture was stirred for 30 min at 0 °C, then heated at 40 °C for 3 hours and mesyl chloride (12 µL) was added. The reaction mixture was heated at 40°C for 16h. The reaction mixture was diluted with EtOAc and water, and the organic layer was washed with brine, dried over MgSO4, filtered and concentrated under reduced pressure. The crude product was purified by column chromatography (Heptane/Ethyl acetate=100/0 to Heptane/Ethyl acetate= 40/60) to give the title compound. MS (ESI): mass calcd. for C₇H₉BrN₂O₂S, 263.9; m/z found, 264.9 [M+H]⁺.

Step B. (S)-N-(5-Chloro-3-methyl-1H-pyrazol-4-yl)-5-fluoro-4-(5-methyl-6-(methylsulfonamido)pyridin-2-yl)-2-(1,1,1-trifluoropropan-2-yl)oxy)benzamide. The title compound was prepared in a manner analogous to Example 1, Step E however using N-(6-bromo-3-methylpyridin-2-yl)methanesulfonamide the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₁H₂₀ClF₄N₅O₄S, 549.1; m/z found, 550.0 [M+H]⁺. ¹H NMR (400 MHz, DMSO) δ = 12.86 (s, 1 H) 10.09 (br s, 1 H) 9.48 (s, 1 H) 7.79 - 7.85 (m, 1 H) 7.67 - 7.75 (m, 1 H) 7.51 - 7.54 (m, 1 H) 7.50 (s, 1 H) 5.17 - 5.26 (m, 1 H) 3.41 (s, 5 H) 2.27 (s, 3 H) 2.16 (s, 3 H) 1.51 (d, J=6.36 Hz, 3 H).

### Example 86: (S)-4-(6-Amino-5-fluoropyridin-2-yl)-N-(5-chloro-3-methyl-1H-pyrazol-4-yl)-5-fluoro-2-((1,1,1 -trifluoropropan-2-yl)oxy)benzamide.

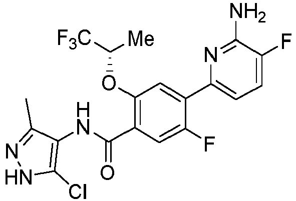

The title compound was prepared in a manner analogous to Example 1, Step E however using 6-bromo-3-fluoropyridin-2-amine as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅ClF₅N₅O₂, 475.1; m/z found, 476.1 [M+H]⁺. ¹H NMR (400 MHz, DMSO) δ = 9.53 (s, 1 H) 8.57 (br d, J=0.98 Hz, 1 H) 7.73 (d, J=5.87 Hz, 1 H) 7.57 (d, J=10.27 Hz, 1 H) 7.22 (d, J=7.34 Hz, 1 H) 5.28 - 5.42 (m, 1 H) 2.16 (s, 3 H) 1.48 (d, J=6.36 Hz, 3 H).

### Example 87: (S)-N-(5-Chloro-3-methyl-1H-pyrazol-4-yl)-5-fluoro-4-(5-(hydroxymethyl)-1-methyl-1H-pyrazol-3 -yl) -2-((1,1,1 -trifluoropropan-2-vl)oxy)benzamide.

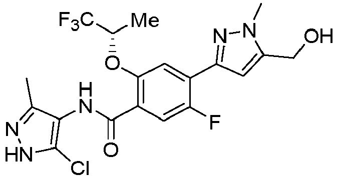

Step A. Methyl 3-bromo-1-methyl-1*H*-pyrazole-5-carboxylate. A mixture of methyl 3-bromo-1*H*-pyrazole-5-carboxylate (3.2 g, 15.6 mmol) and K₂CO₃ (4.3 g, 31.1 mmol) in acetone (45 mL) was stirred at room temperature for 15 minutes under N₂. Mel (2.7 g, 19.02 mmol) in acetone (2.7 mL) was added to reaction mixture. The mixture was stirred at room temperature for 12 hours. The mixture was filtered. The filter cake was washed with ethyl acetate (50 mL × 3). The filtrate was concentrated under vacuum. The crude product was purified by column chromatography (SiO₂, eluent: 0 - 2% ethyl acetate in petroleum ether) to give the title compound as colorless crystal. MS (ESI): mass calcd. for C₆H₇BrN₂O₂, 218.0; m/z found, 219.0 [M+H]⁺. ¹H NMR (400MHz, CDCl₃) δ = 6.82 (s, 1H), 4.16 (s, 3H), 3.90 (s, 3H).

Step B. (3-Bromo-1-methyl-1*H*-pyrazol-5-yl)methanol. NaBH₄ (111 mg, 2.93 mmol) and CaCl₂ (162 mg, 1.46 mmol) were added to the mixture of methyl 3-bromo-1-methyl-1*H*-pyrazole-5-carboxylate (534 mg, 2.44 mmol) in EtOH (8 mL) at 0 °C under N₂. The mixture was stirred at room temperature for 12 hours. EtOH was removed under vacuum. The residue was dissolved in sat. aq. NH₄Cl (60 mL). The mixture extracted with ethyl acetate (50 mL × 3). The organic layer was separated, dried over Na₂SO₄, filtered and concentrated under reduced pressure. The crude was purified by preparative reversed phase HPLC (Stationary phase: Boston Prime C18, 5 µm, 150 x 30 mm; Mobile phase: water (0.05%NH₃H₂O + 10mM NH₄HCO₃) (A) - MeCN (B), gradient elution: 20 - 50% B in A over 7 min, flow rate: 25 mL/min) to give the title compound as white powder. MS (ESI): mass calcd. for C₅H₇BrN₂O, 190.0; m/z found, 191.0, 192.9 [M+H]⁺.-¹H NMR (400MHz, DMSO-*d₆*) δ= 6.27 (s, 1H), 5.36 (t, *J=5.5* Hz, 1H), 4.46 (d, *J=5.5* Hz, 2H), 3.75 (s, 3H).

Step C. (S)-N-(5-Chloro-3-methyl-1H-pyrazol-4-yl)-5-fluoro-4-(5-(hydroxymethyl)-1-methyl-1H-pyrazol-3-yl)-2-((1,1,1-trifluoropropan-2-yl)oxy)benzamide. The title compound was prepared in a manner analogous to Example 1, Step E however using (3-bromo-1-methyl-1H-pyrazol-5-yl)methanol as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₈ClF₄N₅O₃, 475.1; m/z found, 476.1 [M+H]⁺. ¹H NMR (400 MHz, DMSO) δ = 9.11 - 9.53 (m, 1 H) 7.66 - 7.78 (m, 1 H) 7.47 (d, J=10.76 Hz, 1 H) 6.67 (d, J=4.40 Hz, 1 H) 5.30 - 5.47 (m, 2 H) 4.56 (s, 2 H) 3.89 (s, 3 H) 2.15 (s, 3 H) 1.47 (d, J=6.36 Hz, 3 H).

### Example 88: (S)-N-(5-Chloro-3-methyl-1H-pyrazol-4-yl)-5-fluoro-4-(3-oxo-3,4-dihydro-2H-pyrido [3 ,2-b][1,4] oxazin-6-yl)-2-((1,1,1 -trifluoropropan-2-vl)oxy)benzamide.

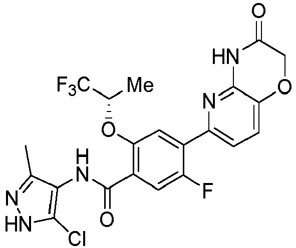

To a stirred solution of 6-bromo-2H-pyrido[3,2-b][1,4]oxazin-3(4H)-one (89 mg, 0.388 mmol), (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A, 173.7 mg, 0.42 mmol), Cs₂CO₃ (379.528 mg, 1.17 mmol) in 1,4-dioxane (3 mL) and water, distilled (690 µL) was purged with N₂, and XPhos Pd G3 (33 mg, 0.039 mmol) was added at RT. The reaction mixture was purged again with N₂ and stirred at 80 °C for 1 hour 30 min. The crude mixture was diluted with EtOAc and water, and the organic layer was washed with brine, dried over MgSO₄, filtered and concentrated under reduced pressure. The residue was purified by silica gel chromatography (Heptane/Ethyl acetate= 85-15 to Heptane/Ethyl acetate= 40-60) to give the title compound. MS (ESI): mass calcd. for C₂₁H₁₆ClF₄N₅O₄, 513.1; m/z found, 514.1 [M+H]⁺. ¹H NMR (400 MHz, DMSO) δ = 12.69 - 13.06 (m, 1 H) 11.10 - 11.62 (m, 1 H) 9.38 - 9.56 (m, 1 H) 7.64 - 7.81 (m, 1 H) 7.48 (s, 3 H) 5.76 (s, 2 H) 4.73 (s, 2 H) 2.15 (s, 3 H) 1.49 (d, J=6.36 Hz, 3 H).

### Example 89: (S)-N-(5-Chloro-3-methyl-1H-pyrazol-4-yl)-5-fluoro-4-(5-(2-hydroxypropan-2-yl)-1-methyl-1H-pyrazol-3 -yl)-2-((1,1,1 -trifluoropropan-2-vl)oxy)benzamide.

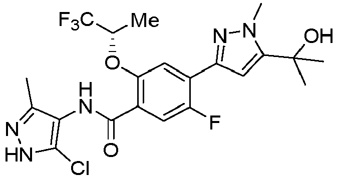

Step A. 2-(3-Bromo-1-methyl-1H-pyrazol-5-yl)propan-2-ol. To a mixture of methyl 3-bromo-1-methyl-1*H*-pyrazole-5-carboxylate (300 mg, 1.37 mmol) in THF (6 mL) was added methylmagnesium bromide (3 M solution in ether, 1.8 mL, 5.4 mmol) at 0 °C under N₂. The mixture was stirred at 0 °C for 1 hour. Sat. aq. NH₄Cl (15 mL) was added to the mixture dropwise at 0 °C under N₂. The mixture extracted with ethyl acetate (20 mL × 3). The organic layer was separated, dried over Na₂SO₄, filtered and concentrated under reduced pressure. The crude was purified by preparative reversed phase HPLC (Stationary phase: Boston Prime C18, 5 µm, 150 x 30 mm; Mobile phase: water (0.05%NH₃H₂O + 10mM NH₄HCO₃) (A) - MeCN (B), gradient elution: 25 - 55% B in A over 7 min, flow rate: 25 mL/min) to give the title compound as white powder. MS (ESI): mass calcd. for C₇H₁₁BrN₂O, 218.0; m/z found, 219.0, 221.0 [M+H]⁺.¹H NMR (400MHz, DMSO-*d₆*) δ= 6.19 (s, 1H), 5.39 (s, 1H), 3.92 (s, 3H), 1.46 (s, 6H).

Step B. (S)-N-(5-Chloro-3-methyl-1H-pyrazol-4-yl)-5-fluoro-4-(5-(2-hydroxypropan-2-yl)-1-methyl-1H-pyrazol-3-yl)-2-((1,1,1-trifluoropropan-2-yl)oxy)benzamide. The title compound was prepared in a manner analogous to Example 1, Step E however using 2-(3-bromo-1-methyl-1H-pyrazol-5-yl)propan-2-ol as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₁H₂₂ClF₄N₅O₃, 503.1; m/z found, 504.1 [M+H]⁺. ¹H NMR (400 MHz, DMSO) δ = 9.39 (s, 1 H) 7.71 (d, J=5.87 Hz, 1 H) 7.46 (d, J=10.76 Hz, 1 H) 6.56 (d, J=3.42 Hz, 1 H) 5.39 (br d, J=6.36 Hz, 1 H) 4.07 (s, 3 H) 2.15 (s, 3 H) 1.55 (s, 6 H) 1.47 (d, J=6.36 Hz, 3 H).

### Example 90: (S)-N-(5-Chloro-3-methyl-1H-pyrazol-4-yl)-5-fluoro-4-(5-fluoro-6-hydroxypyridin-2-yl)-2-((1,1,1 -trifluoropropan-2-vl)oxy)benzamide.

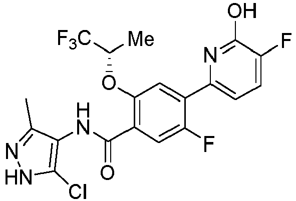

The title compound was prepared in a manner analogous to Example 88 however using 6-chloro-3-fluoropyridin-2-ol as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₄ClF₅N₄O₃, 476.1; m/z found, 477.1

[M+H]⁺. ¹H NMR (400 MHz, DMSO) δ = 12.86 (br s, 1 H) 9.48 (s, 1 H) 7.55 (br s, 1 H) 7.50 (s, 1 H) 7.48 (s, 1 H) 6.38 - 6.56 (m, 1 H) 5.36 - 5.47 (m, 1 H) 2.15 (s, 3 H) 1.50 (d, J=6.36 Hz, 3 H).

### Example 91: N-(5-Chloro-3-methyl-1H-pyrazol-4-yl)-5-fluoro-4-(5-fluoro-6-(2,2,2-trifluoro-1-hvdroxvethvl)pvridin-2-vl)-2-(((S)-1,1,1 -trifluoropropan-2-vl)oxv)benzamide.

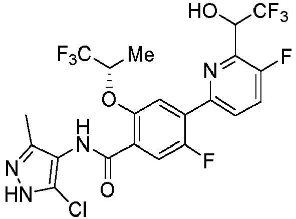

Step A. 1-(6-Chloro-3-fluoropyridin-2-yl)-2,2,2-trifluoroethan-1-ol. To a stirred solution of 6-chloro-3-fluoropicolinaldehyde (50 mg, 0.313 mmol) in THF (500 µL) was added cesium fluoride (43.312 mg, 0.313 mmol). Then the reaction mixture was cooled at 0 °C and (trifluoromethyl)trimethylsilane (55.587 µL, 0.376 mmol). The reaction mixture was stirred at room temperature overnight. A solution of 1N HCl was added and the organic layers were extracted with EtOAc. The combined organics were washed with brine, dried over MgSO₄, filtered and concentrated under reduced pressure to give the title compound. MS (ESI): mass calcd. for C₇H₄ClF₄NO, 229.0; m/z found, 230.0 [M+H]⁺.

Step B. N-(5-Chloro-3-methyl-1H-pyrazol-4-yl)-5-fluoro-4-(5-fluoro-6-(2,2,2-trifluoro-1-hydroxyethyl)pyridin-2-yl)-2-(((S)-1,1,1-trifluoropropan-2-yl)oxy)benzamide. The title compound was prepared in a manner analogous to Example 88 however using 1-(6-chloro-3-fluoropyridin-2-yl)-2,2,2-trifluoroethan-1-ol as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₁H₁₅ClF₈N₄O₃, 558.1; m/z found, 559.0 [M+H]⁺. ¹H NMR (400 MHz, DMSO) δ = 10.74 (br s, 1 H) 8.79 (s, 1 H) 8.12 (d, J=11.74 Hz, 1 H) 8.00 - 8.07 (m, 1 H) 7.59 - 7.71 (m, 2 H) 5.44 (br t, J=5.87 Hz, 1 H) 4.96 (dd, J=11.49, 6.11 Hz, 1 H) 4.70 (br d, J=8.80 Hz, 1 H) 2.32 (s, 3 H) 1.69 - 1.74 (m, 3 H).

### Example 92: (S)-N-(5-Chloro-3-methyl-1H-pyrazol-4-yl)-5-fluoro-4-(4-methyl-3-oxo-3,4-dihydro-2H-pyrido [3,2-b][1,41 oxazin-6-yl)-2-((1,1,1-trifluoropropan-2-vl)oxy)benzamide.

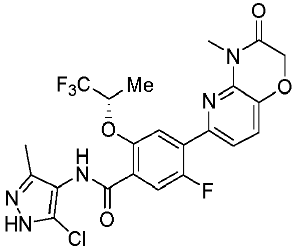

Step A. 6-Bromo-4-methyl-2H-pyrido[3,2-b][1,4]oxazin-3(4H)-one. Mel (41 µL, 0.655 mmol) was added dropwise to a stirred solution of 6-bromo-2H-pyrido[3,2-b][1,4]oxazin-3(4H)-one (100 mg, 0.437 mmol), K₂CO₃ (133 mg, 0.961 mmol) in DMF (1.5 mL) in a sealed tube and under N₂ at 0 °C. The mixture was stirred at rt for 16 h. The mixture was treated with water and extracted with AcOEt. The organic layer was separated, dried over MgSO₄, filtered and concentrated under reduced pressure to give the title compound as a white solid. MS (ESI): mass calcd. for C₈H₇BrN₂O₂, 242.0; m/z found, 243.0 [M+H]⁺. ¹H NMR (400 MHz, CDCl₃) δ = 7.26 (s, 1 H) 7.09 (d, J=3.42 Hz, 2 H) 4.68 (s, 2 H) 3.45 (s, 3 H).

Step B. (S)-N-(5-Chloro-3-methyl-1H-pyrazol-4-yl)-5-fluoro-4-(4-methyl-3-oxo-3,4-dihydro-2H-pyrido[3,2-b][1,4]oxazin-6-yl)-2-((1,1,1-trifluoropropan-2-yl)oxy)benzamide. The title compound was prepared in a manner analogous to Example 88 however using 6-bromo-4-methyl-2H-pyrido[3,2-b][1,4]oxazin-3(4H)-one as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₂H₁₈ClF₄N₅O₄, 527.1; m/z found, 528.2 [M+H]⁺. ¹H NMR (400 MHz, CDCl₃) δ = 9.48 - 9.96 (m, 1 H) 8.81 (s, 1 H) 8.08 (d, J=12.23 Hz, 1 H) 7.86 (d, J=5.87 Hz, 1 H) 7.67 (d, J=8.31 Hz, 1 H) 7.34 (d, J=8.31 Hz, 1 H) 4.90 - 5.03 (m, 1 H) 4.78 (s, 2 H) 3.57 (s, 3 H) 2.33 (s, 3 H) 1.72 (d, J=6.36 Hz, 3 H).

### Example 93: (S)-5-Fluoro-4-(4-methyl-3-oxo-3,4-dihydro-2H-pyrido[3,2-b][1,4]oxazin-6-yl)-N-(3-methyl-5-(trifluoromethyl)-1H-pyrazol-4-yl)-2-((1,1,1-trifluoropropan-2-yl)oxy)benzamide.

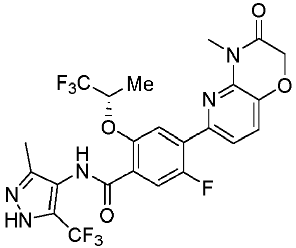

Step A. (*S*)-5-Fluoro-4-(4,4,5,5-tetramethyl-1,3,2-dioxaborolan-2-yl)-2-((1,1,1-trifluoropropan-2-yl)oxy)benzoic acid. To a mixture of (*S*)-4-bromo-5-fluoro-2-((1,1,1-trifluoropropan-2-yl)oxy)benzoic acid (9 g, 24.05 mmol) in dioxane (90 mL) was added bis(pinacolato)diboron (9.2 g, 36.07 mmol) and potassium acetate (7.1 g, 72.15 mmol), followed by the addition of Pd(dppf)Cl₂ (1.76 g, 2.41 mmol) under N₂. The resulting mixture was charged with N₂ and stirred at 80 °C under N₂ overnight. The mixture was cooled to room temperature. The mixture was filtered through silica gel twice and washed with DCM (500 mL). The filtrate was concentrated under reduced pressure to give the title compound (12.7 g crude) as brown gum. MS (ESI): mass calcd. for C₁₆H₁₉BF₄O₅, 378.1; m/z found, 297.0 [M+H]⁺.

Step B. (*S*)-(2-Fluoro-4-((5-methyl-3-(trifluoromethyl)-1*H*-pyrazol-4-yl)carbamoyl)-5-((1,1,1-trifluoropropan-2-yl)oxy)phenyl)boronic acid. To a mixture of (*S*)-5-fluoro-4-(4,4,5,5-tetramethyl-1,3,2-dioxaborolan-2-yl)-2- ((1,1,1-trifluoropropan-2-yl)oxy)benzoic acid (12.7 g crude) in DCM (100 mL) was added Et₃N (3.68 g, 36.33 mmol). The mixture was cooled to 0 °C. HATU (10.7 g, 28.10 mmol) was added in portions and the reaction was stirred at 0 °C for 2 hours. Then 5-methyl-3-(trifluoromethyl)- 1*H-*pyrazol-4-amine (3.52 g, 21.31 mmol) was added in portions. The reaction was stirred at 0 °C for 1 hour and then stirred at room temperature for 16 hours. The mixture was added water (120 mL) and extracted with DCM (200 mL × 3). The combined organic layers were dried over Na₂SO₄, filtered and concentrated under reduced pressure. The residue was purified by preparative reversed phase HPLC (Stationary phase: YMC-Triart Prep C18, 10 µm, 250 x 50 mm; Mobile phase: water (0.225% FA) (A) - MeCN (B), gradient elution: 10 - 50% B in A over 18 min, flow rate: 100 mL/min) to give the title compound as beige powder. MS (ESI): mass calcd. for C₁₅H₁₃BF₇N₃O₄, 443.1; m/z found, 444.0 [M+H]⁺. ¹H NMR (400MHz, DMSO) δ = 13.44 (br s, 1H), 9.49 (s, 1H), 8.46 (br s, 2H), 7.42 (d, J=4.2 Hz, 1H), 7.20 (d, J=8.3 Hz, 1H), 5.26 (td, J=6.4, 12.8 Hz, 1H), 2.17 (s, 3H), 1.44 (d, J=6.3 Hz, 3H); ¹⁹F NMR (376MHz, DMSO) δ = -59.83 - -61.03 (m, 1F), -76.89 - -78.09 (m, 1F), - 111.37 - -113.60 (m, 1F).

Step C. (S)-5-Fluoro-4-(4-methyl-3-oxo-3,4-dihydro-2H-pyrido[3,2-b][1,4]oxazin-6-yl)-N-(3-methyl-5-(trifluoromethyl)-1H-pyrazol-4-yl)-2-((1,1,1-trifluoropropan-2-yl)oxy)benzamide. The title compound was prepared in a manner analogous to Example 88 however using 6-bromo-4-methyl-2H-pyrido[3,2-b][1,4]oxazin-3(4H)-one (Example 92, Step A) and (*S*)-(2-fluoro-4-((5-methyl-3-(trifluoromethyl)-1*H*-pyrazol-4-yl)carbamoyl)-5-((1,1,1-trifluoropropan-2-yl)oxy)phenyl)boronic acid as the boronate. MS (ESI): mass calcd. for C₂₃H₁₈F₇N₅O₄, 561.1; m/z found, 562.2 [M+H]⁺. ¹H NMR (400 MHz, CDCl₃) δ = 10.55 (br s, 1 H) 8.94 (s, 1 H) 8.09 (d, J=12.23 Hz, 1 H) 7.86 (d, J=5.87 Hz, 1 H) 7.68 (d, J=8.31 Hz, 1 H) 7.34 (d, J=8.31 Hz, 1 H) 4.94 - 5.05 (m, 1 H) 4.78 (s, 2 H) 3.57 (s, 4 H) 2.29 (s, 4 H) 1.69 (d, J=6.85 Hz, 4 H).

### Example 94: (S)-5-Fluoro-N-(3-methyl-5-(trifluoromethyl)-1H-pyrazol-4-yl)-4-(3-oxo-3,4-dihydro-2H-pyrido[3,2-b][1,4]oxazin-6-yl)-2-((1,1,1-trifluoropropan-2-vl)oxy)benzamide.

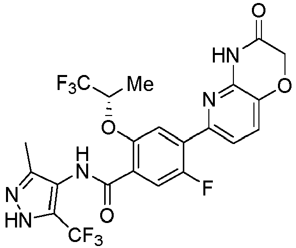

The title compound was prepared in a manner analogous to Example 88 however using 6-bromo-2H-pyrido[3,2-b][1,4]oxazin-3(4H)-one as the aryl halide and (S)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₂H₁₆F₇N₅O₄, 547.1; m/z found, 548.0 [M+H]⁺. ¹H NMR (400 MHz, DMSO) δ = 11.32 - 11.52 (m, 1 H) 9.55 (s, 1 H) 7.75 (d, J=5.87 Hz, 1 H) 7.38 - 7.57 (m, 3 H) 5.24 - 5.35 (m, 1 H) 4.73 (s, 2 H) 2.18 (s, 3 H) 1.48 (d, J=6.36 Hz, 3 H).

### Example 95: (S)-4-(6-Amino-5-chloropyridin-2-yl)-N-(5-chloro-3-methyl-1H-pyrazol-4-yl)-5-fluoro-2-(1,1,1-trifluoropropan-2-vl)oxy)benzamide.

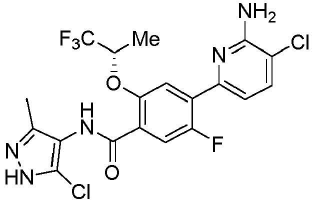

The title compound was prepared in a manner analogous to Example 88 however using 6-bromo-3-chloropyridin-2-amine as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅G₂F₄N₅O₂, 491.1; m/z found, 492.0 [M+H]⁺. ¹H NMR (400 MHz, CDCl₃) δ = 8.82 (s, 1 H) 8.05 (d, J=12.23 Hz, 1 H) 7.77 (d, J=5.87 Hz, 1 H) 7.61 (d, J=7.83 Hz, 1 H) 7.31 (dd, J=8.07, 1.71 Hz, 1 H) 5.03 (br d, J=2.93 Hz, 3 H) 2.31 (s, 3 H) 1.68 (d, J=6.85 Hz, 3 H) 1.60 (br s, 4 H).

### Example 96: (S)-4-(6-Amino-5-fluoropyridin-2-yl)-5-fluoro-N-(3-methyl-5-(trifluoromethyl)-1H-pyrazol-4-yl)-2-((1,1,1 -trifluoropropan-2-yl)oxy)benzamide.

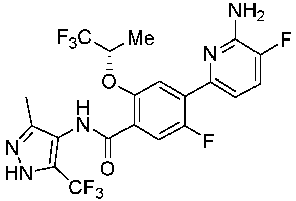

The title compound was prepared in a manner analogous to Example 88 however using 6-bromo-3-fluoropyridin-2-amine as the aryl halide and (S)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₀H₁₅F₈N₅O₂, 509.1; m/z found, 510.1 [M+H]⁺. ¹H NMR (400 MHz, DMSO) δ = 8.27 (d, J=3.42 Hz, 1 H) 7.76 (d, J=5.87 Hz, 1 H) 7.42 (d, J=10.76 Hz, 1 H) 7.22 (dd, J=7.58, 1.71 Hz, 1 H) 6.42 (s, 2 H) 5.31 - 5.48 (m, 1 H) 2.18 (s, 3 H) 1.44 (d, J=6.36 Hz, 3 H).

### Example 97: (S)-4-(6-Amino-5-chloropyridin-2-yl)-5-fluoro-N-(3-methyl-5-(trifluoromethyl)-1H-pyrazol-4-yl)-2-((1,1,1-trifluoropropan-2-vl)oxy)benzamide.

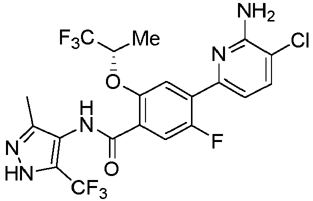

The title compound was prepared in a manner analogous to Example 88 however using 6-bromo-3-chloropyridin-2-amine as the aryl halide and (S)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₀H₁₅ClF₇N₅O₂, 525.1; m/z found, 526.0 [M+H]⁺. ¹H NMR (400 MHz, DMSO) δ = 13.38 - 13.51 (m, 1 H) 9.56 (s, 1 H) 7.75 - 7.78 (m, 1 H) 7.70 - 7.73 (m, 1 H) 7.42 (d, J=10.76 Hz, 1 H) 7.00 - 7.04 (m, 1 H) 6.50 (s, 2 H) 5.26 - 5.37 (m, 1 H) 2.18 (s, 3 H) 1.46 (d, J=6.36 Hz, 4 H).

### Example 98: 4-(5-Chloro-6-(1-hydroxyethyl)pyridin-2-yl)-5-fluoro-N-(3-methyl-5-(trifluoromethyl)-1H-pyrazol-4-yl)-2-(((S)-1,1,1-trifluoropropan-2-yl)oxy)benzamide.

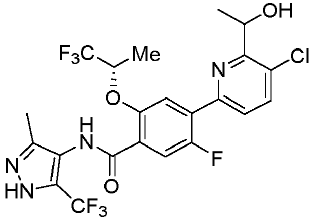

The title compound was prepared in a manner analogous to Example 88 however using 1-(6-bromo-3-chloropyridin-2-yl)ethan-1-ol (Example 80, Step A) as the aryl halide and (S)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₂H₁₈ClF₇N₄O₃, 554.1; m/z found, 555.2 [M+H]⁺. ¹H NMR (400 MHz, DMSO) δ = 9.59 (s, 1 H) 8.03 - 8.09 (m, 1 H) 7.94 (s, 1 H) 7.80 - 7.85 (m, 1 H) 7.44 - 7.50 (m, 1 H) 5.38 - 5.57 (m, 1 H) 5.13 - 5.28 (m, 2 H) 2.19 (s, 3 H) 1.48 (d, J=6.36 Hz, 6 H).

### Example 99: (S)-5-Fluoro-4-(5-fluoro-6-(hydroxymethyl)pyridin-2-yl)-N-(3-methyl-5-(trifluoromethyl)-1H-pyrazol-4-yl)-2-(1,1,1 -trifluoropropan-2-yl)oxv)benzamide.

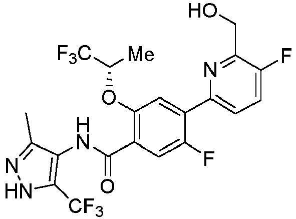

Step A. (6-Chloro-3-fluoropyridin-2-yl)methanol. NaBH₄ (949 mg, 25.07 mmol) was added to the mixture of 6-chloro-3-fluoropicolinaldehyde (1 g, 6.27 mmol) in EtOH (20 mL) at 0 °C under N₂. The mixture was stirred at room temperature overnight. The residue was dissolved in sat. aq. NH₄Cl (15 mL), then EtOH was evaporated under vacuum. The mixture was extracted with ethyl acetate (20 mL × 3). The organic layer was separated, dried over Na₂SO₄, filtered and concentrated. The crude product was purified by column chromatography (SiO₂, gradient elution: 0 - 30% ethyl acetate in petroleum ether). to give the title compound as white solid. MS (ESI): mass calcd. for C₆H₅ClFNO, 161.0; m/z found, 162.0 [M+H]⁺. ¹H NMR (400MHz, DMSO-*d₆*) δ = 7.82 (t, J=8.8 Hz, 1H), 7.54 (dd, J=3.3, 8.7 Hz, 1H), 5.48 (t, J=6.1 Hz, 1H), 4.54 (dd, *J*=2.1, 6.2 Hz, 2H); ¹⁹F NMR (376MHz, DMSO-*d₆*) δ= -129.32 (s, 1F).

Step B. (S)-5-Fluoro-4-(5-fluoro-6-(hvdroxvmethvl)pyridin-2-vl)-N-(3-methvl-5-(trifluoromethyl)-1H-pyrazol-4-yl)-2-((1,1,1-trifluoropropan-2-yl)oxy)benzamide. The title compound was prepared in a manner analogous to Example 88 however using (6-chloro-3-fluoropyridin-2-yl)methanol as the aryl halide and (S)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₁H₁₆F₈N₄O₃, 524.1; m/z found, 525.2 [M+H]⁺. ¹H NMR (400 MHz, DMSO) δ = 12.98 - 13.65 (m, 1 H) 9.58 (s, 1 H) 7.80 - 8.10 (m, 3 H) 7.47 (d, J=10.76 Hz, 1 H) 5.19 - 5.62 (m, 2 H) 4.34 - 4.85 (m, 2 H) 2.19 (s, 3 H) 1.46 (d, J=6.36 Hz, 4 H).

### Example 100: (S)-N-(5-Chloro-3-methyl-1H-pyrazol-4-yl)-5-fluoro-4-(5-fluoro-6-(hydroxymethyl)pyridin-2-yl)-2-((1,1,1-trifluoropropan-2-yl)oxy)benzamide.

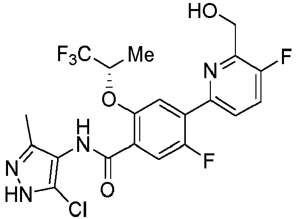

The title compound was prepared in a manner analogous to Example 88 however using (6-chloro-3-fluoropyridin-2-yl)methanol (Example 99, Step A) as the aryl halide and (S)-(4-((5-chloro-3-methyl-1H-pyrazol-4-yl)carbamoyl)-2-fluoro-5-((1,1,1 -trifluoropropan-2-yl)oxy)phenyl)boronic acid (Example 79, Step A) as the boronate. MS (ESI): mass calcd. for C₂₀H₁₆ClF₅N₄O₃, 490.1; m/z found, 491.1 [M+H]⁺. ¹H NMR (400 MHz, DMSO) δ = 12.70 - 13.15 (m, 1 H) 9.46 (s, 1 H) 7.78 - 7.97 (m, 3 H) 7.52 (d, J=10.76 Hz, 1 H) 5.32 - 5.52 (m, 2 H) 4.71 (dd, J=5.38, 1.47 Hz, 2 H) 2.16 (s, 3 H) 1.48 (d, J=6.36 Hz, 3 H).

### Example 101: (S)-5-Fluoro-4-(6-(hydroxymethyl)-5-methylpyrazin-2-yl)-N-(3-methyl-5-(trifluoromethyl)-1H-pyrazol-4-yl)-2-((1,1,1 -trifluoropropan-2-yl)oxv)benzamide.

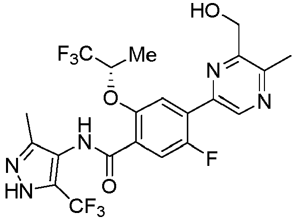

Step A. Methyl 6-chloro-3-methylpyrazine-2-carboxylate. Methyl 3-bromo-6-chloropyrazine-2-carboxylate (1 g, 3.98 mmol) was dissolved in DME (25 mL), then K₂CO₃ (1.1 g, 7.95 mmol) and trimethylboroxine (3.5 M in THF, 0.45 mL, 1.59 mmol) were added Pd(dppf)Cl₂.DCM (324 mg, 397.67 µmol) was added to the above mixture under N₂. The reaction mixture was stirred at 80 °C overnight and cooled to room temperature. Trimethylboroxine (3.5 M in THF, 0.23 mL, 795.33 µmol) was added. The reaction mixture was stirred at 80 °C overnight and cooled to room temperature. The mixture was filtered over Celite^{®}, the solid was rinsed with ethyl acetate (10 mL) and the filtrate was concentrated under reduced pressure. The crude product was purified by flash column chromatography (SiO₂, eluent: 0 - 10% ethyl acetate in petroleum ether) to give the title compound as colorless solid. MS (ESI): mass calcd. for C₇H₇ClN₂O₂, 186.0; m/z found, 187.1 [M+H]⁺.¹H NMR (400MHz, CDCl₃) δ = 8.61 (s, 1H), 3.99 (s, 3H), 2.81 (s, 3H).

Step B. (6-Chloro-3-methylpyrazin-2-yl)methanol. NaBH₄ (97 mg, 2.57 mmol) and CaCl₂ (143 mg, 1.29 mmol) were added to the mixture of methyl 6-chloro-3-methylpyrazine-2-carboxylate (400 mg, 2.14 mmol) in EtOH (25 mL) at 0 °C under N₂. The mixture was stirred at room temperature overnight. EtOH was removed under vacuum. The residue was dissolved in sat. aq. NH₄Cl (15 mL) and extracted with ethyl acetate (15 mL × 3). The organic layer was separated, dried over Na₂SO₄, filtered and concentrated under reduced pressure. The crude product was purified by flash column chromatography (SiO₂, eluent: 0 - 30% ethyl acetate in petroleum ether) to give the title compound as colorless oil. MS (ESI): mass calcd. for C₆H₇ClN₂O, 158.0; m/z found, 159.1 [M+H]⁺.¹H NMR (400MHz, CDCl₃) δ = 8.44 (s, 1H), 4.77 (d, *J=5.1* Hz, 2H), 3.53 (t, J=5.1 Hz, 1H), 2.51 (s, 3H).

Step C. (S)-5-Fluoro-4-(6-(hydroxymethyl)-5-methylpyrazin-2-yl)-N-(3-methyl-5-(trifluoromethyl)-1H-pyrazol-4-yl)-2-((1,1,1-trifluoropropan-2-yl)oxy)benzamide. The title compound was prepared in a manner analogous to Example 88 however using (6-chloro-3-methylpyrazin-2-yl)methanol as the aryl halide and (S)-(2-Fluoro-4-((5-methyl-3-(trifluoromethyl)-1H-pyrazol-4-yl)carbamoyl)-5-((1,1,1-trifluoropropan-2-yl)oxy)phenyl)boronic (Example 93, Step B) as the boronate. MS (ESI): mass calcd. for C₂₁H₁₈F₇N₅O₃, 521.1; m/z found, 522.2 [M+H]⁺. ¹H NMR (400 MHz, DMSO) δ = 9.41 - 9.69 (m, 1 H) 8.77 - 8.85 (m, 1 H) 8.87 (d, J=1.96 Hz, 1 H) 7.89 (d, J=5.87 Hz, 1 H) 7.50 (d, J=10.27 Hz, 1 H) 5.40 - 5.51 (m, 1 H) 5.36 (br s, 1 H) 4.75 (d, J=2.93 Hz, 2 H) 2.62 (s, 3 H) 2.19 (s, 3 H) 1.46 (d, J=6.36 Hz, 3 H).

### Example 102: (S)-N-(5-Chloro-3-methyl-1H-pyrazol-4-yl)-5-fluoro-4-(6-hydroxy-5-methilpyrazin-2-yl)-2-((1,1,1-trifluoropropan-2-yl)oxy)benzamide.

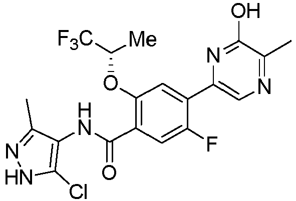

Step A. 3-(Benzyloxy)-5-chloro-2-methylpyrazine. To a solution of BnOH (1.99 g, 18.40 mmol) in THF (45 mL) was added NaH (60%, 675 mg, 16.87 mmol) at 0 °C under N₂, the reaction mixture was stirred at 0 °C for 30 minutes. A solution of 3,5-dichloro-2-methylpyrazine (2.5 g, 15.34 mmol) in THF (5 mL) was slowly added to the mixture at 0 °C. The reaction mixture was stirred at room temperature for 2 hours under N₂. The reaction mixture was diluted with ethyl acetate (50 mL), washed with brine (20 mL). The organic layer was dried with Na₂SO₄, filtered and concentrated under vacuum. The crude product was purified by flash column chromatography (SiO₂, gradient elution: 0 - 10% ethyl acetate in petroleum ether) to give the title compounds (3.5 g) containing inseparable undesired isomer: 3-(benzyloxy)-5-chloro-2-methylpyrazine. MS (ESI): mass calcd. for C₁₂H₁₁ClN₂O, 234.1; m/z found, 234.9 [M+H]⁺. ¹H NMR (400MHz, CDCl₃) δ = 8.06 (s, 1H), 7.50 - 7.39 (m, 5H), 5.47 - 5.36 (m, 2H), 2.50 (s, 3H).

Step B, 6-Chloro-3-methylpyrazin-2-ol. The mixture of 3-(benzyloxy)-5-chloro-2-methylpyrazine and 5-(benzyloxy)-3-chloro- 2-methylpyrazine (1.55 g, 5.17 mmol) was dissolved in toluene (4 mL), and the above solution was added to BCl₃ (1 M solution in toluene, 52 mL, 52 mmol) at -78 °C under N₂. The mixture was stirred at -78 °C for 1 hour, slowly warmed to room temperature and stirred at room temperature overnight. The mixture was quenched with MeOH (10 mL) dropwise at 0 °C and stirred at same temperature for 0.5 hour. The mixture was concentrated under reduced pressure. The crude product was purified by flash column chromatography (SiO₂, gradient elution: 0 - 5% MeOH in DCM) to give the region-isomers. The isomers were separated by preparative reversed phase HPLC (Stationary phase: Boston Green ODS, 5 µm, 150 x 30 mm; Mobile phase: H₂O (0.2% FA) (A) - MeCN (B), gradient elution: 15 - 45% B in A over 7 min, flow rate: 35 mL/min) to give the title compound as white powder. MS (ESI): mass calcd. for C₅H₅ClN₂O, 144.0; m/z found, 145.0 [M+H]⁺. ¹H NMR (400MHz, DMSO) δ = 12.48 (br s, 1H), 8.03 (s, 1H), 2.33 (s, 3H).

Step C. (S)-N-(5-Chloro-3-methyl-1H-pyrazol-4-yl)-5-fluoro-4-(6-hydroxy-5-methylpyrazin-2-yl)-2-(1,1,1-trifluoropropan-2-yl)oxy)benzamide. The title compound was prepared in a manner analogous to Example 88 however using 6-chloro-3-methylpyrazin-2-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₆ClF₄N₅O₃, 473.1; m/z found, 474.1 [M+H]⁺. ¹H NMR (400 MHz, DMSO) δ = 12.87 (br s, 1 H) 9.50 (s, 1 H) 7.60 - 7.68 (m, 1 H) 7.46 - 7.54 (m, 1 H) 5.23 - 5.59 (m, 1 H) 2.36 (s, 3 H) 2.15 (s, 3 H) 1.42 - 1.55 (m, 3 H).

### Example 103: (S)-N-(5-chloro-3-methyl-1H-pyrazol-4-yl)-5-fluoro-4-(5-fluoro-6-(hydroxymethyl)pyrazin-2-yl)-2-((1,1,1 -trifluoropropan-2-yl)oxy)benzamide.

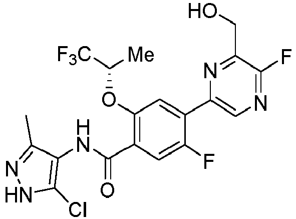

Step A. (3-Amino-6-chloropyrazin-2-yl)methanol. To a mixture of methyl 3-amino-6-chloropyrazine-2-carboxylate (3 g, 15.99 mmol) in THF (150 mL) was added DIBAL-H (1 M solution in toluene, 64 mL, 64 mmol) at -40 °C under N₂. The mixture was warmed to -10 °C and stirred for 1 hour. Sat. aq. Rochelle salt (150 mL) was added to the mixture drop wise under 0 °C. The mixture was stirred for 1 hour at room temperature. The mixture was extracted with ethyl acetate (100 mL × 3). The combined organic layers were washed with brine (400 mL), dried over Na₂SO₄, filtered and concentrated under reduced pressure to give the title compound as a yellow solid. ¹H NMR (400MHz, CDCl₃) δ = 7.98 (s, 1H), 5.17 - 4.84 (m, 2H), 4.72 (d, *J*=5.6 Hz, 2H), 2.71 (t, *J*=5.7 Hz, 1H).

Step B. (6-Chloro-3-fluoropyrazin-2-yl)methanol. Pyridine hydrofluoride (20 mL) was slowly added to (3-amino-6-chloropyrazin-2-yl)methanol (1 g, 6.27 mmol) at 0 °C under N₂. Then NaNO₂ (519 mg, 7.52 mmol) was added to the mixture in portions at 0 °C. The mixture was stirred and warmed to room temperature over a period of 3 hours. The mixture was adjust to pH = 7 with sat. aq. NaHCO₃. The mixture was extracted with ethyl acetate (40 mL × 3). The combined organic layers were washed with brine (100 mL), dried over Na₂SO₄, filtered and concentrated. The crude was purified by column chromatography (SiO₂, gradient elution: 0 - 30% ethyl acetate in petroleum ether) to give the title compound as yellow solid. MS (ESI): mass calcd. for C₅H₄ClFN₂O, 162.0; m/z found, 163.0 [M+H]⁺. ¹H NMR (400MHz, DMSO) δ = 8.43 (d, J=1.3 Hz, 1H), 5.66 (t, J=6.2 Hz, 1H), 4.60 (d, J=6.2 Hz, 2H); ¹⁹F NMR (376MHz, DMSO) δ = -84.24 (s, 1F).

Step C. (S)-N-(5-Chloro-3-methyl-1H-pyrazol-4-yl)-5-fluoro-4-(5-fluoro-6-(hydroxymethyl)pyrazin-2-yl)-2-((1,1,1-trifluoropropan-2-yl)oxy)benzamide. The title compound was prepared in a manner analogous to Example 88 however using (6-chloro-3-fluoropyrazin-2-yl)methanol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅ClF₅N₅O₃, 491.1; m/z found, 492.1 [M+H]⁺. ¹H NMR (400 MHz, DMSO) δ = 12.88 (br s, 1 H) 9.54 (s, 1 H) 8.70 (t, J=1.71 Hz, 1 H) 7.85 (d, J=5.87 Hz, 1 H) 7.58 (d, J=10.76 Hz, 1 H) 5.64 (br s, 1 H) 5.28 - 5.47 (m, 1 H) 4.76 (br d, J=3.91 Hz, 2 H) 2.16 (s, 3 H) 1.48 (d, J=6.36 Hz, 3 H).

### Example 104: (S)-4-(6-Amino-5-methoxypyridin-2-yl)-N-(5-chloro-3-methyl-1H-pyrazol-4-yl)-5-fluoro-2-((1,1,1 -trifluoropropan-2-vl)oxy)benzamide.

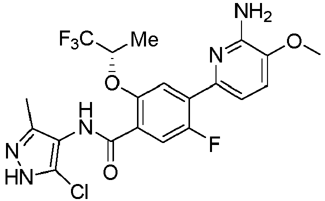

The title compound was prepared in a manner analogous to Example 88 however using 6-bromo-3-methoxypyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate.. MS (ESI): mass calcd. for C₂₀H₁₈ClF₄N₅O₃, 487.1; m/z found, 488.1 [M+H]⁺. ¹H NMR (400 MHz, CDCl₃) δ = 10.17 - 10.50 (m, 1 H) 8.84 (s, 1 H) 8.02 (d, J=12.23 Hz, 1 H) 7.80 (d, J=5.87 Hz, 1 H) 7.37 (dd, J=8.31, 1.47 Hz, 1 H) 7.00 (d, J=8.31 Hz, 1 H) 4.95 - 5.18 (m, 1 H) 4.84 (s, 2 H) 3.92 (s, 3 H) 1.66 (d, J=6.36 Hz, 6 H).

### Example 105: (S)-4-(6-Amino-3-fluoropyridin-2-yl)-N-(5-chloro-3-methyl-1H-pyrazol-4-yl)-5-fluoro-2-(1,1,1-trifluoropropan-2-yl)oxy)benzamide.

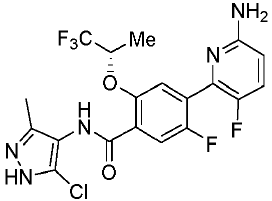

The title compound was prepared in a manner analogous to Example 88 however using 6-bromo-5-fluoropyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅ClF₅N₅O₂, 475.1; m/z found, 476.1 [M+H]⁺. ¹H NMR (400 MHz, CDCl₃) δ = 9.85 - 10.26 (m, 1 H) 8.78 (s, 1 H) 8.07 (d, J=10.27 Hz, 1 H) 7.35 (t, J=8.80 Hz, 1 H) 7.25 (d, J=5.38 Hz, 1 H) 6.59 (dd, J=9.29, 2.93 Hz, 1 H) 4.85 - 5.16 (m, 1 H) 4.51 (s, 2 H) 2.31 (s, 4 H) 1.67 (d, J=6.85 Hz, 4 H).

### Example 106: (S)-4-(6-Amino-4-methoxypyridin-2-yl)-N-(5-chloro-3-methyl-1H-pyrazol-4-yl)-5-fluoro-2-(1,1,1 -trifluoropropan-2-vl)oxy)benzamide.

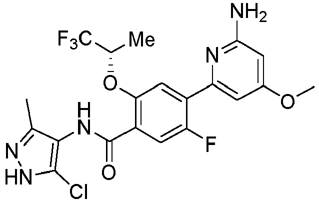

The title compound was prepared in a manner analogous to Example 88 however using 6-chloro-4-methoxypyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₈ClF₄N₅O₃, 487.1; m/z found, 488.1 [M+H]⁺. ¹H NMR (400 MHz, DMSO) δ = 12.86 (s, 1 H) 9.44 (s, 1 H) 7.72 (d, J=5.87 Hz, 1 H) 7.44 (d, J=10.27 Hz, 1 H) 6.56 (t, J=1.71 Hz, 1 H) 6.09 (s, 2 H) 6.05 (d, J=2.45 Hz, 1 H) 5.30 (t, J=6.36 Hz, 1 H) 3.78 (s, 3 H) 2.15 (s, 3 H) 1.47 (d, J=6.36 Hz, 3 H).

### Example 107: (S)-4-(8-Aminoimidazo[1,2-a]pyrazin-6-yl)-N-(5-chloro-3-methyl-1H-pyrazol-4-yl)-5-fluoro-2-((1,1,1-trifluoropropan-2-yl)oxy)benzamide.

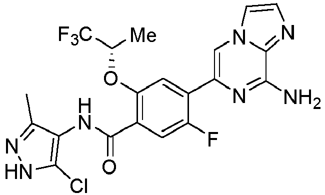

The title compound was prepared in a manner analogous to Example 88 however using 6-bromoimidazo[1,2-a]pyrazin-8-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₆ClF₄N₇O₂, 497.1; m/z found, 498.1 [M+H]⁺. ¹H NMR (400 MHz, DMSO) δ = 12.80 - 12.92 (m, 1 H) 9.45 (s, 1 H) 8.40 (s, 1 H) 8.03 (d, J=0.98 Hz, 1 H) 7.94 (d, J=6.36 Hz, 1 H) 7.58 (d, J=0.98 Hz, 1 H) 7.50 (d, J=11.25 Hz, 1 H) 7.16 (s, 2 H) 5.26 (s, 1 H) 2.16 (s, 3 H) 1.50 (d, J=6.36 Hz, 3 H).

### Example 108: (S)-4-(6-Amino-4-(trifluoromethyl)pyridin-2-yl)-N-(5-chloro-3-methyl-1H-pyrazol-4-yl)-5-fluoro-2-((1,1,1-trifluoropropan-2-yl)oxy)benzamide.

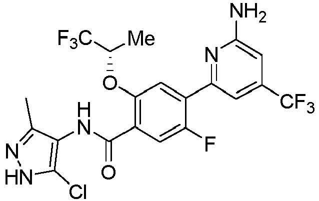

The title compound was prepared in a manner analogous to Example 88 however using substituting 6-chloro-4-(trifluoromethyl)pyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₅ClF₇N₅O₂, 525.1; m/z found, 526.0 [M+H]⁺. ¹H NMR (400 MHz, DMSO) δ = 12.86 (s, 1 H) 9.49 (s, 1 H) 7.74 (d, J=5.87 Hz, 1 H) 7.49 (d, J=10.76 Hz, 1 H) 7.12 (s, 1 H) 6.79 (d, J=4.89 Hz, 3 H) 5.24 - 5.42 (m, 1 H) 2.16 (s, 3 H) 1.47 (d, J=6.36 Hz, 3 H).

### Example 109: (S)-4-(6-Amino-5-(trifluoromethyl)pyridin-2-yl)-N-(5-chloro-3-methyl-1H-pyrazol-4-yl)-5-fluoro-2-((1,1,1-trifluoropropan-2-yl)oxy)benzamide.

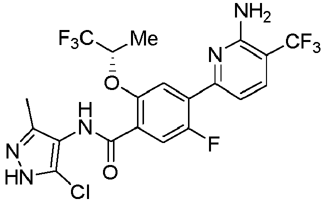

The title compound was prepared in a manner analogous to Example 88 however using 6-chloro-3-(trifluoromethyl)pyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₅ClF₇N₅O₂, 525.1; m/z found, 526.0 [M+H]⁺. ¹H NMR (400 MHz, DMSO) δ = 12.86 (s, 1 H) 9.49 (s, 1 H) 7.87 - 7.94 (m, 1 H) 7.79 (d, J=5.87 Hz, 1 H) 7.50 (d, J=10.27 Hz, 1 H) 7.07 - 7.15 (m, 1 H) 6.67 (s, 2 H) 5.23 - 5.39 (m, 1 H) 2.16 (s, 3 H) 1.48 (d, J=6.36 Hz, 3 H).

### Example 110: (S)-4-(6-Amino-4-chloropyridin-2-yl)-N-(5-chloro-3-methyl-1H-pyrazol-4-yl)-5-fluoro-2-(1,1,1-trifluoropropan-2-vl)oxy)benzamide.

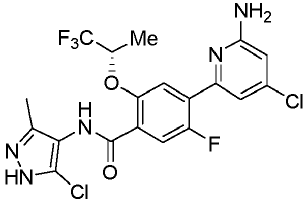

The title compound was prepared in a manner analogous to Example 88 however using 6-bromo-4-chloropyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅G₂F₄N₅O₂, 491.1; m/z found, 492.1 [M+H]⁺. ¹H NMR (400 MHz, DMSO) δ = 12.78 - 12.92 (m, 1 H) 9.47 (s, 1 H) 7.71 (d, J=5.87 Hz, 1 H) 7.46 (d, J=10.76 Hz, 1 H) 6.97 (t, J=1.47 Hz, 1 H) 6.56 - 6.61 (m, 1 H) 6.51 (s, 2 H) 5.28 - 5.43 (m, 1 H) 2.15 (s, 3 H) 1.47 (d, J=6.36 Hz, 3 H).

### Example 111: (S)-4-(6-Amino-3-chloropyridin-2-yl)-N-(5-chloro-3-methyl-1H-pyrazol-4-yl)-5-fluoro-2-(1,1,1-trifluoropropan-2-yl)oxy)benzamide.

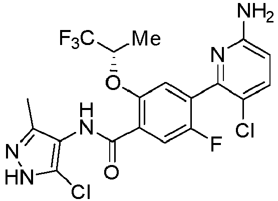

The title compound was prepared in a manner analogous to Example 88 however using 6-bromo-5-chloro-2-pyridinamine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₅G₂F₄N₅O₂, 491.1; m/z found, 492.1 [M+H]⁺. ¹H NMR (400 MHz, DMSO) δ = 12.58 - 13.08 (m, 1 H) 9.45 (t, J=8.07 Hz, 1 H) 7.52 - 7.60 (m, 1 H) 7.36 - 7.47 (m, 2 H) 6.51 - 6.59 (m, 1 H) 6.30 - 6.41 (m, 2 H) 5.29 - 5.43 (m, 1 H) 2.07 - 2.21 (m, 3 H) 1.36 - 1.50 (m, 3 H).

### Example 112: N-(5-Chloro-3-methyl-1H-pyrazol-4-yl)-5-fluoro-4-(6-(1-hydroxyethyl)-5-methoxypyridin-2-yl)-2-(((S)-1,1,1-trifluoropropan-2-vl)oxy)benzamide.

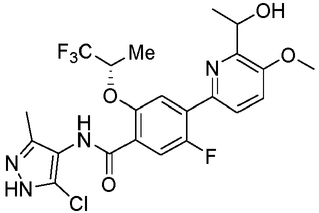

Step A. 1-(6-bromo-3-methoxypyridin-2-yl)ethan-1-ol. To a solution of 6-bromo-3-methoxypicolinaldehyde (30 mg, 0.174 mmol) in THF (700 µL) at -78 °C was added methylmagnesium bromide 3.0 M in diethyl ether (139 µL, 0.417 mmol). The reaction mixture was stirred at -78 °C for 1 hour. The mixture was warmed to room temperature and stirred for 1 hour then quenched with saturated aqueous NH₄Cl and, extracted with EtOAc. The organic layers were washed with brine, dried over MgSO₄, filtered and concentrated to give the title compound. MS (ESI): mass calcd. for C₈H₁₀BrNO₂, 231.0; m/z found, 232.0 [M+H]⁺. ¹H NMR (400 MHz, CDCl₃) δ = 7.34 (d, J=8.31 Hz, 1 H) 7.06 (d, J=8.31 Hz, 1 H) 4.91 - 5.09 (m, 1 H) 3.96 (d, J=7.83 Hz, 1 H) 3.85 (s, 3 H) 1.42 (d, J=6.36 Hz, 3 H).

Step B. N-(5-Chloro-3-methyl-1H-pyrazol-4-yl)-5-fluoro-4-(6-(1-hydroxyethyl)-5-methoxypyridin-2-yl)-2-(((S)-1,1,1-trifluoropropan-2-yl)oxy)benzamide. The title compound was prepared in a manner analogous to Example 88 however using 1-(6-bromo-3-methoxypyridin-2-yl)ethan-1-ol as the aryl halide and acid (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₂H₂₁ClF₄N₄O₄, 516.1; m/z found, 517.1 [M+H]⁺. ¹H NMR (400 MHz, DMSO) δ = 12.85 (br s, 1 H) 9.39 (s, 1 H) 7.96 (dd, J=6.36, 1.96 Hz, 1 H) 7.82 (dd, J=8.56, 1.71 Hz, 1 H) 7.37 - 7.63 (m, 2 H) 5.36 - 5.57 (m, 1 H) 5.08 (t, J=6.60 Hz, 1 H) 4.83 - 4.98 (m, 1 H) 3.91 (s, 3 H) 2.16 (s, 3 H) 1.50 (dd, J=5.87, 3.91 Hz, 3 H) 1.41 (d, J=6.36 Hz, 3 H).

### Example 113: N-(5-Chloro-3-methyl-1H-pyrazol-4-yl)-5-fluoro-4-(6-(1-hydroxyethyl)-5-methoxypyrazin-2-yl)-2-(((S)-1,1,1-trifluoropropan-2-yl)oxy)benzamide.

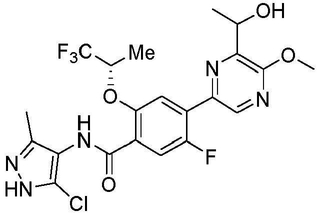

Step A: (6-Bromo-3-methoxypyrazin-2-yl) methanol. To a solution of 6-bromo-3-methoxy-pyrazine-carboxylic methyl ester (900 mg, 3.643 mmol) in DCM (10 mL) was added dropwise diisobutylaluminium hydride 1.0 M in DCM (6.557 mL, 6.557 mmol) at -78 °C under N₂. The reaction mixture was stirred at -78 °C for 1 and a half hours. The reaction mixture was quenched with methanol at - 78 °C. Then saturated ammonium chloride solution was added to the mixture. After stirring at room temperature for 30 minutes, the organic layer was separated, washed with brine, dried over MgSO₄, filtered and concentrated under reduced pressure to give the title compound, which was used without further purification. MS (ESI): mass calcd. for C₆H₇BrN₂O₂, 218.0; m/z found, 219.0 [M+H]⁺. ¹H NMR (400 MHz, CDCl₃) δ = 8.12 (s, 1 H) 4.73 (d, J=5.38 Hz, 2 H) 3.99 (s, 3 H) 3.14 - 3.30 (m, 1 H).

Step B: 6-bromo-3-Methoxypyrazine-2-carbaldehyde. To a solution of (6-bromo-3-methoxypyrazin-2-yl) methanol (462.857 mg, 2.113 mmol) in DCM (6 mL) was added Dess-Martin periodinane (986 mg, 2.32 mmol). The mixture was stirred at room temperature for 16 hours, then quenched with saturated aqueous NaHCO₃. The reaction mixture was extracted with DCM. The combined organics were dried over MgSO₄, filtered and concentrated under reduced pressure to give the title compound. MS (ESI): mass calcd. for C₆H₅BrN₂O₂, 216.0; m/z found, 217.0 [M+H]⁺. ¹H NMR (400 MHz, CDCl₃) δ = 10.15 (s, 1 H) 8.45 (s, 1 H) 4.12 (s, 4 H). It was used without purification for the next step.

Step C: (6-Bromo-3-methoxypyrazin-2-yl) methanol. The title compound was prepared according to the representative procedure of Example 112, Step A, except substituting 6-bromo-3-methoxypyrazine-2-carbaldehyde for 6-bromo-3-methoxypicolinaldehyde. MS (ESI): mass calcd. for C₇H₉BrN₂O₂, 232.0; m/z found, 233.0 [M+H]⁺.

Step D. N-(5-Chloro-3-methyl-1H-pyrazol-4-yl)-5-fluoro-4-(6-(1-hydroxyethyl)-5-methoxypyrazin-2-yl)-2-(((S)-1,1,1-trifluoropropan-2-yl)oxy)benzamide. The title compound was prepared in a manner analogous to Example 88 however using 1-(6-bromo-3-methoxypyrazin-2-yl)ethan-1-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₁H₂₀ClF₄N₅O₄, 517.1; m/z found, 518.1 [M+H]⁺. ¹H NMR (400 MHz, DMSO) δ = 12.85 (s, 1 H) 9.44 (s, 1 H) 8.61 (d, J=1.96 Hz, 1 H) 7.91 (d, J=6.36 Hz, 1 H) 7.54 (d, J=10.76 Hz, 1 H) 5.37 - 5.49 (m, 1 H) 5.17 - 5.23 (m, 1 H) 5.04 (t, J=6.60 Hz, 1 H) 4.02 (s, 3 H) 2.16 (s, 3 H) 1.50 (d, J=6.36 Hz, 3 H) 1.46 (d, J=6.36 Hz, 3 H).

### Example 114: (S)-N-(2-chloro-6-fluorophenyl)-5-fluoro-4-(5-fluoro-6-(hydroxymethyl)pyridin-2-yl)-2-((1,1,1-trifluoropropan-2-yl)oxy)benzamide.

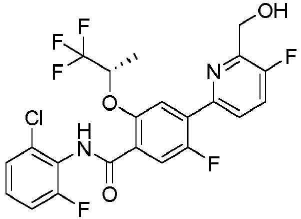

The title compound was prepared in a manner analogous to Example 1, Step E however using 6-chloro-3-fluoropyridin-2-yl)methanol (Example 99, Step A) as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate.MS (ESI): mass calcd. for C₂₂H₁₅ClF₆N₂O₃, 504.8; m/z found, 505.1 [M+H]⁺. ¹H NMR (CHLOROFORM-d, 400 MHz) δ 9.12 (s, 1H), 8.14 (d, 1H, J--11.7 Hz), 7.91 (dd, 1H, J=3.4, 8.3 Hz), 7.75 (d, 1H, J=5.4 Hz), 7.54 (t, 1H, J=8.6 Hz), 7.2-7.3 (m, 2H), 7.14 (t, 1H, *J*=8.8 Hz), 5.06 (quind, 1H, J--6.1, 12.2 Hz), 4.94 (d, 2H, *J*=4.9 Hz), 3.80 (t, 1H, J=4.9 Hz), 1.7-1.7 (m, 3H).

### Example 115: (S)-5-fluoro-4-(5-fluoro-6-hydroxypyridin-2-yl)-N-(3-methyl-5-(trifluoromethyl)-1H-pyrazol-4-yl)-2-((1,1,1 -trifluoropropan-2-vl)oxy)benzamide.

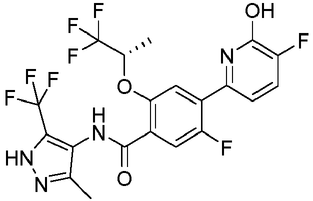

The title compound was prepared in a manner analogous to Example 88 however using 6-chloro-3-fluoropyridin-2-ol as the aryl halide and (*S*)-(2-Fluoro-4-((5-methyl-3-(trifluoromethyl)-1*H-*pyrazol-4-yl)carbamoyl)-5-((1,1,1-trifluoropropan-2-yl)oxy)phenyl)boronic acid (Example 93, Step B) as the boronate. MS (ESI): mass calcd. for C₂₀H₁₄F₈N₄O₃, 510.1; m/z found, 511.2 [M+H]⁺. ¹H NMR (DMSO-d₆, 400 MHz) δ 9.58 (s, 1H), 7.5-7.6 (m, 2H), 7.4-7.5 (m, 1H), 6.4-6.7 (m, 1H), 5.4-5.5 (m, 1H), 2.18 (s, 3H), 1.48 (d, 3H, J=6.4 Hz).

### Example 116: (S)-N-(2-chloro-6-fluorophenyl)-4-(5-chloro-6-hydroxypyridin-2-yl)-5-fluoro-2-((1,1,1-trifluoropropan-2-yl)oxy)benzamide.

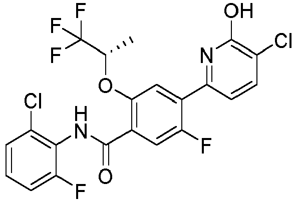

The title compound was prepared in a manner analogous to Example 1, Step E however using 6-bromo-3-chloropyridin-2-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₁H₁₃Cl₂F₅N₂O₃, 506.2; m/z found, 507.2 [M+H]⁺. ¹H NMR (DMSO-d₆, 400 MHz) δ 12.4-12.7 (m, 1H), 10.05 (s, 1H), 7.88 (br d, 1H, J=7.8 Hz), 7.6-7.7 (m, 1H), 7.3-7.5 (m, 4H), 6.5-6.6 (m, 1H), 5.4-5.5 (m, 1H), 1.50 (br d, 3H, J=6.4 Hz).

### Example 117: (S)-4-(3-amino-5-chloropyrazin-2-yl)-N-(2-chloro-6-fluorophenyl)-5-fluoro-2-((1,1,1-trifluoropropan-2-yl)oxy)benzamide.

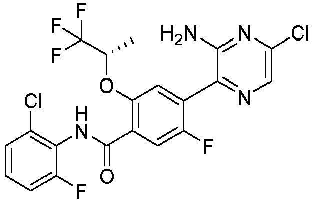

The title compound was prepared in a manner analogous to Example 1, Step E however using 3-bromo-6-chloropyrazin-2-amine as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₀H₁₃Cl₂F₅N₄O₂, 506.3; m/z found, 507.2 [M+H]⁺. ¹H NMR (CHLOROFORM-d, 400 MHz) δ 9.08 (s, 1H), 8.12 (d, 1H, J=10.5 Hz), 8.04 (s, 1H), 7.2-7.3 (m, 3H), 7.13 (t, 1H, J--8.7 Hz), 4.9-5.1 (m, 3H), 1.6-1.7 (m, 3H).

### Example 118: (S)-N-(2-chloro-6-fluorophenyl)-5-fluoro-4-(5,6,7,8-tetrahydro-2,7-naphthyridin-3-yl)-2-((1,1,1-trifluoropropan-2-yl)oxy)benzamide.

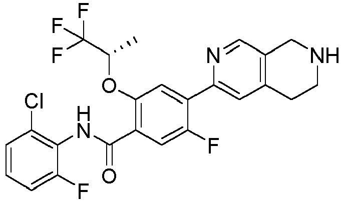

Step A. tert-butyl (S)-6-(4-((2-chloro-6-fluorophenyl)carbamoyl)-2-fluoro-5-((1,1,1-trifluoropropan-2-yl)oxy)phenyl)-3,4-dihydro-2,7-naphthyridine-2(1H)-carboxylate. The title compound was prepared in a manner analogous to Example 1, Step E however using tert-butyl 6-chloro-3,4-dihydro-2,7-naphthyridine-2(1H)-carboxylate as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₉H₂₇ClF₅N₃O₄, 611.2; m/z found, 612.2 [M+H]⁺.

Step B. (S)-N-(2-chloro-6-fluorophenyl)-5-fluoro-4-(5,6,7,8-tetrahydro-2,7-naphthyridin-3-yl)-2-((1,1,1-trifluoropropan-2-yl)oxy)benzamide. To a vial was added tert-butyl (S)-6-(4-((2-chloro-6-fluorophenyl)carbamoyl)-2-fluoro-5-((1,1,1-trifluoropropan-2-yl)oxy)phenyl)-3,4-dihydro-2,7-naphthyridine-2(1H)-carboxylate (50 mg, 0.0817 mmol) and DCM (4 mL) and TFA (1 mL). The reaction was stirred at rt for 4 hrs. The reaction was concentrated, dissolved in DCM (4 mL) and washed with 1N NaOH (1 x 4 mL). the organics were dried, filtered, and concentrated. Purification by chromatography (SiO₂, 0 to 20% MeOH in DCM) yielded the title compound as a white solid. MS (ESI): mass calcd. for C₂₄H₁₉ClF₅N₃O₂ 511.1; m/z found, 512 M+H]⁺. ¹H NMR (CHLOROFORM-d, 400 MHz) δ 9.16 (s, 1H), 8.40 (s, 1H), 8.13 (s, 1H), 8.10 (s, 1H), 7.86 (d, 1H, J=5.9 Hz), 7.70 (s, 1H), 7.3-7.3 (m, 1H), 7.0-7.2 (m, 1H), 5.13 (td, 1H, *J*=6.3, 12.3 Hz), 5.0-5.2 (m, 1H), 4.10 (s, 2H), 3.20 (t, 2H, J=5.9 Hz), 2.88 (t, 2H, J=5.6 Hz), 1.6-1.7 (m, 3H).

### Example 119: (S)-N-(2-chloro-6-fluorophenyl)-5-fluoro-4-(5,6,7,8-tetrahydro-1,8-naphthyridin-2-yl)-2-((1,1,1-trifluoropropan-2-yl)oxy)benzamide.

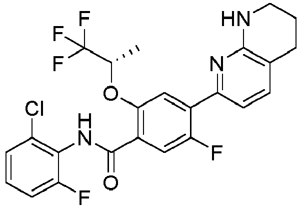

The title compound was prepared according to the representative procedure of Example 118 except substituting tert-butyl 7-chloro-3,4-dihydro-1,8-naphthyridine-1(2H)-carboxylate for tert-butyl 6-chloro-3,4-dihydro-2,7-naphthyridine-2(1H)-carboxylate. MS (ESI): mass calcd. for C₂₄H₁₉ClF₅N₃O₂ 511.1; m/z found, 512 M+H]⁺.¹H NMR (CHLOROFORM-d, 400 MHz) δ 9.15 (s, 1H), 8.06 (d, 1H, J--11.7 Hz), 7.77 (d, 1H, J=5.9 Hz), 7.28 (s, 2H), 7.1-7.3 (m, 3H), 5.08 (td, 1H, J=6.2, 12.6 Hz), 4.99 (br s, 1H), 3.48 (br s, 2H), 2.80 (t, 2H, J=6.1 Hz), 1.9-2.1 (m, 2H), 1.6-1.7 (m, 3H).

### Example 120: (S)-4-(6-amino-5-methylpyridin-2-yl)-N-(5-chloro-3-methyl-1H-pyrazol-4-yl)-5-fluoro-2-(1,1,1-trifluoropropan-2-vl)oxy)benzamide.

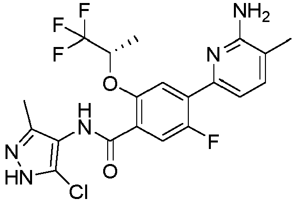

The title compound was prepared in a manner analogous to Example 1, Step E however using 6-bromo-3-methylpyridin-2-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₈ClF₄N₅O₂, 471.1; m/z found, 472.2 [M+H]⁺. ¹H NMR (CHLOROFORM-d, 400 MHz) δ 8.86 (br s, 1H), 7.94 (br d, 1H, J--11.2 Hz), 7.4-7.7 (m, 2H), 6.9-7.1 (m, 1H), 5.2-5.5 (m, 1H), 2.1-2.3 (m, 6H), 1.4-1.7 (m, 3H).

### Example 121: (S)-N-(5-chloro-3-methyl-1H-pyrazol-4-yl)-4-(5-chloro-6-hydroxypyridin-2-yl)-5-fluoro-2-((1,1,1-trifluoropropan-2-vl)oxy)benzamide.

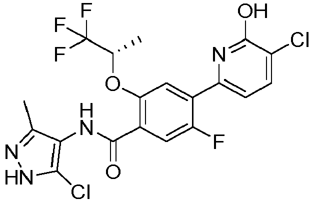

The title compound was prepared in a manner analogous to Example 1, Step E however using 6-bromo-3-chloropyridin-2-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₉H₁₄Cl₂F₄N₄O₃, 492.0; m/z found, 472.2 [M+H]^{+ 1}H NMR (DMSO-d₆, 400 MHz) δ 12.87 (br s, 1H), 9.50 (s, 1H), 7.89 (d, 1H, *J*=7.3 Hz), 7.58 (br d, 1H, *J*=5.4 Hz), 7.51 (d, 1H, *J*=9.8 Hz), 7.15 (br s, 1H), 6.55 (br s, 1H), 5.4-5.5 (m, 1H), 2.15 (s, 3H), 1.50 (br d, 3H, J=5.9 Hz).

### Example 122: (S)-4-(4-amino-5-methylpyridin-2-yl)-N-(5-chloro-3-methyl-1H-pyrazol-4-yl)-5-fluoro-2-(1,1,1-trifluoropropan-2-yl)oxy)benzamide.

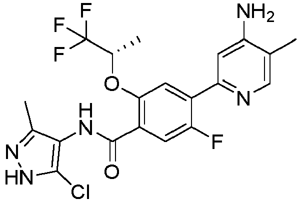

The title compound was prepared in a manner analogous to Example 1, Step E however using 2-bromo-5-methylpyridin-4-amine as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₈ClF₄N₅O₂, 471.1; m/z found, 472.2 [M+H]⁺. ¹H NMR (CHLOROFORM-d, 400 MHz) δ 8.0-8.1 (m, 1H), 7.8-8.0 (m, 1H), 7.5-7.7 (m, 1H), 7.19 (br s, 1H), 5.3-5.5 (m, 1H), 2.1-2.2 (m, 6H), 1.5-1.6 (m, 3H) (four exchangeable protons not observed).

### Example 123: N-(5-chloro-3-methyl-1H-pyrazol-4-yl)-5-fluoro-4-(5-fluoro-6-((*R)-1-hydroxyethyl)pyridin-2-yl)-2-(((S)-1,1,1-trifluoropropan-2-vl)oxy)benzamide.

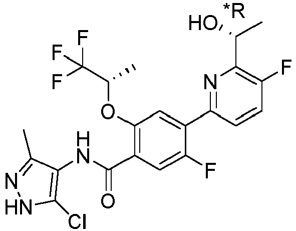

Step A. 1-(6-chloro-3-fluoropyridin-2-yl)ethanol. To a solution of 6-chloro-3-fluoropicolinaldehyde (1.5 g, 9.40 mmol, 1.0 eq.) in THF (30 mL) was added MeMgBr (3 M in Et₂O, 6.26 ml, 18.80 mmol, 2.0 eq.) at 0 °C under N₂. Then the mixture was stirred at 0 °C for 1 hour. The mixtures were quenched with NH₄Cl (5 mL), extracted with DCM (20 mL × 3). The organic layers were dried with Na₂SO₄, filtered, and concentrated under vacuum. The residue was purified by column chromatography (SiO₂, gradient elution: 0 - 30% ethyl acetate in petroleum ether) to give the title compound as light yellow oil. ¹H NMR (400 MHz, CDCl₃) *δ* 7.41 - 7.35 (m, 1H), 7.27 - 7.21 (m, 1H), 5.17 - 5.04 (m, 1H), 3.67 (d, *J* = 7.7 Hz, 1H), 1.28-1.22 (m, 3H); ¹⁹F NMR (376 MHz, CDCl₃) δ -130.42 (s, 1F) ppm.

Step B. N-(5-chloro-3-methyl-1H-pyrazol-4-yl)-5-fluoro-4-(5-fluoro-6-(1-hydroxyethyl)pyridin-2-yl)-2-(((S)-1,1,1-trifluoropropan-2-yl)oxy)benzamide To a solution of (S)-(4-((5-chloro-3-methyl-1H-pyrazol-4-yl)carbamoyl)-2-fluoro-5-((1,1,1- trifluoropropan-2-yl)oxy)phenyl)boronic acid (Example 79, Step A, 1.0 g, 2.44 mmol, 1.0 eq.), 1-(6-chloro-3- fluoropyridin-2-yl)ethanol (428.7 mg, 2.44 mmol, 1.0 eq.) and K₂CO₃ (843.6 mg, 6.10 mmol, 2.5 eq.) in dioxane (24 mL) was added XPhos Pd G3 (310.0 mg, 0.36 mmol, 0.15 eq.), and the reaction mixture was stirred at 90 °C for 3 hours under N₂. The mixture extracted with ethyl acetate (20 mL × 3). The organic layer was separated, washed with brine (20 mL), dried with Na₂SO₄, filtered and concentrated under vacuum. The residue was purified by column chromatography (SiO₂, gradient elution: 0-90% ethyl acetate in petroleum ether) to give the title compound as light yellow sticky. ¹H NMR (400 MHz, CDCl₃) *δ* 9.86 (br s, 1H), 8.81 (s, 1H), 8.12 (d, *J* = 11.8 Hz, 1H), 7.91 (dd, *J* = 2.6, 8.4 Hz, 1H), 7.73 (dd, *J=* 2.4, 5.8 Hz, 1H), 7.55 (t, *J=* 8.8 Hz, 1H), 5.22 (br t, *J=* 6.4 Hz, 1H), 5.10 - 4.95 (m, 1H), 4.27 (br d, *J* = 4.5 Hz, 1H), 2.33 (s, 3H), 1.71 (dd, *J* = 2.0, 6.4 Hz, 3H), 1.60 (br s, 3H); ¹⁹F NMR (376 MHz, CDCl₃) *δ* -78.08 (br d, J=6.9 Hz, 1F), -122.29 (br s, 1F), -126.21 - -126.80 (m, 1F) ppm.

Step C. N-(5-chloro-3-methyl-1H-pyrazol-4-yl)-5-fluoro-4-(5-fluoro-6-((**S*)-1-hydroxyethyl)pyridin-2-yl)-2-(((S)-1,1,1-trifluoropropan-2-yl)oxy)benzamide *&* N-(5-chloro-3-methyl-1H-pyrazol-4-yl)-5-fluoro-4-(5-fluoro-6-((**R*)-1-hydroxyethyl)pyridin-2-yl)-2-(((S)-1,1,1 -trifluoropropan-2-vl)oxy)benzamide The N-(5-chloro-3-methyl-1H-pyrazol-4-yl)-5-fluoro-4-(5-fluoro-6-(1-hydroxyethyl)pyridin-2-yl)-2-(((S)-1,1,1-trifluoropropan-2-yl)oxy)benzamide (788.0 mg, 1.56 mmol) was separated by SFC (Column: DAICEL CHIRALPAK AD(250mm*30mm,10um)); Mobile phase: CO₂(A) - ethanol (0.05% DEA) (B), gradient elution: 20% B in A at 60 mL/min) to give the first eluting compound as off-white powder. MS (ESI): mass calcd. for C₂₁H₁₈ClF₅N₄O₃, 504.1; m/z found, 505.0, [M+H]⁺. ¹H NMR (400 MHz, CDCl₃) *δ* 10.00 (br s, 1H), 8.80 (s, 1H), 8.12 (d, J= 11.8 Hz, 1H), 7.91 (ddd, *J* = 1.5, 3.7, 8.6 Hz, 1H), 7.72 (d, *J* = 5.8 Hz, 1H), 7.55 (t, *J* = 8.8 Hz, 1H), 5.22 (br s, 1H), 5.07 - 4.96 (m, 1H), 4.32 - 4.21 (m, 1H), 2.33 (s, 3H), 1.71 (d, *J* = 6.3 Hz, 3H), 1.60 (br d, *J* = 0.8 Hz, 3H); ¹⁹F NMR (376 MHz, CDCl₃) δ -78.09 (s, 1F), -122.30 (br s, 1F), -126.56 (s, 1F) ppm.

### Example 124: N-(5-chloro-3-methyl-1H-pyrazol-4-yl)-5-fluoro-4-(5-fluoro-6-((S)-1-hydroxyethyl)pyridin-2-yl)-2-(((S)-1,1,1-trifluoropropan-2-yl)oxy)benzamide.

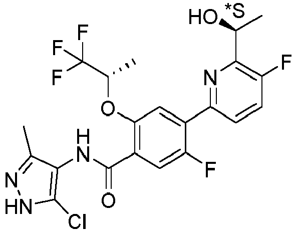

The title compound was described as in Example 123, Step C; however, the 1^{st} peak was isolated. MS (ESI): mass calcd. for C₂₁H₁₈ClF₅N₄O₃, 504.1; m/z found, 505.0, [M+H]⁺.

¹H NMR (400 MHz, CDCl₃) *δ* 10.13 (br s, 1H), 8.81 (s, 1H), 8.12 (d, *J* = 11.8 Hz, 1H), 7.95-7.89 (m, 1H), 7.73 (d, *J* = 6.0 Hz, 1H), 7.55 (t, *J* = 8.8 Hz, 1H), 5.22 (br s, 1H), 5.02 (td, *J* = 6.1, 12.3 Hz, 1H), 4.28 (br s, 1H), 2.33 (s, 3H), 1.70 (d, *J* = 6.6 Hz, 3H), 1.60 (s, 3H); ¹⁹F NMR (376 MHz, CDCl₃) *δ* -78.08 (br d, *J* = 6.9 Hz, 1F), -122.29 (br s, 1F), -126.21 - -126.80 (m, 1F) ppm.

SFC: purity 100 %.

### Example 125: (S)-N-(2-chloro-6-fluorophenyl)-5-fluoro-4-(5-(2-hydroxypropan-2-yl)-6-methylpyrazin-2-yl)-2-((1,1,1-trifluoropropan-2-Yl)oxy)benzamide.

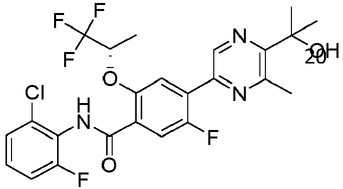

Step A. mixture of 2-(methoxycarbonyl)-3-methylpyrazine 1-oxide & 3-(methoxvcarbonyl)-2-methylpyrazine 1-oxide. To a solution of methyl 3-methylpyrazine-2-carboxylate (7 g, 46.01 mmol) in CHCl₃ (140 mL) was added *m*-CPBA (85%, 12.6 g, 62.11 mmol). The mixture was stirred at 70 °C for 24 hours. To the mixture was added 10% aq. Na₂SO₃ (70 mL) drop wise at 0 °C and stirred for 0.5 hour at same temperature. The mixture was added sat. aq. NaHCO₃ to adjust pH > 7 and extracted with DCM (100 mL × 3). The combined organic layers were dried with Na₂SO₄, filtered and concentrated to give the title compound (7.5 g crude) as red solid. MS (ESI): mass calcd. for C₇H₈N₂O₃, 168.1; m/z found, 168.8 [M+H]⁺.¹H NMR (400MHz, CDCl₃) δ = 8.33 (d, *J*=3.9 Hz, 1H), 8.20 (d, *J*=3.9 Hz, 1H), 3.96 (s, 3H), 2.64 (s, 3H).

Step B. Methyl 5-chloro-3-methylpyrazine-2-carboxylate. A solution of 2-(methoxycarbonyl)-3-methylpyrazine 1-oxide and 3-(methoxycarbonyl)-2-methylpyrazine 1-oxide (7.5 g crude as mixture) in DMF (20 mL) was added to POCl₃ (35.7 g, 232.96 mmol) drop wise at an ice-bath. The reaction mixture was stirred at 100 °C for 1 hour and cold down to room temperature. The mixture was slowly added to sat. aq. Na₂CO₃ (300 mL) with ice-water bath and adjust the pH to 6~8 with Na₂CO₃ powder. The mixture was extracted with DCM (300 mL × 3). The combined organic layers were washed with brine (500 mL), dried over Na₂SO₄, filtered, and concentrated under vacuum. The crude product was purified by column chromatography (SiO₂, gradient elution: 0 - 100% DCM in petroleum ether) to give the mixture of regio-isomers as pale yellow solid. The mixture was separated by SFC: (Column: DAICEL CHIRALCEL OD 250 × 30 mm I.D., 10 µm; mobile phase: CO₂ (A) - EtOH (0.1% NH₃.H₂O) (B); isocratic: 25% B in A; flow rate: 180 mL/min; column temp.: 40 °C; ABPR: 100 bar.) to give the following: The first eluting compound was determined to be methyl 6-chloro-3-methylpyrazine-2-carboxylate (2.8 g, 14.78 mmol, 98% purity) as pale yellow solid. MS (ESI): mass calcd. for C₇H₇ClN₂O₂, 186.0; m/z found, 186.8 [M+H]⁺. ¹H NMR (400MHz, CDCl₃) δ = 8.64 (s, 1H), 4.01 (s, 3H), 2.84 (s, 3H). The second eluting compound was determined to be the title compound (1 g, 4.96 mmol, 93% purity) as pale yellow solid. MS (ESI): mass calcd. for C₇H₇ClN₂O₂, 186.0; m/z found, 187.0 [M+H]⁺. ¹H NMR (400MHz, CDCl₃) δ = 8.52 (s, 1H), 4.02 (s, 3H), 2.87 (s, 3H).

Step C. 2-(5-chloro-3-methylpyrazin-2-yl)propan-2-ol. To a solution of methyl 5-chloro-3-methylpyrazine-2-carboxylate (1 g, 4.96 mmol, 93% purity) in THF (20 mL) was added MeMgBr (3 M solution in ether, 6.6 mL, 19.8 mmol) drop wise at 0 °C under N₂. The resulting mixture was stirred at 0 °C for 1 hour. The reaction mixture was quenched with sat. aq. NH₄Cl (5 mL) at 0 °C. THF was evaporated under vacuum, the aqueous layer was extracted with DCM (15 mL × 3). The combined organic layers were dried over Na₂SO₄, filtered and concentrated. The crude product was purified by column chromatography (SiO₂, gradient elution: 0 - 30% ethyl acetate in petroleum ether) to give the title compound as brown oil. MS (ESI): mass calcd. for C₈H₁₁ClN₂O, 186.1; m/z found, 187.1 [M+H]⁺. ¹H NMR (400MHz, CDCl₃) δ = 8.34 (s, 1H), 4.67 (s, 1H), 2.75 (s, 3H), 1.63 (s, 6H).

Step D: : (S)-N-(2-chloro-6-fluorophenyl)-5-fluoro-4-(5-(2-hydroxypropan-2-yl)-6-methylpyrazin-2-yl)-2-((1,1,1-trifluoropropan-2-yl)oxy)benzamide. The title compound was prepared in a manner analogous to Example 88 however using 2-(5-chloro-3-methylpyrazin-2-yl)propan-2-ol as the aryl halide and Intermediate 1 as the boronate. MS (ESI): mass calcd. for C₂₄H₂₁ClF₅N₃O₃, 529.1; m/z found, 530.2 [M+H]⁺. ¹H NMR (DMSO-d₆, 400 MHz) δ 10.04 (s, 1H), 8.7-8.8 (m, 1H), 7.94 (d, 1H, J=5.9 Hz), 7.56 (d, 1H, *J*=10.3 Hz), 7.3-7.5 (m, 3H), 5.4-5.5 (m, 1H), 5.36 (s, 1H), 2.88 (s, 3H), 1.58 (s, 6H), 1.49 (d, 3H, *J*=6.4 Hz)

### Example 126: (S)-5-fluoro-4-(1-methyl-2,6-dioxo-1,2,3,6-tetrahydropyrimidin-4-yl)-N-(3-methyl-5-(trifluoromethyl)-1H-pyrazol-4-yl)-2-((1,1,1-trifluoropropan-2-yl)oxy)benzamide.

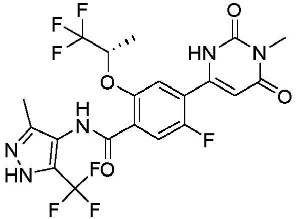

The title compound was prepared in a manner analogous to Example 88 however using 6-chloro-3-methylpyrimidine-2,4(1H,3H)-dione as the aryl halide and (*S*)-(2-Fluoro-4-((5-methyl-3-(trifluoromethyl)-1*H*-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₀H₁₆F₇N₅O₄, 523.1; m/z found, 524.2, [M+H]⁺. ¹H NMR (DMSO-d₆, 400 MHz) δ 9.64 (s, 1H), 7.56 (d, 1H, *J*=5.6 Hz), 7.45 (d, 1H, *J*=10.0 Hz), 5.9-6.0 (m, 1H), 5.4-5.5 (m, 1H), 3.20 (s, 3H), 2.18 (s, 3H), 1.4-1.5 (m, 3H).

### Example 127: (S)-N-(5-chloro-3-methyl-1H-pyrazol-4-yl)-5-fluoro-4-(5-fluoro-6-oxo-1,6-dihydropyrimidin-2-yl)-2-((1,1,1-trifluoropropan-2-yl)oxy)benzamide.

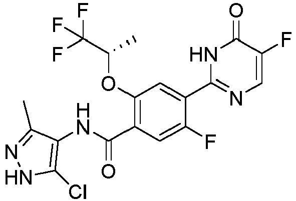

The title compound was prepared in a manner analogous to Example 88 however using 2-chloro-5-fluoropyrimidin-4(3H)-one as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₈H₁₃ClF₅N₅O₃, 477.1; m/z found, 478.2, [M+H]⁺. ¹H NMR (DMSO-d₆, 400 MHz) δ 12.8-12.9 (m, 1H), 9.5-9.6 (m, 1H), 8.1-8.2 (m, 1H), 7.6-7.7 (m, 1H), 7.4-7.5 (m, 1H), 5.2-5.5 (m, 1H), 2.15 (s, 3H), 1.4-1.5 (m, 3H).

### Example 128: (S)-4-(6-amino-5-fluoropyrazin-2-yl)-N-(5-chloro-3-methyl-1H-pyrazol-4-yl)-5-fluoro-2-((1,1,1-trifluoropropan-2-yl)oxy)benzamide.

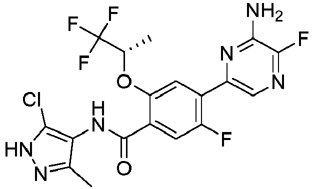

Step A. 3,5-dichloro-2-fluoroyprazine. To a mixture of 3,5-dichloropyrazin-2-amine (1.0 g, 6.10 mmol, 1 eq.) in HF Pyridine (20 mL) was added NaNO₂ (504.86 mg, 7.32 mmol, 1.2 eq.) in portions at 0 °C under N₂. The mixture was stirred and slowly warmed to room temperature over a period of 3 hours. The reaction was adjust to pH ~ 3 with 1 M aq. HCl. The mixture was extracted with DCM (100 mL × 3). The combined organic layers was dried, filtered and concentrated under vacuum. The crude was purified by column chromatography (SiO2, gradient elution: DCM) to give the title compound as pale yellow solid. ¹H NMR (400 MHz, CDCl₃) *δ* 8.16 (d, J = 1.7 Hz, 1H); ¹⁹F NMR (376 MHz, CDCl₃) *δ* -79.36 (1F) ppm.

Step B. tert-butyl (6-chloro-3-fluoropyrazin-2-yl)carbamate. To a mixture of 3,5-dichloro-2-fluoropyrazine (830 mg, 4.97 mmol, 1 eq.), BocNH₂ (699 mg, 5.96 mmol, 1.2 eq.), XantPhos (575.26 mg, 994.20 µmol, 0.2 eq.) and K₃PO₄ (1.58 g, 7.45 mmol, 1.5 eq.) in Dioxane (17 mL) was added Pd(OAc)₂ (112 mg, 497 µmol, 0.1 eq.) in portions. The mixture was stirred at 60 °C for 16 hours under N₂. The mixture was diluted with ethyl acetate (30 mL) and filtered. The filtrate was concentrated under vacuum. The crude was purified by column chromatography (SiO₂, gradient elution: 0 - 7% ethyl acetate in petroleum ether) to give the title compound as light green solid. MS (ESI): mass calcd. for C₉H₁₁ClFN₃O₂, 247.1; m/z found, 191.8 [M+H-56]⁺. ¹H NMR (400 MHz, CDCl₃) *δ* 7.83 (d, J = 2.0 Hz, 1H), 7.03 (br d, J = 9.7 Hz, 1H), 1.55 (s, 9H); ¹⁹F NMR (376 MHz, CDCl₃) *δ* -88.29 (1F) ppm.

Step C. (*S*)-tert-butyl (6-(4-((5-chloro-3-methyl-1*H*-pyrazol-4-yl)carbamoyl)-2-fluoro-5-((1,1,1-trifluoropropan-2-yl)oxy)phenyl)-3-fluoropyrazin-2-yl)carbamate. A mixture of (S)-(4-((5-chloro-3-methyl-1H-pyrazol-4-yl)carbamoyl)-2-fluoro-5-((1,1,1-tri fluoropropan-2-yl)oxy)phenyl)boronic acid (Example 79, Step A, 150 mg, 366.28 µmol, 1 eq.), tert-butyl (6-chloro-3-fluoropyrazin-2-yl)carbamate (103.9 mg, 419.54 µmol, 1.1 eq.), K₃PO₄ (233.3 mg, 1.10 mmol, 3 eq.) in dioxane/H₂O (v/v=5/1, 3 mL) was added Pd-118 (47.7 mg, 73.25 µmol, 0.2 eq.) under N₂. The mixture was stirred at 60 °C for 1 h. The reaction mixture was diluted with ethyl acetate (20 mL), then washed with H₂O 10 mL, and extracted with ethyl acetate 20 mL. The organic layers were dried over anhydrous Na₂SO₄, filtered and concentrated under reduced pressure. The residue was purified by column chromatography (12 g, gradient elution: 0 - 50 % petroleum ether in ethyl acetate) to give the title compound as white solid. MS (ESI): mass calcd. for C₂₃H₂₂ClF₅N₆O₄, 576.13; m/z found, 599.0 [M+Na]⁺. ¹H NMR (400 MHz, CDCl₃) *δ* 9.62 (br s, 1 H), 8.83 (s, 1 H), 8.53 (s, 1 H), 8.04 - 8.15 (m, 2H), 7.14 (s, 1 H), 5.09 (dt, J = 12.22, 6.05 Hz, 1 H), 2.34 (s, 3 H), 1.75 (d, J = 6.56 Hz, 3 H), 1.57 (s, 9 H); ¹⁹F NMR (376 MHz, CDCl₃) *δ-*78.30 (3 F), -85.53 (1 F), -119.62 (1 F) ppm.

Step D. (S)-4-(6-amino-5-fluoropyrazin-2-yl)-N-(5-chloro-3-methyl-1*H*-pyrazol-4-yl)-5-fluoro-2-((1,1,1-trifluoropropan-2-yl)oxy)benzamide. To a solution of (S)-tert-butyl (6-(4-((5-chloro-3-methyl-1H-pyrazol-4-yl)carbamoyl)-2-fluoro -5-((1,1,1-trifluoropropan-2-yl)oxy)phenyl)-3-fluoropyrazin-2-yl)carbamate (90 mg, 133 µmol, 1 eq.) in DCM (10 mL) was added TFA (2 mL) at 0 °C. Then the mixture was stirred at 0 °C for 2 hours. The mixture was concentrated in vacuum to give the residue. Then diluted with DCM (10 mL), alkalized with sat. aq. NaHCO₃ (8 mL, pH-7), then extracted with DCM (20 mL × 2). The combined organic phase was dried with anhydrous Na₂SO₄, filtered and concentrated in vacuum. The crude product was purified by preparative high-performance liquid chromatography [ Column:Boston Prime C18 150 × 30mm × 5um, Condition: A: water(0.05%NH₃H₂O + 10mM NH₄HCO₃), B: ACN, at the beginning: A (55%) and B (45%), at the end: A: (25%) and B (75%), Gradient Time (min) 7; 100%B Hold Time (min) 1; Flow Rate (ml/min) 25] to give the title compound as white powdery solid. MS (ESI): mass calcd. for C₁₈H₁₄ClF₅N₆O₂, 476.08; m/z found, 477.0 [M+H]⁺. ¹H NMR (400 MHz, CDCl₃) *δ* 9.87 (br s, 1 H), 8.80 (s, 1 H), 8.14 (t, J = 2.09 Hz, 1 H), 8.10 (d, J = 11.92 Hz, 1 H), 7.72 (d, J = 5.72 Hz, 1 H), 5.01 - 5.09 (m, 1 H), 4.99 (br s, 2 H), 2.33 (s, 3 H), 1.70 (d, J = 6.44 Hz, 3 H); ¹⁹F NMR (376 MHz, CDCl₃) *δ* 78.17 ( 3 F), -88.46 ( 1 F), -119.31 ( 1 F) ppm.

### Example 129: (S)-4-(6-amino-5-chloropyrazin-2-yl)-N-(5-chloro-3-methyl-1H-pyrazol-4-yl)-5-fluoro-2-((1,1,1-trifluoropropan-2-yl)oxy)benzamide.

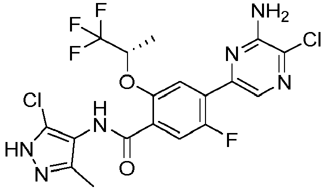

The HCl (4 M solution in ethyl acetate, 10 mL) was added to (S)-tert-butyl (6-(4-((5-chloro-3-methyl-1H-pyrazol-4-yl)carbamoyl)-2-fluoro-5-((1,1,1-trifluoropropan-2-yl)oxy)phenyl)-3-fluoropyrazin-2-yl)carbamate (Example 128, step C, 90 mg, 136.5 µmol, 1 eq.) at 0 °C. Then the mixture was stirred at 0 °C for 2 hours. The mixture was concentrated in vacuum to give the residue. Then diluted with DCM 10 mL, alkalized with sat. aq. NaHCO₃ (8 mL, pH~7) and extracted with DCM 40 mL (20 mL × 2). The combined organic phase was dried with anhydrous Na₂SO₄, filtered and concentrated in vacuum. The crude product was purified by preparative high-performance liquid chromatography: [Column:Boston Prime C18 150 × 30mm × 5um, Condition: A: water(0.05%NH₃H₂O + 10mM NH₄HCO₃), B: ACN, at the beginning: A (55%) and B (45%); at the end: A: (25%) and B (75%), Gradient Time (min) 7; 100%B Hold Time (min) 0; Flow Rate (ml/min) 25.] to give the title compound as light yellow powdery. MS (ESI): mass calcd. for C₁₈H₁₄Cl₂F₄N₆O₂, 492.05; m/z found, 493.0 [M+H]⁺. ¹H NMR (400 MHz, CDCl₃) *δ* 9.79 (brs, 1 H), 8.80 (s, 1 H), 8.39 (d, J = 2.15 Hz, 1 H), 8.10 (d, J = 11.80 Hz, 1 H), 7.74 (d, J = 5.60 Hz, 1 H), 5.17 (s, 2 H), 4.99 - 5.09 (m, 1 H), 2.33 (s, 3 H), 1.70 (d, J = 6.44 Hz, 3 H); ¹⁹F NMR (376 MHz, CDCl₃) *δ* -78.15 ( 3 F), -118.79 (1 F) ppm.

### Example 130: (S)-N-(2-Chloro-6-fluorophenyl)-5-fluoro-4-(2-(2-hydroxypropan-2-yl)-1-methyl-1H-imidazol-4-yl)-2-((1,1,1-trifluoropropan-2-yl)oxy)benzamide.

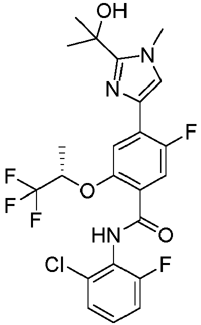

Step A. 2-(4-Bromo-1-methyl-1H-imidazol-2-yl)propan-2-ol. Methylmagnesium bromide 3M in Et₂O (1.8 mL, 5.5 mmol) was added dropwise to a solution of methyl 4-bromo-1-methyl-1H-imidazole-carboxylate (343 mg, 1.56 mmol) in THF (6 mL) under nitrogen at -78°C . The reaction mixture was stirred for 1 hour at -78°C then stirred at room temperature overnight. The reaction mixture was carefully diluted with EtOAc and NH₄Cl solution in water and ice. The layers were separated, and the water layer was extracted one more time with EtOAc. The combined organic layers were once washed with brine, dried over MgSO₄, filtered and concentrated under reduced pressure. The crude product was purified by column chromatography (0-100% heptane/EtOAc) to give the title compound. MS (ESI): mass calcd. for C₇H₁₁BrN₂O, 218.0; m/z found, 219.0 [M+H]⁺. ¹H NMR (400 MHz, Chloroform-*d*) δ 6.77 (s, 1H), 3.82 (s, 3H), 2.22 (q, *J=* 1.2 Hz, 1H), 1.67 (s, 6H) ppm.

Step B. (*S*)-*N*-(2-Chloro-6-fluorophenyl)-5-fluoro-4-(2-(2-hydroxypropan-2-yl)-1-methyl-1H-imidazol-4-yl)-2-((1,1,1-trifluoropropan-2-yl)oxy)benzamide. The title compound was prepared in a manner analogous to Example 88 however using 2-(4-bromo-1-methyl-1H-imidazol-2-yl)propan-2-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₃H₂₁ClF₅N₃O₃, 517.1; m/z found, 518.1 [M+H]⁺. 1H NMR (400 MHz, Chloroform-d) δ 9.14 (s, 1H), 8.02 (d, J = 11.9 Hz, 1H), 7.83 (d, J = 5.7 Hz, 1H), 7.43 (d, J = 4.0 Hz, 1H), 7.32 - 7.18 (m, 2H), 7.12 (m, 1H), 5.10 (m, 1H), 3.89 (s, 3H), 3.04 (s, 1H), 1.73 (s, 6H), 1.66 (d, J = 6.5 Hz, 3H) ppm.

### Example 131: N-(2-Chloro-6-fluorophenyl)-5-fluoro-4-(2-(1-hydroxyethyl)-1-methyl-1H-imidazol-4-yl)-2-(((S)-1,1,1-trifluoropropan-2-yl)oxy)benzamide.

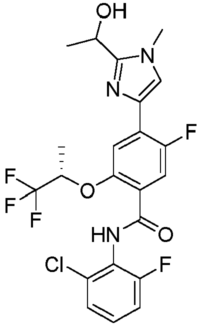

Step A. 4-Bromo-1-methyl-1*H*-imidazole-2-carbaldehyde. To a mixture of methyl 4-bromo-1-methyl-1H-imidazole-2-carboxylate (18 g, 82.2 mmol) in DCM (250 mL) was added DIBALH (1 M in toluene, 150 mL, 150 mmol) at -78°C under N₂. The reaction mixture was stirred at -78 °C for 2 hours. The mixture was slowly added sat. aq. Rochelle salt (100 mL) at -78 °C. The mixture was stirred for 30 minute and then extracted with DCM (100 mL × 3). The organic extracts were dried over Na₂SO₄, filtered and concentrated under reduced pressure. The crude was purified by column chromatography (SiO₂, 0 - 50% ethyl acetate in petroleum ether) to give the title compound as white powder. MS (ESI): mass calcd. for C₅H₅BrN₂O, 188.0; m/z found, 188.8 [M+H]⁺. ¹H NMR (400MHz, CDCl₃) δ = 9.73 (s, 1H), 7.10 (s, 1H), 4.02 (s, 3H) ppm.

Step B. 1-(4-Bromo-1-methyl-1*H*-imidazol-2-yl)ethanol. To a solution of 4-bromo-1-methyl-1H-imidazole-2-carbaldehyde (13.0 g, 68.8 mmol) in THF (200 mL) was added MeMgBr (3 M solution in ether, 60.8 mL, 182.5 mmol) dropwise at 0°C under N₂. The resulting mixture was stirred at room temperature for 1 hour. The mixture was slowly added to sat. aq. NH₄Cl (150 mL) carefully and extracted with DCM (150 mL × 6). The combined organic layers were dried over anhydrous Na₂SO₄, filtered and concentrated under reduced pressure. The crude product was purified by column chromatography (SiO₂, 0 - 100% ethyl acetate in petroleum ether) to give the title compound as light yellow powder. MS (ESI): mass calcd. for C₆H₉BrN₂O, 204.0; m/z found, 207.0 [M+H]⁺. ¹H NMR (400MHz, CDCl₃) δ = 6.75 (s, 1H), 4.88 (q, *J*=6.8 Hz, 1H), 3.70 (s, 4H), 1.58 (d, *J*=6.8 Hz, 3H) ppm.

Step C. *N*-(2-Chloro-6-fluorophenyl)-5-fluoro-4-(2-(1-hydroxyethyl)-1-methyl-1H-imidazol-4-yl)-2-(((*S*)-1,1,1-trifluoropropan-2-yl)oxy)benzamide. The title compound was prepared in a manner analogous to Example 1, Step E however using 1-(4-bromo-1-methyl-1H-imidazol-2-yl)ethan-1-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₂H₁₉ClF₅N₃O₃, 503.1; m/z found, 504.1 [M+H]⁺. 1H NMR (CHLOROFORM-d) Shift: 9.17 (s, 1H), 8.04 (d, J=12.2 Hz, 1H), 7.84 (d, J=5.4 Hz, 1H), 7.45 (d, J=3.9 Hz, 1H), 7.18-7.33 (m, 2H), 7.12 (t, J=8.8 Hz, 1H), 5.10-5.23 (m, 1H), 4.94-5.08 (m, 1H), 3.75 (s, 3H), 2.91 (d, J=6.8 Hz, 1H), 1.63-1.73 (m, 6H) ppm.

### Example 132: (S)-N-(5-Chloro-3-methyl-1H-pyrazol-4-yl)-5-fluoro-4-(2-(2-hydroxypropan-2-yl)-1-methyl-1H-imidazol-4-yl)-2-((1,1,1-trifluoropropan-2-yl)oxy)benzamide.

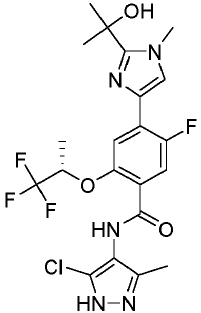

The title compound was prepared in a manner analogous to Example 88 however using 2-(4-bromo-1-methyl-1H-imidazol-2-yl)propan-2-ol (Example 130, Step A) as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. For C₂₁H₂₂ClF₄N₅O₃, 503.1; m/z found, 504.1 [M+H]⁺. 1H NMR (CHLOROFORM-d) Shift: 10.24 (s, 1H), 8.84 (s, 1H), 7.99 (d, J=12.2 Hz, 1H), 7.80 (d, J=5.9 Hz, 1H), 7.43 (d, J=3.9 Hz, 1H), 4.94-5.20 (m, 1H), 3.89 (s, 3H), 3.16 (s, 1H), 2.29 (s, 3H), 1.73 (s, 6H), 1.68 (d, J=6.4 Hz, 3H) ppm.

### Example 133: (S)-5-Fluoro-4-(2-(2-hydroxypropan-2-yl)-1-methyl-1H-imidazol-4-yl)-N-(o-tolyl)-2-((1,1,1-trifluoropropan-2-yl)oxy)benzamide.

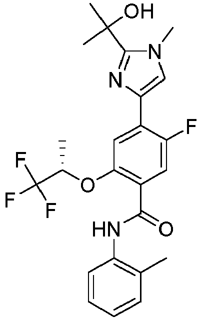

The title compound was prepared in a manner analogous to Example 1, Step E however using 2-(4-bromo-1-methyl-1H-imidazol-2-yl)propan-2-ol (Example 130, Step A) as the aryl halide and (*S*)-5-fluoro-4-(4,4,5,5-tetramethyl-1,3,2-dioxaborolan-2-yl)-*N*-(*o*-tolyl)-2-((1,1,1-trifluoropropan-2-yl)oxy)benzamide (Example 77, Step A) as the boronate. MS (ESI): mass calcd. For C₂₄H₂₅F₄N₃O₃, 479.2; m/z found, 480.1 [M+H]⁺. 1H NMR (CHLOROFORM-d) Shift: 9.16 (s, 1H), 8.01 (d, J=11.7 Hz, 1H), 7.90 (d, J=7.8 Hz, 1H), 7.82 (d, J=5.9 Hz, 1H), 7.43 (d, J=3.9 Hz, 1H), 7.26 (m, 2H), 7.08-7.16 (t, J=7.4 Hz, 1H), 4.96-5.14 (m, 1H), 3.89 (s, 3H), 3.07 (s, 1H), 2.32 (s, 3H), 1.73 (s, 6H), 1.64 (d, J=6.4 Hz, 3H) ppm.

### Example 134: (S)-N-(2-Chloro-6-fluorophenyl)-5-fluoro-4-(5-(2-hydroxypropan-2-yl)-1-methyl-1H-1,2,4-triazol-3-yl)-2-((1,1,1-trifluoropropan-2-yl)oxy)benzamide.

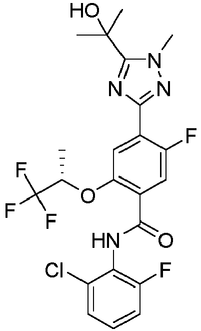

Step A. Mixture of 2-(3-bromo-1-methyl-1H-1,2,4-triazol-5-yl)propan-2-ol and 1-(3-bromo-1-methyl-1H-1,2,4-triazol-5-yl)ethan-1-one. Methylmagnesium bromide 3M in Et₂O (5.2 mL, 15.6 mmol) was added dropwise to a solution of methyl 4-bromo-1-methyl-1H--imidazole-carboxylate (343 mg, 1.56 mmol) in THF (3 mL) under nitrogen at -78 °C. The reaction mixture was stirred for 1 hour at -78°C then stirred at room temperature overnight. The reaction mixture was carefully diluted with EtOAc and NH₄Cl solution in water and ice. The layers were separated, and the water layer was extracted one more time with EtOAc. The combined organic layers were once washed with brine, dried over NaSO₄, filtered and concentrated under reduced pressure to give a mixture of the title compounds (253 mg) which was used without further purification.

Step B. (*S*)-4-(5-Acetyl-1-methyl-1H-1,2,4-triazol-3-yl)-*N*-(2-chloro-6-fluorophenyl)-5-fluoro-2-((1,1,1-trifluoropropan-2-yl)oxy)benzamide. The title compound was prepared in a manner analogous to Example 1, Step E however using the mixture of 1-(3-bromo-1-methyl-1H-1,2,4-triazol-5-yl)ethan-1-one and 2-(3-bromo-1-methyl-1H-1,2,4-triazol-5-yl)propan-2-ol (Step A) as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₆ClF₅N₄O₃, 502.1; m/z found, 503.1 [M+H]⁺.

Step C. (*S*)-*N*-(2-Chloro-6-fluorophenyl)-5-fluoro-4-(5-(2-hydroxypropan-2-yl)-1-methyl-1H-1,2,4-triazol-3-yl)-2-((1,1,1-trifluoropropan-2-yl)oxy)benzamide. To a solution of (*S*)-4-(5-acetyl-1-methyl-1H-1,2,4-triazol-3-yl)-*N*-(2-chloro-6-fluorophenyl)-5-fluoro-2-((1,1,1-trifluoropropan-2-yl)oxy)benzamide (50 mg, 0.1 mmol) in THF (1.0 mL) at -78 °C was added MeMgBr (99 µL, 3 M in ether, 0.3 mmol). The mixture was warmed to room temperature and stirred for 1 h then quenched with saturated aqueous NH₄Cl. The organics were extracted with EtOAc, washed with brine and dried over sodium sulfate. The mixture was filtered and concentrated under reduced pressure. Purification by column chromatography (SiO₂, 30-60% EtOAc/heptane) afforded the title compound as a white solid. MS (ESI): mass calcd. For C₂₂H₂₀ClF₅N₄O₃, 518.1; m/z found, 519.2 [M+H]⁺. 1H NMR (CHLOROFORM-d) Shift: 9.11 (s, 1H), 8.11 (d, J=10.8 Hz, 1H), 7.76 (d, J=5.9 Hz, 1H), 7.19-7.33 (m, 2H), 7.09-7.18 (m, 1H), 5.01-5.15 (m, 1H), 4.17 (s, 3H), 2.42 (s, 1H), 1.77 (s, 6H), 1.67 (d, J=6.8 Hz, 3H) ppm.

### Example 135: N-(2-Chloro-6-fluorophenyl)-5-fluoro-4-(5-(1-hydroxyethyl)-1-methyl-1H-1,2,4-triazol-3-yl)-2-(((S)-1,1,1-trifluoropropan-2-yl)oxy)benzamide.

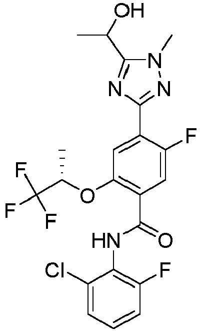

To a solution of (*S*)-4-(5-acetyl-1-methyl-1H-1,2,4-triazol-3-yl)-*N*-(2-chloro-6-fluorophenyl)-5-fluoro-2-((1,1,1-trifluoropropan-2-yl)oxy)benzamide (Example 134, Step B, 61 mg, 0.12 mmol) in EtOH (1.2 mL) at 0 °C was added NaBH₄ (14 mg, 0.36 mmol). The mixture was warmed to room temperature and stirred for 1 h then concentrated. The crude residue was redissolved in EtOAc and water and the organics extracted. The combined organic layers were washed with brine, dried over sodium sulfate, filtered and concentrated. Purification by column chromatography (SiO₂, 50-70% EtOAc/heptane) afforded the title compound as a white solid. MS (ESI): mass calcd. For C₂₁H₁₈ClF₅N₄O₃, 504.1; m/z found, 505.2 [M+H]⁺. 1H NMR (400 MHz, Chloroform-d) δ 9.12 (s, 1H), 8.13 (d, J = 11.2 Hz, 1H), 7.78 (d, J = 5.4 Hz, 1H), 7.33-7.20 (m, 2H), 7.16 - 7.09 (m, 1H), 5.22 - 5.02 (m, 2H), 4.03 (s, 3H), 2.77 (d, J = 6.4 Hz, 1H), 1.70 (d, J = 6.6 Hz, 3H), 1.67 (d, J = 6.5 Hz, 3H) ppm.

### Example 136: N-(5-Chloro-3-methyl-1H-pyrazol-4-yl)-5-fluoro-4-(5-(1-hydroxyethyl)-1-methyl-1H-1,2,4-triazol-3-yl)-2-(((S)-1,1,1-trifluoropropan-2-yl)oxy)benzamide.

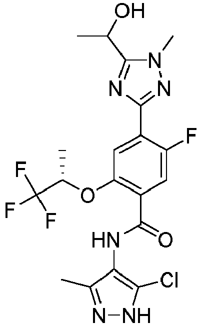

Step A. (*S*)-4-(5-Acetyl-1-methyl-1H-1,2,4-triazol-3-yl)-*N*-(5-chloro-3-methyl-1H-pyrazol-4-yl)-5-fluoro-2-((1,1,1-trifluoropropan-2-yl)oxy)benzamide. The title compound was prepared in a manner analogous to Example 1, Step E however using the mixture of 2-(3-bromo-1-methyl-1H-1,2,4-triazol-5-yl)propan-2-ol and 1-(3-bromo-1-methyl-1H-1,2,4-triazol-5-yl)ethan-1-one (Example 134, Step A) as the aryl halide and (*S*)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. For C₁₉H₁₇ClF₄N₆O₃, 488.1; m/z found, 489.1 [M+H]⁺. 1H NMR (400 MHz, Chloroform-d) δ 9.64 (s, 1H), 8.79 (s, 1H), 8.13 (d, J = 11.2 Hz, 1H), 7.78 (d, J = 5.4 Hz, 1H), 5.06 (m, 1H), 4.30 (s, 3H), 2.81 (s, 3H), 2.33 (s, 3H), 1.71 (d, J = 6.5 Hz, 3H) ppm.

Step B. *N*-(5-Chloro-3-methyl-1H-pyrazol-4-yl)-5-fluoro-4-(5-(1-hydroxyethyl)-1-methyl-1H-1,2,4-triazol-3-yl)-2-(((*S*)-1,1,1-trifluoropropan-2-yl)oxy)benzamide. The title compound was prepared according to the representative procedure of Example 135. MS (ESI): mass calcd. For C₁₉H₁₉ClF₄N₆O₃, 490.1; m/z found, 491.1 [M+H]⁺. 1H NMR (400 MHz, Chloroform-d) δ 9.64 (s, 1H), 8.80 (s, 1H), 8.10 (d, J = 11.3 Hz, 1H), 7.76 (d, J = 5.5 Hz, 1H), 5.14 (m, 1H), 5.06 (m, 1H), 4.05 (s, 3H), 2.48 (d, J = 6.9 Hz, 1H), 2.33 (s, 3H), 1.71 (d, *J* = 6.7 Hz, 3H), 1.69 (d, *J* = 6.5 Hz, 3H) ppm.

### Example 137: (S)-N-(5-Chloro-3-methyl-1H-pyrazol-4-yl)-5-fluoro-4-(5-(2-hydroxypropan-2-yl)-1-methyl-1H-1,2,4-triazol-3-yl)-2-((1,1,1-trifluoropropan-2-yl)oxy)benzamide.

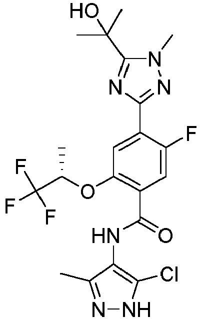

Step A. Methyl 3-bromo-1-methyl-1H-1,2,4-triazole-5-carboxylate. To a solution of methyl 3-bromo-1H-1,2,4-triazole-5-carboxylate (20 g, 97.1 mmol) and K₂CO₃ (26.8 g, 193.9 mmol) in acetone (350 mL) was added dropwise of MeI (29.0 g, 204.6 mmol) at room temperature. Then the resulting mixture was stirred at room temperature for 2 hours. The reaction mixture was poured into H₂O (500 mL), extracted with DCM (300 mL × 2). The combined organic layers was dried over Na₂SO₄, filtered and concentrated under reduced pressure. The crude product was purified by column chromatography (SiO₂, 0 - 20% ethyl acetate in petroleum ether) to give the title compound as a white solid. MS (ESI): mass calcd. for C₅H₆BrN₃O₂, 219.0; m/z found, 220.0 [M+H]⁺. ¹H NMR (400MHz, CDCl₃) δ = 4.24 (s, 3H), 4.02 (s, 3H) ppm.

Step B. 2-(3-Bromo-1-methyl-1H-1,2,4-triazol-5-yl)propan-2-ol. To solution of methyl 3-bromo-1-methyl-1H-1,2,4-triazole-5-carboxylate (8 g, 34.8 mmol) in THF (160 mL) was added MeMgBr (3 M solution in ether, 77.6 mL, 232.7 mmol) dropwise at 0 °C. Then the resulting mixture was stirred at 0 °C for 4 hours. The reaction mixture was slowly poured into sat. aq. NH₄Cl (200 mL), extracted with DCM (200 mL × 2). The combined organic layers was dried over Na₂SO₄, filtered and concentrated under reduced pressure. The crude product was purified by column chromatography (SiO₂, 0 - 50% ethyl acetate in petroleum ether) to give the title compound as a white solid. MS (ESI): mass calcd. for C₆H₁₀BrN₃O, 219.0; m/z found, 220.0 [M+H]⁺. ¹H NMR (400MHz, CDCl₃) δ = 4.05 (s, 3H), 2.25 (s, 1H), 1.69 (s, 6H) ppm.

Step C. (*S*)-*N*-(5-Chloro-3-methvl-1H-pyrazol-4-yl)-5-fluoro-4-(5-(2-hydroxypropan-2-yl)-1-methyl-1H-1,2,4-triazol-3-yl)-2-((1,1,1-trifluoropropan-2-yl)oxy)benzamide. The title compound was prepared according to the representative procedure of Example 88 except substituting 2-(3-bromo-1-methyl-1H-1,2,4-triazol-5-yl)propan-2-ol. MS (ESI): mass calcd. For C₂₀H₂₁ClF₄N₆O₃, 504.1; m/z found, 505.1 [M+H]⁺. 1H NMR (400 MHz, Chloroform-d) δ 10.79 (s, 1H), 8.81 (s, 1H), 8.04 (d, J = 11.2 Hz, 1H), 7.74 (d, J = 5.4 Hz, 1H), 5.04 (m, 1H), 4.16 (s, 3H), 2.80 (s, 1H), 2.29 (s, 3H), 1.76 (s, 6H), 1.69 (d, J = 6.5 Hz, 3H) ppm.

### Example 138: (S)-N-(2-Chloro-6-fluorophenyl)-5-fluoro-4-(4-(2-hydroxypropan-2-yl)-5-(trifluoromethyl)thiazol-2-yl)-2-((1,1,1-trifluoropropan-2-yl)oxy)benzamide.

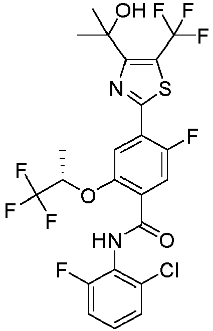

Step A. Methyl 2-amino-5-iodothiazole-4-carboxylate. To a solution of methyl 2-amino-5-iodothiazole-4-carboxylate (1 g, 3.52 mmol) in MeCN (6.6 mL) was added CuCl (523 mg, 5.28 mmol) followed by t-butyl nitrite (837 µL, 7.04 mmol). The mixture was stirred at room temperature overnight. The mixture was concentrated under reduced pressure. The residue was quenched with sat. aq. NH₄Cl (20 mL), extracted with ethyl acetate (15 mL × 3) and the combined extracts were dried over Na₂SO₄, filtered and concentrated under reduced pressure. The crude product was purified by column chromatography (SiO₂, 0 - 10% ethyl acetate in petroleum ether) to give the title compound as white solid. MS (ESI): mass calcd. for C₅H₃ClINO₂S, 302.9; m/z found, 303.9 [M+H]⁺. ¹H NMR (400MHz, CDCl₃) δ = 3.97 (s, 3H) ppm.

Step B. Methyl 2-chloro-5-(trifluoromethvl)thiazole-4-carboxylate. To a solution of methyl 2-amino-5-iodothiazole-4-carboxylate (1 g, 3.15 mmol) and CuI (901 mg, 4.73 mmol) in DMF (20 mL) was added methyl 2,2-difluoro-2-(fluorosulfonyl)acetate (1.20 mL, 9.46 mmol). The resulting solution was stirred at 80°C for 2 hours. The mixture was diluted with ethyl acetate (100 mL) and washed with brine (100 mL × 3). The aqueous layer was extracted with ethyl acetate (100 mL × 3), dried over Na₂SO₄, filtered and concentrated under reduced pressure. The crude product was purified by column chromatography (SiO₂, 0 - 10% ethyl acetate in petroleum ether) to give the title compound as colorless oil. MS (ESI): mass calcd. for C₆H₃ClF₃NO₂S, 245.0; m/z found, 246.0 [M+H]⁺. ¹H NMR (400MHz, CDCl₃) δ = 4.02 (s, 3H); ¹⁹F NMR (376 MHz, CDCl₃) δ = -53.38 ppm.

Step C. 2-(2-Chloro-5-(trifluoromethyl)thiazol-4-yl)propan-2-ol. To a solution of methyl 2-chloro-5-(trifluoromethyl)thiazole-4-carboxylate (900 mg, 3.66 mmol) in THF (39 mL) was added methylmagnesium bromide (3 M solution in ether, 2.4 mL, 7.2 mmol) dropwise at 0°C under N₂. The resulting mixture was stirred at 0°C for 1 hour. The reaction mixture was quenched with sat. aq. NH₄Cl (20 mL) and the organic solvent was removed under vacuum. The resulting mixture was extracted with DCM (20 mL × 3). The combined organic layers were washed with brine (20 mL), dried over Na₂SO₄, filtered and concentrated under reduced pressure. The crude product was purified by column chromatography (SiO₂, 0 - 5% ethyl acetate in petroleum ether) to give the title compound as pale yellow oil. MS (ESI): mass calcd. for C₇H₇ClF₃NOS, 245.0; m/z found, 246.0 [M+H]⁺. ¹H NMR (400MHz, DMSO-*d₆*) δ = 5.52 (s, 1H), 1.48 (s, 6H); ¹⁹F NMR (376MHz, DMSO-*d₆*) δ = -47.31 ppm.

Step D. (*S*)-*N*-(2-Chloro-6-fluorophenyl)-5-fluoro-4-(4-(2-hydroxypropan-2-yl)-5-(trifluoromethyl)thiazol-2-yl)-2-(Y 1,1,1 -trifluoropropan-2-yl)oxy)benzamide. The title compound was prepared in a manner analogous to Example 1, Step E however using 2-(2-chloro-5-(trifluoromethyl)thiazol-4-yl)propan-2-ol as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₃H₁₇ClF₈N₂O₃S, 588.1; m/z found, 589.0 [M+H]⁺. 1H NMR (400 MHz, Chloroform-d) δ 9.07 (s, 1H), 8.20 (d, J = 11.4 Hz, 1H), 7.97 (d, J = 5.3 Hz, 1H), 7.35 - 7.22 (m, 2H), 7.19 - 7.10 (m, 1H), 5.17 - 4.95 (m, 1H), 2.98 (s, 1H), 1.73 (s, 6H), 1.70 (d, J = 6.5 Hz, 3H) ppm.

### Example 139: (S)-5-fluoro-4-(5-(2-hydroxypropan-2-yl)-1-methyl-1H-1,2,4-triazol-3-yl)-N-(o-tolyl)-2-((1,1,1-trifluoropropan-2-yl)oxy)benzamide.

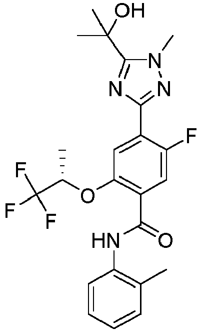

Step A. (*S*)-4-(5-Acetyl-1-methyl-1H-1,2,4-triazol-3-yl)-5-fluoro-*N*-(*o*-tolyl)-2-((1,1,1-trifluoropropan-2-yl)oxy)benzamide. The title compound was prepared in a manner analogous to Example 1, Step E however using the mixture of 2-(3-bromo-1-methyl-1H-1,2,4-triazol-5-yl)propan-2-ol and 1-(3-bromo-1-methyl-1H-1,2,4-triazol-5-yl)ethan-1-one (Example 134, Step A) as the aryl halide and (*S*)-5-fluoro-4-(4,4,5,5-tetramethyl-1,3,2-dioxaborolan-2-yl)-*N*-(*o-*tolyl)-2-((1,1,1-trifluoropropan-2-yl)oxy)benzamide (Example 77, Step A) as the boronate. MS (ESI): mass calcd. For C₂₂H₂₀F₄N₄O₃, 503.1; m/z found, 504.1 [M+H]⁺.

Step B. (*S*)-5-Fluoro-4-(5-(2-hydroxypropan-2-yl)-1-methyl-1H-1,2,4-triazol-3-yl)-*N*-(*o*-tolyl)-2-((1,1,1-trifluoropropan-2-yl)oxy)benzamide. The title compound was prepared according to the representative procedure of Example 134, Step C except substituting (*S*)-4-(5-acetyl-1-methyl-1H-1,2,4-triazol-3-yl)-5-fluoro-*N*-(*o*-tolyl)-2-((1,1,1-trifluoropropan-2-yl)oxy)benzamide. MS (ESI): mass calcd. For C₂₃H₂₄F₄N₄O₃, 480.2; m/z found, 481.0 [M+H]⁺. 1H NMR (400 MHz, Chloroform-d) δ 9.11 (s, 1H), 8.08 (d, J = 11.3 Hz, 1H), 7.90 (d, J = 7.7 Hz, 1H), 7.75 (d, J = 5.4 Hz, 1H), 7.32 - 7.21 (m, 2H), 7.18 - 7.09 (m, 1H), 5.12 - 4.93 (m, 1H), 4.17 (s, 3H), 2.42 (s, 1H), 2.33 (s, 3H), 1.77 (s, 6H), 1.64 (d, J = 6.5 Hz, 3H) ppm.

### Example 140: (S)-N-(5-Chloro-3-methyl-1H-pyrazol-4-yl)-5-fluoro-4-(4-(2-hydroxypropan-2-yl)-5-(trifluoromethyl)thiazol-2-yl)-2-((1,1,1-trifluoropropan-2-yl)oxy)benzamide.

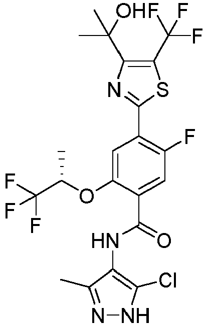

The title compound was prepared in a manner analogous to Example 88 however using 2-(2-chloro-5-(trifluoromethyl)thiazol-4-yl)propan-2-ol (Example 138, Step C) as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. For C₂₁H₁₈ClF₇N₄O₃S, 574.1; m/z found, 575.1 [M+H]⁺. 1H NMR (400 MHz, Chloroform-d) δ 9.95 (s, 1H), 8.77 (s, 1H), 8.17 (d, J = 11.4 Hz, 1H), 7.96 (d, J = 5.4 Hz, 1H), 5.04 (m, 1H), 3.00 (s, 1H), 2.33 (s, 3H), 1.75 - 1.69 (m, 9H) ppm.

### Example 141: (S)-4-(4-((2-Chloro-6-fluorophenyl)carbamoyl)-2-fluoro-5-((1,1,1-trifluoropropan-2-yl)oxy)phenyl)-1-methyl-1H-imidazole-2-carboxamide.

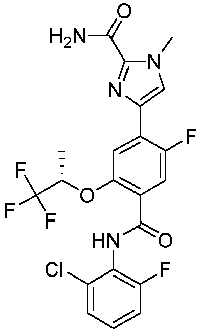

Step A. 4-Bromo-1-methyl-1H-imidazole-2-carboxylic acid. To a solution of methyl 4-bromo-1-methyl-1H-imidazole-2-carboxylate (450 mg, 2.1 mmol) in dioxane was added LiOH (246 mg, 10.3 mmol) was added and the mixture stirred at room temperature for 2h. The solution was acidified with HCl and extracted with EtOAc. The organic layers were dried over sodium sulfate, filtered and concentrated under reduced pressure to give the title compound (383 mg, 91%) which was used without further purification for the next step. MS (ESI): mass calcd. for C₅H₅BrN₂O₂, 203.9; m/z found, 204.9 [M+H]⁺.

Step B. 4-Bromo-1-methyl-1H-imidazole-2-carboxamide. To a solution of 4-bromo-1-methyl-1H-imidazole-2-carboxylic acid (227 mg, 1.1 mmol) and HATU (505 mg, 1.3 mmol) in DMF (2.1 mL) was added Et₃N (1.5 mL, 0.728 g/mL, 11.1 mmol) followed by NH₄Cl (592 mg, 11.1 mmol). The reaction was stirred for 72 h at room temperature then diluted with EtOAc and water. The organics were extracted with EtOAc (3x), washed with brine, dried over sodium sulfate, filtered and concentrated. Purification by column chromatography (SiO₂, 10-40% EtOAc/heptane) afforded the title compound as a white solid. MS (ESI): mass calcd. For C₅H₆BrN₃O, 203.0; m/z found, 206.0 [M+H]⁺.

Step C. (*S*)-4-(4-((2-Chloro-6-fluorophenyl)carbamoyl)-2-fluoro-5-((1,1,1-trifluoropropan-2-yl)oxy)phenyl)-1-methyl-1H-imidazole-2-carboxamide. The title compound was prepared in a manner analogous to Example 1, Step E however using 4-bromo-1-methyl-1H-imidazole-2-carboxamide as the aryl halide and (S)-N-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. For C₂₁H₁₆ClF₅N₄O₃, 502.1; m/z found, 503.1 [M+H]⁺.1H NMR (400 MHz, Chloroform-d) δ 9.13 (s, 1H), 8.08 (d, J = 11.7 Hz, 1H), 7.82 (d, J = 5.6 Hz, 1H), 7.60 (d, J = 3.8 Hz, 1H), 7.35 - 7.20 (m, 3H), 7.16 - 7.07 (m, 1H), 5.45 (s, 1H), 5.18 - 4.99 (m, 1H), 4.13 (s, 3H), 1.68 (d, J = 6.5 Hz, 3H) ppm.

### Example 142: (S)-4-(4-((5-chloro-3-methyl-1H-pyrazol-4-yl)carbamoyl)-2-fluoro-5-((1,1,1-trifluoropropan-2-yl)oxy)phenyl)-1-methyl-1H-imidazole-2-carboxamide.

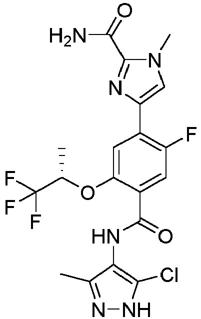

The title compound was prepared in a manner analogous to Example 88 however using 4-bromo-1-methyl-1H-imidazole-2-carboxamide (Example 141, Step B) as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. For C₁₉H₁₇ClF₄N₆O₃, 488.1; m/z found, 489.1 [M+H]⁺. 1H NMR (400 MHz, Chloroform-d) δ 9.76 (s, 1H), 8.82 (s, 1H), 8.04 (d, J = 11.8 Hz, 1H), 7.80 (d, J = 5.6 Hz, 1H), 7.59 (d, J = 3.8 Hz, 1H), 7.28 (br s, 1H), 5.48 (br s, 1H), 5.08 (m, 1H), 4.13 (s, 3H), 2.32 (s, 3H), 1.70 (d, J = 6.5 Hz, 3H) ppm.

### Example 143: 5-Fluoro-4-(2-(1-hydroxyethyl)-1-methyl-1H-imidazol-4-yl)-N-(o-tolyl)-2-(((S)-1,1,1-trifluoropropan-2-yl)oxy)benzamide.

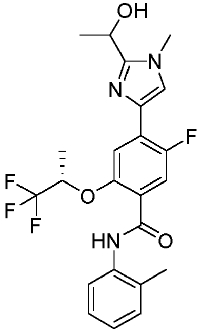

The title compound was prepared in a manner analogous to Example 88 however using 1-(4-bromo-1-methyl-1H-imidazol-2-yl)ethan-1-ol (Example 131, Step B) as the aryl halide and (5)-5-fluoro-4-(4,4,5,5-tetramethyl-1,3,2-dioxaborolan-2-yl)-N (o-tolyl)-2-((1,1,1-trifluoropropan-2-yl)oxy)benzamide (Example 77, step A) as the boronate. MS (ESI): mass calcd. For C₂₃H₂₃F₄N₃O₃, 465.2; m/z found, 466.1 [M+H]⁺. 1H NMR (400 MHz, Chloroform-d) δ 9.17 (s, 1H), 8.02 (d, J = 12.0 Hz, 1H), 7.90 (d, J = 7.9 Hz, 1H), 7.82 (d, J = 5.7 Hz, 1H), 7.43 (d, J = 3.8 Hz, 1H), 7.34 - 7.21 (m, 2H), 7.20 - 7.05 (m, 1H), 5.22 - 5.06 (m, 1H), 5.05 - 4.93 (m, 1H), 3.73 (d, J = 3.3 Hz, 3H), 3.16 - 3.00 (m, 1H), 2.33 (s, 3H), 1.77 - 1.61 (m, 6H) ppm.

### Example 144: 5-Fluoro-4-(5-(1-hydroxyethyl)-1-methyl-1H-1,2,4-triazol-3-yl)-N-(o-tolyl)-2-(((S)-1,1,1-trifluoropropan-2-yl)oxy)benzamide.

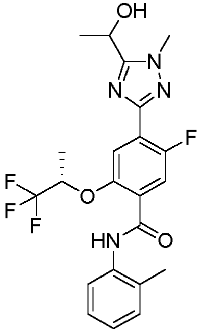

Step A. 3-Bromo-1-methyl-1*H*-1,2,4-triazole-5-carbaldehyde. At -70°C, and under an atmosphere of nitrogen, DIBAL-H (1 M solution in toluene, 95.3 mL, 95.3 mmol) was added dropwise to a mixture of methyl 3-bromo-1-methyl-1*H* 1,2,4- triazole-5-carboxylate (10.5 g, 47.7 mmol) in DCM (160 mL). The reaction mixture was stirred at -70°C for 1 hour. The reaction mixture was quenched by added sat. aq. Rochelle salt (100 mL), the mixture was stirred for 1 hour. Then extracted with DCM (100 mL × 8). The combined organic layers was dried over Na₂SO₄, filtered and concentrated under reduced pressure. The crude was purified by column chromatography (SiO₂, 0 - 20% ethyl acetate/petroleum ether) to give the title compound as colorless oil. ¹H NMR (400MHz, CDCl₃) δ = 9.92 - 9.86 (m, 1H), 4.22 - 4.16 (m, 3H) ppm.

Step B. 1-(3-Bromo-1-methyl-1*H*-1,2,4-triazol-5-yl)ethanol. To a solution of 3-bromo-1-methyl-1*H*-1,2,4-triazole-5-carbaldehyde (7 g, 36.8 mmol) in THF (160 mL) was cooled to 0°C, MeMgBr (3 M solution in ether, 36.84 mL, 110.5 mmol) was added dropwise. Then the resulting mixture was stirred at room temperature for 1 hour. The reaction mixture was slowly poured into sat. aq. NH₄Cl (100 mL), extracted with DCM (100 mL × 2). The combined organic layers was dried over Na₂SO₄, filtered and concentrated under reduced pressure. The crude was purified by column chromatography (SiO₂, 0 - 60% ethyl acetate/petroleum ether) to give the title compound as colorless oil. MS (ESI): mass calcd. for C₅H₈BrN₃O, 205.0; m/z found, 206.0 [M+H]⁺. ¹H NMR (400MHz, CDCl₃) δ = 5.01 (br t, J=6.5 Hz, 1H), 3.93 (s, 3H), 2.55 (br d, J=7.3 Hz, 1H), 1.65 (d, J=6.5 Hz, 3H) ppm.

Step C. 5-Fluoro-4-(5-(1-hydroxyethyl)-1-methyl-1H-1,2,4-triazol-3-yl)-*N*-(*o*-tolyl)-2-(((*S*)-1,1,1-trifluoropropan-2-yl)oxy)benzamide. The title compound was prepared in a manner analogous to Example 88 however using 1-(3-bromo-1-methyl-1H-1,2,4-triazol-5-yl)ethan-1-ol as the aryl halide and (*S*)-5-fluoro-4-(4,4,5,5-tetramethyl-1,3,2-dioxaborolan-2-yl)-*N*-(*o*-tolyl)-2-((1,1,1-trifluoropropan-2-yl)oxy)benzamide (Example 77, step A) as the boronate. MS (ESI): mass calcd. For C₂₂H₂₂F₄N₄O₃, 466.2; m/z found, 467.3 [M+H]⁺. 1H NMR (400 MHz, Chloroform-d) δ 9.11 (s, 1H), 8.10 (d, J = 11.3 Hz, 1H), 7.90 (d, J = 7.9 Hz, 1H), 7.77 (d, J = 5.5 Hz, 1H), 7.26 (m, 2H), 7.18 -7.11 (m, 1H), 5.23 - 5.10 (m, 1H), 5.10 - 4.97 (m, 1H), 4.04 (s, 3H), 2.61 (d, J = 6.8 Hz, 1H), 2.33 (s, 3H), 1.71 (d, J = 6.6 Hz, 3H), 1.65 (d, J = 6.5 Hz, 3H) ppm.

### Example 145: N-(5-Chloro-3-methyl-1H-pyrazol-4-yl)-5-fluoro-4-(2-(1-hydroxyethyl)-1-methyl-1H-imidazol-4-yl)-2-(((S)-1,1,1-trifluoropropan-2-yl)oxy)benzamide.

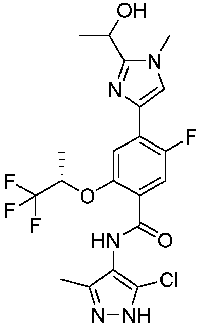

The title compound was prepared in a manner analogous to Example 88 however using 1-(3-bromo-1-methyl-1H-1,2,4-triazol-5-yl)ethan-1-ol (Example 131, Step B) as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. For C₂₀H₂₀ClF₄N₅O₃, 489.1; m/z found, 490.0 [M+H]⁺. 1H NMR (400 MHz, Chloroform-d) δ 9.87 (s, 1H), 8.84 (s, 1H), 8.00 (d, J = 11.9 Hz, 1H), 7.82 (d, J = 5.7 Hz, 1H), 7.45 (d, J = 3.9 Hz, 1H), 5.18 - 5.07 (m, 1H), 5.07 - 4.96 (m, 1H), 3.76 (s, 3H), 2.91 (d, J = 6.3 Hz, 1H), 2.31 (s, 3H), 1.68 (dd, J = 6.6, 1.2 Hz, 6H) ppm.

### Example 146: (S)-5-Fluoro-4-(2-(hydroxymethyl)-1-methyl-1H-imidazol-4-yl)-N-(o-tolyl)-2-((1,1,1-trifluoropropan-2-yl)oxy)benzamide.

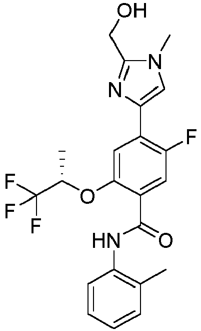

The title compound was prepared in a manner analogous to Example 88 however using (4-bromo-1-methyl-1H-imidazol-2-yl)methanol (Example 5, Step A) as the aryl halide and (*S*)-5-fluoro-4-(4,4,5,5-tetramethyl-1,3,2-dioxaborolan-2-yl)-*N*-(*o*-tolyl)-2-((1,1,1-trifluoropropan-2-yl)oxy)benzamide (Example 77, Step A) as the boronate. MS (ESI): mass calcd. For C₂₂H₂₁F₄N₃O₃, 451.2; m/z found, 452.1 [M+H]⁺. 1H NMR (400 MHz, Methanol-d4) δ 7.92 (d, J = 5.9 Hz, 1H), 7.70 (d, J = 11.6 Hz, 1H), 7.64 (d, J = 4.0 Hz, 1H), 7.58 (dd, J = 7.9, 1.4 Hz, 1H), 7.31 - 7.13 (m, 3H), 5.31 (m, 1H), 4.74 (s, 2H), 3.83 (s, 3H), 2.32 (s, 3H), 1.64 (d, J = 6.4 Hz, 3H) ppm.

### Example 147: (S)-5-Fluoro-4-(2-(2-hydroxypropan-2-yl)-1-methyl-1H-imidazol-4-yl)-N-(5-methyl-3-(trifluoromethyl)-1H-pyrazol-4-yl)-2-((1,1,1-trifluoropropan-2-yl)oxy)benzamide.

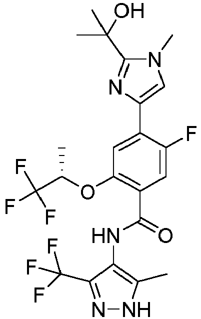

The title compound was prepared in a manner analogous to Example 88 however using 2-(4-bromo-1-methyl-1H-imidazol-2-yl)propan-2-ol (Example 130, Step A) as the aryl halide and (*S*)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₂H₂₂F₇N₅O₃, 537.2; m/z found, 538.2 [M+H]⁺. 1H NMR (400 MHz, Methanol-d4) δ 7.92 (d, J = 5.9 Hz, 1H), 7.63 (d, J = 11.5 Hz, 1H), 7.55 (d, J = 4.1 Hz, 1H), 5.40 - 5.27 (m, 1H), 3.96 (s, 3H), 2.26 (s, 3H), 1.70 (s, 6H), 1.64 - 1.59 (d, 3H) ppm.

### Example 148: (S)-5-fluoro-4-(5-(2-hydroxypropan-2-yl)-1-methyl-1H-1,2,4-triazol-3-yl)-N-(3-methyl-5-(trifluoromethyl)-1H-pyrazol-4-yl)-2-((1,1,1-trifluoropropan-2-yl)oxy)benzamide.

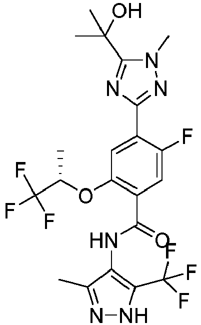

The title compound was prepared in a manner analogous to Example 88 however using 2-(3-bromo-1-methyl-1H-1,2,4-triazol-5-yl)propan-2-ol (Example 137, Step B) as the aryl halide and (*S*)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₁H₂₁F₇N₆O₃, 538.2; m/z found, 539.3 [M+H]⁺. 1H NMR (400 MHz, Chloroform-d) δ 10.80 (s, 1H), 8.93 (s, 1H), 8.08 (d, J = 11.2 Hz, 1H), 7.74 (d, J = 5.4 Hz, 1H), 5.11 - 4.98 (m, 1H), 4.17 (s, 3H), 2.56 (s, 1H), 2.26 (s, 3H), 1.77 (s, 6H), 1.66 (d, J = 6.4 Hz, 3H) ppm.

### Example 149: (S)-N-(5-Chloro-3-methyl-1H-pyrazol-4-yl)-4-(1-cyclopropyl-5-(2-hydroxypropan-2-yl)-1H-pyrazol-3-yl)-5-fluoro-2-((1,1,1-trifluoropropan-2-yl)oxy)benzamide.

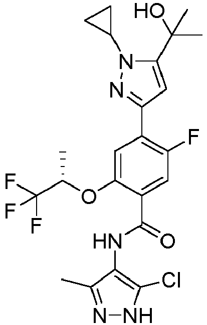

Step A. Ethyl 3-bromo-1-cyclopropyl-1H-pyrazole-5-carboxylate. To a mixture of ethyl 3-bromo-1H-pyrazole-5-carboxylate (8 g, 36.5 mmol), cyclopropylboronic acid (7.84 g, 91.3 mmol) and Na₂CO₃ (9.7 g, 91.3 mmol) in DCE (125 mL) at 70°C was added a heated suspension of 2,2'-bipyridine (5.70 g, 36.5 mmol) and Cu(OAc)₂ (6.63 g, 36.5 mmol) in DCE (25 mL). The reaction mixture was then stirred vigorously under O₂ (15 psi) at 70°C overnight. The reaction mixture was cooled to room temperature, filtered through a pad of celite, and the solid was rinsed with DCM (100 mL). The filtrate was concentrated under reduced pressure and purified by column chromatography (SiO₂, 0 - 20% ethyl acetate/petroleum ether) to give the title compound as yellow oil. MS (ESI): mass calcd. for C₉H₁₁BrN₂O₂, 258.0; m/z found, 259.0 [M+H]⁺. ¹H NMR (400MHz, CDCl₃) δ = 6.82 (s, 1H), 4.37 (q, *J*=7.2 Hz, 2H), 4.27 (tt, J=3.8, 7.5 Hz, 1H), 1.39 (t, *J*=7.1 Hz, 3H), 1.30 - 1.26 (m, 2H), 1.09 - 1.02 (m, 2H) ppm.

Step B. 2-(3-Bromo-1-cyclopropyl-1H-pyrazol-5-yl)propan-2-ol. To a solution of ethyl 3-bromo-1-cyclopropyl-1H-pyrazole-5-carboxylate (1.5 g, 5.8 mmol) in THF (44 mL) was added MeMgBr (3 M solution in ether, 8.5 mL, 25.5 mmol) dropwise at 0°C under N₂. The resulting mixture was stirred at 0°C for 1 hour. The reaction mixture was quenched with sat. aq. NH₄Cl (10 mL) at 0°C. THF was evaporated under vacuum, the aqueous layer was extracted with DCM (50 mL × 3). The combined organic layers were dried over Na₂SO₄, filtered and concentrated under reduced pressure at room temperature. The residue was purified by preparative reverse phase HPLC (Stationary phase: Xtimate C18, 5 µm, 150 × 40 mm; Mobile phase: H₂O (0.05% NH₃H₂O) (A) - MeCN (B), gradient elution: 30 - 60% B in A over 9 min, flow rate: 60 mL/min) to give the title compound as white powder. MS (ESI): mass calcd. for C₉H₁₃BrN₂O, 244.0; m/z found, 245.0, 246.9 [M+H]⁺. ¹H NMR (400MHz, CDCl₃) δ = 6.11 (s, 1H), 4.10 (tt, *J*=3.9, 7.6 Hz, 1H), 1.85 (s, 1H), 1.70 (s, 6H), 1.40 - 1.32 (m, 2H), 1.04 - 0.95 (m, 2H) ppm.

Step C. (*S*)-*N*-(5-chloro-3-methyl-1H-pyrazol-4-yl)-4-(1-cyclopropyl-5-(2-hydroxypropan-2-yl)-1H-pyrazol-3-yl)-5-fluoro-2-((1,1,1-trifluoropropan-2-yl)oxy)benzamide. The title compound was prepared in a manner analogous to Example 88 however using 2-(3-bromo-1-cyclopropyl-1H-pyrazol-5-yl)propan-2-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₃H₂₄ClF₄N₅O₃, 529.2; m/z found, 530.1 [M+H]⁺. 1H NMR (400 MHz, Chloroform-d) δ 9.86 (s, 1H), 8.80 (s, 1H), 8.01 (d, J = 11.8 Hz, 1H), 7.68 (d, J = 5.7 Hz, 1H), 6.67 (d, J = 4.1 Hz, 1H), 5.07 - 4.96 (m, 1H), 4.31 -4.23 (m, 1H), 2.30 (s, 3H), 1.94 (s, 1H), 1.78 (s, 6H), 1.67 (d, J = 6.5 Hz, 3H), 1.51 - 1.44 (m, 2H), 1.11 - 1.03 (m, 2H) ppm.

### Example 150: N-(5-Chloro-3-methyl-1H-pyrazol-4-yl)-5-fluoro-4-(1-methyl-2-(2,2,2-trifluoro-1-hydroxyethyl)-1H-imidazol-4-yl)-2-(((S)-1,1,1-trifluoropropan-2-yl)oxy)benzamide.

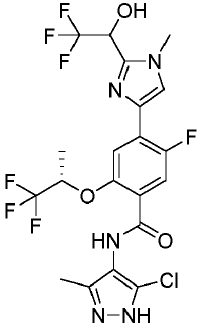

Step A. 1-(4-Bromo-1-methyl-1H-imidazol-2-yl)-2,2,2-trifluoroethan-1-ol. To a solution of 4-bromo-1-methyl-1*H*-imidazole-2-carbaldehyde (Example 131, Step A) (100 mg, 0.53 mmol) in THF (0.8 mL) was added CsF (80 mg, 0.53 mmol). The mixture was cooled to 0 °C then (trifluoromethyl)trimethylsilane (94 µL, 0.962 g/mL, 0.64 mmol) was added. The reaction mixture was stirred at room temperature overnight. Water and 1N HCl were added then the organics extracted with EtOAc. The combined organics were dried over sodium sulfate, filtered and concentrated to give the title compound as a white solid that was used without further purification (136 mg).

Step B. *N*-(5-Chloro-3-methyl-1H-pyrazol-4-yl)-5-fluoro-4-(1-methyl-2-(2,2,2-trifluoro-1-hydroxyethyl)-1H-imidazol-4-yl)-2-(((*S*)-1,1,1-trifluoropropan-2-yl)oxy)benzamide. The title compound was prepared according to the representative procedure of Example 88 except substituting 1-(4-bromo-1-methyl-1H-imidazol-2-yl)-2,2,2-trifluoroethan-1-ol as the aryl halide. MS (ESI): mass calcd. for C₂₀H₁₇ClF₇N₅O₃, 543.1; m/z found, 544.0 [M+H]⁺. 1H NMR (400 MHz, Chloroform-d) δ 9.65 (s, 1H), 8.82 (s, 1H), 8.02 (d, J = 11.9 Hz, 1H), 7.78 (dd, J = 5.7, 3.5 Hz, 1H), 7.55 (d, J = 3.8 Hz, 1H), 5.23 - 5.02 (m, 2H), 4.14 (d, J = 8.0 Hz, 1H), 3.78 (s, 3H), 2.31 (s, 3H), 1.69 (d, J = 6.5 Hz, 3H) ppm.

### Example 151: N-(2-Chloro-6-fluorophenyl)-5-fluoro-4-(5-((*R)-1-hydroxyethyl)-1-methyl-1H-pyrazol-3-yl)-2-(((S)-1,1,1-trifluoropropan-2-yl)oxy)benzamide.

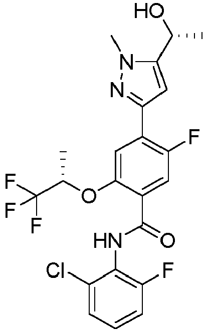

Step A. Methyl 3-bromo-1-methyl-1*H*-pyrazole-5-carboxylate. A mixture of 3-bromo-1-methyl-1H-pyrazole-5-carboxylic acid (3.2 g, 15.6 mmol) and K₂CO₃ (4.3 g, 31.1 mmol) in acetone (45 mL) was stirred at room temperature for 15 minutes under N₂. Mel (2.7 g, 19 mmol) in acetone (2.7 mL) was added to reaction mixture. The mixture was stirred at room temperature for 12 hours. The mixture was filtered. The filter cake was washed with ethyl acetate (50 mL × 3). The filtrate was concentrated under reduced pressure. The crude product was purified by column chromatography (SiO₂, 0 - 2% ethyl acetate/petroleum ether) to give the title compound as colorless crystal. MS (ESI): mass calcd. for C₆H₇BrN₂O₂, 218.0; m/z found, 219.0 [M+H]⁺. ¹H NMR (400MHz, CDCl₃) δ = 6.82 (s, 1H), 4.16 (s, 3H), 3.90 (s, 3H) ppm.

Step B. (3-Bromo-1-methyl-1*H*-pyrazol-5-yl)methanol. NaBH₄ (111 mg, 2.93 mmol) and CaCl₂ (162 mg, 1.46 mmol) were added to the mixture of methyl 3-bromo-1-methyl-1*H*-pyrazole-5-carboxylate (534 mg, 2.44 mmol) in EtOH (8 mL) at 0°C under N₂. The mixture was stirred at room temperature for 12 hours. EtOH was removed under reduced pressure. The residue was dissolved in sat. aq. NH₄Cl (60 mL). The mixture extracted with ethyl acetate (50 mL × 3). The organic layer was separated, dried over Na₂SO₄, filtered and evaporated under reduced pressure. The crude was purified by preparative reversed phase HPLC (Stationary phase: Boston Prime C18, 5 µm, 150 × 30 mm; Mobile phase: water (0.05%NH₃H₂O + 10mM NH₄HCO₃) (A)-MeCN (B), gradient elution: 20 - 50% B in A over 7 min, flow rate: 25 mL/min) to give the title compound as white powder. MS (ESI): mass calcd. for C₅H₇BrN₂O, 190.0; m/z found, 191.0, 192.9 [M+H]⁺. ¹H NMR (400MHz, DMSO-*d₆*) δ= 6.27 (s, 1H), 5.36 (t, *J*=5.5 Hz, 1H), 4.46 (d, *J*=5.5 Hz, 2H), 3.75 (s, 3H) ppm.

Step C. 3-Bromo-1-methyl-1*H*-pyrazole-5-carbaldehyde. To a mixture of (3-bromo-1-methyl-1*H*-pyrazol-5-yl)methanol (513 mg, 2.69 mmol) in DCM (30 mL) was added MnO₂ (3.5 g, 40.28 mmol). The reaction mixture was stirred at room temperature for 32 hours. The mixture was filtered over a pad of celite. The solid was rinsed with DCM (50 mL × 3), the filtrate was concentrated under reduced pressure to give the title compound as yellow solid. MS (ESI): mass calcd. for C₅H₅BrN₂O, 188.0; m/z found, 191.0 [M+H]⁺. ¹H NMR (400 MHz, CDCl₃) δ = 9.81 (s, 1H), 6.89 (s, 1H), 4.16 (s, 3H) ppm.

Step D. 1-(3-Bromo-1-methyl-1*H*-pyrazol-5-yl)ethanol. To a solution of 3-bromo-1-methyl-1*H-*pyrazole-5-carbaldehyde (481 mg, 2.55 mmol) in THF (15 mL) was added MeMgBr (3 M solution in ether, 3.39 mL, 10.17 mmol) dropwise at 0°C under N₂. The resulting mixture was stirred at 0°C for 1 hour. Sat. aq. NH₄Cl (20 mL) was added to the mixture dropwise at 0°C under N₂. The mixture was extracted with EtOAc (35 mL × 3). The combined organic layers were separated, washed with brine (60 mL), dried over Na₂SO₄, filtered and concentrated under reduced pressure to give the title compound as pale yellow oil. MS (ESI): mass calcd. for C₆H₉BrN₂O, 204.0; m/z found, 205.1, 207.0 [M+H]⁺. ¹H NMR (400MHz, CDCl₃) δ = 6.20 (s, 1H), 4.90 (q, *J*=6.2 Hz, 1H), 3.94 - 3.82 (m, 3H), 1.59 (d, *J*=6.6 Hz, 3H) ppm.

Step E. (**S*)-1-(3-Bromo-1-methyl-1*H*-pyrazol-5-yl)ethanol and (**R*)-1-(3-bromo-1-methyl-1*H-*pyrazol-5-yl)ethanol. 1-(3-Bromo-1-methyl-1*H*-pyrazol-5-yl)ethanol (600 mg, 2.93 mmol) was separated by SFC (Stationary phase: DAICEL CHIRALPAK AD-H, 5 µm 250mm × 30mm); Mobile phase: Supercritical CO₂ (A) - EtOH (0.1% NH₃.H₂O) (B), gradient elution: 15% B in A at 60 mL/min) to give:
The first eluting compound (**S*)-1-(3-Bromo-1-methyl-1*H*-pyrazol-5-yl)ethanol_as yellow oil. MS (ESI): mass calcd. for C₆H₉BrN₂O, 204.0; m/z found, 205.0, 206.9 [M+H]⁺. ¹H NMR (400MHz, DMSO-*d₆*) δ = 6.25 (s, 1H), 5.39 (d, *J=5.5* Hz, 1H), 4.78 (quin, *J*=6.2 Hz, 1H), 3.77 (s, 3H), 1.38 (d, *J*=6.6 Hz, 3H) ppm. SFC: purity 99.71%, retention time 2.769 min; method: AD-3_EtOH(DEA)_5_40_2,5ML_7MIN.

The second eluting compound (**R*)-1-(3-bromo-1-methyl-1*H*-pyrazol-5-yl)ethanol_as yellow oil. MS (ESI): mass calcd. for C₆H₉BrN₂O, 204.0; m/z found, 205.0, 206.9 [M+H]⁺. ¹H NMR (400MHz, DMSO-*d₆*) δ = 6.25 (s, 1H), 5.39 (d, *J*=5.6 Hz, 1H), 4.78 (quin, *J*=6.3 Hz, 1H), 3.77 (s, 3H), 1.38 (d, *J*=6.6 Hz, 3H) ppm. SFC: purity 99.37%, retention time 2.943 min; method: AD-3_EtOH(DEA)_5_40_2,5ML_7MIN.

Step F. *N*-(2-Chloro-6-fluorophenyl)-5-fluoro-4-(5-((**R*)-1-hydroxyethyl)-1-methyl-1H-pyrazol-3-yl)-2-(((*S*)-1,1,1-trifluoropropan-2-yl)oxy)benzamide. The title compound was prepared in a manner analogous to Example 88 however using (**R*)-1-(3-bromo-1-methyl-1H-pyrazol-5-yl)ethan-1-ol as the aryl halide and (**R*)-1-(3-bromo-1-methyl-1H-pyrazol-5-yl)ethan-1-ol and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₂H₁₉ClF₅N₃O₃, 503.1; m/z found, 504.1 [M+H]⁺. 1H NMR (400 MHz, Chloroform-d) δ 9.14 (s, 1H), 8.06 (d, J = 11.7 Hz, 1H), 7.73 (d, J = 5.6 Hz, 1H), 7.32 - 7.19 (m, 2H), 7.16-7.06 (m, 1H), 6.75 (d, 1H), 5.17 - 5.03 (m, 1H), 5.03 - 4.92 (m, 1H), 4.02 (s, 3H), 1.80 (d, J = 6.5 Hz, 1H), 1.72 - 1.63 (m, 6H) ppm.

### Example 152: N-(2-Chloro-6-fluorophenyl)-5-fluoro-4-(5-((*S)-1-hydroxyethyl)-1-methyl-1H-pyrazol-3-yl)-2-(((S)-1,1,1-trifluoropropan-2-yl)oxy)benzamide.

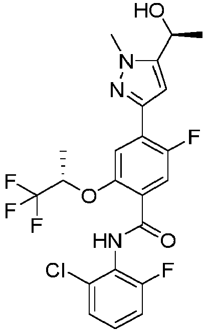

*N*-(2-Chloro-6-fluorophenyl)-5-fluoro-4-(5-((**S*)-1-hydroxyethyl)-1-methyl-1H-pyrazol-3-yl)-2-(((*S*)-1,1,1-trifluoropropan-2-yl)oxy)benzamide. The title compound was prepared in a manner analogous to Example 88 however using (**S*)-1-(3-bromo-1-methyl-1H-pyrazol-5-yl)ethan-1-ol (Example 151, Step E) as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₂H₁₉ClF₅N₃O₃, 503.1; m/z found, 504.1 [M+H]⁺. 1H NMR (400 MHz, Chloroform-d) δ 9.13 (s, 1H), 8.07 (d, J = 11.7 Hz, 1H), 7.73 (d, J = 5.6 Hz, 1H), 7.33 - 7.19 (m, 2H), 7.16 - 7.09 (m, 1H), 6.75 (d, 1H), 5.15 - 5.04 (m, 1H), 5.02 - 4.94 (m, 1H), 4.03 (s, 3H), 1.72 (d, J = 6.4 Hz, 1H), 1.70 - 1.64 (m, 6H) ppm.

### Example 153: 5-Fluoro-4-(2-((*S)-1-hydroxyethyl)-1-methyl-1H-imidazol-4-yl)-N-(3-methyl-5-(trifluoromethyl)-1H-pyrazol-4-yl)-2-(((S)-1,1,1-trifluoropropan-2-yl)oxy)benzamide.

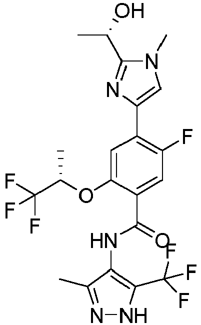

Step A. (**S*)-1-(4-Bromo-1-methyl-1*H*-imidazol-2-yl)ethanol and (**R*)-1-(4-bromo-1-methyl-1*H*-imidazol-2-yl)ethanol. 1-(4-bromo-1-methyl-1*H*-imidazol-2-yl)ethanol (Example 131, Step B) (8 g, 39.01 mmol) was separated by SFC (Stationary phase: DAICEL CHIRALPAK AD-H, 5 µm, 250mm × 30mm); Mobile phase: Supercritical CO₂ (A) - EtOH (0.1% NH₃.H₂O) (B), gradient elution: 15% B in A at 60 mL/min) to give:
The first eluting compound (**S*)-1-(4-bromo-1-methyl-1*H*-imidazol-2-yl)ethanol_as white powder. MS (ESI): mass calcd. for C₆H₉BrN₂O, 204.0; m/z found, 207.0 [M+H]⁺. ¹H NMR (400MHz, CDCl₃) δ = 6.78 (s, 1H), 4.89 (q, *J*=6.6 Hz, 1H), 3.70 (s, 3H), 3.10 (br s, 1H), 1.61 (d, J=6.5 Hz, 3H) ppm. SFC: purity 99.85%, retention time 2.512 min.

The second eluting compound (*R)-1-(4-bromo-1-methyl-1H-imidazol-2-yl)ethan-1-ol as white powder. MS (ESI): mass calcd. for C₆H₉BrN₂O, 204.0; m/z found, 205.0 [M+H]⁺. ¹H NMR (400MHz, CDCl₃) δ = 6.77 (s, 1H), 4.89 (q, *J*=6.6 Hz, 1H), 3.70 (s, 3H), 3.36 (br s, 1H), 1.60 (d, *J*=6.7 Hz, 3H) ppm. SFC: purity 99.82%, retention time 2.72 min.

Step B. 5-Fluoro-4-(2-((**S*)-1-hydroxyethyl)-1-methyl-1H-imidazol-4-yl)-*N*-(3-methyl-5-(trifluoromethyl)-1H-pyrazol-4-yl)-2-(((*S*)-1,1,1-trifluoropropan-2-yl)oxy)benzamide. The title compound was prepared in a manner analogous to Example 88 however using (**S*)-1-(4-bromo-1-methyl-1H-imidazol-2-yl)ethan-1-ol as the aryl halide and (*S*)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate. MS (ESI): mass calcd. for C₂₁H₂₀F₇N₅O₃, 523.1; m/z found, 524.1 [M+H]⁺. 1H NMR (400 MHz, Chloroform-d) δ 10.20 (s, 1H), 8.97 (s, 1H), 8.01 (d, J = 11.9 Hz, 1H), 7.81 (d, J = 5.7 Hz, 1H), 7.46 (d, J = 4.0 Hz, 1H), 5.18 - 5.07 (m, 1H), 5.06-4.96 (m, 1H), 3.76 (s, 3H), 2.83 (d, J = 6.6 Hz, 1H), 2.30 (s, 3H), 1.68 (d, J = 6.6 Hz, 3H), 1.67-1.64 (m, 3H) ppm.

### Example 154: (S)-4-(2-Fluoro-4-((3-methyl-5-(trifluoromethyl)-1H-pyrazol-4-yl)carbamoyl)-5-((1,1,1-trifluoropropan-2-yl)oxy)phenyl)-1-methyl-1H-imidazole-2-carboxamide.

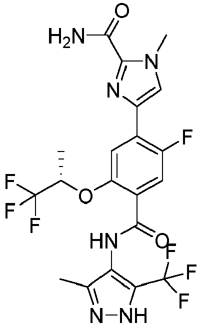

The title compound was prepared in a manner analogous to Example 88 however using 4-bromo-1-methyl-1H-imidazole-2-carboxamide (Example 141, Step B) as the aryl halide and (*S*)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate . MS (ESI): mass calcd. for C₂₀H₁₇F₇N₆O₃, 522.1; m/z found, 523.2 [M+H]⁺. 1H NMR (400 MHz, Chloroform-d) δ 10.05 (s, 1H), 8.95 (s, 1H), 8.05 (d, J = 11.8 Hz, 1H), 7.79 (d, J = 5.6 Hz, 1H), 7.60 (d, J = 3.8 Hz, 1H), 5.46 (s, 1H), 5.15 - 5.04 (m, 1H), 4.13 (s, 3H), 2.33 (s, 3H), 1.67 (d, J = 6.5 Hz, 3H) ppm.

### Example 155: 5-Fluoro-4-(2-((*R)-1-hydroxyethyl)-1-methyl-1H-imidazol-4-yl)-N-(3-methyl-5-(trifluoromethyl)-1H-pyrazol-4-yl)-2-(((S)-1,1,1-trifluoropropan-2-yl)oxy)benzamide.

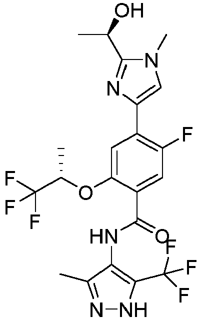

5-Fluoro-4-(2-((**R*)-1-hydroxyethyl)-1-methyl-1H-imidazol-4-yl)-*N*-(3-methyl-5-(trifluoromethyl)-1H-pyrazol-4-yl)-2-(((*S*)-1,1,1-trifluoropropan-2-yl)oxy)benzamide. The title compound was prepared in a manner analogous to Example 88 however using (**R*)-1-(4-bromo-1-methyl-1H-imidazol-2-yl)ethan-1-ol (Example 153, Step A) as the aryl halide and (*S*)-(2-fluoro-4-((5-methyl-3-(trifluoromethyl)-1H-pyrazol-4-yl)carbamoyl)-5-((1,1,1-trifluoropropan-2-yl)oxy)phenyl)boronic acid (Example 93, Step B) as the boronate . MS (ESI): mass calcd. for C₂₁H₂₀F₇N₅O₃, 523.1; m/z found, 524.2 [M+H]⁺. 1H NMR (400 MHz, Chloroform-d) δ 10.25 (s, 1H), 8.97 (s, 1H), 8.01 (d, J = 11.9 Hz, 1H), 7.81 (d, J = 5.7 Hz, 1H), 7.46 (d, J = 3.9 Hz, 1H), 5.19 - 5.07 (m, 1H), 5.07 - 4.97 (m, 1H), 3.76 (s, 3H), 2.89 (s, 1H), 2.30 (s, 3H), 1.72 - 1.62 (m, 6H) ppm.

### Example 156: 5-Fluoro-4-(5-((*S)-1-hydroxyethyl)-1-methyl-1H-1,2,4-triazol-3-yl)-N-(o-tolyl)-2-(((S)-1,1,1-trifluoropropan-2-yl)oxy)benzamide.

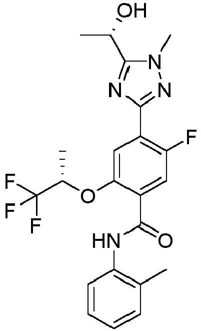

Step A. (**S*)-1-(3-Bromo-1-methyl-1*H*-1,2,4-triazol-5-yl)ethanol and (**R*)-1-(3-bromo-1-methyl-1*H*-1,2,4-triazol-5-yl)ethanol. The 1-(3-bromo-1-methyl-1*H*-1,2,4-triazol-5-yl)ethanol (Example 144, Step B) (6.9 g, 33.5 mmol) was separated by SFC (Stationary phase: DAICEL CHIRALPAK IC, 10 µm 250 mm × 50 mm); Mobile phase: Supercritical CO₂ (A) - EtOH (0.1% NH₃.H₂O) (B), gradient elution: 30% B in A at 200 mL/min) to give:
The first eluting compound (**S*)-1-(3-Bromo-1-methyl-1*H*-1,2,4-triazol-5-yl)ethanol as white solid. MS (ESI): mass calcd. for C₅H₈BrN₃O, 205.0; m/z found, 206.0, [M+H]⁺.

¹H NMR (400MHz, DMSO-*d₆*) δ = 5.01 (t, J=6.8 Hz, 1H), 3.93 (s, 3H), 2.82 (d, J=7.3 Hz, 1H), 1.64 (d, J=6.5 Hz, 3H) ppm. SFC: purity 99.89 %, retention time 2.226 min.

The second eluting compound (**R*)-1-(3-bromo-1-methyl-1H-1,2,4-triazol-5-yl)ethan-1-ol as white solid. MS (ESI): mass calcd. for C₅H₈BrN₃O, 205.0; m/z found, 206.0, [M+H]⁺. ¹H NMR (400MHz, DMSO-*d₆*) δ = 5.01 (t, J=6.9 Hz, 1H), 3.93 (s, 3H), 2.67 (d, J=7.3 Hz, 1H), 1.64 (d, J=6.8 Hz, 3H) ppm. SFC: purity 97.68 %, retention time 2.673 min.

Step B. 5-Fluoro-4-(5-((**S*)-1-hydroxyethyl)-1-methyl-1H-1,2,4-triazol-3-yl)-*N*-(*o*-tolyl)-2-(((*S*)-1,1,1-trifluoropropan-2-yl)oxy)benzamide. The title compound was prepared in a manner analogous to Example 88 however using (**S*)-1-(3-bromo-1-methyl-1H-1,2,4-triazol-5-yl)ethan-1-ol as the aryl halide and (*S*)-5-fluoro-4-(4,4,5,5-tetramethyl-1,3,2-dioxaborolan-2-yl)-*N*-(*o-*tolyl)-2-((1,1,1-trifluoropropan-2-yl)oxy)benzamide (Example 77, step A) as the boronate . MS (ESI): mass calcd. for C₂₂H₂₂F₄N₄O₃, 466.2; m/z found, 467.3 [M+H]⁺. 1H NMR (400 MHz, Chloroform-d) δ 9.11 (s, 1H), 8.10 (d, J = 11.3 Hz, 1H), 7.90 (d, J = 7.9 Hz, 1H), 7.77 (d, J = 5.5 Hz, 1H), 7.26 (m, 2H), 7.18 -7.11 (m, 1H), 5.23 - 5.10 (m, 1H), 5.10 - 4.97 (m, 1H), 4.01 (s, 3H), 2.97 (d, J = 6.6 Hz, 1H), 2.33 (s, 3H), 1.69 (d, J = 6.6 Hz, 3H), 1.65 (d, J = 6.5 Hz, 3H) ppm.

### Example 157: 5-Fluoro-4-(5-((*R)-1-hydroxyethyl)-1-methyl-1H-1,2,4-triazol-3-yl)-N-(o-tolyl)-2-(((S)-1,1,1-trifluoropropan-2-yl)oxy)benzamide.

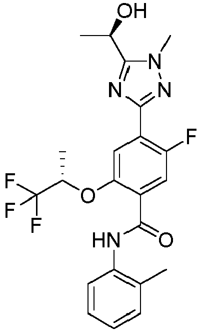

The title compound was prepared in a manner analogous to Example 88 however using (**R*)-1-(3-bromo-1-methyl-1H-1,2,4-triazol-5-yl)ethan-1-ol (Example 156, Step A) as the aryl halide and (*S*)-5-fluoro-4-(4,4,5,5-tetramethyl-1,3,2-dioxaborolan-2-yl)-*N*-(*o*-tolyl)-2-((1,1,1-trifluoropropan-2-yl)oxy)benzamide (Example 77, step A) as the boronate . MS (ESI): mass calcd. for C₂₂H₂₂F₄N₄O₃, 466.2; m/z found, 467.2 [M+H]⁺. 1H NMR (400 MHz, Chloroform-d) δ 9.11 (s, 1H), 8.10 (d, J= 11.3 Hz, 1H), 7.90 (d, J = 7.9 Hz, 1H), 7.77 (d, J = 5.5 Hz, 1H), 7.26 (m, 2H), 7.18 - 7.11 (m, 1H), 5.23 - 5.10 (m, 1H), 5.10 - 4.97 (m, 1H), 4.02 (s, 3H), 2.86 (d, J = 6.8 Hz, 1H), 2.33 (s, 3H), 1.71 (d, J = 6.6 Hz, 3H), 1.65 (d, J = 6.5 Hz, 3H) ppm.

### Example 158: (S)-N-(5-Chloro-3-methyl-1H-pyrazol-4-yl)-5-fluoro-4-(2-(3-hydroxyoxetan-3-yl)-1-methyl-1H-imidazol-4-yl)-2-((1,1,1-trifluoropropan-2-yl)oxy)benzamide.

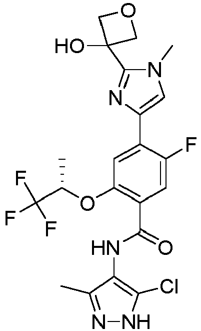

Step A. 3-(4-Bromo-1-methyl-1H-imidazol-2-yl)oxetan-3-ol. A solution of LDA (2 M solution in THF and n-heptane, 22.1 mL, 44.2 mmol) was added to a solution of 4-bromo-1-methyl-1H-imidazole (2.8 g, 17.39 mmol) in THF (15 mL) at -78°C. The resulting mixture was stirred at-78°C for 0.5 hour, oxetan-3-one (1.5 g, 20.84 mmol) in THF (9 mL) was added. The mixture was stirred at -78°C for 5 hours. The mixture were washed with sat. aq. NH₄Cl (150 mL) and extracted with DCM:MeOH (v/v=10/1, 120 mL × 3). The combined organic phase was dried with anhydrous Na₂SO₄, filtered and concentrated in vacuum. The residue was purified by preparative reversed phase HPLC (Stationary phase: Xtimate C18, 5 µm, 150 × 40 mm; Mobile phase: water (0.05% NH₃H₂O) (A) - MeCN (B), gradient elution: 5 - 35% B in A over 9 min, flow rate: 60 mL/min) to give the title compound as white powder. MS (ESI): mass calcd. for C₇H₉BrN₂O₂, 232.0; m/z found, 232.9 [M+H]⁺. ¹H NMR (400MHz, CDCl₃) δ = 6.77 (s, 1H), 5.34 (s, 1H), 5.08 (d, *J*=6.9 Hz, 2H), 4.86 (d, *J*=7.0 Hz, 2H), 3.66 (s, 3H) ppm.

Step B. (*S*)-*N*-(5-Chloro-3-methyl-1H-pyrazol-4-yl)-5-fluoro-4-(2-(3-hydroxyoxetan-3-yl)-1-methyl-1H-imidazol-4-yl)-2-((1,1,1-trifluoropropan-2-yl)oxy)benzamide. The title compound was prepared in a manner analogous to Example 88 however using 3-(4-bromo-1-methyl-1H-imidazol-2-yl)oxetan-3-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate . MS (ESI): mass calcd. for C₂₁H₂₀ClF₄N₅O₄, 517.1; m/z found, 518.1 [M+H]⁺. 1H NMR (400 MHz, Chloroform-d) δ 9.73 (s, 1H), 8.83 (s, 1H), 8.01 (d, J = 11.8 Hz, 1H), 7.74 (d, J = 5.7 Hz, 1H), 7.46 (d, J = 3.8 Hz, 1H), 5.23 (d, J = 7.0 Hz, 1H), 5.15 (d, J = 7.1 Hz, 1H), 5.19 - 5.04 (m, 1H), 4.99 (d, *J* = 7.0 Hz, 2H), 4.43 (s, 1H), 3.72 (s, 3H), 2.31 (s, 3H), 1.70 (d, J = 6.5 Hz, 3H) ppm.

### Example 159: N-(5-chloro-3-methyl-1H-pyrazol-4-yl)-4-(5-chloro-6-(1-hydroxyethyl)pyridin-2-yl)-5-fluoro-2-(((S)-1,1,1-trifluoropropan-2-yl)oxy)benzamide.

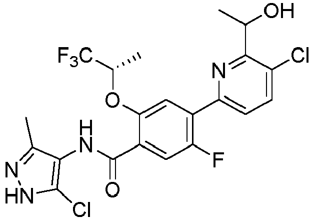

The title compound was prepared in a manner analogous to Example 1, Step E however using 1-(6-bromo-3-chloropyridin-2-yl)ethan-1-ol (Example 80, Step A) as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate . MS (ESI): mass calcd. for C₂₁H₁₈Cl₂F₄N₄O₃, 520.07; m/z found, 521.1 [M+H]⁺. ¹H NMR (400 MHz, DMSO) δ = 12.79-12.93 (m, 1H), 9.49 (s, 1H), 8.06 (d, J=8.80 Hz, 1H), 7.91-7.98 (m, 1H), 7.83 (d, J=8.31 Hz, 1H), 7.54 (d, J=10.76 Hz, 1H), 5.41-5.52 (m, 1H), 5.15-5.29 (m, 2H), 2.16 (s, 3H), 1.46-1.53 (m, 6H).

### Example 160: (S)-N-(5-chloro-3-methyl-1H-pyrazol-4-yl)-4-(5-chloro-6-(2-hydroxypropan-2-yl)pyridin-2-yl)-5-fluoro-2-((1,1,1-trifluoropropan-2-yl)oxy)benzamide.

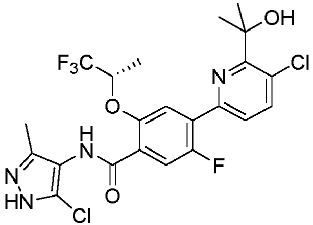

The title compound was prepared in a manner analogous to Example 1, Step E however using 2-(6-Bromo-3-chloropyridin-2-yl)propan-2-ol (Example 74, Step A) as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate . MS (ESI): mass calcd. for C₂₂H₂₀Cl₂F₄N₄O₃, 534.08; m/z found, 535.0 [M+H]⁺. ¹H NMR (400 MHz, CDCl₃) δ = 10.16 (br s, 1H), 8.80 (s, 1H), 8.09-8.16 (m, 1H), 7.84-7.90 (m, 2H), 7.69-7.73 (m, 1H), 5.92-5.96 (m, 1H), 4.95-5.09 (m, 1H), 2.33 (s, 3H), 1.77-1.80 (m, 6H), 1.68-1.71 (m, 3H).

### Example 161: (S)-N-(2-chloro-6-fluorophenyl)-5-fluoro-4-(5-(2-hydroxypropan-2-yl)pyrazin-2-yl)-2-((1,1,1-trifluoropropan-2-yl)oxy)benzamide.

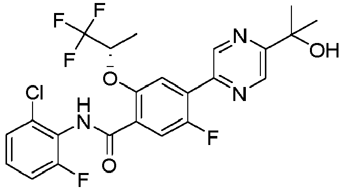

Step A. (S)-4-(5-acetylpyrazin-2-yl)-N-(2-chloro-6-fluorophenyl)-5-fluoro-2-((1,1,1-trifluoropropan-2-yl)oxy)benzamide. The title compound was prepared in a manner analogous to Example 1, Step E however using 1-(5-chloropyrazin-2-yl)ethanone as the aryl halide and (*S*)-*N-*(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₂H₁₅ClF₅N₃O₃, 499.07; m/z found, 500.1 [M+H]⁺. ¹H NMR (400MHz, CDCl₃) δ = 9.34 - 9.24 (m, 2H), 9.10 (s, 1H), 8.19 (d, *J*=11.7 Hz, 1H), 7.94 (d, *J*=5.5 Hz, 1H), 7.31 - 7.24 (m, 2H), 7.16 - 7.09 (m, 1H), 5.10 (td, *J*=6.0, 12.2 Hz, 1H), 2.76 (s, 3H), 1.69 (d, *J*=6.4 Hz, 3H); ¹⁹F NMR (376MHz, CDCl₃) δ = -78.16 (d, *J*=5.1 Hz, 1F), -113.66 (s, 1F), -119.29 (dd, *J*=5.1, 11.7 Hz, 1F).

### Step B. (S)-N-(2-chloro-6-fluorophenyl)-5-fluoro-4-(5-(2-hydroxypropan-2-yl)pyrazin-2-yl)-2-((1,1,1-trifluoropropan-2-yl)oxy)benzamide

A mixture of (S)-4-(5-acetylpyrazin-2-yl)-N-(2-chloro-6-fluorophenyl)-5-fluoro-2-((1,1,1-trifluoropropan-2-yl)oxy)benzamide (125 mg, 250 µmol) in THF (2 mL) was cooled to 0 °C. Then MeMgBr (3 M solution in ether, 0.15 mL, 450 µmol) was added dropwise under N₂. The mixture was stirred at 0 °C for 2 hours then was warmed to room temperature overnight. Another 0.1 mL MeMgBr (3 M solution in ether, 300 µmol) was added to the mixture at 0 °C. The mixture was stirred at 0 °C for 2 hours then was warmed to room temperature overnight. The mixture was quenched with saturated aqueous NH₄Cl (4 mL), extracted with ethyl acetate (5 mL × 3), dried over Na₂SO₄, and concentrated under reduced pressure. The residue was purified by column chromatography (SiO₂, gradient elution: 0 - 25% ethyl acetate in petroleum ether), then further purified by preparative high-performance liquid chromatography (Stationary phase: Boston Prime C18, 5 µm, 150 × 30 mm; Mobile phase: water (0.04% NH₃H₂O + 10 mM NH₄HCO₃) (A) - MeCN (B), gradient elution: 55 - 85% B in A over 7 min, flow rate: 25 mL/min) to give the title compound as off-white powder. MS (ESI): mass calcd. for C₂₃H₁₉ClF₅N₃O₃, 515.10; m/z found, 516.0 [M+H]⁺. ¹H NMR (400MHz, CDCl₃) δ = 9.13 (s, 1H), 9.10 (s, 1H), 8.89 (s, 1H), 8.16 (d, *J*=11.7 Hz, 1H), 7.87 (d, *J*=5.7 Hz, 1H), 7.31 - 7.25 (m, 1H), 7.24 - 7.19 (m, 1H), 7.12 (t, *J*=8.8 Hz, 1H), 5.08 (td, *J*=6.3, 12.4 Hz, 1H), 3.76 (s, 1H), 1.70 - 1.62 (m, 9H); ¹⁹F NMR (376MHz, CDCl₃) δ = -78.20 (d, *J*=6.6 Hz, 1F), -113.41 - 113.81 (m, 1F), -120.25 - 120.66 (m, 1F).

### Example 162: (S)-N-(2-Chloro-6-fluorophenyl)-4-(1-cyclopropyl-3-(hydroxymethyl)-1H-pyrazol-5-yl)-5-fluoro-2-((1,1,1-trifluoropropan-2-yl)oxy)benzamide.

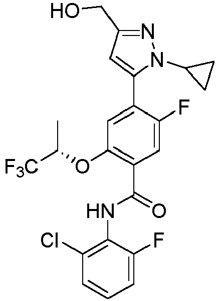

Step A. Di-tert-butyl 1-cyclopropylhydrazine-1,2-dicarboxylate. To a stirred solution of di-tert-butyl diazene-1,2-dicarboxylate (10 g, 43.4 mmol) in DMF (100 mL), was added cyclopropylboronic acid (7.5 g, 86.9 mmol) and Cu(OAc)₂ (789 mg, 4.34 mmol). The reaction mixture was stirred at 75 °C overnight then cooled down to room temperature. The reaction mixture was filtered through a pad of Celite^{®}. The filtrate was diluted with water (100 mL) and extracted with ethyl acetate (300 mL×3). The organic layer was dried over Na₂SO₄, filtered and concentrated under reduced pressure. The residue was purified by flash column chromatography (SiO₂, gradient eluent: 0 - 30% ethyl acetate in petroleum ether) to give title compound as a yellow oil. MS (ESI): mass calcd. for C₁₃H₂₄N₂O₄; m/z found, 272.2; m/z found, 273.1 [M+H]⁺. ¹H NMR (400MHz, CDCl₃) δ = 6.47 - 5.95 (m, 1H), 2.92 (br s, 1H), 1.48 - 1.38 (m, 18H), 0.70 (br s, 4H).

Step B. Cyclopropylhydrazine. A mixture of di-tert-butyl 1-cyclopropylhydrazine-1,2-dicarboxylate (8 g, 29.4 mmol) and HCl/ ethyl acetate (4 M solution, 24 mL, 96 mmol) in DCM (15 mL) was stirred at room temperature for 16 hours. The reaction was concentrated to give crude title compound as a light yellow solid. It was directly used for the next step without further purification.

Step C. Methyl 1-cyclopropyl-5-hydroxy-1H-pyrazole-3-carboxylate. To a solution of cyclopropylhydrazine (4 g, 22.0 mmol) and dimethyl acetylenedicarboxylate (2.8 g, 19.8 mmol) in MeOH/H₂O (v/v, 3/1, 8 mL) was added Et₃N (6.1 mL, 44.1 mmol) at room temperature. The mixture reaction was stirred at 60 °C for 1 hour then cooled down to room temperature. The mixture reaction was concentrated. The residue was purified by preparative reversed phase HPLC (Stationary phase: Phenomenex Gemini-NX C18, 5 µm, 150 × 30 mm; Mobile phase: water (0.05% HCl) (A) - MeCN (B), gradient elution: 15 - 45% B in A over 7 min, flow rate: 25 mL/min) to give title compound as a light yellow solid. MS (ESI): mass calcd. for C₈H₁₀N₂O₃, 182.1; m/z found, 183.1 [M+H]+._¹H NMR (400MHz, DMSO-*d₆*) δ = 10.08 (s, 1H), 5.99 (s, 1H), 4.13 (tt, *J=* 7.5, 3.9Hz, 1H), 3.78 (s, 3H), 0.94 - 1.05 (m, 2H), 0.82 - 0.93 (m, 2H).

Step D. Methyl 1-cyclopropyl-5-(((trifluoromethyl)sulfonyl)oxy)-1H-pyrazole-3-carboxylate. Trifluoromethanesulfonic anhydride (923 mg, 3.27 mmol) was added into a solution of methyl 1-cyclopropyl-5-hydroxy-1H-pyrazole-3-carboxylate (400 mg, 2.18 mmol) and Et₃N (0.9 mL, 6.54 mmol) in DCM (5 mL) at room temperature. The reaction was stirred at room temperature overnight. The mixture was diluted by DCM (20 mL) and water (10 mL). The organic layer was separated and washed by brine (10 mL), dried by Na₂SO₄, filtered and concentrated under reduced pressure. The residue was purified by flash column chromatography (SiO₂, gradient elution: 0 - 30% ethyl acetate in petroleum ether) to give title compound as a light yellow oil. MS (ESI): mass calcd. for C₉H₉F₃N₂O₅S, 314.0; m/z found, 315.1 [M+H]+. ¹H NMR (400MHz, CDCl₃) δ = 6.63 (s, 1H), 3.90 (s, 3H), 3.43 - 3.54 (m, 1H), 1.26 - 1.34 (m, 2H), 1.10 - 1.20 (m, 2H)

Step E. (S)-Methyl 5-(4-((2-chloro-6-fluorophenyl)carbamoyl)-2-fluoro-5-((1,1,1-trifluoropropan-2-yl)oxy)phenyl)-1-cyclopropyl-1H-pyrazole-3-carboxylate. The title compound was prepared in a manner analogous to Example 48, Step B however using methyl 1-cyclopropyl-5-(((trifluoromethyl)sulfonyl)oxy)-1H-pyrazole-3-carboxylate as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₄H₁₉ClF₅N₃O₄, 543.1; m/z found, 544.1 [M+H]+. ¹H NMR (400MHz, CDCl₃) δ = 9.00 (s, 1H), 8.14 (d, *J* = 10.1 Hz, 1H), 7.24 - 7.32 (m, 2H), 7.13 (t, *J* = 8.6 Hz, 1H), 7.08 (d, *J* = 5.3 Hz, 1H), 6.92 (s, 1H), 4.82 - 5.04 (m, 1H), 3.93 (s, 3H), 3.54 - 3.66 (m, 1H), 1.66 (d, *J* = 6.4 Hz, 3H), 1.08 - 1.15 (m, 2H), 0.93 - 1.03 (m, 2H); ¹⁹F NMR (376MHz, CDCl₃) δ = -78.04 (br d, *J* = 7.3 Hz, 1F), -114.45 - 111.20 (m, 1F), -118.42 - 116.25 (m, 1F).

Step F. (S)-N-(2-Chloro-6-fluorophenyl)-4-(1-cyclopropyl-3-(hydroxymethyl)-1H-pyrazol-5-yl)-5-fluoro-2-((1,1,1-trifluoropropan-2-yl)oxy)benzamide. A solution of intermediate (S)-methyl 5-(4-((2-chloro-6-fluorophenyl)carbamoyl)-2-fluoro-5-((1,1,1-trifluoropropan-2-yl)oxy)phenyl)-1-cyclopropyl-1H-pyrazole-3-carboxylate (80 mg, 147.1 µmol) in 2 mL THF was slowly added to a stirred mixture of LiAlH₄ (11.2 mg, 294.2 µmmol) in 3 mL THF at 0 °C. The mixture was stirred at room temperature for 0.5 hour. Water (11.2 µL), 15% aqueous NaOH solution (7.5 µL) and water (33.6 µL) were slowly added to the mixture in order at 0°C. Then Na₂SO₄ (10 g) was added to the mixture and stirred at room temperature for 0.5 hour. The mixture was filtered, and filtrate was concentrated. The residue was purified by preparative reversed phase HPLC (Stationary phase: Boston Prime C18, 5 µm, 150 × 30 mm; Mobile phase: water (0.04% NH₃H₂O + 10 mM NH₄HCO₃) (A) - MeCN (B), gradient elution: 55 - 75% B in A over 7 min, flow rate: 25 mL/min) to give title compound as a white powder. MS (ESI): mass calcd. for C₂₃H₁₉ClF₅N₃O₃, 515.1; m/z found, 516.2 [M+H]+. ¹H NMR (400MHz, CDCl₃) δ = 9.04 (s, 1H), 8.14 (d, *J* = 10.3 Hz, 1H), 7.30 - 7.33 (m, 1H), 7.26 (s, 1H), 7.12 - 7.18 (m, 1H), 7.11 (d, *J* = 5.3 Hz, 1H), 6.41 (s, 1H), 4.91 (dt, *J* = 12.1, 6.1 Hz, 1H), 4.72 (d, *J* = 5.5 Hz, 2H), 3.47 - 3.62 (m, 1H), 1.96 (br s, 1H), 1.67 (d, *J* = 6.5 Hz, 3H), 0.99 - 1.09 (m, 2H), 0.90 - 0.97 (m, 2H); ¹⁹F NMR (376MHz, CDCl₃) δ ppm -78.04 (br d, *J* = 8.6 Hz, 1F), -113.71 (br d, *J* = 8.6 Hz, 1F), -117.71 (br d, *J* = 8.6 Hz, 1F).

### Example 163: (S)-ethyl 4-(4-((2-chloro-6-fluorophenyl)carbamoyl)-2-fluoro-5-((1,1,1-trifluoropropan-2-yl)oxy)phenyl)-1-cyclopropyl-1H-imidazole-2-carboxylate.

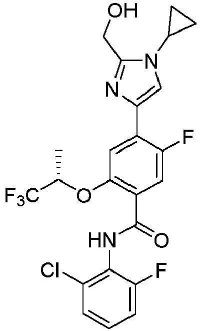

Step A. Ethyl 1-cyclopropyl-1H-imidazole-2-carboxylate. A pre-heated suspension of 2,2'-bipyridine (2.2 g, 14.3 mmol) and Cu(OAc)₂ (2.6 g, 14.3 mmol) in DCE (5 mL) was added to a mixture of ethyl 1*H*-imidazole-2-carboxylate (2 g, 14.3 mmol), cyclopropylboronic acid (3.1 g, 35.7 mmol), Na₂CO₃ (3.8 g, 35.7 mmol) in DCE (15 mL) at 70 °C. The reaction mixture was then stirred vigorously under air at 70 °C overnight then cooled down to room temperature. The reaction mixture was cooled and filtered. The filtrate was concentrated under reduced pressure. The residue was purified by flash column chromatography (SiO₂, gradient elution: 0 - 50% ethyl acetate in petroleum ether) to give title compound as yellow oil. MS (ESI): mass calcd. for C₉H₁₂N₂O₂, 180.1; m/z found, 181.1 [M+H]⁺. ¹H NMR (400MHz, CDCl₃) δ = 7.05 (s, 1H), 7.01 (s, 1H), 4.37 - 4.42 (m, 2H), 3.79 - 3.85 (m, 1H), 1.40 (t, *J* = 7.2 Hz, 3H), 1.06 - 1.15 (m, 2H), 0.89 - 0.95 (m, 2H).

Step B. Ethyl 4-bromo-1-cyclopropyl-1H-imidazole-2-carboxylate. NBS (429 mg, 2.4 mmol) was added to a mixture of ethyl 1-cyclopropyl-1H-imidazole-2-carboxylate (400 mg, 2.2 mmol) in DCM (12 mL) at 0 °C. The mixture was warmed to room temperature and stirred at room temperature for 3 hours. The reaction mixture was quenched with 5 N aqueous NaOH solution (12 mL) at 0 °C and extracted with DCM (30 mL×3). The organic layer was dried over Na₂SO₄, filtered and concentrated under reduced pressure. The residue was purified by flash column chromatography (SiO₂, gradient elution: 0 - 30% ethyl acetate in petroleum ether) to give title compound as yellow solid. MS (ESI): mass calcd. for C₉H₁₁BrN₂O₂, 258.0; m/z found, 260.8 [M+H]⁺. ¹H NMR (400MHz, CDCl₃) δ = 7.02 (s, 1H), 4.42 (q, *J* = 7.0 Hz, 2H), 3.79 - 3.89 (m, 1H), 1.42 (t, *J* = 7.2 Hz, 3H), 1.10- 1.19 (m, 2H), 0.89 - 1.00 (m, 2H).

Step C. (S)-Ethyl 4-(4-((2-chloro-6-fluorophenyl)carbamoyl)-2-fluoro-5-((1,1,1-trifluoropropan-2-yl)oxy)phenyl)-1-cyclopropyl-1H-imidazole-2-carboxylate. The title compound was prepared in a manner analogous to Example 48, Step B however using ethyl 4-bromo-1-cyclopropyl-1H-imidazole-2-carboxylate as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₅H₂₁ClF₅N₃O₄, 557.11; m/z found, 558.1 [M+H]⁺.

Step D. (S)-N-(2-Chloro-6-fluorophenyl)-4-(1-cyclopropyl-2-(hydroxymethyl)-1H-imidazol-4-yl)-5-fluoro-2-((1,1,1-trifluoropropan-2-yl)oxy)benzamide. A solution of (S)-ethyl 4-(4-((2-chloro-6-fluorophenyl)carbamoyl)-2-fluoro-5-((1,1,1-tri fluoropropan-2-yl)oxy)phenyl)-1-cyclopropyl-1H-imidazole-2-carboxylate (70 mg, 106.3 µmol) in 2 mL THF was slowly added to a stirred mixture of LiAlH₄ (8.07 mg, 212.5 mmol) in 3 mL THF at 0 °C. The mixture was stirred at room temperature for 0.5 hour. Water (8.07 µL), 10% aqueous NaOH solution (8.07 µL) and water (24.21 µL) were slowly added to the mixture in order at 0 °C. Then Na₂SO₄ was added to the mixture and stirred at room temperature for 0.5 hour. The mixture was filtered, and filtrate was concentrated under reduced pressure. The residue was purified by preparative reversed phase HPLC (Stationary phase: Boston Prime C18, 5 µm, 150 × 30 mm; Mobile phase: water (0.04% NH₃H₂O + 10 mM NH₄HCO₃) (A) - MeCN (B), gradient elution: 45 - 75% B in A over 7 min, flow rate: 25 mL/min) to give title compound as an off-white powder. MS (ESI): mass calcd. for C₂₃H₁₉ClF₅N₃O₃, 515.1; m/z found, 516.1 [M+H]⁺. ¹H NMR (400MHz, CDCl₃) δ = 9.18 (s, 1H), 8.05 (d, *J* = 12.0 Hz, 1H), 7.88 (d, *J* = 5.8 Hz, 1 H), 7.51 (d, *J* = 3.8 Hz, 1H), 7.27 - 7.31 (m, 1H), 7.20 - 7.25 (m, 1H), 7.09 - 7.16 (m, 1H), 5.27 (dt, *J* = 12.3, 6.1 Hz, 1H), 4.82-4.91 (m, 2H), 4.72 (br s, 1H), 3.31 - 3.43 (m, 1H), 1.67 (d, *J* = 6.5 Hz, 3H), 1.05 - 1.18 (m, 4H); ¹⁹F NMR (376MHz, CDCl₃) δ ppm -78.34 (s, 1F), -113.64 (s, 1F), -120.69 (s, 1F).

### Example 164: (S)-N-(2-Chloro-6-fluorophenyl)-4-(1-cyclopropyl-5-(hydroxymethyl)-1H-pyrazol-3-yl)-5-fluoro-2-((1,1,1-trifluoropropan-2-yl)oxy)benzamide.

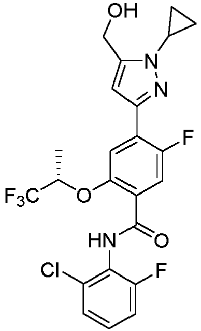

Step A. Methyl 3-bromo-1-cyclopropyl-1*H*-pyrazole-5-carboxylate. To a mixture of methyl 3-bromo-1*H*-pyrazole-5-carboxylate (1.5 g, 7.32 mmol), cyclopropylboronic acid (1.57 g, 18.3 mmol), Na₂CO₃ (1.94 g, 18.3 mmol) in DCE (25 mL) at 70 °C was added a heated suspension of 2,2'-bipyridine (1.14 g, 7.32 mmol) and Cu(OAc)₂ (1.33 g, 7.32 mmol) in DCE (5 mL). The reaction mixture was then stirred vigorously under air at 70 °C overnight. The reaction mixture was cooled and filtered. The solvent was removed to give the crude product. The residue was purified by flash column chromatography (SiO₂, gradient elution: 0 - 50% ethyl acetate in petroleum ether) to give the title compound as a yellow oil. MS (ESI): mass calcd. for C₈H₉BrN₂O₂, 244.0; m/z found, 246.8 [M+H]⁺.

¹H NMR (400MHz, CDCl₃) δ = 6.80 (s, 1H), 4.26 (tt, J=3.9, 7.5 Hz, 1H), 3.90 (s, 3H), 1.27-1.21 (m, 2H), 1.12 - 0.98 (m, 2H).

Step B. (*S*)-Methyl 3-(4-((2-chloro-6-fluorophenyl)carbamoyl)-2-fluoro-5-((1,1,1-trifluoropro pan-2-yl)oxy)phenyl)-1-cyclopropyl-1*H*-pyrazole-5-carboxylate. The title compound was prepared in a manner analogous to Example 48, Step B however using methyl 3-bromo-1-cyclopropyl-1*H*-pyrazole-5-carboxylate as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₄H₁₉ClF₅N₃O₄, 543.1; m/z found, 544.1 [M+H]⁺. ¹H NMR (400MHz, CDCl₃) δ = 9.10 (s, 1H), 8.08 (d, *J*=11.8 Hz, 1H), 7.72 (d, *J*=5.8 Hz, 1H), 7.38 (d, *J*=4.0 Hz, 1H), 7.27 - 7.32 (m, 1H), 7.21 - 7.26 (m, 1H), 7.08 - 7.17 (m, 1H), 5.05 (dt, *J*=12.4, 6.2 Hz, 1H), 4.42 (tt, *J*=7.5, 3.9 Hz, 1H), 3.95 (s, 3H), 1.67 (d, *J*=6.5 Hz, 3H), 1.34 - 1.42 (m, 2H), 1.12 - 1.19 (m, 2H); ¹⁹F NMR (376MHz, CDCl₃) δ = -78.22 (s, 1F), -113.70 (br s, 1F), -121.76 (s, 1F).

Step C. (*S*)-*N*-(2-Chloro-6-fluorophenyl)-4-(1-cyclopropyl-5-(hydroxymethyl)-1*H*-pyrazol-3-yl)-5-fluoro-2-((1,1,1-trifluoropropan-2-yl)oxy)benzamide. To a stirred mixture of LiAlH₄ (11.9 mg, 313.5 µmol) in THF (3 mL) was added a solution of (*S*)-methyl 3-(4-((2-chloro-6-fluorophenyl)carbamoyl)-2-fluoro-5-((1,1,1-trifluoropropan-2-yl) oxy)phenyl)-1-cyclopropyl-1*H*-pyrazole-5-carboxylate (85 mg, 156.3 µmol) in THF (2 mL) slowly at 0 °C. The mixture was stirred at room temperature for 0.5 hour. Water (12 µL), 15% aqueous NaOH solution (8 µL) and water (36 µL) were added to the mixture, in order, slowly at 0 °C. Then Na₂SO₄ was added to the mixture and stirred at room temperature for 0.5 hour. The mixture was filtered, and filtrate was concentrated under reduced pressure. The residue was purified by preparative reversed phase HPLC (Stationary phase: Boston Prime C18, 5 µm, 150 × 30 mm; Mobile phase: water (0.04% NH₃H₂O + 10 mM NH₄HCO₃) (A) - MeCN (B), gradient elution: 55 - 85% B in A over 7 min, flow rate: 25 mL/min) to give the title compound as an off-white powder. MS (ESI): mass calcd. for C₂₃H₁₉ClF₅N₃O₃, 515.1; m/z found, 516.1 [M+H]⁺.

¹H NMR (400MHz, CDCl₃) δ = 9.13 (s, 1H), 8.06 (d, *J*=11.5 Hz, 1H), 7.73 (d, *J*=5.8 Hz, 1H), 7.32 - 7.27 (m, 1H), 7.25 - 7.20 (m, 1H), 7.16 - 7.08 (m, 1H), 6.79 (d, *J*=4.3 Hz, 1H), 5.09 (td, *J*=6.3, 12.4 Hz, 1H), 4.86 (d, *J*=5.8 Hz, 2H), 3.74 - 3.55 (m, 1H), 1.87 (t, *J*=6.0 Hz, 1H), 1.66 (d, *J*=6.5 Hz, 3H), 1.40 - 1.30 (m, 2H), 1.20 - 1.04 (m, 2H); ¹⁹F NMR (376MHz, CDCl₃) δ = -78.27 (s, 1F), -113.68 (br s, 1F), -122.12 (s, 1F).

### Example 165: (S)-N-(2-Chloro-6-fluorophenyl)-4-(1-cyclopropyl-5-(hydroxymethyl)-1H-1,2,4-triazol-3-yl)-5-fluoro-2-((1,1,1-trifluoropropan-2-yl)oxy)benzamide.

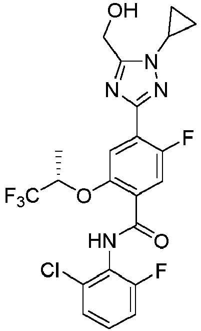

Step A. Methyl 3-bromo-1-cyclopropyl-1H-1,2,4-triazole-5-carboxylate. A mixture methyl 3-bromo-1*H*-1,2,4-triazole-5-carboxylate (5 g, 24.3 mmol) in DCE (25 mL) was stirred at room temperature for 15 minutes. Cyclopropylboronic acid (4.2 g, 48.5 mmol) and Na₂CO₃ (5.1 g, 48.5 mmol) were added to the mixture and stirred at room temperature. Cu(OAc)₂ (4.4 g, 24.3 mmol) and 2,2'-bipyridine (3.8 g, 24.3 mmol) in DCE (20 mL) was heating at 70 °C and the hot mixture was added to the above mixture. The reaction mixture was stirred at 70 °C overnight. EtOAc (120 mL) was added to the mixture. The mixture was filtered, and filter cake was washed with EtOAc (100 mL). The organic layer was washed with brine (120 mL), dried over Na₂SO₄, filtered and concentrated under reduced pressure. The residue was purified by flash column chromatography (SiO₂, petroleum ether/ethyl acetate =1/0 to 10/1). The obtained yellow solid was separated by Prep-SFC (Stationary phase: DAICEL CHIRALCEL OJ-H, 5 µm, 250 × 30 mm; Mobile phase: Supercritical CO₂ (A) - EtOH (0.1% NH₃.H₂O) (B), gradient elution: 15% B in A at 60 mL/min). The pure fractions were collected and concentrated to dryness to give the title compound as a yellow solid. MS (ESI): mass calcd. for C₇H₈BrN₃O₂, 245.0; m/z found, 248.0 [M+H]⁺.

Step B. (3-Bromo-1-cyclopropyl-1*H*-1,2,4-triazol-5-yl)methanol. NaBH₄ (46 mg, 1.21 mmol) and CaCl₂ (67.3 mg, 0.61 mmol) were added to the mixture of methyl 3-bromo-1-cyclopropyl-1*H*-1,2,4-triazole-5-carboxylate (250 mg, 1.01 mmol) in EtOH (8 mL) at 0 °C under N₂. The mixture was stirred at room temperature for 12 hours. The solvent was removed under vacuum. The residue was dissolved in sat. aq. NH₄Cl (35 mL). The mixture was extracted with ethyl acetate (25 mL × 3). The organic layers were separated, washed with brine (50 mL), dried over Na₂SO₄, filtered and concentrated under reduced pressure to give the title compound as white solid. MS (ESI): mass calcd. for C₆H₈BrN₃O, 217.0; m/z found, 218.1 [M+H]⁺. ¹H NMR (400 MHz, CDCl₃) δ = 4.84 (d, *J*=3.3 Hz, 2H), 3.90 - 4.09 (m, 1H), 3.53 (tt, *J*=7.2, 3.7 Hz, 1H), 1.23 - 1.29 (m, 2H), 1.10- 1.18 (m, 2H).

Step C. (*S*)-*N*-(2-Chloro-6-fluorophenyl)-4-(1-cyclopropyl-5-(hydroxymethyl)-1*H*-1,2,4-triazol-3-yl)-5-fluoro-2-((1,1,1-trifluoropropan-2-yl)oxy)benzamide. The title compound was prepared in a manner analogous to Example 1, Step E however using (3-bromo-1-cyclopropyl-1*H*-1,2,4-triazol-5-yl)methanol as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₂H₁₈ClF₅N₄O₃, 516.1; m/z found, 517.0 [M+H]⁺. ¹H NMR (400MHz, DMSO-*d*₆) δ = 10.04 (s, 1H), 7.81 (d, J=5.8 Hz, 1H), 7.33 - 7.53 (m, 4H), 5.71 (t, J=5.8 Hz, 1H), 5.38 (dt, J=12.8, 6.4 Hz, 1H), 4.75 (d, J=6.0 Hz, 2H), 3.82 (tt, J=7.3, 3.7 Hz, 1H), 1.46 (d, J=6.3 Hz, 3H), 1.20 (dq, J=8.0, 3.9 Hz, 2H), 1.08 - 1.15 (m, 2H); ¹⁹F NMR (376MHz, DMSO-*d*₆) δ ppm -77.00 (s, 3F), -115.41 (br s, 1F), -119.33 (s, 1F).

### Example 166: (S)-N-(3-Chloro-5-methyl-1H-pyrazol-4-yl)-5-fluoro-4-(5-(hydroxymethyl)-1-methyl-1H-1,2,4-triazol-3-yl)-2-((1,1,1-trifluoropropan-2-yl)oxy)benzamide.

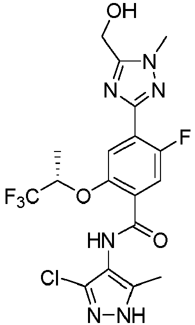

Step A. (*S*)-Methyl 5-fluoro-4-(5-(hydroxymethyl)-1-methyl-1H-1,2,4-triazol-3-yl)-2-((1,1,1-trifluoropropan-2-yl)oxy)benzoate. The title compound was prepared in a manner analogous to Example 48, Step B however using (3-bromo-1-methyl-1*H*-1,2,4-triazol-5-yl)methanol as the aryl halide and (S)-methyl 5-fluoro-4-(4,4,5,5-tetramethyl-1,3,2-dioxaborolan-2-yl)-2-((1,1,1-trifluoropropan-2-yl)oxy)benzoate (Intermediate 2) as the boronate . MS (ESI): mass calcd. for C₁₅H₁₅F₄N₃O₄, 377.1; m/z found, 378.1 [M+H]⁺. ¹H NMR (400MHz, CDCl₃) δ = 7.72 (d, J=5.8 Hz, 1H), 7.67 (d, .7=10.8 Hz, 1H), 4.87 (s, 2H), 4.71 - 4.82 (m, 2H), 4.02 (s, 3H), 3.92 (s, 3H), 1.57 (d, *J*=6.5 Hz, 3H); ¹⁹F NMR (376MHz, CDCl₃) δ = -78.34 (s, 2F), -119.44 (s, 1F).

Step B. (*S*)-5-Fluoro-4-(5-(hydroxymethyl)-1-methyl-1*H*-1,2,4-triazol-3-yl)-2-((1,1,1)-trifluoropropan-2-yl)oxy)benzoic acid. To the mixture of (*S*)-methyl 5-fluoro-4-(5-(hydroxymethyl)-1-methyl-1*H-*1,2,4-triazol-3-yl)-2-((1,1,1-trifluoropropan-2-yl)oxy)benzoate (100 mg, 237.8 µmol) in MeOH (3 mL) was added 2 M aq. NaOH (0.60 mL, 1.20 mmol), the mixture was stirred at 40 °C for 2 hours. The mixture was concentrated under vacuum, then 2 M aq. HCl was added to adjust the pH to 2-3. The mixture was extracted with ethyl acetate (25 mL × 3). The combined organic phases were washed with brine (30 mL), dried over anhydrous Na₂SO₄, filtered and concentrated in vacuum to give the title compound as yellow solid. MS (ESI): mass calcd. for C₁₄H₁₃F₄N₃O₄, 363.1; m/z found, 364.0 [M+H]⁺.-¹H NMR (400MHz, DMSO-*d*₆) δ = 13.23 (s, 1H), 7.75 (d, J=5.8 Hz, 1H), 7.58 (d, J=10.5 Hz, 1H), 5.66 - 5.75 (m, 1H), 5.24 (dt, J=12.8, 6.4 Hz, 1H), 4.68 (d, J=5.8 Hz, 2H), 3.95 (s, 3H), 1.43 (d, J=6.3 Hz, 3H); ¹⁹F NMR (376MHz, DMSO-*d*₆) δ = -77.02 (s, 3F), -119.35 (br s, 1F).

Step C. (*S*)-*N*-(3-Chloro-5-methyl-1*H*-pyrazol-4-yl)-5-fluoro-4-(5-(hydroxymethyl)-1-methyl-1*H*-1,2,4-triazol-3-yl)-2-((1,1,1-trifluoropropan-2-yl)oxy)benzamide. To the mixture of (S)-5-fluoro-4-(5-(hydroxymethyl)-1-methyl-1*H*-1,2,4-triazol-3-yl)- 2-((1,1,1-trifluoropropan-2-yl)oxy)benzoic acid (65 mg, 151.3 µmol) and 3-chloro-5-methyl-1*H*-pyrazol-4-amine (19.9 mg, 151.3 µmol) in DCM (5 mL) was added Et₃N (45.9 mg, 453.7 µmol), the mixture was stirred at room temperature for 10 minutes, then T₃P (50% solution in ethyl acetate, 144 mg, 226.9 µmol) was added. The resulting reaction mixture was heated at 50 °C and stirred for 12 hours. H₂O (15 mL) was added to the mixture and extracted with DCM (15 mL × 3). The combined organic layers were washed with brine (30 mL), dried over Na₂SO₄ and concentrated under reduced pressure. The crude product was purified by preparative high-performance liquid chromatography (Stationary phase: Boston Prime C18, 5 µm, 150 × 30 mm; Mobile phase: water (0.04% NH₃H₂O + 10 mM NH₄HCO₃) (A) - MeCN (B), gradient elution: 25 - 55% B in A over 7 min, flow rate: 25 mL/min) to give the title compound as off-white powder. MS (ESI): mass calcd. for C₁₈H₁₇ClF₄N₆O₃, 476.1; m/z found, 477.0 [M+H]⁺.

¹H NMR (400MHz, DMSO-*d*₆) δ = 12.84 (br s, 1H), 9.48 (s, 1H), 7.79 (d, J=5.7 Hz, 1H), 7.48 (d, J=10.3 Hz, 1H), 5.70 (t, J=5.8 Hz, 1H), 5.37 (dt, *J*=12.8, 6.4 Hz, 1H), 4.69 (d, J=5.8 Hz, 2H), 3.96 (s, 3H), 2.15 (s, 3H), 1.46 (d, J=6.3 Hz, 3H); ¹⁹F NMR (376MHz, DMSO-*d*₆) δ = -77.06 (s, 3F), -119.64 (s, 1F).

### Example 167: (S)-N-(2-Chloro-6-fluorophenyl)-5-fluoro-4-(4-(hydroxymethyl)-5-(trifluoromethyl) thiazol-2-yl)-2-((1,1,1-trifluoropropan-2-yl)oxy)benzamide.

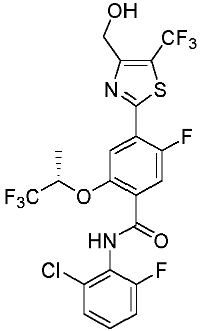

Step A. Methyl 2-amino-5-iodothiazole-4-carboxylate. To a solution of methyl 2-amino-5-iodothiazole-4-carboxylate (1 g, 3.52 mmol) in MeCN (6.6 mL) was added CuCl (523 mg, 5.28 mmol) followed by t-butyl nitrite (837 µL, 7.04 mmol). The mixture was stirred at room temperature overnight. The mixture was concentrated under reduced pressure. The residue was quenched with sat. aq. NH₄Cl (20 mL), extracted with ethyl acetate (15 mL × 3) and the combined extracts were dried over Na₂SO₄, filtered and concentrated under reduced pressure. The crude product was purified by flash column chromatography (SiO₂, gradient elution: 0 - 10% ethyl acetate in petroleum ether) to give the title compound as white solid. MS (ESI): mass calcd. for C₅H₃ClINO₂S, 302.9; m/z found, 303.9 [M+H]⁺.¹H NMR (400MHz, CDCl₃) δ = 3.97 (s, 3H).

Step B. Methyl 2-chloro-5-(trifluoromethyl)thiazole-4-carboxylate. To a solution of methyl 2-amino-5-iodothiazole-4-carboxylate (1 g, 3.15 mmol) and CuI (901 mg, 4.73 mmol) in DMF (20 mL) was added methyl 2,2-difluoro-2-(fluorosulfonyl)acetate (1.20 mL, 9.46 mmol). The resulting solution was stirred at 80 °C for 2 hours. The mixture was diluted with ethyl acetate (100 mL) and washed with brine (100 mL × 3). The aqueous layer was extracted with ethyl acetate (100 mL × 3), dried over Na₂SO₄, filtered and concentrated under reduced pressure. The crude product was purified by flash column chromatography (SiO₂, gradient elution: 0 - 10% ethyl acetate in petroleum ether) to give the title compound as a colorless oil. MS (ESI): mass calcd. for C₆H₃ClF₃NO₂S, 245.0; m/z found, 246.0 [M+H]⁺.¹H NMR (400MHz, CDCl₃) δ = 4.02 (s, 3H); ¹⁹F NMR (376 MHz, CDCl₃) δ = -53.38 (s, 1F).

Step C. (*S*)-Methyl 2-(4-((2-chloro-6-fluorophenyl)carbamoyl)-2-fluoro-5-((1,1,1-trifluoropropan-2-yl)oxy)phenyl)-5-(trifluoromethyl)thiazole-4-carboxylate. The title compound was prepared in a manner analogous to Example 48, Step B however using methyl 2-chloro-5-(trifluoromethyl)thiazole-4-carboxylate as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₂H₁₃ClF₈N₂O₄S, 588.0; m/z found, 589.0 [M+H]⁺. ¹H NMR (400MHz, CDCl₃) δ = 9.09 (s, 1H), 8.24 (d, *J*=11.3 Hz, 1H), 8.09 (d, J=5.3 Hz, 1H), 7.35 - 7.27 (m, 2H), 7.15 (t, J=8.7 Hz, 1H), 5.21 (td, J=6.0, 12.2 Hz, 1H), 4.06 (s, 3H), 1.70 (d, *J*=6.5 Hz, 3H); ¹⁹F NMR (376MHz, CDCl₃) δ = -52.99 (s, 1F), -77.50 - -78.31 (m, 1F), -113.65 (br s, 1F), -116.58 (s, 1F).

Step D. (*S*)-*N*-(2-Chloro-6-fluorophenyl)-5-fluoro-4-(4-(hydroxymethyl)-5-(trifluorometyl) thiazol-2-yl)-2-((1,1,1-trifluoropropan-2-y)oxy)benzamide. NaBH₄ (5.4 mg, 142.7 µmol) and CaCl₂ (7.9 mg, 71.2 µmol) were added to the mixture of (*S*)-methyl 2-(4-((2-chloro-6-fluorophenyl)carbamoyl)-2-fluoro-5-((1,1,1-trifluoropropan-2-yl) oxy)phenyl)-5-(trifluoromethyl)thiazole-4-carboxylate (60 mg, 101.9 µmol) in EtOH (3 mL) at 0 °C under N₂. The mixture was stirred at room temperature overnight. The mixture was concentrated under reduced pressure. The residue was dissolved in sat. aq. NH₄Cl (5 mL) and extracted with ethyl acetate (15 mL × 3). The organic layer was separated, dried over Na₂SO₄, filtered and concentrated under reduced pressure. The crude product was purified by preparative reversed phase HPLC (Stationary phase: Boston Prime C18, 5 µm, 150 × 30 mm; Mobile phase: water (0.04% NH₃H₂O + 10 mM NH₄HCO₃) (A) - MeCN (B), gradient elution: 65 - 95% B in A over 7 min, flow rate: 30 mL/min) to give the title compound as a white powder. MS (ESI): mass calcd. for C₂₁H₁₃CIF₈N₂O₃S, 560.0; m/z found, 561.0 [M+H]⁺. ¹H NMR (400MHz, CDCl₃) δ = 9.10 (s, 1H), 8.24 (d, *J*=11.5 Hz, 1H), 8.06 (d, J=5.3 Hz, 1H), 7.35 - 7.29 (m, 2H), 7.21 - 7.10 (m, 1H), 5.15 (quind, J=6.0, 12.2 Hz, 1H), 4.97 (dd, J=1.0, 6.3 Hz, 2H), 2.58 (t, J=6.3 Hz, 1H), 1.73 (d, J=6.5 Hz, 3H); ¹⁹F NMR (376MHz, CDCl₃) δ = -52.33 (s, 1F), -78.08 (s, 1F), -113.65 (br s, 1F), -116.50 (br s, 1F).

### Example 168: (S)-5-Fluoro-4-(1-methyl-1H-1,2,4-triazol-3-yl)-N-(5-methyl-3-(trifluoromethyl)-1H-pyrazol-4-yl)-2-((1,1,1-trifluoropropan-2-vl)oxy)benzamide.

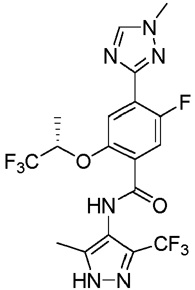

Step A. (*S*)-Methyl 5-fluoro-4-(1-methyl-1*H*-1,2,4-triazol-3-yl)-2-(1,1,1-trifluoropropan-2-vl)oxv)benzoate. The title compound was prepared in a manner analogous to Example 128, Step C however using 3-bromo-1-methyl-1*H*-1,2,4-triazole as the aryl halide and (*S*)-methyl 5-fluoro-4-(tetramethyl-1,3,2-dixaborolan-2-yl)-2- ((1,1,1-trifluoropropan-2-yl)oxy)benzoate (Intermediate 2) as the boronate . MS (ESI): mass calcd. for C₁₄H₁₃F₄N₃O₃; 347.1; m/z found, 348.1 [M+H]⁺. ¹H NMR (400MHz, CDCl₃) δ = 8.13 (s, 1H), 7.77 (d, J=5.8 Hz, 1H), 7.68 (d, *J*=10.8 Hz, 1H), 4.75 (spt, *J*=6.3 Hz, 1H), 4.03 (s, 3H), 3.91 (s, 3H), 1.56 (d, *J*=6.5 Hz, 3H); ¹⁹F NMR (376MHz, CDCl₃) δ = -78.38 (s, 1F), -119.24 (s, 1F).

Step B. (*S*)-5-Fluoro-4-(1-methyl-1*H*-1,2,4-triazol-3-yl)-2-((1,1,1-trifluoropropan-2-yl)oxy)benzoic acid. To a mixture of (*S*)-methyl 5-fluoro-4-(1-methyl-1H-1,2,4-triazol-3-yl)-2-((1,1,1- trifluoropropan-2-yl)oxy)benzoate (65 mg, 187.2 µmol) in THF/MeOH/H₂O (v/v/v, 1/1/1, 2.4 mL) was added NaOH (15 mg, 375 µmol). The reaction mixture was stirred at room temperature for 2 hours. To the aqueous phase was added 2 M aq. HCl to adjust the pH to 3-5, then the mixture was extracted with ethyl acetate (5 mL × 2). The organic layer was separated, dried over Na₂SO₄, filtered and concentrated under reduced pressure to give the title compound as a white solid. MS (ESI): mass calcd. for C₁₃H₁₁F₄N₃O_{3;} 333.1; m/z found, 333.9 [M+H]⁺. ¹H NMR (400MHz, CDCl₃) δ = 8.18 (s, 1H), 7.96 (d, J=10.6 Hz, 1H), 7.84 (d, J=5.3 Hz, 1H), 5.00 (td, J=6.1, 12.2 Hz, 1H), 4.05 (s, 3H), 1.64 (d, J=6.4 Hz, 3H); ¹⁹F NMR (376MHz, CDCl₃) δ = - 78.42 (d, J=5.1 Hz, 1F), -117.61 (br s, 1F).

Step C. (*S*)-5-Fluoro-4-(1-methyl-1*H*-1,2,4-triazol-3-yl)-*N*-(5-methyl-3-(trifluoromethyt)-1*H-*pyrazol-4-yl)-2-((1,1,1-trifluoropropan-2-yl)oxy)benzamide. POCl₃ (89.5 mg, 583.7 µmol) was added drop-wise to the mixture of (*S*)-5-fluoro-4-(1-methyl-1*H*-1,2,4-triazol-3-yl)-2-((1,1,1-trifluoropropan-2-yl)oxy)benzoic acid (50 mg, 145.5 µmol), 5-methyl-3-(trifluoromethyl)-1*H-*pyrazol-4-amine (31 mg, 187.7 µmol) and pyridine (115 mg, 1.45 mmol) was in DCM (1 mL) at room temperature. The resulting reaction mixture was stirred at room temperature for 2 hours. The mixture was quenched with sat. aq. NaHCO₃ (2 mL) and extracted with DCM (5 mL × 3). The organic layer was dried over Na₂SO₄, filtered and concentrated under reduced pressure. The crude product was further purified by preparative reversed phase HPLC (Stationary phase: Phenomenex Gemini-NX C18, 5 µm, 150 × 30 mm; Mobile phase: water (0.04% NH₃H₂O + 10mM + NH₄HCO₃) (A) - ACN (B), gradient elution: 35 - 65% B in A over 2 min, flow rate: 25 mL/min) to give the title compound as an off-white powder. MS (ESI): mass calcd. for C₁₈H₁₅F₇N₆O₂; 480.1; m/z found, 481.0 [M+H]⁺. ¹H NMR (400MHz, CDCl₃) δ = 10.88 (br s, 1H), 8.95 (s, 1H), 8.19 (s, 1H), 8.15 (d, *J*=11.3 Hz, 1H), 7.84 (d, J=5.3 Hz, 1H), 5.16 - 5.03 (m, 1H), 4.08 (s, 3H), 2.31 (s, 3H), 1.68 (d, *J*=6.5 Hz, 3H); ¹⁹F NMR (376MHz, CDCl₃) δ = -62.02 (s, 1F), -78.52 (s, 1F), -118.55 (s, 1F).

### Example 169: (S)-N-(3-chloro-5-methyl-1H-yl)-pyrazol-4-yl)-5-fluoro-4-(6-(hydroxymethyl)-5-methylpyrazin-2-yl)-2-((1,1,1-trifluoropropan-2-vl)oxy)benzamide.

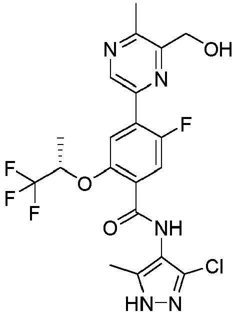

Step A. methyl (*S*)-5-fluoro-4-(6-(hydroxymethyl)-5-methylpyrazin-2-yl)-2-((1,1,1-trifluoropropan-2-yl)oxy)benzoate. The title compound was prepared in a manner analogous to Example 48, Step B however using (6-Chloro-3-methylpyrazin-2-yl)methanol (example 101, Step B) as the aryl halide and (*S*)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₁₇H₁₆F₄N₂O₄, 388.1; m/z found, 389.2 [M+H]⁺. 1H NMR (400 MHz, CHLOROFORM-d) d ppm 8.97 (d, J=2.3 Hz, 1 H), 7.67 - 7.73 (m, 2 H), 4.86 (d, J=4.8 Hz, 2 H), 4.67 - 4.77 (m, 1 H), 3.94 (s, 3 H), 2.58 (s, 3 H), 1.60 - 1.60 (m, 3 H). 19F NMR (376 MHz, CHLOROFORM-d) d ppm -78.04 (s, 1 F), -121.13 (s, 1 F).

Step B. (S)-N-(3-chloro-5-methyl-1H-pyrazol-4-yl)-5-fluoro-4-(6-(hydroxymethyl)-5-methylpyrazin-2-yl)-2-((1,1,1-trifluoropropan-2-yl)oxy)benzamide. The title compound was prepared in a manner analogous to Example 62, Steps C-D, however using methyl (S)-5-fluoro-4-(6-(hydroxymethyl)-5-methylpyrazin-2-yl)-2-((1,1,1-trifluoropropan-2-yl)oxy)benzoate instead of Methyl (S)-4-(5-cyclopropylpyridin-2-yl)-5-fluoro-2-((1,1,1-trifluoropropan-2-yl)oxy)benzoate. MS (ESI): mass calcd. for C₂₀H₁₈F₄N₅O₃, 487.1; m/z found, 488.2 [M+H]⁺. 1H NMR (400MHz, CHLOROFORM-d) d = 10.50 (br s, 1H), 9.02 (s, 1H), 8.79 (s, 1H), 8.13 (d, J=11.5 Hz, 1H), 7.73 (d, J=5.5 Hz, 1H), 5.01 (td, J=6.0, 12.0 Hz, 1H), 4.87 (br s, 2H), 4.01 (br s, 1H), 2.59 (s, 3H), 2.32 (s, 3H), 1.70 (br s, 3H) 19F NMR (376MHz, CHLOROFORM-d) d = -78.10 (br d, J=6.6 Hz, 1F), -120.57 (dt, J=4.0, 7.9 Hz, 1F).

### Example 170: (S)-5-Fluoro-4-(5-(hydroxymethyl)-1-methyl-1H-1,2,4-triazol-3-yl)-N-(o-tolyl)-2-((1,1,1-trifluoropropan-2-yl)oxy)benzamide.

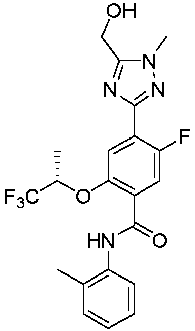

Step A. (*S*)-5-Fluoro-4-(5-(hydroymethyl)-1-methyl-1*H*-1,2,4-triazol-3-yl)-*N*-(*o*-tolyl)-2-((1,1,1-trifluoropropan-2-yl)oxy)benzamide. The title compound was prepared in a manner analogous to Example 128, Step C however using (3-bromo-1-methyl-1*H*-1,2,4-triazol-5-yl)methanol (Example 66, Step B) as the aryl halide and (*S*)-5-fluoro-4-(4,4,5,5-tetramethyl-1,3,2-dioxaborolan-2-yl)-*N*-(*o*-tolyl)- 2-((1,1,1-trifluoropropan-2-yl)oxy)benzamide (Example 77, Step A) as the boronate . MS (ESI): mass calcd. for C₂₁H₂₀F₄N₄O₃, 452.1; m/z found, 453.1 [M+H]⁺. ¹H NMR (400MHz, CDCl₃) δ = 9.12 (s, 1H), 8.09 (d, *J*=11.2 Hz, 1H), 7.89 (br d, *J*=7.9 Hz, 1H), 7.76 (d, *J=5.5* Hz, 1H), 7.29 (br s, 1H), 7.25 (br s, 1H), 7.19 - 7.12 (m, 1H), 5.09 (td, *J*=6.2, 12.3 Hz, 1H), 4.86 (br d, *J*=6.0 Hz, 2H), 4.18 (br t, *J*=6.2 Hz, 1H), 4.01 (s, 3H), 2.33 (s, 3H), 1.65 (d, *J*=6.4 Hz, 3H); ¹⁹F NMR (376MHz, CDCl₃) δ = -78.17 (br d, J=5.9 Hz, 3F), - 118.56 (br dd, *J*=5.5, 11.4 Hz, 1F).

### Example 171: (S)-5-Fluoro-4-(5-(hydroxymethyl)-1-methyl-1H-pyrazol-3-yl)-N-(o-tolyl)-2-((1,1,1-trifluoropropan-2-yl)oxy)benzamide.

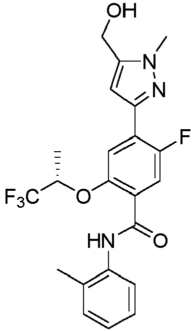

Step A. (*S*)-Methyl-5-fluoro-4-(5-(hydroxymethyl)-1-methyl-1*H*-pyrazol-3-yl)-2-((1,1,1-trifluoropropan-2-vl)oxv)benzoate. To a solution of (*S*)-methyl 5-fluoro-4-(4,4,5,5-tetramethyl-1,3,2-dioxaborolan-2-yl)- 2-((1,1,1-trifluoropropan-2-yl)oxy)benzoate (Intermediate 2, 300 mg, 650.3 µmol) in dioxane/H₂O (v/v, 5/1, 10 mL) was added (3-bromo-1-methyl-1*H*-pyrazol-5-yl)methanol (137 mg, 715.3 µmol), K₂CO₃ (270 mg, 1.95 mmol) and XPhos Pd G3 (55 mg, 65.0 µmol) respectively, and the reaction mixture was stirred at 60 °C for 12 hours under N₂. The reaction mixture was cooled down to room temperature. Ethyl acetate (40 mL) was added to the mixture, then filtered through a pad of Celite^{®}. The solid was rinsed with ethyl acetate (30 mL × 2). The filtrate was washed with brine (50 mL), dried with Na₂SO₄, filtered and concentrated under reduced pressure. The crude product was purified by flash column chromatography (SiO₂, eluent: 0 - 50% ethyl acetate in petroleum ether) to give the title compound as yellow. MS (ESI): mass calcd. for C₁₆H₁₆F₄N₂O₄, 376.1; m/z found, 377.1 [M+H]⁺. ¹H NMR (400MHz, CDCl₃) δ= 7.69 (d, *J*=6.0 Hz, 1H), 7.62 (d, *J*=11.2 Hz, 1H), 6.73 (d, *J*=4.2 Hz, 1H), 4.83 - 4.75 (m, 1H), 4.74 (d, *J*=5.1 Hz, 2H), 4.00 (s, 3H), 3.91 (s, 3H), 1.56 (s, 3H); ¹⁹F NMR (376MHz, CDCl₃) δ = - 78.37 (s, 3F), -122.74 (s, 1F).

Step B. (*S*)-5-Fluoro-4-(5-(hydroxymethyl)-1-methyl-1*H*-pyrazol-3-yl)-2-((1,1,1-trifluoropropan-2-vl)oxv)benzoic acid. To a mixture of (*S*)-methyl 5-fluoro-4-(5-(hydroxymethyl)-1-methyl-1*H*-pyrazol-3-yl)- 2-((1,1,1-trifluoropropan-2-yl)oxy)benzoate (215 mg, 571.4 µmol) in MeOH (11 mL) was added 2 M aq. NaOH (1.43 mL, 2.86 mmol). The mixture was stirred at room temperature for 12 hours. The mixture was concentrated under reduced pressure, then 2 M aq. HCl was added to adjust the pH to 2-3. The mixture was extracted with ethyl acetate (25 mL × 3). The combined organic phases were washed with brine (30 mL), dried over anhydrous Na₂SO₄, filtered and concentrated under reduced pressure to give the title compound as a yellow solid. MS (ESI): mass calcd. for C₁₅H₁₄F₄N₂O₄, 362.1; m/z found, 363.1 [M+H]⁺. ¹H NMR (400MHz, CDCl₃) δ= 7.94 (d, J--11.1 Hz, 1H), 7.77 (d, J=5.6 Hz, 1H), 6.78 (d, J=4.4 Hz, 1H), 5.06 (td, J=6.1, 12.2 Hz, 1H), 4.76 (s, 2H), 4.02 (s, 3H), 1.67 (d, J=6.4 Hz, 3H); ¹⁹F NMR (376MHz, CDCl₃) δ = -78.45 (s, 3F), -120.97 (s, 1F).

Step C. (*S*)-5-Fluoro-4-(5-(hydroxymethyl)-1-methyl-1*H*-pyrazol-3-yl)-*N*-(*o*-tolyl)-2-((1,1,1-trifluoropropan-2-vl)oxy)benzamide. A mixture of (*S*)-5-fluoro-4-(5-(hydroxymethyl)-1-methyl-1*H*-pyrazol-3-yl)-2- ((1,1,1-trifluoropropan-2-yl)oxy)benzoic acid (180 mg, 496.9 µmol), *o-*toluidine (58.6 mg, 546.5 µmol), DIEA (193 mg, 1.49 mmol) and T₃P (50% solution in ethyl acetate, 474.3 mg, 745.3 µmol) in DCM (8 mL) was stirred at 50 °C for 12 hours. LCMS showed 51% desired mass was detected. Sat. aq. NH₄Cl (30 mL) was added to the mixture and extracted with DCM (20 mL × 3). The organic layer was washed with brine (50 mL), dried with Na₂SO₄, filtered and concentrated under reduced pressure. The crude product was purified by column chromatography (SiO₂, eluent: 0 - 50% ethyl acetate in petroleum ether) to give the crude. The crude was further purified by preparative high-performance liquid chromatography (Stationary phase: Boston Prime C18, 5 µm, 150 × 30 mm; Mobile phase: water (0.04% NH₃H₂O + 10 mM NH₄HCO₃) (A) - MeCN (B), gradient elution: 55 - 85% B in A over 7 min, flow rate: 25 mL/min) to give the title compound as a white powder. MS (ESI): mass calcd. for C₂₂H₂₁F₄N₃O₃, 451.2; m/z found, 452.1 [M+H]⁺. ¹H NMR (400MHz, CDCl₃) δ= 9.63 (s, 1H), 7.75 (d, J=5.8 Hz, 1H), 7.59 - 7.50 (m, 2H), 7.26 (d, *J*=7.5 Hz, 1H), 7.24 - 7.18 (m, 1H), 7.17 - 7.11 (m, 1H), 6.67 (d, J=3.9 Hz, 1H), 5.47 - 5.40 (m, 1H), 5.38 (t, *J*=5.6 Hz, 1H), 4.57 (d, J=5.6 Hz, 2H), 3.90 (s, 3H), 2.27 (s, 3H), 1.48 (d, J=6.4 Hz, 3H); ¹⁹F NMR (376MHz, CDCl₃) δ = -77.09 (br s, 3F), - 123.24 (s, 1F).

### Example 172: (S)-5-Fluoro-4-(2-(hydroymethyl)-1-methyl-1H-imidazol-4-yl)-N-(2-methoxy-3,5-dimethylpyridin-4-yl)-2-((1,1,1-trifluoropropan-2-yl)oxy)benzamide.

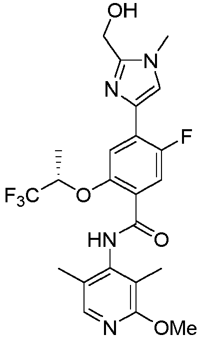

Step A. (*S*)-4-Bromo-5-fluoro-*N*-(2-methoxy-3,5-dimethylpyridin-4-yl)-2-((1,1,1-trifluoropropan-2-vl)oxv)benzamide. The mixture of (*S*)-4-bromo-5-fluoro-2-((1,1,1-trifluoropropan-2-yl)oxy)benzoic acid (Example 76, Step B, 500 mg, 1.34 mmol), 2-methoxy-3,5-dimethylpyridin-4-amine, 204 mg, 1.34 mmol) and pyridine (528 mg, 6.68 mmol) was dissolved in DCM (10 mL). POCl₃ (410 mg, 2.67 mmol) was added to the mixture at room temperature. The resulting reaction mixture was stirred at room temperature for 7 hours. The mixture was quenched with sat. aq. NaHCO₃ (20 mL) and extracted with DCM (30 mL × 3). The combined organic layers were dried over Na₂SO₄, filtered, and concentrated under reduced pressure. The residue was dissolved in MeOH (8 mL), K₂CO₃ (3 g) was added to the mixture. The mixture was stirred at room temperature for 0.5 hours and filtered. The solid was rinsed with ethyl acetate (10 mL × 3). The filtrate was concentrated and purified by flash column chromatography (SiO₂, eluent: 0 - 25% ethyl acetate in petroleum ether) to give the title compound as yellow solid. MS (ESI): mass calcd. for C₁₈H₁₇BrF₄N₂O₃, 464.0; m/z found, 466.3 [M+H]⁺. ¹H NMR (400MHz, CDCl₃) δ = 8.76 (s, 1H), 8.08 (d, *J*=8.9 Hz, 1H), 7.92 (s, 1H), 7.25 (d, *J*=5.0 Hz, 1H), 5.01 - 4.82 (m, 1H), 3.96 (s, 3H), 2.15 (s, 3H), 2.10 (s, 3H), 1.66 (d, J=6.4 Hz, 3H); ¹⁹F NMR (376MHz, CDCl₃) δ = -78.24 (s, 1F), -113.28 (s, 1F).

Step B. (*S*)-5-Fluoro-*N*-(2-methoxy-3,5-dimethylpyridin-4-yl)-4-(4,4,5,5-tetramethyl-1,3,2-dioxaborolan-2-yl)-2-(Y 1,1,1 -trifluoropropan-2-vl)oxy)benzamide. To the mixture of (*S*)-4-bromo-5-fluoro-*N*-(2-methoxy-3,5-dimethylpyridin-4-yl)- 2-((1,1,1-trifluoropropan-2-yl)oxy)benzamide (100 mg, 203.2 µmol) in dioxane (3.5 mL) was added bis(pinacolato)diboron (62 mg, 243.8 µmol) and KOAc (80 mg, 812.7 µmol), Pd(dppf)Cl₂ (15 mg, 20.5 µmol) was added under N₂. The resulting mixture was charged with N₂ and heated at 100 °C and stirred for 16 hours. The mixture was filtered, the filtrate was concentrated under reduced pressure to give the title compound (190 mg crude). MS (ESI): mass calcd. for C₂₄H₂₉BF₄N₂O₅, 512.2; m/z found, 431.0 [M+H]⁺.

Step C. (*S*)-5-Fluoro-4-(5-(hydroxymethyl)-1-methyl-1*H*-pyrazol-3-yl)-*N*-(*o*-tolyl)-2-((1,1,1-trifluoropropan-2-yl)oxy)benzamide. The title compound was prepared in a manner analogous to Example 128, Step C however using (4-bromo-1-methyl-1*H*-imidazol-2-yl)methanol (Example 5, Step A) as the aryl halide and (*S*)-5-fluoro-*N*-(2-methoxy-3,5-dimethylpyridin-4-yl)- 4-(4,4,5,5-tetramethyl-1,3,2-dioxaborolan-2-yl)-2-((1,1,1-trifluoropropan-2-yl)oxy)benzamide as the boronate . MS (ESI): mass calcd. for C₂₃H₂₄F₄N₄O₄, 496.2; m/z found, 497.1 [M+H]⁺.

¹H NMR (400MHz, CDCl₃) δ = 8.99 (s, 1H), 8.04 (d, *J*=11.8 Hz, 1H), 7.90 (s, 1H), 7.81 (d, *J*=5.4 Hz, 1H), 7.49 (d, J=3.9 Hz, 1H), 5.41 - 5.10 (m, 1H), 4.77 (br s, 2H), 3.96 (s, 3H), 3.83 (br s, 1H), 3.77 (s, 3H), 2.16 (s, 3H), 2.12 (s, 3H), 1.67 (br d, J=6.6 Hz, 3H); ¹⁹F NMR (376MHz, CDCl₃) δ = -78.40 (s, 1F), -120.79 (s, 1F).

### Example 173: (S)-5-fluoro-4-(6-(hydroxymethyl)-5-methylpyrazin-2-yl)-N-(2-methoxy-3,5-dimethylpyridin-4-yl)-2-((1,1,1-trifluoropropan-2-yl)oxy)benzamide.

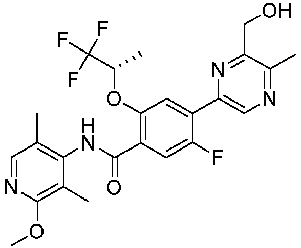

The title compound was prepared in a manner analogous to Example 128, Step C however using (6-Chloro-3-methylpyrazin-2-yl)methanol (example 101, Step B) as the aryl halide (*S*)-5-Fluoro-*N-*(2-methoxy-3,5-dimethylpyridin-4-yl)-4-(4,4,5,5-tetramethyl-1,3,2-dioxaborolan-2-yl)-2-((1,1,1-trifluoropropan-2-yl)oxy)benzamide (Example 172, Step B)as the boronate. MS (ESI): mass calcd. for C₂₄H₂₄F₄N₄O₄, 508.2; m/z found, 509.2 [M+H]⁺. 1H NMR (400MHz, CHLOROFORM-d) d = 9.05 (s, 1H), 8.95 (s, 1H), 8.20 (d, J=11.3 Hz, 1H), 7.94 (s, 1H), 7.75 (d, J=5.3 Hz, 1H), 5.08 (td, J=6.2, 12.2 Hz, 1H), 4.90 (br s, 2H), 3.98 (s, 4H), 2.62 (s, 3H), 2.19 (s, 3H), 2.15 (s, 3H), 1.70 (br d, J=6.3 Hz, 3H). 19F NMR (376MHz, CHLOROFORM-d) d = - 78.14 (s, 1F), -120.62 (s, 1F)

### Example 174: (S)-N-(2-Chlorophenyl)-5-fluoro-4-(2-(2-hydroxypropan-2-yl)-1-methyl-1H-imidazol-4-yl)-2-((1,1,1-trifluoropropan-2-yl)oxy)benzamide.

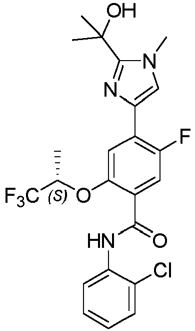

Step A. (S)-Methyl 5-fluoro-4-(2-(2-hydroxypropan-2-yl)-1-methyl-1*H*-imidazol-4-yl)-2-((1,1,1-trifluoropropan-2-yl)oxy)benzoate. The title compound was prepared in a manner analogous to Example 171, Step A however using 2-(4-bromo-1-methyl-1*H*- imidazol-2-yl)propan-2-ol (Example 130, Step A) as the aryl halide and (S)-methyl 5-fluoro-4-(4,4,5,5-tetramethyl-1,3,2-dioxaborolan-2-yl)-2- ((1,1,1-trifluoropropan-2-yl)oxy)benzoate (Intermediate 2) as the boronate . MS (ESI): mass calcd. for C₁₈H₂₀F₄N₂O₄, 404.1; m/z found, 405.0 [M+H]⁺. ¹H NMR (400MHz, CDCl₃) δ = 7.78 (d, J=6.0 Hz, 1H), 7.58 (d, J=11.5 Hz, 1H), 7.38 (d, J=4.0 Hz, 1H), 4.83 - 4.71 (m, 1H), 3.89 (s, 3H), 3.86 (s, 3H), 3.03 (s, 1H), 1.71 (s, 6H), 1.56 (d, J=6.5 Hz, 3H).

Step B. (*S*)-*N*-(2-Chlorophenyl)-5-fluoro-4-(2-(2-hydroxypropan-2-yl)-1-methyl-1*H*-imidazol-4-yl)-2-((1,1,1-trifluoropropan-2-yl)oxy)benzamide. To a solution of 2-chloroaniline (94.7 mg, 741.9 µmol) in DCE (2.4 mL) was added dropwise AlMe₃ (2 M solution in toluene, 0.36 mL, 0.72 mmol) at 0 °C, the mixture was stirred at room temperature for 0.5 hour. (S)-methyl 5-fluoro-4-(2-(2-hydroxypropan-2-yl) -1-methyl-1H-imidazol-4-yl)-2-((1,1,1-trifluoropropan-2-yl)oxy)benzoate (100 mg, 242.1 µmol) was added. Then the resulting mixture was stirred at 80 °C for 12 hours. The reaction was quenched by added 1 M aq. HCl (0.6 mL), diluted with H₂O (3 mL), extracted with DCM (10 mL × 3). The combined organic layer was dried over Na₂SO₄, filtered and concentrated under reduced pressure. The crude was purified by flash column chromatography (SiO₂, gradient elution: 0 - 30% ethyl acetate in petroleum ether) to give the title compound as a white solid. MS (ESI): mass calcd. for C₂₃H₂₂ClF₄N₃O₃, 499.1; m/z found, 500.0 [M+H]⁺. ¹H NMR (400MHz, CDCl₃) δ = 9.82 (br s, 1H), 8.53 (br d, J=8.0 Hz, 1H), 7.95 (br d, J=11.8 Hz, 1H), 7.86 (br d, J=5.0 Hz, 1H), 7.42 (br s, 2H), 7.33 (br t, J=7.5 Hz, 1H), 7.09 (br t, J=7.3 Hz, 1H), 5.18 - 4.91 (m, 1H), 3.89 (s, 3H), 3.11 (br s, 1H), 1.73 (br s, 6H), 1.70 - 1.65 (m, 3H); ¹⁹F NMR (376MHz, CDCl₃) δ = -77.72 (br s, 1F), -116.42 - -125.56 (m, 1F).

### Example 175: (S)-5-Fluoro-4-(2-(2-hydroxypropan-2-yl)-1H-methyl-1H-imidazol-4-yl)-N-(o-D₃-tolyl)-2-((1,1,1-trifluoropropan-2-yl)oxy)benzamide.

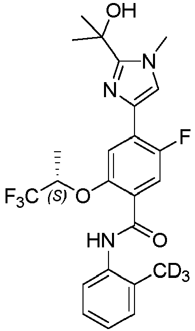

The title compound was prepared in a manner analogous to Example 174, Step B however using o-D₃-toluidine as the amine and (S)-Methyl 5-fluoro-4-(2-(2-hydroxypropan-2-yl)-1-methyl-1*H-*imidazol-4-yl)-2-((1,1,1-trifluoropropan-2-yl)oxy)benzoate (Example 174, Step B) as the ester. MS (ESI): mass calcd. for C₂₄H₂₂D₃F₄N₃O₃, 482.2; m/z found, 483.1 [M+H]⁺. ¹H NMR (400MHz, CDCl₃) δ = 9.17 (br s, 1H), 8.00 (br d, J=12.0 Hz, 1H), 7.91 (br d, J=7.8 Hz, 1H), 7.83 (br d, J=5.5 Hz, 1H), 7.43 (br d, J=3.8 Hz, 1H), 7.27 - 7.20 (m, 2H), 7.17 - 7.08 (m, 1H), 5.07 (td, J=6.1, 12.4 Hz, 1H), 3.89 (s, 3H), 3.13 (s, 1H), 1.73 (s, 6H), 1.65 - 1.63 (m, 3H); ¹⁹F NMR (376MHz, CDCl₃) δ = -78.15 (s, 1F), -120.65 (s, 1F).

### Example 176: (S)-N-(2-chloro-4-methylpyridin-3-yl)-5-fluoro-4-(2-(2-hydroxypropan-2-yl)-1-methyl-1H-imidazol-4-yl)-2-((1,1,1-trifluoropropan-2-yl)oxy)benzamide.

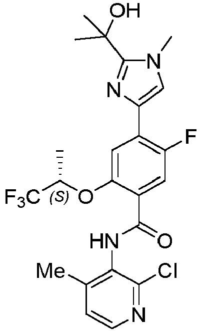

The title compound was prepared in a manner analogous to Example 174, Step B however using 2-chloro-4-methylpyridin-3-amine as the amine and (S)-Methyl 5-fluoro-4-(2-(2-hydroxypropan-2-yl)-1-methyl-1H-imidazol-4-yl)-2-((1,1,1-trifluoropropan-2-yl)oxy)benzoate ( Example 174, Step B) as the ester. MS (ESI): mass calcd. for C₂₃H₂₃ClF₄N₄O₃, 514.1; m/z found, 515.1 [M+H]⁺. ¹H NMR (400MHz, CDCl₃) δ = 9.25 (br s, 1H), 8.21 (br d, J=4.8 Hz, 1H), 8.01 (br d, J=11.8 Hz, 1H), 7.84 (br d, J=5.5 Hz, 1H), 7.45 (br d, J=3.8 Hz, 1H), 7.19 (br d, J=4.8 Hz, 1H), 5.23 - 5.06 (m, 1H), 3.90 (s, 3H), 3.07 (s, 1H), 2.36 (s, 3H), 1.74 (s, 6H), 1.70 (br d, J=6.3 Hz, 3H); ¹⁹F NMR (376MHz, CDCl₃) δ = -78.19 (br s, 1F), -120.96 (br s, 1F).

### Example 177: (S)-N-(3-chloro-2-methoxy-5-methylpyridin-4-yl)-5-fluoro-4-(2-(2-hydroxypropan-2-yl)-1-methyl-1H-imidazol-4-yl)-2-((1,1,1-trifluoropropan-2-yl)oxy)benzamide.

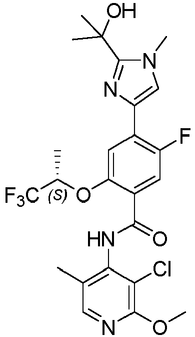

To a solution of 3-chloro-2-methoxy-5-methylpyridin-4-amine (83.6 mg, 483.7 µmol) in THF (4.5 mL) was added dropwise LiHMDS (1 M solution in THF, 0.72 mL, 0.72 mmol) at 0 °C. The mixture was stirred at 0 °C for 0.5 hour. (S)-methyl 5-fluoro-4-(2-(2-hydroxypropan -2-yl)-1-methyl-1*H*-imidazol-4-yl)-2-((1,1,1-trifluoropropan-2-yl)oxy)benzoate (Example 174, Step B, 100 mg, 242.1 µmol) in THF (1.5 mL) was added. Then the resulting mixture was stirred at 15 °C for 12 hours. The reaction was quenched by added 1 M aq. HCl (0.6 mL), diluted with H₂O (3 mL), extracted with DCM (3 mL × 3). The combined organic layers were dried over Na₂SO₄, filtered and concentrated under reduced pressure. The crude was purified by flash column chromatography (SiO₂, gradient elution: 0 - 30% ethyl acetate in petroleum ether) to give the title compound as an off-white solid. MS (ESI): mass calcd. For C₂₄H₂₅ClF₄N₄O₄, 544.2; m/z found, 545.1 [M+H]⁺. ¹H NMR (400MHz, CDCl₃) δ = 9.32 (s, 1H), 8.01 (d, J=11.8 Hz, 1H), 7.96 (s, 1H), 7.83 (br d, J=5.6 Hz, 1H), 7.44 (d, J=3.7 Hz, 1H), 5.19 - 5.04 (m, 1H), 4.03 (s, 3H), 3.89 (s, 3H), 3.10 (s, 1H), 2.22 (s, 3H), 1.73 (s, 6H), 1.68 (br d, J=6.2 Hz, 3H); ¹⁹F NMR (376MHz, CDCl₃) δ = -78.17 (s, 1F), -120.91 (s, 1F).

### Example 178: (S)-N-(2-Chloro-6-fluorophenyl)-4-cyclopropyl-2-(2-hydroxypropan-2-yl)-1H-imidazol-4-yl)-5-fluoro-2-(1,1,1-trifluoropropan-2-yl)oxy)benzamide.

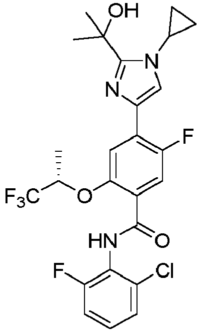

Step A. Ethyl 1-cyclopropyl-1H-imidazole-2-carboxylate. To a mixture of ethyl 1H-imidazole-2-carboxylate (50 g, 356.8 mmol), cyclopropylboronic acid (76.6 g, 892 mmol), Na₂CO₃ (94.5 g, 892 mmol) in DCE (500 mL) at 70 °C was added a heated suspension of Cu(OAc)₂ (64.8 g, 356.8 mmol) and 2,2'-bipyridine (55.7 g, 356.8 mmol) in DCE (250 mL). The reaction mixture was then stirred vigorously under air at 70 °C for 16 hours. The reaction mixture was cooled to room temperature and filtered. The filtrate was concentrated under reduced pressure. The crude product was purified by flash column chromatography twice (SiO₂, 0 - 80% ethyl acetate in petroleum ether) to give the title compound as yellow oil. MS (ESI): mass calcd. for C₉H₁₂N₂O₂, 180.1; m/z found, 181.1 [M+H]⁺. ¹H NMR (400MHz, CDCl₃) δ = 7.08 (br d, J=15.1 Hz, 2H), 4.45 - 4.41 (m, 2H), 3.90 - 3.85 (m, 1H), 1.47 - 1.43 (m, 3H), 1.19 - 1.12 (m, 2H), 1.00 - 0.93 (m, 2H).

Step B. Ethyl 4-bromo-1-cyclopropyl-1H-imidazole-2-carboxylate. NBS (7.3 g, 41.1 mmol) was added to ethyl 1-cyclopropyl-1H-imidazole-2-carboxylate (9.6 g, 37.4 mmol) in DCM (50 mL) at 0 °C. The mixture was stirred at 45 °C for 3 hours. The reaction mixture was concentrated under reduced pressure, and 5 N aq. NaOH (12 mL) was added to the residue at 0 °C, followed by extraction with DCM (30 mL × 3). The organic layer was dried with Na₂SO₄, filtered and concentrated under reduced pressure. The crude product was purified by flash column chromatography (SiO₂, gradient elution: 0 - 35% ethyl acetate in petroleum ether) to give the title compound as yellow solid. MS (ESI): mass calcd. for C₉H₁₁BrN₂O₂, 258.0; m/z found, 261.0 [M+H]⁺. ¹H NMR (400MHz, CDCl₃) δ = 7.04 (s, 1H), 4.50 - 4.40 (m, 2H), 3.86 (tt, J=3.9, 7.3 Hz, 1H), 1.45 (dt, J=0.9, 7.1 Hz, 3H), 1.20 - 1.13 (m, 2H), 1.01 - 0.95 (m, 2H).

Step C. 2-(4-Bromo-1-cyclopropyl-1H-imidazol-2-yl)propan-2-ol. To a solution of ethyl 4-bromo-1-cyclopropyl-1H-imidazole-2-carboxylate (1 g, 3.8 mmol) in THF (16 mL) was added MeMgBr (3 M solution in ether, 3.79 mL, 11.4 mmol) dropwise at 0 °C under N₂. The resulting mixture was stirred at 0 °C for 1 hour. The mixture was diluted by H₂O (10 mL) and extracted with DCM (20 mL × 3). The organic layer was dried over Na₂SO₄, filtered and concentrated under reduced pressure. The crude product was purified by column chromatography (SiO₂, 0 - 100% ethyl acetate in petroleum ether) to give the title compound as yellow solid. MS (ESI): mass calcd. for C₉H₁₃BrN₂O, 244.0; m/z found, 245.0, 247.0 [M+H]⁺. ¹H NMR (400MHz, CDCl₃) δ = 6.81 - 6.72 (m, 1H), 3.73 - 3.60 (m, 1H), 2.92 (br s, 1H), 1.75 - 1.70 (m, 6H), 1.14 - 1.00 (m, 4H).

Step D: (S)-*N*-(2-Chloro-6-fluorophenyl)-4-(1-cyclopropyl-2-(2-hydroxypropan-2-yl)-1*H-*imidazol-4-yl)-5-fluoro-2-((1,1,1 -trifluoropropan-2-yl)oxy)benzamide. The title compound was prepared in a manner analogous to Example 171, Step A however using 2-(4-bromo-1-cyclopropyl-1*H*-imidazol- 2-yl)propan-2-ol as the aryl halide and (*S*)-*N*-(2-chloro -6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate . MS (ESI): mass calcd. for C₂₅H₂₃ClF₅N₃O₃, 543.1; m/z found, 544.2 [M+H]⁺. ¹H NMR (400MHz, CDCl₃) δ = 9.07 (s, 1H), 7.96 (d, *J*=11.9 Hz, 1H), 7.74 (d, J=5.7 Hz, 1H), 7.34 (d, J=3.7 Hz, 1H), 7.23 - 7.20 (m, 1H), 7.15 (dt, J=5.7, 8.1 Hz, 1H), 7.09 - 7.01 (m, 1H), 5.07 - 4.96 (m, 1H), 3.74 (s, 1H), 3.56 - 3.46 (m, 1H), 1.70 (s, 6H), 1.58 (d, *J*=6.6 Hz, 3H), 1.11 - 1.06 (m, 4H); ¹⁹F NMR (376MHz, CDCl₃) δ = -78.24 - 78.30 (m, 1F), -113.64 - 113.68 (m, 1F), -120.72 - 120.75 (m, 1F).

### Example 179: (S)-N-(2-Chloro-6-fluorophenyl)-4-(1-cyclopropyl-5-(2-hydroxypropan-2-yl)-1H-1,2,4-triazol-3-yl)-5-fluoro-2-((1,1,1 -trifluoropropan-2-vl)oxy)benzamide.

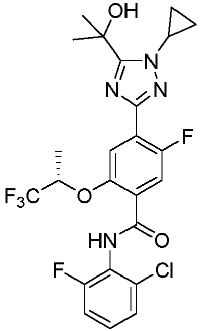

Step A. Ethyl 3-bromo-1-cyclopropyl-1*H*-1,2,4-triazole-5-carboxylate. A mixture of ethyl 3-bromo-1*H*-1,2,4-triazole-5-carboxylate (40 g, 181.8 mmol) in DCE (400 mL) was stirred at room temperature for 15 minutes. Cyclopropylboronic acid (31.2 g, 363.6 mmol) and Na₂CO₃ (38.5 g, 363.6 mmol) were added to the mixture and stirred at room temperature. Cu(OAc)₂ (33.0 g, 181.8 mmol) and 2,2'-bipyridine (28.4 g, 181.8 mmol) in DCE (400 mL) was heating at 70 °C for 0.5 hour and the hot mixture was added to the above mixture. The reaction mixture was stirred at 70 °C overnight under air atmosphere. The mixture was cooled to room temperature and DCM (500 mL) was added. The mixture was filtered, and filter cake was washed with DCM (300 mL × 3). The organic layer was separated, washed with 1 M aq. HCl (300 mL × 2), sat. aq. NaHCO₃ (400 mL × 2) and brine (800 mL). The organic layer was separated, dried over Na₂SO₄, filtered and concentrated under reduced pressure. The crude product was purified by flash column chromatography (SiO₂, eluent: 0 - 5% ethyl acetate in petroleum ether). The mixture was separated by SFC: (Column: DAICEL CHIRALCEL OJ 250 × 30 mm I.D., 10 µm; mobile phase: CO₂ (A) - EtOH (0.1%NH3H2O); isocratic: 15% B in A; flow rate: 200 mL/min; column temp.:40 °C; ABPR: 100 bar.) to give the title compound as yellow oil. ¹H NMR (400MHz, CDCl₃) δ = 4.54 - 4.43 (m, 3H), 1.46 (t, J=7.2 Hz, 3H), 1.39 - 1.33 (m, 2H), 1.20 - 1.12 (m, 2H).

Step B. 2-(3-bromo-1-cyclopropyl-1*H*-1,2,4-triazol-5-yl)propan-2-ol. To a mixture of ethyl 3-bromo-1-cyclopropyl-1*H*-1,2,4-triazole-5-carboxylate (1 g, 3.84 mmol) in THF (18 mL) was added methylmagnesium bromide (3 M solution in ether, 5.1 mL, 15.3 mmol) at 0 °C under N₂. The mixture was stirred at 0 °C for 1 hour. Sat. aq. NH₄Cl (20 mL) was added to the mixture drop wise at 0 °C under N₂. The mixture extracted with ethyl acetate (35 mL × 3). The organic layer was separated, dried over Na₂SO₄, filtered and concentrated under reduce pressure. The crude product was purified by preparative reversed phase HPLC (Stationary phase: Boston Prime C18, 5 µm, 150 × 40 mm; Mobile phase: water (0.05%NH₃H₂O) (A) - MeCN (B), gradient elution: 20- 50% B in A over 9 min, flow rate: 60 mL/min) to give the title compound as white solid. MS (ESI): mass calcd. for C₈H₁₂BrN₃O, 245.0; m/z found, 246.0, 248.0 [M+H]⁺. ¹H NMR (400MHz, CDCl₃) δ = 5.82 (s, 1H), 4.35 (tt, J=3.9, 7.6 Hz, 1H), 1.55 (s, 6H), 1.19 - 1.12 (m, 2H), 1.06 - 0.99 (m, 2H).

Step C. (*S*)-*N*-(2-chloro-6-fluorophenyl)-4-(1-cyclopropyl-5-(2-hydroxypropan-2-yl)-1*H*-1,2,4-triazol-3-yl)-5-fluoro-2-((1,1,1-trifluoropropan-2-yl)oxy)benzamide. The title compound was prepared in a manner analogous to Example 128, Step C however using 2-(3-bromo-1-cyclopropyl-1*H*-1,2,4-triazol-5-yl)propan-2-ol as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₈H₁₂BrN₃O, 245.0; m/z found, 246.0, 248.0 [M+H]⁺. ¹H NMR (400MHz, DMSO-*d*₆) δ = 10.03 (s, 1H), 7.75 (d, *J=5.5* Hz, 1H), 7.52 - 7.31 (m, 4H), 5.81 (s, 1H), 5.40 - 5.31 (m, 1H), 4.45 (td, J=3.4, 7.3 Hz, 1H), 1.66 (s, 6H), 1.46 (d, J=6.3 Hz, 3H), 1.27 (br d, J=3.1 Hz, 2H), 1.09 (br d, J=5.0 Hz, 2H); ¹⁹F NMR (376MHz, DMSO-*d*₆) δ = -76.99 (s, 1F), -115.42 (br s, 1F), -119.36 (s, 1F).

### Example 180: (S)-N-(5-Chloro-3-methyl-1H-pyrazol-4-yl)-5-fluoro-4-(4-(2-hydroxypropan-2-yl)-5-methylthiazol-2-yl)-2-((1,1,1-trifluoropropan-2-yl)oxy)benzamide.

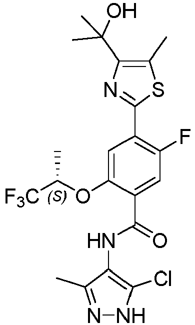

Step A. Ethyl 2-chloro-5-methylthiazole-4-carboxylate. To a solution of t-BuONO (2.21 g, 21.5 mmol) and CuCl₂ (2.16 g, 16.1 mmol) in MeCN (30 mL) was added ethyl 2-amino-5-methylthiazole-4-carboxylate (2.0 g, 10.7 mmol). Then the mixture was stirred at 80 °C for 16 hours. The mixture was quenched with sat. aq. NH₄Cl solution (30 mL). Then the mixture was poured into water (20 mL) and extracted with ethyl acetate (20 mL × 2). The combined organic phase was washed with brine (20 mL × 3), dried with anhydrous Na₂SO₄, filtered and concentrated under reduced pressure. The crude product was purified by flash column chromatography (SiO₂, gradient elution: 0 - 10% ethyl acetate in petroleum ether) to give the title compound as light yellow solid. MS (ESI): mass calcd. for C₇H₈ClNO₂S, 205.0; m/z found, 206.1 [M+H]⁺. ¹H NMR (400MHz, CDCl₃) δ = 4.34 (q, *J*=7.2 Hz, 2H), 2.70 (s, 3H), 1.37 (t, *J*=7.2 Hz, 3H).

Step B. 2-(2-Chloro-5-methylthiazol-4-yl)propan-2-ol. To a solution of ethyl 2-chloro-5-methylthiazole-4-carboxylate (1.67 g, 8.14 mmol) in THF (25 mL) was added MeMgBr (3M in ether, 13.56 ml, 40.7 mmol) at 0 °C. The mixture was stirred at 0 °C for 1 hour then stirred at room temperature for 1 h. The mixture was quenched with sat. aq. NH₄Cl (15 mL) at 0 °C. Then the mixture was poured into water (20 mL) and extracted with ethyl acetate (20 mL × 3). The combined organic phase was washed with brine (20 mL × 3), dried with anhydrous Na₂SO₄, filtered and concentrated under reduced pressure. The crude product was purified by flash column chromatography (SiO₂, gradient elution: 0 - 25% ethyl acetate in petroleum ether) to give the title compound as light yellow solid. MS (ESI): mass calcd. for C₇H₁₀ClNOS, 191.0; m/z found, 192.0 [M+H]⁺. ¹H NMR (400MHz, DMSO-d₆) δ = 5.90 (s, 1H), 2.31 (s, 3H), 1.49 (s, 6H).

Step C: *(S)*-N-(5-Chloro-3-methvl-1*H*-pyrazol-4-yl)-5-fluoro-4-(4-(2-hydroxypropan-2-yl)-5-methylthiazol-2-yl)-2-((1,1,1-trifluoropropan-2-yl)oxy(benzamide. The title compound was prepared in a manner analogous to Example 171, Step A however using 2-(2-chloro-5-methylthiazol-4-yl)propan-2-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₁H₂₁ClF₄N₄O₃S, 520.1; m/z found, 521.1 [M+H]⁺. ¹H NMR (400MHz, CDCl₃) δ = 10.44 (br s, 1H), 8.83 (s, 1H), 8.10 (d, *J*=11.6 Hz, 1H), 7.97 (d, *J=5.5* Hz, 1H), 5.21 - 5.06 (m, 1H), 2.65 (s, 3H), 2.32 (s, 4H), 1.76 (s, 6H), 1.71 (d, *J*=6.4 Hz, 3H); ¹⁹F NMR (376MHz, CDCl₃) δ = -78.22 (s, 1F), -118.47 (s, 1F).

### Example 181: N-(2-Chloro-6-fluorophenyl)-4-(1-cyclopropyl-5-((*R)-1-hvdroxyethyl)-1H-1,2,4-triazol-3-yl)-5-fluoro-2-(((S)-1,1,1-trifluoropropan-2-yl)oxy)benzamide.

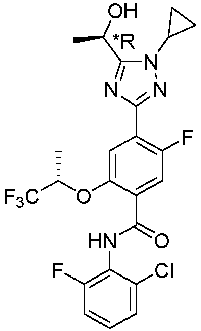

Step A. (3-Bromo-1-cyclopropyl-1*H*-1,2,4-triazol-5-yl)methanol. NaBH₄ (175 mg, 4.63 mmol) and CaCl₂ (256 mg, 2.31 mmol) were added to the mixture of ethyl 3-bromo-1-cyclopropyl-1*H-*1,2,4-triazole-5-carboxylate (Example 179, Step A, 1 g, 3.85 mmol) in EtOH (20 mL) at 0 °C under N₂. The mixture was stirred at room temperature for 12 hours. EtOH was removed under vacuum. The residue was dissolved in sat. aq. NH₄Cl (60 mL) and extracted with ethyl acetate (50 mL × 3). The organic layer was separated, washed with brine (100 mL), dried over Na₂SO₄, filtered and concentrated under reduced pressure to give the title compound as yellow powder which was used without further purification. MS (ESI): mass calcd. for C₆H₈BrN₃O, 217.0; m/z found, 217.9, 220.0 [M+H]⁺. ¹H NMR (400MHz, DMSO-*d*₆) δ = 5.69 (t, J=6.0 Hz, 1H), 4.61 (d, J=6.0 Hz, 2H), 3.78 - 3.64 (m, 1H), 1.14 - 1.07 (m, 2H), 1.07 - 1.01 (m, 2H).

Step B. 3-Bromo-1-cyclopropyl-1*H*-1,2,4-triazole-5-carbaldehyde. To a mixture of (3-bromo-1-cyclopropyl-1*H*-1,2,4-triazol-5-yl)methanol (710 mg, 3.26 mmol) in DCM (20 mL) was added MnO₂ (4.3 g, 49.5 mmol). The reaction mixture was stirred at room temperature for 16 hours. The mixture was filtered over a pad of Celite^{®}. The solid was rinsed with DCM (35 mL × 3), the filtrate was concentrated under reduced pressure to give the title compound as colorless gum which was used without further purification. ¹H NMR (400MHz, CDCl₃) δ = 9.95 (s, 1H), 4.43 (tt, J=3.9, 7.5 Hz, 1H), 1.40 - 1.34 (m, 2H), 1.22 - 1.15 (m, 2H).

Step C. 1-(3-Bromo-1-cyclopropyl-1*H*-1,2,4-triazol-5-yl)ethanol. To a solution of 3-bromo-1-cyclopropyl-1*H*-1,2,4-triazole-5-carbaldehyde (480 mg, 2.22 mmol) in THF (8 mL) was added MeMgBr (3 M solution in ether, 1.48 mL, 4.44 mmol) drop wise at 0 °C under N₂. The resulting mixture was stirred at 0 °C for 1 hour. Sat. aq. NH₄Cl (15 mL) was added to the mixture drop wise at 0 °C under N₂. The mixture was extracted with ethyl acetate (10 mL × 3). The combined organic layers were separated, washed with brine (20 mL), dried over Na₂SO₄, filtered and concentrated under reduced pressure to give the title compound as yellow oil. MS (ESI): mass calcd. for C₇H₁₀BrN₃O, 231.0; m/z found, 232.1 [M+H]⁺.

Step D. (**S*)-1-1-(3-Bromo-1-cyclopropyl-1*H*-1,2,4-triazol-5-yl)ethanol & (**R*)-1-(3-Bromo-1-cyclopropyl-1*H*-1,2,4-triazol-5-yl)ethanol. The 1-(3-bromo-1-cyclopropyl-1*H*-1,2,4-triazol-5-yl)ethanol (450 mg, 1.94 mmol) was separated by SFC (Stationary phase: DAICEL CHIRALPAK IG, 10 µm 250mm × 30mm); Mobile phase: Supercritical CO₂ (A) - EtOH (0.1% NH₃.H₂O) (B), gradient elution: 20% B in A at 60 mL/min) to give:
The first eluting compound (**S*)-1-(3-bromo-1-cyclopropyl-1*H-*1,2,4-triazol-5-yl)ethanol as yellow oil. MS (ESI): mass calcd. for C₇H₁₀BrN₃O, 231.0; m/z found, 232.1 [M+H]⁺. ¹H NMR (400MHz, CDCl₃) δ = 5.13 (quin, J=6.9 Hz, 1H), 3.62 - 3.52 (m, 1H), 1.67 (d, *J*=6.7 Hz, 3H), 1.34 - 1.30 (m, 1H), 1.27 - 1.22 (m, 1H), 1.16 - 1.11 (m, 2H).

SFC: purity 100%, retention time 2.084 min; method: IG-3_EtOH(DEA))_5_40_2,5ML_7MIN.

The second eluting compound (**R*)-1-(3-bromo-1-cyclopropyl-1*H*-1,2,4-triazol-5-yl)ethanol as yellow oil. MS (ESI): mass calcd. for C₇H₁₀BrN₃O, 231.0; m/z found, 234.1 [M+H]⁺. ¹H NMR (400MHz, CDCl₃) δ = 5.13 (q, J=6.6 Hz, 1H), 3.57 (tt, J=3.7, 7.3 Hz, 1H), 1.67 (d, *J*=6.7 Hz, 3H), 1.36 - 1.30 (m, 1H), 1.27 - 1.20 (m, 1H), 1.17 - 1.11 (m, 2H).

Step E. *N*-(2-Chloro-6-fluorophenyl)-4-(1-cyclopropyl-5-((**R*)-1-hydroxyethyl)-1*H*-1,2,4-triazol-3-yl)-5-fluoro-2-(((*S*)-1,1,1-trifluoropropan-2-yl)oxy)benzamide. The title compound was prepared in a manner analogous to Example 128, Step C however using (**R*)-1-(3-bromo-1-cyclopropyl-1*H*-1,2,4-triazol -5-yl)ethanol (first eluting compound, Step D). as the aryl halide and (*S*)-*N*-(2-chloro-6-fluorophenyl)-5-fluoro-4-(4,4,5,5-tetramethyl-1,3,2-dioxaborolan-2-yl)-2-((1,1,1-trifluoropropan-2-yl)oxy)benzamide (Intermediate 1) as the boronate. MS (ESI): mass calcd. for C₂₃H₂₀ClF₅N₄O₃, 530.1; m/z found, 530.9 [M+H]⁺. ¹H NMR (400MHz, DMSO-*d*₆) δ = 10.05 (s, 1H), 7.78 (d, *J*=5.6 Hz, 1H), 7.51 - 7.32 (m, 4H), 5.74 (d, *J=5.5* Hz, 1H), 5.37 (td, *J*=6.5, 12.7 Hz, 1H), 5.15 (quin, *J*=6.3 Hz, 1H), 3.92 (tt, *J*=3.8, 7.3 Hz, 1H), 1.57 (d, *J*=6.6 Hz, 3H), 1.46 (d, *J*=6.3 Hz, 3H), 1.26 - 1.18 (m, 2H), 1.16 - 1.06 (m, 2H); ¹⁹F NMR (376MHz, DMSO-*d₆*) δ = -77.00 (s, 1F), -115.43 (br s, 1F), -119.27 (s, 1F).

### Example 182: N-(2-chloro-6-fluorophenyl)-4-(1-cyclopropyl-5-((S)-1-hydroxyethyl)-1H-1,2,4-triazol-3-yl)-5-fluoro-2-(((S)-1,1,1-trifluoropropan-2-yl)oxy)benzamide.

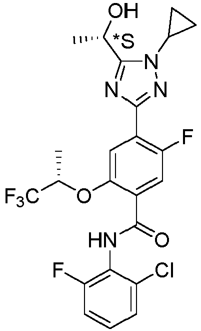

The title compound was prepared according to the representative procedure of Example 170, Step A except substituting Intermediate 1 and (**S*)-1-(3-bromo-1-cyclopropyl-1*H*-1,2,4-triazol - 5-yl)ethanol (second eluting compound, Example 181, Step D). The crude product was purified by flash column chromatography (SiO₂, eluent: 0 - 35% ethyl acetate in petroleum ether) to give the yellow solid. The yellow solid was purified by preparative reversed phase HPLC (Stationary phase: Boston Prime C18, 5 µm, 150 × 30 mm; Mobile phase: water (0.05%NH₃H₂O + 10mM NH₄HCO₃) (A) - MeCN (B), gradient elution: 55- 85% B in A over 7 min, flow rate: 25 mL/min) to give the title compound as white powder. MS (ESI): mass calcd. for C₂₃H₂₀ClF₅N₄O₃, 530.1; m/z found, 530.9 [M+H]⁺. ¹H NMR (400MHz, DMSO-*d*₆) δ = 10.05 (s, 1H), 7.78 (d, J=5.6 Hz, 1H), 7.51 - 7.33 (m, 4H), 5.74 (d, *J=5.5* Hz, 1H), 5.44 - 5.30 (m, 1H), 5.19 - 5.09 (m, 1H), 3.92 (tt, J=3.8, 7.3 Hz, 1H), 1.57 (d, J=6.6 Hz, 3H), 1.46 (d, J=6.3 Hz, 3H), 1.26 - 1.17 (m, 2H), 1.16 - 1.07 (m, 2H); ¹⁹F NMR (376MHz, DMSO-*d*₆) δ = -76.99 (br s, 1F), -115.43 (br s, 1F), -119.29 (s, 1F).

### Example 183: (S)-N-(2-(Difluoromethyl)phenyl)-5-fluoro-4-(2-(2-hvdroxypropan-2-yl)-1-methyl-1H-imidazol-4-yl)-2-((1,1,1-trifluoropropan-2-yl)oxy)benzamide.

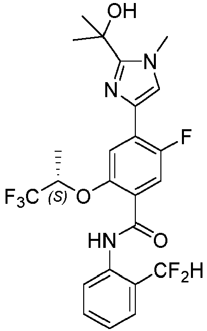

Step A. (S)-5-Fluoro-4-(2-(2-hydroxypropan-2-yl)-1-methyl-1*H*-imidazol-4-yl)-2-((1,1,1-trifluoropropan-2-yl)oxy)benzoic acid. To a solution of (S)-methyl 5-fluoro-4-(2-(2-hydroxypropan-2-yl)-1-methyl-1H- imidazol-4-yl)-2-((1,1,1-trifluoropropan-2-yl)oxy)benzoate (Example 174, Step B, 200 mg, 483.7 µmol) in THF (3 mL), and stirred at room temperature. LiOH·H₂O (101.5 mg, 2.42 mmol) in H₂O (3 mL) was added dropwise. The resulting mixture was stirred at room temperature for 16 hours. The reaction mixture was added 1M aq. HCl (0.6 mL) to pH=3. The mixture was extracted with ethyl acetate (3 mL × 2). The combined organic layers was dried over Na₂SO₄, filtered and concentrated under reduced pressure to give the title compound as a white solid. ¹H NMR (400MHz, CDCl₃) δ = 7.81 (d, J=6.3 Hz, 1H), 7.60 - 7.52 (m, 2H), 5.01 (quind, J=6.3, 12.7 Hz, 1H), 3.97 (s, 3H), 1.69 (s, 6H), 1.53 (d, J=6.3 Hz, 3H).

Step B. *(S)*-*N*-(2-(Difluoromethyl)phenyl)-5-fluoro-4-(2-(2-hydroxylpropan-2-yl)-1-methyl-1*H-*imidazol-4-yl)-2-((1,1,1-trifluoropropan-2-yl)oxy)benzamide. To a solution of (S)-5-fluoro-4-(2-(2-hydroxypropan-2-yl)-1-methyl-1*H*-imidazol-4-yl) -2-((1,1,1-trifluoropropan-2-yl)oxy)benzoic acid (110 mg, 281.8 µmol) in DCM (6 mL) was added POCl₃ (216.1 mg, 1.41 mmol). Then 2-(difluoromethyl)aniline hydrochloride (101.2 mg, 565.5 µmol) and pyridine (216.1 mg, 2.82 mmol) was added. The resulting mixture was stirred at room temperature for 12 hours. The reaction mixture was slowly poured into H₂O (6 mL) and extracted with DCM (12 mL × 2). The combined organic layers were dried over Na₂SO₄, filtered and concentrated under reduced pressure. The crude was purified by flash column chromatography (SiO₂, gradient elution: 0 - 30% ethyl acetate in petroleum ether) to give the crude compound as a white solid. The white solid was purified by preparative reversed phase HPLC (Stationary phase: Boston Prime C18, 5µm, 150 × 30 mm ; Mobile phase: water (0.05% NH₃H₂O + 10 mM NH₄HCO₃) (A) - MeCN (B), gradient elution: 42 - 72% B in A over 7 min, flow rate: 35 mL/min) to give the title compound as a white solid. MS (ESI): mass calcd. for C₂₄H₂₃F₆N₃O₃, 515.2; m/z found, 515.9 [M+H]⁺. ¹H NMR (400MHz, CDCl₃) δ = 9.48 (s, 1H), 7.97 (d, J=11.8 Hz, 1H), 7.90 (d, J=8.3 Hz, 1H), 7.84 (d, J=5.8 Hz, 1H), 7.62 - 7.50 (m, 2H), 7.44 (d, J=3.8 Hz, 1H), 7.36 - 7.29 (m, 1H), 6.95 - 6.61 (m, 1H), 5.05 (td, J=6.2, 12.4 Hz, 1H), 3.89 (s, 3H), 3.06 (s, 1H), 1.73 (s, 6H), 1.66 (d, J=6.5 Hz, 3H) ; ¹⁹F NMR (376MHz, CDCl₃) δ = -78.25 (s, 1F), -110.93 - -114.37 (m, 1F), - 120.71 (s, 1F).

### Example 184: (S)-N-(5-Chloro-3-methyl-1H-pyrazol-4-yl)-4-(5-chloro-4-(2-hydroxypropan-2-yl)thiazol-2-yl)-5-fluoro-2-(1,1,1-trifluoropropan-2-yl)oxy)benzamide.

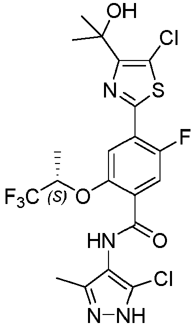

Step A. Methyl 2-amino-5-chlorothiazole-4-carboxylate. A mixture of methyl 2-aminothiazole-4-carboxylate (10.00 g, 63.2 mmol) in MeCN (125 mL) was added NCS (8.86 g, 66.4 mmol) room temperature. The mixture was stirred at 80 °C overnight. The mixture was concentrated to about 30 mL. The remaining mixture was filtered, and the filter cake was collected, dried under vacuum to give the title compound as brown solid. MS (ESI): mass calcd. for C₅H₅ClN₂O₂S, 192.0; m/z found, 192.9 [M+H]⁺. ¹H NMR (400 MHz, CDCl₃) δ 5.46 (br s, 2H), 3.91 (s, 3H) ppm.

Step B. Methyl 2-((*tert*-butoxycarbonyl)amino)-5-chlorothiazole-4-carboxylate. To a solution of methyl 2-amino-5-chlorothiazole-4-carboxylate (4.50 g, 23.4 mmol) in DCM/THF (v/v, 1/1, 60 mL) was added Boc₂O (5.61 g, 25.7 mmol), DMAP (856 mg, 7.01 mmol) and TEA (2.36 g, 23.4 mmol) at room temperature. The mixture was stirred at 60 °C for 24 hours. The mixture was poured into H₂O (80 mL) and extracted with DCM (80 mL × 2). The combined organic layer was dried over Na₂SO₄, filtered and concentrated under reduced pressure. The crude was purified by flash column chromatography (SiO₂, gradient elution: 0 - 25% ethyl acetate in petroleum ether) to give the title compound as light red solid. MS (ESI): mass calcd. for C₁₀H₁₃ClN₂O₄S, 292.0; m/z found, 236.8 [M+H]⁺. ¹H NMR (400 MHz, CDCl₃) *δ* 8.35 (br s, 1H), 3.93 (s, 3H), 1.53 (s, 9H) ppm.

Step C. *tert*-butyl (5-Chloro-4-(2-hydroxypropan-2-yl)thiazol-2-yl)carbamate. To a solution of methyl 2-((tert-butoxycarbonyl)amino)-5-chlorothiazole-4-carboxylate (1.00 g, 3.42 mmol) in THF (15 mL) was added MeMgBr (3 M solution in Et₂O, 5.98 mL) at 0 °C. The mixture was stirred at room temperature for 2 hours. The mixture was poured into sat. aq. NH₄Cl (100 mL) carefully and extracted with DCM (100 mL × 2). The combined organic layers were dried over anhydrous Na₂SO₄, filtered and concentrated under reduced pressure. The crude was purified by flash column chromatography (SiO₂, gradient elution: 0 - 50% ethyl acetate in petroleum ether) to give the title compound as white solid. MS (ESI): mass calcd. for C₁₁H₁₇ClN₂O₃S, 292.1; m/z found, 293.1 [M+H]⁺. ¹H NMR (400 MHz, CDCl₃) δ 8.48 (br s, 1H), 4.00 (br s, 1H), 1.61 - 1.58 (m, 6H), 1.54 (s, 9H) ppm.

Step D. 2-(2-Amino-5-chlorothiazol-4-yl)propan-2-ol. To a solution of *tert*-butyl (5-chloro-4-(2-hydroxypropan-2-yl)thiazol-2-yl)carbamate (1.00 g, 1.76 mmol) in DCM (10 mL) was added TFA (3.20 g, 28.1 mmol) at 0 °C. The mixture was stirred at the room temperature for 20 hours.

The mixture was concentrated under reduced pressure. The residue was dissolved with DCM (30 mL) and alkalified to pH=10. The mixture was extracted with DCM (20 mL × 6). The combined organic phase was dried, filtered and concentrated under reduced pressure. The crude product was purified by preparative reversed phase HPLC (Stationary phase: Boston Prime C18, 5 µm, 150 × 30 mm ; Mobile phase: water (0.05% NH₃H₂O + 10 mM NH₄HCO₃) (A) - MeCN (B), gradient elution: 20 - 50% B in A over 7 min, flow rate: 25 mL/min). The pure fractions were collected and concentrated under vacuum to remove MeCN, then alkalified to pH=10. The mixture was extracted with DCM (50 mL × 3). The combined organic layers were dried, filtered and concentrated under reduced pressure to give the title compound as light yellow solid. MS (ESI): mass calcd. for C₆H₉ClN₂OS, 192.7; m/z found, 193.0 [M+H]⁺. ¹H NMR (400 MHz, CDCl₃) *δ* 4.83 (br s, 2H), 3.64 (s, 1H), 1.54 (s, 6H) ppm.

Step E. 2-(2-Bromo-5-chlorothiazol-4-yl)propan-2-ol. To the mixture of t-BuONO (344 mg, 3.33 mmol) and CuBr₂ (559 mg, 2.50 mmol) in acetonitrile (15 mL) was added 2-(2-amino-5-chlorothiazol-4-yl)propan-2-ol (330 mg, 1.67 mmol), the mixture was stirred at 80 °C for 16 hours. The reaction mixture was cooled to room temperature. The mixture was quenched with sat. aq. NH₄Cl solution (5 mL), extracted with DCM (20 mL × 3). The organic layer was separated, then dried over Na₂SO₄, filtered and concentrated under reduced pressure. The residue was purified by flash column chromatography (SiO₂, gradient elution: 0 - 50% ethyl acetate in petroleum ether) to give the title compound as white solid. MS (ESI): mass calcd. for C₆H₇BrClNOS, 254.9; m/z found, 239.9 [M-18]⁺. ¹H NMR (400MHz, CDCl₃) *δ* 3.11 (s, 1H), 1.57 (s, 6H).

Step F: (*S*)-*N*-(5-Chloro-3-methyl-1*H-*pyrazol-4-yl)-4-(5-chloro-4-(2-hydroxypropan-2-yl)thiazol-2-yl)-5-fluoro-2-(1,1,1-trifluoropropan-2-yl)oxy)benzamide. The title compound was prepared in a manner analogous to Example 128, Step C however using 2-(2-bromo-5-chlorothiazol-4-yl)propan-2-ol as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid as the boronate. ¹H NMR (400MHz, CDCl₃) δ = 10.33 (br s, 1H), 8.79 (s, 1H), 8.15 (d, *J*=11.4 Hz, 1H), 7.91 (d, J=5.4 Hz, 1H), 5.07 (spt, J=6.1 Hz, 1H), 3.85 (br s, 1H), 2.34 (s, 3H), 1.75 (s, 6H), 1.72 (d, J=6.6 Hz, 3H); ¹⁹F NMR (376MHz, CDCl₃) δ = -78.12 (br s, 1F), -118.21 (br s, 1F).

### Example 185: (S)-N-(2-Chloro-6-fluorophenyl)-5-fluoro-4-(2-(2-hydroxypropan-2-yl)-1H-imidazol-4-yl)-2-((1,1,1-trifluoropropan-2-yl)oxy)benzamide.

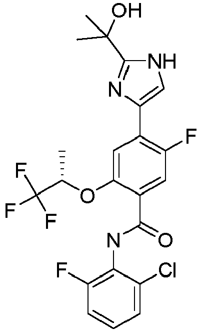

Step A. 2-(4-Bromo-1*H*-imidazol-2-yl)propan-2-ol. MeMgBr (3 M solution in ether, 4.57 mL, 13.7 mmol) was added slowly into the solution of ethyl 4-bromo-1*H*-imidazole-2-carboxylate (500 mg, 2.28 mmol) in THF (15 mL) at 0 °C under N₂. After addition, the mixture was stirred at 0 °C for 0.5 hour. Sat. aq. NH₄Cl (10 mL) was added dropwise into. The mixture was diluted H₂O (10 ml) and extracted with DCM (20 ml × 3). The organic layer was separated, washed with brine (50 mL), dried with Na₂SO₄, filtered and concentrated under reduced pressure. The crude was purified by flash column chromatography (SiO₂, eluent: 0 - 100% ethyl acetate in petroleum ether) to give the title compound as a yellow powder. MS (ESI): mass calcd. for C₆H₉BrN₂O, 204.0; m/z found, 207.0 [M+H]⁺. ¹H NMR (400MHz, DMSO-*d₆*) δ = 12.14 (br s, 1H), 7.12 (d, J=2.0 Hz, 1H), 5.43 (s, 1H), 1.48 (s, 6H)

Step B. (*S*)-*N*-(2-Chloro-5-fluorophenyl)-5-fluoro-4-(2-(2-hydroxypropan-2-yl)-1*H*-imidazol-4-yl)-2-((1,1,1-trifluoropropan-2-yl)oxy)benzamide. The title compound was prepared in a manner analogous to Example 128, Step C however using 2-(4-bromo-1*H*-imidazol-2-yl)propan-2-ol as the aryl halide and Intermediate 1 as the boronate. ¹H NMR (400MHz, CDCl₃) δ = 9.47 (br s, 1H), 9.19 (br s, 1H), 8.06 (br d, *J*=12.2 Hz, 1H), 7.89 (br d, *J*=5.0 Hz, 1H), 7.58 (br s, 1H), 7.32 (s, 1H), 7.26 - 7.22 (m, 1H), 7.18 - 7.10 (m, 1H), 5.26 - 5.08 (m, 1H), 2.37 (br s, 1H), 1.74 (s, 6H), 1.69 (d, *J*=6.6 Hz, 3H); ¹⁹F NMR (376MHz, CDCl₃) δ = -78.25 (br s, 1F), -113.65 (br s, 1F), -120.52 (br s, 1F).

### Example 186: (S)-4-(2-Amino-1-methyl-1H-imidazol-4-yl)-N-(2-chloro-6-fluorophenyl)-5-fluoro-2-(1,1,1-trifluoropropan-2-vl)oxy)benzamide.

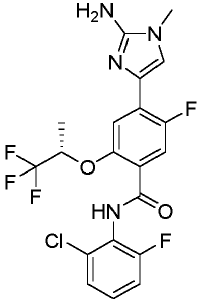

The title compound was prepared in a manner analogous to Example 128, Step C however using 4-bromo-1-methyl-1*H*-imidazol-2-amine hydrochloride as the aryl halide and Intermediate 1 as the boronate. ¹H NMR (400MHz, CDCl₃) δ = 9.16 (s, 1H), 8.00 (d, J=12.0 Hz, 1H), 7.69 (br d, J=5.7 Hz, 1H), 7.26 - 7.04 (m, 3H), 5.15 (td, J=6.2, 12.3 Hz,1H), 4.25 (br s, 2H), 3.50 (s, 3H), 1.92 (br s, 1H), 1.64 (br d, J=6.4 Hz, 3H); ¹⁹F NMR (376MHz, CDCl₃) δ = -78.30 (br s, 1F), - 113.65 (br s, 1F), -120.53 (br s, 1F).

### Example 187: N-(3-Chloro-5-methyl-1H-pyrazol-4-yl)-5-fluoro-4-((*R)-7-hydroxy-6,7-dihydro-5H-pyrrolo[1,2-a]imidazol-2-yl)-2-(((S)-1,1,1-trifluoropropan-2-yl)oxy)benzamide.

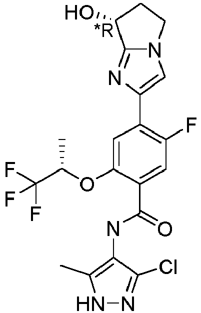

Step A. 6,7-Dihydro-5*H*-pyrrolo[1,2-a]imidazol-7-ol. To a solution of 6,7-dihydro-5*H-*pyrrolo[1,2-a]imidazol-7-ol (7 g, 56.4 mmol) in THF (175 mL) was added NBS (21.1 g, 118.4 mmol) at 0°C. Then the mixture was stirred at room temperature overnight. The reaction mixture was quenched with sat. aq. Na₂S₂O₃ (50 mL), extracted with DCM 900 mL (450 mL × 2). The organic layers were dried with Na₂SO₄, filtered, and concentrated under reduced pressure. The residue was purified by flash column chromatography (SiO₂, gradient elution: 0 - 4% MeOH in DCM) to give the title compound as light yellow solid. ¹H NMR (400MHz, MeOD) δ = ppm 5.08 (dd, *J*=7.27, 3.22 Hz, 1 H) 4.12 (ddd, *J*=10.82, 7.90, 6.08 Hz, 1 H) 3.96 (ddd, *J*=10.91, 8.52, 4.41 Hz, 1 H) 2.86 - 2.98 (m, 1 H) 2.40 (dddd, J=13.72, 7.91, 4.35, 3.46 Hz, 1 H).

Step B. 2-Bromo-6,7-dihydro-5*H*-pyrrolo[1,2-a]imidazol-7-ol. To a solution of 2,3-dibromo-6,7-dihydro-5*H*-pyrrolo[1,2-a]imidazol-7-ol (1.7 g, 6.03 mmol) in THF (45 mL) was cooled to 0°C, MeMgBr (3 M solution in ether, 11.6 mL, 34.8 mmol) was added by dropwise. Then the resulting mixture was stirred at 0°C for 1 hour. The reaction mixture was slowly poured into sat. aq. NH₄Cl (7 mL), extracted with DCM (20 mL × 3). The combined organic layers were dried over Na₂SO₄, filtered and concentrated under reduced pressure. The crude was purified by flash column chromatography (SiO₂, gradient elution: 0 - 2% ethyl acetate in petroleum ether) to give the title compound as white solid. MS (ESI): mass calcd. for C₆H₇BrN₂O, 201.9; m/z found, 203.1 [M+H]⁺. ¹H NMR (400MHz, MeOD) δ = 7.11 (s, 1H), 5.03 (dd, J=3.2, 7.2 Hz, 1H), 4.21 - 4.13 (m, 1H), 3.99 (ddd, J=4.3, 8.5, 11.0 Hz, 1H), 2.99 - 2.78 (m, 1H), 2.43 - 2.32 (m, 1H).

Step C. (**S*)-2-bromo-6,7-dihydro-5*H*-pyrrolo[1,2-a]imidazol-7-ol & (**R*)-2-bromo-6,7-dihvdro-5*H*-pyrrolo[1,2-a]imidazol-7-ol. The 2-bromo-6,7-dihydro-5*H*-pyrrolo[1,2-a]imidazol-7-ol (0.99 g, 4.87 mmol) was separated by SFC (Column: REGIS (S,S)Whelk-01 (250 mm* 30 mm, 5 um)); Mobile phase: 0.05%DEA ETOH, gradient elution: 20% B in A at 200 mL/min) to give:
The first eluting compound (*S)-2-bromo-6,7-dihydro-5H-pyrrolo[1,2-a]imidazol-7-ol as white powder. MS (ESI): mass calcd. for C₆H₇BrN₂O, 201.9; m/z found, 203.0, [M+H]⁺.

¹H NMR (400MHz, MeOD) δ = 7.11 (s, 1H), 5.03 (dd, J=3.2, 7.2 Hz, 1H), 4.17 (ddd, J=6.4, 7.6, 11.0 Hz, 1H), 3.99 (ddd, J=4.2, 8.5, 11.0 Hz, 1H), 2.91 - 2.77 (m, 1H), 2.44 - 2.24 (m, 1H).

SFC: purity 99.46 %, retention time 2.417 min.

The second eluting compound (*R)-2-bromo-6,7-dihydro-5H-pyrrolo[1,2-a]imidazol-7-ol as white powder. MS (ESI): mass calcd. for C₆H₇BrN₂O, 201.9; m/z found, 203.0, [M+H]⁺.

¹H NMR (400MHz, MeOD) δ = 7.11 (s, 1H), 5.03 (dd, J=3.2, 7.2 Hz, 1H), 4.17 (ddd, J=6.4, 7.7, 11.0 Hz, 1H), 3.99 (ddd, J=4.3, 8.5, 11.0 Hz, 1H), 2.99 - 2.82 (m, 1H), 2.48 - 2.27 (m, 1H).

SFC: purity 99.69 %, retention time 2.677 min.

Step D: *N*-(3-Chloro-5-methyl-1*H*-pyrazol-4-yl)-5-fluoro-4-((**R*)-7-hydroxy-6,7-dihydro-5*H-*pyrrolo[1,2-a]imidazol-2-yl)-2-((*(S)*-1,1,1-trifluoropropan-2-yl)oxy)benzamide. The title compound was prepared according to the representative procedure of Example 171, Step A except substituting (*R)-2-bromo-6,7-di hydro-5*H*-pyrrolo[1,2-a]imidazol-7-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₈ClF₄N₅O₃, 487.8; m/z found, 487.9 [M+H]⁺. ¹H NMR (400MHz, CDCl₃) δ = 10.32 (br s, 1H), 8.89 (s, 1H), 8.02 (d, J=12.0 Hz, 1H), 7.90 (d, J=5.8 Hz, 1H), 7.70 (br s, 1H), 7.55 (d, J=3.7 Hz,1H), 5.43 (td, J=3.6, 7.4 Hz, 1H), 5.40 - 5.30 (m, 1H), 4.29 (ddd, J=5.6, 8.2, 11.0 Hz, 1H), 4.08 (ddd, *J*=5.0, 8.4, 11.0 Hz, 1H), 3.17 - 2.97 (m, 1H), 2.61 (dq,*J*=4.8, 8.6 Hz, 1H), 2.31 (s, 3H), 1.64 (s, 3H); 19F NMR (376MHz, CDCl₃) δ = -72.34 - -82.59 (m, 1F), -116.30 - -124.41 (m, 1F).

### Example 188: N-(3-Chloro-5-methyl-1H-pyrazol-4-yl)-5-fluoro-4-((*S)-7-hydroxy-6,7-dihydro-5H-pyrrolo[1,2-a]imidazol-2-yl)-2-(((S)-1,1,1-trifluoropropan-2-yl)oxy)benzamide.

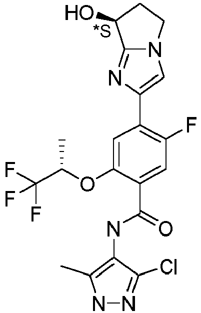

The title compound was prepared according to the representative procedure of Example 171, Step A except substituting (*S)-2-bromo-6,7-di hydro-5*H-*pyrrolo[1,2-a]imidazol-7-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₈ClF₄N₅O₃, 487.1; m/z found, 488.0 [M+H]⁺. ¹H NMR (400MHz, CDCl₃) δ = 10.32 (br s, 1H), 8.89 (s, 1H), 8.02 (d, *J*=12.0 Hz, 1H), 7.90 (d, *J*=5.8 Hz, 1H), 7.70 (br s, 1H), 7.55 (d, *J*=3.7 Hz,1H), 5.43 (td, J=3.6, 7.4 Hz, 1H), 5.40 - 5.30 (m, 1H), 4.29 (ddd, *J*=5.6, 8.2, 11.0 Hz, 1H), 4.08 (ddd, *J*=5.0*,* 8.4, 11.0 Hz, 1H), 3.17 - 2.97 (m, 1H), 2.61 (dq,J=4.8, 8.6 Hz, 1H), 2.31 (s, 3H), 1.64 (s, 3H); 19F NMR (376MHz, CDCl₃) δ = -72.34 - -82.59 (m, 1F), -116.30 - -124.41 (m, 1F).

### Example 189: N-(5-Chloro-3-methyl-1H-pyrazol-4-yl)-5-fluoro-4-((S)-7-hydroxy-7-methyl-6,7-dihydro-5H-pyrrolo[1,2-a]imidazol-2-yl)-2-(((S)-1,1,1-trifluoropropan-2-yl)oxy)benzamide.

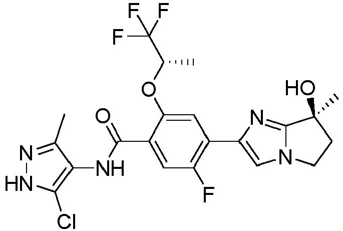

Step A. 2,3-Dibromo-5*H*-pyrrolo[1,2-a]imidazol-7(6*H*)-one. A solution of 2,3-dibromo-6,7-dihydro-5*H*-pyrrolo[1,2-a]imidazol-7-ol (2.00 g, 7.09 mmol, 1.0 eq.) in DCM (45 mL) was added MnO₂ (3.7 g, 42.5 mmol, 6.0 eq.). The reaction mixture was stirred at room temperature for 12 hours. The reaction mixture was filtered and the filter cake was washed with DCM (20 mL). The filtrate was purified by flash column chromatography (SiO₂, gradient elution: 0 - 50% ethyl acetate in petroleum ether). The pure fractions were collected and dried under vacuum to give the title compound as a yellow solid. MS (ESI): mass calcd. for C₆H₄Br₂N₂O, 279.9; m/z found, 280.9 [M+H]⁺. ¹H NMR (400 MHz, CDCl₃) δ 4.39 - 4.22 (m, 2H), 3.28 - 3.13 (m, 2H) ppm.

Step B. 2-Bromo-7-methyl-6,7-dihydro-5*H*-pyrrolo[1,2-a]imidazol-7-ol. To a solution of 2,3-dibromo-5*H*-pyrrolo[1,2-a]imidazol-7(6*H*)-one (1.8 g, 6.43 mmol, 1.0 eq.) in THF (20 mL) was added MeMgBr (3 M in Et₂O, 10.72 mL, 35.15 mmol, 5.0 eq.) at 0 °C under N₂. The mixture was stirred at room temperature for 5 hours. The mixture was slowly added to sat. aq. NH₄Cl (50 mL) and extracted with ethyl acetate (70 mL× 2). The combined organic layers were dried over anhydrous Na₂SO₄, filtered and concentrated under reduced pressure. The crude product was purified by preparative reversed phase HPLC (Stationary phase: Phenomenex Gemini NX, 5µm, 150 × 30 mm ; Mobile phase: water (0.05% NH₃H₂O + 10 mM NH₄HCO₃) (A) - MeCN (B), gradient elution: 2 - 30% B in A over 7 min, flow rate: 35 mL/min)to give the title compound as light yellow powder. MS (ESI): mass calcd. for C₇H₉BrN₂O, 217.0; m/z found, 219.0 [M+H]⁺. ¹H NMR (400 MHz, CDCl₃) *δ* 6.84 (s, 1H), 4.60 (s, 1H), 4.16 (td, *J=* 7.3, 10.7 Hz, 1H), 3.94 (ddd, *J=* 3.6, 8.3, 10.7 Hz, 1H), 2.73 (ddd, *J=* 3.6, 7.5, 13.4 Hz, 1H), 2.57 (ddd, *J=* 7.0, 8.3, 13.4 Hz, 1H), 1.71 (s, 3H) ppm.

Step C. (**S*)-2-Bromo-7-methyl-6,7-dihydro-5*H*-pyrrolo[1,2-a]imidazol-7-ol and (**R*)-2-bromo-7-methyl-6,7-dihydro-5*H-*pryrolo[1,2-a]imidazol-7-ol. The 2-bromo-7-methyl-6,7-dihydro-577-pyrrolo[1,2-a]imidazol-7-ol (280 mg, 1.29 mmol) was separated by SFC (Stationary phase: DAICEL CHIRALPAK AD, 10 µm, 250mm × 30mm); Mobile phase: Supercritical CO₂ (A) - EtOH (0.1% NH₃.H₂O) (B), gradient elution: 20% B in A at 60 mL/min) to give:
The first eluting compound (**S*)-2-bromo-7-methyl-6,7-dihydro-5*H*-pyrrolo[1,2-a]imidazol-7-ol white powder. MS (ESI): mass calcd. for C₇H₉BrN₂O, 217.1; m/z found, 219.0 [M+H]⁺. ¹H NMR (400 MHz, CDCl₃) *δ* 6.83 (s, 1H), 4.16 (td, *J=* 7.3, 10.8 Hz, 1H), 3.94 (ddd, *J=* 3.3, 8.2, 10.9 Hz, 1H), 3.36 (br s, 1H), 2.72 (ddd, *J=* 3.3,7.6, 13.2 Hz, 1H), 2.63 - 2.49 (m, 1H), 1.74 (s, 3H) ppm.

SFC: purity 99.98%, retention time 2.489 min.

The second eluting compound was purified by SFC again at the same condition to give (*R)-2-bromo-7-methyl-6,7-dihydro-5H-pyrrolo[1,2-a]imidazol-7-ol as white powder. MS (ESI): mass calcd. for C₇H₉BrN₂O, 217.1; m/z found, 217.0 [M+H]⁺

¹H NMR (400 MHz, CDCl₃) δ 6.84 (s, 1H), 4.16 (td, *J=* 7.3, 10.5 Hz, 1H), 4.02 - 3.88 (m, 1H), 3.20 (br s, 1H), 2.86 - 2.66 (m, 1H), 2.58 (td,J= 7.7, 13.6 Hz, 1H), 1.74 (s, 3H) ppm.

SFC: purity 99.88%, retention time 3.16 min.

Step D. N-(5-Chloro-3-methyl-1*H*-pyrazol-4-yl)-5-fluoro-4-((**S*)-7-hydroxy-7-methyl-6,7-dihydro-5*H*-pyrrolo[1,2-a]imidazol-2-yl)-2-(((*S*)-1,1,1-trifluoropropan-2-yl)oxy)benzamide.

The title compound was prepared according to the representative procedure of Example 171, Step A except substituting (*S)-2-bromo- 7-methyl-6,7-dihydro-5*H*-pyrrolo[1,2-a]imidazol-7-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step a) as the boronate. MS (ESI): mass calcd. for C₂₁H₂₀ClF₄N₅O₃, 501.1; m/z found, 502.0 [M+H]⁺. ¹H NMR (400MHz, CDCl₃) δ = 9.96 (br s, 1H), 8.85 (s, 1H), 8.01 (d, J=11.9 Hz, 1H), 7.82 (d, J=5.7 Hz, 1H), 7.44 (d, J=3.9 Hz, 1H), 5.26 - 5.16 (m, 1H), 4.19 - 4.07 (m, 1H), 4.02 - 3.91 (m, 1H), 3.76 (br s, 1H), 2.79 - 2.58 (m, 2H), 2.31 (s, 3H), 1.85 (s, 3H), 1.68 (d, J=6.4 Hz, 3H); ¹⁹F NMR (376MHz, CDCl₃) δ = -78.31 (s, 1F), -120.53 (s, 1F).

### Example 190: (S)-5-Fluoro-4-(5-(2-hydroxypropan-2-yl)-1-methyl-1H-1,2,4-triazol-3-yl)-N-(o-D₃-tolyl)-2-((1,1,1-trifluoropropan-2-yl)oxy)benzamide.

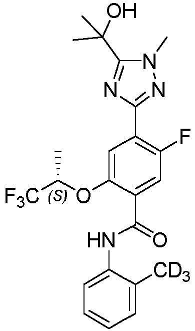

Step A. (*S*)-5-Fluoro-4-(5-(2-hydroxpropan-2-yl)-1-methyl-1*H*-1,2,4-triazol-3-yl)-2-((1,1,1-trifluoropropan-2-yl)oxy)benzoic acid. To a mixture of (S)-5-fluoro-4-(4,4,5,5-tetramethyl-1,3,2-dioxaborolan-2-yl)-2-((1,1,1- trifluoropropan-2-yl)oxy)benzoic acid (Example 76, Step C, 1.50 g, 3.73 mmol), 2-(3-bromo-1- methyl-1H-1,2,4-triazol-5-yl)propan-2-ol (Example 134, Step X, 820.6 mg, 3.73 mmol) and K₂CO₃ (1.55 g, 11.19 mmol, 3.0 eq.) in Dioxane/H₂O (v/v, 5/1, 30 mL) was added Pd-118 (364.6 mg, 559.3 µmol) at room temperature. The mixture was stirred at 95 °C for 12 hours under N₂. The mixture was concentrated under reduced pressure. H₂O (100 mL) and DCM (50 mL) was added to the mixture and filtered. The filtrate was extracted with DCM (30 mL × 3) to remove the impurity. The aqueous phase was acidified to pH=3-4 by addition of 1M aq. HCl and extracted with ethyl acetate (30 mL × 3). The combined organic layers was dried, filtered and concentrated under reduced pressure to give the crude product. The crude was triturated with mixed solvents petroleum ether/ethyl acetate (v/v, 20/1, 15 mL). The suspension was filtered and the filter cake was dried under vacuum to give the title compound as a light brown solid. MS (ESI): mass calcd for C₁₆H₁₇F₄N₃O₄, 391.1; m/z found, 392.1 [M+H]⁺. ¹H NMR (400 MHz, CDCl₃) *δ* 7.96 (d, *J=* 10.6 Hz, 1H), 7.77 (d, *J=* 5.5 Hz, 1H), 5.00 (spt, *J* = 6.1 Hz, 1H), 4.18 (s, 3H), 1.77 (s, 6H), 1.66 (d, *J=* 6.4 Hz, 3H); ¹⁹F NMR (376MHz, CDCl₃) *δ* = -78.36 (3F), -117.15 (1F) ppm.

Step B. (S)-Methyl 5-fluoro-4-(5-(2-hydroxypropan-2-yl)-1-methyl-1*H*-1,2,4-triazol-3-yl)-2-((1,1,1-trifluoropropan-2-yl)oxy)benzoate. To a solution of (S)-5-fluoro-4-(5-(2-hydroxypropan-2-yl)-1-methyl-1*H*-1,2,4-triazol-3-yl)-2-((1,1,1-trifluoropropan-2-yl)oxy)benzoic acid (700 mg, 1.79 mmol, 1.0 eq.) in DCM/MeOH (v/v, 10/1, 44 mL), then TMSCHN₂ (2 M solution in hexane, 5.37 mL, 10.73 mmol, 6.0 eq.) was dropwise added. The resulting mixture was stirred at room temperature for 2 hours. The reaction mixture was concentrated under reduced pressure to give the title compound as white solid. MS (ESI): mass calcd. for C₁₇H₁₉F₄N₃O₄, 405.10; m/z found, 406.00 [M+H]⁺. ¹H NMR (400 MHz, CDCl₃) *δ* 7.72 (d, *J=* 5.8 Hz, 1H), 7.66 (d, *J=* 10.5 Hz, 1H), 4.75 (td, *J=* 6.2, 12.5 Hz, 1H), 4.16 (s, 3H), 3.92 (s, 3H), 2.40 (s, 1H), 1.76 (s, 6H), 1.57 (d, *J* = 6.5 Hz, 3H) ppm.

Step C: *(S)*-5-Fluoro-4-(5-(2-hydropropan-2-yl)-1-methyl-1*H*-1,2,4-triazol-3-yl)-N-(o-D₃-tolyl)-2-((1,1,1-trifluorpropan-2-yl)oxy)benzamide. To a solution of o-D₃-toluidine (51.53 mg, 467.7 µmol, 2.0 eq.) in DCE (2.5 mL) was added dropwise AlMe₃ (2 M solution in toluene, 0.35 mL, 701.5 µmol, 3.0 eq.) at 0 °C. The mixture was stirred at room temperature for 0.5 hour. (S)-methyl 5-fluoro-4-(5-(2- hydroxypropan-2-yl)-1-methyl-1*H*-1,2,4-triazol-3-yl)-2-((1,1,1-trifluoropropan-2-yl)oxy)benzoate (100 mg, 233.8 µmol, 1.0 eq.) was added. Then the resulting mixture was stirred at 80 °C for 12 hours. The reaction was quenched by added sat. aq. NaHCO₃ (3 mL), extracted with DCM (3 mL × 3). The combined organic layers were dried over Na₂SO₄, filtered and concentrated under reduced pressure. The crude was purified by preparative reversed phase HPLC (Stationary phase: Boston Prime C18, 5µm, 150 × 30 mm ; Mobile phase: water (0.05% NH₃H₂O + 10 mM NH₄HCO₃) (A) - MeCN (B), gradient elution: 51 - 81% B in A over 7 min, flow rate: 35 mL/min) to give the title compound as white solid. MS (ESI): mass calcd. for C₂₃H₂₁D₃F₄N₄O₃, 483.20; m/z found, 484.10 [M+H]⁺. ¹H NMR (400 MHz, CDCl₃) δ 9.11 (s, 1H), 8.08 (d, *J* = 11.3 Hz, 1H), 7.91 (d, *J* = 8.0 Hz, 1H), 7.76 (d, *J* = 5.5 Hz, 1H), 7.29 (br d, *J* = 1.8 Hz, 1H), 7.26 - 7.23 (m, 1H), 7.18 - 7.11 (m, 1H), 5.03 (td, *J=* 6.3, 12.5 Hz, 1H), 4.17 (s, 3H), 2.50 (s, 1H), 1.77 (s, 6H), 1.65 (d, *J* = 6.5 Hz, 3H); ¹⁹F NMR (376 MHz, CDCl₃) δ -78.11 (3F), -118.12 (1F) ppm.

### Example 191: (S)-N-(2-Chloro-4-methylpyridin-3-yl)-5-fluoro-4-(5-(2-hydroxypropan-2-yl)-1-methyl-1H-1,2,4-triazol-3-yl)-2-((1,1,1-trifluoropropan-2-yl)oxy)benzamide.

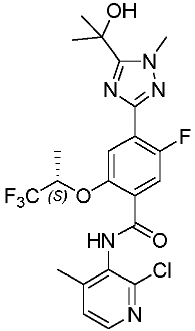

The title compound was prepared according to the representative procedure of Example 190, Step C except substituting 2-chloro-4-methylpyridin-3-amine. MS (ESI): mass calcd. for C₂₂H₂₂ClF₄N₅O₃, 515.10; m/z found, 516.10 [M+H]⁺. ¹H NMR (400 MHz, CDCl₃) *δ* 9.21 (s, 1H), 8.21 (d, *J* = 5.0 Hz, 1H), 8.10 (d, *J* = 11.0 Hz, 1H), 7.77 (d, *J* = 5.3 Hz, 1H), 7.20 (d, *J* = 5.0 Hz, 1H), 5.11 (td, *J=* 6.1, 12.1 Hz, 1H), 4.18 (s, 3H), 2.46 (s, 1H), 2.36 (s, 3H), 1.78 (s, 6H), 1.70 (d, *J=* 6.5 Hz, 3H); ¹⁹F NMR (376 MHz, CDCl₃) δ -78.10 (3F), -118.37 (1F) ppm.

### Example 192: (S)-N-(2-Chlorophenyl)-5-fluoro-4-(5-(2-hydroxypropan-2-yl)-1-methyl-1H-1,2,4-triazol-3-yl)-2-((1,1,1 -trifluoropropan-2-vl)oxy)benzamide.

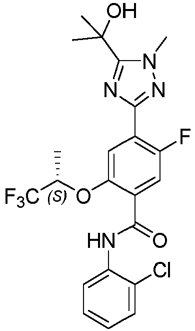

The title compound was prepared according to the representative procedure of Example 190, Step C except substituting 2-chloroaniline. MS (ESI): mass calcd. for C₂₂H₂₁ClF₄N₄O₃, 500.10; m/z found, 501.10[M+H]⁺. ¹H NMR (400 MHz, CDCl₃) δ 9.78 (s, 1H), 8.53 (d, *J=* 8.0 Hz, 1H), 8.04 (d, *J* = 11.0 Hz, 1H), 7.79 (d, J = 5.3 Hz, 1H), 7.43 (d, J = 8.0 Hz, 1H), 7.34 (t, J = 7.5 Hz, 1H), 7.15 - 7.05 (m, 1H), 5.08 - 4.92 (m, 1H), 4.17 (s, 3H), 2.53 (s, 1H), 1.77 (s, 6H), 1.68 (d, *J=* 6.5 Hz, 3H); ¹⁹F NMR (376 MHz, CDCl₃) δ -77.68 (3F), -118.14 (1F) ppm.

### Example 193: N-(5-Chloro-3-methyl-1H-pyrazol-4-yl)-5-fluoro-4-((*R)-7-hydroxy-7-methyl-6,7-dihydro-5H-pyrrolo[1,2-a]midazol-2-yl)-2-(((S)-1,1,1-trifluoropropan-2-yl)oxy)benzamide.

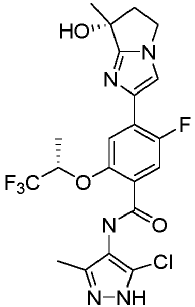

The title compound was prepared according to the representative procedure of Example 171 except substituting (*R)-2-bromo- 7-methyl-6,7-dihydro-5*H*-pyrrolo[1,2-a]imidazol-7-ol as the aryl halide and (S)-(4-((5-Chloro-3-methyl-1H-pyrazol-4-yl)carbamoyl)-2-fluoro-5-((1,1,1-trifluoropropan-2-yl)oxy)phenyl)boronic acid (Example 79, Step a) as the boronate. MS (ESI): mass calcd. for C₂₁H₂₀ClF₄N₅O₃,501.9; m/z found, 502.0 [M+H]⁺.

¹H NMR (400 MHz, CDCl₃) *δ* 10.24 (br s, 1H), 8.87 (s, 1H), 8.01 (d, *J* = 11.9 Hz, 1H), 7.87 (d, *J* = 5.7 Hz, 1H), 7.48 (d, *J* = 3.9 Hz, 1H), 5.22 (td, *J=* 6.3, 12.5 Hz, 1H), 4.37 - 4.10 (m, 2H), 4.02 (ddd, *J=* 4.6, 8.0, 10.9 Hz, 1H), 2.85 - 2.61 (m, 2H), 2.31 (s, 3H), 1.84 (s, 3H), 1.66 (d, *J=* 6.4 Hz, 3H); ¹⁹F NMR (376 MHz, CDCl₃) *δ* -78.24 (3F), -120.60 (1F) ppm.

### Example 194: (S)-N-(2-Chloro-6-fluorophenyl)-5-fluoro-4-(2-hydroxy-1-methyl-1H-imidazol-4-yl)-2-((1,1,1-trifluoropropan-2-yl)oxy)benzamide.

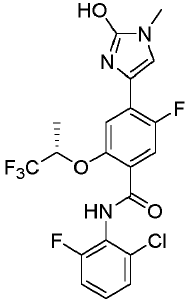

Step A. 2-(Benzyloxy)-4,5-dibromo-1-methyl-1H-imidazole. To a solution of BnOH (6.78 g, 62.7 mmol, 10.0 eq.) in THF (20 mL) was added NaH (2.25 g, 56.5 mmol, 9.0 eq., 60%), carefully. The mixture was stirred at 80 °C for 20 minute, then cooled to 0 °C. Then a solution of 2,4,5-tribromo-1-methyl-1H-imidazole (2 g, 6.27 mmol, 1.0 eq.) in THF (20 mL) was added into the mixture. The reaction mixture was stirred at 80°C overnight. The mixture was quenched by addition of sat. aq. NH₄Cl (10 mL) and extracted with ethyl acetate (2 × 20 mL). The combined organic phase was dried, filtered and concentrated under reduced pressure. The crude was purified by flash column chromatography (SiO₂, gradient elution: 0 - 8% ethyl acetate in petroleum ether). The pure fraction was concentrated under reduced pressure to give the tittle compound as light yellow solid. MS (ESI): mass calcd. for C₁₁H₁₀Br₂N₂O, 343.9; m/z found, 346.8 [M+H]⁺. ¹H NMR (400 MHz, CDCl₃) *δ* 7.46 - 7.35 (m, 5H), 5.36 (s, 2H), 3.38 (s, 3H) ppm.

Step B. 2-(Benzyloxy)-4-bromo-1-methyl-1H-imidazole. 2-(benzyloxy)-4,5-dibromo-1-methyl-1H-imidazole (2 g, 5.78 mmol, 1.0 eq.) was dissolved in THF (30 mL) purged with N₂ and cooled to -78 °C. n-BuLi (2.5 M, 2.83 mL, 7.08 mmol, 1.2 eq.) was added dropwise to the mixture. The reaction mixture was stirred at -78 °C for 20 mins. The reaction mixture was quenched with sat.NH₄Cl (5 mL). H₂O (20 mL) was added to the mixture. The mixture was extracted with ethyl acetate (20 mL × 3). The combined organic phase was washed with brine (15 mL×3), dried with anhydrous Na₂SO₄, filtered and concentrated under reduced pressere. The crude was purified by flash column chromatography (SiO₂, gradient elution: 0 - 18% ethyl acetate in petroleum ether) to give the tittle compound as light yellow sticky. MS (ESI): mass calcd. for C₁₁H₁₁BrN₂O, 266.0; m/z found, 269.0 [M+H]⁺. ¹H NMR (400 MHz, CDCl₃) *δ* 7.50 - 7.35 (m, 5H), 6.50 (s, 1H), 5.37 (s, 2H), 3.39 (s, 3H) ppm.

Step C. (S)-4-(2-(Benzyloxy)-1-methyl-1H-imidazol-4-yl)-*N*-(2-chloro-6-fluorophenyl)-5-fluoro-2-((1,1,1-trifluoropropan-2-yl)oxy)benzamide. To a mixture of (S)-N-(2-chloro-6-fluorophenyl)-5-fluoro-4-(4,4,5,5-tetramethyl-1,3,2- dioxaborolan-2-yl)-2-((1,1,1-trifluoropropan-2-yl)oxy)benzamide (150 mg, 296.6 µmοl, 1.0 eq.), 2-(benzyloxy)-4-bromo-1-methyl-1H-imidazole (79.2 mg, 296.6 µmοl, 1.0 eq.) and K₂CO₃(102.4 mg, 741.6 µmοl, 2.5 eq.) in dioxane/H₂O (v/v, 4/1, 5 mL) was added Pd-118 (29 mg, 44.5 µmοl, 0.15 eq.) under N₂. The mixture was stirred at 90 °C overnight. H₂O (20 mL) was added to the mixture. The mixture was extracted with ethyl acetate (20 mL × 3) and the combined organic phase was washed with brine (15 mL×3), dried with anhydrous Na₂SO4, filtered and concentrated under reduced pressure. The crude was purified by flash column chromatography (SiO₂, gradient elution: 0 - 20% ethyl acetate in petroleum ether) to give the tittle compound as light yellow sticky. MS (ESI): mass calcd. For C₁₁H₁₀Br₂N₂O, 343.9; m/z found, 346.8 [M+H]⁺.

¹H NMR (400 MHz, CDCl₃) *δ* 9.18 (s, 1H), 8.03 (d, *J* = 12.0 Hz, 1H), 7.78 (d, *J* = 5.8 Hz, 1H), 7.53 (d, *J* = 6.5 Hz, 2H), 7.47 - 7.37 (m, 3H), 7.33 - 7.29 (m, 1H), 7.24 (br d, *J* = 5.5 Hz, 1H), 7.20 (d, *J* = 3.8 Hz, 1H), 7.18 - 7.11 (m, 1H), 5.51 (s, 2H), 5.14 (td, *J* = 6.3, 12.4 Hz, 1H), 3.51 (s, 3H), 1.69 (d, *J* = 6.5 Hz, 3H); ¹⁹F NMR (376 MHz, CDCl₃) *δ* -78.24 (3F), -113.61 (1F), - 120.79 (1F) ppm.

Step D. (S)-N-(2-Chloro-6-fluorophenyl)-5-fluoro-4-(2-hydroxy-1-methyl-1H-imidazol-4-yl)-2-((1,1,1-trifluoropropan-2-yl)oxy)benzamide. BCl₃ (1 M solution in toluene, 1.06 mL, 1.06 mmol, 5.0 eq.) was added to a stirred solution of (S)-4-(2-(benzyloxy)-1-methyl-1H-imidazol-4-yl)-*N-*(2-chloro-6-fluorophenyl)-5-fluoro-2-((1,1,1-trifluoropropan-2-yl)oxy)benzamide (123 mg, 212.7 µmοl, 1.0 eq.) in DCM (4 mL) at -78 °C, and the mixture was stirred at -78 °C for 1 hour. The mixture was quenched with MeOH (2 mL) at -78 °C and stirred at -78 °C for 0.5 hour. Then sat. aq. NaHCO₃ (20 mL) was added and the mixture was extracted with DCM (20 mL × 3). The combined organic phase was dried with anhydrous Na₂SO₄, filtered and concentrated under reduced pressure. The crude product was purified by preparative reversed phase HPLC (Stationary phase: Boston Prime C18, 5 µm, 150 × 30 mm; Mobile phase: water (0.05% NH₃H₂O + 10 mM NH₄HCO₃) (A) - MeCN (B), gradient elution: 50 - 80% B in A over 7 min, flow rate: 25 mL/min) to give the title compound as white powder. MS (ESI): mass calcd. for C₂₀H₁₅ClF₅N₃O₃, 475.0; m/z found, 476.0 [M+H]⁺.

¹H NMR (400 MHz, CDCl₃) *δ* 12.45 (br s, 1H), 9.18 (s, 1H), 8.08 (d, *J* = 12.2 Hz, 1H), 7.60 (d, *J* = 5.7 Hz, 1H), 7.33 - 7.29 (m, 1H), 7.26 - 7.20 (m, 1H), 7.17 -7.10 (m, 1H), 6.99 (t, *J* = 2.4 Hz, 1H), 5.61 (s, 1H), 3.37 (s, 3H), 1.71 (d, *J* = 6.3 Hz, 3H); ¹⁹F NMR (376 MHz, CDCl₃) *δ* -78.22 (3F), -113.55 (1F), -120.11 (1F) ppm.

### Example 195: (S)-N-(3-chloro-2-methoxy-5-methylpyridin-4-yl)-5-fluoro-4-(5-(2-hydroxypropan-2-yl)-1-methyl-1H-1,2,4-triazol-3-yl)-2-((1,1,1-trifluoropropan-2-yl)oxy)benzamide.

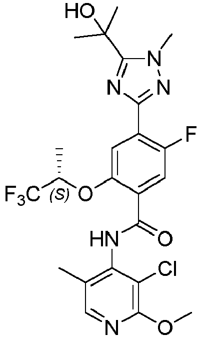

To a solution of (S)-methyl 5-fluoro-4-(5-(2-hydroxypropan-2-yl)-1-methyl-1*H*-1,2,4- triazol-3-yl)-2-((1,1,1-trifluoropropan-2-yl)oxy)benzoate (Example 190, Step B, 100 mg, 233.8 µmοl) and 3-chloro-2-methoxy-5-methylpyridin-4-amine (80.73 mg, 467.7 µmοl) in THF (3 mL) was added dropwise LiHMDS (1 M solution in THF, 0.70 mL, 0.7 mmol) at 0 °C. The mixture was stirred at 15 °C for 12 hours. The reaction was quenched by adding sat. aq. NaHCO₃ (3 mL) and subsequently extracted with DCM (3 × 3 mL). The combined organic layers were dried over Na₂SO4, filtered and concentrated under reduced pressure. The crude was purified by preparative reversed phase HPLC (Stationary phase: Boston Prime C18, 5µm, 150 × 30 mm ; Mobile phase: water (0.05% NH₃H₂O + 10 mM NH₄HCO₃) (A) - MeCN (B), gradient elution: 47 - 77% B in A over 7 min, flow rate: 35 mL/min) to give the title compound as white solid. MS (ESI): mass calcd. for C₂₃H₂₄ClF₄N₅O₄, 545.10; m/z found, 546.10 [M+H]⁺.

¹H NMR (400 MHz, CDCl₃) *δ* 9.28 (s, 1H), 8.11 (d, *J* = 11.3 Hz, 1H), 7.98 (s, 1H), 7.77 (d, *J* = 5.3 Hz, 1H), 5.10 (td, *J* = 6.2, 12.2 Hz, 1H), 4.18 (s, 3H), 4.03 (s, 3H), 2.39 (s, 1H), 2.23 (s, 3H), 1.78 (s, 6H), 1.69 (d, *J* = 6.3 Hz, 3H); ¹⁹F NMR (376 MHz, CDCl₃) *δ* -78.09 (3F), -118.30 (1F) ppm.

### Example 196: (S)-N-(2-(Difluoromethyl)phenyl)-5-fluoro-4-(5-(2-hydroxypropan-2-yl)-1-methyl-1H-1,2,4-triazol-3-yl)-2-((1,1,1-trifluoropropan-2-yl)oxy)benzamide.

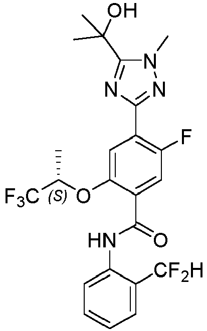

To a solution of (*S*)-5-fluoro-4-(5-(2-hydroxypropan-2-yl)-1-methyl-1*H*-1,2,4-triazol-3-yl)-2-((1,1,1-trifluoropropan-2-yl)oxy)benzoic acid (Example 190, Step A, 100 mg, 255.6 µmοl) in DCM (5 mL) was added pyridine (202.1 mg, 2.56 mmol) and 2-(difluoromethyl)aniline hydrochloride (91.8 mg, 511.1 µmol). Then POCl₃ (195.9 mg, 1.28 mmol) was added. The resulting mixture was stirred at room temperature for 12 hours. The reaction mixture was slowly poured into sat. aq. NaHCO₃ (3 mL), extracted with DCM (2 × 3 mL). The combined organic layers were dried over Na₂SO₄, filtered and concentrated under reduced pressure. The crude was purified by preparative reversed phase HPLC (Stationary phase: Boston Prime C18, 5µm, 150 × 30 mm ; Mobile phase: water (0.05% NH₃H₂O + 10 mM NH₄HCO₃) (A) - MeCN (B), gradient elution: 47 - 77% B in A over 7 min, flow rate: 35 mL/min) to give the title compound as white solid. MS (ESI): mass calcd. for C₂₃H₂₂F₆N₄O₃, 516.2; m/z found, 517.1 [M+H]⁺. ¹H NMR (400 MHz, CDCl₃) *δ* 9.45 (s, 1H), 8.05 (d, *J* = 11.3 Hz, 1H), 7.94 (d, *J* = 8.0 Hz, 1H), 7.77 (d, *J* = 5.3 Hz, 1H), 7.62 - 7.51 (m, 2H), 7.37 - 7.29 (m, 1H), 6.95 - 6.57 (m, 1H), 5.08 - 4.94 (m, 1H), 4.17 (s, 3H), 2.42 (s, 1H), 1.77 (s, 6H), 1.66 (d, *J* = 6.5 Hz, 3H); ¹⁹F NMR (376 MHz, CDCl₃) *δ* - 78.25 (3F), -110.93 - -114.37 (2F), -120.71 (1F) ppm.

### Example 197: N-(5-chloro-3-methyl-1H-pyrazol-4-yl)-5-fluoro-4-(6-((*S)-1-hydroxyethyl)-5-methylpyrazin-2-yl)-2-(((S)-1,1,1-trifluoropropan-2-yl)oxy)benzamide.

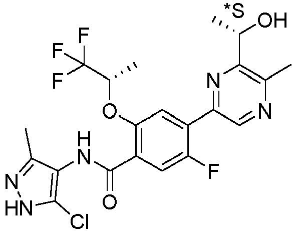

Step A, 1-(6-chloro-3-methylpyrazin-2-yl)ethanol. The 1-(6-chloro-3-methylpyrazin-2-yl)ethanol (Example 84, Step B, 6 g, 32.47 mmol) was separated by chiral SFC: (Column: ChiralPak AD-3 150×4.6mm I.D., 3um; mobile phase: CO₂ (A) - Ethanol (0.05% DEA) (B); Gradient: from 5% to 40% of B in 5.5min , then 5% of B for 1.5 min ; flow rate: 2.5 mL/min; column temp.:40 °C; ABPR: 100 bar.) to give two title compounds.

The first eluting compound: (*S)-1-(6-chloro-3-methylpyrazin-2-yl)ethan-1-ol as light yellow gum. MS (ESI): mass calcd. for C₇H₉ClN₂O, 172.04; m/z found, 173.0 [M+H]⁺. ¹H NMR (400 MHz, CDCl₃) *δ* 8.42 (s, 1H), 5.01 (qd, *J* = 6.5, 8.6 Hz, 1H), 3.60 - 3.54 (m, 1H), 2.56 (s, 3H), 1.47 (d, *J* = 6.4 Hz, 3H) ppm;

SFC: purity 99.932 %, retention time 2.901 min; method: AD-3_EtOH (DEA)_5_40_2,5M.

The second eluting compound: (*R)-1-(6-chloro-3-methylpyrazin-2-yl)ethan-1-ol as light yellow gum. MS (ESI): mass calcd. for C₇H₉ClN₂O, 172.04; m/z found, 173.0 [M+H]⁺. ¹H NMR (400 MHz, CDCl₃) *δ* 8.42 (s, 1H), 5.07 - 4.94 (m, 1H), 3.57 (d, *J* = 8.7 Hz, 1H), 2.56 (s, 3H), 1.47 (d, *J* = 6.4 Hz, 3H) ppm;

SFC: purity 99.052 %, retention time 3.205 min; method: AD-3_EtOH(DEA)_5_40_2,5M.

### Step B. N-(5-chloro-3-methyl-1H-pyrazol-4-yl)-5-fluoro-4-(6-((*S)-1-hydroxyethyl)-5-methylpyrazin-2-yl)-2-(((S)-1,1,1-trifluoropropan-2-yl)oxy)benzamide

A mixture of (*S*)-(4-((5-chloro-3-methyl-1*H*-pyrazol-4-yl)carbamoyl)-2-fluoro-5-((1,1,1-tri fluoropropan-2-yl)oxy)phenyl)boronic acid (400 mg, 976.73 µmοl, 1.0 eq.), (**S*)-1-(6- chloro-3-methylpyrazin-2-yl)ethanol (185.5 mg, 1.07 mmol, 1.1 eq.), K₃PO₄ (622.0 mg, 2.93 mmol, 3.0 eq.) and Pd-118 (127.3 mg, 195.08 µmοl, 0.2 eq.) in dioxane/H₂O (v/v, 5/1, 9.6 mL) was stirred at 80 °C for 1 hour under N₂. The mixture was extracted with ethyl acetate (20 mL × 3). The combined organic layers was dried over Na₂SO₄, filtered and concentrated under reduced pressure. The crude was purified by column chromatography (SiO₂, gradient elution: 0 - 62% ethyl acetate in petroleum ether) to give the title compound as light yellow powder. MS (ESI): mass calcd. for C₂₁H₂₀ClF₄N₅O₃, 501.9; m/z found, 502.1 [M+H]⁺. ¹H NMR (400 MHz, CDCl₃) *δ* 9.76 (br s, 1H), 9.03 (d, *J* = 2.5 Hz, 1H), 8.79 (s, 1H), 8.15 (d, *J* = 11.5 Hz, 1H), 7.73 (d, *J* = 5.5 Hz, 1H), 5.15 (t, *J* = 6.7 Hz, 1H), 5.01 (td, *J* = 6.1, 12.2 Hz, 1H), 4.04 (br d, *J* = 7.5 Hz, 1H), 2.68 (s, 3H), 2.34 (s, 3H), 1.72 (d, *J* = 6.5 Hz, 3H), 1.56 (s, 3H); ¹⁹F NMR (376 MHz, CDCl₃) *δ* - 78.05 (3F), -120.42 (1F) ppm. SFC: purity 100.00%.

### Example 198 . N-(5-chloro-3-methyl-1H-pyrazol-4-yl)-5-fluoro-4-(6-((*R)-1-hydroxyethyl)-5-methylpyrazin-2-yl)-2-(((S)-1,1,1-trifluoropropan-2-yl)oxy)benzamide.

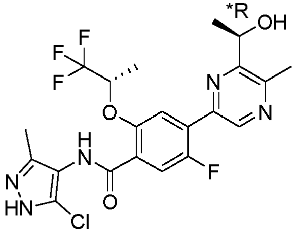

The title compound was obtained as described in Example 197, but substituting (*R)-1-(6-chloro-3-methylpyrazin-2-yl)ethan-1-ol for (*S)-1-(6-chloro-3-methylpyrazin-2-yl)ethan-1-ol in Step B. MS (ESI): mass calcd. for C₂₁H₂₀ClF₄N₅O₃, 501.9; m/z found, 502.0 [M+H]⁺. ¹H NMR (400 MHz, CDCl₃) *δ* 10.49 (br s, 1H), 9.03 (d, *J* = 2.1 Hz, 1H), 8.80 (s, 1H), 8.14 (d, *J* = 11.6 Hz, 1H), 7.73 (d, *J* = 5.6 Hz, 1H), 5.15 (br s, 1H), 5.01 (spt, *J* = 6.1 Hz, 1H), 4.07 (br s, 1H), 2.68 (s, 3H), 2.33 (s, 3H), 1.71 (d, *J* = 6.4 Hz, 3H), 1.56 (d, *J* = 6.4 Hz, 3H); ¹⁹F NMR (376 MHz, CDCl₃) *δ* -78.04 (3F), -120.43 (1F) ppm. SFC: purity 97.519%.

### Example 199: (S)-N-(5-Chloro-3-methyl-1H-pyrazol-4-yl)-5-fluoro-4-(4-(2-hydroxypropan-2-yl)-5-methyloxazol-2-yl)-2-((1,1,1-trifluoropropan-2-yl)oxy)benzamide.

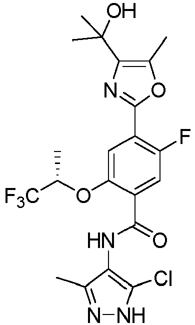

Step A. Methyl 2-iodo-5-methyloxazole-4-carboxylate. To a solution of methyl 5-methyloxazole-4-carboxylate (200.0 mg, 1.42 mmol) in THF (7 mL) was cooled to -78 °C. LiHMDS (1 M solution in THF, 2.13 mL) was added dropwise followed by I₂ (575.5 mg, 2.27 mmol) in THF (3 mL) was added. The resulting mixture was stirred at -78 °C for 1.5 hours. The reaction mixture was slowly poured into sat. aq. Na₂S₂O₃ (10 mL), extracted with DCM (10 mL × 3). The combined organic layers were dried over Na₂SO₄, filtered and concentrated under reduced pressure to give the title compound as brown solid. MS (ESI): mass calcd. for C₆H₆INO₃, 266.90; m/z found, 267.70 [M+H]⁺. ¹H NMR (400 MHz, CDCl₃) *δ* 3.92 (s, 3H), 2.69 - 2.64 (m, 3H) ppm.

Step B. (S)-5-Fluoro-4-(4-(methoxycarbonyl)-5-methyloxazol-2-yl)-2-((1,1,1-trifluoropropan-2-yl)oxy)benzoic acid. To a mixture of (S)-5-fluoro-4-(4,4,5,5-tetramethyl-1,3,2-dioxaborolan-2-yl)-2-((1,1,1-tri fluoropropan-2-yl)oxy)benzoic acid (Example 76, Step C, 220.0 mg, 581.8 µmol), methyl 2-iodo-5- methyloxazole-4-carboxylate (189.9 mg, 640 µmol), K₃PO₄ (370.5 mg, 1.73 mmol) and Pd-118 (74.5 mg, 116.4 µmοl) in dioxane/H₂O (v/v, 5/1, 4.8 mL) was stirred at 60 °C for 2 hours under N₂. 1M aq. HCl (2 mL) was added to the reaction mixture and it was extracted with ethyl acetate (4 mL × 3). The combined organic layers were dried over Na₂SO₄, filtered and concentrated under reduced pressure. The crude was purified by prep-TLC (SiO₂, ethyl acetate/petroleum ether = 2/1 (1% of AcOH)) to give the title compound as white solid. MS (ESI): mass calcd. for C₁₆H₁₃F₄NO₆, 391.10; m/z found, 391.90 [M+H]⁺. ¹H NMR (400 MHz, CDCl₃) *δ* 7.80 (d, *J* = 5.8 Hz, 1H), 7.67 (d, *J* = 10.5 Hz, 1H), 5.40 (td, *J* = 6.5, 12.9 Hz, 1H), 3.85 (s, 3H), 2.70 (s, 3H), 1.44 (d, *J* = 6.3 Hz, 3H) ppm.

Step C. (S)-Methyl 2-(4-((5-chloro-3-methyl-1*H*-pyrazol-4-yl)carbamoyl)-2-fluoro-5-((1,1,1-trifluoropropan-2-vl)oxy)phenyl)-5-methyloxazole-4-carboxylate. To a mixture of (S)-5-fluoro-4-(4-(methoxycarbonyl)-5-methyloxazol-2-yl)-2-((1,1,1-tri fluoropropan-2-yl)oxy)benzoic acid (200.0 mg, 306.7 µmοl), 5-chloro-3-methyl- 1*H*-pyrazol-4-amine (52.5 mg, 398.9 µmοl) in MeCN (6 mL) was added DIEA (198.3 mg, 1.53 mmol), then T₃P (585.7 mg, 920.4 µmοl) was added. The resulting mixture was stirred at room temperature for 2 hours. The reaction mixture was diluted with ethyl acetate (15 mL) and washed with brine (5 mL). The combined organic layers were dried over Na₂SO₄, filtered and concentrated under reduced pressure. The crude was purified by prep-TLC (SiO₂, ethyl acetate/petroleum ether = 3/1) to give the title compound as white solid. MS (ESI): mass calcd. for C₂₀H₁₇ClF₄N₄O₅, 504.10; m/z found, 505.00 [M+H]⁺. ¹H NMR (400 MHz, CDCl₃) *δ* 10.26 (br s, 1H), 8.77 (s, 1H), 8.14 (d, *J* = 11.2 Hz, 1H), 7.79 (d, *J* = 5.2 Hz, 1H), 5.18 - 5.03 (m, 1H), 3.99 (s, 3H), 2.78 (s, 3H), 2.32 (s, 3H), 1.68 (d, *J* = 6.6 Hz, 3H) ppm.

Step D. (*S*)-*N*-(5-Chloro-3-methyl-1H-pyrazol-4-yl)-5-fluoro-4-(4-(2-hydroxypropan-2-yl)-5-methyloxazol-2-yl)-2-((1,1,1-trifluoropropan-2-yl)oxy)benzamide. To a solution of (S)-methyl 2-(4-((5-chloro-3-methyl-1*H*-pyrazol-4-yl)carbamoyl)-2-fluoro-5- ((1,1,1-trifluoropropan-2-yl)oxy)phenyl)-5-methyloxazole-4-carboxylate (130.0 mg, 216.6 µmοl) in THF (5 mL) was cooled to 0 °C, MeMgBr (3 M solution in ether, 0.72 mL, 2.16 mmol) was added dropwise. Then the resulting mixture was stirred at room temperature for 12 hours. The reaction mixture was slowly poured into sat. aq. NH₄Cl (3 mL), extracted with ethyl acetate (3 mL × 3). The combined organic layers were dried over Na₂SO₄, filtered and concentrated under reduced pressure. The crude was purified by flash column chromatography (SiO₂, gradient elution: 0 - 50% ethyl acetate in petroleum ether) to give the crude compound. The crude compound was purified by preparative reversed phase HPLC (Stationary phase: Boston Prime C18, 5µm, 150 × 30 mm; Mobile phase: water (0.05% NH₃H₂O + 10 mM NH₄HCO₃) (A) - MeCN (B), gradient elution: 40 - 70% B in A over 7 min, flow rate: 30 mL/min) to give the title compound as white solid. MS (ESI): mass calcd. for C₂₁H₂₁ClF₄N₄O₄, 504.10; m/z found, 505.00 [M+H]⁺. ¹H NMR (400 MHz, CDCl₃) *δ* 9.78 (br s, 1H), 8.77 (s, 1H), 8.10 (d, *J* = 11.3 Hz, 1H), 7.67 (d, *J* = 5.5 Hz, 1H), 5.05 (td, *J* = 6.2, 12.5 Hz, 1H), 2.58 (s, 1H), 2.55 (s, 3H), 2.33 (s, 3H), 1.71 (d, *J* = 6.5 Hz, 3H), 1.64 (s, 6H); ¹⁹F NMR(376 MHz, CDCl₃) *δ* -78.16 (3F), -118.53 (1F) ppm.

### Example 200: (S)-N-(5-chloro-3-methyl-1H-pyrazol-4-yl)-5-fluoro-4-(4-(2-hydroxypropan-2-yl)-1,5-dimethyl-1H-imidazol-2-yl)-2-((1,1,1 -trifluoropropan-2-vl)oxy)benzamide.

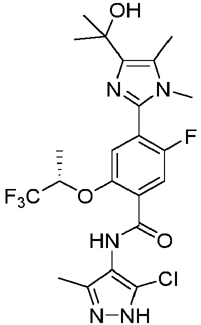

Step A. Ethyl 2-bromo-1,5-dimethyl-1*H*-imidazole-4-carboxylate. To a stirred mixture of ethyl 2-bromo-5-methyl-1*H*-imidazole-4-carboxylate (3.0 g, 12.9 mmol) and K₂CO₃ (3.56 g, 25.7 mmol) in DMF (45 mL) was added MeI (5.80 g, 40.9 mmol). The resulting mixture was stirred at room temperature for 2 hours. The reaction mixture was diluted with ethyl acetate (50 mL) and washed with brine (20 mL). The combined organic layers were dried over Na₂SO₄, filtered and concentrated under reduced pressure. The crude was purified by flash column chromatography (SiO₂, gradient elution: 0 - 50% ethyl acetate in petroleum ether) to give the title compound as white solid. MS (ESI): mass calcd. for C₈H₁₁BrN₂O₂, 246.00; m/z found, 248.70 [M+H]⁺. ¹H NMR (400 MHz, CDCl₃) *δ* 4.37 (q, *J* = 7.1 Hz, 2H), 3.55 (s, 3H), 2.58 (s, 3H), 1.39 (t, *J* = 7.2 Hz, 3H) ppm.

Step B. (S)-4-(4-(Ethoxycarbonyl)-1,5-dimethyl-1*H*-imidazol-2-yl)-5-fluoro-2-((1,1,1-trifluoropropan-2-yl)oxy)benzoic acid. The title compound was prepared according to the representative procedure of Example 199, Step B except substituting ethyl 2-bromo-1,5-dimethyl-1*H*-imidazole-4-carboxylate. MS (ESI): mass calcd. for C₁₈H₁₈F₄N₂O₅, 418.10; m/z found, 419.10 [M+H]⁺. ¹H NMR (400 MHz, CDCl₃) *δ* 13.82 - 12.68 (m, 1H), 7.65 (d, *J* = 9.5 Hz, 1H), 7.49 (d, *J* = 5.5 Hz, 1H), 5.43 - 5.26 (m, 1H), 4.25 (q, *J* = 7.1 Hz, 2H), 3.47 (d, *J* = 1.8 Hz, 3H), 2.56 (s, 3H), 1.42 (d, *J* = 6.3 Hz, 3H), 1.28 (t, *J* = 7.0 Hz, 3H) ppm.

Step C. (S)-Ethyl 2-(4-((5-chloro-3-methyl-1*H*-pyrazol-4-yl)carbamoyl)-2-fluoro-5-((1,1,1-trifluoropropan-2-yl)oxyphenyl)-1,5-dimethyl-1*H*-imidazole-4-carboxylate. The title compound was prepared according to the representative procedure of Example 199, Step C except substituting (S)-4-(4-(ethoxycarbonyl)-1,5-dimethyl-1*H*-imidazol-2-yl)-5-fluoro-2-((1,1,1-trifluoropropan-2-yl)oxy)benzoic acid. MS (ESI): mass calcd. for C₂₂H₂₂ClF₄N₅O₄, 531.10; m/z found, 532.10 [M+H]⁺. ¹H NMR (400 MHz, CDCl₃) *δ* 8.80 (s, 1H), 8.09 (d, *J* = 10.3 Hz, 1H), 7.39 (d, *J* = 5.5 Hz, 1H), 5.12 - 4.97 (m, 1H), 4.42 (q, *J* = 7.0 Hz, 2H), 3.56 - 3.53 (m, 3H), 2.65 (s, 3H), 2.32 (s, 3H), 1.65 (d, *J* = 6.3 Hz, 3H), 1.41 (t, *J* = 7.0 Hz, 3H) ppm.

Step D. (S)-N-(5-chloro-3-methyl-1*H*-pyrazol-4-yl)-5-fluoro-4-(4-(2-hydroxypropan-2-yl)-1,5-dimethyl-1*H*-imidazol-2-yl)-2-((1,1,1-trifluoropropan-2-yl)oxy)benzamide. The title compound was prepared according to the representative procedure of Example 199, Step D except substituting (S)-ethyl 2-(4-((5-chloro-3-methyl-1*H*-pyrazol-4-yl)carbamoyl)-2-fluoro-5- ((1,1,1-trifluoropropan-2-yl)oxy)phenyl)-1,5-dimethyl-1*H*-imidazole-4-carboxylate. MS (ESI): mass calcd. for C₂₂H₂₄ClF₄N₅O₃, 517.20; m/z found, 518.20 [M+H]⁺. ¹H NMR (400 MHz, CDCl₃) *δ* 9.85 (br s, 1H), 8.81 (s, 1H), 8.10 (d, J=10.6 Hz, 1H), 7.31 (br s, 1H), 5.10 - 4.94 (m, 1H), 3.49 (d, J=3.5 Hz, 3H), 3.43 (br s, 1H), 2.41 (s, 3H), 2.35 - 2.32 (m, 3H), 1.69 (d, J=6.4 Hz, 3H), 1.64 (s, 6H); ¹⁹F NMR (376 MHz, CDCl₃) *δ* -78.24 (3F), -119.08 (1F) ppm.

### Example 201: 4-(6-amino-5-chloropyridin-2-yl)-N-(5-chloro-3-methyl-1H-pyrazol-4-yl)-5-fluoro-2-isopropoxybenzamide.

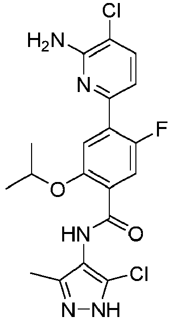

### Step A. 4-bromo-5-fluoro-2-isopropoxybenzonitrile.

A mixture of propan-2-ol (5.38 g, 89.5 mmol) in THF (60 mL) was added to the mixture of NaH (60% purity, 3.85 g, 96.3 mmol) in THF (80 mL) dropwise at 0°C under N₂. The mixture was stirred at room temperature for 30 minutes. A solution of 4-bromo-2,5-difluorobenzonitrile (15 g, 68.8 mmol) in THF (100 mL) was added to the mixture dropwise at 0°C under N₂. The mixture was stirred at room temperature for 12 hours. Sat. aq. NH₄Cl (300 mL) was added to the mixture dropwise at 0°C. The mixture was extracted with ethyl acetate (200 mL × 3). The combined organic layers were washed with brine (400 mL), dried over Na₂SO₄, filtered and concentrated under reduced pressure. The residue was purified by column chromatography (SiO₂, petroleum ether/ethyl acetate = 1/0 to 45/1) to give the title compound as yellow solid.

¹H NMR (400MHz, CDCl₃) δ = 7.31 (d, *J*=7.4 Hz, 1H), 7.17 (d, *J*=5.5 Hz, 1H), 4.63 - 4.52 (m, 1H), 1.41 (d, *J*=6.1 Hz, 6H); ¹⁹F NMR (376MHz, CDCl₃) δ = -115.71 (s, 1F).

### Step B. 4-bromo-5-fluoro-2-isopropoxybenzoic acid.

To a mixture of 4-bromo-5-fluoro-2-isopropoxybenzonitrile (16 g, 55.8 mmol) in EtOH (110 mL) was added 2 M aq. NaOH (130 mL, 260 mmol) under N₂. The mixture was stirred at 90°C for 16 hours under N₂. EtOH was removed under reduced pressure. H₂O (200 mL) was added, and the mixture was adjusted to pH = 3 with 2 M aq. HCl at 0°C. The mixture was extracted with ethyl acetate (200 mL × 3). The combined organic layers were washed with brine (500 mL), dried over Na₂SO₄, filtered and concentrated under reduced pressure to give the title compound as yellow solid. MS (ESI): mass calcd. for C₁₀H₁₀BrFO₃, 276.0; m/z found, 237.0 [M+H]⁺.

¹H NMR (400MHz, DMSO-*d₆*) δ = 12.94 (br s, 1H), 7.54 (d, *J*=8.8 Hz, 1H), 7.48 (d, *J*=5.7 Hz, 1H), 4.69 - 4.60 (m, 1H), 1.24 (d, *J*=6.0 Hz, 6H).

### Step C. 5-fluoro-2-isopropoxy-4-(4,4,5,5-tetramethyl-1,3,2-dioxaborolan-2-yl)benzoic acid.

To a mixture of 4-bromo-5-fluoro-2-isopropoxybenzoic acid (5 g, 15.7 mmol, 87% purity) in dioxane (60 mL) was added bis(pinacolato)diboron (5.98 g, 23.6 mmol) and potassium acetate (4.62 g, 47.1 mmol), followed by the addition of Pd(dppf)Cl_{2.}DCM (1.28 g, 1.57 mmol) under N₂. The resulting mixture was charged with N₂ and stirred at 85°C under N₂ for 16 hours. The mixture was cooled to room temperature. The mixture was filtered through silica gel and washed with DCM (200 mL × 2), the filtrate was concentrated under reduced pressure to give the residue (6.5 g crude). It was directly used for the next step.

### Step D. (4-((5-chloro-3-methyl-1H-pyrazol-4-yl)carbamoyl)-2-fluoro-5-isopropoxyphenyl)boronic acid.

To a mixture of 5-fluoro-2-isopropoxy-4-(4,4,5,5-tetramethyl-1,3,2-dioxaborolan-2-yl) benzoic acid (8.1 g crude) in DCM (83 mL) was added Et₃N (2.77 g, 27.4 mmol). The mixture was cooled to 0°C. HATU (8.32 g, 21.9 mmol) was added in portions and the reaction was stirred at 0°C for 1 hour. Then 3-chloro-5-methyl-1*H*-pyrazol-4-amine (2.4 g, 18.2 mmol) was added in portions at 0°C. The reaction was warmed to room temperature slowly and stirred at room temperature for 12 hours. The mixture was added water (100 mL) and extracted with DCM (200 mL × 3), the combined organic layers were dried over Na₂SO₄, filtered and concentrated under reduced pressure. The residue was purified by preparative reversed phase HPLC (Stationary phase: YMC Triart C18, 7 µm, 250 × 50 mm; Mobile phase: water (0.225% FA) (A) - MeCN (B), gradient elution: 10 - 50% B in A over 20 min, flow rate: 120 mL/min) to give the title compound as yellow powder. MS (ESI): mass calcd. for C₁₄H₁₆BClFN₃O₄, 355.1; m/z found, 356.0 [M+H]⁺. ¹H NMR (400MHz, DMSO-*d₆*) δ = 12.84 (br s, 1H), 9.39 (s, 1H), 8.42 (br s, 2H), 7.38 (d, *J*=9.1 Hz, 1H), 7.30 (d, *J*=4.5 Hz, 1H), 4.76 (spt, *J*=6.0 Hz, 1H), 2.15 (s, 3H), 1.35 (d, *J*=6.1 Hz, 6H); ¹⁹F NMR (376MHz, DMSO-*d₆*) δ = -114.07 (br s, 1F).

### Step E. 4-(6-amino-5-chloropyridin-2-yl)-N-(5-chloro-3-methyl-1H-pyrazol-4-yl)-5-fluoro-2-isopropoxybenzamide.

The title compound was prepared in a manner analogous to Example 171, Step A however using 6-bromo-3-chloropyridin-2-amine as the aryl halide and (4-((5-chloro-3-methyl-1H-pyrazol-4-yl)carbamoyl)-2-fluoro-5-isopropoxyphenyl)boronic acid as the boronate. MS (ESI): mass calcd. for C₁₉H₁₈Cl₂FN₅O₂, 437.1; m/z found, 438.1 [M+H]⁺. ¹H NMR (400 MHz, DMSO-d6) δ =12.61 - 13.09 (m, 1 H) 9.41 (s, 1 H) 7.67 - 7.74 (m, 2H) 7.59 (d, J=11.25 Hz, 1 H) 7.05 (dd, J=8.07, 1.71 Hz, 1 H) 6.51 (s, 2H) 4.81 (quin, J=5.99 Hz, 1 H) 2.18 (s, 3 H) 1.39 (d, J=5.87 Hz, 6 H).

### Example 202: 4-(6-amino-4-(trifluoromethyl)pyridin-2-yl)-N-(5-chloro-3-methyl-1H-pyrazol-4-vl)-5-fluoro-2-isopropoxybenzamide.

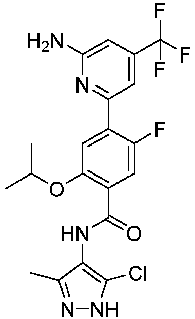

The title compound was prepared in a manner analogous to Example 201, Step E however using 6-chloro-4-(trifluoromethyl) pyridin-2-amine as the amine and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₂₀H₁₈ClF₄N₅O₂, 471.1; m/z found, 472.0 [M+H]⁺. ¹H NMR (400MHz, DMSO-d6) δ = 12.87 (s, 1 H) 9.44 (s, 1 H) 7.69 (d, J=6.36 Hz, 1 H) 7.61 (d, J=11.74 Hz, 1 H) 7.15 (s, 1 H) 6.79 (s, 3 H) 4.73 - 4.91 (m, 1 H) 2.18 (s, 3 H) 1.39 (s, 3 H) 1.38 (s, 3 H).

### Example 203: 4-(6-amino-5-(trifluoromethyl)pyridin-2-yl)-N-(5-chloro-3-methyl-1H-pyrazol-4-vl)-5-fluoro-2-isopropoxybenzamide.

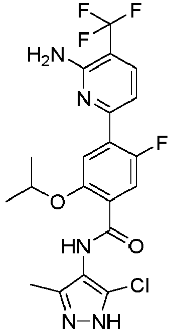

The title compound was prepared in a manner analogous to Example 201, Step E however using 6-chloro-3-(trifluoromethyl) pyridin-2-amine as the amine and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₂₀H₁₈ClF₄N₅O₂, 471.1; m/z found, 472.1 [M+H]⁺.¹H NMR (400MHz, DMSO-d6) δ= 12.88 (s, 1 H) 9.44 (s, 1 H) 7.90 (d, J=7.83 Hz, 1 H) 7.72 (d, J=5.87 Hz, 1 H) 7.61 (d, J=11.25 Hz, 1 H) 7.11 - 7.18 (m, 1 H) 6.68 (s, 2 H) 4.82 (quin, J=6.11 Hz, 1 H) 2.18 (s, 3 H) 1.40 (s, 3 H) 1.38 (s, 3 H).

### Example 204: 4-(6-amino-3-fluoropyridin-2-yl)-N-(5-chloro-3-methyl-1H-pyrazol-4-yl)-5-fluoro-2-isopropoxybenzamide.

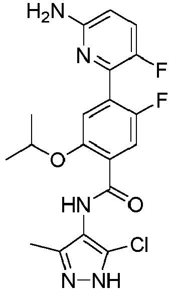

The title compound was prepared in a manner analogous to Example 201, Step E however using 6-bromo-5-fluoropyridin-2-amine as the amine and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₁₉H₁₈ClF₂N₅O₂, 421.1; m/z found, 422.1 [M+H]⁺.¹H NMR (400MHz, DMSO-d6) δ = 12.84 - 12.93 (m, 1 H) 9.44 (s, 1 H) 7.56 (d, J=9.78 Hz, 1 H) 7.38 - 7.50 (m, 1 H) 7.27 (d, J=5.87 Hz, 1 H) 6.56 (dd, J=8.80, 2.93 Hz, 1 H) 6.11 (s, 2H) 4.73 - 4.81 (m, 1 H) 2.18 (s, 3 H) 1.36 (d, J=5.87 Hz, 6 H)

### Example 205: 4-(8-aminoimidazo[1,2-a]pyrazin-6-yl)-N-(5-chloro-3-methyl-1H-pyrazol-4-yl)-5-fluoro-2-isopropoxybenzamide.

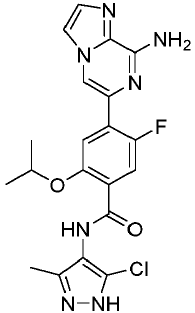

The title compound was prepared in a manner analogous to Example 201, Step E however using 6-bromoimidazo[1,2-a]pyrazin-8-amine as the amine and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₂₀H₁₉ClFN₇O₂, 443.1; m/z found, 444.1 [M+H]⁺.¹H NMR (400MHz, DMSO-d6) δ = 12.87 (s, 1 H) 9.42 (s, 1 H) 8.44 (s, 1 H) 8.04 (d, J=0.98 Hz, 1 H) 7.91 (d, J=6.36 Hz, 1 H) 7.63 (d, J=12.23 Hz, 1 H) 7.58 (d, J=0.98 Hz, 1 H) 7.17 (s, 2 H) 4.73 - 4.90 (m, 1 H) 2.18 (s, 3 H) 1.41 (d, J=5.87 Hz, 6 H).

### Example 206: 4-(6-amino-5-methoxypyridin-2-yl)-N-(5-chloro-3-methyl-1H-pyrazol-4-yl)-5-fluoro-2-isopropoxybenzamide.

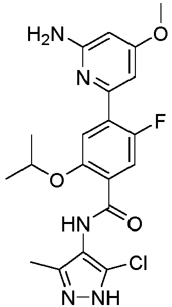

The title compound was prepared in a manner analogous to Example 201, Step E however using 6-chloro-4-methoxypyridin-2-amine and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₂₀H₂₁ClFN₅O₃, 433.1; m/z found, 434.1 [M+H]⁺.¹H NMR (400MHz, DMSO-d6) δ = 12.87 (br s, 1 H) 9.40 (s, 1 H) 7.74 (d, J=6.36 Hz, 1 H) 7.58 (d, J=12.23 Hz, 1 H) 7.07 - 7.19 (m, 2 H) 5.96 (s, 2 H) 4.74 - 4.85 (m, 1 H) 3.84 (s, 3 H) 2.17 (s, 3 H) 1.40 (d, J=5.87 Hz, 6 H).

### Example 207: 4-(6-amino-4-methoxypyridin-2-yl)-N-(5-chloro-3-methyl-1H-pyrazol-4-yl)-5-fluoro-2-isopropoxybenzamide.

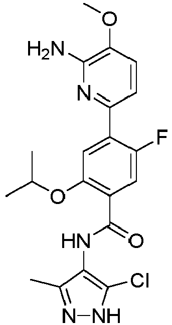

The title compound was prepared in a manner analogous to Example 201, Step E however using 6-chloro-3-methoxypyridin-2-amine and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₂₀H₂₁ClFN₅O₃, 433.1; m/z found, 434.1 [M+H]⁺.¹H NMR (400 MHz, CHLOROFORM-d) δ =9.79 - 10.19 (m, 1 H) 9.61 (s, 1 H) 8.03 (d, J=12.23 Hz, 1 H) 7.72 (d, J=5.87 Hz, 1 H) 6.79 - 7.03 (m, 1 H) 6.04 (d, J=1.96 Hz, 1 H) 4.77 - 5.06 (m, 1 H) 4.53 (s, 2 H) 3.86 (s, 3 H) 2.33 (s, 3 H) 1.51 (d, J=6.36 Hz, 6 H).

### Example 208: N-(5-Chloro-3-methyl-1H-pyrazol-4-yl)-5-fluoro-4-(2-(2-hydroxypropan-2-yl)-1-methyl-1H-imidazol-4-yl)-2-isopropoxybenzamide.

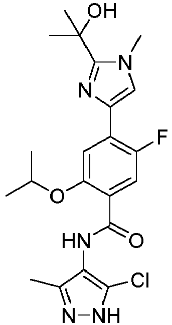

The title compound was prepared in a manner analogous to Example 201, Step E however using 2-(4-bromo-1-methyl-1H-imidazol-2-yl)propan-2-ol (Example 130, Step A) and (4-((5-chloro-3-methyl-1H-pyrazol-4-yl)carbamoyl)-2-fluoro-5-isopropoxyphenyl)boronic acid (Example 201, Step D) as the boronate. MS (ESI): mass calcd. for C₂₁H₂₅ClFN₅O₃, 449.2; m/z found, 450.0 [M+H]⁺. ¹H NMR (400 MHz, Chloroform-*d*) δ 9.62 (s, 2H), 7.97 (d, *J* = 12.1 Hz, 1H), 7.82 (d, *J* = 6.0 Hz, 1H), 7.42 (d, *J* = 4.0 Hz, 1H), 5.04 - 4.90 (m, 1H), 3.87 (s, 3H), 3.25 (s, 1H), 2.35 (s, 3H), 1.72 (s, 6H), 1.52 (d, *J* = 6.1 Hz, 6H).

### Example 209: (S)-N-(2-chloro-6-fluorophenyl)-5-fluoro-6-(5-(2-hydroxypropan-2-yl)-1-methyl-1H-1,2,4-triazol-3-yl)-2-((1,1,1-trifluoropropan-2-yl)oxy)nicotinamide.

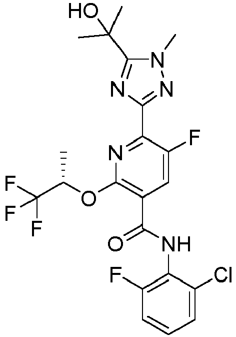

Step A. (*S*)-6-chloro-5-fluoro-2-((1,1,1-trifluoropropan-2-yl)oxy)nicotinic acid. To a solution of (,S)-1,1,1-trifluoropropan-2-ol (2.58 g, 22.62 mmol, 0.95 eq.) in DMF (10 mL) was added NaH (1.91 g, 47.62 mmol, 2.0 eq.) at 0 °C. The mixture was stirred at the room temperature for 1 hour. Then 2,6-dichloro-5-fluoronicotinic acid (5.0 g, 23.81 mmol, 1.0 eq.) in DMF (30 mL) was added to the mixture. The mixture was stirred at the room temperature overnight. The mixture was added to sat. aq. NH₄Cl solution (40 mL) slowly. The mixture was added HCl (1 M solution in H₂O, 20 mL) to adjust pH to around 2. Then it was extracted with ethyl acetate (60 mL × 2).The combined organic phase was dried by Na₂SO4, filtered and concentrated under vacuum to give the title compound (8.10 g, 10.75 mmol, crude) as yellow oil. MS (ESI): mass calcd. for C₉H₆ClF₄NO₃, 287.0; m/z found, 288.0 [M+H]⁺.

Step B. (*S*)-methyl 6-chloro-5-fluoro-2-((1,1,1-trifluoropropan-2-yl)oxy)nicotinate. To a mixture of (*S*)-6-chloro-5-fluoro-2-((1,1,1-trifluoropropan-2-yl)oxy)nicotinic acid (8.1 g, crude, 1.0 eq.) in MeOH (80 mL) was added SOCl₂ (8.38 g, 70.41 mmol, 5.0 eq.) at 0 °C. The mixture was stirred at 70 °C for 2 hours. The mixture was added H₂O (100 mL) and extracted with ethyl acetate (200 mL × 2). The combined organic phase was washed with brine (100 mL), dried with anhydrous Na₂SO₄, filtered and concentrated in vacuum. The crude was purified by column chromatography (SiO₂, gradient elution: 0 - 3% ethyl acetate in petroleum ether) to give the title compound (5.6 g, 18.54 mmol, crude) as colorless oil. MS (ESI): mass calcd. for C₁₀H₈ClF₄NO₃, 301.0; m/z found, 302.0 [M+H]⁺.

Step C. (*S*)-6-chloro-*N*-(2-chloro-6-fluorophenyl)-5-fluoro-2-((1,1,1-trifluoropropan-2-yl)oxy)nicotinamide. 2-chloro-6-fluoroaniline (5.41 g, 37.13 mmol, 2.0 eq.) was dissolved in DCM (30 mL), and AlMe₃ (2 M solution in toluene, 13.93 mL, 27.85 mmol, 3.0 eq.) was added under N_{2.} After stirring 0.5 hour, the mixture of (*S*)-methyl 6-chloro-5-fluoro-2-((1,1,1-trifluoropropan-2-yl) oxy)nicotinate (5.6 g, crude, 1.0 eq.) in DCM (40 mL) was added. The mixture was stirred at room temperature overnight. The mixture was poured into water (100 mL) and extracted with DCM (100 mL × 2). The combined organic phase was dried with anhydrous Na₂SO₄, filtered and concentrated in vacuum. The crude was purified by column chromatography (SiO₂, gradient elution: 0 - 2.2% ethyl acetate in petroleum ether) to give the title compound as light yellow solid. MS (ESI): mass calcd. for C₁₅H₉Cl₂F₅N₂O₂, 414.0; m/z found, 415.0 [M+H]⁺. ¹H NMR (400 MHz, CDCl₃) *δ* 9.03 (br s, 1H), 8.46 (d, *J* = 7.8 Hz, 1H), 7.33 - 7.29 (m, 1H), 7.27 - 7.23 (m, 1H), 7.20 - 7.11 (m, 1H), 5.95 (spt, *J* = 6.4 Hz, 1H), 1.67 (d, *J* = 6.5 Hz, 3H); ¹⁹F NMR (376MHz, CDCl₃) *δ* -78.20 (3F), -113.06 (1F), -127.22 (1F) ppm.

Step D. 2-(1-methyl-3-(tributylstannyl)-1*H*-1,2,4-triazol-5-yl)propan-2-ol. To a solution of 2-(3-bromo-1-methyl-1*H*-1,2,4-triazol-5-yl)propan-2-ol (100 mg, 454 µmοl, 1.0 eq.) and Hexa-n-butylditin (450 mg, 776 µmοl, 2.0 eq.) in toluene (1 mL) was added Pd(PPh₃)₂Cl₂ (31.9 mg, 45 µmοl, 0.1 eq.) under N₂. The mixture was stirred at 100 °C overnight. The mixture was filtered. The filter cake was washed by sat. aq. KF solution (1 mL) and discarded. The filtrate as yellow oil was used directly in the next step. MS (ESI): mass calcd. for C₁₈H₃₇N₃OSn, 431.2; m/z found, 432.0 [M+H]⁺.

Step E. (*S*)-*N*-(2-chloro-6-fluorophenyl)-5-fluoro-6-(5-(2-hydroxypropan-2-yl)-1-methyl-1*H-*1,2,4-triazol-3-yl)-2-((1,1,1-trifluoropropan-2-yl)oxy)nicotinamide. To a solution of (*S*)-6-chloro-*N*-(2-chloro-6-fluorophenyl)-5-fluoro-2-((1,1,1-trifluoropropan- 2-yl)oxy)nicotinamide (100 mg, 240 ummol, 1.0 eq.), 2-(1-methyl-3-(tributylstannyl)-1*H*- 1,2,4-triazol-5-yl)propan-2-ol (5.00 g, crude, 1.0 eq.) and Pd(PPh₃)₂Cl₂ (16.9 mg, 24.1 µmοl, 0.1 eq.) in dioxane (1 mL) was stirred at 100 °C for 40 hours under N₂. The mixture was added sat. aq. KF solution (15 mL) and stirred for 2 minutes. The mixture was extracted with ethyl acetate (20 mL × 2). The combined organic layer was concentrated in vacuo. The crude product was purified by preparative reversed phase HPLC (Stationary phase: Phenomenex Gemini-NX, 5µm, 150 × 30 mm; Mobile phase: water (0.05% NH₃H₂O + 10 mM NH₄HCO₃) (A) - MeCN (B), gradient elution: 40 - 70% B in A over 7 min, flow rate: 30 mL/min) to give the title compound as white powder. MS (ESI): mass calcd. For C₂₁H₁₉ClF₅N₅O₃, 519.1; m/z found, 520.1[M+H]⁺. ¹H NMR (400MHz, CDCl₃) *δ* 9.23 (s, 1H), 8.50 (d, *J* = 9.8 Hz, 1H), 7.34 - 7.28 (m, 1H), 7.26 - 7.22 (m, 1H), 7.19 - 7.11 (m, 1H), 6.32 - 6.10 (m, 1H), 4.21 (s, 3H), 2.43 (s, 1H), 1.79 (s, 6H), 1.69 (d, *J* = 6.4Hz, 3H); ¹⁹F NMR (376MHz, CDCl₃) *δ* -78.10 (3F), -112.93 (1F), -127.90 (1F) ppm.

### Example 210: (S)-N-(2-chloro-6-fluorophenyl)-3-fluoro-5'-methyl-6-((1,1,1-trifluoropropan-2-yl)oxy)-[2.2'-bipyridine]-5-carboxamide.

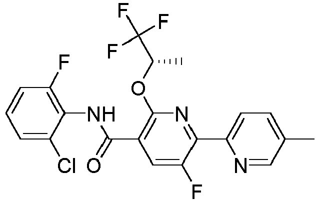

To a solution of (*S*)-6-chloro-*N*-(2-chloro-6-fluorophenyl)-5-fluoro-2-((1,1,1-trifluoropropan- 2-yl)oxy)nicotinamide (Example 209, Step C) 100 mg, 240 ummol, 1.0 eq.), 5-methyl-2-(tributylstannyl)pyridine (101.2 mg, 264 ummol, 1.1 eq.) and Pd(PPh₃)₂Cl₂ (16.9 mg, 24.1 µmol, 0.1 eq.) in dioxane (1.2 mL) was stirred at 100 °C for 12 hours under N₂. The mixture was added sat. aq. KF solution (10 mL) and stirred for 2 minutes. The mixture was extracted with ethyl acetate (20 mL × 2). The combined organic layer was concentrated in vacuo. The crude product was purified by preparative reversed phase HPLC (Stationary phase: Phenomenex Gemini-NX, 5µm, 150 × 30 mm ; Mobile phase: water (0.05% NH₃H₂O + 10 mM NH₄HCO₃) (A) - MeCN (B), gradient elution: 57 - 87% B in A over 7 min, flow rate: 30 mL/min) to give the title compound as white solid. MS (ESI): mass calcd. For C₂₁H₁₅ClF₅N₃O₂, 471.1; m/z found, 472.0[M+H]⁺. ¹H NMR (400 MHz, CDCl₃) *δ* 9.23 (s, 1H), 8.65 (s, 1H), 8.52 (d, *J* = 10.4 Hz, 1H), 7.91 (d, *J* = 8.1 Hz, 1H), 7.69 (dd, *J* = 1.9, 8.1 Hz, 1H), 7.37 - 7.29 (m, 1H), 7.27 - 7.21 (m, 1H), 7.20 - 7.09 (m, 1H), 6.13 (spt, *J* = 6.5 Hz, 1H), 2.46 (s, 3H), 1.70 (d, *J* = 6.6 Hz, 3H); ¹⁹F NMR (376MHz, CDCl₃) *δ* -78.03 (3F), -112.91 (1F), -130.06 (1F) ppm.

### Example 211: (S)-4-(6-amino-5-cyanopyridin-2-yl)-N-(5-chloro-3-methyl-1H-pyrazol-4-yl)-5-fluoro-2-((1,1,1-trifluoropropan-2-yl)oxy)benzamide.

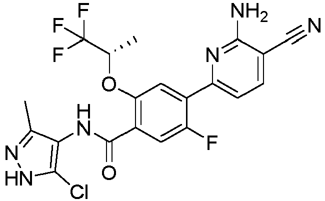

The title compound was prepared in a manner analogous to Example 88 however using 2-amino-6-chloronicotinonitrile as the aryl halide and (S)-(4-((5-chloro-3-methyl-1H-pyrazol-4-yl)carbamoyl)-2-fluoro-5-((1,1,1-trifluoropropan-2-yl)oxy)phenyl)boronic acid (Example 79, Step A) as the boronate. MS (ESI): mass calcd. for C₂₀H₁₅ClF₄N₆O₂, 482.1; m/z found, 483.2, [M+H]⁺. ¹H NMR (DMSO-d₆, 400 MHz) δ 12.85 (br s, 1H), 9.48 (s, 1H), 8.03 (d, 1H, *J*=7.8 Hz), 7.75 (d, 1H, *J*=5.9 Hz), 7.49 (d, 1H, *J*=10.5 Hz), 7.0-7.1 (m, 3H), 5.2-5.4 (m, 1H), 2.15 (s, 3H), 1.48 (d, 3H, *J*=6.4 Hz).

### Biological Data

DHODH inhibitory activities of the compounds of Examples 1-211 were assessed using the following assays. The half maximal inhibitory concentration values (IC₅₀) are summarized in Table 1.

### BIOLOGICAL ASSAYS

### In vitro Assay: DHODH enzymatic assay

To detect DHODH enzyme activities, dichloroindophenol (DCIP) is added as the final electron acceptor in the assay. DCIP can accept electrons from the reduced coenzyme Q generated in the assay, or from dihydroorotate (DHO) via FMN by binding presumably to the ubiquinone pocket. DCIP solutions are blue, with an intense absorbance around 600 nm, but becomes colorless upon reduction (J. Biol. Chem. (1986) 261, 11386). The assay buffer contained 50 nM HEPES, pH 7.5, 150 mM NaCl, 0.5 mM EDTA, and 0.1% Triton X-100 in MilliQ water. Substrate consisting of 20 mM DHO, 5mM CoQ₆, and 1mM DCIP in assay buffer, initiates the reaction. The assay is run in end-point mode by quenching the reaction with the potent DHODH inhibitor brequinar. Absorbance measurements were obtained using the BMG Phera Star plate-reading spectrophotomer. Purified human DHODH was purchased from Proteros (cat. No. PR-0044). Chemicals were purchased from Sigma-Aldrich, Teknova, and Avanti Polar Lipids. Liquid handling was performed using Labcyte Echo and Formulatrix Tempest.

### In vitro Assay: MOLM-13 Cellular Assay

MOLM-13 cells (human AML cells) were obtained from DSMZ and were maintained in RPMI 1640 + Glutamax + 25mM HEPES (Invitrogen, catalog number 72400) supplemented with 10% heat inactivated fetal bovine serum (FBS; Invitrogen, catalog number 16140). The day prior to assay set-up, cells were pelleted, resuspended in fresh media, counted, and cells were plated at 0.4 × 10⁶ cell/mL in a T150 flask. On the day of the assay, cells were pelleted, resuspend in fresh media, counted and seeded at 5,000 cells/well in white opaque 96-well tissue culture treated microplates (Perkin Elmer, catalog number 6005680). Cells were exposed to different concentrations of test compounds at 37 °C, 5% CO₂ for 72 hours immediately after seeding. Cell viability was acquired on a Perkin Elmer Envision 2104 multilabel reader using the CellTiter-Glo assay (Promega) according to the manufacturer's instructions.

**Table 1.**

| **example #** | **hDHODH IC₅₀ (nM)** | **MOLM-13 Cell Viability IC₅₀ (nM)** |
|---|---|---|
| 1 | 32.1 | 7 |
| 2 | 2.06 | |
| 3 | 352 | |
| 4 | 0.287 | 0.05 |
| 5 | 0.37 | 0.1 |
| 6 | 0.466 | 0.2 |
| 7 | 872 | |
| 8 | 228 | 50 |
| 9 | 4.84 | 0.5 |
| 10 | -0.4 | 0.1 |
| 11 | 0.943 | 0.3 |
| 12 | 2.87 | 2 |
| 13 | 3.52 | 0.7 |
| 14 | 0.533 | 0.2 |
| 15 | 19.9 | 8 |
| 16 | 110 | 20 |
| 17 | 33.6 | 8 |
| 18 | 1.75 | 1 |
| 19 | >10000 | >100 |
| 20 | 9.36 | 3 |
| 21 | 69.5 | 10 |
| 22 | 4.01 | 0.9 |
| 23 | 0.589 | 0.4 |
| 24 | 30.1 | 10 |
| 25 | 1.97 | 0.4 |
| 26 | 5.51 | 3 |
| 27 | 1.42 | 2 |
| 28 | 278 | >100 |
| 29 | 35.3 | 10 |
| 30 | 5.02 | 40 |
| 31 | 0.274 | 0.3 |
| 32 | 3.5 | 3 |
| 33 | <0.17 | 0.2 |
| 34 | 1.6 | 2 |
| 35 | 0.775 | 1 |
| 36 | 0.545 | 1 |
| 37 | 0.62 | 2 |
| 38 | 6.32 | 1 |
| 39 | <0.17 | 0.9 |
| 40 | 2943 | >100 |
| 41 | 0.266 | 3 |
| 42 | 4.73 | 2 |
| 43 | 5.54 | 4 |
| 44 | 491 | >100 |
| 45 | 6.7 | >100 |
| 46 | 2.38 | 1 |
| 47 | 38.5 | >100 |
| 48 | 0.317 | 0.1 |
| 49 | 0.591 | 20 |
| 50 | 1.08 | 0.2 |
| 51 | <0.17 | 0.09 |
| 52 | 1.18 | 0.8 |
| 53 | 0.246 | 0.2 |
| 54 | 0.361 | 0.2 |
| 55 | 22.2 | 10 |
| 56 | 31.8 | 20 |
| 57 | 4.28 | 2 |
| 58 | 5.1 | 4 |
| 59 | 20.4 | 8 |
| 60 | 1.79 | 2 |
| 61 | 13.6 | 7 |
| 62 | 0.229 | 0.2 |
| 63 | 0.484 | 0.9 |
| 64 | 68.5 | >100 |
| 65 | 3.26 | 2 |
| 66 | 1.01 | 0.5 |
| 67 | 11.4 | 10 |
| 68 | | |
| 69 | 0.195 | 0.03 |
| 70 | 1.23 | 0.1 |
| 71 | 2 | 0.3 |
| 72 | 0.665 | 0.4 |
| 73 | 0.365 | 0.2 |
| 74 | 0.243 | 0.2 |
| 75 | 0.998 | 5 |
| 76 | 0.398 | 0.4 |
| 77 | 0.251 | 0.1 |
| 78 | 11 | 6 |
| 79 | 0.181 | 0.08 |
| 80 | 0.554 | 0.3 |
| 81 | 0.699 | 2 |
| 82 | 3.22 | 8 |
| 83 | 0.208 | 0.4 |
| 84 | 0.465 | 2 |
| 85 | 22.2 | 60 |
| 86 | 0.417 | 0.5 |
| 87 | 0.616 | 2 |
| 88 | 0.267 | 0.2 |
| 89 | 0.906 | 3 |
| 90 | 0.735 | 4 |
| 91 | 0.577 | 2 |
| 92 | 24 | 30 |
| 93 | 25.7 | 9 |
| 94 | 0.313 | 0.1 |
| 95 | 0.284 | 0.07 |
| 96 | 0.375 | 0.3 |
| 97 | 0.192 | 0.03 |
| 98 | 0.396 | 0.4 |
| 99 | 0.211 | 0.1 |
| 100 | 0.191 | 0.3 |
| 101 | <0.17 | 0.2 |
| 102 | 0.279 | 7 |
| 103 | 14.2 | >100 |
| 104 | 0.368 | 0.1 |
| 105 | 0.765 | 0.5 |
| 106 | 0.955 | 2 |
| 107 | 0.324 | 0.2 |
| 108 | 9.8 | 50 |
| 109 | 0.214 | 0.07 |
| 110 | 1.46 | 7 |
| 111 | 6.31 | 3 |
| 112 | 0.631 | 0.7 |
| 113 | 1.94 | 10 |
| 114 | 0.412 | 0.1 |
| 115 | 0.694 | 2 |
| 116 | 0.72 | 0.7 |
| 117 | 34 | 8 |
| 118 | 138 | 60 |
| 119 | 0.202 | 0.06 |
| 120 | 0.338 | 0.07 |
| 121 | 50.3 | 2 |
| 122 | 0.47 | 1 |
| 123 | 0.507 | 2 |
| 124 | 0.309 | 0.7 |
| 125 | 22 | 3 |
| 126 | 0.436 | 7 |
| 127 | 1.19 | >100 |
| 128 | | >100 |
| 129 | | 0.9 |
| 130 | 0.19 | 0.02 |
| 131 | 0.309 | 0.06 |
| 132 | 0.333 | 0.3 |
| 133 | 0.179 | 0.02 |
| 134 | 0.237 | 0.06 |
| 135 | 0.637 | 0.2 |
| 136 | 0.48 | 3 |
| 137 | 0.499 | 1 |
| 138 | 1127 | 90 |
| 139 | 0.315 | 0.1 |
| 140 | 314 | >100 |
| 141 | 1.43 | 0.2 |
| 142 | 0.352 | 0.6 |
| 143 | 0.386 | 0.1 |
| 144 | 0.752 | 0.3 |
| 145 | 0.193 | 0.7 |
| 146 | 0.616 | 0.3 |
| 147 | 0.409 | 0.2 |
| 148 | 0.61 | 0.6 |
| 149 | 168 | >100 |
| 150 | 0.319 | 0.6 |
| 151 | 4.83 | 1 |
| 152 | 5.16 | 2 |
| 153 | 0.407 | 0.5 |
| 154 | 0.753 | 0.4 |
| 155 | 0.427 | 0.4 |
| 156 | 0.9 | 0.2 |
| 157 | 2.05 | 0.4 |
| 158 | 0.612 | 2 |
| 159 | 0.295 | 0.6 |
| 160 | 5.26 | 9 |
| 161 | 10.3 | 3 |
| 162 | >10000 | >100 |
| 163 | 1.56 | 1 |
| 164 | 0.895 | 0.3 |
| 165 | 2.08 | 1 |
| 166 | 0.259 | 4 |
| 167 | 2.38 | 2 |
| 168 | 13.1 | 30 |
| 169 | 0.219 | 0.7 |
| 170 | 2.58 | 2 |
| 171 | 6.39 | 2 |
| 172 | 1.52 | 2 |
| 173 | 0.662 | 1 |
| 174 | 0.664 | 0.07 |
| 175 | 0.225 | 0.03 |
| 176 | 0.345 | 0.1 |
| 177 | 0.424 | 0.05 |
| 178 | 26.9 | 3 |
| 179 | 37.3 | 4 |
| 180 | 3.94 | 5 |
| 181 | 31.5 | 7 |
| 182 | 9.54 | 3 |
| 183 | 0.464 | 0.05 |
| 184 | 2 | 2 |
| 185 | 12.3 | 1 |
| 186 | 23.9 | 6 |
| 187 | 0.459 | 3 |
| 188 | 1.62 | 8 |
| 189 | 2.42 | 6 |
| 190 | 0.481 | 0.09 |
| 191 | 0.971 | 0.4 |
| 192 | 0.942 | 0.1 |
| 193 | 3.92 | 7 |
| 194 | 52.1 | 7 |
| 195 | 0.603 | 0.1 |
| 196 | 0.779 | 0.1 |
| 197 | | 2 |
| 198 | | 0.9 |
| 199 | | 0.8 |
| 200 | | 5 |
| 201 | 0.288 | 0.09 |
| 202 | 109 | >100 |
| 203 | 0.215 | 0.08 |
| 206 | 5.45 | 8 |
| 208 | 0.307 | 0.3 |
| 209 | 1.0 | |
| 210 | 0.47 | 0.5 |
| 211 | 2.09 | 1 |

While the foregoing specification teaches the principles of the present invention, with examples provided for the purpose of illustration, it will be understood that the practice of the invention encompasses all of the usual variations, adaptations and/or modifications as come within the scope of the following claims.

## Claims

1. A compound of Formula I 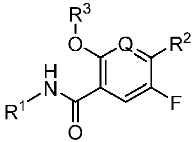 wherein
Q is CH or N;
R¹ is 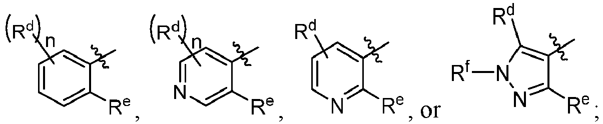 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is
imidazolyl; wherein said imidazolyl is optionally substituted with up to three substituents independently selected from the group comprising 3-hydroxyoxetanyl, -NH₂, -OH, -CN, or R^{a};
triazolyl; wherein said triazolyl is optionally substituted with one or two groups independently selected from the group comprising R^{a};
pyrazinyl; wherein said pyrazinyl is optionally substituted with up to three substituents independently selected from the group comprising -NHSO₂CH₃, -NHC(O)CH₃, -OCH₃, - OH, -NH2, -CO₂H, -Cl, -F, or R^{a};
pyrazinonyl; wherein said pyrazinonyl is optionally substituted with one or two groups independently selected from the group comprising R^{a};
thiazolyl; wherein said thiazolyl is optionally substituted with one or two substituents independently selected from the group comprising R^{a};
5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
5,6,7,8-tetrahydro-[1,2,4]triazolo[1,5-a]pyrazinyl; wherein said 5,6,7,8-tetrahydro-[1,2,4]triazolo[4,3-a]pyrazinyl is optionally substituted with R^{a};
pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
pyridazinyl; wherein said pyridazinyl is optionally substituted with one or two groups independently selected from the group comprising R^{a}, -Cl, or -CO₂H;
4,5,6,7-tetrahydrothiazolo[5,4-c]pyridinyl; wherein said 4,5,6,7-tetrahydrothiazolo[5,4-c]pyridinyl is optionally substituted with R^{a};
pyrazolyl; wherein said pyrazolyl is optionally substituted with one or two substituents independently selected from the group comprising R^{a};
oxazolyl; wherein said oxazolyl is optionally substituted with one or two substituents independently selected from the group comprising R^{a};
1,3,4-oxadiazolyl; wherein said 1,3,4-oxadiazolyl is optionally substituted with R^{a};
5,6,7,8-tetrahydroimidazo[1,5-*a*]pyridinyl; wherein said 5,6,7,8-tetrahydroimidazo[1,5-*a*]pyridinyl is optionally substituted with R^{a};
1,2,4-thiadiazolyl; wherein said 1,2,4-thiadiazolyl is optionally substituted with R^{a};
5,6,7,8-tetrahydro-1,7-naphthyridinyl; wherein said 5,6,7,8-tetrahydro-1,7-naphthyridinyl is optionally substituted with R^{a};
5,6,7,8-tetrahydro-1,6-naphthyridinyl; wherein said 5,6,7,8-tetrahydro-1,6-naphthyridinyl is optionally substituted with R^{a};
5,6,7,8-tetrahydro-1,8-naphthyridinyl; wherein said 5,6,7,8-tetrahydro-1,8-naphthyridinyl is optionally substituted with R^{a};
1,2,3,4-tetrahydro-2,7-naphthyridinyl; wherein said 1,2,3,4-tetrahydro-2,7-naphthyridinyl is optionally substituted with R^{a};
2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with R^{a};
2,3-dihydro-1*H*-pyrrolo[2,3-*b*]pyridinyl; wherein said 2,3-dihydro-1*H*-pyrrolo[2,3-*b*]pyridinyl is optionally substituted with R^{a};
imidazo[1,2-*a*]pyrazinyl, wherein said imidazo[1,2-*a*]pyrazinyl is optionally substituted with -NH₂ or R^{a};
2,6-dioxo-1,2,3,6-tetrahydropyrimidinyl, wherein said 2,6-dioxo-1,2,3,6-tetrahydropyrimidinyl is optionally substituted with R^{a};
6-oxo-1,6-dihydropyrimidinyl, wherein said 6-oxo-1,6-dihydropyrimidinyl is optionally substituted with fluorine or R^{a};
6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of R^{a} and -OH;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -OH, -CH₂OH, or up to two fluorine atoms;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together together to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said -C₍₁₋₆₎alkyl is optionally substituted with up to six fluorine atoms;
or a pharmaceutically acceptable salt, solvate, stereoisomer, isotopic variant, or N-oxide thereof.

2. The compound of claim 1 which is Formula II 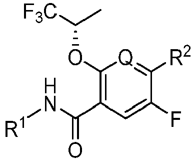 wherein
Q is CH or N;
R¹ is 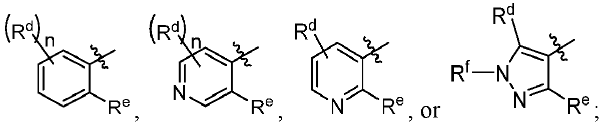 wherein
R^{d} is selected from the group consisting of: -H, -Cl, -F, and -C₍₁₋₃₎alkyl; wherein said -C(i-₃₎alkyl is optionally substituted with -OH, -OCH₃, -SCH₃, or -OCF₃; or said -C₍₁₋₃₎alkyl is optionally substituted with up to three fluorine atoms;
R^{e} is selected from the group consisting of: -Cl, -F, -CD₃, -C₍₁₋₃₎alkyl, and cyclopropyl; wherein said -C₍₁₋₃₎alkyl is optionally substituted with -OH, -OCH₃, -SCH₃, or -OCF₃; or said -C₍₁₋₃₎alkyl is optionally substituted with up to three fluorine atoms;
R^{f} is -H; and
n is 1, or 2;
R² is
imidazolyl; wherein said imidazolyl is optionally substituted with up to three substituents independently selected from the group comprising 3-hydroxyoxetanyl, -NH₂, -OH, -CN, or R^{a};
triazolyl; wherein said triazolyl is optionally substituted with one or two groups independently selected from the group comprising R^{a};
pyrazinyl; wherein said pyrazinyl is optionally substituted with up to three substituents independently selected from the group comprising -NHSO₂CH₃, -NHC(O)CH₃, -OCH₃, - OH, -NH2, -CO₂H, -Cl, -F, or R^{a};
pyrazinonyl; wherein said pyrazinonyl is optionally substituted with one or two groups independently selected from the group comprising R^{a};
thiazolyl; wherein said thiazolyl is optionally substituted with one or two substituents independently selected from the group comprising R^{a};
5,6,7,8-tetrahydro-[1,2,4]triazolo[4,3-*a*]pyrazinyl;
5,6,7,8-tetrahydro-[1,2,4]triazolo[1,5-*a*]pyrazinyl;
pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
pyridazinyl; wherein said pyridazinyl is optionally substituted with one or two groups independently selected from the group comprising R^{a};
4,5,6,7-tetrahydrothiazolo[5,4-*c*]pyridinyl;
pyrazolyl; wherein said pyrazolyl is optionally substituted with one or two substituents independently selected from the group comprising R^{a};
oxazolyl; wherein said oxazolyl is optionally substituted with one or two substituents independently selected from the group comprising R^{a};
1,3,4-oxadiazolyl; wherein said 1,3,4-oxadiazolyl is substituted with cyclopropyl;
5,6,7,8-tetrahydroimidazo[1,5-*a*]pyridinyl; wherein said 5,6,7,8-tetrahydroimidazo[1,5-*a*]pyridinyl is optionally substituted with R^{a};
1,2,4-thiadiazolyl; wherein said 1,2,4-thiadiazolyl is substituted with cyclopropyl;
5,6,7,8-tetrahydro-1,7-naphthyridinyl;
5, 6, 7, 8-tetrahy dro-1, 6-naphthyridinyl;
5, 6, 7, 8-tetrahy dro-1, 8-naphthyridinyl;
1,2,3,4-tetrahydro-2,7-naphthyridinyl;
2*H*-pyrido[3,2-*b*][1,4]oxazin-3(4*H*)-onyl, wherein said 2*H*-pyrido[3,2-b][1,4]oxazin-3(4*H*)-onyl is optionally substituted with R^{a};
2,3-dihydro-1*H*-pyrrolo[2,3-*b*]pyridinyl;
imidazo[1,2-*a*]pyrazinyl, wherein said imidazo[1,2-*a*]pyrazinyl is substituted with -NH₂;
2,6-dioxo-1,2,3,6-tetrahydropyrimidinyl, wherein said 2,6-dioxo-1,2,3,6-tetrahydropyrimidinyl is optionally substituted with R^{a};
6-oxo-1,6-dihydropyrimidinyl, wherein said 6-oxo-1,6-dihydropyrimidinyl is substituted with fluorine;
6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of R^{a} and -OH;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
or a pharmaceutically acceptable salt, solvate, stereoisomer, isotopic variant, or N-oxide thereof.

3. The compound of claim 1 or claim 2, wherein:
(a) Q is CH or N;
R¹ is 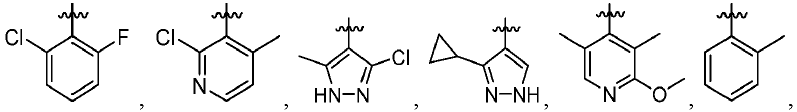 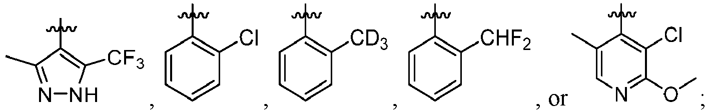
R² is
imidazolyl; wherein said imidazolyl is optionally substituted with up to three substituents independently selected from the group comprising 3-hydroxyoxetanyl, -NH₂, -OH, -CN, or R^{a};
triazolyl; wherein said triazolyl is optionally substituted with one or two groups independently selected from the group comprising R^{a};
pyrazinyl; wherein said pyrazinyl is optionally substituted with up to three substituents independently selected from the group comprising -OCH₃, -OH, -NH₂, -CO₂H, -Cl, -F, or R^{a};
pyrazinonyl; wherein said pyrazinonyl is optionally substituted with one or two groups independently selected from the group comprising R^{a};
thiazolyl; wherein said thiazolyl is optionally substituted with two substituents independently selected from the group comprising R^{a};
5,6,7,8-tetrahydro-[1,2,4]triazolo[4,3-*a*]pyrazinyl;
5,6,7,8-tetrahydro-[1,2,4]triazolo[1,5-*a*]pyrazinyl;
pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
pyridazinyl; wherein said pyridazinyl is optionally substituted with R^{a};
4,5,6,7-tetrahydrothiazolo[5,4-c]pyridinyl;
pyrazolyl; wherein said pyrazolyl is optionally substituted with one or two substituents independently selected from the group comprising R^{a};
oxazolyl; wherein said oxazolyl is optionally substituted with two substituents independently selected from R^{a};
1,3,4-oxadiazolyl; wherein said 1,3,4-oxadiazolyl is substituted with cyclopropyl;
5,6,7,8-tetrahydroimidazo[1,5-*a*]pyridinyl wherein said 5,6,7,8-tetrahydroimidazo[1,5-*a*]pyridinyl is optionally substituted with R^{a};
1,2,4-thiadiazolyl; wherein said 1,2,4-thiadiazolyl is substituted with cyclopropyl;
5,6,7,8-tetrahydro-1,7-naphthyridinyl;
5,6,7,8-tetrahydro-1,6-naphthyridinyl;
5,6,7,8-tetrahydro-1,8-naphthyridinyl;
1,2,3,4-tetrahydro-2,7-naphthyridinyl;
2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with -CH₃;
2,3-dihydro-1*H-*pyrrolo[2,3-b]pyridinyl;
imidazo[1,2-*a*]pyrazinyl, wherein said imidazo[1,2-*a*]pyrazinyl is substituted with -NH₂;
2,6-dioxo-1,2,3,6-tetrahydropyrimidinyl, wherein said 2,6-dioxo-1,2,3,6-tetrahydropyrimidinyl is substituted with -CH₃;
6-oxo-1,6-dihydropyrimidinyl, wherein said 6-oxo-1,6-dihydropyrimidinyl is substituted with fluorine;
6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of -CH₃ and -OH;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
or a pharmaceutically acceptable salt, solvate, stereoisomer, isotopic variant, or N-oxide thereof; or
(b) Q is CH or N;
R¹ is 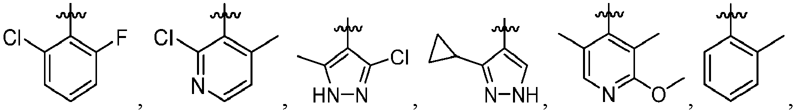 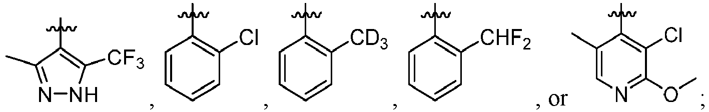
R² is
imidazolyl; wherein said imidazolyl is substituted with one, two, or three substituents independently selected from the group comprising -C₍₁₋₃₎alkyl, 3-hydroxyoxetanyl, cyclopropyl, -C(O)NH₂, -NH₂, -OH, or -CN; wherein said -C₍₁₋₃₎alkyl is optionally substituted with -OH and additionally optionally substituted with up to three fluorine atoms;
triazolyl; wherein said triazolyl is substituted with up to two groups independently selected from the group comprising -C(O)NH₂, -CF₃, cyclopropyl, or -C₍₁₋₃₎alkyl, wherein said -C₍₁₋₃₎alkyl is optionally substituted with -OH;
pyrazinyl; wherein said pyrazinyl is optionally substituted with up to three substituents independently selected from the group comprising -C₍₁₋₃₎alkyl, -OCH₃, -OH, -NH₂, cyclopropyl, -C(O)NH₂, -CO₂H, -Cl, -F, or -CF₃; wherein said cyclopropyl is optionally substituted with -CH₂OH; and wherein said -C₍₁₋₃₎alkyl is optionally substituted with - OH;
pyrazinonyl; wherein said pyrazinonyl is optionally substituted with one or two -C₍₁₋₃₎alkyl groups;
thiazolyl; wherein said thiazolyl is substituted with two substituents independently selected from the group comprising -C₍₁₋₃₎alkyl, cyclopropyl, -Cl, or -C(O)NH₂; wherein said -C₍₁₋₃₎alkyl, is optionally substituted with -OH, or up to three fluorine atoms;
5,6,7,8-tetrahydro-[1,2,4]triazolo[4,3-*a*]pyrazinyl;
5,6,7,8-tetrahydro-[1,2,4]triazolo[1,5-*a*]pyrazinyl;
pyridinyl; wherein said pyridinyl is substituted with one or two substituents independently selected from the group comprising -CONH₂, -C₍₁₋₃₎alkyl, cyclopropyl, - Cl, -F, -CN, -NHC(O)CH₃, -NHSO₂CH₃, -CO₂H, -OCH₃, -OH, or -NH₂; wherein said - C₍₁₋₃₎alkyl is optionally substituted with up to three fluorine atoms, or the -C₍₁₋₃₎alkyl is optionally substituted with -OH and additionally optionally substituted with up to three fluorine atoms;
pyridazinyl; wherein said pyridazinyl is substituted with -C₍₁₋₃₎alkyl; wherein said -C(i-₃₎alkyl is optionally substituted with -OH;
4,5,6,7-tetrahydrothiazolo[5,4-c]pyridinyl;
pyrazolyl; wherein said pyrazolyl is substituted with two substituents independently selected from the group comprising -C₍₁₋₃₎alkyl, and cyclopropyl; wherein said -C₍₁₋₃₎alkyl is optionally substituted with -OH;
oxazolyl; wherein said oxazolyl is optionally substituted with two substituents independently selected from -C₍₁₋₃₎alkyl; wherein said -C₍₁₋₃₎alkyl is optionally substituted with -OH;
1,3,4-oxadiazolyl; wherein said 1,3,4-oxadiazolyl is substituted with cyclopropyl;
5,6,7,8-tetrahydroimidazo[1,5-*a*]pyridinyl wherein said 5,6,7,8-tetrahydroimidazo[1,5-*a*]pyridinyl is substituted with -C₍₁₋₃₎alkyl; wherein said -C₍₁₋₃₎alkyl is optionally substituted with -OH;
1,2,4-thiadiazolyl; wherein said 1,2,4-thiadiazolyl is substituted with cyclopropyl;
5,6,7,8-tetrahydro-1,7-naphthyridinyl;
5,6,7,8-tetrahydro-1,6-naphthyridinyl;
5,6,7,8-tetrahydro-1,8-naphthyridinyl;
1,2,3,4-tetrahydro-2,7-naphthyridinyl;
2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with -CH₃;
2,3-dihydro-1*H*-pyrrolo[2,3-*b*]pyridinyl;
imidazo[1,2-*a*]pyrazinyl, wherein said imidazo[1,2-*a*]pyrazinyl is substituted with -NH₂;
2,6-dioxo-1,2,3,6-tetrahydropyrimidinyl, wherein said 2,6-dioxo-1,2,3,6-tetrahydropyrimidinyl is substituted with -CH₃;
6-oxo-1,6-dihydropyrimidinyl, wherein said 6-oxo-1,6-dihydropyrimidinyl is substituted with fluorine;
6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of -CH₃ and -OH;
or a pharmaceutically acceptable salt, solvate, stereoisomer, isotopic variant, or N-oxide thereof.

4. The compound of any of claims 1-3, wherein
Q is CH or N;
R¹ is 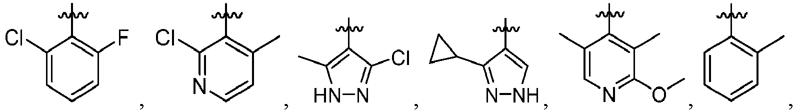 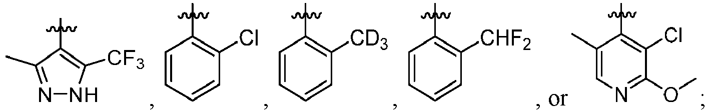
R² is
imidazolyl; wherein said imidazolyl is substituted with one, two, or three substituents independently selected from the group comprising -C₍₁₋₃₎alkyl, 3-hydoxyoxetanyl,
cyclopropyl, -C(O)NH₂, -NH₂, -OH, or -CN; wherein said -C₍₁₋₃₎alkyl is optionally substituted with -OH and additionally optionally substituted with up to three fluorine atoms;
triazolyl; wherein said triazolyl is substituted with up to two groups independently selected from the group comprising -C(O)NH₂, -CF₃, cyclopropyl, or -C₍₁-₃₎alkyl, wherein said -C₍₁₋₃₎alkyl is optionally substituted with -OH;
pyrazinyl; wherein said pyrazinyl is optionally substituted with up to three substituents independently selected from the group comprising -C₍₁-₃₎alkyl, -OCH₃, -OH, -NH₂, cyclopropyl, -C(O)NH₂, -CO₂H, -Cl, -F, or -CF₃, wherein said cyclopropyl is optionally substituted with -CH₂OH, and wherein said -C₍₁₋₃₎alkyl is optionally substituted with - OH;
pyrazinonyl; wherein said pyrazinonyl is substituted with -CH₃ and -CH₂CH₂CH₃;
thiazolyl; wherein said thiazolyl is substituted with two substituents independently selected from the group comprising -C₍₁₋₃₎alkyl, cyclopropyl, -Cl, or -C(O)NH₂; wherein said -C₍₁₋₃₎alkyl is optionally substituted with -OH, or up to three fluorine atoms;
5,6,7,8-tetrahydro-[1,2,4]triazolo[4,3-*a*]pyrazinyl;
5,6,7,8-tetrahydro-[1,2,4]triazolo[1,5-*a*]pyrazinyl;
pyridinyl; wherein said pyridinyl is substituted with one or two substituents independently selected from the group comprising -CONH₂, -CF₃, -C₍₁-₃₎alkyl, cyclopropyl, -Cl, -F, -CN, -NHC(O)CH₃, -NHSO₂CH₃, -CO₂H, -OCH₃, -OH, or -NH₂, wherein said -C₍₁₋₃₎alkyl is optionally substituted with -OH and additionally optionally substituted with up to three fluorine atoms;
pyridazinyl; wherein said pyridazinyl is substituted with -CH₃;
4,5,6,7-tetrahydrothiazolo[5,4-c]pyridinyl;
pyrazolyl; wherein said pyrazolyl is substituted with two substituents independently selected from the group comprising -C₍₁₋₃₎alkyl, and cyclopropyl, wherein said -C₍₁₋₃₎alkyl is optionally substituted with -OH;
oxazolyl; wherein said oxazolyl is substituted with two substituents independently selected from -C₍₁₋₃₎alkyl; wherein said -C₍₁₋₃₎alkyl is optionally substituted with -OH;
1,3,4-oxadiazolyl; wherein said 1,3,4-oxadiazolyl is substituted with cyclopropyl;
5,6,7,8-tetrahydroimidazo[1,5-*a*]pyridinyl; wherein said 5,6,7,8-tetrahydroimidazo[1,5-*a*]pyridinyl is substituted with -CH₂OH;
1,2,4-thiadiazolyl; wherein said 1,2,4-thiadiazolyl is substituted with cyclopropyl;
5,6,7,8-tetrahydro-1,7-naphthyridinyl;
5,6,7,8-tetrahydro-1,6-naphthyridinyl;
5,6,7,8-tetrahydro-1,8-naphthyridinyl;
1,2,3,4-tetrahydro-2,7-naphthyridinyl;
2*H*-pyrido[3,2-*b*][1,4]oxazin-3(4*H*)-onyl, wherein said 2*H*-pyrido[3,2-*b*][1,4]oxazin-3(4*H*)-onyl is optionally substituted with -CH₃;
2,3-dihydro-1*H*-pyrrolo[2,3-*b*]pyridinyl;
imidazo[1,2-*a*]pyrazinyl, wherein said imidazo[1,2-*a*]pyrazinyl is substituted with -NH₂;
2,6-dioxo-1,2,3,6-tetrahydropyrimidinyl, wherein said 2,6-dioxo-1,2,3,6-tetrahydropyrimidinyl is substituted with -CH₃;
6-oxo-1,6-dihydropyrimidinyl, wherein said 6-oxo-1,6-dihydropyrimidinyl is substituted with fluorine;
6,7-dihydro-5*H*-pyrrolo[1,2-*a*]imidazolyl, wherein said 6,7-dihydro-5*H*-pyrrolo[1,2-*a*]imidazolyl is optionally substituted with one or two substituents selected from the group consisting of -CH₃ and -OH;
or a pharmaceutically acceptable salt, solvate, stereoisomer, isotopic variant, or N-oxide thereof.

5. The compound of any of claims 1-4, wherein the compound is selected from the group consisting of: 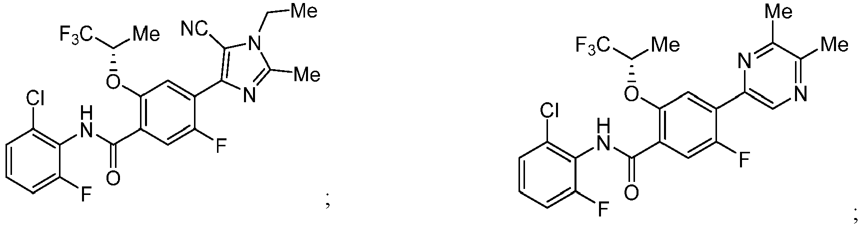 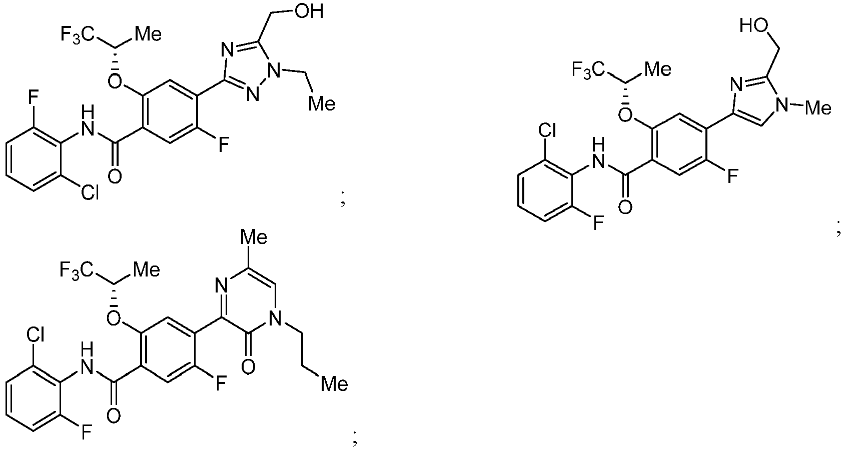 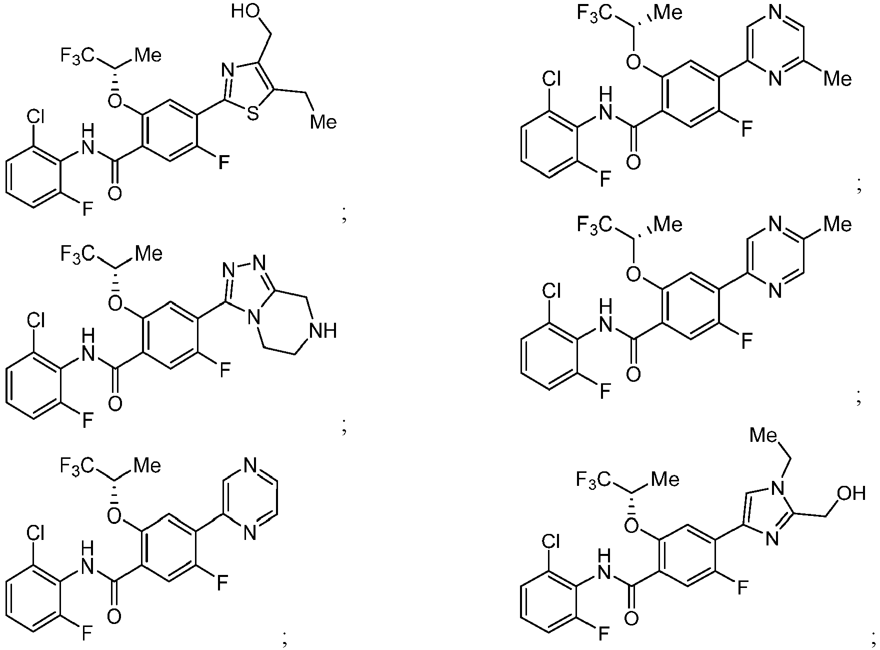 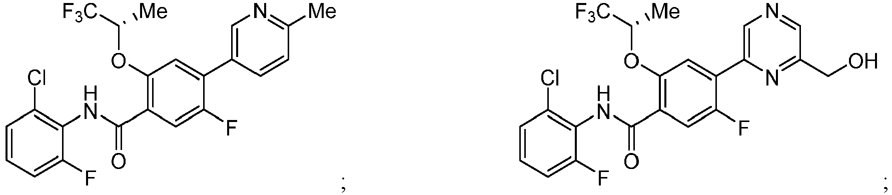 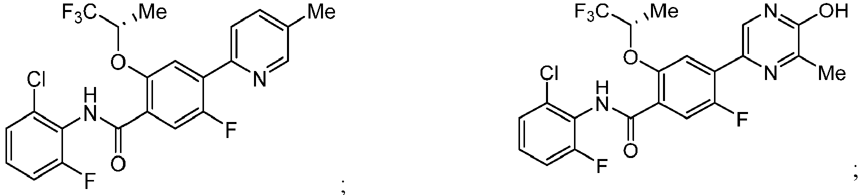 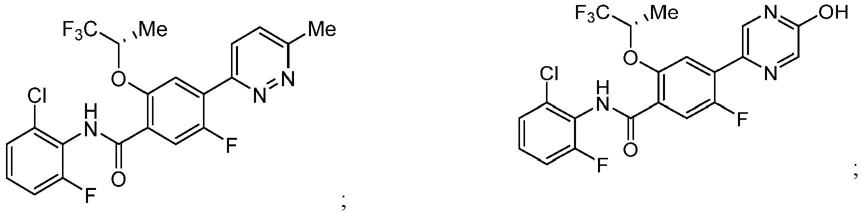 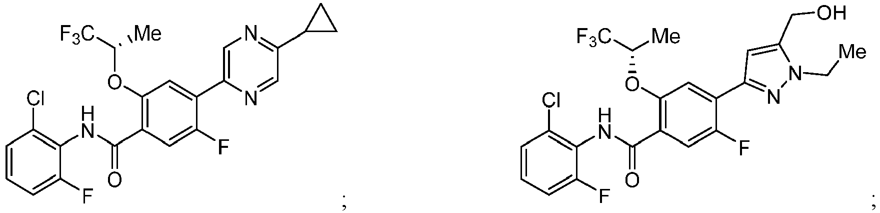 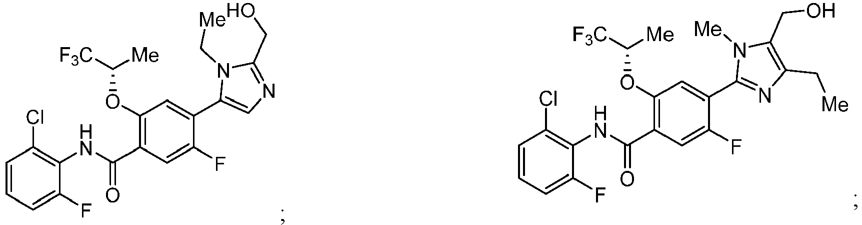 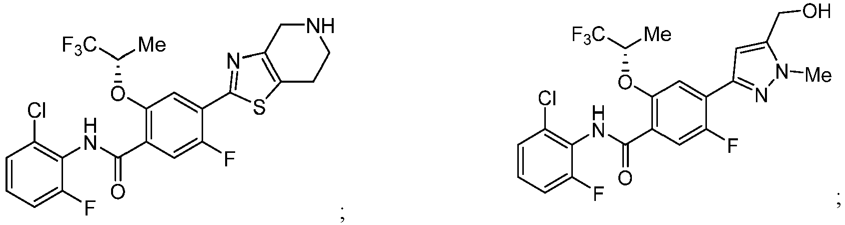 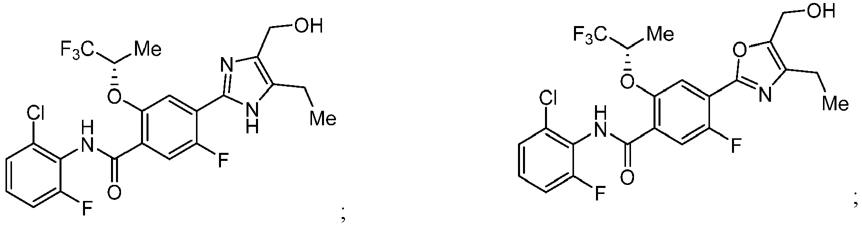 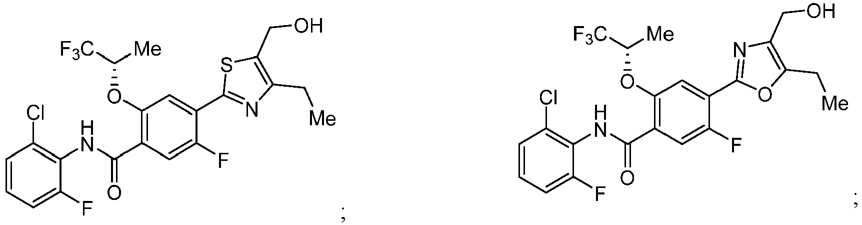 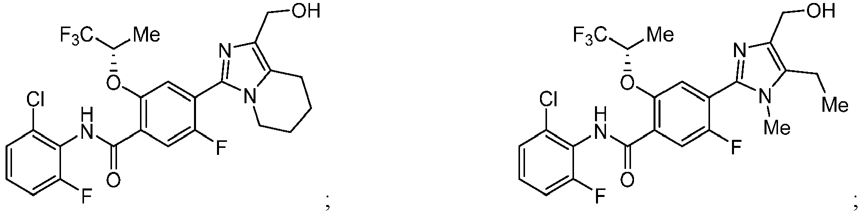 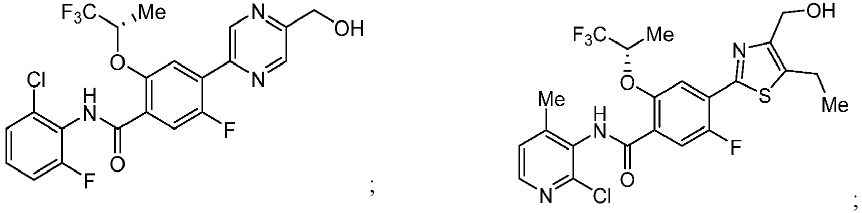 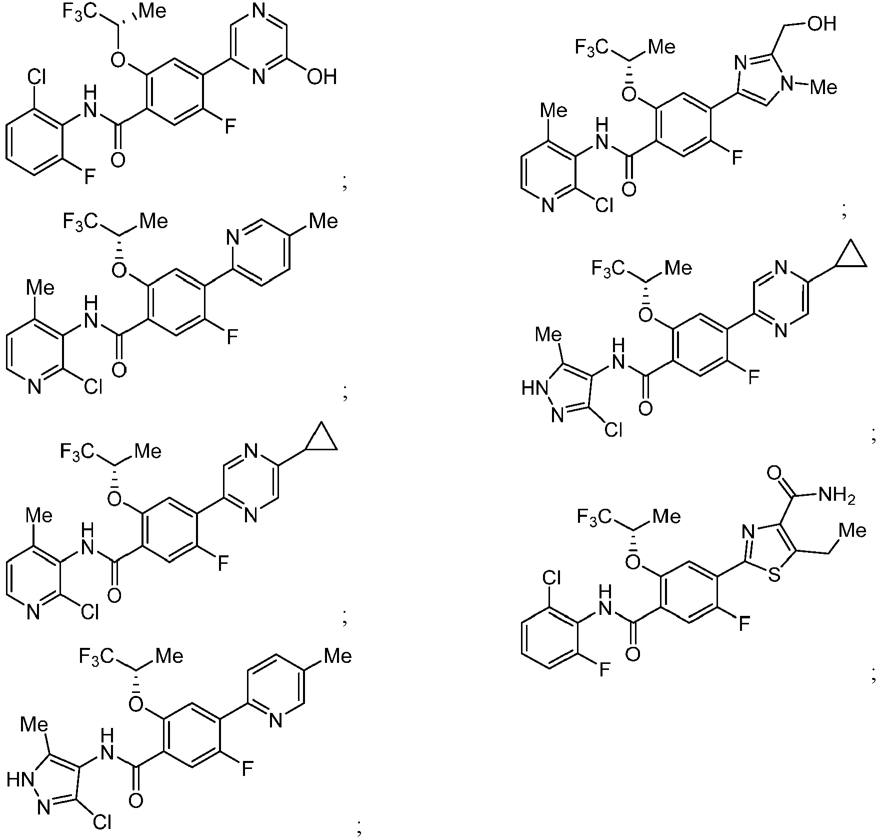 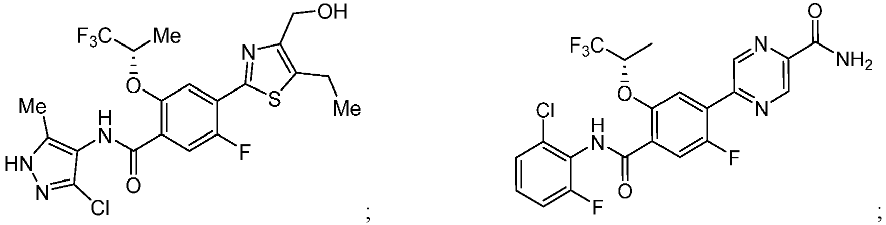 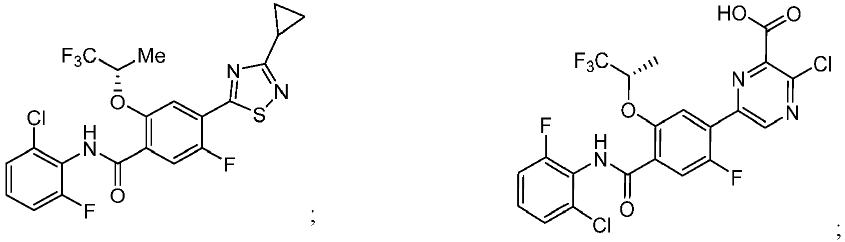 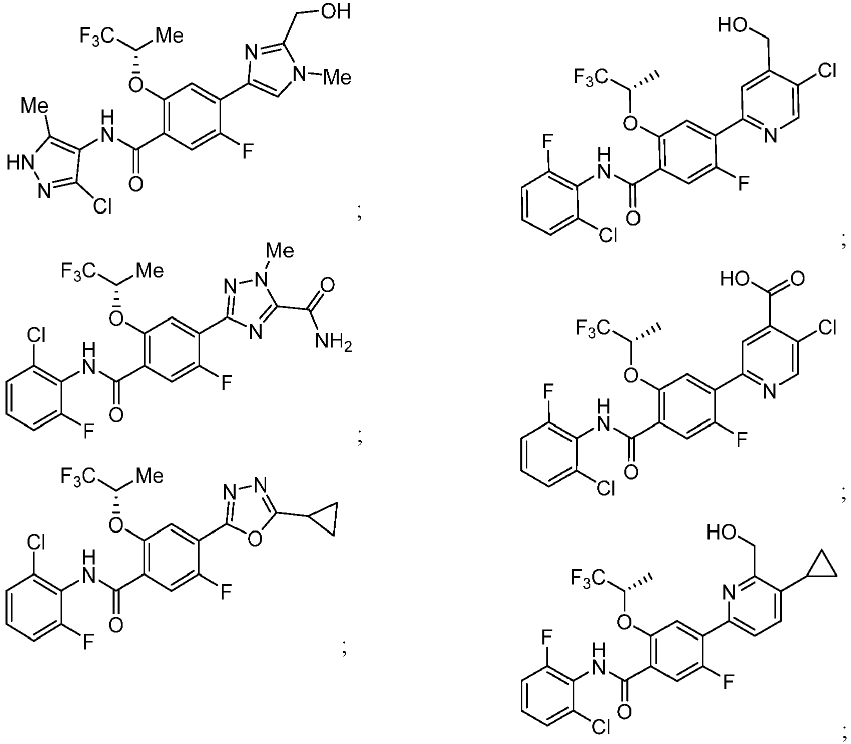 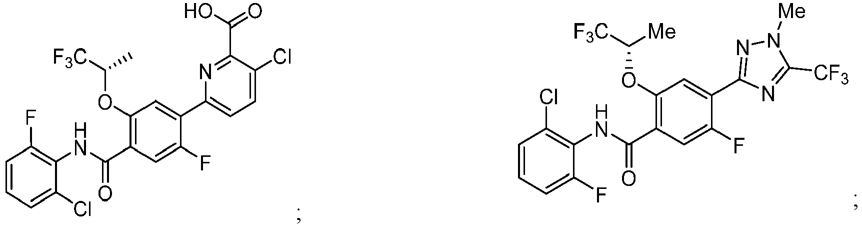 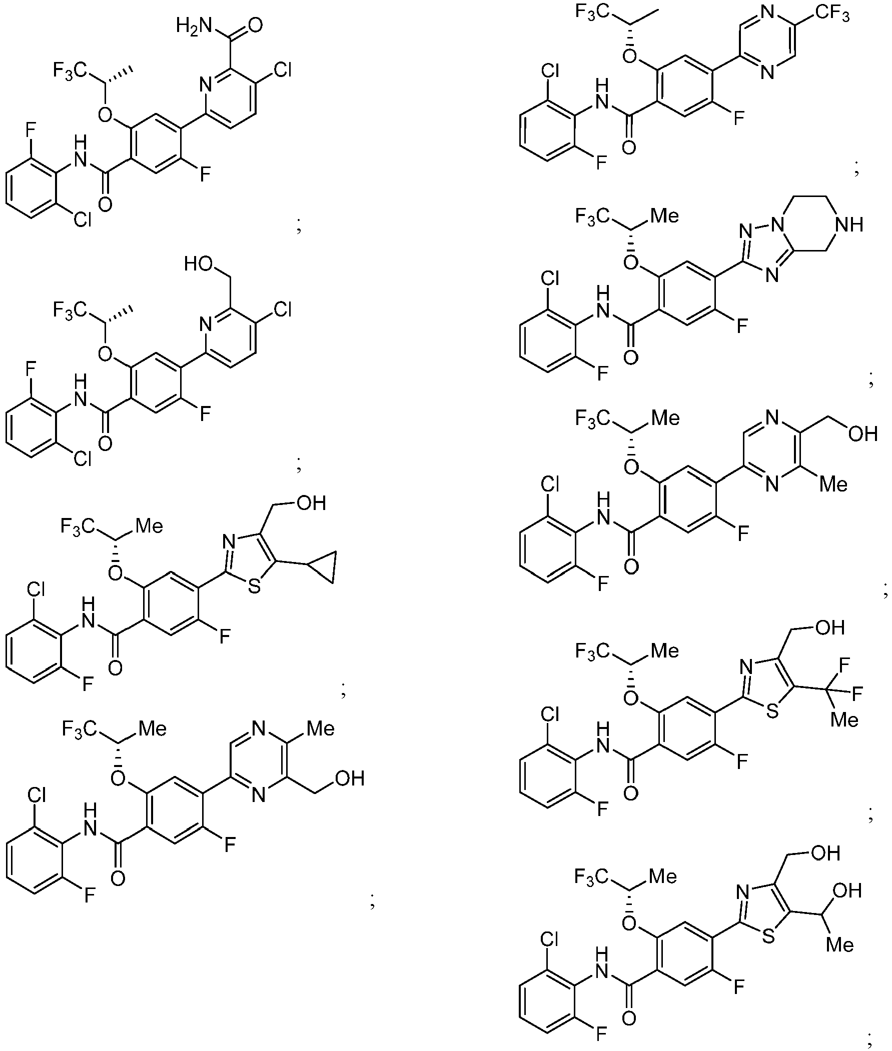 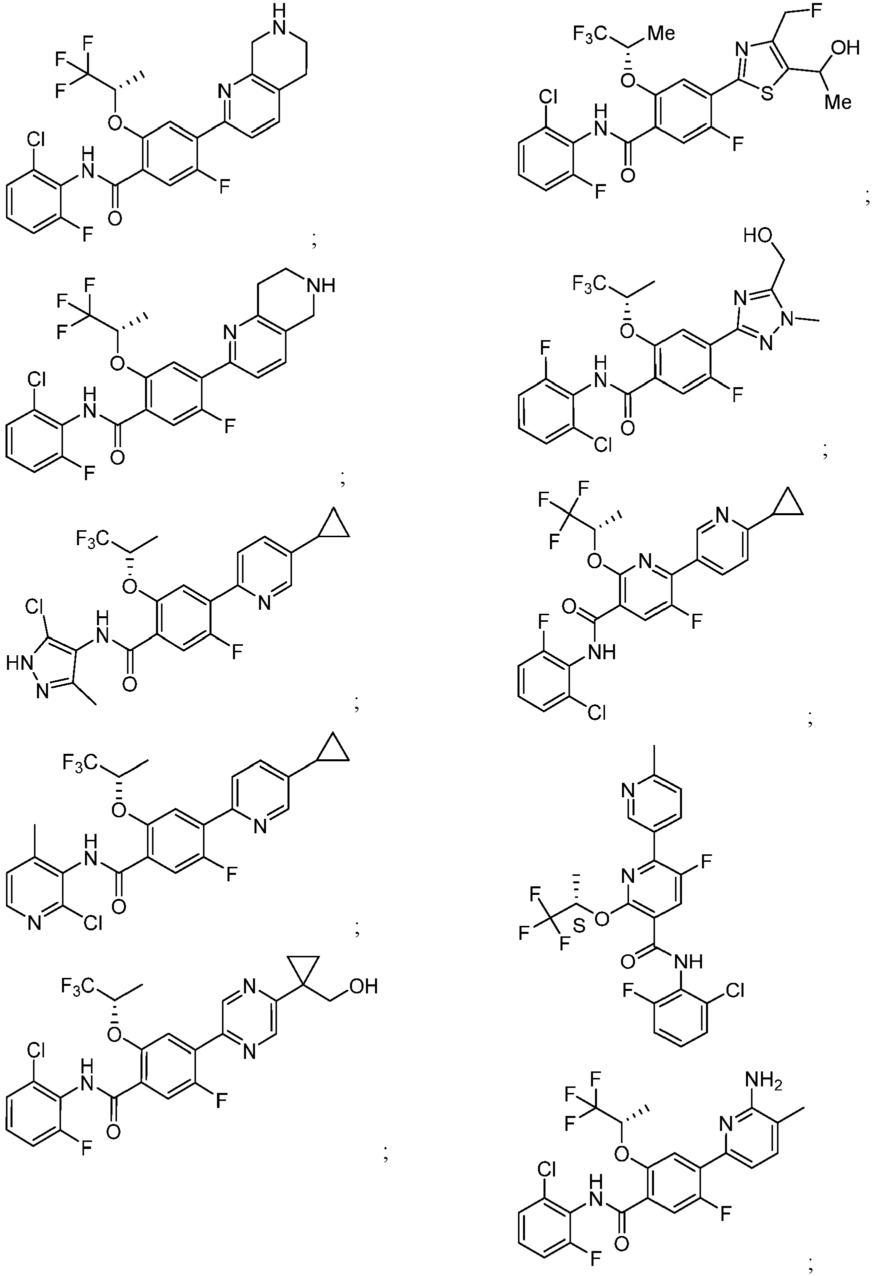 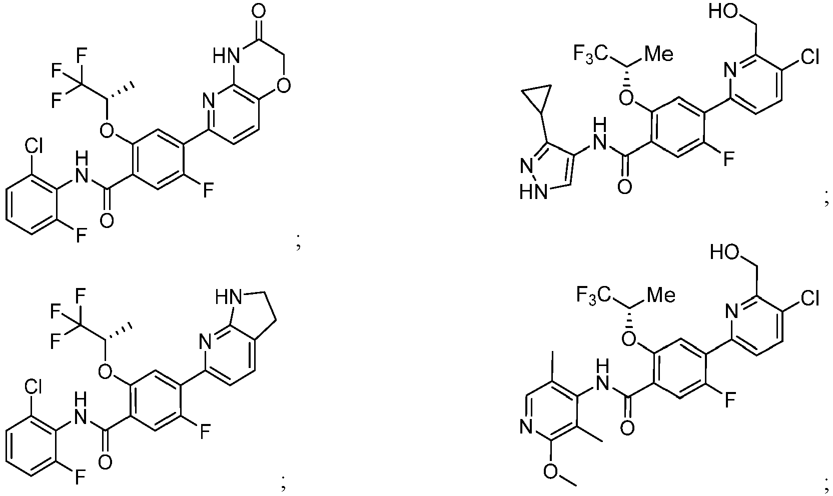 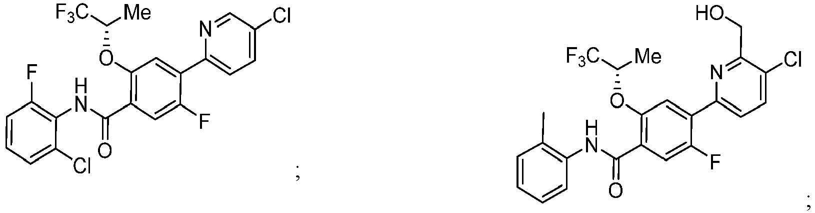 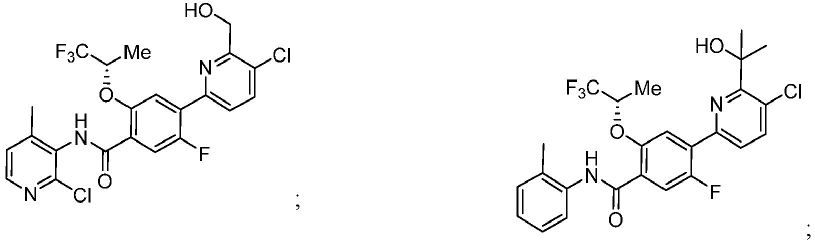 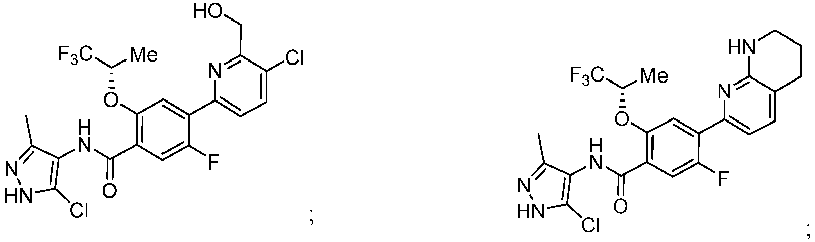 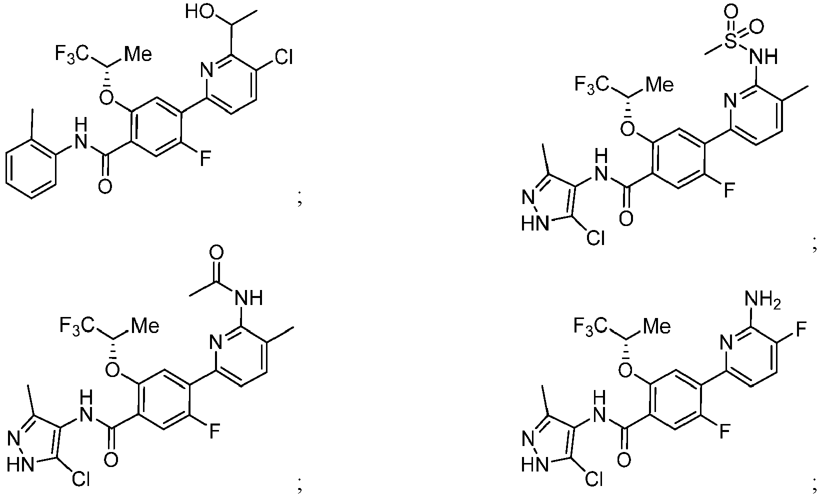 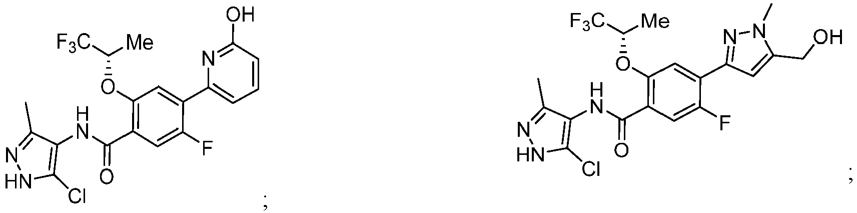 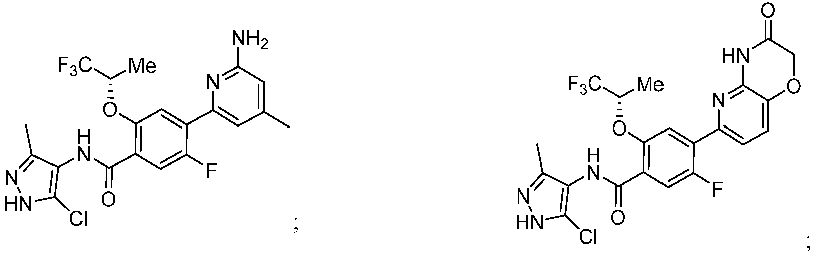 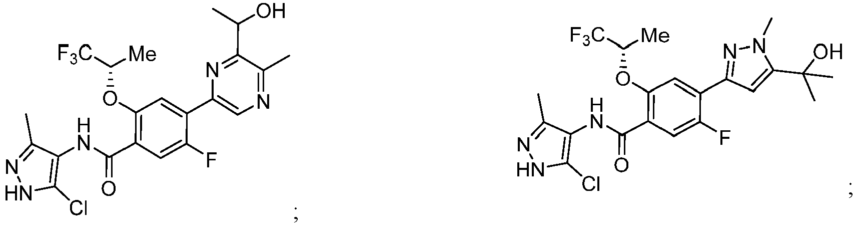 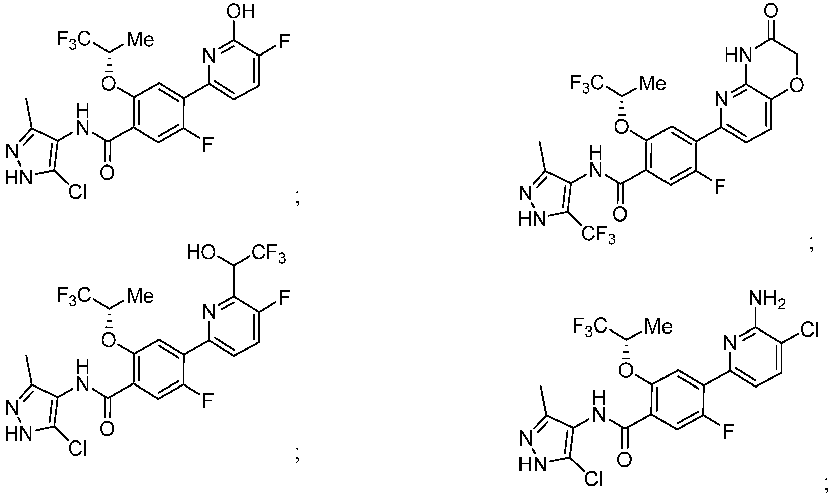 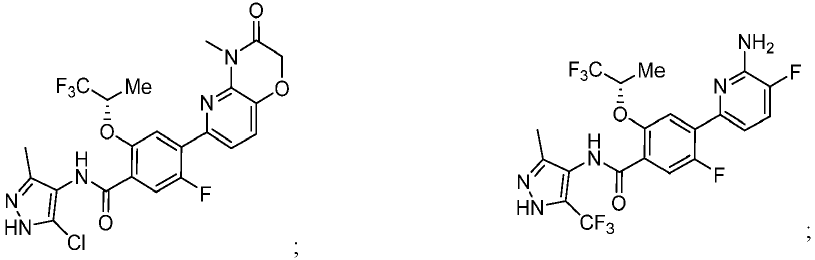 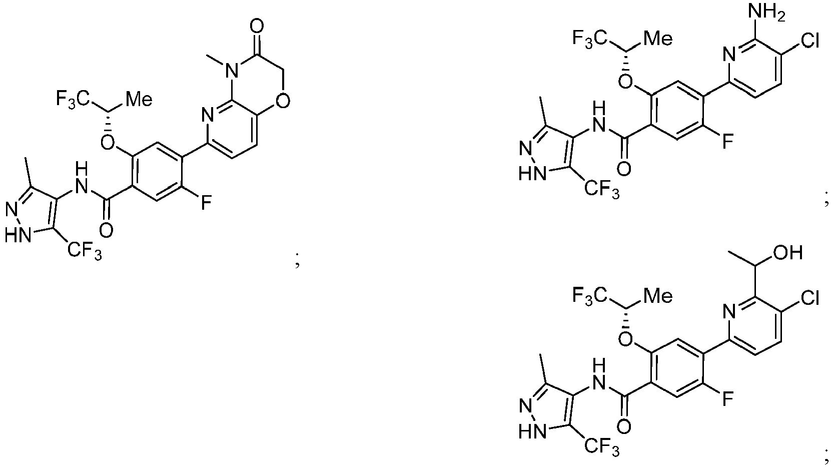 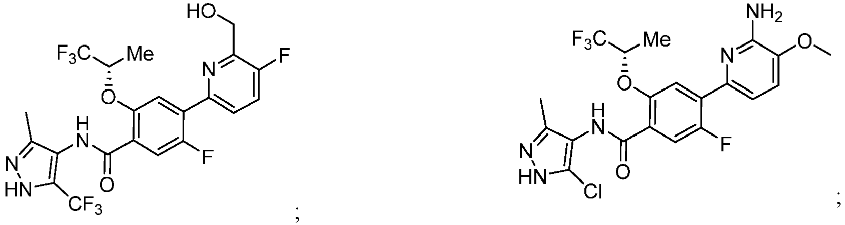 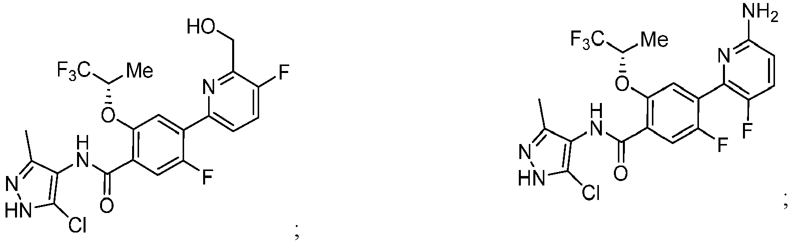 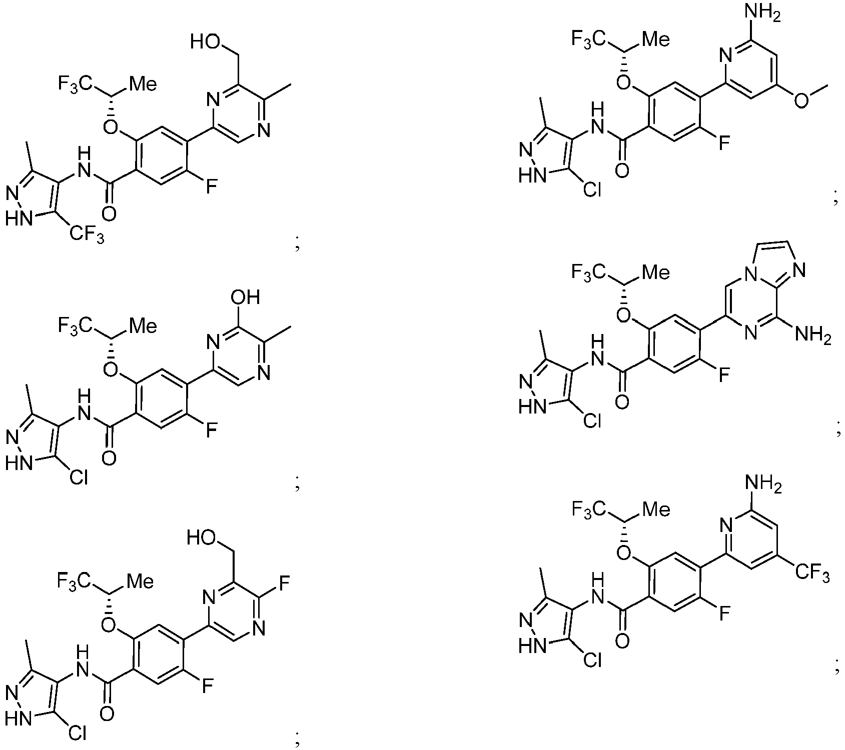 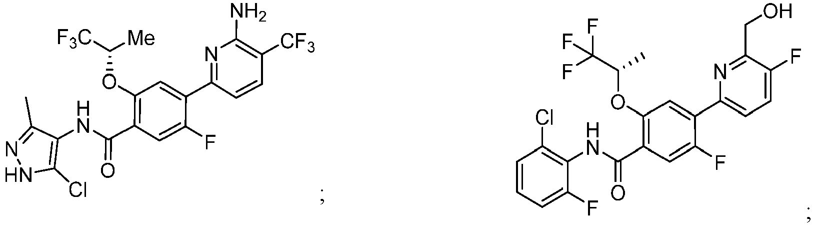 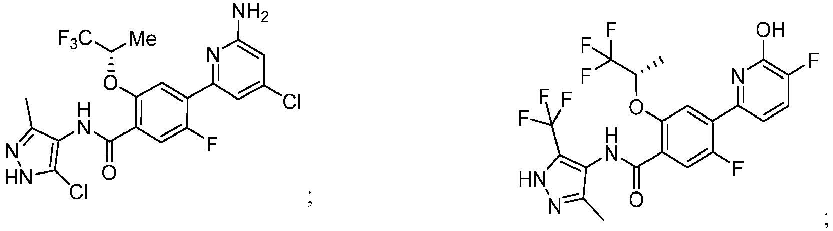 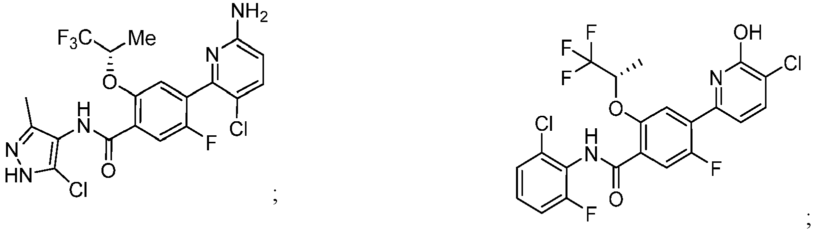 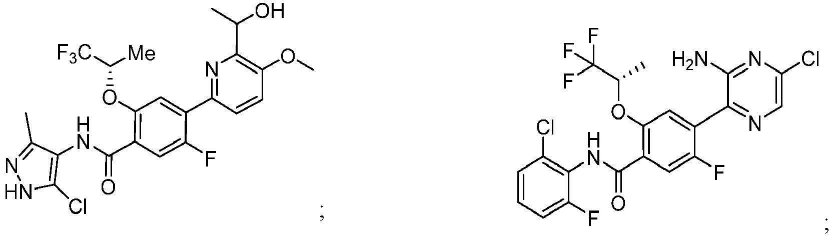 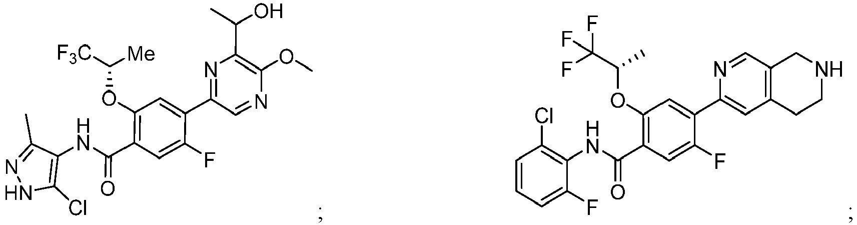 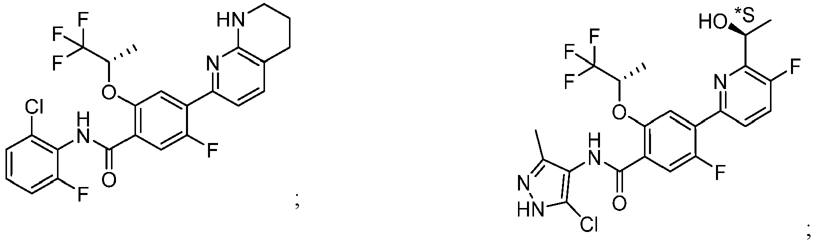 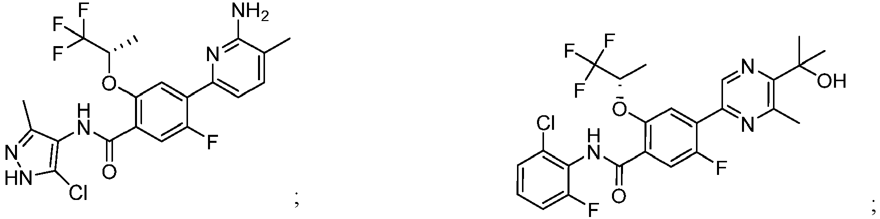 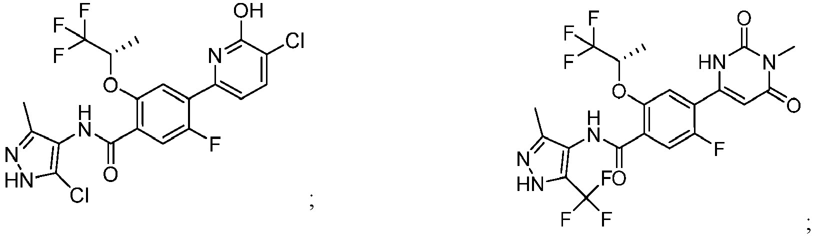 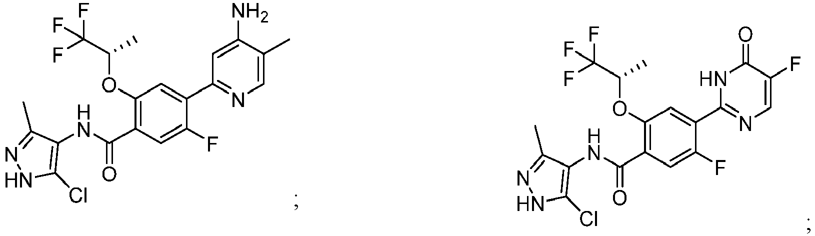 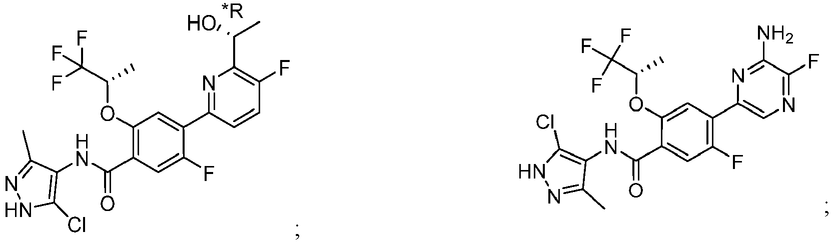 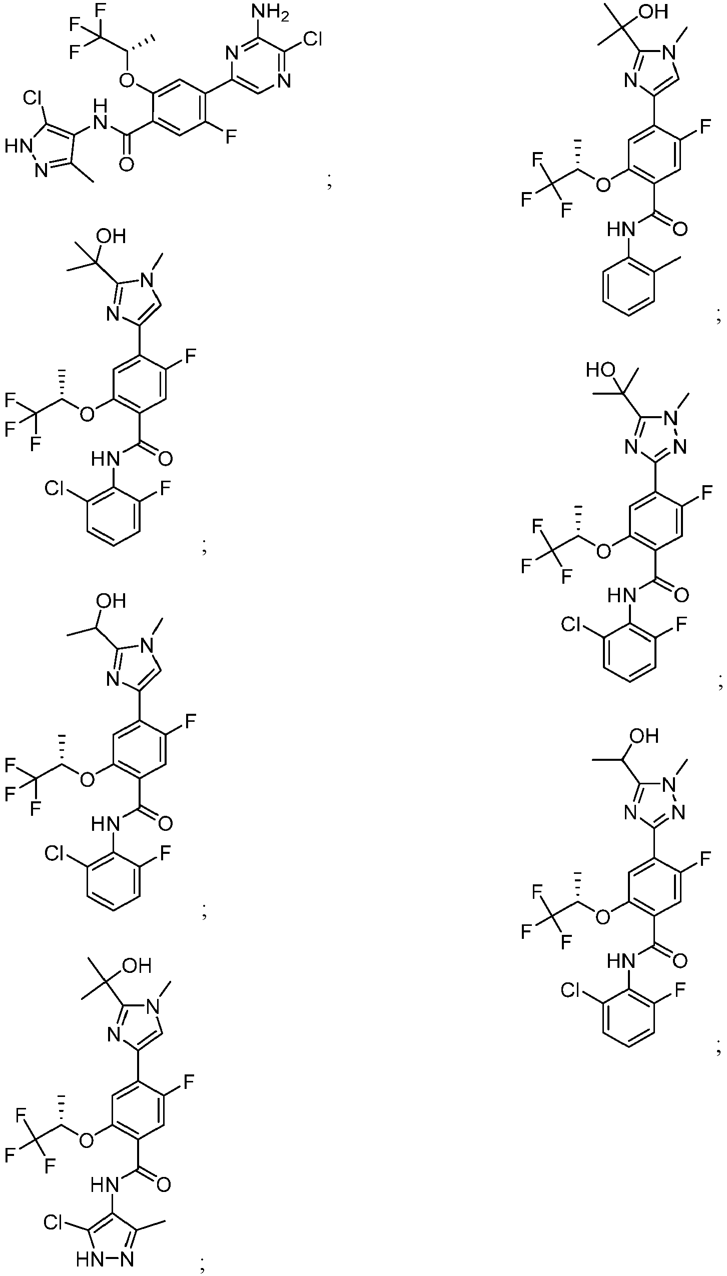 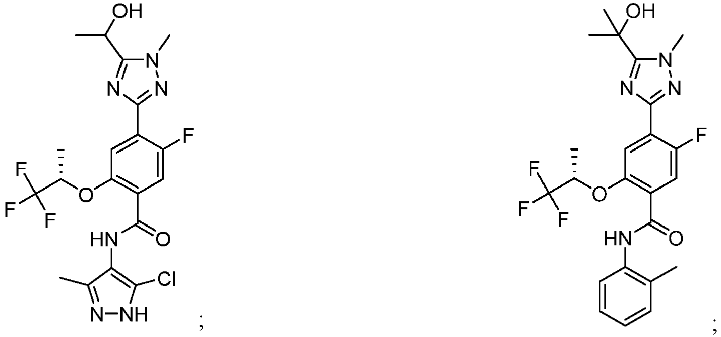 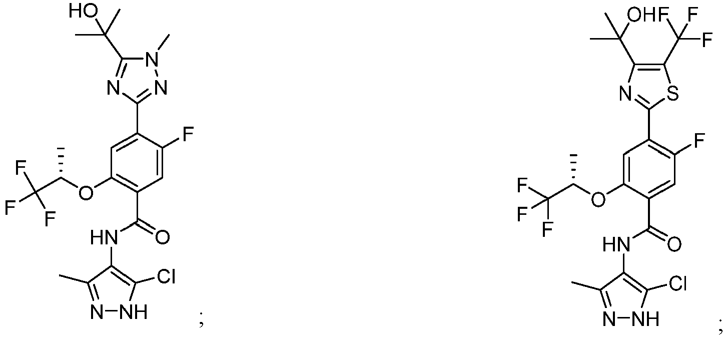 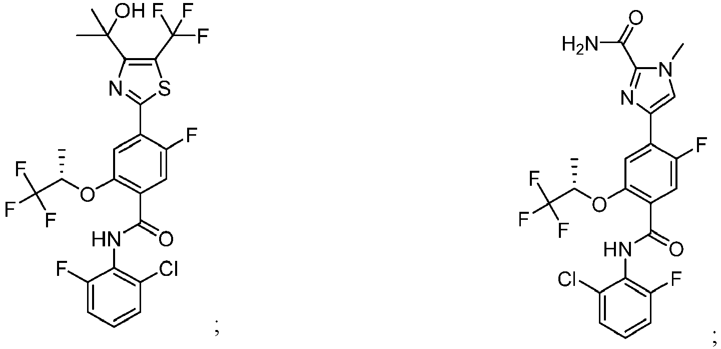 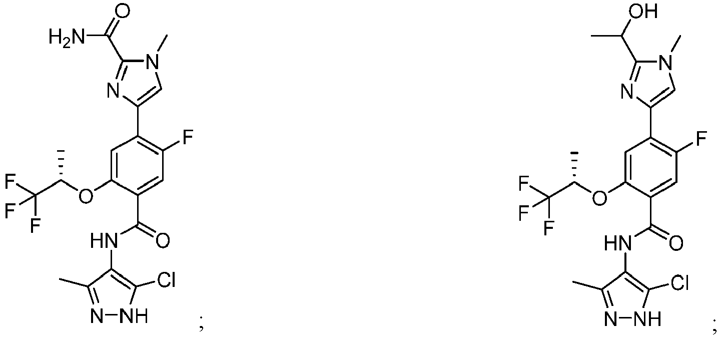 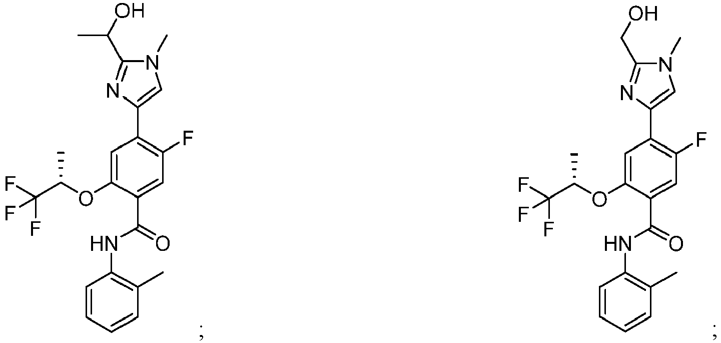 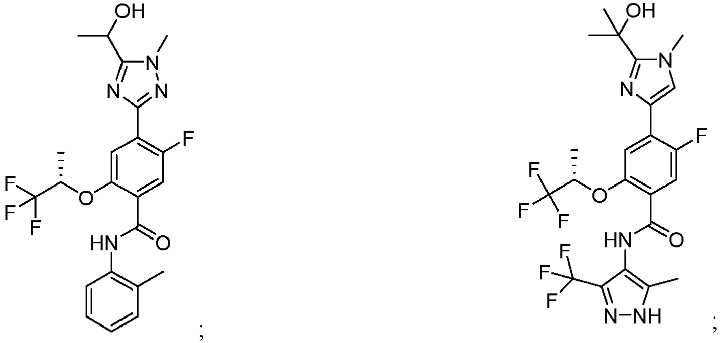 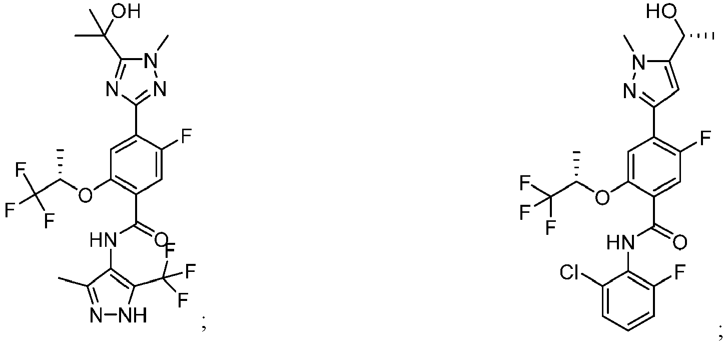 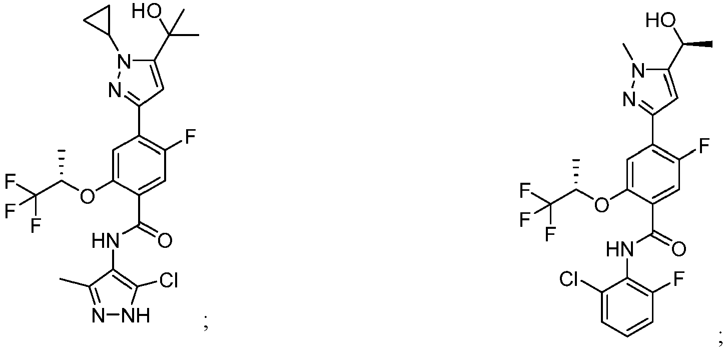 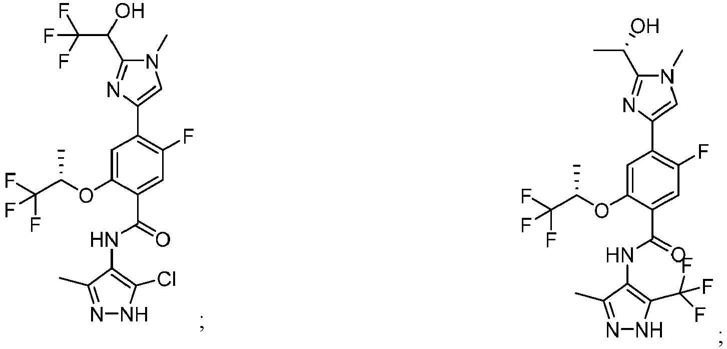 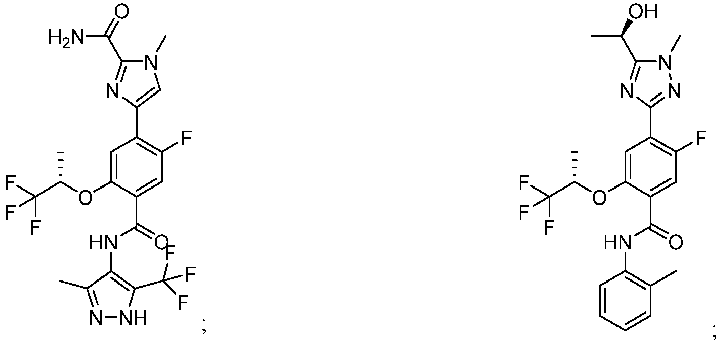 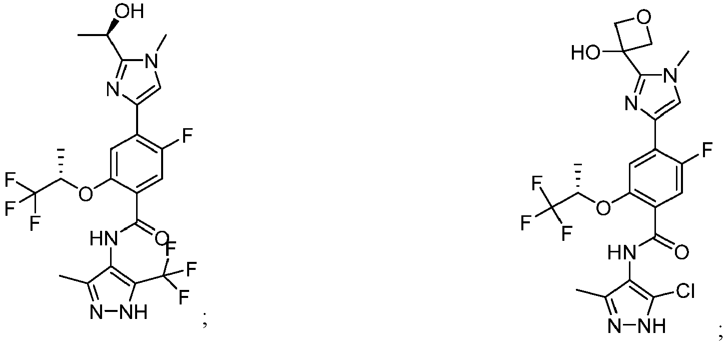 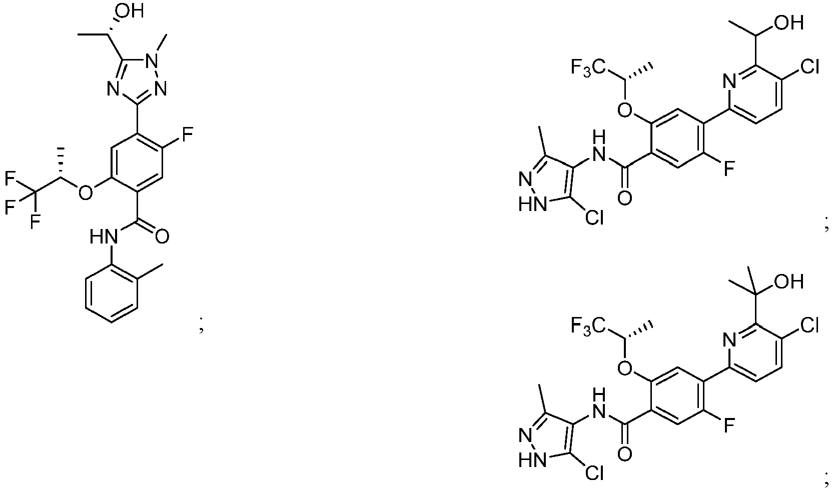 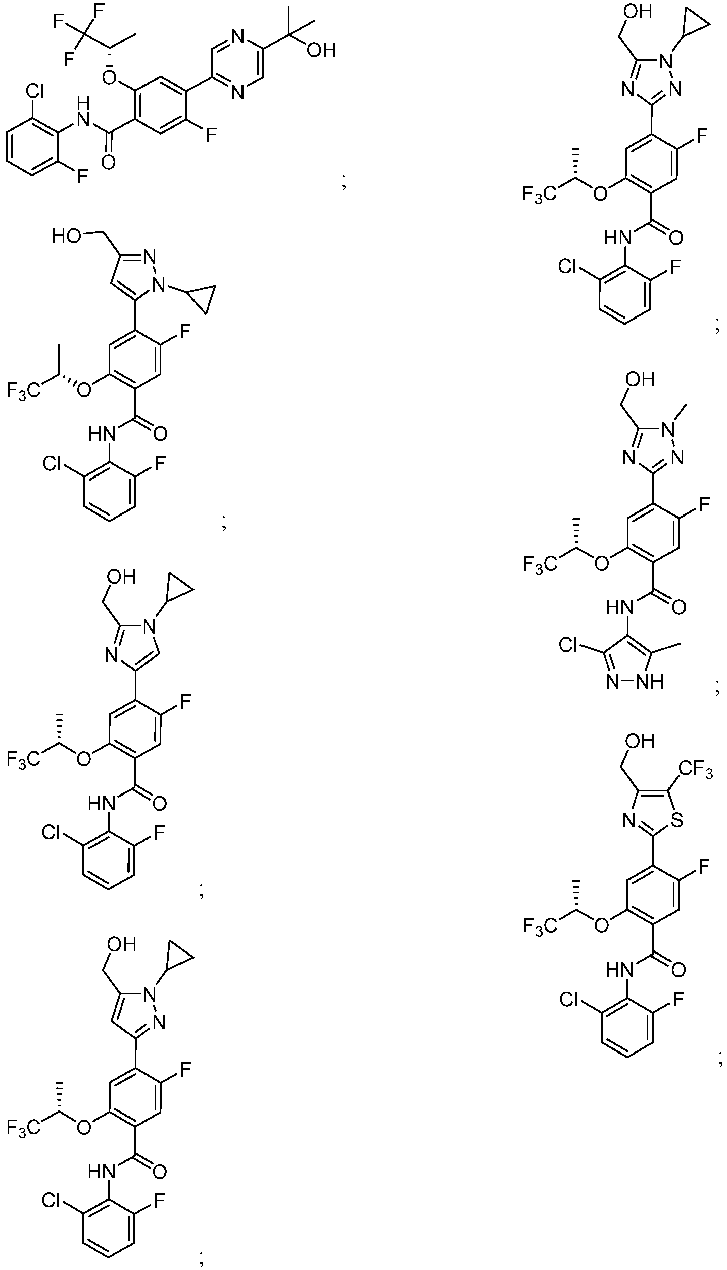 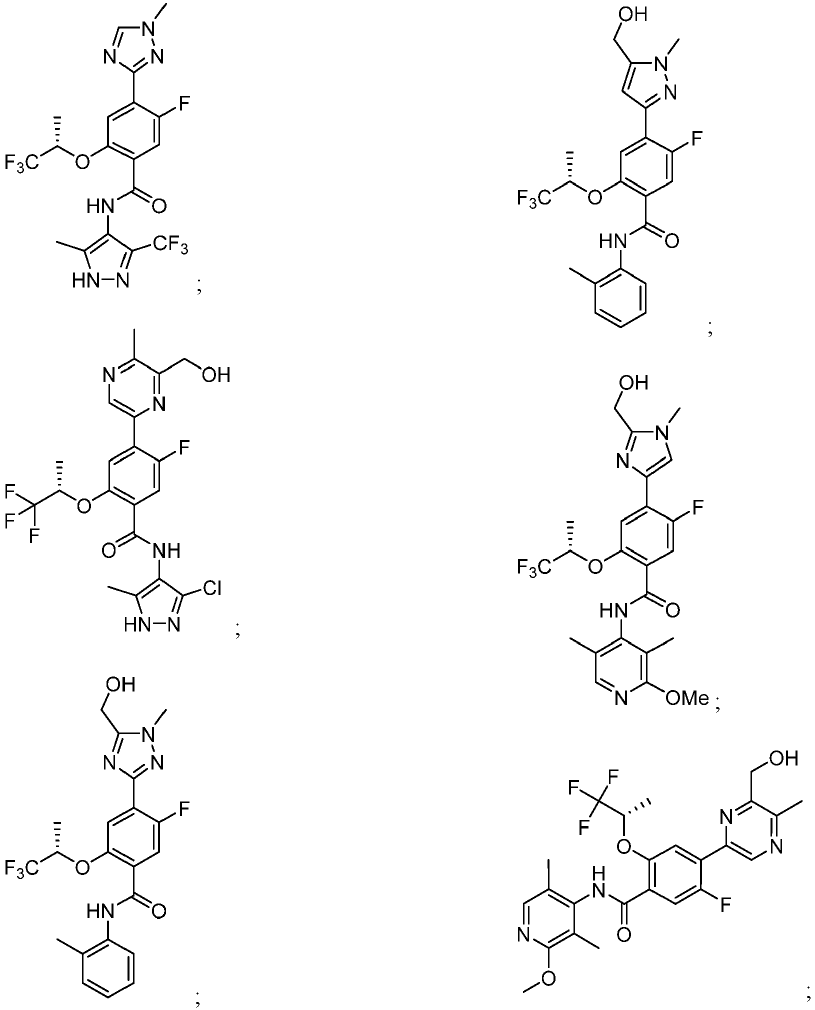 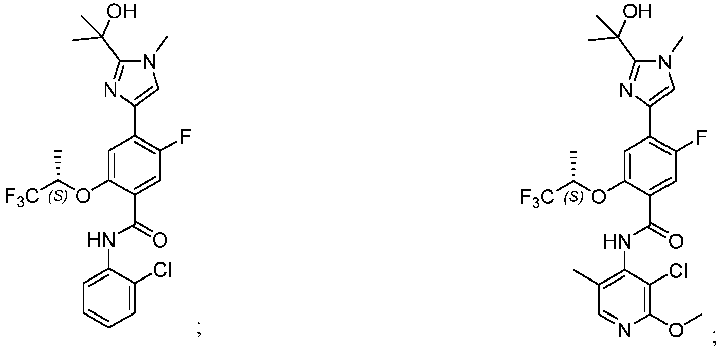 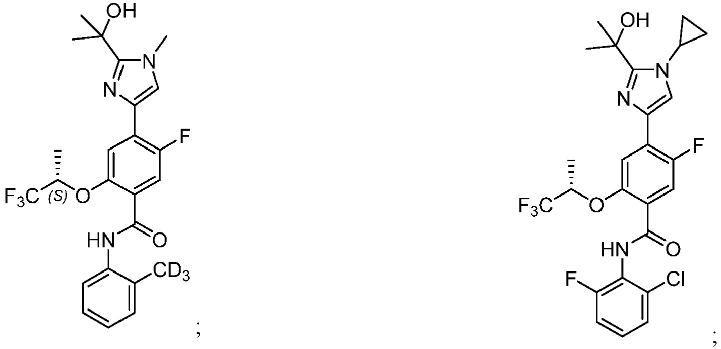 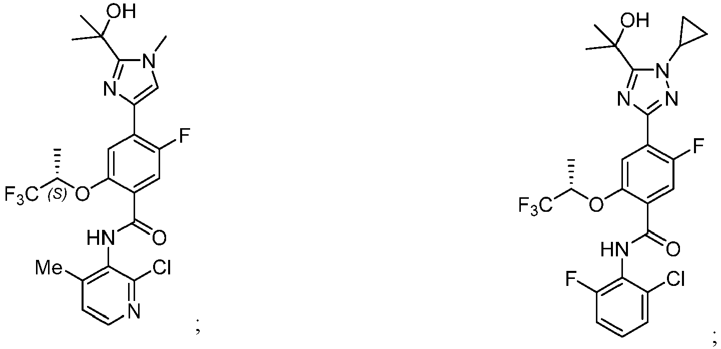 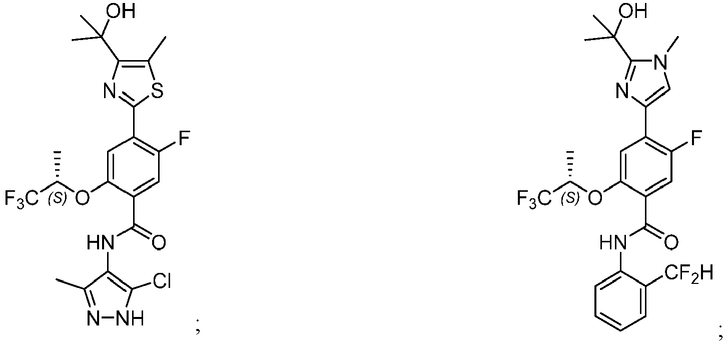 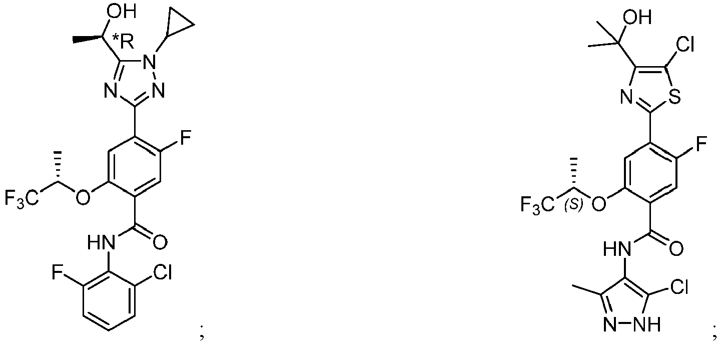 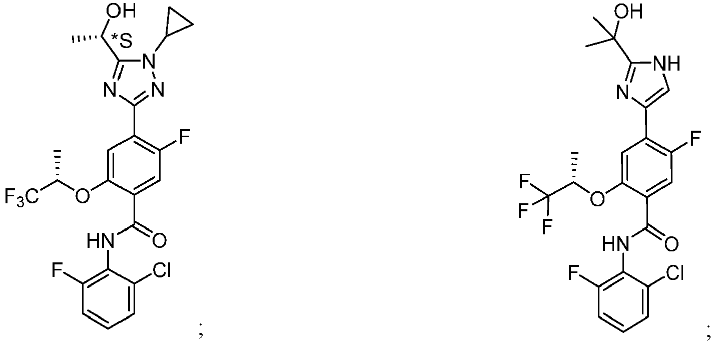 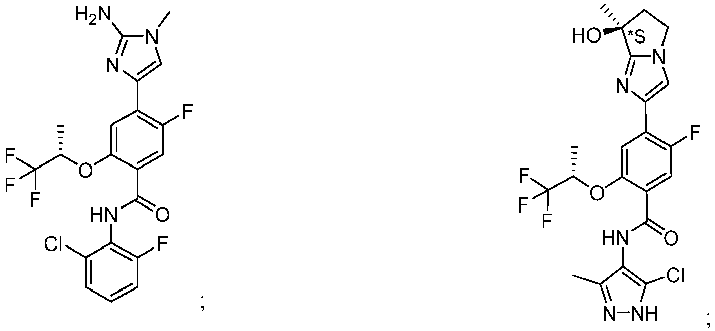 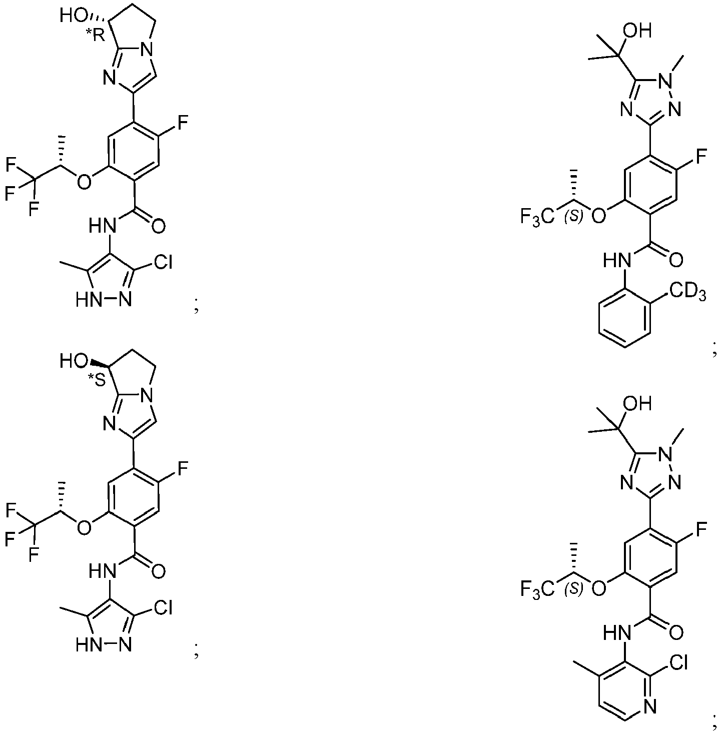 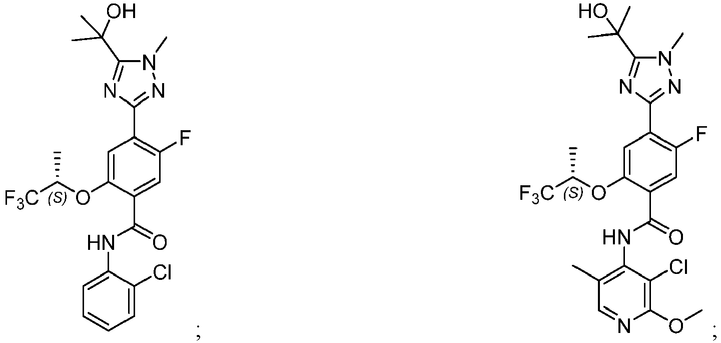 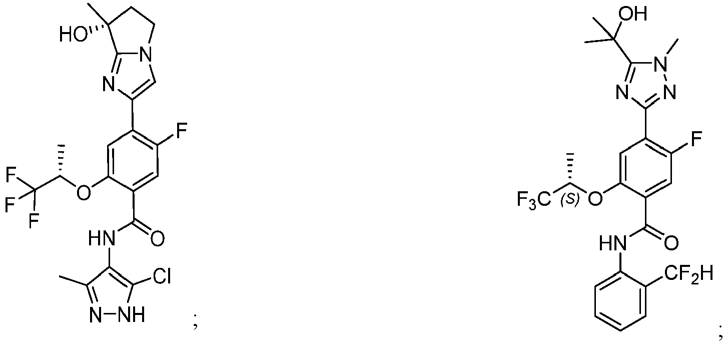 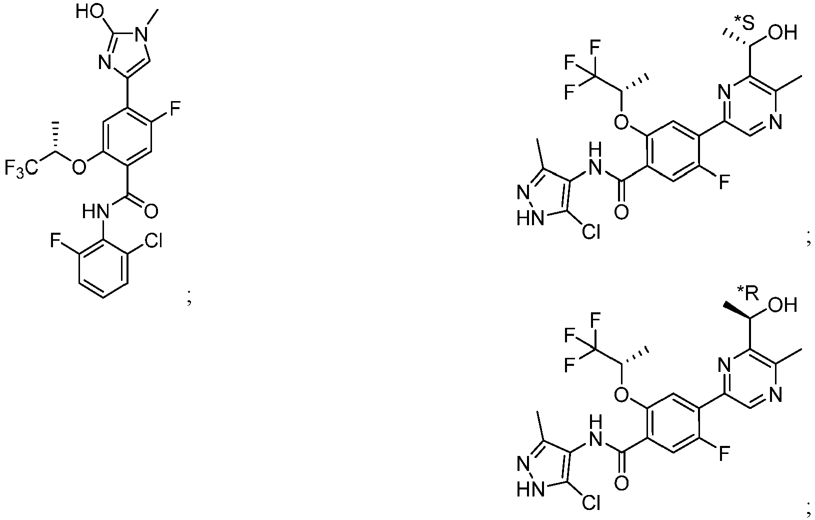 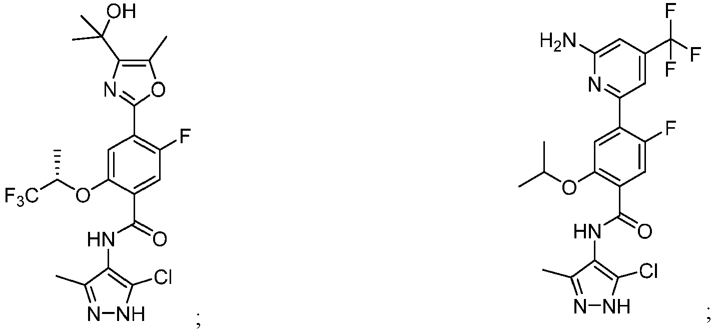 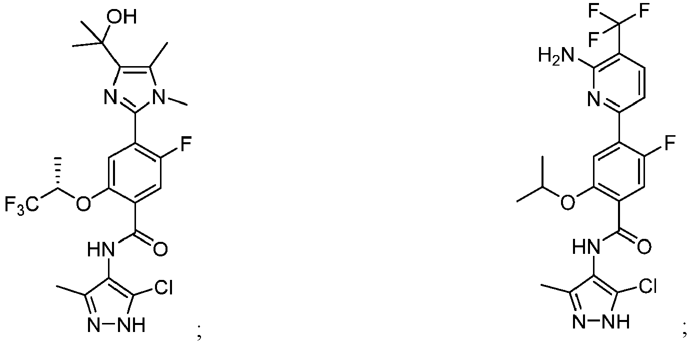 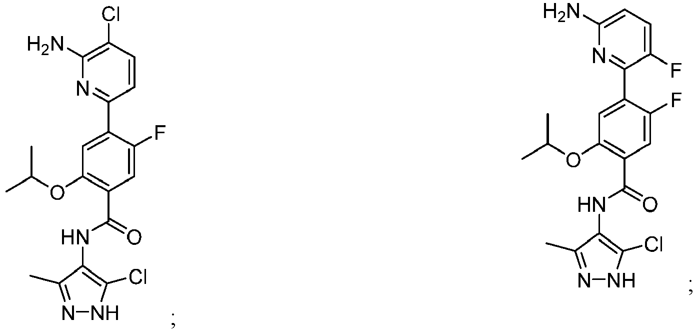 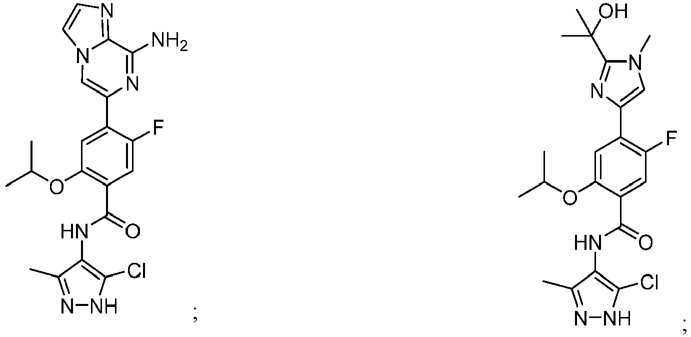 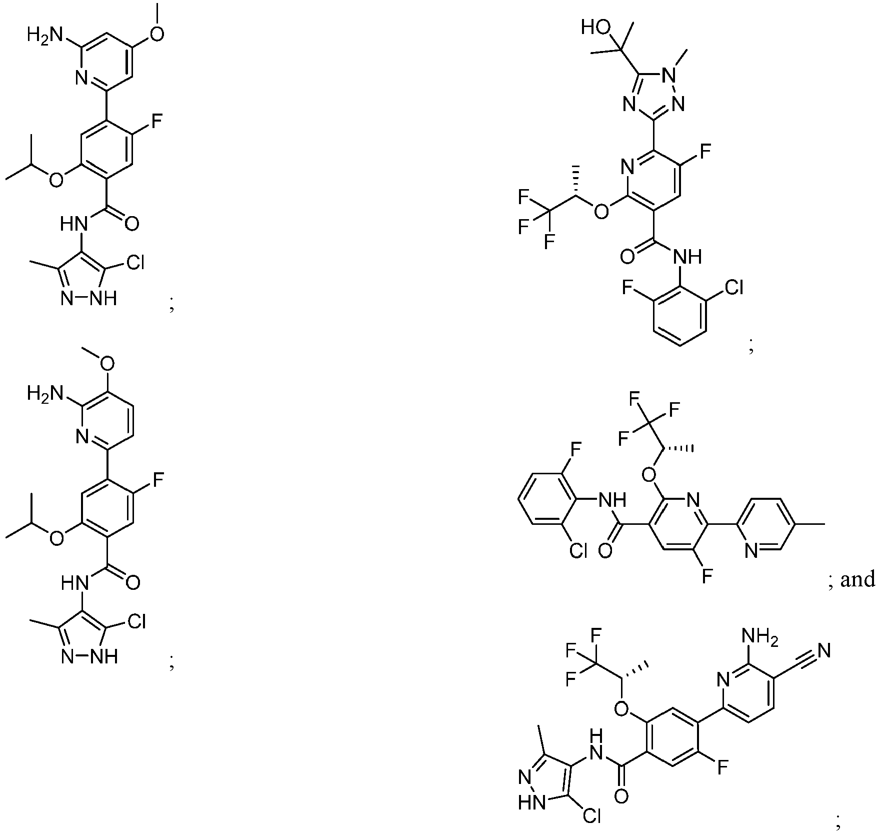 or a pharmaceutically acceptable salt, solvate, stereoisomer, isotopic variant, or N-oxide thereof.

6. The compound of claim 1 wherein:
(a) Q is CH or N;
R¹ is 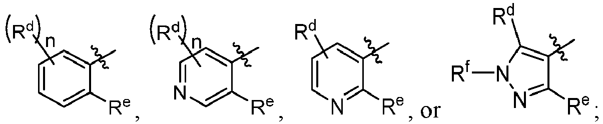 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is imidazolyl; wherein said imidazolyl is optionally substituted with up to three substituents independently selected from the group comprising 3-hydroxyoxetanyl, -NH₂, -OH, -CN, or R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(b) Q is CH or N;
R¹ is 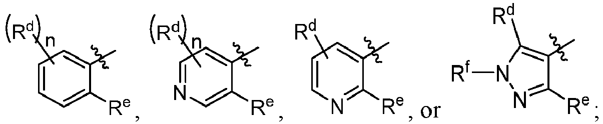 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is triazolyl; wherein said triazolyl is optionally substituted with one or two groups independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together together to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(c) Q is CH or N;
R¹ is 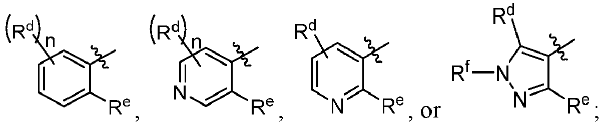 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is pyrazinyl; wherein said pyrazinyl is optionally substituted with up to three substituents independently selected from the group comprising -NHSO₂CH₃, -NHC(O)CH₃, -OCH₃, - OH, -NH2, -CO₂H, -Cl, -F, or R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(d) Q is CH or N;
R¹ is 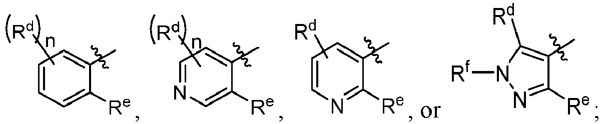 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is pyrazinonyl; wherein said pyrazinonyl is optionally substituted with one or two groups independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(e) Q is CH or N;
R¹ is 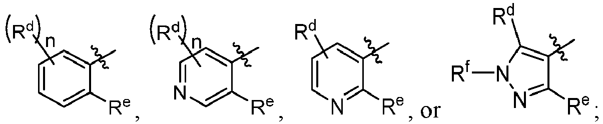 wherein
R^{d} is selected from the group consisting of: -H, -Cl, -F, and -C(₁₋₃)alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C(₁₋₃)alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is thiazolyl; wherein said thiazolyl is optionally substituted with one or two substituents independently selected from the group comprising R^{a};
R^{a} is -C(O)NH₂, cyclopropyl, or -C(₁₋₃)alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(f) Q is CH or N;
R¹ is 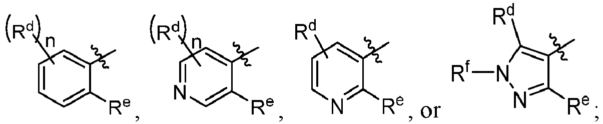 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 5,6,7,8-tetrahydro-[1,2,4]triazolo[4,3-*a*]pyrazinyl; wherein said 5,6,7,8-tetrahydro-[1,2,4]triazolo[4,3-*a*]pyrazinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(g) Q is CH or N;
R¹ is 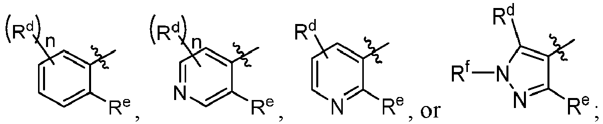 wherein
R^{d} is selected from the group consisting of: -H, -Cl, -F, and -C(₁₋₃)alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is pyridinyl; wherein said pyridinyl is optionally substituted with one or two substituents independently selected from the group comprising R^{a}, -Cl, -F, -CN, -NHC(O)CH₃, - NHSO₂CH₃, -CO₂H, -OCH₃, -OH, or -NH₂;
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(h) Q is CH or N;
R¹ is 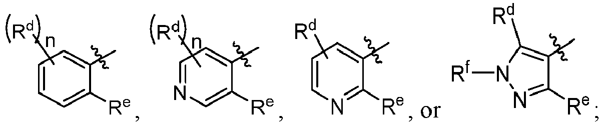 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is pyridazinyl; wherein said pyridazinyl is optionally substituted with one or two groups independently selected from the group comprising R^{a}, -Cl, or -CO₂H;
R^{a} is -C(O)NH₂, cyclopropyl, or -C(₁₋₃)alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(i) Q is CH or N;
R¹ is 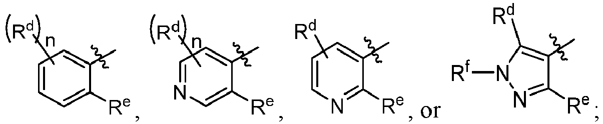 wherein
R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C(i-₃₎alkyl is optionally substituted with up to six fluorine atoms;
R^{e} is selected from the group consisting of: -Cl, -F, and -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 4,5,6,7-tetrahydrothiazolo[5,4-c]pyridinyl; wherein said 4,5,6,7-tetrahydrothiazolo[5,4-c]pyridinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(j) Q is CH or N;
R¹ is 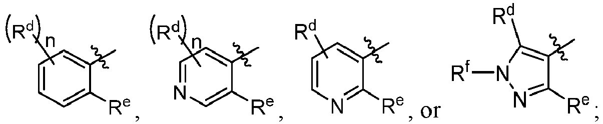 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C(₁₋₃)alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is pyrazolyl; wherein said pyrazolyl is optionally substituted with one or two substituents independently selected from the group comprising R^{a};
R^{a} is -C(O)NH₂, cyclopropyl, or -C(₁₋₃)alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(k) Q is CH or N;
R¹ is 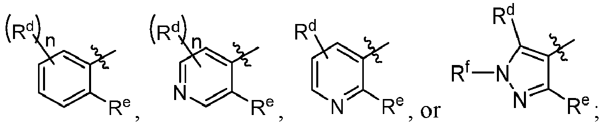 wherein
R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C(i-₃₎alkyl is optionally substituted with up to six fluorine atoms;
R^{e} is selected from the group consisting of: -Cl, -F, and -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is oxazolyl; wherein said oxazolyl is optionally substituted with one or two substituents independently selected from the group comprising R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(l) Q is CH or N;
R¹ is 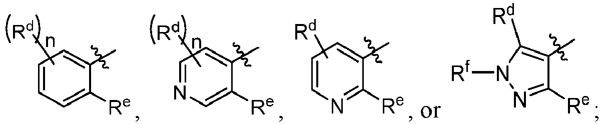 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 1,3,4-oxadiazolyl; wherein said 1,3,4-oxadiazolyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(m) Q is CH or N;
R¹ is 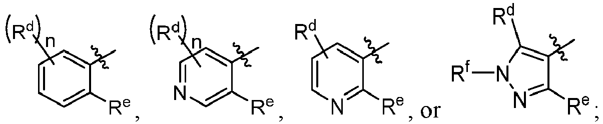 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 5,6,7,8-tetrahydroimidazo[1,5-*a*]pyridinyl; wherein said 5,6,7,8-tetrahydroimidazo[1,5-*a*]pyridinyl is optionally substituted with R^{a};
R^{a} is -C(O)NH₂, cyclopropyl, or -C(₁₋₃)alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(n) Q is CH or N;
R¹ is 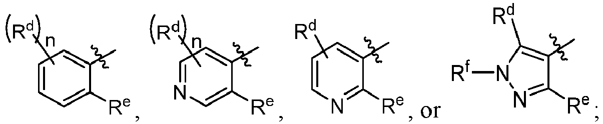 wherein
R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C(i-₃₎alkyl is optionally substituted with up to six fluorine atoms;
R^{e} is selected from the group consisting of: -Cl, -F, and -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 1,2,4-thiadiazolyl; wherein said 1,2,4-thiadiazolyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof;
or
(o) Q is CH or N;
R¹ is 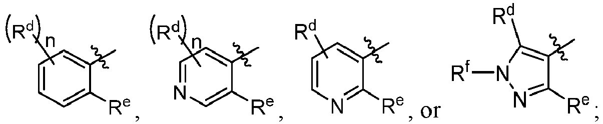 wherein
R^{d} is selected from the group consisting of: -H, -Cl, -F, -C₍₁₋₃₎alkyl; wherein said -C₍₁₋₃₎alkyl is optionally substituted with: -OH, -OCH₃, -SCH₃, or -OCF₃; and wherein said -C(i-₃₎alkyl is optionally substituted with up to six fluorine atoms;
R^{e} is selected from the group consisting of: -Cl, -F, and -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 5,6,7,8-tetrahydro-1,7-naphthyridinyl; wherein said 5,6,7,8-tetrahydro-1,7-naphthyridinyl is optionally substituted with R^{a}; or
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said -C₍₁₋₆₎alkyl is optionally substituted with up to six fluorine atoms;
or a pharmaceutically acceptable salt, solvate, stereoisomer, isotopic variant, or N-oxide thereof;
or
(p) Q is CH or N;
R¹ is 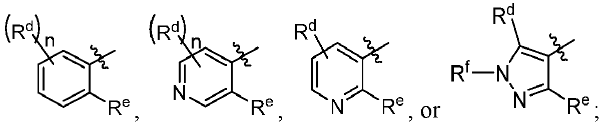 wherein
R^{d} is selected from the group consisting of: -H, -Cl, -F, and -C₍₁₋₃₎alkyl; wherein said -C(i-₃₎alkyl is optionally substituted with: -OH, -OCH₃, -SCH₃, or -OCF₃; and wherein said - C₍₁₋₃₎alkyl is optionally substituted with up to six fluorine atoms;
R^{e} is selected from the group consisting of: -Cl, -F, -C₍₁₋₃₎alkyl, -CD₃, and cyclopropyl; wherein said -C₍₁₋₃₎alkyl is optionally substituted with: -OH, -OCH₃, -SCH₃, or -OCF₃; and wherein said -C₍₁₋₃₎alkyl is optionally substituted with up to six fluorine atoms;
R^{f} is selected from the group consisting of: -H, and -C₍₁₋₃₎alkyl; wherein said -C₍₁₋₃₎alkyl is optionally substituted with a member selected from the group consisting of: -OH, - OCH₃, -SCH₃, and -OCF₃; and wherein said -C₍₁₋₃₎alkyl is optionally substituted with up to six fluorine atoms; and
n is 1, or 2;
R² is 5,6,7,8-tetrahydro-1,6-naphthyridinyl; wherein said 5,6,7,8-tetrahydro-1,6-naphthyridinyl is optionally substituted with R^{a};
R^{a} is -C(O)NH₂, cyclopropyl, or -C₍₁₋₃₎alkyl; wherein said -C₍₁₋₃₎alkyl is optionally substituted with up to three fluorine atoms, or the -C₍₁₋₃₎alkyl is optionally substituted with -OH and additionally optionally substituted with up to three fluorine atoms; and wherein said cyclopropyl is optionally substituted with -CH₂OH;
R³ is -CHR^{b}R^{c}; wherein
R^{b} is -C₍₃₋₆₎cycloalkyl, or -C₍₁₋₆₎alkyl; wherein said -C₍₁₋₆₎alkyl is optionally substituted with -Cl, -OH, or -OCH₃; and wherein said -C₍₁₋₆₎alkyl is optionally substituted with up to six fluorine atoms;
R^{c} is -CH₃ or -CHF₂; or R^{b} and R^{c} may be taken together with the carbon atom to which they are bound to form -C₍₃₋₆₎cycloalkyl; wherein said -C₍₃₋₆₎cycloalkyl is optionally substituted with up to four substituents independently selected from the group consisting of: -Cl, -F, -OH, and -C₍₁₋₆₎alkyl; wherein said C₍₁₋₆₎alkyl is optionally substituted with up to six fluorine atoms;
or a pharmaceutically acceptable salt, solvate, stereoisomer, isotopic variant, or N-oxide thereof.

7. The compound of claim 4 selected from the group consisting of:
(a) 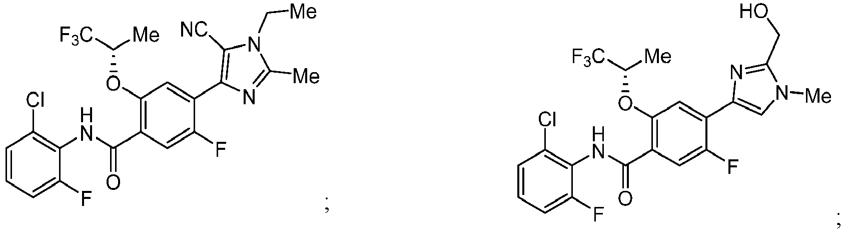 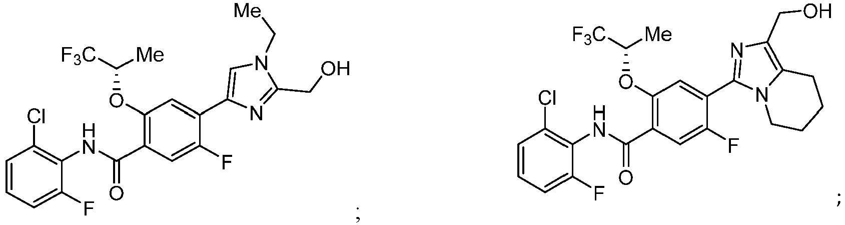 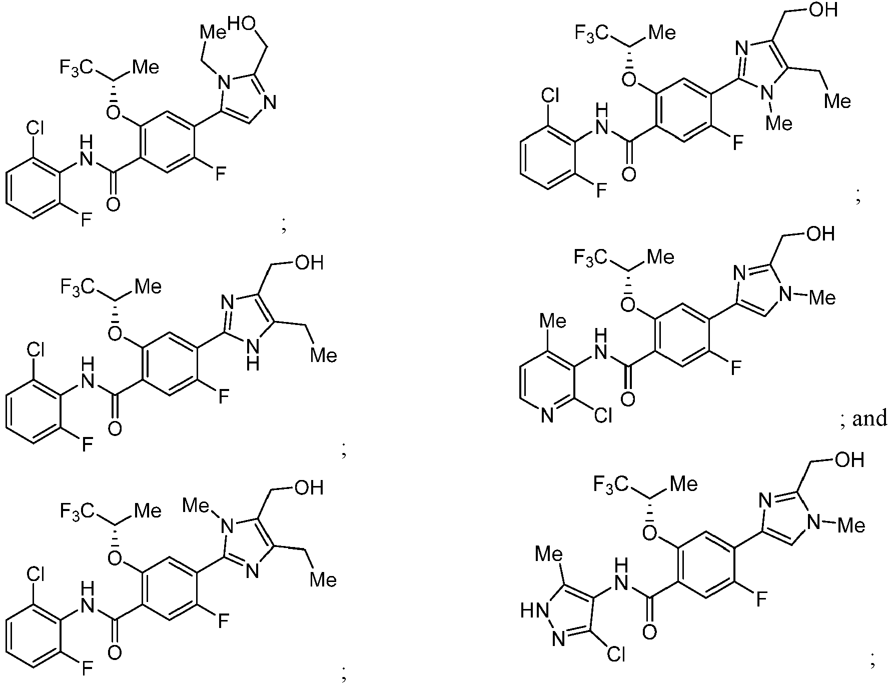 or a pharmaceutically acceptable salt, solvate, stereoisomer, isotopic variant, or N-oxide thereof;
or
(b) 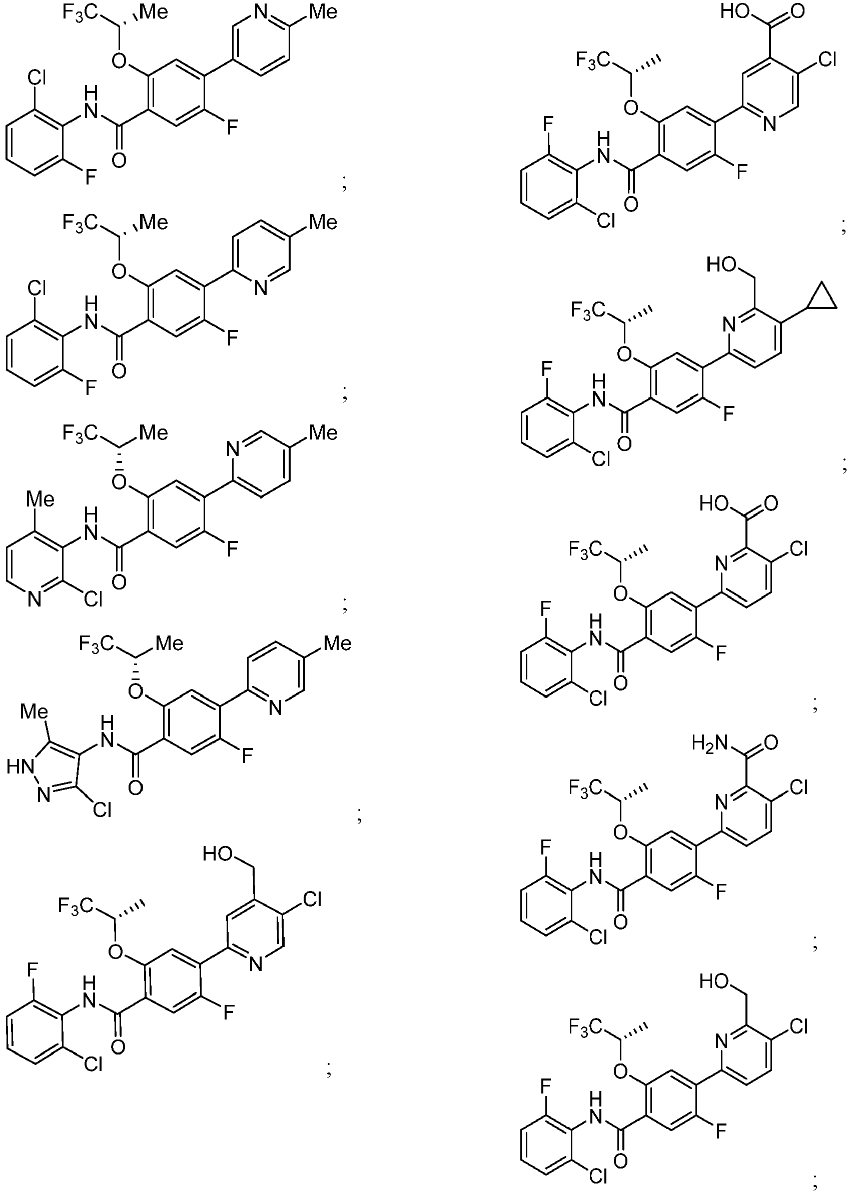 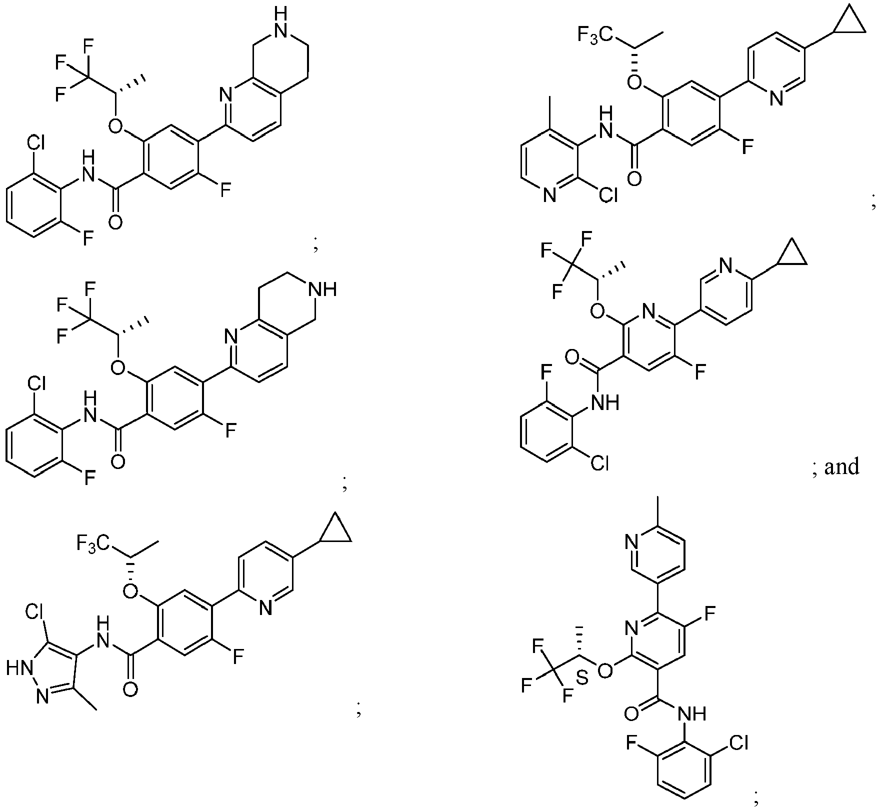 or a pharmaceutically acceptable salt, solvate, stereoisomer, isotopic variant, or N-oxide thereof;
or
(c) 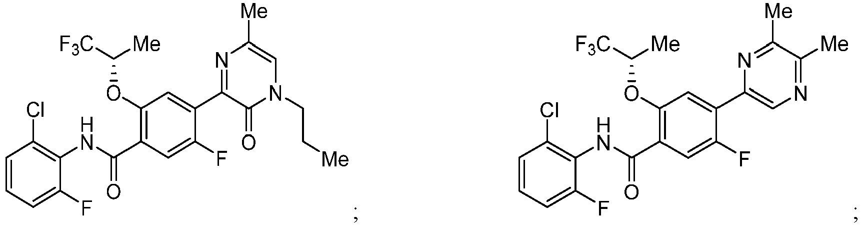 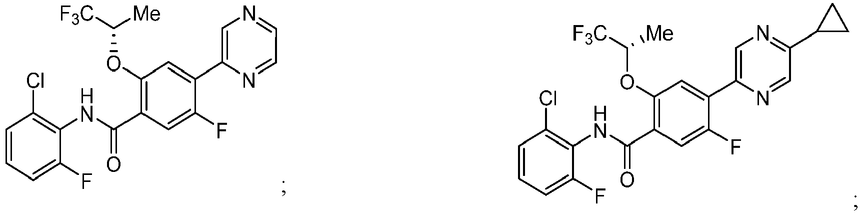 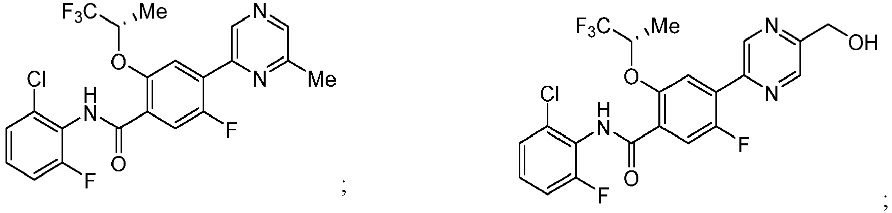 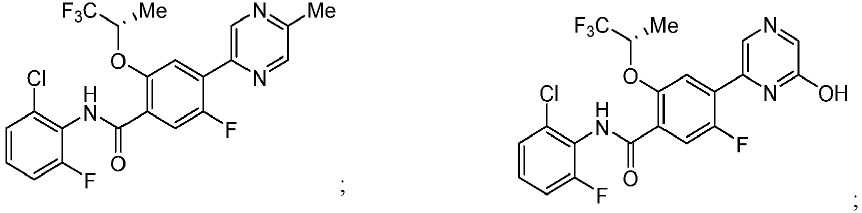 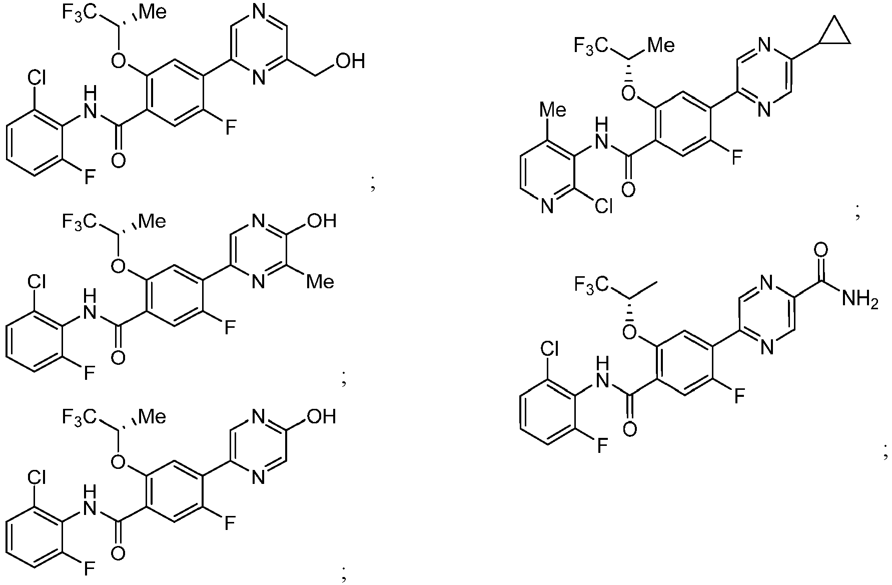 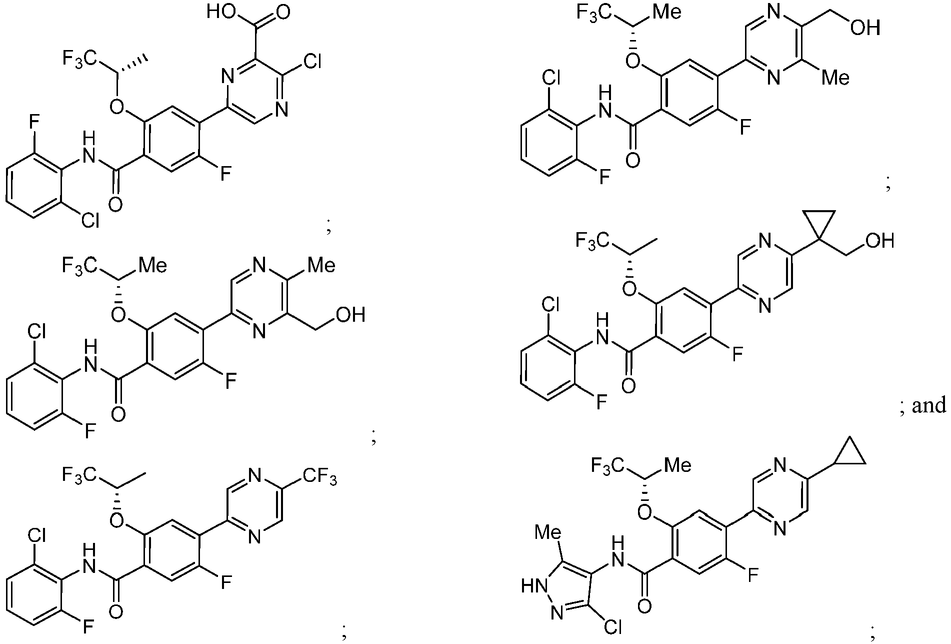 or a pharmaceutically acceptable salt, solvate, stereoisomer, isotopic variant, or N-oxide thereof;
or
(d) 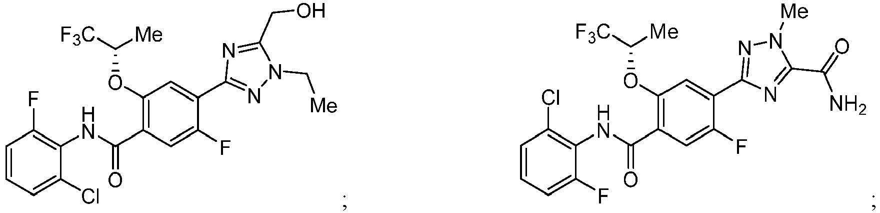 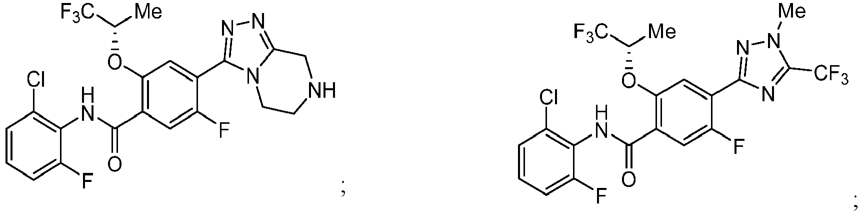 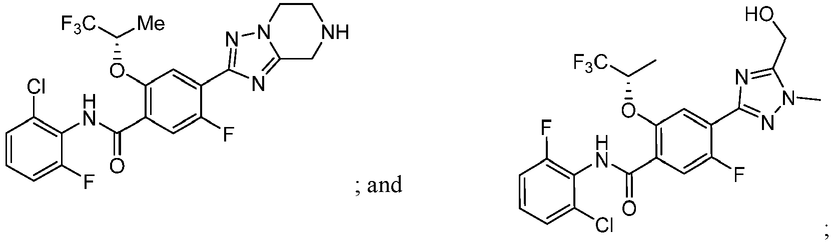 or a pharmaceutically acceptable salt, solvate, stereoisomer, isotopic variant, or N-oxide thereof;
or
(e) 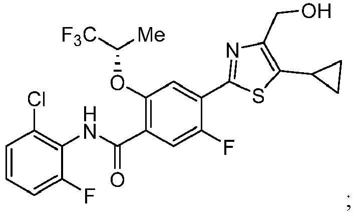 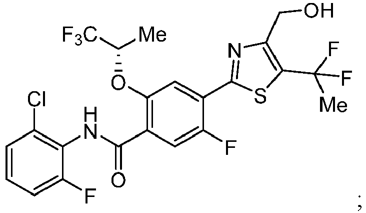 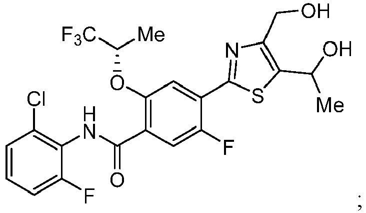 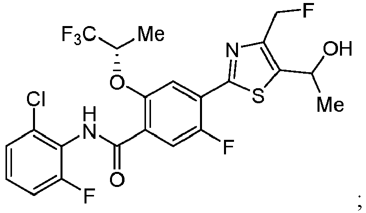 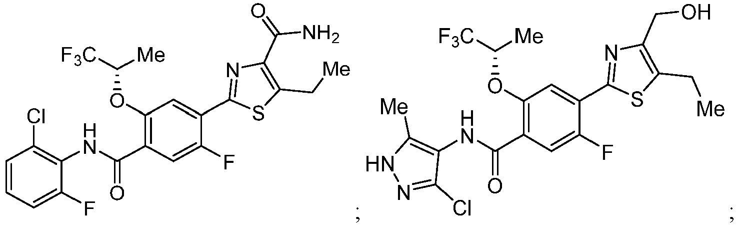 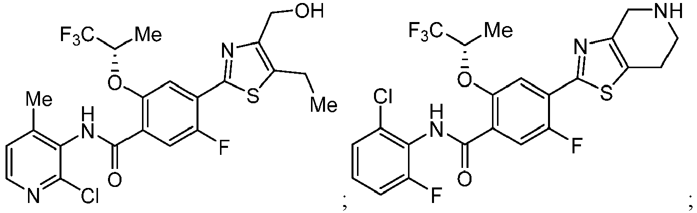 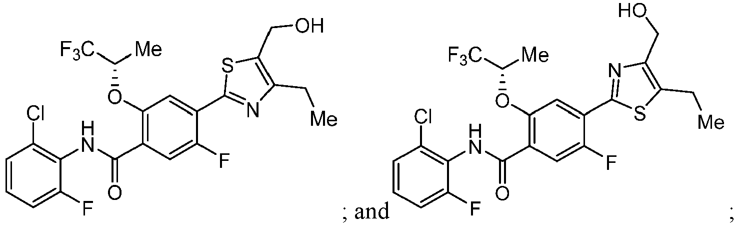 or a pharmaceutically acceptable salt, solvate, stereoisomer, isotopic variant, or N-oxide thereof;
or
(f) 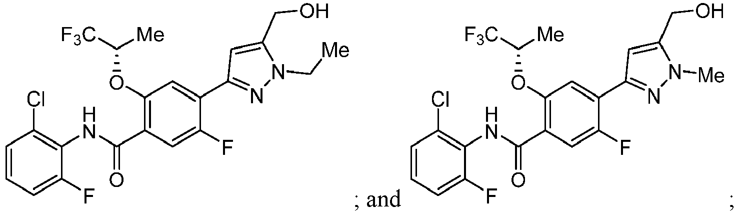 or a pharmaceutically acceptable salt, solvate, stereoisomer, isotopic variant, or N-oxide thereof;
or
(g) 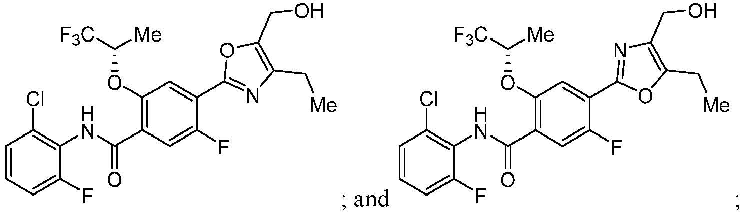 or a pharmaceutically acceptable salt, solvate, stereoisomer, isotopic variant, or N-oxide thereof.

8. The compound of claim 4 which is:
(a) 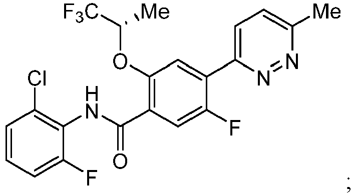 or a pharmaceutically acceptable salt, solvate, stereoisomer, isotopic variant, or N-oxide thereof;
or
(b) 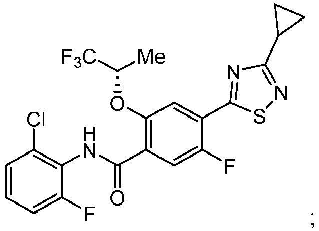 or a pharmaceutically acceptable salt, solvate, stereoisomer, isotopic variant, or N-oxide thereof;
or
(c) 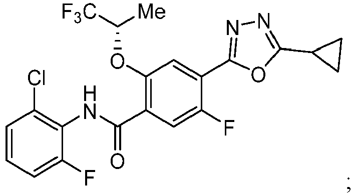 or a pharmaceutically acceptable salt, solvate, stereoisomer, isotopic variant, or N-oxide thereof.

9. The compound of claim 4 selected from the group consisting of:
(a) 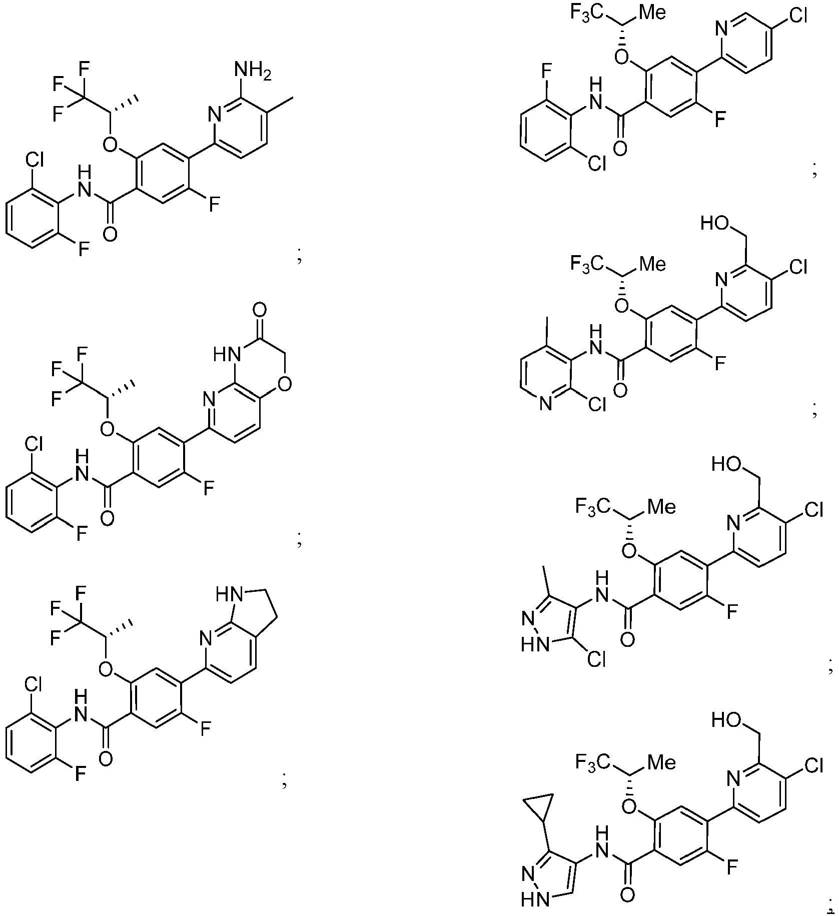 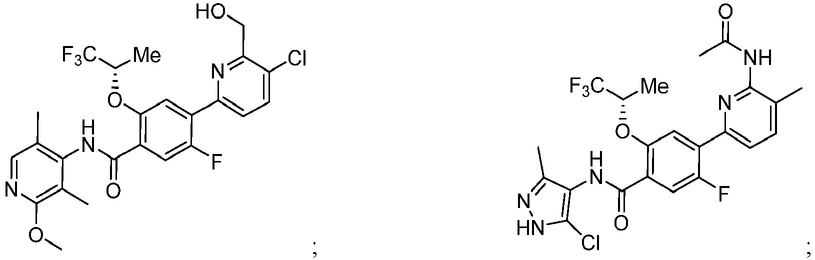 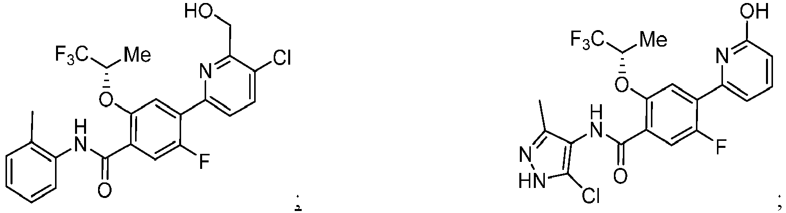 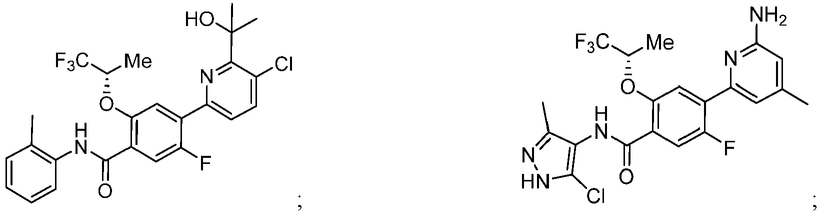 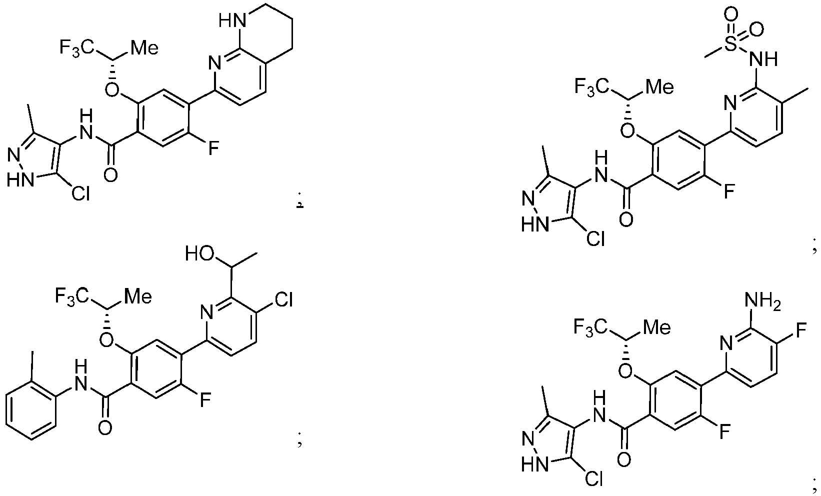 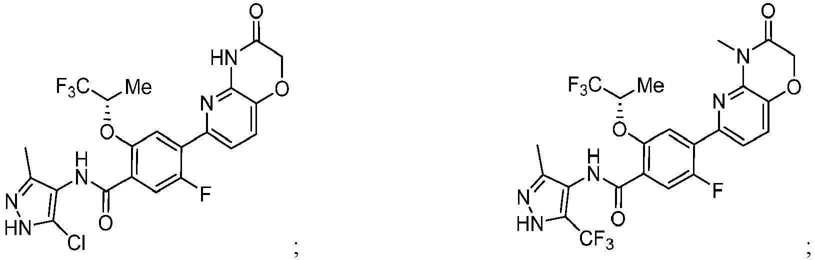 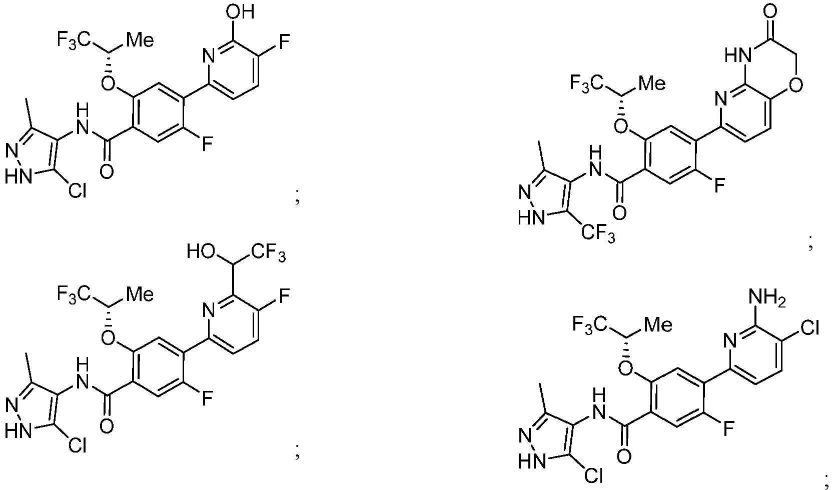 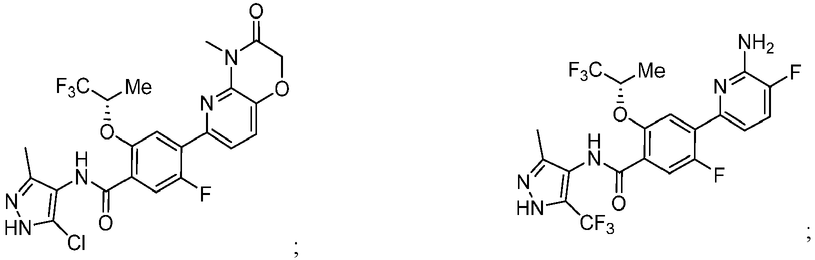 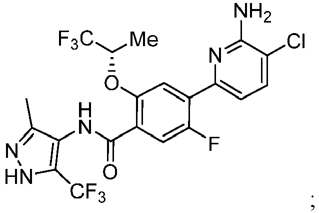 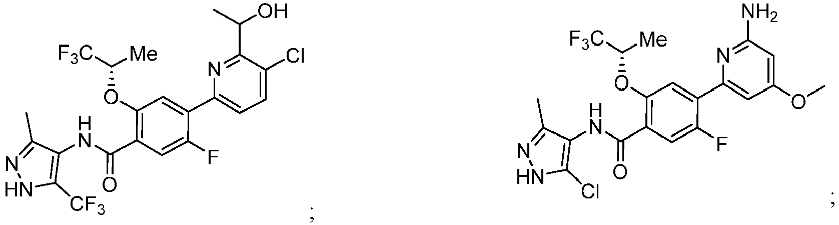 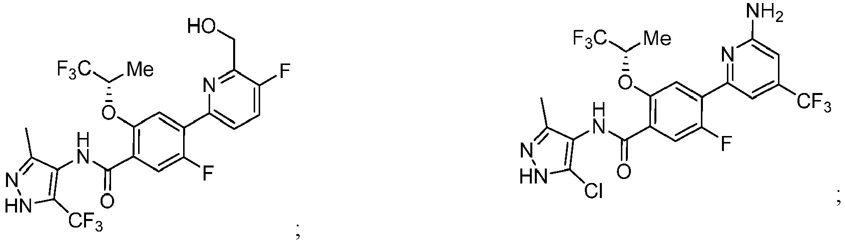 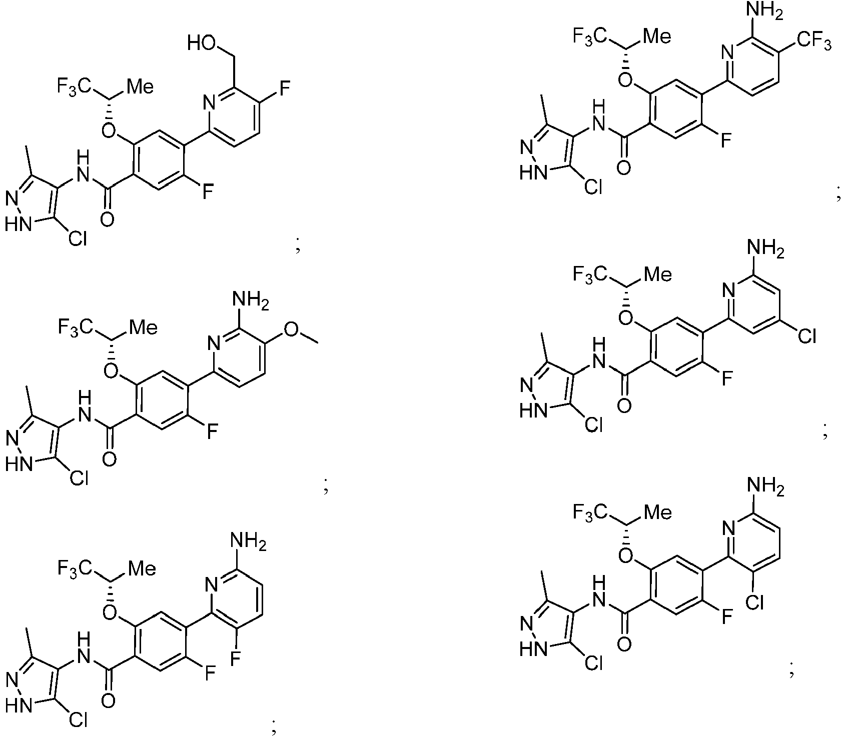 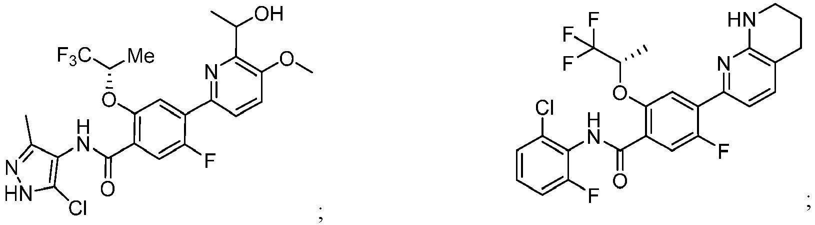 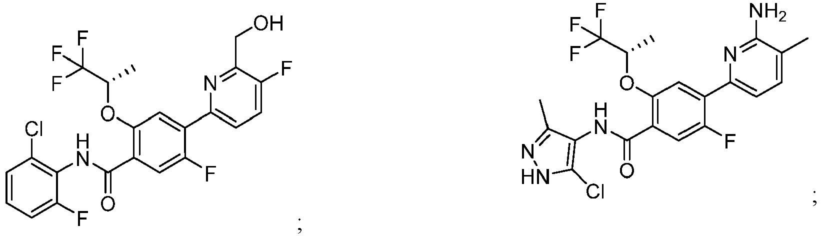 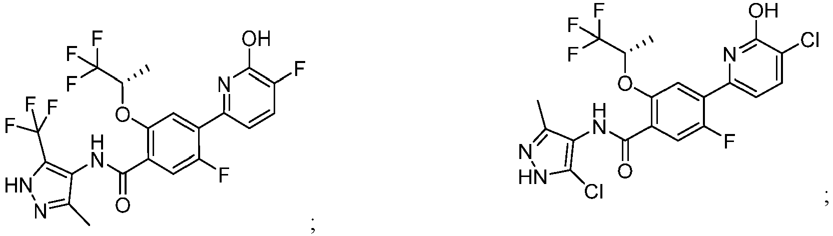 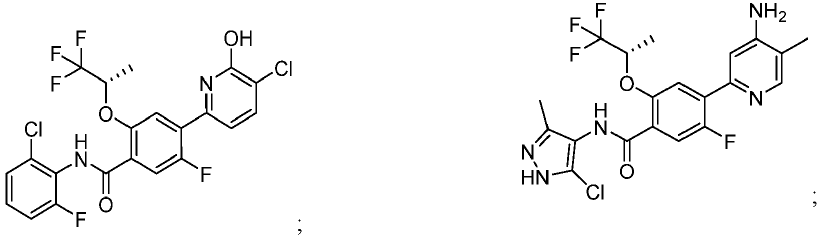 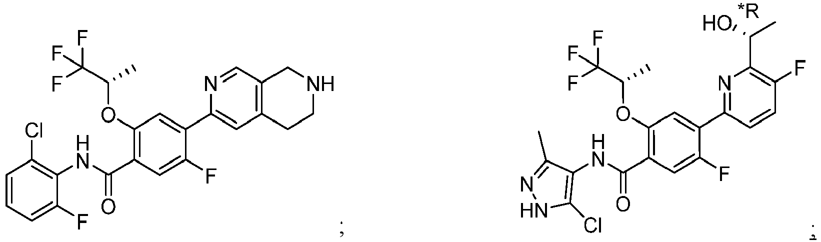 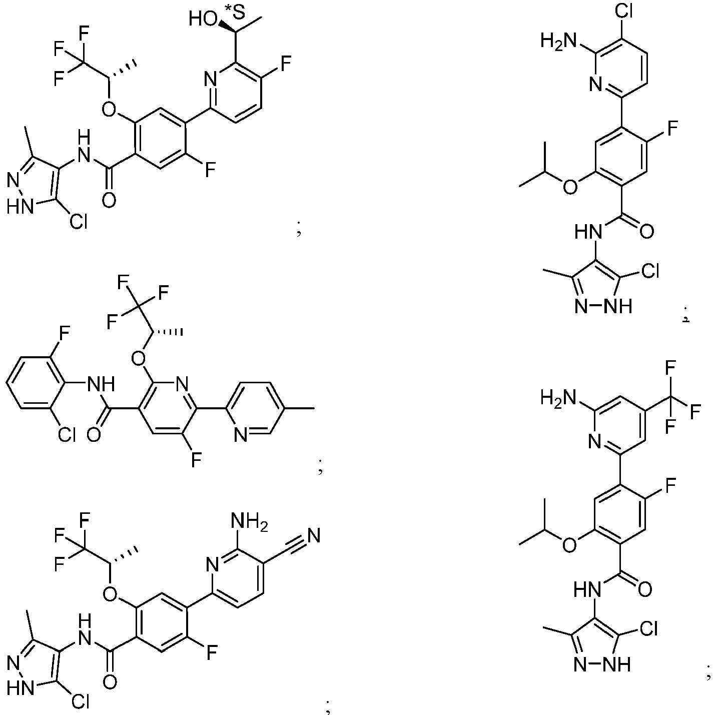 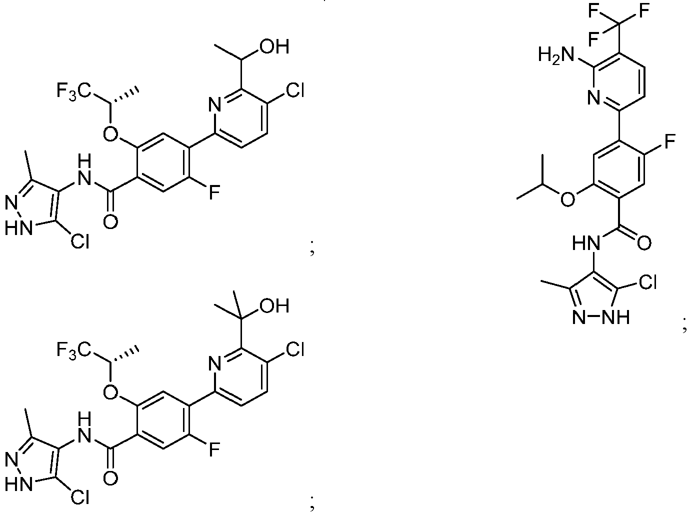 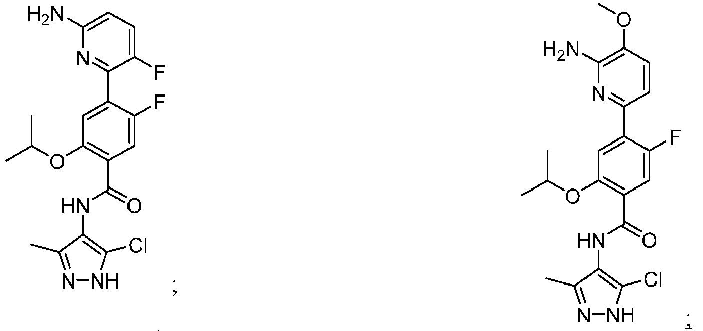 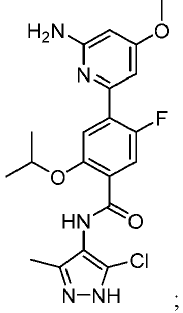 and
or a pharmaceutically acceptable salt, solvate, stereoisomer, isotopic variant, or N-oxide thereof;
or
(b) 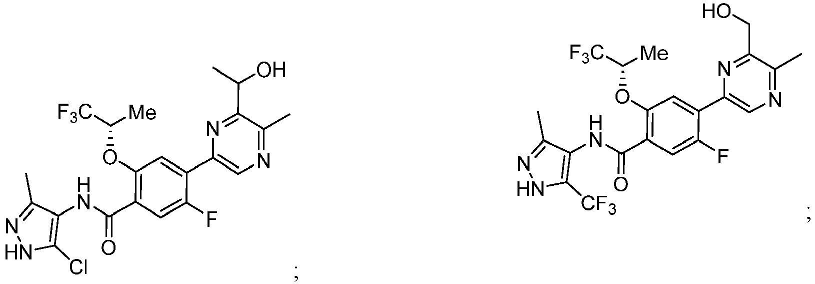 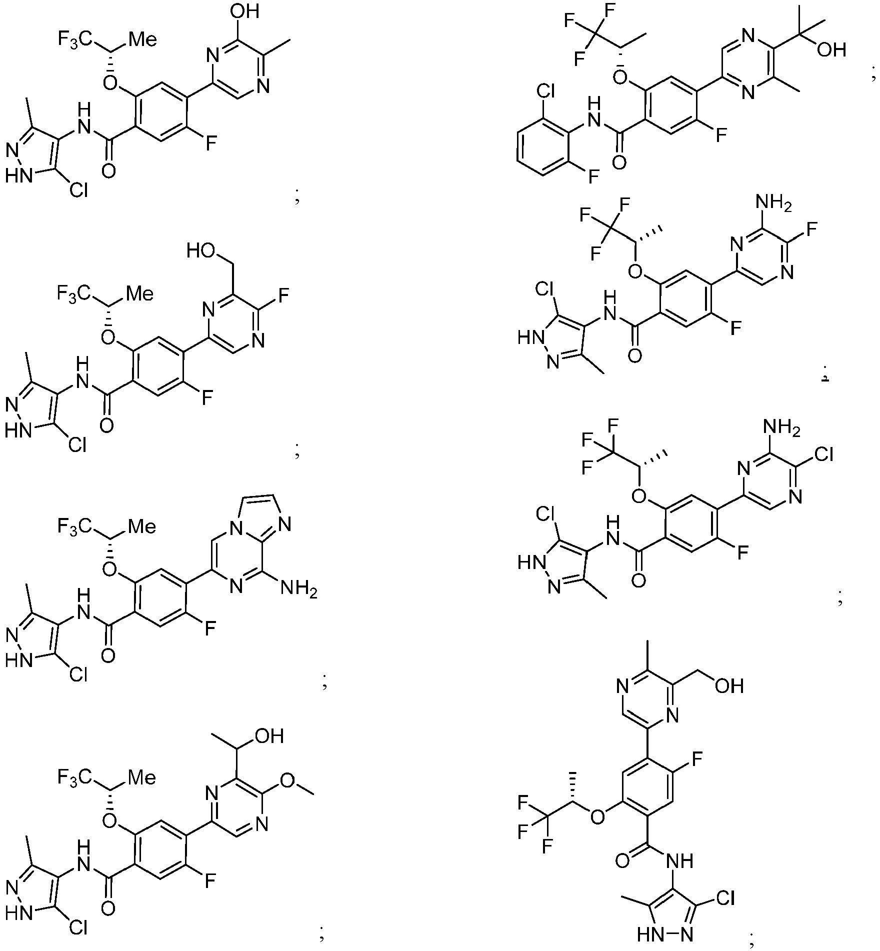 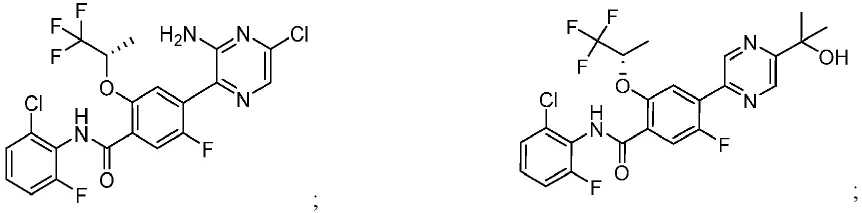 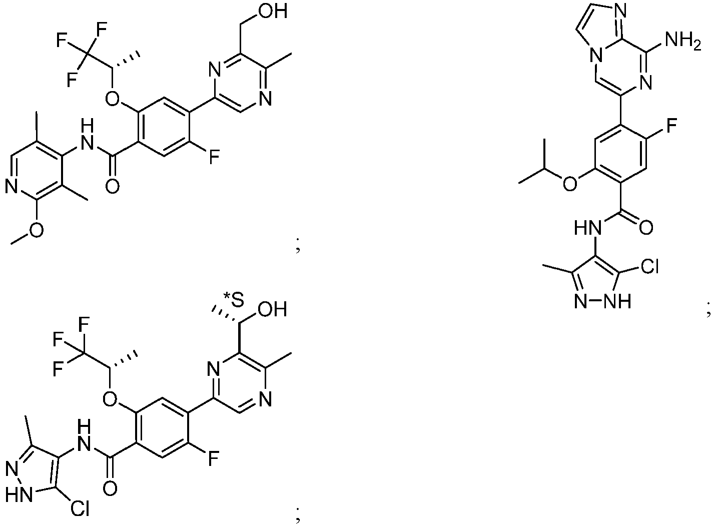 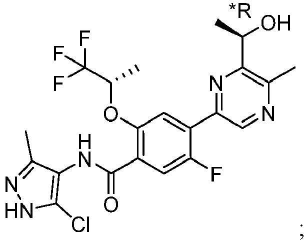 and
or a pharmaceutically acceptable salt, solvate, stereoisomer, isotopic variant, or N-oxide thereof;
or
(c) 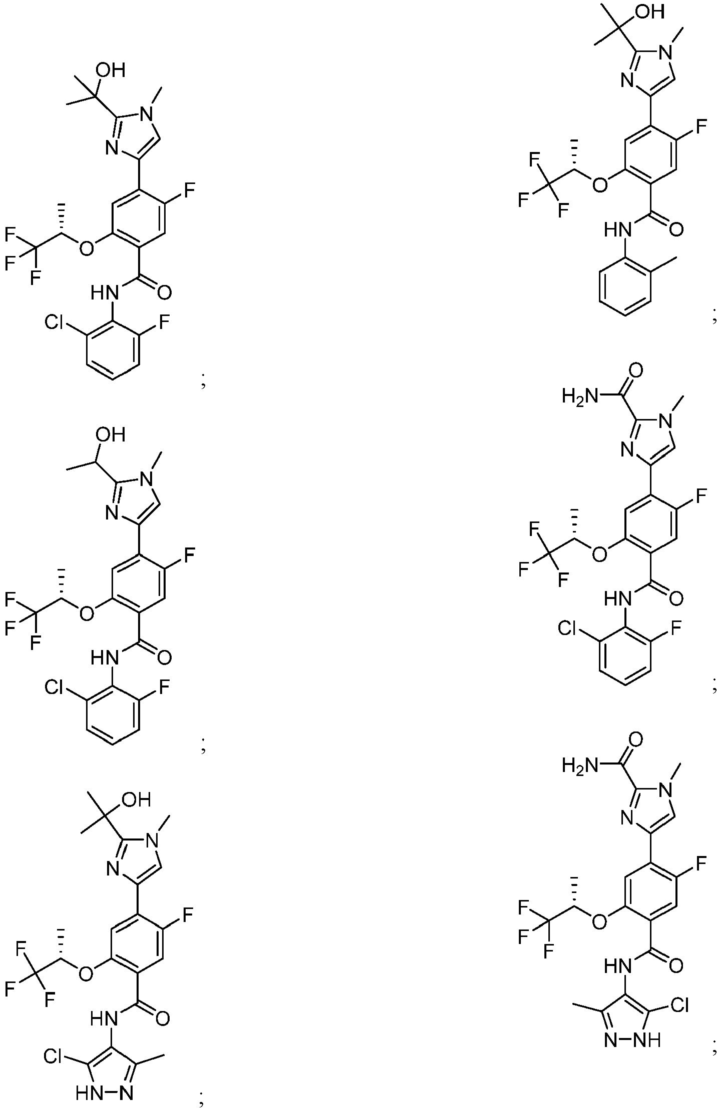 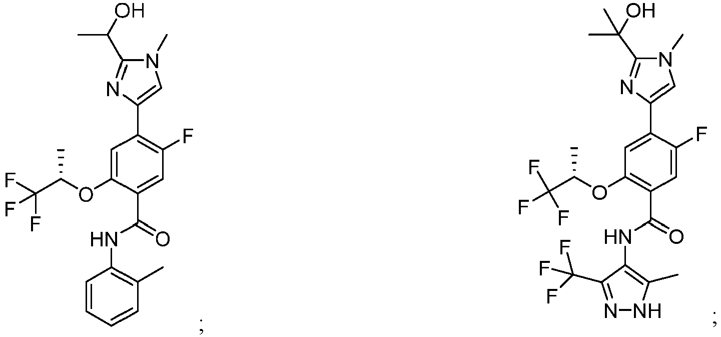 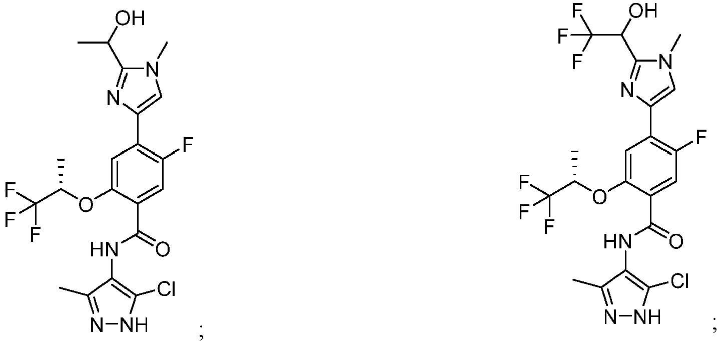 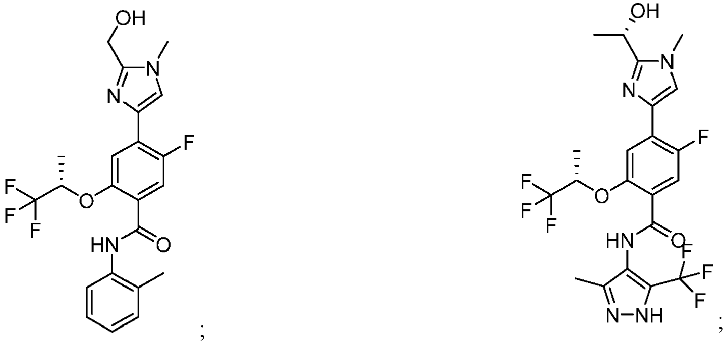 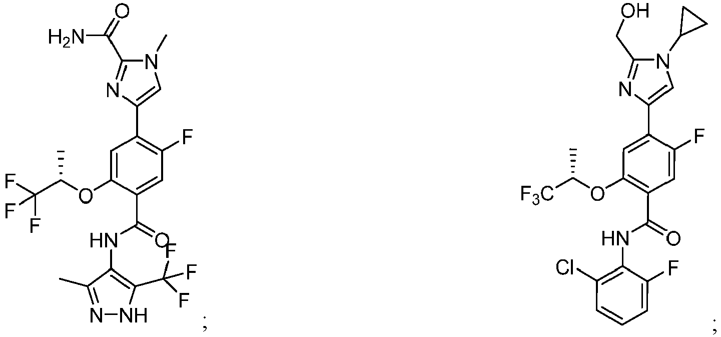 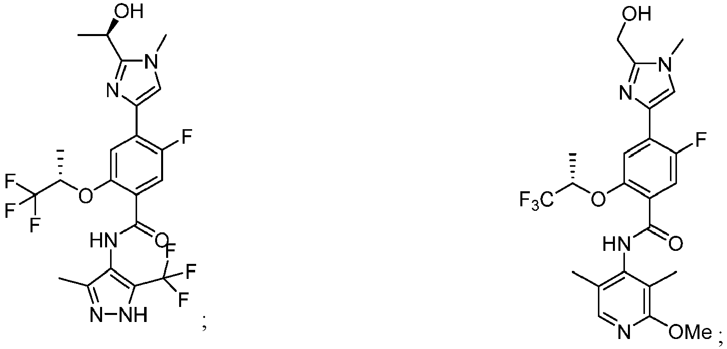 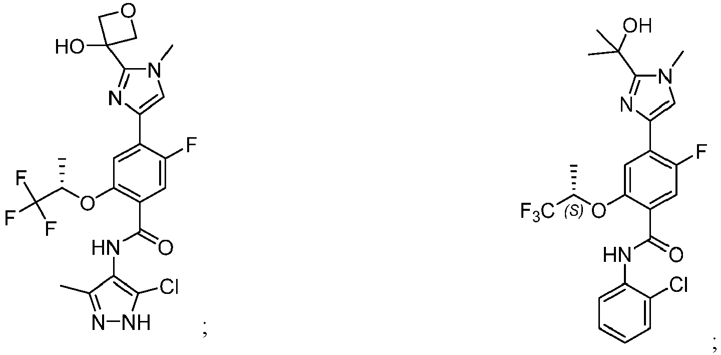 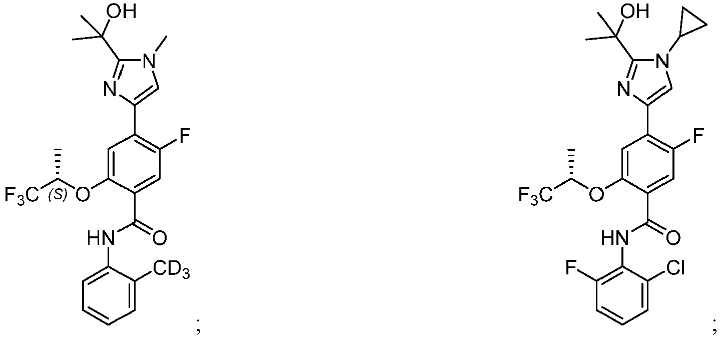 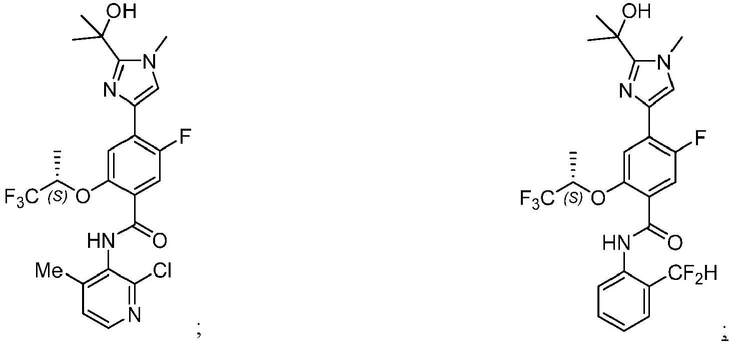 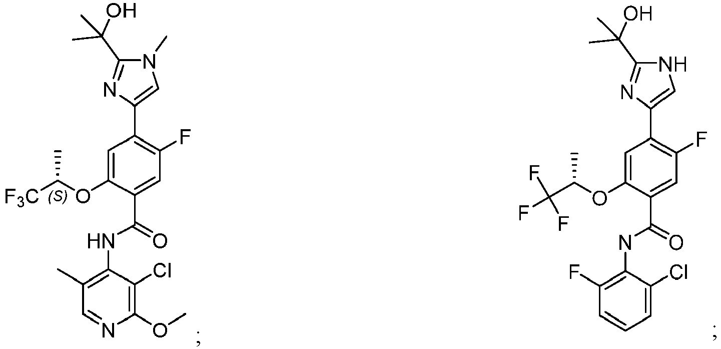 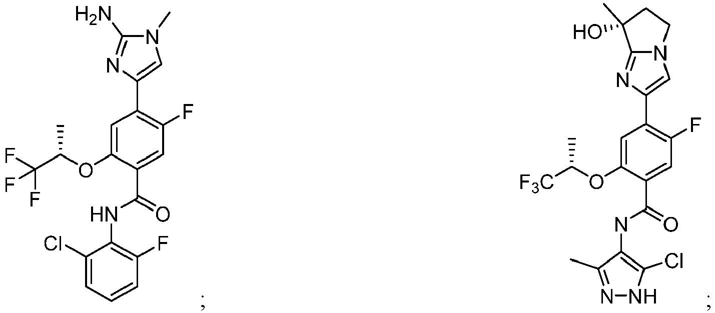 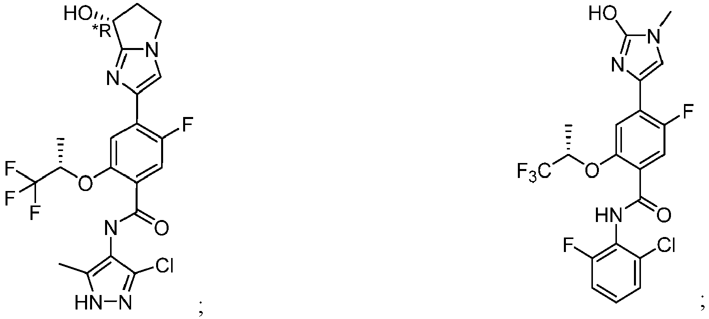 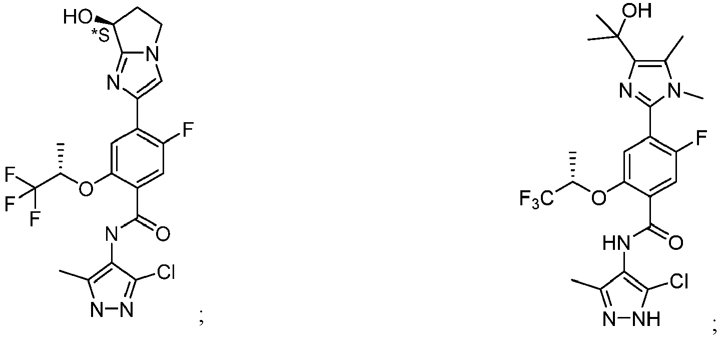 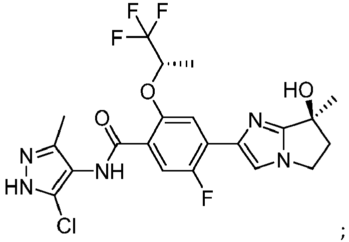 and 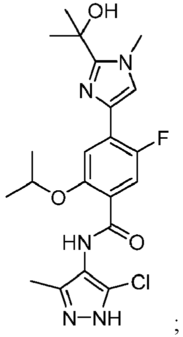 or a pharmaceutically acceptable salt, solvate, stereoisomer, isotopic variant, or N-oxide thereof;
or
(d) 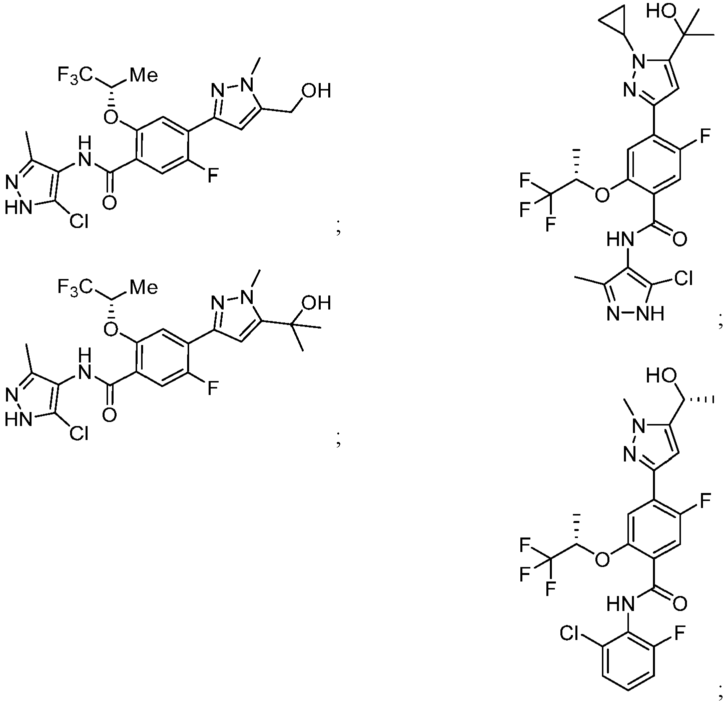 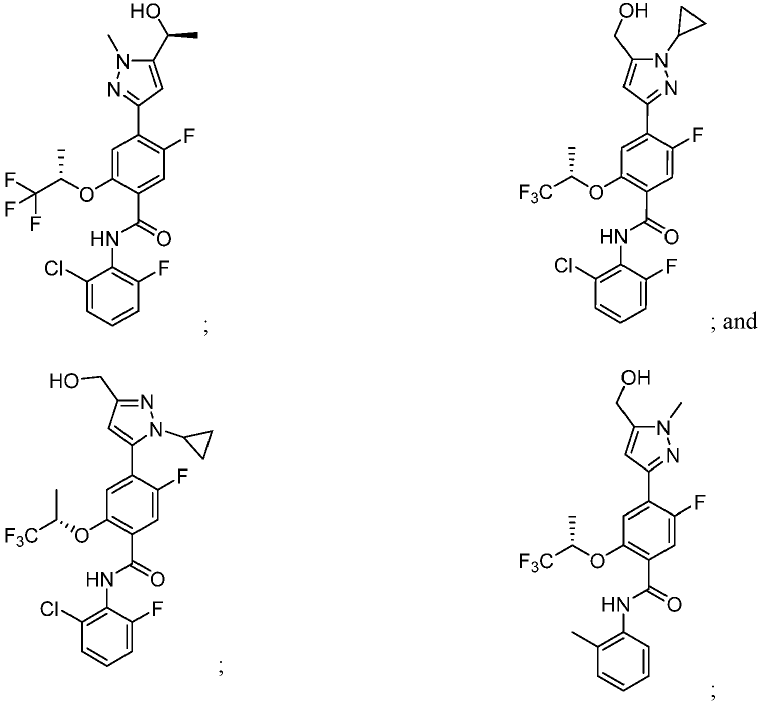
or a pharmaceutically acceptable salt, solvate, stereoisomer, isotopic variant, or N-oxide thereof.

10. The compound of claim 4 which is: 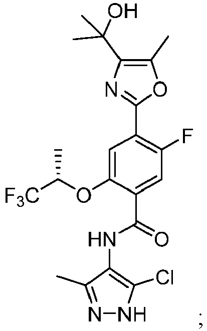 or a pharmaceutically acceptable salt, solvate, stereoisomer, isotopic variant, or N-oxide thereof.

11. The compound of claim 4 selected from the group consisting of:
(a) 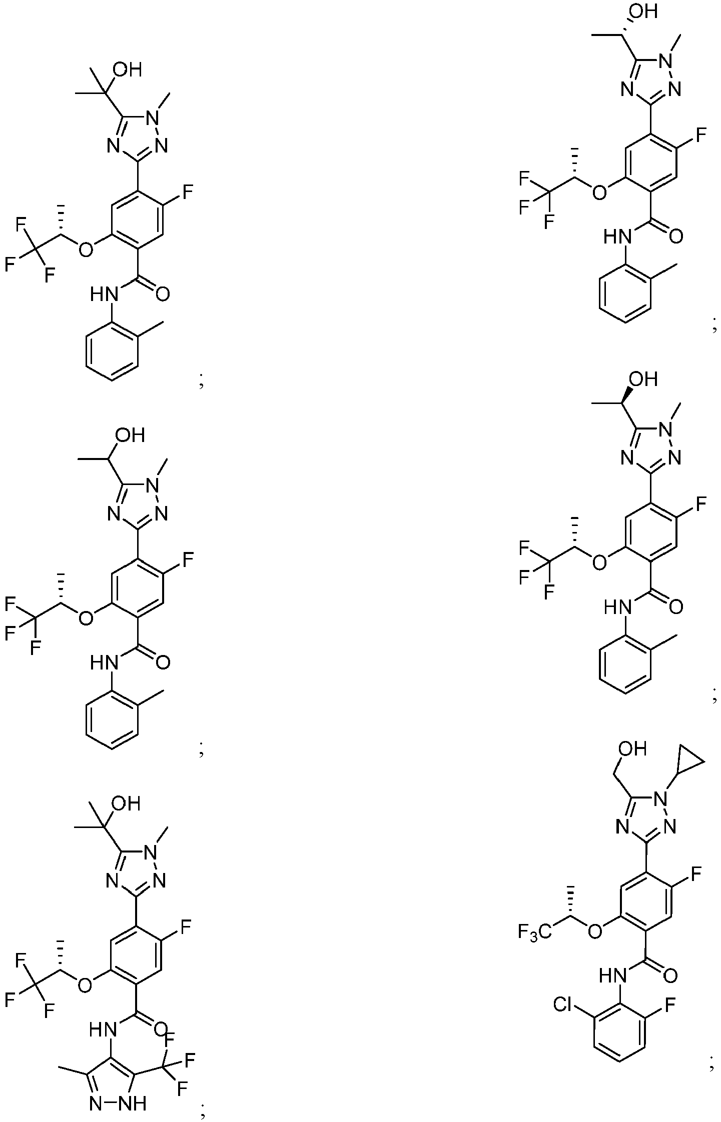 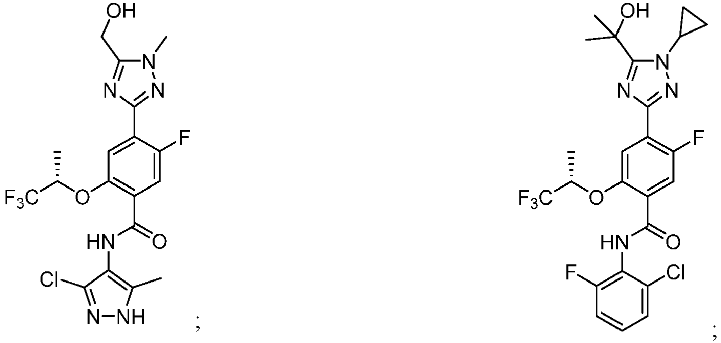 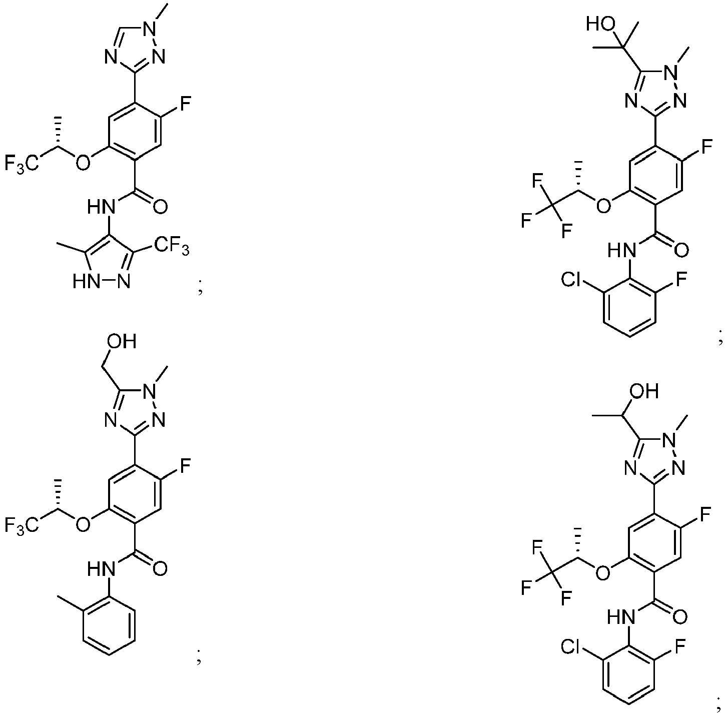 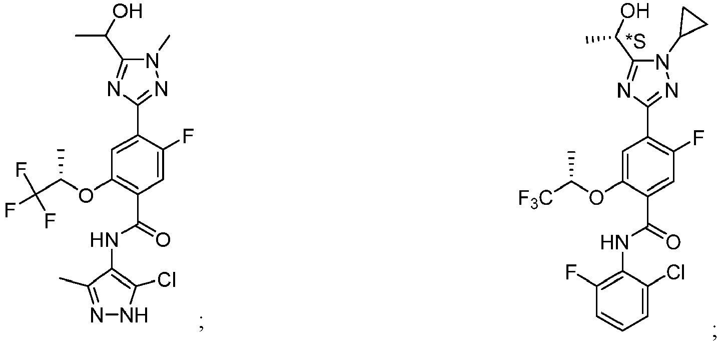 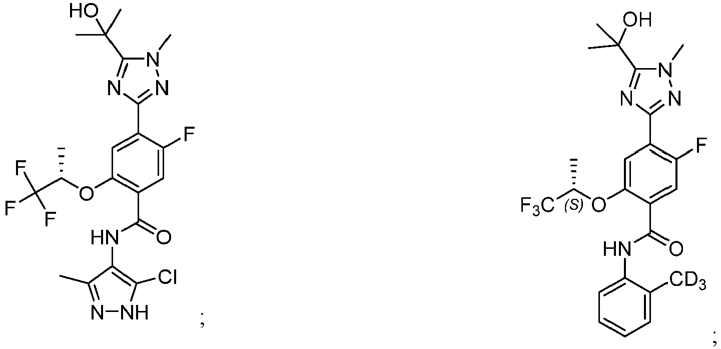 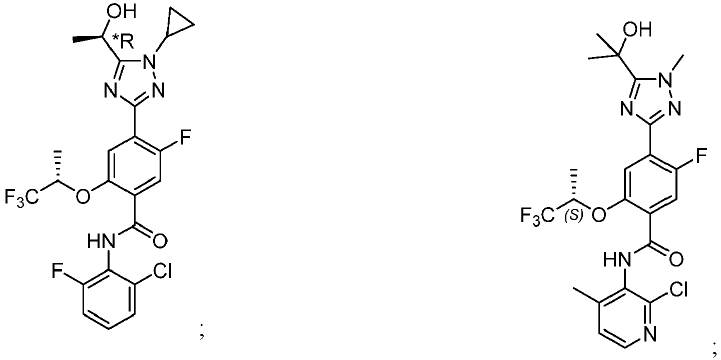 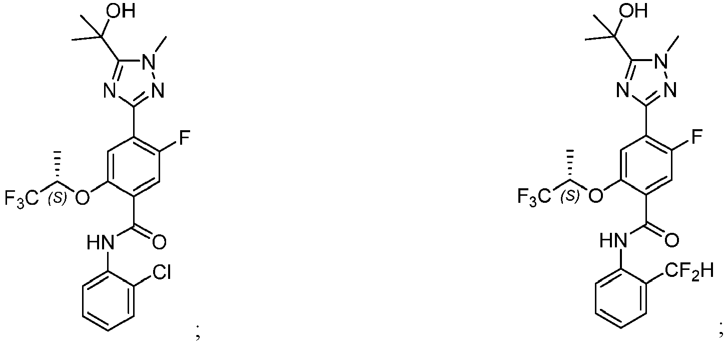 and 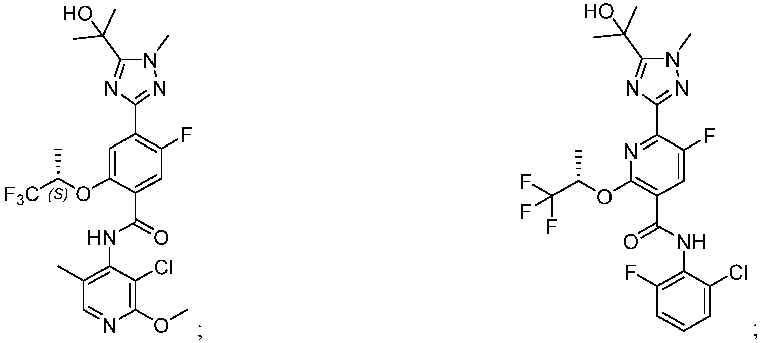 or a pharmaceutically acceptable salt, solvate, stereoisomer, isotopic variant, or N-oxide thereof;
or
(b) 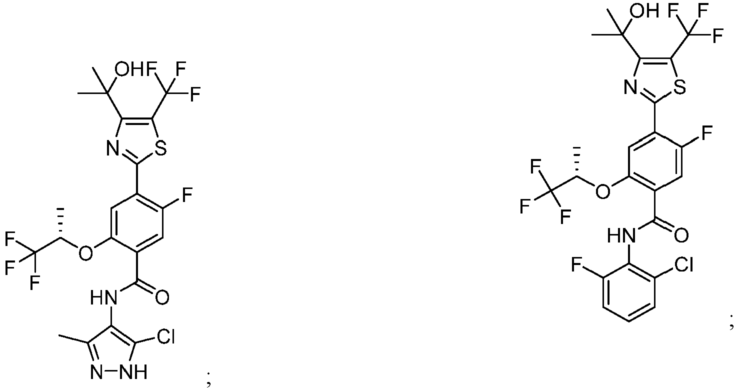 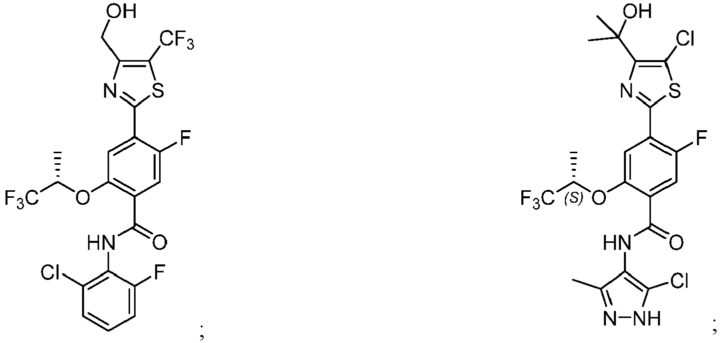 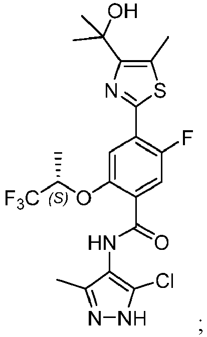 and
or a pharmaceutically acceptable salt, solvate, stereoisomer, isotopic variant, or N-oxide thereof;
or
(c) 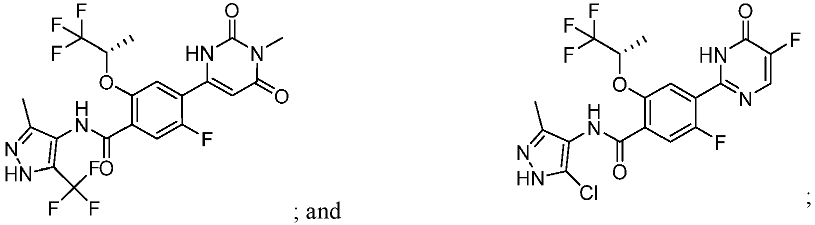 or a pharmaceutically acceptable salt, solvate, stereoisomer, isotopic variant, or N-oxide thereof.

12. A pharmaceutical composition comprising: (A) a compound according to any of claims 1 to 11, or a pharmaceutically acceptable salt, solvate, stereoisomer, isotopic variant, or N-oxide thereof; and (B) at least one pharmaceutically acceptable excipient.

13. A pharmaceutical composition comprising an effective amount of a compound according to claim 1 selected from the group consisting of:
(a) 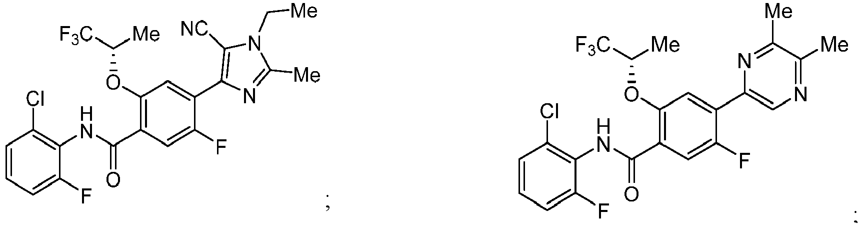 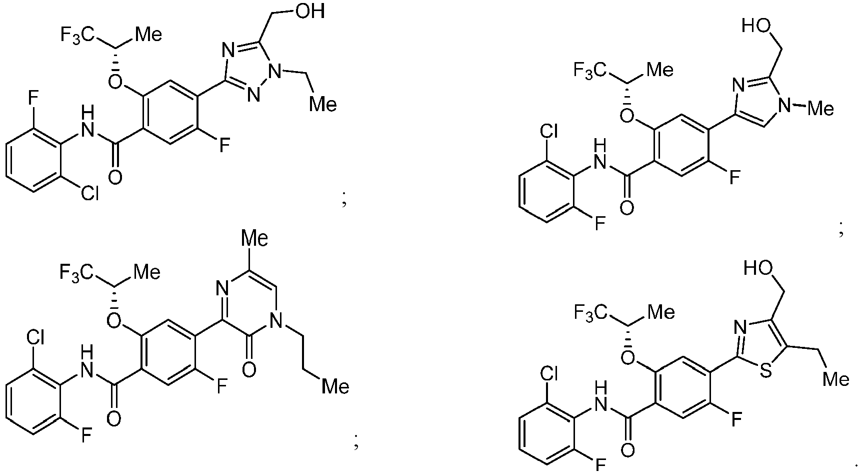 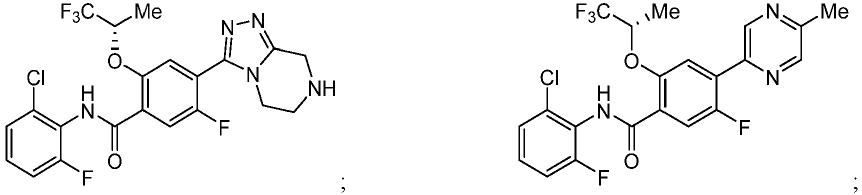 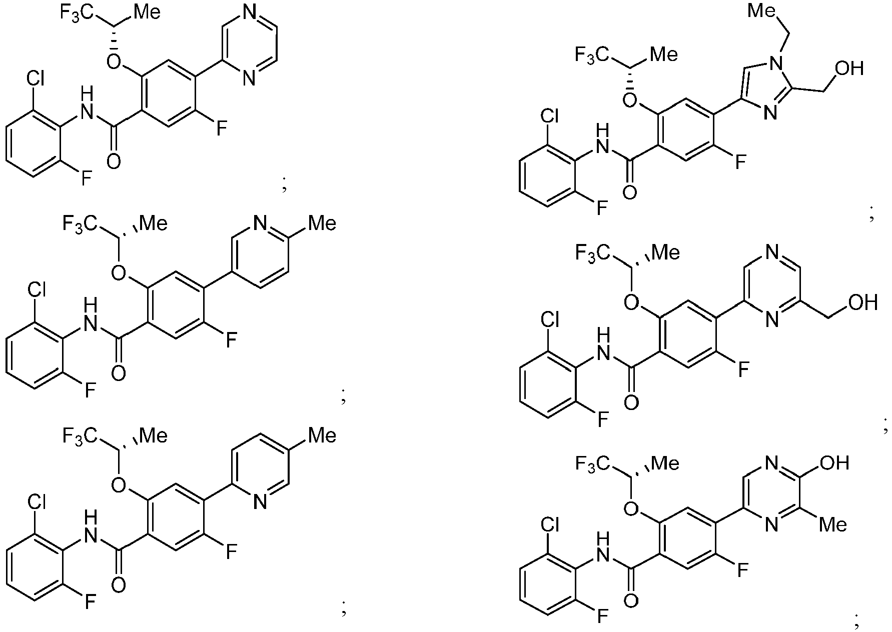 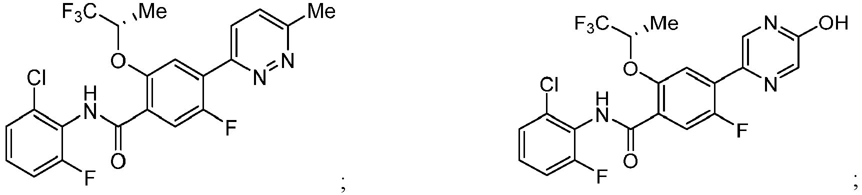 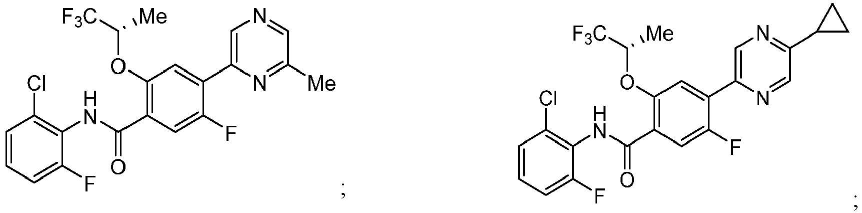 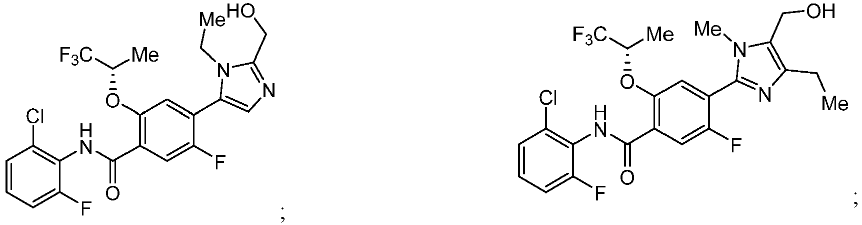 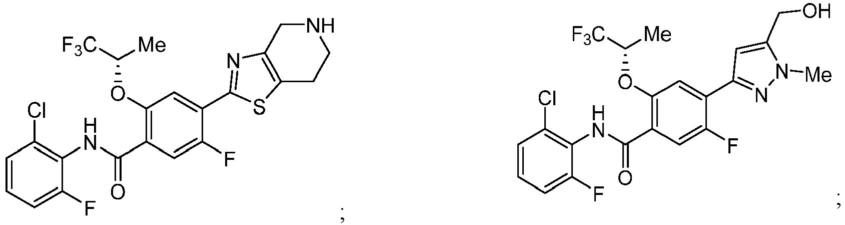 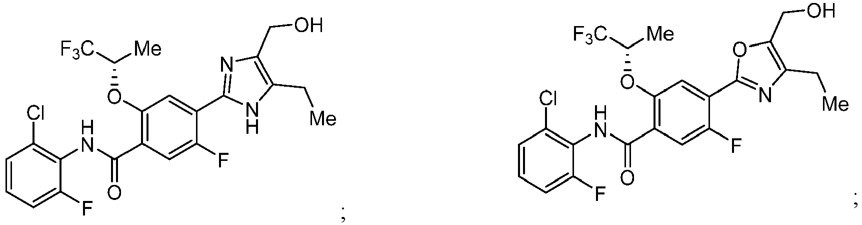 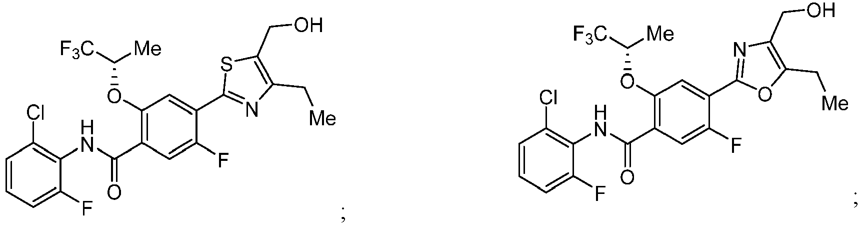 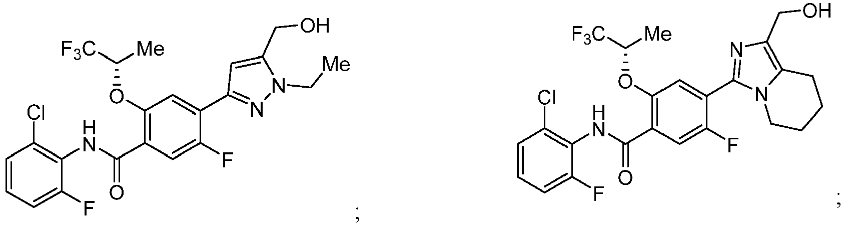 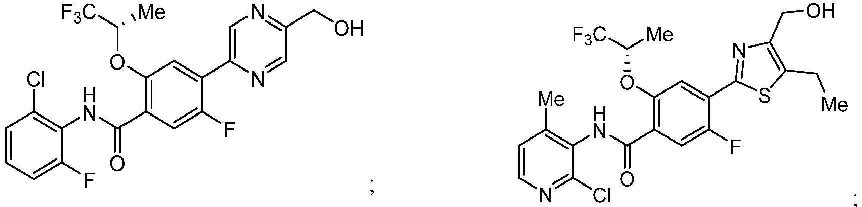 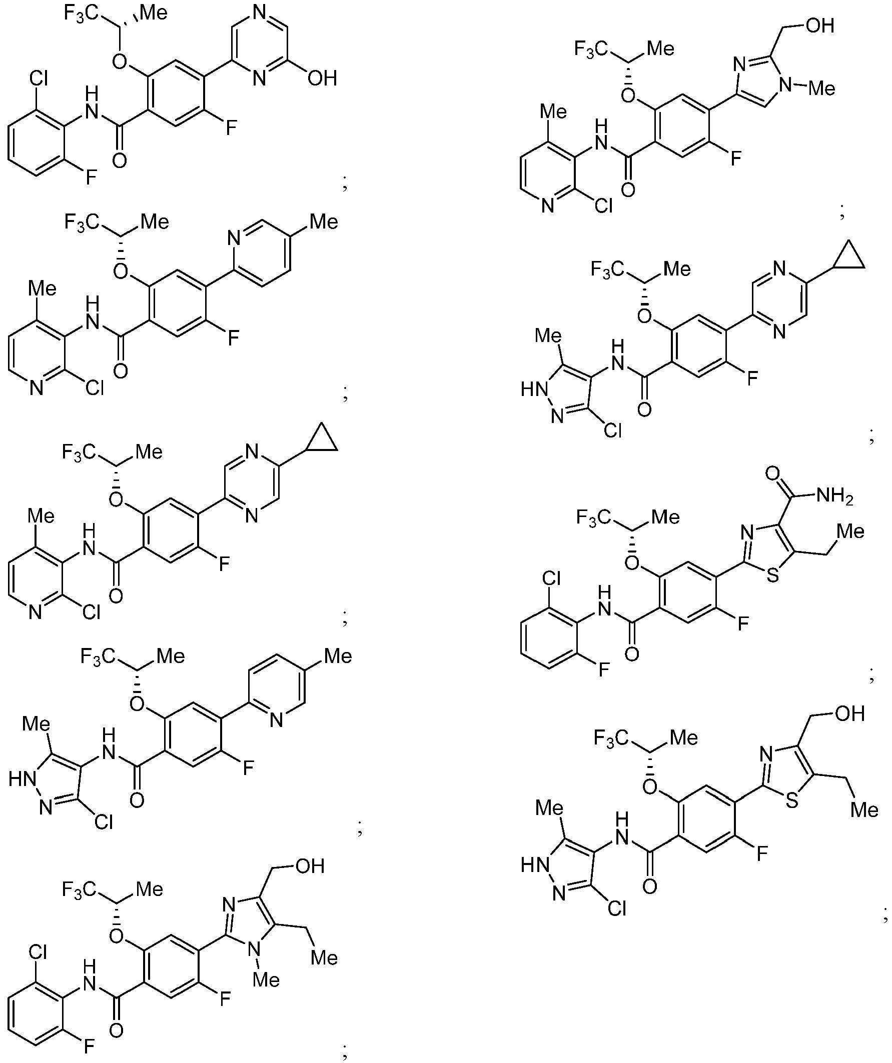 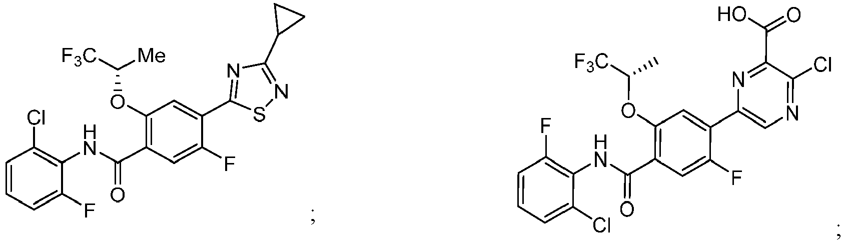 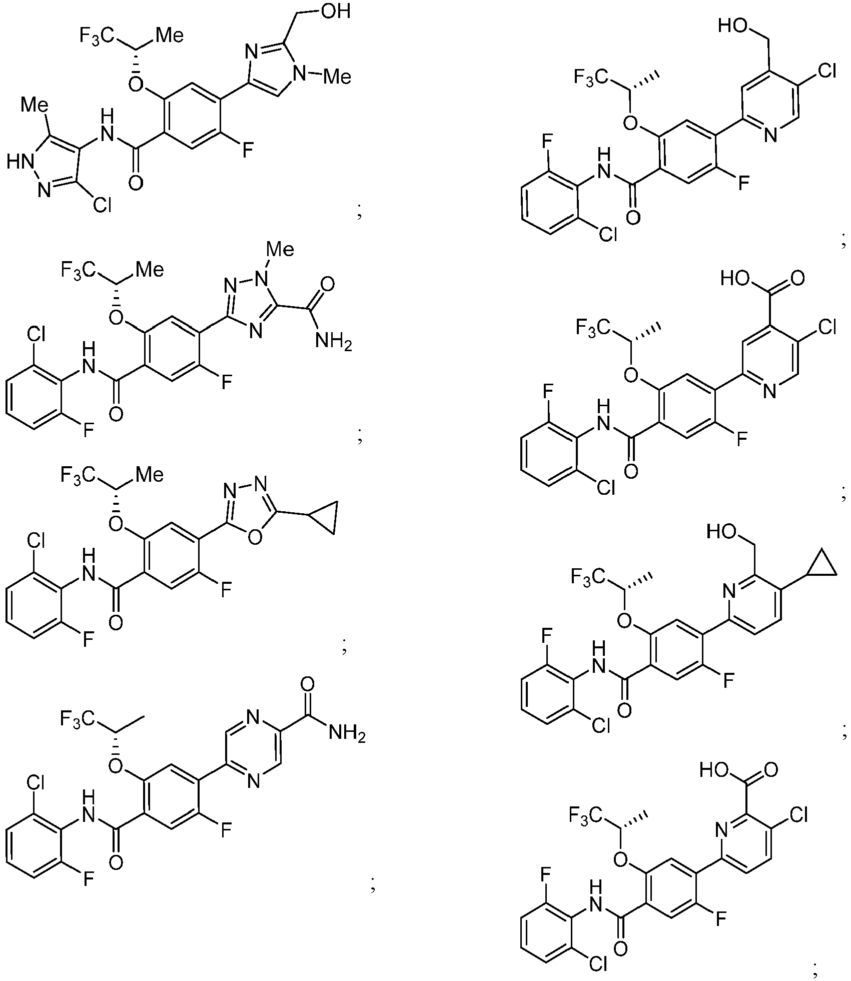 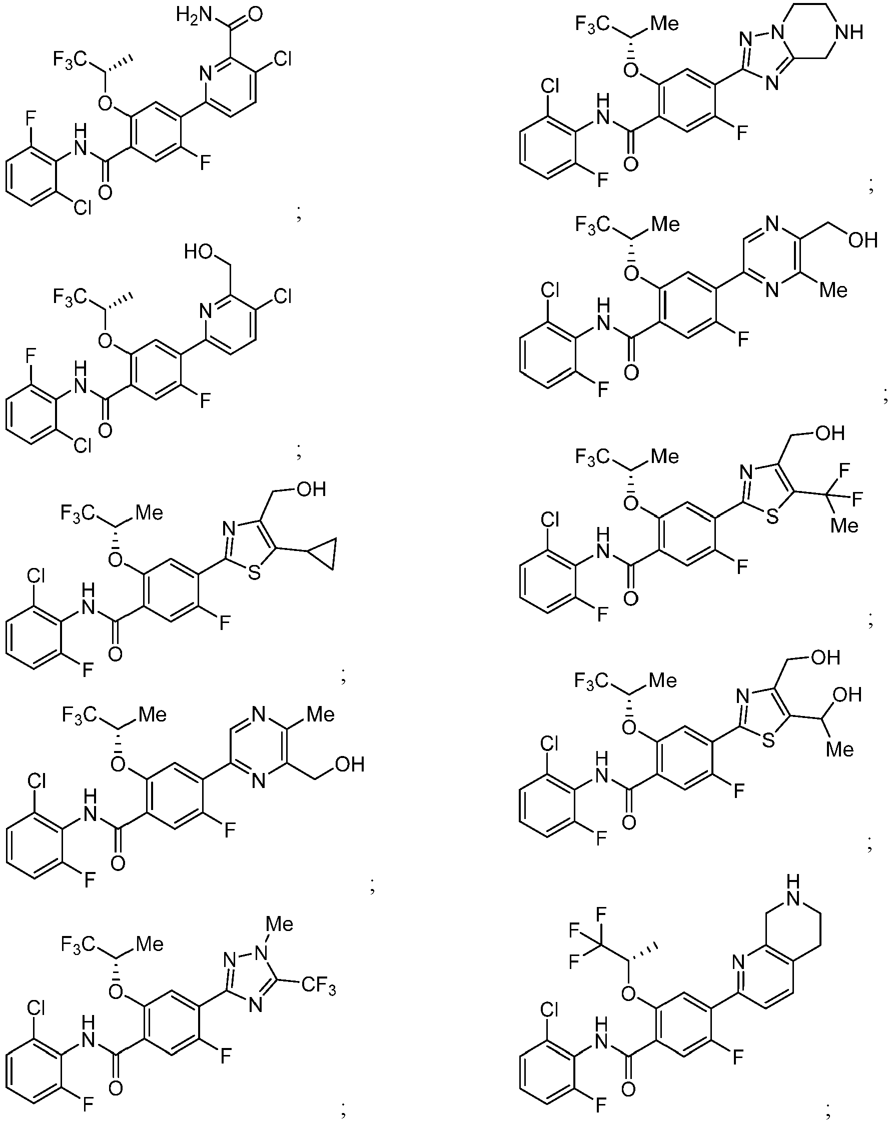 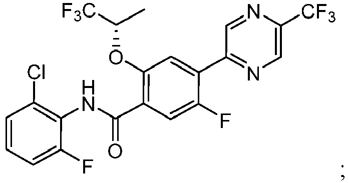 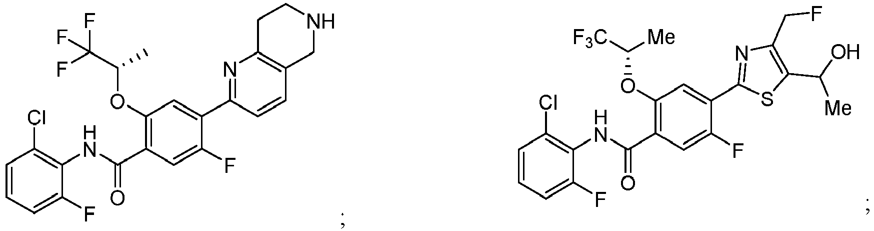 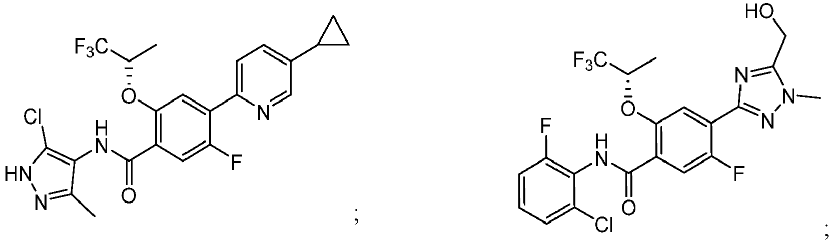 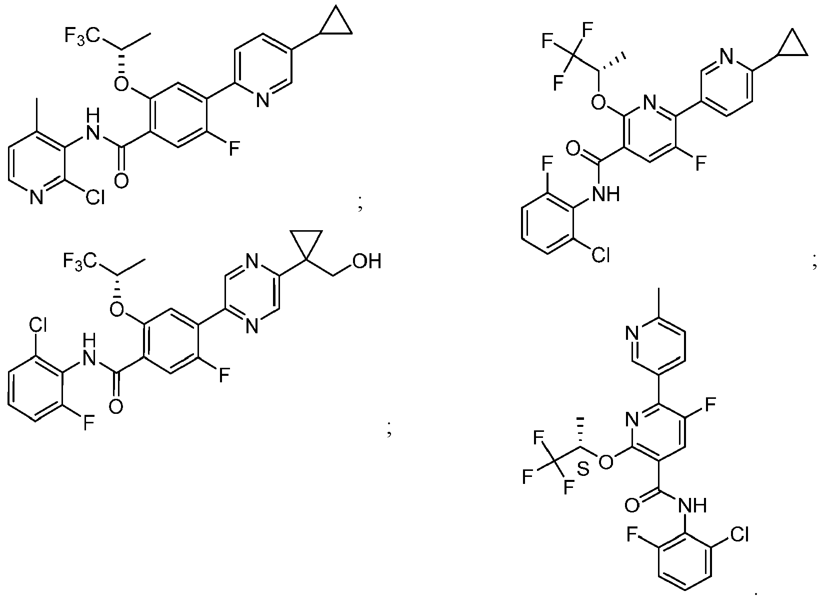 and or a pharmaceutically acceptable salt, solvate, stereoisomer, isotopic variant, or N-oxide thereof; and at least one pharmaceutically acceptable excipient; or
(b) 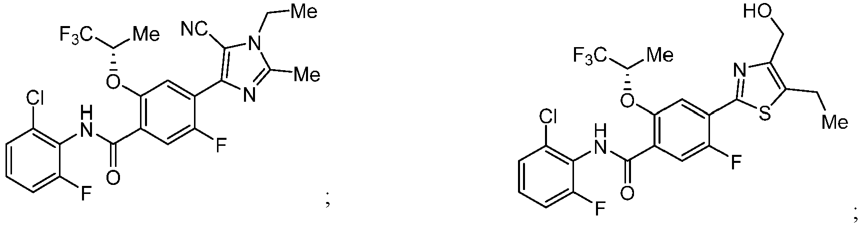 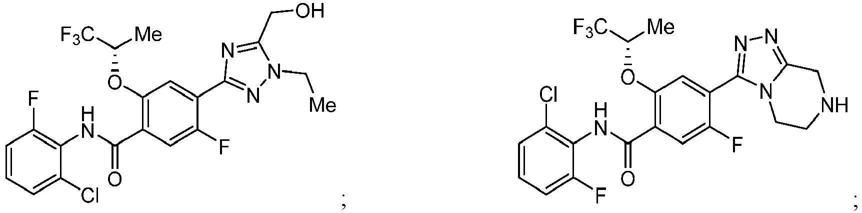 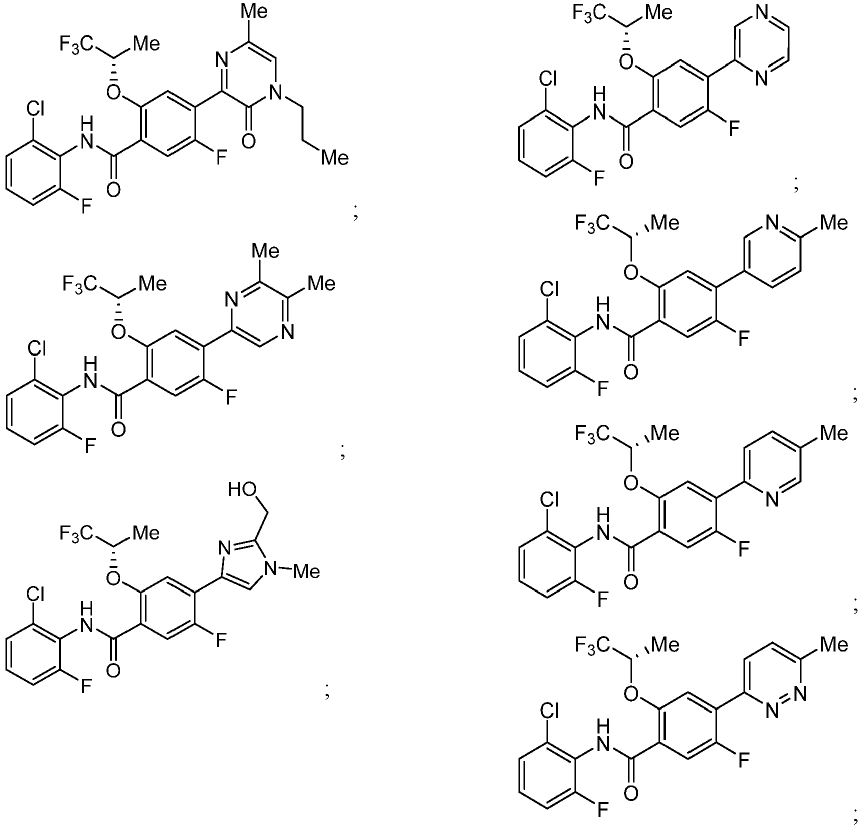 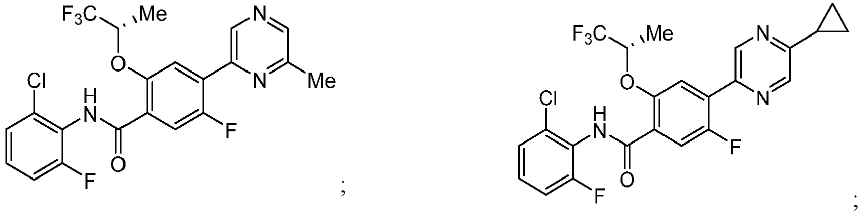 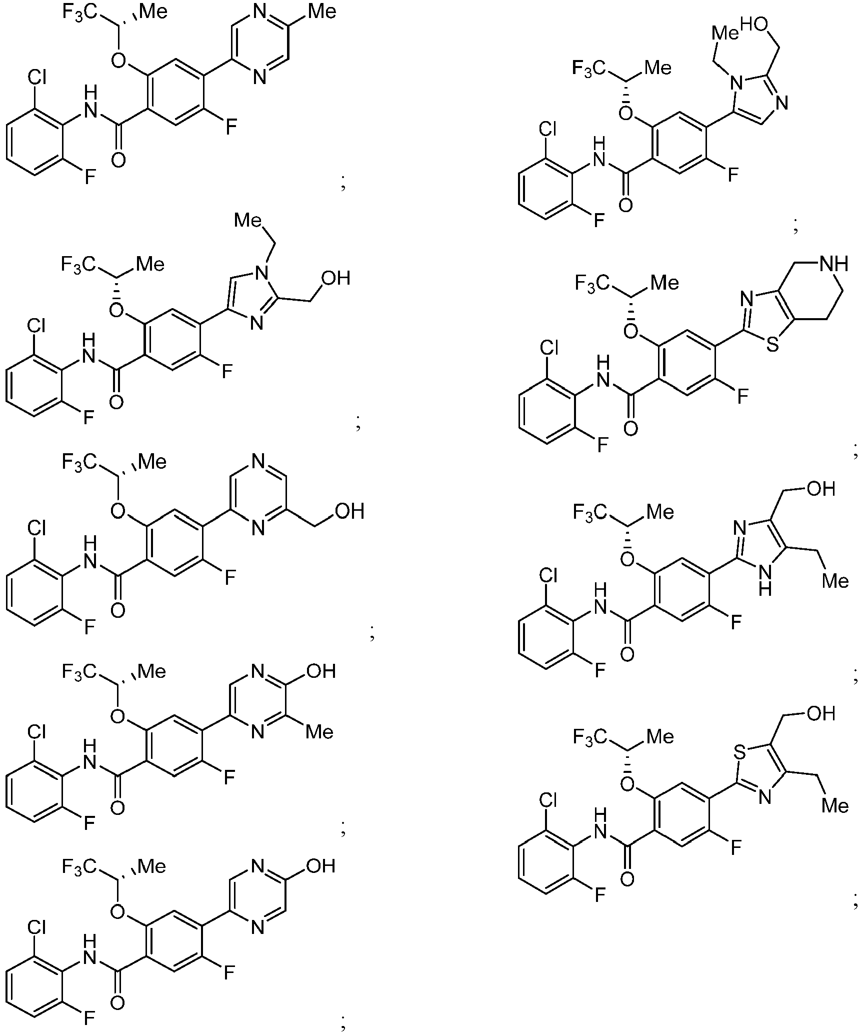 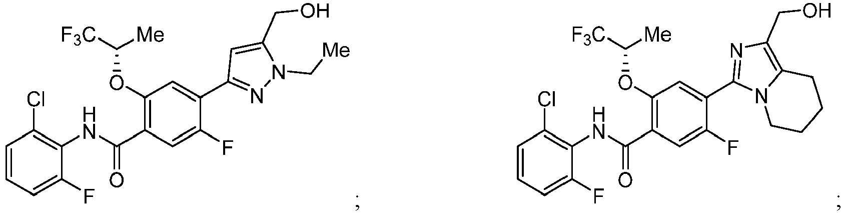 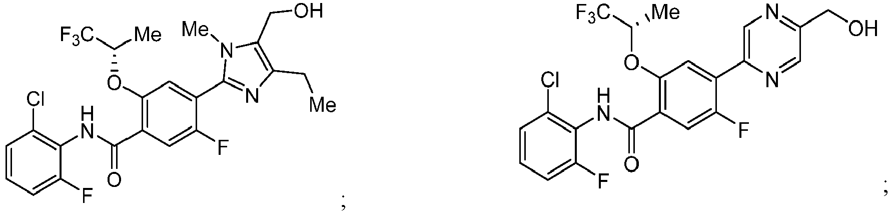 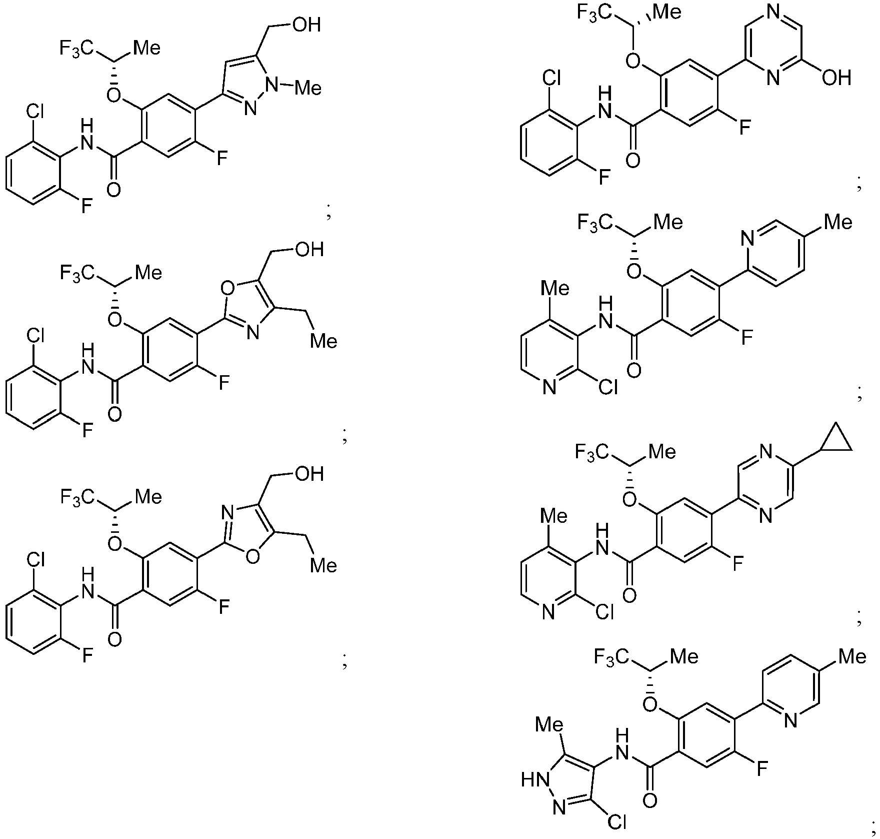 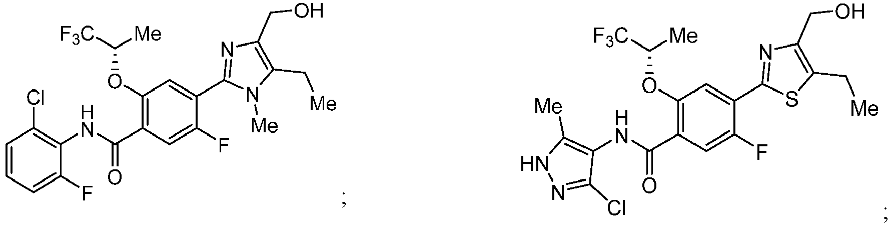 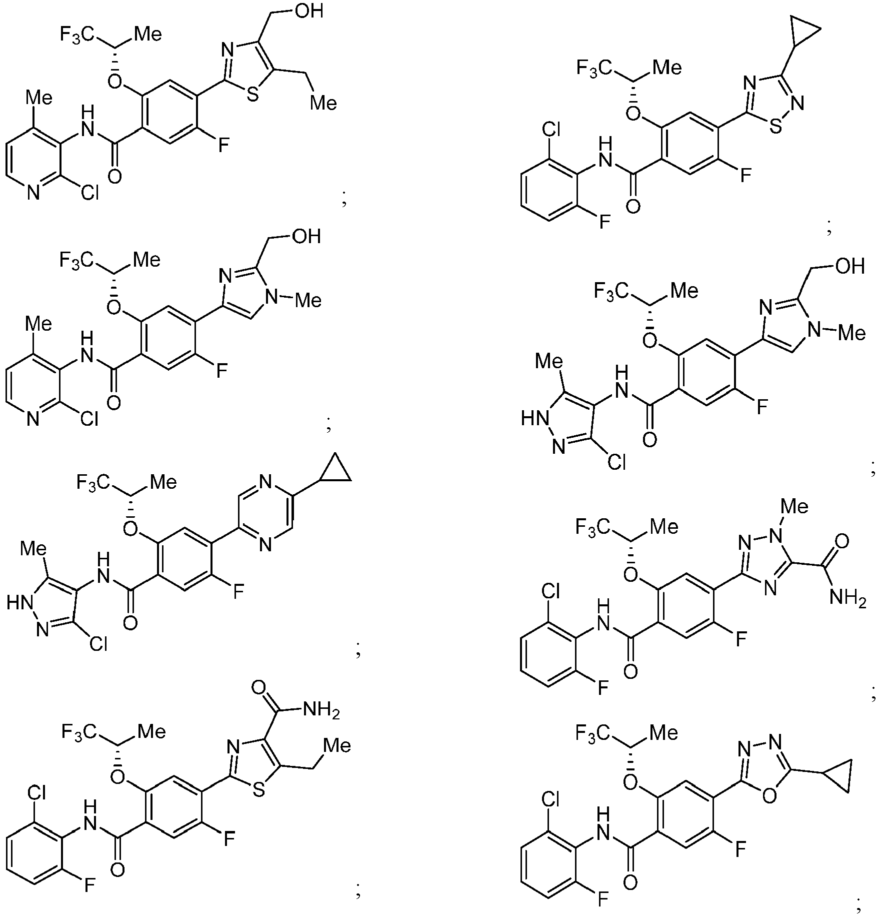 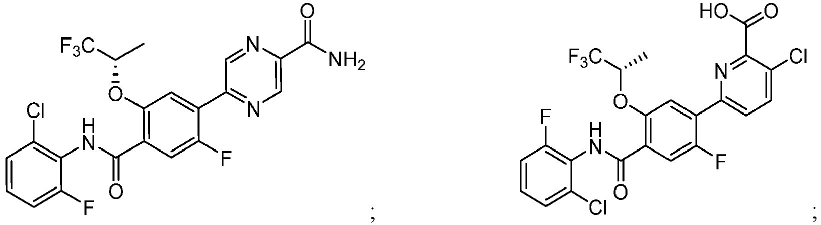 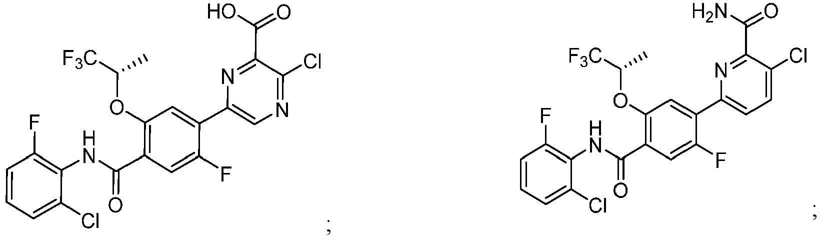 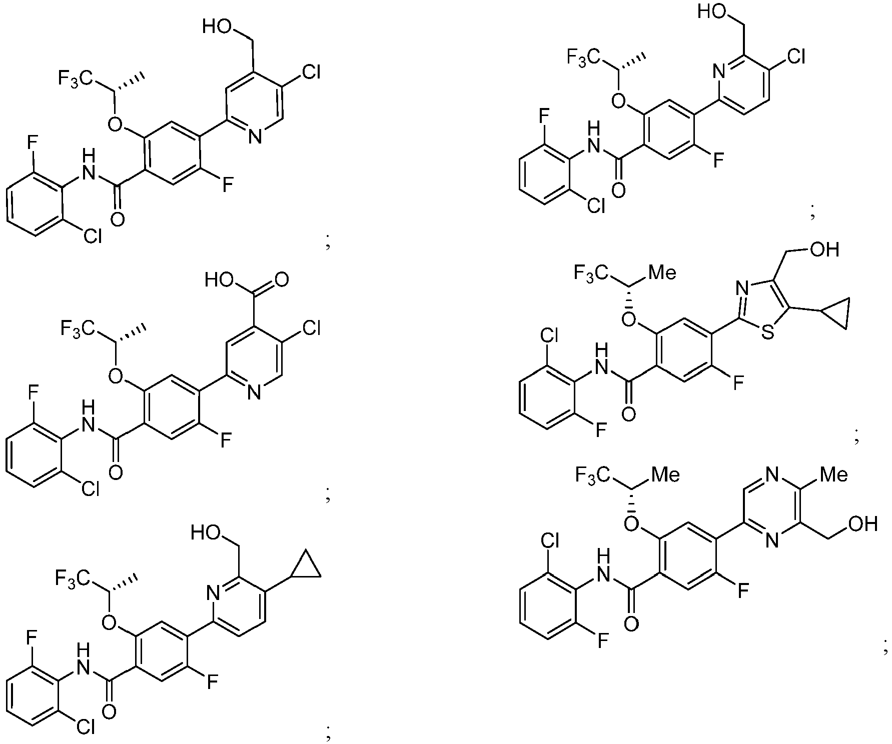 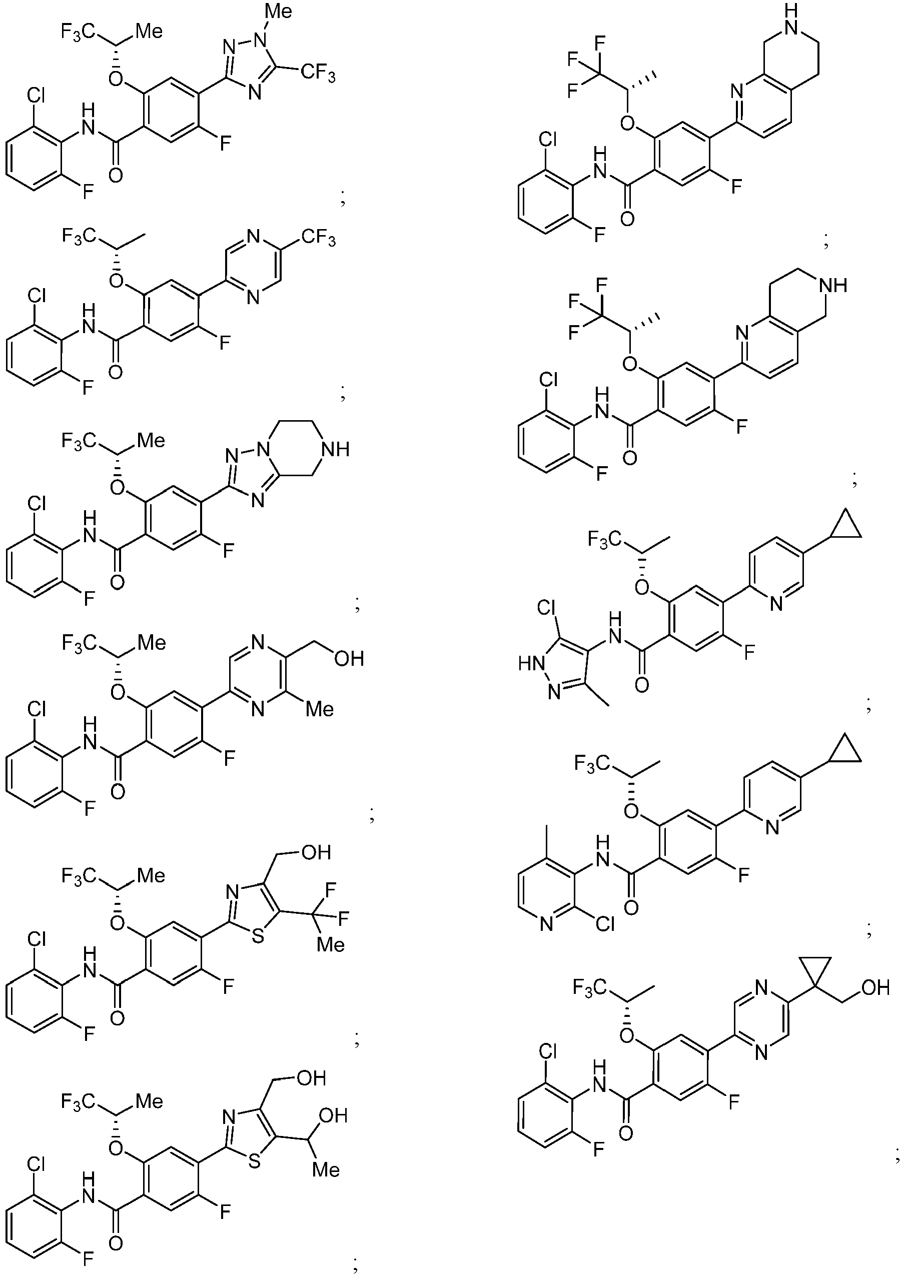 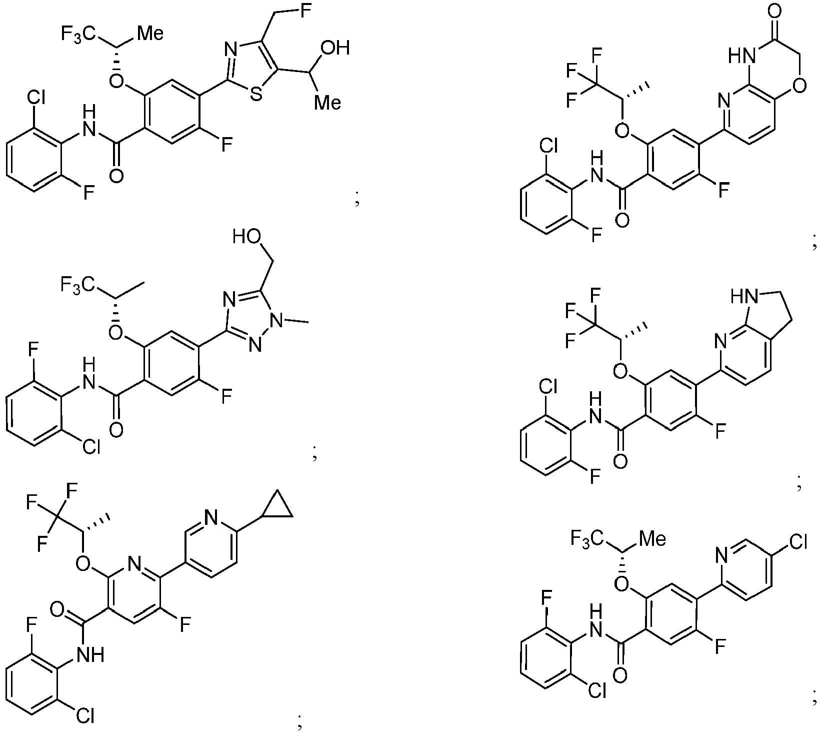 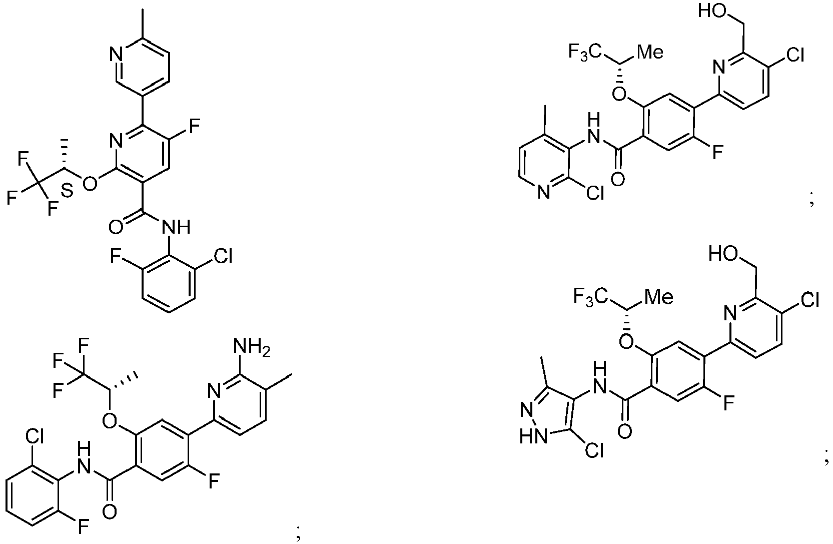 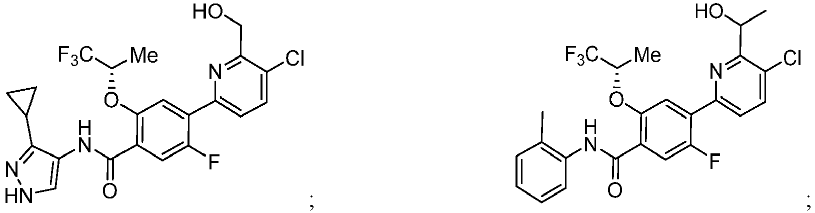 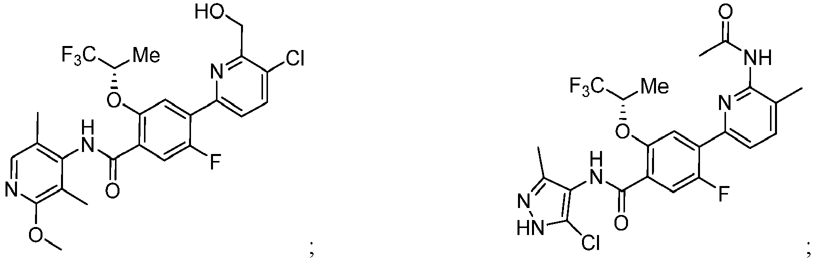 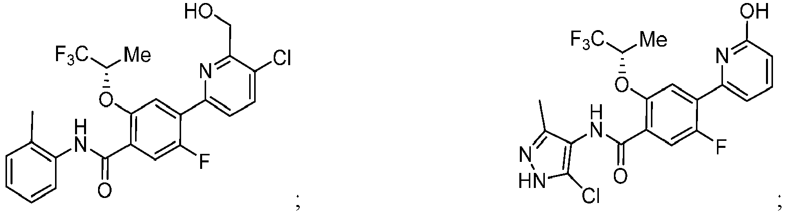 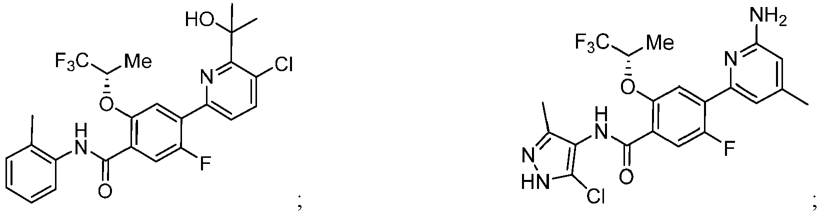 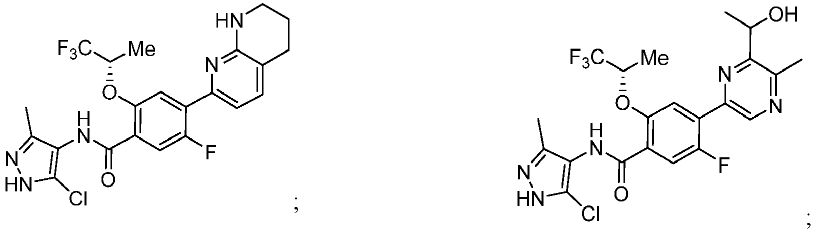 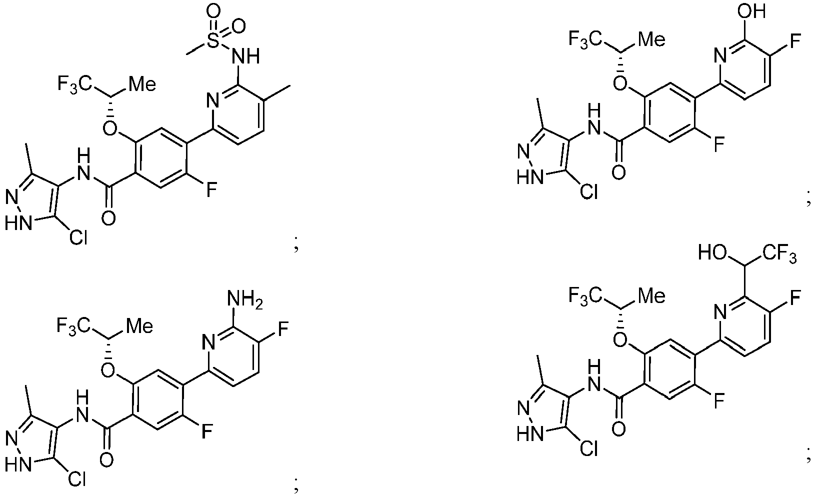 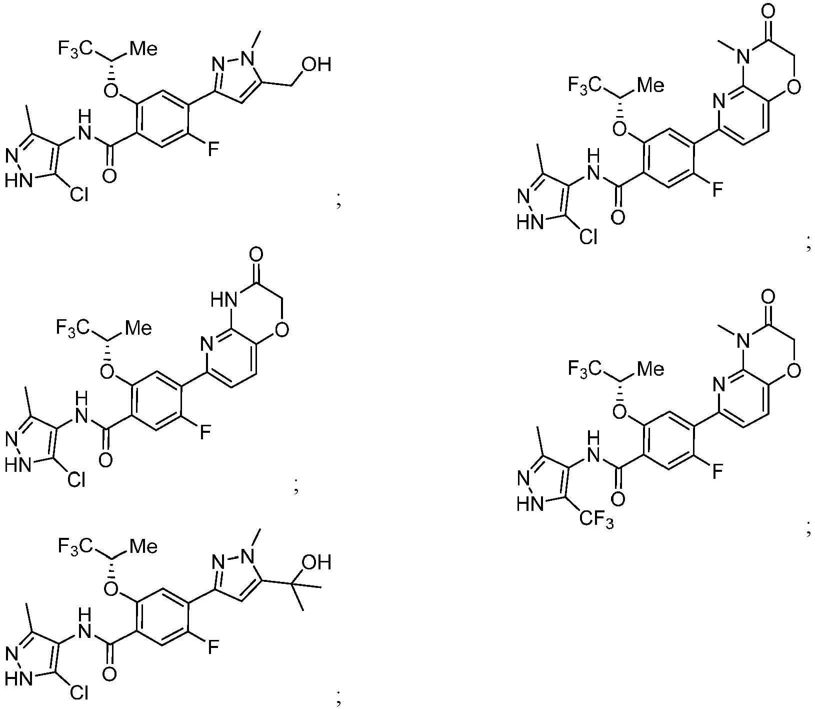 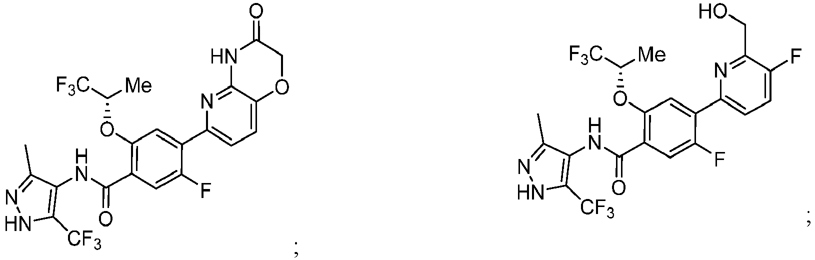 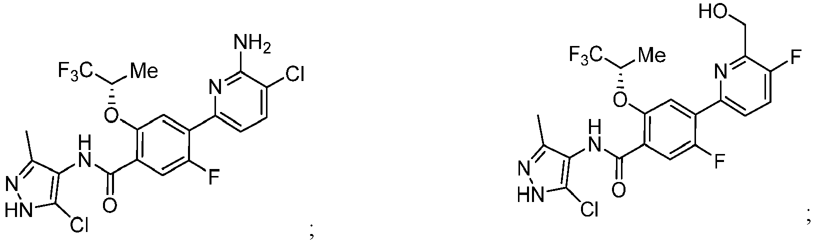 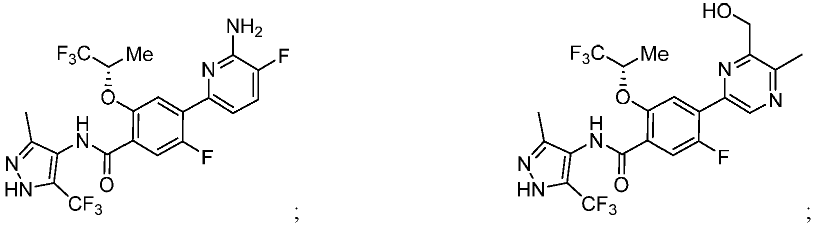 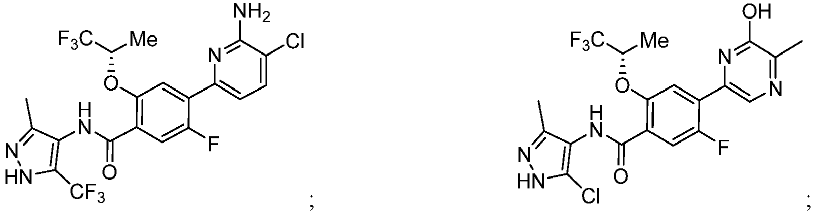 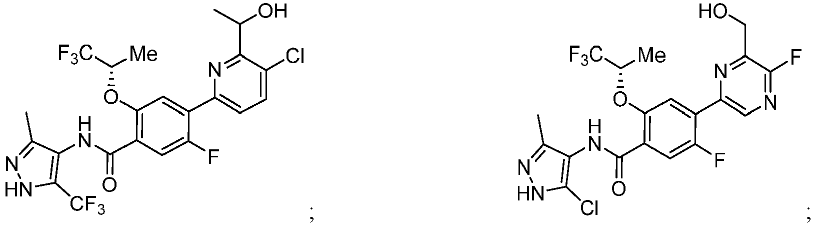 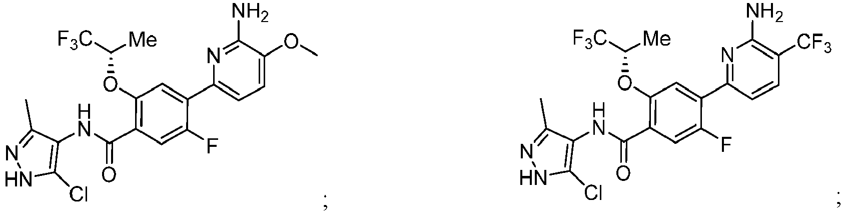 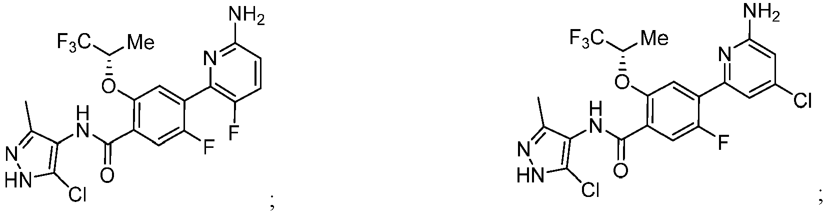 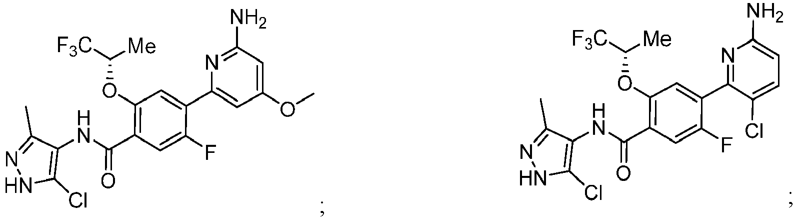 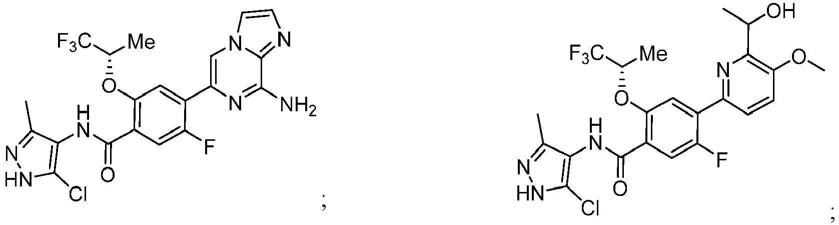 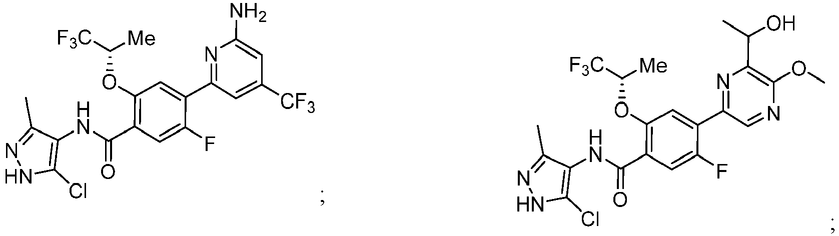 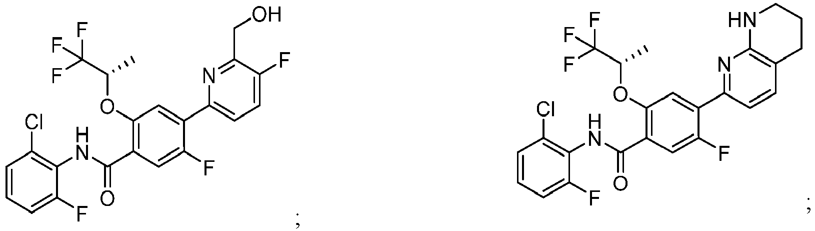 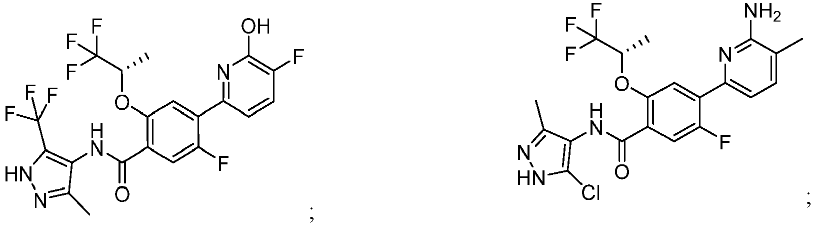 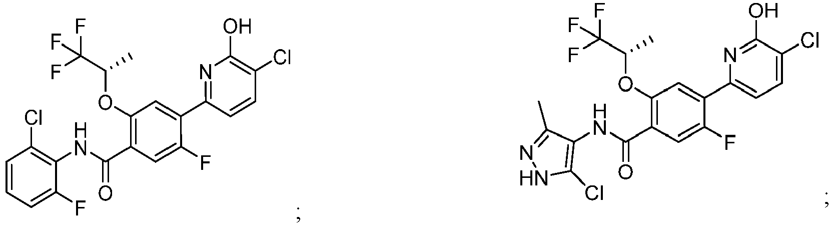 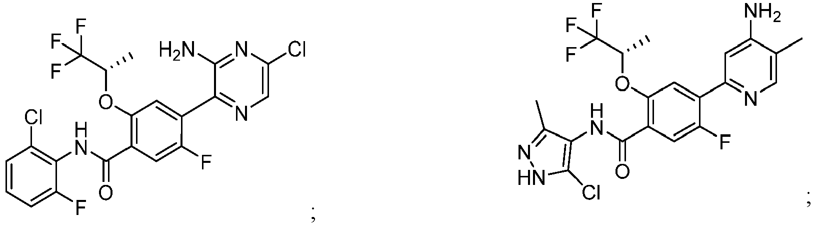 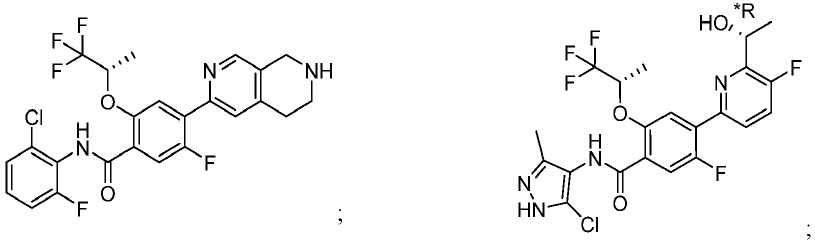 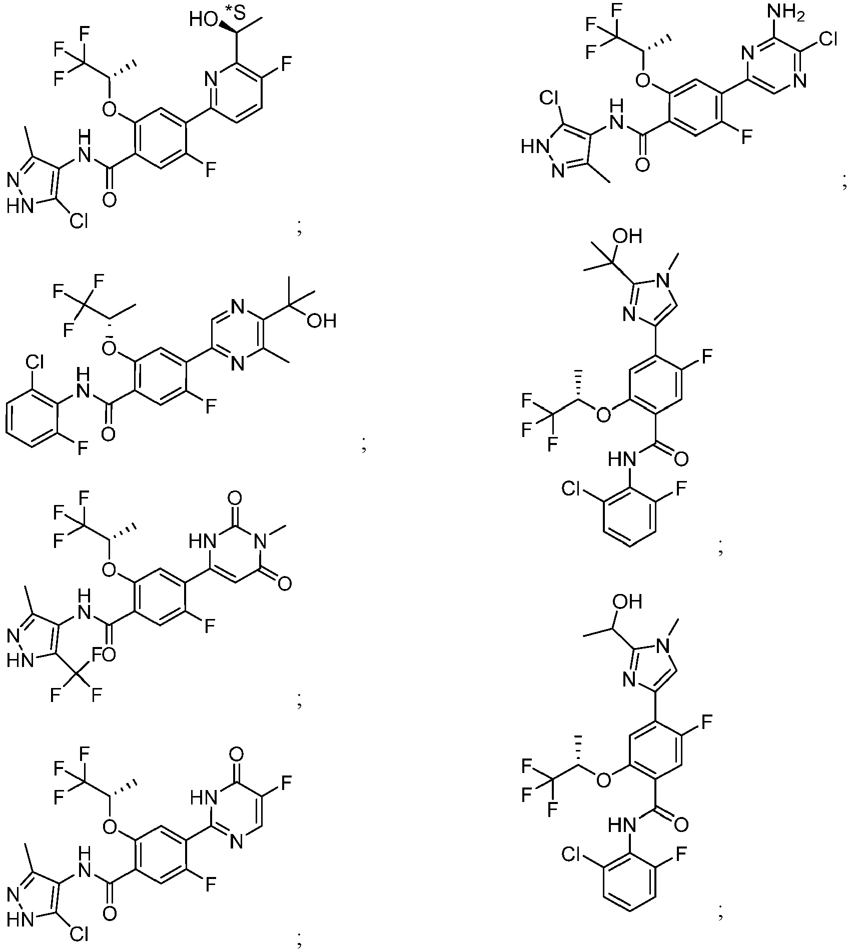 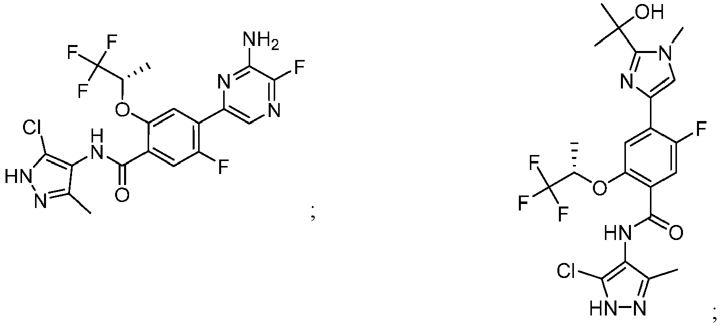 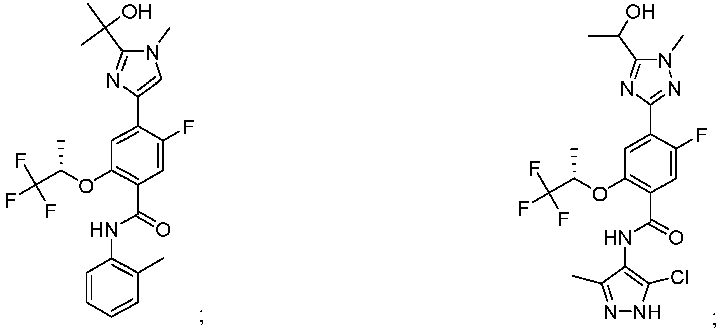 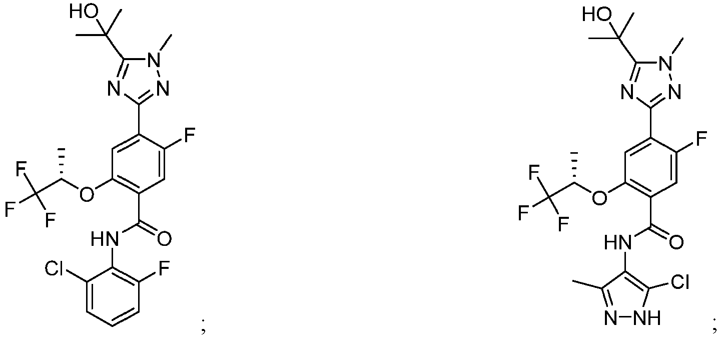 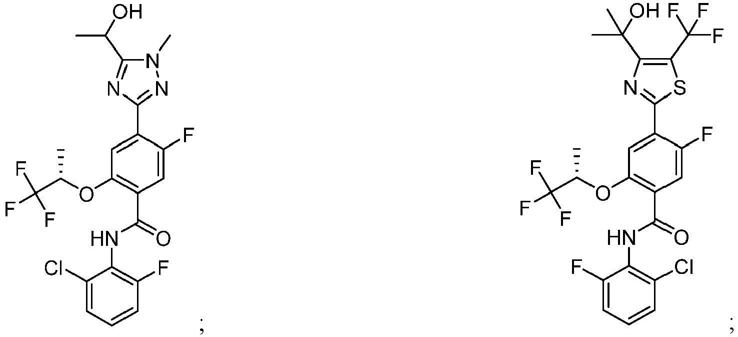 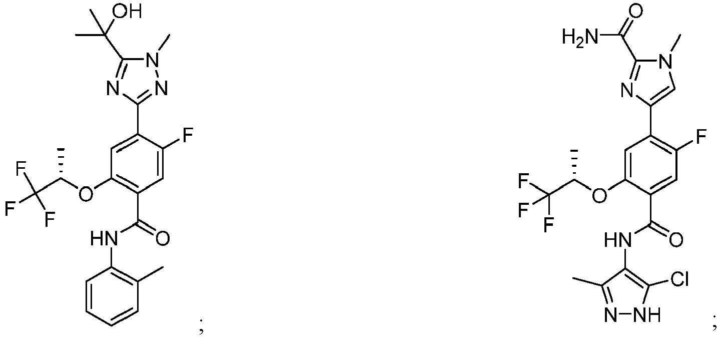 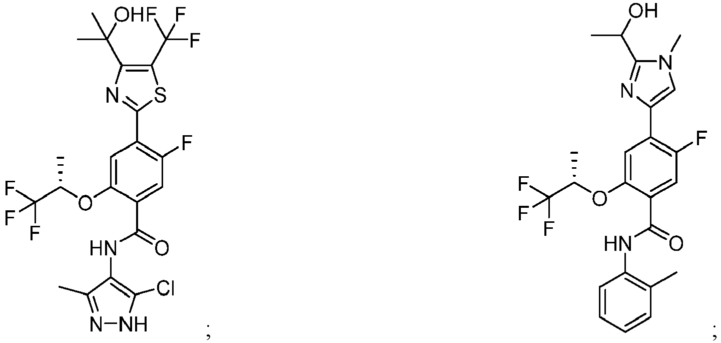 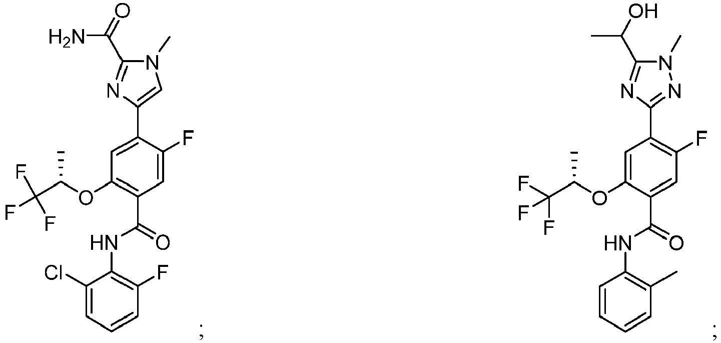 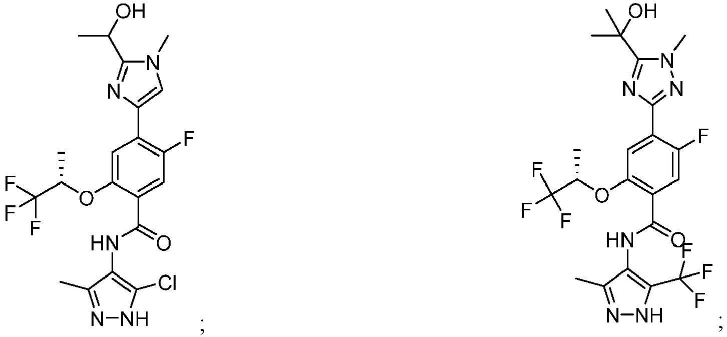 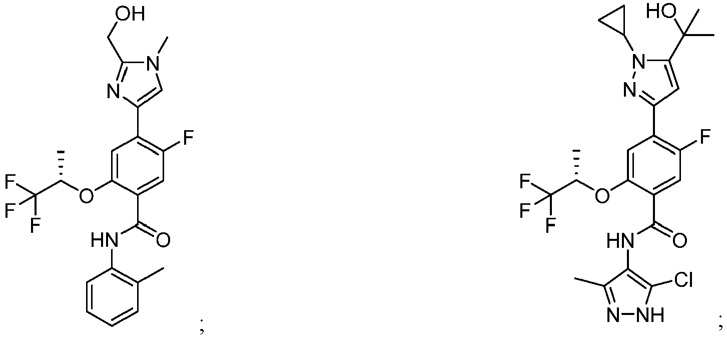 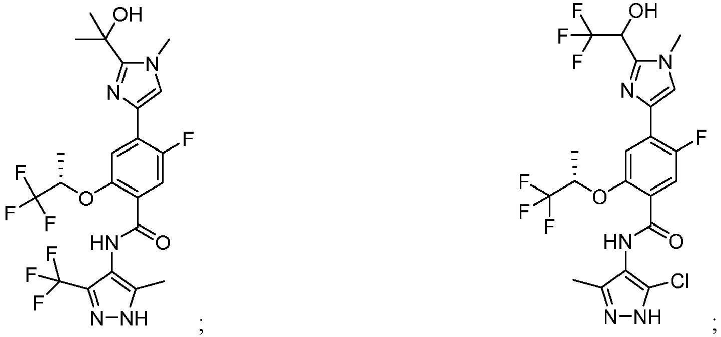 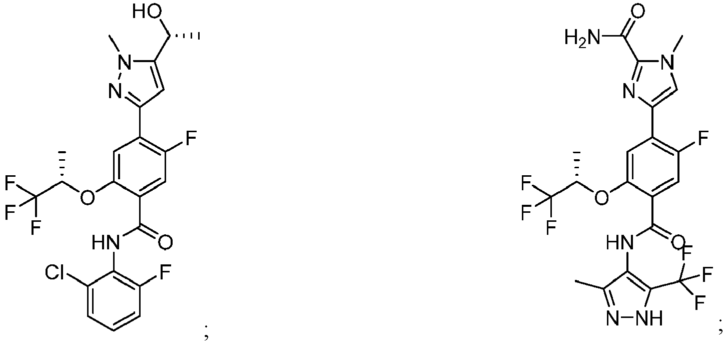 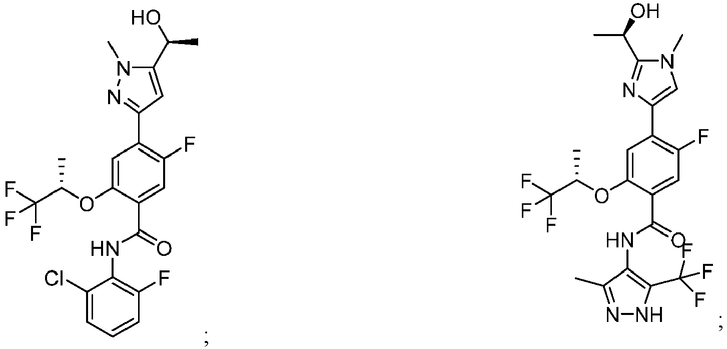 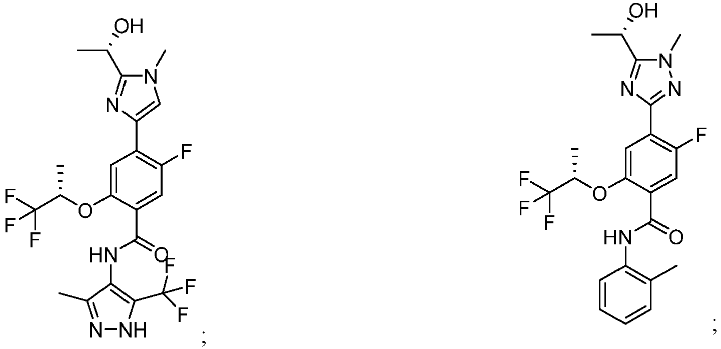 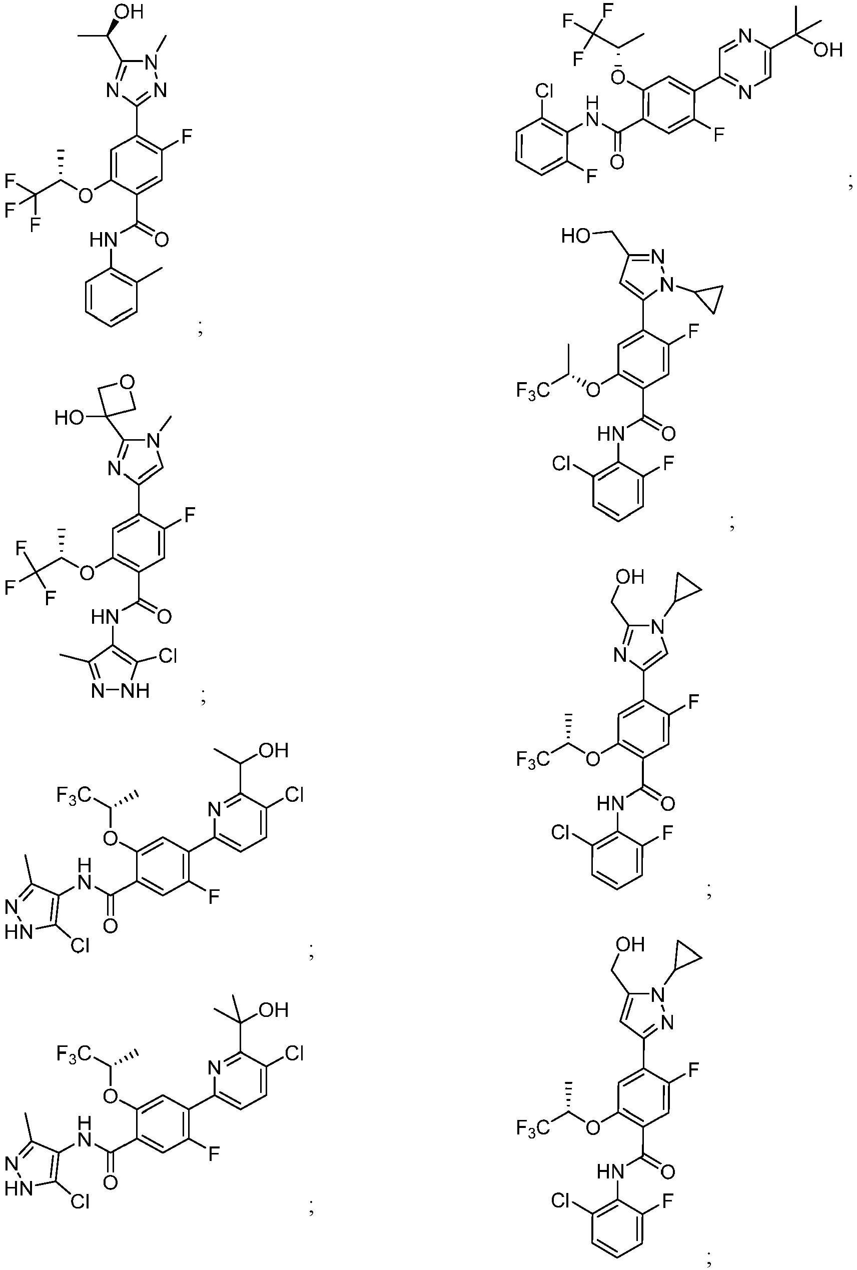 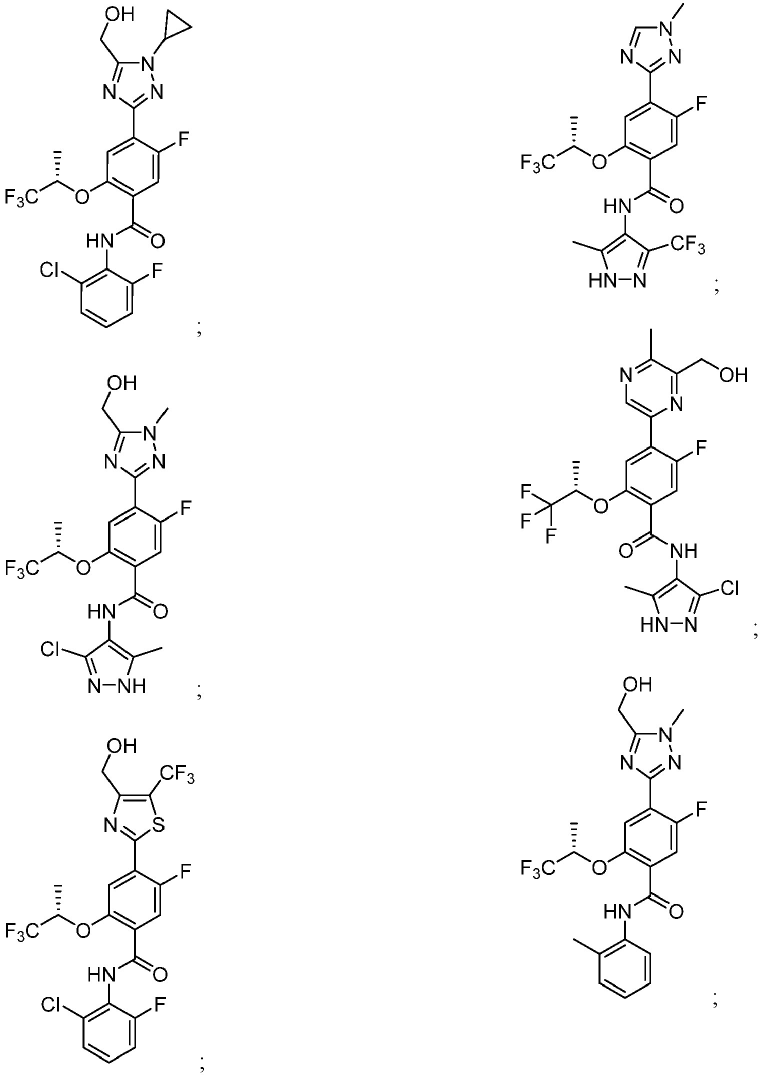 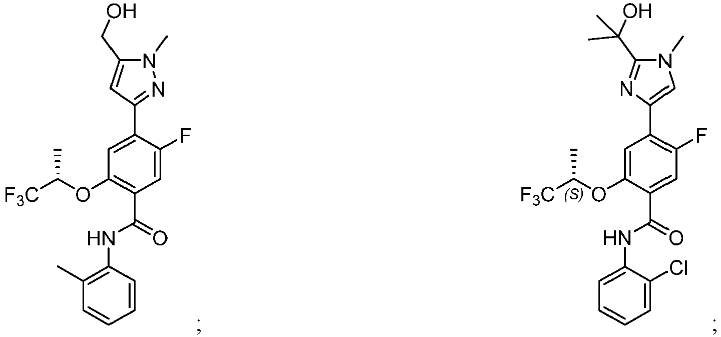 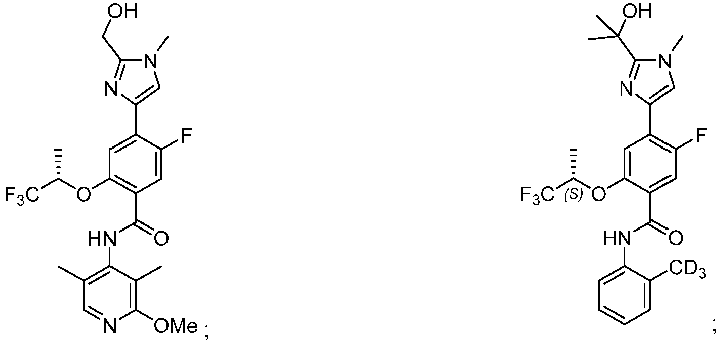 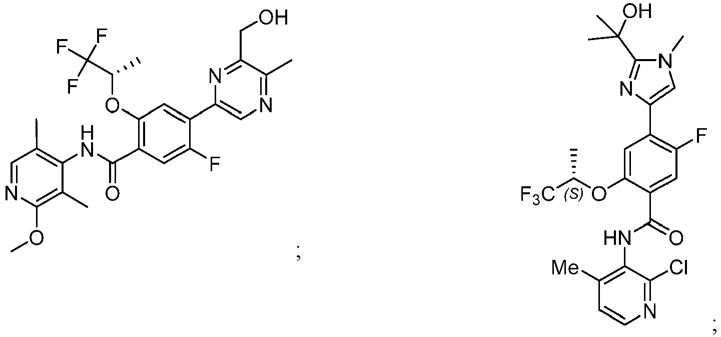 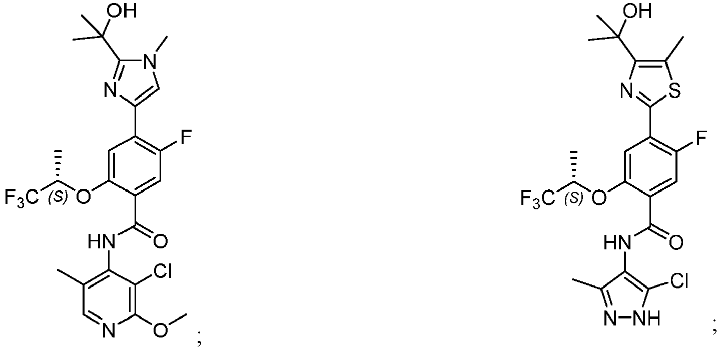 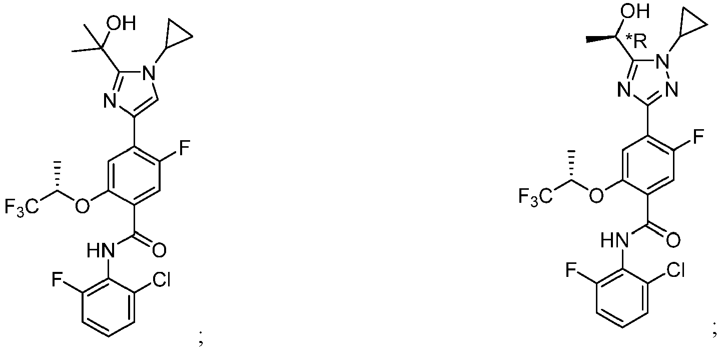 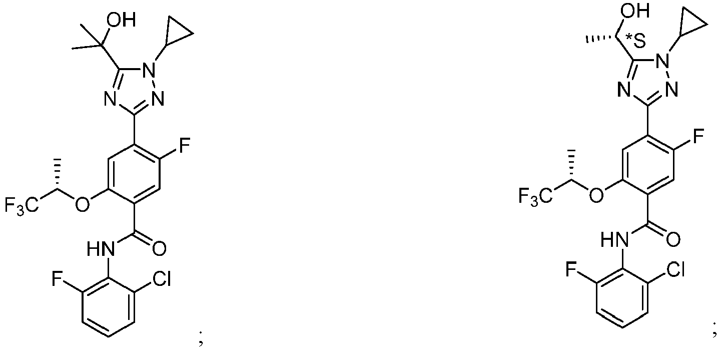 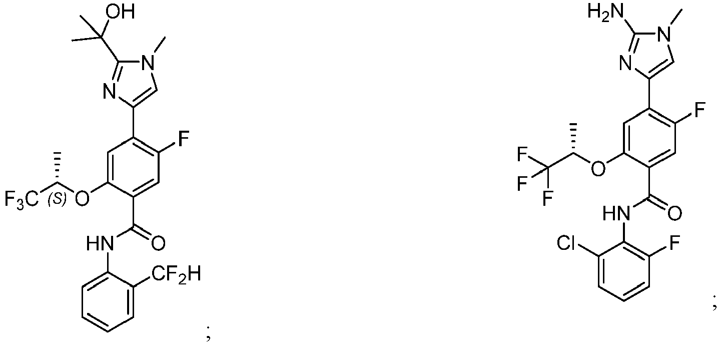 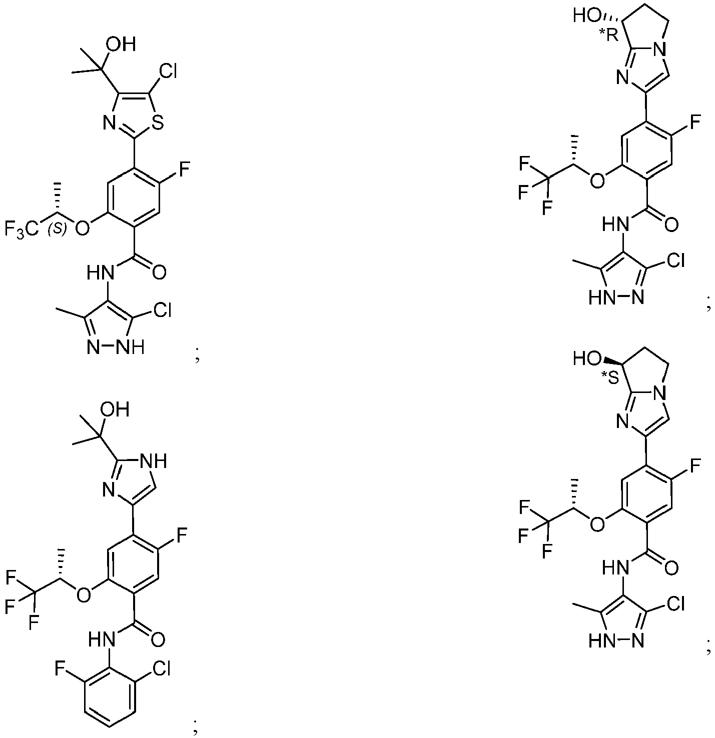 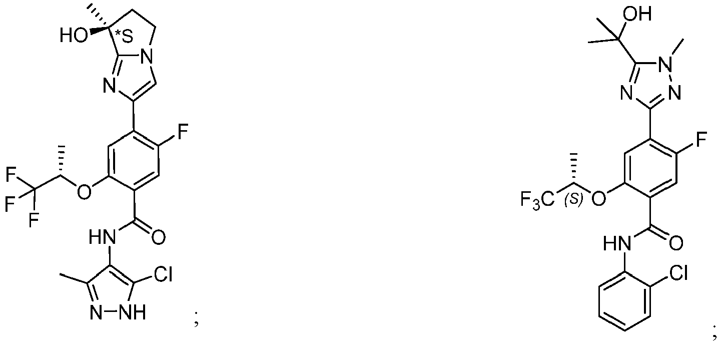 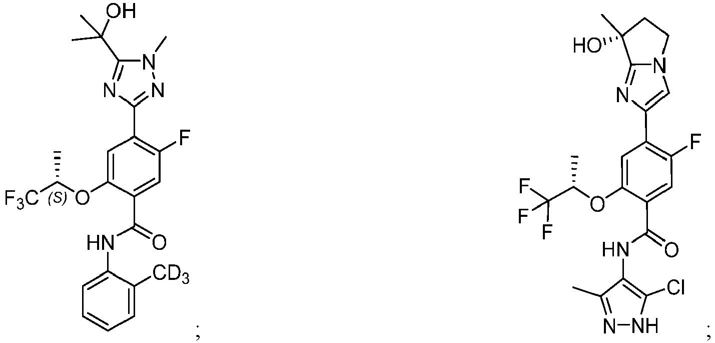 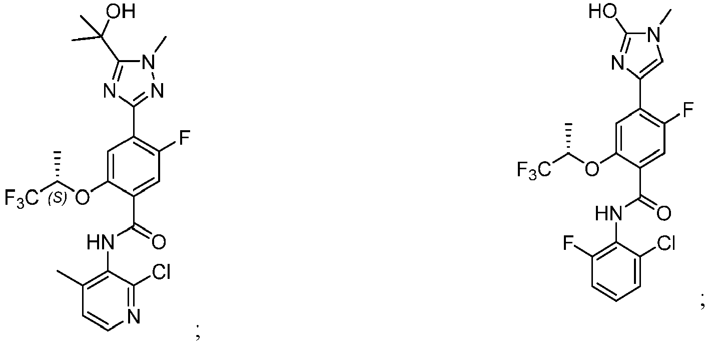 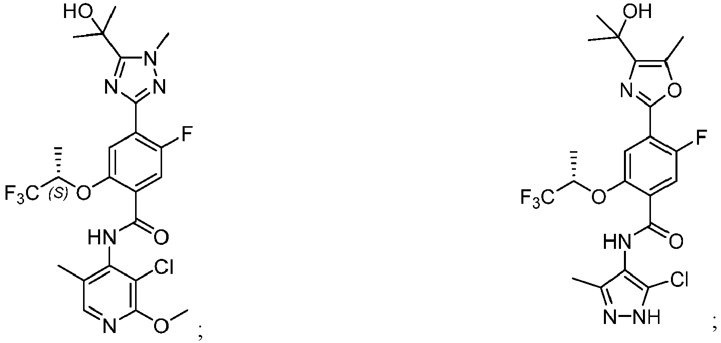 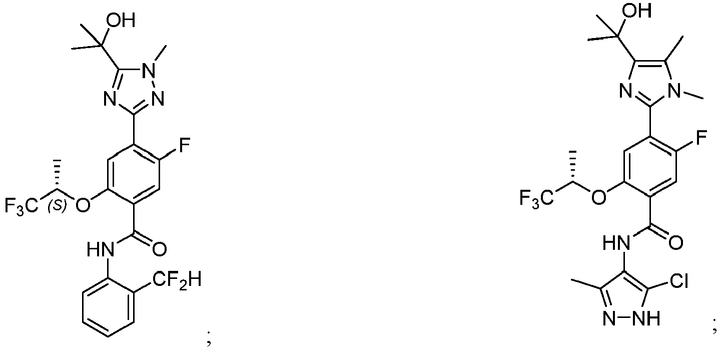 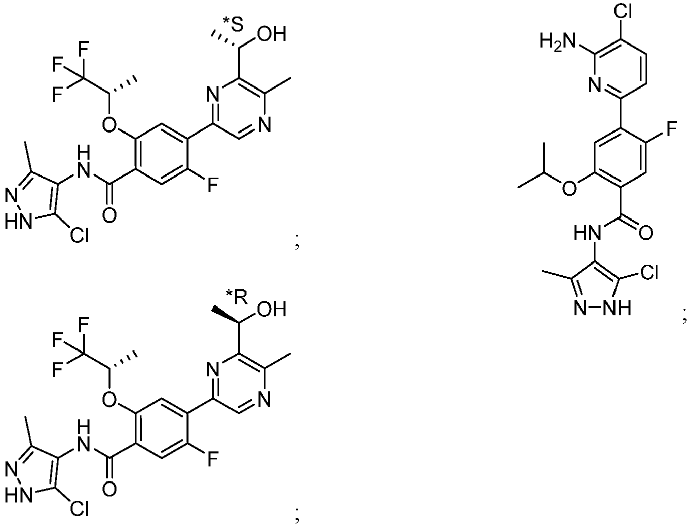 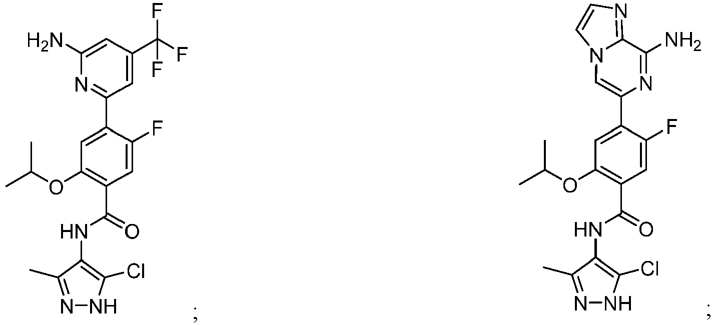 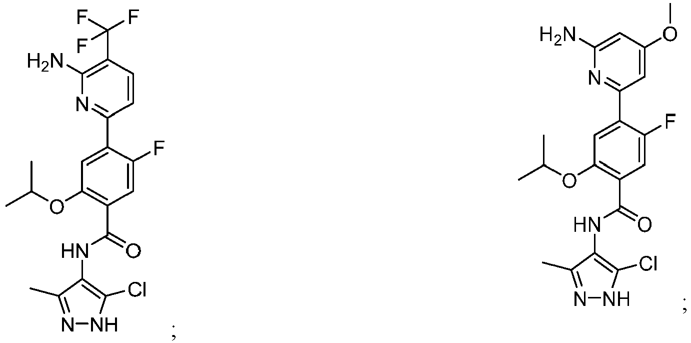 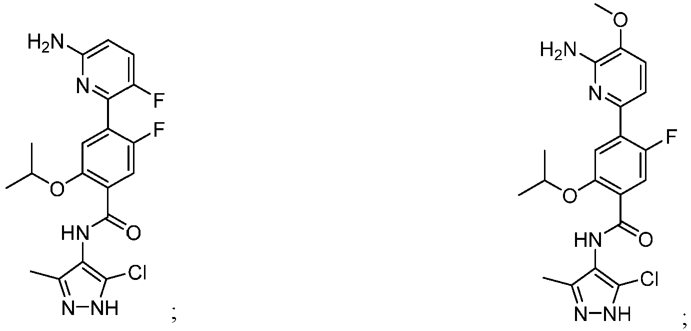 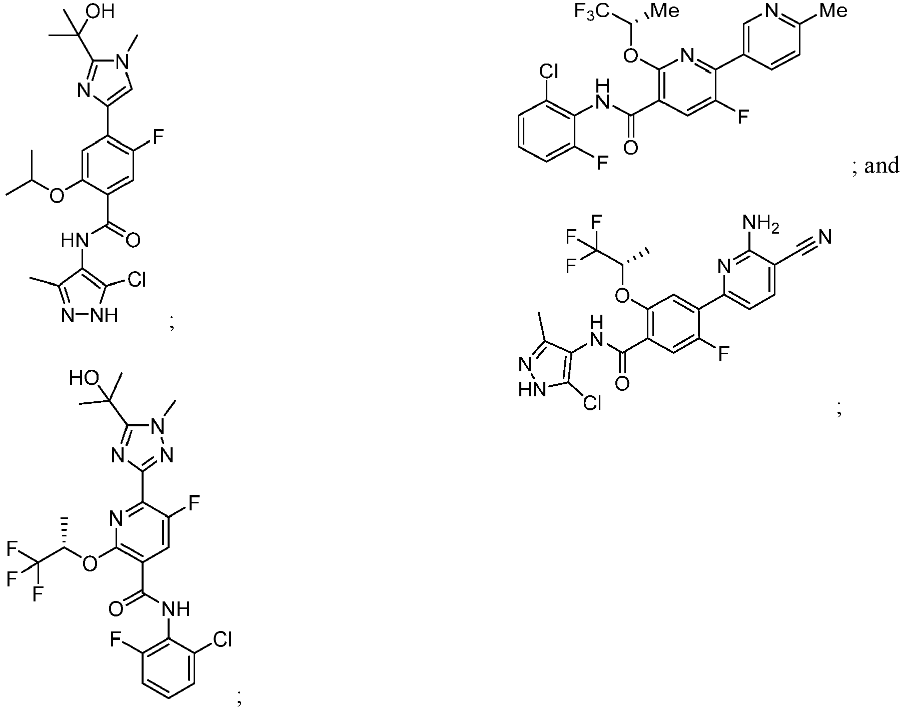 or a pharmaceutically acceptable salt, solvate, stereoisomer, isotopic variant, or N-oxide thereof; and at least one pharmaceutically acceptable excipient.

14. At least one compound or a pharmaceutically acceptable salt, solvate, stereoisomer, isotopic variant, or N-oxide thereof according to any of claims 1 to 11 for use in treating a subject suffering from or diagnosed with a disease, disorder, or medical condition comprising inhibiting or altering dihydroorotate oxygenase enzyme activity in the subject, wherein the disorder, disease or medical condition is selected from the group consisting of: inflammatory disorders, autoimmune disorders, and cancer.

15. At least one compound or a pharmaceutically acceptable salt, solvate, stereoisomer, isotopic variant, or N-oxide thereof for use according to claim 14, wherein the disorder, disease or medical condition is selected from the group consisting of: lymphomas, leukemias, carcinomas, and sarcomas.

16. At least one compound or a pharmaceutically acceptable salt, solvate, stereoisomer, isotopic variant, or N-oxide thereof for use according to claim 14, wherein the disorder, disease or medical condition is selected from the group consisting of: acute lymphoblastic leukemia, acute myeloid leukemia, (acute) T-cell leukemia, acute lymphoblastic leukemia, acute lymphocytic leukemia, acute monocytic leukemia, acute promyelocytic leukemia, bisphenotypic B myelomonocytic leukemia, chronic lymphocytic leukemia, chronic myelogenous leukemia, chronic myeloid leukemia, chronic myelomonocytic leukemia, large granular lymphocytic leukemia, plasma cell leukemia, and also myelodysplastic syndrome, which can develop into an acute myeloid leukemia.

17. At least one compound or a pharmaceutically acceptable salt, solvate, stereoisomer, isotopic variant, or N-oxide thereof for use according to claim 14, wherein the disorder, disease or medical condition is acute myeloid leukemia.

## Patentansprüche

1. Verbindung der Formel I 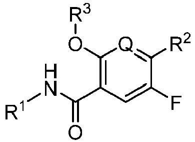 wobei
Q für CH oder N steht;
R¹ für 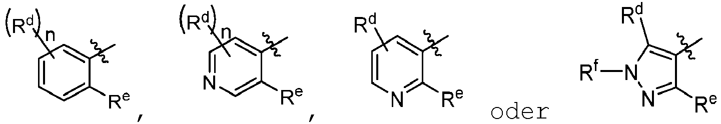 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C₍₁₋₃₎-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Imidazolyl; wobei das Imidazolyl gegebenenfalls durch bis zu drei Substituenten, die unabhängig aus der Gruppe umfassend 3-Hydroxyoxetanyl, -NH₂, -OH, -CN oder R^{a} ausgewählt sind, substituiert ist;
Triazolyl; wobei das Triazolyl gegebenenfalls durch eine oder zwei Gruppen, die unabhängig aus der Gruppe umfassend R^{a} ausgewählt sind, substituiert ist;
Pyrazinyl; wobei das Pyrazinyl gegebenenfalls durch bis zu drei Substituenten, die unabhängig aus der Gruppe umfassend -NHSO₂CH₃, -NHC(O)CH₃, -OCH₃, -OH, -NH₂, -CO₂H, -Cl, -F oder R^{a} ausgewählt sind, substituiert ist;
Pyrazinonyl; wobei das Pyrazinonyl gegebenenfalls durch eine oder zwei Gruppen, die unabhängig aus der Gruppe umfassend R^{a} ausgewählt sind, substituiert ist;
Thiazolyl; wobei das Thiazolyl gegebenenfalls durch einen oder zwei Substituenten, die unabhängig aus der Gruppe umfassend R^{a} ausgewählt sind, substituiert ist;
5,6,7,8-Tetrahydro-[1,2,4]triazolo[4,3-a]pyrazinyl; wobei das 5,6,7,8-Tetrahydro-[1,2,4]triazolo[4,3-a]pyrazinyl gegebenenfalls durch R^{a} substituiert ist;
5,6,7,8-Tetrahydro-[1,2,4]triazolo[1,5-a]pyrazinyl; wobei das 5,6,7,8-Tetrahydro-[1,2,4]triazolo[4,3-a]pyrazinyl gegebenenfalls durch R^{a} substituiert ist;
Pyridinyl; wobei das Pyridinyl gegebenenfalls durch einen oder zwei Substituenten, die unabhängig aus der Gruppe umfassend R^{a}, -Cl, -F, -CN, -NHC(O)CH₃, -NHSO₂CH₃, -CO₂H, -OCH₃, -OH oder -NH₂ ausgewählt sind, substituiert ist;
Pyridazinyl; wobei das Pyridazinyl gegebenenfalls durch eine oder zwei Gruppen, die unabhängig aus der Gruppe umfassend R^{a}, -Cl oder -CO₂H ausgewählt sind, substituiert ist;
4,5,6,7-Tetrahydrothiazolo[5,4-c]pyridinyl; wobei das 4,5,6,7-Tetrahydrothiazolo[5,4-c]pyridinyl gegebenenfalls durch R^{a} substituiert ist;
Pyrazolyl; wobei das Pyrazolyl gegebenenfalls durch einen oder zwei Substituenten, die unabhängig aus der Gruppe umfassend R^{a} ausgewählt sind, substituiert ist;
Oxazolyl; wobei das Oxazolyl gegebenenfalls durch einen oder zwei Substituenten, die unabhängig aus der Gruppe umfassend R^{a} ausgewählt sind, substituiert ist;
1,3,4-Oxadiazolyl; wobei das 1,3,4-Oxadiazolyl gegebenenfalls durch R^{a} substituiert ist;
5,6,7,8-Tetrahydroimidazo[1,5-a]pyridinyl; wobei das 5,6,7,8-Tetrahydroimidazo[1,5-a]pyridinyl gegebenenfalls durch R^{a} substituiert ist;
1,2,4-Thiadiazolyl; wobei das 1,2,4-Thiadiazolyl gegebenenfalls durch R^{a} substituiert ist;
5,6,7,8-Tetrahydro-1,7-naphthyridinyl; wobei das 5,6,7,8-Tetrahydro-1,7-naphthyridinyl gegebenenfalls durch R^{a} substituiert ist;
5,6,7,8-Tetrahydro-1,6-naphthyridinyl; wobei das 5,6,7,8-Tetrahydro-1,6-naphthyridinyl gegebenenfalls durch R^{a} substituiert ist;
5,6,7,8-Tetrahydro-1,8-naphthyridinyl; wobei das 5,6,7,8-Tetrahydro-1,8-naphthyridinyl gegebenenfalls durch R^{a} substituiert ist;
1,2,3,4-Tetrahydro-2,7-naphthyridinyl; wobei das 1,2,3,4-Tetrahydro-2,7-naphthyridinyl gegebenenfalls durch R^{a} substituiert ist;
2H-Pyrido[3,2-b] [1,4]oxazin-3(4*H*)-onyl, wobei das *2H-*Pyrido[3,2-b][1,4]oxazin-3(4H)-onyl gegebenenfalls durch R^{a} substituiert ist;
2,3-Dihydro-1*H*-pyrrolo[2,3-b]pyridinyl; wobei das 2,3-Dihydro-1*H*-pyrrolo[2,3-*b*]pyridinyl gegebenenfalls durch R^{a} substituiert ist;
Imidazo[1,2-a]pyrazinyl, wobei das Imidazo[1,2-a]pyrazinyl gegebenenfalls durch -NH₂ oder R^{a} substituiert ist;
2,6-Dioxo-1,2,3,6-tetrahydropyrimidinyl, wobei das 2,6-Dioxo-1,2,3,6-tetrahydropyrimidinyl gegebenenfalls durch R^{a} substituiert ist;
6-Oxo-1,6-dihydropyrimidinyl, wobei das 6-Oxo-1,6-dihydropyrimidinyl gegebenenfalls durch Fluor oder R^{a} substituiertes;
6,7-Dihydro-5*H*-pyrrolo[1,2-a]imidazolyl, wobei das 6,7-Dihydro-5*H*-pyrrolo[1,2-a]imidazolyl gegebenenfalls durch einen oder zwei Substituenten, die aus der Gruppe bestehend aus R^{a} und -OH ausgewählt sind, substituiert ist;
R^{a} für -C(O)NH₂, Cyclopropyl oder -C₍₁₋₃₎-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -OH, -CH₂OH oder bis zu zwei Fluoratome substituiert ist;
R³ für -CHR^{b}R^{c} steht; wobei
R^{b} für -C₍₃₋₆₎-Cycloalkyl oder -C₍₁₋₆₎-Alkyl; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammengenommen -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das -C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

2. Verbindung nach Anspruch 1, bei der es sich um Formel II handelt: 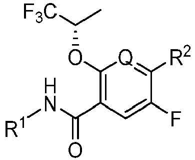 wobei
Q für CH oder N steht;
R¹ für 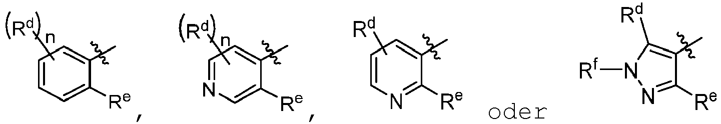 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist oder das -C₍₁₋₃₎-Alkyl gegebenenfalls durch bis zu drei Fluoratome substituiert ist;
R^{e} aus der Gruppe bestehend aus: -Cl, -F, -CD₃, -C₍₁₋₃₎-Alkyl und Cyclopropyl ausgewählt ist; wobei das -C₍₁₋₃₎-Alkyl gegebenenfalls durch -OH, -OCH₃, -SCH₃ oder -OCF₃ substituiert ist oder das -C₍₁₋₃₎-Alkyl gegebenenfalls durch bis zu drei Fluoratome substituiert ist;
R^{f} für -H steht; und
n für 1 oder 2 steht;
R² für Folgendes steht:
Imidazolyl; wobei das Imidazolyl gegebenenfalls durch bis zu drei Substituenten, die unabhängig aus der Gruppe umfassend 3-Hydroxyoxetanyl, -NH₂, -OH, -CN oder R^{a} ausgewählt sind, substituiert ist;
Triazolyl; wobei das Triazolyl gegebenenfalls durch eine oder zwei Gruppen, die unabhängig aus der Gruppe umfassend R^{a} ausgewählt sind, substituiert ist;
Pyrazinyl; wobei das Pyrazinyl gegebenenfalls durch bis zu drei Substituenten, die unabhängig aus der Gruppe umfassend -NHSO₂CH₃, -NHC(O)CH₃, -OCH₃, -OH, -NH₂, -CO₂H, -Cl, -F oder R^{a} ausgewählt sind, substituiert ist;
Pyrazinonyl; wobei das Pyrazinonyl gegebenenfalls durch eine oder zwei Gruppen, die unabhängig aus der Gruppe umfassend R^{a} ausgewählt sind, substituiert ist;
Thiazolyl; wobei das Thiazolyl gegebenenfalls durch einen oder zwei Substituenten, die unabhängig aus der Gruppe umfassend R^{a} ausgewählt sind, substituiert ist;
5,6,7,8-Tetrahydro-[1,2,4]triazolo[4,3-a]pyrazinyl;
5,6,7,8-Tetrahydro-[1,2,4]triazolo[1,5-a]pyrazinyl;
Pyridinyl; wobei das Pyridinyl gegebenenfalls durch einen oder zwei Substituenten, die unabhängig aus der Gruppe umfassend R^{a}, -Cl, -F, -CN, -NHC(O)CH₃, -NHSO₂CH₃, -CO₂H, -OCH₃, -OH oder -NH₂ ausgewählt sind, substituiert ist;
Pyridazinyl; wobei das Pyridazinyl gegebenenfalls durch eine oder zwei Gruppen, die unabhängig aus der Gruppe umfassend R^{a}, -Cl oder -CO₂H ausgewählt sind, substituiert ist;
Pyrazolyl; wobei das Pyrazolyl gegebenenfalls durch einen oder zwei Substituenten, die unabhängig aus der Gruppe umfassend R^{a} ausgewählt sind, substituiert ist;
Oxazolyl; wobei das Oxazolyl gegebenenfalls durch einen oder zwei Substituenten, die unabhängig aus der Gruppe umfassend R^{a} ausgewählt sind, substituiert ist;
1,3,4-Oxadiazolyl; wobei das 1,3,4-Oxadiazolyl durch Cyclopropyl substituiert ist;
5,6,7,8-Tetrahydroimidazo[1,5-a]pyridinyl; wobei das 5,6,7,8-Tetrahydroimidazo[1,5-a]pyridinyl gegebenenfalls durch R^{a} substituiert ist;
1,2,4-Thiadiazolyl; wobei das 1,2,4-Thiadiazolyl durch Cyclopropyl substituiert ist;
5,6,7,8-Tetrahydro-1,7-naphthyridinyl;
5,6,7,8-Tetrahydro-1,6-naphthyridinyl;
5,6,7,8-Tetrahydro-1,8-naphthyridinyl;
1,2,3,4-Tetrahydro-2,7-naphthyridinyl;
2*H*-Pyrido[3,2-*b*][1,4]oxazin-3(4*H*)-onyl, wobei das *2H-*Pyrido[3,2-*b*][1,4]oxazin-3(4*H*)-onyl gegebenenfalls durch R^{a} substituiert ist;
2,3-Dihydro-1*H*-pyrrolo[2,3-*b*]pyridinyl;
Imidazo[1,2-a]pyrazinyl, wobei das Imidazo[1,2-a]pyrazinyl durch -NH₂ substituiert ist;
2,6-Dioxo-1,2,3,6-tetrahydropyrimidinyl, wobei das 2,6-Dioxo-1,2,3,6-tetrahydropyrimidinyl gegebenenfalls durch R^{a} substituiert ist;
6-Oxo-1,6-dihydropyrimidinyl, wobei das 6-Oxo-1,6-dihydropyrimidinyl durch Fluor substituiert ist;
6,7-Dihydro-5*H*-pyrrolo[1,2-*a*]imidazolyl, wobei das 6,7-Dihydro-5*H*-pyrrolo[1,2-*a*]imidazolyl gegebenenfalls durch einen oder zwei Substituenten, die aus der Gruppe bestehend aus R^{a} und -OH ausgewählt sind, substituiert ist;
R^{a} für -C(O)NH₂, Cyclopropyl oder -C₍₁₋₃₎-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

3. Verbindung nach Anspruch 1 oder Anspruch 2, wobei:
(a) Q für CH oder N steht;
R¹ für 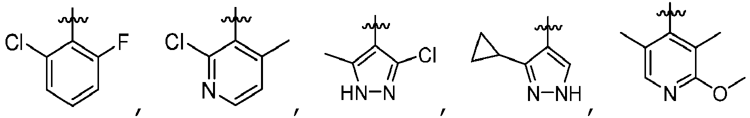 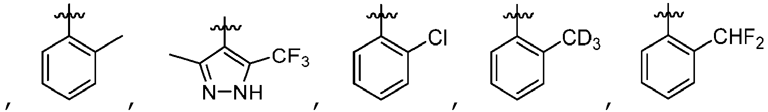 oder 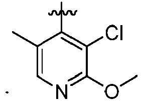 steht;
R² für Folgendes steht:
Imidazolyl; wobei das Imidazolyl gegebenenfalls durch bis zu drei Substituenten, die unabhängig aus der Gruppe umfassend 3-Hydroxyoxetanyl, -NH₂, -OH, -CN oder R^{a} ausgewählt sind, substituiert ist;
Triazolyl; wobei das Triazolyl gegebenenfalls durch eine oder zwei Gruppen, die unabhängig aus der Gruppe umfassend R^{a} ausgewählt sind, substituiert ist;
Pyrazinyl; wobei das Pyrazinyl gegebenenfalls durch bis zu drei Substituenten, die unabhängig aus der Gruppe umfassend -OCH₃, -OH, -NH₂, -CO₂H, -Cl, -F oder R^{a} ausgewählt sind, substituiert ist;
Pyrazinonyl; wobei das Pyrazinonyl gegebenenfalls durch eine oder zwei Gruppen, die unabhängig aus der Gruppe umfassend R^{a} ausgewählt sind, substituiert ist;
Thiazolyl; wobei das Thiazolyl gegebenenfalls durch zwei Substituenten, die unabhängig aus der Gruppe umfassend R^{a} ausgewählt sind, substituiert ist;
5,6,7,8-Tetrahydro-[1,2,4]triazolo[4,3-a]pyrazinyl;
5,6,7,8-Tetrahydro-[1,2,4]triazolo[1,5-a]pyrazinyl;
Pyridinyl; wobei das Pyridinyl gegebenenfalls durch einen oder zwei Substituenten, die unabhängig aus der Gruppe umfassend R^{a}, -Cl, -F, -CN, -NHC(O)CH₃, -NHSO₂CH₃, -CO₂H, -OCH₃, -OH oder -NH₂ ausgewählt sind, substituiert ist;
Pyridazinyl; wobei das Pyridazinyl gegebenenfalls durch R^{a} substituiert ist;
4,5,6,7-Tetrahydrothiazolo[5,4-c]pyridinyl;
Pyrazolyl; wobei das Pyrazolyl gegebenenfalls durch einen oder zwei Substituenten, die unabhängig aus der Gruppe umfassend R^{a} ausgewählt sind, substituiert ist;
Oxazolyl; wobei das Oxazolyl gegebenenfalls durch bis zu zwei Substituenten, die unabhängig aus R^{a} ausgewählt sind, substituiert ist;
1,3,4-Oxadiazolyl; wobei das 1,3,4-Oxadiazolyl durch Cyclopropyl substituiert ist;
5,6,7,8-Tetrahydroimidazo[1,5-*a*]pyridinyl, wobei das 5,6,7,8-tetrahydroimidazo[1,5-*a*]pyridinyl gegebenenfalls durch R^{a} substituiert ist;
1,2,4-Thiadiazolyl; wobei das 1,2,4-Thiadiazolyl durch Cyclopropyl substituiert ist;
5,6,7,8-Tetrahydro-1,7-naphthyridinyl;
5,6,7,8-Tetrahydro-1,6-naphthyridinyl;
5,6,7,8-Tetrahydro-1,8-naphthyridinyl;
1,2,3,4-Tetrahydro-2,7-naphthyridinyl;
2*H*-Pyrido[3,2-*b*][1,4]oxazin-3(4*H*)-onyl, wobei das *2H-*Pyrido[3,2-*b*][1,4]oxazin-3(4*H*)-onyl gegebenenfalls durch -CH₃ substituiert ist;
2,3-Dihydro-1*H*-pyrrolo[2,3-*b*]pyridinyl;
Imidazo[1,2-a]pyrazinyl, wobei das Imidazo[1,2-a]pyrazinyl durch -NH₂ substituiert ist;
2,6-Dioxo-1,2,3,6-tetrahydropyrimidinyl, wobei das 2,6-Dioxo-1,2,3,6-tetrahydropyrimidinyl durch -CH₃ substituiert ist;
6-Oxo-1,6-dihydropyrimidinyl, wobei das 6-Oxo-1,6-dihydropyrimidinyl durch Fluor substituiert ist;
6,7-Dihydro-5*H*-pyrrolo[1,2-*a*]imidazolyl, wobei das 6,7-Dihydro-5*H*-pyrrolo[1,2-*a*]imidazolyl gegebenenfalls durch einen oder zwei Substituenten, die aus der Gruppe bestehend aus -CH₃ und -OH ausgewählt sind, substituiert ist;
R^{a} für -C(O)NH₂, Cyclopropyl oder -C₍₁₋₃₎-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(b) Q für CH oder N steht;
R¹ für 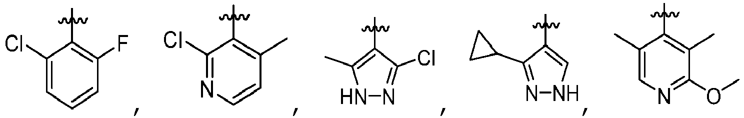 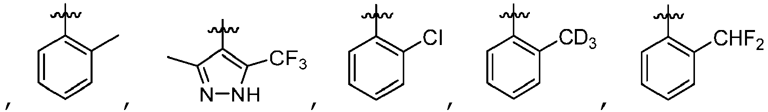 oder 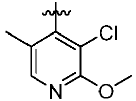 steht;
R² für Folgendes steht:
Imidazolyl; wobei das Imidazolyl durch einen, zwei oder drei Substituenten, die unabhängig aus der Gruppe umfassend -C₍₁₋₃₎-Alkyl, 3-Hydroxyoxetanyl, Cyclopropyl, - C(O)NH₂, -NH₂, -OH oder -CN ausgewählt sind, substituiert ist; wobei das -C₍₁₋₃₎-Alkyl gegebenenfalls durch -OH substituiert ist und zusätzlich gegebenenfalls durch bis zu drei Fluoratome substituiert ist;
Triazolyl; wobei das Triazolyl durch bis zu zwei Gruppen, die unabhängig aus der Gruppe umfassend - C(O)NH₂, -CF₃, Cyclopropyl oder -C₍₁₋₃₎-Alkyl ausgewählt sind, substituiert ist, wobei das -C₍₁₋₃₎-Alkyl gegebenenfalls durch substituiert ist -OH;
Pyrazinyl; wobei das Pyrazinyl gegebenenfalls durch bis zu drei Substituenten, die unabhängig aus der Gruppe umfassend -C₍₁₋₃₎-Alkyl, -OCH₃, -OH, -NH₂, Cyclopropyl, - C(O)NH₂, -CO₂H, -Cl, -F oder -CF₃ ausgewählt sind, substituiert ist; wobei das Cyclopropyl gegebenenfalls durch -CH₂OH substituiert ist und wobei das -C₍₁₋₃₎-Alkyl gegebenenfalls durch -OH substituiert ist;
Pyrazinonyl; wobei das Pyrazinonyl gegebenenfalls durch eine oder zwei -C₍₁₋₃₎-Alkylgruppen substituiert ist;
Thiazolyl; wobei das Thiazolyl durch zwei Substituenten, die unabhängig aus der Gruppe umfassend - C₍₁₋₃₎-Alkyl, Cyclopropyl, -Cl oder -C(O)NH₂ ausgewählt sind, substituiert ist; wobei das -C₍₁₋₃₎-Alkyl gegebenenfalls durch -OH oder bis zu drei Fluoratome substituiert ist;
5,6,7,8-Tetrahydro-[1,2,4]triazolo[4,3-a]pyrazinyl;
5,6,7,8-Tetrahydro-[1,2,4]triazolo[1,5-*a*]pyrazinyl;
Pyridinyl; wobei das Pyridinyl durch einen oder zwei Substituenten, die unabhängig aus der Gruppe umfassend - CONH₂, -C₍₁₋₃₎-Alkyl, Cyclopropyl, -Cl, -F, -CN, - NHC(O)CH₃, -NHSO₂CH₃, -CO₂H, -OCH₃, -OH oder -NH₂ ausgewählt sind, substituiert ist; wobei das -C₍₁₋₃₎-Alkyl gegebenenfalls durch bis zu drei Fluoratome substituiert ist oder das gegebenenfalls durch -OH substituiert ist und zusätzlich gegebenenfalls durch bis zu drei Fluoratome substituiert ist;
Pyridazinyl; wobei das Pyridazinyl durch -C₍₁₋₃₎-Alkyl substituiert ist; wobei das -C₍₁₋₃₎-Alkyl gegebenenfalls durch -OH substituiert ist;
4,5,6,7-Tetrahydrothiazolo[5,4-*c*]pyridinyl;
Pyrazolyl; wobei das Pyrazolyl durch zwei Substituenten, die unabhängig aus der Gruppe umfassend - C₍₁₋₃₎-Alkyl und Cyclopropyl ausgewählt sind substituiert ist; wobei das -C₍₁₋₃₎-Alkyl gegebenenfalls durch -OH substituiert ist;
Oxazolyl; wobei das Oxazolyl gegebenenfalls durch zwei Substituenten, die unabhängig aus -C₍₁₋₃₎-Alkyl ausgewählt sind, substituiert ist; wobei das -C₍₁₋₃₎-Alkyl gegebenenfalls durch -OH substituiert ist;
1,3,4-Oxadiazolyl; wobei das 1,3,4-Oxadiazolyl durch Cyclopropyl substituiert ist;
5,6,7,8-Tetrahydroimidazo[1,5-a]pyridinyl wobei das 5,6,7,8-Tetrahydroimidazo[1,5-a]pyridinyl durch -C₍₁₋₃₎-Alkyl substituiert ist; wobei das -C₍₁₋₃₎-Alkyl gegebenenfalls durch -OH substituiert ist;
1,2,4-Thiadiazolyl; wobei das 1,2,4-Thiadiazolyl durch Cyclopropyl substituiert ist;
5,6,7,8-Tetrahydro-1,7-naphthyridinyl;
5,6,7,8-Tetrahydro-1,6-naphthyridinyl;
5,6,7,8-Tetrahydro-1,8-naphthyridinyl;
1,2,3,4-Tetrahydro-2,7-naphthyridinyl;
2*H*-Pyrido[3,2-*b*][1,4]oxazin-3(4*H*)-onyl, wobei das *2H-*pyrido[3,2-*b*][1,4]oxazin-3(4*H*)-onyl gegebenenfalls durch -CH₃ substituiert ist;
2,3-Dihydro-1*H*-pyrrolo[2,3-*b*]pyridinyl;
Imidazo[1,2-*a*]pyrazinyl, wobei das Imidazo[1,2-a]pyrazinyl durch -NH₂ substituiert ist;
2,6-Dioxo-1,2,3,6-tetrahydropyrimidinyl, wobei das 2,6-Dioxo-1,2,3,6-tetrahydropyrimidinyl durch -CH₃ substituiert ist;
6-Oxo-1,6-dihydropyrimidinyl, wobei das 6-Oxo-1,6-dihydropyrimidinyl durch Fluor substituiert ist;
6,7-Dihydro-5*H*-pyrrolo[1,2-*a*]imidazolyl, wobei das 6,7-Dihydro-5*H*-pyrrolo[1,2-*a*]imidazolyl gegebenenfalls durch einen oder zwei Substituenten, die aus der Gruppe bestehend aus -CH₃ und -OH ausgewählt sind, substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

4. Verbindung nach einem der Ansprüche 1-3, wobei
Q für CH oder N steht;
R¹ für 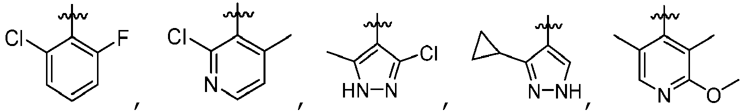 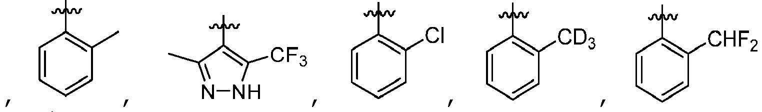 oder 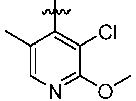 steht;
R² für Folgendes steht:
Imidazolyl; wobei das Imidazolyl durch einen, zwei oder drei Substituenten, die unabhängig aus der Gruppe umfassend -C₍₁₋₃₎-Alkyl, 3-Hydroxyoxetanyl, Cyclopropyl, - C(O)NH₂, -NH₂, -OH oder -CN ausgewählt sind, substituiert ist; wobei das -C₍₁₋₃₎-Alkyl gegebenenfalls durch -OH substituiert ist und zusätzlich gegebenenfalls durch bis zu drei Fluoratome substituiert ist;
Triazolyl; wobei das Triazolyl durch bis zu zwei Gruppen, die unabhängig aus der Gruppe umfassend - C(O)NH₂, -CF₃, Cyclopropyl oder -C₍₁₋₃₎-Alkyl ausgewählt sind, substituiert ist, wobei das -C₍₁₋₃₎-Alkyl gegebenenfalls durch -OH substituiert ist;
Pyrazinyl; wobei das Pyridazinyl gegebenenfalls durch bis zu drei Substituenten, die unabhängig aus der Gruppe umfassend -C₍₁₋₃₎-Alkyl, -OCH₃, -OH, -NH₂, Cyclopropyl, - C(O)NH₂, -CO₂H, -Cl, -F oder -CF₃ ausgewählt sind, substituiert ist, wobei das Cyclopropyl gegebenenfalls durch -CH₂OH substituiert ist und wobei das -C₍₁₋₃₎-Alkyl gegebenenfalls durch -OH substituiert ist;
Pyrazinonyl; wobei das Pyrazinonyl durch -CH₃ und - CH₂CH₂CH₃ substituiert ist;
Thiazolyl; wobei das Thiazolyl durch zwei Substituenten, die unabhängig aus der Gruppe umfassend - C₍₁₋₃₎-Alkyl, Cyclopropyl, -Cl oder -C(O)NH₂ ausgewählt sind, substituiert ist; wobei das -C₍₁₋₃₎-Alkyl gegebenenfalls durch -OH oder bis zu drei Fluoratome substituiert ist;
5,6,7,8-Tetrahydro-[1,2,4]triazolo[4,3-a]pyrazinyl;
5,6,7,8-Tetrahydro-[1,2,4]triazolo[1,5-a]pyrazinyl;
Pyridinyl; wobei das Pyridinyl durch einen oder zwei Substituenten, die unabhängig aus der Gruppe umfassend - CONH₂, -CF₃, -C₍₁₋₃₎-Alkyl, Cyclopropyl, -Cl, -F, -CN, - NHC(O)CH₃, -NHSO₂CH₃, -CO₂H, -OCH₃, -OH oder -NH₂ ausgewählt sind, substituiert ist, wobei das -C₍₁₋₃₎-Alkyl gegebenenfalls durch -OH substituiert ist und zusätzlich gegebenenfalls durch bis zu drei Fluoratome substituiert ist;
Pyridazinyl; wobei das Pyridazinyl durch -CH₃ substituiert ist;
4,5,6,7-Tetrahydrothiazolo[5,4-c]pyridinyl;
Pyrazolyl; wobei das Pyrazolyl durch zwei Substituenten, die unabhängig aus der Gruppe umfassend - C₍₁₋₃₎-Alkyl und Cyclopropyl ausgewählt sind, substituiert ist, wobei das -C₍₁₋₃₎-Alkyl gegebenenfalls durch -OH substituiert ist;
Oxazolyl; wobei das Oxazolyl durch zwei Substituenten, die unabhängig aus -C₍₁₋₃₎-Alkyl ausgewählt sind, substituiert ist; wobei das -C₍₁₋₃₎-Alkyl gegebenenfalls durch -OH substituiert ist;
1,3,4-Oxadiazolyl; wobei das 1,3,4-Oxadiazolyl durch Cyclopropyl substituiert ist;
5,6,7,8-Tetrahydroimidazo[1,5-*a*]pyridinyl; wobei das 5,6,7,8-Tetrahydroimidazo[1,5-*a*]pyridinyl durch -CH₂OH substituiert ist;
1,2,4-Thiadiazolyl; wobei das 1,2,4-Thiadiazolyl durch Cyclopropyl substituiert ist;
5,6,7,8-Tetrahydro-1,7-naphthyridinyl;
5,6,7,8-Tetrahydro-1,6-naphthyridinyl;
5,6,7,8-Tetrahydro-1,8-naphthyridinyl;
1,2,3,4-Tetrahydro-2,7-naphthyridinyl;
2*H*-Pyrido[3,2-*b*][1,4]oxazin-3(4*H*)-onyl, wobei das *2H-*Pyrido[3,2-*b*][1,4]oxazin-3(4*H*)-onyl gegebenenfalls durch -CH₃ substituiert ist;
2,3-Dihydro-1*H*-pyrrolo[2,3-*b*]pyridinyl;
Imidazo[1,2-*a*]pyrazinyl, wobei das Imidazo[1,2-a]pyrazinyl durch -NH₂ substituiert ist;
2,6-Dioxo-1,2,3,6-tetrahydropyrimidinyl, wobei das 2,6-Dioxo-1,2,3,6-tetrahydropyrimidinyl durch -CH₃ substituiert ist;
6-Oxo-1,6-dihydropyrimidinyl, wobei das 6-Oxo-1,6-dihydropyrimidinyl durch Fluor substituiert ist;
6,7-Dihydro-5*H*-pyrrolo[1,2-*a*]imidazolyl, wobei das 6,7-Dihydro-5*H*-pyrrolo[1,2-*a*]imidazolyl gegebenenfalls durch einen oder zwei Substituenten, die aus der Gruppe bestehend aus -CH₃ und -OH ausgewählt sind, substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

5. Verbindung nach einem der Ansprüche 1-4, wobei die Verbindung ausgewählt ist aus der Gruppe bestehend aus: 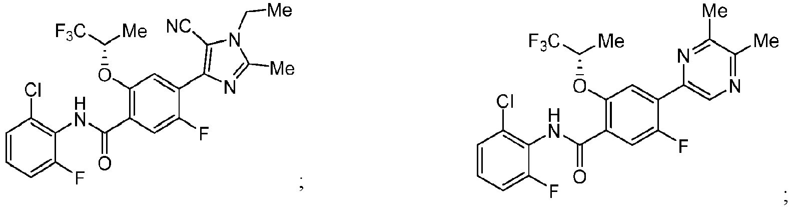 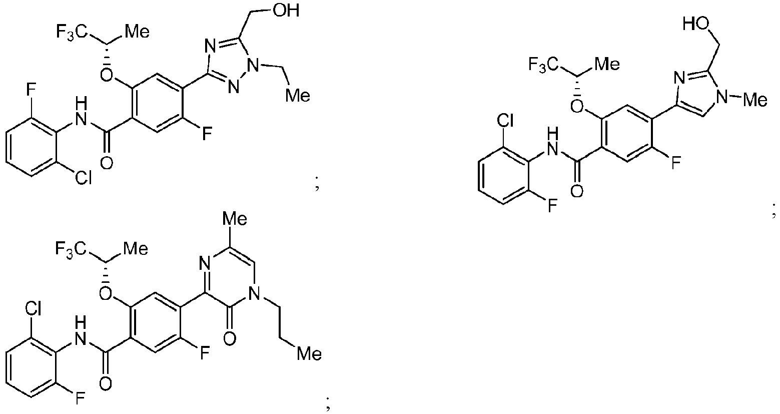 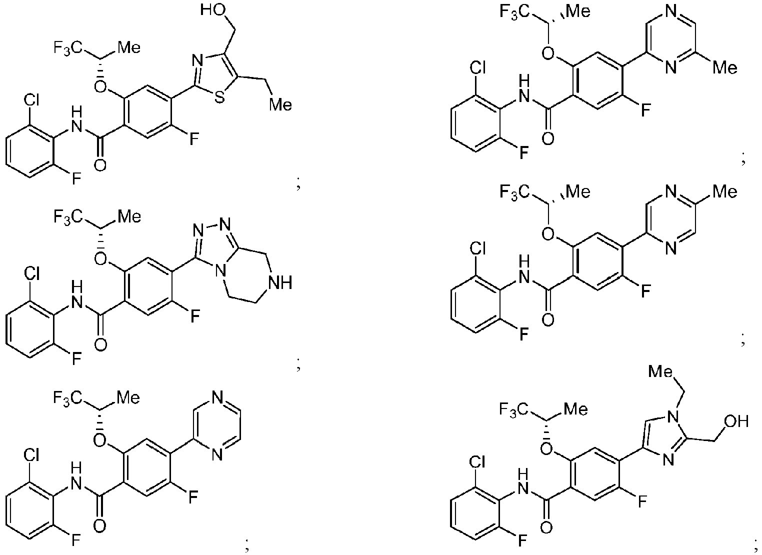 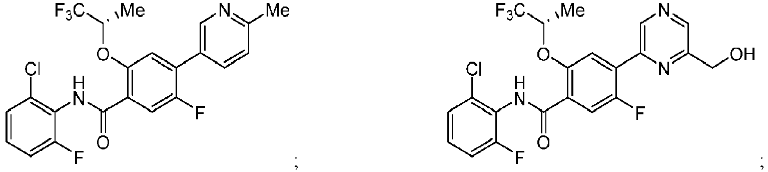 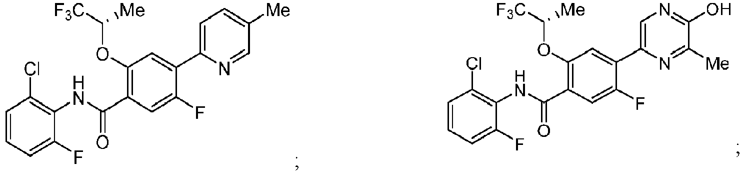 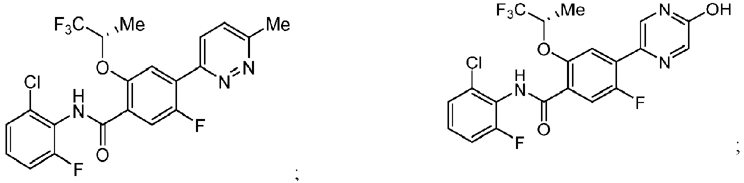 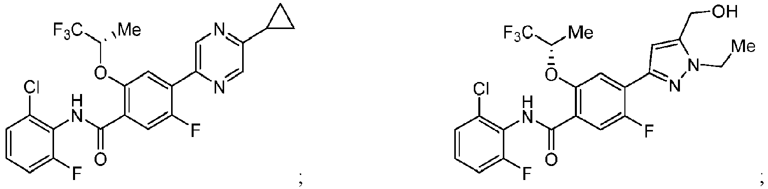 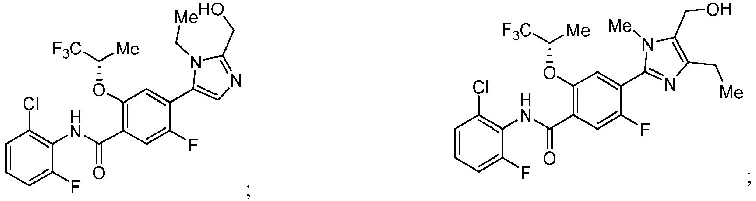 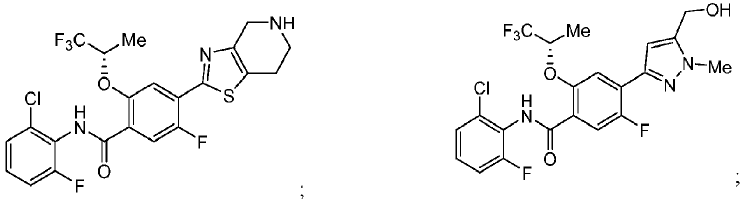 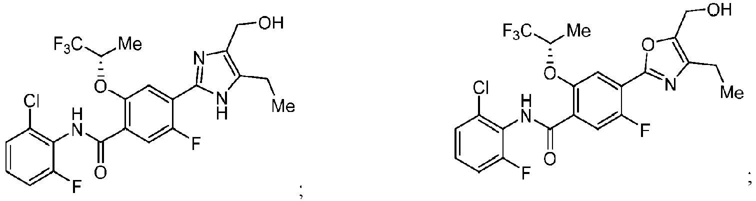 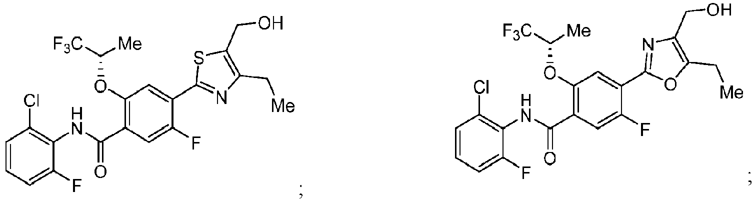 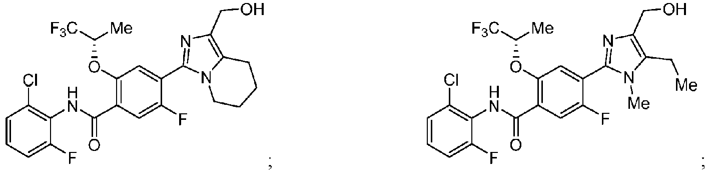 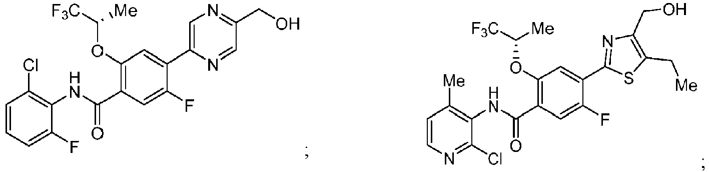 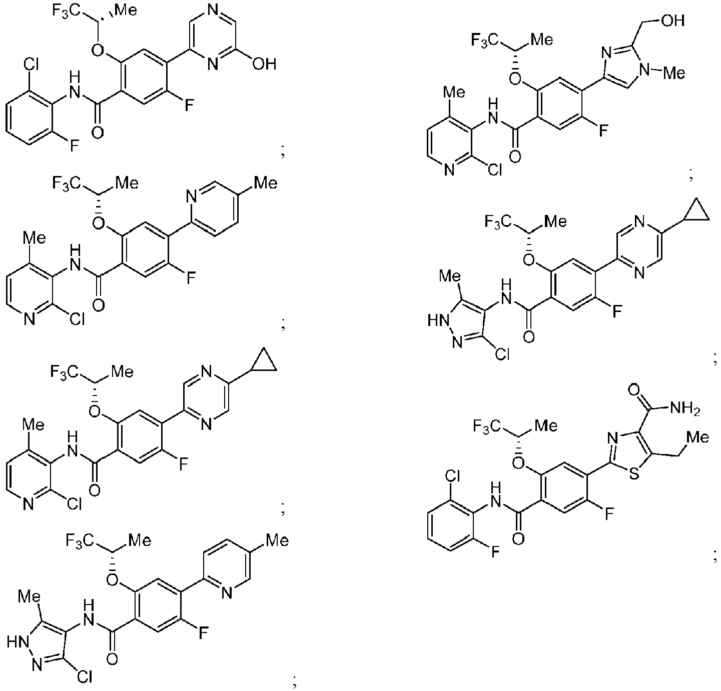 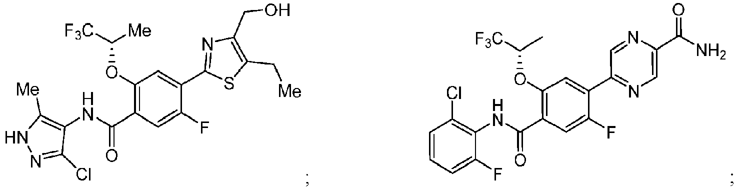 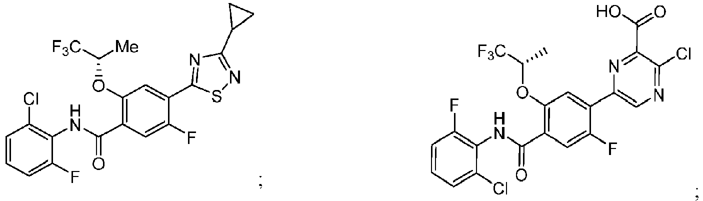 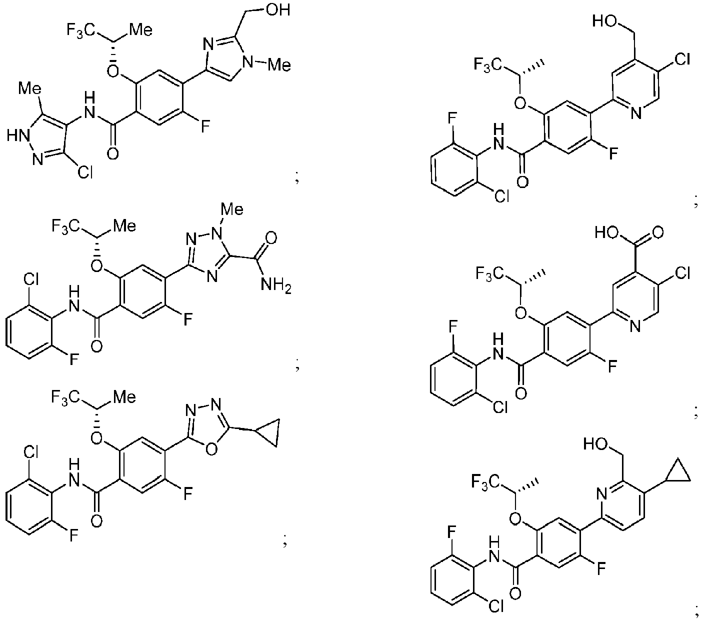 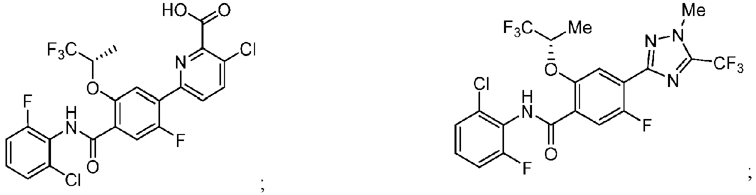 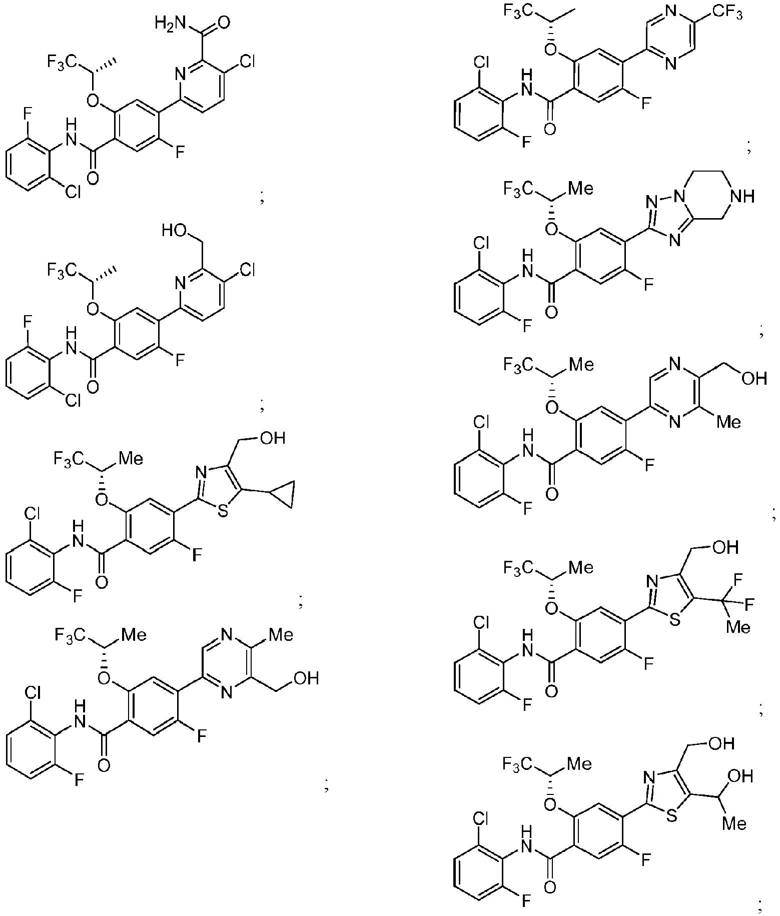 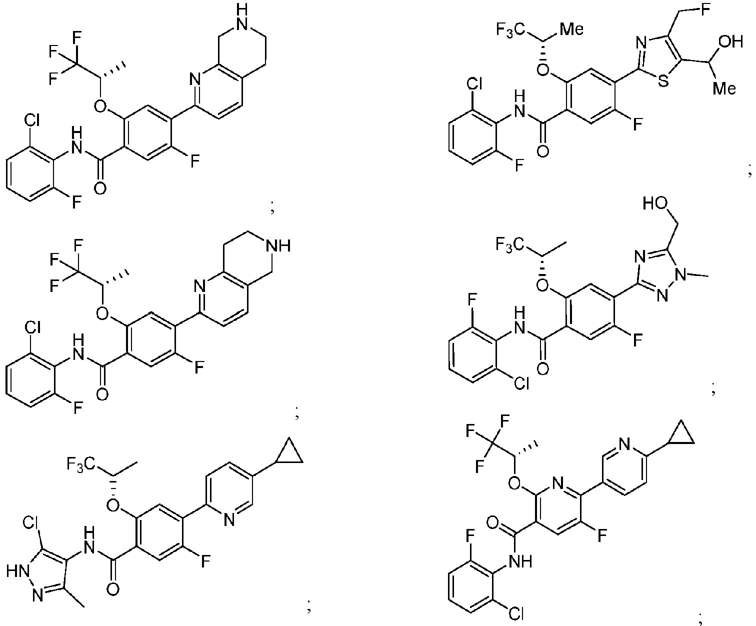 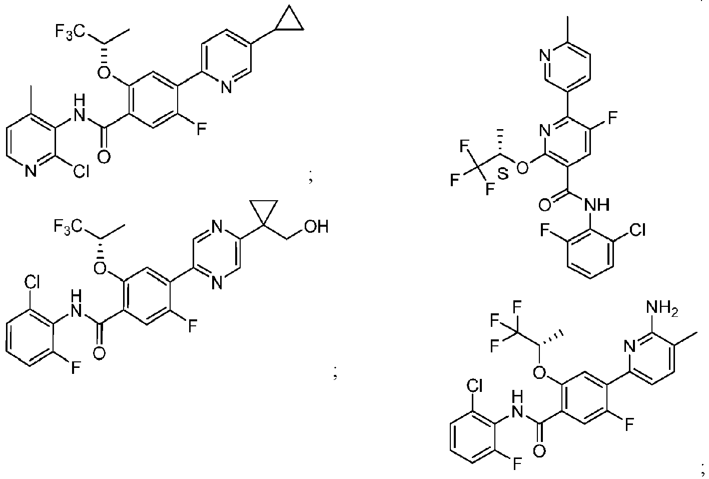 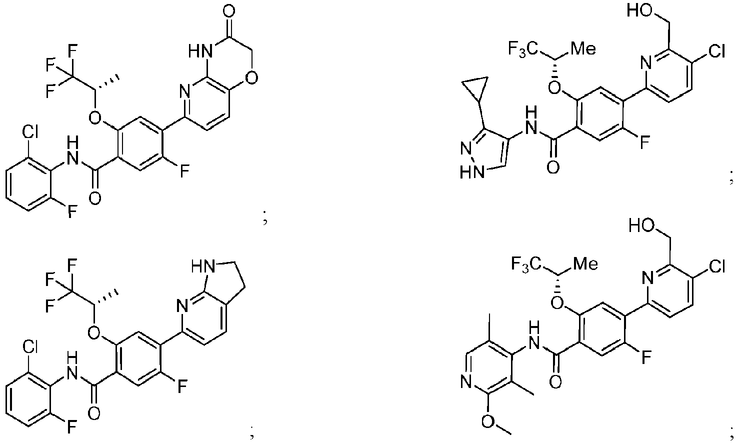 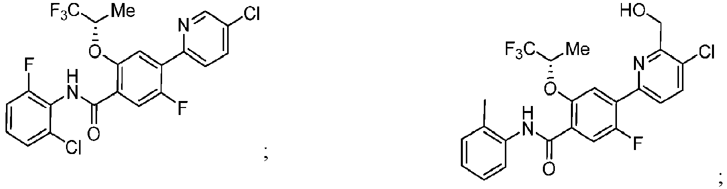 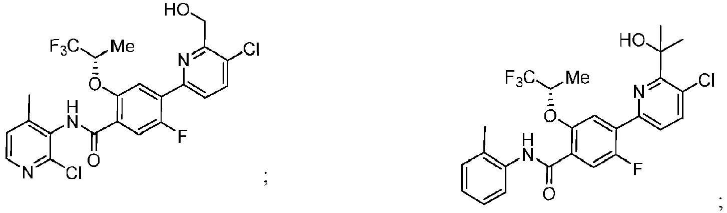 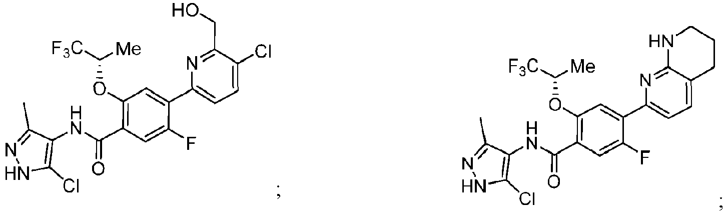 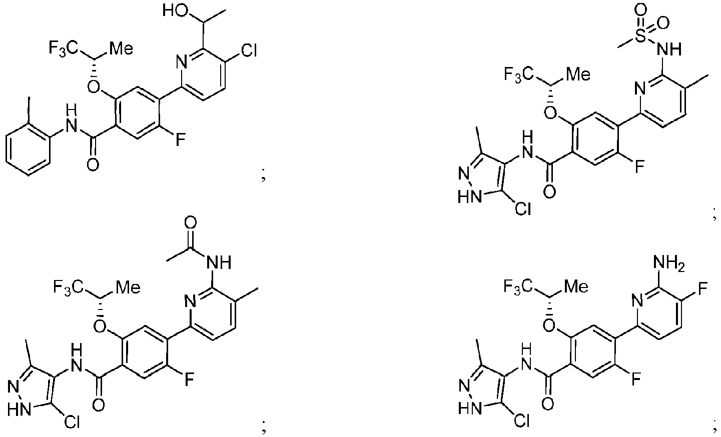 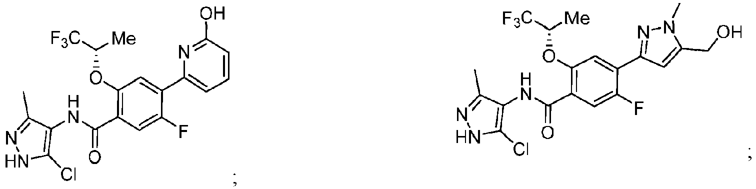 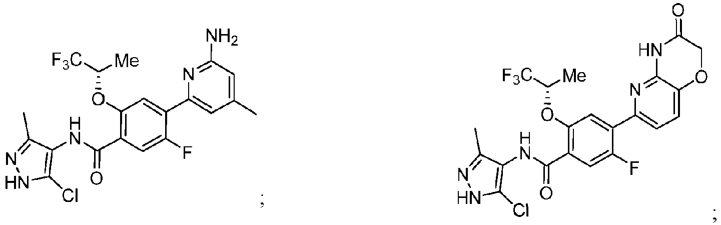 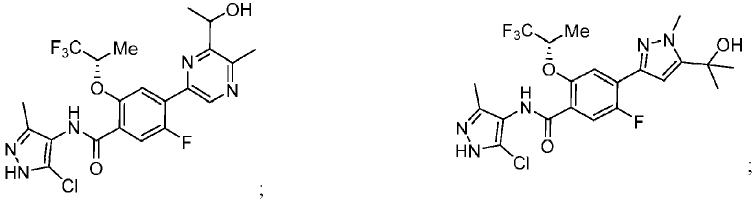 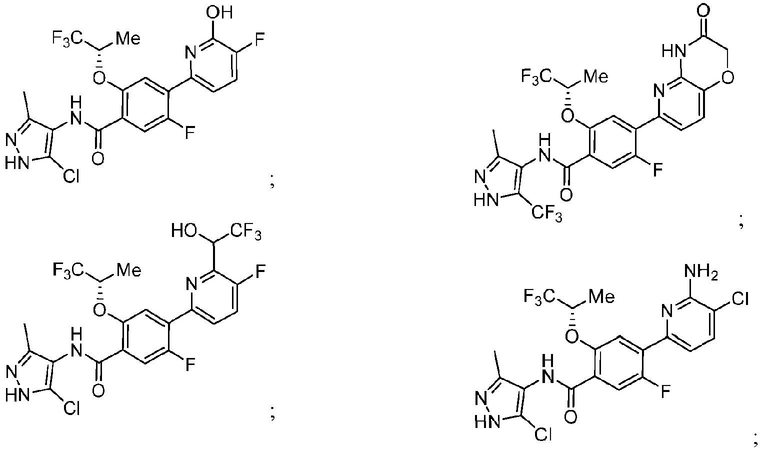 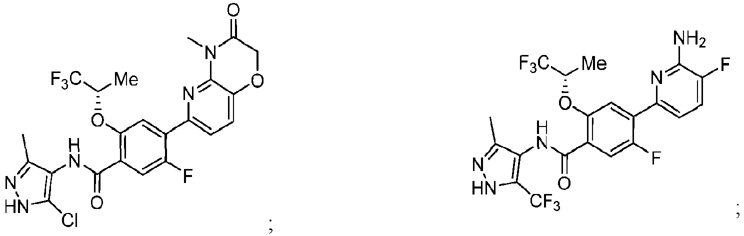 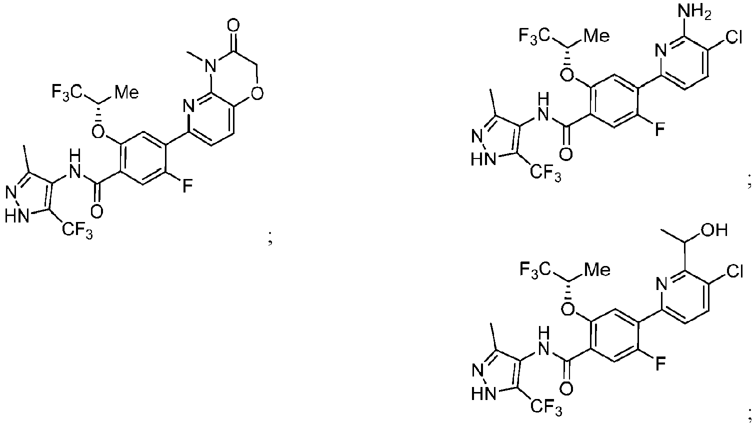 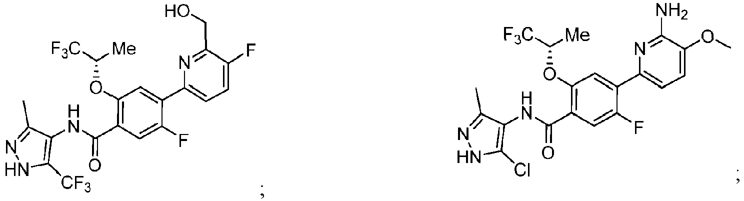 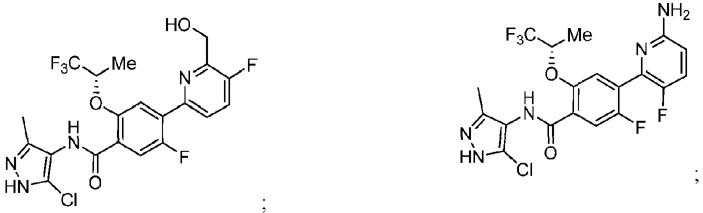 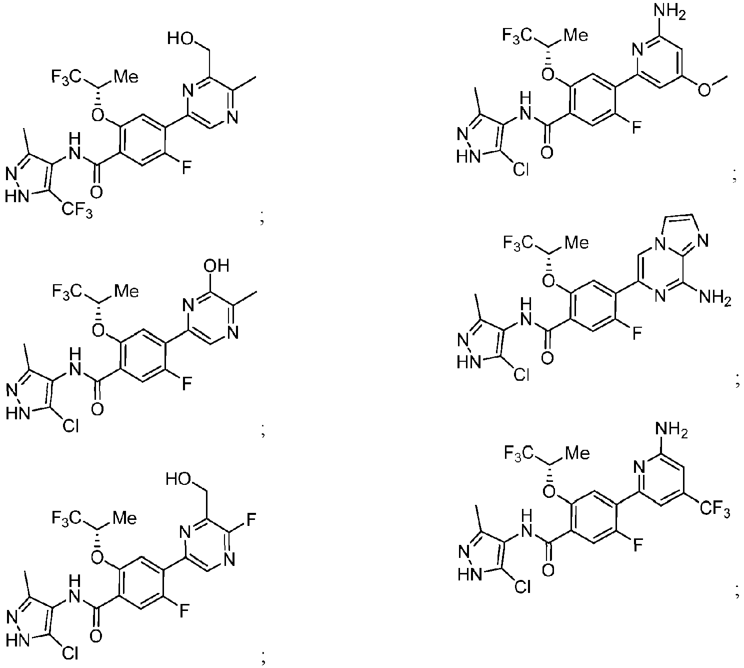 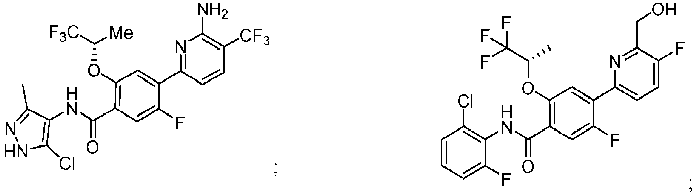 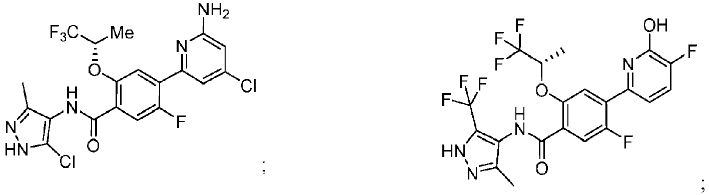 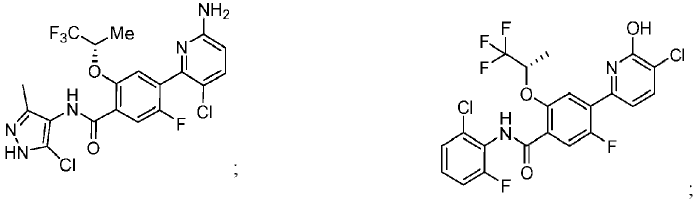 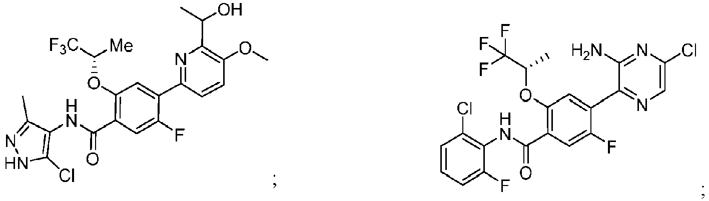 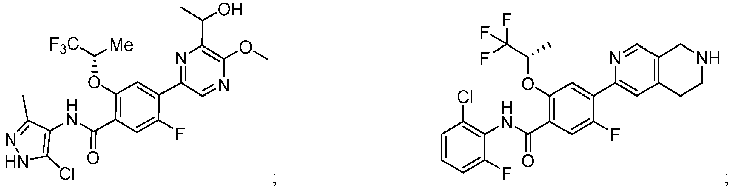 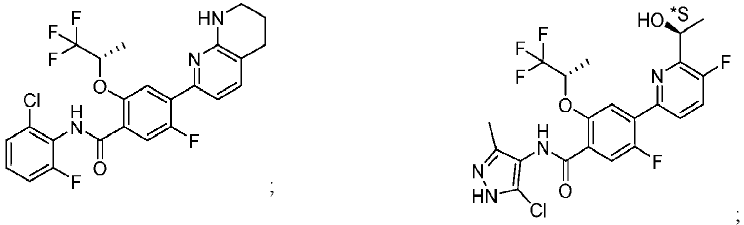 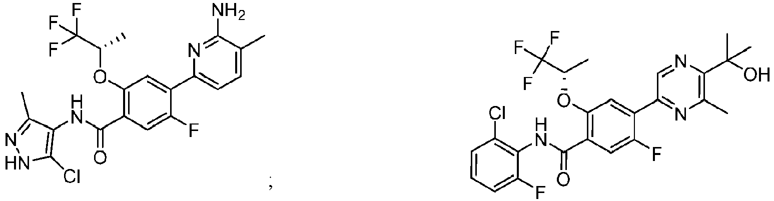 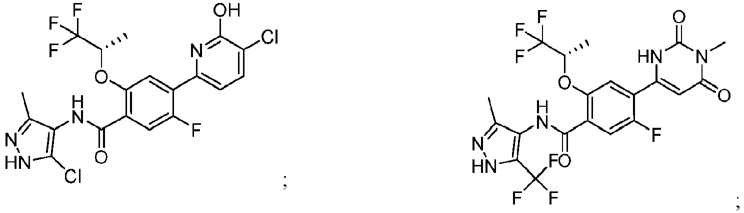 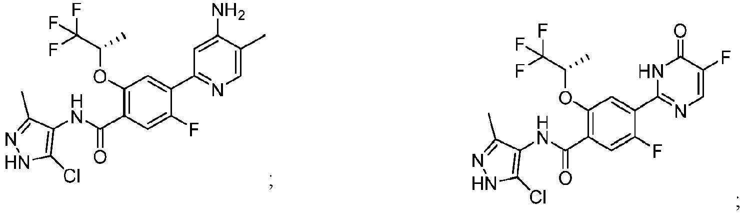 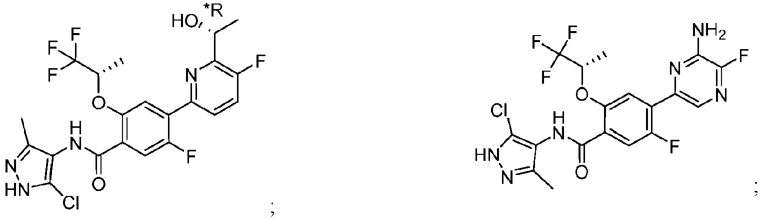 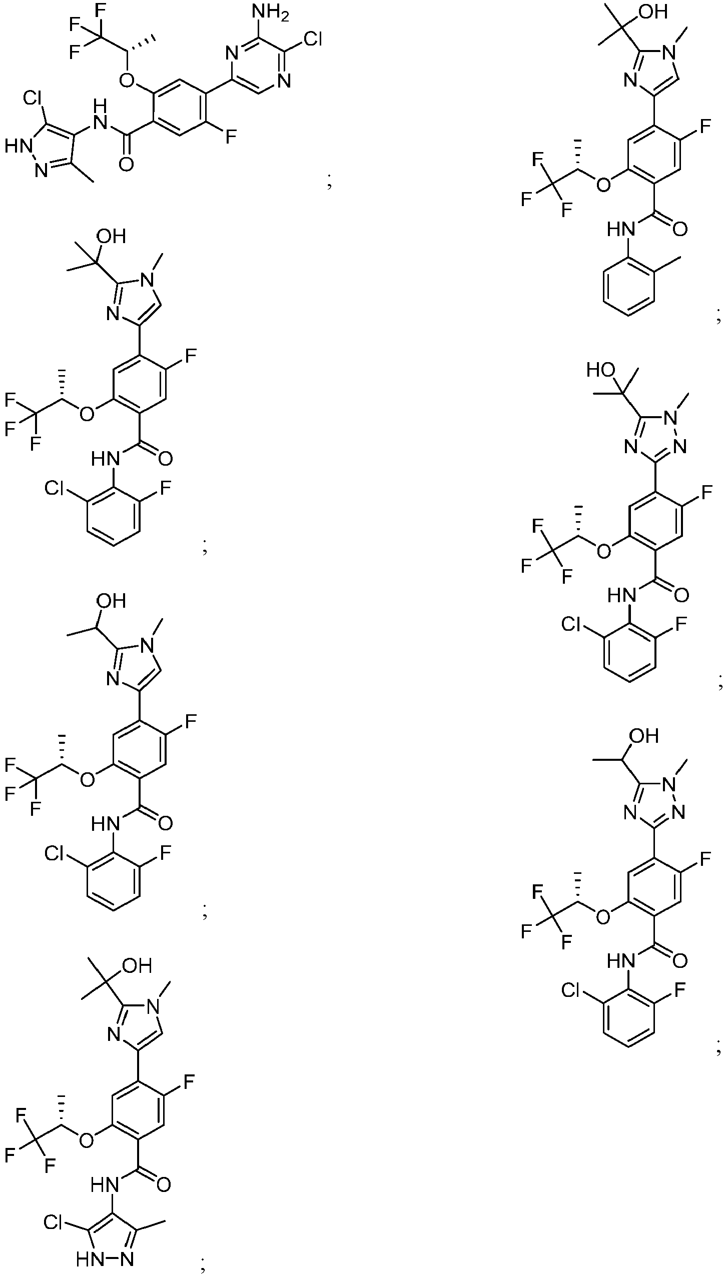 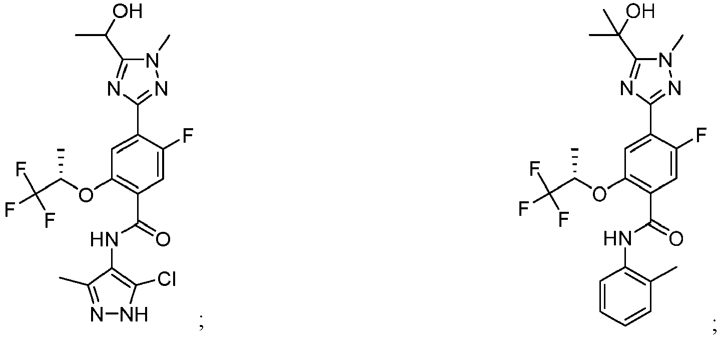 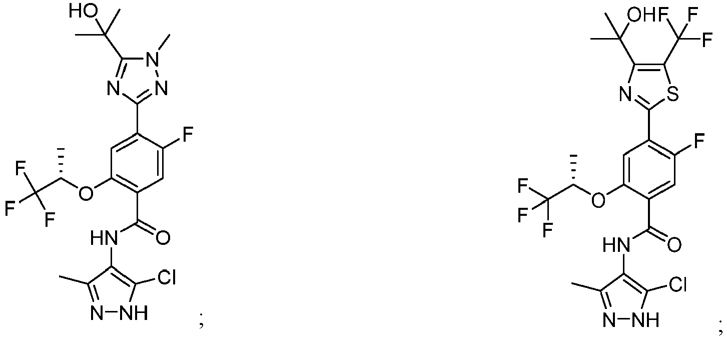 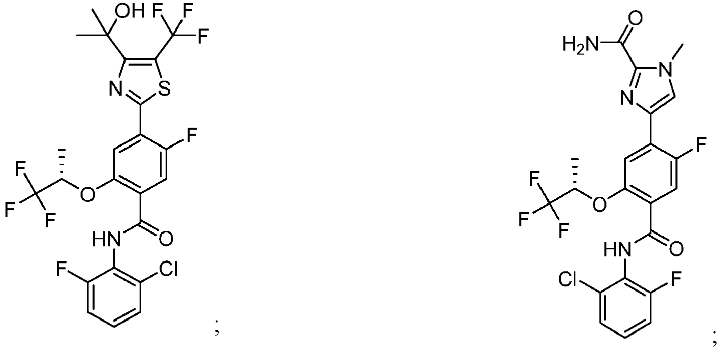 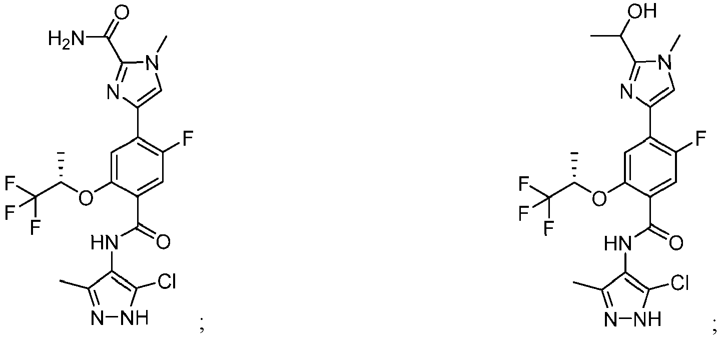 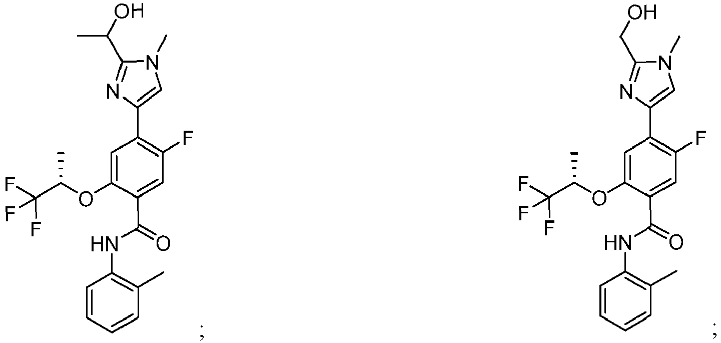 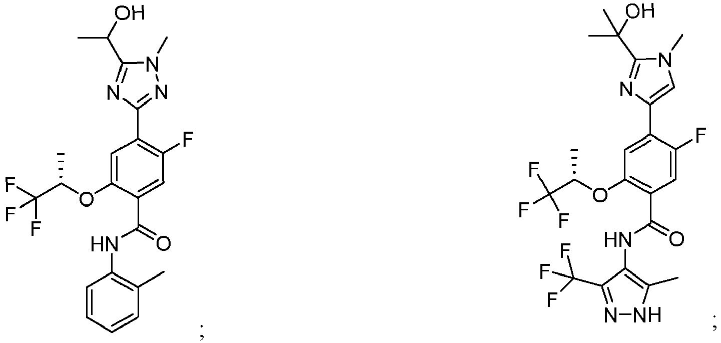 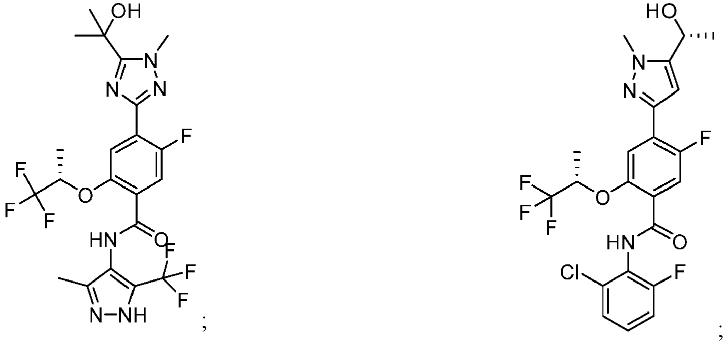 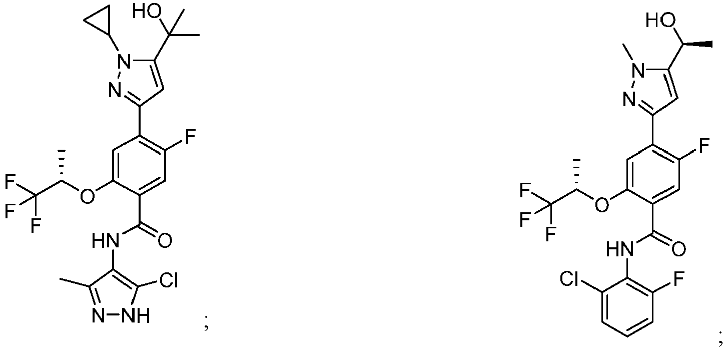 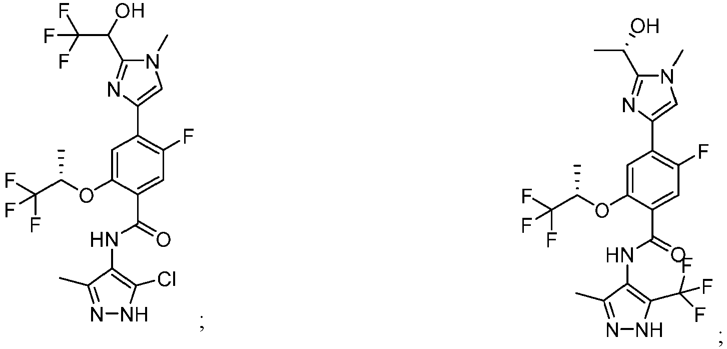 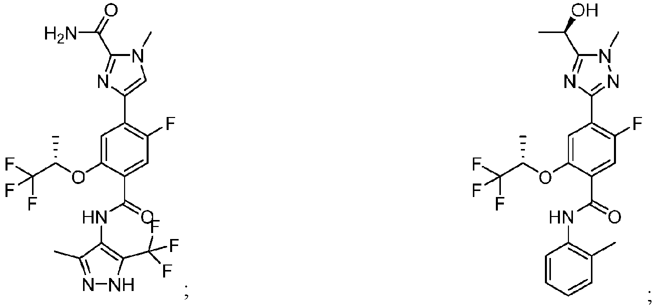 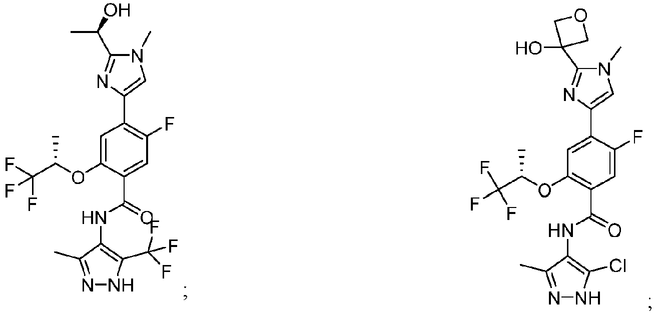 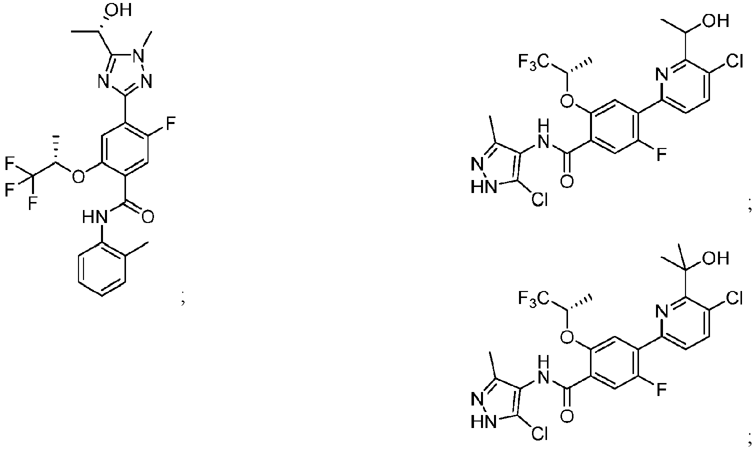 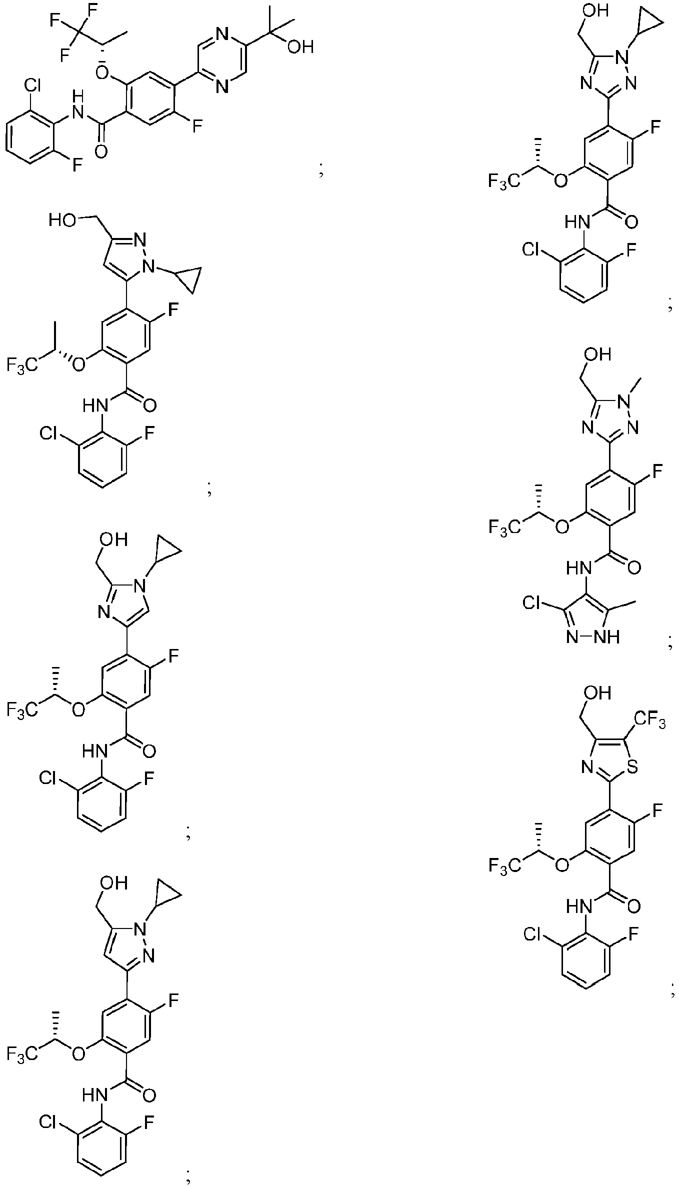 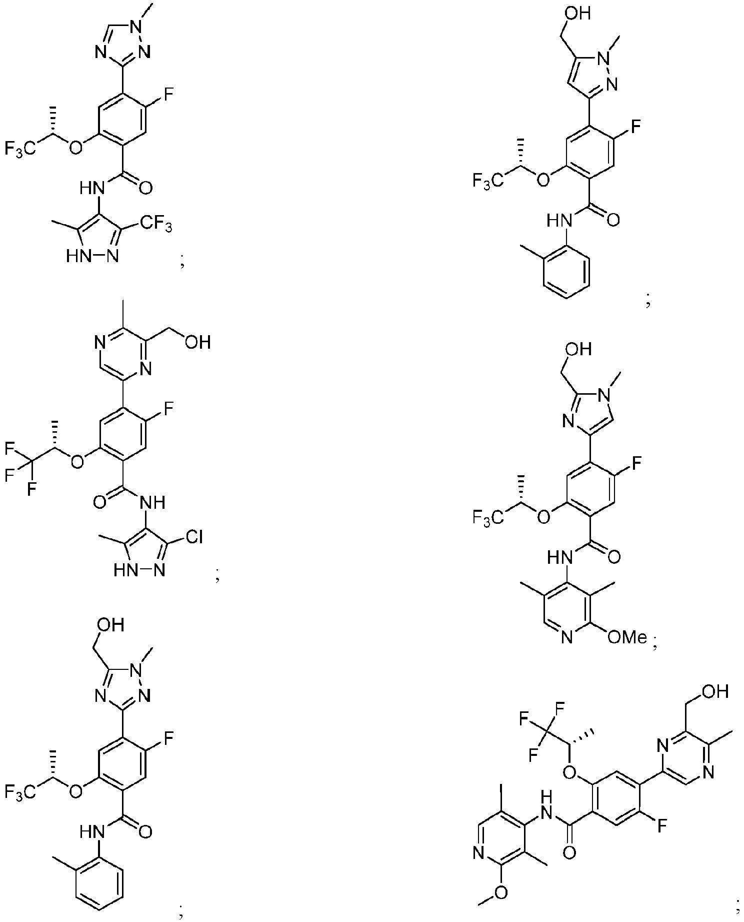 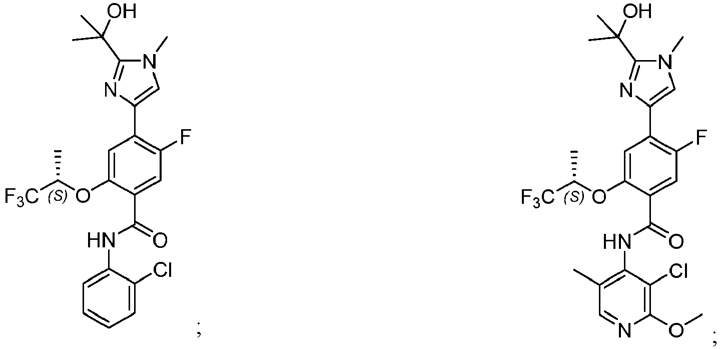 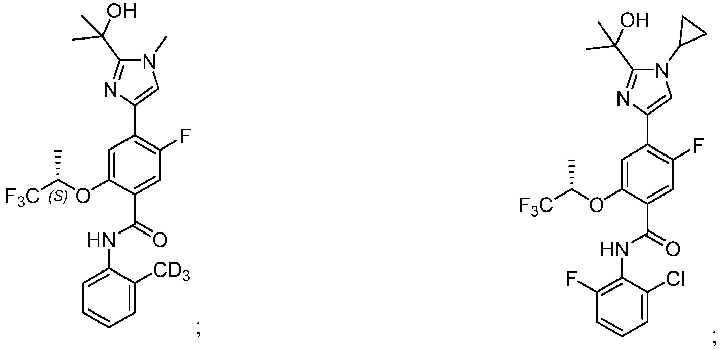 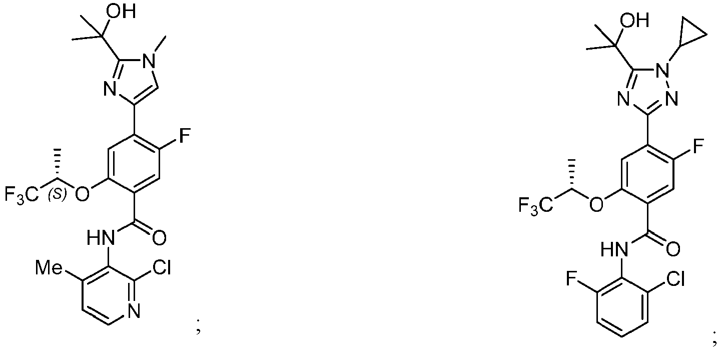 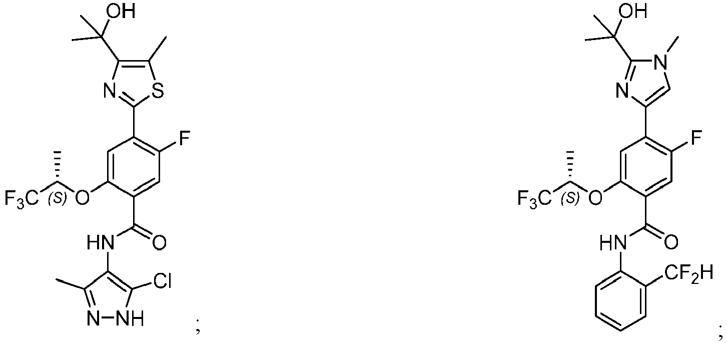 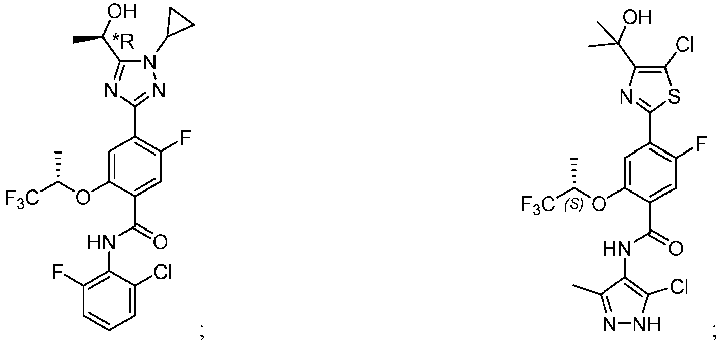 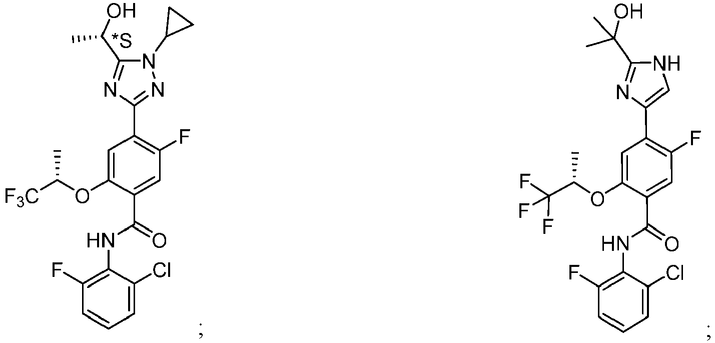 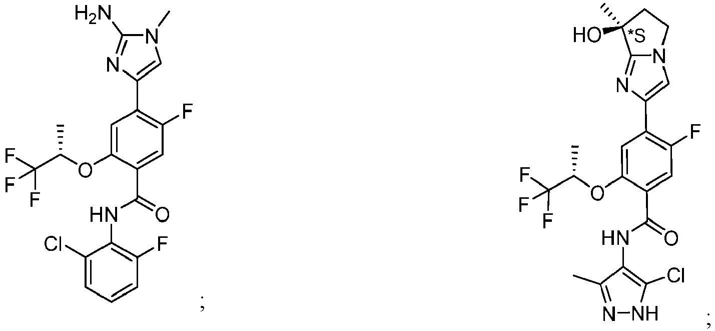 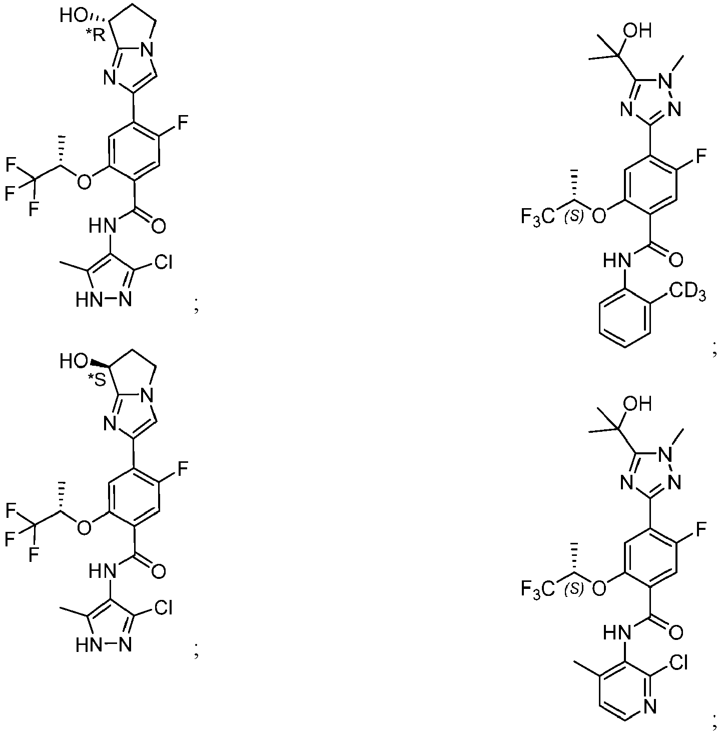 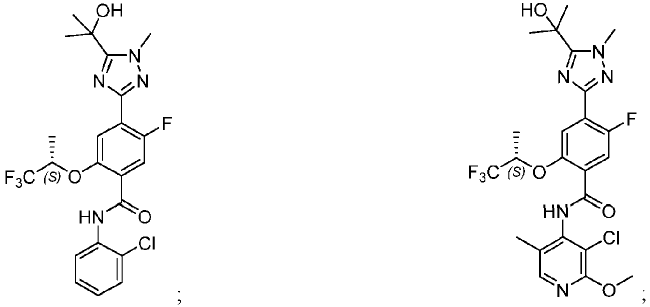 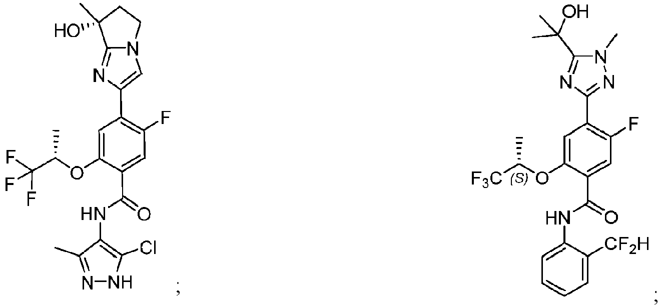 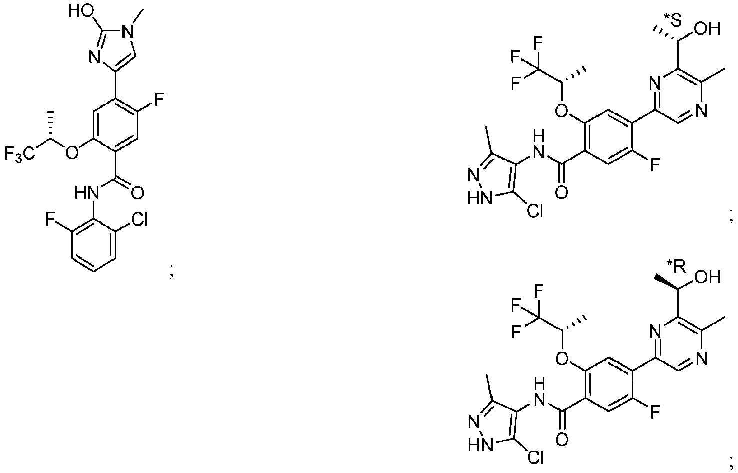 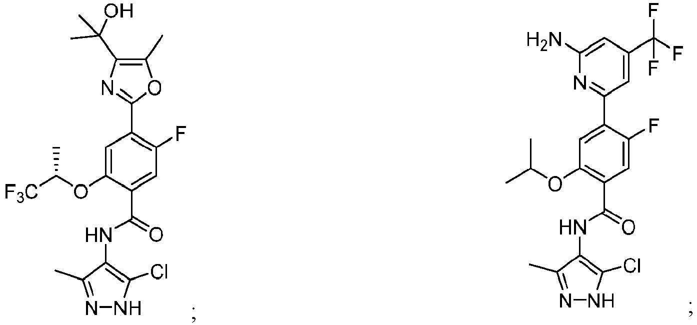 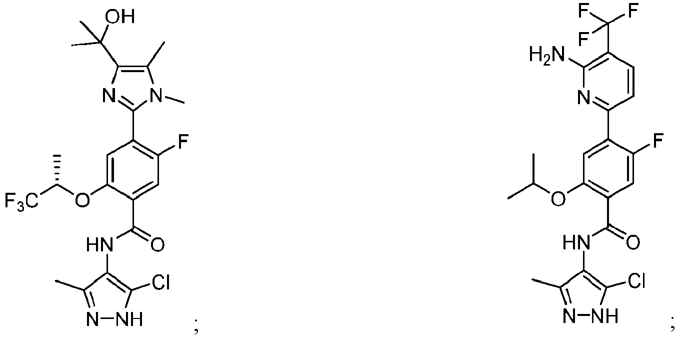 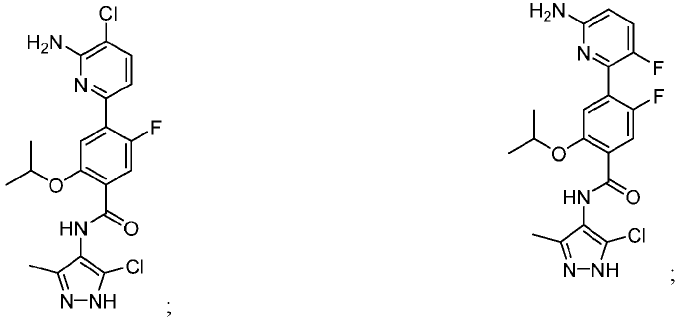 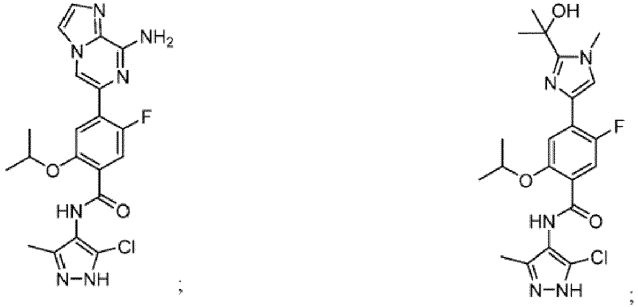 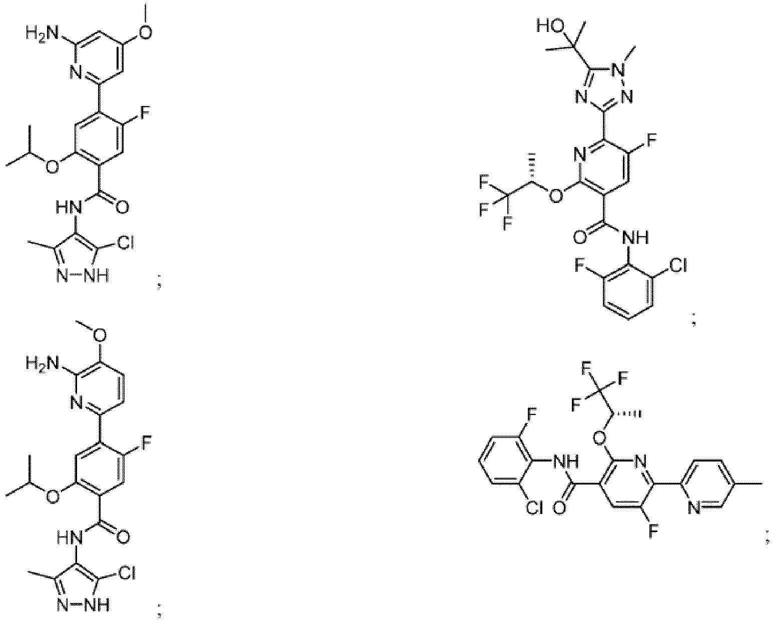 und 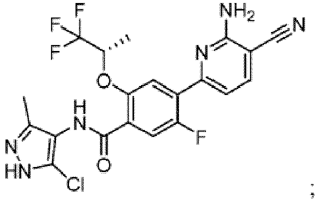 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

6. Verbindung nach Anspruch 1, wobei:
(a) Q für CH oder N steht;
R¹ für 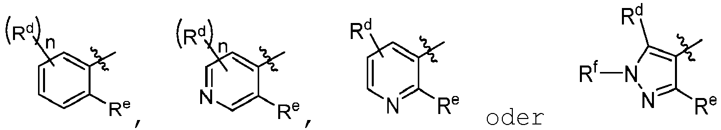 steht; wobei
R^{d} aus der Gruppe bestehend aus -H, -Cl, -F und -C(₁₋₃)-Alkyl ausgewählt ist; wobei das -C(₁₋₃)-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Imidazolyl; wobei das Imidazolyl gegebenenfalls durch bis zu drei Substituenten, die unabhängig aus der Gruppe umfassend 3-Hydroxyoxetanyl, -NH₂, -OH, -CN oder R^{a} ausgewählt sind, substituiert ist;
R^{a} für -C(O)NH₂, Cyclopropyl oder -C₍₁₋₃₎-Alkyl steht; wobei das -C(₁₋₃)-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(b) Q für CH oder N steht;
R¹ für 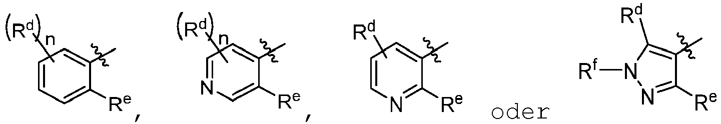 steht; wobei
R^{d} aus der Gruppe bestehend aus -H, -Cl, -F und -C₍₁₋₃₎-Alkyl ausgewählt ist; wobei das -C(₁₋₃)-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C₍₁₋₃₎-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Triazolyl; wobei das Triazolyl gegebenenfalls durch eine oder zwei Gruppen, die unabhängig aus der Gruppe umfassend R^{a} ausgewählt sind, substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(c) Q für CH oder N steht;
R¹ für 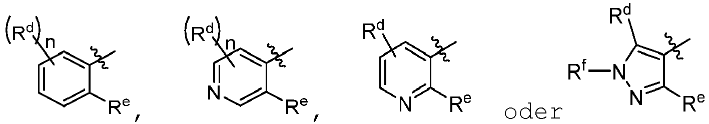 steht; wobei
R^{d} aus der Gruppe bestehend aus -H, -Cl, -F und -C₍₁₋₃₎-Alkyl ausgewählt ist; wobei das -C(₁₋₃)-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C(₁₋₃)-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Pyrazinyl; wobei das Pyrazinyl gegebenenfalls durch bis zu drei Substituenten, die unabhängig aus der Gruppe umfassend -NHSO₂CH₃, -NHC(O)CH₃, -OCH₃, -OH, -NH₂, -CO₂H, -Cl, -F oder R^{a} ausgewählt sind, substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(d) Q für CH oder N steht;
R¹ für 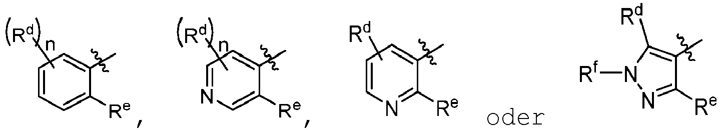 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Pyrazinonyl; wobei das Pyrazinonyl gegebenenfalls durch eine oder zwei Gruppen, die unabhängig aus der Gruppe umfassend R^{a} ausgewählt sind, substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(e) Q für CH oder N steht;
R¹ für 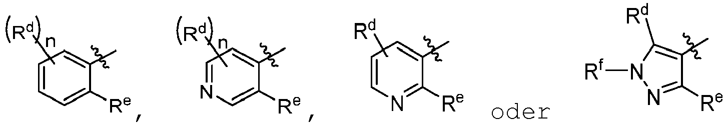 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C(₁₋₃)-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C(₁₋₃)-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Thiazolyl; wobei das Thiazolyl gegebenenfalls durch einen oder zwei Substituenten, die unabhängig aus der Gruppe umfassend R^{a} ausgewählt sind, substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(f) Q für CH oder N steht;
R¹ für 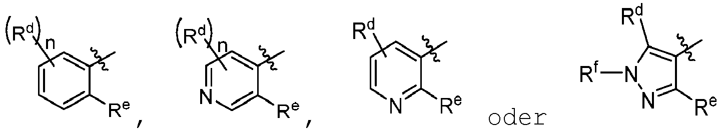 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C₍₁₋₃₎-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
5,6,7,8-Tetrahydro-[1,2,4]triazolo[4,3-a]pyrazinyl;
wobei das 5,6,7,8-Tetrahydro-[1,2,4]triazolo[4,3-a]pyrazinyl gegebenenfalls durch R^{a} substituiert ist;
R^{a} für -C(O)NH₂, Cyclopropyl oder -C₍₁₋₃₎-Alkyl steht;
wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(g) Q für CH oder N steht;
R¹ für 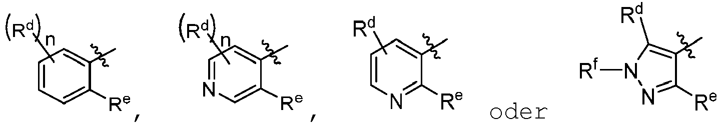 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C(₁₋₃)-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Pyridinyl; wobei das Pyridinyl gegebenenfalls durch einen oder zwei Substituenten, die unabhängig aus der Gruppe umfassend R^{a}, -Cl, -F, -CN, -NHC(O)CH₃, -NHSO₂CH₃, -CO₂H, -OCH₃, -OH oder -NH₂ ausgewählt sind, substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(h) Q für CH oder N steht;
R¹ für 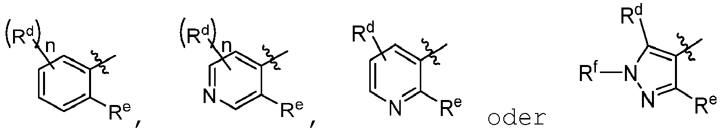 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Pyridazinyl; wobei das Pyridazinyl gegebenenfalls durch eine oder zwei Gruppen, die unabhängig aus der Gruppe umfassend R^{a}, -Cl oder -CO₂H ausgewählt sind, substituiert ist;
R^{a} für -C(O)NH₂, Cyclopropyl oder -C₍₁₋₃₎-Alkyl steht; wobei das -C(₁₋₃)-Alkyl gegebenenfalls durch bis zu drei Fluoratome substituiert ist oder das -C₍₁₋₃₎Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(i) Q für CH oder N steht;
R¹ für 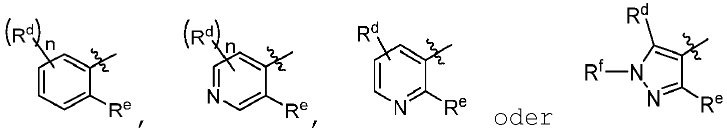 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C₍₁₋₃₎-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
4,5,6,7-Tetrahydrothiazolo[5,4-c]pyridinyl; wobei das 4,5,6,7-Tetrahydrothiazolo[5,4-c]pyridinyl gegebenenfalls durch R^{a} substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(j) Q für CH oder N steht;
R¹ für 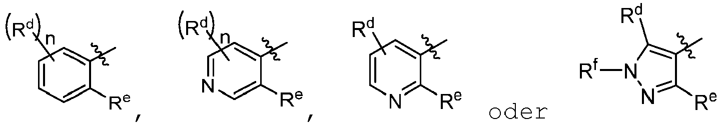 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Pyrazolyl; wobei das Pyrazolyl gegebenenfalls durch einen oder zwei Substituenten, die unabhängig aus der Gruppe umfassend R^{a} ausgewählt sind, substituiert ist;
R^{a} für -C(O)NH₂, Cyclopropyl oder -C₍₁₋₃₎-Alkyl steht; wobei das -C(₁₋₃)-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(k) Q für CH oder N steht;
R¹ für 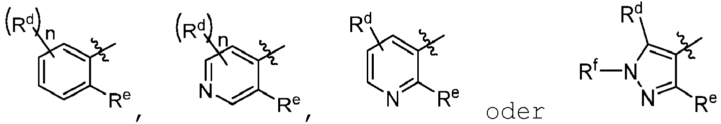 steht; wobei
R^{d} aus der Gruppe bestehend aus -H, -Cl, -F und -C₍₁₋₃₎-Alkyl ausgewählt ist; wobei das -C(₁₋₃)-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
Oxazolyl; wobei das Oxazolyl gegebenenfalls durch einen oder zwei Substituenten, die unabhängig aus der Gruppe umfassend R^{a} ausgewählt sind, substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(l) Q für CH oder N steht;
R¹ für 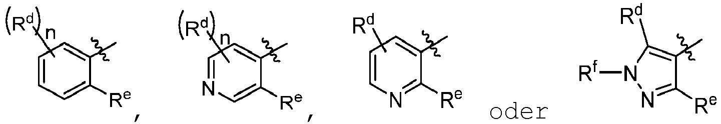 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C(₁₋₃)-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C(₁₋₃)-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
1,3,4-Oxadiazolyl; wobei das 1,3,4-Oxadiazolyl gegebenenfalls durch R^{a} substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(m) Q für CH oder N steht;
R¹ für 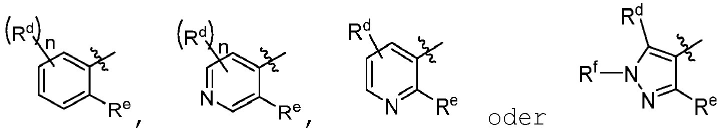 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
5,6,7,8-Tetrahydroimidazo[1,5-a]pyridinyl; wobei das 5,6,7,8-Tetrahydroimidazo[1,5-a]pyridinyl gegebenenfalls durch R^{a} substituiert ist;
R^{a} für -C(O)NH₂, Cyclopropyl oder -C₍₁₋₃₎-Alkyl steht; wobei das -C(₁₋₃)-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(n) Q für CH oder N steht;
R¹ für 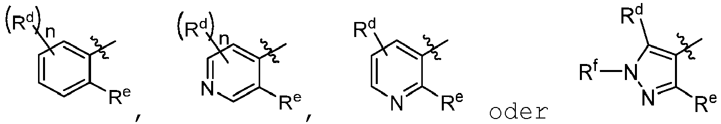 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
1,2,4-Thiadiazolyl; wobei das 1,2,4-Thiadiazolyl gegebenenfalls durch R^{a} substituiert ist;
R^{a} für -C(O)NH₂, Cyclopropyl oder -C₍₁₋₃₎-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(o) Q für CH oder N steht;
R¹ für 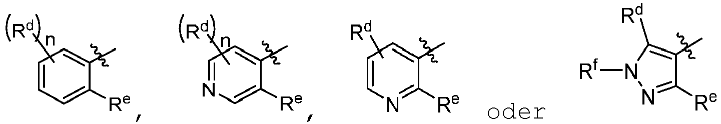 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
5,6,7,8-Tetrahydro-1,7-naphthyridinyl; wobei das 5,6,7,8-Tetrahydro-1,7-naphthyridinyl gegebenenfalls durch R^{a} substituiert ist; oder
R^{a} für -C(O)NH₂, Cyclopropyl oder -C₍₁₋₃₎-Alkyl steht; wobei das -C(₁₋₃)-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(p) Q für CH oder N steht;
R¹ für 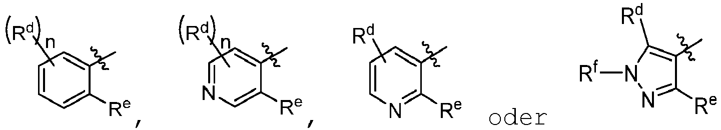 steht; wobei
R^{d} aus der Gruppe bestehend aus -H, -Cl, -F und -C₍₁₋₃₎-Alkyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{e} aus der Gruppe bestehend aus -Cl, -F, -C₍₁₋₃₎-Alkyl, -CD₃ und Cyclopropyl ausgewählt ist; wobei das -C₍₁₋₃₎-Alkyl gegebenenfalls durch -OH, -OCH₃, -SCH₃ oder -OCF₃ substituiert ist und wobei das -C₍₁₋₃₎-Alkyl gegebenenfalls durch bis zu sechs Fluoratome substituiert ist;
R^{f} aus der Gruppe bestehend aus -H und -C₍₁₋₃₎-Alkyl ausgewählt ist; wobei das -C(₁₋₃)-Alkyl gegebenenfalls durch ein Mitglied aus der Gruppe bestehend aus -OH, - OCH₃, -SCH₃ oder -OCF₃ substituiert ist und wobei das - C₍₁₋₃₎-Alkyl gegebenenfalls durch bis zu sechs Fluoratome substituiert ist; und
n für 1 oder 2 steht;
R² für Folgendes steht:
5,6,7,8-Tetrahydro-1,6-naphthyridinyl; wobei das 5,6,7,8-Tetrahydro-1,6-naphthyridinyl gegebenenfalls durch R^{a} substituiert ist;
R^{a} für -C(O)NH₂, Cyclopropyl oder -C(₁₋₃)-Alkyl steht; wobei das -C₍₁₋₃₎-Alkyl gegebenenfalls durch bis zu drei Fluoratome substituiert ist oder das -C₍₁₋₃₎-Alkyl gegebenenfalls durch -OH substituiert ist und zusätzlich gegebenenfalls durch bis zu drei Fluoratome substituiert ist und wobei das Cyclopropyl gegebenenfalls durch -CH₂OH substituiert ist;
R³ für -CHR^{b}R^{c} steht; wobei
R^{b} für -C₍₃₋₆₎-Cycloalkyl oder -C₍₁₋₆₎-Alkyl steht; wobei das -C₍₁₋₆₎-Alkyl gegebenenfalls durch -Cl, -OH oder -OCH₃ substituiert ist und wobei das -C₍₁₋₆₎-Alkyl gegebenenfalls durch bis zu sechs Fluoratome substituiert ist;
R^{c} für -CH₃ oder -CHF₂ steht oder R^{b} und R^{c} zusammen mit dem Kohlenstoffatom, an das sie gebunden sind, -C₍₃₋₆₎-Cycloalkyl bilden können; wobei das -C₍₃₋₆₎-Cycloalkyl gegebenenfalls durch bis zu vier Substituenten, die unabhängig aus der Gruppe bestehend aus -Cl, -F, -OH und -C₍₁₋₆₎-Alkyl ausgewählt sind, substituiert ist; wobei das C₍₁₋₆₎-Alkyl gegebenenfalls durch bis zu sechs Fluoratome substituiert ist;
oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

7. Verbindung nach Anspruch 4, ausgewählt aus der Gruppe bestehend aus:
(a) 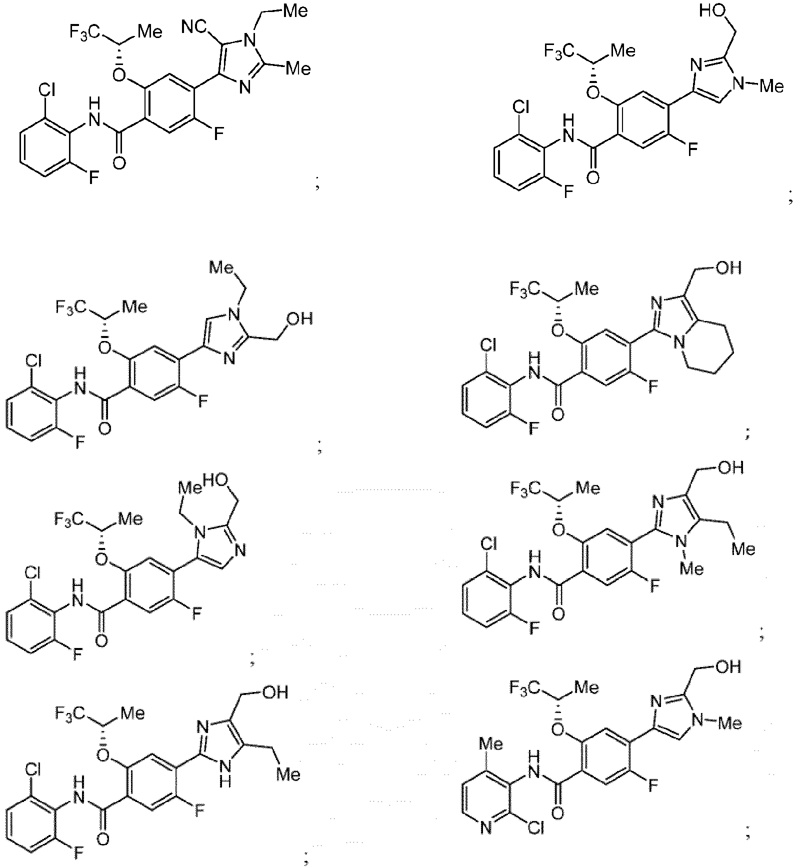 und 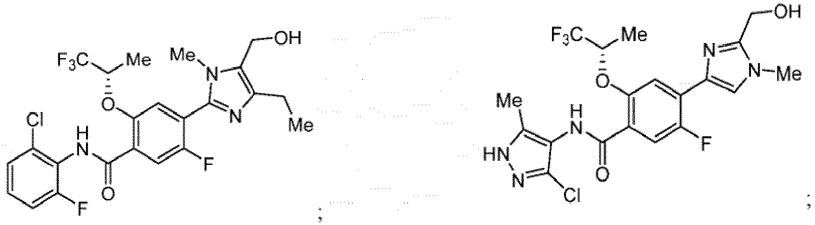 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(b) 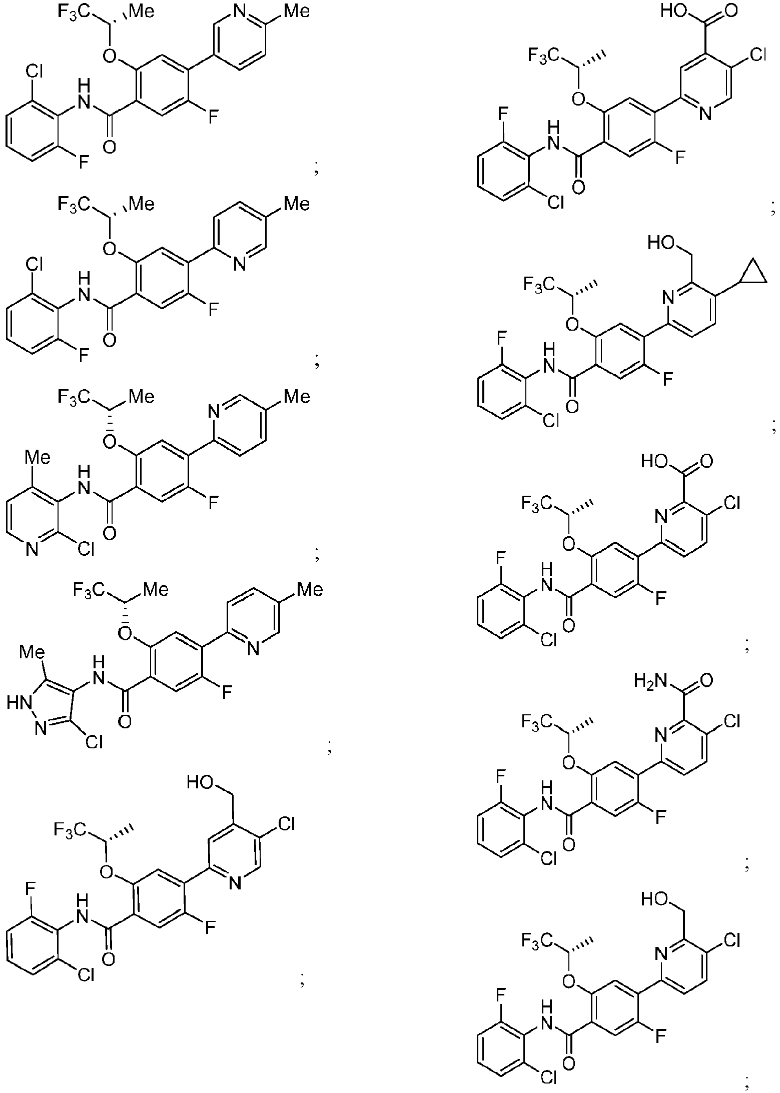 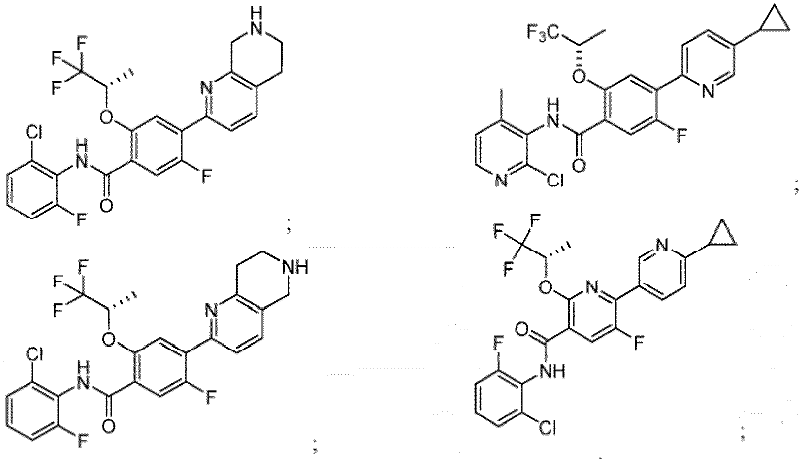 und 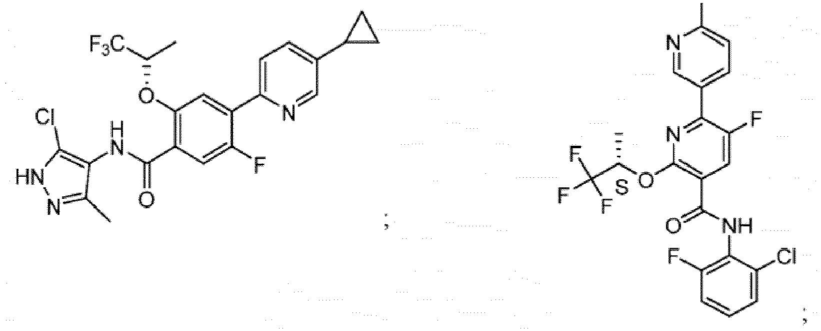 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(c) 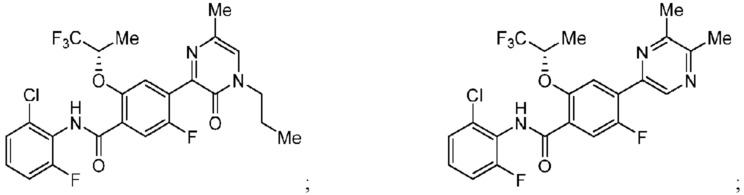 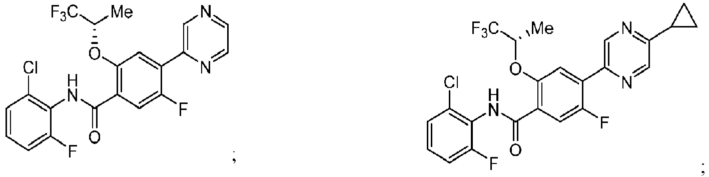 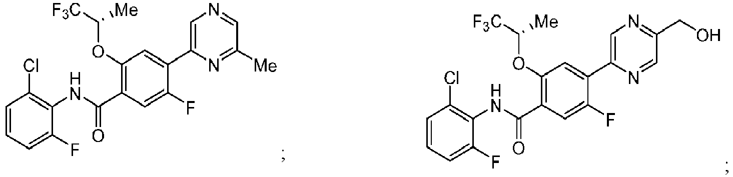 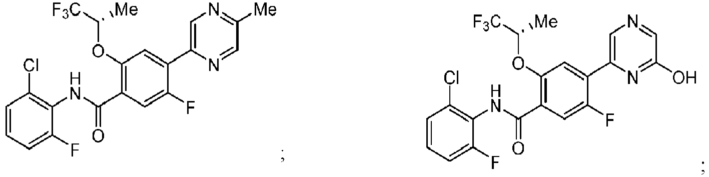 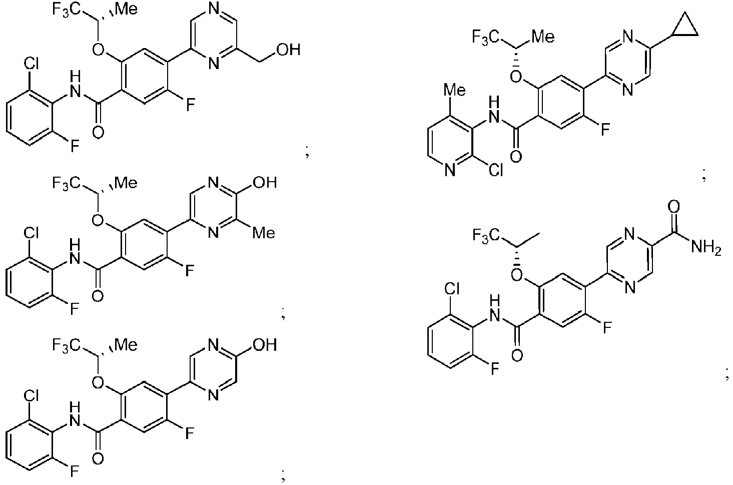 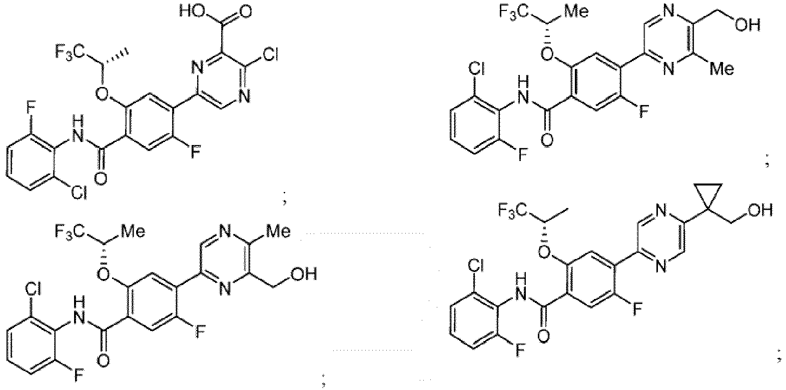 und 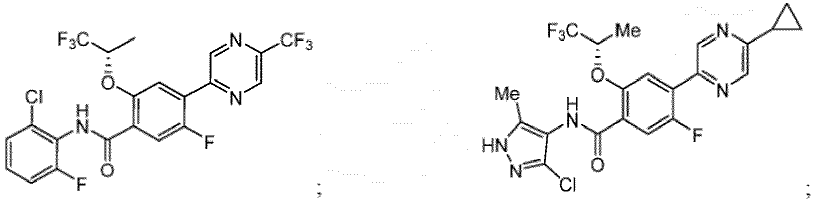 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(d) 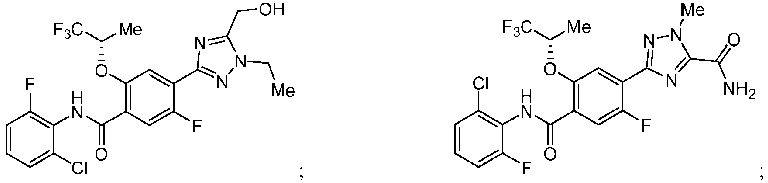 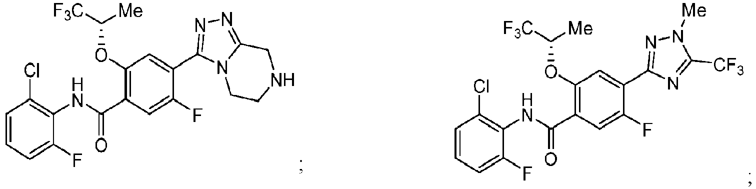 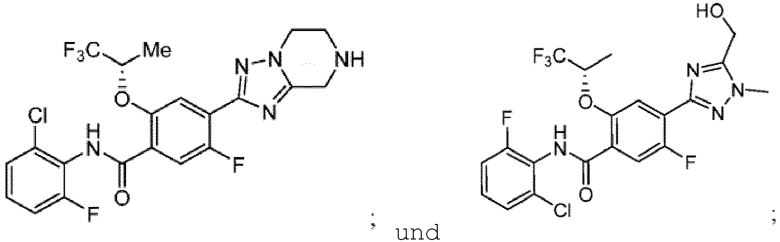 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(e) 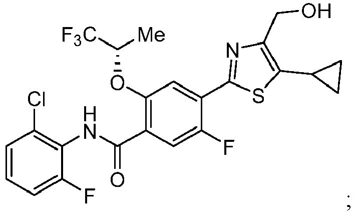 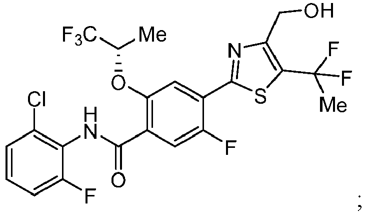 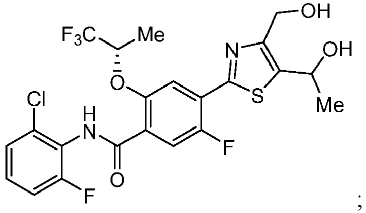 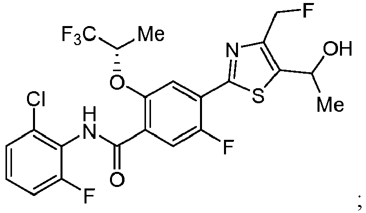 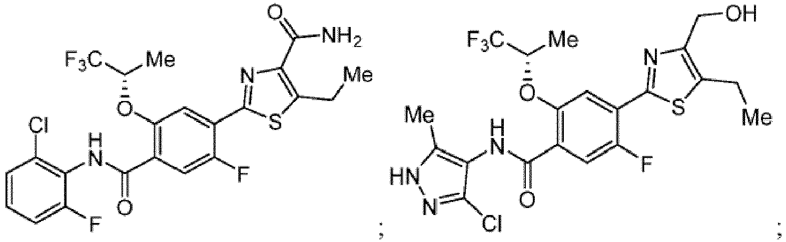 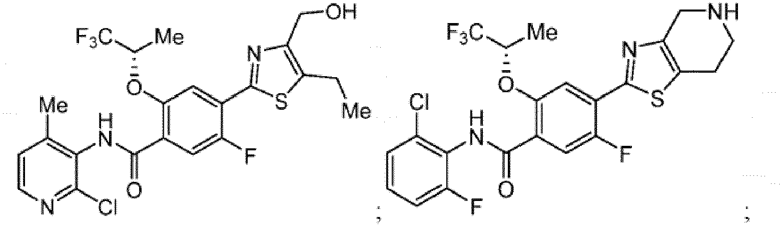 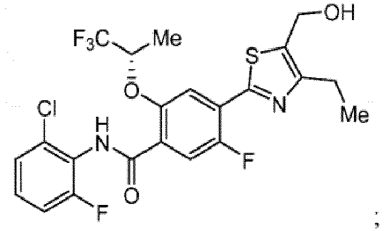 und 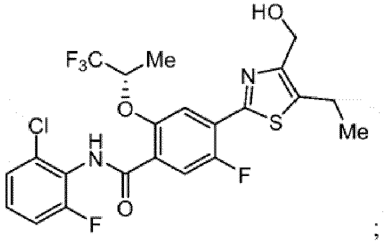 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(f) 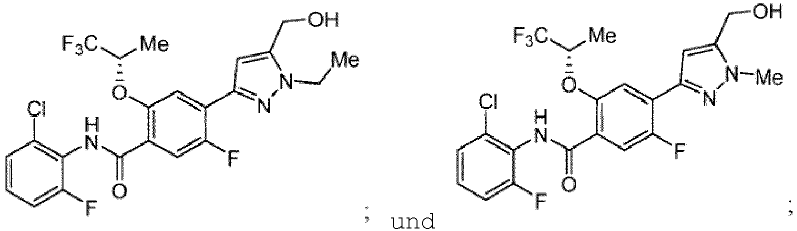 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(g) 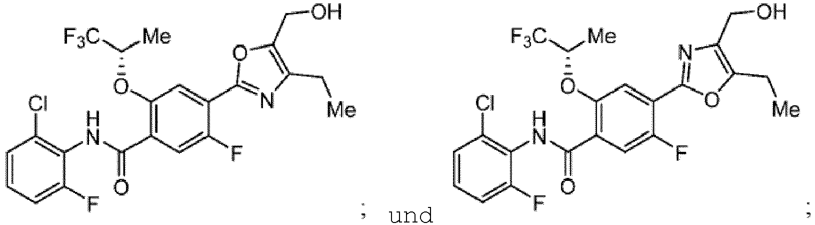 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

8. Verbindung nach Anspruch 4, bei der es sich um Folgendes handelt:
(a) 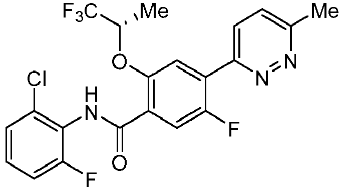 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(b) 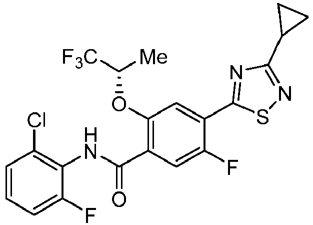 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(c) 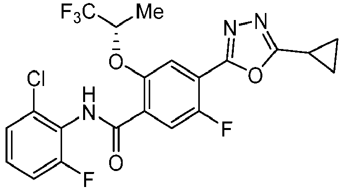 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

9. Verbindung nach Anspruch 4, ausgewählt aus der Gruppe bestehend aus:
(a) 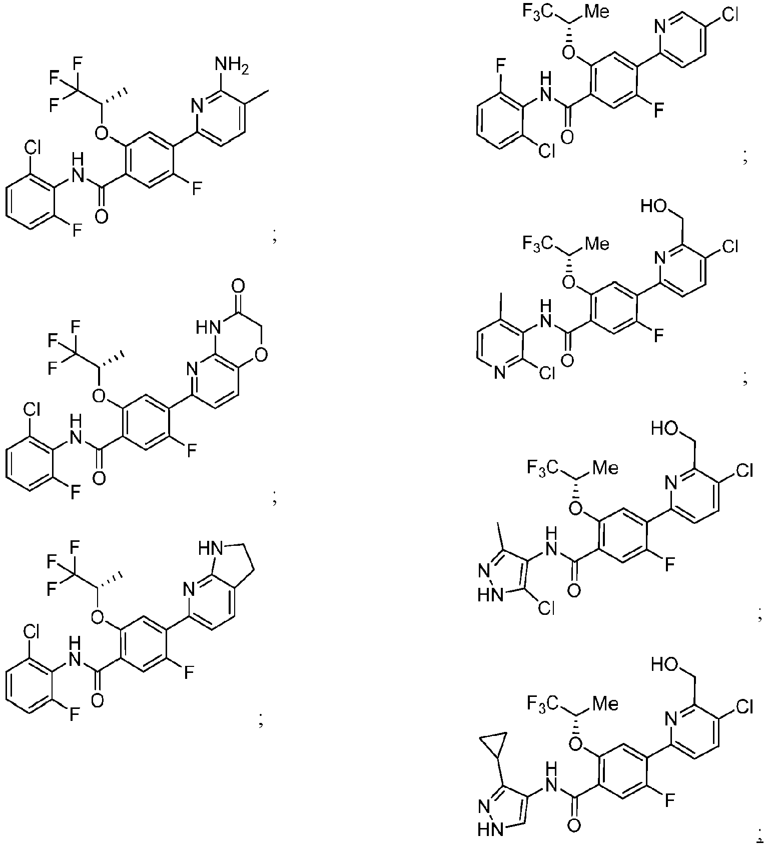 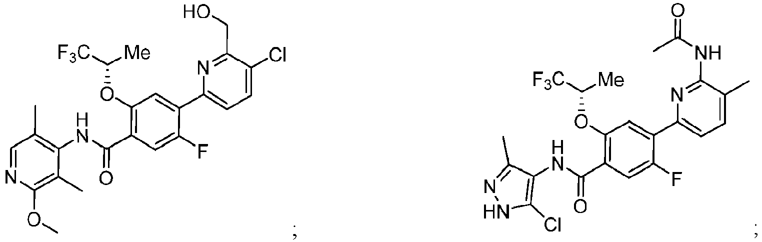 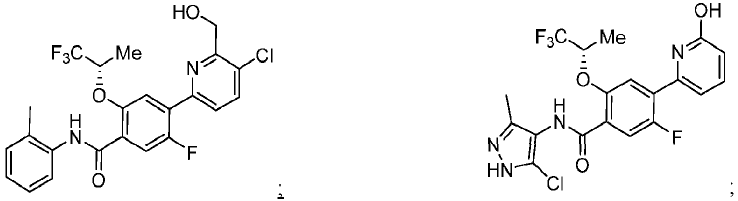 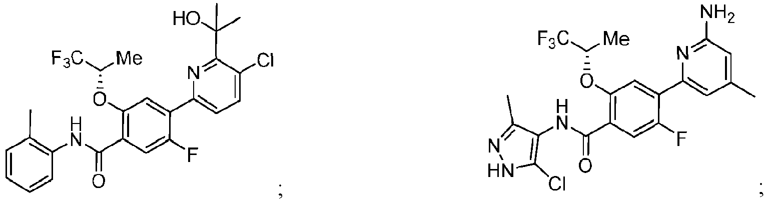 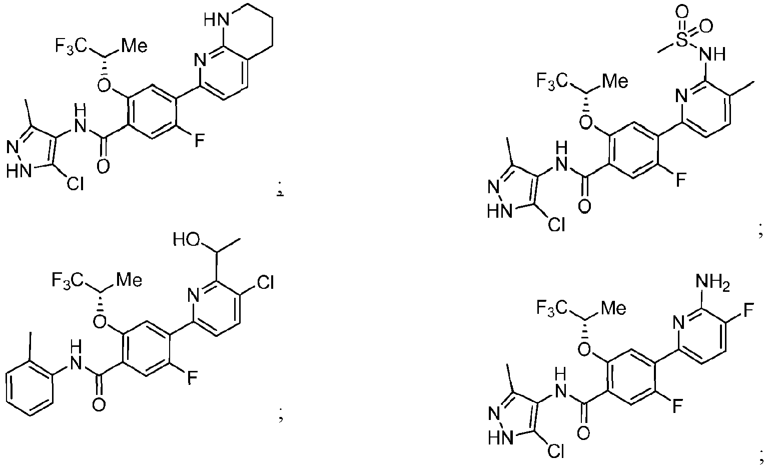 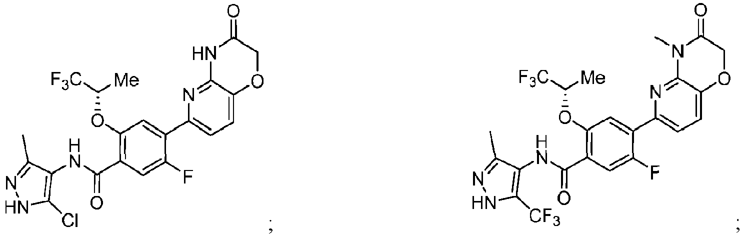 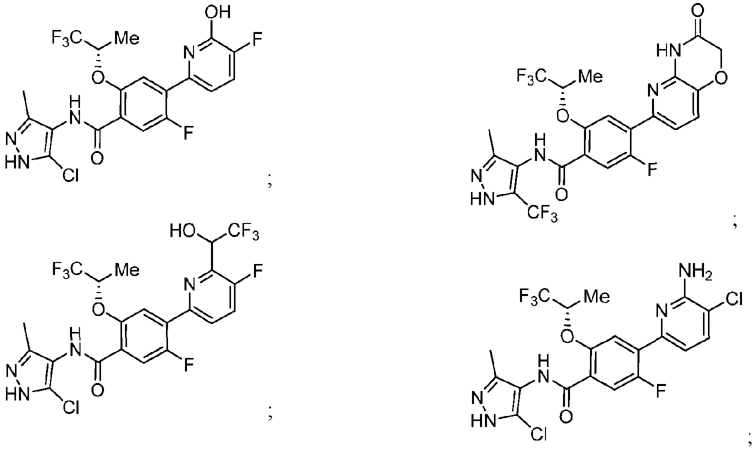 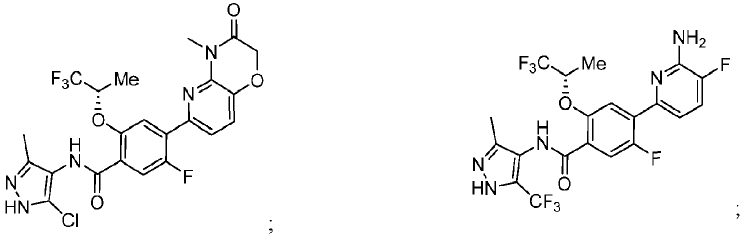 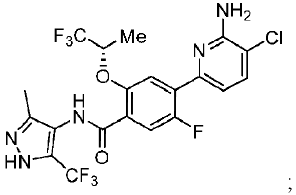 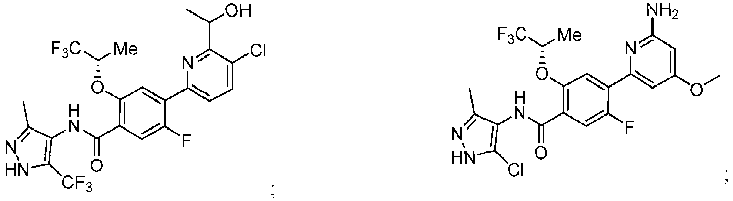 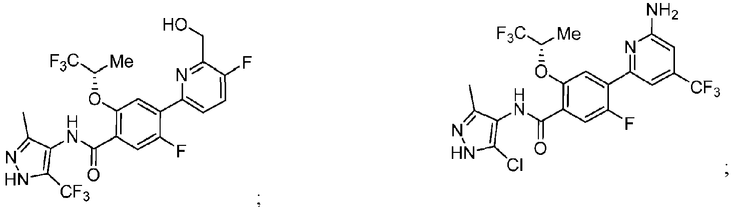 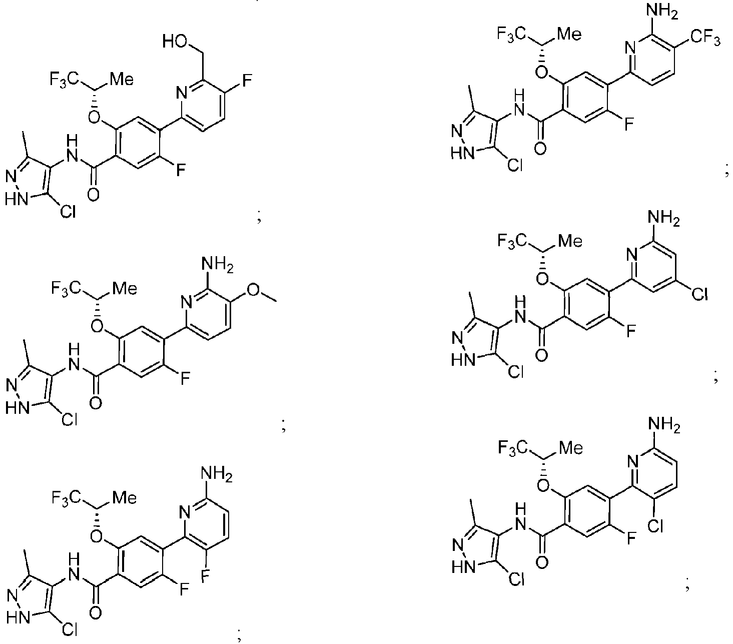 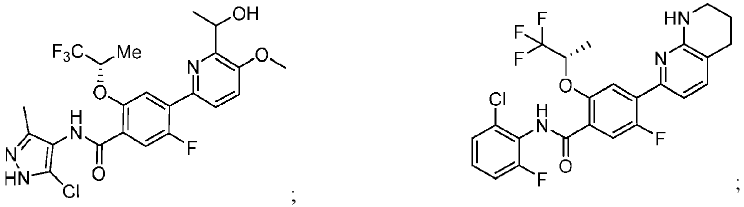 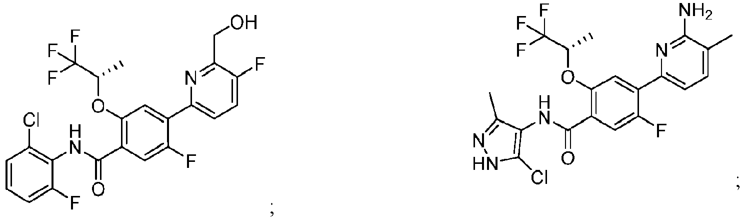 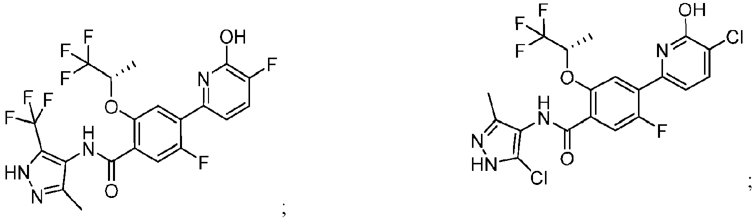 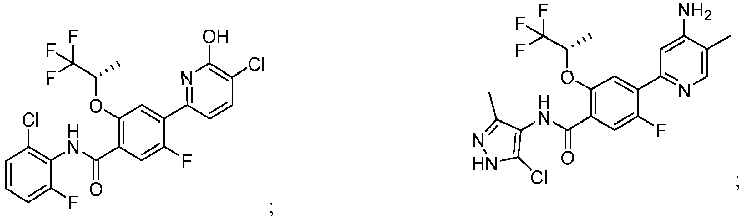 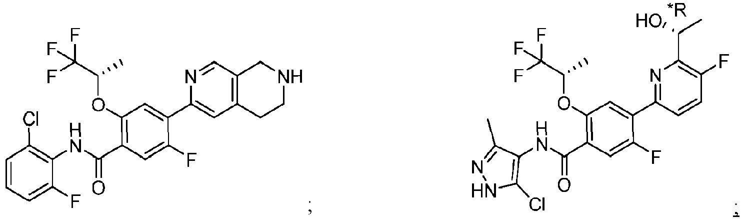 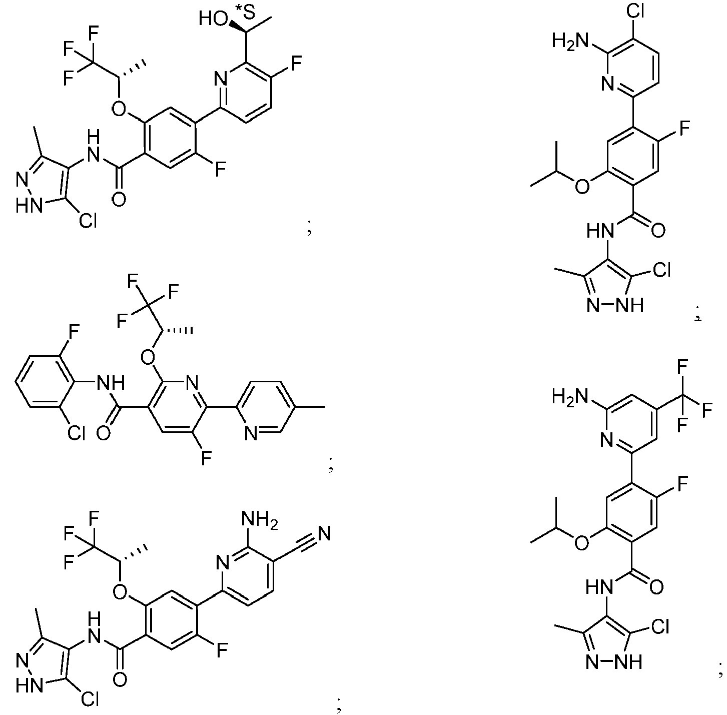 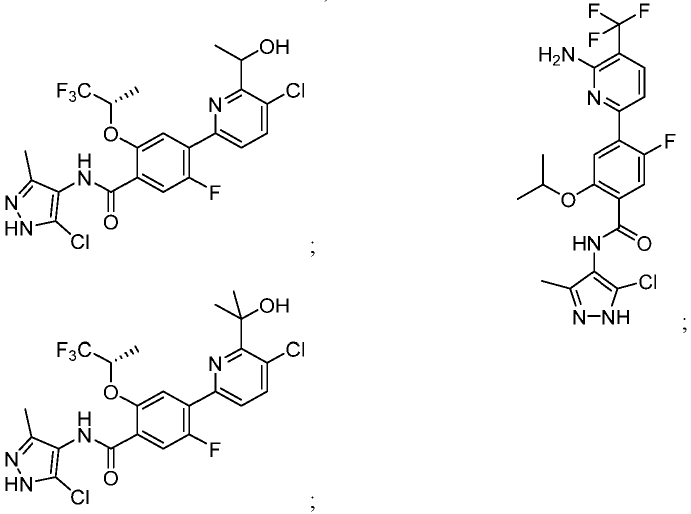 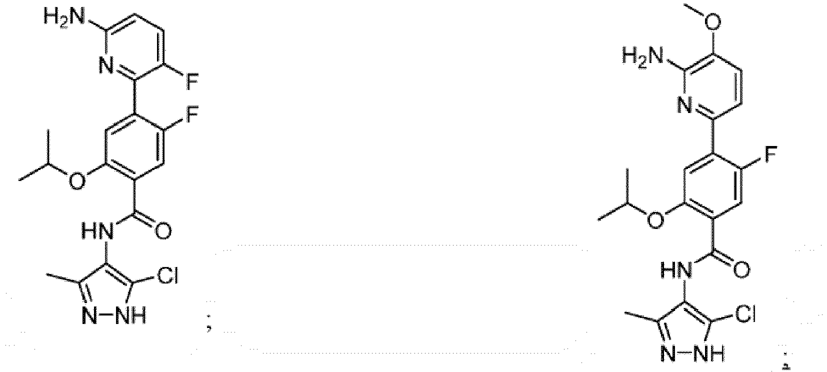 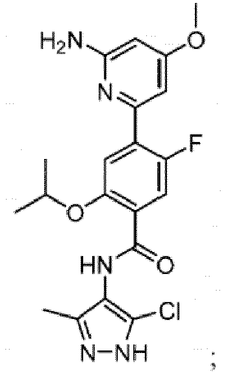 und oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(b) 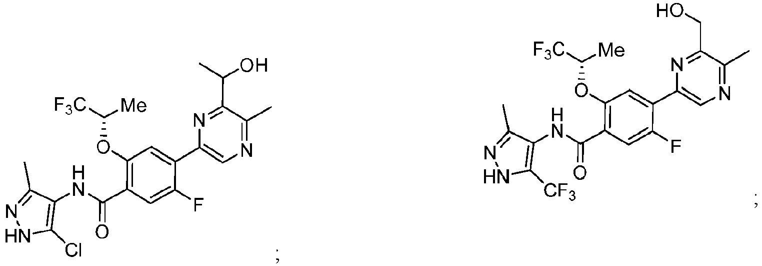 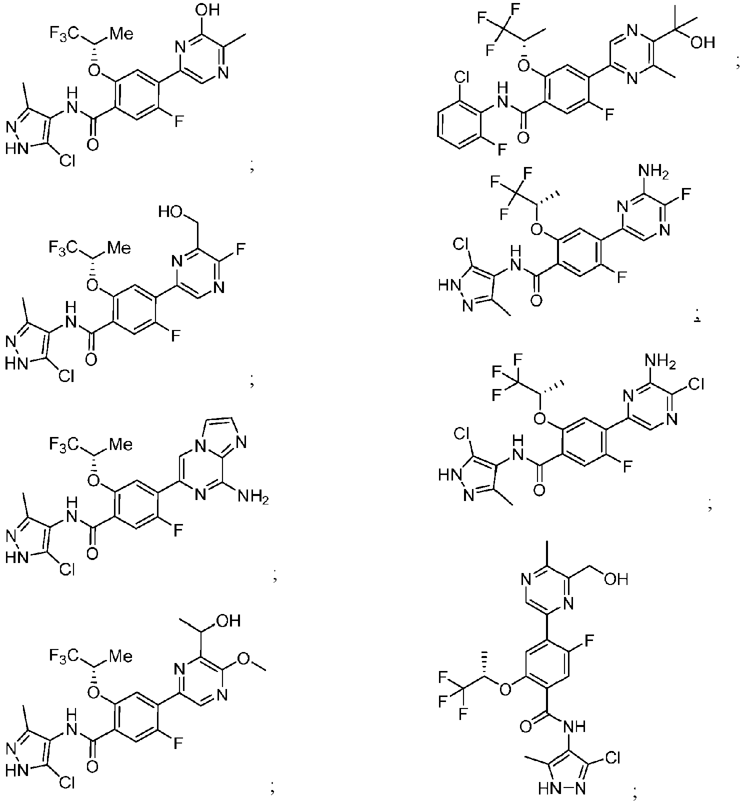 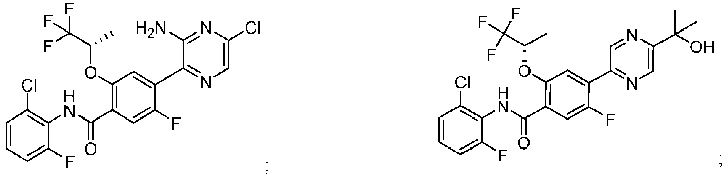 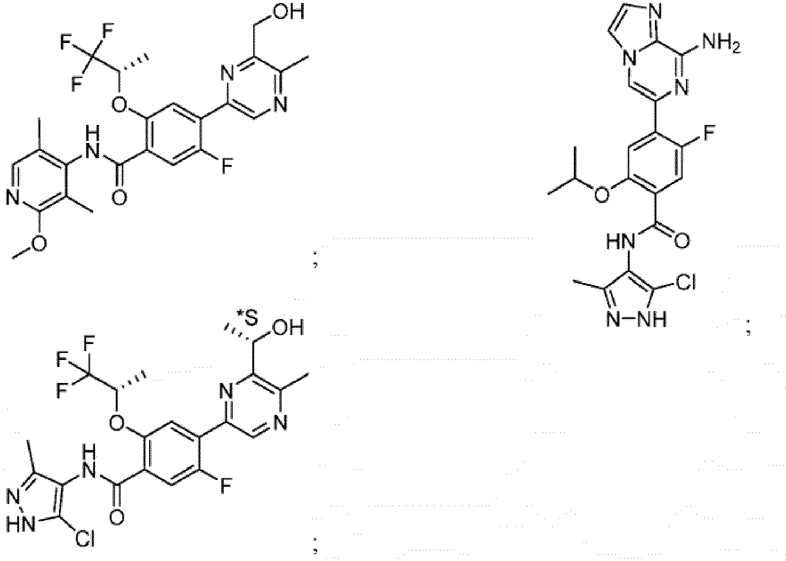 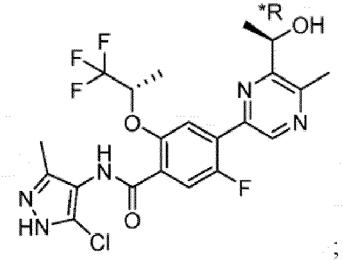 und oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(c) 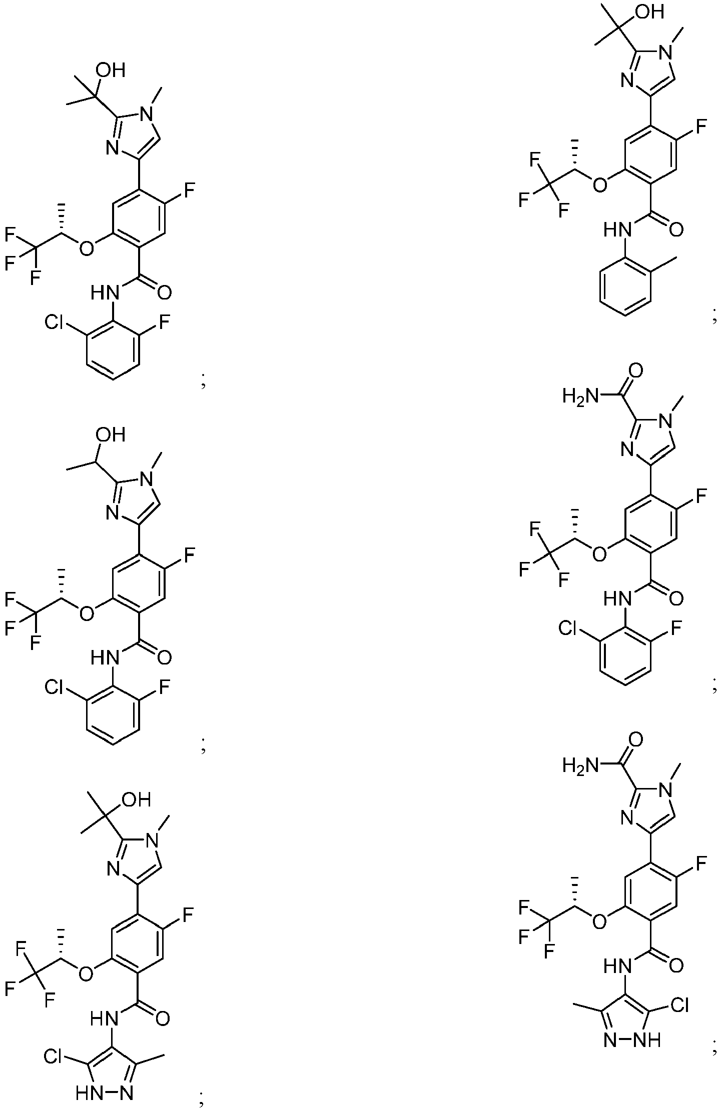 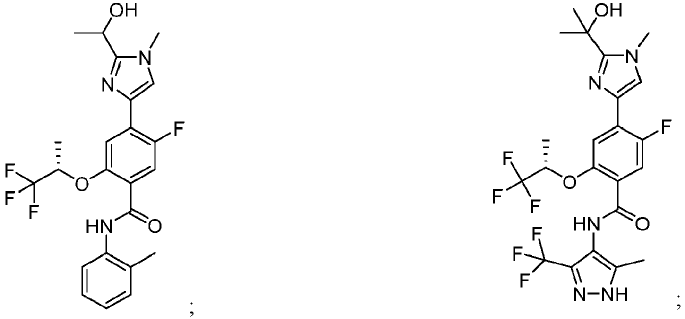 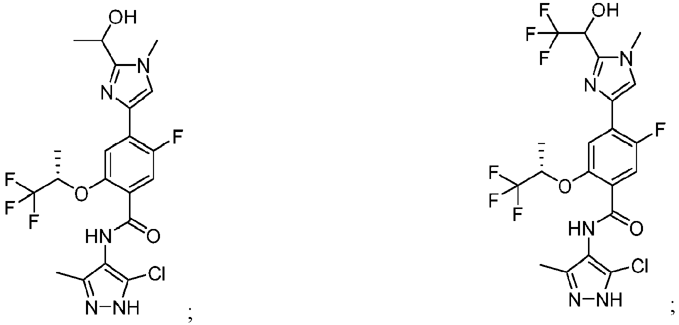 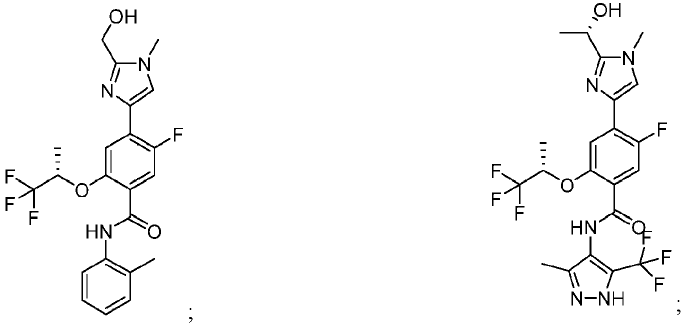 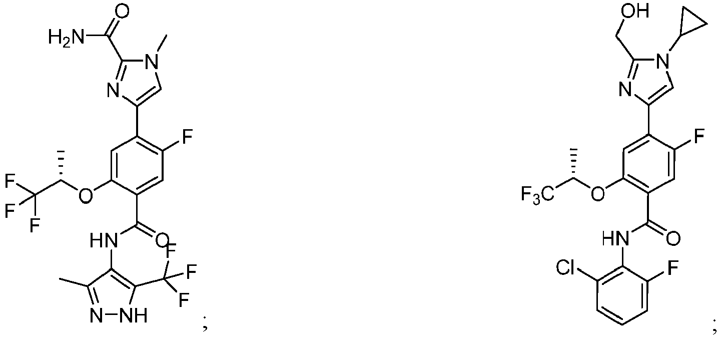 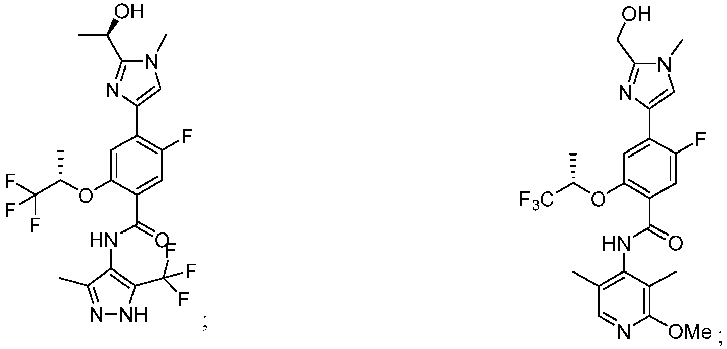 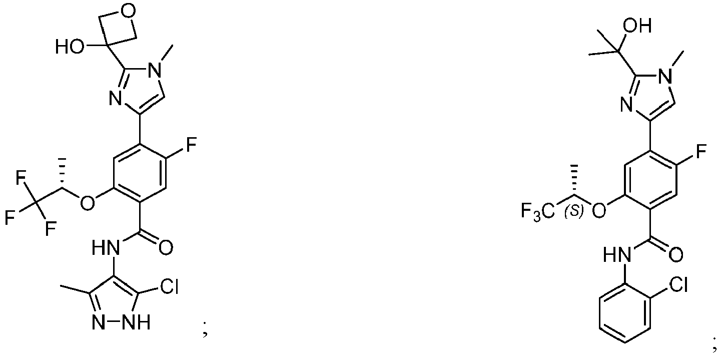 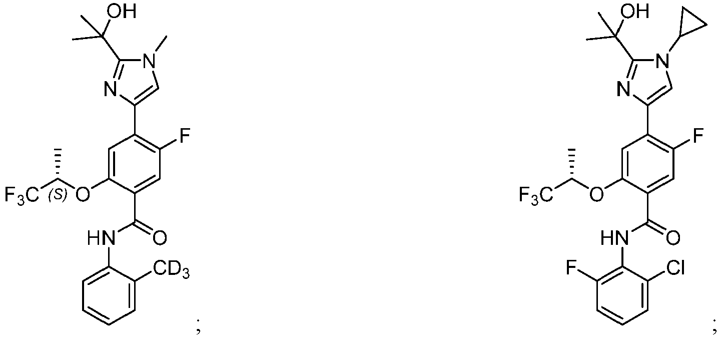 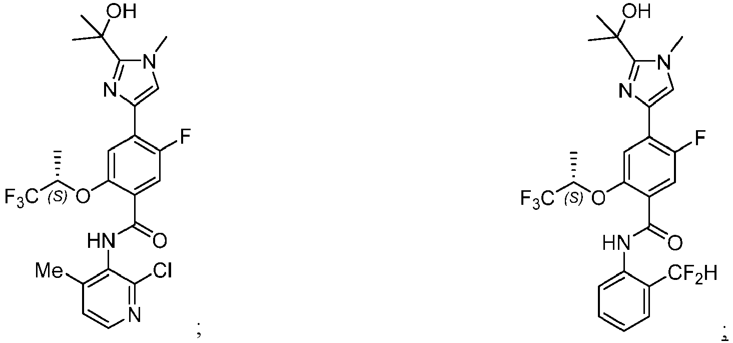 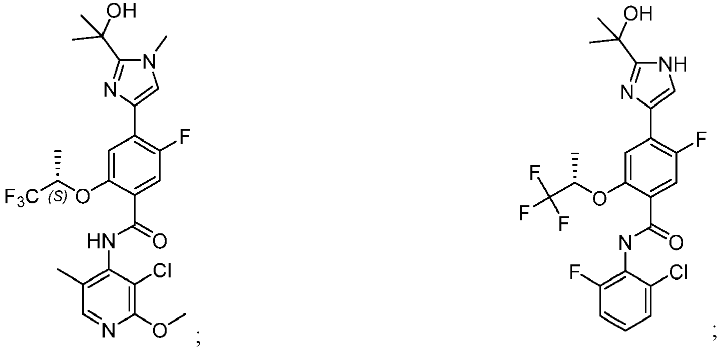 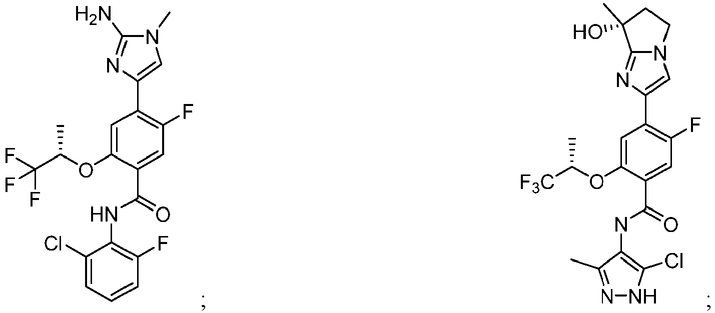 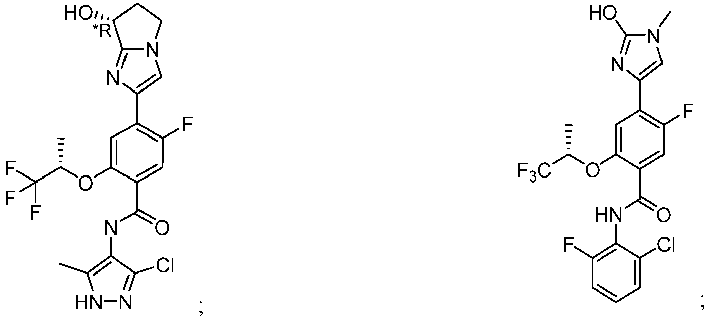 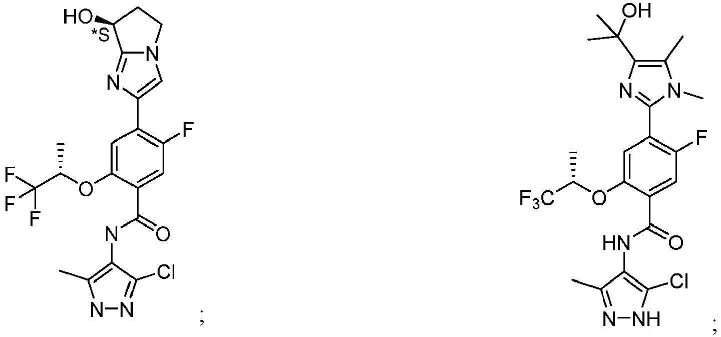 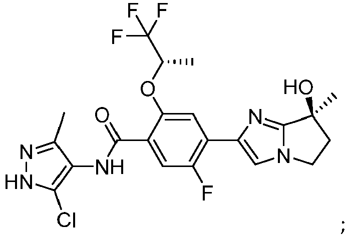 und 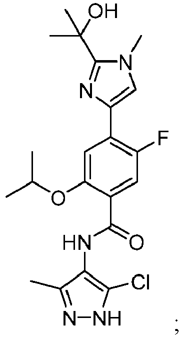 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(d) 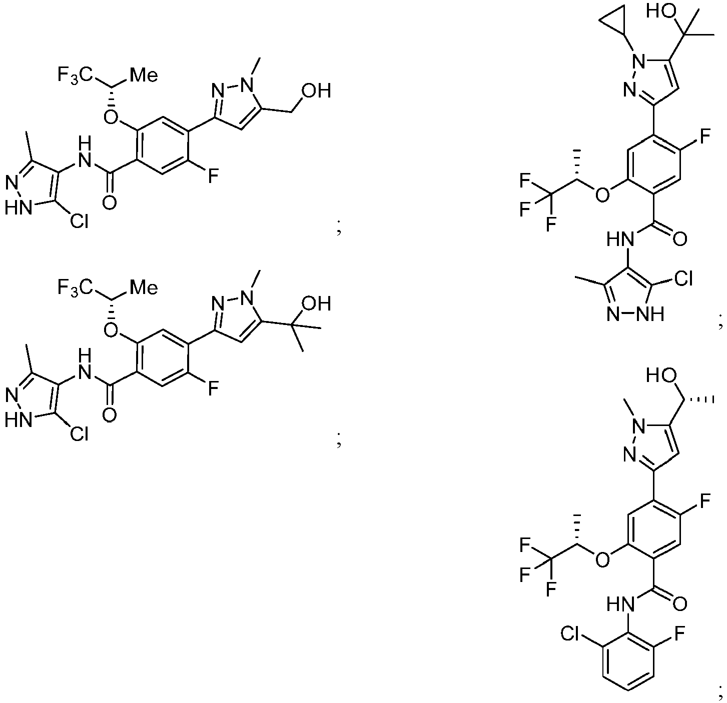 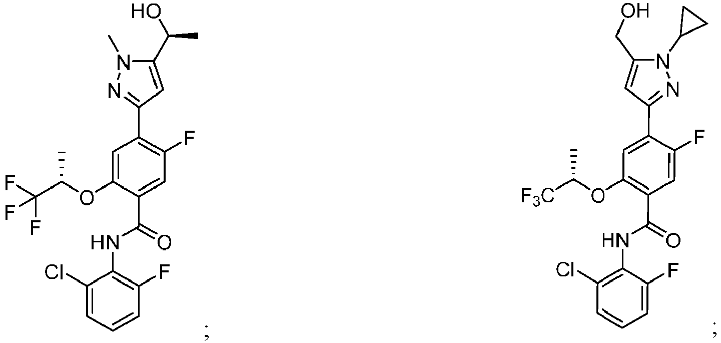 und 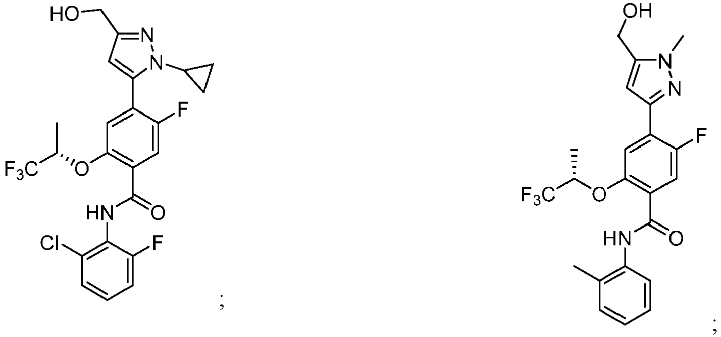 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

10. Verbindung nach Anspruch 4, bei der es sich um 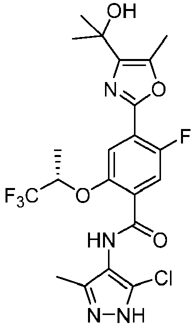 handelt; oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

11. Verbindung nach Anspruch 4, ausgewählt aus der Gruppe bestehend aus:
(a) 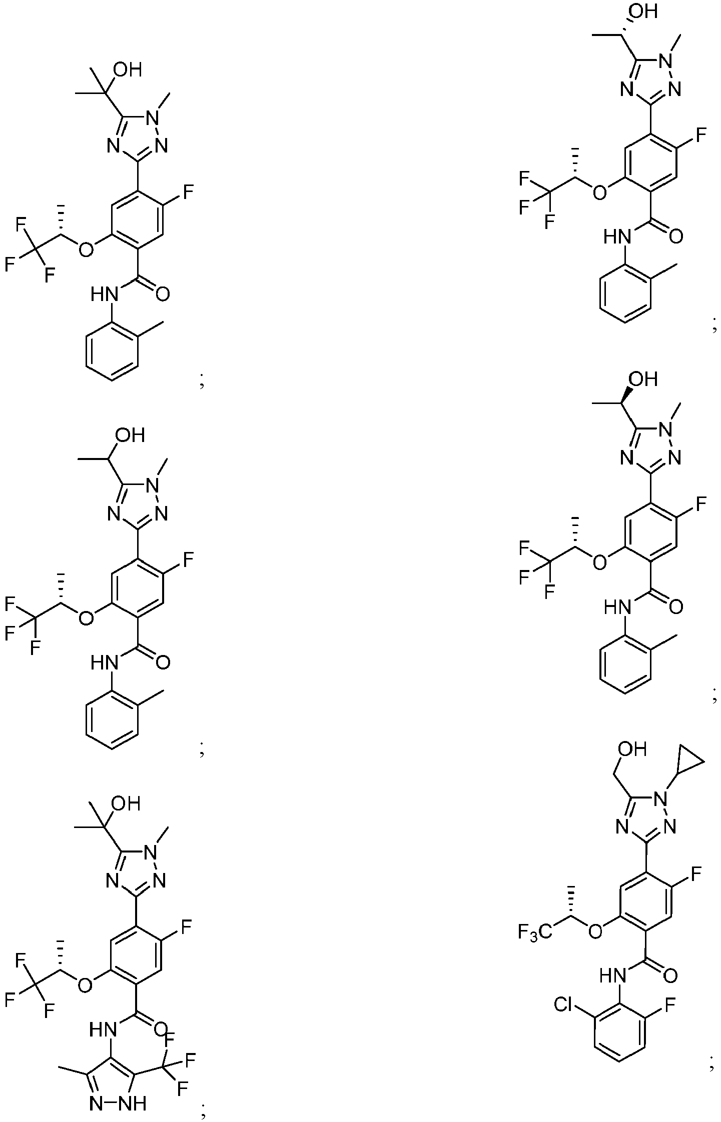 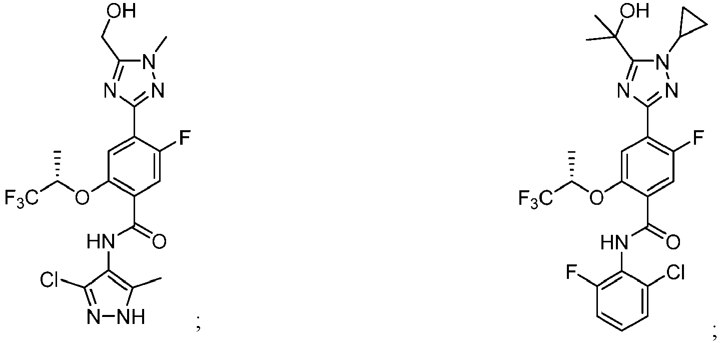 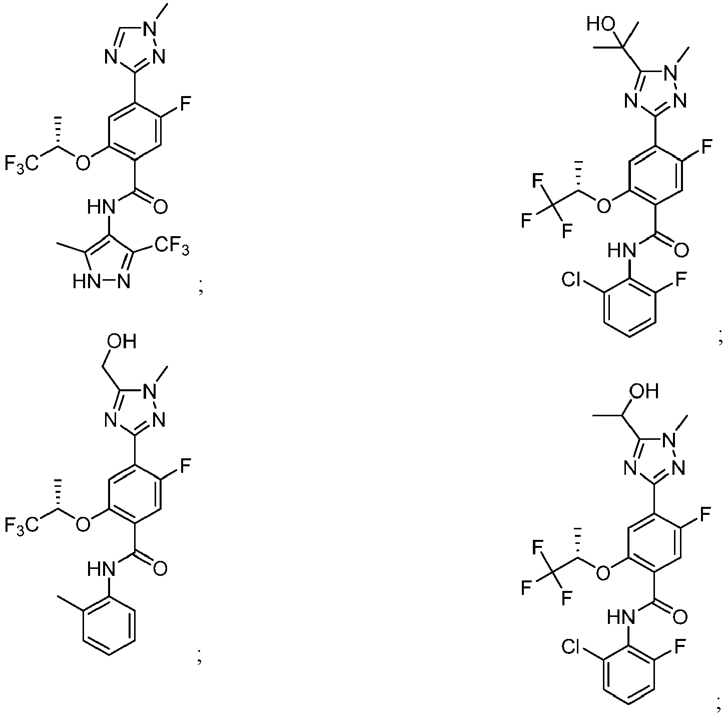 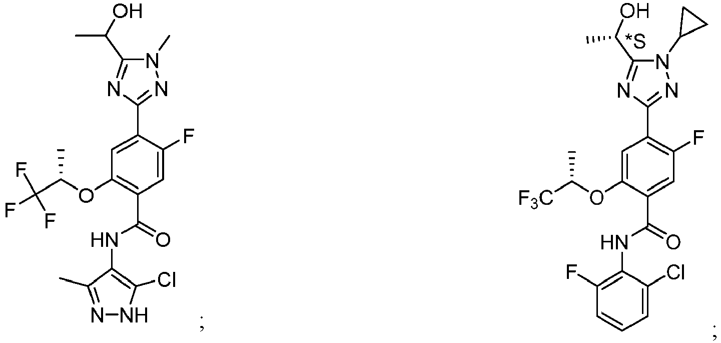 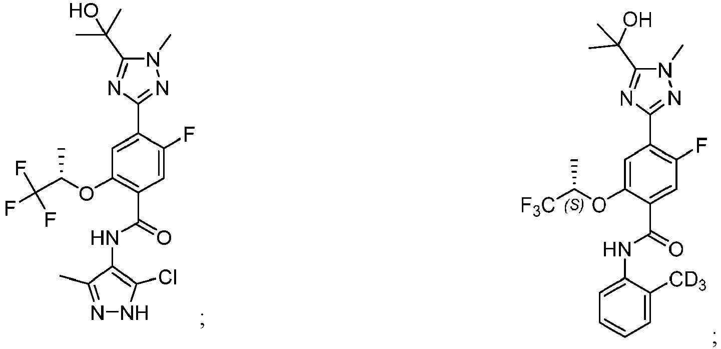 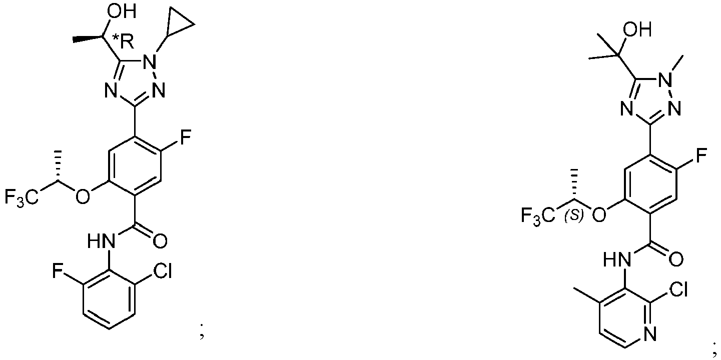 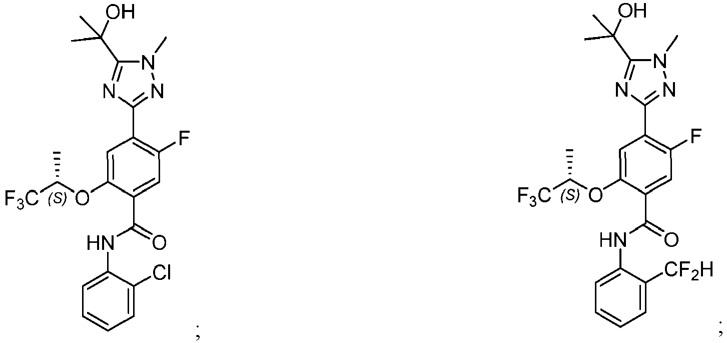 und 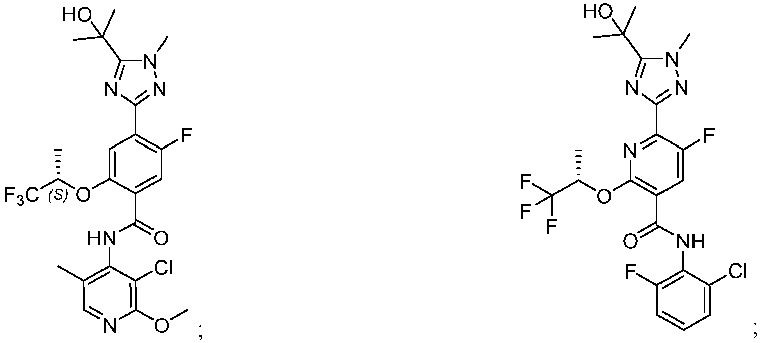 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(b) 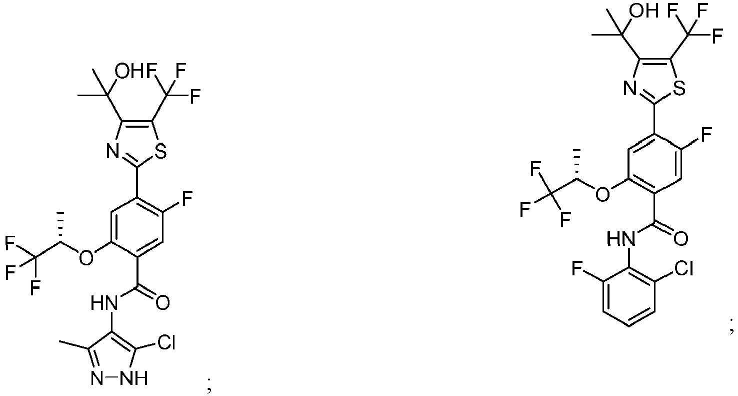 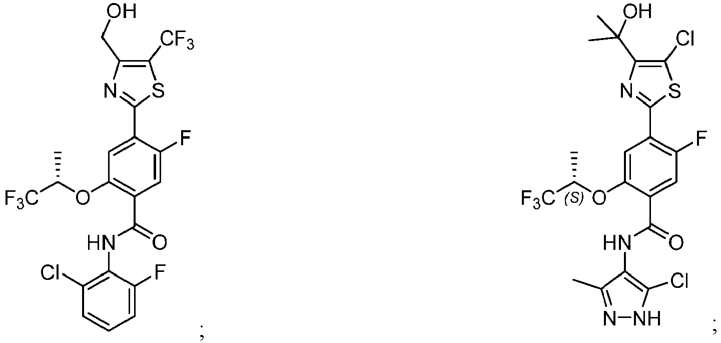 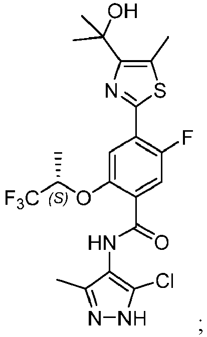 und oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon; oder
(c) 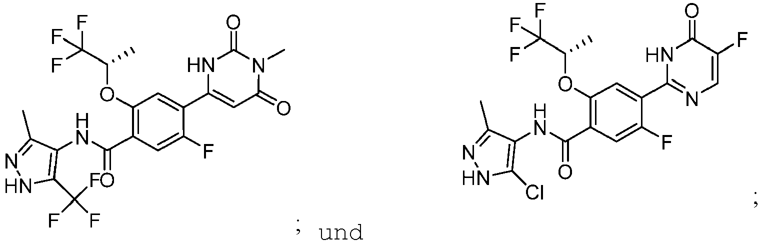 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon.

12. Pharmazeutische Zusammensetzung, umfassend: (A) eine Verbindung nach einem der Ansprüche 1 bis 11 oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon und (B) mindestens einen pharmazeutisch unbedenklichen Hilfsstoff.

13. Pharmazeutische Zusammensetzung, umfassend eine wirksame Menge einer Verbindung nach Anspruch 1, ausgewählt aus der Gruppe bestehend aus:
(a) 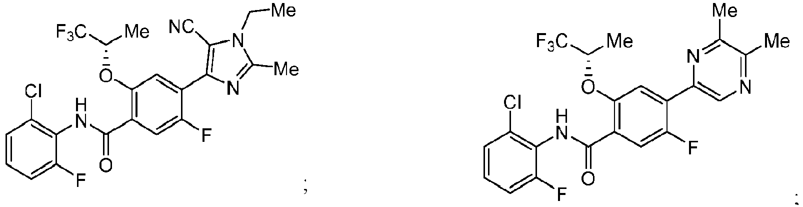 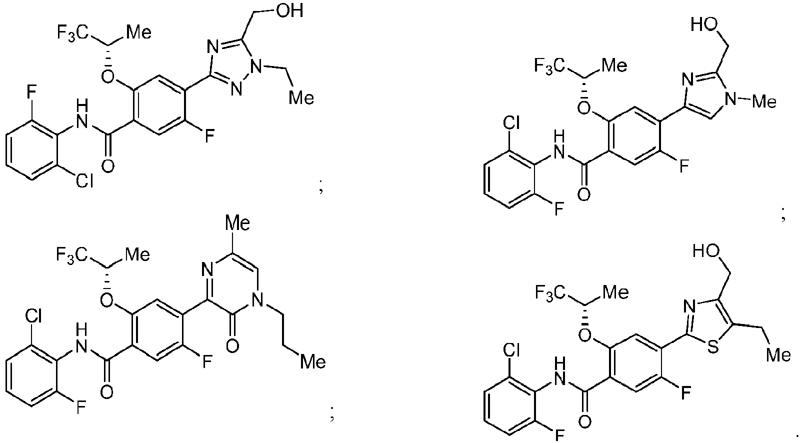 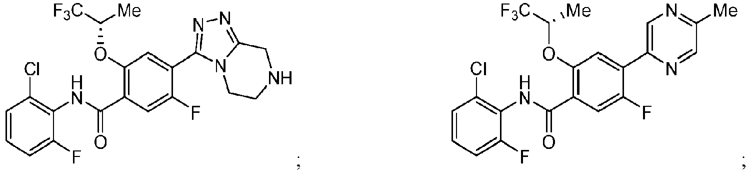 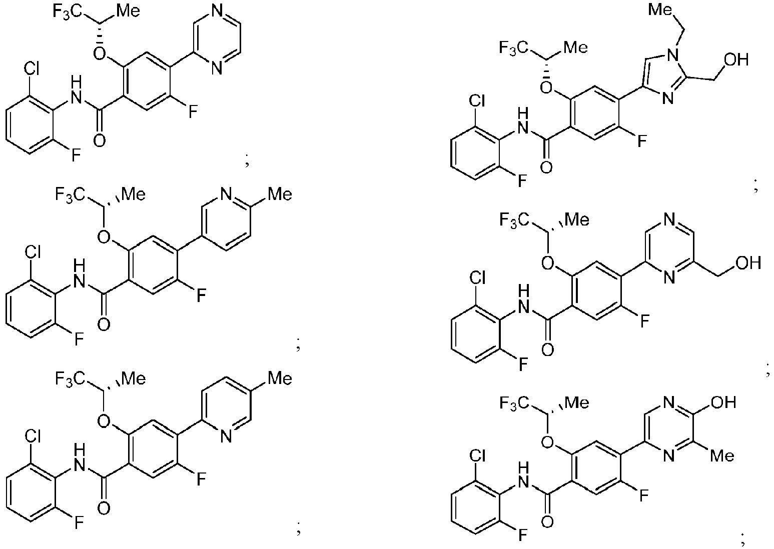 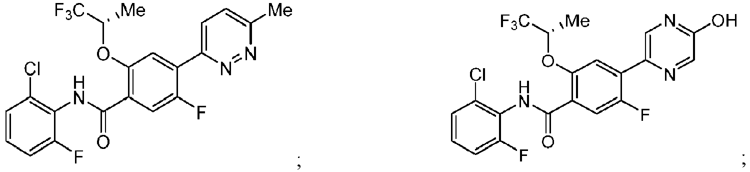 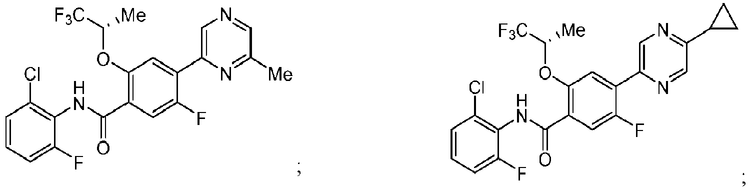 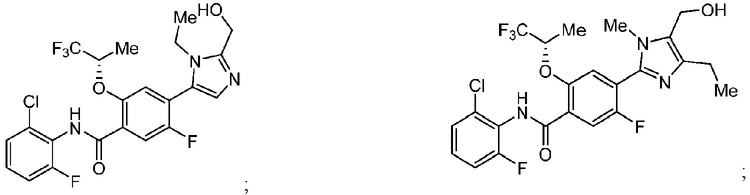 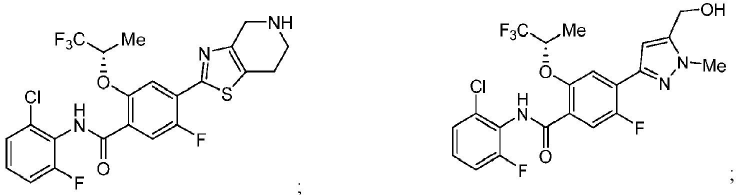 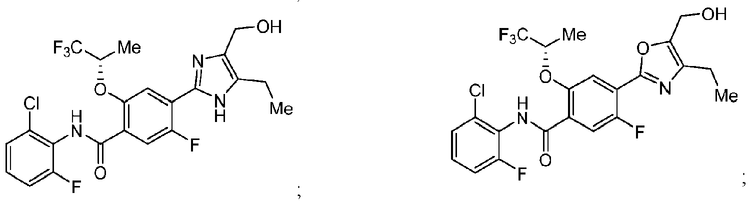 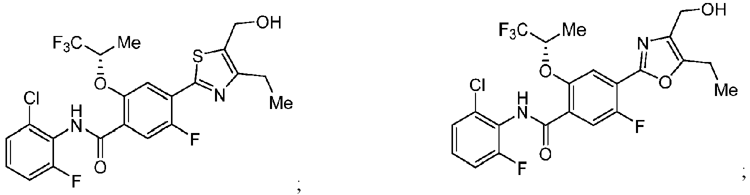 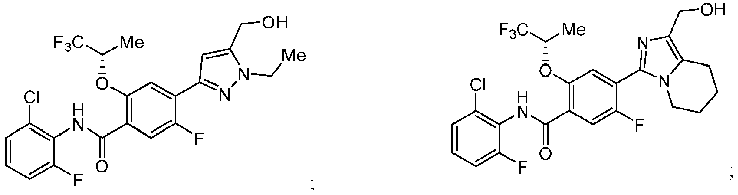 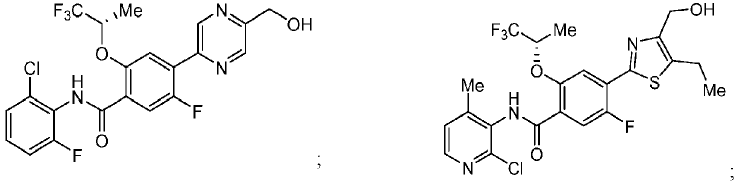 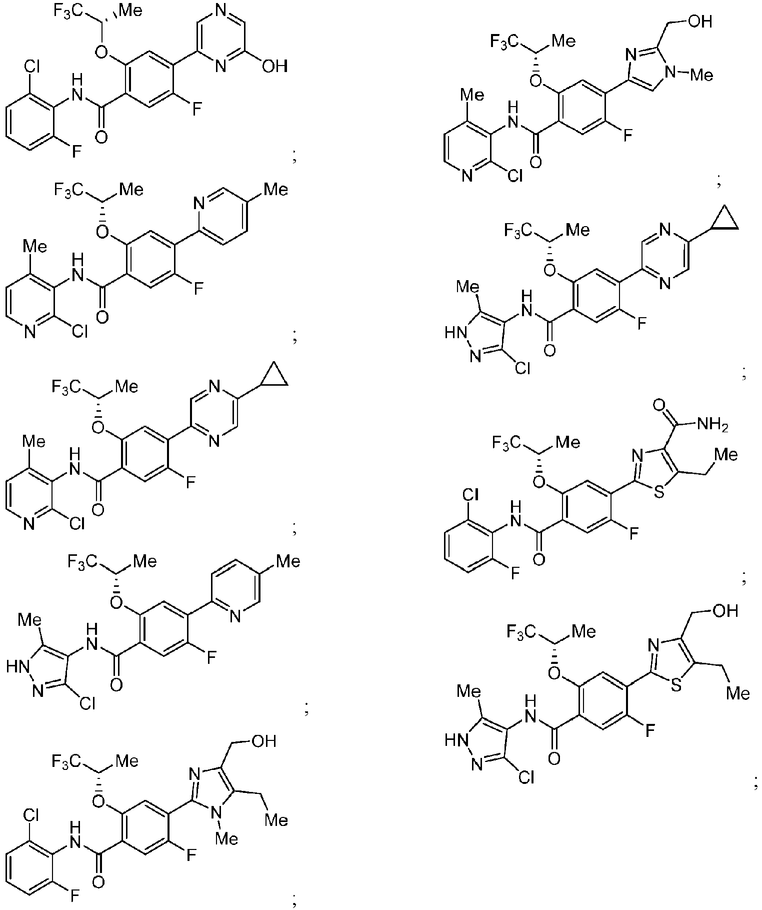 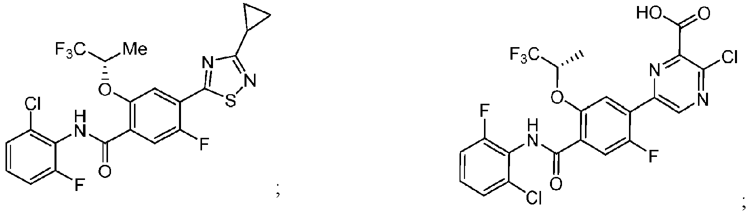 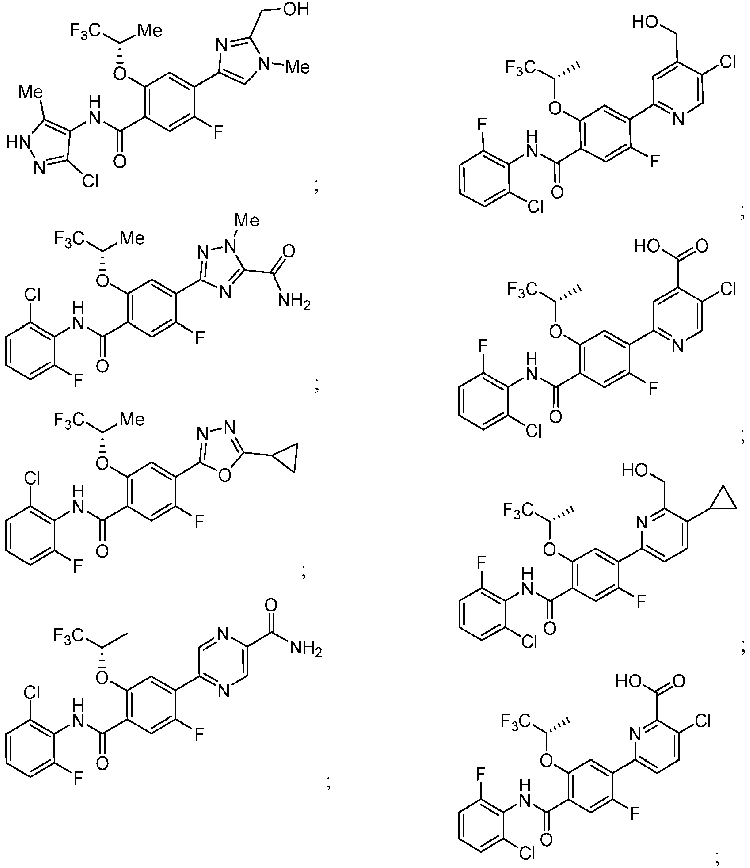 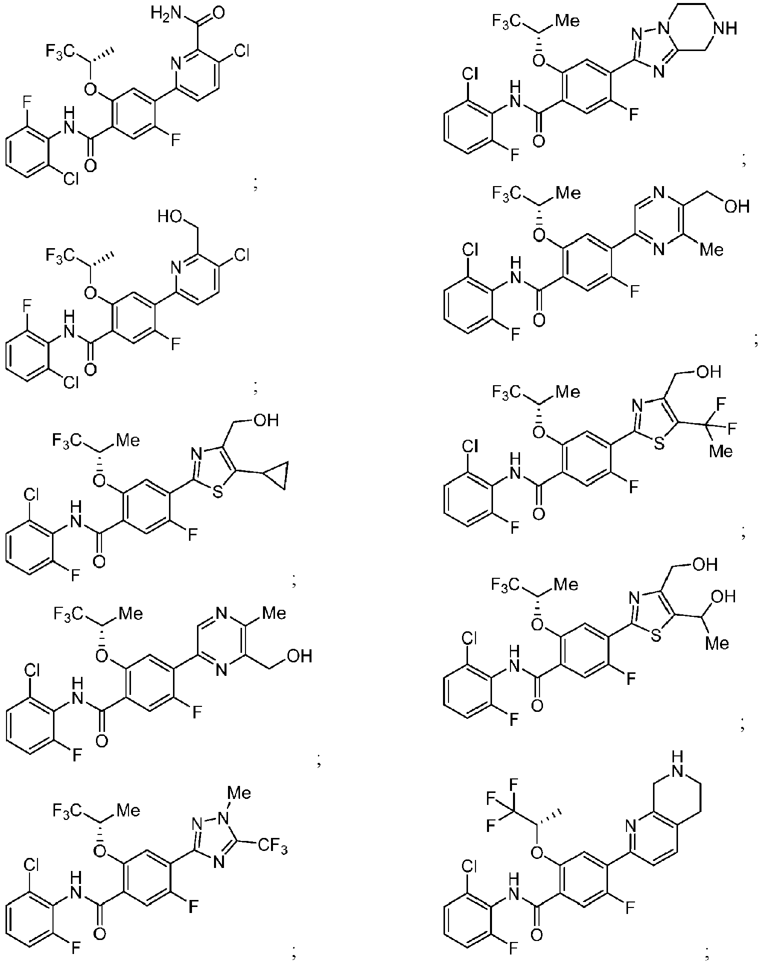 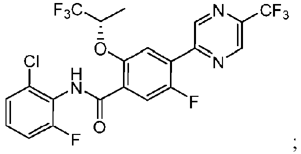 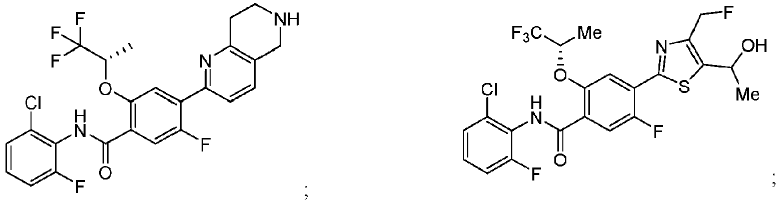 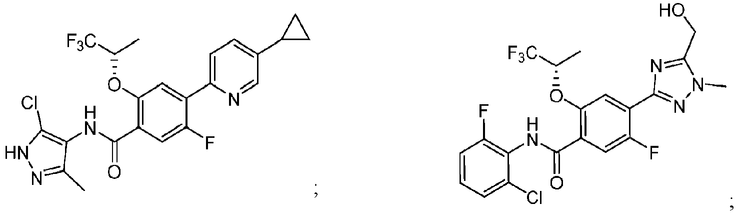 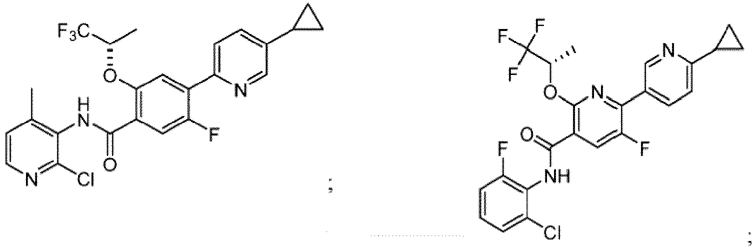 und 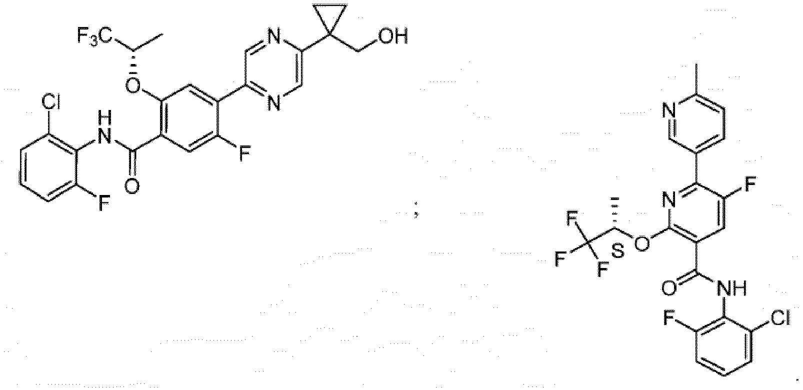 oder eines pharmazeutisch unbedenklichen Salzes, eines pharmazeutisch unbedenklichen Solvats, eines pharmazeutisch unbedenklichen Stereoisomers, einer pharmazeutisch unbedenklichen Isotopenvariante oder eines pharmazeutisch unbedenklichen N-Oxids davon; und mindestens einen pharmazeutisch unbedenklichen Hilfsstoff; oder
(b) 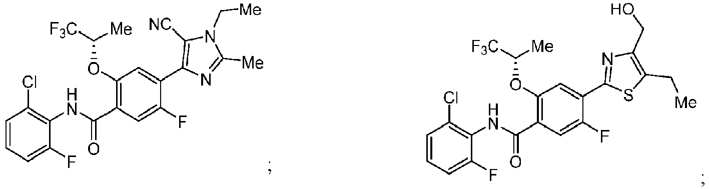 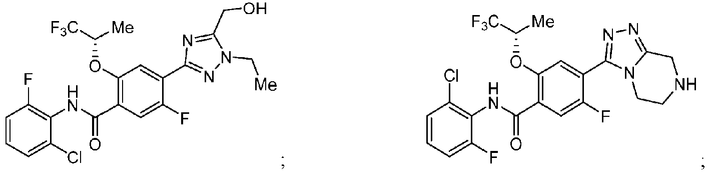 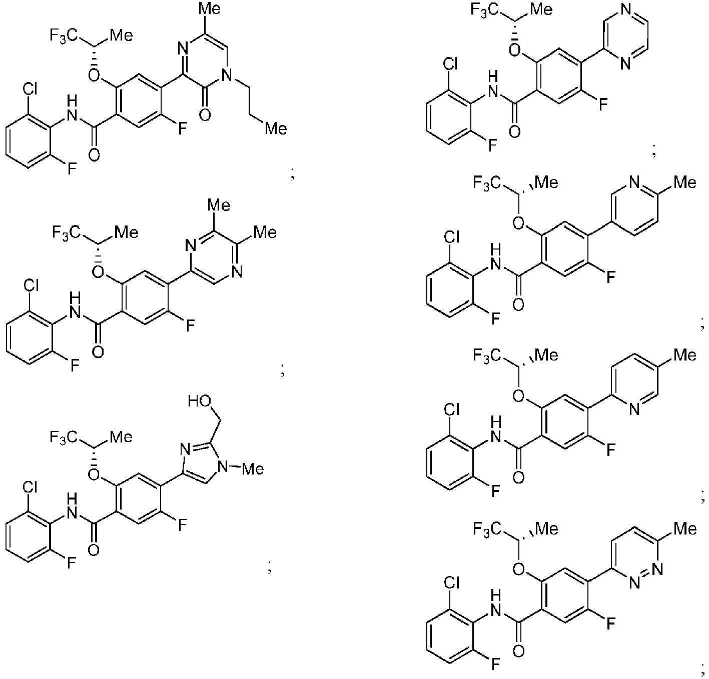 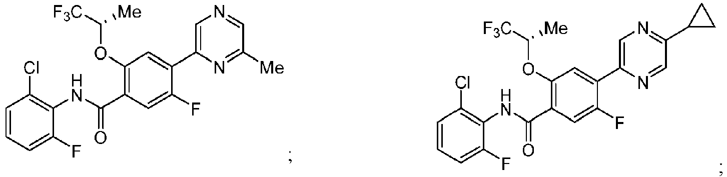 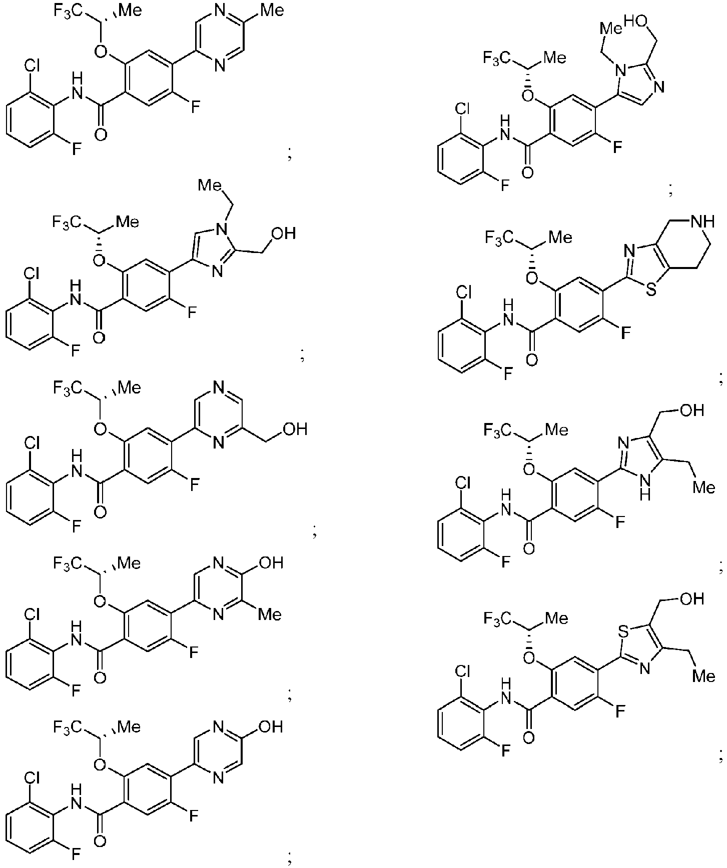 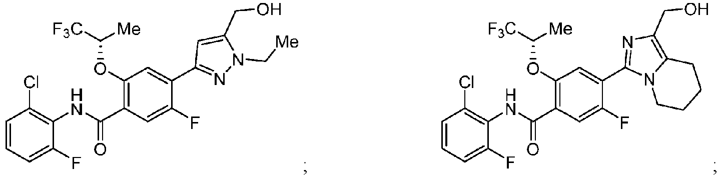 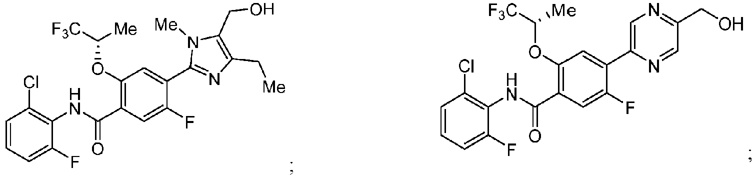 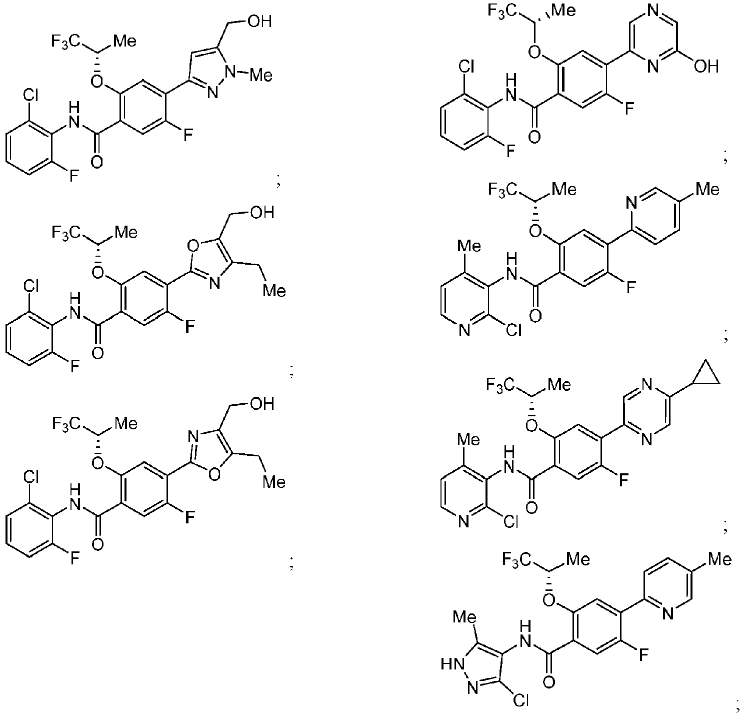 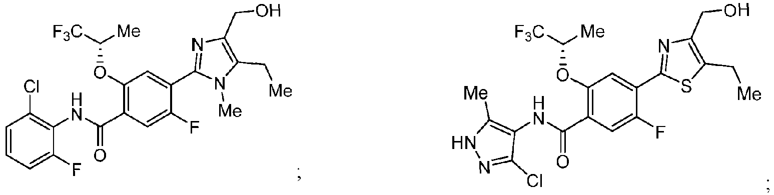 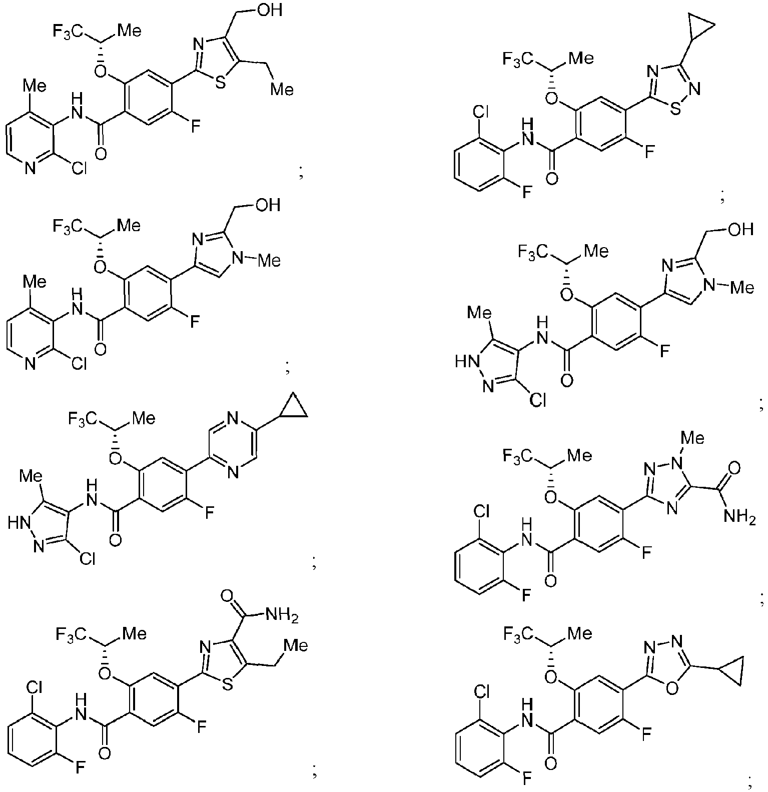 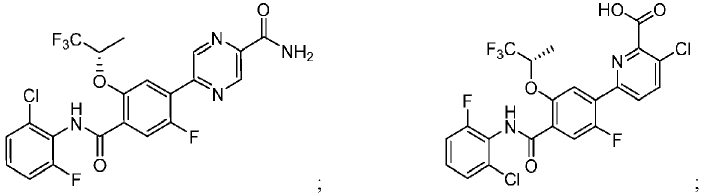 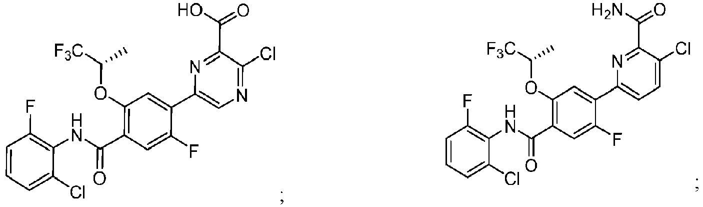 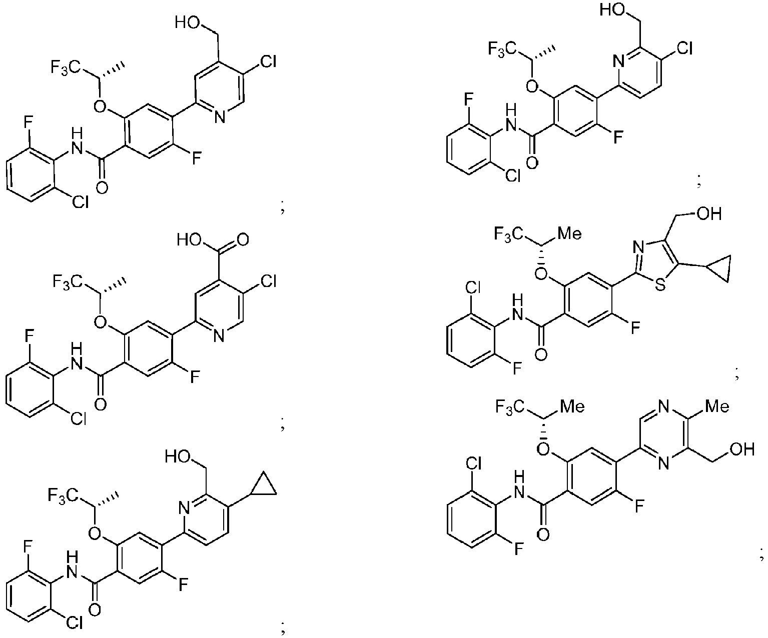 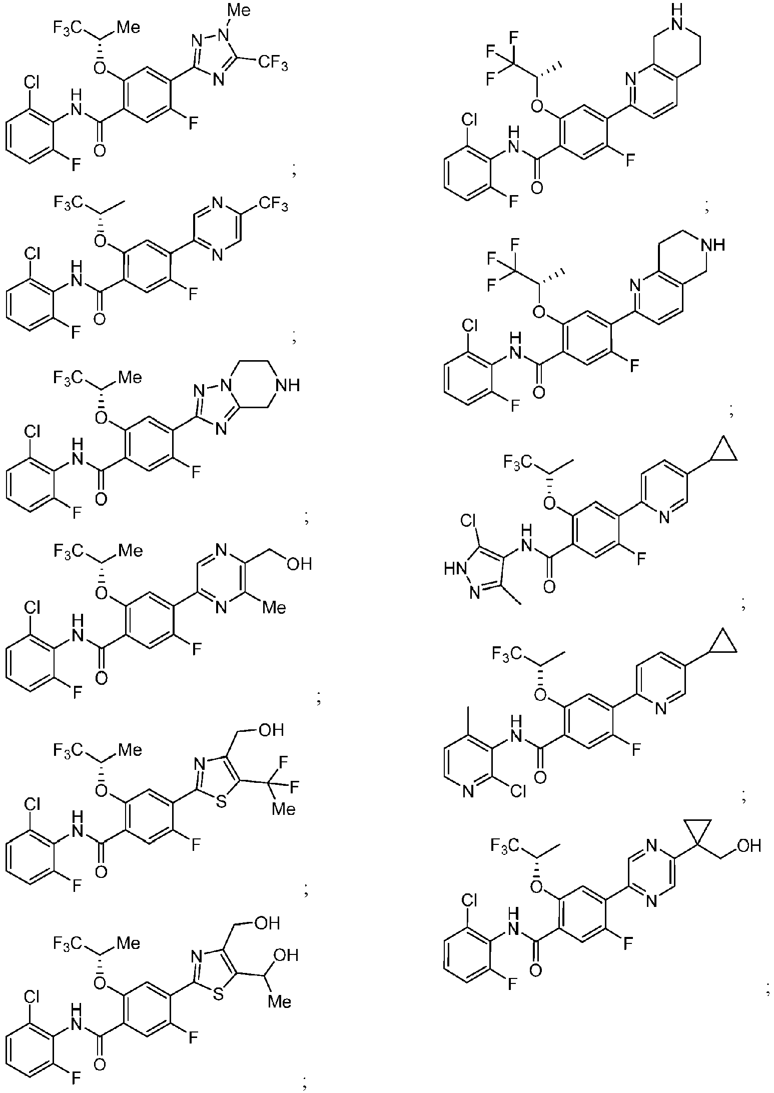 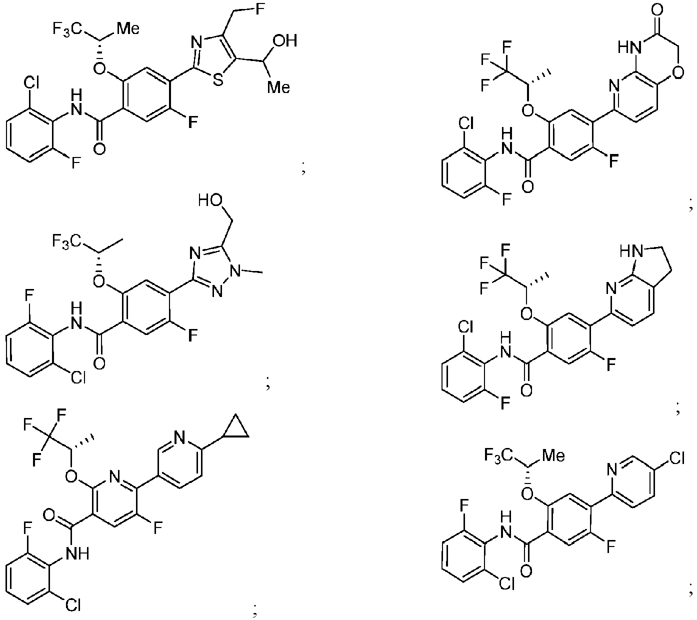 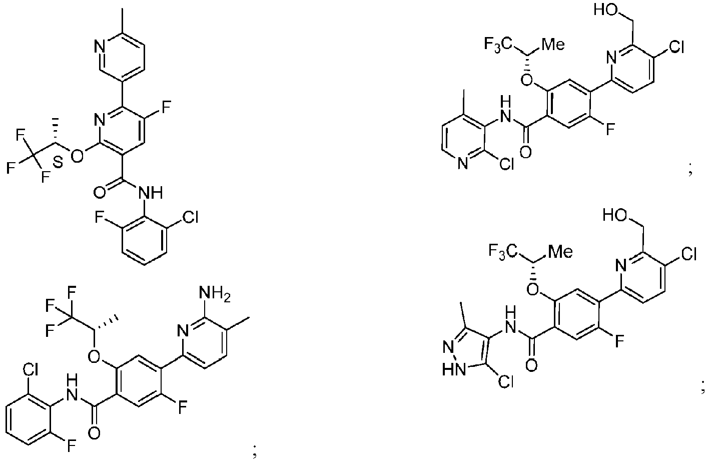 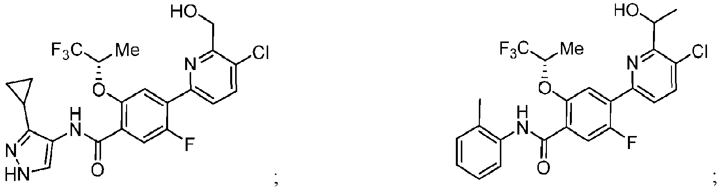 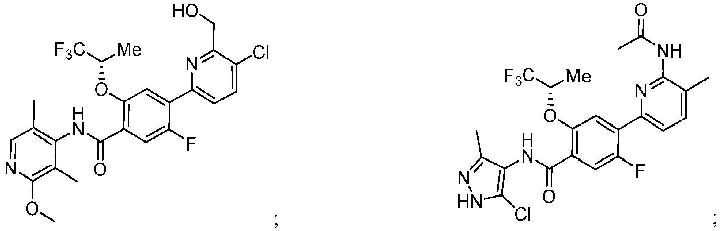 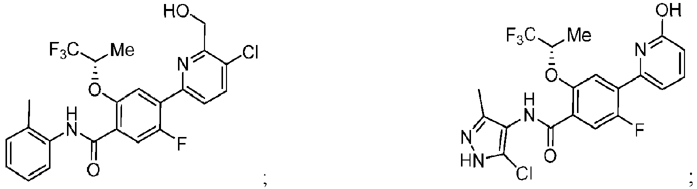 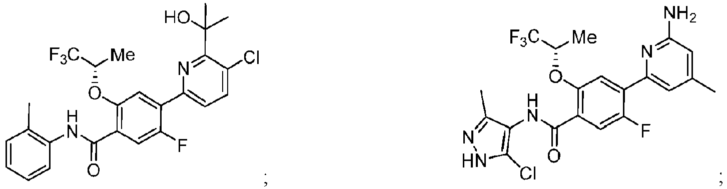 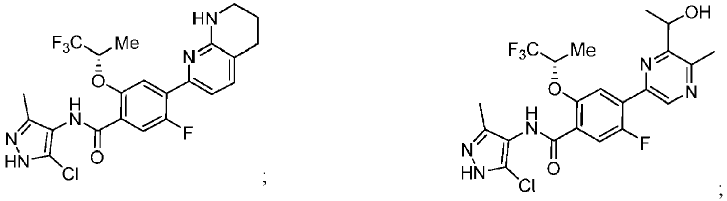 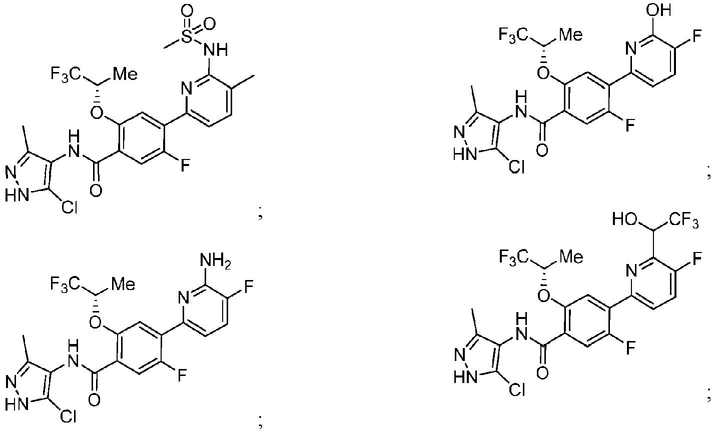 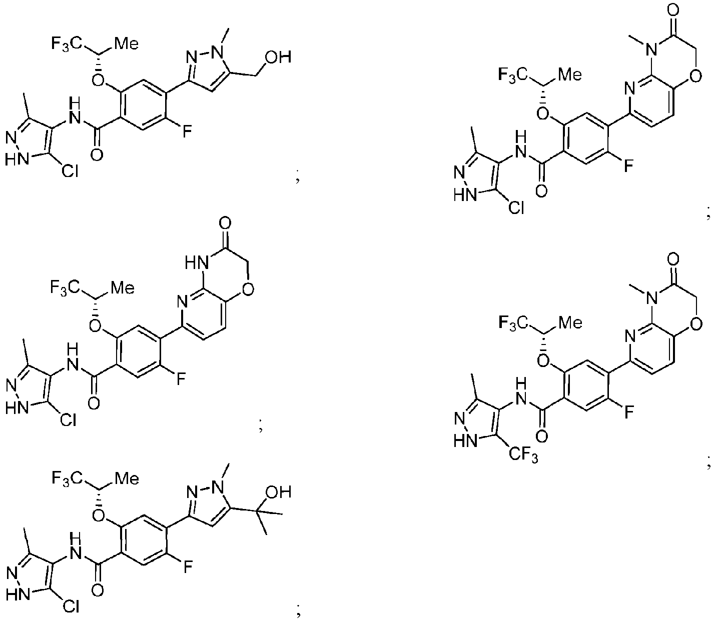 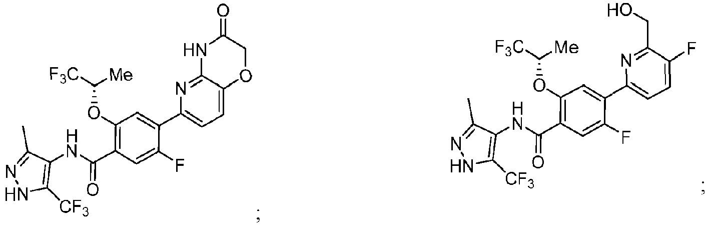 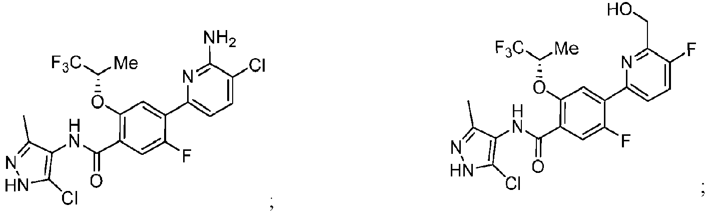 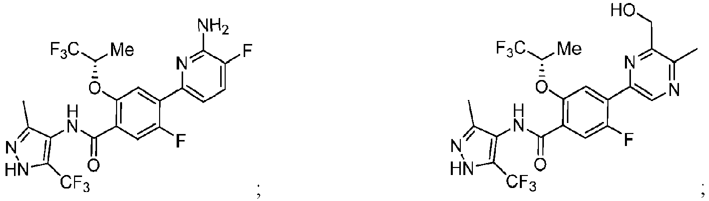 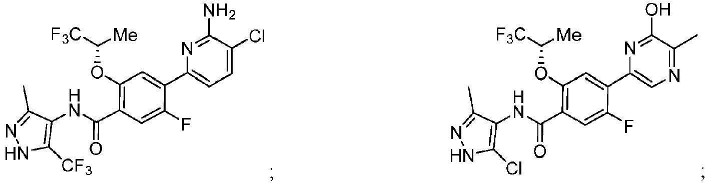 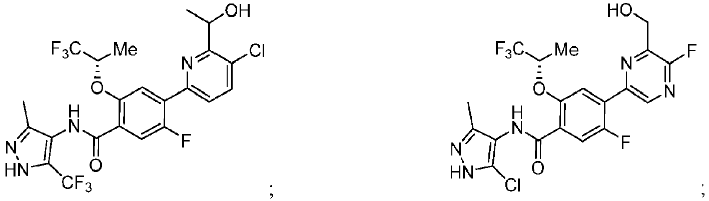 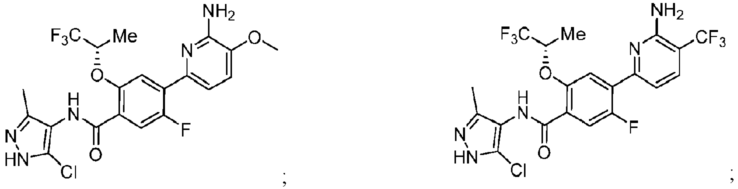 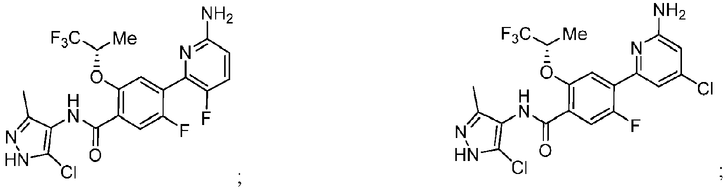 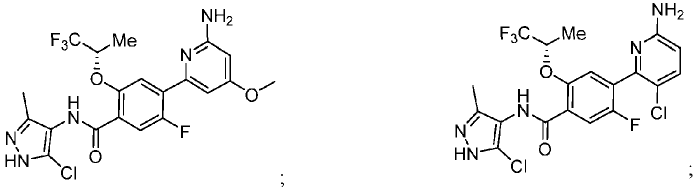 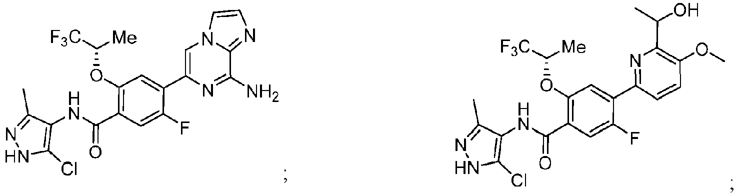 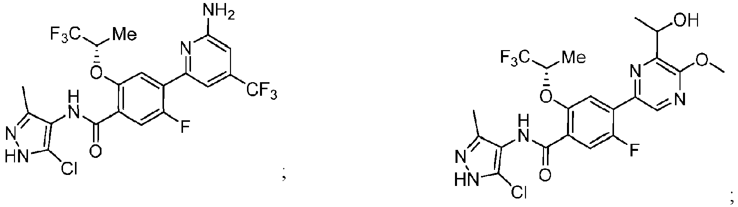 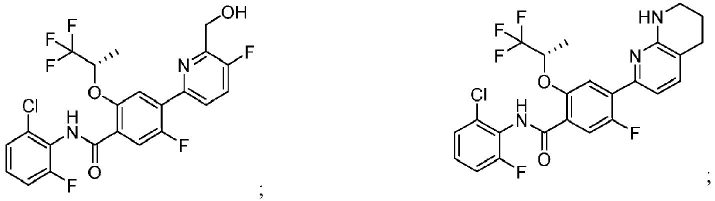 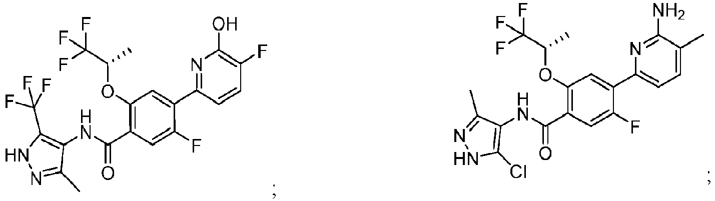 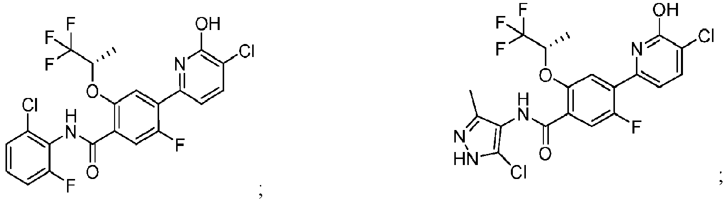 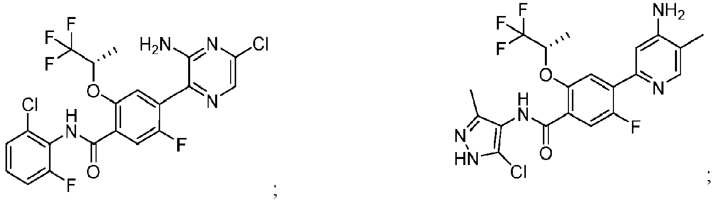 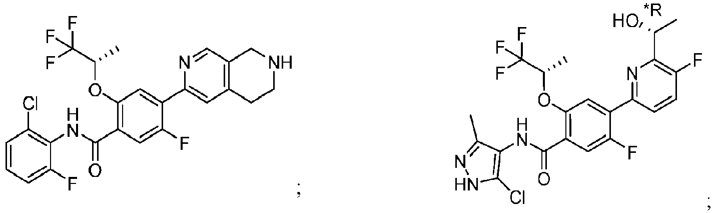 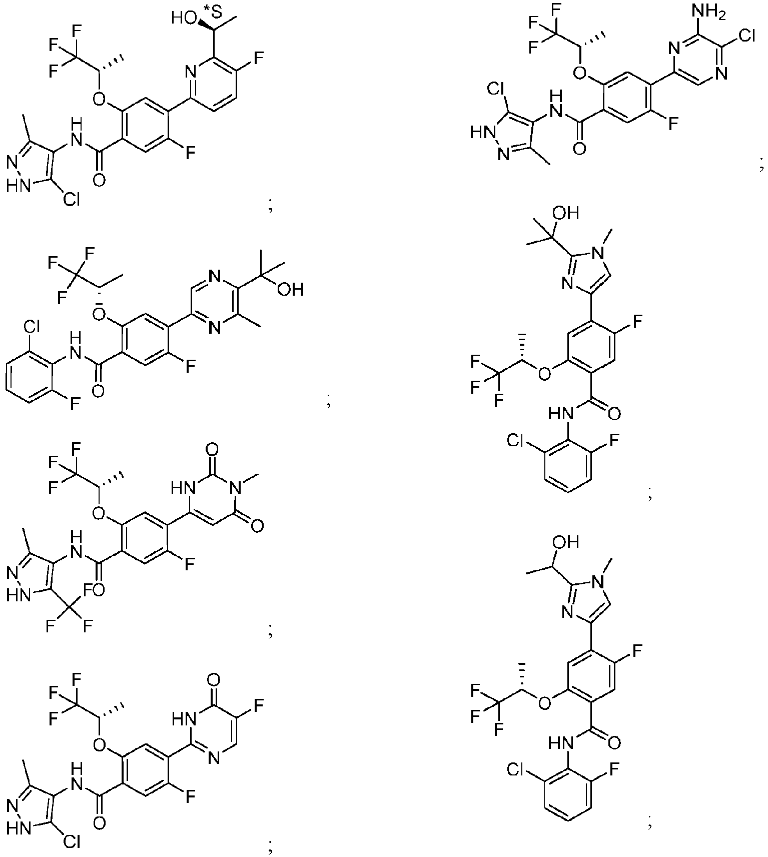 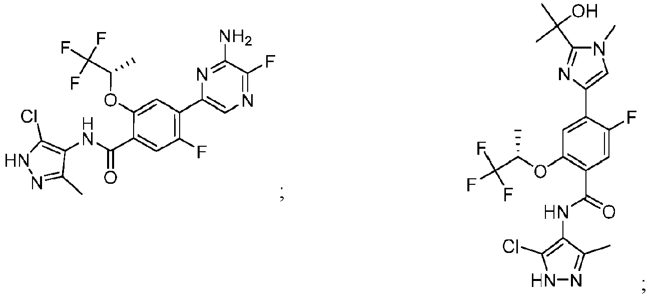 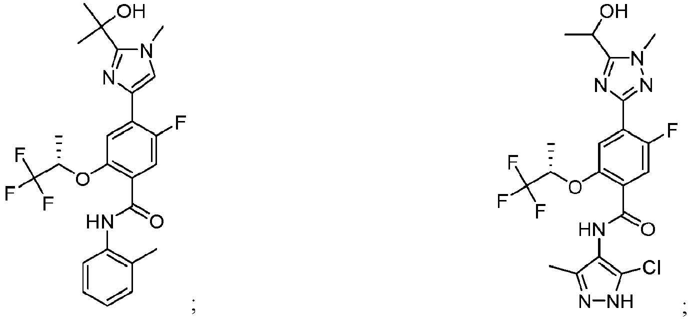 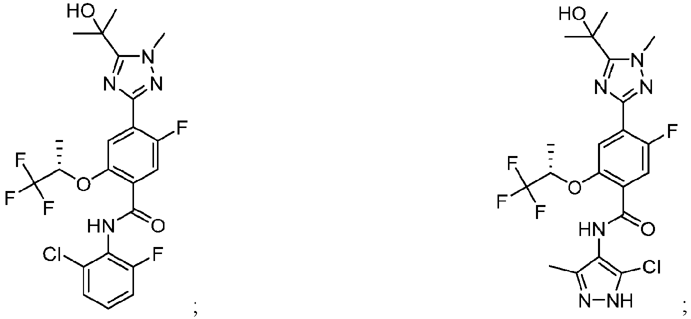 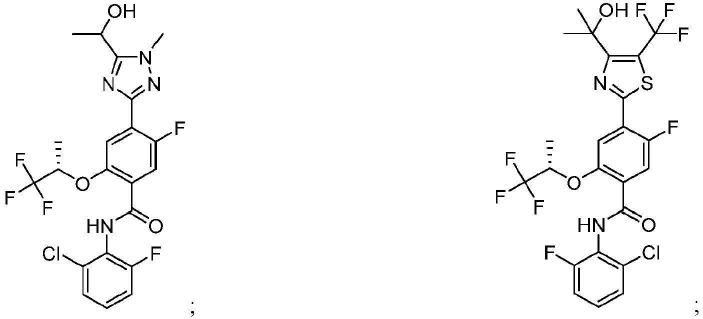 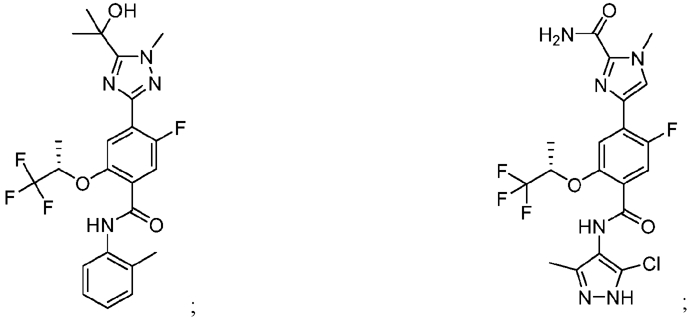 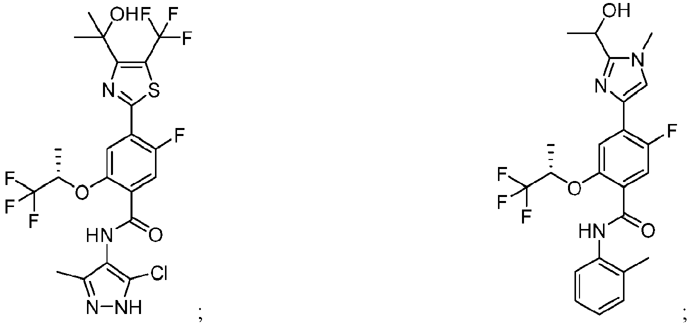 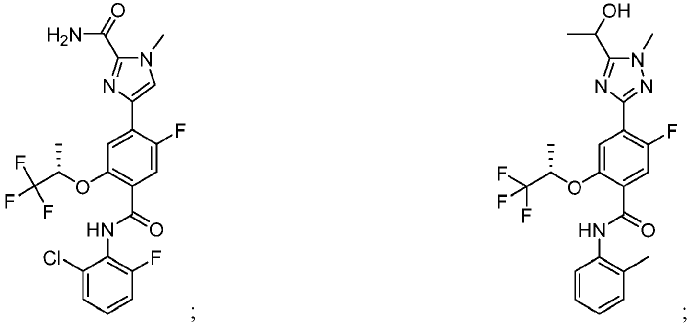 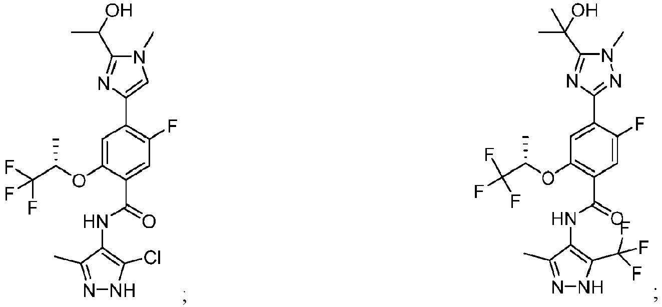 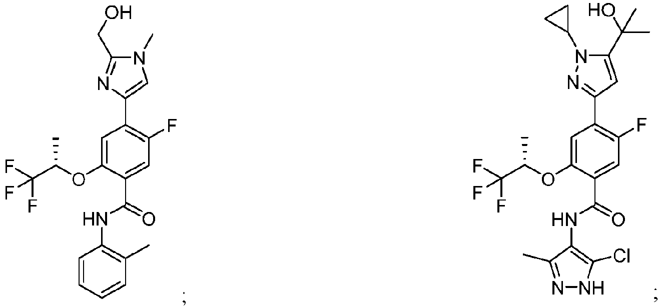 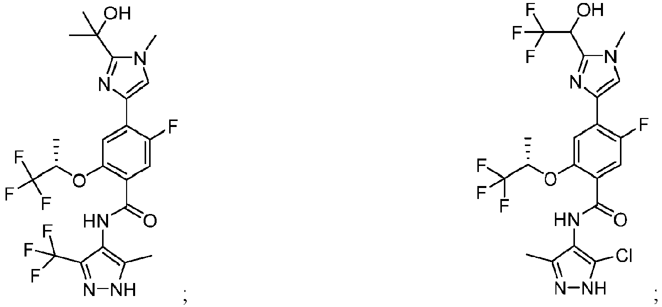 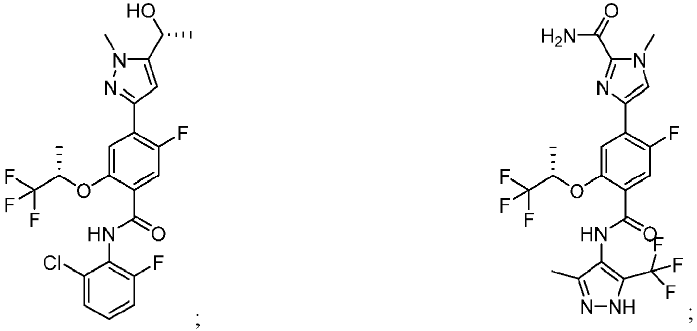 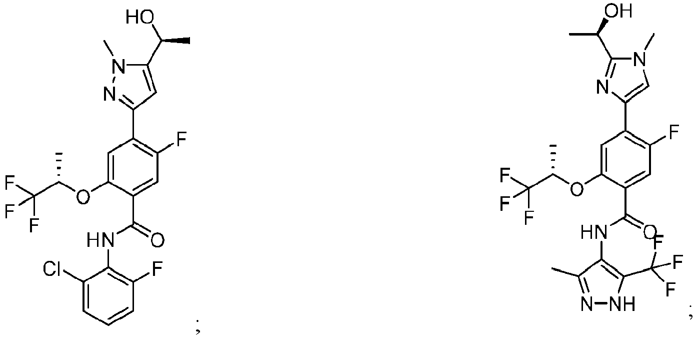 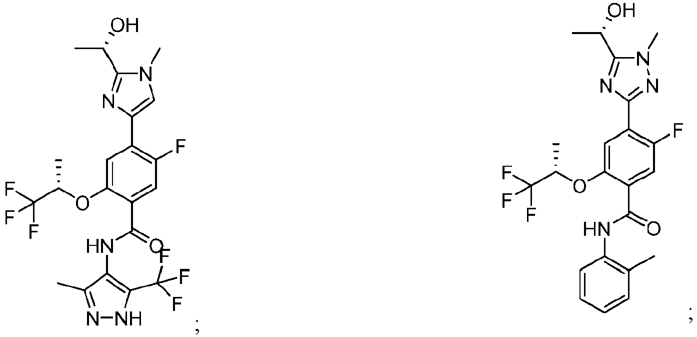 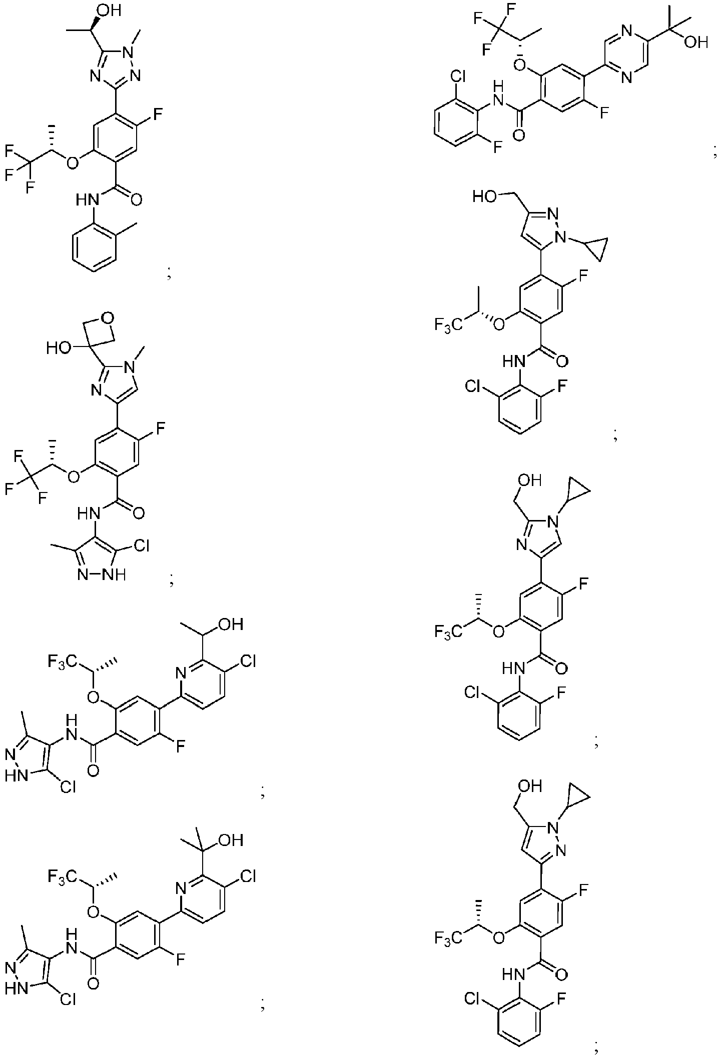 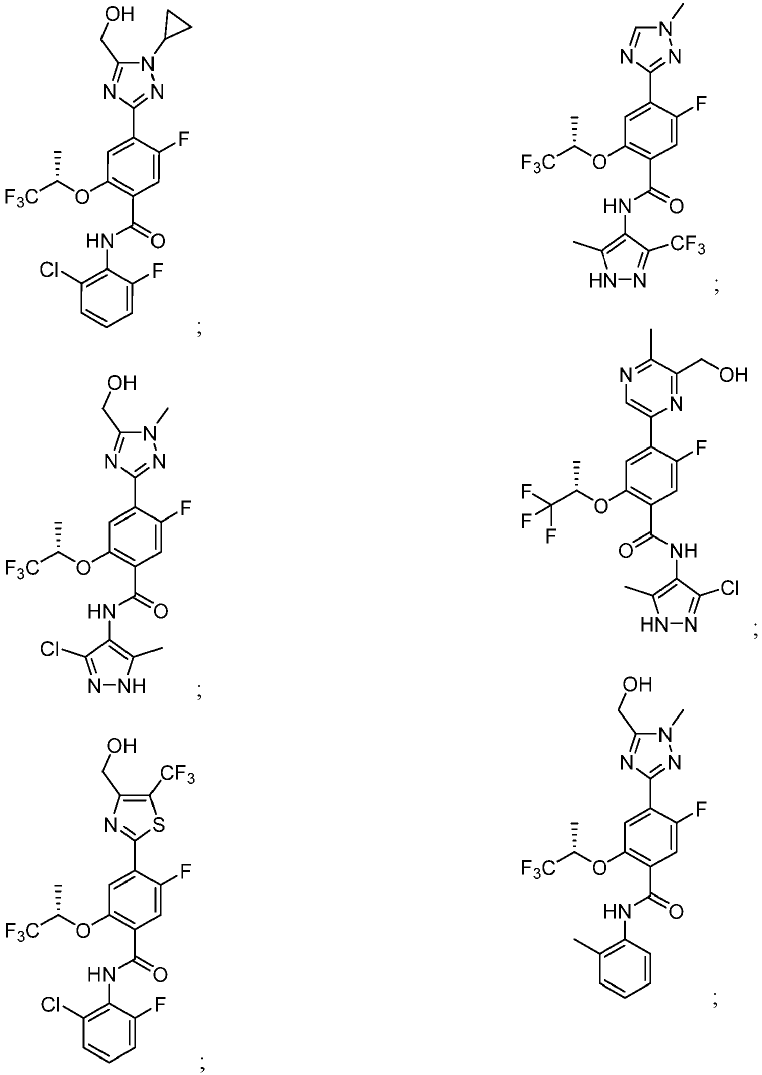 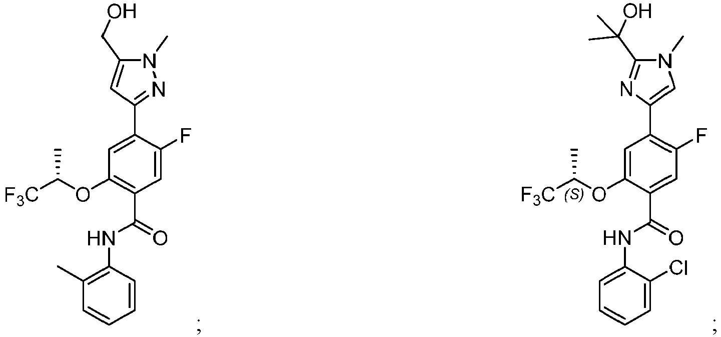 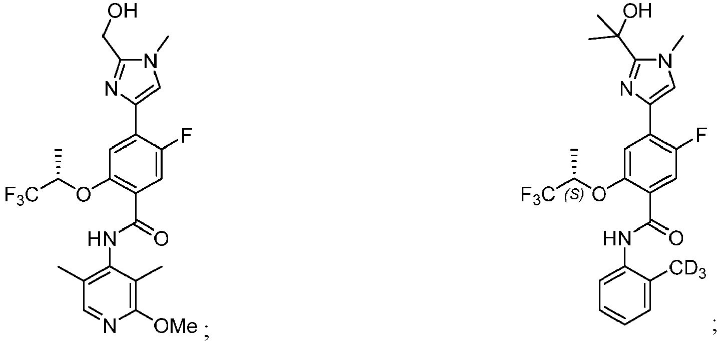 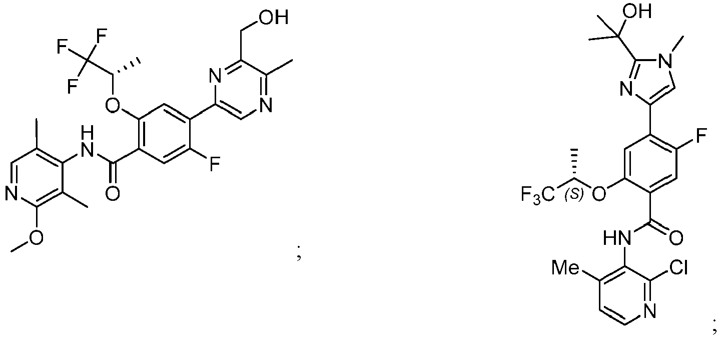 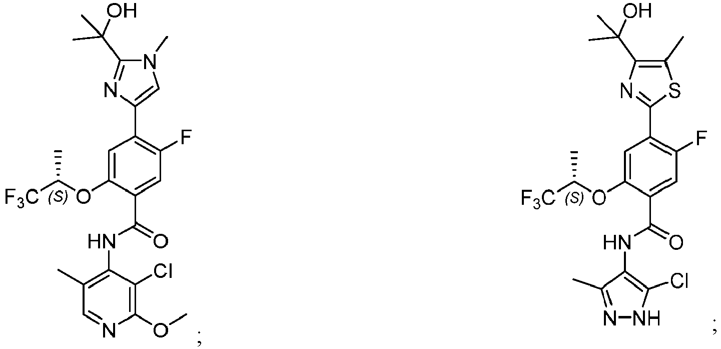 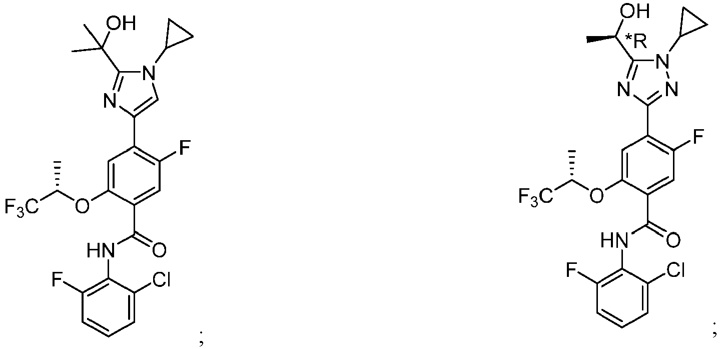 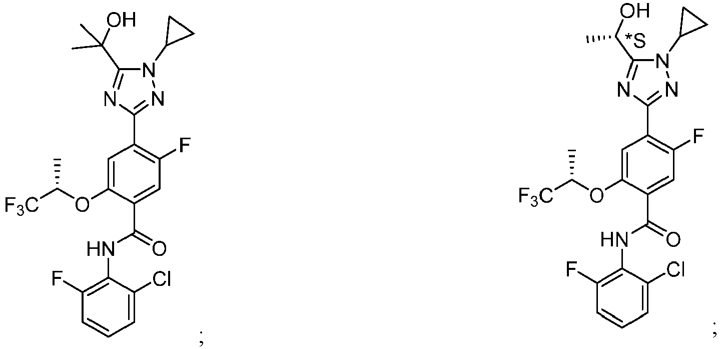 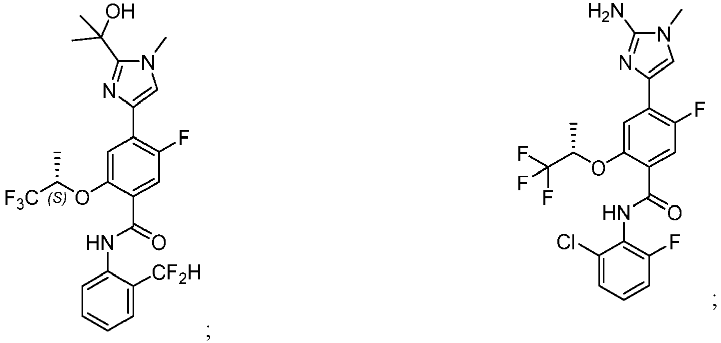 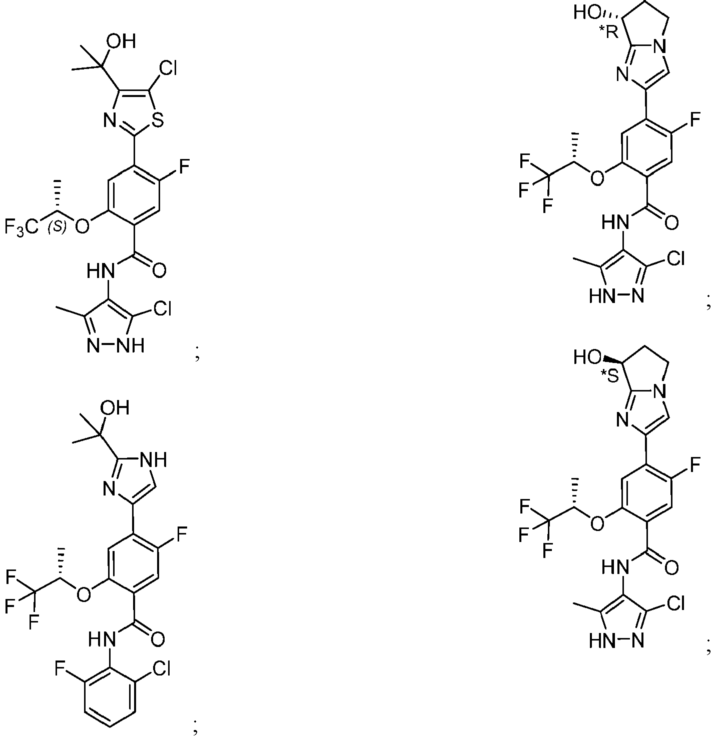 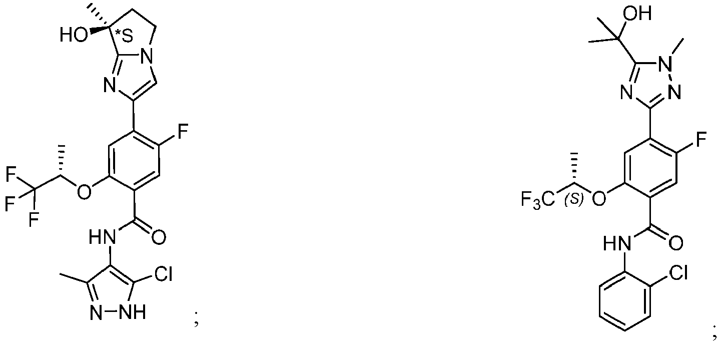 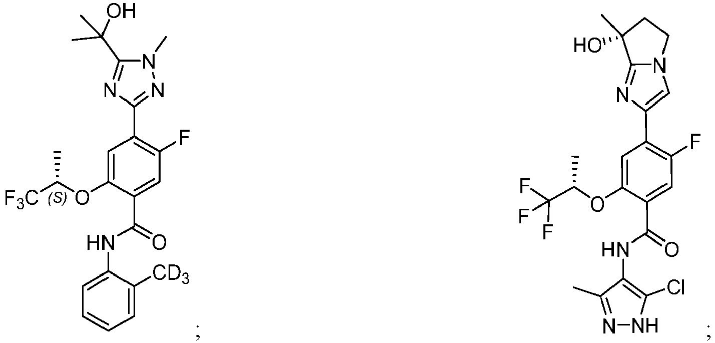 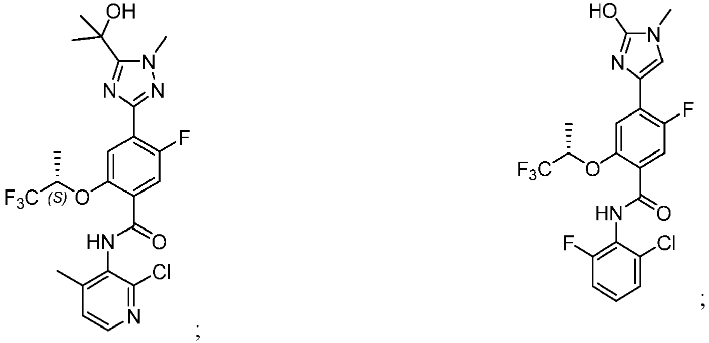 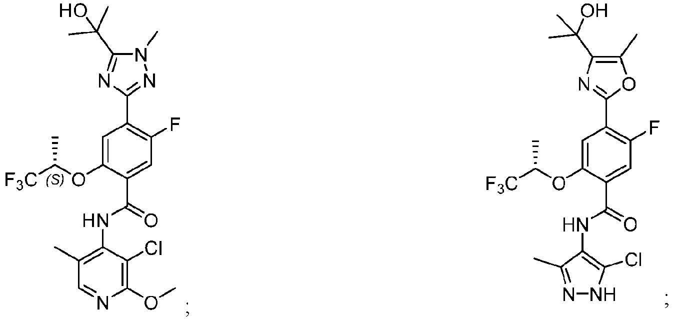 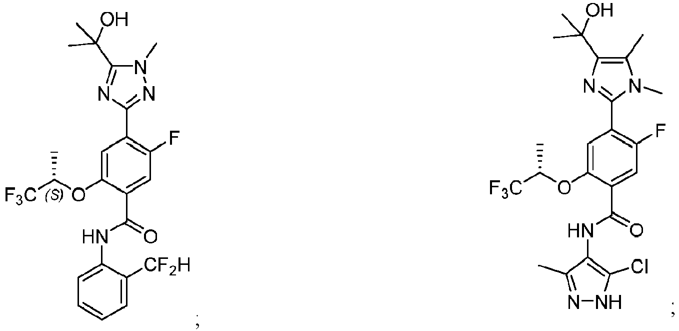 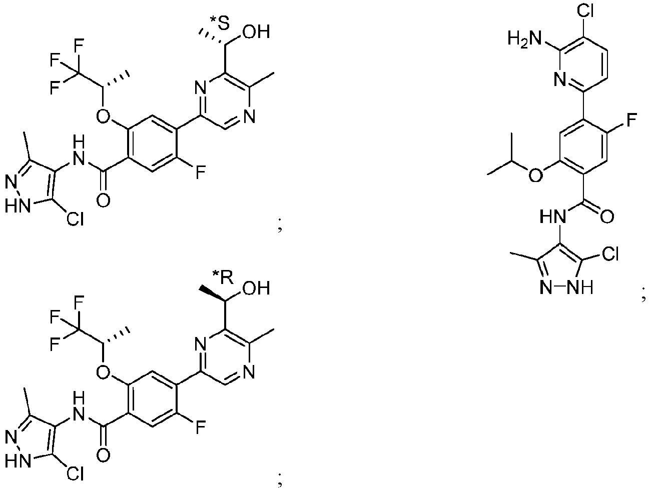 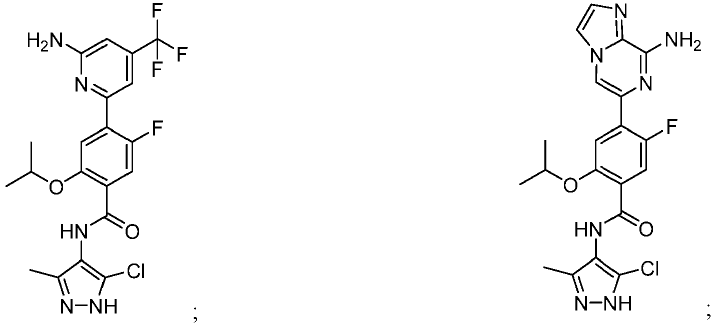 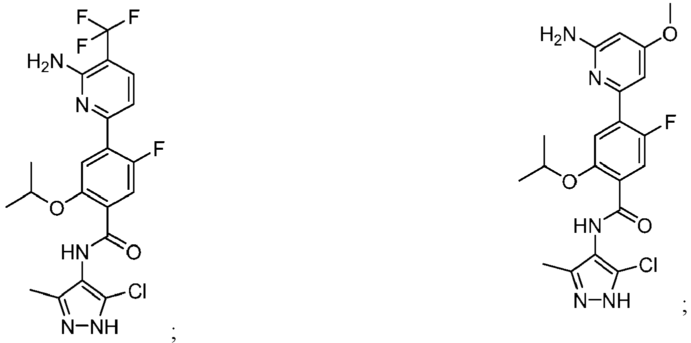 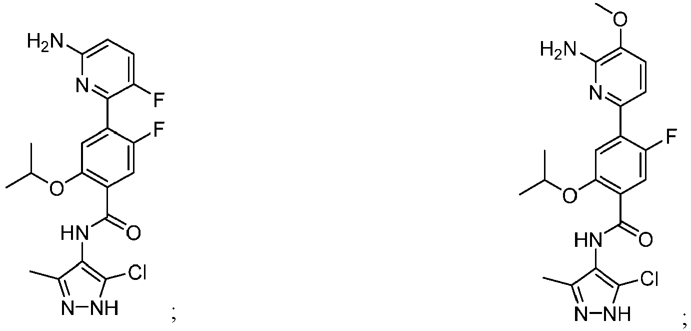 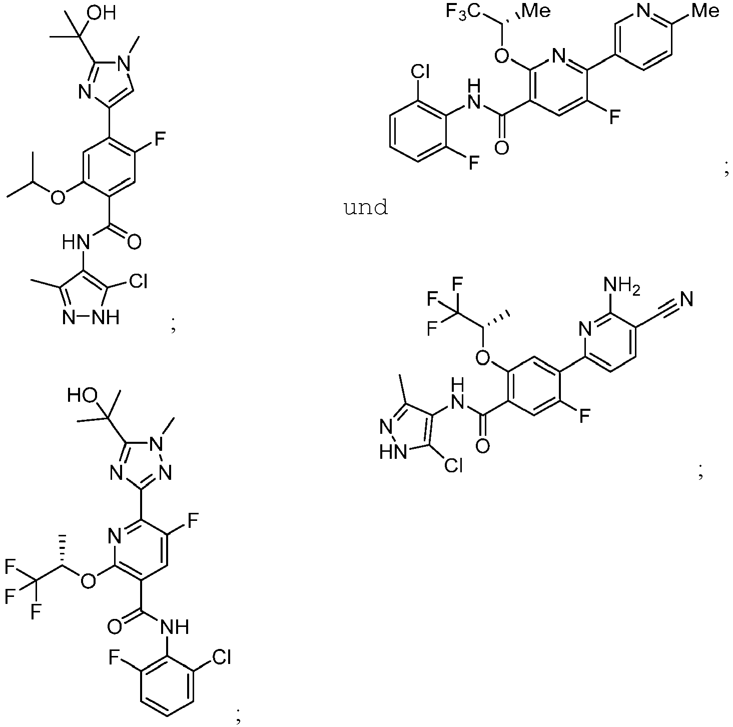 oder eines pharmazeutisch unbedenklichen Salzes, eines pharmazeutisch unbedenklichen Solvats, eines pharmazeutisch unbedenklichen Stereoisomers, einer pharmazeutisch unbedenklichen Isotopenvariante oder eines pharmazeutisch unbedenklichen N-Oxids davon; und mindestens einen pharmazeutisch unbedenklichen Hilfsstoff.

14. Mindestens eine Verbindung oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon nach einem der Ansprüche 1 bis 11 zur Verwendung bei der Behandlung eines Individuums, das an einer Erkrankung, einer Störung oder einem medizinischen Leiden leidet oder damit diagnostiziert ist, umfassend das Inhibieren oder Ändern von Dihydroorotatoxygenaseenzymaktivität bei dem Individuum, wobei die Erkrankung, die Störung bzw. das medizinische Leiden aus der Gruppe bestehend aus entzündlichen Störungen, Autoimmunstörungen und Krebs ausgewählt ist.

15. Mindestens eine Verbindung oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon zur Verwendung nach Anspruch 14, wobei die Erkrankung, die Störung bzw. das medizinische Leiden aus der Gruppe bestehend Lymphomen, Leukämien, Karzinom und Sarkomen ausgewählt ist.

16. Mindestens eine Verbindung oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon zur Verwendung nach Anspruch 14, wobei die Erkrankung, die Störung bzw. das medizinische Leiden aus der Gruppe bestehend aus akuter lymphoblastischer Leukämie, akuter myeloischer Leukämie, (akuter) T-Zell-Leukämie, akuter lymphoblastischer Leukämie, akuter lymphatischer Leukämie, akuter Monozytenleukämie, akuter Promyeolozytenleukämie, bisphänotypischer B-Myelomonozytenleukämie, chronischer lymphatischer Leukämie, chronischer myelogener Leukämie, chronischer myeloischer Leukämie, chronischer Myelomonozytenleukämie, großkörniger lymphatischer Leukämie, Plasmazellleukämie sowie myelodysplastischem Syndrom, das sich zu einer akute myeloische Leukämie entwickeln kann, ausgewählt ist.

17. Mindestens eine Verbindung oder ein pharmazeutisch unbedenkliches Salz, ein pharmazeutisch unbedenkliches Solvat, ein pharmazeutisch unbedenkliches Stereoisomer, eine pharmazeutisch unbedenkliche Isotopenvariante oder ein pharmazeutisch unbedenkliches N-Oxid davon zur Verwendung nach Anspruch 14, wobei es sich bei der Erkrankung, der Störung bzw. dem medizinischen Leiden um akute myeloische Leukämie handelt.

## Revendications

1. Composé de formule I 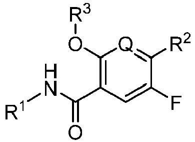
Q étant CH ou N ;
R¹ étant 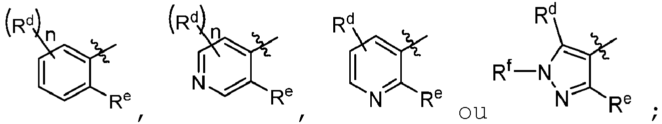
R^{d} étant choisi dans le groupe constitué par : -H, - Cl, -F et -C₍₁₋₃₎alkyle; ledit -C₍₁₋₃₎alkyle étant éventuellement substitué par : -OH, -OCH₃, -SCH₃ ou - OCF₃; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
imidazolyle ; ledit imidazolyle étant éventuellement substitué par jusqu'à trois substituants indépendamment choisis dans le groupe comprenant 3-hydroxyoxétanyle, - NH₂, -OH, -CN ou R^{a} ;
triazolyle ; ledit triazolyle étant éventuellement substitué par un ou deux groupes indépendamment choisis dans le groupe comprenant R^{a} ;
pyrazinyle ; ledit pyrazinyle étant éventuellement substitué par jusqu'à trois substituants indépendamment choisis dans le groupe comprenant -NHSO₂CH₃, -NHC(O)CH₃, -OCH₃, -OH, -NH₂, -CO₂H, -Cl, -F ou R^{a} ;
pyrazinonyle ; ledit pyrazinonyle étant éventuellement substitué par un ou deux groupes indépendamment choisis dans le groupe comprenant R^{a} ;
thiazolyle ; ledit thiazolyle étant éventuellement substitué par un ou deux substituants indépendamment choisis dans le groupe comprenant R^{a} ;
5,6,7,8-tétrahydro-[1,2,4]triazolo[4,3-a]pyrazinyle ; ledit 5,6,7,8-tétrahydro-[1,2,4]triazolo[4,3-a]pyrazinyle étant éventuellement substitué par R^{a} ;
5,6,7,8-tétrahydro-[1,2,4]triazolo[1,5-a]pyrazinyle ; ledit 5,6,7,8-tétrahydro-[1,2,4]triazolo[4,3-a]pyrazinyle étant éventuellement substitué par R^{a} ;
pyridinyle ; ledit pyridinyle étant éventuellement substitué par un ou deux substituants indépendamment choisis dans le groupe comprenant R^{a}, -Cl, -F, -CN, - NHC(O)CH₃, -NHSO₂CH₃, -CO₂H, -OCH₃, -OH ou -NH₂ ;
pyridazinyle ; ledit pyridazinyle étant éventuellement substitué par un ou deux groupes indépendamment choisis dans le groupe comprenant R^{a}, -Cl ou -CO₂H ;
4,5,6,7-tétrahydrothiazolo[5,4-c]pyridinyle ; ledit 4,5,6,7-tétrahydrothiazolo[5,4-c]pyridinyle étant éventuellement substitué par R^{a} ;
pyrazolyle ; ledit pyrazolyle étant éventuellement substitué par un ou deux substituants indépendamment choisis dans le groupe comprenant R^{a} ;
oxazolyle ; ledit oxazolyle étant éventuellement substitué par un ou deux substituants indépendamment choisis dans le groupe comprenant R^{a} ;
1,3,4-oxadiazolyle ; ledit 1,3,4-oxadiazolyle étant éventuellement substitué par R^{a} ;
5,6,7,8-tétrahydroimidazo[1,5-*a*]pyridinyle; ledit 5,6,7,8-tétrahydroimidazo[1,5-a]pyridinyle étant éventuellement substitué par R^{a} ;
1,2,4-thiadiazolyle ; ledit 1,2,4-thiadiazolyle étant éventuellement substitué par R^{a} ;
5,6,7,8-tétrahydro-1,7-naphtyridinyle ; ledit 5,6,7,8-tétrahydro-1,7-naphtyridinyle étant éventuellement substitué par R^{a} ;
5,6,7,8-tétrahydro-1,6-naphtyridinyle ; ledit 5,6,7,8-tétrahydro-1,6-naphtyridinyle étant éventuellement substitué par R^{a} ;
5,6,7,8-tétrahydro-1,8-naphtyridinyle ; ledit 5,6,7,8-tétrahydro-1,8-naphtyridinyle étant éventuellement substitué par R^{a} ;
1,2,3,4-tétrahydro-2,7-naphtyridinyle ; ledit 1,2,3,4-tétrahydro-2,7-naphtyridinyle étant éventuellement substitué par R^{a} ;
2*H*-pyrido[3,2-*b*][1,4]oxazin-3(4*H*)-onyle, ledit 2*H-*pyrido[3,2-*b*][1,4]oxazin-3(4*H*)-onyle étant éventuellement substitué par R^{a} ;
2,3-dihydro-1*H*-pyrrolo[2,3-*b*]pyridinyle ; ledit 2,3-dihydro-1*H*-pyrrolo[2,3-*b*]pyridinyle étant éventuellement substitué par R^{a} ;
imidazo[1,2-*a*]pyrazinyle, ledit imidazo[1,2-*a*]pyrazinyle étant éventuellement substitué par -NH₂ ou R^{a} ;
2,6-dioxo-1,2,3,6-tétrahydropyrimidinyle, ledit 2,6-dioxo-1,2,3,6-tétrahydropyrimidinyle étant éventuellement substitué par R^{a} ;
6-oxo-1,6-dihydropyrimidinyle, ledit 6-oxo-1,6-dihydropyrimidinyle étant éventuellement substitué par fluor ou R^{a} ;
6,7-dihydro-5H-pyrrolo[1,2-a]imidazolyle, ledit 6,7-dihydro-5*H*-pyrrolo[1,2-*a*]imidazolyle étant éventuellement substitué par un ou deux substituants choisis dans le groupe constitué par R^{a} et -OH ;
R^{a} étant -C(O)NH₂, cyclopropyle ou -C₍₁₋₃₎alkyle; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -OH, -CH₂OH ou jusqu'à deux atomes de fluor ;
R³ étant -CHR^{b}R^{c} ;
R^{b} étant -C₍₋₃₋₆₎cycloalkyle ou -C₍₁₋₆₎alkyle ; ledit -C₍₁₋₆₎alkyle étant éventuellement substitué par -Cl, -OH ou - OCH₃; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit -C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e).

2. Composé selon la revendication 1 qui est de formule II 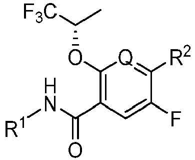
Q étant CH ou N ;
R¹ étant 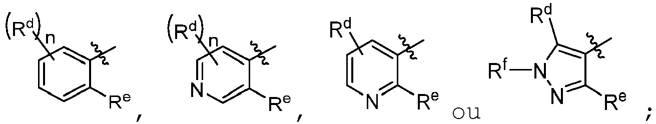
R^{d} étant choisi dans le groupe constitué par : -H, - Cl, -F et -C₍₁₋₃₎alkyle ; ledit -C₍₁₋₃₎alkyle étant éventuellement substitué par -OH, -OCH₃, -SCH₃ ou -OCF₃ ; ou ledit -C₍₁₋₃₎alkyle étant éventuellement substitué par jusqu'à trois atomes de fluor ;
R^{e} étant choisi dans le groupe constitué par : -Cl, - F, -CD₃, -C₍₁₋₃₎alkyle et cyclopropyle ; ledit -C₍₁₋₃₎alkyle étant éventuellement substitué par -OH, -OCH₃, -SCH₃ ou - OCF₃ ; ou ledit -C₍₁₋₃₎alkyle étant éventuellement substitué par jusqu'à trois atomes de fluor ;
R^{f} étant -H ; et
n étant 1 ou 2 ;
R² étant
imidazolyle ; ledit imidazolyle étant éventuellement substitué par jusqu'à trois substituants indépendamment choisis dans le groupe comprenant 3-hydroxyoxétanyle, - NH₂, -OH, -CN ou R^{a} ;
triazolyle ; ledit triazolyle étant éventuellement substitué par un ou deux groupes indépendamment choisis dans le groupe comprenant R^{a} ;
pyrazinyle ; ledit pyrazinyle étant éventuellement substitué par jusqu'à trois substituants indépendamment choisis dans le groupe comprenant -NHSO₂CH₃, -NHC(O)CH₃, -OCH₃, -OH, -NH₂, -CO₂H, -Cl, -F ou R^{a} ;
pyrazinonyle ; ledit pyrazinonyle étant éventuellement substitué par un ou deux groupes indépendamment choisis dans le groupe comprenant R^{a} ;
thiazolyle ; ledit thiazolyle étant éventuellement substitué par un ou deux substituants indépendamment choisis dans le groupe comprenant R^{a} ;
5,6,7,8-tétrahydro-[1,2,4]triazolo[4,3-a]pyrazinyle ;
5,6,7,8-tétrahydro-[1,2,4]triazolo[1,5-a]pyrazinyle ;
pyridinyle ; ledit pyridinyle étant éventuellement substitué par un ou deux substituants indépendamment choisis dans le groupe comprenant R^{a}, -Cl, -F, -CN, - NHC(O)CH₃, -NHSO₂CH₃, -CO₂H, -OCH₃, -OH ou -NH₂ ;
pyridazinyle ; ledit pyridazinyle étant éventuellement substitué par un ou deux groupes indépendamment choisis dans le groupe comprenant R^{a} ;
4,5,6,7-tétrahydrothiazolo[5,4-c]pyridinyle ;
pyrazolyle ; ledit pyrazolyle étant éventuellement substitué par un ou deux substituants indépendamment choisis dans le groupe comprenant R^{a} ;
oxazolyle ; ledit oxazolyle étant éventuellement substitué par un ou deux substituants indépendamment choisis dans le groupe comprenant R^{a} ;
1,3,4-oxadiazolyle ; ledit 1,3,4-oxadiazolyle étant substitué par cyclopropyle ;
5,6,7,8-tétrahydroimidazo[1,5-*a*]pyridinyle ; ledit 5,6,7,8-tétrahydroimidazo[1,5-*a*]pyridinyle étant éventuellement substitué par R^{a} ;
1,2,4-thiadiazolyle ; ledit 1,2,4-thiadiazolyle étant substitué par cyclopropyle ;
5,6,7,8-tétrahydro-1,7-naphtyridinyle ;
5,6,7,8-tétrahydro-1,6-naphtyridinyle ;
5,6,7,8-tétrahydro-1,8-naphtyridinyle ;
1,2,3,4-tétrahydro-2,7-naphtyridinyle ;
2*H*-pyrido[3,2-*b*][1,4]oxazin-3(4*H*)-onyle, ledit 2*H-*pyrido[3,2-*b*][1,4]oxazin-3(4*H*)-onyle étant éventuellement substitué par R^{a} ;
2,3-dihydro-1*H*-pyrrolo[2,3-b]pyridinyle ;
imidazo[1,2-*a*]pyrazinyle, ledit imidazo[1,2-a]pyrazinyle étant substitué par -NH₂ ;
2,6-dioxo-1,2,3,6-tétrahydropyrimidinyle, ledit 2,6-dioxo-1,2,3,6-tétrahydropyrimidinyle étant éventuellement substitué par R^{a} ;
6-oxo-1,6-dihydropyrimidinyle, ledit 6-oxo-1,6-dihydropyrimidinyle étant substitué par fluor ;
6,7-dihydro-5*H*-pyrrolo[1,2-a]imidazolyle, ledit 6,7-dihydro-5*H*-pyrrolo[1,2-*a*]imidazolyle étant éventuellement substitué par un ou deux substituants choisis dans le groupe constitué par R^{a} et -OH ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
ou sel, solvate, stéréoisomère, variante isotopique ou N-oxyde pharmaceutiquement acceptable correspondant(e)(e).

3. Composé selon la revendication 1 ou la revendication 2,
(a) Q étant CH ou N ;
R¹ étant 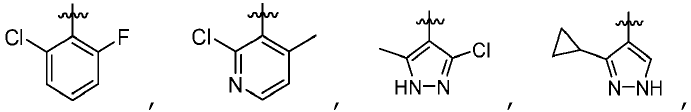 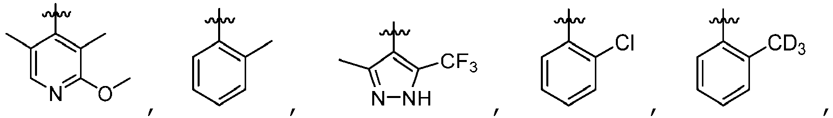 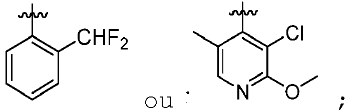
R² étant
imidazolyle ; ledit imidazolyle étant éventuellement substitué par jusqu'à trois substituants indépendamment choisis dans le groupe comprenant 3-hydroxyoxétanyle, - NH₂, -OH, -CN ou R^{a} ;
triazolyle ; ledit triazolyle étant éventuellement substitué par un ou deux groupes indépendamment choisis dans le groupe comprenant R^{a} ;
pyrazinyle ; ledit pyrazinyle étant éventuellement substitué par jusqu'à trois substituants indépendamment choisis dans le groupe comprenant -OCH₃, -OH, -NH₂, -CO₂H, -Cl, -F ou R^{a} ;
pyrazinonyle ; ledit pyrazinonyle étant éventuellement substitué par un ou deux groupes indépendamment choisis dans le groupe comprenant R^{a} ;
thiazolyle ; ledit thiazolyle étant éventuellement substitué par deux substituants indépendamment choisis dans le groupe comprenant R^{a} ;
5,6,7,8-tétrahydro-[1,2,4]triazolo[4,3-*a*]pyrazinyle ;
5,6,7,8-tétrahydro-[1,2,4]triazolo[1,5-a]pyrazinyle ;
pyridinyle ; ledit pyridinyle étant éventuellement substitué par un ou deux substituants indépendamment choisis dans le groupe comprenant R^{a}, -Cl, -F, -CN, - NHC(O)CH₃, -NHSO₂CH₃, -CO₂H, -OCH₃, -OH ou -NH₂ ;
pyridazinyle ; ledit pyridazinyle étant éventuellement substitué par R^{a} ;
4,5,6,7-tétrahydrothiazolo[5,4-c]pyridinyle ;
pyrazolyle ; ledit pyrazolyle étant éventuellement substitué par un ou deux substituants indépendamment choisis dans le groupe comprenant R^{a} ;
oxazolyle ; ledit oxazolyle étant éventuellement substitué par deux substituants indépendamment choisis parmi R^{a} ;
1,3,4-oxadiazolyle ; ledit 1,3,4-oxadiazolyle étant substitué par cyclopropyle ;
5,6,7,8-tétrahydroimidazo[1,5-*a*]pyridinyle ; ledit 5,6,7,8-tétrahydroimidazo[1,5-*a*]pyridinyle étant éventuellement substitué par R^{a} ;
1,2,4-thiadiazolyle ; ledit 1,2,4-thiadiazolyle étant substitué par cyclopropyle ;
5,6,7,8-tétrahydro-1,7-naphtyridinyle ;
5,6,7,8-tétrahydro-1,6-naphtyridinyle ;
5,6,7,8-tétrahydro-1,8-naphtyridinyle ;
1,2,3,4-tétrahydro-2,7-naphtyridinyle ;
2*H*-pyrido[3,2-*b*][1,4]oxazin-3(4*H*)-onyle, ledit *2H-*pyrido[3,2-*b*][1,4]oxazin-3(4*H*)-onyle étant éventuellement substitué par -CH₃ ;
2,3-dihydro-1*H*-pyrrolo[2,3-*b*]pyridinyle ;
imidazo[1,2-*a*]pyrazinyle, ledit imidazo[1,2-*a*]pyrazinyle étant substitué par -NH₂ ;
2,6-dioxo-1,2,3,6-tétrahydropyrimidinyle, ledit 2,6-dioxo-1,2,3,6-tétrahydropyrimidinyle étant substitué par -CH₃ ;
6-oxo-1,6-dihydropyrimidinyle, ledit 6-oxo-1,6-dihydropyrimidinyle étant substitué par fluor ;
6,7-dihydro-5*H*-pyrrolo[1,2-*a*]imidazolyle, ledit 6,7-dihydro-5*H*-pyrrolo[1,2-*a*]imidazolyle étant éventuellement substitué par un ou deux substituants choisis dans le groupe constitué par -CH₃ et -OH ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
ou sel, solvate, stéréoisomère, variante isotopique ou N-oxyde pharmaceutiquement acceptable correspondant(e)(e) ; ou
(b) Q étant CH ou N ;
R¹ étant 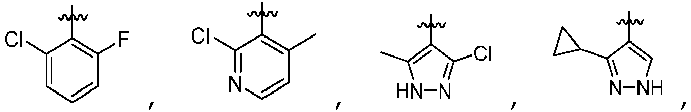 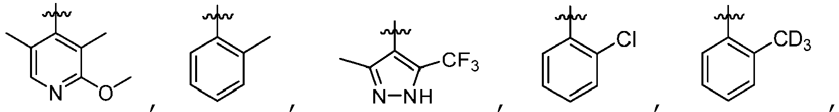 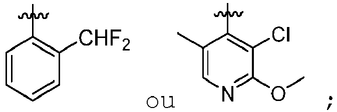
R² étant
imidazolyle ; ledit imidazolyle étant substitué par un, deux ou trois substituants indépendamment choisis dans le groupe comprenant -C₍₁₋₃₎alkyle, 3-hydroxyoxétanyle, cyclopropyle, -C(O)NH₂, -NH₂, -OH ou - CN ; ledit -C₍₁₋₃₎alkyle étant éventuellement substitué par -OH et de plus éventuellement substitué par jusqu'à trois atomes de fluor ;
triazolyle ; ledit triazolyle étant substitué par jusqu'à deux groupes indépendamment choisis dans le groupe comprenant -C(O)NH₂, -CF₃, cyclopropyle ou -C₍₁₋₃₎alkyle, ledit -C₍₁₋₃₎alkyle étant éventuellement substitué par -OH ;
pyrazinyle ; ledit pyrazinyle étant éventuellement substitué par jusqu'à trois substituants indépendamment choisis dans le groupe comprenant -C₍₁₋₃₎alkyle, -OCH₃, - OH, -NH₂, cyclopropyle, -C(O)NH₂, -CO₂H, -Cl, -F ou -CF₃ ; ledit cyclopropyle étant éventuellement substitué par - CH₂OH ; et ledit -C₍₁₋₃₎alkyle étant éventuellement substitué par -OH ;
pyrazinonyle ; ledit pyrazinonyle étant éventuellement substitué par un ou deux groupes -C₍₁₋₃₎alkyle ;
thiazolyle ; ledit thiazolyle étant substitué par deux substituants indépendamment choisis dans le groupe comprenant -C₍₁₋₃₎alkyle, cyclopropyle, -Cl ou -C(O)NH₂ ; ledit -C₍₁₋₃₎alkyle étant éventuellement substitué par -OH ou jusqu'à trois atomes de fluor ;
5,6,7,8-tétrahydro-[1,2,4]triazolo[4,3-a]pyrazinyle ;
5,6,7,8-tétrahydro-[1,2,4]triazolo[1,5-a]pyrazinyle ;
pyridinyle ; ledit pyridinyle étant substitué par un ou deux substituants indépendamment choisis dans le groupe comprenant -CONH₂, -C₍₁₋₃₎alkyle, cyclopropyle, - Cl, -F, -CN, -NHC(O)CH₃, -NHSO₂CH₃, -CO₂H, -OCH₃, -OH ou - NH₂ ; ledit -C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ;
pyridazinyle ; ledit pyridazinyle étant substitué par -C₍₁₋₃₎alkyle ; ledit -C₍₁₋₃₎alkyle étant éventuellement substitué par -OH ;
4,5,6,7-tétrahydrothiazolo[5,4-c]pyridinyle ;
pyrazolyle ; ledit pyrazolyle étant substitué par deux substituants indépendamment choisis dans le groupe comprenant -C₍₁₋₃₎alkyle et cyclopropyle ; ledit -C₍₁₋₃₎alkyle étant éventuellement substitué par -OH ;
oxazolyle ; ledit oxazolyle étant éventuellement substitué par deux substituants indépendamment choisis parmi -C₍₁₋₃₎alkyle ; ledit -C₍₁₋₃₎alkyle étant éventuellement substitué par -OH ;
1,3,4-oxadiazolyle ; ledit 1,3,4-oxadiazolyle étant substitué par cyclopropyle ;
5,6,7,8-tétrahydroimidazo[1,5-*a*]pyridinyle ledit 5,6,7,8-tétrahydroimidazo[1,5-a]pyridinyle étant substitué par -C₍₁₋₃₎alkyle ; ledit -C₍₁₋₃₎alkyle étant éventuellement substitué par -OH ;
1,2,4-thiadiazolyle ; ledit 1,2,4-thiadiazolyle étant substitué par cyclopropyle ;
5,6,7,8-tétrahydro-1,7-naphtyridinyle ;
5,6,7,8-tétrahydro-1,6-naphtyridinyle ;
5,6,7,8-tétrahydro-1,8-naphtyridinyle ;
1,2,3,4-tétrahydro-2,7-naphtyridinyle ;
2*H*-pyrido[3,2-*b*] [1,4]oxazin-3(4*H*)-onyle, ledit *2H-*pyrido[3,2-*b*][1,4]oxazin-3(4*H*)-onyle étant éventuellement substitué par -CH₃ ;
2,3-dihydro-1*H*-pyrrolo[2,3-*b*]pyridinyle ;
imidazo[1,2-a]pyrazinyle, ledit imidazo[1,2-a]pyrazinyle étant substitué par -NH₂ ;
2,6-dioxo-1,2,3,6-tétrahydropyrimidinyle, ledit 2,6-dioxo-1,2,3,6-tétrahydropyrimidinyle étant substitué par -CH₃ ;
6-oxo-1,6-dihydropyrimidinyle, ledit 6-oxo-1,6-dihydropyrimidinyle étant substitué par fluor ;
6,7-dihydro-5*H*-pyrrolo[1,2-a]imidazolyle, ledit 6,7-dihydro-5*H*-pyrrolo[1,2-*a*]imidazolyle étant éventuellement substitué par un ou deux substituants choisis dans le groupe constitué par -CH₃ et -OH ;
ou sel, solvate, stéréoisomère, variante isotopique ou N-oxyde pharmaceutiquement acceptable correspondant (e) (e) .

4. Composé selon l'une quelconque des revendications 1 à 3,
Q étant CH ou N ;
R¹ étant 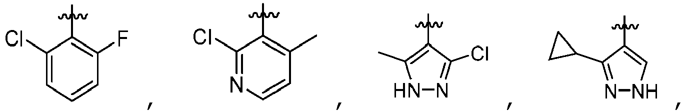 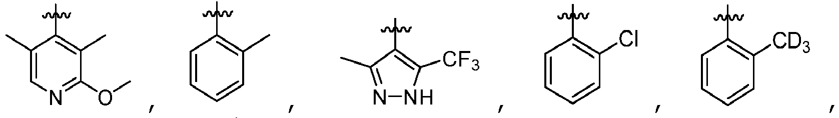 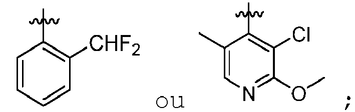
R² étant
imidazolyle ; ledit imidazolyle étant substitué par un, deux ou trois substituants indépendamment choisis dans le groupe comprenant -C₍₁₋₃₎alkyle, 3-hydoxyoxétanyle, cyclopropyle, -C(O)NH₂, -NH₂, -OH ou - CN ; ledit -C₍₁₋₃₎alkyle étant éventuellement substitué par -OH et de plus éventuellement substitué par jusqu'à trois atomes de fluor ;
triazolyle ; ledit triazolyle étant substitué par jusqu'à deux groupes indépendamment choisis dans le groupe comprenant -C(O)NH₂, -CF₃, cyclopropyle ou -C₍₁₋₃₎alkyle, ledit -C₍₁₋₃₎alkyle étant éventuellement substitué par -OH ;
pyrazinyle ; ledit pyrazinyle étant éventuellement substitué par jusqu'à trois substituants indépendamment choisis dans le groupe comprenant -C₍₁₋₃₎alkyle, -OCH₃, - OH, -NH₂, cyclopropyle, -C(O)NH₂, -CO₂H, -Cl, -F ou -CF₃, ledit cyclopropyle étant éventuellement substitué par - CH₂OH, et ledit -C₍₁₋₃₎alkyle étant éventuellement substitué par -OH ;
pyrazinonyle ; ledit pyrazinonyle étant substitué par -CH₃ et -CH₂CH₂CH₃ ;
thiazolyle ; ledit thiazolyle étant substitué par deux substituants indépendamment choisis dans le groupe comprenant -C₍₁₋₃₎alkyle, cyclopropyle, -Cl ou -C(O)NH₂ ; ledit -C₍₁₋₃₎alkyle étant éventuellement substitué par -OH ou jusqu'à trois atomes de fluor ;
5,6,7,8-tétrahydro-[1,2,4]triazolo[4,3-*a*]pyrazinyle ;
5,6,7,8-tétrahydro-[1,2,4]triazolo[1,5-a]pyrazinyle ;
pyridinyle ; ledit pyridinyle étant substitué par un ou deux substituants indépendamment choisis dans le groupe comprenant -CONH₂, -CF₃, -C₍₁₋₃₎alkyle, cyclopropyle, -Cl, -F, -CN, -NHC(O)CH₃, -NHSO₂CH₃, -CO₂H, -OCH₃, -OH ou -NH₂, ledit -C₍₁₋₃₎alkyle étant éventuellement substitué par -OH et de plus éventuellement substitué par jusqu'à trois atomes de fluor ;
pyridazinyle ; ledit pyridazinyle étant substitué par -CH₃ ;
4,5,6,7-tétrahydrothiazolo[5,4-c]pyridinyle ; pyrazolyle ; ledit pyrazolyle étant substitué par deux substituants indépendamment choisis dans le groupe comprenant -C₍₁₋₃₎alkyle et cyclopropyle, ledit -C₍₁₋₃₎alkyle étant éventuellement substitué par -OH ;
oxazolyle ; ledit oxazolyle étant substitué par deux substituants indépendamment choisis parmi-C₍₁₋₃₎alkyle ; ledit -C₍₁₋₃₎alkyle étant éventuellement substitué par - OH ;
1,3,4-oxadiazolyle ; ledit 1,3,4-oxadiazolyle étant substitué par cyclopropyle ;
5,6,7,8-tétrahydroimidazo[1,5-*a*]pyridinyle ledit 5,6,7,8-tétrahydroimidazo[1,5-a]pyridinyle étant substitué par -CH₂OH ;
1,2,4-thiadiazolyle ; ledit 1,2,4-thiadiazolyle étant substitué par cyclopropyle ;
5,6,7,8-tétrahydro-1,7-naphtyridinyle ;
5,6,7,8-tétrahydro-1,6-naphtyridinyle ;
5,6,7,8-tétrahydro-1,8-naphtyridinyle ;
1,2,3,4-tétrahydro-2,7-naphtyridinyle ;
2*H*-pyrido[3,2-*b*] [1,4]oxazin-3(4*H*)-onyle, ledit *2H-*pyrido[3,2-*b*] [1,4]oxazin-3(4*H*)-onyle étant éventuellement substitué par -CH₃ ;
2,3-dihydro-1*H*-pyrrolo[2,3-b]pyridinyle ;
imidazo[1,2-a]pyrazinyle, ledit imidazo[1,2-a]pyrazinyle étant substitué par -NH₂ ;
2,6-dioxo-1,2,3,6-tétrahydropyrimidinyle, ledit 2,6-dioxo-1,2,3,6-tétrahydropyrimidinyle étant substitué par -CH₃ ;
6-oxo-1,6-dihydropyrimidinyle, ledit 6-oxo-1,6-dihydropyrimidinyle étant substitué par fluor ;
6,7-dihydro-5*H*-pyrrolo[1,2-*a*]imidazolyle, ledit 6,7-dihydro-5*H*-pyrrolo[1,2-a]imidazolyle étant éventuellement substitué par un ou deux substituants choisis dans le groupe constitué par -CH₃ et -OH ;
ou sel, solvate, stéréoisomère, variante isotopique ou N-oxyde pharmaceutiquement acceptable correspondant (e) (e) .

5. Composé selon l'une quelconque des revendications 1 à 4, le composé étant choisi dans le groupe constitué par 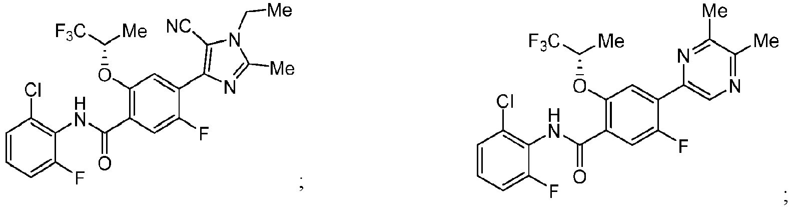 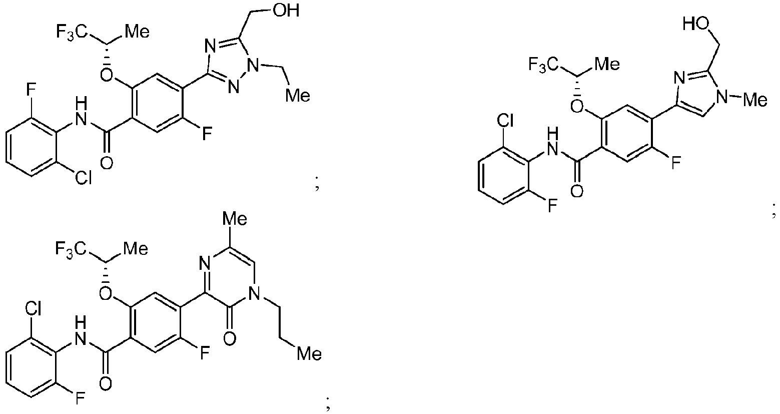 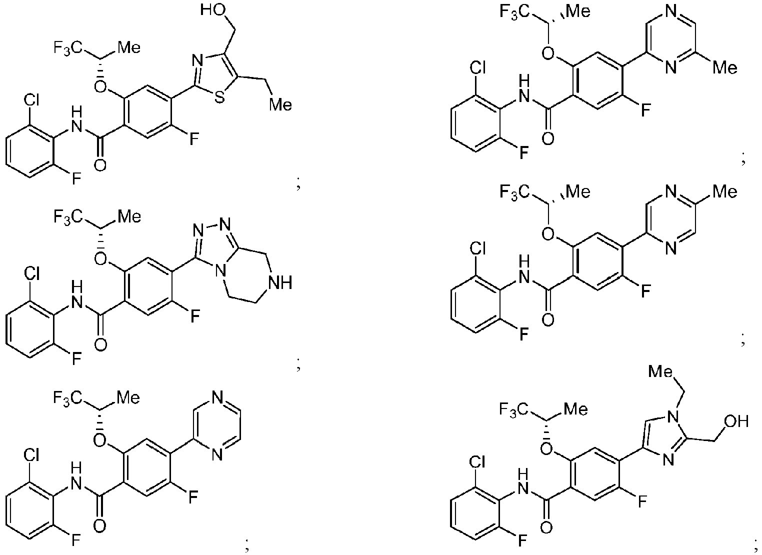 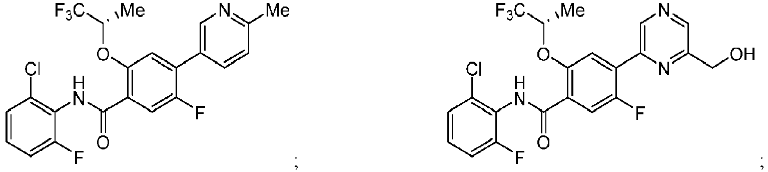 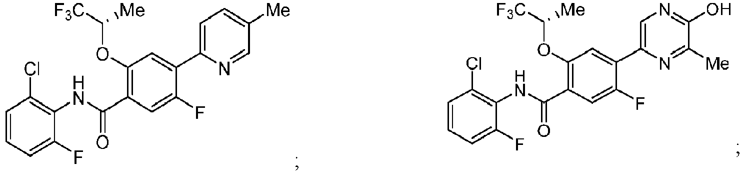 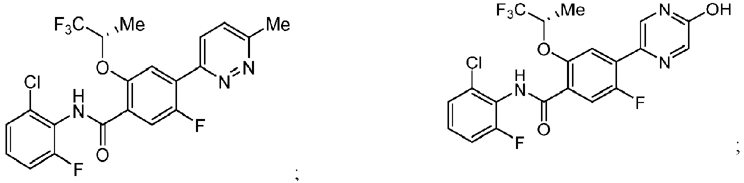 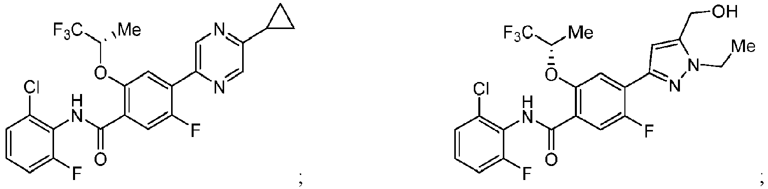 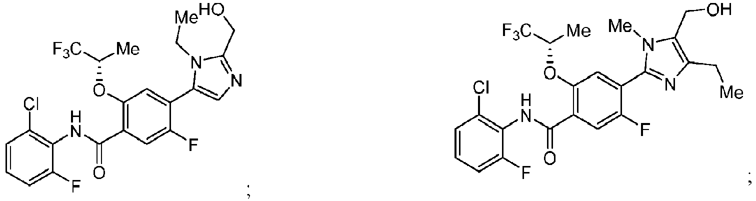 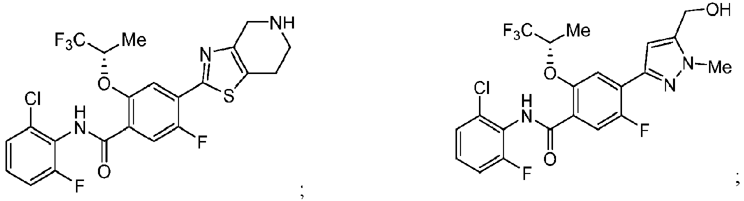 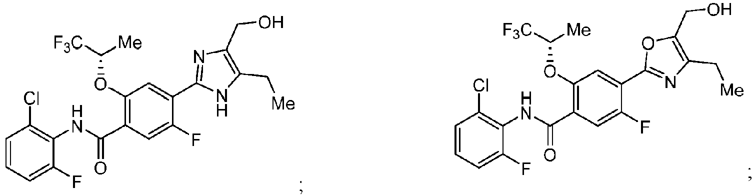 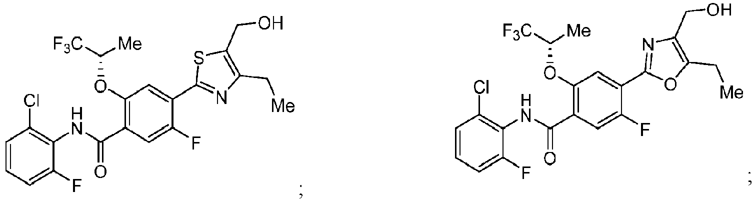 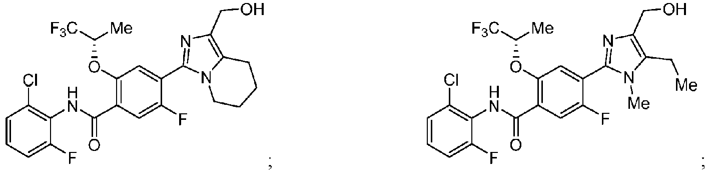 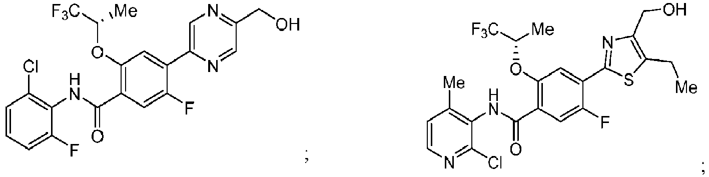 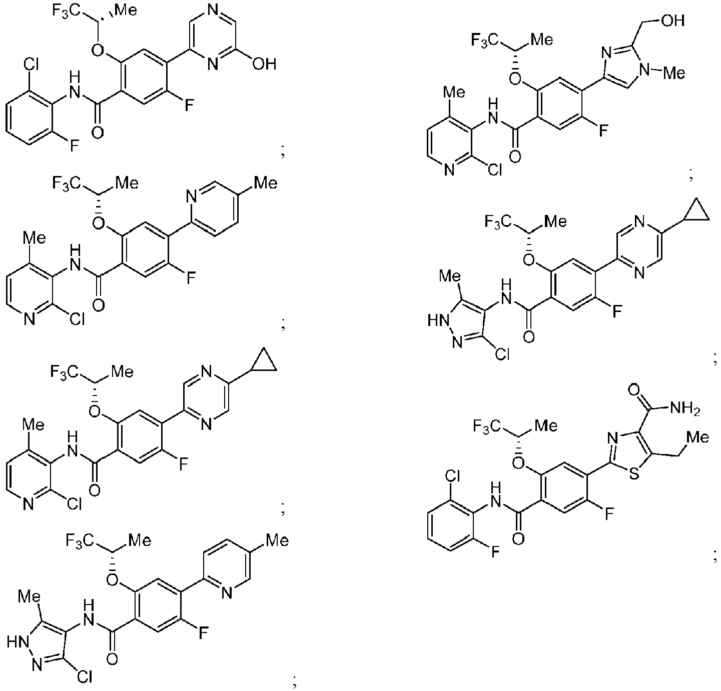 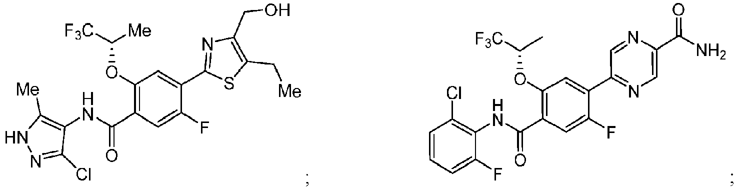 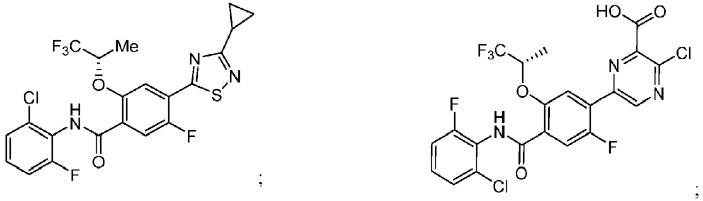 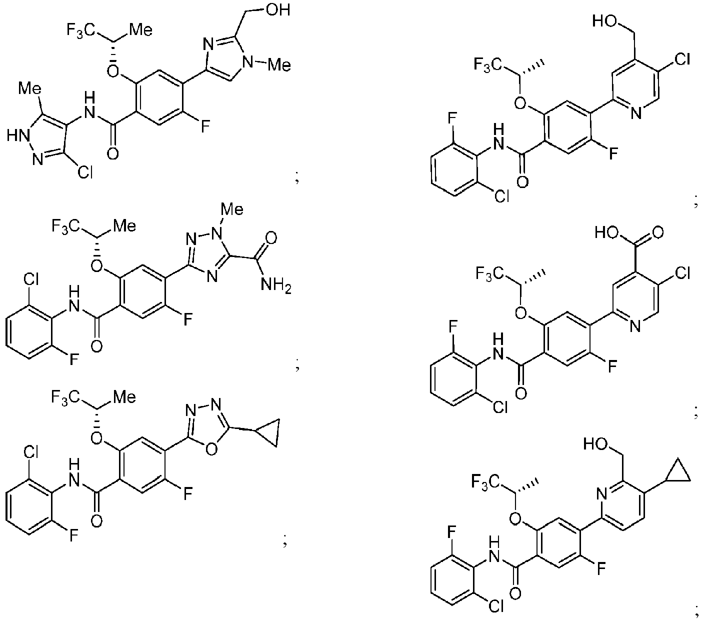 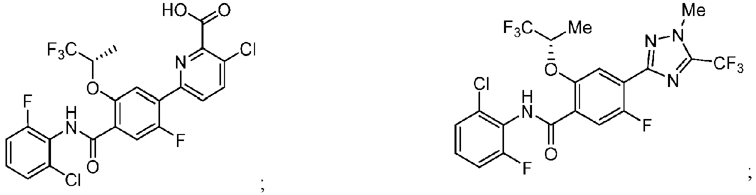 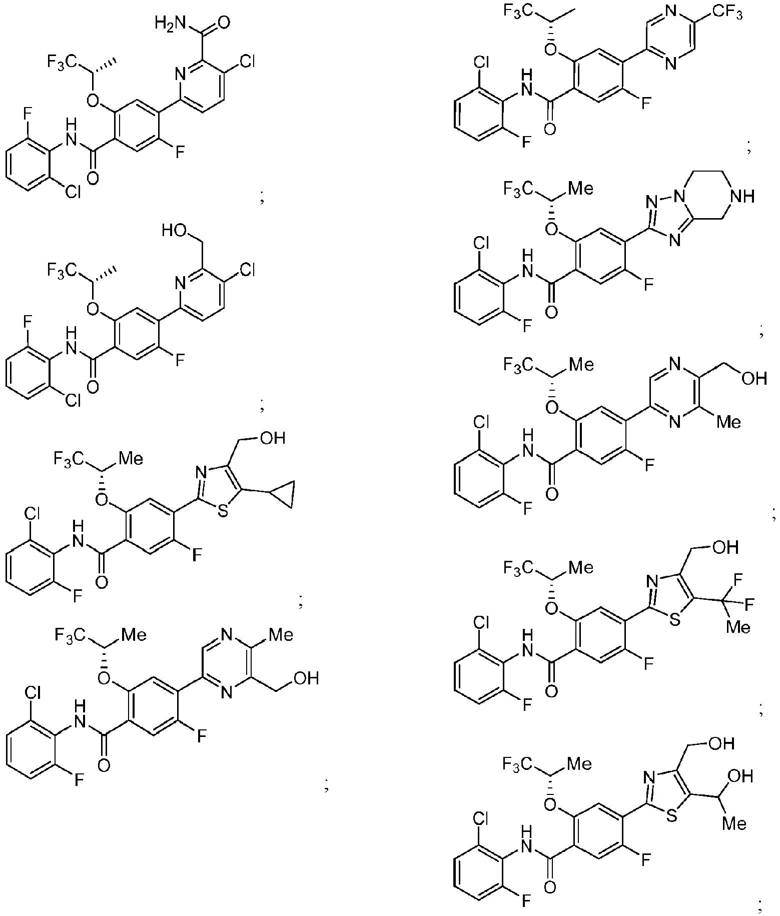 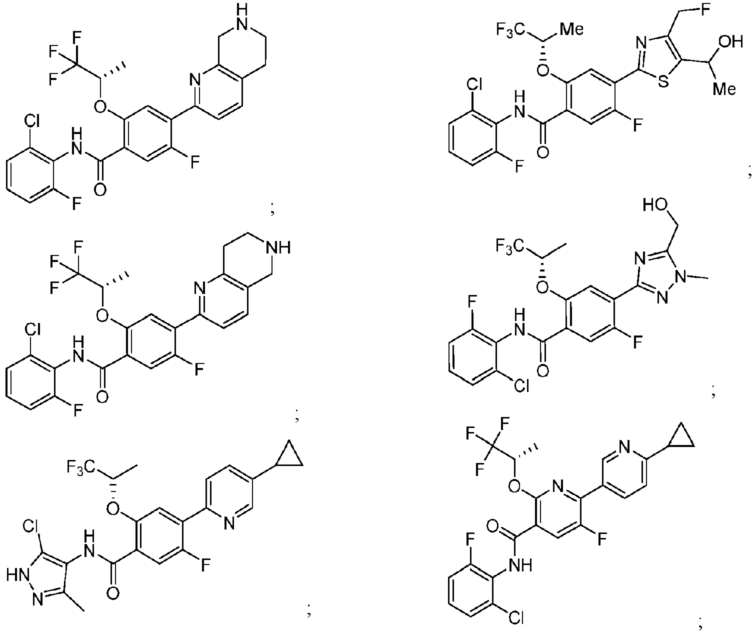 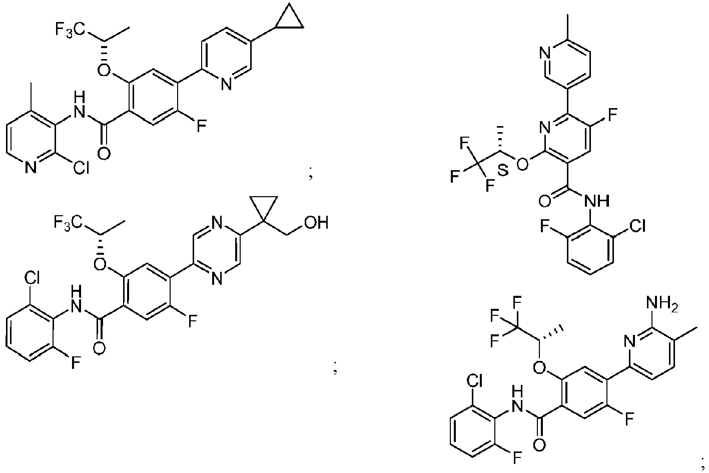 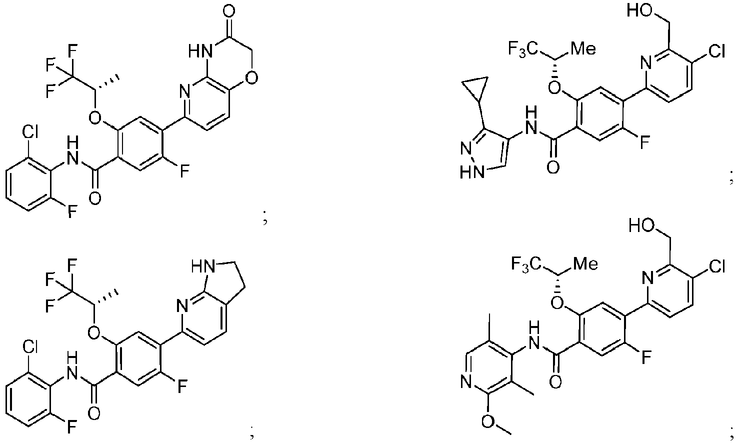 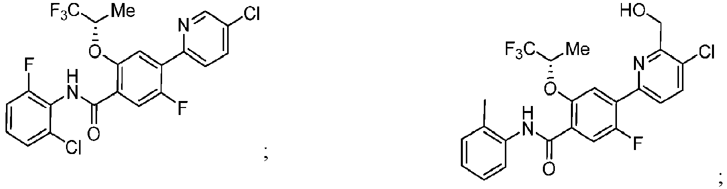 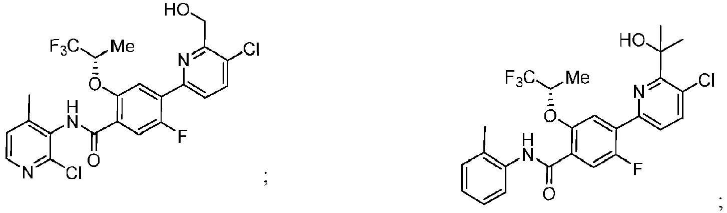 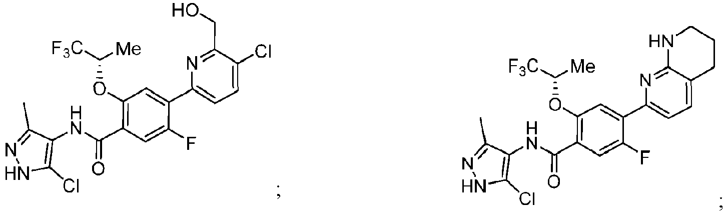 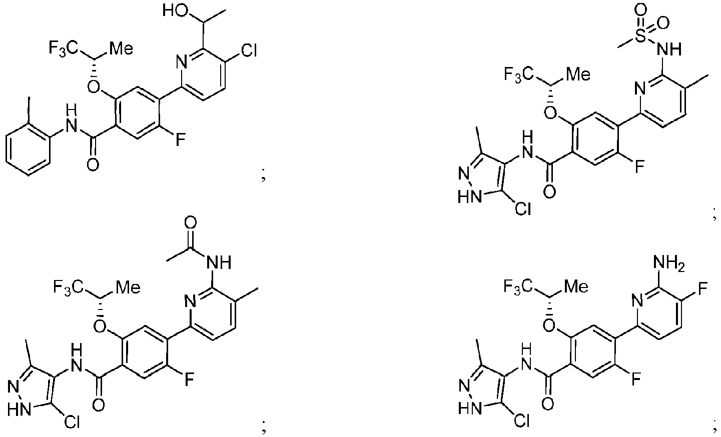 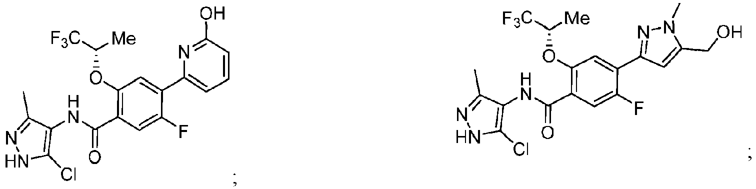 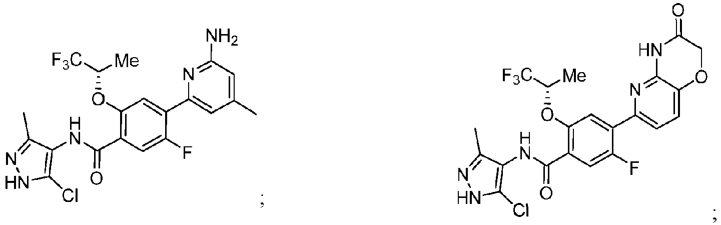 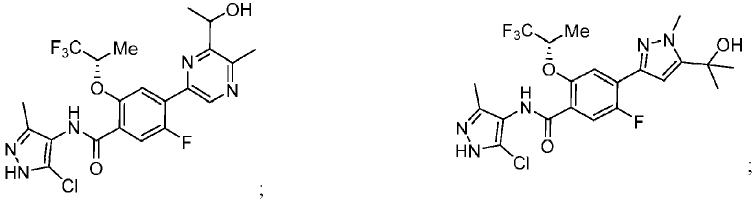 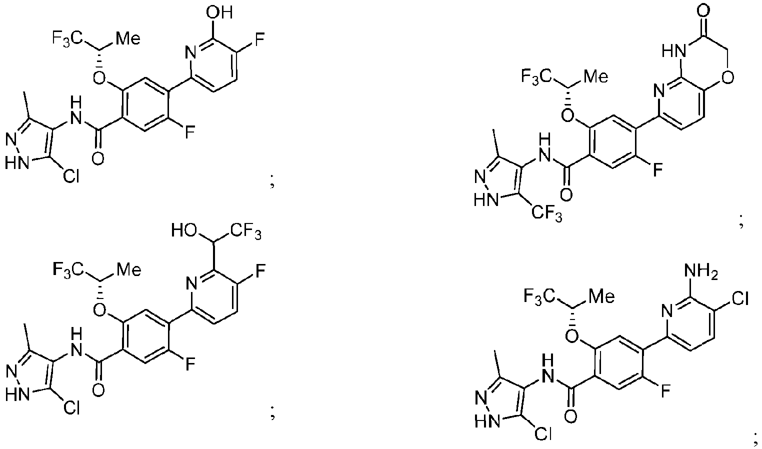 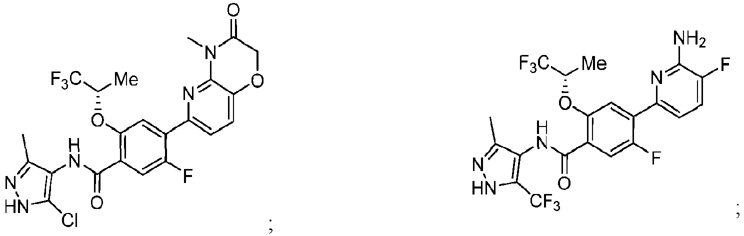 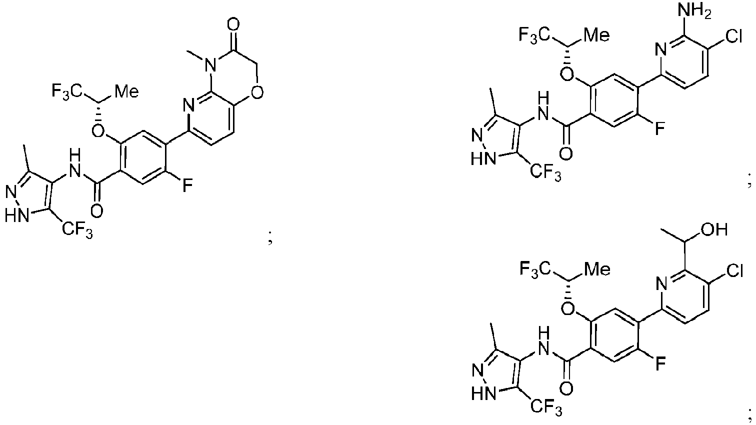 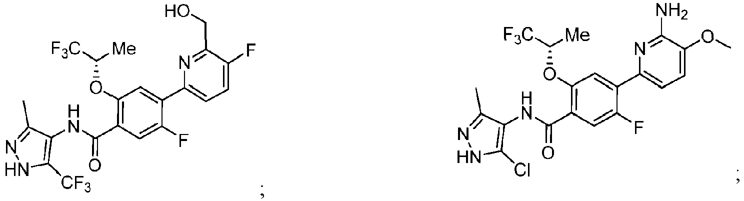 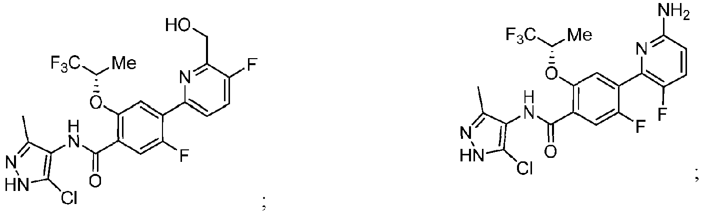 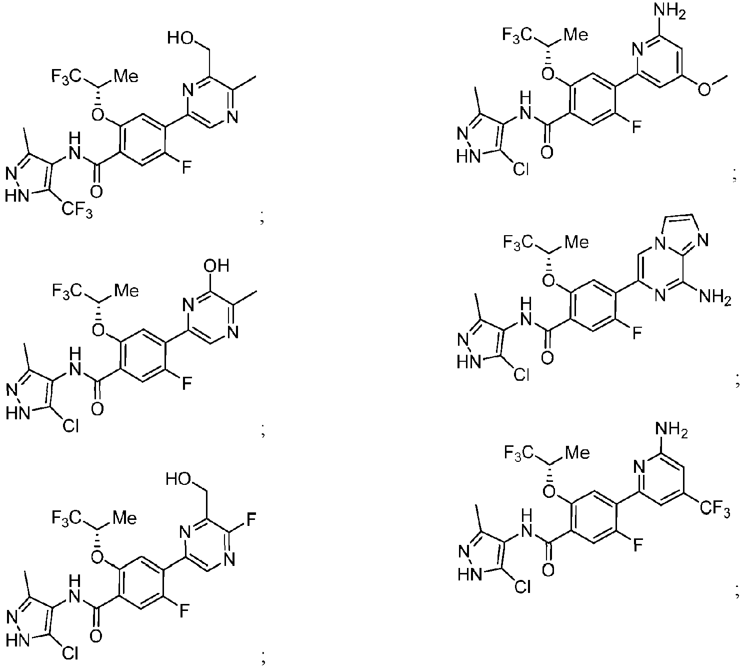 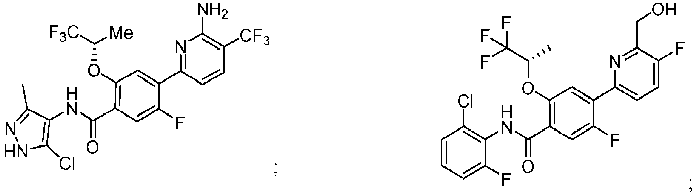 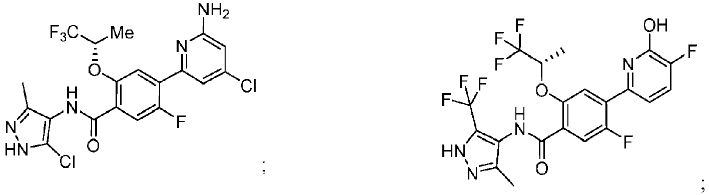 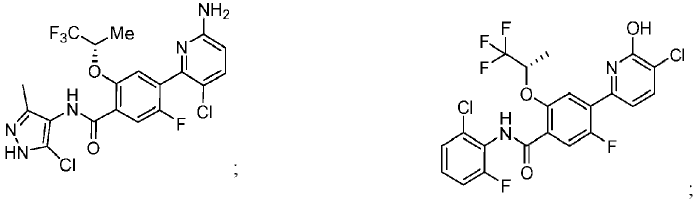 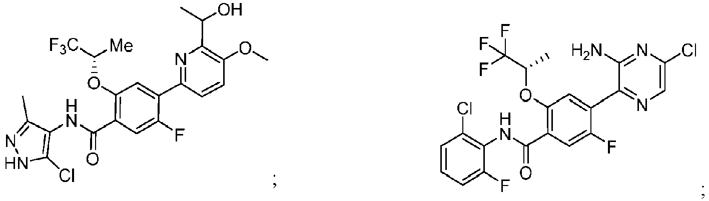 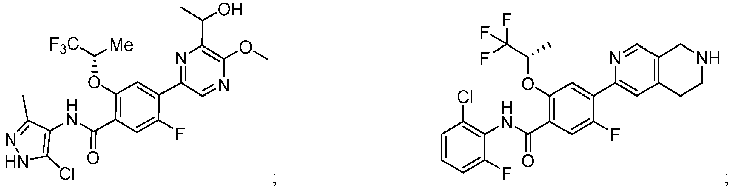 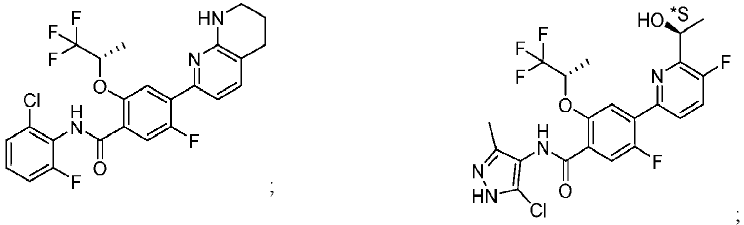 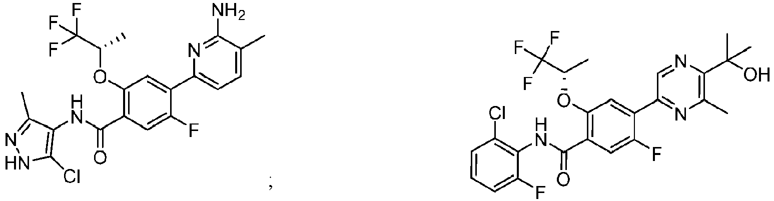 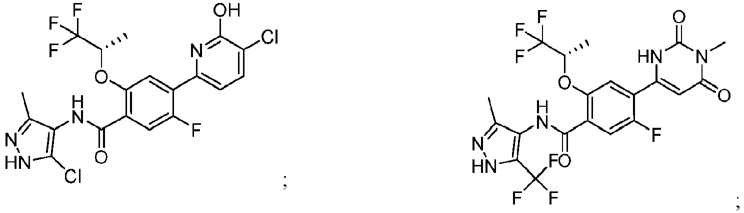 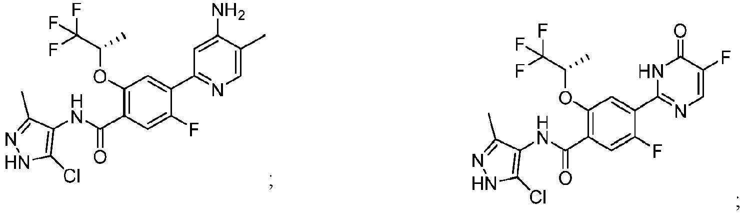 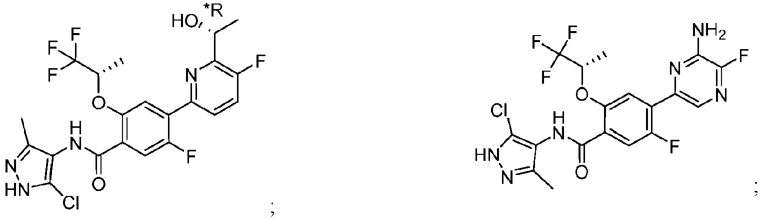 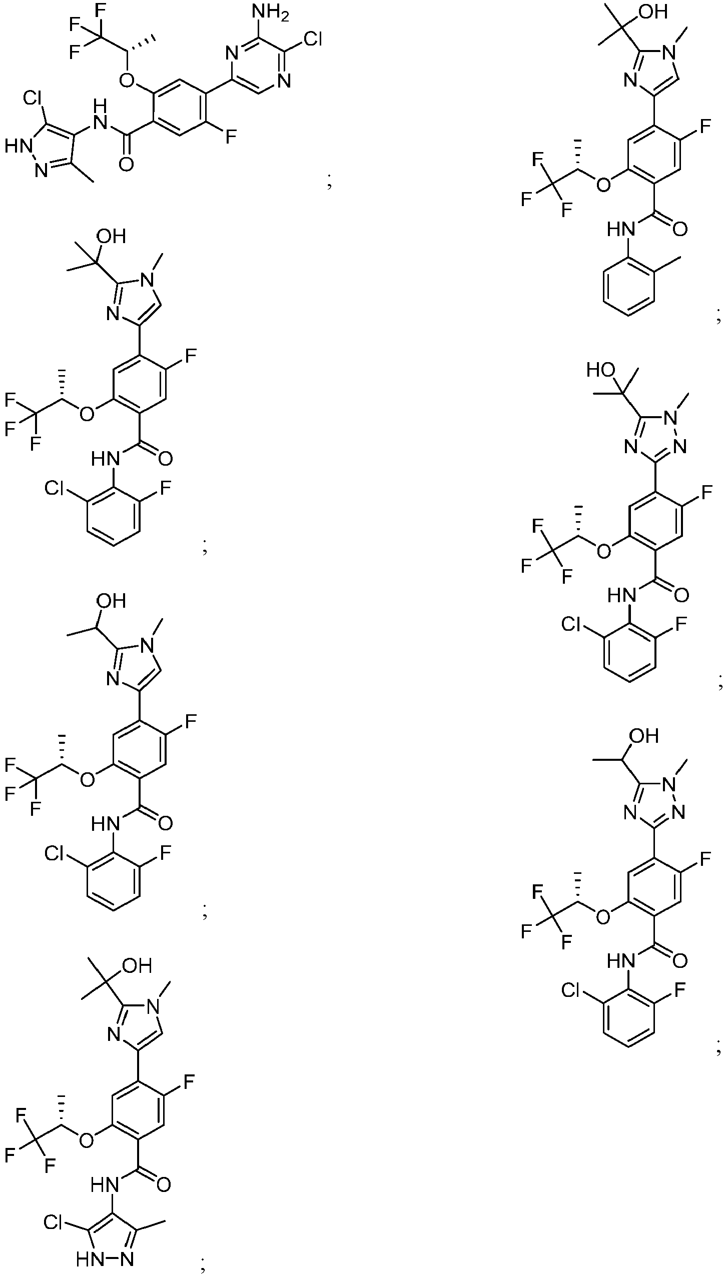 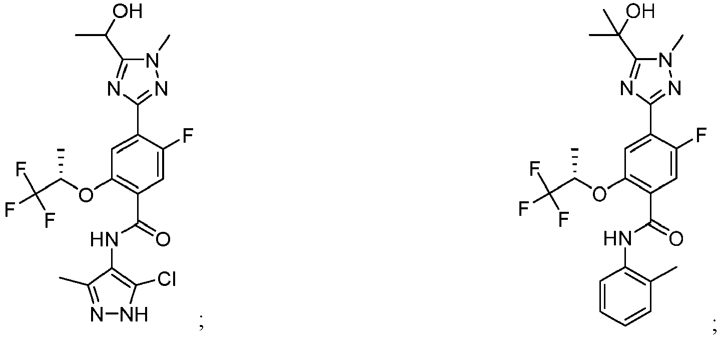 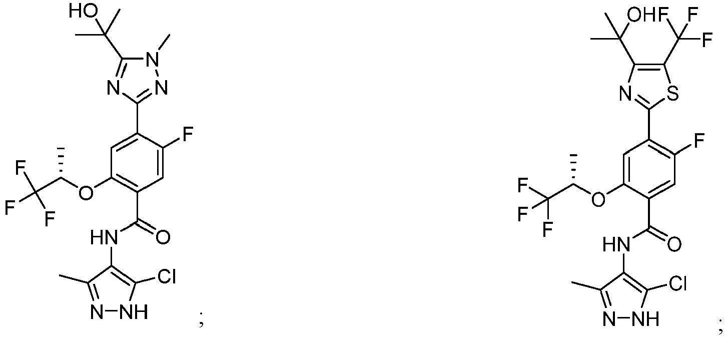 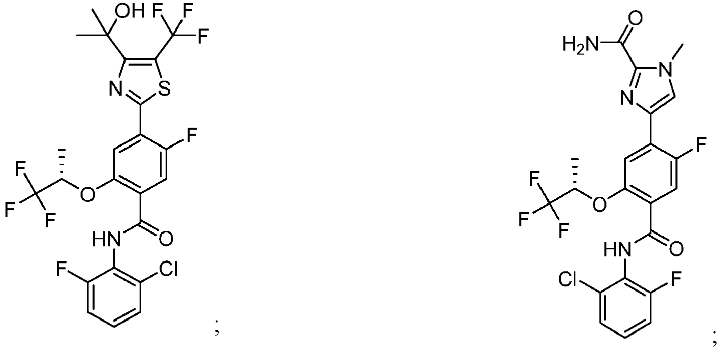 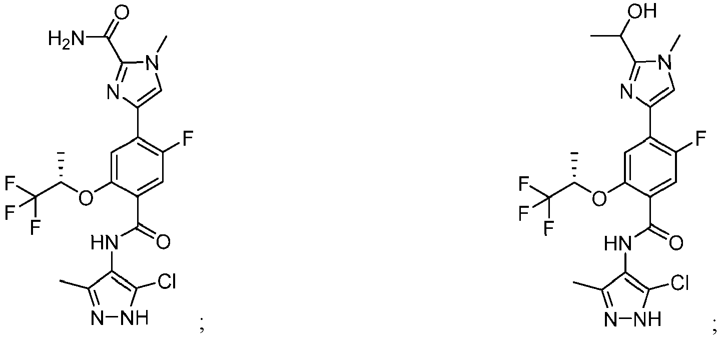 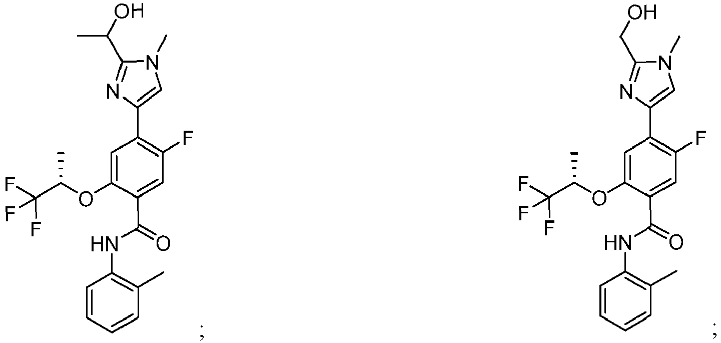 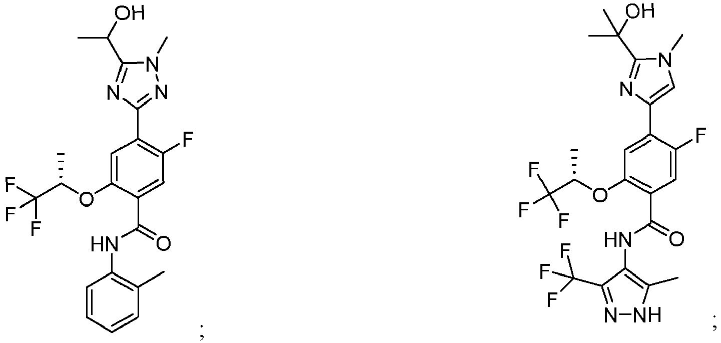 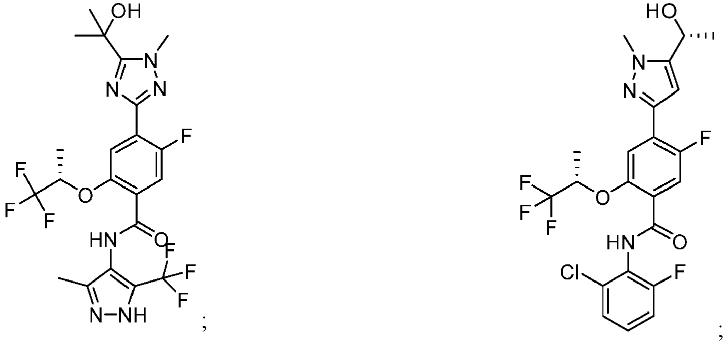 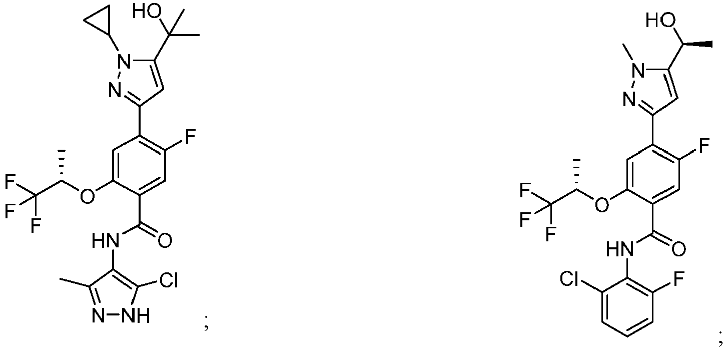 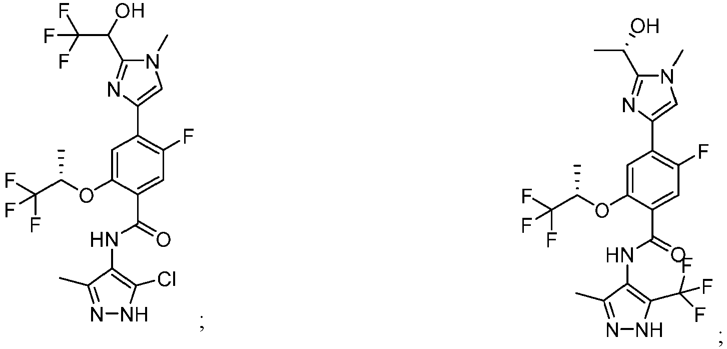 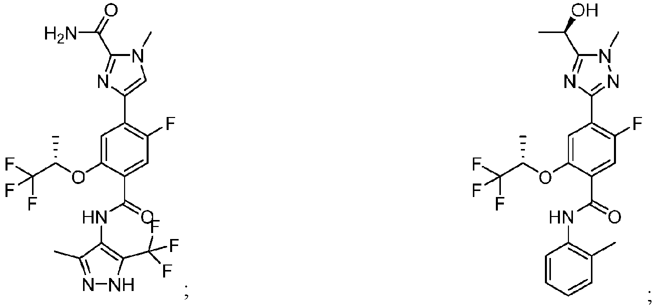 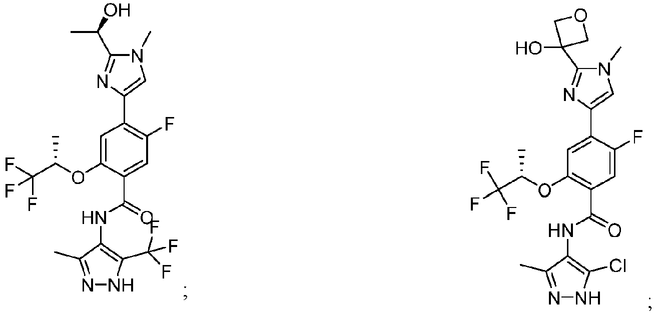 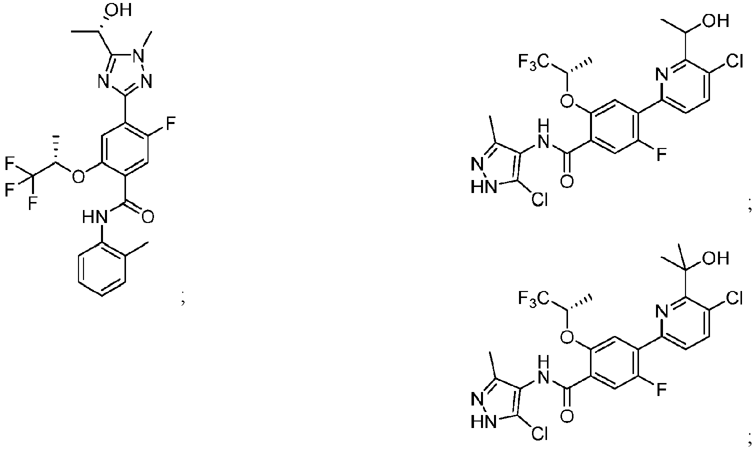 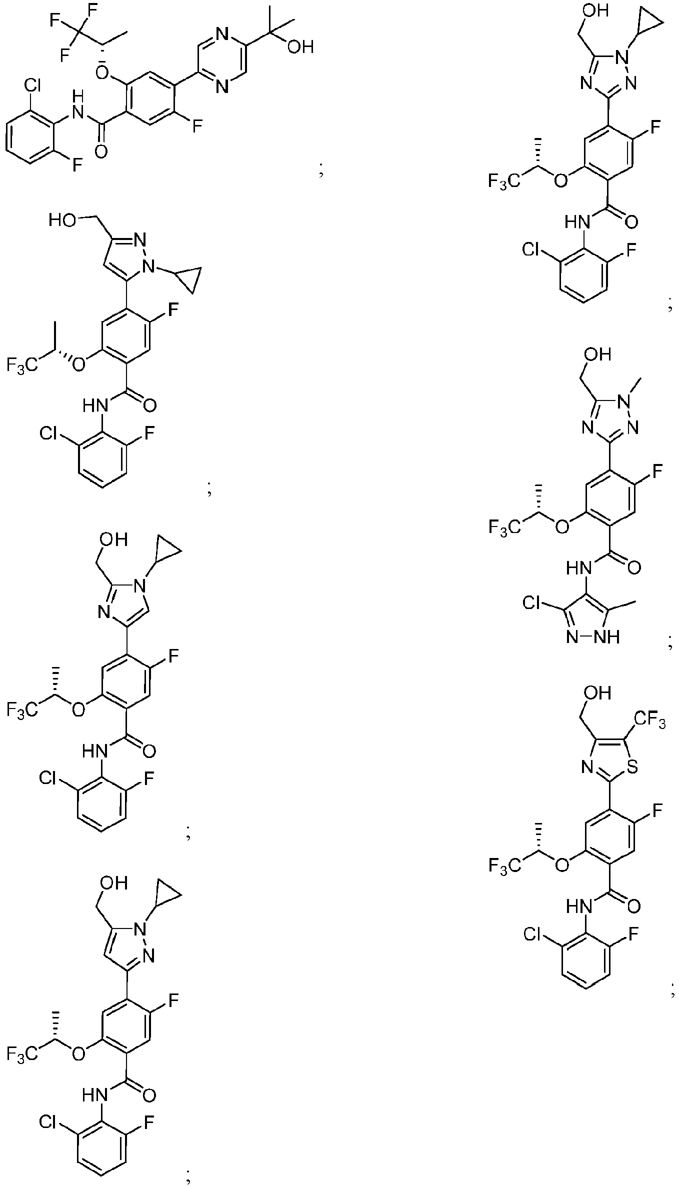 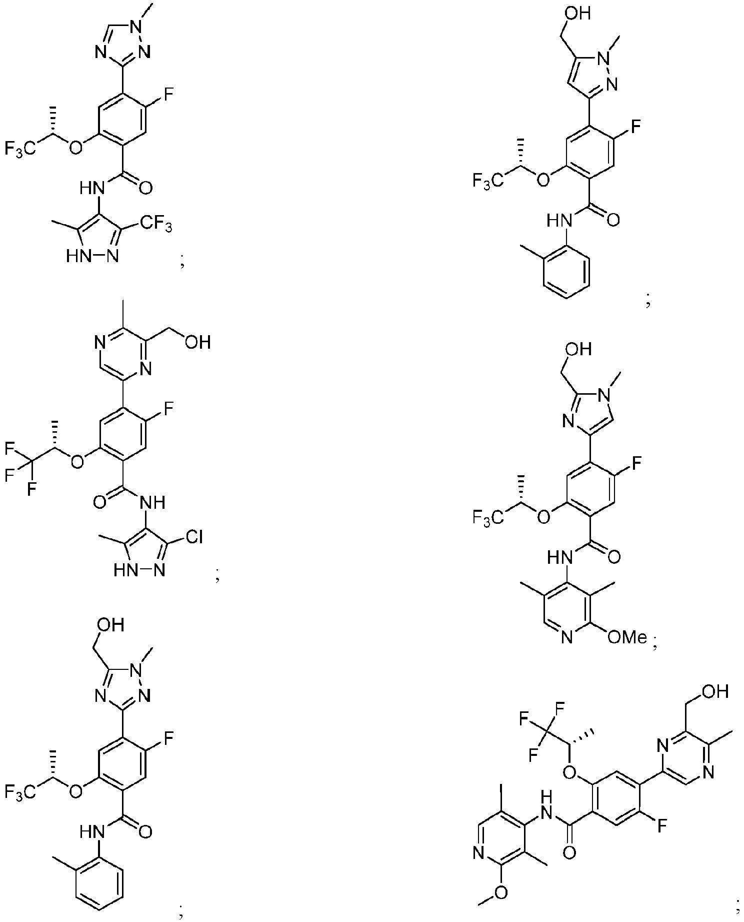 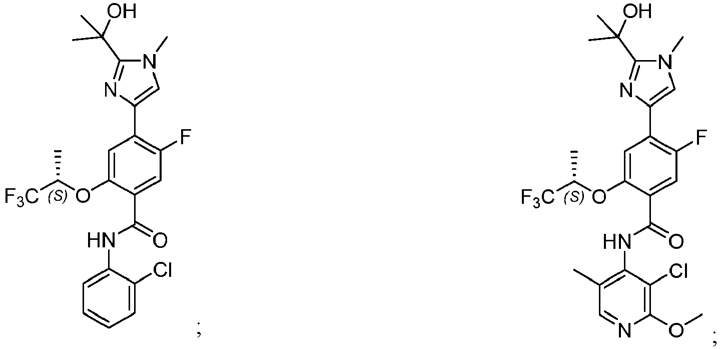 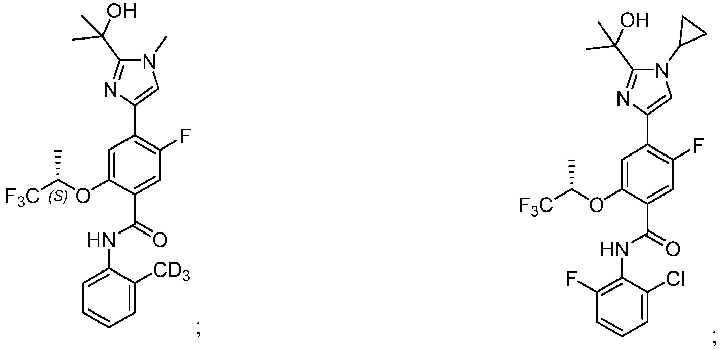 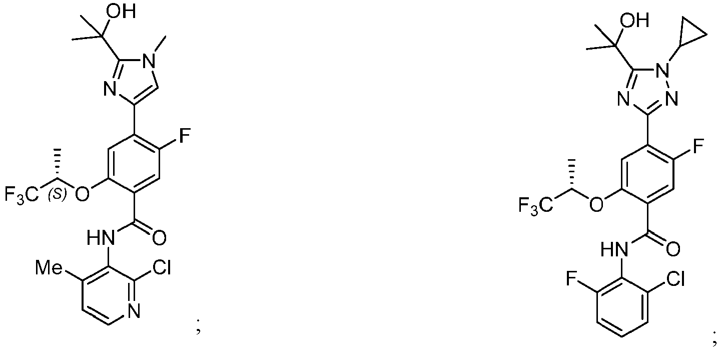 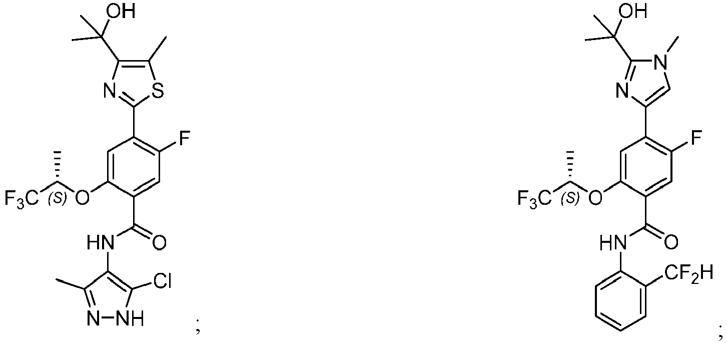 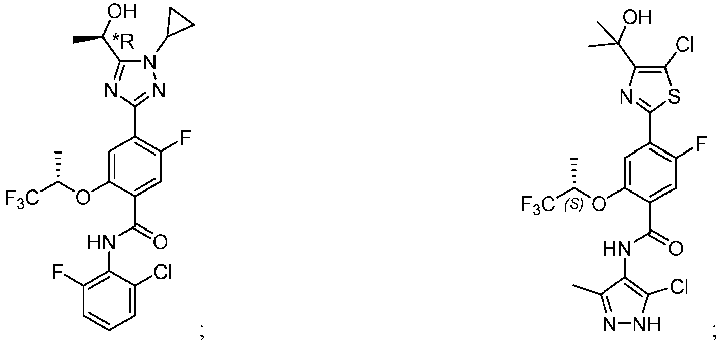 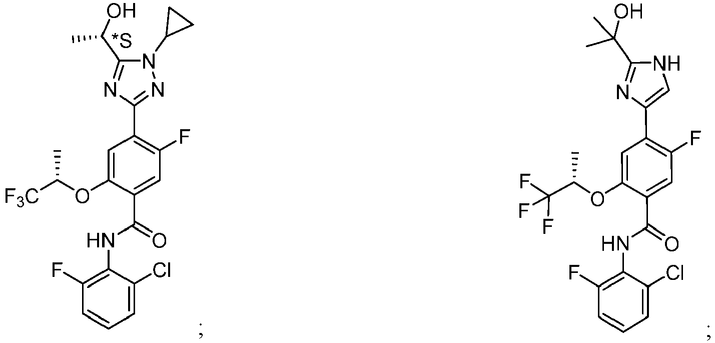 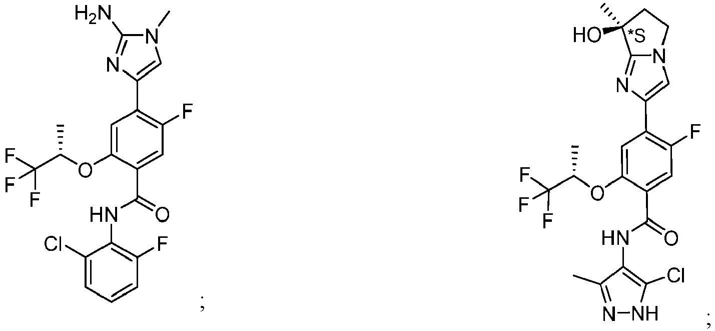 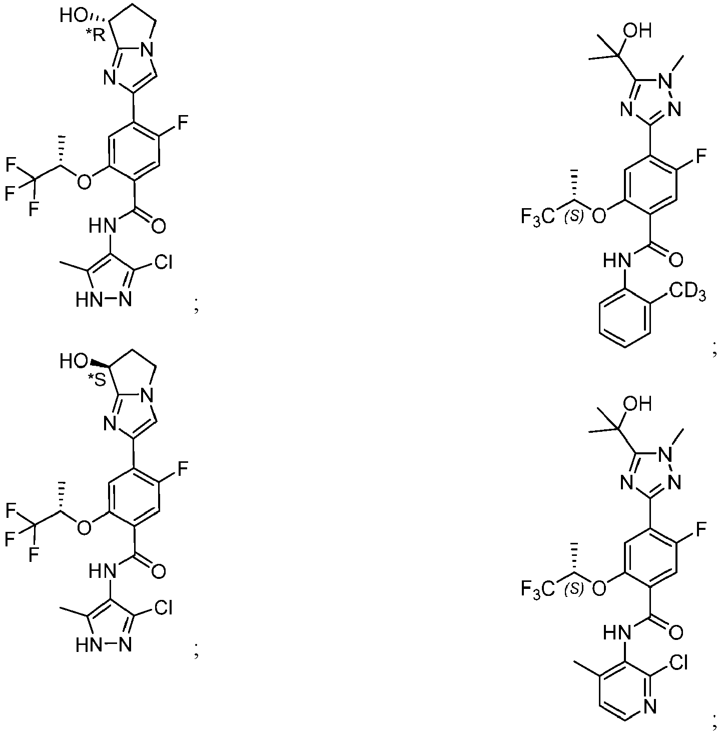 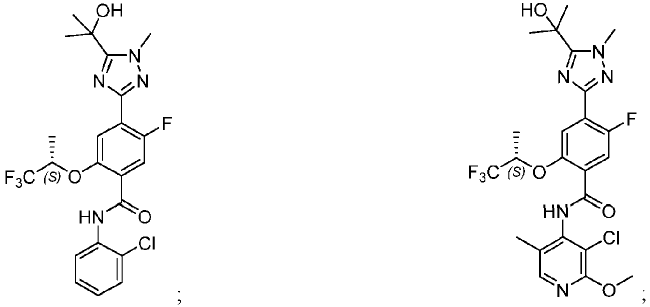 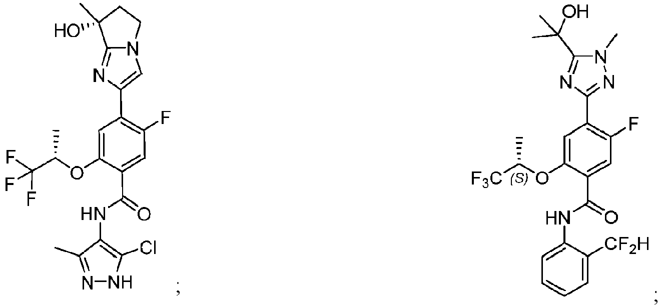 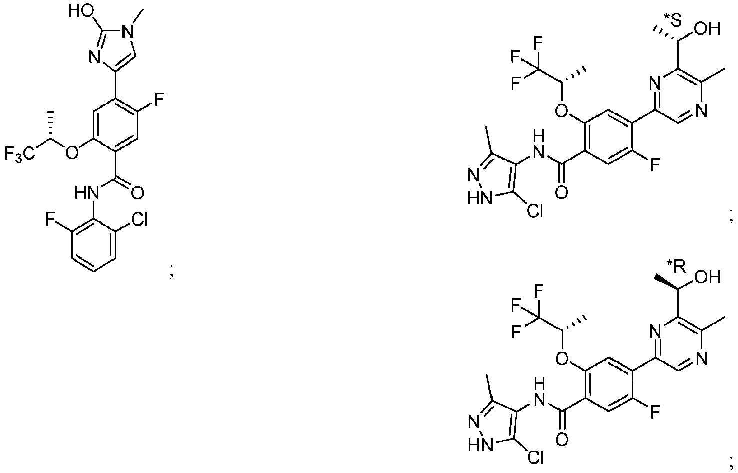 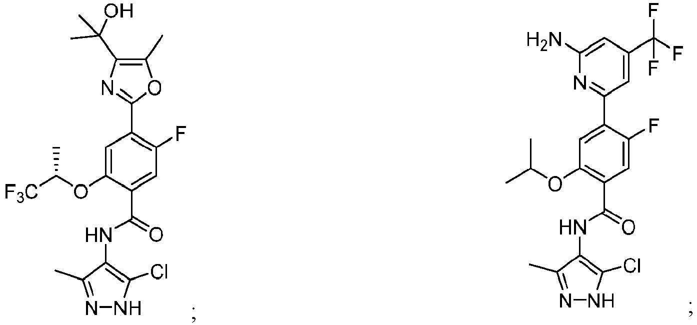 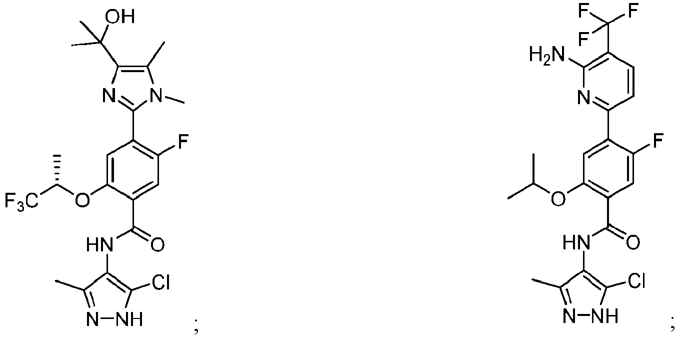 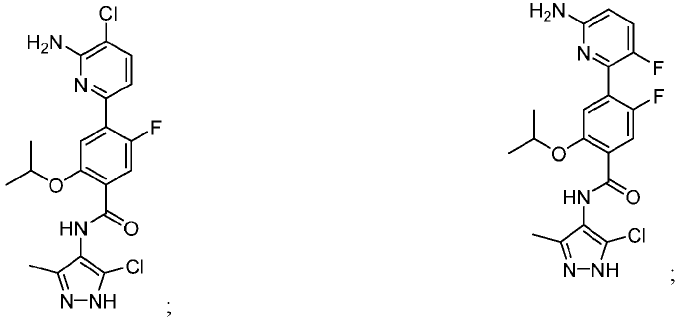 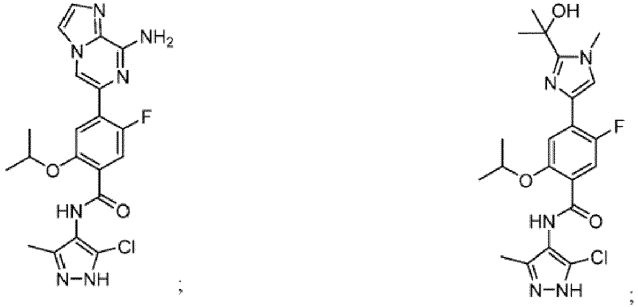 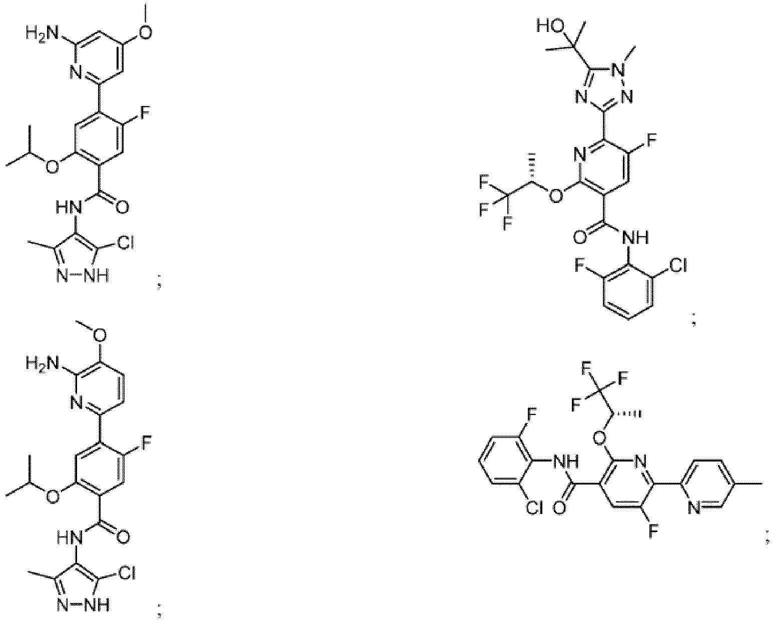 et 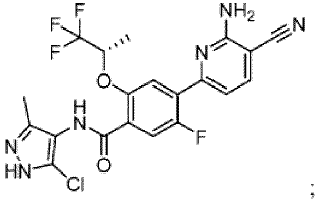 ou sel, solvate, stéréoisomère, variante isotopique ou N-oxyde pharmaceutiquement acceptable correspondant (e) (e) .

6. Composé selon la revendication 1 :
(a) Q étant CH ou N ;
R¹ étant 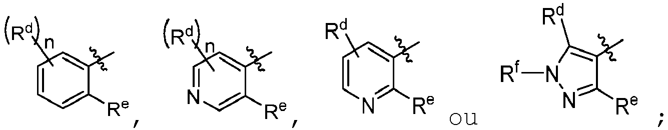
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
imidazolyle ; ledit imidazolyle étant éventuellement substitué par jusqu'à trois substituants indépendamment choisis dans le groupe comprenant 3-hydroxyoxétanyle, - NH₂, -OH, -CN ou R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(b) Q étant CH ou N ;
R¹ étant 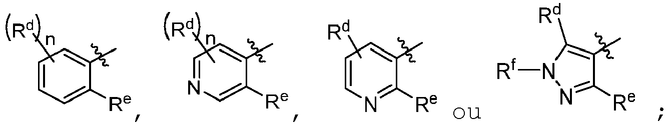
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
triazolyle ; ledit triazolyle étant éventuellement substitué par un ou deux groupes indépendamment choisis dans le groupe comprenant R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(c) Q étant CH ou N ;
R¹ étant 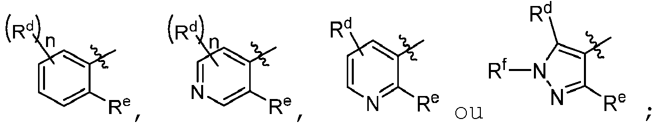
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
pyrazinyle ; ledit pyrazinyle étant éventuellement substitué par jusqu'à trois substituants indépendamment choisis dans le groupe comprenant -NHSO₂CH₃, -NHC(O)CH₃, -OCH₃, -OH, -NH₂, -CO₂H, -Cl, -F ou R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(d) Q étant CH ou N ;
R¹ étant 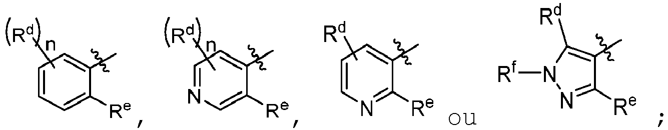
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
pyrazinonyle ; ledit pyrazinonyle étant éventuellement substitué par un ou deux groupes indépendamment choisis dans le groupe comprenant R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(e) Q étant CH ou N ;
R¹ étant 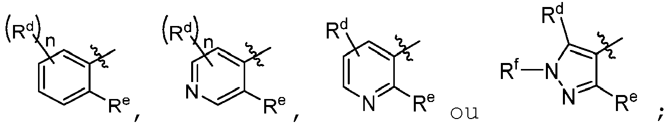
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
thiazolyle ; ledit thiazolyle étant éventuellement substitué par un ou deux substituants indépendamment choisis dans le groupe comprenant R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(f) Q étant CH ou N ;
R¹ étant 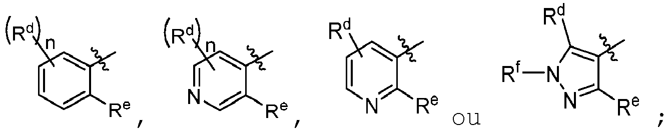
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
5,6,7,8-tétrahydro-[1,2,4]triazolo[4,3-a]pyrazinyle ; ledit 5,6,7,8-tétrahydro-[1,2,4]triazolo[4,3-a]pyrazinyle étant éventuellement substitué par R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(g) Q étant CH ou N ;
R¹ étant 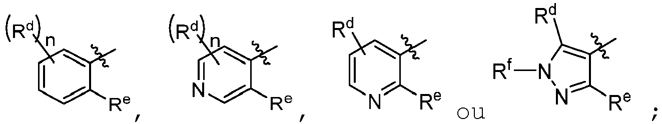
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
pyridinyle ; ledit pyridinyle étant éventuellement substitué par un ou deux substituants indépendamment choisis dans le groupe comprenant R^{a}, -Cl, -F, -CN, - NHC(O)CH₃, -NHSO₂CH₃, -CO₂H, -OCH₃, -OH ou -NH₂ ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(h) Q étant CH ou N ;
R¹ étant 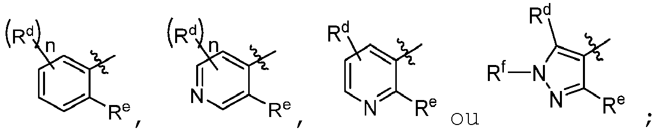
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
pyridazinyle ; ledit pyridazinyle étant éventuellement substitué par un ou deux groupes indépendamment choisis dans le groupe comprenant R^{a}, -Cl ou -CO₂H ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(i) Q étant CH ou N ;
R¹ étant 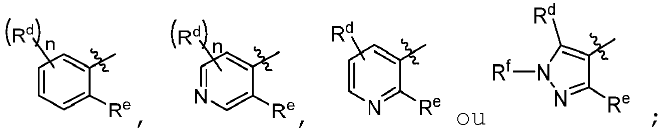
R^{d} étant choisi dans le groupe constitué par : -H, - Cl, -F, -C₍₁₋₃₎alkyle; ledit -C₍₁₋₃₎alkyle étant éventuellement substitué par : -OH, -OCH₃, -SCH₃ ou - OCF₃; et ledit -C₍₁₋₃₎alkyle étant éventuellement substitué par jusqu'à six atomes de fluor ;
R^{e} étant choisi dans le groupe constitué par : -Cl, - F et -C₍₁₋₃₎alkyle, -CD₃ et cyclopropyle ; ledit C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
4,5,6,7-tétrahydrothiazolo[5,4-c]pyridinyle ; ledit 4,5,6,7-tétrahydrothiazolo[5,4-c]pyridinyle étant éventuellement substitué par R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(j) Q étant CH ou N ;
R¹ étant 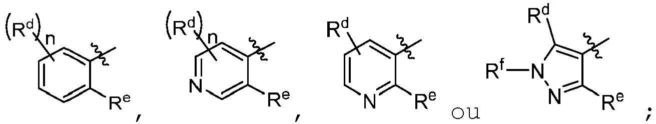
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
pyrazolyle ; ledit pyrazolyle étant éventuellement substitué par un ou deux substituants indépendamment choisis dans le groupe comprenant R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(k) Q étant CH ou N ;
R¹ étant 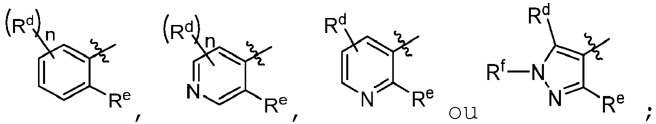
R^{d} étant choisi dans le groupe constitué par : -H, - Cl, -F, -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F et -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
oxazolyle ; ledit oxazolyle étant éventuellement substitué par un ou deux substituants indépendamment choisis dans le groupe comprenant R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(l) Q étant CH ou N ;
R¹ étant 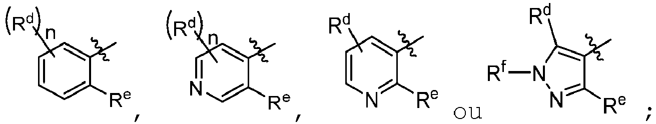
R^{d} étant choisi dans le groupe constitué par : -H, - Cl, -F et -C₍₁₋₃₎alkyle ; ledit -C₍₁₋₃₎alkyle étant éventuellement substitué par : -OH, -OCH₃, -SCH₃ ou - OCF₃; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
1,3,4-oxadiazolyle ; ledit 1,3,4-oxadiazolyle étant éventuellement substitué par R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle; ledit -C₍₃₋₆₎cycloalkyle étant éventuellement substitué par jusqu'à quatre substituants indépendamment choisis dans le groupe constitué par : -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(m) Q étant CH ou N ;
R¹ étant 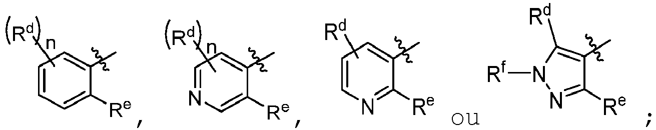
R^{d} étant choisi dans le groupe constitué par : -H, - Cl, -F et -C₍₁₋₃₎alkyle; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par : -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
5,6,7,8-tétrahydroimidazo[1,5-a]pyridinyle ; ledit 5,6,7,8-tétrahydroimidazo[1,5-a]pyridinyle étant éventuellement substitué par R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par . -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(n) Q étant CH ou N ;
R¹ étant 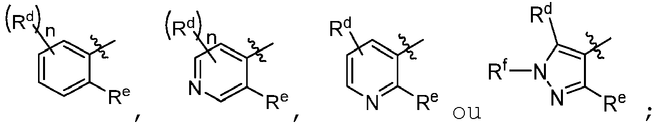
R^{d} étant choisi dans le groupe constitué par . -H, - Cl, -F, -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par . -Cl, - F et -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
1,2,4-thiadiazolyle ; ledit 1,2,4-thiadiazolyle étant éventuellement substitué par R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par . -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(o) Q étant CH ou N ;
R¹ étant 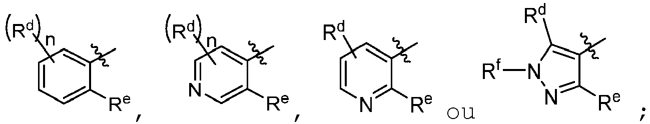
R^{d} étant choisi dans le groupe constitué par . -H, - Cl, -F, -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par . -Cl, - F et -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
5,6,7,8-tétrahydro-1,7-naphtyridinyle ; ledit 5,6,7,8-tétrahydro-1,7-naphtyridinyle étant éventuellement substitué par R^{a} ; ou
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou - OCH₃ ; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle ; ledit -C₍₃₋₆₎cycloalkyle étant éventuellement substitué par jusqu'à quatre substituants indépendamment choisis dans le groupe constitué par . -Cl, -F, -OH et -C₍₁₋₆₎alkyle ; ledit -C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e) ; ou
(p) Q étant CH ou N ;
R¹ étant 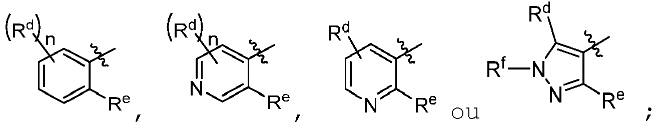
R^{d} étant choisi dans le groupe constitué par . -H, - Cl, -F et -C₍₁₋₃₎alkyle ; ledit -C₍₁₋₃₎alkyle étant éventuellement substitué par : -OH, -OCH₃, -SCH₃ ou - OCF₃ ; et ledit -C₍₁₋₃₎alkyle étant éventuellement substitué par jusqu'à six atomes de fluor ;
R^{e} étant choisi dans le groupe constitué par . -Cl, - F, -C₍₁₋₃₎alkyle, -CD₃ et cyclopropyle ; ledit -C₍₁₋₃₎alkyle étant éventuellement substitué par : -OH, -OCH₃, -SCH₃ ou -OCF₃ ; et ledit -C₍₁₋₃₎alkyle étant éventuellement substitué par jusqu'à six atomes de fluor ;
R^{f} étant choisi dans le groupe constitué par : -H et -C₍₁₋₃₎alkyle ; ledit -C₍₁₋₃₎alkyle étant éventuellement substitué par un membre choisi dans le groupe constitué par : -OH, -OCH₃, -SCH₃ et -OCF₃ ; et ledit -C₍₁₋₃₎alkyle étant éventuellement substitué par jusqu'à six atomes de fluor ; et
n étant 1 ou 2 ;
R² étant
5,6,7,8-tétrahydro-1,6-naphtyridinyle ; ledit 5,6,7,8-tétrahydro-1,6-naphtyridinyle étant éventuellement substitué par R^{a} ;
R^{a} étant -C(O)NH₂, cyclopropyle ou -C₍₁₋₃₎alkyle ; ledit - C₍₁₋₃₎alkyle étant éventuellement substitué par jusqu'à trois atomes de fluor, ou le -C₍₁₋₃₎alkyle étant éventuellement substitué par -OH et de plus éventuellement substitué par jusqu'à trois atomes de fluor ; et ledit cyclopropyle étant éventuellement substitué par -CH₂OH ;
R³ étant -CHR^{b}R^{c} ;
R^{b} étant -C₍₃₋₆₎cycloalkyle ou -C₍₁₋₆₎alkyle ; ledit -C₍₁₋₆₎alkyle étant éventuellement substitué par -Cl, -OH ou-OCH₃; et ledit -C₍₁₋₆₎alkyle étant éventuellement substitué par jusqu'à six atomes de fluor ;
R^{c} étant -CH₃ ou -CHF₂ ; ou R^{b} et R^{c} pouvant être pris ensemble avec l'atome de carbone auquel ils sont liés pour former -C₍₃₋₆₎cycloalkyle; ledit -C₍₃₋₆₎cycloalkyle étant éventuellement substitué par jusqu'à quatre substituants indépendamment choisis dans le groupe constitué par . -Cl, -F, -OH et -C₍₁₋₆₎alkyle ; ledit C₍₁₋₆₎alkyle étant éventuellement substitué par jusqu'à six atomes de fluor ;
ou sel, solvate, stéréoisomère, variante isotopique ou N-oxyde pharmaceutiquement acceptable correspondant(e)(e).

7. Composé selon la revendication 4 choisi dans le groupe constitué par :
(a) 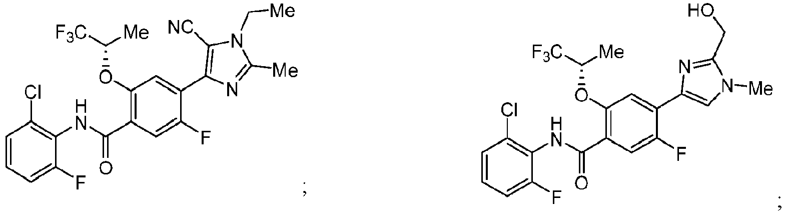 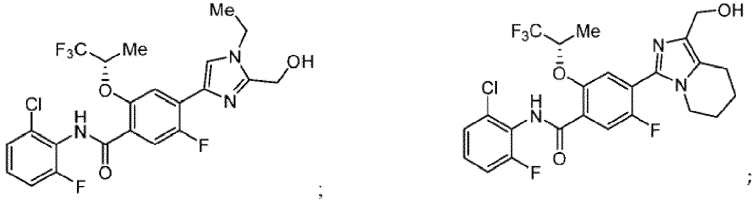 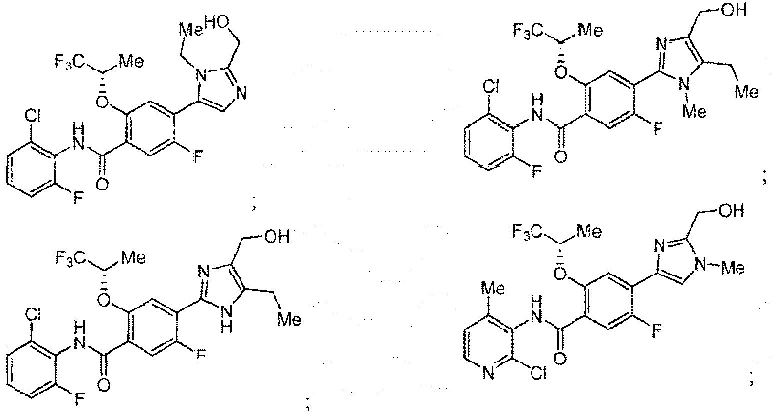 et 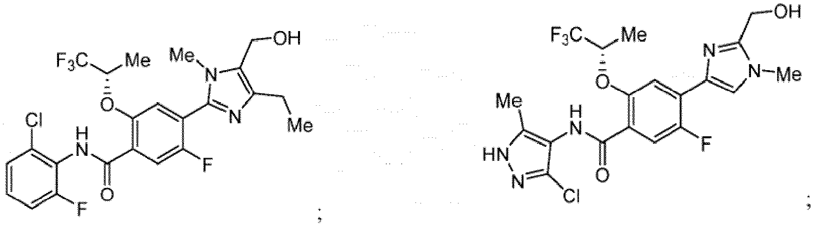 ou sel, solvate, stéréoisomère, variante isotopique ou N-oxyde pharmaceutiquement acceptable correspondant(e)(e) ; ou
(b) 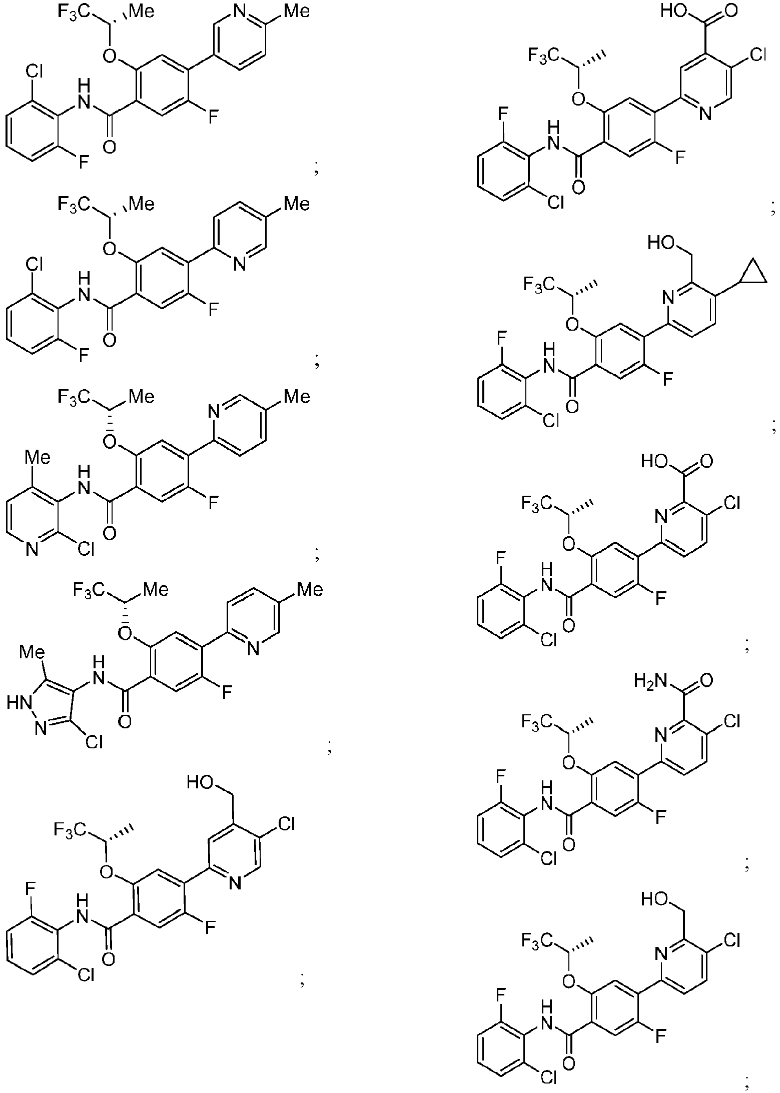 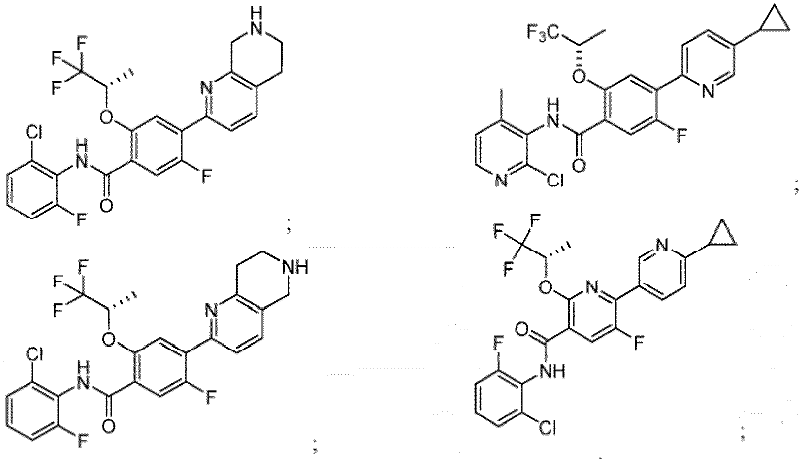 et 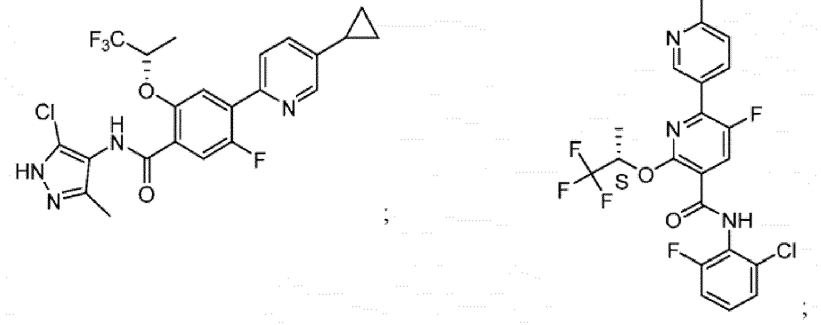 ou sel, solvate, stéréoisomère, variante isotopique ou N-oxyde pharmaceutiquement acceptable correspondant(e)(e) ; ou
(c) 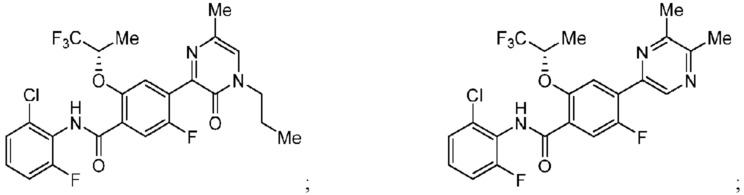 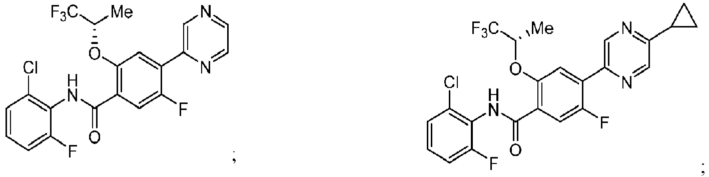 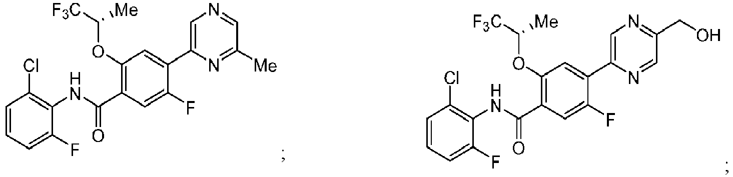 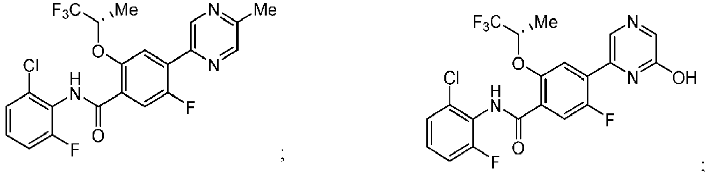 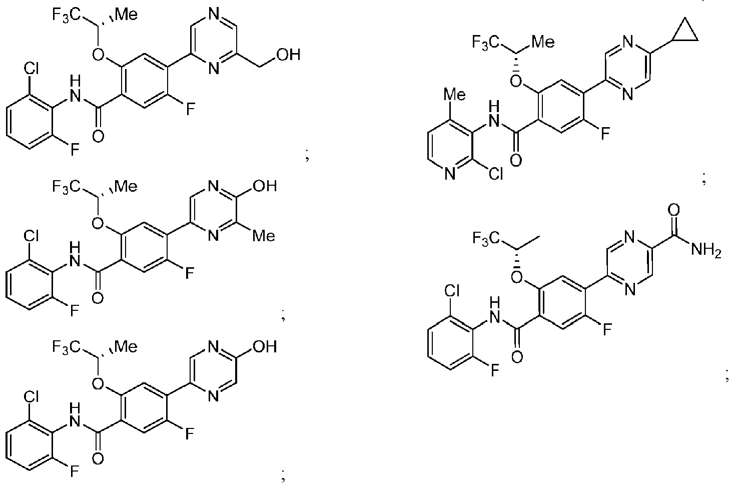 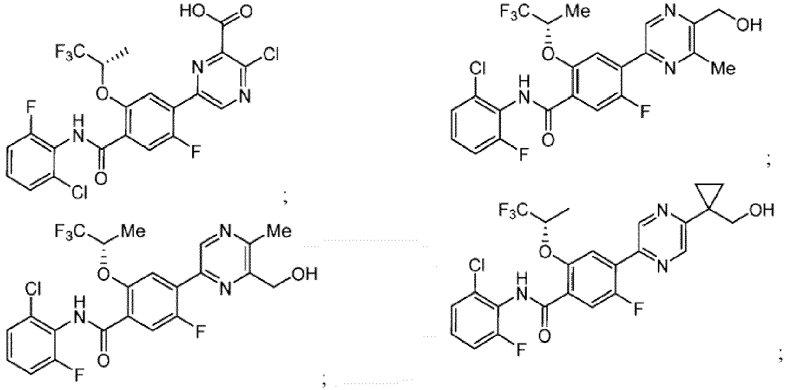 et 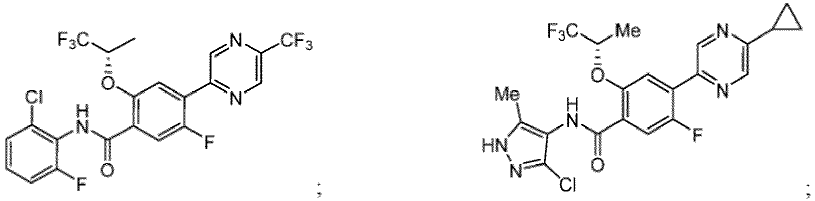 ou sel, solvate, stéréoisomère, variante isotopique ou N-oxyde pharmaceutiquement acceptable correspondant(e) ; ou
(d) 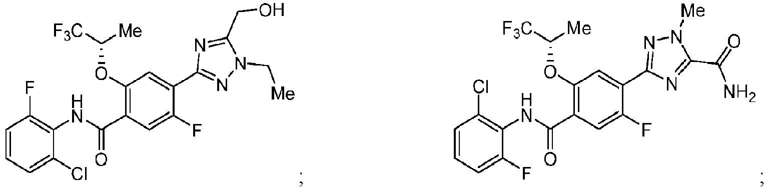 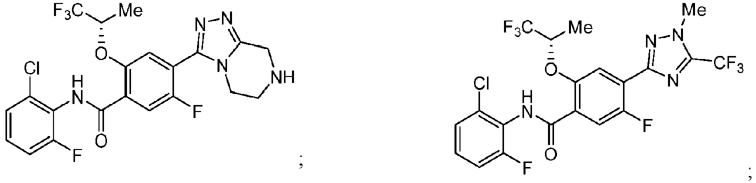 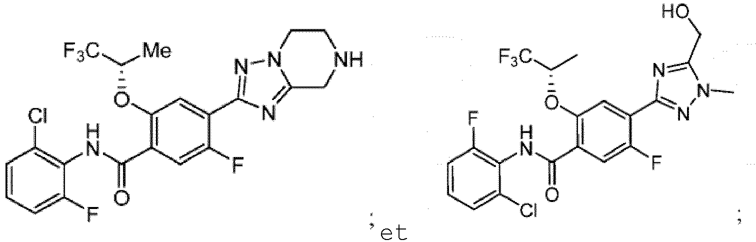 ou sel, solvate, stéréoisomère, variante isotopique ou N-oxyde pharmaceutiquement acceptable correspondant(e) ; ou
(e) 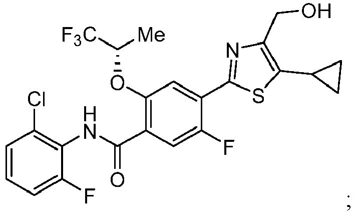 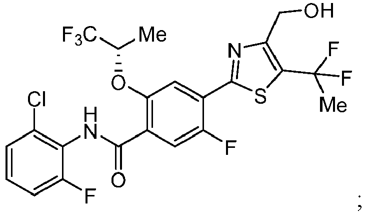 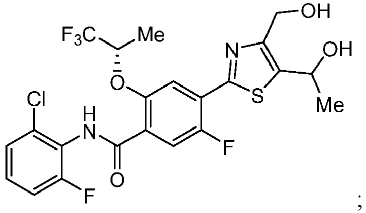 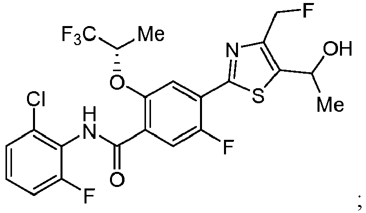 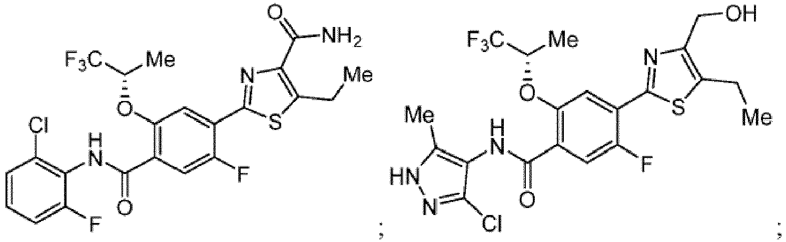 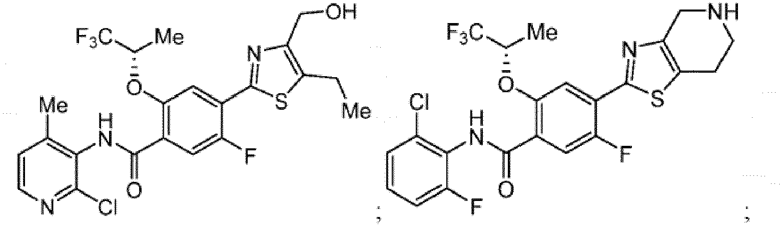 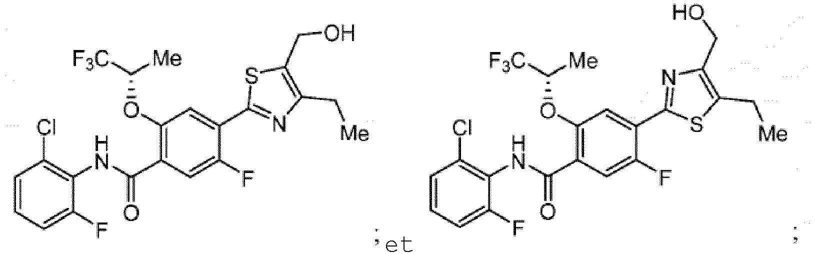 ou sel, solvate, stéréoisomère, variante isotopique ou N-oxyde pharmaceutiquement acceptable correspondant(e) ; ou
(f) 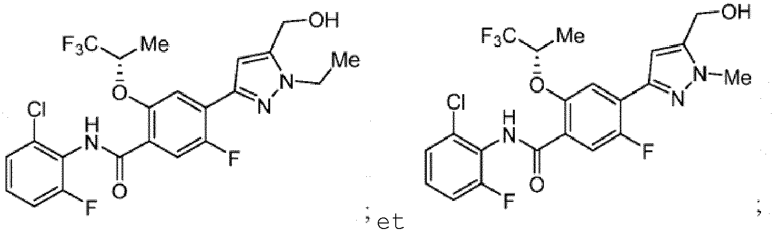 ou sel, solvate, stéréoisomère, variante isotopique ou N-oxyde pharmaceutiquement acceptable correspondant(e) ; ou
(g) 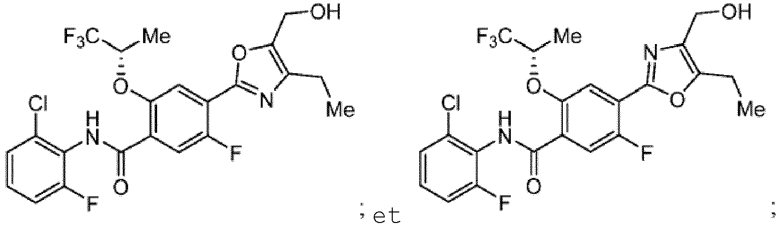 ou sel, solvate, stéréoisomère, variante isotopique ou N-oxyde pharmaceutiquement acceptable correspondant(e).

8. Composé selon la revendication 4 qui est :
(a) 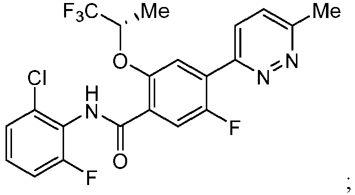 ou un sel, un solvate, un stéréoisomère, une variante isotopique ou un N-oxyde pharmaceutiquement acceptable correspondant(e) ; ou
(b) 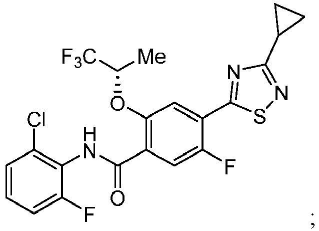 ou un sel, un solvate, un stéréoisomère, une variante isotopique ou un N-oxyde pharmaceutiquement acceptable correspondant(e) ; ou
(c) 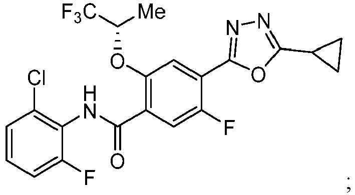 ou un sel, un solvate, un stéréoisomère, une variante isotopique ou un N-oxyde pharmaceutiquement acceptable correspondant(e).

9. Composé selon la revendication 4 choisi dans le groupe constitué par :
(a) 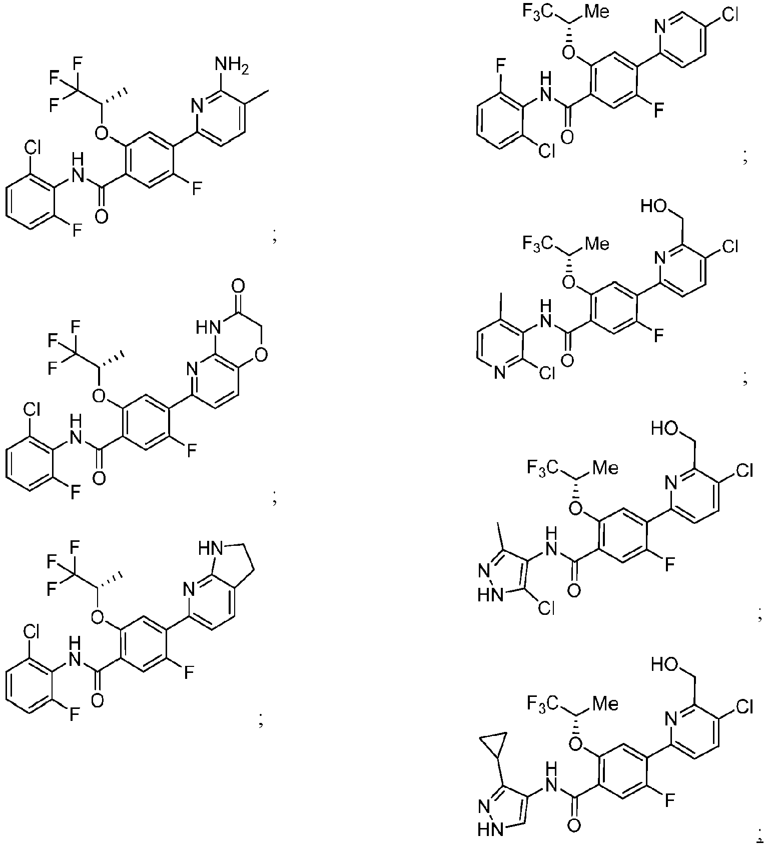 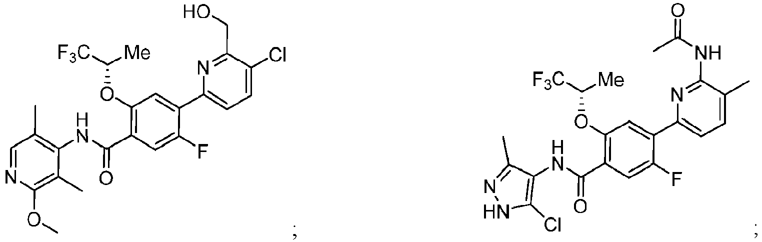 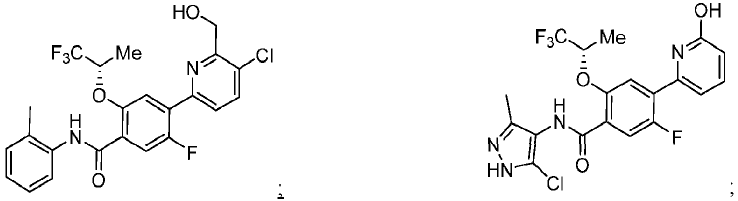 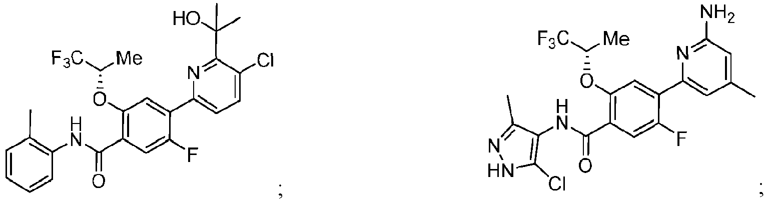 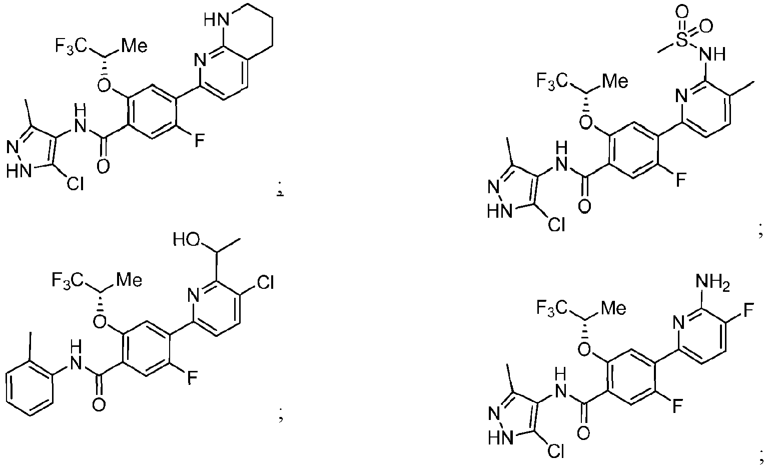 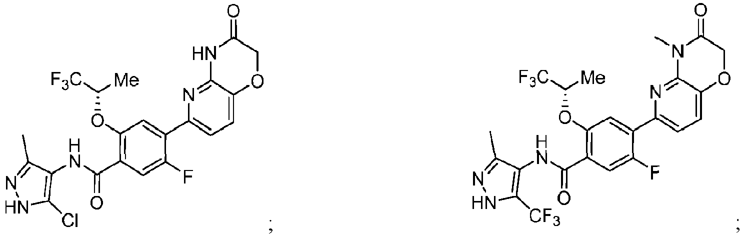 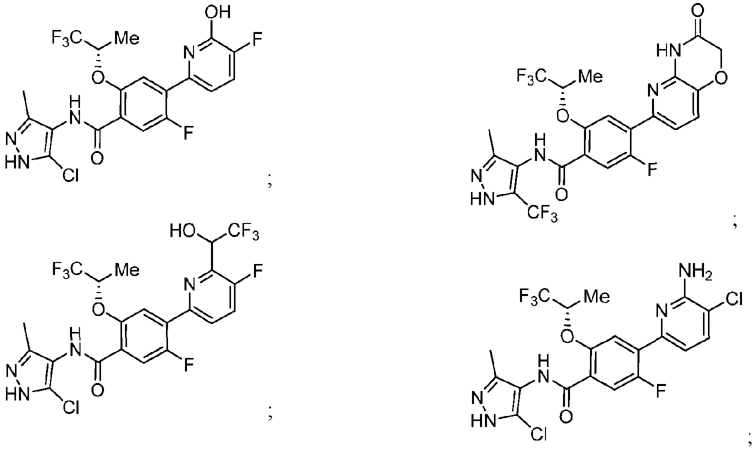 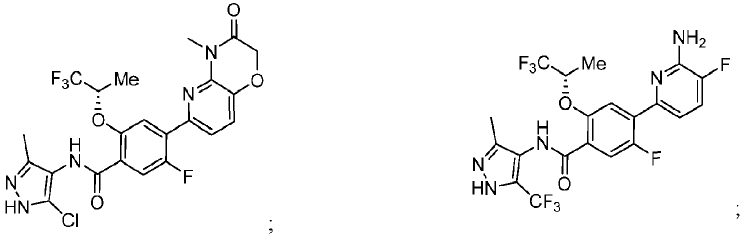 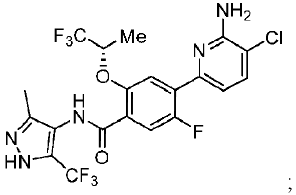 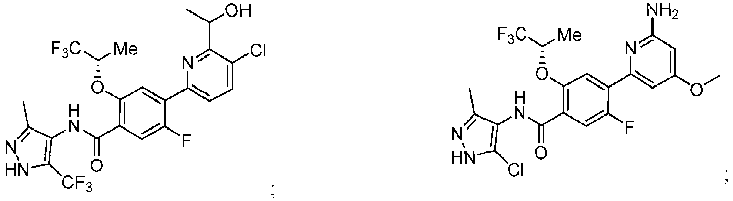 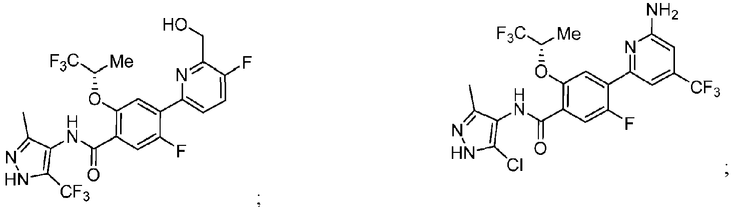 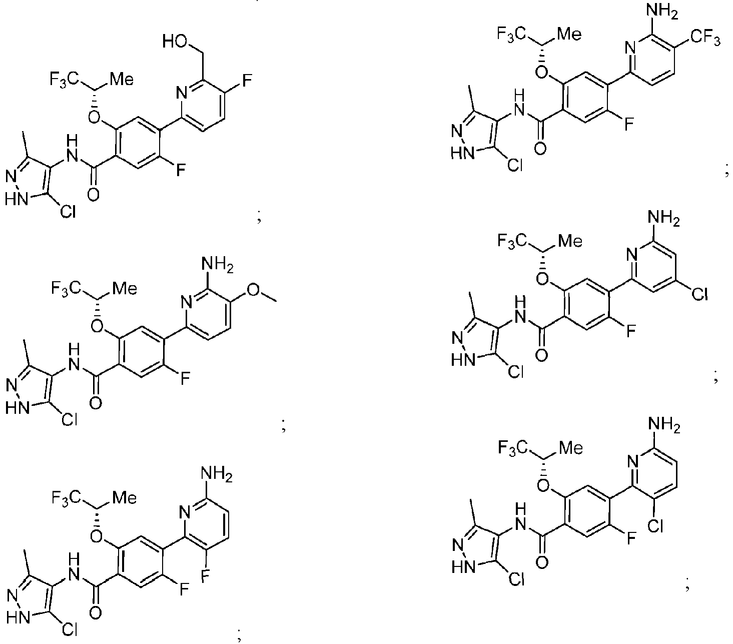 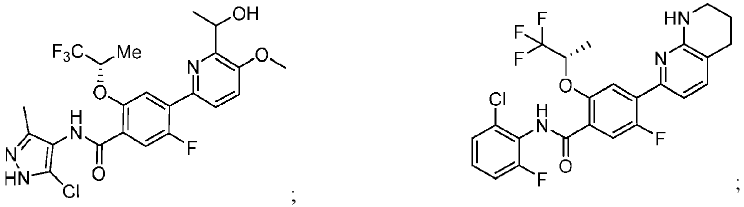 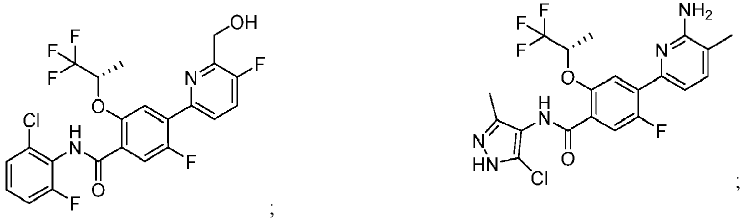 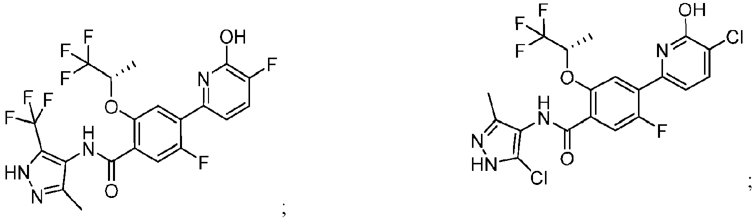 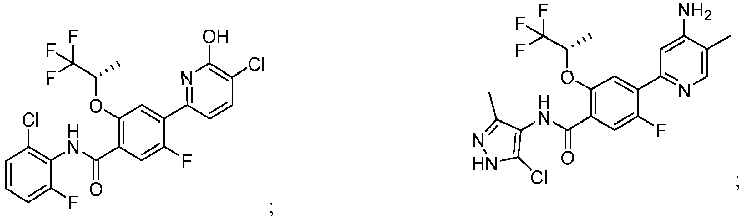 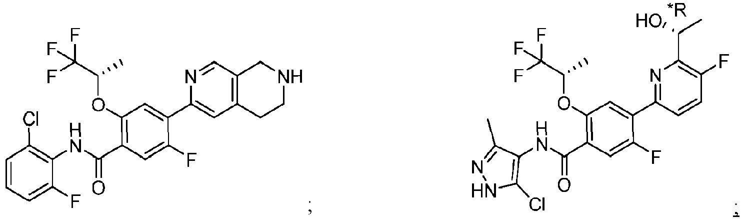 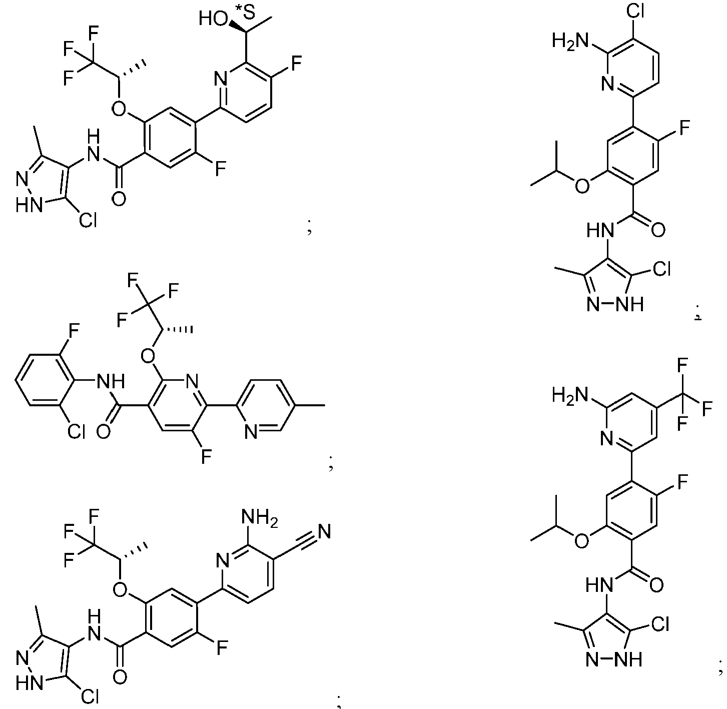 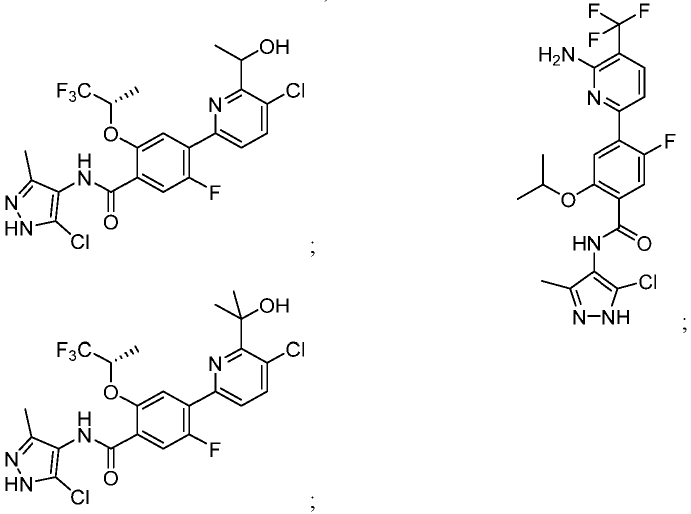 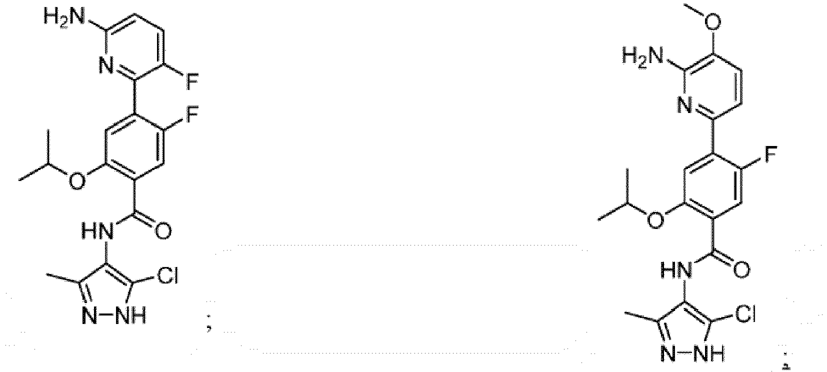 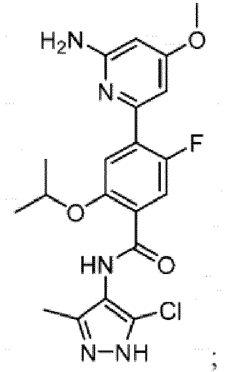 et ou sel, solvate, stéréoisomère, variante isotopique ou N-oxyde pharmaceutiquement acceptable correspondant(e) ; ou
(b) 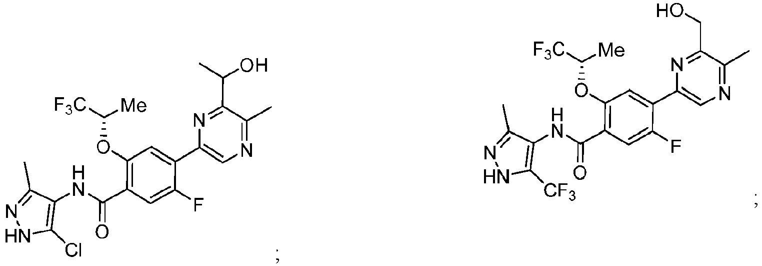 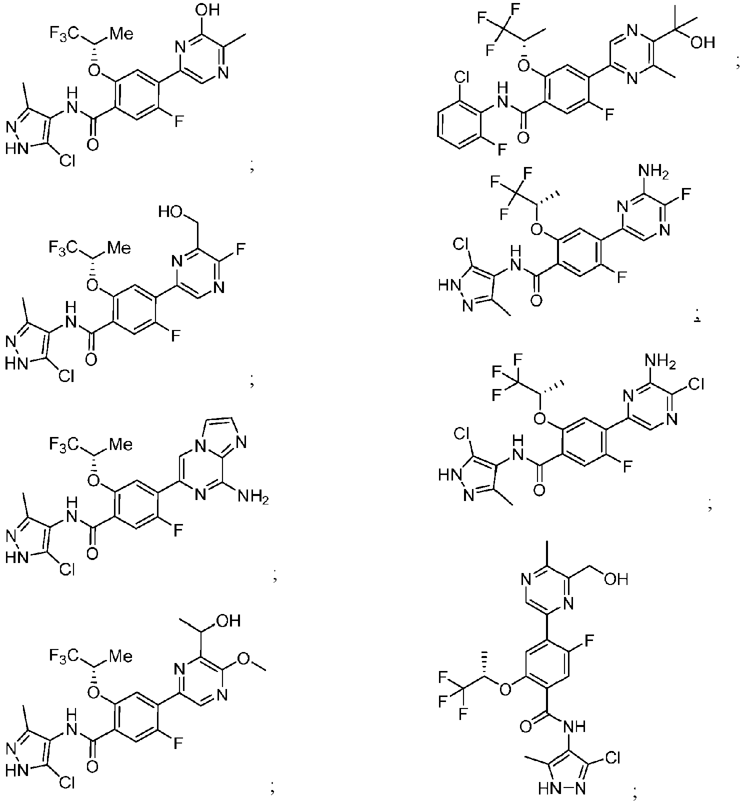 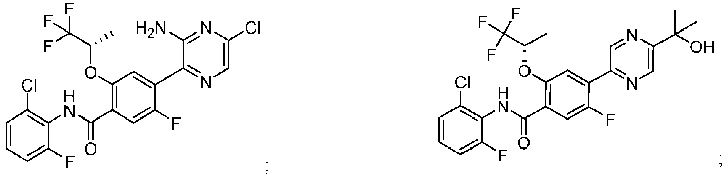 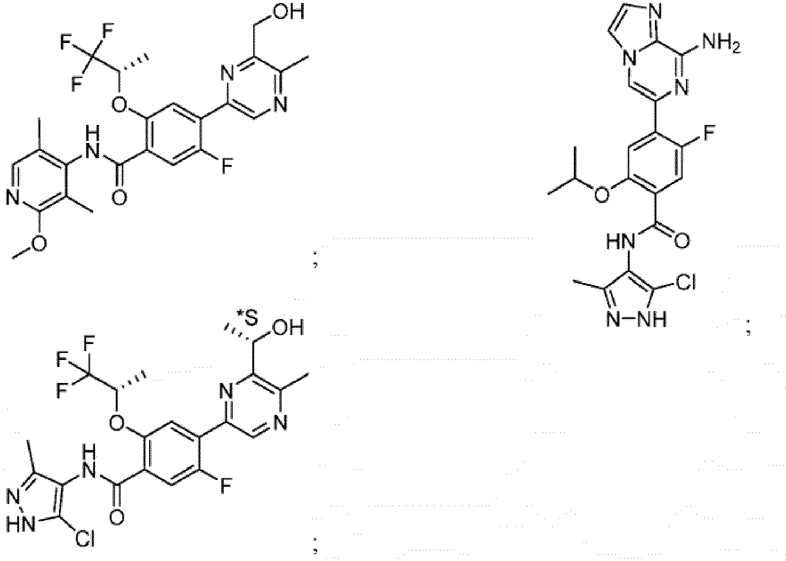 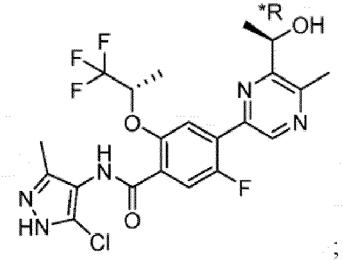 et ou sel, solvate, stéréoisomère, variante isotopique ou N-oxyde pharmaceutiquement acceptable correspondant(e) ; ou
(c) 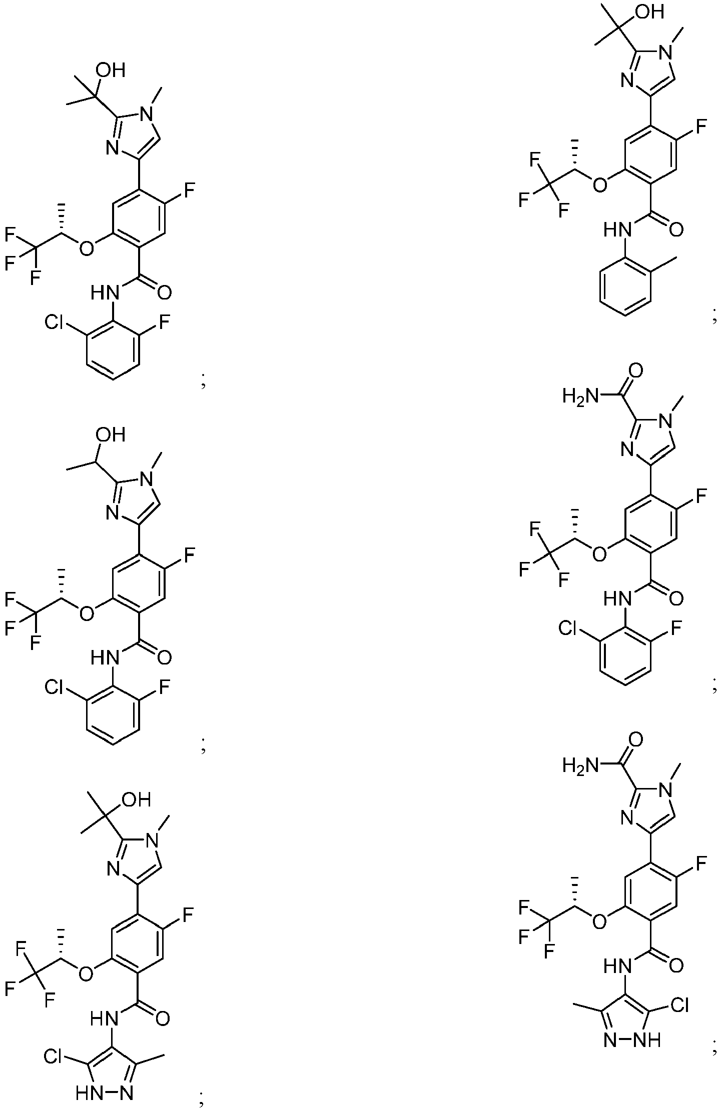 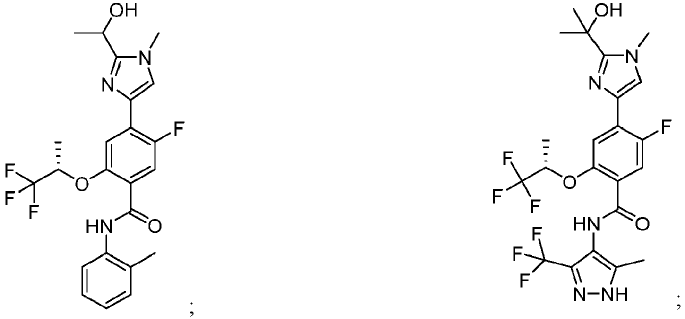 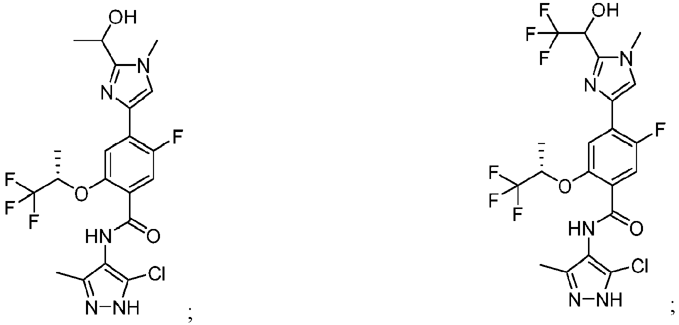 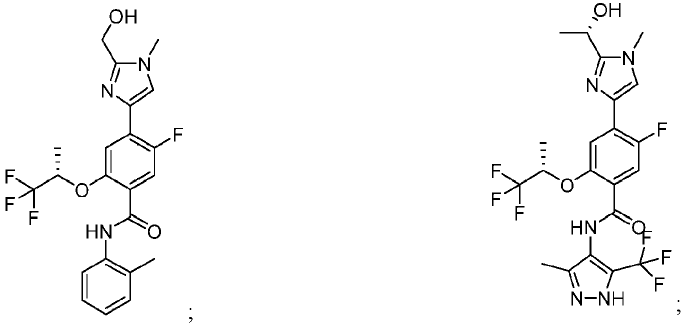 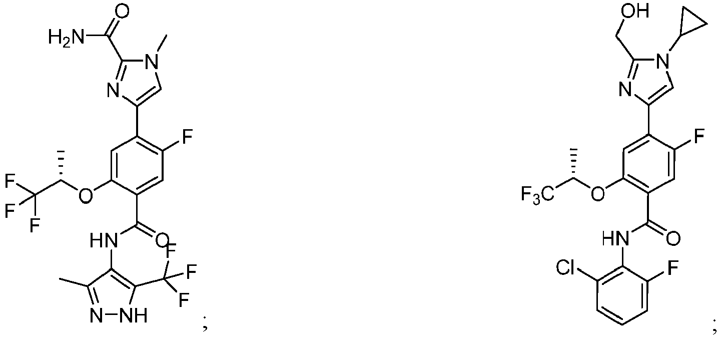 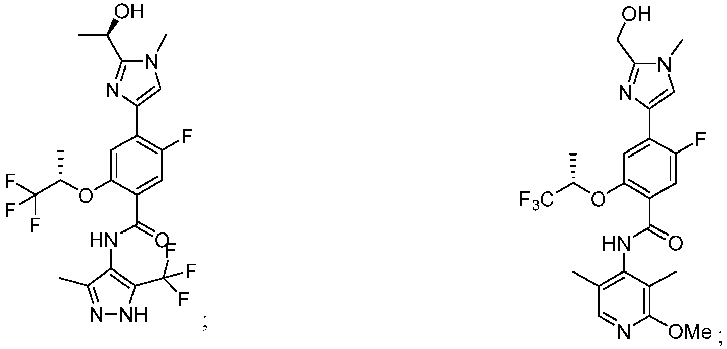 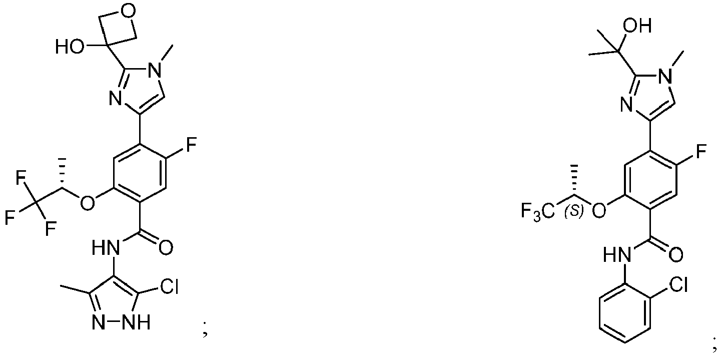 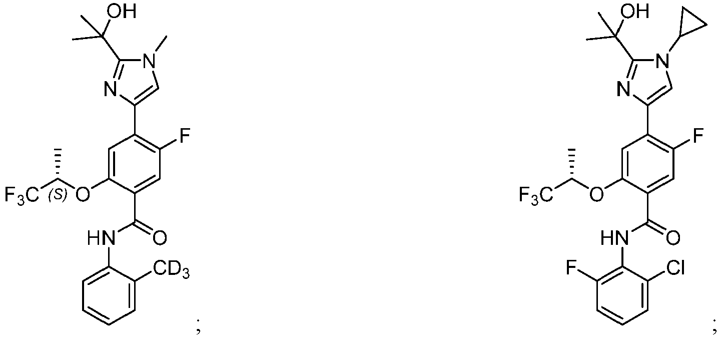 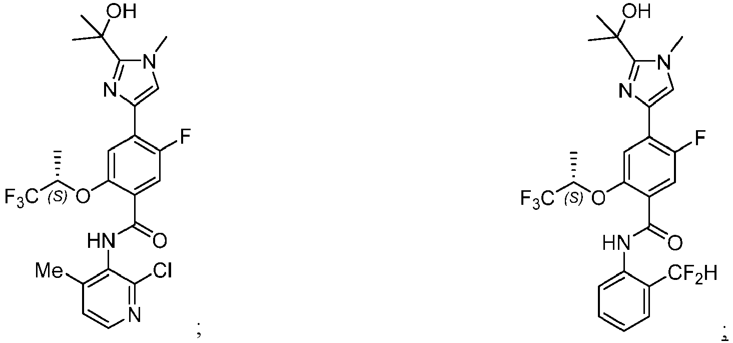 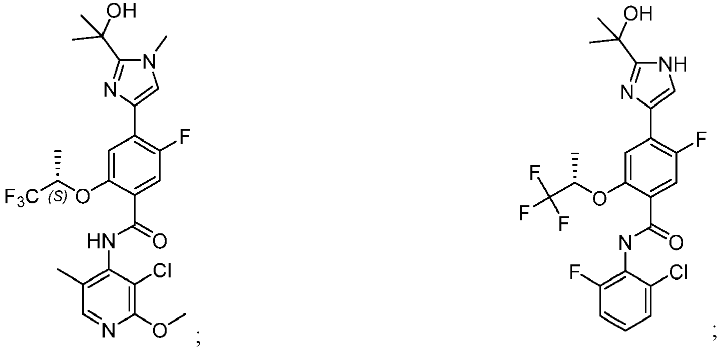 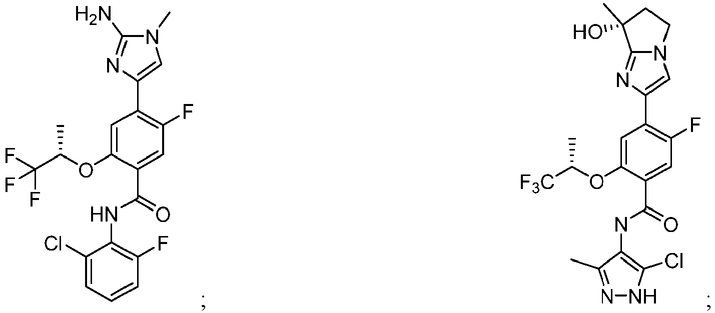 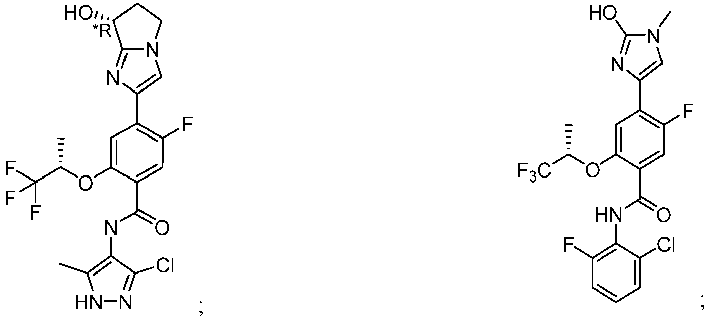 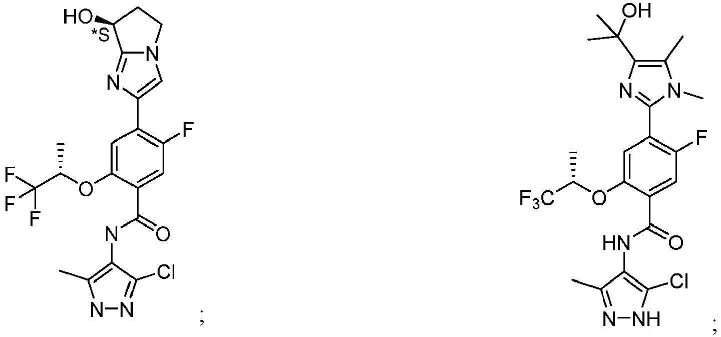 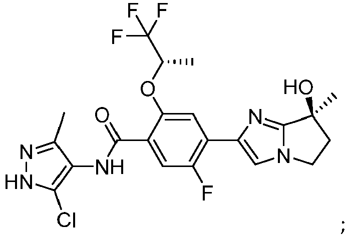 et 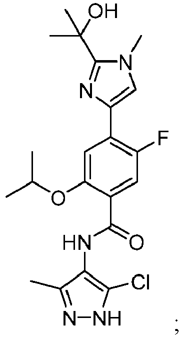 ou sel, solvate, stéréoisomère, variante isotopique ou N-oxyde pharmaceutiquement acceptable correspondant(e) ; ou
(d) 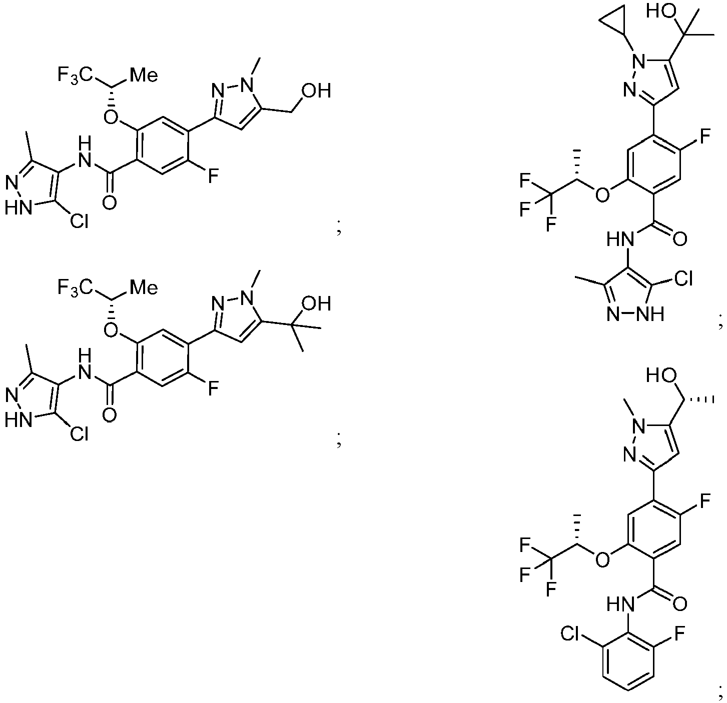 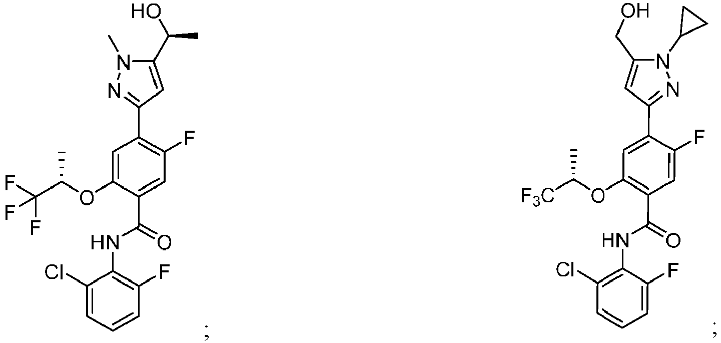 et 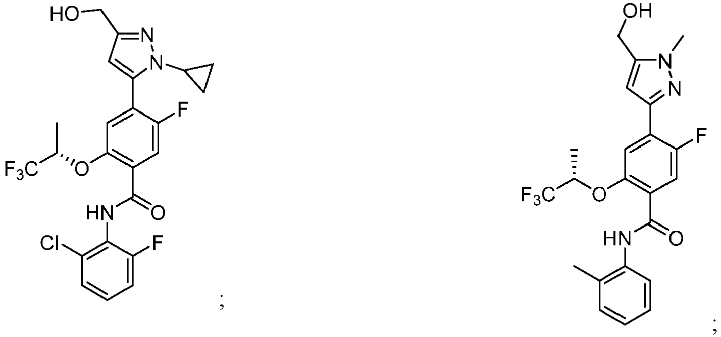 ou sel, solvate, stéréoisomère, variante isotopique ou N-oxyde pharmaceutiquement acceptable correspondant(e).

10. Composé selon la revendication 4 qui est : 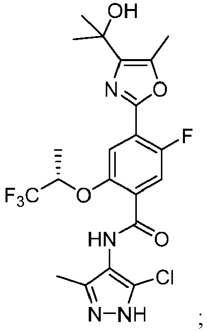 ou un sel, un solvate, un stéréoisomère, une variante isotopique ou un N-oxyde pharmaceutiquement acceptable correspondant(e) .

11. Composé selon la revendication 4 choisi dans le groupe constitué par :
(a) 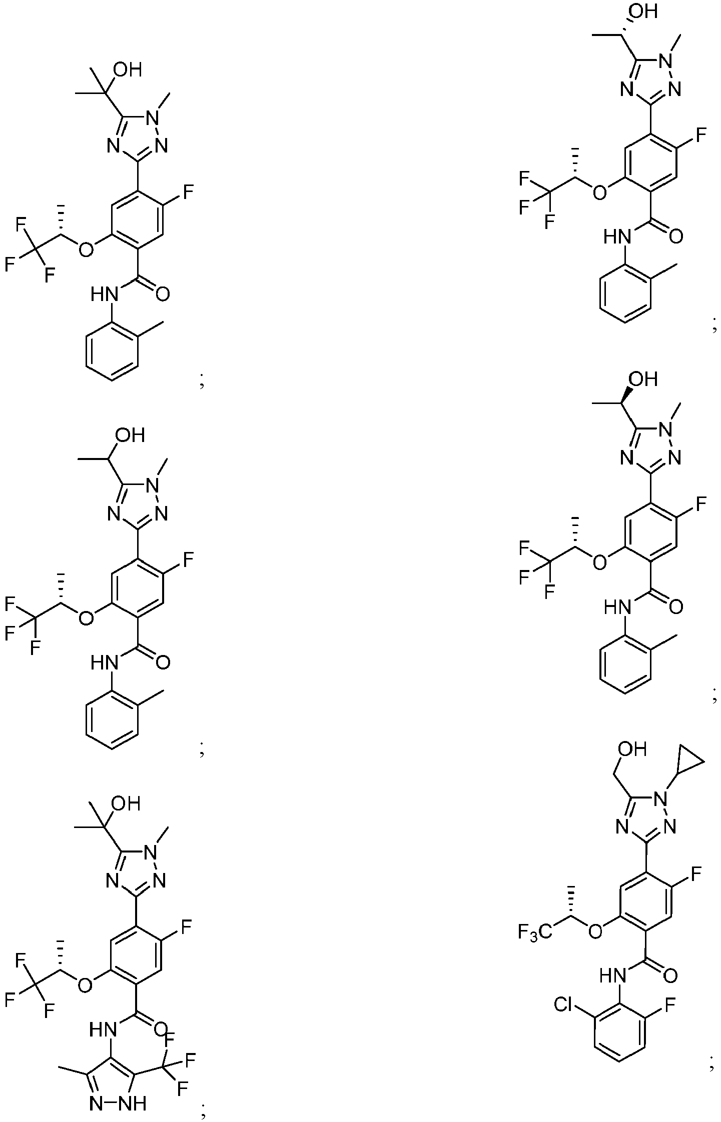 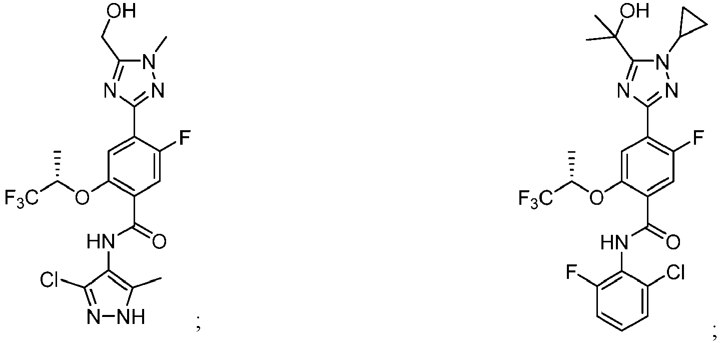 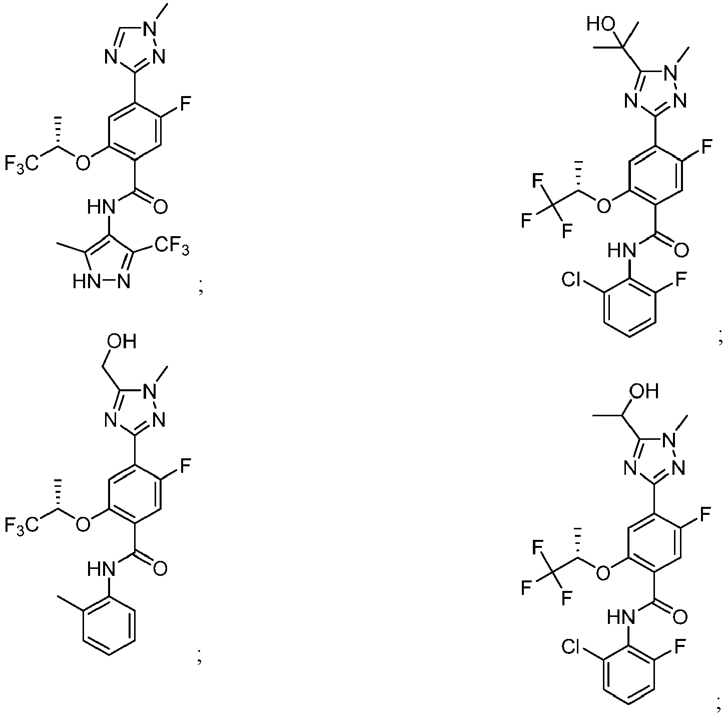 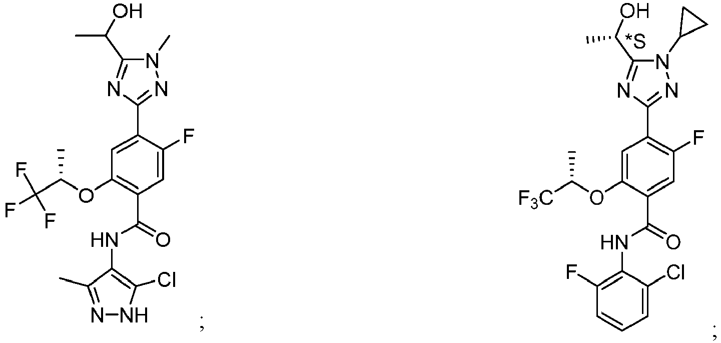 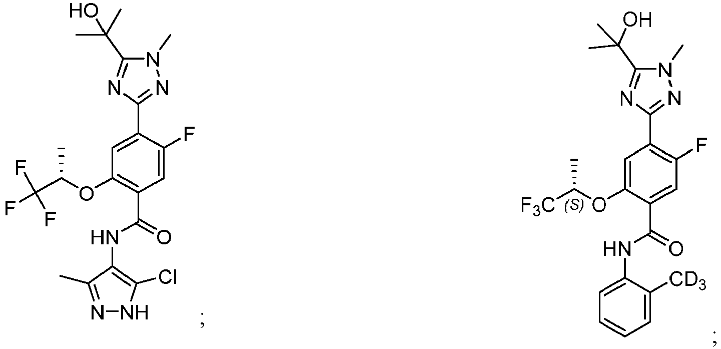 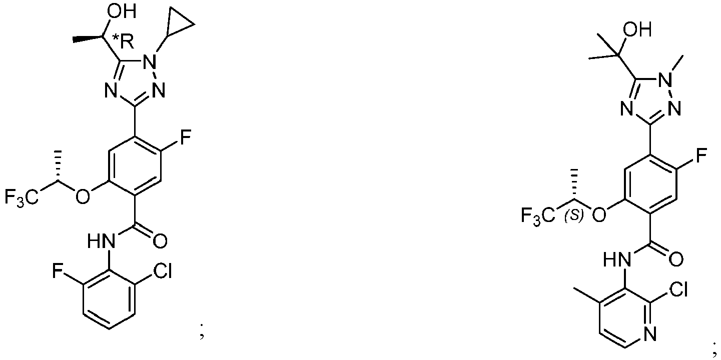 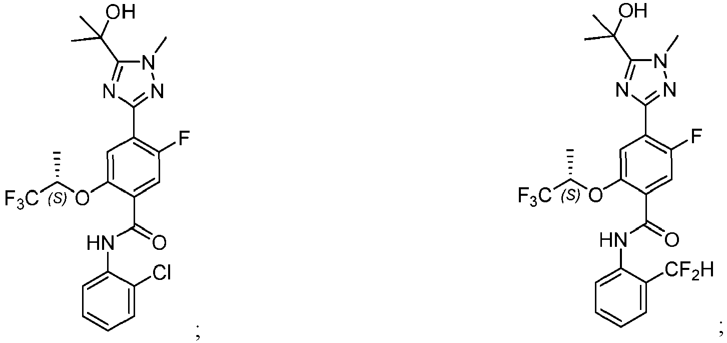 et 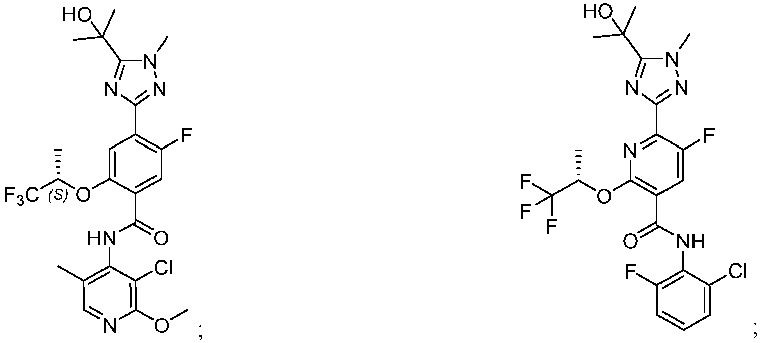 ou sel, solvate, stéréoisomère, variante isotopique ou N-oxyde pharmaceutiquement acceptable correspondant(e) ; ou
(b) 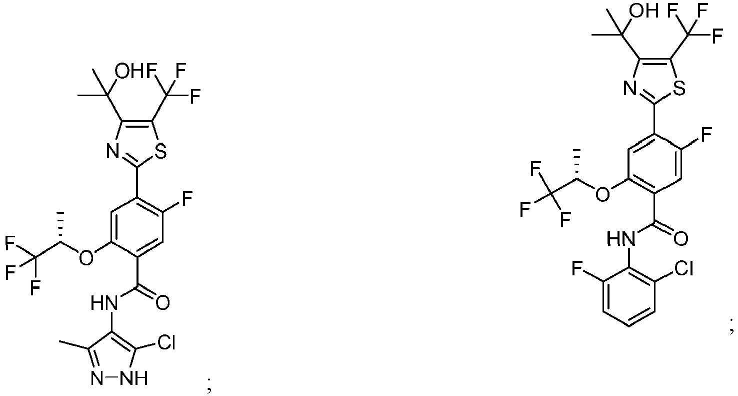 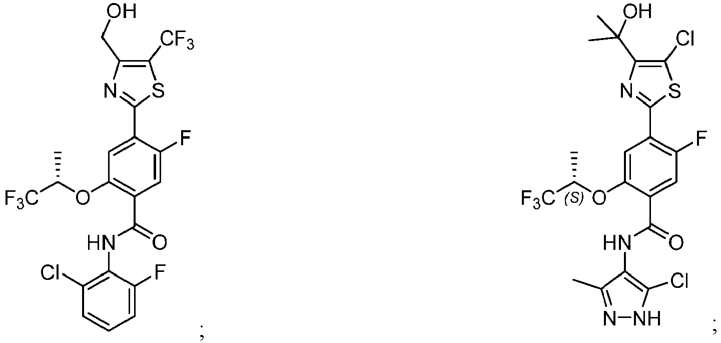 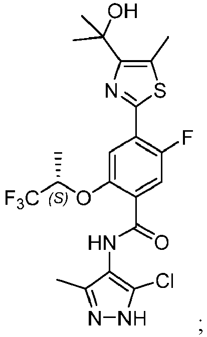 et ou sel, solvate, stéréoisomère, variante isotopique ou N-oxyde pharmaceutiquement acceptable correspondant(e) ; ou
(c) 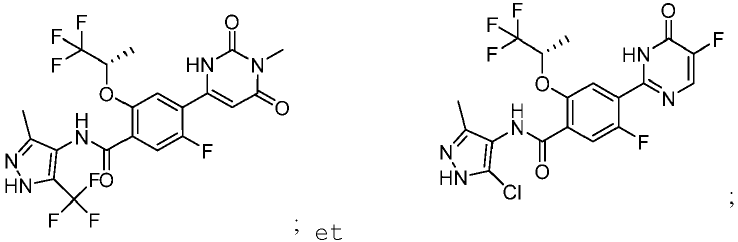 ou sel, solvate, stéréoisomère, variante isotopique ou N-oxyde pharmaceutiquement acceptable correspondant(e).

12. Composition pharmaceutique comprenant : (A) un composé selon l'une quelconque des revendications 1 à 11 ou un sel, un solvate, un stéréoisomère, une variante isotopique ou un N-oxyde pharmaceutiquement acceptable correspondant(e) ; et (B) au moins un excipient pharmaceutiquement acceptable.

13. Composition pharmaceutique comprenant une quantité efficace d'un composé selon la revendication 1 choisi dans le groupe constitué par :
(a) 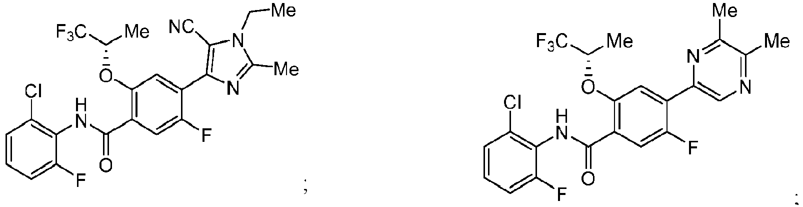 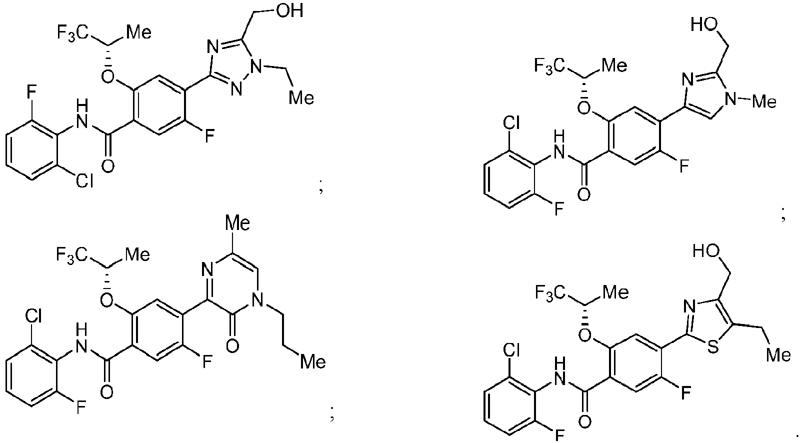 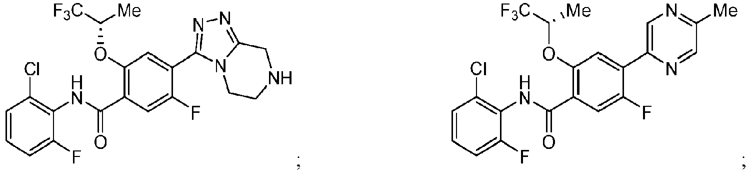 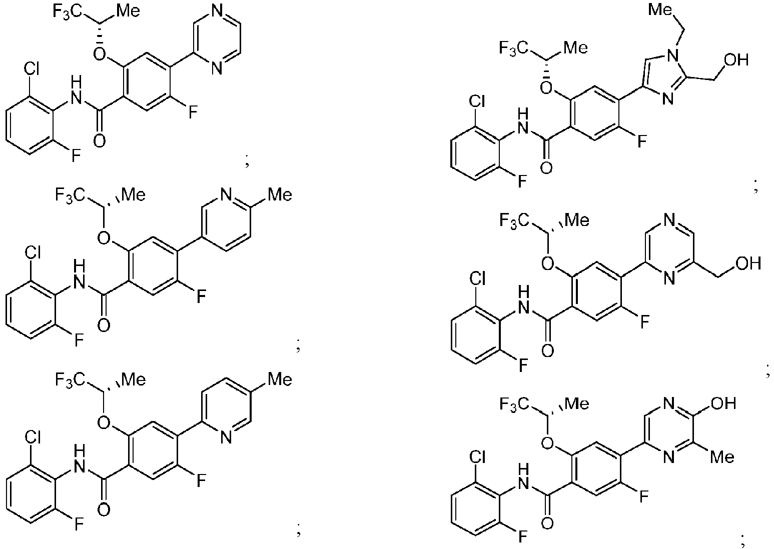 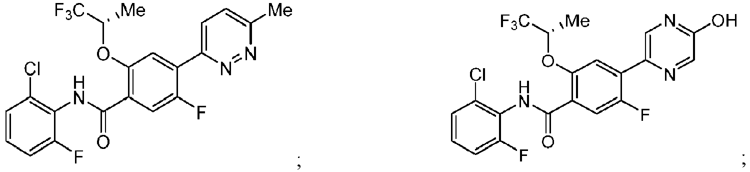 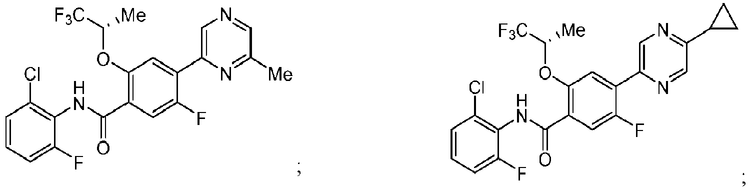 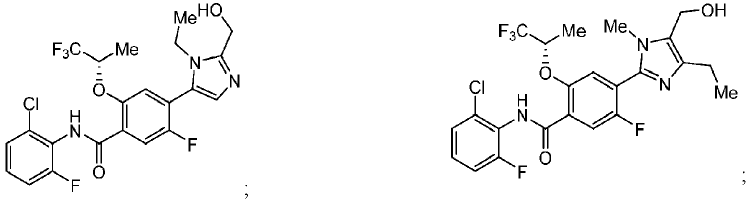 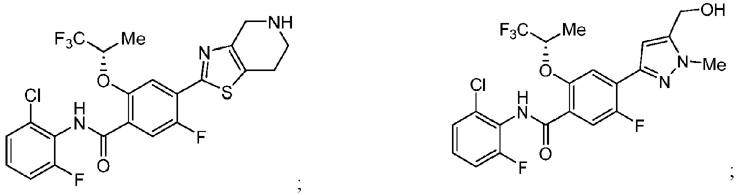 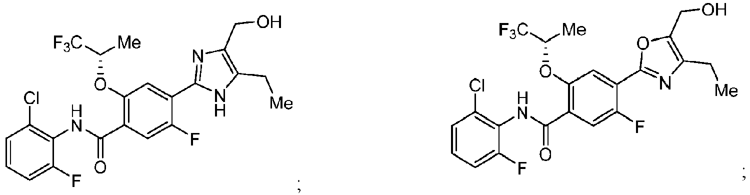 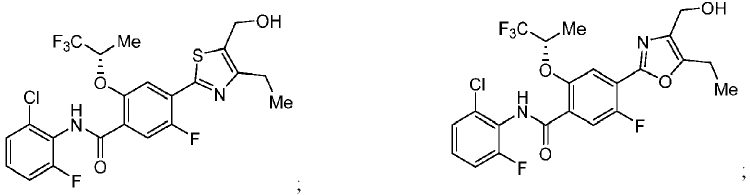 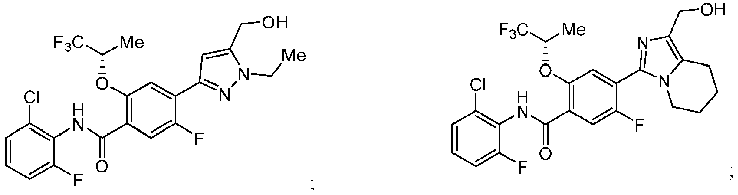 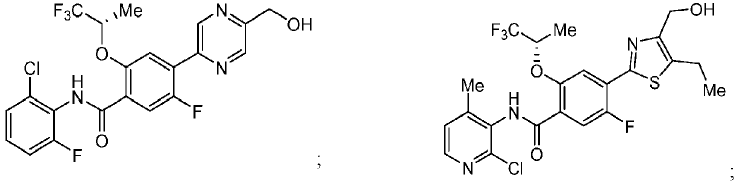 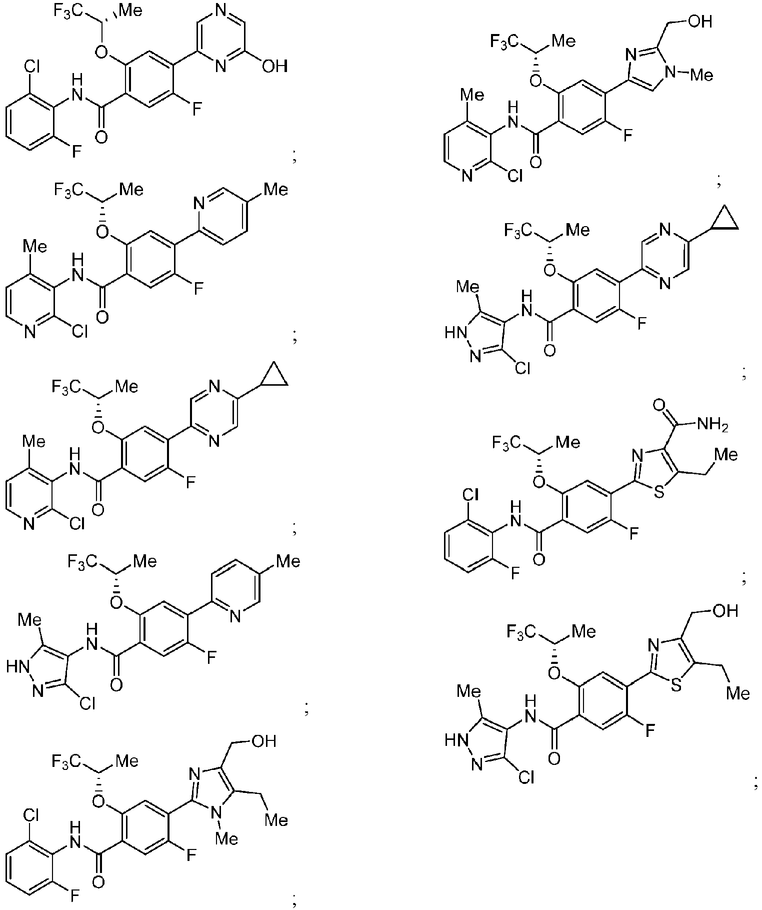 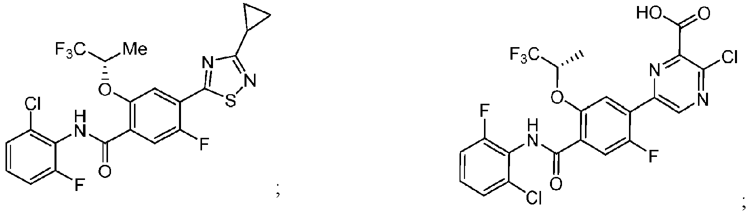 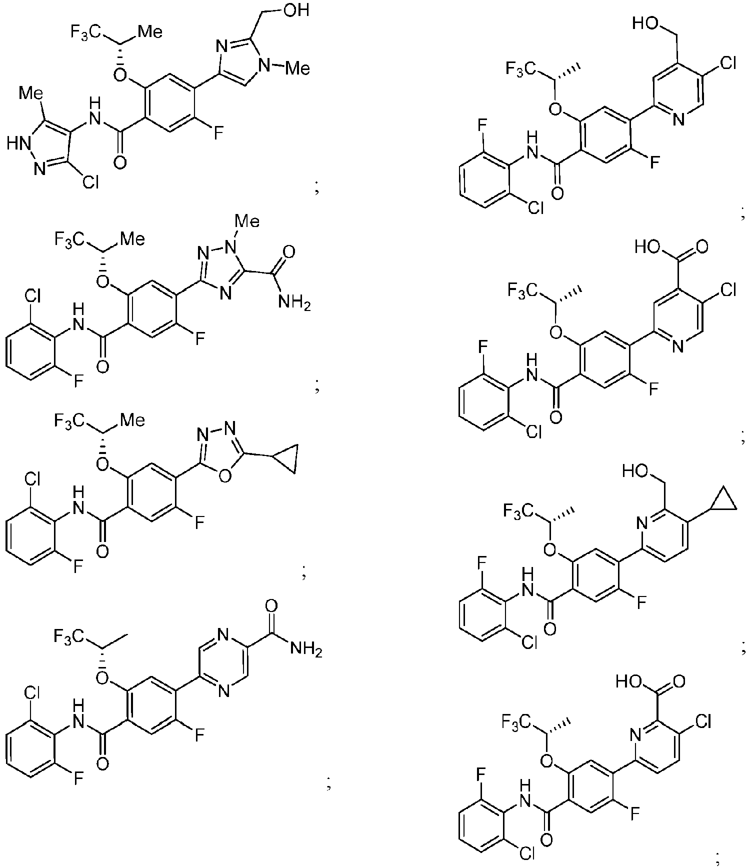 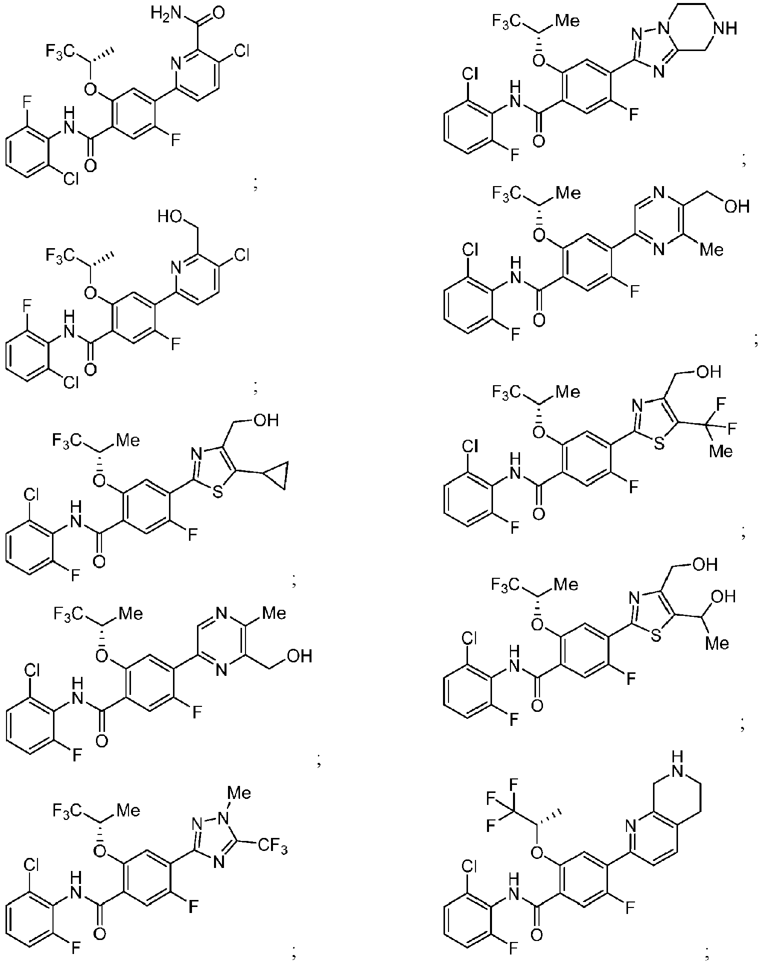 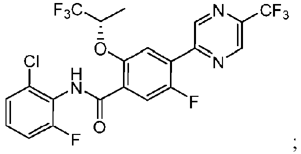 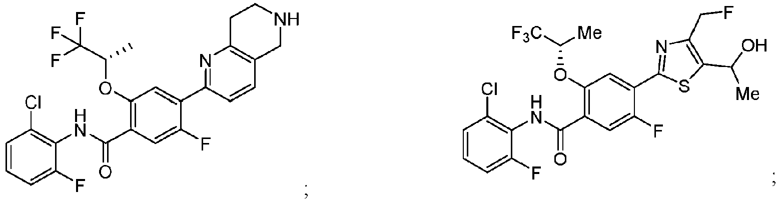 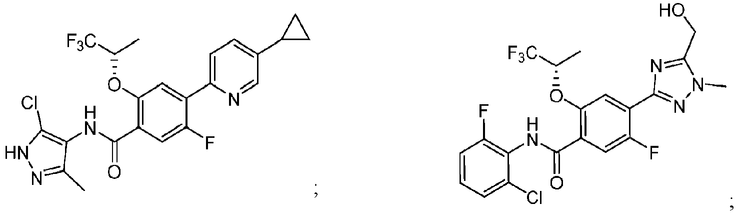 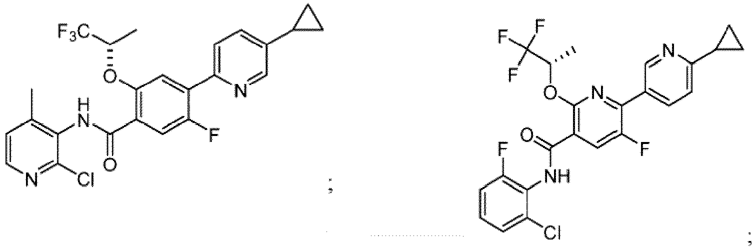 et 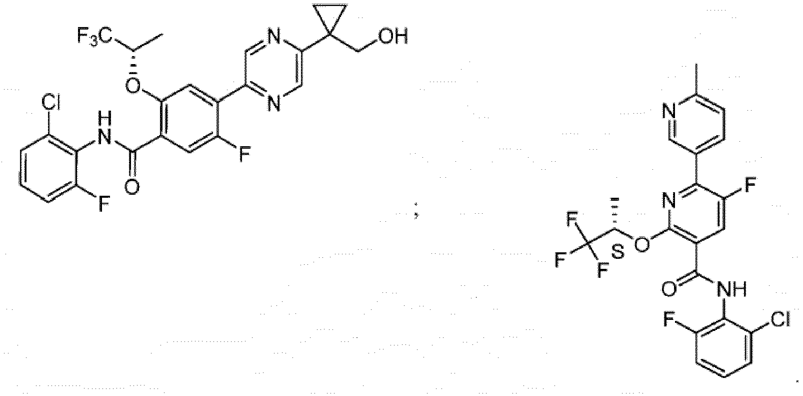 ou un sel, un solvate, un stéréoisomère, une variante isotopique ou un N-oxyde pharmaceutiquement acceptable correspondant(e) ; et au moins un excipient pharmaceutiquement acceptable ; ou
(b) 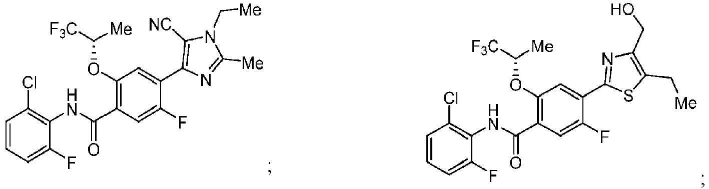 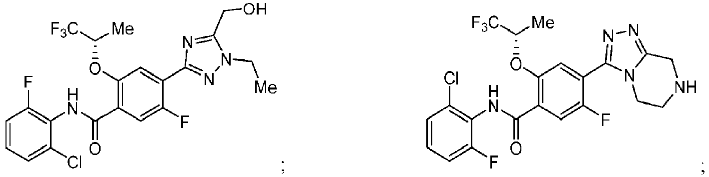 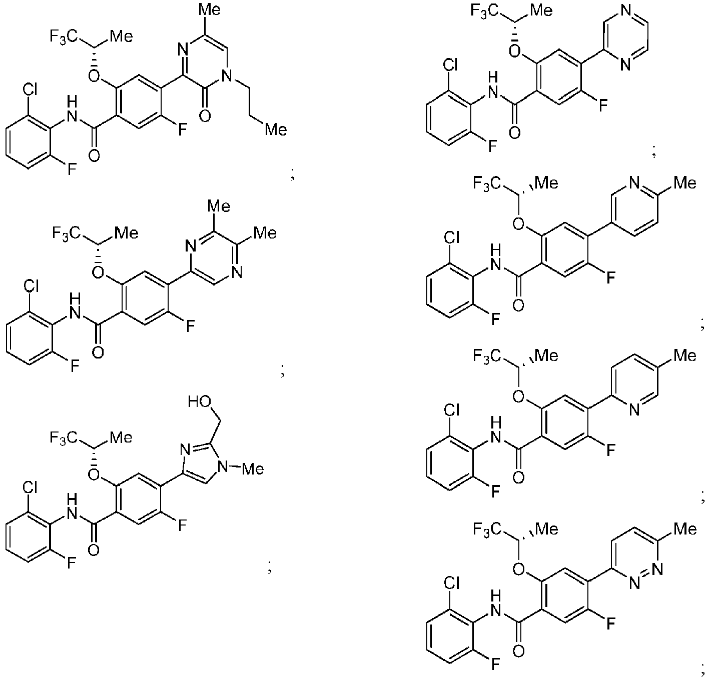 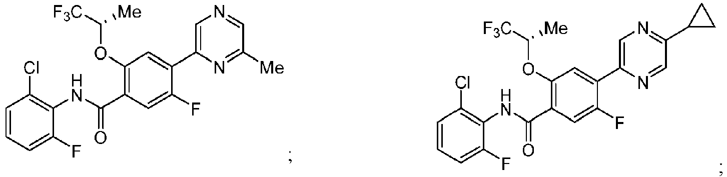 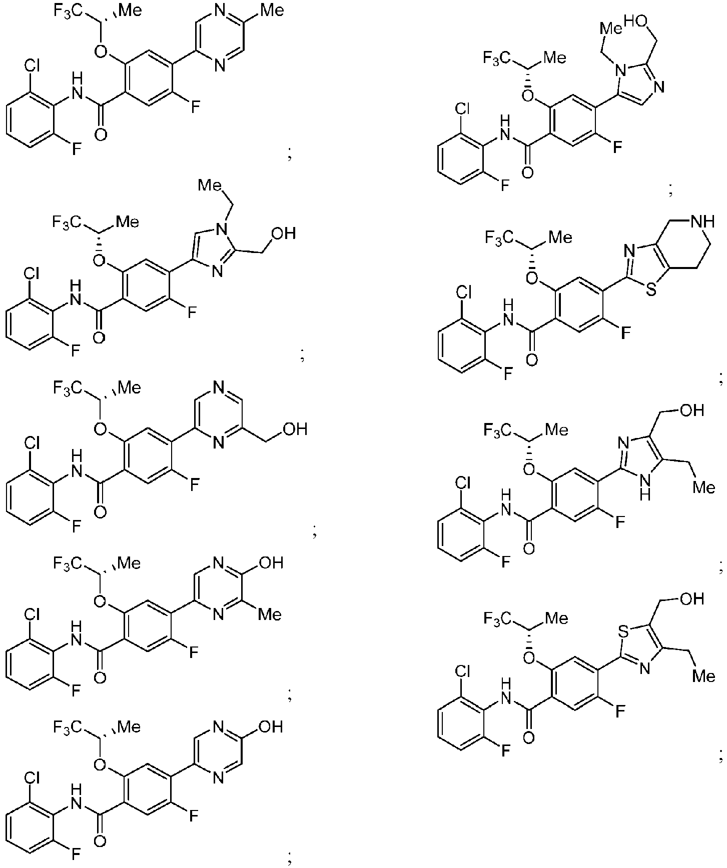 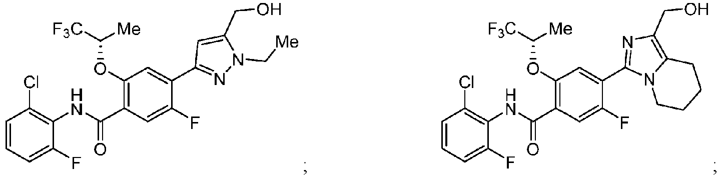 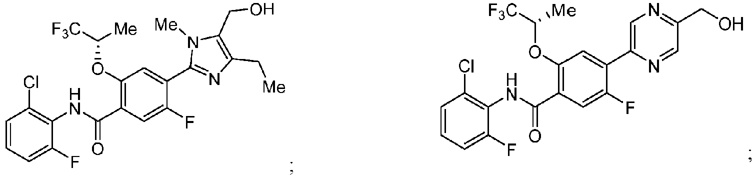 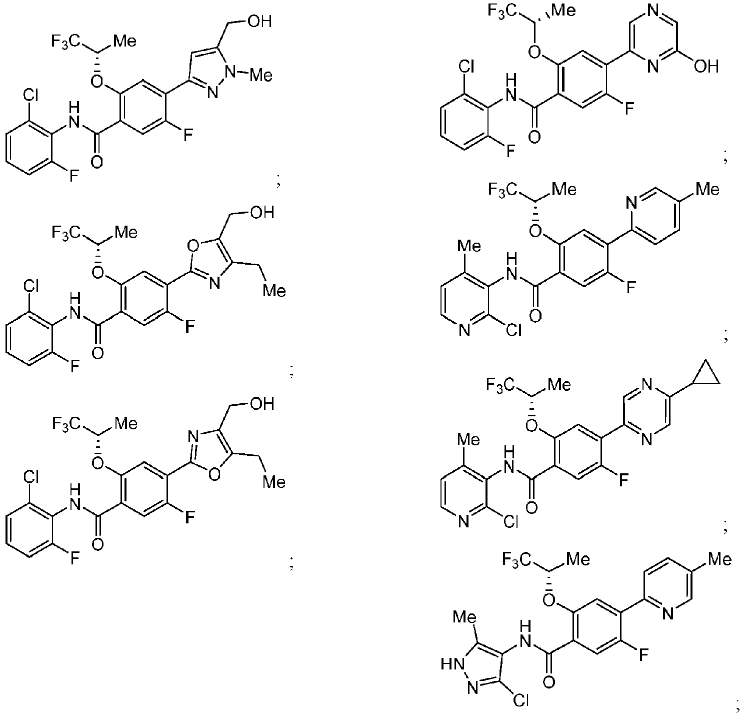 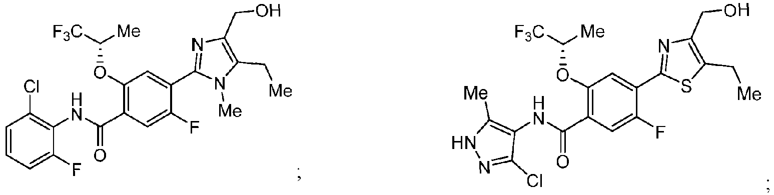 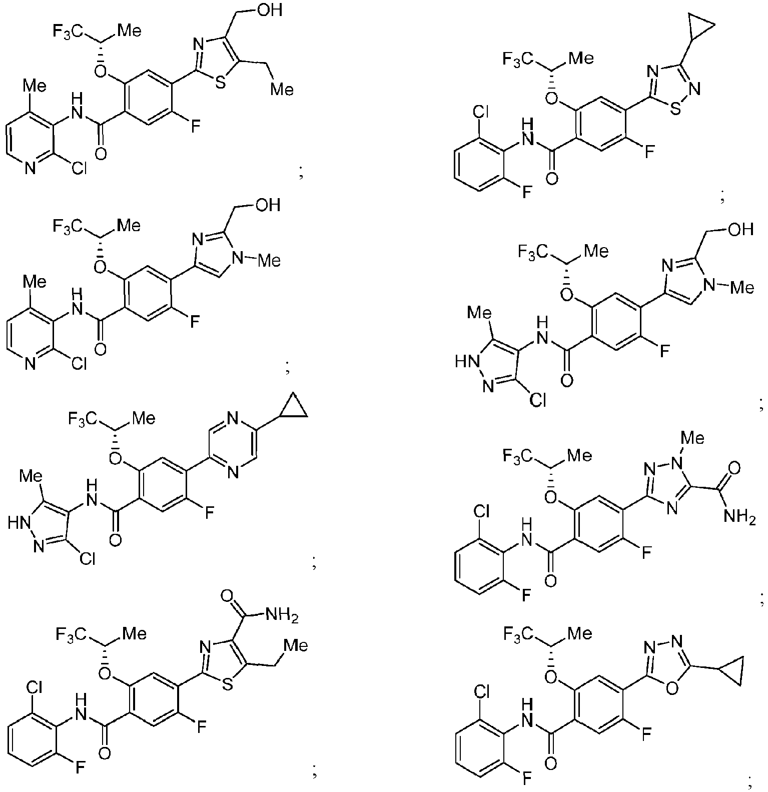 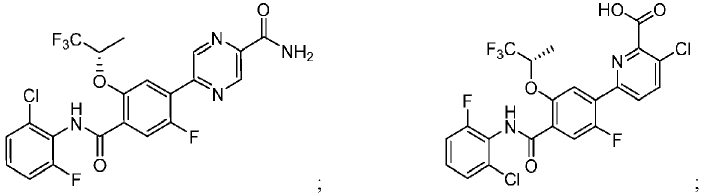 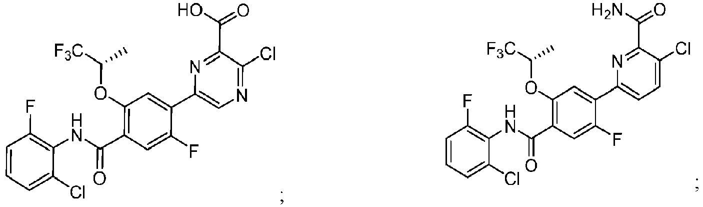 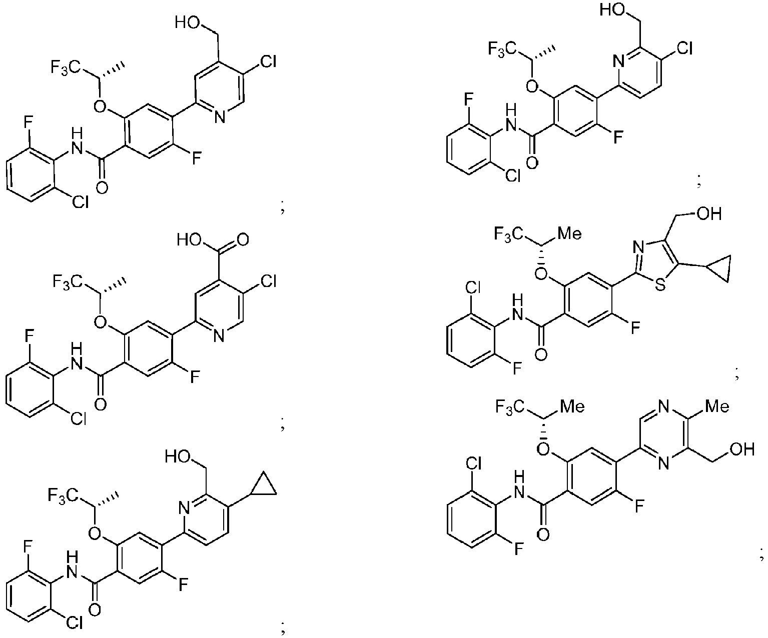 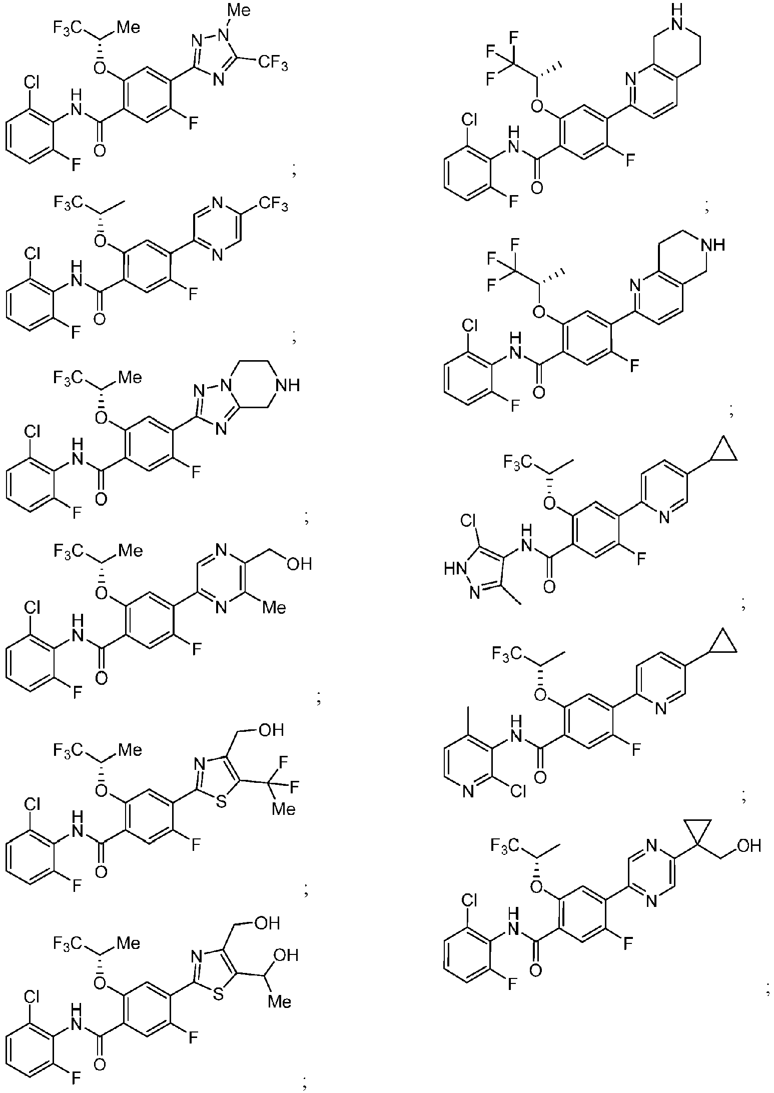 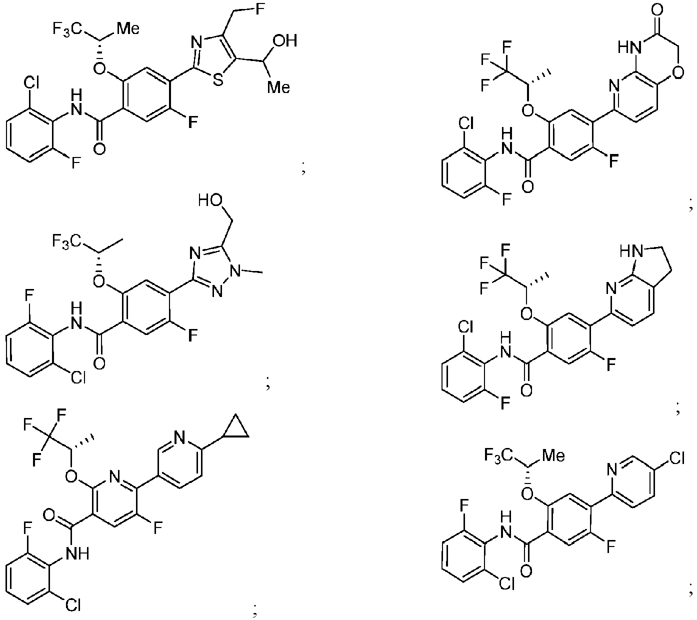 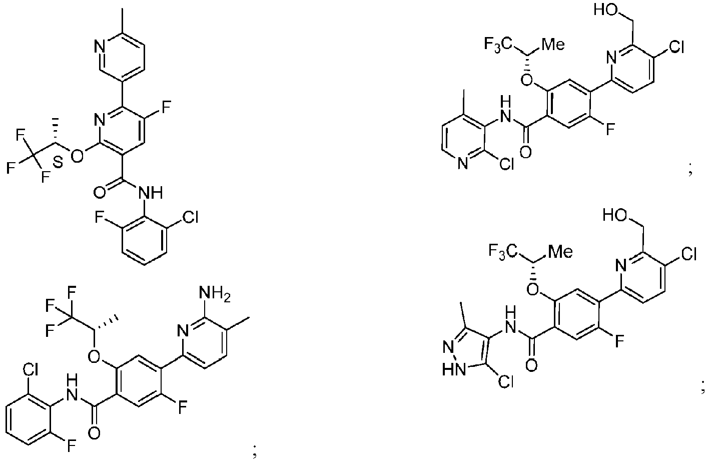 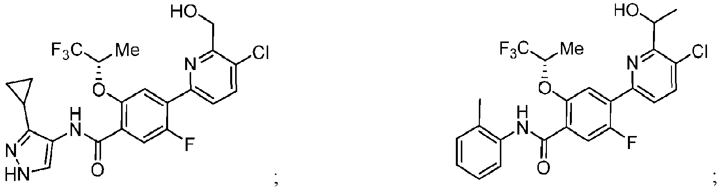 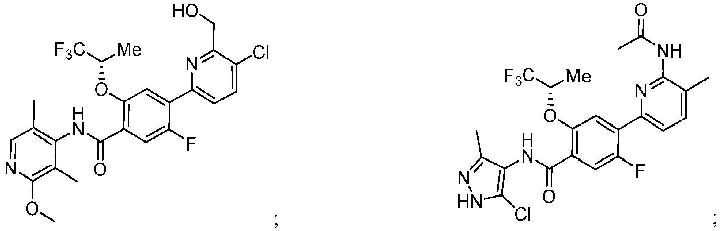 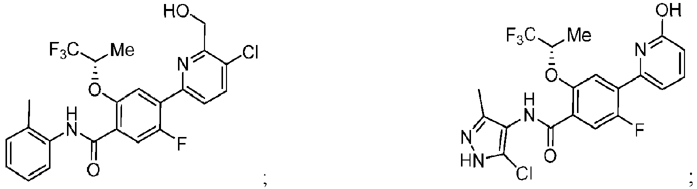 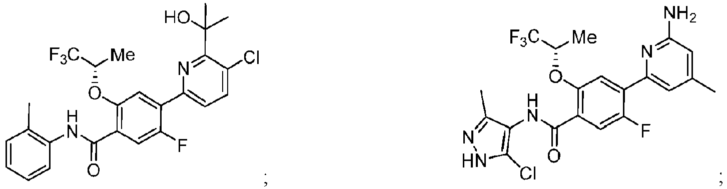 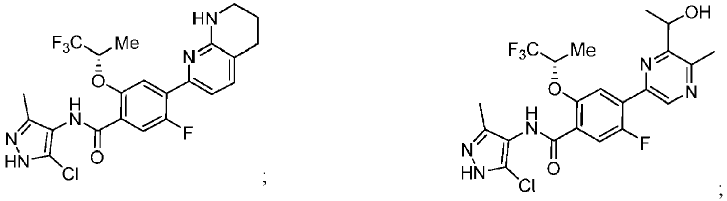 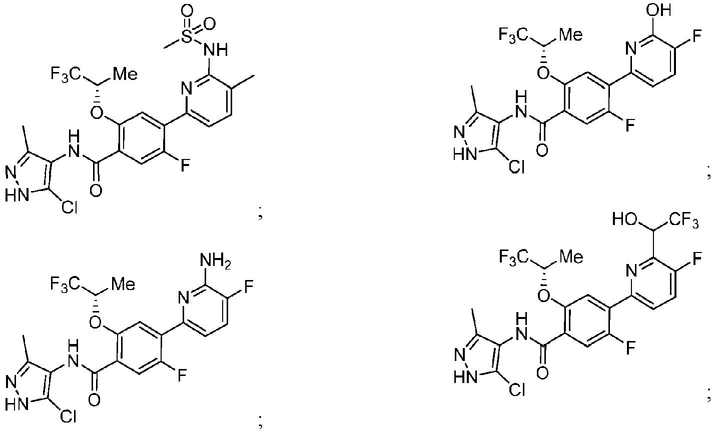 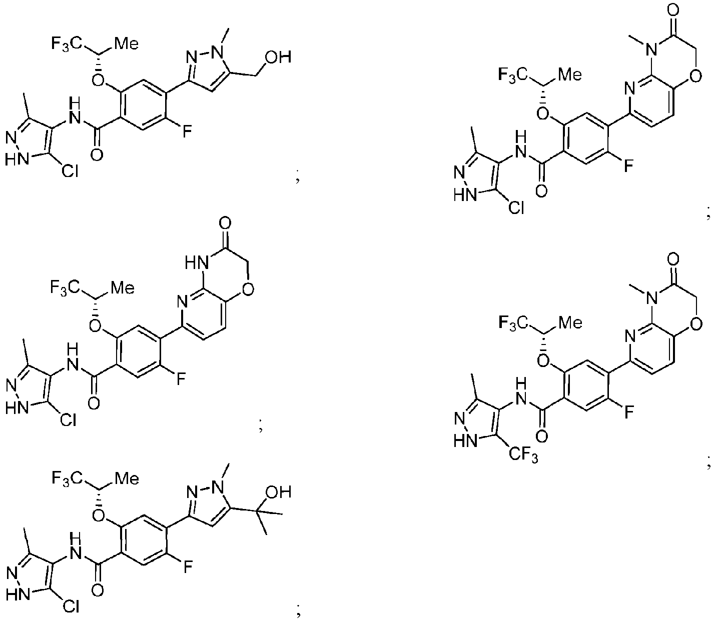 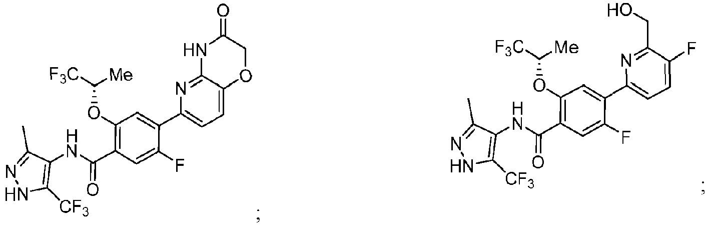 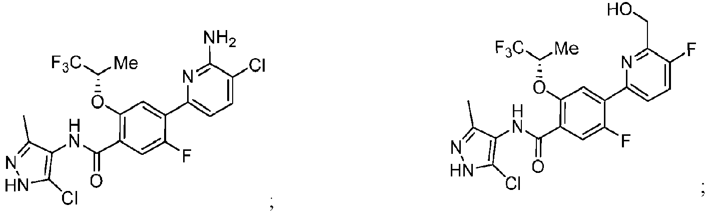 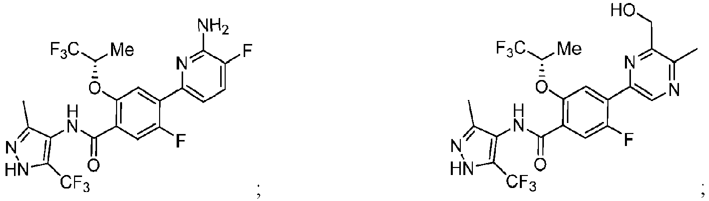 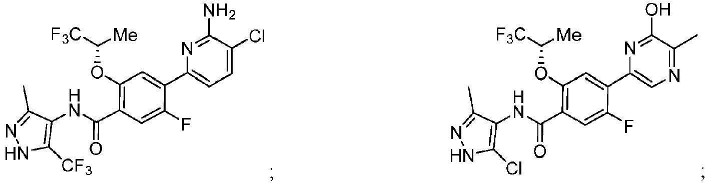 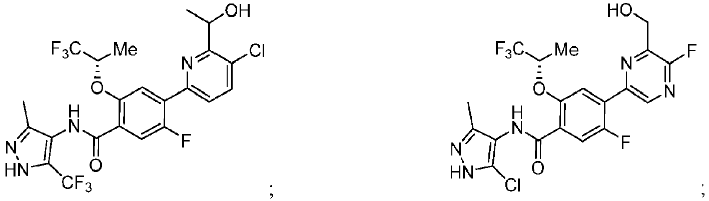 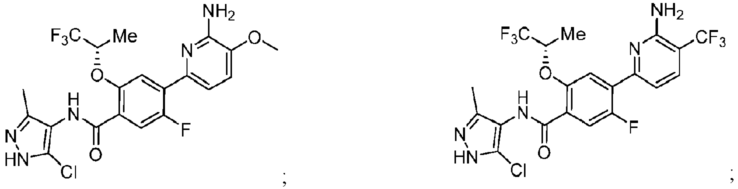 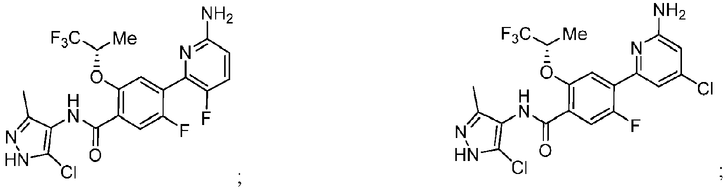 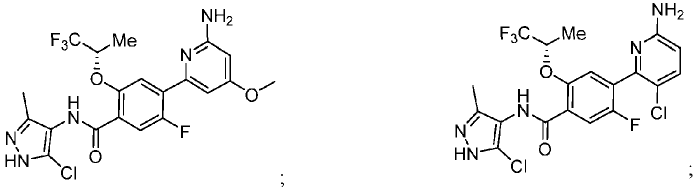 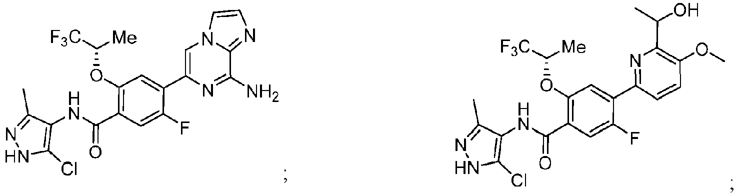 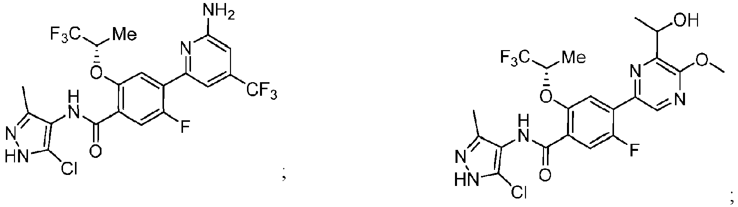 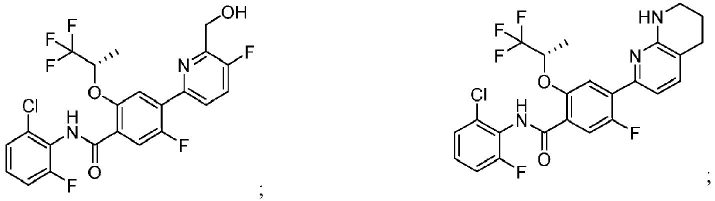 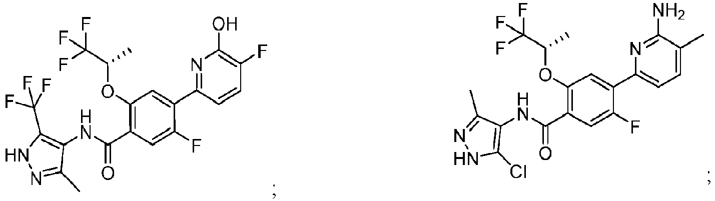 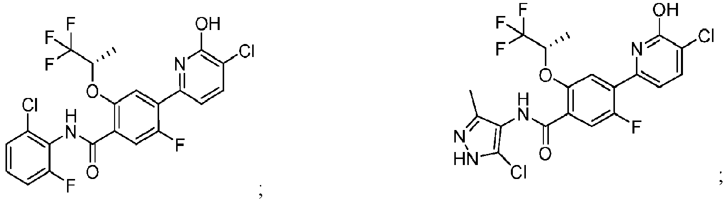 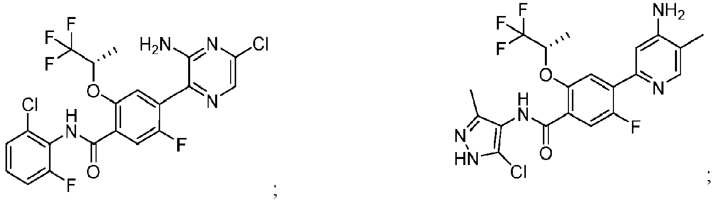 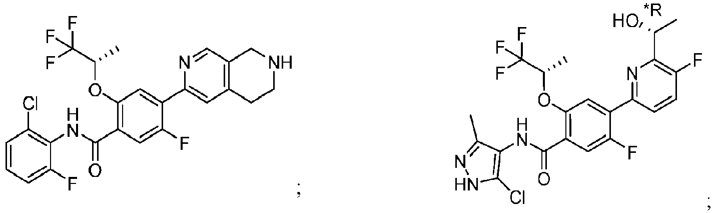 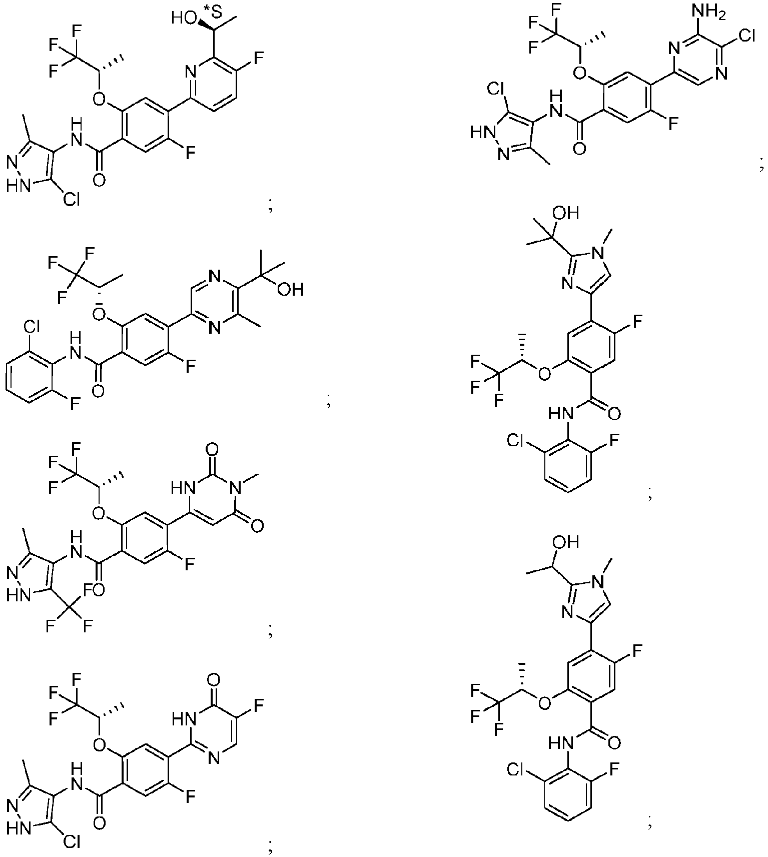 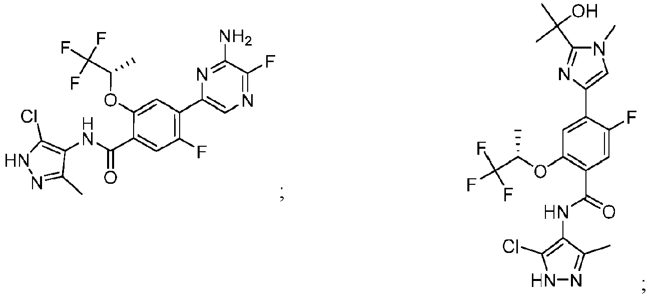 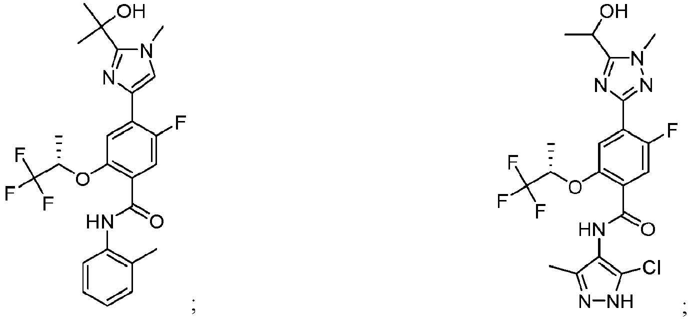 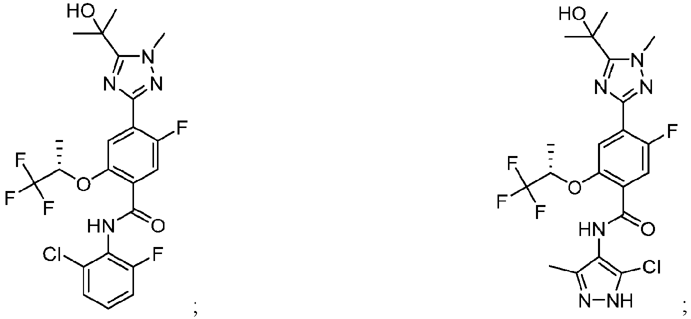 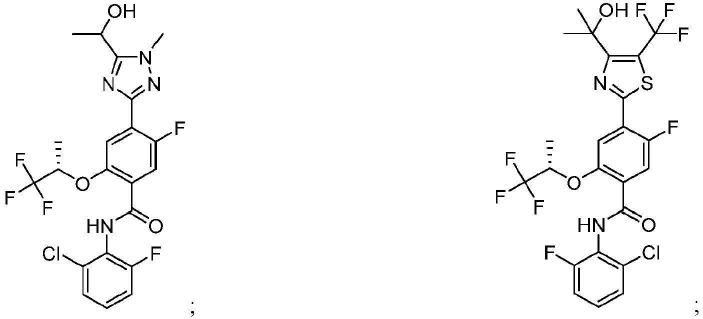 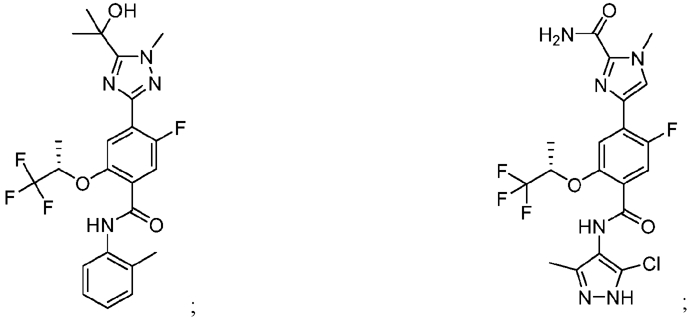 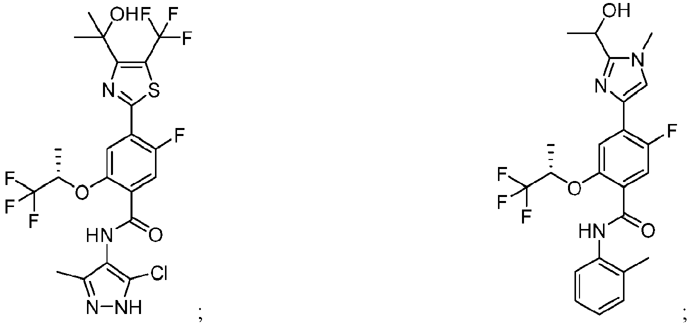 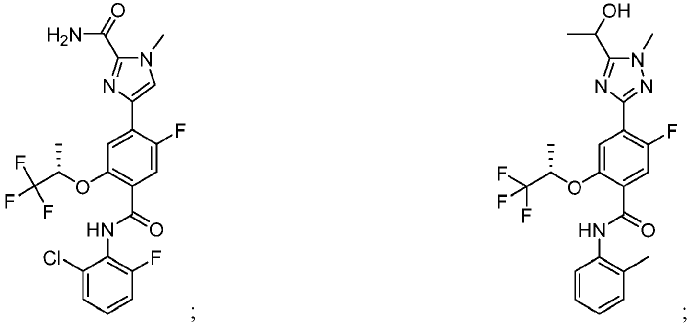 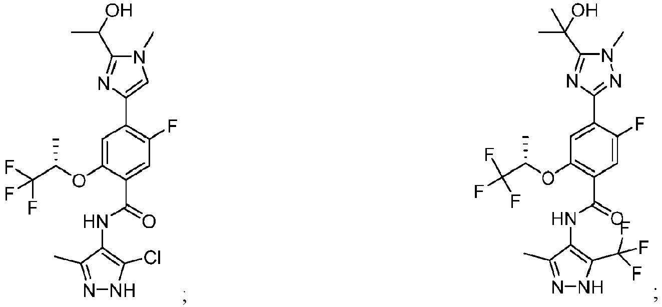 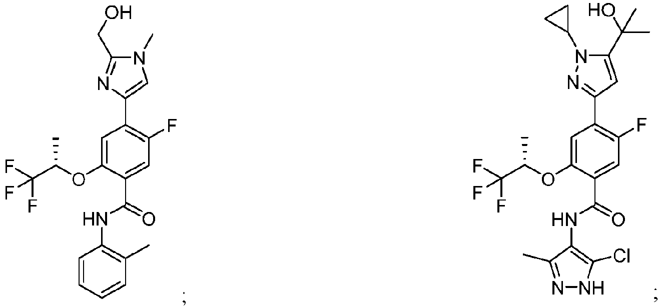 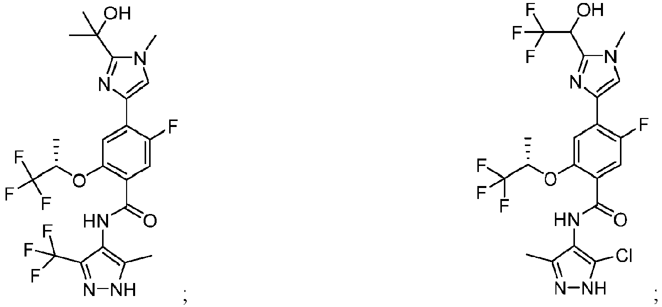 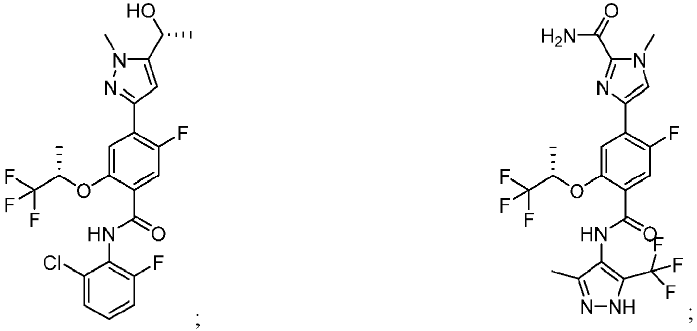 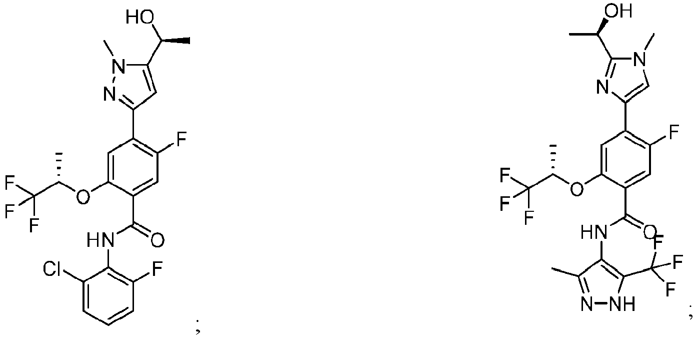 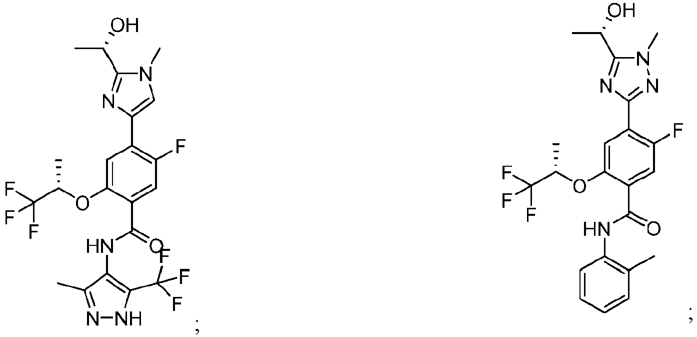 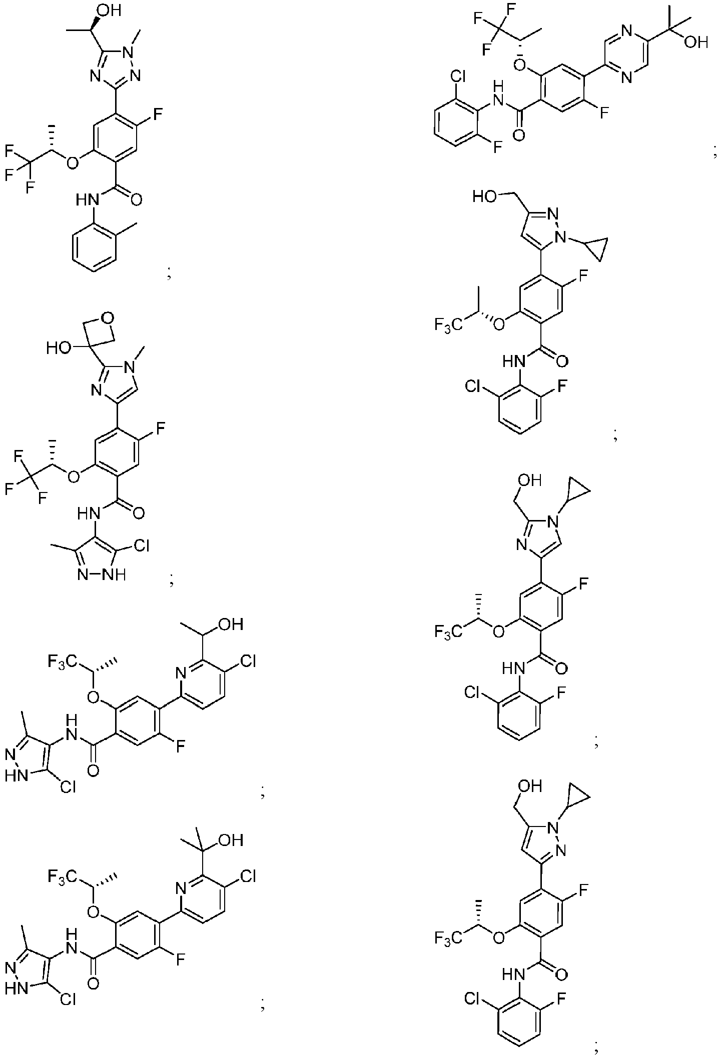 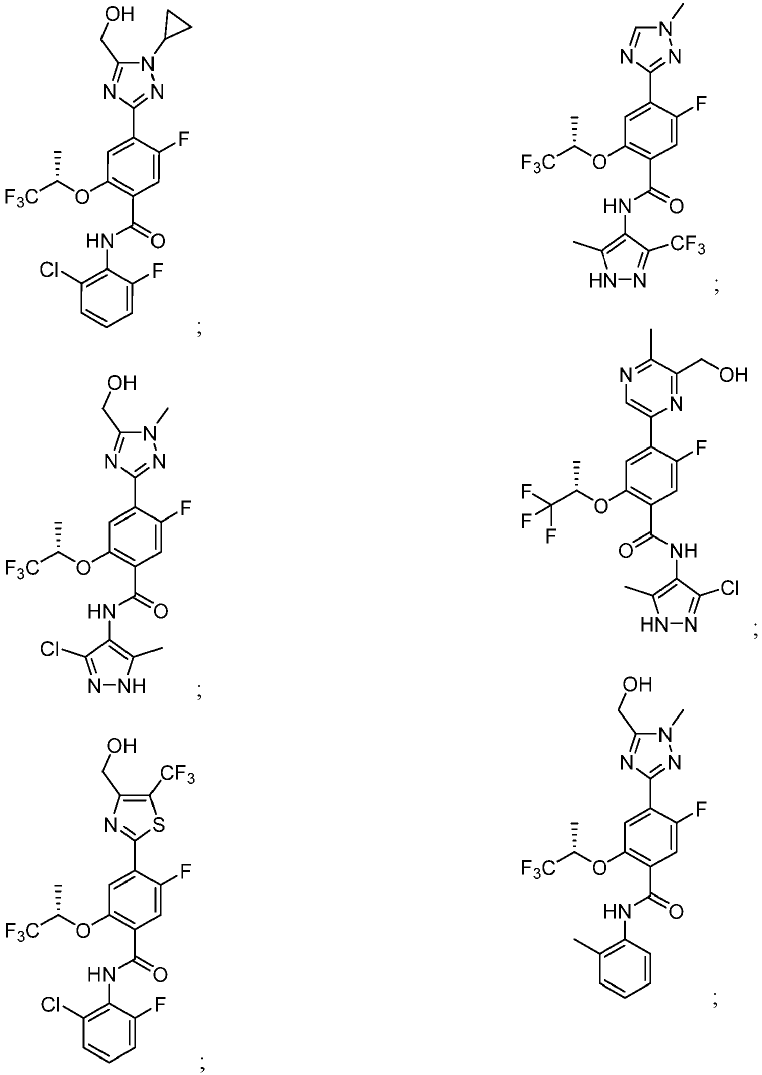 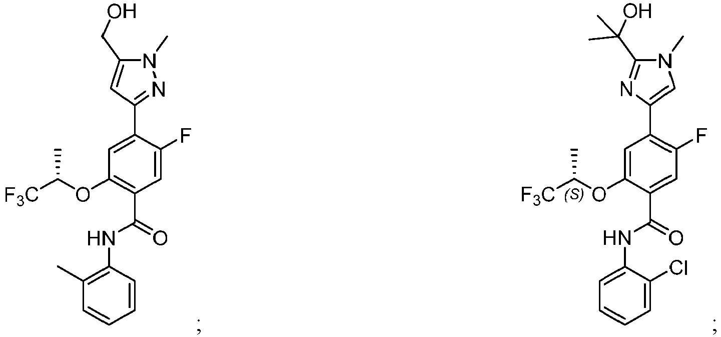 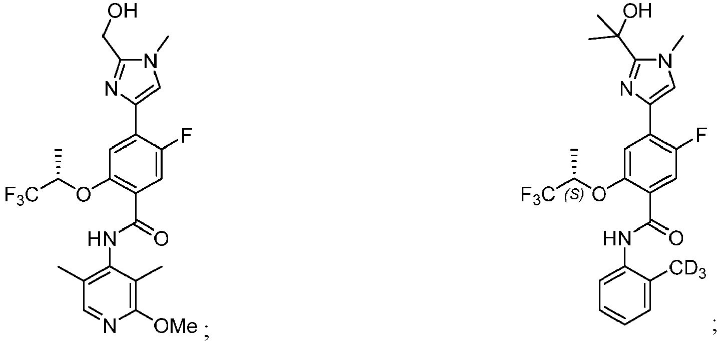 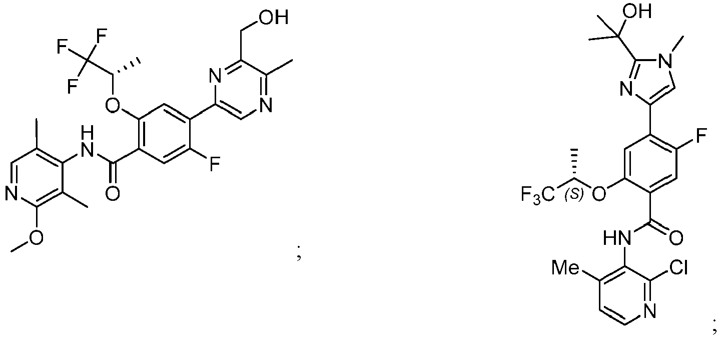 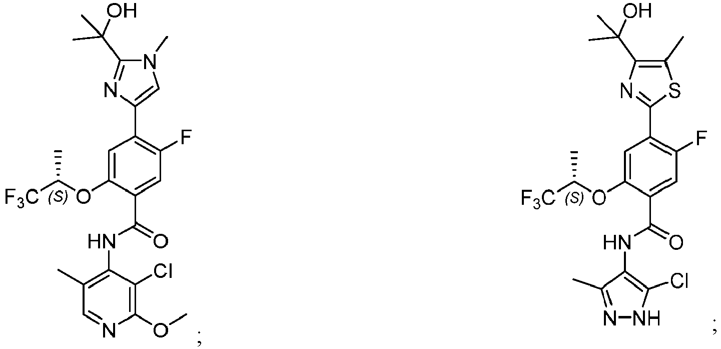 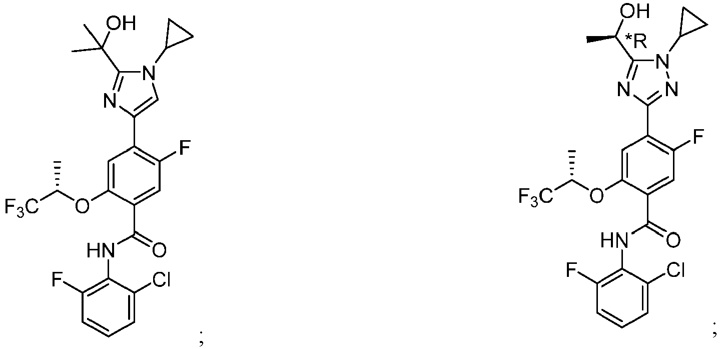 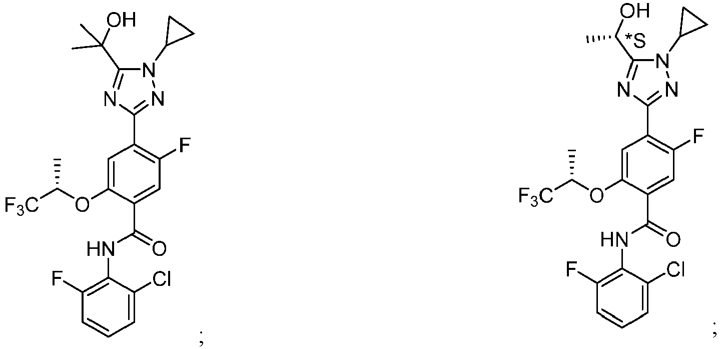 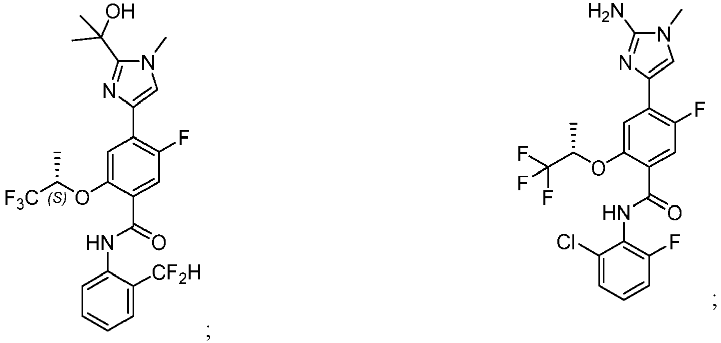 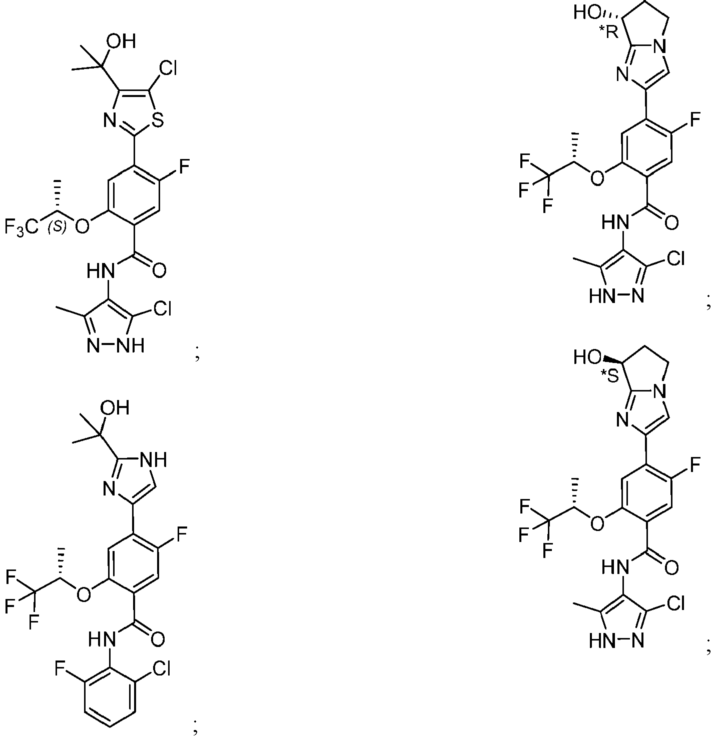 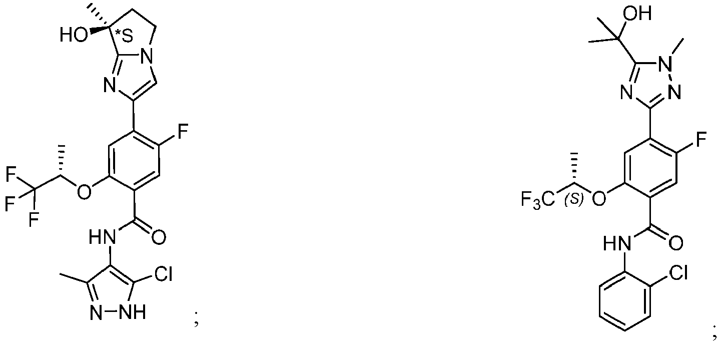 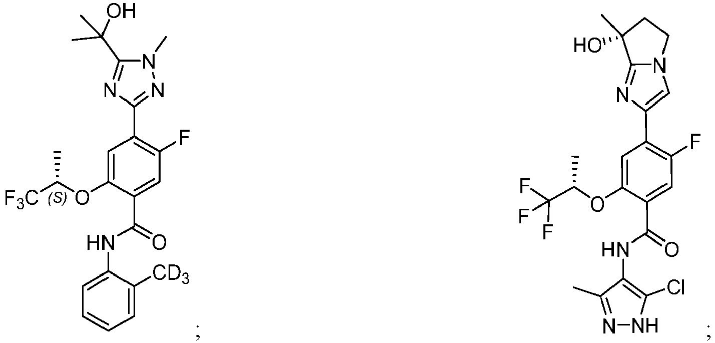 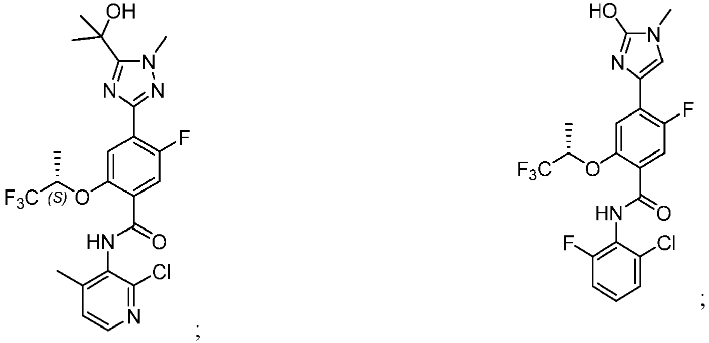 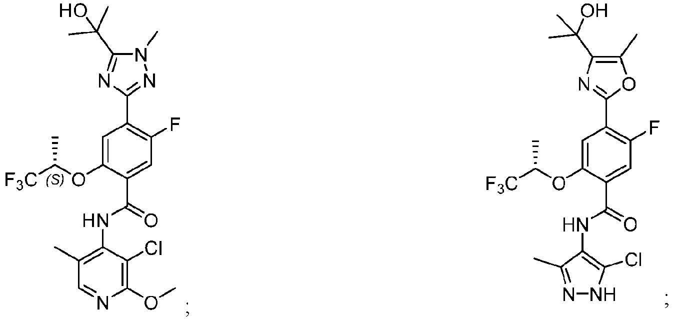 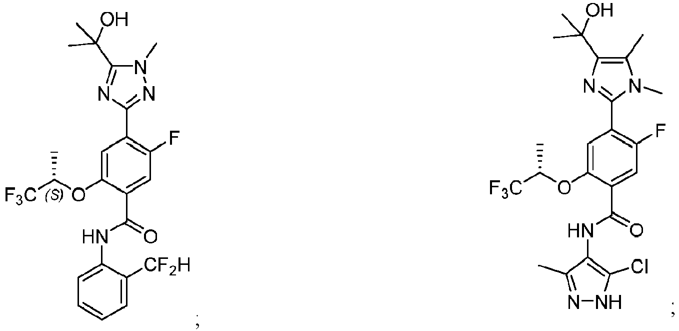 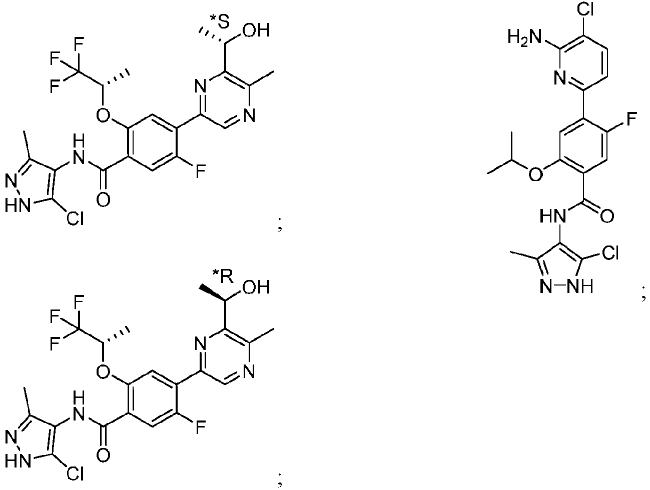 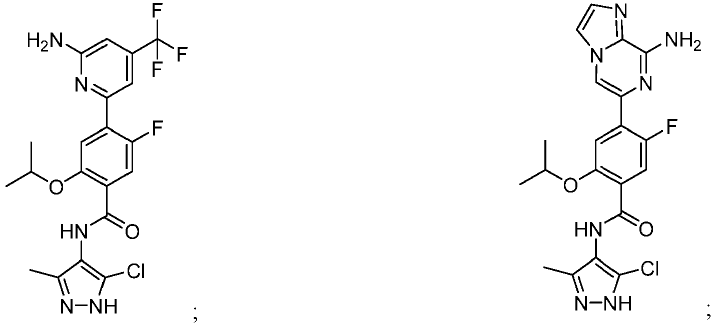 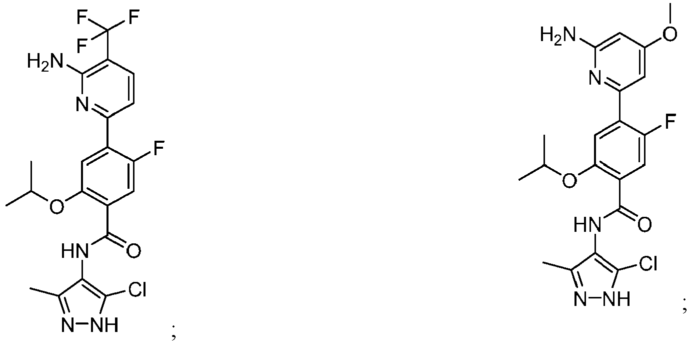 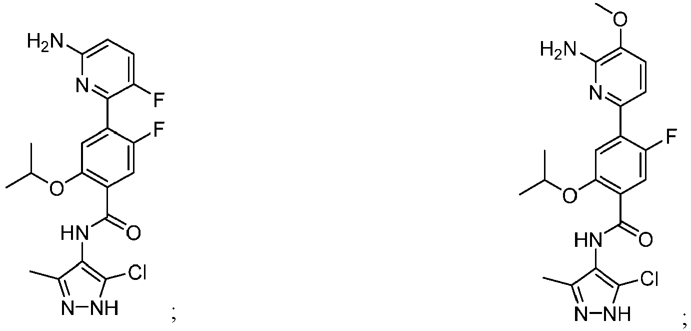 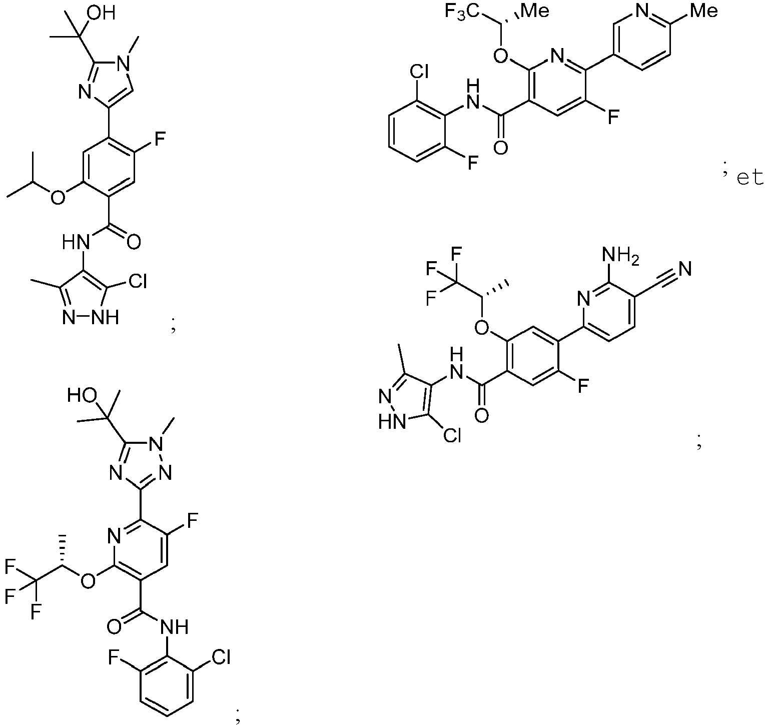 ou un sel, un solvate, un stéréoisomère, une variante isotopique ou un N-oxyde pharmaceutiquement acceptable correspondant(e) ; et au moins un excipient pharmaceutiquement acceptable.

14. Au moins un composé ou un sel, un solvate, un stéréoisomère, une variante isotopique ou un N-oxyde pharmaceutiquement acceptable correspondant(e) selon l'une quelconque des revendications 1 à 11 pour une utilisation dans le traitement d'un sujet souffrant ou diagnostiqué d'une maladie, d'un trouble ou d'une affection médicale comprenant l'inhibition ou la modification de l'activité de l'enzyme dihydroorotate oxygénase chez le sujet, le trouble, la maladie ou l'affection médicale étant choisi(e) dans le groupe constitué par : troubles inflammatoires, troubles auto-immuns et cancer.

15. Au moins un composé ou un sel, un solvate, un stéréoisomère, une variante isotopique ou un N-oxyde pharmaceutiquement acceptable correspondant(e) pour une utilisation selon la revendication 14, le trouble, la maladie ou l'affection médicale étant choisi(e) dans le groupe constitué par : lymphomes, leucémies, carcinomes et sarcomes.

16. Au moins un composé ou un sel, un solvate, un stéréoisomère, une variante isotopique ou un N-oxyde pharmaceutiquement acceptable correspondant(e) pour une utilisation selon la revendication 14, le trouble, la maladie ou l'affection médicale étant choisi(e) dans le groupe constitué par : leucémie lymphoblastique aiguë, leucémie myéloïde aiguë, leucémie à cellules T (aiguë), leucémie lymphoblastique aiguë, leucémie lymphocytaire aiguë, leucémie monocytaire aiguë, leucémie promyélocytaire aiguë, leucémie myélomonocytaire bisphénotypique B, leucémie lymphocytaire chronique, leucémie myélogène chronique, leucémie myéloïde chronique, leucémie myélomonocytaire chronique, leucémie lymphocytaire granulaire de grande taille, leucémie plasmocytaire et également syndrome myélodysplasique, qui peut se développer en leucémie myéloïde aiguë.

17. Au moins un composé ou un sel, un solvate, un stéréoisomère, une variante isotopique ou un N-oxyde pharmaceutiquement acceptable correspondant(e) pour une utilisation selon la revendication 14, le trouble, la maladie ou l'affection médicale étant une leucémie myéloïde aiguë.
